# EUROPEAN PATENT SPECIFICATION

(11) **EP 3 892 618 B1**
(45) Date of publication and mention of the grant of the patent: **10.04.2024**
(21) Application number: 20741322.0
(22) Date of filing: 14.01.2020
(51) Int. Cl.: C07D 401/12, C07D 413/12, A01N 43/40, A01N 43/653, A01N 43/713

(54) **4-PYRIDINYL FORMAMIDE COMPOUND OR DERIVATIVE THEREOF, PREPARATION METHOD THEREFOR, HERBICIDAL COMPOSITION AND USE THEREOF**
4-PYRIDINYLFORMAMIDVERBINDUNG ODER DERIVAT DAVON, HERSTELLUNGSVERFAHREN DAVON, HERBIZIDZUSAMMENSETZUNG UND VERWENDUNG DAVON
COMPOSÉ 4-PYRIDINYLE FORMAMIDE OU DÉRIVÉ DE CELUI-CI, PROCÉDÉ DE PRÉPARATION CORRESPONDANT, COMPOSITION HERBICIDE ET UTILISATION ASSOCIÉES

(30) Priority: 14.01.2019 CN 201910031819
(43) Date of publication of application: 13.10.2021
(73) Proprietor: Qingdao KingAgroot Chemical Compound Co., Ltd., Qingdao, Shandong 266000 (CN)
(72) Inventor: LIAN, Lei, Qingdao, Shandong 266000 (CN); HUA, Rongbao, Qingdao, Shandong 266000 (CN); PENG, Xuegang, Qingdao, Shandong 266000 (CN); ZHAO, De, Qingdao, Shandong 266000 (CN); CUI, Qi, Qingdao, Shandong 266000 (CN)
(74) Representative: Finnegan Europe LLP
(86) International application number: PCT/CN2020/071932
(87) International publication number: WO 2020/147705

(56) References cited:
- WO-A1-2012/123416
- WO-A1-2014/184074
- WO-A1-2018/050677
- CN-A- 104 220 434
- CN-A- 104 411 698
- CN-A- 104 854 104
- CN-A- 105 073 719
- CN-A- 105 658 067
- DATABASE REGISTRY 30 May 2019 (2019-05-30), Database accession no. 2320606-21-9
- DATABASE REGISTRY 14 December 2017 (2017-12-14), Database accession no. 2158120-95-5
- DATABASE REGISTRY 8 December 2017 (2017-12-08), Database accession no. 2154210-09-8
- DATABASE REGISTRY 3 December 2017 (2017-12-03), Database accession no. 2150258-78-7
- DATABASE REGISTRY 16 September 2016 (2016-09-16), Database accession no. 1994473-87-8
- DATABASE REGISTRY 9 September 2016 (2016-09-09), Database accession no. 1990275-01-8
- DATABASE REGISTRY 30 August 2016 (2016-08-30), Database accession no. 1983206-31-0
- DATABASE REGISTRY 28 August 2016 (2016-08-28), Database accession no. 1981525-56-7
- DATABASE REGISTRY 28 August 2016 (2016-08-28), Database accession no. 1981512-07-5
- DATABASE REGISTRY 28 August 2016 (2016-08-28), Database accession no. 1981512-01-9
- DATABASE REGISTRY 28 August 2016 (2016-08-28), Database accession no. 1981497-90-8
- DATABASE REGISTRY 28 August 2016 (2016-08-28), Database accession no. 1981419-87-7
- DATABASE REGISTRY 28 August 2016 (2016-08-28), Database accession no. 1981397-34-5
- DATABASE REGISTRY 28 August 2016 (2016-08-28), Database accession no. 1981395-51-0
- DATABASE REGISTRY 28 August 2016 (2016-08-28), Database accession no. 1981395-42-9
- DATABASE REGISTRY 28 August 2016 (2016-08-28), Database accession no. 1981392-81-7
- DATABASE REGISTRY 28 August 2016 (2016-08-28), Database accession no. 1981372-05-7
- DATABASE REGISTRY 28 August 2016 (2016-08-28), Database accession no. 1981189-00-7
- DATABASE REGISTRY 28 August 2016 (2016-08-28), Database accession no. 1981186-17-7
- DATABASE REGISTRY 28 August 2016 (2016-08-28), Database accession no. 1981173-34-5
- DATABASE REGISTRY 28 August 2016 (2016-08-28), Database accession no. 1981170-79-9
- DATABASE REGISTRY 28 August 2016 (2016-08-28), Database accession no. 1981135-59-4
- DATABASE REGISTRY 28 August 2016 (2016-08-28), Database accession no. 1981004-73-2
- DATABASE REGISTRY 28 August 2016 (2016-08-28), Database accession no. 1981004-05-0
- DATABASE REGISTRY 28 August 2016 (2016-08-28), Database accession no. 1981001-84-6
- DATABASE REGISTRY 28 August 2016 (2016-08-28), Database accession no. 1981001-76-6
- DATABASE REGISTRY 18 August 2016 (2016-08-18), Database accession no. 1974653-47-8
- DATABASE REGISTRY 18 August 2016 (2016-08-18), Database accession no. 1974551-12-6
- DATABASE REGISTRY 12 August 2016 (2016-08-12), Database accession no. 1972455-84-7
- DATABASE REGISTRY 17 June 2016 (2016-06-17), Database accession no. 1933955-97-5
- DATABASE REGISTRY 14 June 2016 (2016-06-14), Database accession no. 1931544-41-0
- DATABASE REGISTRY 12 June 2016 (2016-06-12), Database accession no. 1929905-53-2
- DATABASE REGISTRY 10 June 2016 (2016-06-10), Database accession no. 1929180-35-7
- DATABASE REGISTRY 22 May 2016 (2016-05-22), Database accession no. 1915305-47-3
- DATABASE REGISTRY 20 May 2016 (2016-05-20), Database accession no. 1914838-15-5
- DATABASE REGISTRY 20 May 2016 (2016-05-20), Database accession no. 1914474-00-2
- DATABASE REGISTRY 10 July 2015 (2015-07-10), Database accession no. 1798578-76-3
- DATABASE REGISTRY 10 July 2015 (2015-07-10), Database accession no. 1798480-74-6
- DATABASE REGISTRY 3 June 2015 (2015-06-03), Database accession no. 1772342-99-0
- DATABASE REGISTRY 24 May 2015 (2015-05-24), Database accession no. 1711184-58-5
- DATABASE REGISTRY 14 May 2015 (2015-05-14), Database accession no. 1704011-60-8
- DATABASE REGISTRY 12 May 2015 (2015-05-12), Database accession no. 1602529-54-3
- DATABASE REGISTRY 2 May 2014 (2014-05-02), Database accession no. 1595886-66-0
- DATABASE REGISTRY 27 December 2013 (2013-12-27), Database accession no. 1504812-52-5
- DATABASE REGISTRY 17 December 2013 (2013-12-17), Database accession no. 1497292-38-2
- DATABASE REGISTRY 5 December 2013 (2013-12-05), Database accession no. 1487135-99-8
- DATABASE REGISTRY 27 November 2013 (2013-11-27), Database accession no. 1482249-37-5

## Description

### Technical Field

The invention belongs to the technical field of pesticides, and specifically relates to a 4-pyridinyl formamide compound or derivative thereof, preparation method therefor, herbicidal composition and use thereof.

### Technical background

Weed control is one of the most important links in the course of achieving high-efficiency agriculture. Various herbicides are available in the market, for example, WO2013087577A1 etc. disclose certain arylformamide compounds and their use as herbicides. However, due to the continuous expansion of the market, the resistance of weeds, the service life of chemicals and the economical efficiency of chemicals, as well as the increasing emphasis on the environment, especially the resistance of the mainstream ALS inhibitor and ACCe inhibitor herbicides is becoming more and more serious. In rice fields and wheat fields, more and more efficient, selective herbicides that can solve resistance are needed. This requires scientists to continuously research and develop new high-efficiency, safe, economic herbicides with different action modes.

### Invention contents

The present invention provides a 4-pyridinyl formamide compound or derivative thereof, preparation method therefor, herbicidal composition and use thereof. The compound has excellent herbicidal activity and a higher crop safety, and establishes good selectivity, especially for key crops such as wheat and rice.

The technical solution adopted by the invention is as follows:
A 4-pyridinyl formamide compound or derivative thereof, as shown in Formula I:

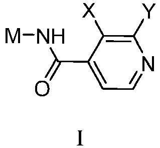

Wherein, X, and Y each independently represent nitro, halogen, cyano, formyl, thiocyano, mercapto; alkyl, alkenyl, alkynyl, cycloalkyl, cycloalkenyl, cycloalkylalkyl, or cycloalkenylalkyl, which is with or without halogen; OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -alkyl-C(S)R¹, -alkyl-C(S)OR¹, -alkyl-C(O)SR¹, -alkyl-C(S)SR¹, -alkyl-SC(O)R¹, -alkyl-OC(S)R¹, -alkyl-SC(S)R¹, -O-alkyl-NR⁴R⁵, -S-alkyl-NR⁴R⁵, -alkyl-O-alkyl-NR⁴R⁵, -alkyl-S-alkyl-NR⁴R⁵, -alkyl-(C=S)ₙ-NR⁴R⁵, -NH-alkyl-NR⁴R⁵, -alkyl-OR¹, -alkyl-COR¹, -alkyl-CO₂R¹, -alkyl-OCOR¹, -alkyl-NR³COR¹, -alkyl-SO₂OR¹, -alkyl-NR³SO₂R², -alkyl-OSO₂R², -alkyl-S(O)ₘR², -alkyl-CONR⁴R⁵, -alkyl-SO₂NR⁴R⁵, NR⁴R⁵, 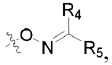 P(O)(OR⁶)₂, CH₂P(O)(OR⁶)₂, SO₂NR⁴R⁵-alkyl-S(O)ₘR², -alkyl-CN, -alkylaryl, -alkylheteroaryl, -alkylheterocyclyl, aryl, heteroaryl, or heterocyclyl;
R¹, R³, R⁴, and R⁵ each independently represent hydrogen, aryl, arylalkyl, heteroaryl, heteroarylalkyl, alkyl, halogenated alkyl, alkenyl, halogenated alkenyl, alkynyl, halogenated alkynyl, cycloalkyl, halogenated cycloalkyl, alkoxyalkyl, or cycloalkylalkyl, wherein the last 10 groups as mentioned are each substituted by s groups selected from the group consisting of cyano, halogen, nitro, thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and alkoxyalkoxycarbonyl;
R² represents aryl, arylalkyl, heteroaryl, heteroarylalkyl, alkyl, alkenyl, alkynyl, cycloalkyl, or cycloalkylalkyl, wherein the last 5 groups as mentioned are each substituted by s groups selected from the group consisting of cyano, halogen, nitro, thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and alkoxyalkoxycarbonyl;
R⁶ represents methyl, or ethyl;
R⁷, and R⁸ each independently represent hydrogen, alkyl, alkenyl, or alkynyl;
R⁹ represents alkyl, alkenyl, or alkynyl; M represents 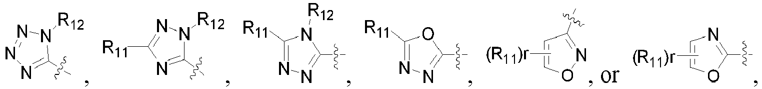 which is unsubstituted or substituted;
R₁₁ represents hydrogen, halogen, cyano, nitro, alkyl which is unsubstituted or substituted with a substituent selected from R₁₃, cycloalkyl which is unsubstituted or substituted with a substituent selected from R₁₄, alkenyl, halogenated alkenyl, alkynyl, halogenated alkynyl, cycloalkenyl, NH₂, aminoacyl, carboxyl, alkoxyalkoxycarbonyl, OR₁₅, -alkyl-OR₁₅, C(O)R₁₆, -alkyl-C(O)R₁₆, C(O)OR₁₆, -alkyl-C(O)OR₁₆, S(O)ₘR₁₆, -alkyl-S(O)ₘR₁₆, -N(R₁₆)₂, -NHR₁₆, -C(O)NHR₁₆, -C(O)N(R₁₆)₂, -NHC(O)R₁₇, heterocyclyl, heterocyclylalkyl, heterocyclyloxy, heterocyclylcarbonyl, aryl, arylalkyl, aryloxy, arylcarbonyl, heteroaryl, heteroarylalkyl, heteroaryloxy, or heteroarylcarbonyl;
R₁₂ represents hydrogen, alkyl which is unsubstituted or substituted with a substituent selected from R₁₈, cycloalkyl, halogenated cycloalkyl, alkenyl, halogenated alkenyl, alkynyl, halogenated alkynyl, cycloalkenyl, alkoxyalkyl, alkylthioalkyl, alkoxycarbonylalkyl, aryl, or arylalkyl; when M is 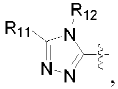 -(CH₂)₄- or -CH=CH-CH=CH- formed by R₁₁ and R₁₂, the nitrogen atom bound to R₁₂ and the carbon atom bound to R₁₁ together form a 6-membered ring;
R₁₅ represents alkyl which is unsubstituted or substituted with a substituent selected from R₂₁, cycloalkyl, halogenated cycloalkyl, alkenyl, halogenated alkenyl, alkynyl, halogenated alkynyl, cycloalkenyl, or phenyl;
R₁₆ represents alkyl, halogenated alkyl, cycloalkyl, alkenyl, halogenated alkenyl, alkynyl, halogenated alkynyl, or cycloalkenyl;
R₂₁ represents halogen, cyano, cycloalkyl, hydroxy, mercapto, alkoxy, C(O)R₂₂, carboxyl, alkoxycarbonyl, alkoxyalkoxycarbonyl, -S(O)ₘ-alkyl, heteroaryl, heterocyclyl, or phenyl which is unsubstituted or substituted with one or more groups selected from R₂₃;
R₁₇, and R₂₂ each independently represent hydrogen, alkyl, or N(R₂₄)R₂₅;
R₂₃ represents halogen, cyano, nitro, alkyl, alkyl which is unsubstituted or substituted with a substituent selected from R₃₁, cycloalkyl, halogenated cycloalkyl, alkenyl, halogenated alkenyl, alkynyl, halogenated alkynyl, cycloalkenyl, alkylcarbonyl, cycloalkylcarbonyl, halogenated alkylcarbonyl, halogenated cycloalkylcarbonyl, alkoxycarbonyl, halogenated alkoxycarbonyl, alkylaminocarbonyl, halogenated alkylaminocarbonyl, bis(alkyl)aminocarbonyl, OR₃₂, S(O)ₘR₃₃, alkylaminosulfonyl, bis(alkyl)aminosulfonyl, NH₂, alkylamino, bis(alkyl)amino, aryl, heteroaryl, or heterocyclyl;
R₂₄, and R₂₅ each independently represent hydrogen, alkyl, or phenyl; or,
alkylidene chain formed by R₂₄ and R₂₅, and the nitrogen atom(s) bound to R₂₄ and R₂₅ together form a 3-7-membered ring, said alkylidene chain optionally contains one O, S, S(O), S(O)₂, NH, orN-alkyl and optionally substituted by oxo or thio group;
R₁₃, R₁₄, R₁₈, and R₃₁ each independently represent halogen, cyano, nitro, carboxyl, alkoxycarbonyl, alkoxyalkoxycarbonyl, S(O)ₘR₄₁, OR₄₂, aryl, heteroaryl, or heterocyclyl;
R₃₂ represents hydrogen, alkyl, halogenated alkyl, cycloalkyl, halogenated cycloalkyl, alkenyl, halogenated alkenyl, alkynyl, halogenated alkynyl, or cycloalkenyl;
R₃₃ represents alkyl, halogenated alkyl, cycloalkyl, alkenyl, halogenated alkenyl, alkynyl, halogenated alkynyl, or cycloalkenyl;
R₄₁, and R₄₂ each independently represent hydrogen, alkyl, halogenated alkyl, cycloalkyl, halogenated cycloalkyl, alkenyl, halogenated alkenyl, alkynyl, halogenated alkynyl, cycloalkenyl, or phenyl;
r represents 1 or 2;
m represents 0, 1 or 2;
n represents 0, or 1;
s represents 0, 1, 2 or 3.

Preferably, X, and Y each independently represent nitro, halogen, cyano, formyl, thiocyano, mercapto; C1-C8 alkyl, C2-C8 alkenyl, C2-C8 alkynyl, C3-C8 cycloalkyl, C3-C8 cycloalkenyl, C3-C8 cycloalkyl-C1-C6 alkyl, or C3-C8 cycloalkenyl-C1-C6 alkyl, which is with or without halogen; OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -(C1-C6)alkyl-C(S)R¹, -(C1-C6)alkyl-C(S)OR¹, -(C1-C6)alkyl-C(O)SR¹, -(C1-C6)alkyl-C(S)SR¹, -(C1-C6)alkyl-SC(O)R¹, -(C1-C6)alkyl-OC(S)R¹, -(C1-C6)alkyl-SC(S)R¹, -O-(C1-C6)alkyl-NR⁴R⁵, -S-(C1-C6)alkyl-NR⁴R⁵, -(C1-C6)alkyl-O-(C1-C6)alkyl-NR⁴R⁵, -(C1-C6)alkyl-S-(C1-C6)alkyl-NR⁴R⁵, -(C1-C6)alkyl-(C=S)ₙ-NR⁴R⁵, -NH-(C1-C6)alkyl-NR⁴R⁵, -(C1-C6)alkyl-OR¹, -(C1-C6)alkyl-COR¹, -(C1-C6)alkyl-CO₂R¹, -(C1-C6)alkyl-OCOR¹, -(C1-C6)alkyl-NR³COR¹, -(C1-C6)alkyl-SO₂OR¹, -(C1-C6)alkyl-NR³SO₂R², -(C1-C6)alkyl-OSO₂R², -alkyl-S(O)ₘR², -(C1-C6)alkyl-CONR⁴R⁵, -(C1-C6)alkyl-SO₂NR⁴R⁵, NR⁴R⁵, 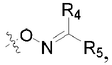 P(O)(OR⁶)₂, CH₂P(O)(OR⁶)₂, SO₂NR⁴R⁵-(C1-C6)alkyl-S(O)ₘR², -(C1-C6)alkyl-CN, -(C1-C6)alkylaryl, -(C1-C6)alkylheteroaryl, -(C1-C6)alkylheterocyclyl, aryl, heteroaryl, or heterocyclyl;
R¹, R³, R⁴, and R⁵ each independently represent hydrogen, aryl, aryl-C1-C6 alkyl, heteroaryl, heteroaryl-C1-C6 alkyl, C1-C8 alkyl, halogenated C1-C8 alkyl, C2-C8 alkenyl, halogenated C2-C8 alkenyl, C2-C8 alkynyl, halogenated C2-C8 alkynyl, C3-C8 cycloalkyl, halogenated C3-C8 cycloalkyl, C1-C8 alkoxy-C1-C6 alkyl, or C3-C8 cycloalkyl-C1-C6 alkyl, wherein the last 10 groups as mentioned are each substituted by s groups selected from the group consisting of cyano, halogen, nitro, thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and C1-C8 alkoxy-C1-C6 alkoxycarbonyl;
R² represents aryl, aryl-C1-C6 alkyl, heteroaryl, heteroaryl-C1-C6 alkyl, C1-C8 alkyl, C2-C8 alkenyl, C2-C8 alkynyl, C3-C8 cycloalkyl, or C3-C8 cycloalkyl-C1-C6 alkyl, wherein the last 5 groups as mentioned are each substituted by s groups selected from the group consisting of cyano, halogen, nitro, thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and C1-C8 alkoxy-C1-C6 alkoxycarbonyl;
R⁶ represents methyl, or ethyl;
R⁷, and R⁸ each independently represent hydrogen, C1-C8 alkyl, C2-C8 alkenyl, or C2-C8 alkynyl;
R⁹ represents C1-C8 alkyl, C2-C8 alkenyl, or C2-C8 alkynyl;
M represents 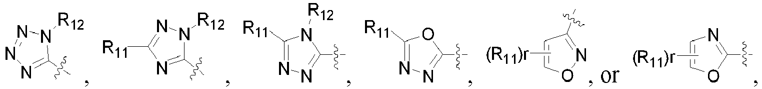 which is unsubstituted or substituted;
R₁₁ represents hydrogen, halogen, cyano, nitro, C1-C8 alkyl which is unsubstituted or substituted with a substituent selected from R₁₃, C3-C8 cycloalkyl which is unsubstituted or substituted with a substituent selected from R₁₄, C2-C8 alkenyl, halogenated C2-C8 alkenyl, C2-C8 alkynyl, halogenated C2-C8 alkynyl, C3-C8 cycloalkenyl, NH₂, aminoacyl, carboxyl, C1-C8 alkoxy-C1-C6 alkoxycarbonyl, OR₁₅, -(C1-C6)alkyl-OR₁₅, C(O)R₁₆, -(C1-C6)alkyl-C(O)R₁₆, C(O)OR₁₆, -(C1-C6)alkyl-C(O)OR₁₆, S(O)ₘR₁₆, -(C1-C6)alkyl-S(O)ₘR₁₆, -N(R₁₆)₂, -NHR₁₆, -C(O)NHR₁₆, -C(O)N(R₁₆)₂, -NHC(O)R₁₇, heterocyclyl, heterocyclyl-C1-C6 alkyl, heterocyclyloxy, heterocyclylcarbonyl, aryl, aryl-C1-C6 alkyl, aryloxy, arylcarbonyl, heteroaryl, heteroaryl-C1-C6 alkyl, heteroaryloxy, or heteroarylcarbonyl;
R₁₂ represents hydrogen, C1-C8 alkyl which is unsubstituted or substituted with a substituent selected from R₁₈, C3-C8 cycloalkyl, halogenated C3-C8 cycloalkyl, C2-C8 alkenyl, halogenated C2-C8 alkenyl, C2-C8 alkynyl, halogenated C2-C8 alkynyl, C3-C8 cycloalkenyl, C1-C8 alkoxy-C1-C6 alkyl, C1-C8 alkylthio-C1-C6 alkyl, C1-C8 alkoxycarbonyl-C1-C6 alkyl, aryl, or aryl-C1-C6 alkyl;
when M is 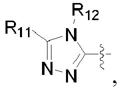 -(CH₂)₄- or -CH=CH-CH=CH- formed by R₁₁ and R₁₂, the nitrogen atom bound to R₁₂ and the carbon atom bound to R₁₁ together form a 6-membered ring;
R₁₅ represents C1-C8 alkyl which is unsubstituted or substituted with a substituent selected from R₂₁, C3-C8 cycloalkyl, halogenated C3-C8 cycloalkyl, C2-C8 alkenyl, halogenated C2-C8 alkenyl, C2-C8 alkynyl, halogenated C2-C8 alkynyl, C3-C8 cycloalkenyl, or phenyl;
R₁₆ represents C1-C8 alkyl, halogenated C1-C8 alkyl, C3-C8 cycloalkyl, C2-C8 alkenyl, halogenated C2-C8 alkenyl, C2-C8 alkynyl, halogenated C2-C8 alkynyl, or C3-C8 cycloalkenyl;
R₂₁ represents halogen, cyano, C3-C8 cycloalkyl, hydroxy, mercapto, C1-C8 alkoxy, C(O)R₂₂, carboxyl, C1-C8 alkoxycarbonyl, C1-C8 alkoxy-C1-C6 alkoxycarbonyl, -S(O)ₘ-(C1-C8)alkyl, heteroaryl, heterocyclyl, or phenyl which is unsubstituted or substituted with one or more groups selected from R₂₃;
R₁₇, and R₂₂ each independently represent hydrogen, C1-C8 alkyl, or N(R₂₄)R₂₅;
R₂₃ represents halogen, cyano, nitro, C1-C8 alkyl, C1-C8 alkyl which is unsubstituted or substituted with a substituent selected from R₃₁, C3-C8 cycloalkyl, halogenated C3-C8 cycloalkyl, C2-C8 alkenyl, halogenated C2-C8 alkenyl, C2-C8 alkynyl, halogenated C2-C8 alkynyl, C3-C8 cycloalkenyl, C1-C8 alkylcarbonyl, C3-C8 cycloalkylcarbonyl, halogenated C1-C8 alkylcarbonyl, halogenated C3-C8 cycloalkylcarbonyl, C1-C8 alkoxycarbonyl, halogenated C1-C8 alkoxycarbonyl, C1-C8 alkylaminocarbonyl, halogenated C1-C8 alkylaminocarbonyl, bis(C1-C8 alkyl)aminocarbonyl, OR₃₂, S(O)ₘR₃₃, C1-C8 alkylaminosulfonyl, bis(C1-C8 alkyl)aminosulfonyl, NH₂, C1-C8 alkylamino, bis(C1-C8 alkyl)amino, aryl, heteroaryl, or heterocyclyl;
R₂₄, and R₂₅ each independently represent hydrogen, C1-C8 alkyl, or phenyl; or,
C2-C8 alkylidene chain formed by R₂₄ and R₂₅, and the nitrogen atom(s) bound to R₂₄ and R₂₅ together form a 3-7-membered ring, said C2-C8 alkylidene chain optionally contains one O, S, S(O), S(O)₂, NH, or N-alkyl and optionally substituted by oxo or thio group;
R₁₃, R₁₄, R₁₈, and R₃₁ each independently represent halogen, cyano, nitro, carboxyl, C1-C8 alkoxycarbonyl, C1-C8 alkoxy-C1-C8 alkoxycarbonyl, S(O)ₘR₄₁, OR₄₂, aryl, heteroaryl, or heterocyclyl;
R₃₂ represents hydrogen, C1-C8 alkyl, halogenated C1-C8 alkyl, C3-C8 cycloalkyl, halogenated C3-C8 cycloalkyl, C2-C8 alkenyl, halogenated C2-C8 alkenyl, C2-C8 alkynyl, halogenated C2-C8 alkynyl, or C3-C8 cycloalkenyl;
R₃₃ represents C1-C8 alkyl, halogenated C1-C8 alkyl, C3-C8 cycloalkyl, C2-C8 alkenyl, halogenated C2-C8 alkenyl, C2-C8 alkynyl, halogenated C2-C8 alkynyl, or C3-C8 cycloalkenyl;
R₄₁, and R₄₂ each independently represent hydrogen, C1-C8 alkyl, halogenated C1-C8 alkyl, C3-C8 cycloalkyl, halogenated C3-C8 cycloalkyl, C2-C8 alkenyl, halogenated C2-C8 alkenyl, C2-C8 alkynyl, halogenated C2-C8 alkynyl, C3-C8 cycloalkenyl, or phenyl;
r represents 1, or 2;
m represents 0, 1, or 2;
n represents 0, or 1;
s represents 0, 1, 2, or 3.

More preferably, X represents nitro, halogen, cyano, formyl, thiocyano, mercapto; C1-C6 alkyl, C2-C6 alkenyl, C2-C6 alkynyl, C3-C6 cycloalkyl, C3-C6 cycloalkenyl, C3-C6 cycloalkyl-C1-C3 alkyl, or C3-C6 cycloalkenyl-C1-C3 alkyl, which is with or without halogen; OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -(C1-C3)alkyl-C(S)R¹, -(C1-C3)alkyl-C(S)OR¹, -(C1-C3)alkyl-C(O)SR¹, -(C1-C3)alkyl-C(S)SR¹, -(C1-C3)alkyl-SC(O)R¹, -(C1-C3)alkyl-OC(S)R¹, -(C1-C3)alkyl-SC(S)R¹, -O-(C1-C3)alkyl-NR⁴R⁵, -S-(C1-C3)alkyl-NR⁴R⁵, -(C1-C3)alkyl-O-(C1-C3)alkyl-NR⁴R⁵, -(C1-C3)alkyl-S-(C1-C3)alkyl-NR⁴R⁵, -(C1-C3)alkyl-(C=S)ₙ-NR⁴R⁵, -NH-(C1-C3)alkyl-NR⁴R⁵, -(C1-C3)alkyl-OR¹, -(C1-C3)alkyl-COR¹, -(C1-C3)alkyl-CO₂R¹, -(C1-C3)alkyl-OCOR¹, -(C1-C3)alkyl-NR³COR¹, -(C1-C3)alkyl-SO₂OR¹, -(C1-C3)alkyl-NR³SO₂R², -(C1-C3)alkyl-OSO₂R², -alkyl-S(O)ₘR², -(C1-C3)alkyl-CONR⁴R⁵, -(C1-C3)alkyl-SO₂NR⁴R⁵, NR⁴R⁵, 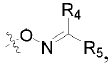 P(O)(OR⁶)₂, CH₂P(O)(OR⁶)₂, SO₂NR⁴R⁵-(C1-C3)alkyl-S(O)ₘR², -(C1-C3)alkyl-CN, -(C1-C3)alkylaryl, -(C1-C3)alkylheteroaryl, -(C1-C3)alkylheterocyclyl, aryl, heteroaryl, or heterocyclyl;
Y represents C1-C6 alkyl, C2-C6 alkenyl, C2-C6 alkynyl, C3-C6 cycloalkyl, or C3-C6 cycloalkenyl, which is with or without halogen; OR¹, S(O)ₘR², NR⁴R⁵, heterocyclyl, aryl, or heteroaryl;
R¹, R³, R⁴, and R⁵ each independently represent hydrogen, aryl, aryl-C1-C3 alkyl, heteroaryl, heteroaryl-C1-C3 alkyl, C1-C6 alkyl, halogenated C1-C6 alkyl, C2-C6 alkenyl, halogenated C2-C6 alkenyl, C2-C6 alkynyl, halogenated C2-C6 alkynyl, C3-C6 cycloalkyl, halogenated C3-C6 cycloalkyl, C1-C6 alkoxy-C1-C3 alkyl, or C3-C6 cycloalkyl-C1-C3 alkyl, wherein the last 10 groups as mentioned are each substituted by s groups selected from the group consisting of cyano, halogen, nitro, thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and C1-C6 alkoxy-C1-C3 alkoxycarbonyl;
R² represents aryl, aryl-C1-C3 alkyl, heteroaryl, heteroaryl-C1-C3 alkyl, C1-C6 alkyl, C2-C6 alkenyl, C2-C6 alkynyl, C3-C6 cycloalkyl, or C3-C6 cycloalkyl-C1-C3 alkyl, wherein the last 5 groups as mentioned are each substituted by s groups selected from the group consisting of cyano, halogen, nitro, thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and C1-C6 alkoxy-C1-C3 alkoxycarbonyl;
R⁶ represents methyl, or ethyl;
R⁷, and R⁸ each independently represent hydrogen, C1-C6 alkyl, C2-C6 alkenyl, or C2-C6 alkynyl;
R⁹ represents C1-C6 alkyl, C2-C6 alkenyl, or C2-C6 alkynyl;
M represents 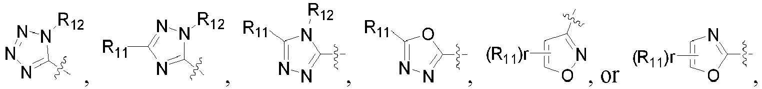 which is unsubstituted or substituted;
R₁₁ represents hydrogen, halogen, cyano, nitro, C1-C6 alkyl which is unsubstituted or substituted with a substituent selected from R₁₃, C3-C6 cycloalkyl which is unsubstituted or substituted with a substituent selected from R₁₄, C2-C6 alkenyl, halogenated C2-C6 alkenyl, C2-C6 alkynyl, halogenated C2-C6 alkynyl, C3-C6 cycloalkenyl, NH₂, aminoacyl, carboxyl, C1-C6 alkoxy-C1-C3 alkoxycarbonyl, OR₁₅, -(C1-C3)alkyl-OR₁₅, C(O)R₁₆, -(C1-C3)alkyl-C(O)R₁₆, C(O)OR₁₆, -(C1-C3)alkyl-C(O)OR₁₆, S(O)ₘR₁₆, -(C1-C3)alkyl-S(O)ₘR₁₆, -N(R₁₆)₂, -NHR₁₆, -C(O)NHR₁₆, -C(O)N(R₁₆)₂, -NHC(O)R₁₇, heterocyclyl, heterocyclyl-C1-C3 alkyl, heterocyclyloxy, heterocyclylcarbonyl, aryl, aryl-C1-C3 alkyl, aryloxy, arylcarbonyl, heteroaryl, heteroaryl-C1-C3 alkyl, heteroaryloxy, or heteroarylcarbonyl;
R₁₂ represents hydrogen, C1-C6 alkyl which is unsubstituted or substituted with a substituent selected from R₁₈, C3-C6 cycloalkyl, halogenated C3-C6 cycloalkyl, C2-C6 alkenyl, halogenated C2-C6 alkenyl, C2-C6 alkynyl, halogenated C2-C6 alkynyl, C3-C6 cycloalkenyl, C1-C6 alkoxy-C1-C3 alkyl, C1-C6 alkylthio-C1-C3 alkyl, C1-C6 alkoxycarbonyl-C1-C3 alkyl, aryl, or aryl-C1-C3 alkyl;
when M is 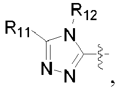 -(CH₂)₄- or -CH=CH-CH=CH- formed by R₁₁ and R₁₂, the nitrogen atom bound to R₁₂ and the carbon atom bound to R₁₁ together form a 6-membered ring;
R₁₅ represents C1-C6 alkyl which is unsubstituted or substituted with a substituent selected from R₂₁, C3-C6 cycloalkyl, halogenated C3-C6 cycloalkyl, C2-C6 alkenyl, halogenated C2-C6 alkenyl, C2-C6 alkynyl, halogenated C2-C6 alkynyl, C3-C6 cycloalkenyl, or phenyl;
R₁₆ represents C1-C6 alkyl, halogenated C1-C6 alkyl, C3-C6 cycloalkyl, C2-C6 alkenyl, halogenated C2-C6 alkenyl, C2-C6 alkynyl, halogenated C2-C6 alkynyl, or C3-C6 cycloalkenyl;
R₂₁ represents halogen, cyano, C3-C6 cycloalkyl, hydroxy, mercapto, C1-C6 alkoxy, C(O)R₂₂, carboxyl, C1-C6 alkoxycarbonyl, C1-C6 alkoxy-C1-C3 alkoxycarbonyl, -S(O)ₘ-(C1-C6)alkyl, heteroaryl, heterocyclyl, or phenyl which is unsubstituted or substituted with 1~3 groups selected from R₂₃;
R₁₇, and R₂₂ each independently represent hydrogen, C1-C6 alkyl, or N(R₂₄)R₂₅;
R₂₃ represents halogen, cyano, nitro, C1-C6 alkyl, C1-C6 alkyl which is unsubstituted or substituted with a substituent selected from R₃₁, C3-C6 cycloalkyl, halogenated C3-C6 cycloalkyl, C2-C6 alkenyl, halogenated C2-C6 alkenyl, C2-C6 alkynyl, halogenated C2-C6 alkynyl, C3-C6 cycloalkenyl, C1-C6 alkylcarbonyl, C3-C6 cycloalkylcarbonyl, halogenated C1-C6 alkylcarbonyl, halogenated C3-C6 cycloalkylcarbonyl, C1-C6 alkoxycarbonyl, halogenated C1-C6 alkoxycarbonyl, C1-C6 alkylaminocarbonyl, halogenated C1-C6 alkylaminocarbonyl, bis(C1-C6 alkyl)aminocarbonyl, OR₃₂, S(O)ₘR₃₃, C1-C6 alkylaminosulfonyl, bis(C1-C6 alkyl)aminosulfonyl, NH₂, C1-C6 alkylamino, bis(C1-C6 alkyl)amino, aryl, heteroaryl, or heterocyclyl;
R₂₄, and R₂₅ each independently represent hydrogen, C1-C6 alkyl, or phenyl; or,
C2-C6 alkylidene chain formed by R₂₄ and R₂₅, and the nitrogen atom(s) bound to R₂₄ and R₂₅ together form a 3-7-membered ring, said C2-C6 alkylidene chain optionally contains one O, S, S(O), S(O)₂, NH, or N-alkyl and optionally substituted by oxo or thio group;
R₁₃, R₁₄, R₁₈, and R₃₁ each independently represent halogen, cyano, nitro, carboxyl, C1-C6 alkoxycarbonyl, C1-C6 alkoxy-C1-C6 alkoxycarbonyl, S(O)ₘR₄₁, OR₄₂, aryl, heteroaryl, or heterocyclyl;
R₃₂ represents hydrogen, C1-C6 alkyl, halogenated C1-C6 alkyl, C3-C6 cycloalkyl, halogenated C3-C6 cycloalkyl, C2-C6 alkenyl, halogenated C2-C6 alkenyl, C2-C6 alkynyl, halogenated C2-C6 alkynyl, or C3-C6 cycloalkenyl;
R₃₃ represents C1-C6 alkyl, halogenated C1-C6 alkyl, C3-C6 cycloalkyl, C2-C6 alkenyl, halogenated C2-C6 alkenyl, C2-C6 alkynyl, halogenated C2-C6 alkynyl, or C3-C6 cycloalkenyl;
R₄₁, and R₄₂ each independently represent hydrogen, C1-C6 alkyl, halogenated C1-C6 alkyl, C3-C6 cycloalkyl, halogenated C3-C6 cycloalkyl, C2-C6 alkenyl, halogenated C2-C6 alkenyl, C2-C6 alkynyl, halogenated C2-C6 alkynyl, C3-C6 cycloalkenyl, or phenyl;
r represents 1, or 2;
m represents 0, 1, or 2;
n represents 0, or 1;
s represents 0, 1, 2, or 3.

Further preferably, X represents halogen, nitro, cyano, OR¹, S(O)ₘR², NR³COR¹, NR⁴R⁵, 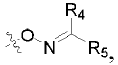 C1-C6 alkoxy-C1-C3 alkyl; C1-C6 alkyl, C2-C6 alkenyl, C2-C6 alkynyl, or C3-C6 cycloalkyl, which is with or without halogen; 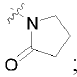 or phenyl which is unsubstituted or substituted with 1, 2, or 3 groups selected from halogen, and C1-C6 alkoxy;
Y represents C1-C6 alkyl, C2-C6 alkenyl, C2-C6 alkynyl, C3-C6 cycloalkyl, or C3-C6 cycloalkenyl, which is with or without halogen; OR¹, S(O)ₘR², NR⁴R⁵, heterocyclyl, aryl, or heteroaryl;
R¹, R³, R⁴, and R⁵ each independently represent hydrogen, C1-C6 alkyl, halogenated C1-C6 alkyl, phenyl; or benzyl which is unsubstituted or substituted with 1, 2, or 3 groups selected from halogen, and C1-C6 alkoxy;
R² represents C1-C6 alkyl, or halogenated C1-C6 alkyl;
M represents 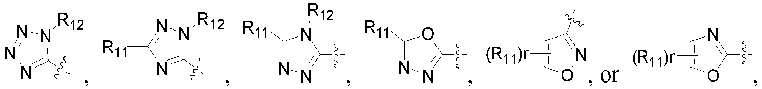 which is unsubstituted or substituted;
R₁₁ represents hydrogen, C1-C6 alkyl, C3-C6 cycloalkyl, halogenated C1-C6 alkyl, C1-C6 alkoxy, C1-C6 alkylthio, halogen, C1-C6 alkylamino, bis(C1-C6 alkyl)amino, cyano, nitro, C1-C6 alkylcarbonyl, C1-C6 alkoxycarbonyl, C1-C6 alkoxycarbonyl-C1-C3 alkyl, aminocarbonyl, C1-C6 alkylcarbonylamino, C1-C6 alkoxy-C1-C3 alkyl, C1-C6 alkylthio-C1-C3 alkyl, C1-C6 alkylsulfinyl-C1-C3 alkyl, C1-C6 alkylsulfonyl-C1-C3 alkyl; or benzyl, phenoxy, benzoyl, pyridyl, 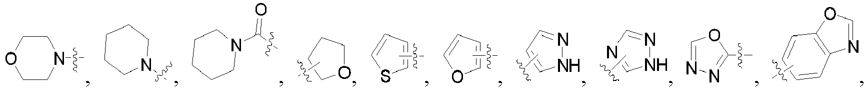 or 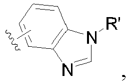 which is unsubstituted or substituted with 1, 2, or 3 groups selected from halogen, and C1-C6 alkoxy;
R₁₂ represents hydrogen, C1-C6 alkyl, C2-C6 alkenyl, C1-C6 alkoxy-C1-C3 alkyl, C1-C6 alkylthio-C1-C3 alkyl, C1-C6 alkoxycarbonyl-C1-C3 alkyl; or phenyl, or benzyl which is unsubstituted or substituted with at least one group selected from halogen, and halogenated C1-C6 alkyl;
r represents 1, or 2;
m represents 0, 1, or 2;
wherein, the "heterocyclyl" is 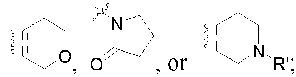 the "aryl" is phenyl, naphthyl, or 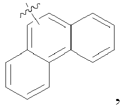 which is unsubstituted or substituted; the "heteroaryl" is 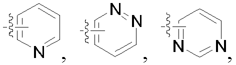 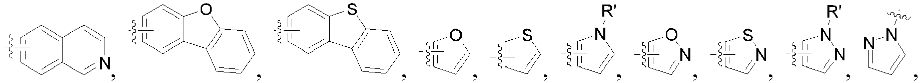 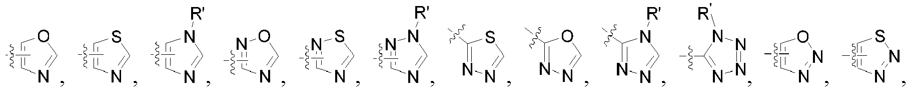 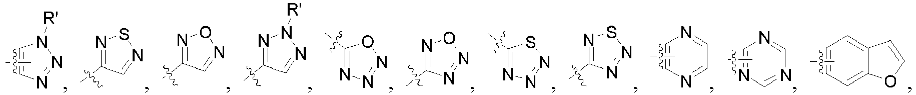 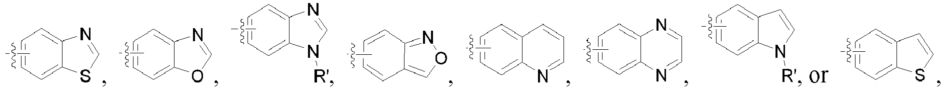 which is unsubstituted or substituted; the aforementioned "substituted" respectively refers to being substituted by 1~3 groups selected from: halogen, nitro, cyano, thiocyano, cyano C1-C3 alkyl, hydroxy, hydroxy C1-C3 alkyl, mercapto, carboxyl, formyl, 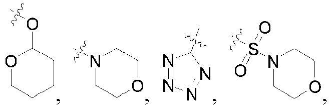 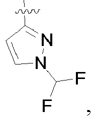 phenyl, phenyl substituted by C1-C6 alkyl, phenoxy, benzyloxy; amino, aminoalkyl, or aminocarbonyl group which is unsubstituted or substituted by one or two groups selected from C1-C6 alkyl, COR", and COOR"; and C1-C6 alkyl, C2-C6 alkenyl, C3-C6 cycloalkyl, C1-C6 alkoxy-C1-C3 alkyl, C1-C6 alkylthio-C1-C3 alkyl, C1 -C6 alkylcarbonylthio, C3-C6 cycloalkyl substituted with C1-C6 alkyl, OR", SR", SOR", COR", COOR", or SO₂R", which is with or without halogen; or, two adjacent substitutable positions of the above-mentioned "heterocyclyl", "aryl", "heteroaryl" groups are linked with -OCH₂CH₂-, -OCH₂O-, -OCH₂CH₂O-, or -CH=CH-CH=CH-group to form a ring, wherein the -OCH₂CH₂-, -OCH₂O-, -OCH₂CH₂O-, or -CH=CH-CH=CH-group, which is with or without halogen;
R' each independently represents hydrogen, C1-C6 alkyl, C1-C6 alkylcarbonyl, halogenated C1-C6 alkyl, 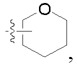 C1-C6 alkoxycarbonyl, C1-C6 alkoxy-C1-C3 alkyl, or benzyl;
R" each independently represents C1-C6 alkyl, or C2-C6 alkenyl.

Further preferably, X represents chlorine, fluorine, bromine, methyl, ethyl, isopropyl, vinyl, allyl, ethynyl, cyclopropyl, trifluoromethyl, 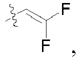 hydroxy, methoxy, ethyloxy, methylthio, ethylthio, 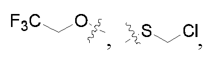 nitro, cyano, methylsulfinyl, ethylsulfinyl, methylsulfonyl, amino, monomethylamino, dimethylamino, acetamido, 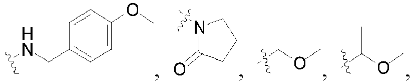 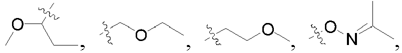 phenyl, phenoxy, or 4-fluorophenyl;
Y represents methyl, ethyl, vinyl, methoxy, 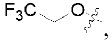 phenoxy, benzyloxy, methylthio, propylthio, tert-butylthio, benzylthio, 4-methoxybenzylthiol, propylsulfinyl, amino, monomethylamino, dimethylamino, anilino, p-methoxybenzylamino, tert-butyl, cyclopropyl, cyclohexyl, 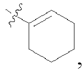 heterocyclyl, aryl, or heteroaryl;
M represents 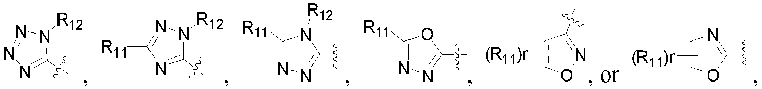 which is unsubstituted or substituted;
Rn each independently represents hydrogen, methyl, ethyl, n-propyl, isopropyl, cyclopropyl, monochloromethyl, trifluoromethyl, methoxy, isopropyloxy, methylthio, fluorine, chlorine, bromine, iodine, dimethylamino, ethylamino, cyano, nitro, acetyl, methoxycarbonyl; phenyl which is unsubstituted or substituted by 1, 2, or 3 groups selected from chlorine, and methoxy; pyridyl, 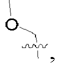 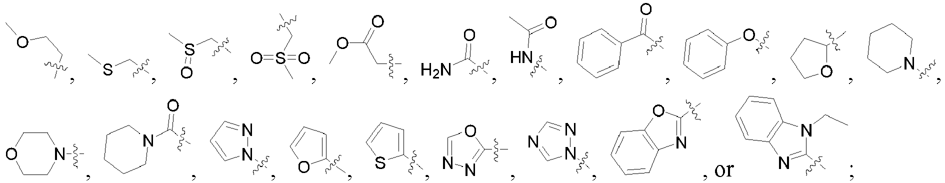
R₁₂ each independently represents hydrogen, methyl, ethyl, n-propyl, isopropyl, allyl, 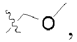 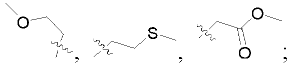 or phenyl, or benzyl which is unsubstituted or substituted by 1, 2, or 3 groups selected from chlorine, and trifluoromethyl;
r represents 1, or 2; wherein, the "heterocyclyl" is 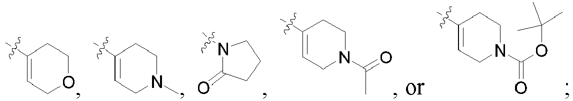 the "aryl" is phenyl, naphthyl, or 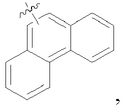 which is unsubstituted or substituted; the "heteroaryl" is 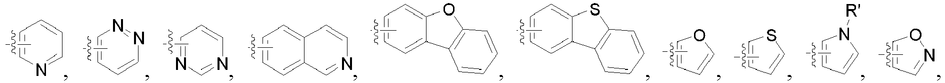 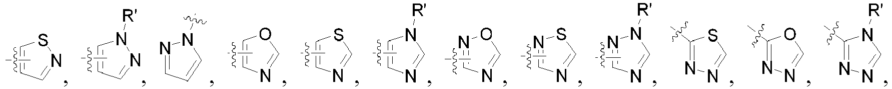 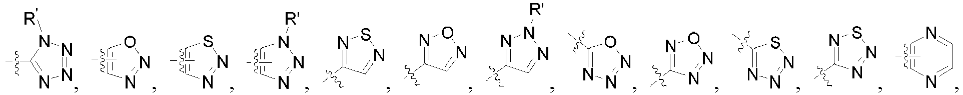 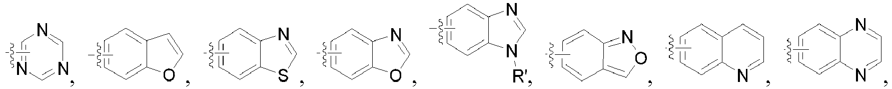 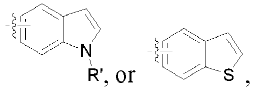 which is unsubstituted or substituted; the aforementioned "substituted" refers to being substituted by 1, 2, or 3 groups selected from: methyl, ethyl, isopropyl, n-butyl, tert-butyl, n-pentyl, vinyl, cyclopropyl, 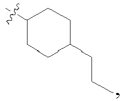 fluorine, chlorine, bromine, iodine, monobromomethyl, difluoromethyl, trifluoromethyl, methoxy, ethoxy, propoxy, butoxy, trifluoromethoxy, hydroxyl, hydroxymethyl, amino, cyano, cyanomethyl, thiocyano, mercapto, nitro, carboxy, formyl, acetyl, methoxycarbonyl, tert-butyloxycarbonyl, methylthio, isopropylthio, methylsulfinyl, methylsulfonyl, 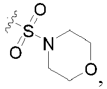 dimethylamino, aminocarbonyl, dimethylaminocarbonyl, acetylamino, 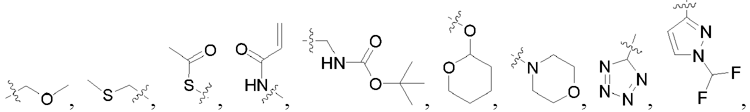 phenyl, 4-ethylphenyl, phenoxy, and benzyloxy; or, two adjacent substitutable positions of the above-mentioned "aryl", "heteroaryl" groups are linked with -OCH₂CH₂-, -OCH₂O-, -OCH₂CH₂O- , or -CH=CH-CH=CH- group to form a ring, wherein the -OCH₂CH₂-, -OCH₂O-, -OCH₂CH₂O-, or -CH=CH-CH=CH- group is with or without halogen;
R' each independently represents hydrogen, methyl, ethyl, n-propyl, isopropyl, difluoromethyl, 3,3,3-trifluoroethyl, benzyl, 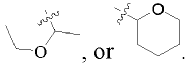 It should be particularly pointed out that when X is fluorine, Y is not amino, monomethylamino, monoethylamino and monopropylamino; when M is 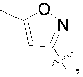 X and Y are not methyl at the same time.

The structural formula of the derivative is 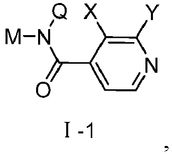 wherein Q represents hydrogen, -alkyl-Q₁, OQ₂, SQ₂, COQ₂, COOQ₂, CON(Q₃)₂, N(Q₃)₂, NQ₄COOQ₂, NQ₄CON(Q₃)₂; alkyl, alkenyl, alkynyl, or cycloalkyl, which is with or without halogen; or arylalkyl, or heteroarylalkyl, which is unsubstituted or substituted;
Q₁ each independently represents alkenyl, alkynyl, or cycloalkyl, which is with or without halogen; CN, OQ₁₁, OCOQ₁₁, COOQ₁₁, COQ₁₁, -O-(C=O)-O-Q₁₁, OSO₂Q₁₂, SO₂OQ₁₁, -S(O)ₚQ₁₂, N(Q₁₃)₂, CON(Q₁₃)₂, SO₂N(Q₁₃)₂, NQ₁₄COQ₁₁, NQ₁₄SO₂Q₁₂, or -O-(C=O)-N(Q₁₃)₂;
Q₂, and Q₁₁ each independently represent hydrogen; alkyl, alkenyl, alkynyl, cycloalkyl, cycloalkylalkyl, cycloalkenyl, or alkoxyalkyl, which is with or without halogen; or heterocyclyl, heterocyclylalkyl, heterocyclyloxyalkyl, aryl, arylalkyl, aryloxyalkyl, heteroaryl, heteroarylalkyl, or heteroaryloxyalkyl, which is unsubstituted or substituted;
Q₁₂ each independently represents alkyl, alkenyl, alkynyl, cycloalkyl, cycloalkylalkyl, cycloalkenyl, or alkoxyalkyl, which is with or without halogen; or heterocyclyl, heterocyclylalkyl, aryl, arylalkyl, heteroaryl, or heteroarylalkyl, which is unsubstituted or substituted;
Q₃, Q₄, Q₁₃, and Q₁₄ each independently represent hydrogen, nitro, alkoxyaminocarbonyl, trialkylsilyl, dialkylphosphonyl, N(Q₂₁)₂, CON(Q₂₁)₂, OQ₂₁, COQ₂₁, CO₂Q₂₁, COSQ₂₁, OCOQ₂₁, S(O)ₚQ₂₂, alkyl, haloalkyl, alkenyl, haloalkenyl, alkynyl, haloalkynyl, cycloalkyl, cycloalkenyl, halocycloalkyl, alkoxyalkyl, cycloalkylalkyl, aryl, arylalkyl, aryloxy, aryloxyalkyl, arylalkyloxy, arylcarbonyl, arylsulfonyl, heteroaryl, heteroarylalkyl, heteroaryloxy, heteroaryloxyalkyl, heteroarylalkyloxy, heteroarylcarbonyl, heteroarylsulfonyl, heterocyclyl, heterocyclylalkyl, heterocyclyloxy, heterocyclooxyalkyl, heterocyclylalkyloxy, heterocyclylcarbonyl, heterocyclylsulfonyl, aryl-NQ₂₁-alkyl, heteroaryl-NQ₂₁-alkyl, or heterocyclyl-NQ₂₁-alkyl, wherein the last 35 groups as mentioned each are independently substituted by 0, 1, 2, or 3 groups selected from the group consisting of cyano, halogen, nitro, cyanothio, OQ₂₁, S(O)ₚQ₂₂, N(Q₂₁)₂, NQ₂₁OQ₂₁, COQ₂₁, OCOQ₂₁, SCOQ₂₂, NQ₂₁COQ₂₁, NQ₂₁SO₂Q₂₂, CO₂Q₂₁, COSQ₂₁, CON(Q₂₁)₂, and alkoxyalkoxycarbonyl;
Q₂₁ each independently represents hydrogen, alkyl, alkenyl, alkynyl, cycloalkyl, or cycloalkylalkyl;
Q₂₂ each independently represents alkyl, alkenyl, alkynyl, cycloalkyl, or cycloalkylalkyl;
p each independently represents 0, 1, or 2.

Preferably, Q represents hydrogen, -(C1-C6)alkyl-Q₁, OQ₂, SQ₂, COQ₂, COOQ₂, CON(Q₃)₂, N(Q₃)₂, NQ₄COOQ₂, NQ₄CON(Q₃)₂; C1-C6 alkyl, C2-C6 alkenyl, C2-C6 alkynyl, or C3-C6 cycloalkyl, which is with or without halogen; or aryl-C1-C6 alkyl, or heteroaryl-C1-C6 alkyl, which is unsubstituted or substituted;
Q₁ each independently represents C2-C6 alkenyl, C2-C6 alkynyl, or C3-C6 cycloalkyl, which is with or without halogen; CN, OQ₁₁, OCOQ₁₁, COOQ₁₁, COQ₁₁, -O-(C=O)-O-Q₁₁, OSO₂Q₁₂, SO₂OQ₁₁, -S(O)ₚQ₁₂, N(Q₁₃)₂, CON(Q₁₃)₂, SO₂N(Q₁₃)₂, NQ₁₄COQ₁₁, NQ₁₄SO₂Q₁₂, or -O-(C=O)-N(Q₁₃)₂;
Q₂, and Q₁₁ each independently represent hydrogen; C1-C6 alkyl, C2-C6 alkenyl, C2-C6 alkynyl, C3-C6 cycloalkyl, C3-C6 cycloalkyl-C1-C6 alkyl, C3-C6 cycloalkenyl, or C1-C6 alkoxy-C1-C6 alkyl, which is with or without halogen; or heterocyclyl, heterocyclyl-C1-C6 alkyl, heterocyclyloxy-C1-C6 alkyl, aryl, aryl-C1-C6 alkyl, aryloxy-C1-C6 alkyl, heteroaryl, heteroaryl-C1-C6 alkyl, or heteroaryloxy-C1-C6 alkyl, which is unsubstituted or substituted;
Q₁₂ each independently represents C1-C6 alkyl, C2-C6 alkenyl, C2-C6 alkynyl, C3-C6 cycloalkyl, C3-C6 cycloalkyl-C1-C6 alkyl, C3-C6 cycloalkenyl, or C1-C6 alkoxy-C1-C6 alkyl, which is with or without halogen; or heterocyclyl, heterocyclyl-C1-C6 alkyl, aryl, aryl-C1-C6 alkyl, heteroaryl, or heteroaryl-C1-C6 alkyl, which is unsubstituted or substituted;
Q₃, Q₄, Q₁₃, and Q₁₄ each independently represent hydrogen, nitro, C1-C6 alkoxyaminocarbonyl, tri-C1-C6 alkylsilyl, di-C1-C6 dialkylphosphonyl, N(Q₂₁)₂, CON(Q₂₁)₂, OQ₂₁, COQ₂₁, CO₂Q₂₁, COSQ₂₁, OCOQ₂₁, S(O)ₚQ₂₂, C1-C6 alkyl, halo-C1-C6 alkyl, C2-C6 alkenyl, halo-C2-C6 alkenyl, C2-C6 alkynyl, halo-C2-C6 alkynyl, C3-C6 cycloalkyl, C3-C6 cycloalkenyl, halo-C3-C6 cycloalkyl, C1-C6 alkoxy-C1-C6 alkyl, C3-C6 cycloalkyl-C1-C6 alkyl, aryl, aryl-C1-C6 alkyl, aryloxy, aryloxy-C1-C6 alkyl, aryl-C1-C6 alkyloxy, arylcarbonyl, arylsulfonyl, heteroaryl, heteroaryl-C1-C6 alkyl, heteroaryloxy, heteroaryloxy-C1-C6 alkyl, heteroaryl-C1-C6 alkyloxy, heteroarylcarbonyl, heteroarylsulfonyl, heterocyclyl, heterocyclyl-C1-C6 alkyl, heterocyclyloxy, heterocyclooxy-C1-C6 alkyl, heterocyclyl-C1-C6 alkyloxy, heterocyclylcarbonyl, heterocyclylsulfonyl, aryl-NQ₂₁-(C1-C6)alkyl, heteroaryl-NQ₂₁-(C1-C6)alkyl, or heterocyclyl-NQ₂₁-(C1-C6)alkyl, wherein the last 35 groups as mentioned each are independently substituted by 0, 1, 2, or 3 groups selected from the group consisting of cyano, halogen, nitro, cyanothio, OQ₂₁, S(O)ₚQ₂₂, N(Q₂₁)₂, NQ₂₁OQ₂₁, COQ₂₁, OCOQ₂₁, SCOQ₂₂, NQ₂₁COQ₂₁, NQ₂₁SO₂Q₂₂, CO₂Q₂₁, COSQ₂₁, CON(Q₂₁)₂, and C1-C6 alkoxy-C1-C6 alkoxycarbonyl;
Q₂₁ each independently represents hydrogen, C1-C6 alkyl, C2-C6 alkenyl, C2-C6 alkynyl, C3-C6 cycloalkyl, or C3-C6 cycloalkyl-C1-C6 alkyl;
Q₂₂ each independently represents C1-C6 alkyl, C2-C6 alkenyl, C2-C6 alkynyl, C3-C6 cycloalkyl, or C3-C6 cycloalkyl-C1-C6 alkyl;
p each independently represents 0, 1, or 2.

In addition, the derivatives also include salts commonly used in pesticides, for example, it can be processed into an metal salt, an amine salt, a sulfonium salt or a phosphonium salt; or, when a basic moiety is present in the molecule, it can be processed into, for example, a sulfate, a hydrochloride, a nitrate, a phosphate, etc. When these salts are used as herbicides in agriculture or horticulture, they are also included in the present invention. In the present invention, the "metal salt" may be, for example, an alkali metal salt, an alkaline earth metal salt, an aluminum salt or an iron salt; the "alkali metal salt" may be, for example, a sodium salt, a potassium salt or a lithium salt, preferably a sodium salt or a potassium salt. In the present invention, the "alkaline earth metal salt" may be, for example, a calcium salt or a magnesium salt, preferably a calcium salt. In the present invention, the "amine salt" may be, for example, a secondary alkylamine salt, a tertiary alkylamine salt or a quaternary alkylammonium salt; a primary alkanolamine salt, a secondary alkanolamine salt, a tertiary alkanolamine salt or a quaternary alkanoammonium salt; a primary alkylalkanolamine salt, a secondary alkylalkanolamine salt, a tertiary alkylalkanolamine salt or a quaternary alkylalkanolammonium salt; or a primary alkoxyalkanolamine salt, a secondary alkoxyalkanolamine salt, a tertiary alkoxyalkanolamine salt or a quaternary alkoxyalkanolammonium salt, preferably, wherein the alkyl, alkanol and alkoxy are independently saturated and independently contain 1-4 carbon atoms, and may also be substituted by phenyl and/or halogen, more preferably, ethanolamine salt, dimethylethanolamine salt, triethanolamine salt, dimethylamine salt, triethylamine salt, isopropylamine salt, choline salt, diglycolamine salt, 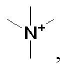 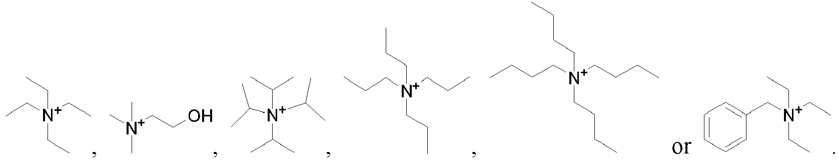 In the present invention, the "sulfonium salt" and "phosphonium salt" may be, for example, an alkylsulfonium salt, an alkylphosphonium salt or an alkanolphosphonium salt, preferably, wherein the alkyl is independently saturated and independently contain 1-4 carbon atoms, and may also be substituted by phenyl and/or halogen, more preferably,

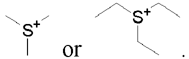

Solvates of the compounds of the invention are also included in the invention.

Wherein, unless otherwise specified, the "heterocyclyl" of the present invention includes but not limited to, 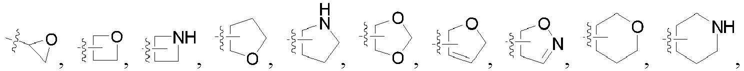 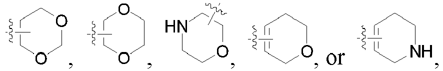 which has 0, 1 or 2 oxo groups; the "aryl" includes but not limited to, phenyl, naphthyl, 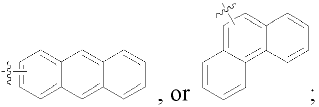 the "heteroaryl" is an aromatic cyclic group having, for example, 3 to 6 ring atoms and optionally fused with a benzo ring, 1 to 4 (for example, 1, 2 , 3 or 4) of the ring atoms are heteroatoms (for example, oxygen, nitrogen and/or sulfur). For example, 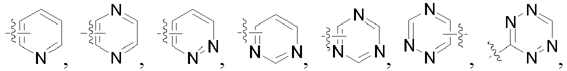 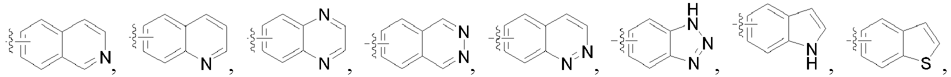 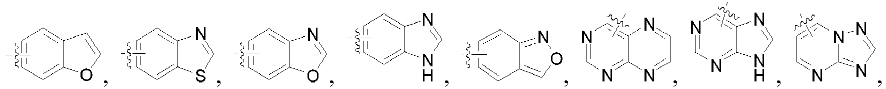 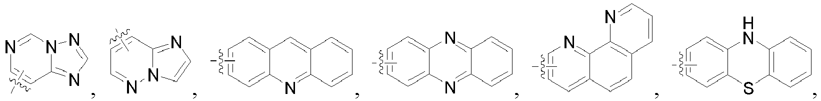 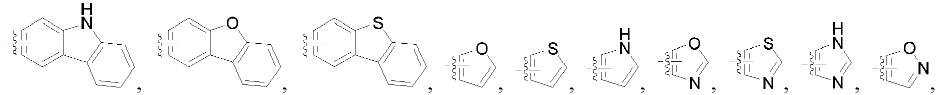 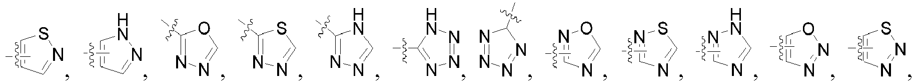 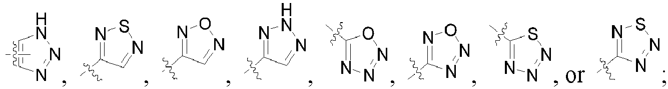 the above-mentioned groups are respectively unsubstituted or substituted by at least one group selected from: halogen, nitro, cyano, thiocyano, cyanoalkyl, mercapto, hydroxy, hydroxyalkyl, carboxyl, formyl, trialkylsilyl, dialkylphosphonyl; heterocyclyl, heterocyclylalkyl, aryl, arylalkyl, heteroaryl, or heteroarylalkyl, which is unsubstituted or substituted; alkyl, alkenyl, alkynyl, cycloalkyl, cycloalkenyl, cycloalkylalkyl, cycloalkyl substituted by alkyl, OR", SR", - alkyl-OR", -O-alkyl-OR", -alkyl-SR", COR", -alkyl-COR", -O-alkyl-COR", COOR", -alkyl-COOR", -O-alkyl-COOR", COSR", SOR", SO₂R", -O-SO₂R", -alkyl-SO₂R", OCOR", -alkyl-OCOR", or SCOR" group, which is with or without halogen; and amino, aminoalkyl, aminocarbonyl, aminocarbonylalkyl, or aminosulfonyl group which is unsubstituted or substituted by one or two groups selected from R", COR", COOR", SO₂R", and OR", wherein the R", COR", COOR", SO₂R", or OR" is with or without halogen; or, two adjacent substitutable positions of the above-mentioned "heterocyclyl", "aryl", "heteroaryl" groups are linked with -OCH₂CH₂-, -OCH₂O-, -OCH₂CH₂O- or -CH=CH-CH=CH- group to form a ring, wherein the -OCH₂CH₂-, -OCH₂O-, -OCH₂CH₂O- or -CH=CH-CH=CH- group is with or without halogen;
R" each independently represents alkyl, alkenyl, alkynyl, cycloalkyl, cycloalkylalkyl, cycloalkenyl; or heterocyclyl, heterocyclylalkyl, aryl, arylalkyl, heteroaryl, or heteroarylalkyl, which is unsubstituted or substituted.

Preferably, the above-mentioned groups are respectively unsubstituted or substituted by at least one group selected from: halogen, nitro, cyano, thiocyano, cyano C1-C6 alkyl, mercapto, hydroxy, hydroxy C1-C6 alkyl, carboxyl, formyl, tri(C1-C6 alkyl)silyl, di(C1-C6 alkyl)phosphonyl; heterocyclyl, heterocyclyl-C1-C6 alkyl, aryl, aryl-C1-C6 alkyl, heteroaryl, or heteroaryl-C1-C6 alkyl, which is unsubstituted or substituted; C1-C6 alkyl, C2-C6 alkenyl, C2-C6 alkynyl, C3-C6 cycloalkyl, C3-C6 cycloalkenyl, C3-C6 cycloalkyl-C1-C6 alkyl, C3-C6 cycloalkyl substituted by C1-C6 alkyl, OR", SR", -(C1-C6 alkyl)-OR", -O-(C1-C6 alkyl)-OR", -(C1-C6 alkyl)-SR", COR", -(C1-C6 alkyl)-COR", -O-(C1-C6 alkyl)-COR", COOR", -(C1-C6 alkyl)-COOR", -O-(C1-C6 alkyl) -COOR", COSR", SOR", SO₂R", -O-SO₂R", -(C1-C6 alkyl)-SO₂R", OCOR", -(C1-C6 alkyl) -OCOR", or SCOR", which is with or without halogen; and amino, amino-C1-C6 alkyl, aminocarbonyl, aminocarbonyl-C1-C6 alkyl, or aminosulfonyl group , which is unsubstituted or substituted by one or two groups selected from R", COR", COOR", SO₂R", and OR", wherein the R", COR", COOR", SO₂R", or OR" is with or without halogen; or, two adjacent substitutable positions of the above-mentioned "heterocyclyl", "aryl", "heteroaryl" groups are linked with -OCH₂CH₂-, -OCH₂O-, -OCH₂CH₂O-, or -CH=CH-CH=CH- group to form a ring, wherein the -OCH₂CH₂-, -OCH₂O-, -OCH₂CH₂O-, or -CH=CH-CH=CH- group is with or without halogen;
R" each independently represents C1-C6 alkyl, C2-C6 alkenyl, C2-C6 alkynyl, C3-C6 cycloalkyl, C3-C6 cycloalkyl-C1-C6 alkyl, C3-C6 cycloalkenyl; or heterocyclyl, heterocyclyl-C1-C6 alkyl, aryl, aryl-C1-C6 alkyl, heteroaryl, or heteroaryl-C1-C6 alkyl, which is unsubstituted or substituted.

More preferably, the "heterocyclyl" is 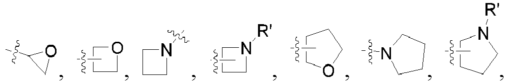 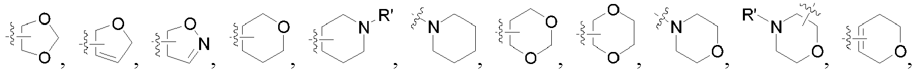 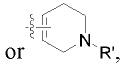 which has 0, 1, or 2 oxo groups; the "aryl" is phenyl, naphthyl, 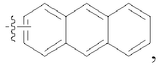 or 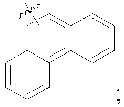 the "heteroaryl" is 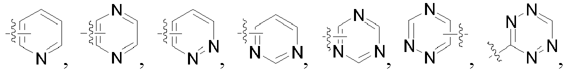 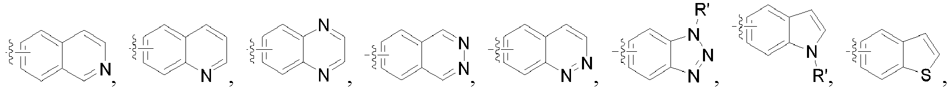 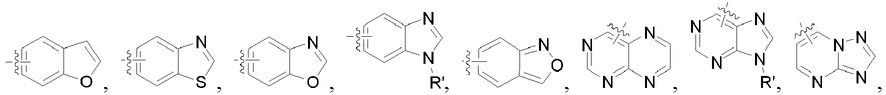 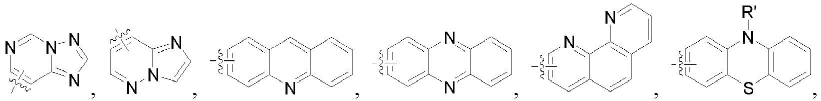 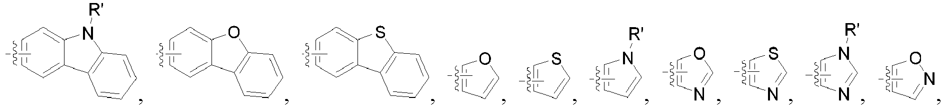 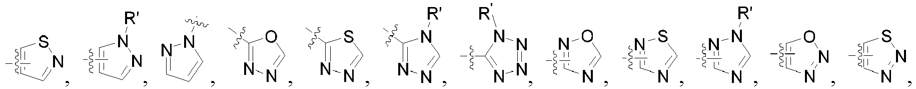 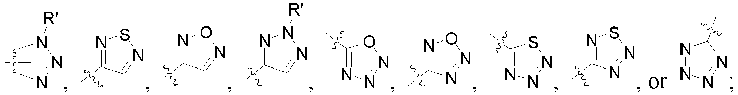 the above-mentioned groups are respectively unsubstituted or substituted by 1~3 groups selected from: halogen, nitro, cyano, thiocyano, cyano C1-C6 alkyl, mercapto, hydroxy, hydroxy C1-C6 alkyl, carboxyl, formyl; 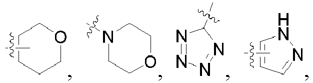 phenyl, or benzyl group, which is unsubstituted or substituted by 1~3 groups selected from halogen, hydroxy, nitro, cyano, amino, carboxyl, C1-C6 alkyl with or without halogen, C2-C6 alkenyl with or without halogen, C2-C6 alkynyl with or without halogen, C3-C6 cycloalkyl with or without halogen, C1-C6 alkoxy with or without halogen, C1-C6 alkoxycarbonyl with or without halogen, C1-C6 alkylacyl with or without halogen, C1-C6 alkylacyloxy with or without halogen, C1-C6 alkylamino with or without halogen, and C1-C6 alkylsulfonyl with or without halogen; C1-C6 alkyl, C2-C6 alkenyl, C2-C6 alkynyl, C3-C6 cycloalkyl, C3-C6 cycloalkyl-C1-C6 alkyl, C3-C6 cycloalkyl substituted by C1-C6 alkyl, OR", SR", -(C1-C6) alkyl -OR", -O-(C1-C6) alkyl -OR", -(C1-C6) alkyl -SR", COR", -(C1-C6) alkyl -COR", -O-(C1-C6) alkyl -COR", COOR", -(C1-C6) alkyl -COOR", -O-(C1-C6) alkyl -COOR", COSR", SOR", SO₂R", -O-SO₂R", -(C1-C6) alkyl -SO₂R", OCOR", -(C1-C6) alkyl -OCOR", or SCOR" group, which is with or without halogen; and amino, aminoalkyl, aminocarbonyl, or aminosulfonyl group, which is unsubstituted or substituted by one or two groups selected from R", COR", COOR", SO₂R", and OR", wherein the R", COR", COOR", SO₂R", or OR" is with or without halogen; or, two adjacent substitutable positions of the above-mentioned "heterocyclyl", "aryl", "heteroaryl" groups are linked with -OCH₂CH₂-, -OCH₂O-, -OCH₂CH₂O-, or -CH=CH-CH=CH- group to form a ring, wherein the -OCH₂CH₂-, -OCH₂O-, -OCH₂CH₂O-, or -CH=CH-CH=CH- group is with or without halogen;
R' each independently represents hydrogen, nitro, hydroxy, amino; C1-C6 alkyl, C2-C6 alkenyl, C2-C6 alkynyl, C3-C6 cycloalkyl, C3-C6 cycloalkenyl, C3-C6 cycloalkyl-C1-C6 alkyl, 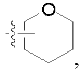 C1-C6 alkoxy, C2-C6 alkenyloxy, C2-C6 alkynyloxy, C3-C6 cycloalkyloxy, C1-C6 alkoxy-C1-C6 alkyl, C1-C6 alkoxycarbonyl, C1-C6 alkylthiocarbonyl, C1-C6 alkylsulfonyl, C1-C6 alkylsulfonyl-C1-C6 alkyl, C1-C6 alkylcarbonyl, C1-C6 alkylcarbonyl-C1-C6 alkyl, C1-C6 alkylacyloxy, C1-C6 alkylamino, C1-C6 alkylaminocarbonyl, C1-C6 alkoxyaminocarbonyl, C1-C6 alkoxycarbonyl-C1-C6 alkyl, C1-C6 alkylaminocarbonyl-C1-C6 alkyl, tri(C1-C6 alkyl)silyl, or di(C1-C6 alkyl)phosphonyl, which is with or without halogen; or phenyl, or benzyl which is unsubstituted or substituted by 1~3 groups selected from halogen, hydroxy, nitro, cyano, amino, carboxyl, C1-C6 alkyl with or without halogen, C2-C6 alkenyl with or without halogen, C2-C6 alkynyl with or without halogen, C3-C6 cycloalkyl with or without halogen, C1-C6 alkoxy with or without halogen, C1-C6 alkoxycarbonyl with or without halogen, C1-C6 alkylacyl with or without halogen, C1-C6 alkylacyloxy with or without halogen, C1-C6 alkylamino with or without halogen, and C1-C6 alkylsulfonyl with or without halogen;
R" each independently represents C1-C6 alkyl, C2-C6 alkenyl, C2-C6 alkynyl, C3-C6 cycloalkyl, C3-C6 cycloalkyl-C1-C6 alkyl, C3-C6 cycloalkenyl; or 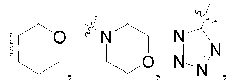 phenyl, or benzyl which is unsubstituted or substituted by 1~3 groups selected from halogen, hydroxy, nitro, cyano, amino, carboxyl, C1-C6 alkyl with or without halogen, C2-C6 alkenyl with or without halogen, C2-C6 alkynyl with or without halogen, C3-C6 cycloalkyl with or without halogen, C1-C6 alkoxy with or without halogen, C1-C6 alkoxycarbonyl with or without halogen, C1-C6 alkylacyl with or without halogen, C1-C6 alkylacyloxy with or without halogen, C1-C6 alkylamino with or without halogen, and C1-C6 alkylsulfonyl with or without halogen.

In the definition of the compound represented by the above Formula and all of the following structural formulas, the technical terms used, whether used alone or used in compound word, represent the following substituents: an alkyl having more than two carbon atoms may be linear or branched. For example, the alkyl in the compound word "-alkyl-OR¹" may be -CH₂-, -CH₂CH₂-, -CH(CH₃)-, -C(CH₃)₂-, and the like. The alkyl is, for example, C₁ alkyl: methyl; C₂ alkyl: ethyl; C₃ alkyl: propyl such as n-propyl or isopropyl; C₄ alkyl: butyl such as n-butyl, isobutyl, tert-butyl or 2-butyl; C₅ alkyl: pentyl such as n-pentyl; C₆ alkyl: hexyl such as n-hexyl, isohexyl and 1,3-dimethylbutyl. Similarly, the alkenyl is, for example, allyl, 1-methylprop-2-en-1-yl, 2-methylprop-2-en-1-yl, but-2-en-1-yl, butyl-3-en-1-yl, 1-methylbut-3-en-1-yl and 1-methylbut-2-en-1-yl. The alkynyl is, for example, propargyl, but-2-yn-1-yl, but-3-yn-1-yl, 1-methylbut-3-yn-1-yl. The multiple bond(s) may be placed at any position of each unsaturated group. The cycloalkyl is a carbocyclic saturated ring system having, for example, three to six carbon atoms, such as cyclopropyl, cyclobutyl, cyclopentyl or cyclohexyl. Similarly, the cycloalkenyl is monocycloalkenyl having, for example, three to six carbon ring members, such as cyclopropenyl, cyclobutenyl, cyclopentenyl, and cyclohexenyl, wherein double bond can be at any position. Halogen is fluorine, chlorine, bromine or iodine.

If a group is substituted by a group, which should be understood to mean that the group is substituted by one or more groups, which are same or different groups, selected from the mentioned groups. In addition, the same or different substitution characters contained in the same or different substituents are independently selected, and may be the same or different. This is also applicable to ring systems formed with different atoms and units. Meanwhile, the scope of the claims will exclude those compounds chemically unstable under standard conditions known to those skilled in the art.

In addition, unless specifically defined, the term occurring after multiple juxtaposed substituents (separated by "," or "or") in the present invention has a limiting effect on each of the substituents, such as the term "with or without halogen" in "alkyl, alkenyl, alkynyl, cycloalkyl, cycloalkenyl, cycloalkylalkyl, or cycloalkenylalkyl, which is with or without halogen" has a limiting effect on each of the following groups "alkyl", "alkenyl", "alkynyl", "cycloalkyl", "cycloalkenyl", "cycloalkylalkyl" and "cycloalkenylalkyl"; a group (including heterocyclyl, aryl, heteroaryl, etc.) without being specified a linking site may be attached at any site, including a C or N site; if it is substituted, the substituent may be substituted at any site as long as it comply with the valence bond theory. For example, if the heteroaryl 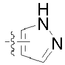 is substituted with one methyl, it can be 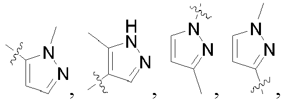 etc..

Depending on the property of substituents and the linkage manner thereof, the compound of Formula I may exist as a stereoisomer. For example, if a compound has one or more asymmetric carbon atoms, it may has enantiomers and diastereomers. The stereoisomer can be obtained from the mixtures obtained in the preparation by conventional separation methods, for example by chromatographic separation. The stereoisomer may also be prepared selectively by using stereoselective reactions and using optically active starting materials and/or auxiliaries. The present invention also relates to all stereoisomers and mixtures thereof which are included in the general Formula I but are not specifically defined.

A method for preparing the 4-pyridinyl formamide compound I, which comprises the following steps:
subjecting the compound of the general formula II and the compound of the general formula III to amidation reaction to obtain the compound of the general formula I, the chemical reaction scheme is as follows:

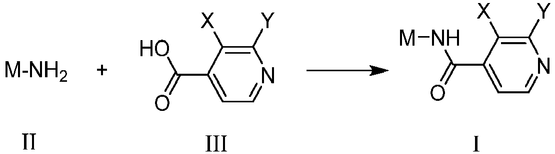

The reaction is carried out in the presence of a halogenating agent, a catalyst and a solvent; preferably, the halogenating agent is SOCl₂, the catalyst is 4-dimethylaminopyridine, and the solvent is pyridine;

Or the reaction is carried out in the presence of a condensing agent and a solvent; preferably, the condensing agent is CDI, DCC, DBU or a combination thereof, and the solvent is methylene chloride.

The preparation method of its derivative 1-1 includes the following steps:
subjecting the compound of the general formula I and Hal-Q to reaction to obtain the compound of the general formula 1-1, the chemical reaction scheme is as follows:

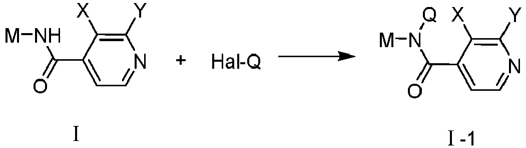

Wherein, Hal represents halogen, preferably fluorine, chlorine or bromine;

The reaction is carried out in the presence of a base and a solvent; preferably, the base is selected from one or a combination of potassium carbonate and sodium carbonate, and the solvent is one or more selected from the group consisting of DCM, DCE, ACN, THF, and DMF; or the reaction is carried out in the presence of a catalyst and a solvent; preferably, the catalyst is 4-dimethylaminopyridine, and the solvent is pyridine.

In addition, the compound of the general formula II and the compound of the general formula III are known in principle, and can be prepared, for example, by the methods described in WO199835967A2, CN103459386A, CN103987701A, WO2017055146A, WO2013017559A1, WO2012028579A1, WO2011035874A1. Moreover, the compounds of the present invention can also be prepared by referring to the methods described therein.

A herbicidal composition comprising (i) the 4-pyridinyl formamide compound or derivative thereof as shown in general formula I; preferably, further comprising (ii) one or more further herbicides and/or safeners; more preferably, further comprising (iii) agrochemically acceptable formulation auxiliaries.

A method for controlling a weed which comprises applying a herbicidally effective amount of at least one of the 4-pyridinyl formamide compound or derivative thereof or the herbicidal composition as above-described to a plant or a weed area.

Use of at least one of the 4-pyridinyl formamide compound or derivative thereof or the herbicidal composition as above-described for controlling a weed, preferably, wherein the 4-pyridinyl formamide compound or derivative thereof is used for preventing and/or controlling a weed in a useful crop, wherein the useful crop is a transgenic crop or a crop treated by gene editing technique.

The compounds of the formula I according to the invention have an outstanding herbicidal activity against a broad spectrum of economically important monocotyledonous and dicotyledonous harmful plants. The active compounds also act efficiently on perennial weeds which produce shoots from rhizomes, root stocks or other perennial organs and which are difficult to control. In this context, it is generally immaterial whether the substances are applied pre-sowing, pre-emergence or post-emergence. Specifically, examples may be mentioned of some representatives of the monocotyledonous and dicotyledonous weed flora which can be controlled by the compounds according to the invention, without these being a restriction to certain species. Examples of weed species on which the active compounds act efficiently are, from amongst the monocotyledons, Avena, Lolium, Alopecurus, Phalaris, Echinochloa, Digitaria, Setaria and also Cyperus species from the annual sector and from amongst the perennial species Agropyron, Cynodon, Imperata and Sorghum, and also perennial Cyperus species.

In the case of the dicotyledonous weed species, the spectrum of action extends to species such as, for example, Galium, Viola, Veronica, Lamium, Stellaria, Amaranthus, Sinapis, Ipomoea, Sida, Matricaria and Abutilon from amongst the annuals, and Convolvulus, Cirsium, Rumex and Artemisia in the case of the perennial weeds. The active compounds according to the invention also effect outstanding control of harmful plants which occur under the specific conditions of rice growing such as, for example, Echinochloa, Sagittaria, Alisma, Eleocharis, Scirpus and Cyperus. If the compounds according to the invention are applied to the soil surface prior to germination, then the weed seedlings are either prevented completely from emerging, or the weeds grow until they have reached the cotyledon stage but then their growth stops, and, eventually, after three to four weeks have elapsed, they die completely. In particular, the compounds according to the invention exhibit excellent activity against Apera spica venti, Chenopodium album, Lamium purpureum, Polygonum convulvulus, Stellaria media, Veronica hederifolia, Veronica persica, Viola tricolor and against Amaranthus, Galium and Kochia species.

Although the compounds according to the invention have an excellent herbicidal activity against monocotyledonous and dicotyledonous weeds, crop plants of economically important crops such as, for example, wheat, barley, rye, rice, corn, sugarbeet, cotton and soya, are not damaged at all, or only to a negligible extent. In particular, they have excellent compatibility in cereals, such as wheat, barley and corn, in particular wheat. For these reasons, the present compounds are highly suitable for selectively controlling undesired plant growth in plantings for agricultural use or in plantings of ornamentals.

Owing to their herbicidal properties, these active compounds can also be employed for controlling harmful plants in crops of known or still to be developed genetically engineered plants. The transgenic plants generally have particularly advantageous properties, for example resistance to certain pesticides, in particular certain herbicides, resistance to plant diseases or causative organisms of plant diseases, such as certain insects or microorganisms such as fungi, bacteria or viruses. Other particular properties relate, for example, to the quantity, quality, storage-stability, composition and to specific ingredients of the harvested product. Thus, transgenic plants having an increased starch content or a modified quality of the starch or those having a different fatty acid composition of the harvested produce are known.

The use of the compounds of the formula I according to the invention or their derivatives in economically important transgenic crops of useful and ornamental plants, for example of cereal, such as wheat, barley, rye, oats, millet, rice, maniok and corn, or else in crops of sugarbeet, cotton, soya, rapeseed, potato, tomato, pea and other vegetable species is preferred. The compounds of the formula I can preferably be used as herbicides in crops of useful plants which are resistant or which have been made resistant by genetic engineering toward the phytotoxic effects of the herbicides.

Conventional ways for preparing novel plants which have modified properties compared to known plants comprise, for example, traditional breeding methods and the generation of mutants. Alternatively, novel plants having modified properties can be generated with the aid of genetic engineering methods (see, for example, EP-A 0 221 044, EP-A 0 131 624). For example, there have been described several cases of:
- genetically engineered changes in crop plants in order to modify the starch synthesized in the plants (for example WO 92/11376, WO 92/14827, WO 91/19806),
- transgenic crop plants which are resistant to certain herbicides of the glufosinate- (cf., for example, EP-A 0 242 236, EP-A 0 242 246) or glyphosate-type (WO 92/00377), or of the sulfonylurea-type (EP-A 0 257 993, U.S. Pat. No. 5,013,659A),
- transgenic crop plants, for example cotton, having the ability to produce Bacillus thuringiensis toxins (Bt toxins) which impart resistance to certain pests to the plants (EP-A 0 142 924, EP-A 0 193 259),
- transgenic crop plants having a modified fatty acid composition (WO 91/13972).

Numerous molecular biological techniques which allow the preparation of novel transgenic plants having modified properties are known in principle; see, for example, Sambrook et al., 1989, Molecular Cloning, A Laboratory Manual, 2nd ed. Cold Spring Harbor Laboratory Press, Cold Spring Harbor, N.Y; or Winnacker "Gene und Klone" [Genes and Clones], VCH Weinheim, 2nd edition 1996, or Christou, "Trends in Plant Science" 1 (1996) 423-431). In order to carry out such genetic engineering manipulations, it is possible to introduce nucleic acid molecules into plasmids which allow a mutagenesis or a change in the sequence to occur by recombination of DNA sequences. Using the abovementioned standard processes it is possible, for example, to exchange bases, to remove partial sequences or to add natural or synthetic sequences. To link the DNA fragments with each other, it is possible to attach adaptors or linkers to the fragments.

Plant cells having a reduced activity of a gene product can be prepared, for example, by expressing at least one appropriate antisense-RNA, a sense-RNA to achieve a cosuppression effect, or by expressing at least one appropriately constructed ribozyme which specifically cleaves transcripts of the above-mentioned gene product.

To this end it is possible to employ both DNA molecules which comprise the entire coding sequence of a gene product including any flanking sequences that may be present, and DNA molecules which comprise only parts of the coding sequence, it being necessary for these parts to be long enough to cause an antisense effect in the cells. It is also possible to use DNA sequences which have a high degree of homology to the coding sequences of a gene product but which are not entirely identical.

When expressing nucleic acid molecules in plants, the synthesized protein can be localized in any desired compartment of the plant cells. However, to achieve localization in a certain compartment, it is, for example, possible to link the coding region with DNA sequences which ensure localization in a certain compartment. Such sequences are known to the person skilled in the art (see, for example, Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

The transgenic plant cells can be regenerated to whole plants using known techniques. The transgenic plants can in principle be plants of any desired plant species, i.e. both monocotyledonous and dicotyledonous plants. In this manner, it is possible to obtain transgenic plants which have modified properties by overexpression, suppression or inhibition of homologous (=natural) genes or gene sequences or by expression of heterologous (=foreign) genes or gene sequences.

When using the active compounds according to the invention in transgenic crops, in addition to the effects against harmful plants which can be observed in other crops, there are frequently effects which are specific for the application in the respective transgenic crop, for example a modified or specifically broadened spectrum of weeds which can be controlled, modified application rates which can be used for the application, preferably good combinability with the herbicides to which the transgenic crops are resistant, and an effect on the growth and the yield of the transgenic crop plants. The invention therefore also provides for the use of the compounds according to the invention as herbicides for controlling harmful plants in transgenic crop plants.

In addition, the substances according to the invention have outstanding growth-regulating properties in crop plants. They engage in the plant metabolism in a regulating manner and can this be employed for the targeted control of plant constituents and for facilitating harvesting, for example by provoking desiccation and stunted growth. Furthermore, they are also suitable for generally regulating and inhibiting undesirable vegetative growth, without destroying the plants in the process. Inhibition of vegetative growth plays an important role in many monocotyledon and dicotyledon crops because lodging can be reduced hereby, or prevented completely.

The compounds according to the invention can be applied in the customary formulations in the form of wettable powders, emulsifiable concentrates, sprayable solutions, dusts or granules. The invention therefore also provides herbicidal compositions comprising compounds of the formula I. The compounds of the formula I can be formulated in various ways depending on the prevailing biological and/or chemico-physical parameters. Examples of suitable formulation options are: wettable powders (WP), water-soluble powders (SP), water-soluble concentrates, emulsifiable concentrates (EC), emulsions (EW), such as oil-in-water and water-in-oil emulsions, sprayable solutions, suspension concentrates (SC), oil dispersions (OD), oil- or water-based dispersions, oil-miscible solutions, dusts (DP), capsule suspensions (CS), seed-dressing compositions, granules for broadcasting and soil application, granules (GR) in the form of microgranules, spray granules, coating granules and adsorption granules, water-dispersible granules (WG), water-soluble granules (SG), ULV formulations, microcapsules and waxes. These individual formulation types are known in principle and are described, for example, in Winnacker-Küchler, "Chemische Technologie" [Chemical Technology], Volume 7, C. Hauser Verlag Munich, 4th. Edition 1986; Wade van Valkenburg, "Pesticide Formulations", Marcel Dekker, N.Y., 1973; K. Martens, "Spray Drying" Handbook, 3rd Ed. 1979, G. Goodwin Ltd. London.

The necessary formulation auxiliaries, such as inert materials, surfactants, solvents and other additives, are likewise known and are described, for example, in Watkins, "Handbook of Insecticide Dust Diluents and Carriers", 2nd Ed., Darland Books, Caldwell N.J., H. v. Olphen, "Introduction to Clay Colloid Chemistry"; 2nd Ed., J. Wiley & Sons, N.Y; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y 1964; Schönfeldt, "Grenzflüchenaktive Athylenoxidaddkte" [Surface-active ethylene oxide adducts], Wiss. Verlagagesell. Stuttgart 1976; Winnacker-Küchler, "Chemische Technologie" [Chemical Technology], Volume 7, C. Hauser Verlag Munich, 4th Edition 1986.

Wettable powders are preparations which are uniformly dispersible in water and which contain, in addition to the active compound and as well as a diluent or inert substance, surfactants of ionic and/or nonionic type (wetting agents, dispersants), for example polyethoxylated alkyl phenols, polyethoxylated fatty alcohols, polyethoxylated fatty amines, fatty alcohol polyglycol ethersulfates, alkanesulfonates, alkylbenzenesulfonates, sodium ligninsulfonate, sodium 2,2'-dinaphthylmethane-6,6'-disulfonate, sodium dibutyinaphthalenesulfona-te or else sodium oleoylmethyltaurinate. To prepare the wettable powders, the herbicidally active compounds are finely ground, for example in customary apparatus such as hammer mills, fan mills and air-jet mills, and are mixed simultaneously or subsequently with the formulation auxiliaries.

Emulsifiable concentrates are prepared by dissolving the active compound in an organic solvent, for example butanol, cyclohexanone, dimethylformamide, xylene or else relatively high-boiling aromatic compounds or hydrocarbons or mixtures of the solvents, with the addition of one or more surfactants of ionic and/or nonionic type (emulsifiers). Examples of emulsifiers which can be used are calcium alkylarylsulfonates, such as Ca dodecylbenzenesulfonate, or nonionic emulsifiers, such as fatty acid polyglycol esters, alkylaryl polyglycol ethers, fatty alcohol polyglycol ethers, propylene oxide-ethylene oxide condensation products, alkyl polyethers, sorbitan esters, for example sorbitan fatty acid esters or polyoxyethylene sorbitan esters, for example polyoxyethylene sorbitan fatty acid esters.

Dusts are obtained by grinding the active compound with finely divided solid substances, for example talc, natural clays, such as kaolin, bentonite and pyrophyllite, or diatomaceous earth. Suspension concentrates can be water- or oil-based. They can be prepared, for example, by wet milling using commercially customary bead mills, with or without the addition of surfactants as already mentioned above, for example, in the case of the other formulation types.

Emulsions, for example oil-in-water emulsions (EW), can be prepared for example by means of stirrers, colloid mills and/or static mixers using aqueous organic solvents and, if desired, surfactants as already mentioned above, for example, in the case of the other formulation types.

Granules can be prepared either by spraying the active compound onto adsorptive, granulated inert material or by applying active-compound concentrates to the surface of carriers such as sand, kaolinites or granulated inert material, by means of adhesive binders, for example polyvinyl alcohol, sodium polyacrylate or else mineral oils. Suitable active compounds can also be granulated in the manner which is customary for the preparation of fertilizer granules, if desired as a mixture with fertilizers. Water-dispersible granules are generally prepared by the customary processes, such as spray-drying, fluidized-bed granulation, disk granulation, mixing using high-speed mixers, and extrusion without solid inert material.

For the preparation of disk, fluidized-bed, extruder and spray granules, see for example processes in "Spray-Drying Handbook" 3rd ed. 1979, G. Goodwin Ltd., London; J. E. Browning, "Agglomeration", Chemical and Engineering 1967, pages 147 ff.; "Perry's Chemical Engineer's Handbook", 5th Ed., McGraw-Hill, New York 1973, pp. 8-57. For further details on the formulation of crop protection products, see for example G. C. Klingman, "Weed Control as a Science", John Wiley and Sons Inc., New York, 1961, pages 81-96 and J. D. Freyer, S. A. Evans, "Weed Control Handbook", 5th Ed., Blackwell Scientific Publications, Oxford, 1968, pages 101-103.

The agrochemical formulations generally contain from 0.1 to 99% by weight, in particular from 0.1 to 95% by weight, of active compound of the formula I. In wettable powders the concentration of active compound is, for example, from about 10 to 99% by weight, the remainder to 100% by weight consisting of customary formulation constituents. In emulsifiable concentrates the concentration of active compound can be from about 1 to 90%, preferably from 5 to 80%, by weight. Formulations in the form of dusts contain from 1 to 30% by weight of active compound, preferably most commonly from 5 to 20% by weight of active compound, while sprayable solutions contain from about 0.05 to 80%, preferably from 2 to 50%, by weight of active compound. In the case of water-dispersible granules the content of active compound depends partly on whether the active compound is in liquid or solid form and on the granulation auxiliaries, fillers, etc. that are used. In water-dispersible granules the content of active compound, for example, is between 1 and 95% by weight, preferably between 10 and 80% by weight.

In addition, the formulations of active compound may comprise the tackifiers, wetting agents, dispersants, emulsifiers, penetrants, preservatives, antifreeze agents, solvents, fillers, carriers, colorants, antifoams, evaporation inhibitors and pH and viscosity regulators which are customary in each case.

Based on these formulations it is also possible to produce combinations with other pesticidally active substances, for example insecticides, acaricides, herbicides and fungicides, and also with safeners, fertilizers and/or growth regulators, for example in the form of a ready-mix or tank mix.

Suitable active compounds which can be combined with the active compounds according to the invention in mixed formulations or in a tank mix are, for example, known active compounds as described in for example World Herbicide New Product Technology Handbook, China Agricultural Science and Farming Techniques Press, 2010.9 and in the literature cited therein. For example the following active compounds may be mentioned as herbicides which can be combined with the compounds of the formula I (note: the compounds are either named by the "common name" in accordance with the International Organization for Standardization (ISO) or by the chemical names, if appropriate together with a customary code number): acetochlor, butachlor, alachlor, propisochlor, metolachlor, s-metolachlor, pretilachlor, propachlor, ethachlor, napropamide, R-left handed napropamide, propanil, mefenacet, diphenamid, diflufenican, ethaprochlor, beflubutamid, bromobutide, dimethenamid, dimethenamid-P, etobenzanid, flufenacet, thenylchlor, metazachlor, isoxaben, flamprop-M-methyl, flamprop-M-propyl, allidochlor, pethoxamid, chloranocryl, cyprazine, mefluidide, monalide, delachlor, prynachlor, terbuchlor, xylachlor, dimethachlor, cisanilide, trimexachlor, clomeprop, propyzamide, pentanochlor, carbetamide, benzoylprop-ethyl, cyprazole, butenachlor, tebutam, benzipram, mogrton, dichlofluanid, naproanilide, diethatyl-ethyl, naptalam, flufenacet, EL-177, benzadox, chlorthiamid, chlorophthalimide, isocarbamide, picolinafen, atrazine, simazine, prometryn, cyanatryn, simetryn, ametryn, propazine, dipropetryn, SSH-108, terbutryn, terbuthylazine, triaziflam, cyprazine, proglinazine, trietazine, prometon, simetone, aziprotryne, desmetryn, dimethametryn, procyazine, mesoprazine, sebuthylazine, secbumeton, terbumeton, methoprotryne, cyanatryn, ipazine, chlorazine, atraton, pendimethalin, eglinazine, cyanuric acid, indaziflam, chlorsulfuron, metsulfuron-methyl, bensulfuron methyl, chlorimuron-ethyl, tribenuron-methyl, thifensulfuron-methyl, pyrazosulfuron-ethyl, mesosulfuron, iodosulfuron-methyl sodium, foramsulfuron, cinosulfuron, triasulfuron, sulfometuron methyl, nicosulfuron, ethametsulfuron-methyl, amidosulfuron, ethoxysulfuron, cyclosulfamuron, rimsulfuron, azimsulfuron, flazasulfuron, monosulfuron, monosulfuron-ester, flucarbazone-sodium, flupyrsulfuron-methyl, halosulfuron-methyl, oxasulfuron, imazosulfuron, primisulfuron, propoxycarbazone, prosulfuron, sulfosulfuron, trifloxysulfuron, triflusulfuron-methyl, tritosulfuron, sodium metsulfuron methyl, flucetosulfuron, HNPC-C, orthosulfamuron, propyrisulfuron, metazosulfuron, acifluorfen, fomesafen, lactofen, fluoroglycofen, oxyfluorfen, chlornitrofen, aclonifen, ethoxyfen-ethyl, bifenox, nitrofluorfen, chlomethoxyfen, fluorodifen, fluoronitrofen, furyloxyfen, nitrofen, TOPE, DMNP, PPG1013, AKH-7088, halosafen, chlortoluron, isoproturon, linuron, diuron, dymron, fluometuron, benzthiazuron, methabenzthiazuron, cumyluron, ethidimuron, isouron, tebuthiuron, buturon, chlorbromuron, methyldymron, phenobenzuron, SK-85, metobromuron, metoxuron, afesin, monuron, siduron, fenuron, fluothiuron, neburon, chloroxuron, noruron, isonoruron, 3-cyclooctyl-1, thiazfluron, tebuthiuron, difenoxuron, parafluron, methylamine tribunil, karbutilate, trimeturon, dimefuron, monisouron, anisuron, methiuron, chloreturon, tetrafluron, phenmedipham, phenmedipham-ethyl, desmedipham, asulam, terbucarb, barban, propham, chlorpropham, rowmate, swep, chlorbufam, carboxazole, chlorprocarb, fenasulam, BCPC, CPPC, carbasulam, butylate, benthiocarb, vernolate, molinate, triallate, dimepiperate, esprocarb, pyributicarb, cycloate, avadex, EPTC, ethiolate, orbencarb, pebulate, prosulfocarb, tiocarbazil, CDEC, dimexano, isopolinate, methiobencarb, 2,4-D butyl ester, MCPA-Na, 2,4-D isooctyl ester, MCPA isooctyl ester, 2,4-D sodium salt, 2,4-D dimethyla mine salt, MCPA-thioethyl, MCPA, 2,4-D propionic acid, high 2,4-D propionic acid salt, 2,4-D butyric acid, MCPA propionic acid, MCPA propionic acid salt, MCPA butyric acid, 2,4,5-D, 2,4,5-D propionic acid, 2,4,5-D butyric acid, MCPA amine salt, dicamba, erbon, chlorfenac, saison, TBA, chloramben, methoxy-TBA, diclofop-methyl, fluazifop-butyl, fluazifop-p-butyl, haloxyfop-methyl, haloxyfop-P, quizalofop-ethyl, quizalofop-p-ethyl, fenoxaprop-ethy, fenoxaprop-p-ethyl, propaquizafop, cyhalofop-butyl, metamifop, clodinafop-propargyl, fenthiaprop-ethyl, chloroazifop-propynyl, poppenate-methyl, trifopsime, isoxapyrifop, paraquat, diquat, oryzalin, ethalfluralin, isopropalin, nitralin, profluralin, prodinamine, benfluralin, fluchloraline, dinitramina, dipropalin, chlornidine, methalpropalin, dinoprop, glyphosate, anilofos, glufosinate ammonium, amiprophos-methyl, sulphosate, piperophos, bialaphos-sodium, bensulide, butamifos, phocarb, 2,4-DEP, H-9201, zytron, imazapyr, imazethapyr, imazaquin, imazamox, imazamox ammonium salt, imazapic, imazamethabenz-methyl, fluroxypyr, fluroxypyr isooctyl ester, clopyralid, picloram, trichlopyr, dithiopyr, haloxydine, 3,5,6-trichloro-2-pyridinol, thiazopyr, fluridone, aminopyralid, diflufenzopyr, triclopyr-butotyl, Cliodinate, sethoxydim, clethodim, cycloxydim, alloxydim, clefoxydim, butroxydim, tralkoxydim, tepraloxydim, buthidazole, metribuzin, hexazinone, metamitron, ethiozin, ametridione, amibuzin, bromoxynil, bromoxynil octanoate, ioxynil octanoate, ioxynil, dichlobenil, diphenatrile, pyraclonil, chloroxynil, iodobonil, flumetsulam, florasulam, penoxsulam, metosulam, cloransulam-methyl, diclosulam, pyroxsulam, benfuresate, bispyribac-sodium, pyribenzoxim, pyriftalid, pyriminobac-methyl, pyrithiobac-sodium, benzobicylon, mesotrione, sulcotrione, tembotrione, tefuryltrione, bicyclopyrone, ketodpiradox, isoxaflutole, clomazone, fenoxasulfone, methiozolin, fluazolate, pyraflufen-ethyl, pyrazolynate, difenzoquat, pyrazoxyfen, benzofenap, nipyraclofen, pyrasulfotole, topramezone, pyroxasulfone, cafenstrole, flupoxam, aminotriazole, amicarbazone, azafenidin, carfentrazone-ethyl, sulfentrazone, bencarbazone, benzfendizone, butafenacil, bromacil, isocil, lenacil, terbacil, flupropacil, cinidon-ethyl, flumiclorac-pentyl, flumioxazin, propyzamide, MK-129, flumezin, pentachlorophenol, dinoseb, dinoterb, dinoterb acetate, dinosam, DNOC, chloronitrophene, medinoterb acetate, dinofenate, oxadiargyl, oxadiazon, pentoxazone, Flufenacet, fluthiacet-methyl, fentrazamide, flufenpyr-ethyl, pyrazon, brompyrazon, metflurazon, kusakira, dimidazon, oxapyrazon, norflurazon, pyridafol, quinclorac, quinmerac, bentazone, pyridate, oxaziclomefone, benazolin, clomazone, cinmethylin, ZJ0702, pyribambenz-propyl, indanofan, sodium chlorate, dalapon, trichloroacetic acid, monochloroacetic acid, hexachloroacetone, flupropanate, cyperquat, bromofenoxim, epronaz, methazole, flurtamone, benfuresate, ethofumesate, tioclorim, chlorthal, fluorochloridone, tavron, acrolein, bentranil, tridiphane, chlorfenpropmethyl, thidiarizonaimin, phenisopham, busoxinone, methoxyphenone, saflufenacil, clacyfos, chloropon, alorac, diethamquat, etnipromid, iprymidam, ipfencarbazone, thiencarbazone-methyl, pyrimisulfan, chlorflurazole, tripropindan, sulglycapin, prosulfalin, cambendichlor, aminocyclopyrachlor, rodethanil, benoxacor, fenclorim, flurazole, fenchlorazole-ethyl, cloquintocet-mexyl, oxabetrinil, MG/91, cyometrinil, DKA-24, mefenpyr-diethyl, furilazole, fluxofenim, isoxadifen-ethyl, dichlormid, halauxifen-methyl, DOW florpyrauxifen, UBH-509, D489, LS 82-556, KPP-300, NC-324, NC-330, KH-218, DPX-N8189, SC-0744, DOWCO535, DK-8910, V-53482, PP-600, MBH-001, KIH-9201, ET-751, KIH-6127 and KIH-2023.

In the context of the present specification, if an abbreviation of a generic name of an active compound is used, it includes in each case all customary derivatives, such as esters and salts, as well as isomers, in particular optical isomers, especially one or more commercially available forms. If the generic name denotes an ester or a salt, it also includes in each case all other conventional derivatives, such as other esters and salts, free acids and neutral compounds, as well as isomers, in particular optical isomers, especially one or more commercially available forms. The chemical name given to a compound means at least one compound encompassed by the generic name, and generally the preferred compound. In the case of sulfonamides such as sulfonylurea, salts also include salts formed by the exchange of cations with hydrogen atoms in the sulfonamide group. For example, 2,4-D or 2,4-DB derivatives include but are not limited to: 2,4- D or 2,4-DB salt such as sodium salt, potassium salt, dimethylammonium salt, triethanolammonium salt, isopropylamine salt, choline, etc., and 2,4-D or 2,4-DB esters such as methyl ester, ethyl ester, butyl ester, isooctyl ester, etc.; 2-methyl-4-chloro derivatives include but are not limited to: 2-methyl-4-chloro sodium salt, -potassium salt, -dimethylammonium salt, -isopropylamine salt, etc., as well as 2-methyl-4-chloro methyl ester, -ethyl ester, -isooctyl ester, -thioethyl, etc..

For use, the formulations which are present in commercially available form are, if appropriate, diluted in the customary manner, for example using water in the case of wettable powders, emulsifiable concentrates, dispersions and water-dispersible granules. Products in the form of dusts, granules for soil application or broadcasting and sprayable solutions are usually not further diluted with other inert substances prior to use. The application rate of the compounds of the formula I required varies with the external conditions, such as temperature, humidity, the nature of the herbicide used and the like. It can vary within wide limits, for example between 0.001 and 1.0 kg a.i./ha or more of active substance, but it is preferably between 0.005 and 750 g a.i./ha, in particular between 0. 005 and 500 g a.i./ha.

### Specific Mode for Carrying out the Invention

The following embodiments are used to illustrate the present invention in detail and should not be taken as any limit to the present invention. The scope of the invention would be explained through the Claims.

In view of economics and variety of a compound, we preferably synthesized several compounds, part of which are listed in the following Table A1. The structure and information of a certain compound are shown in Table A1. The compounds in Table A1 are listed for further explication of the present invention, other than any limit therefor. The subject of the present invention should not be interpreted by those skilled in the art as being limited to the following compounds.

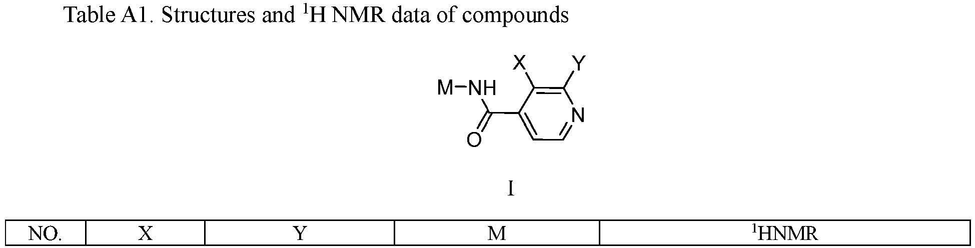

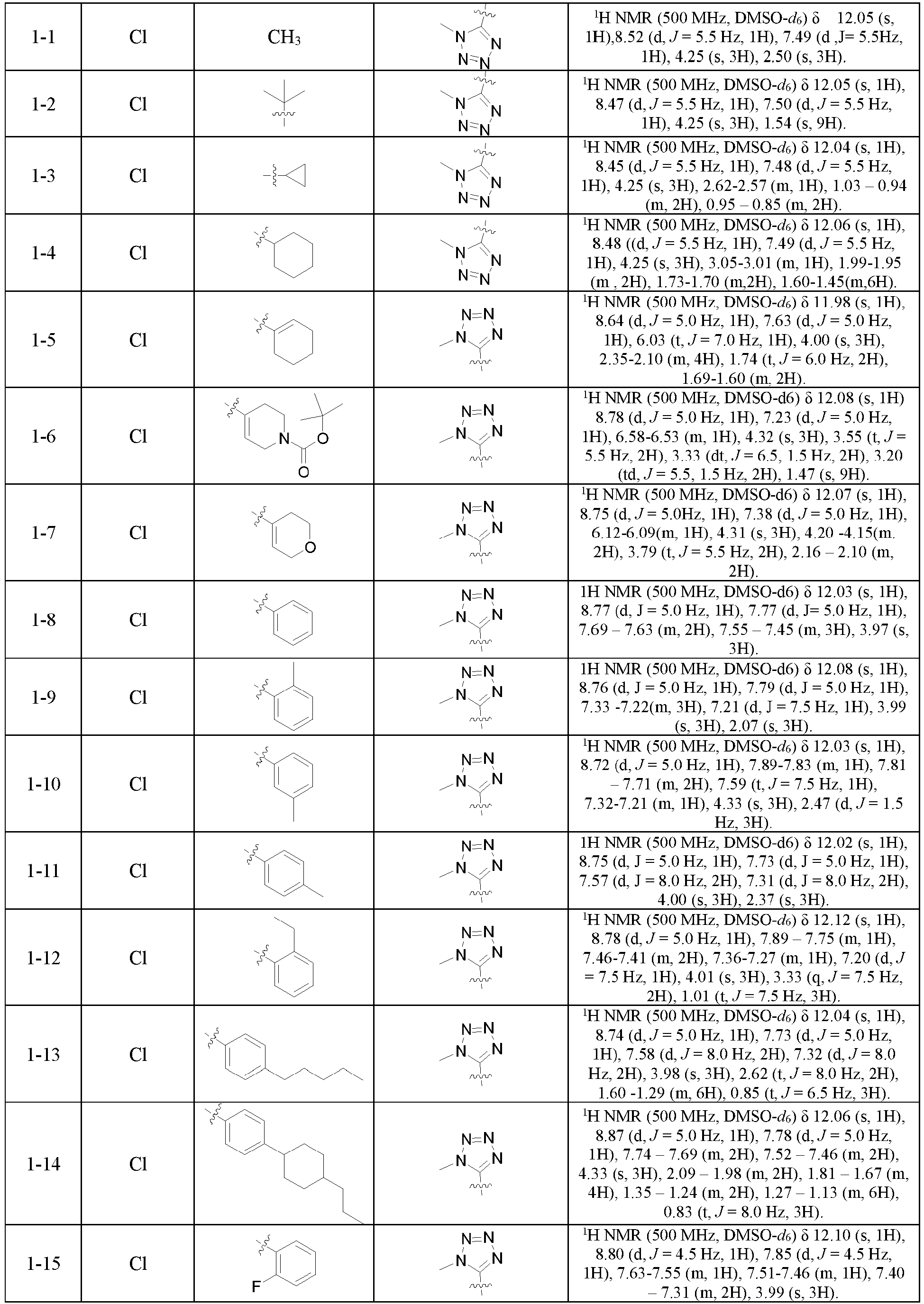

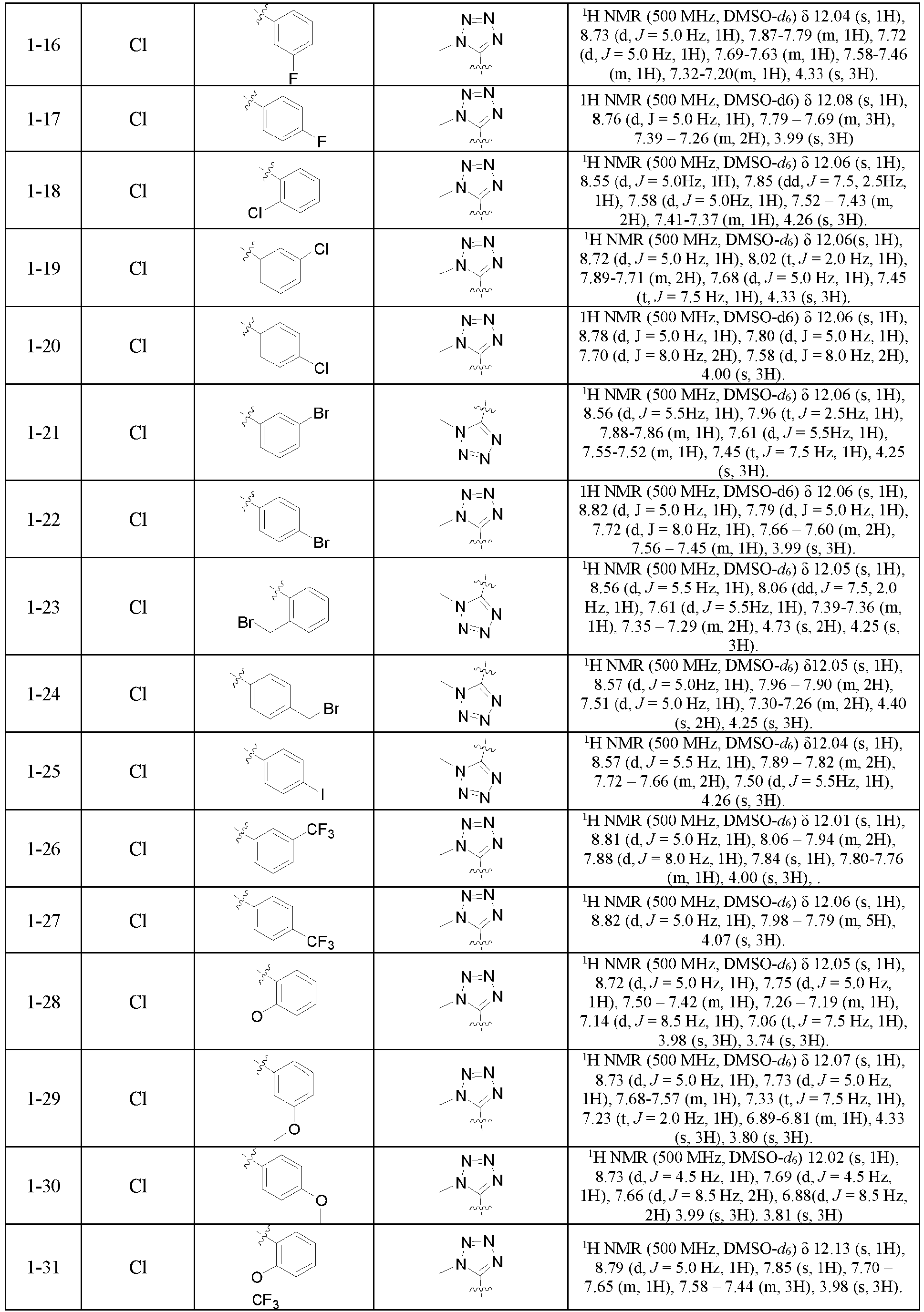

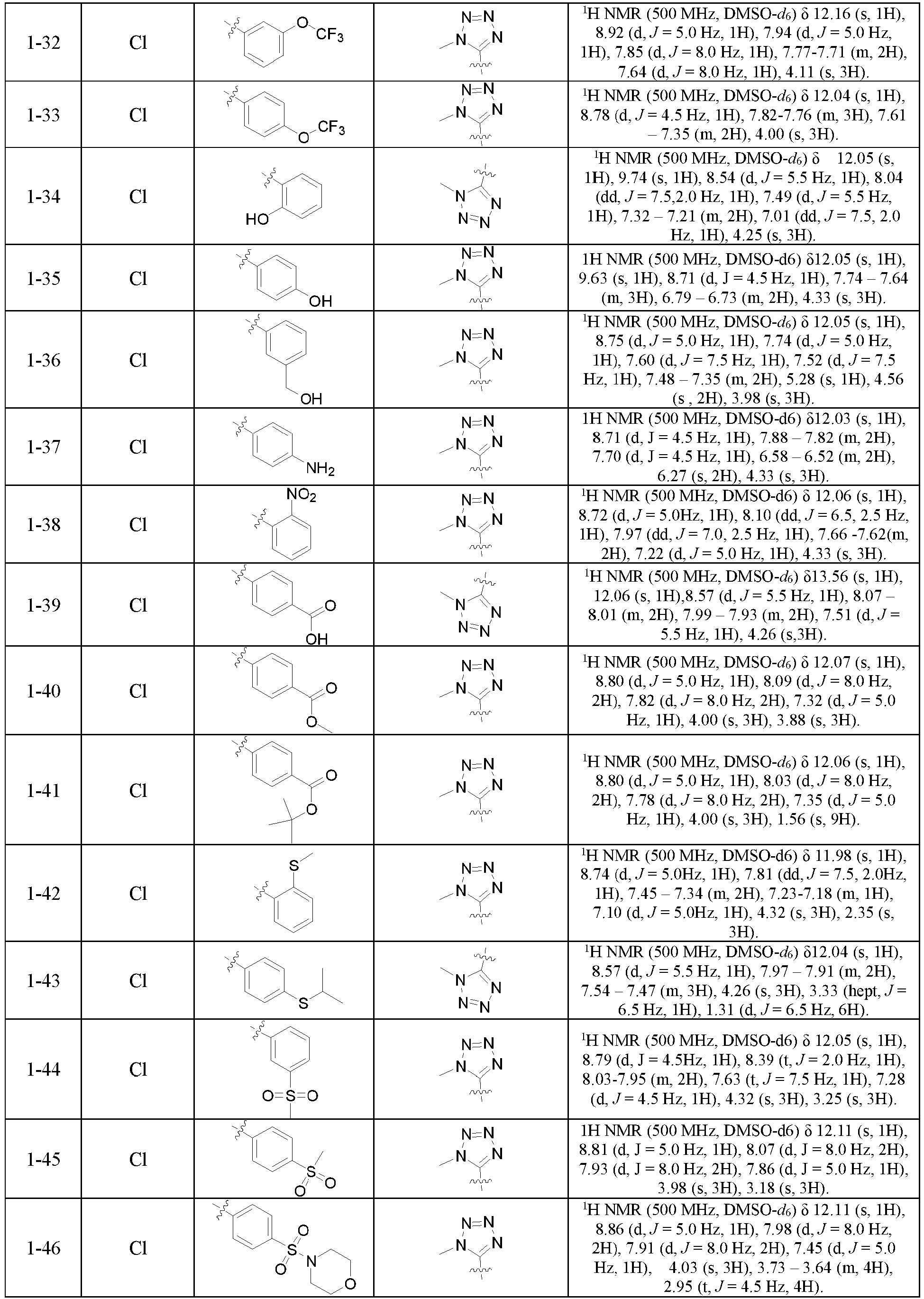

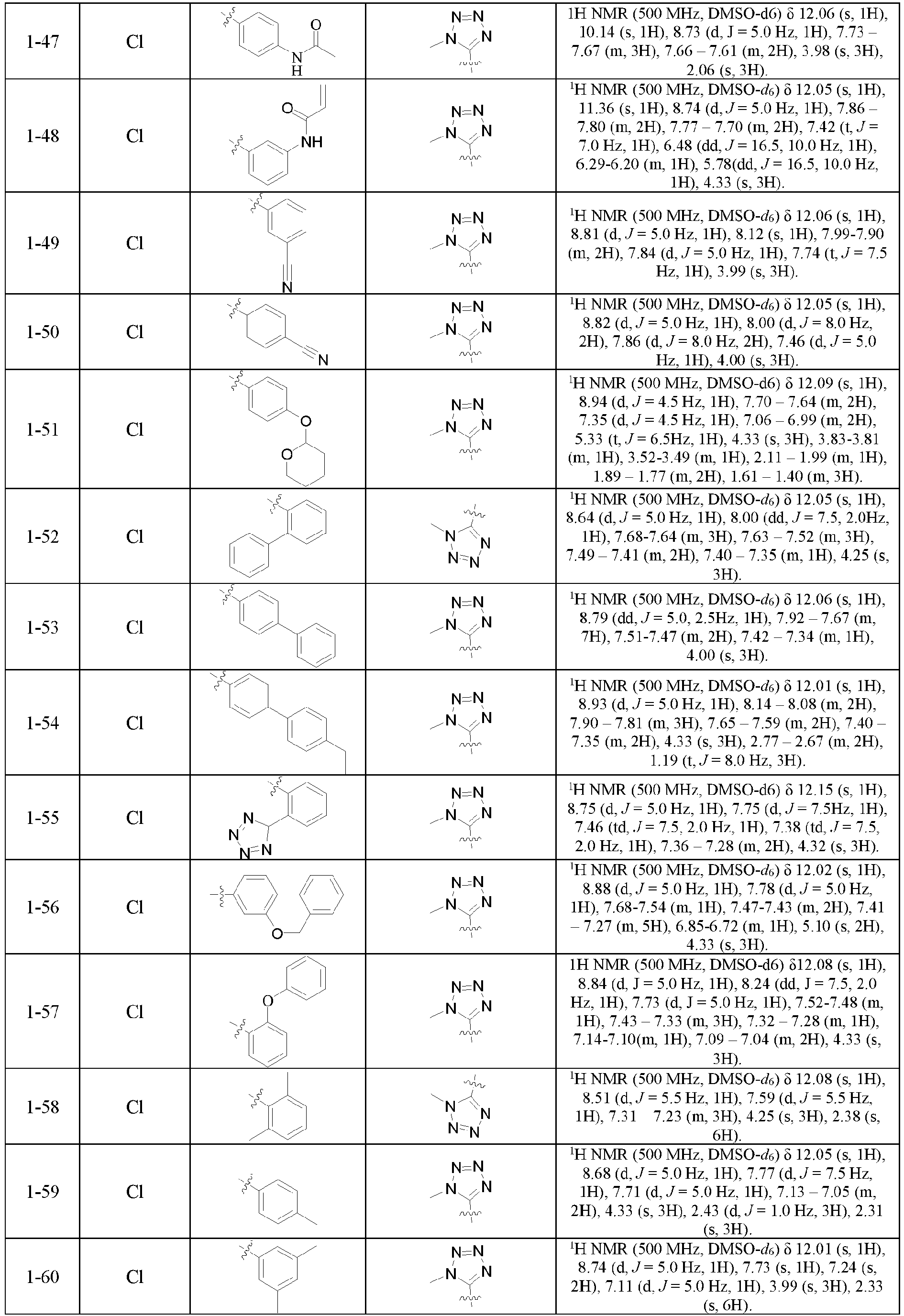

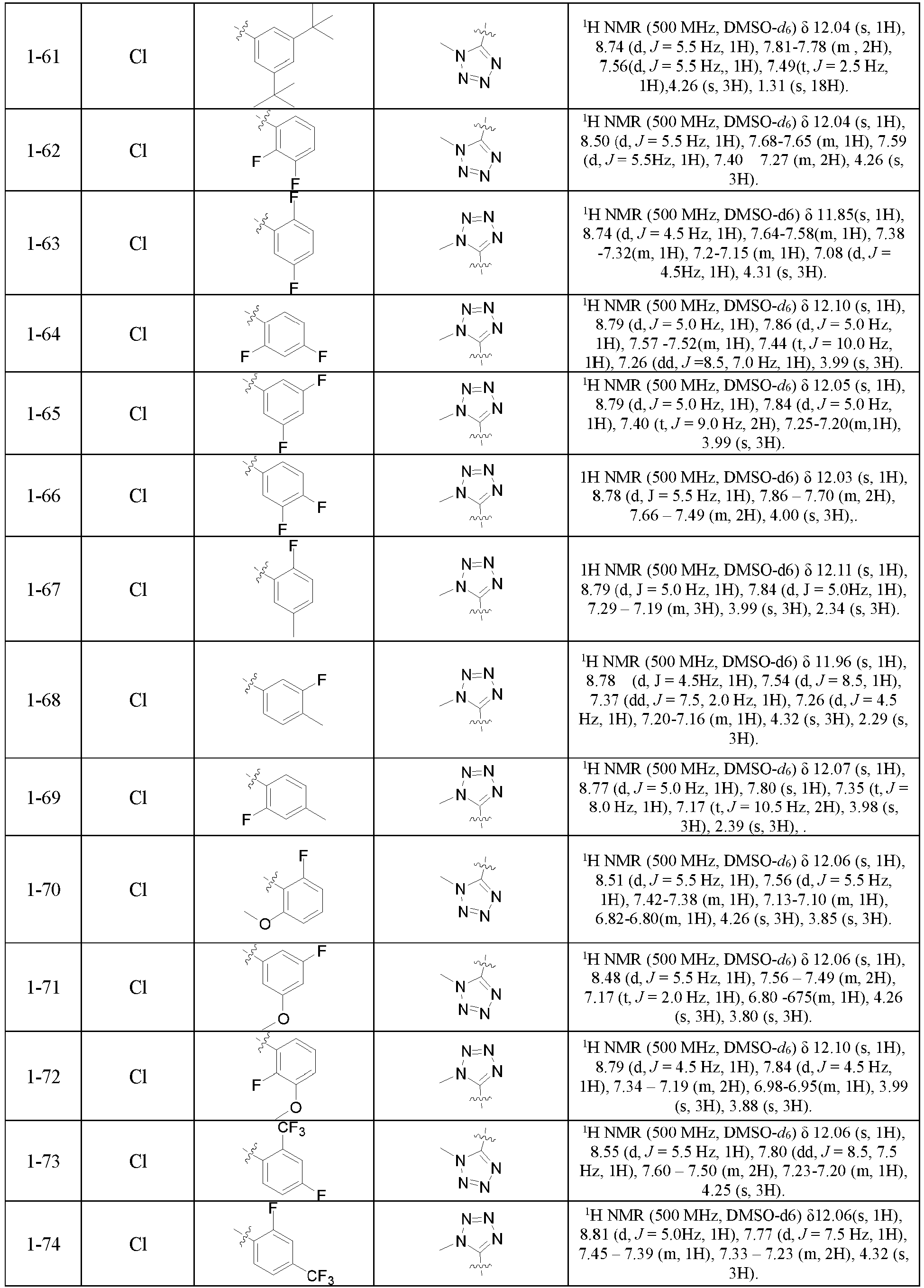

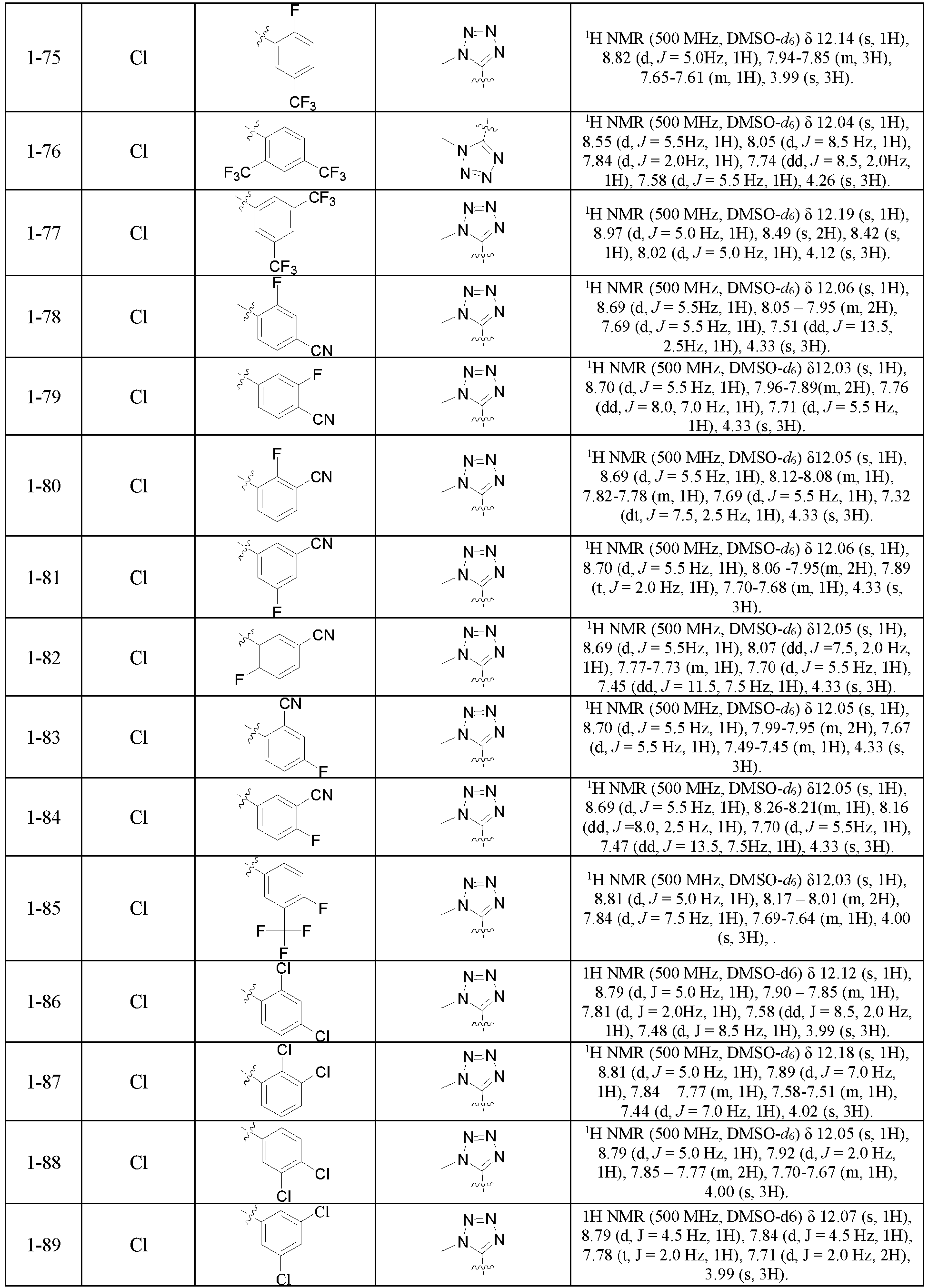

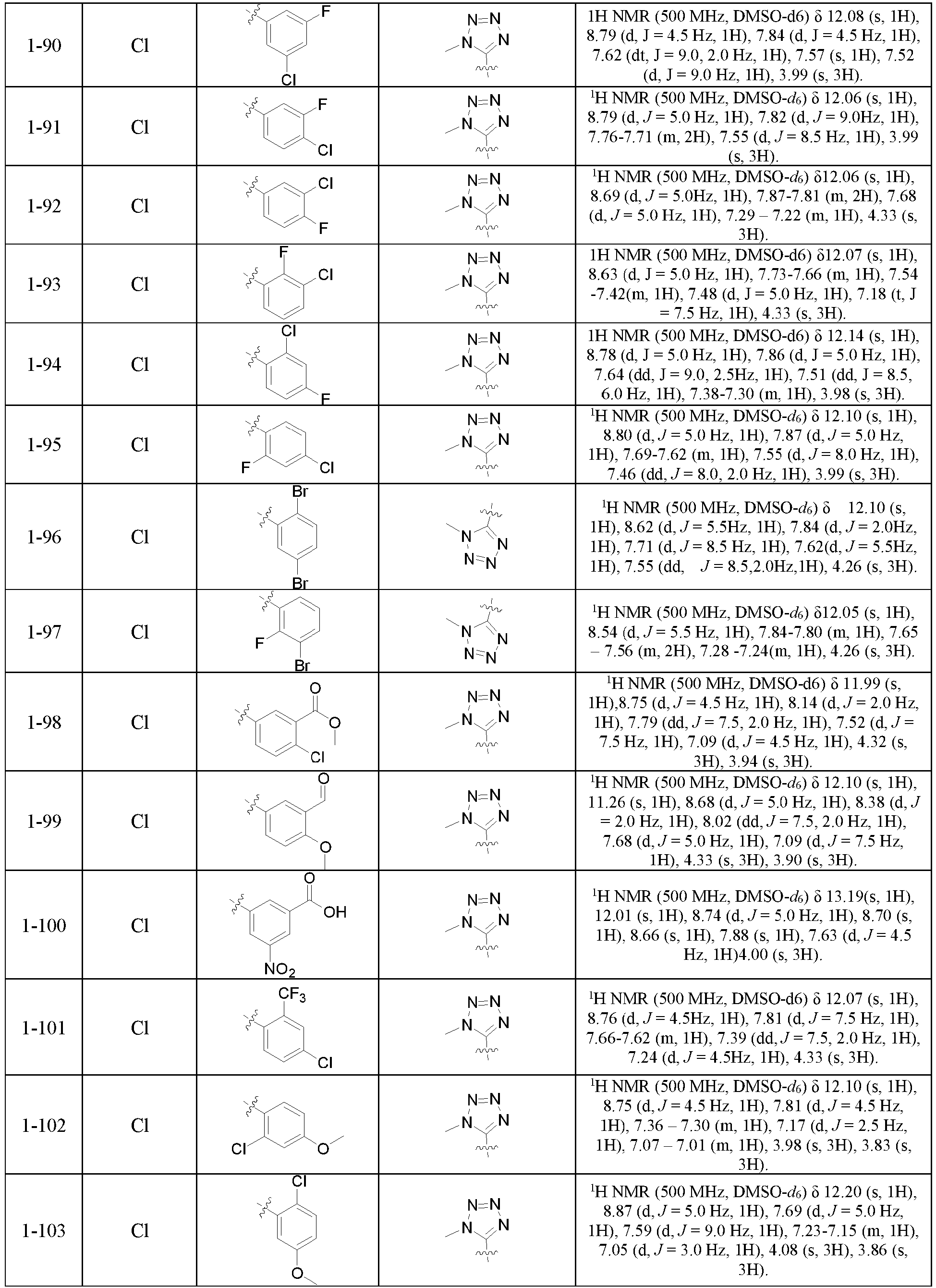

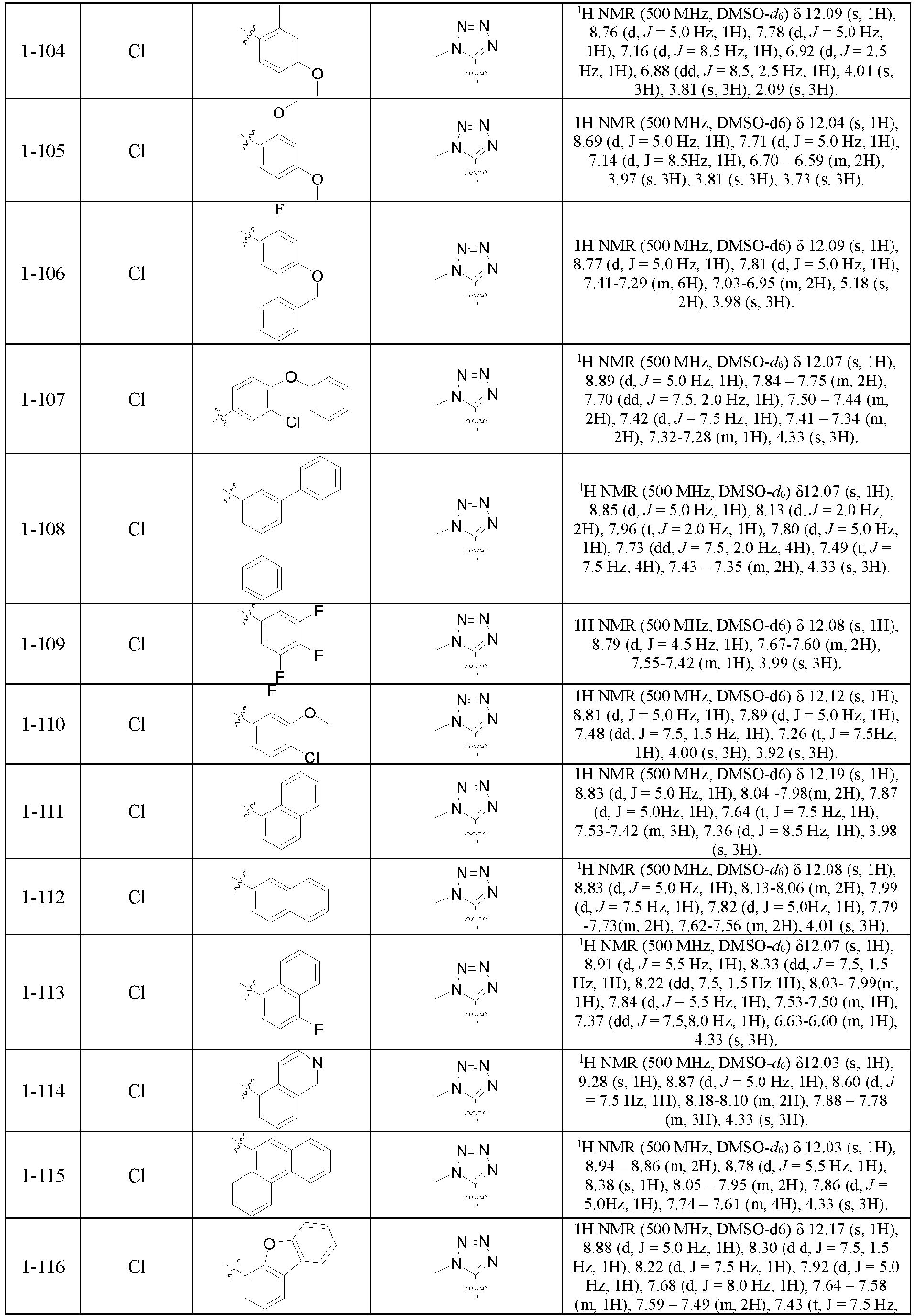

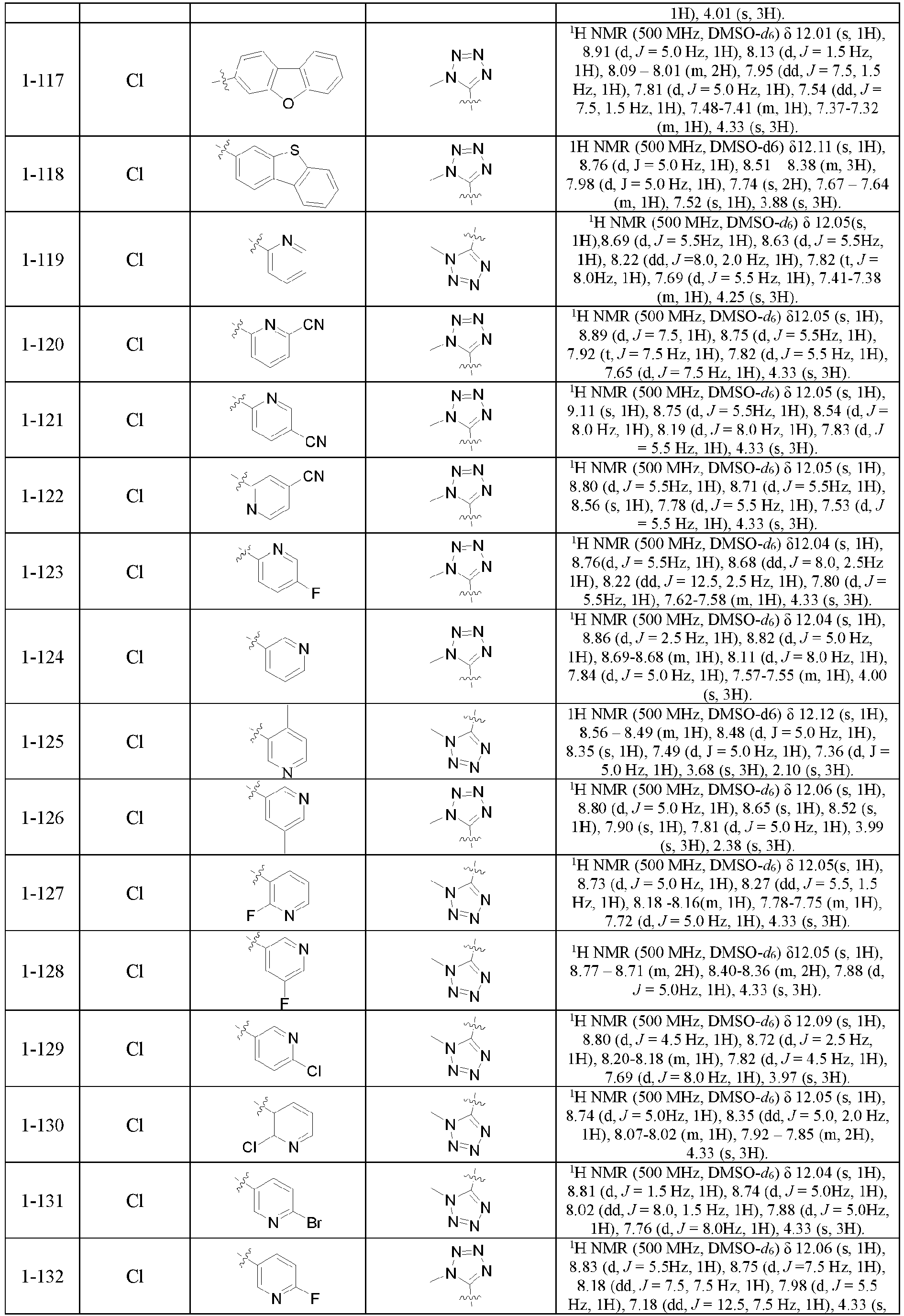

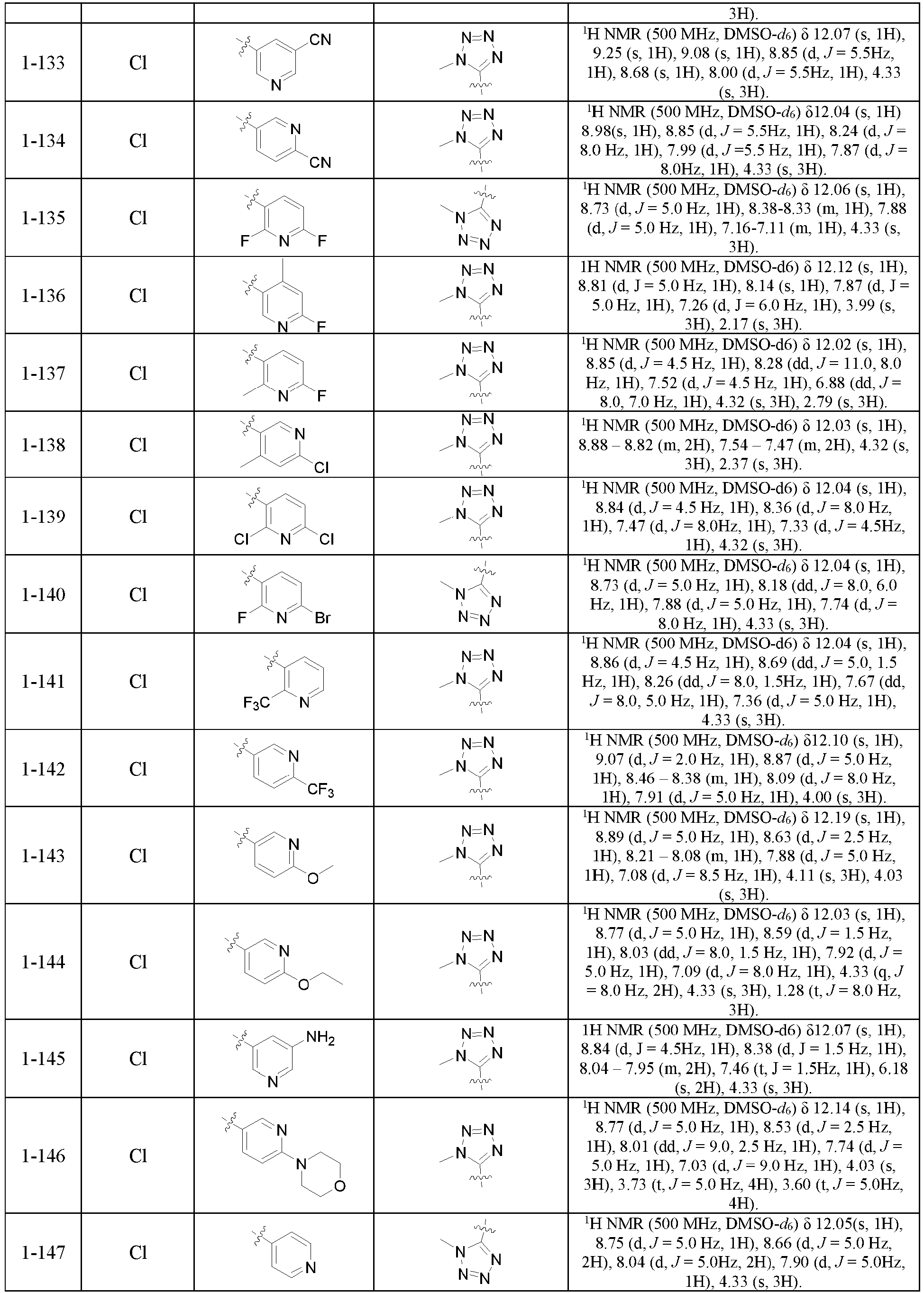

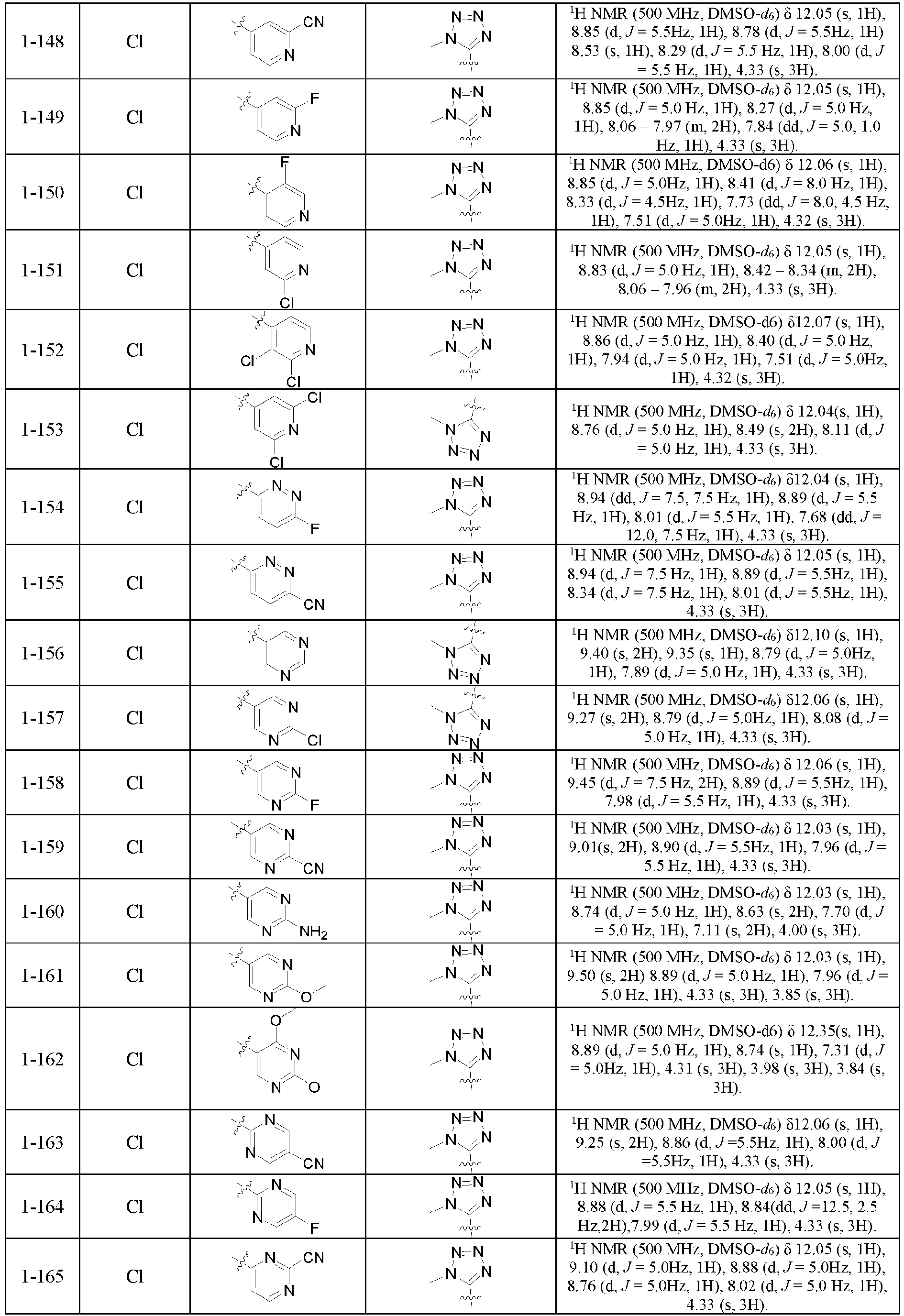

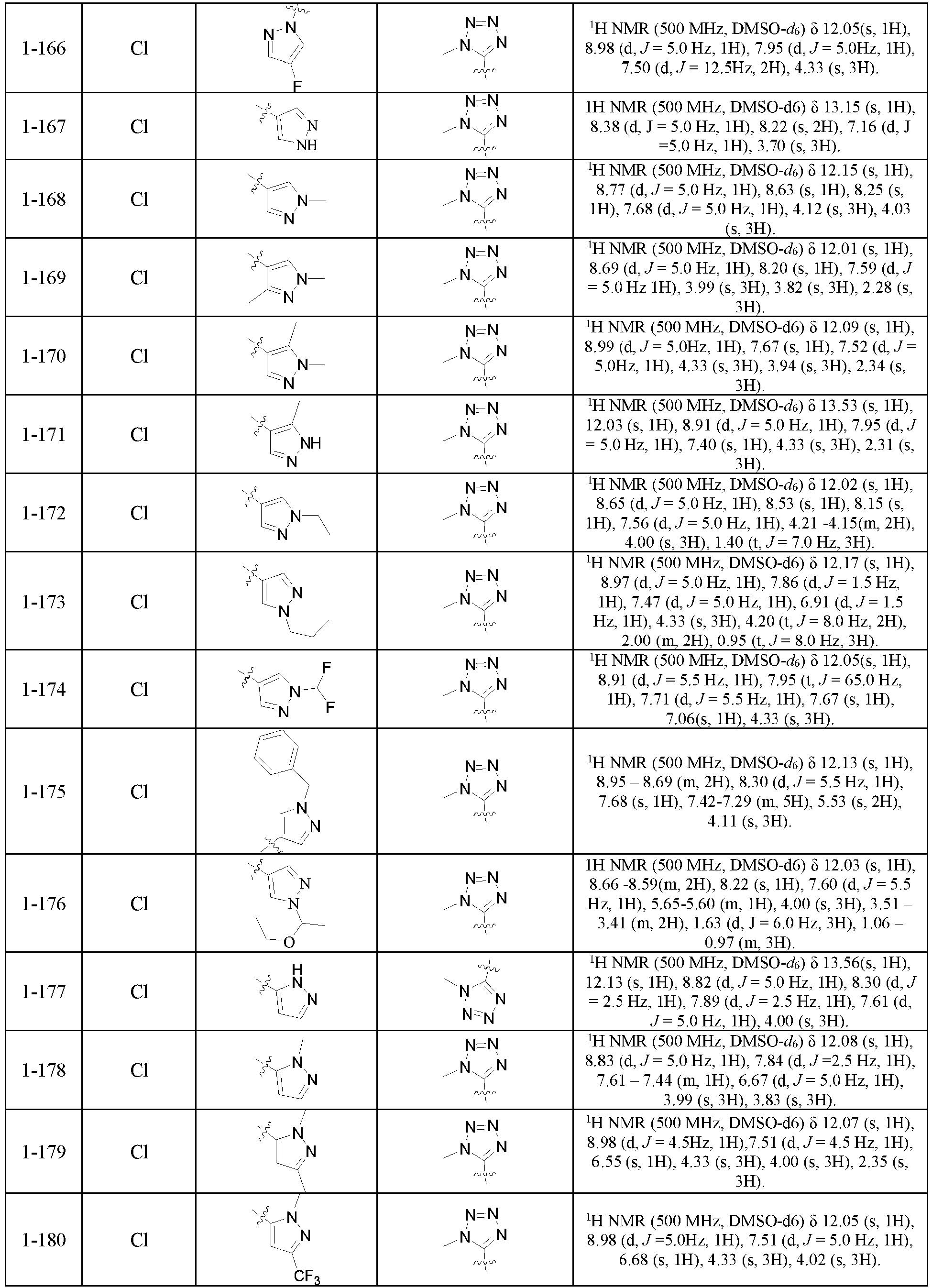

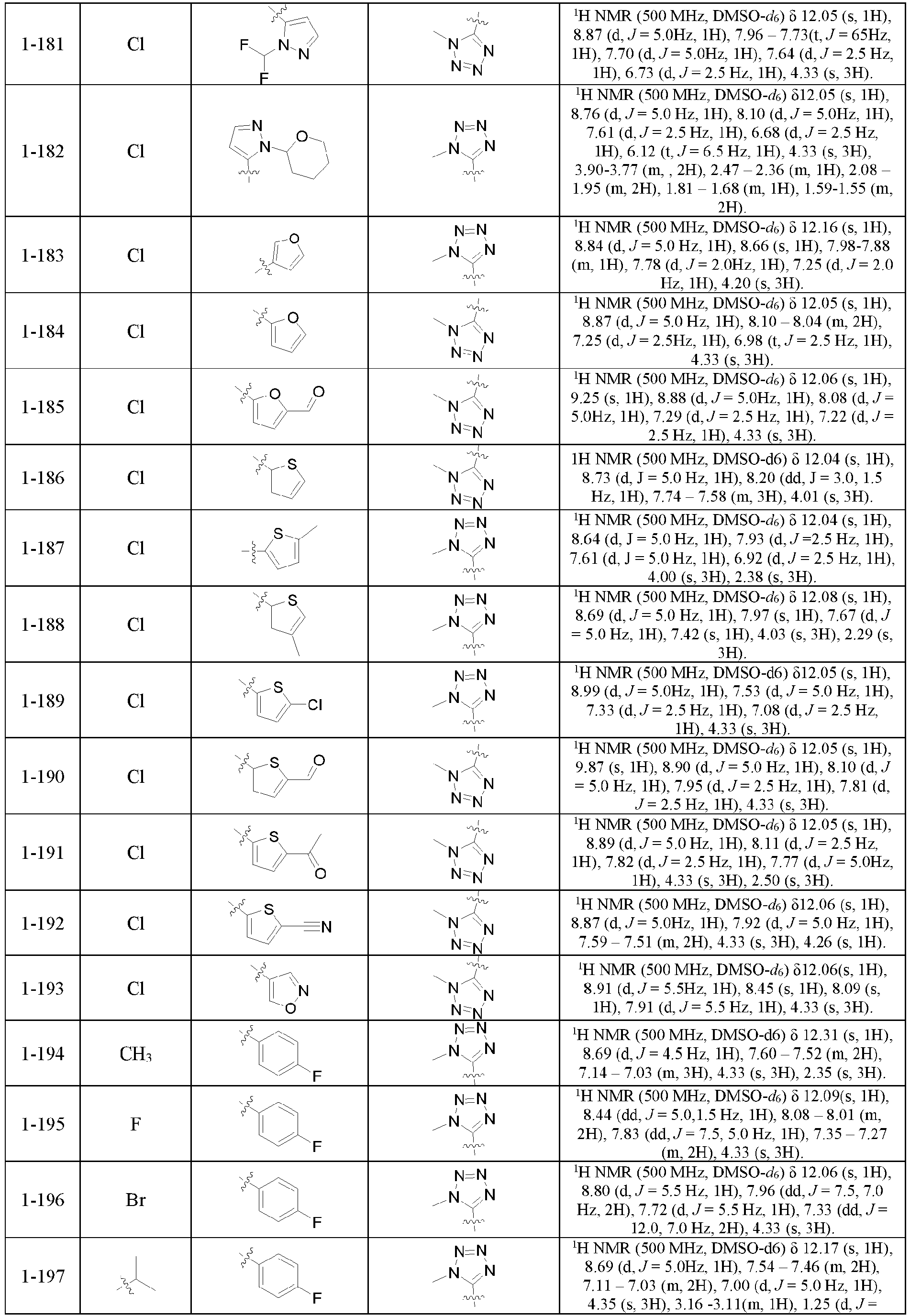

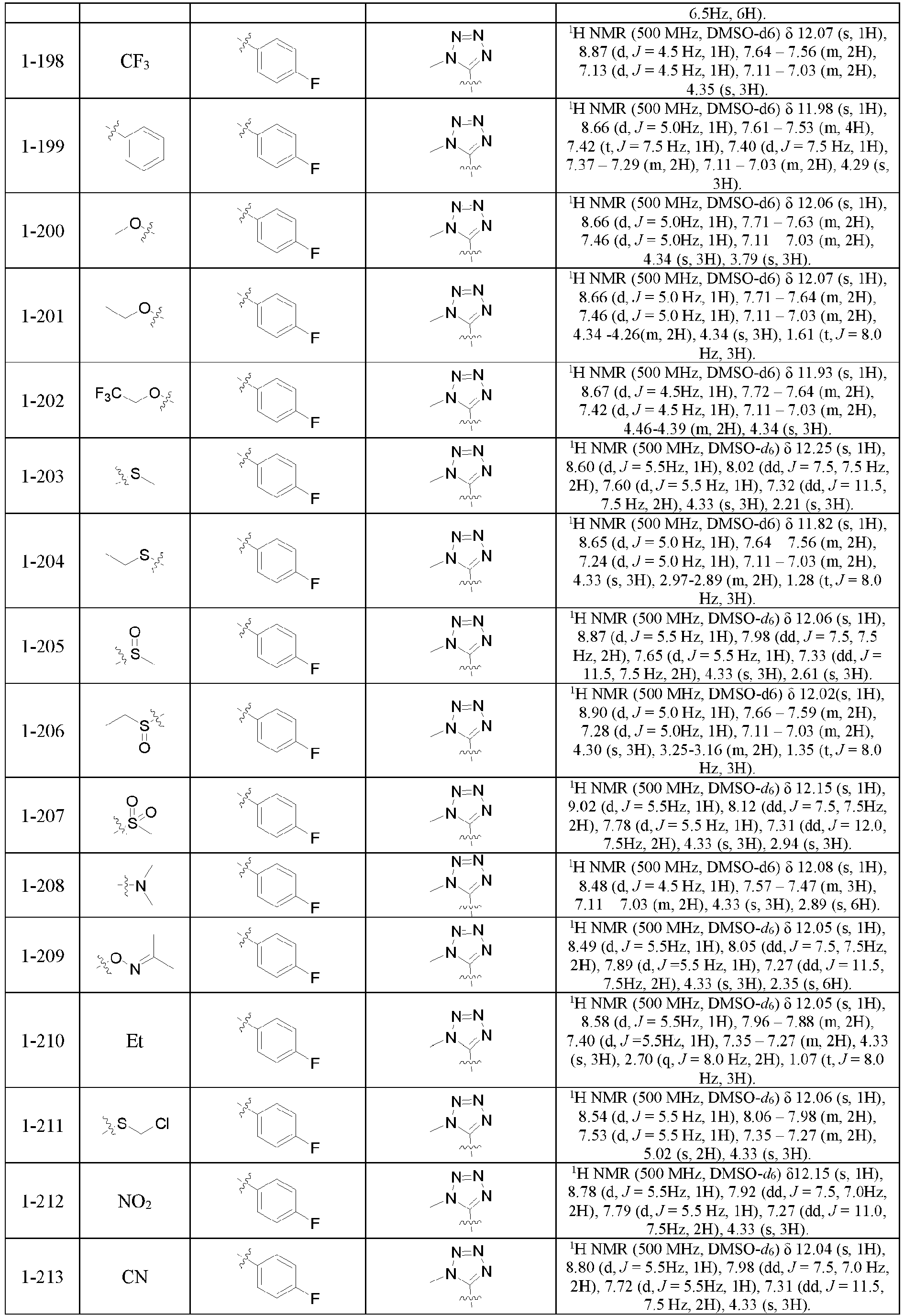

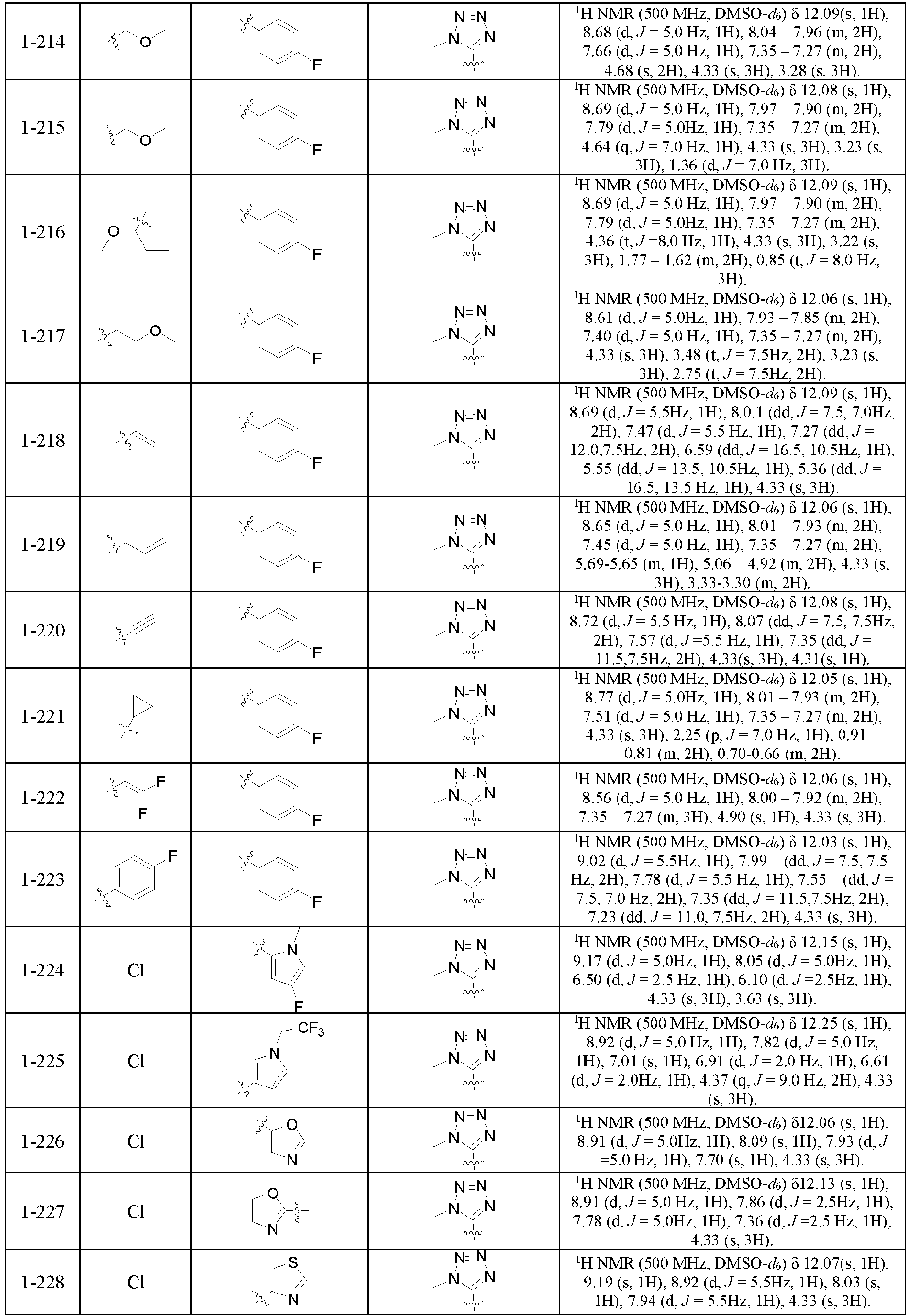

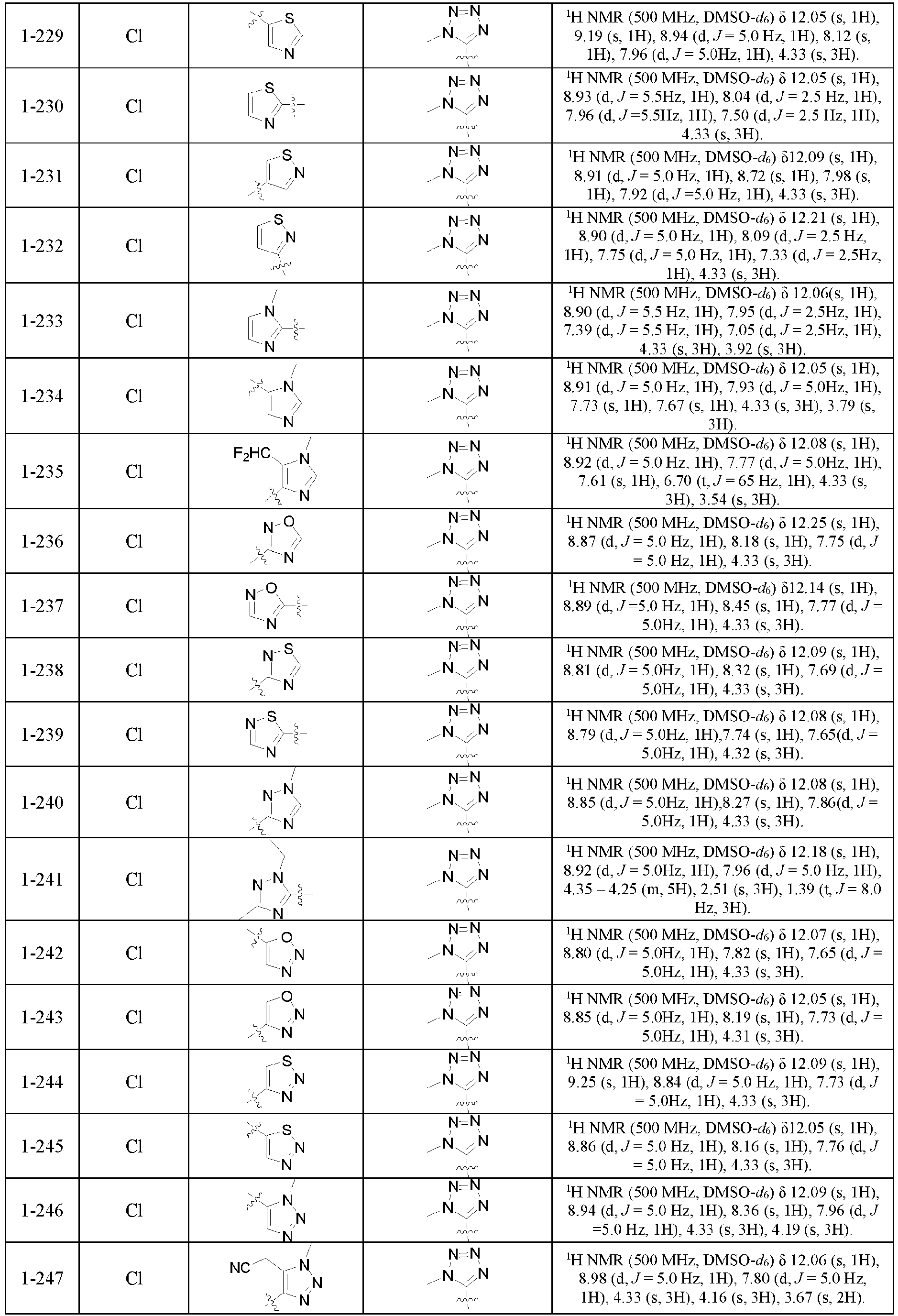

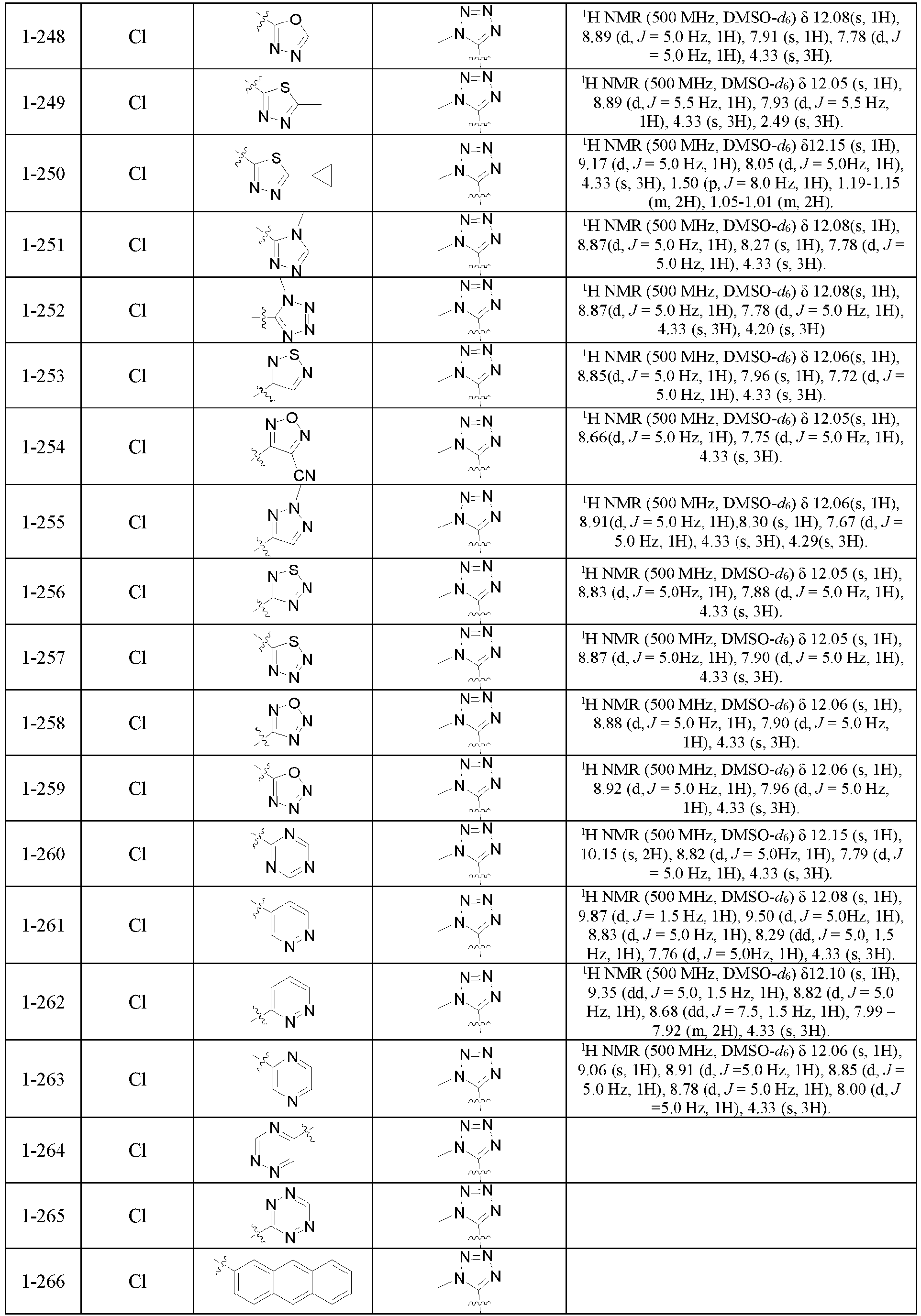

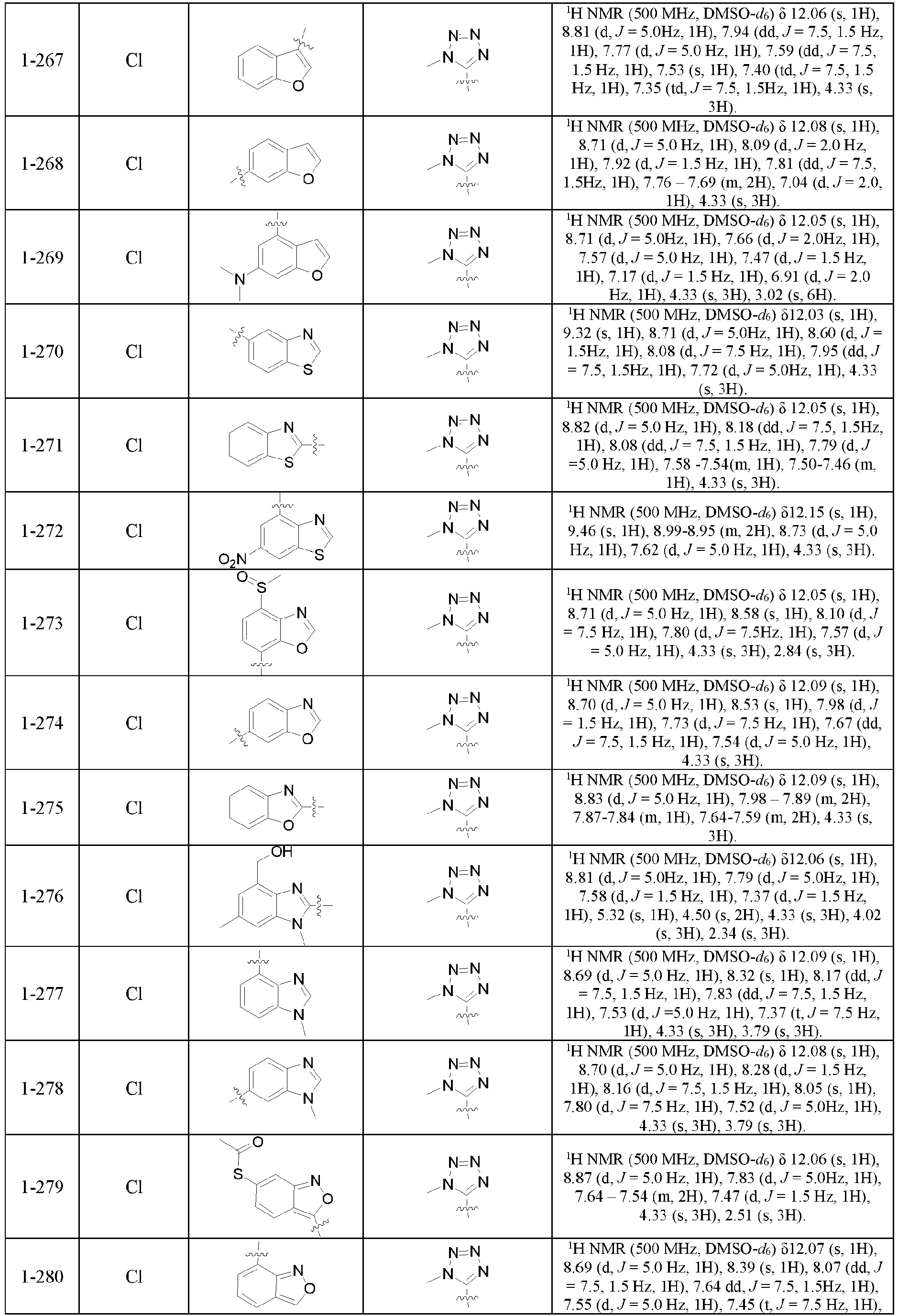

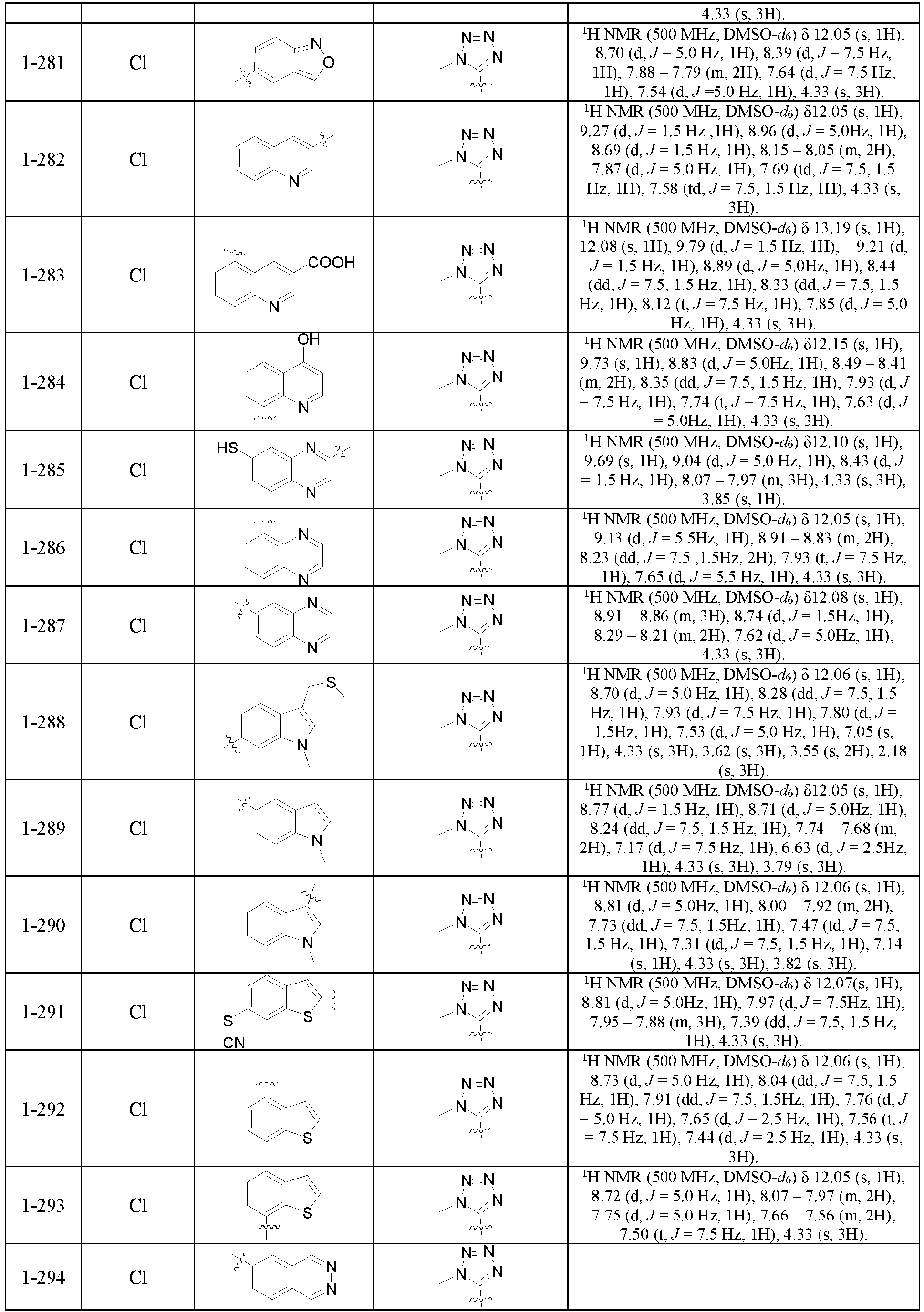

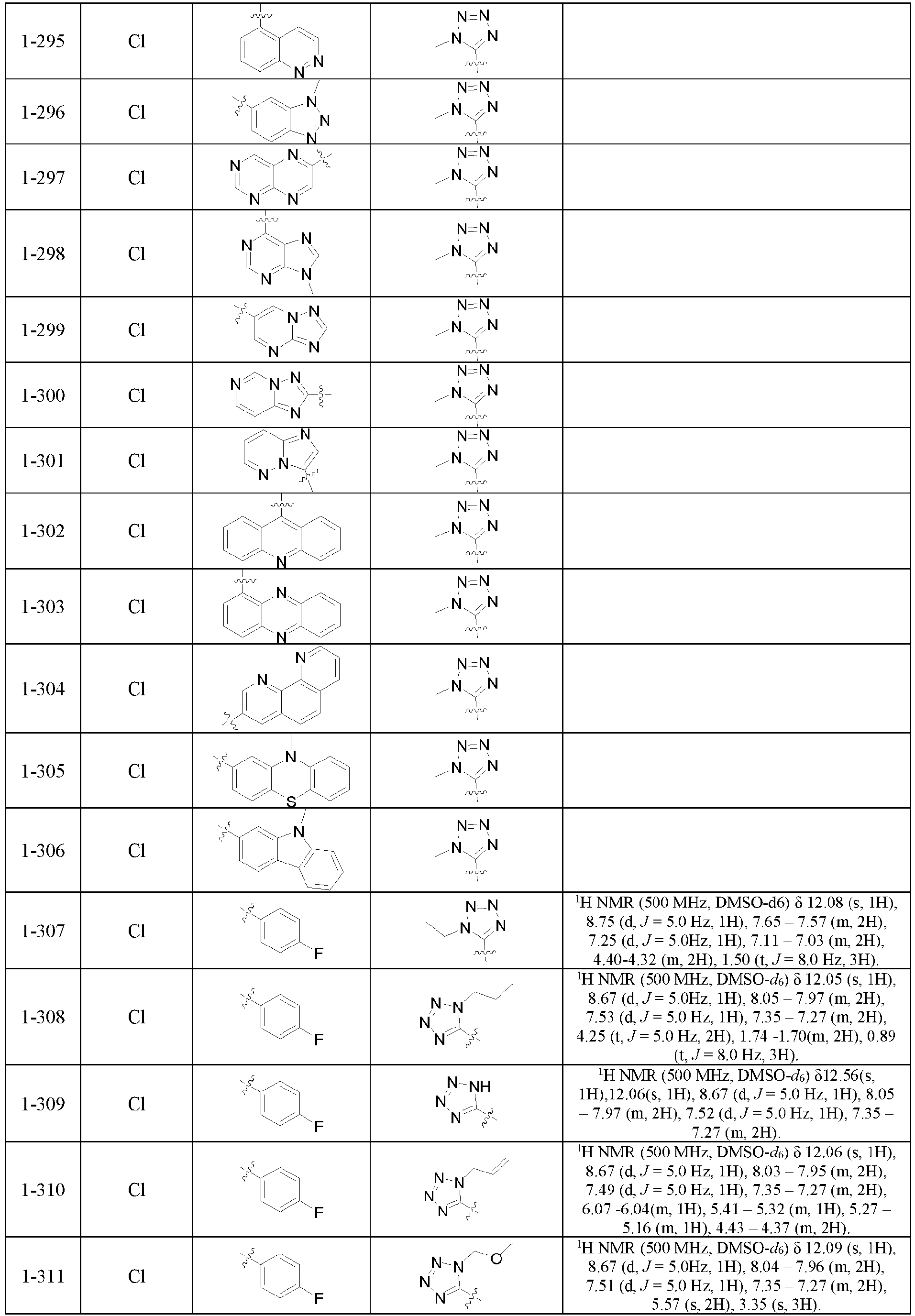

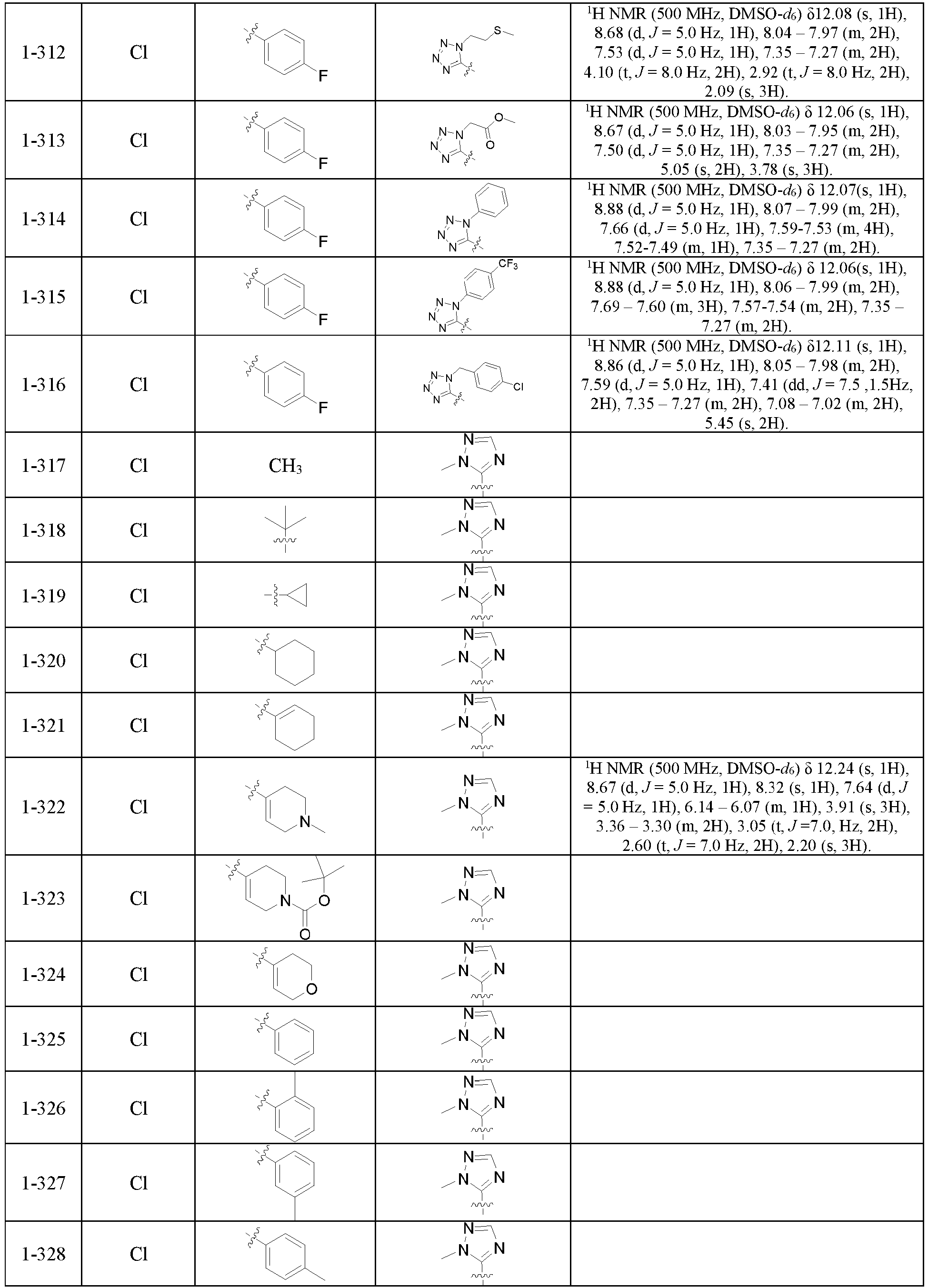

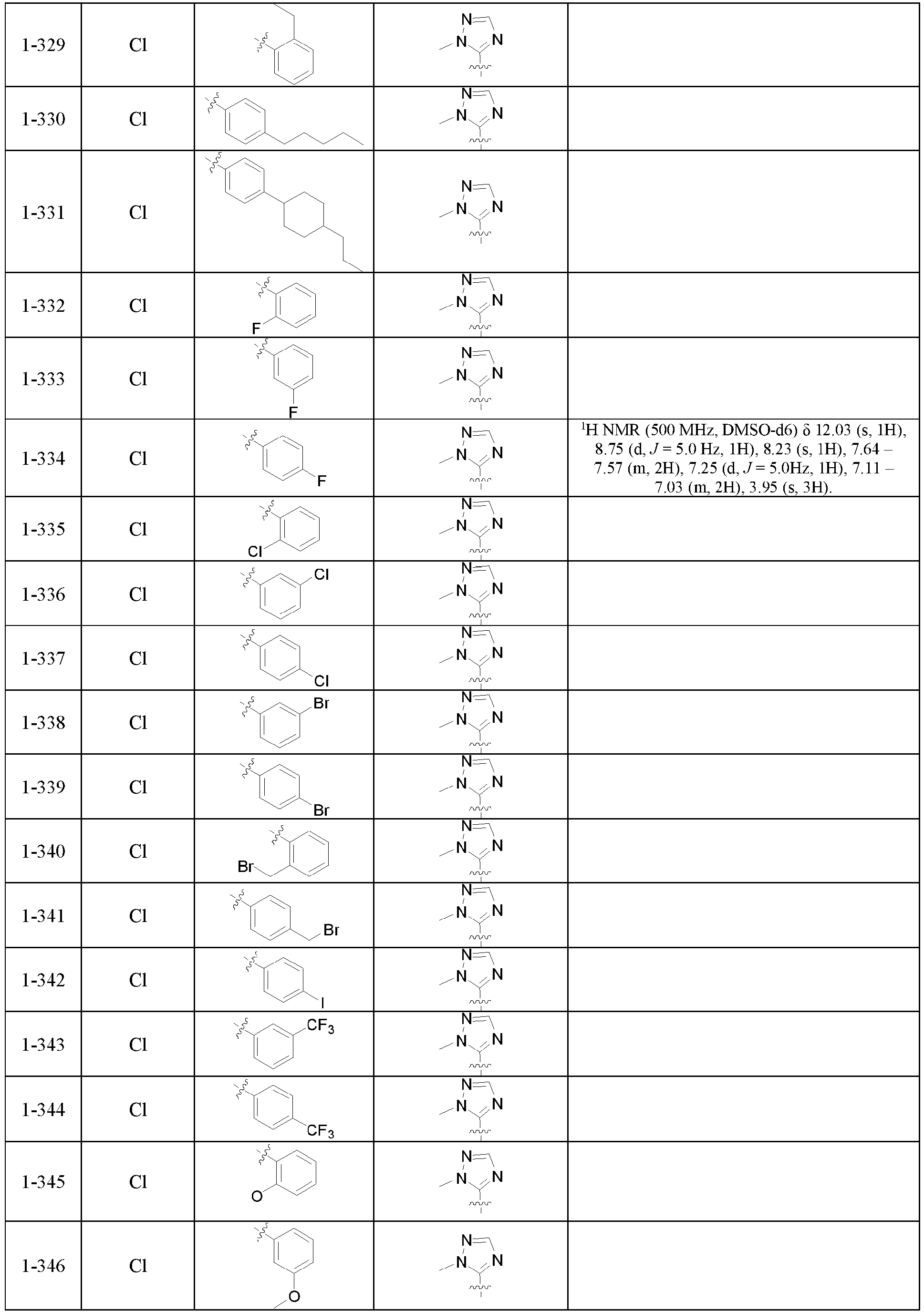

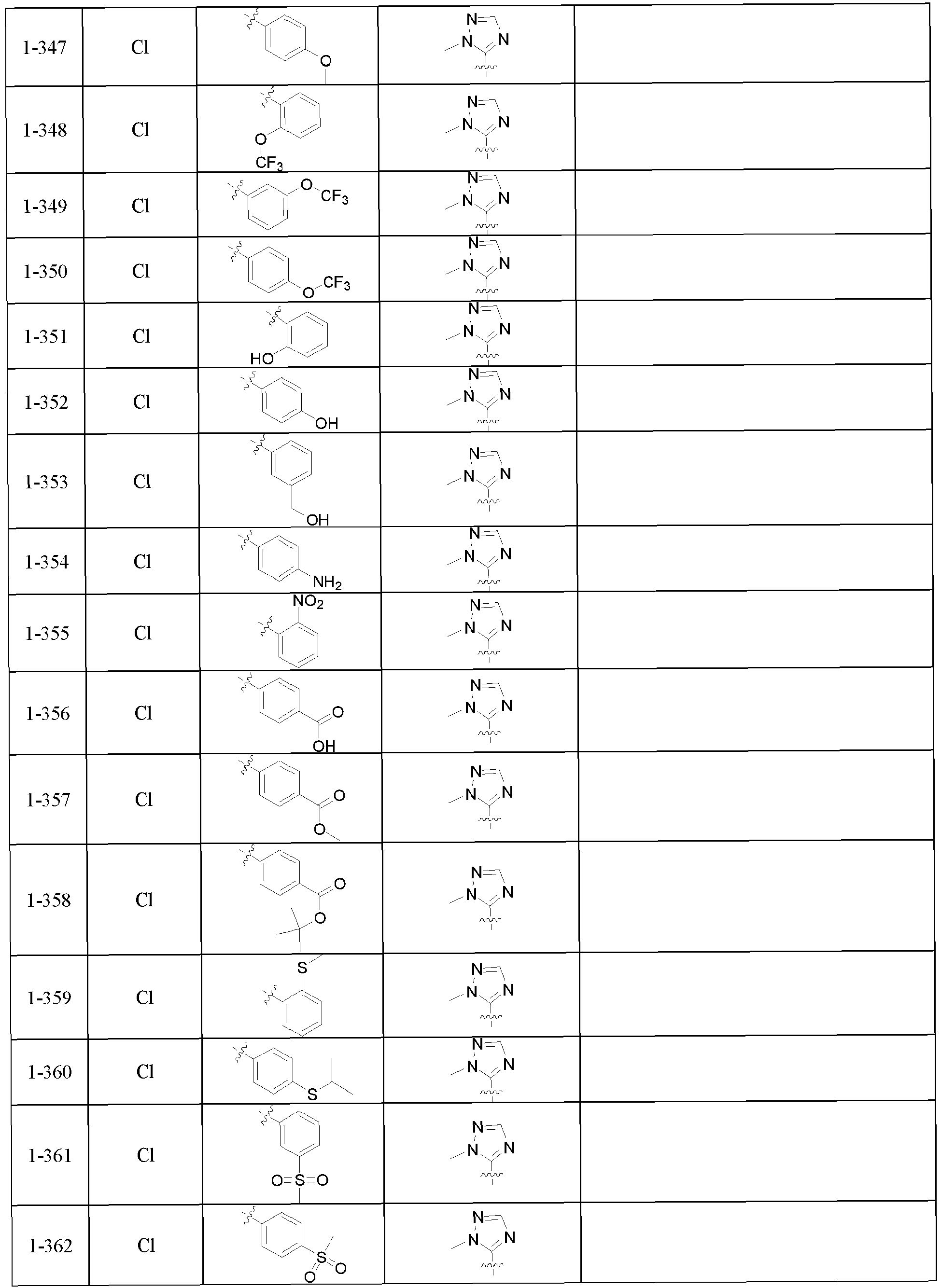

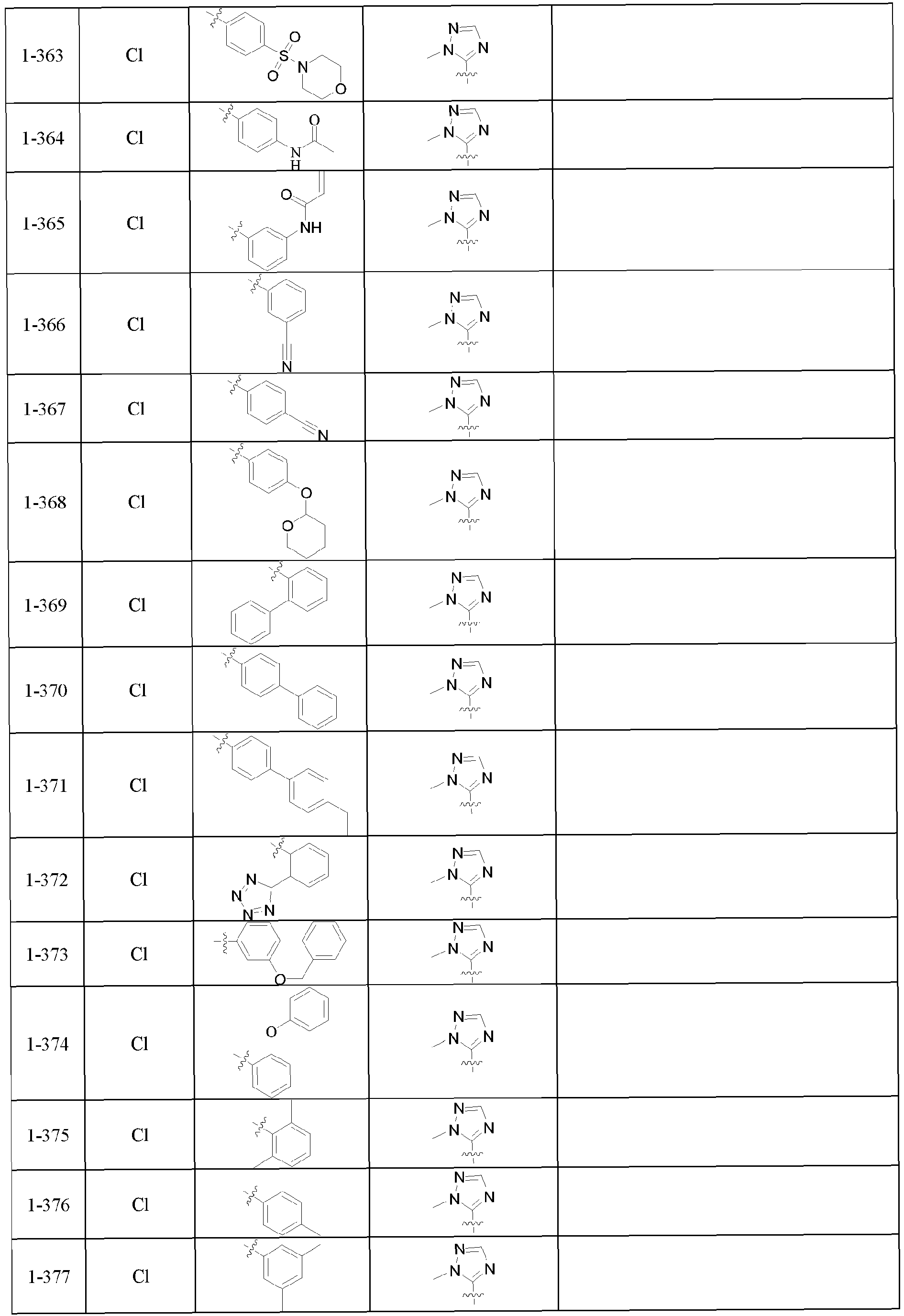

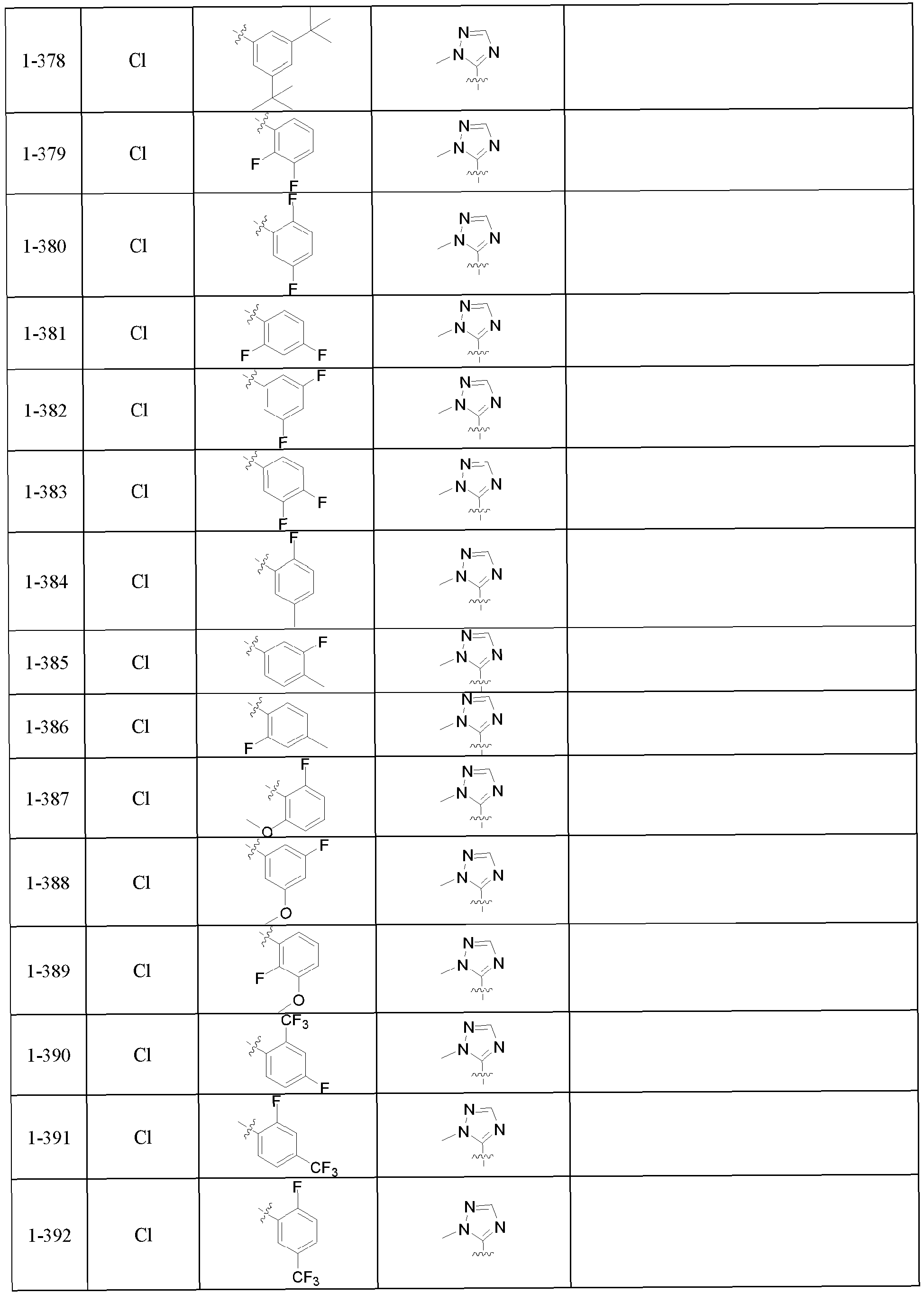

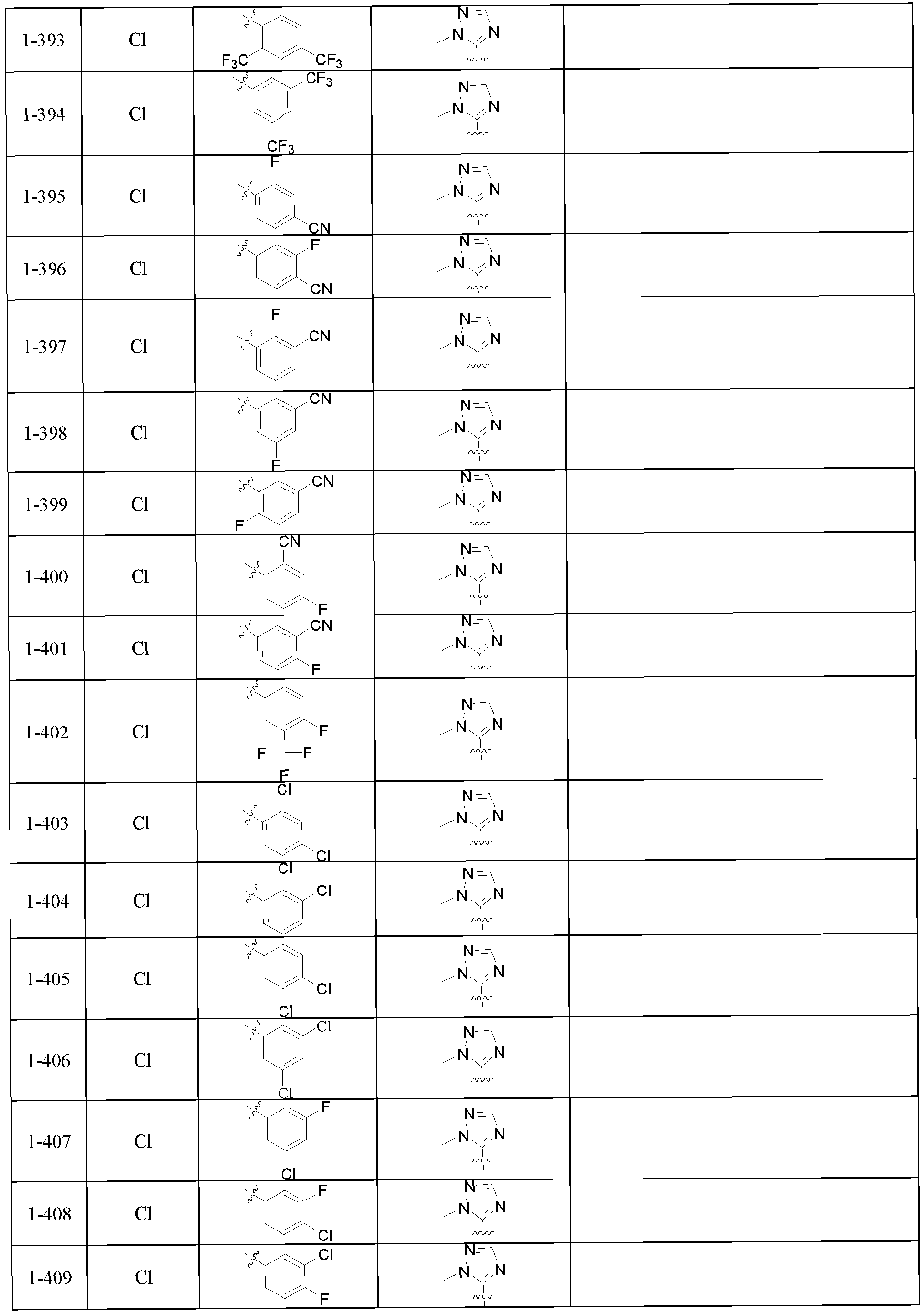

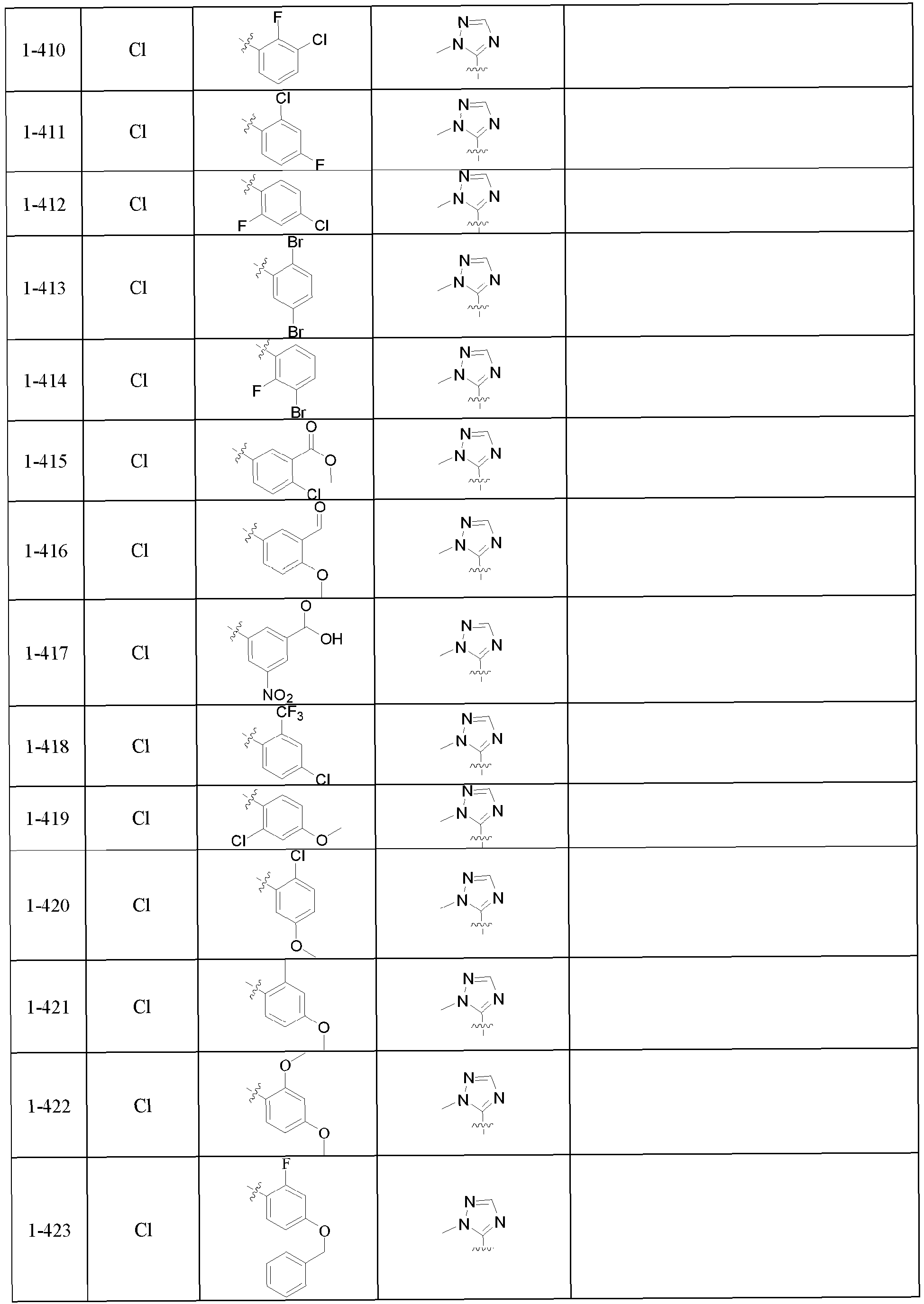

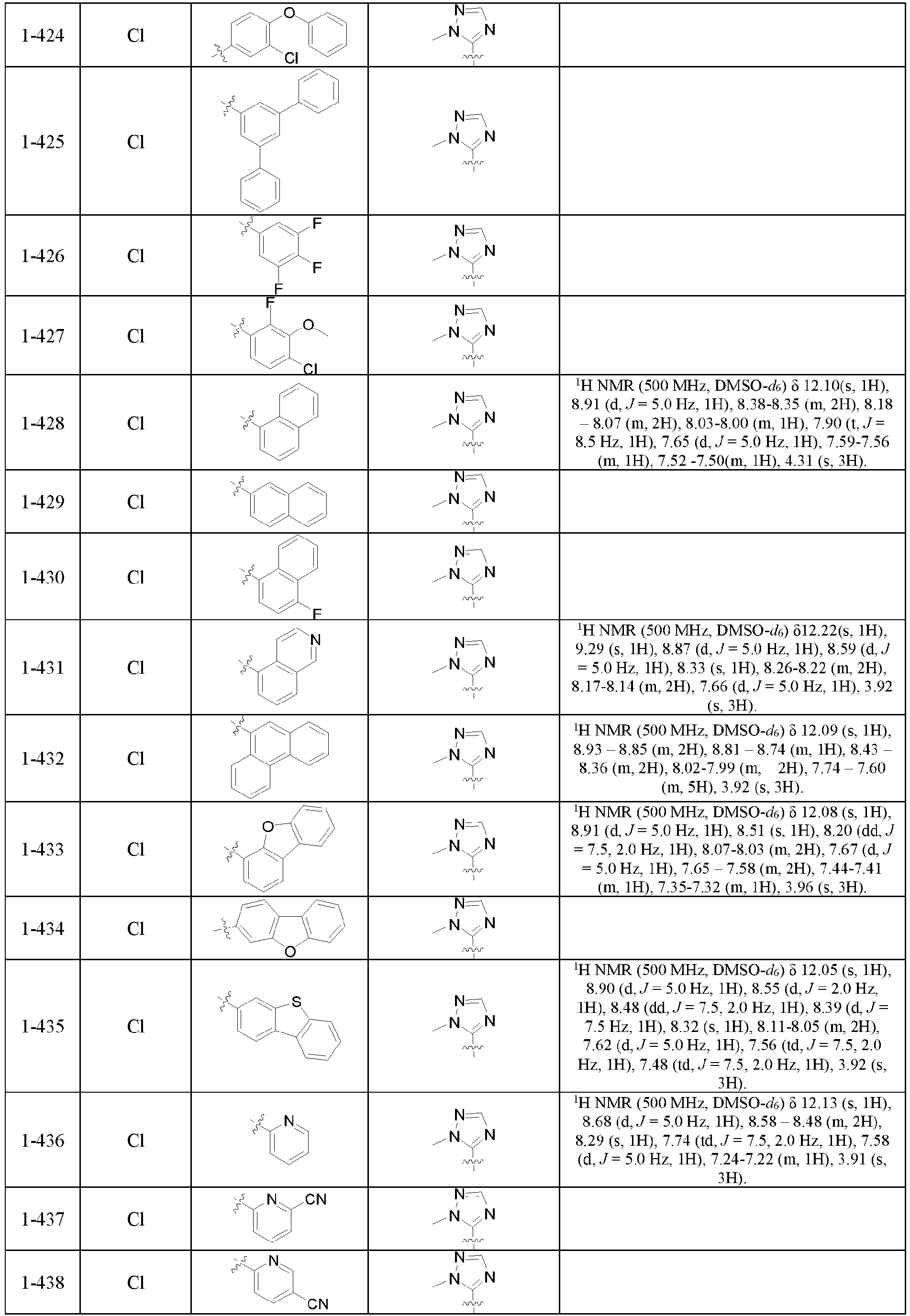

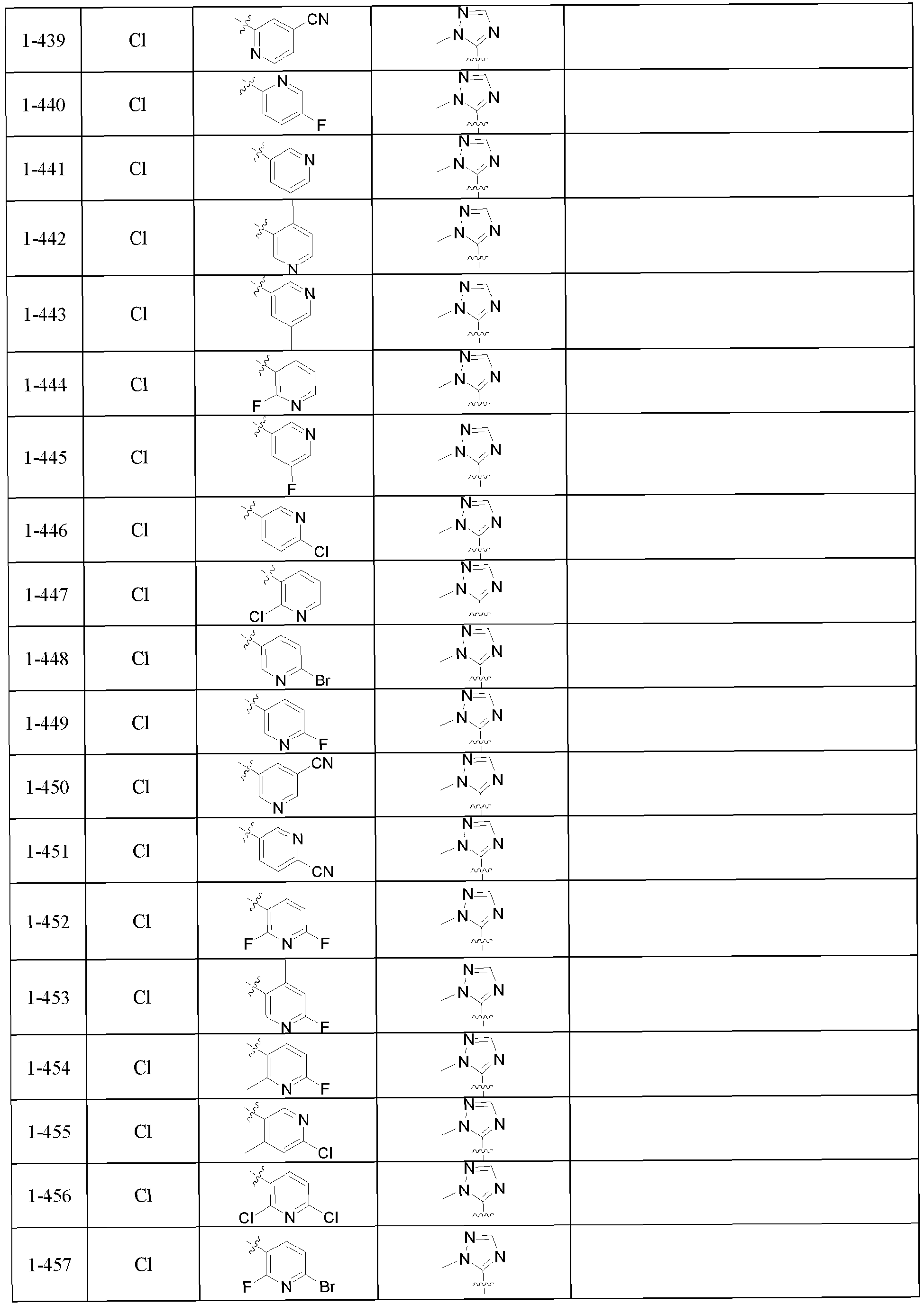

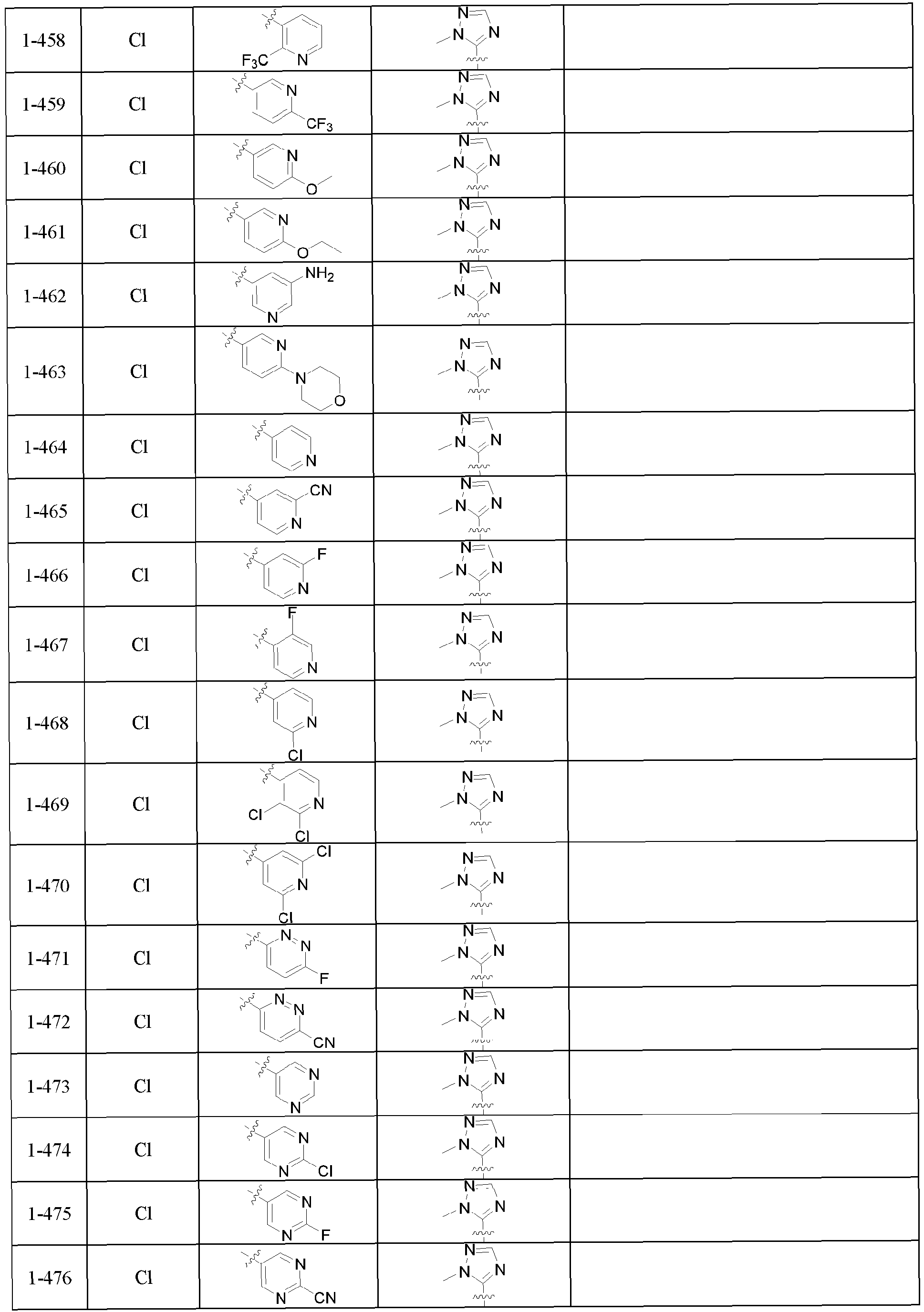

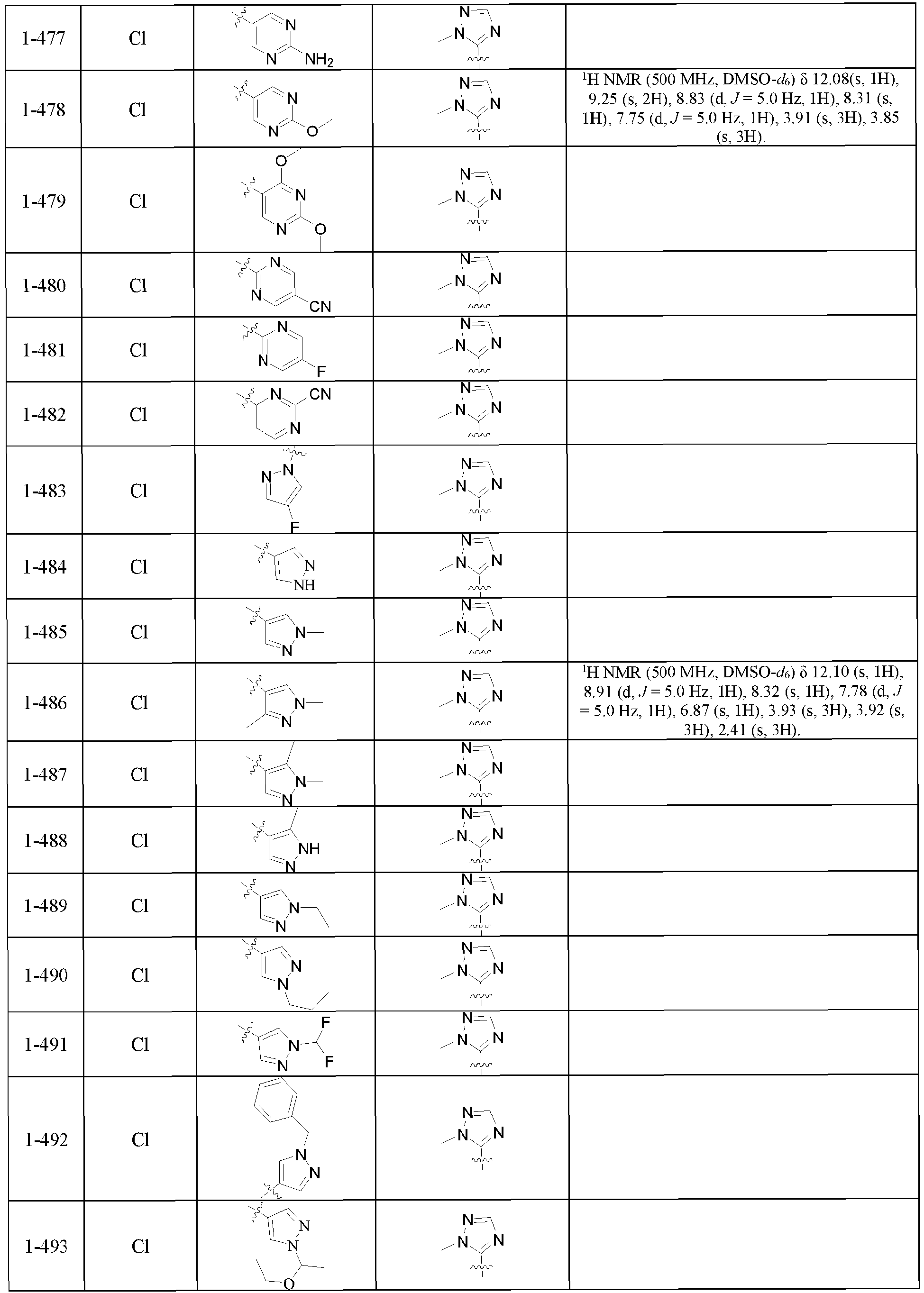

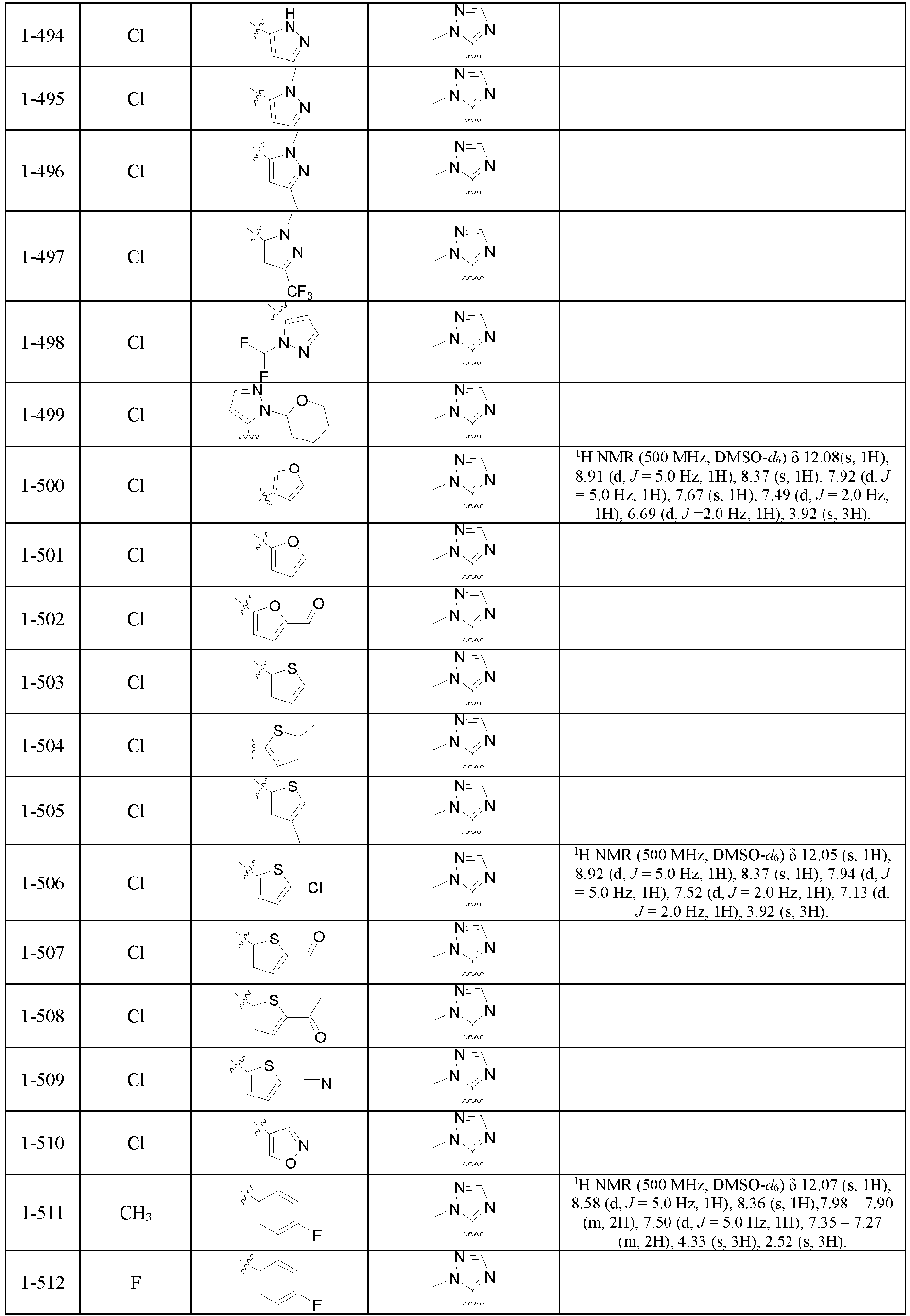

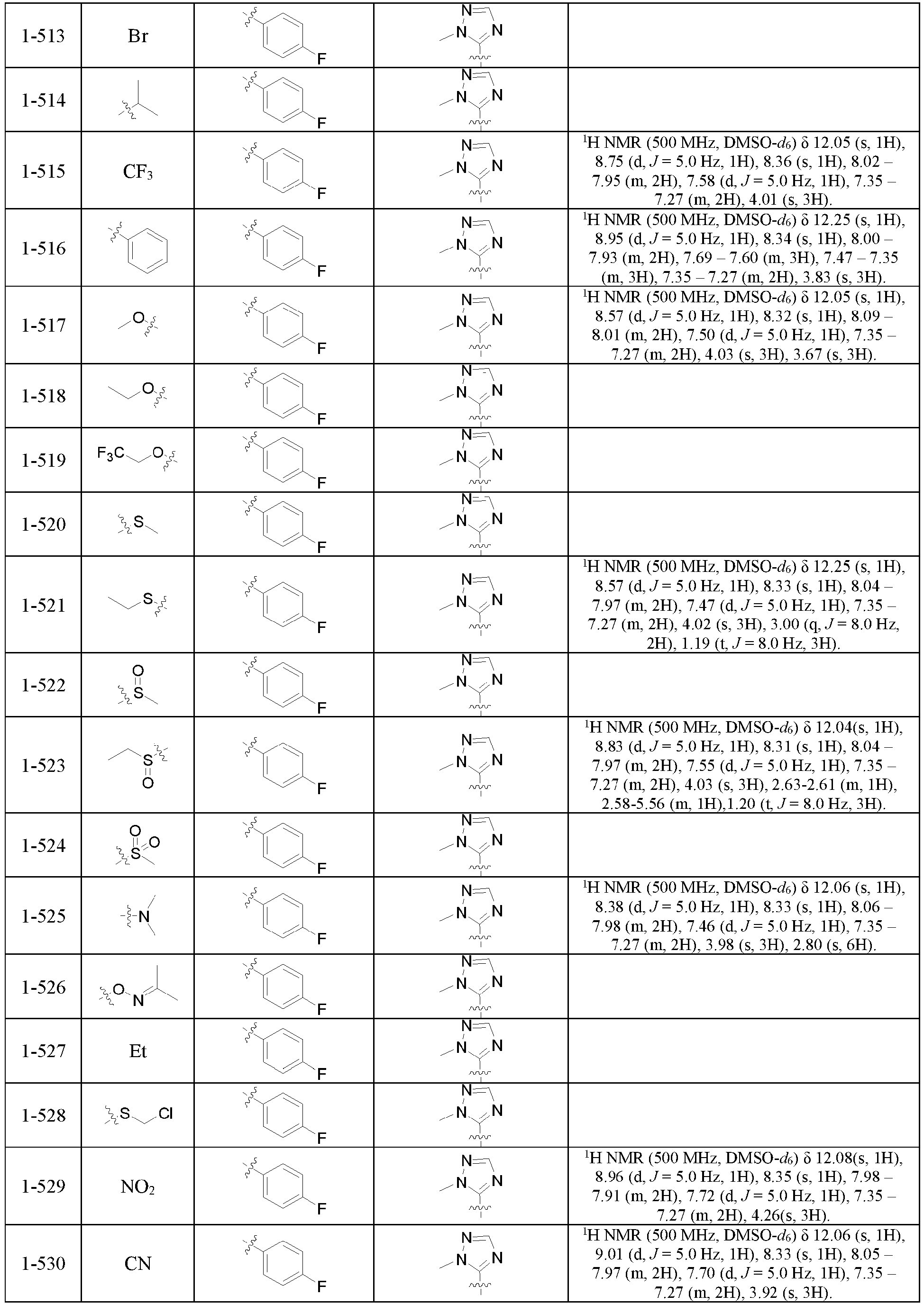

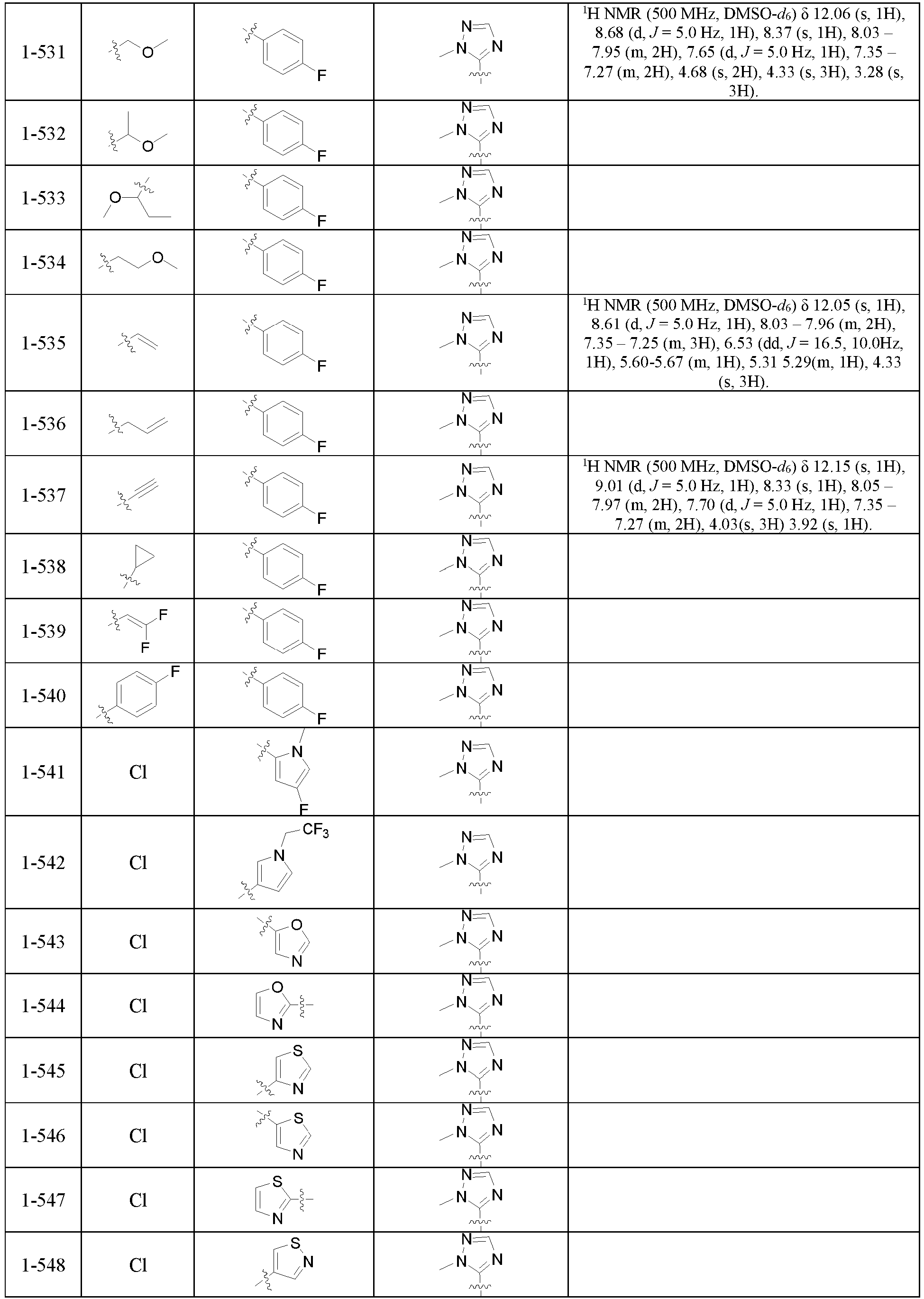

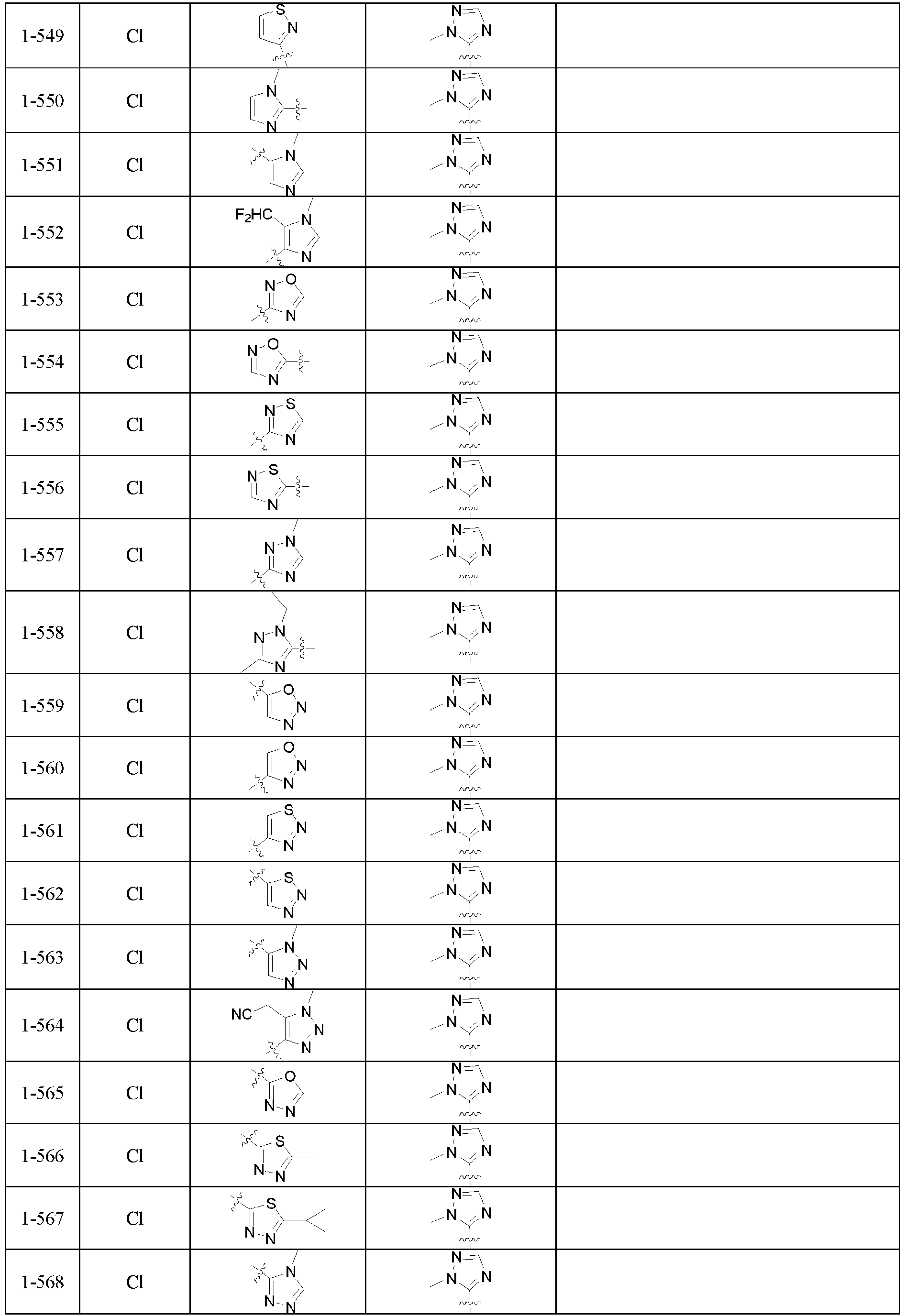

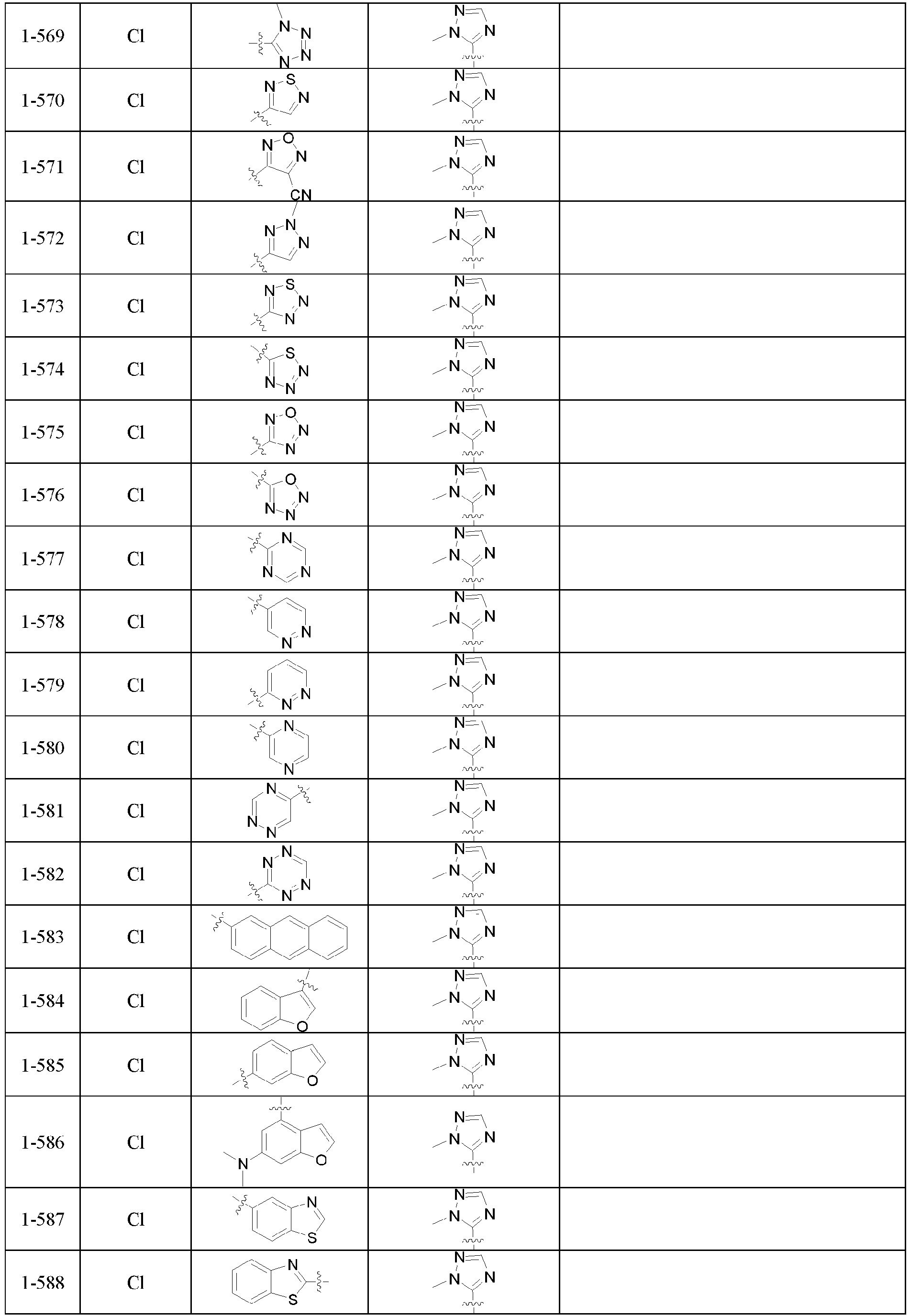

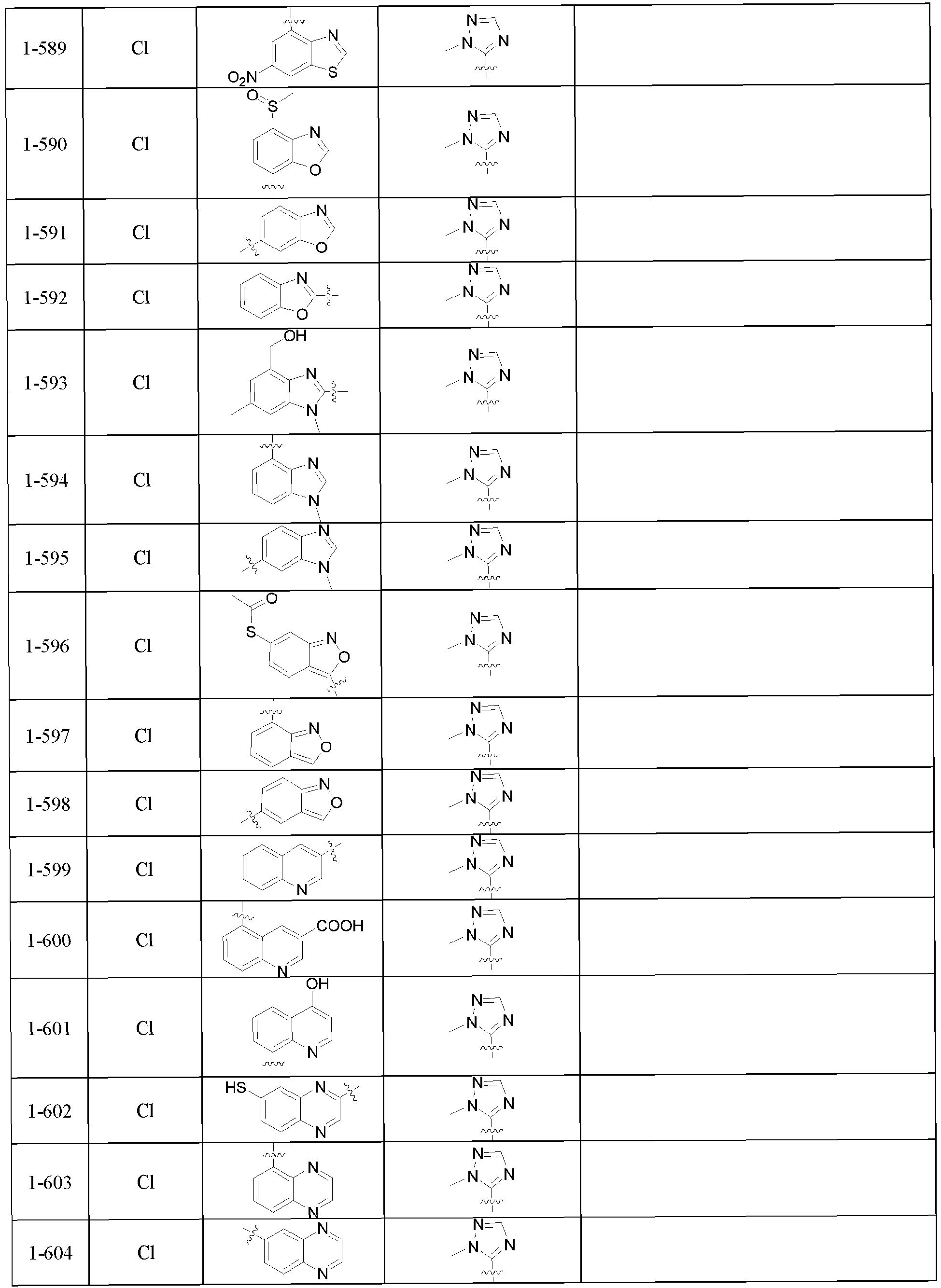

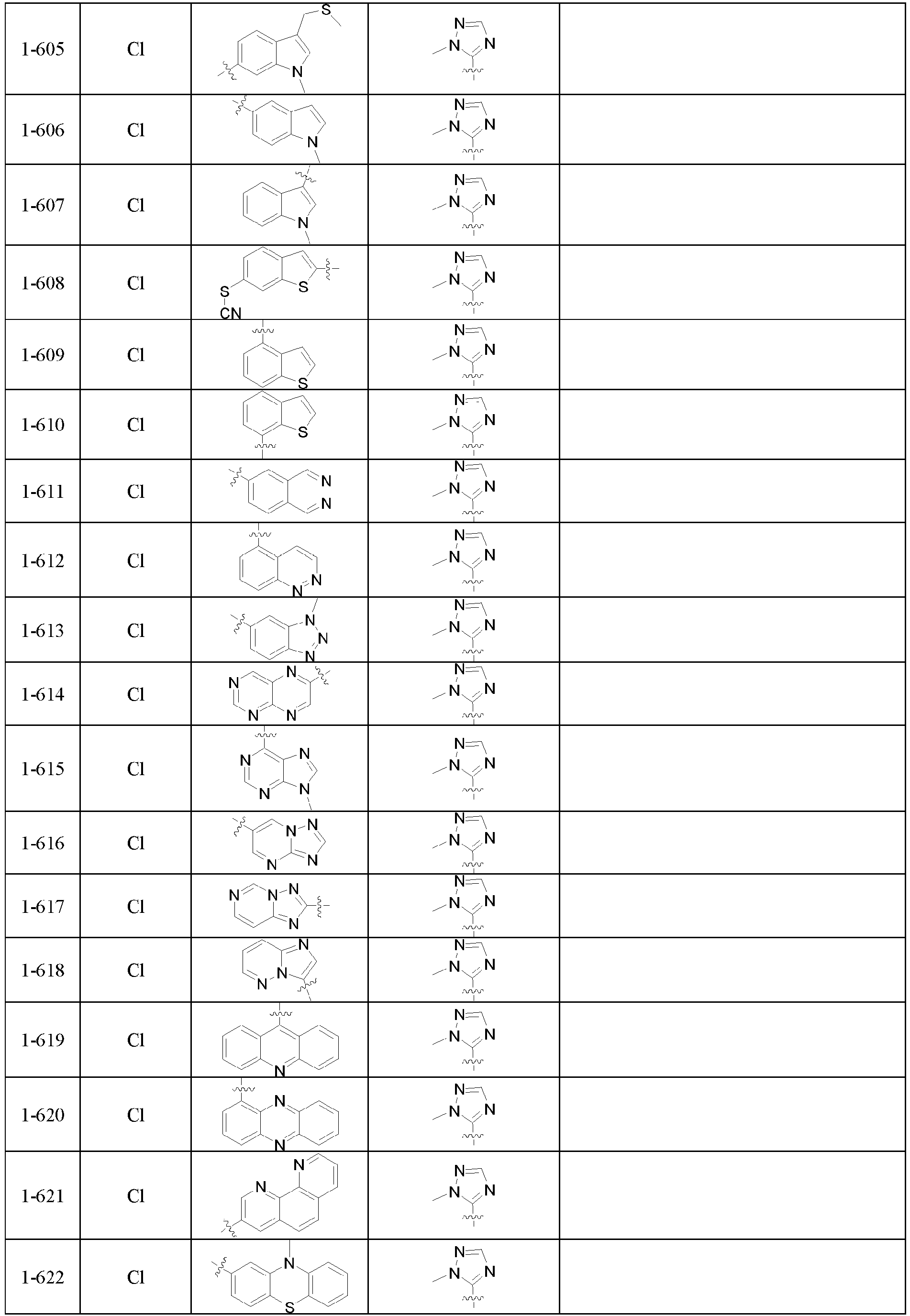

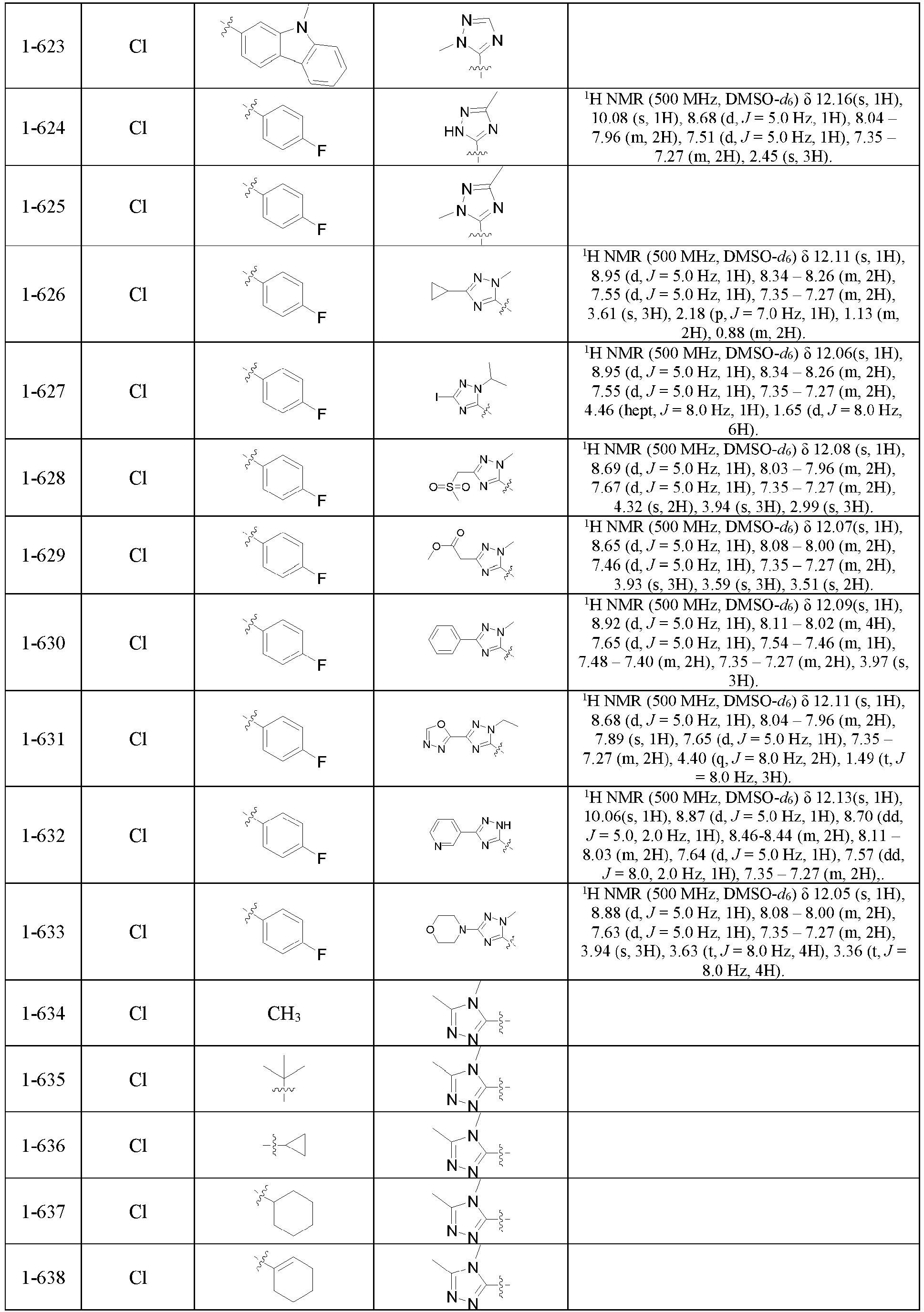

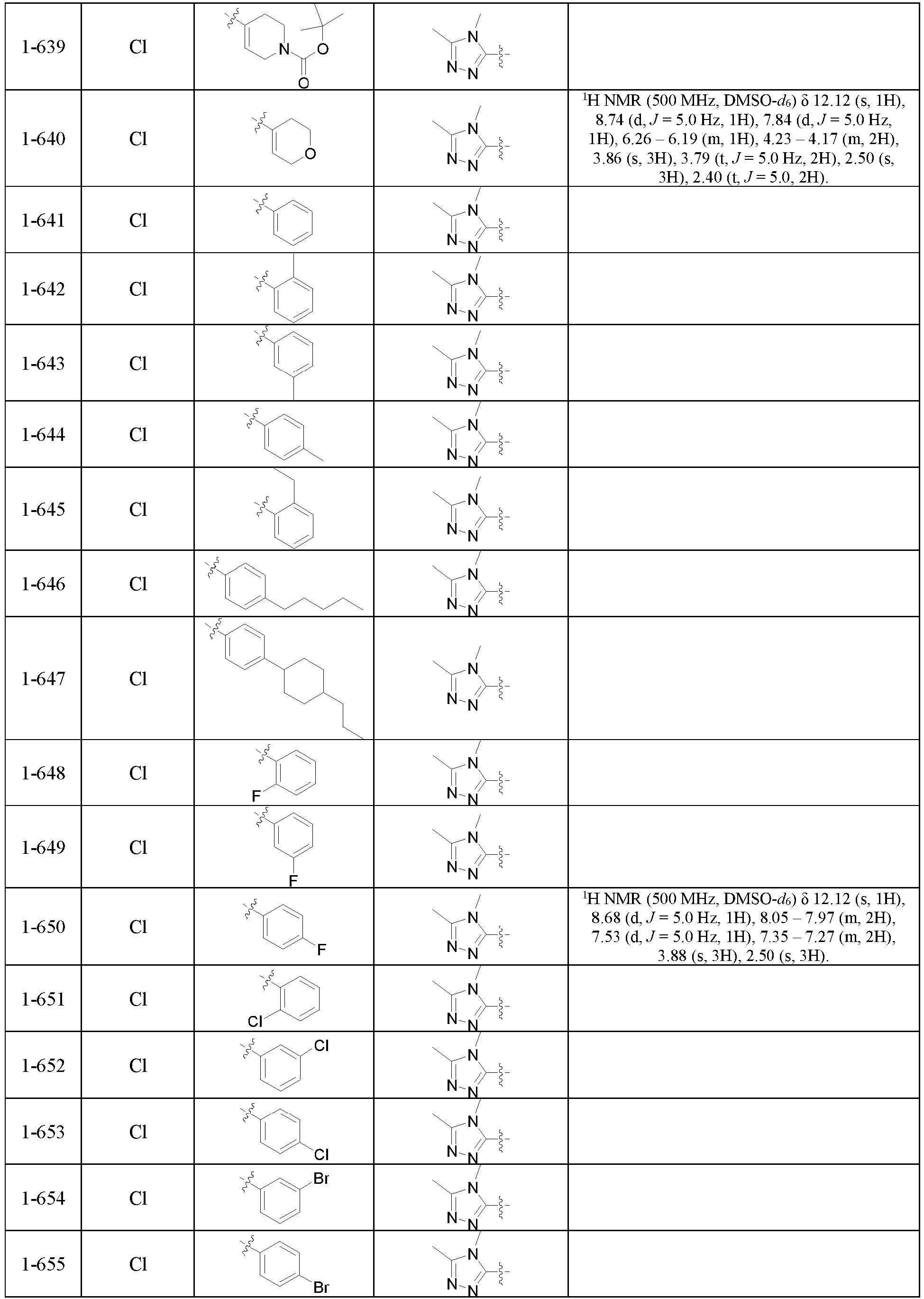

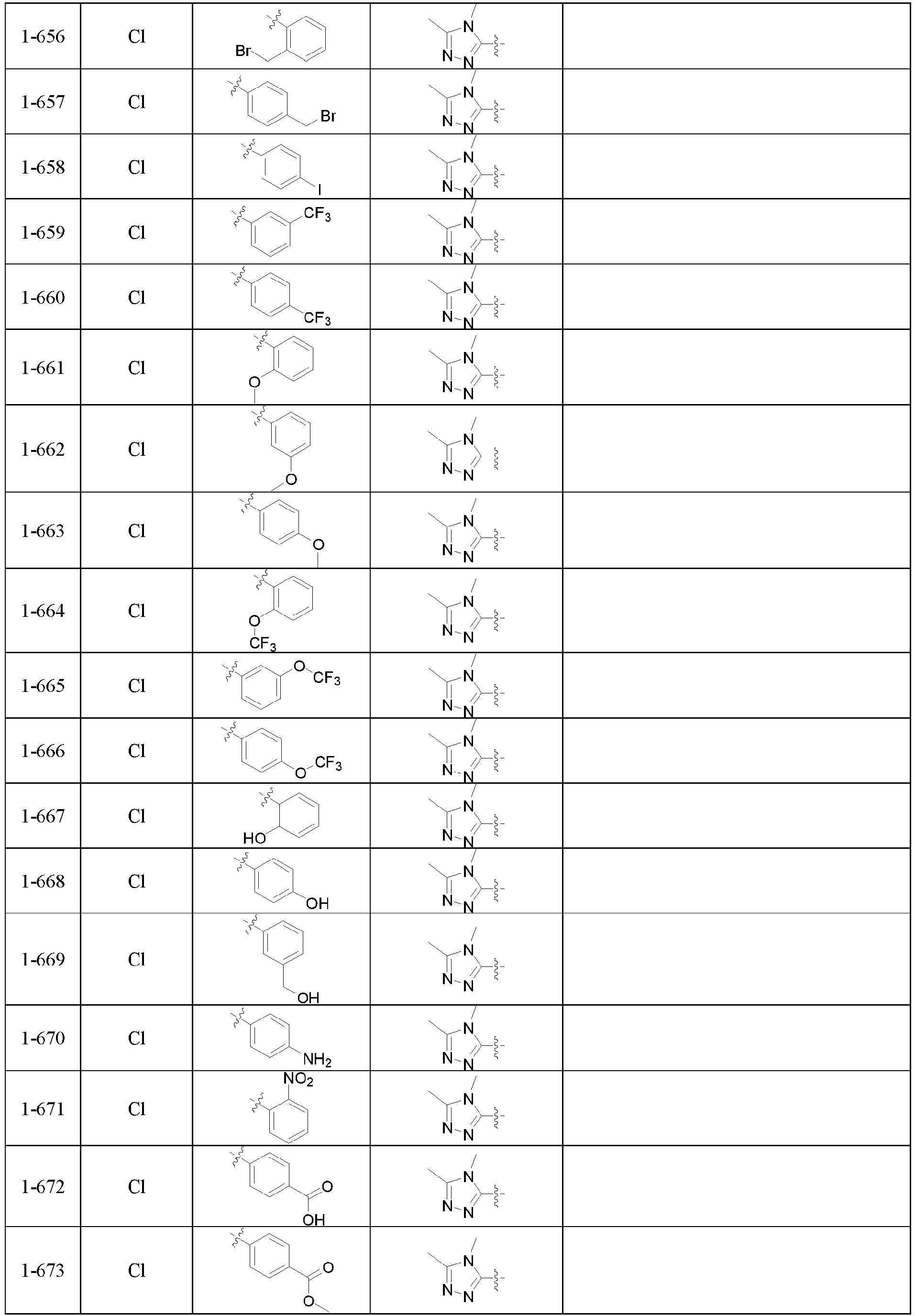

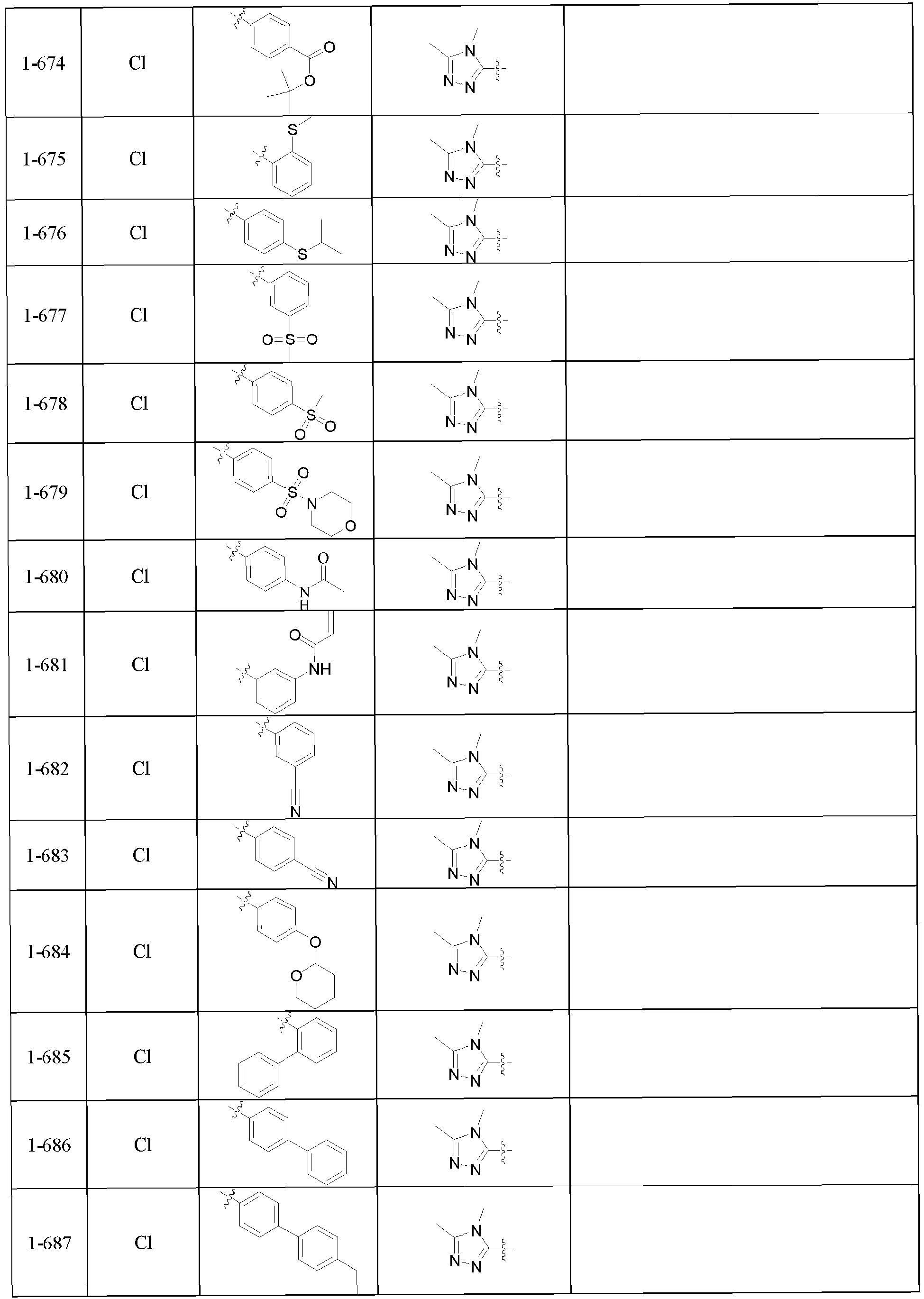

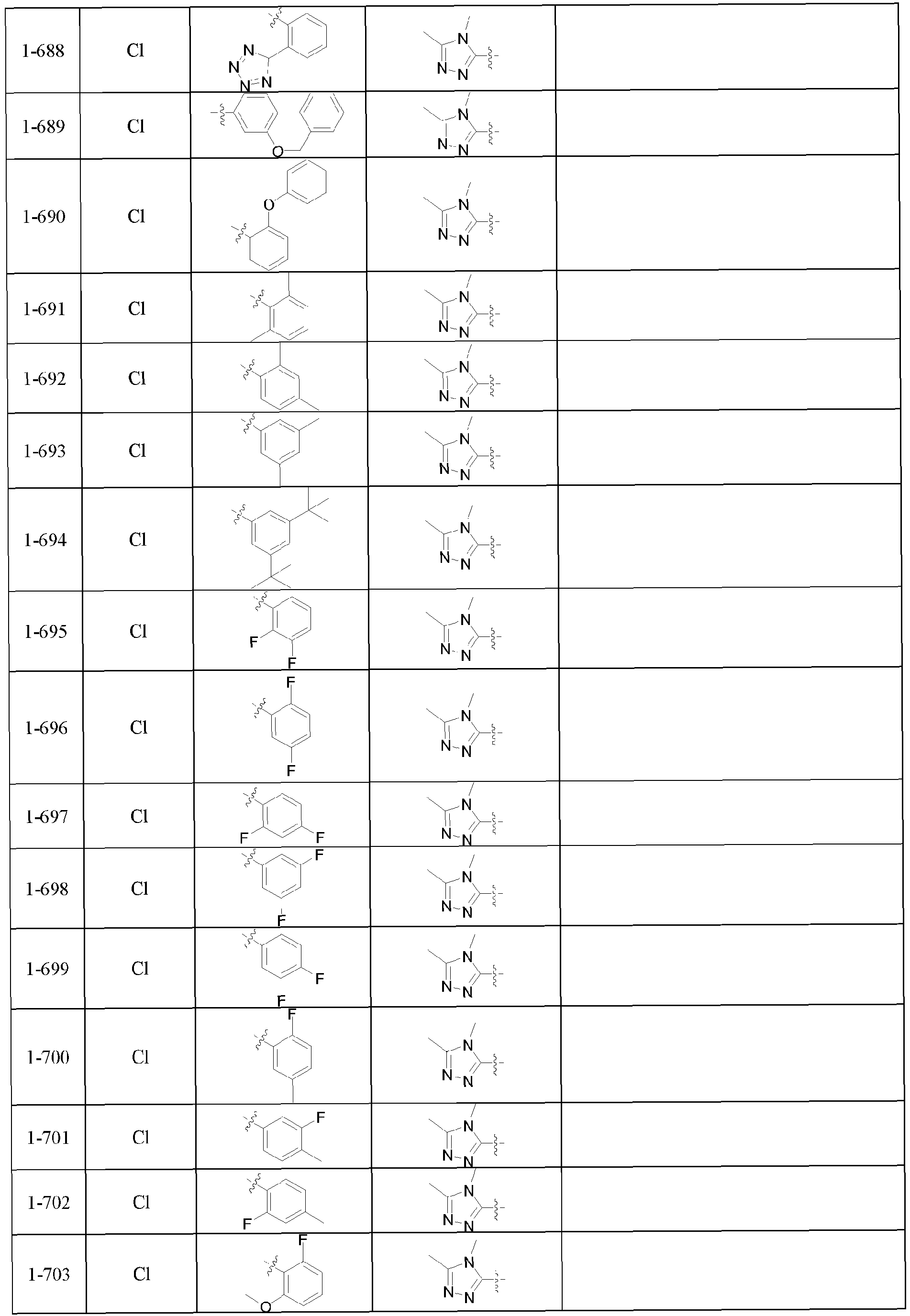

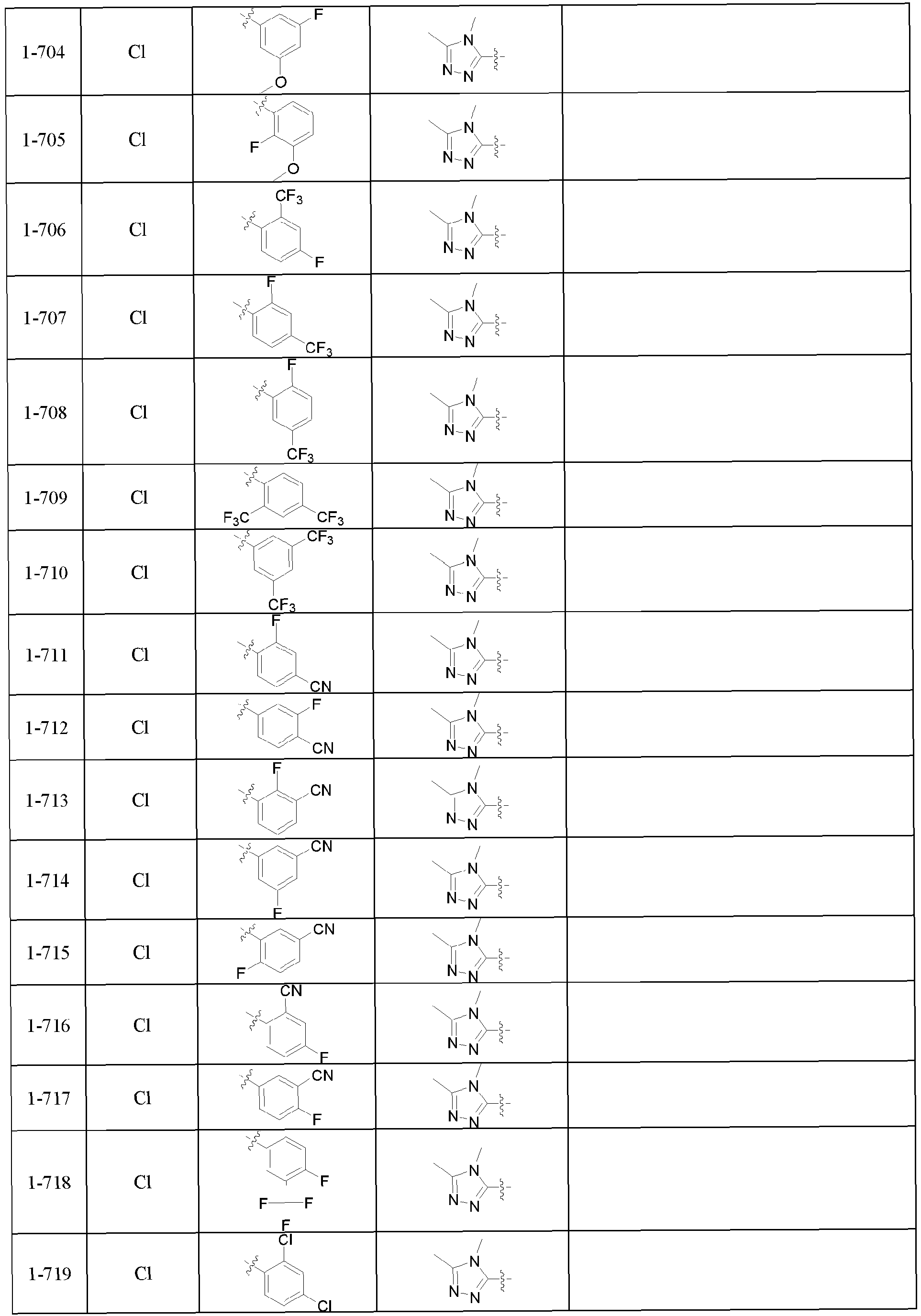

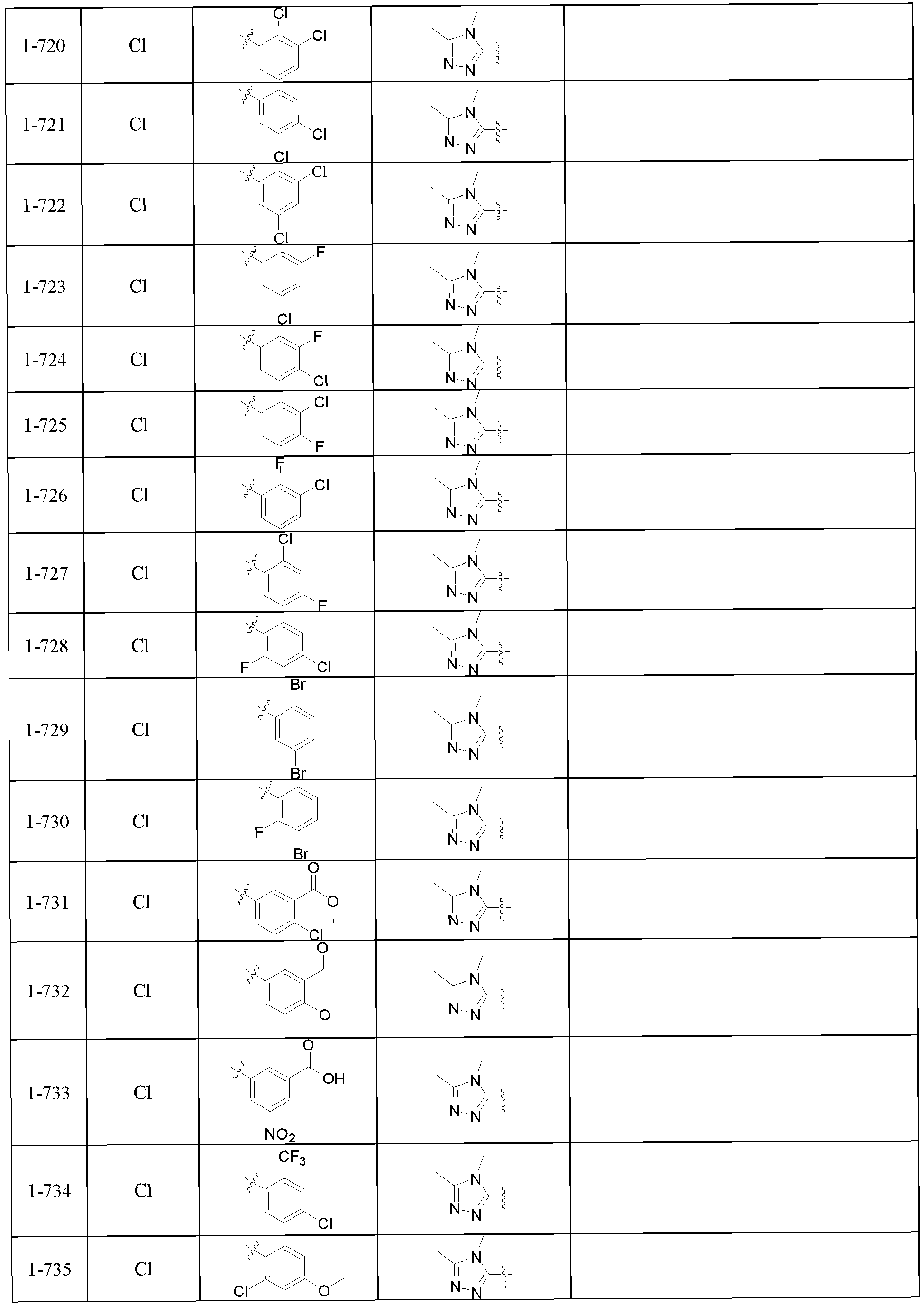

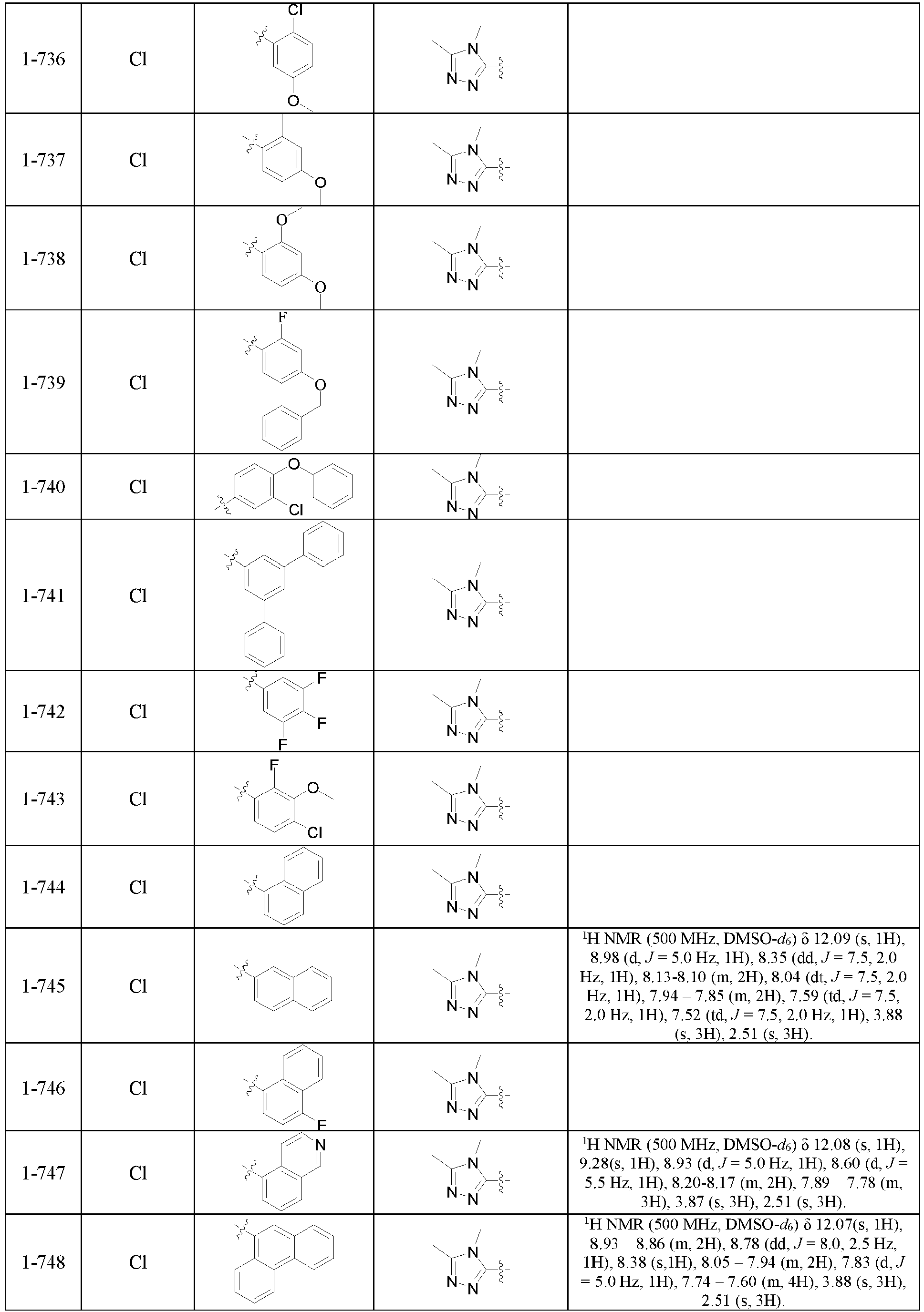

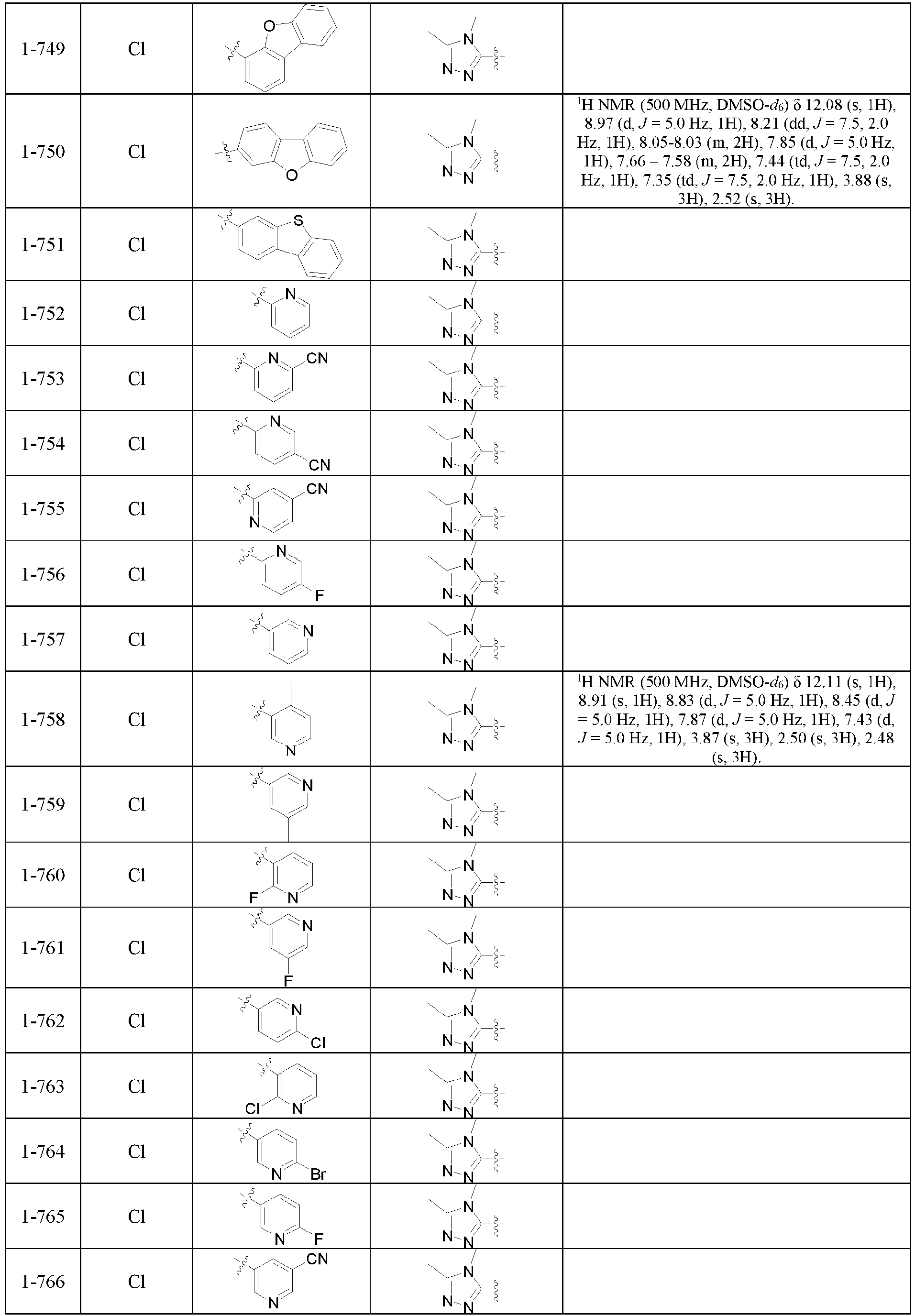

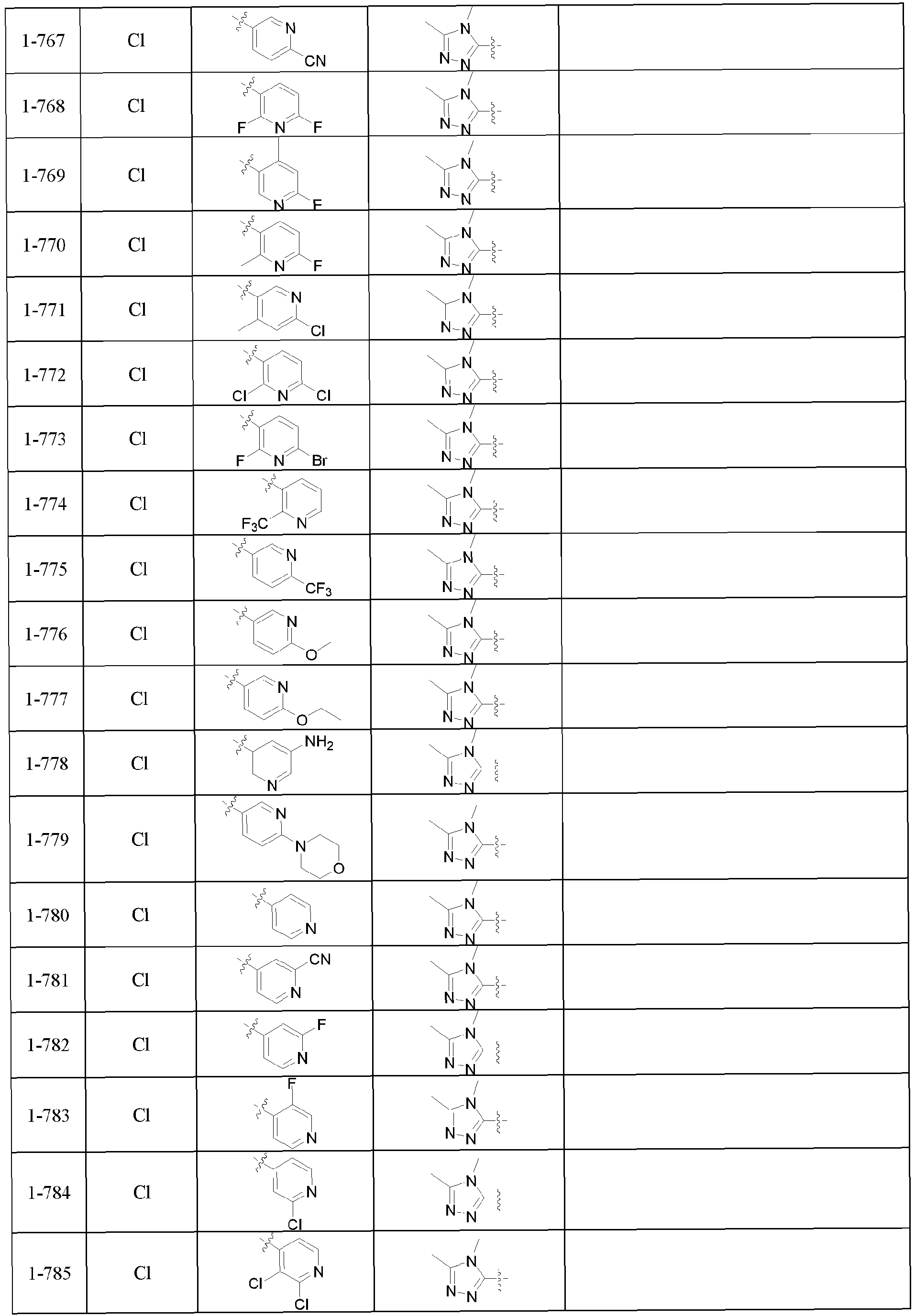

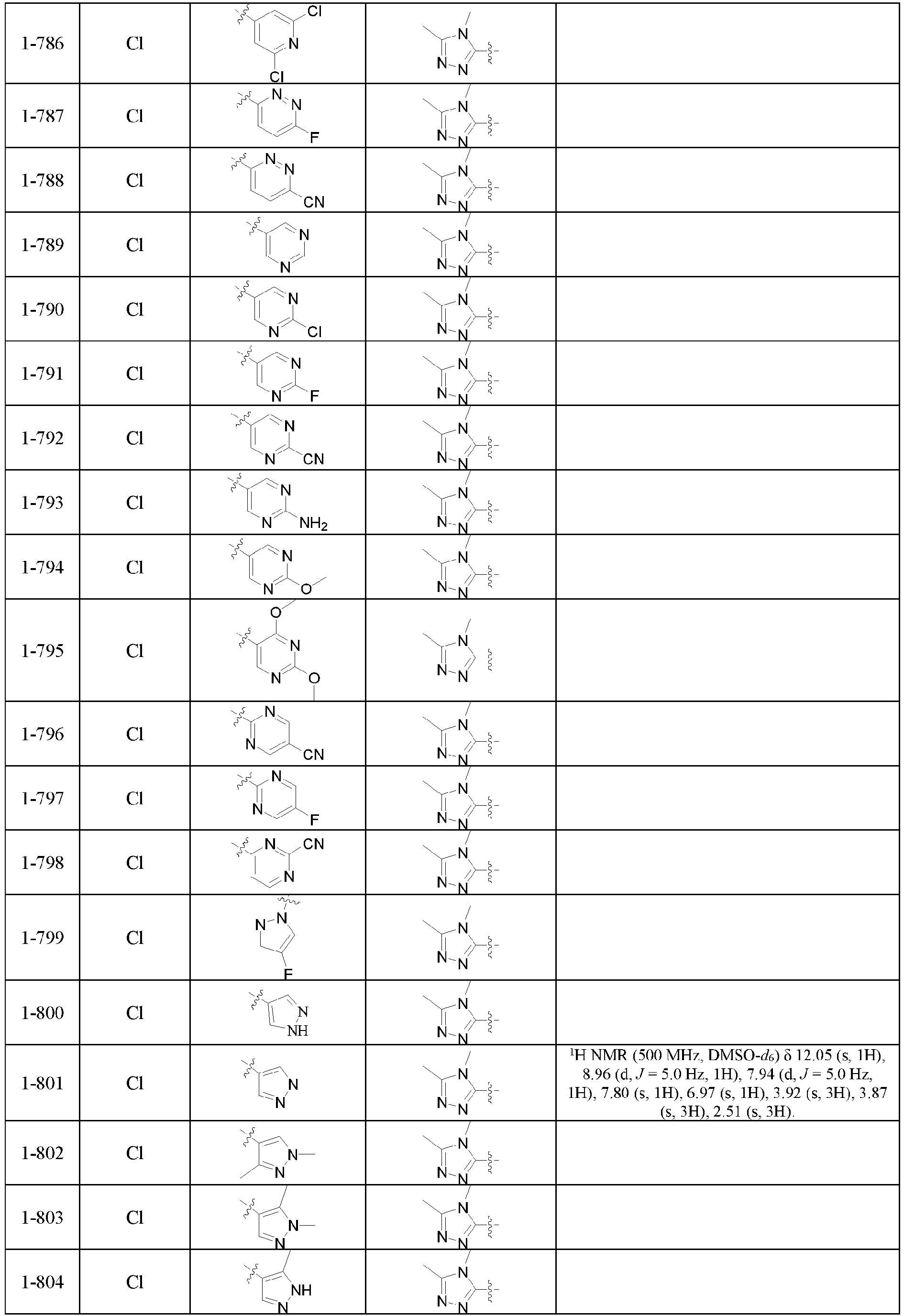

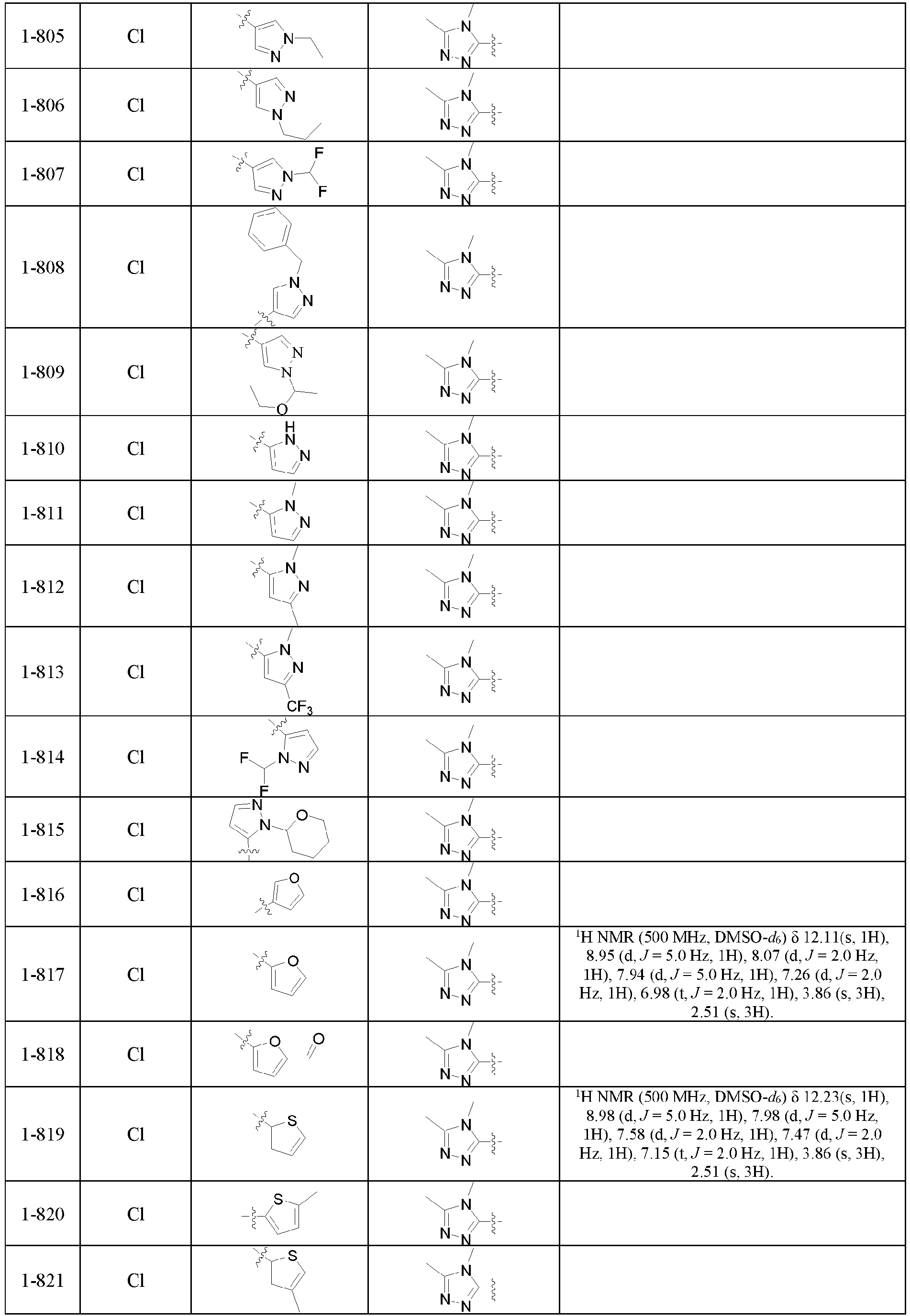

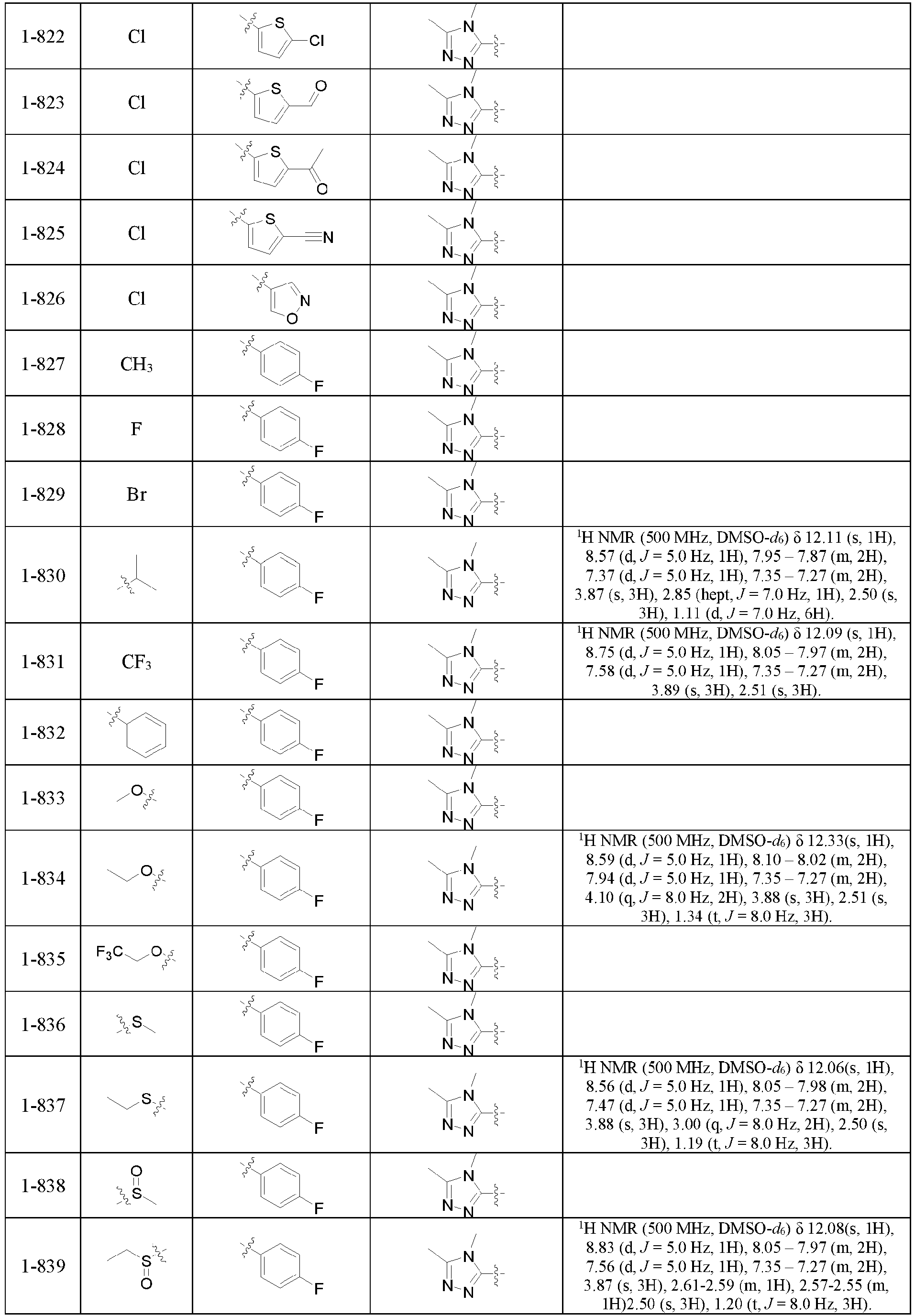

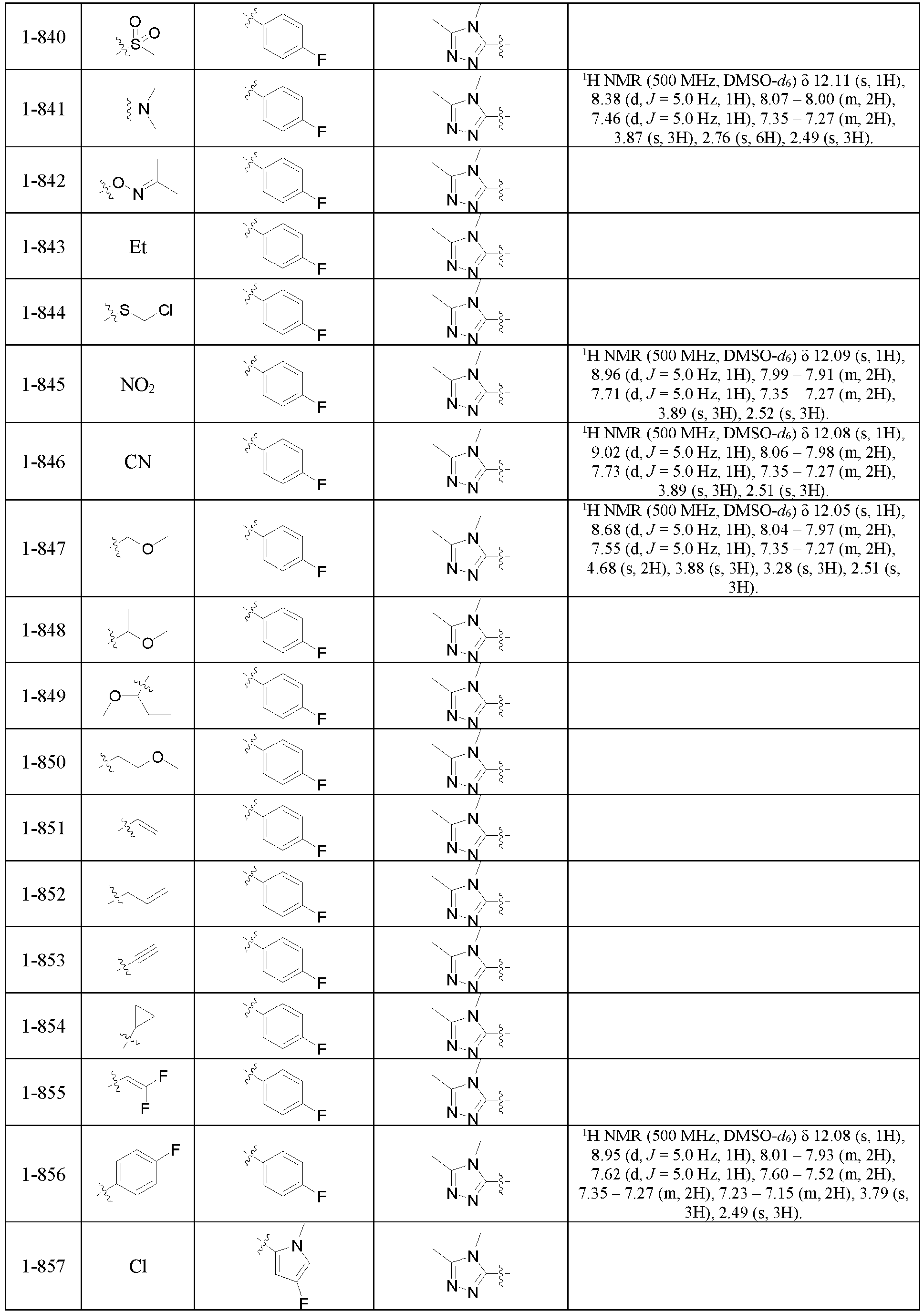

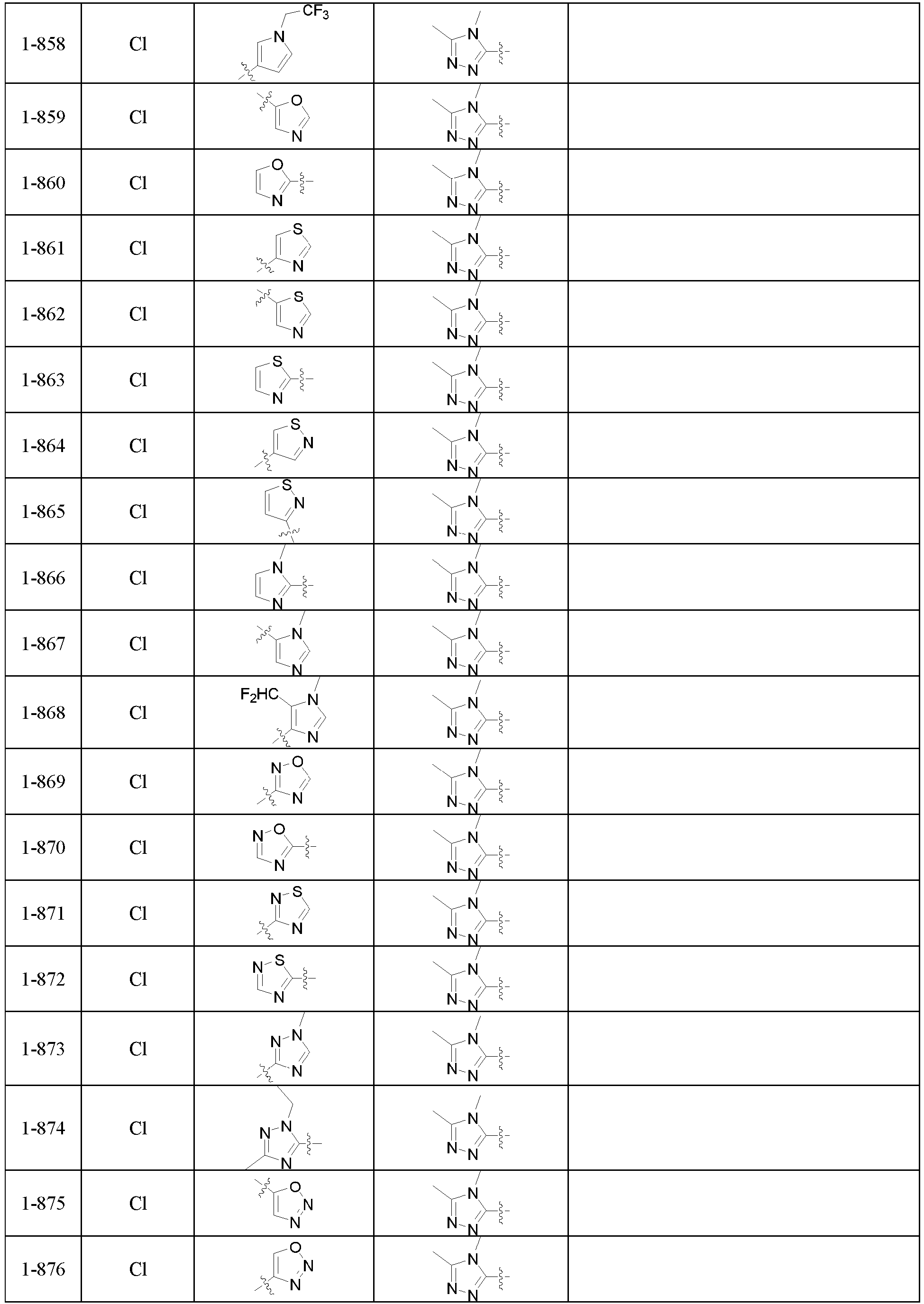

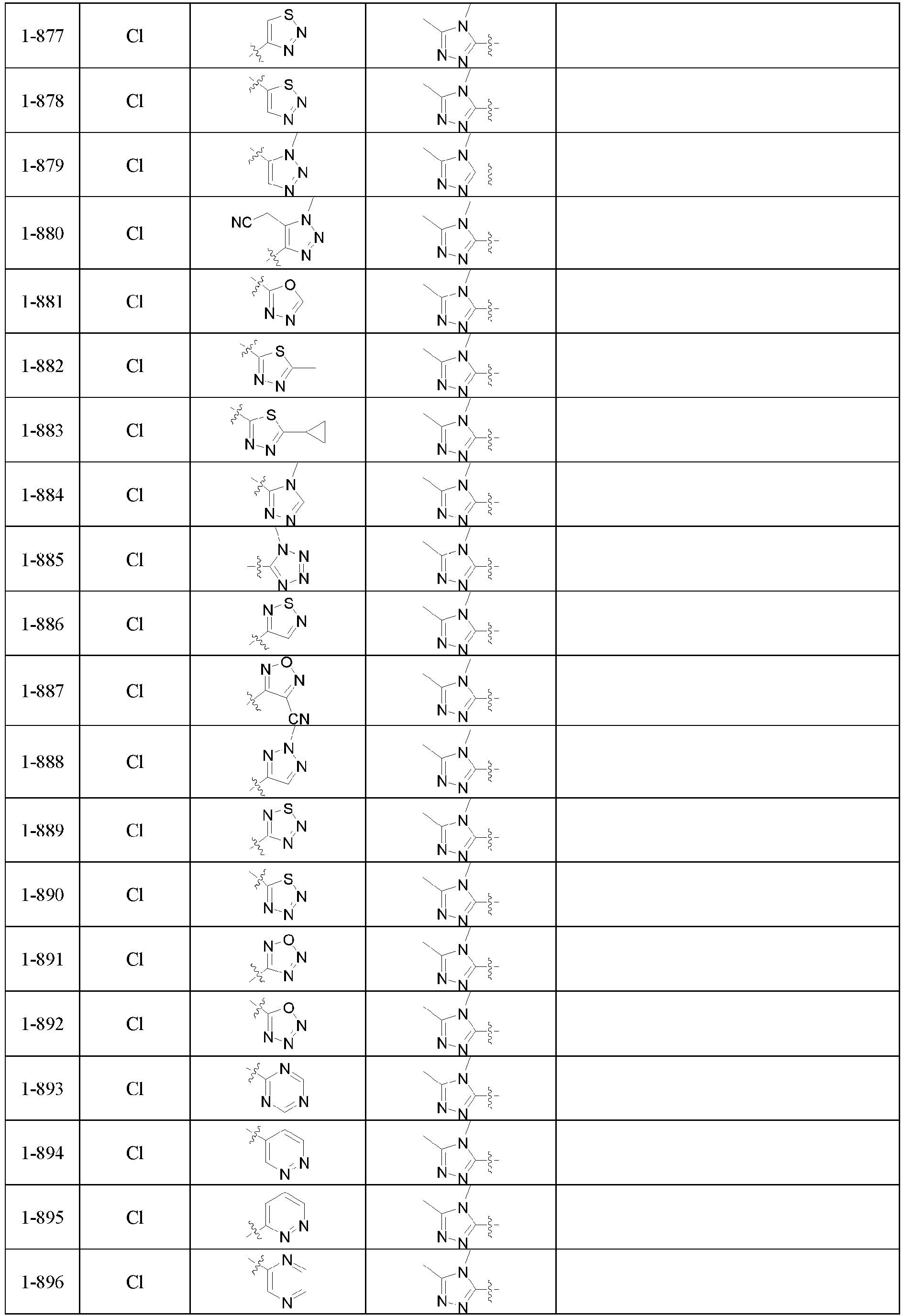

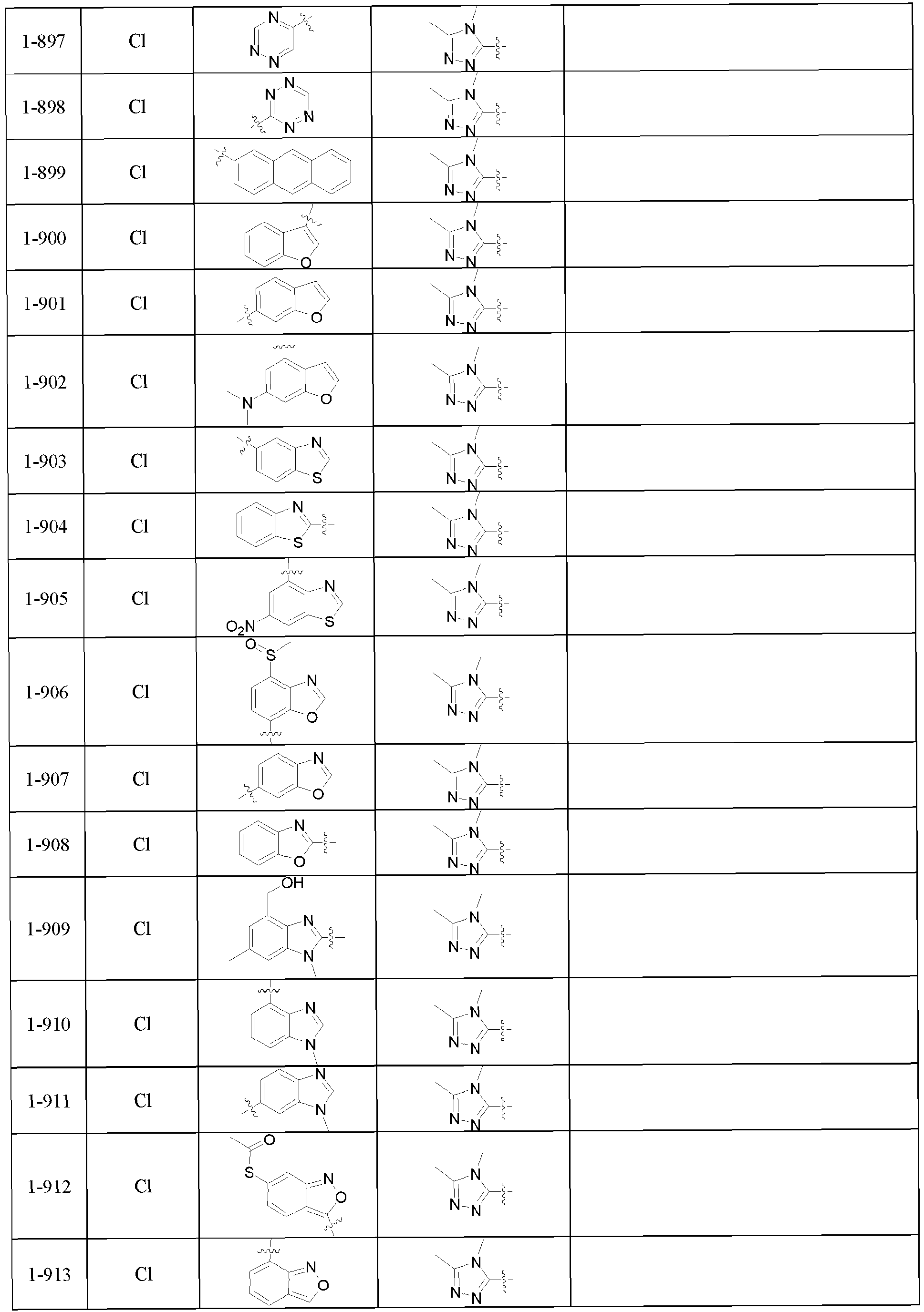

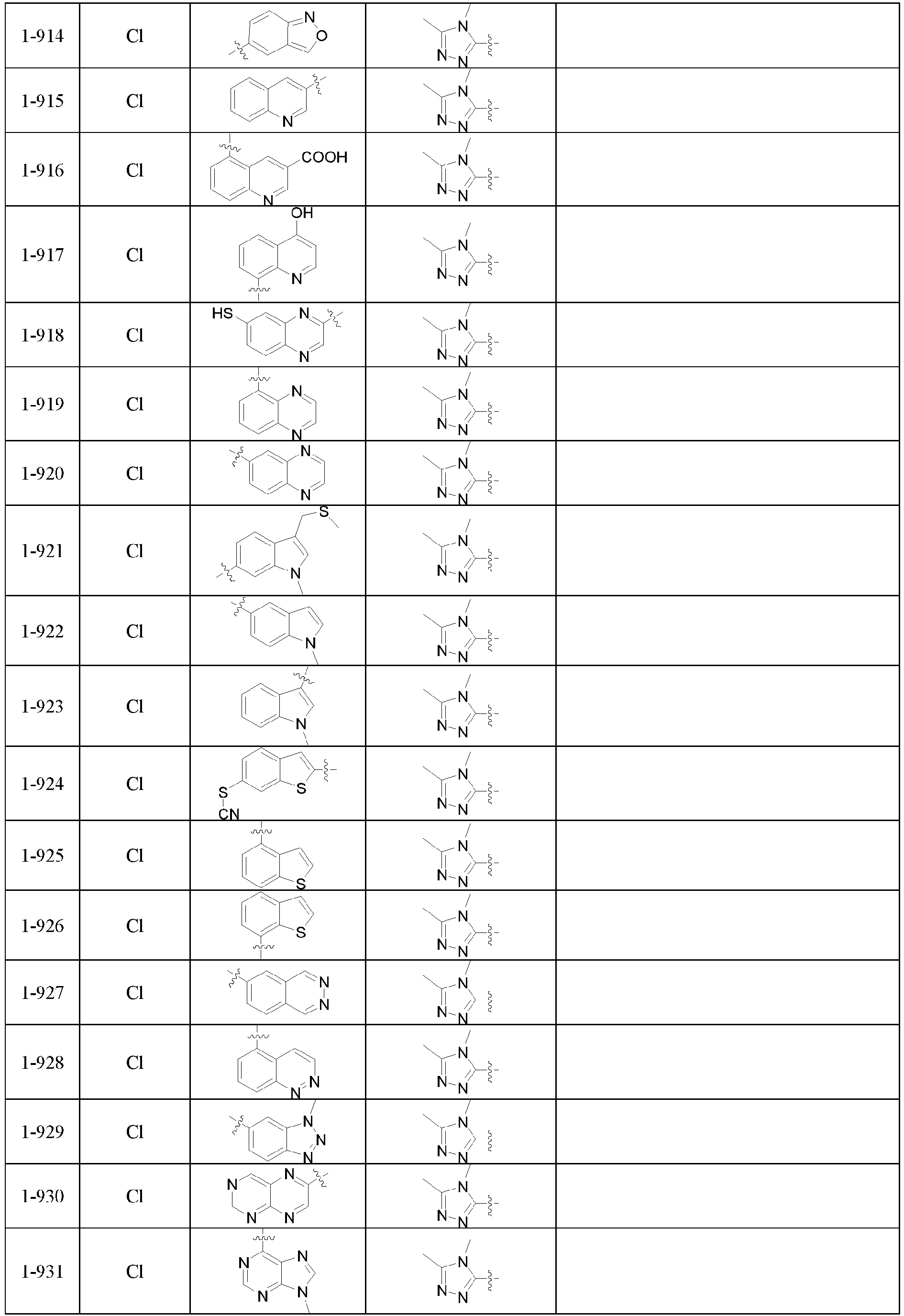

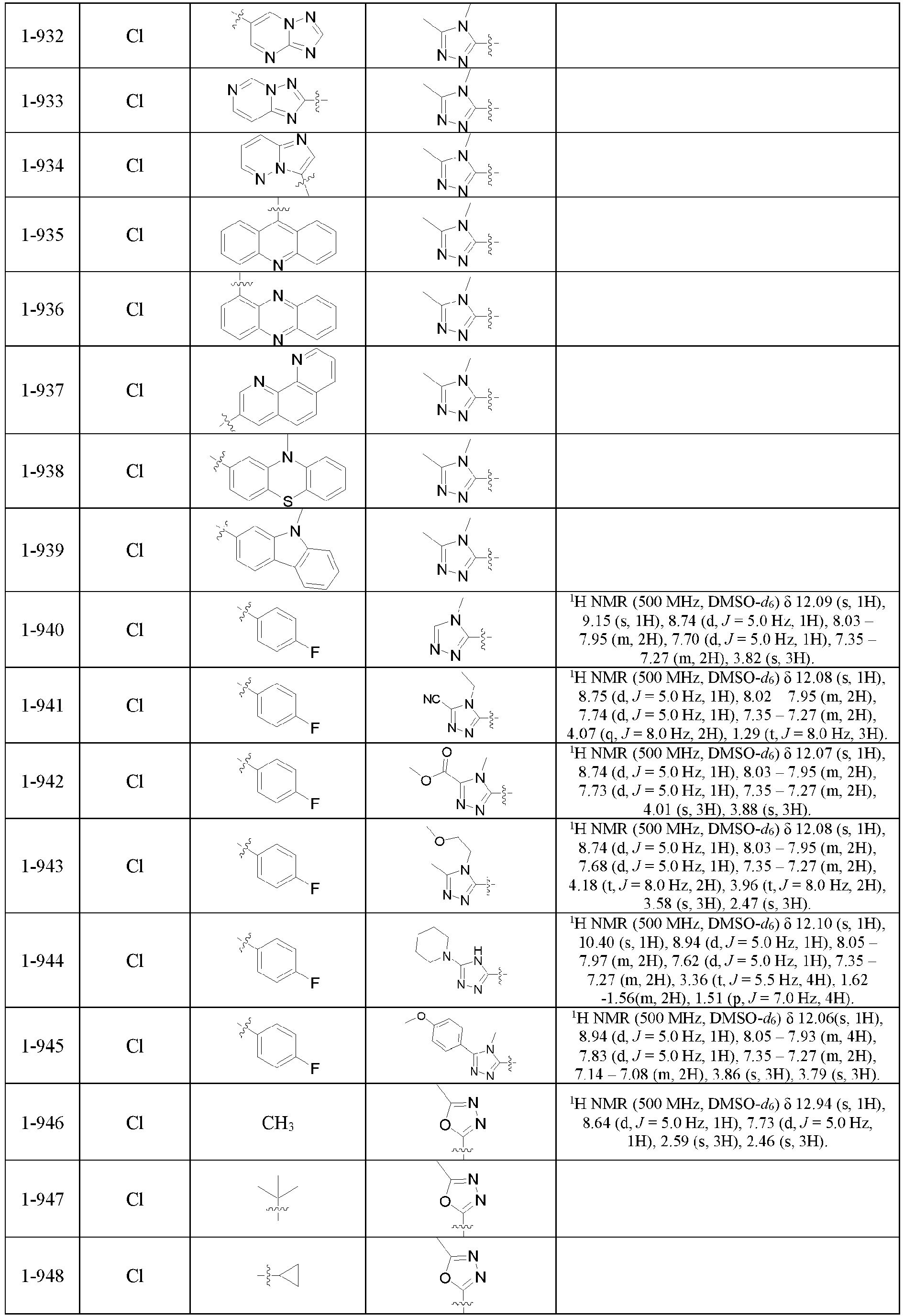

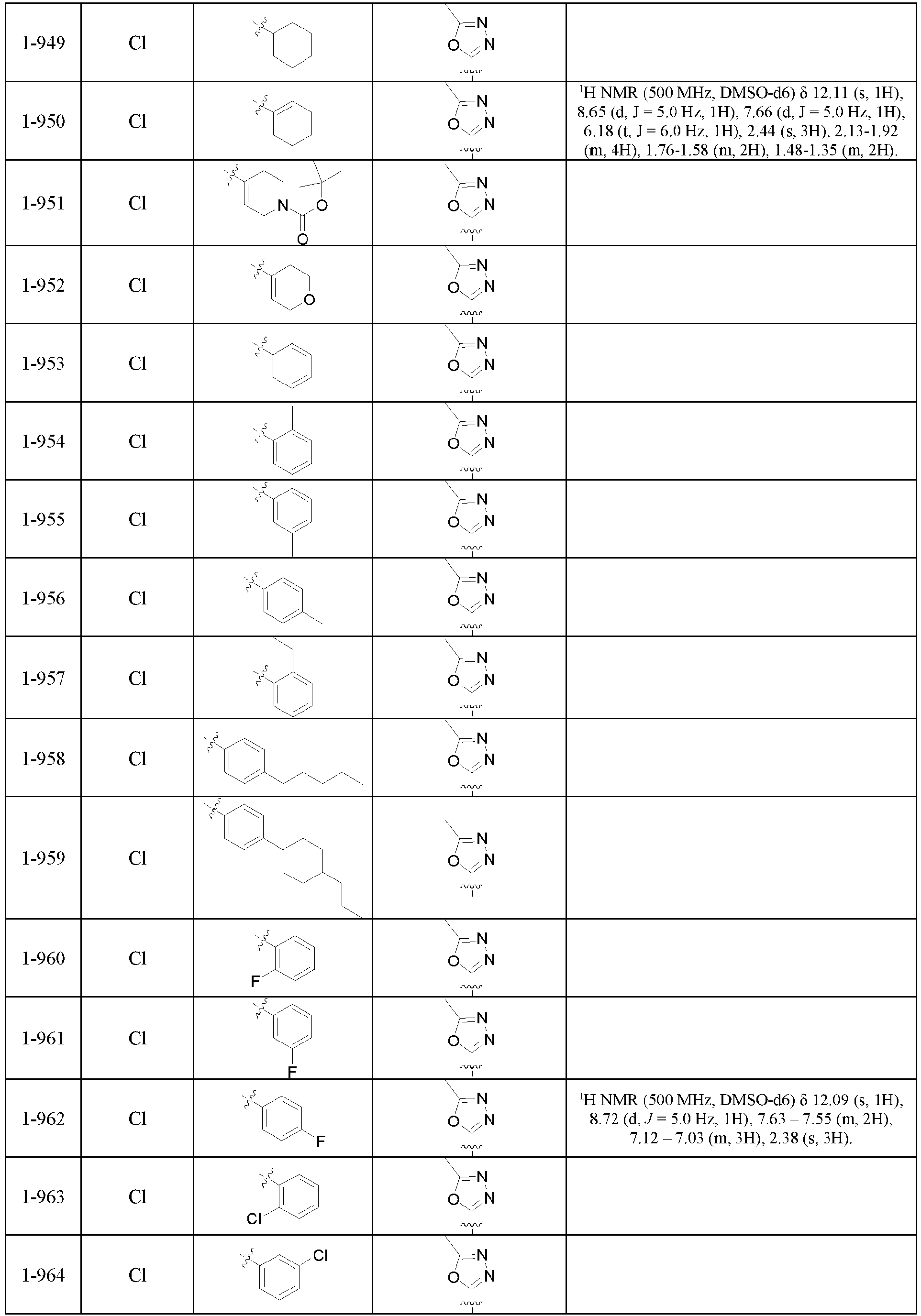

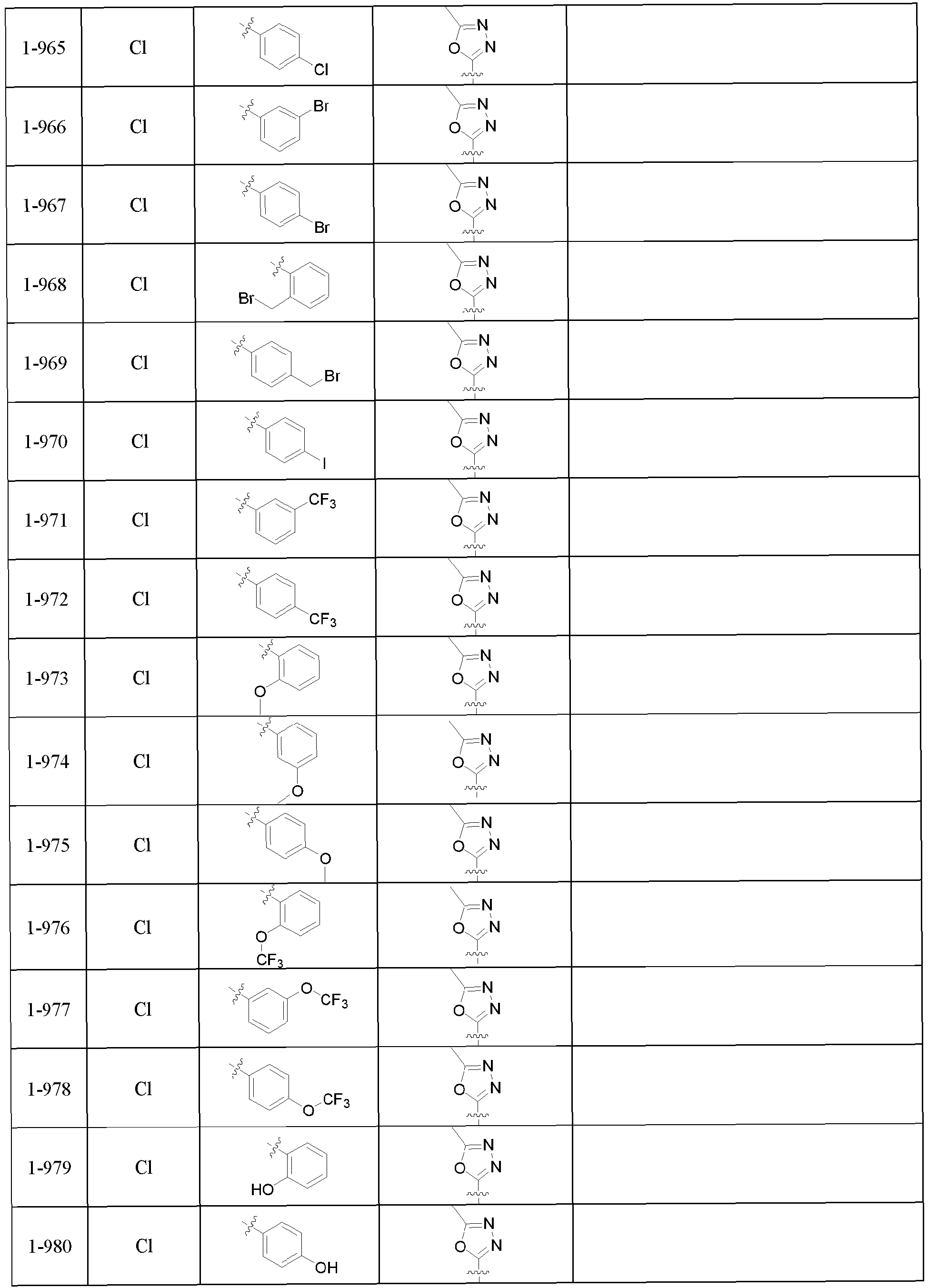

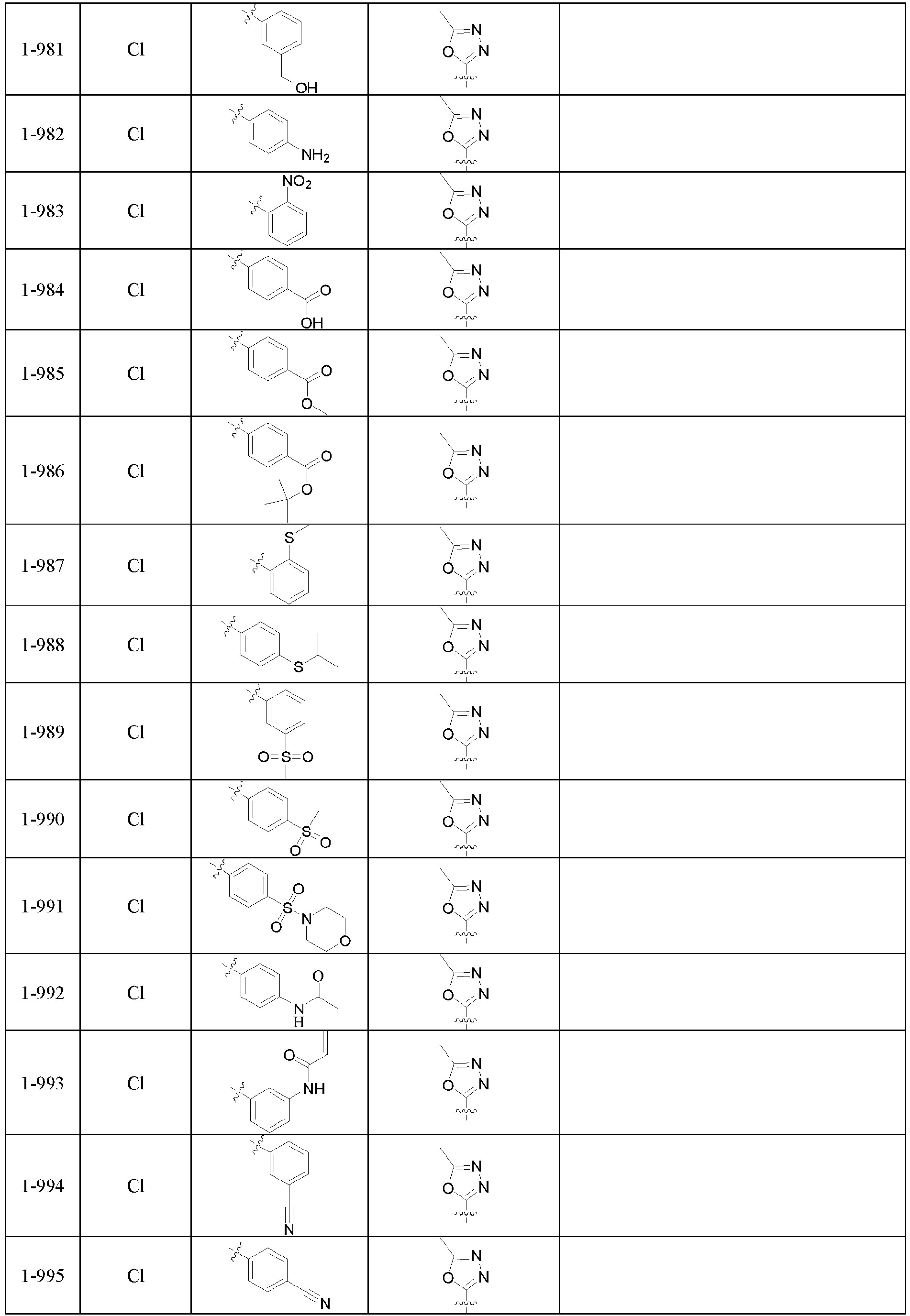

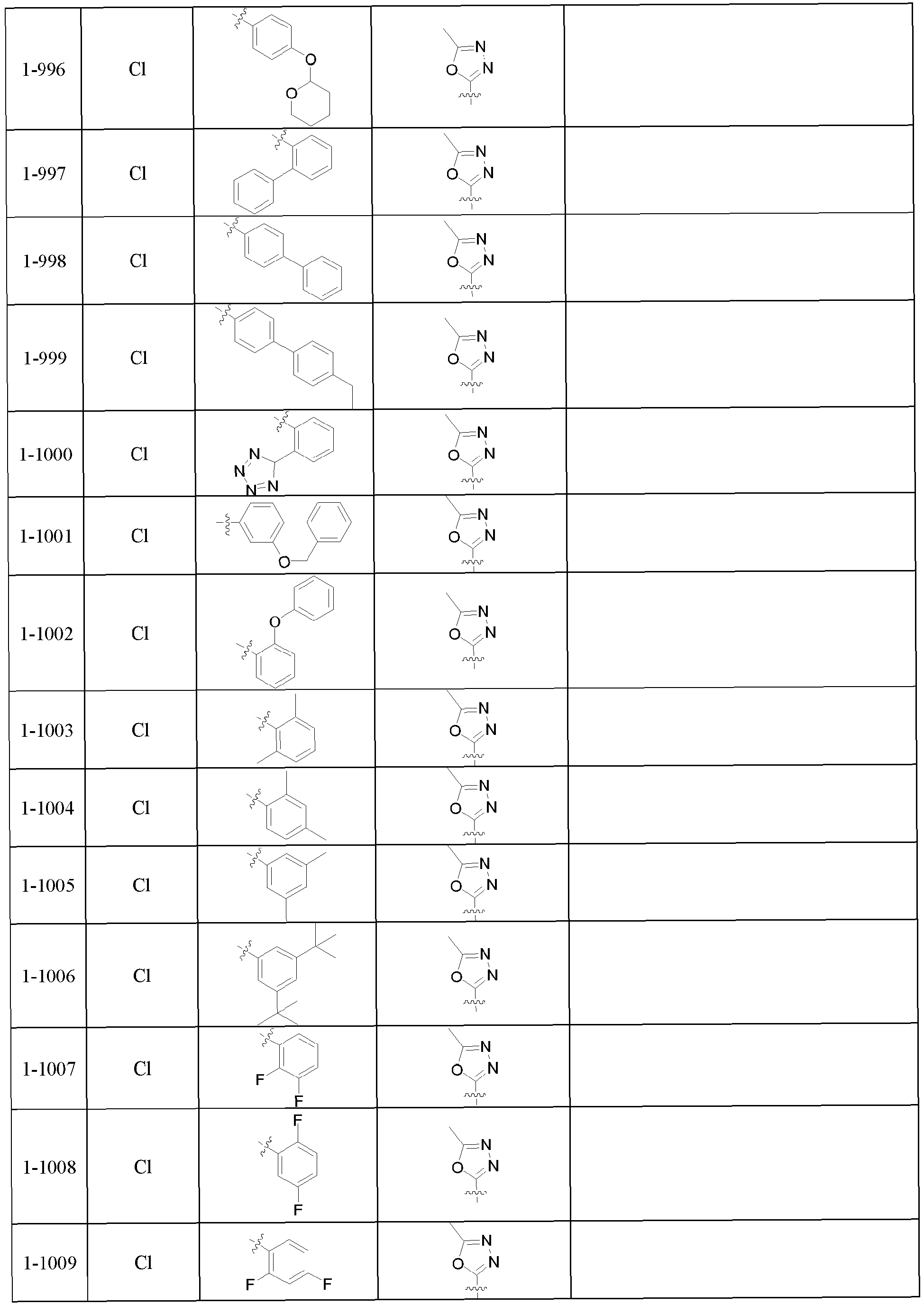

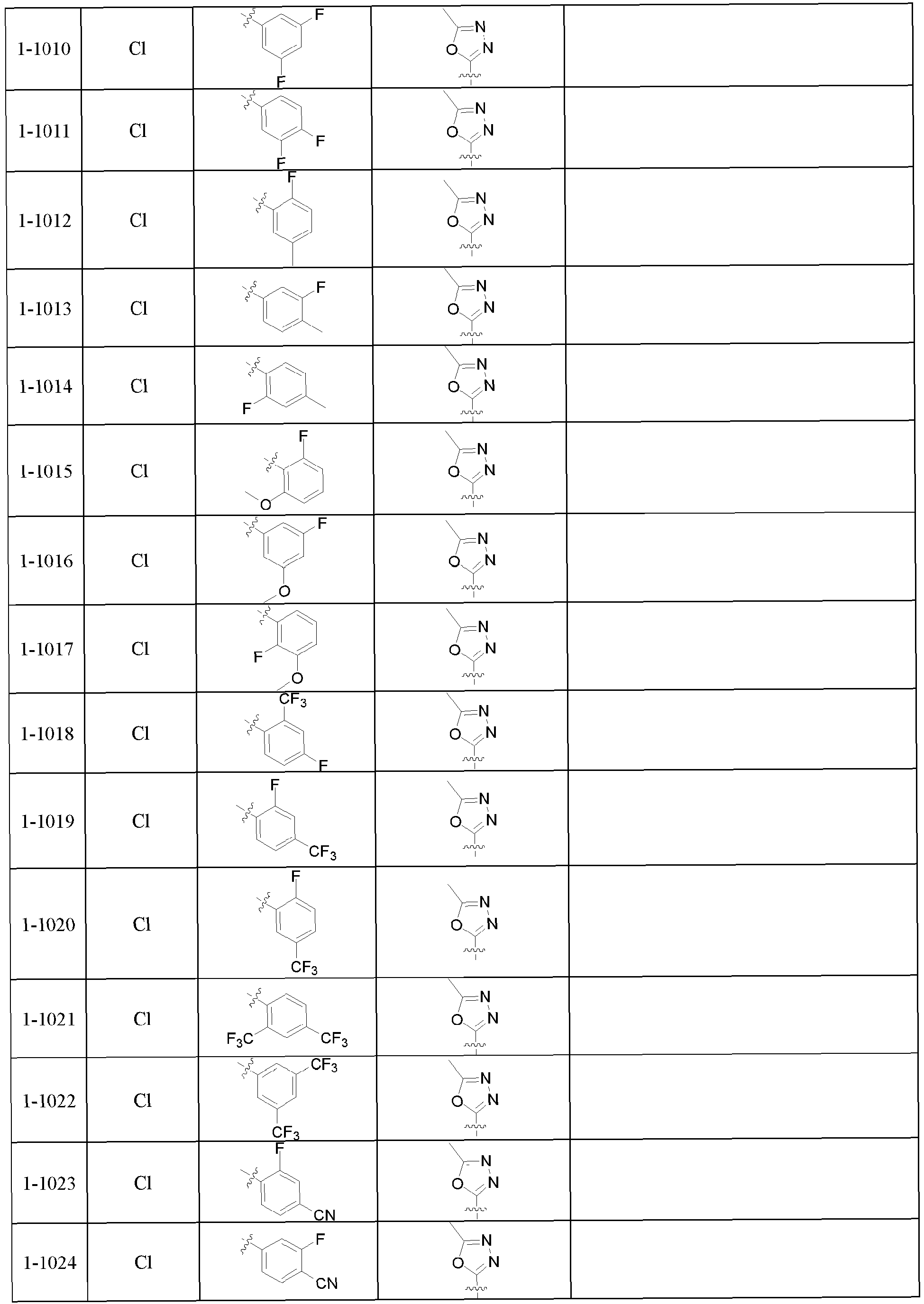

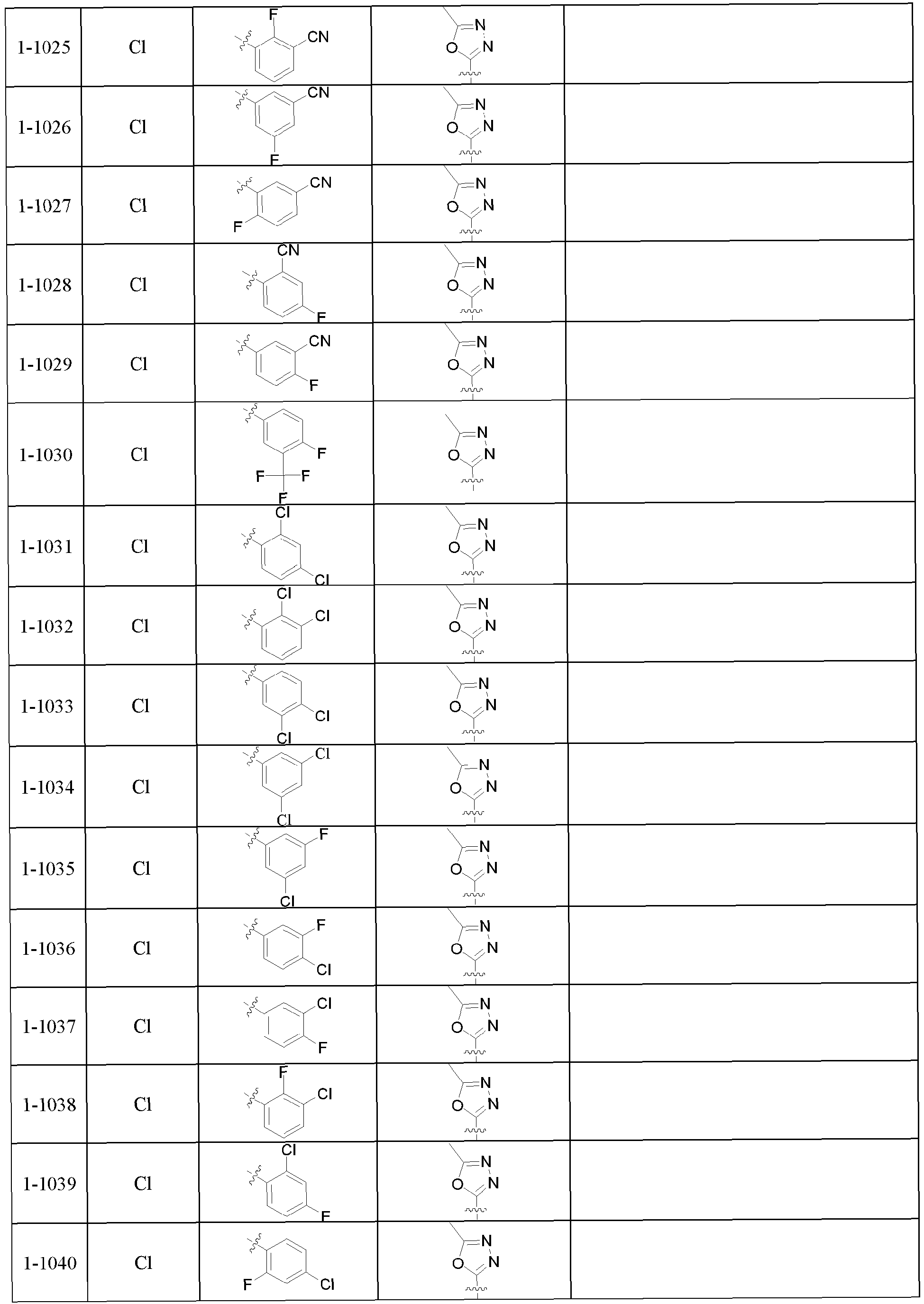

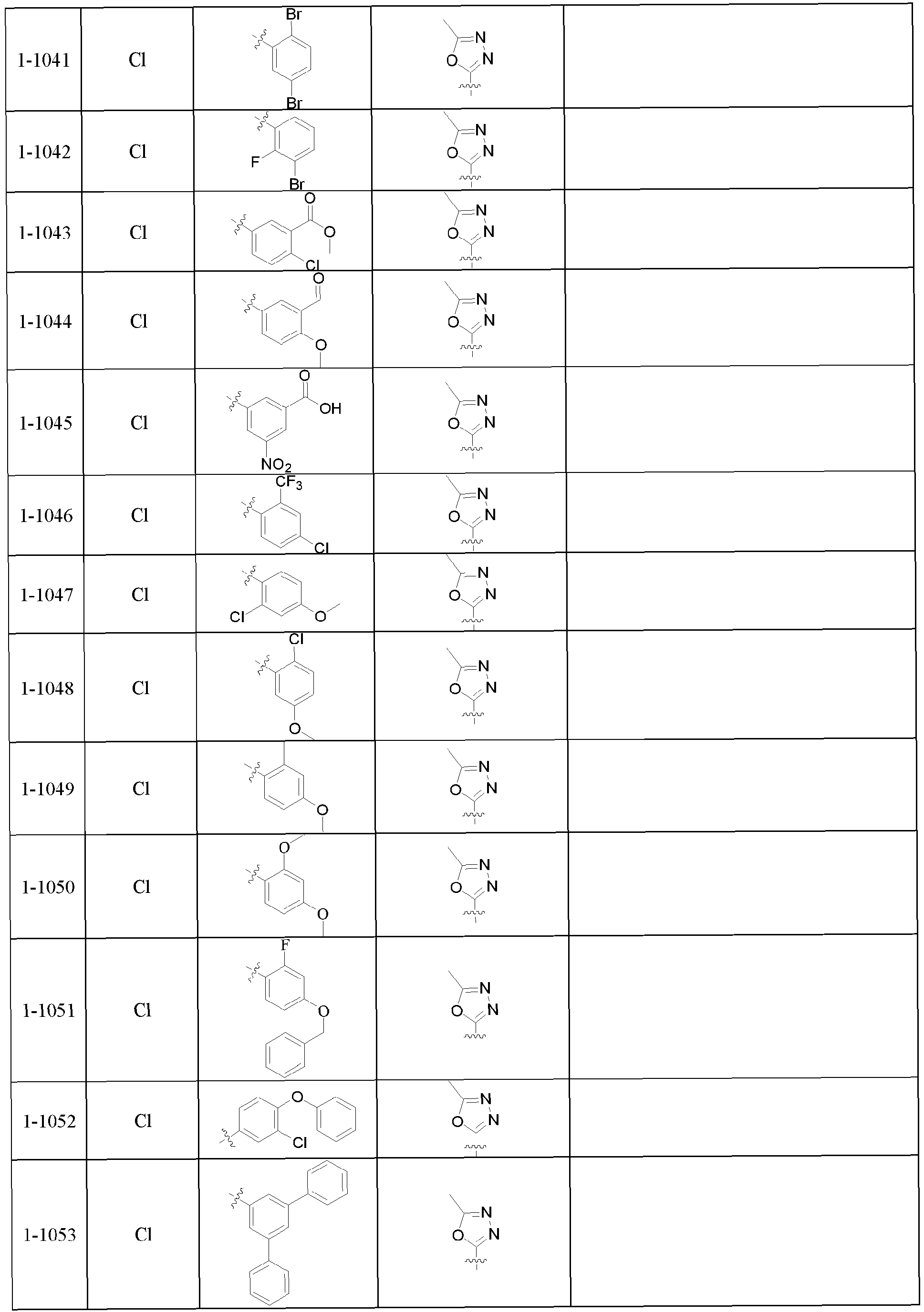

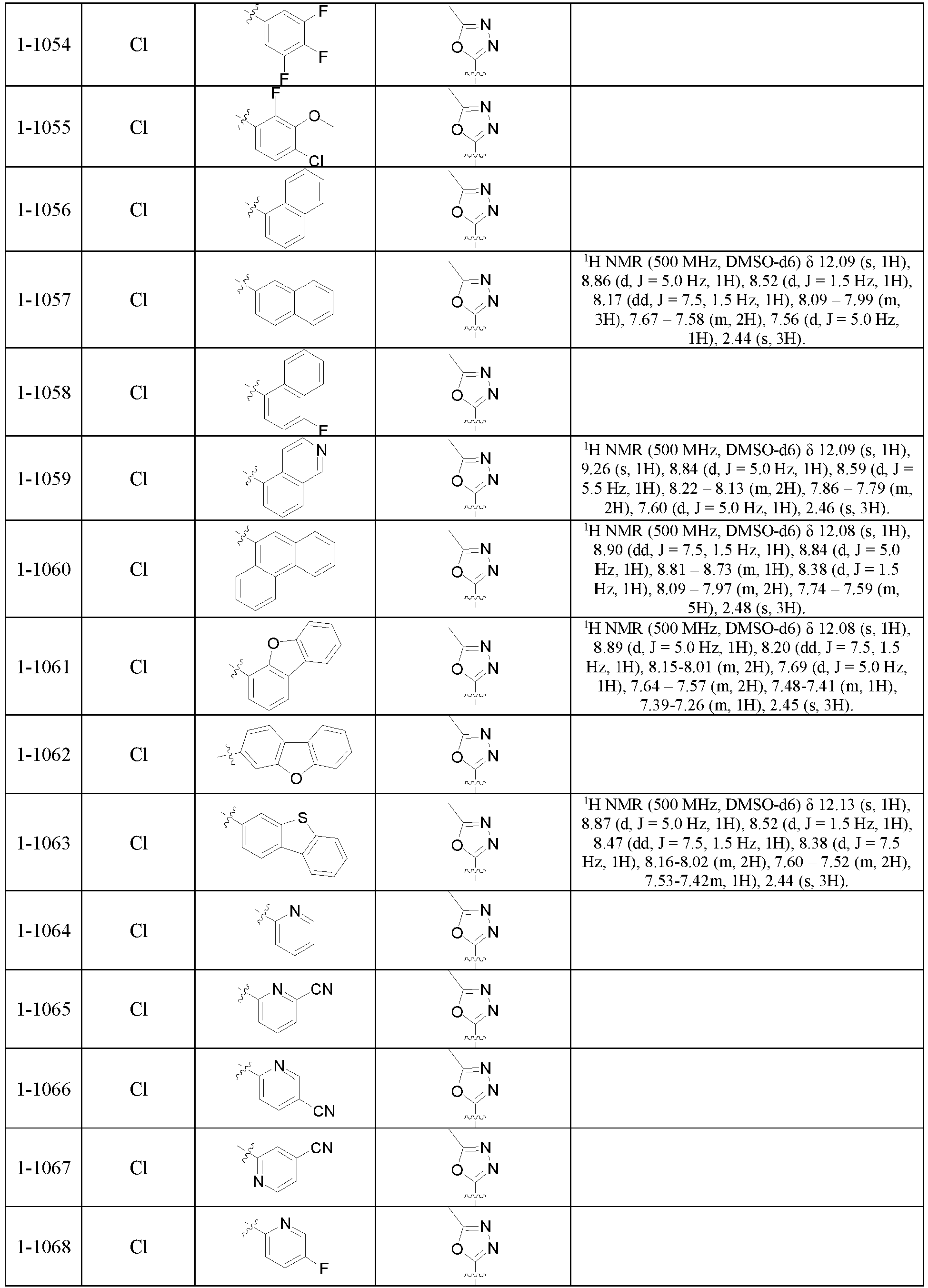

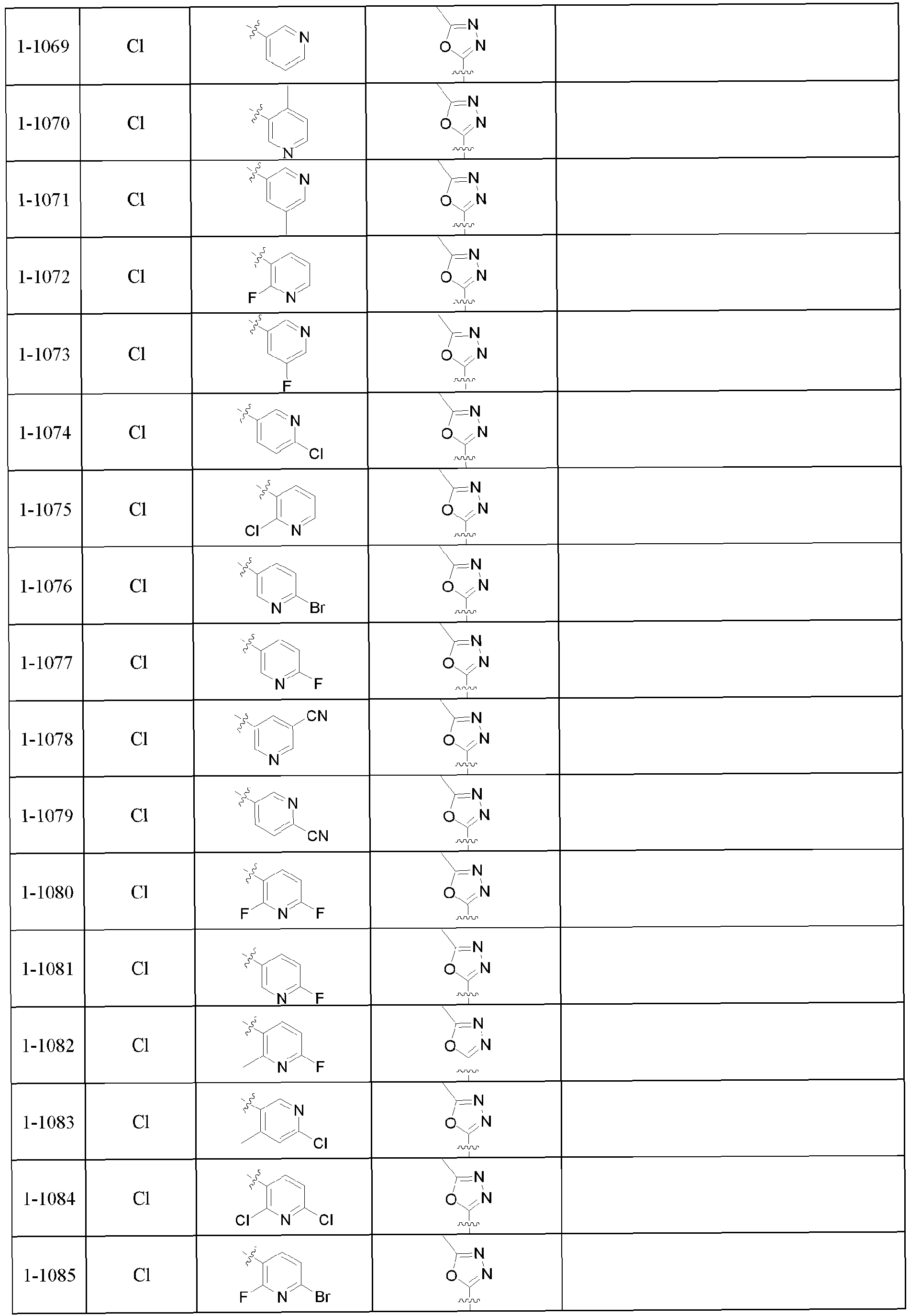

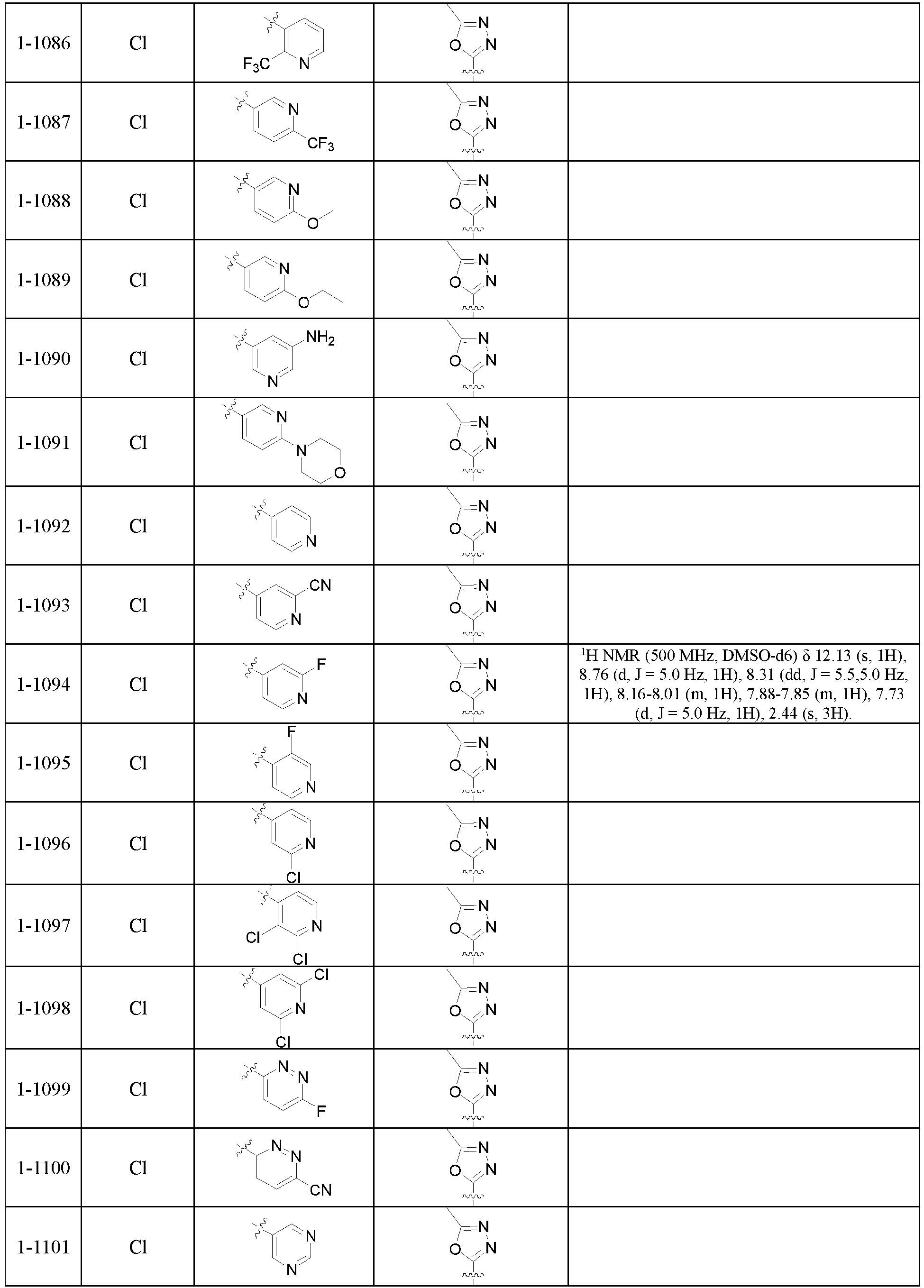

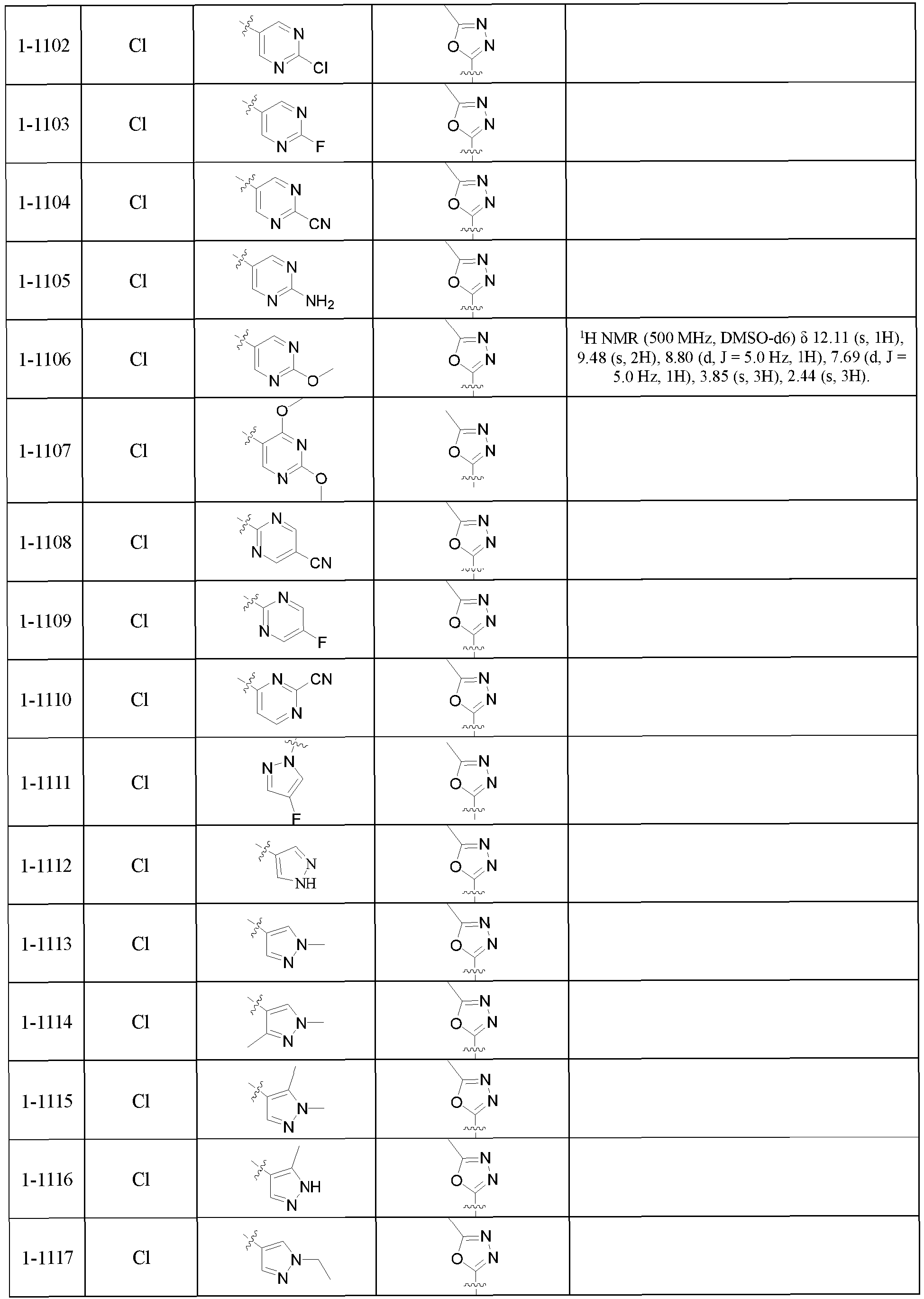

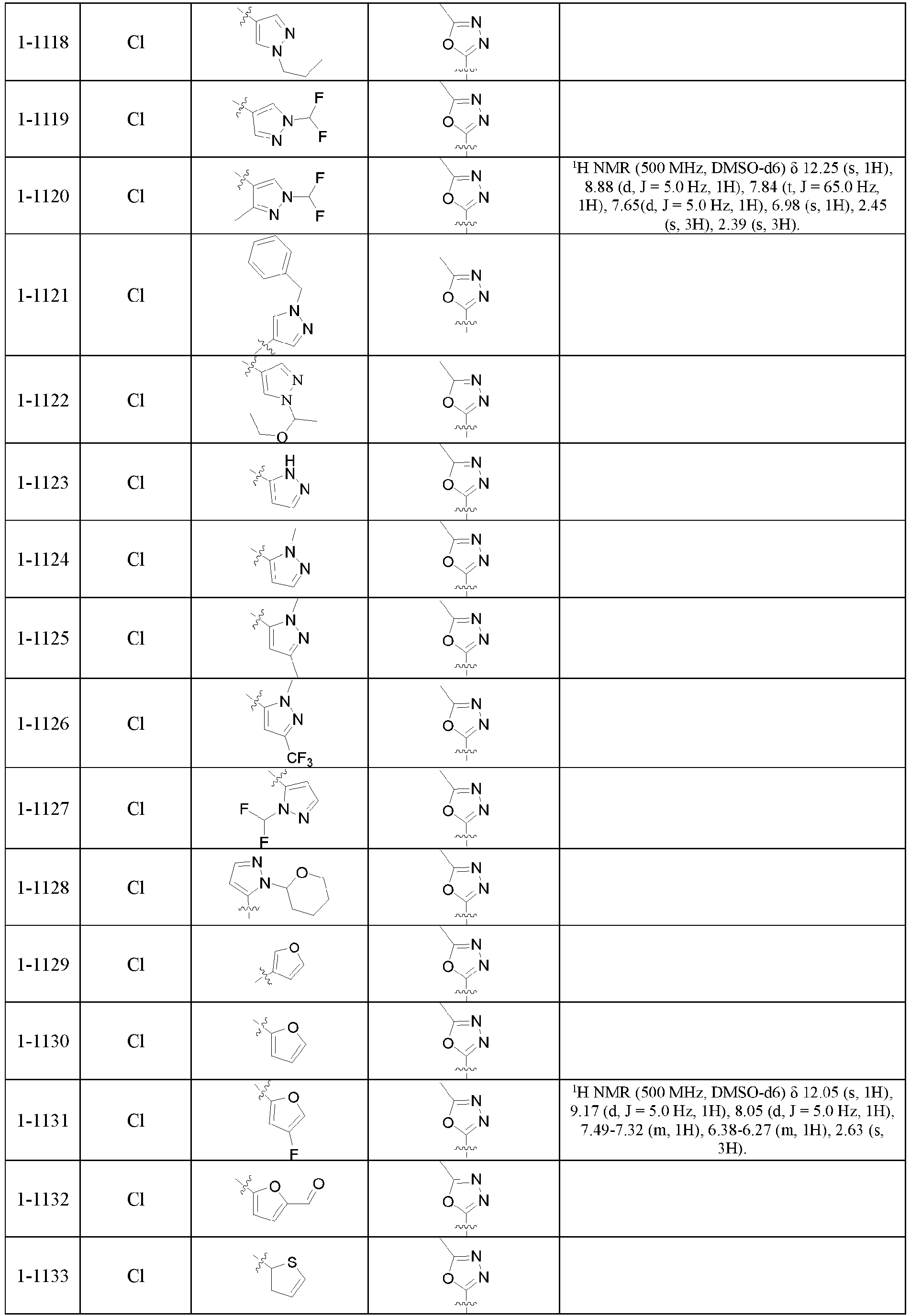

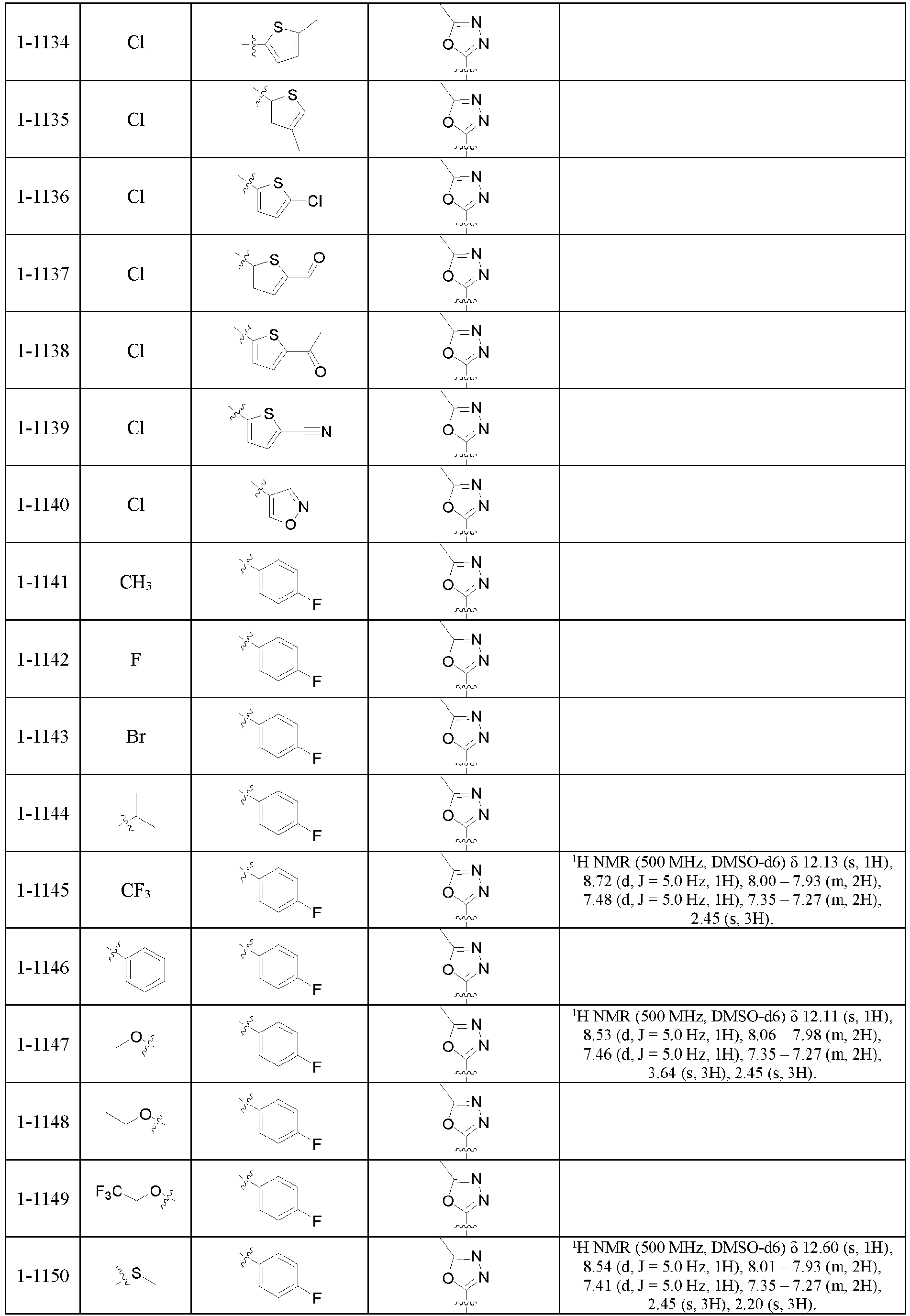

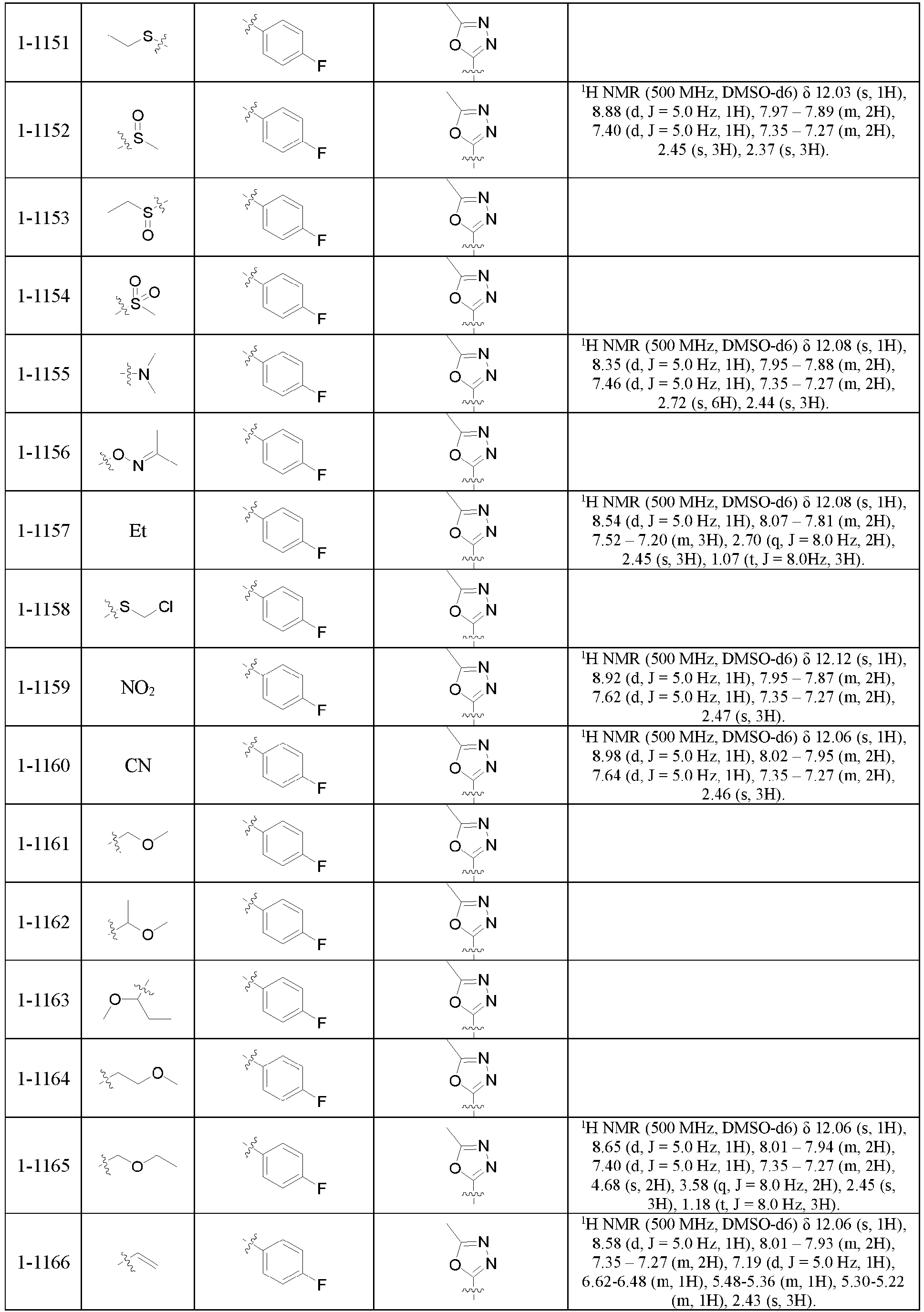

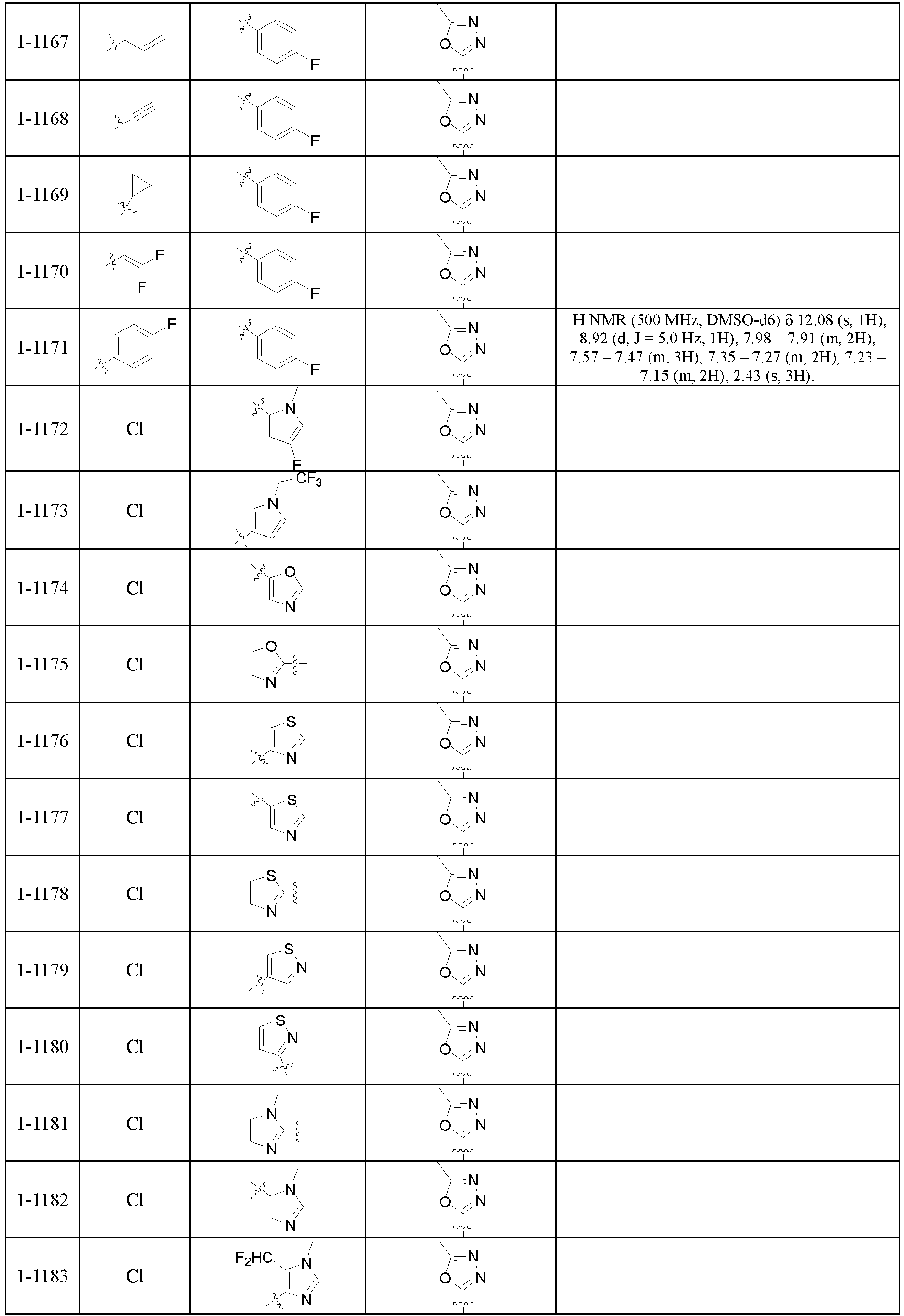

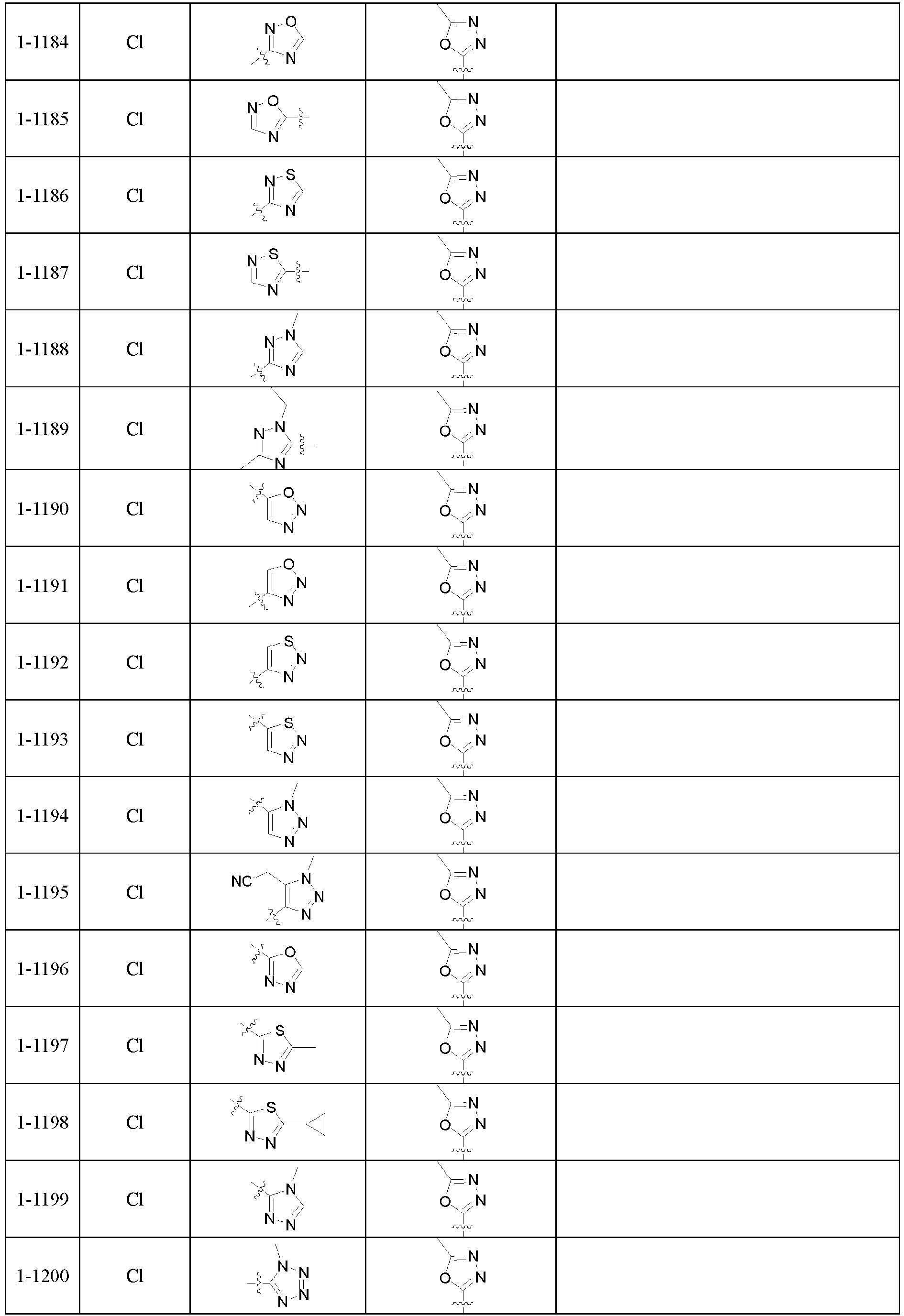

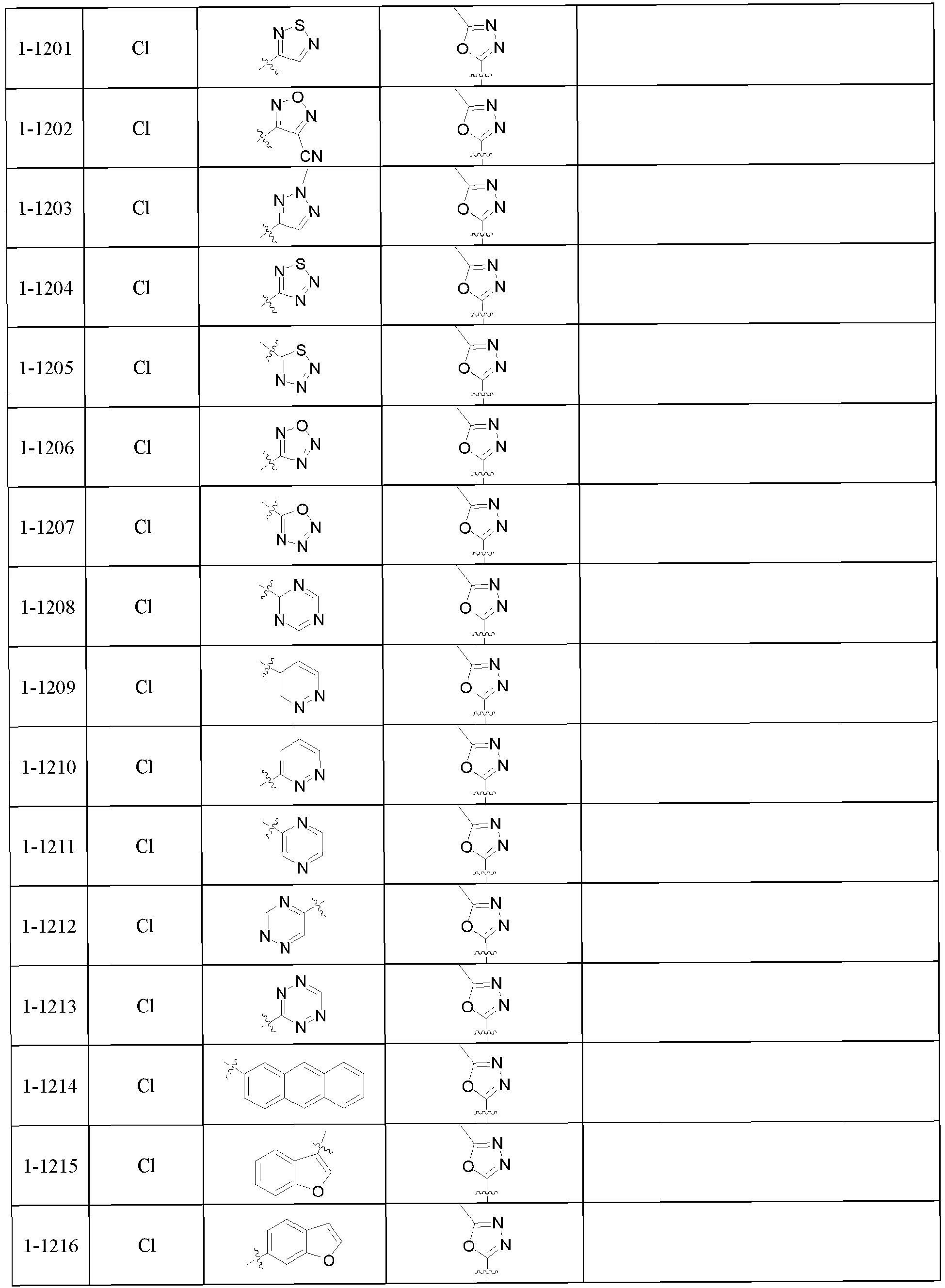

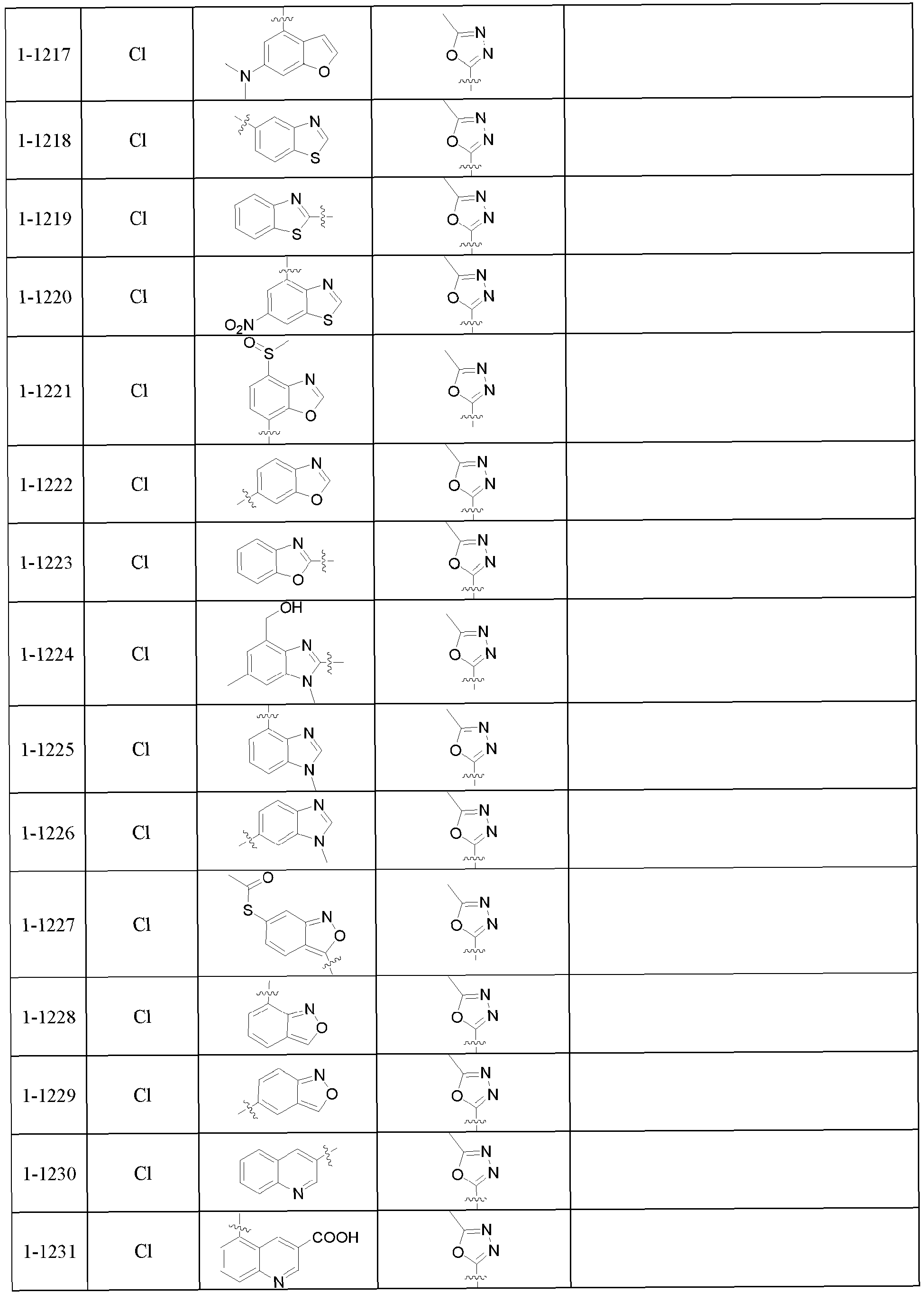

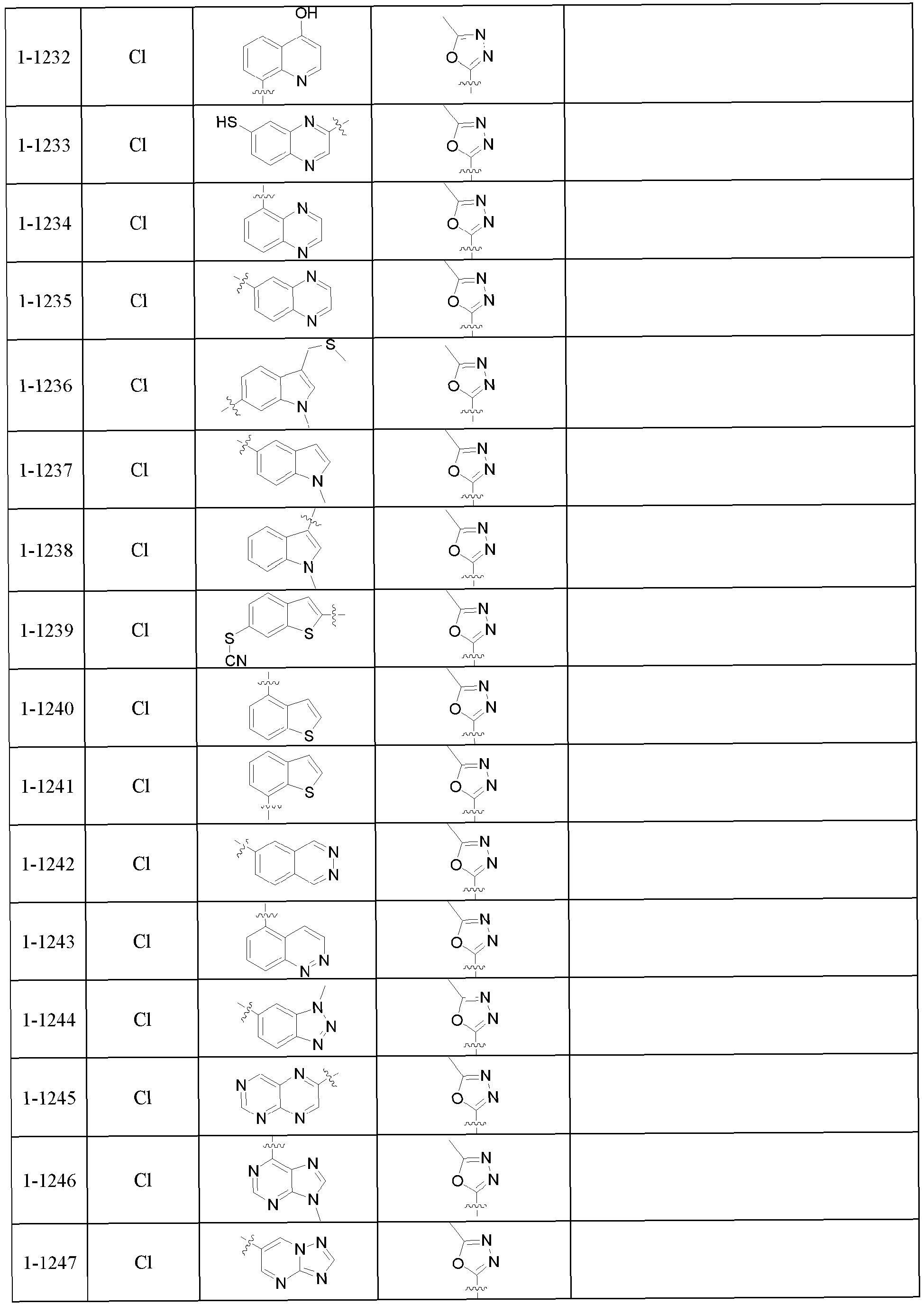

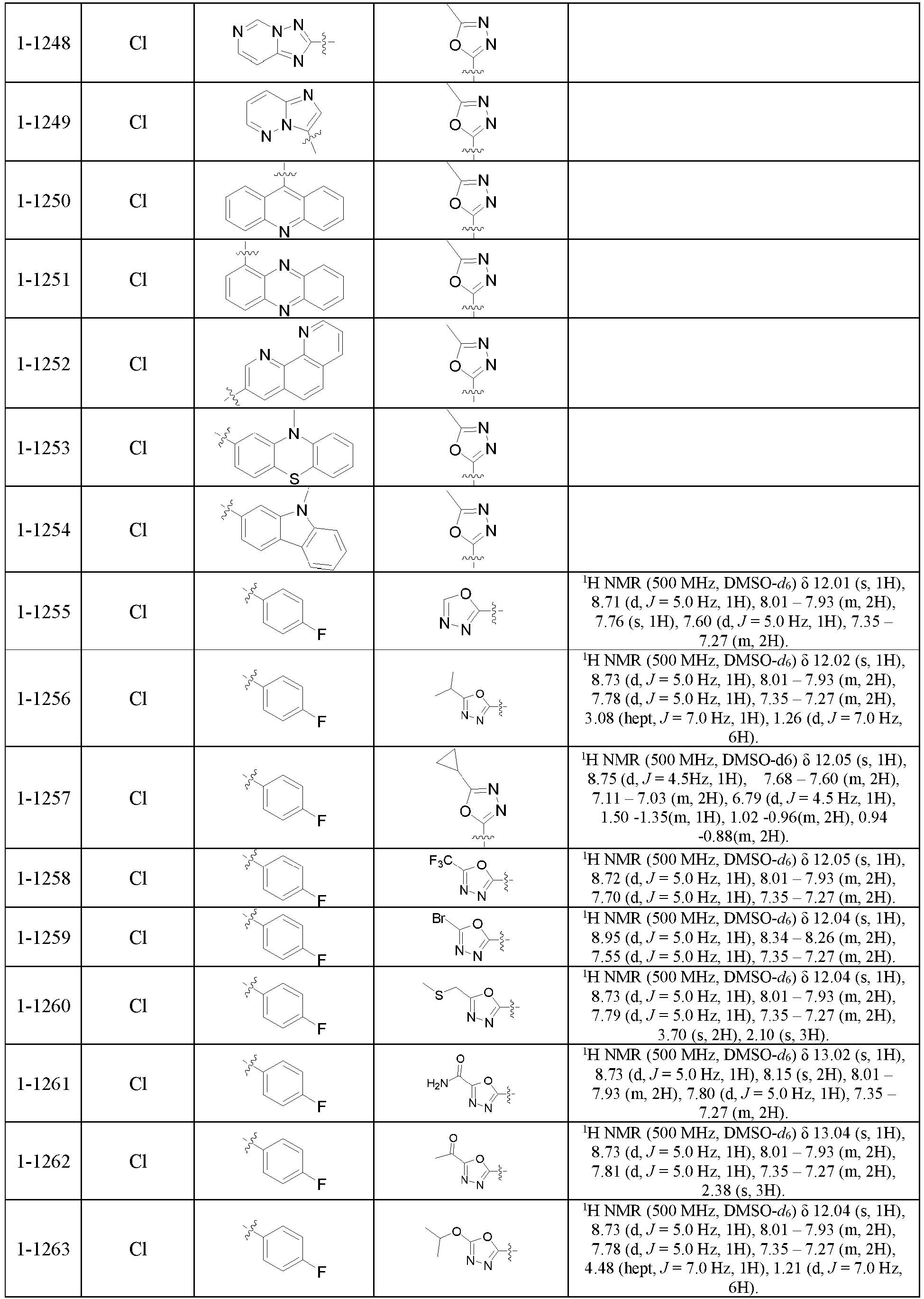

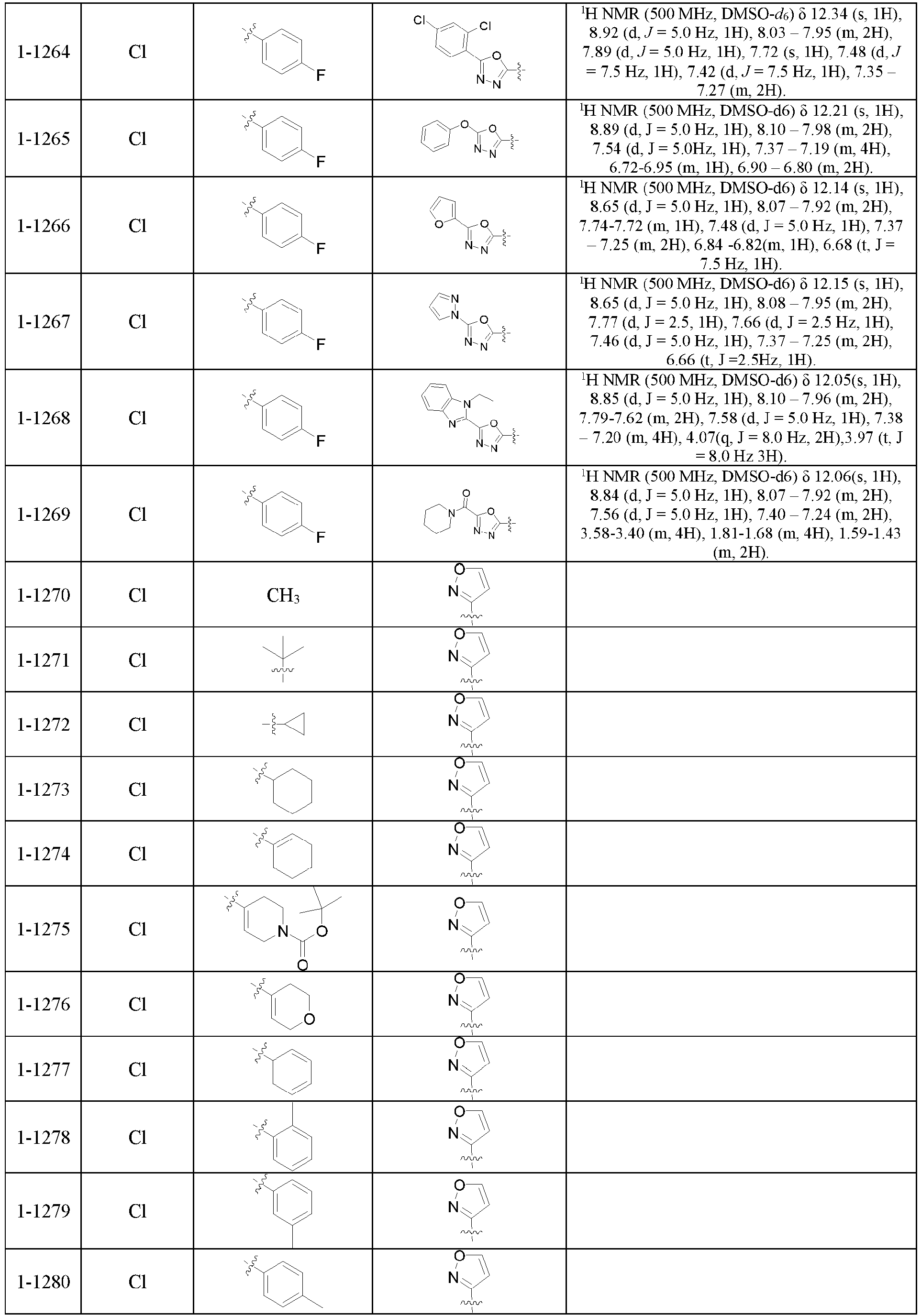

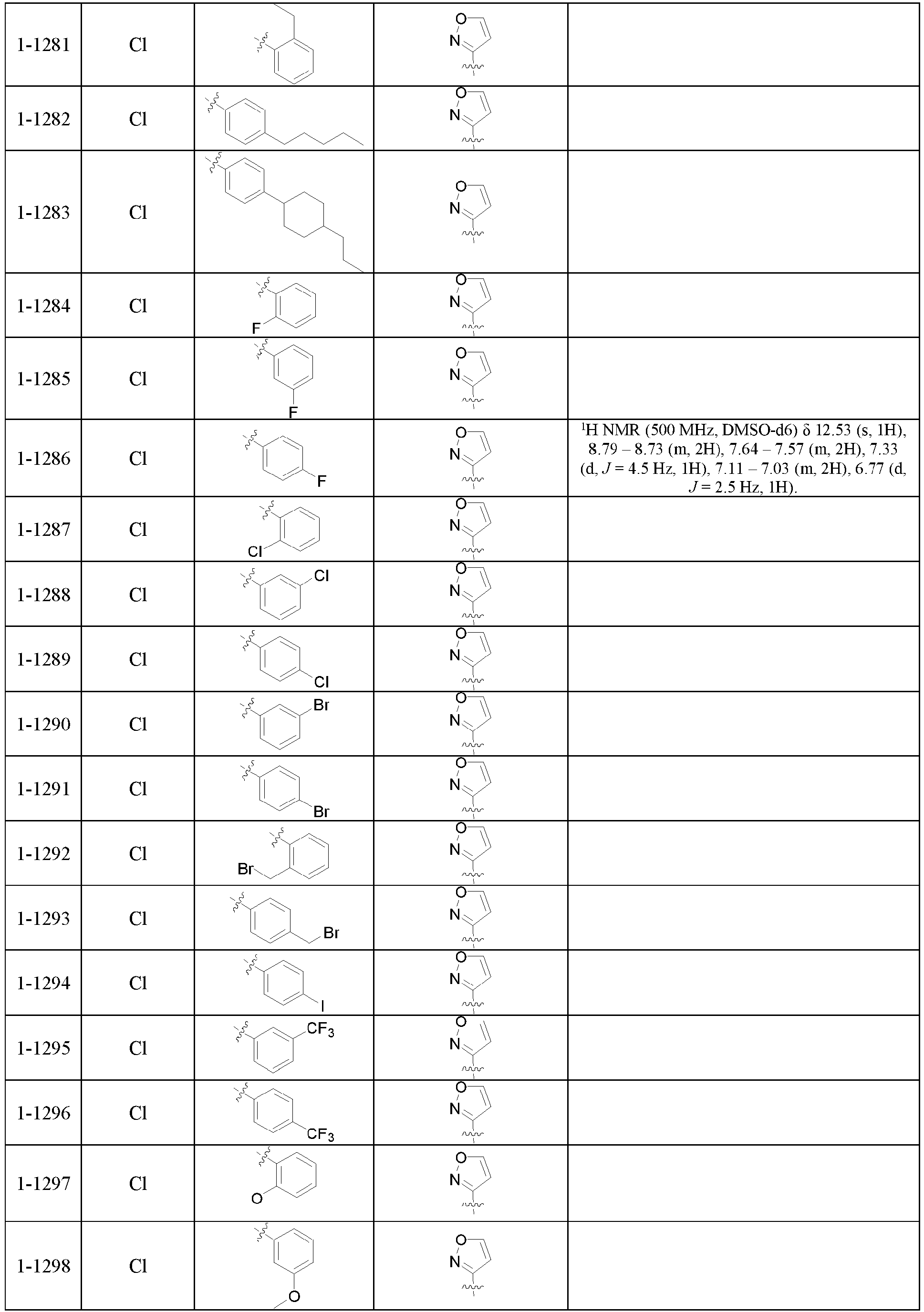

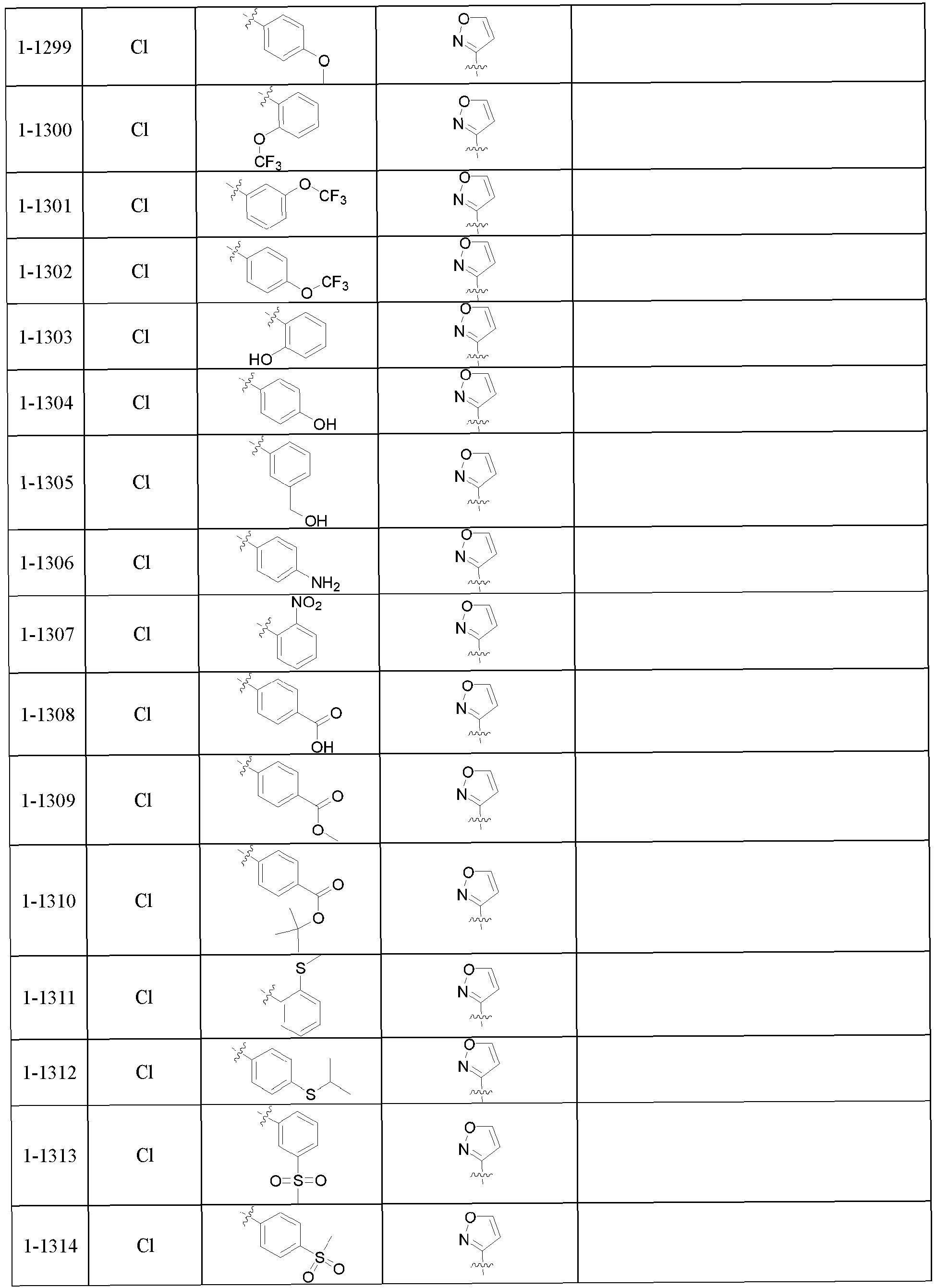

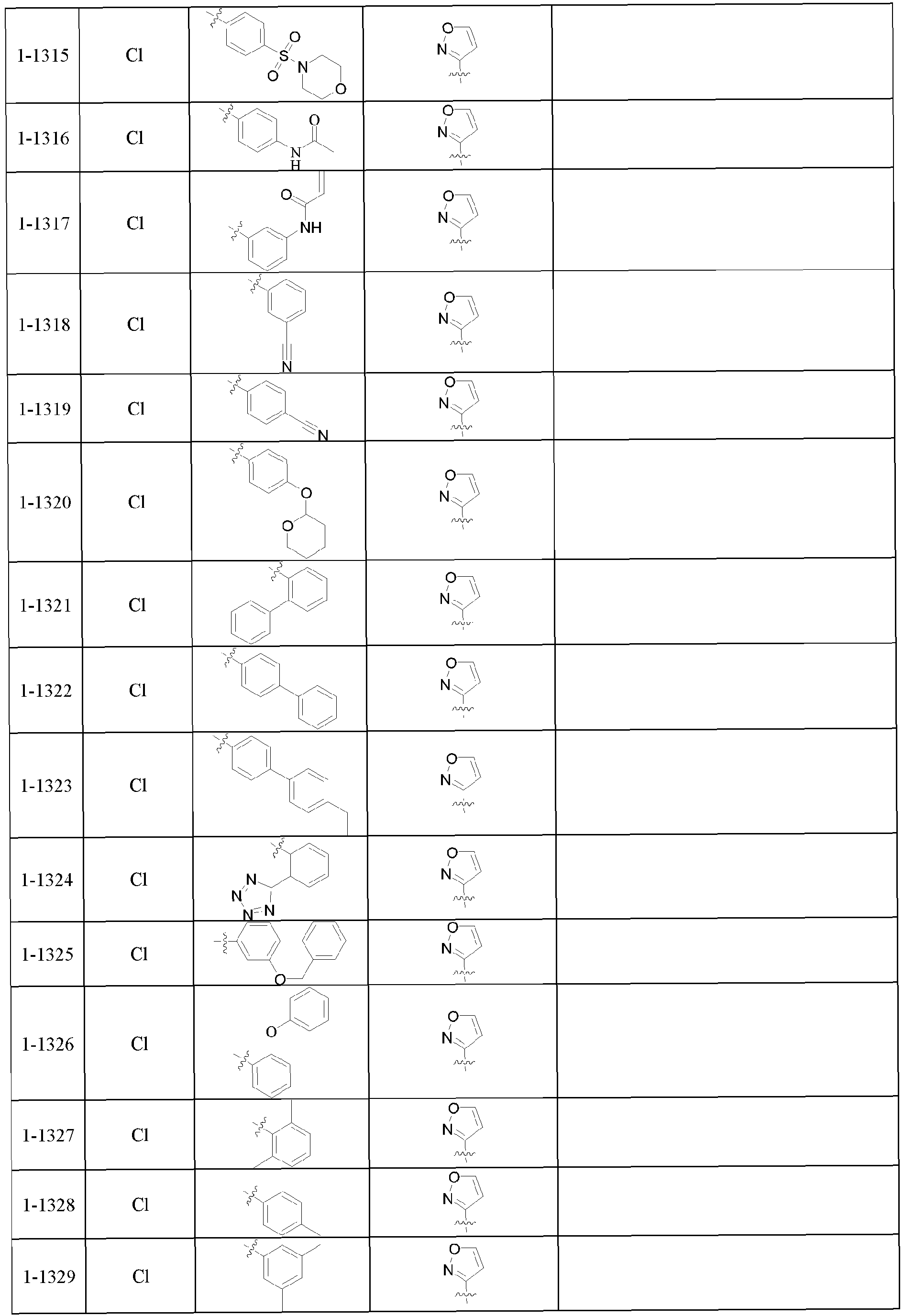

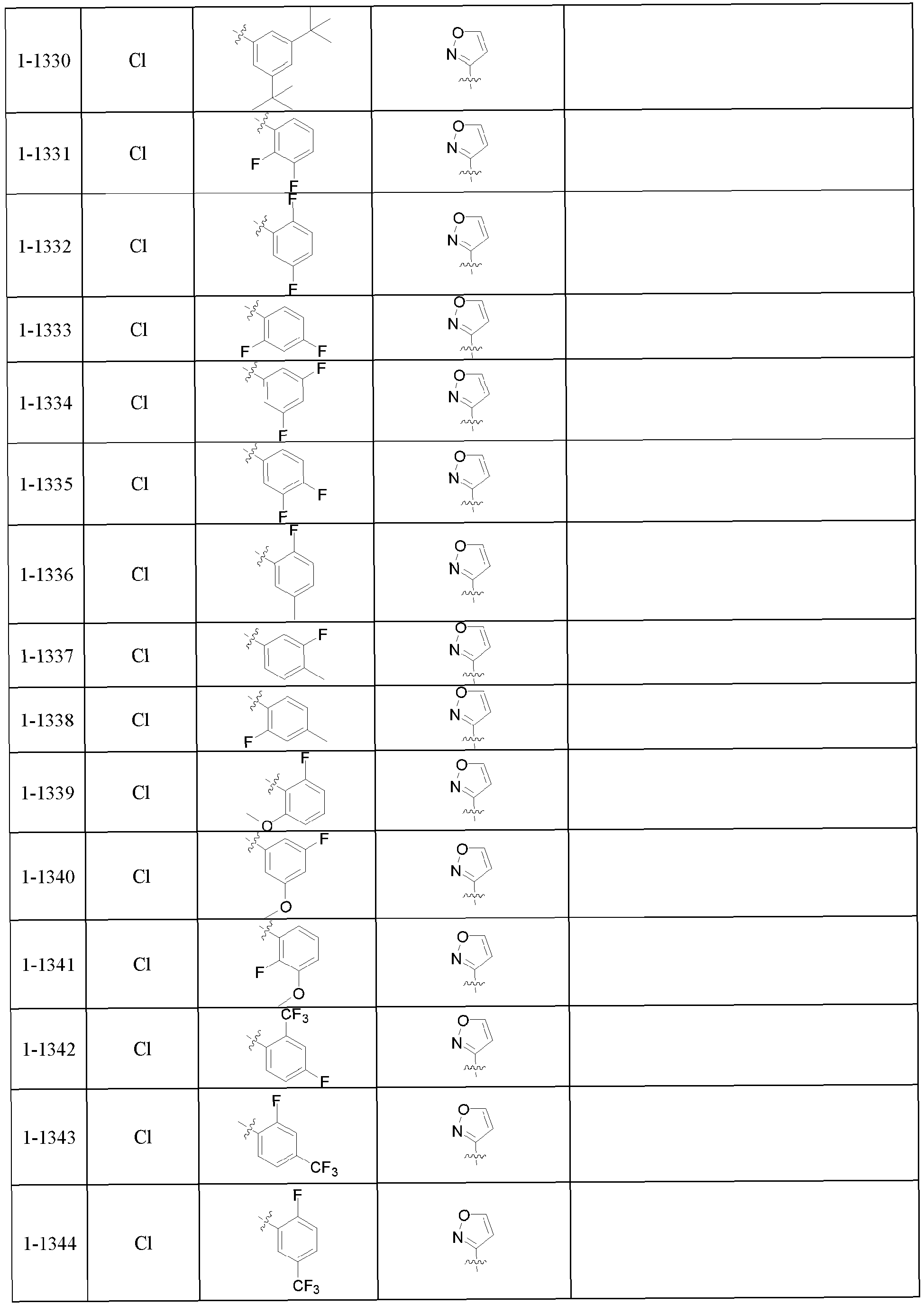

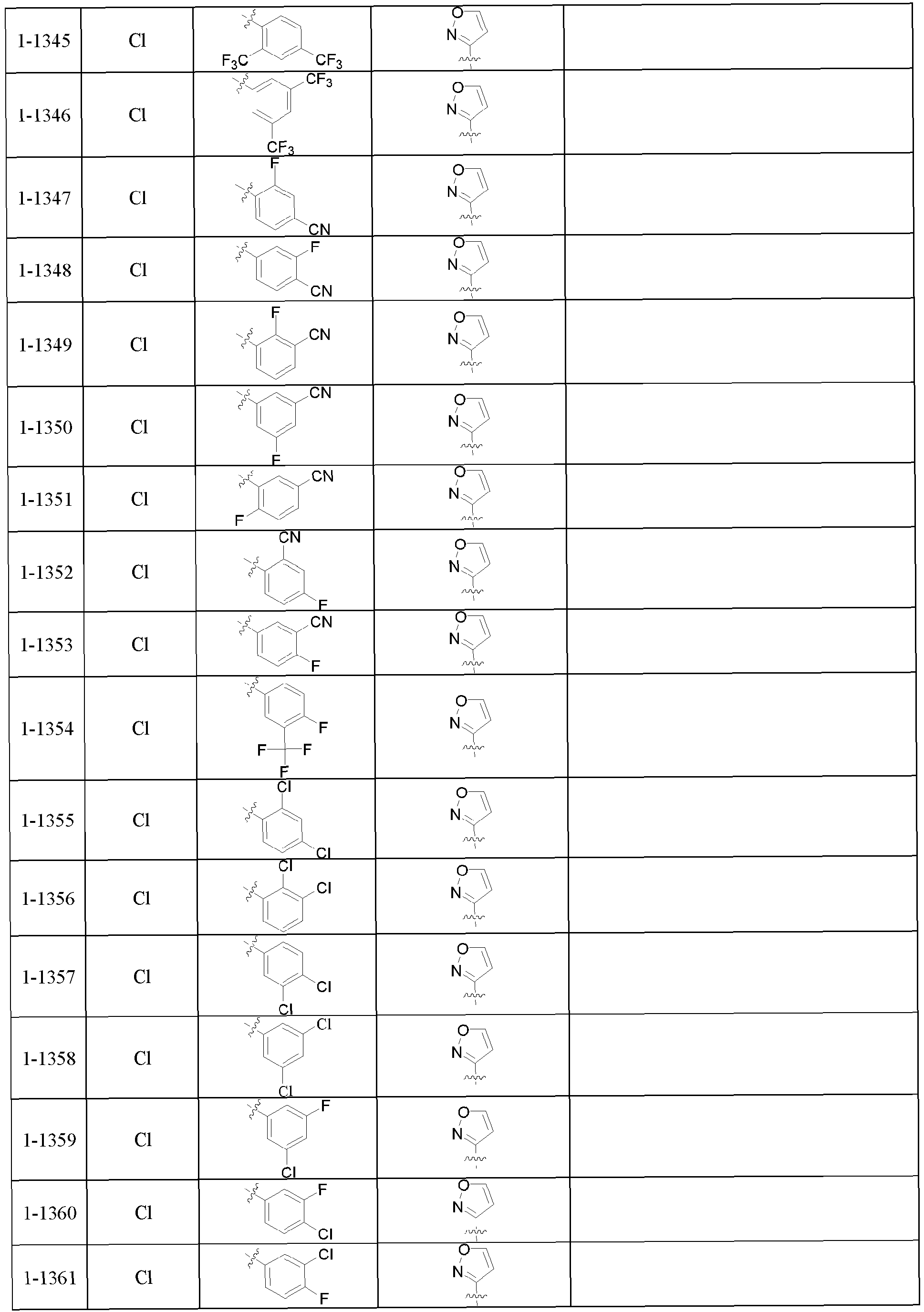

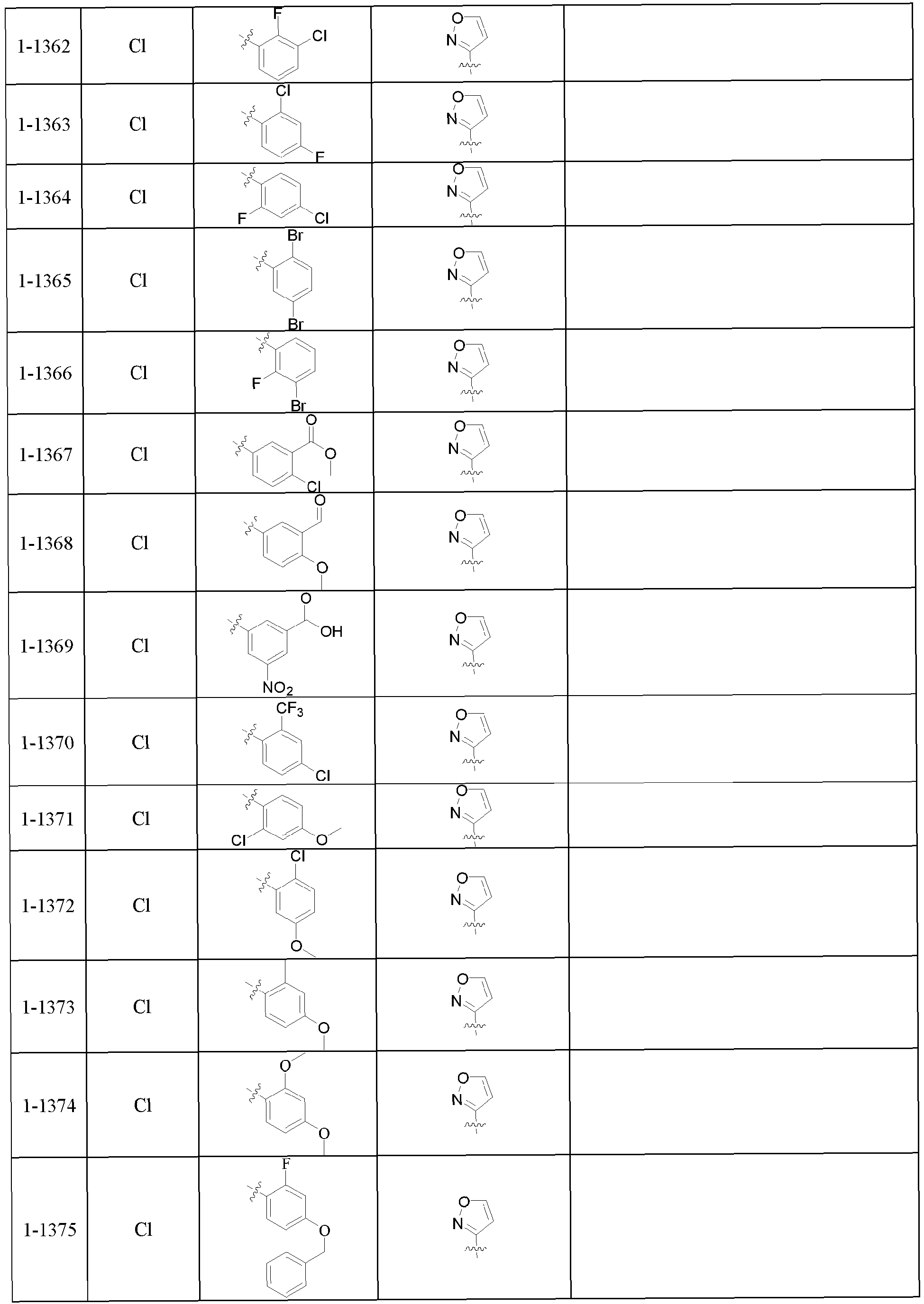

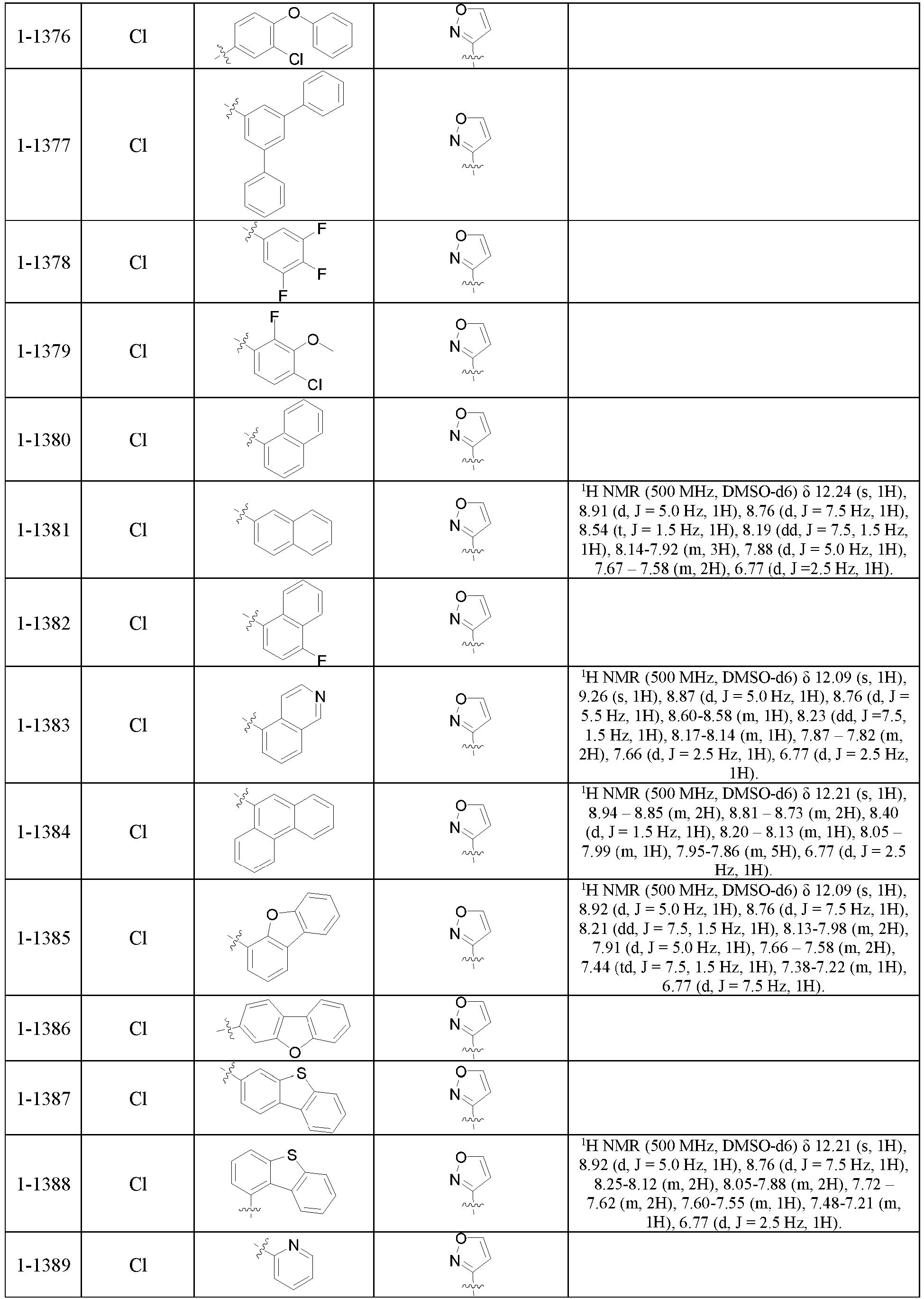

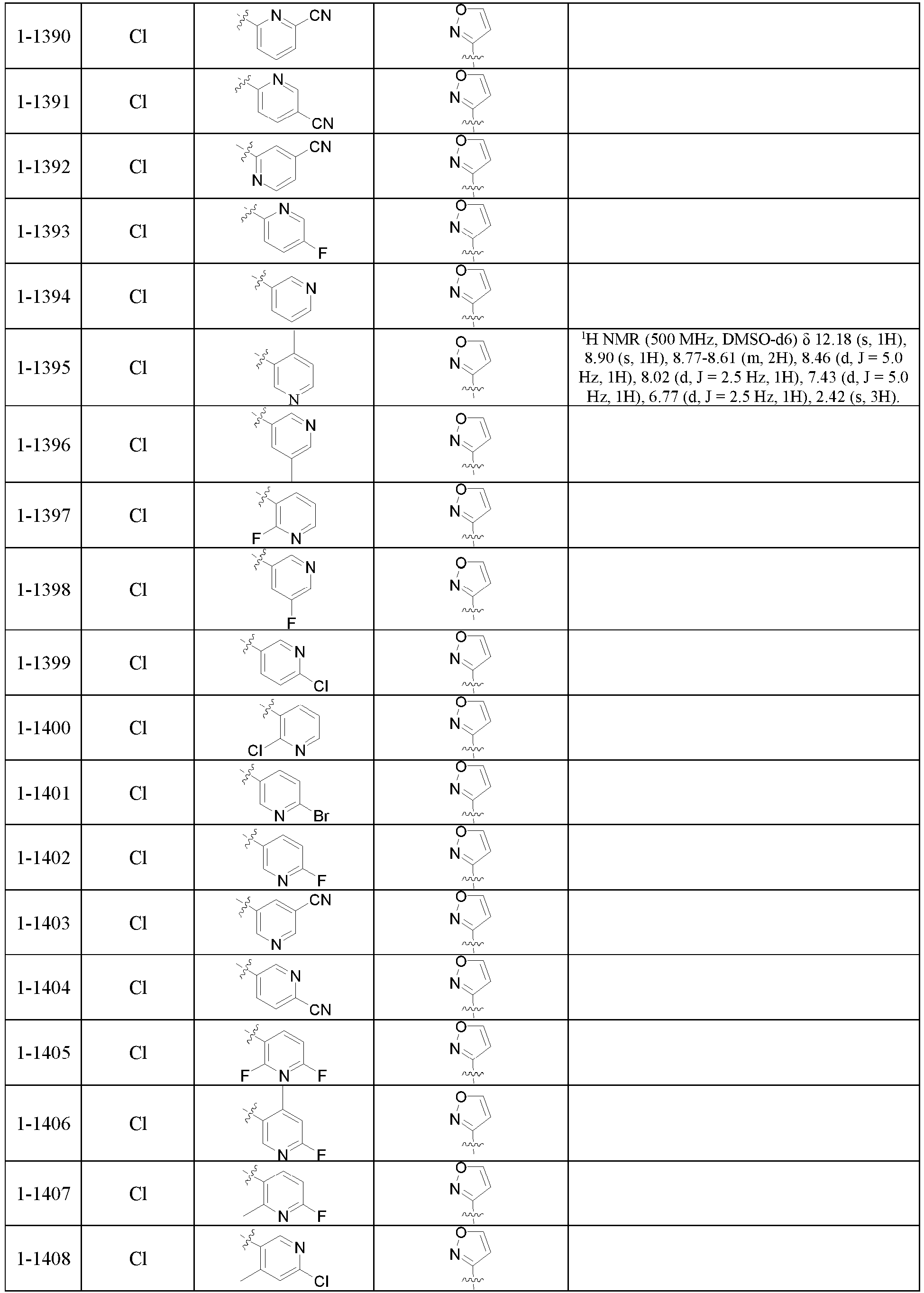

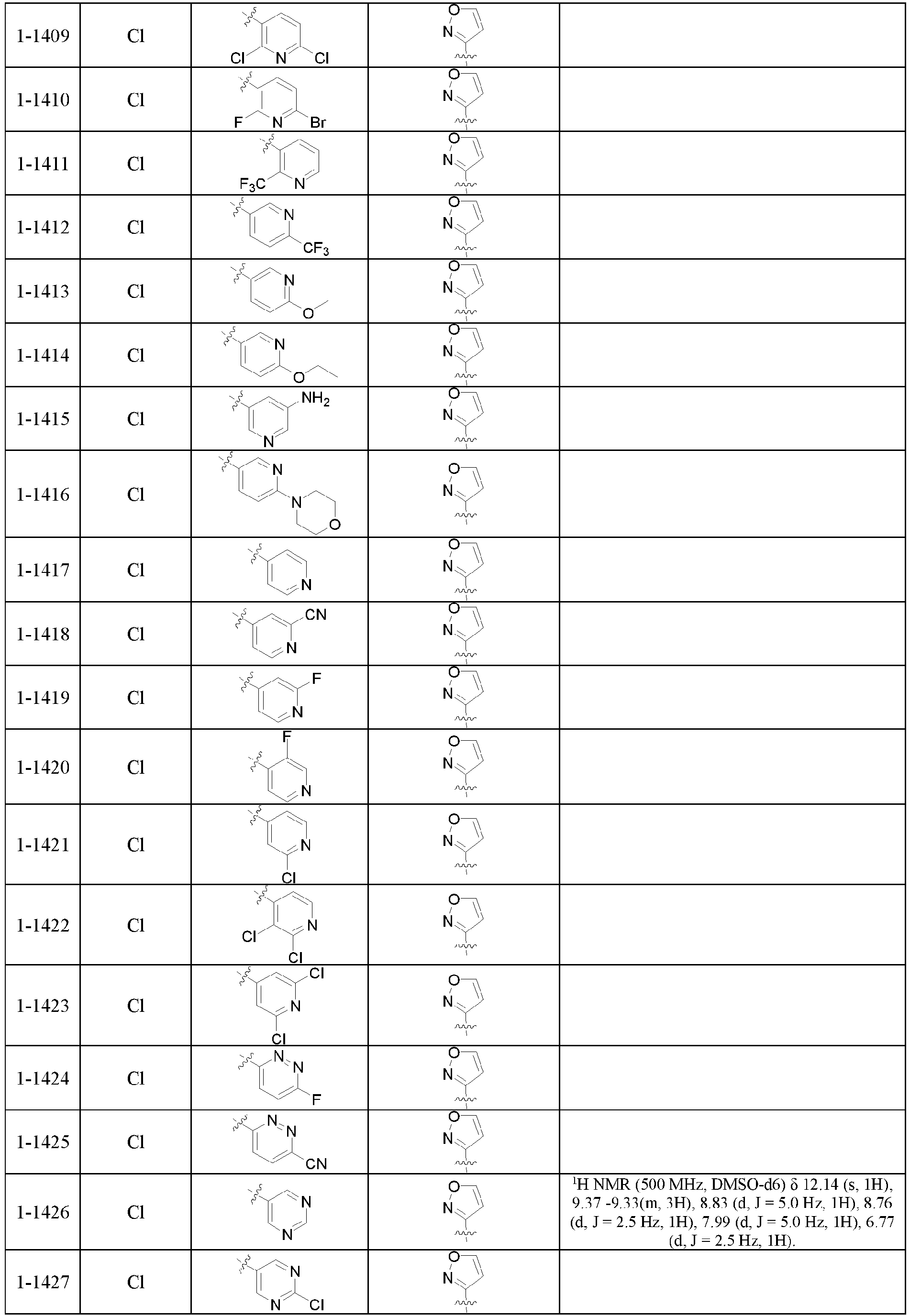

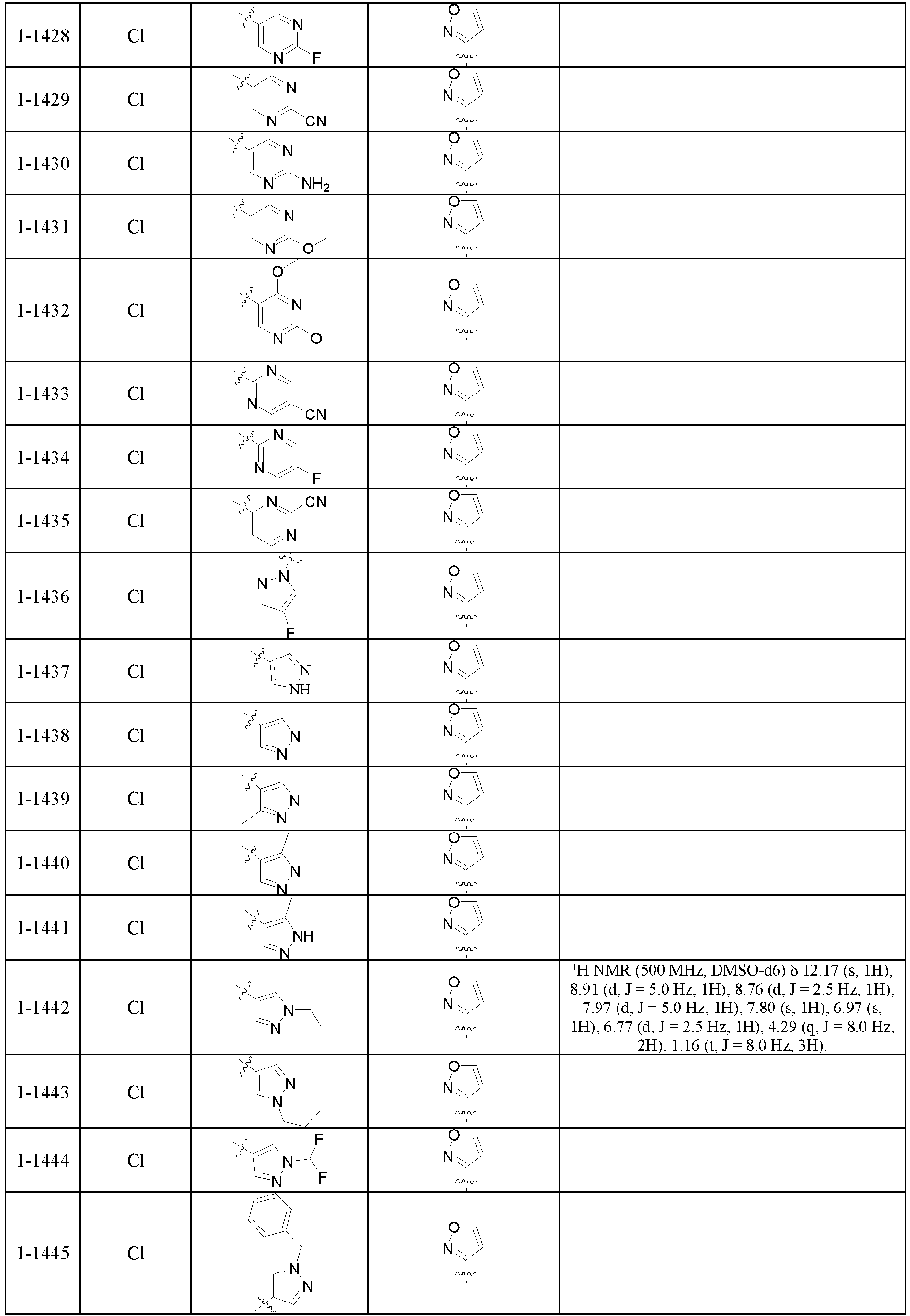

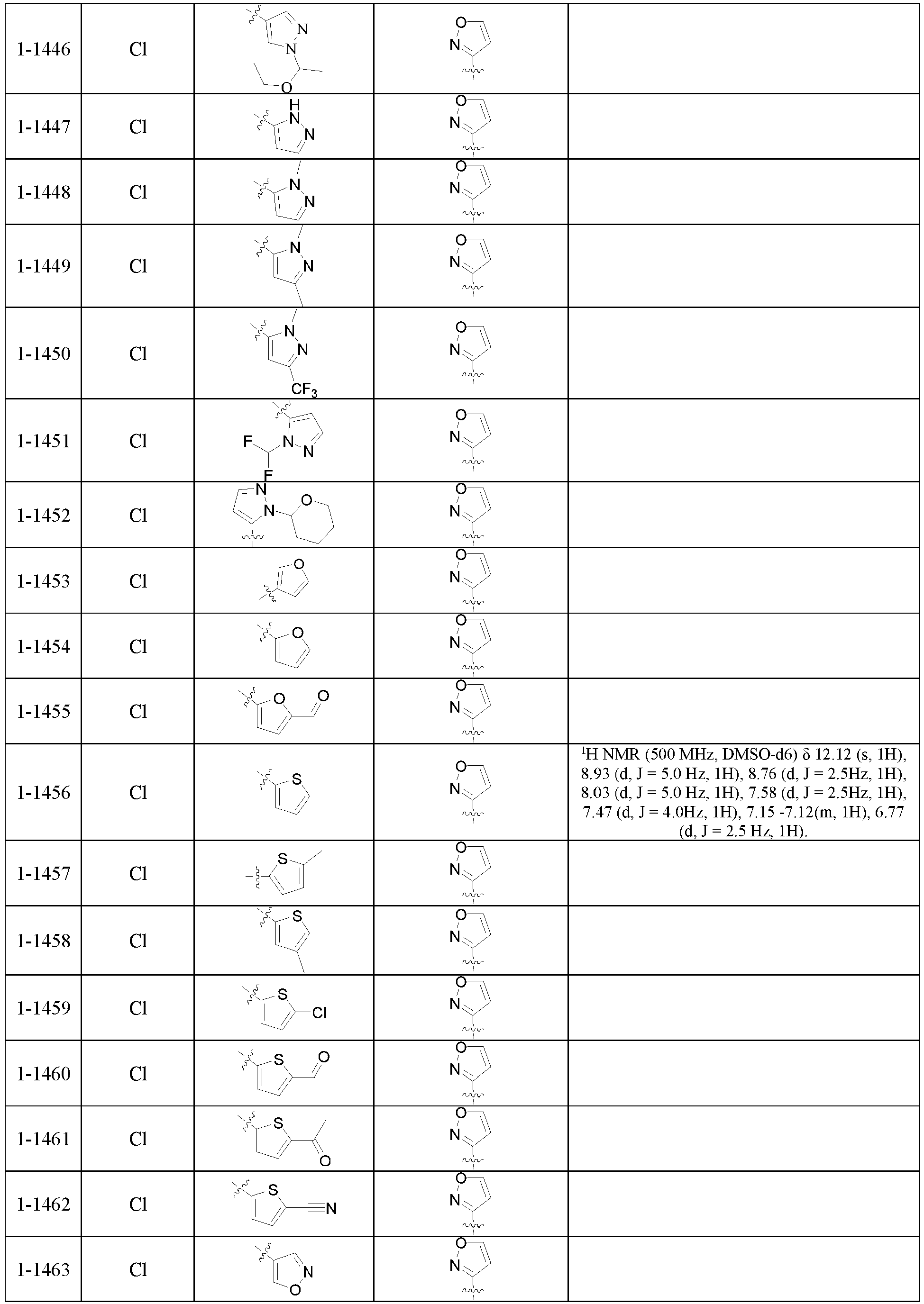

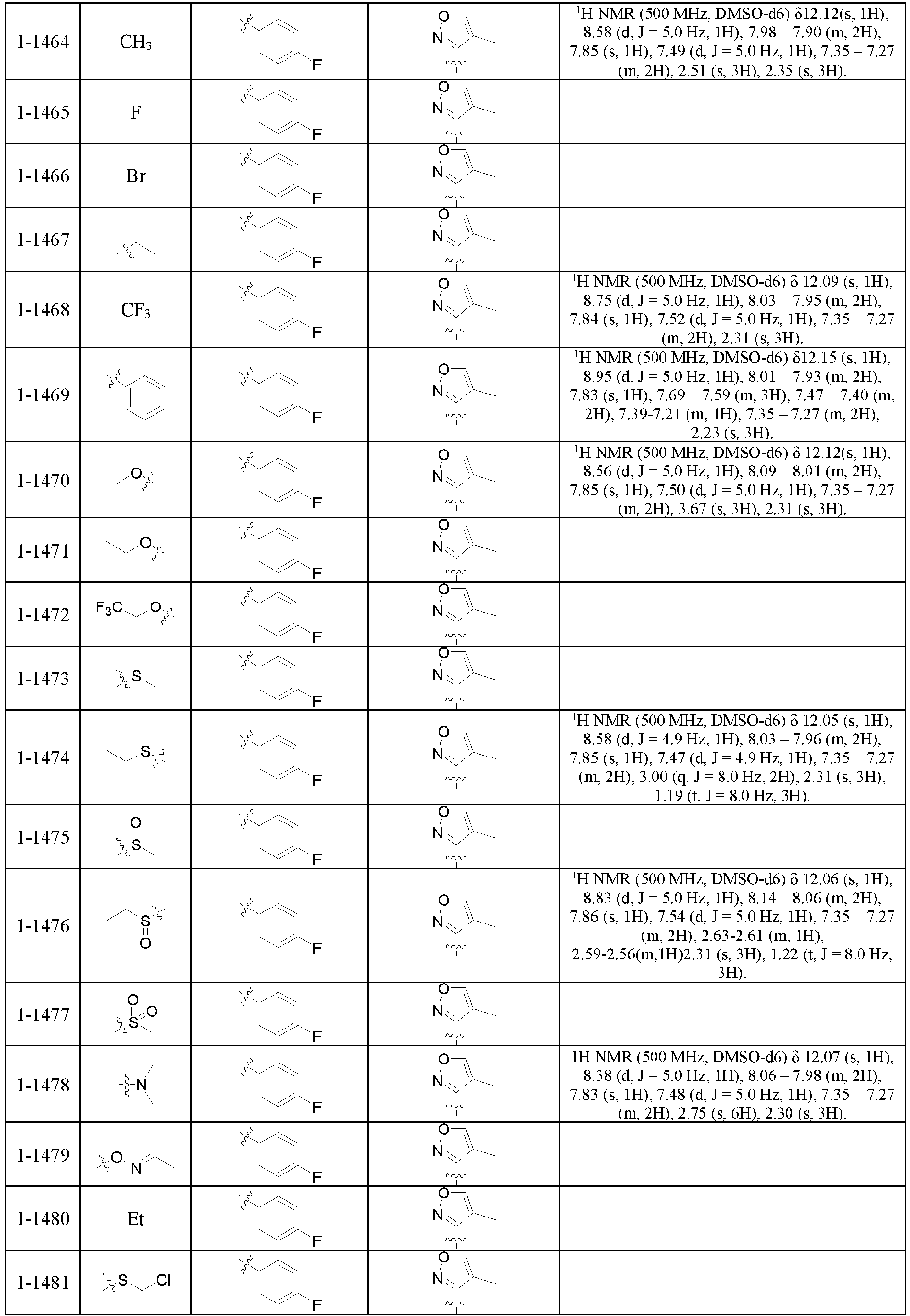

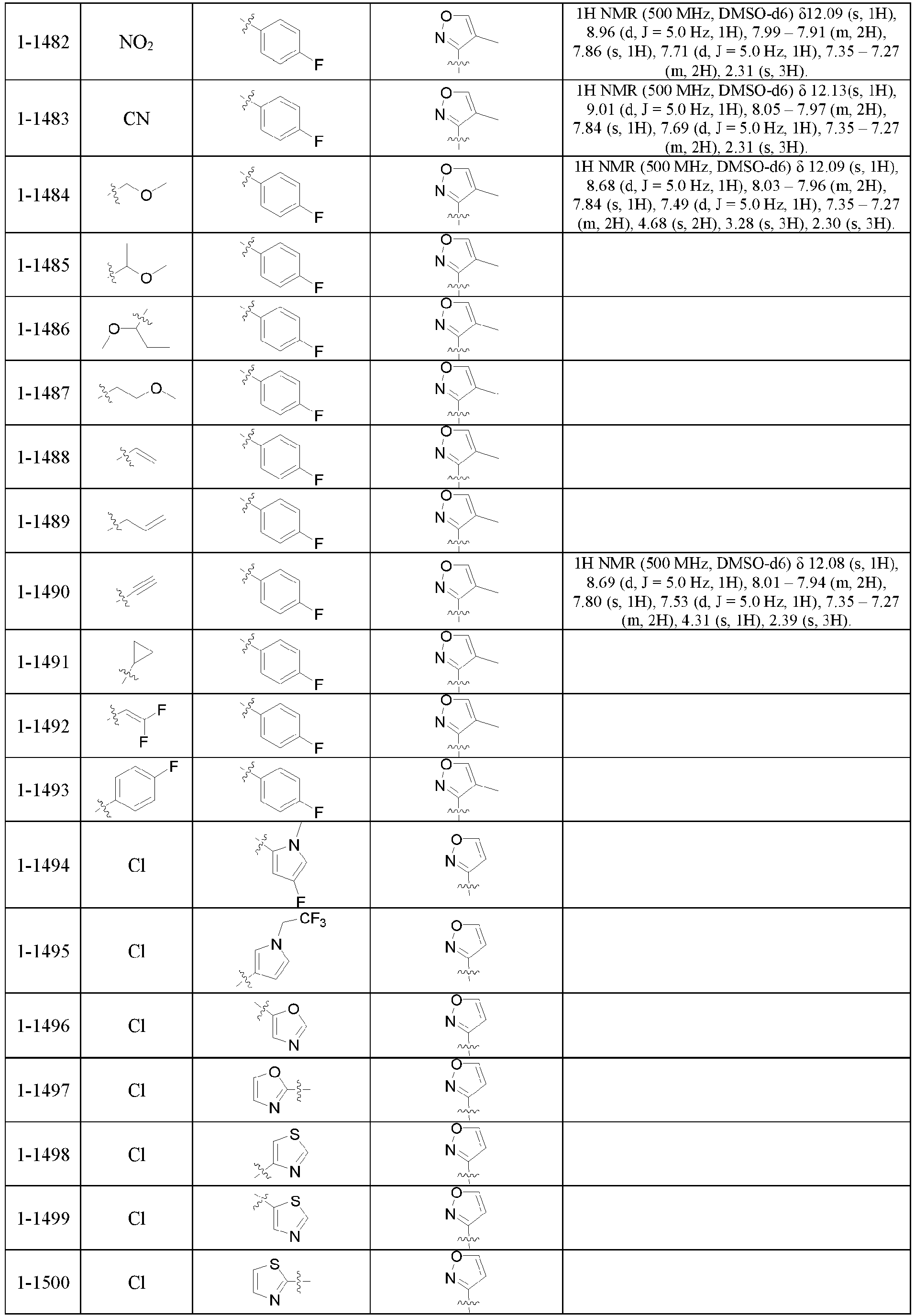

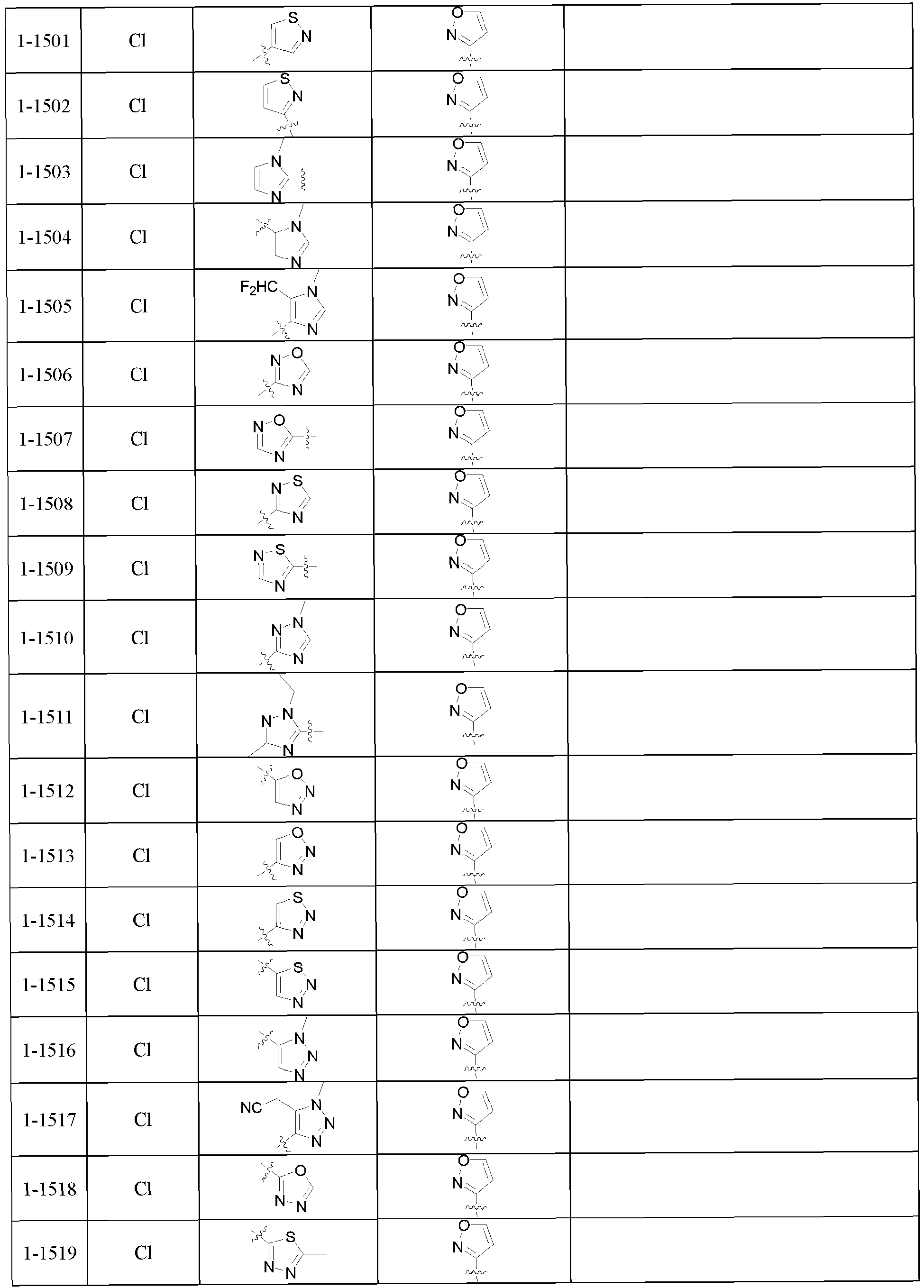

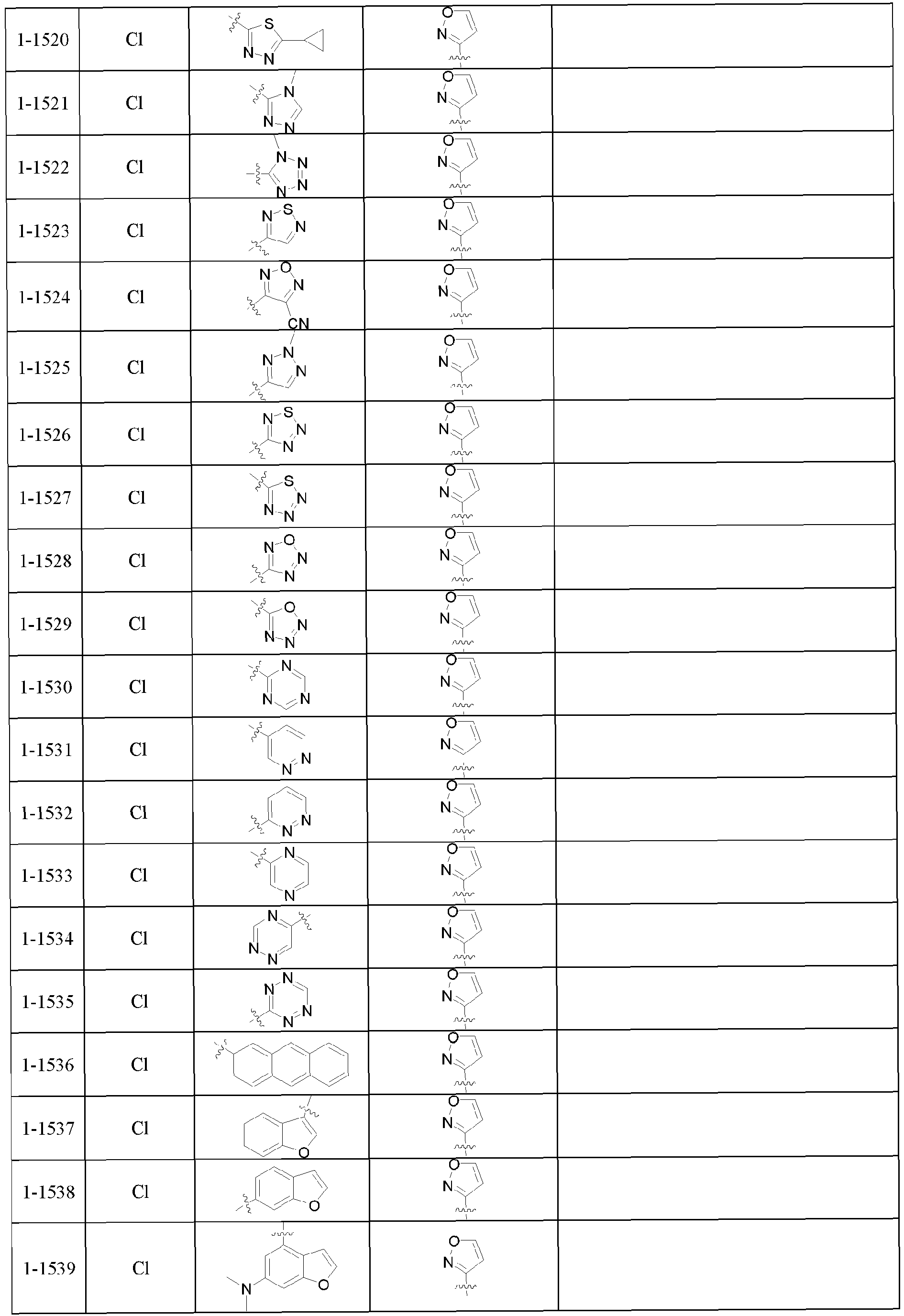

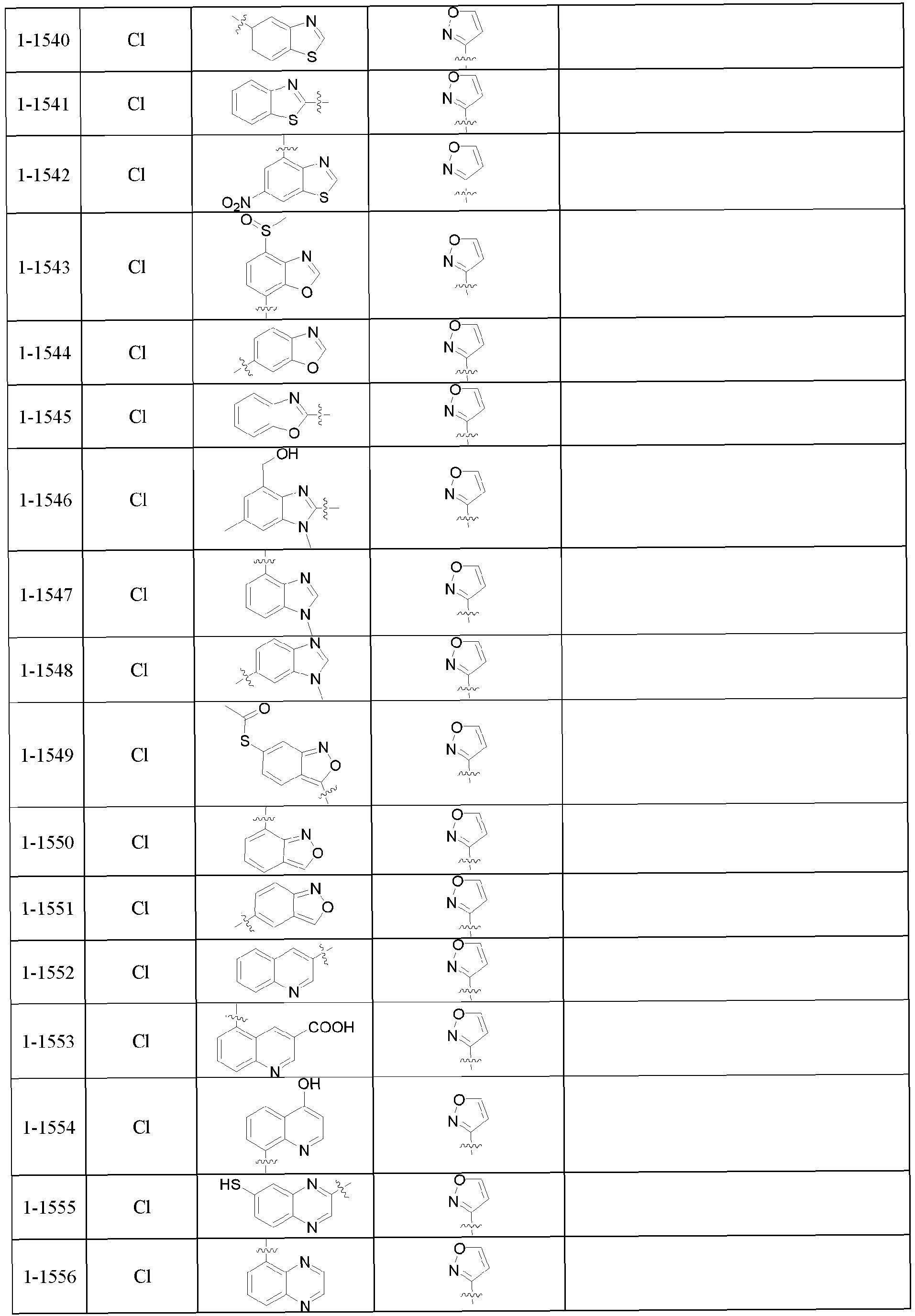

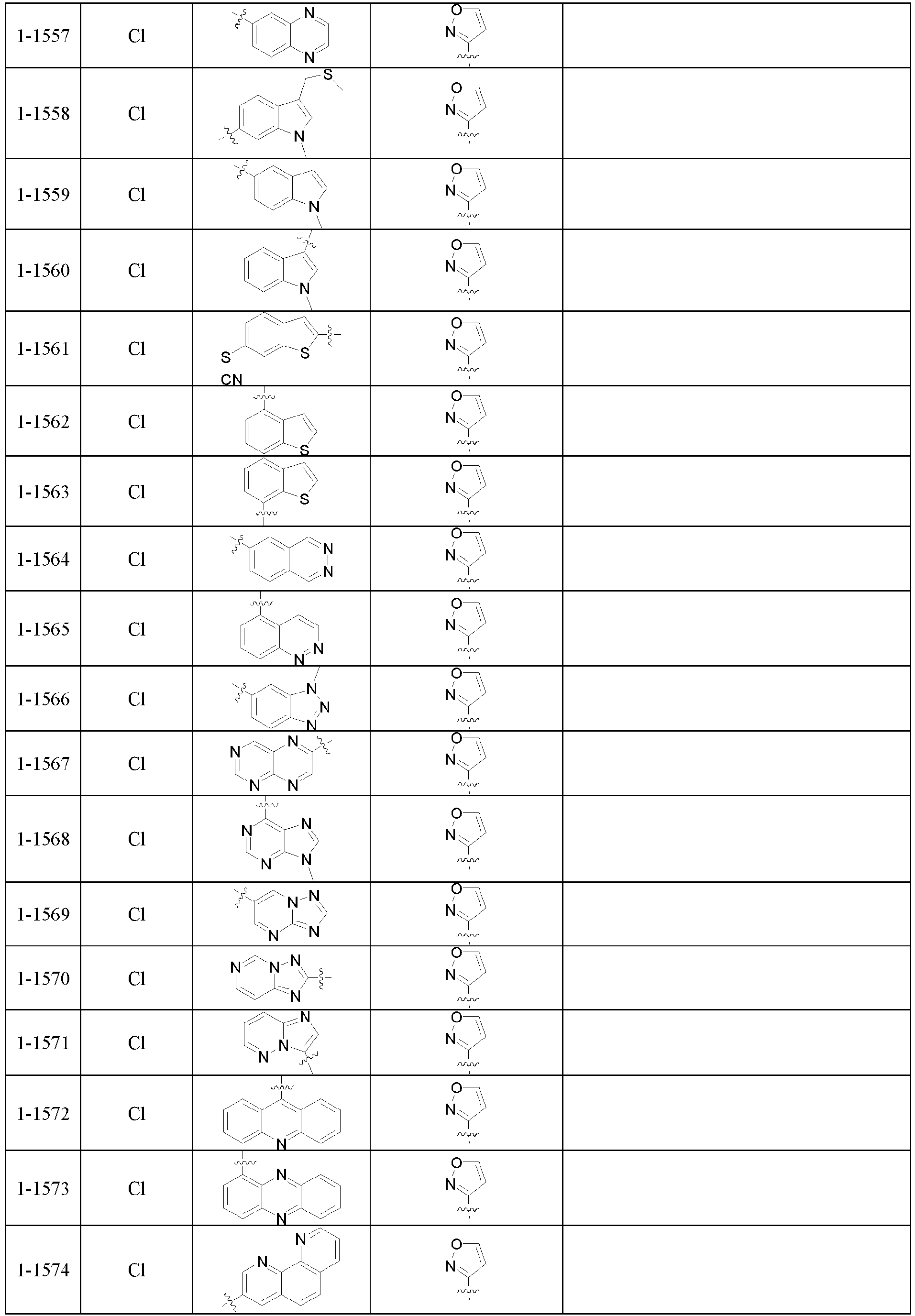

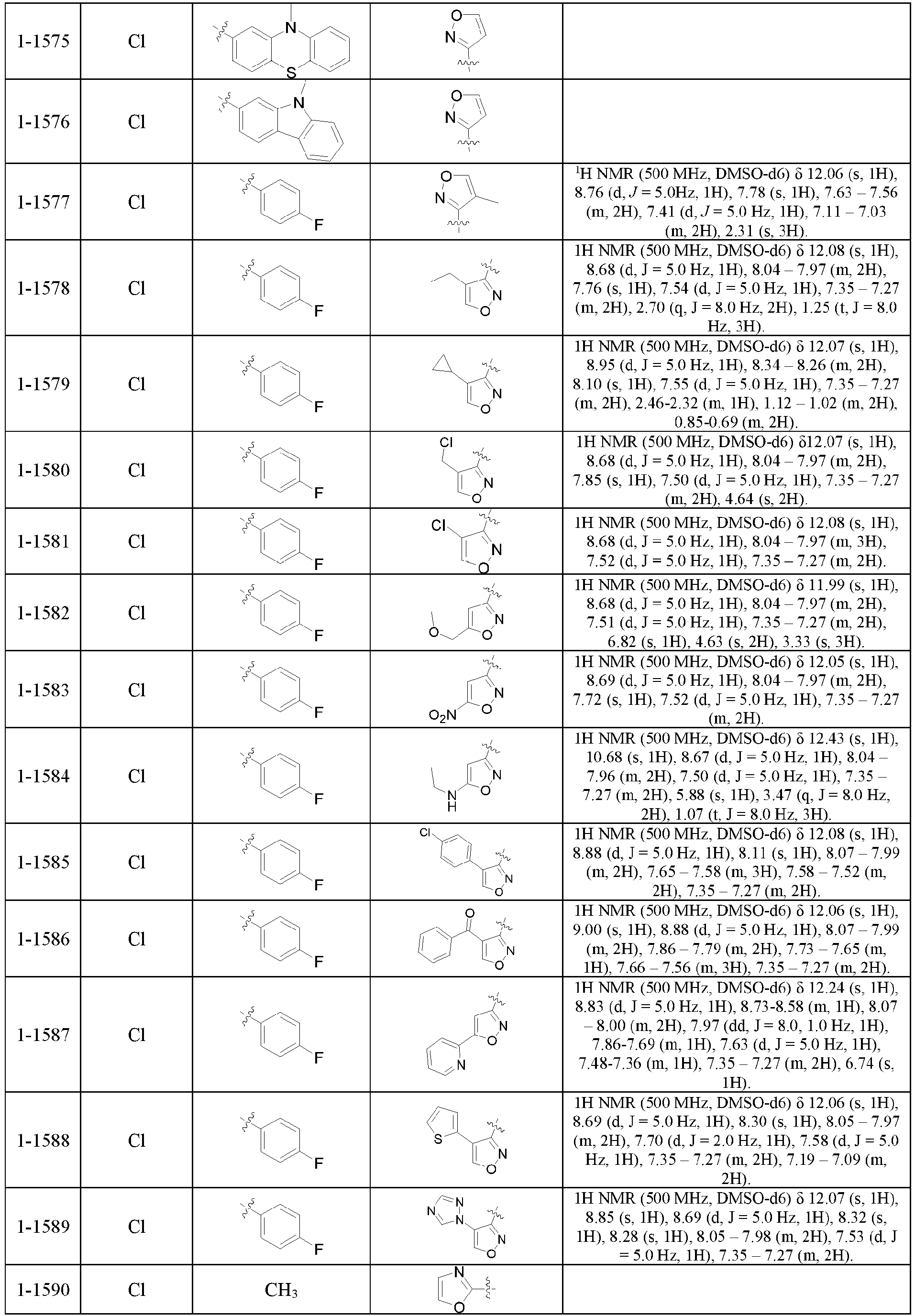

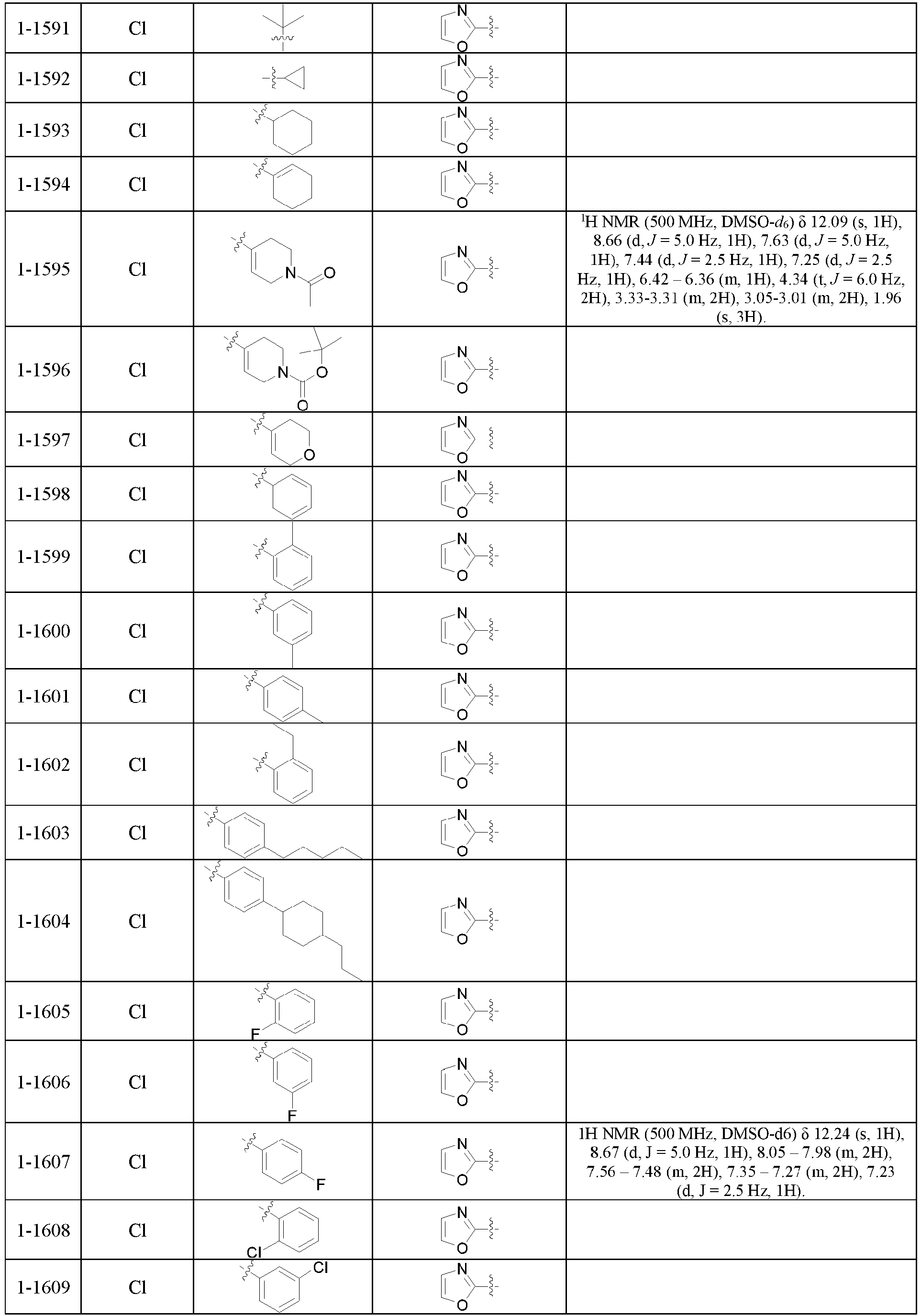

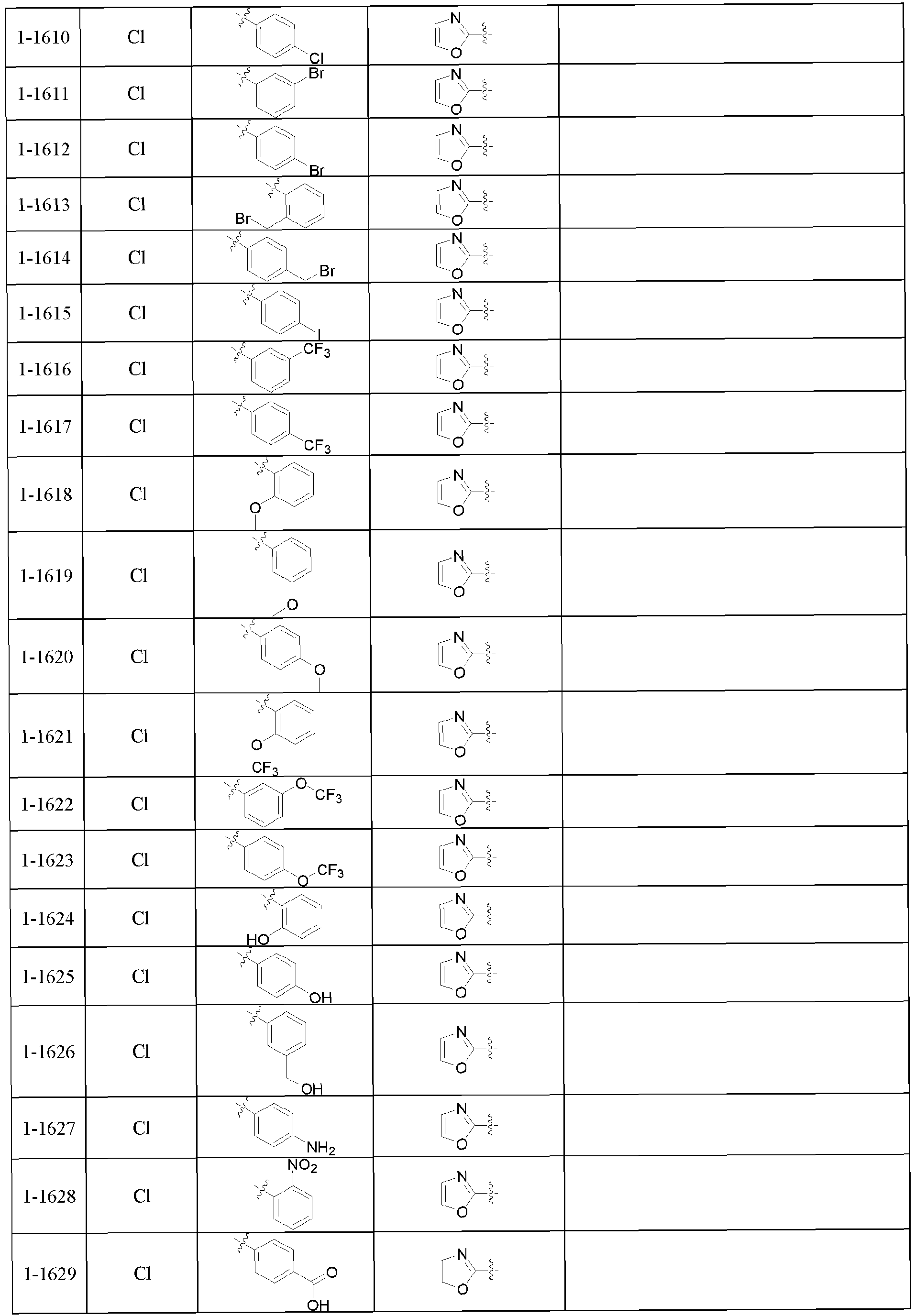

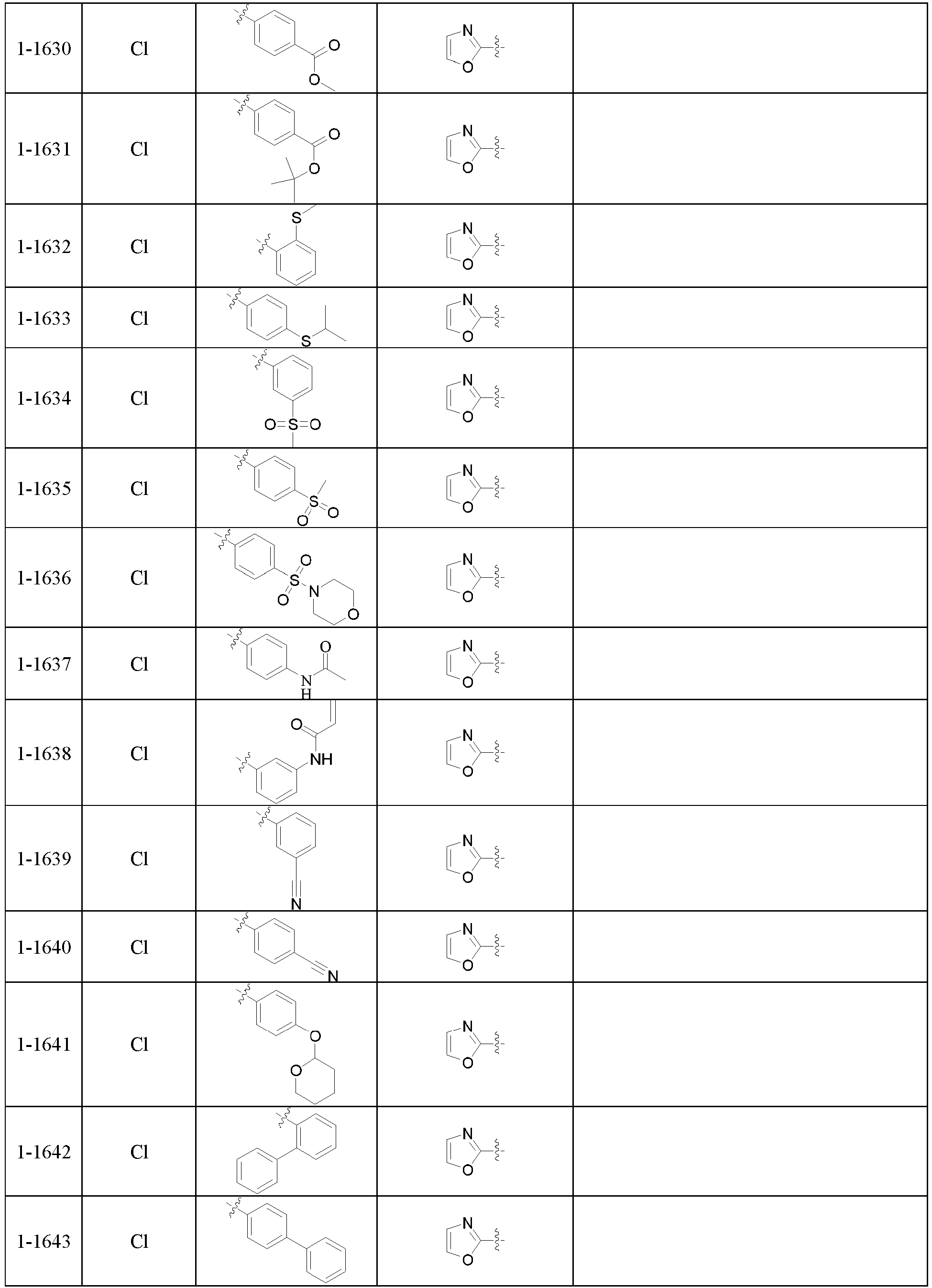

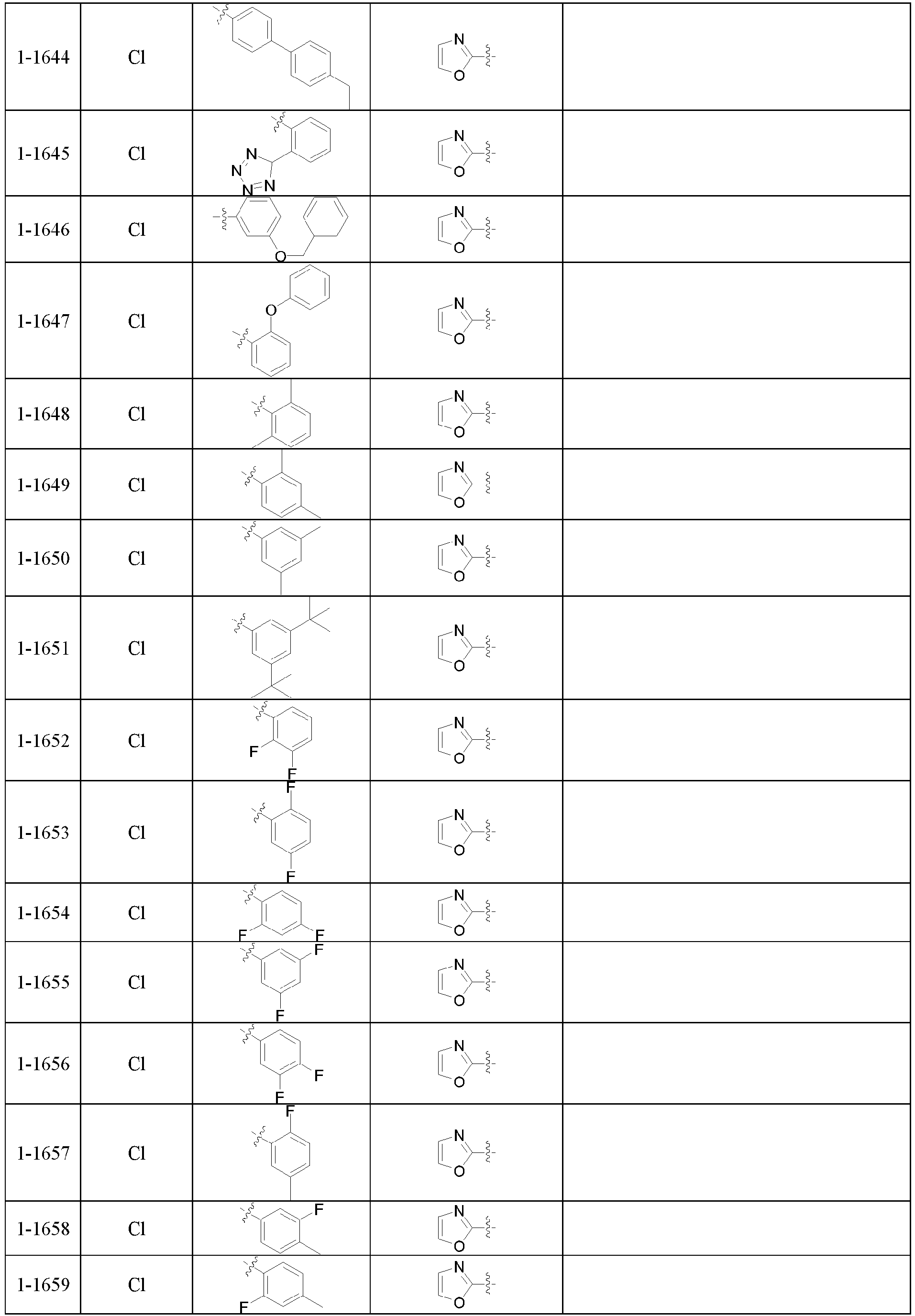

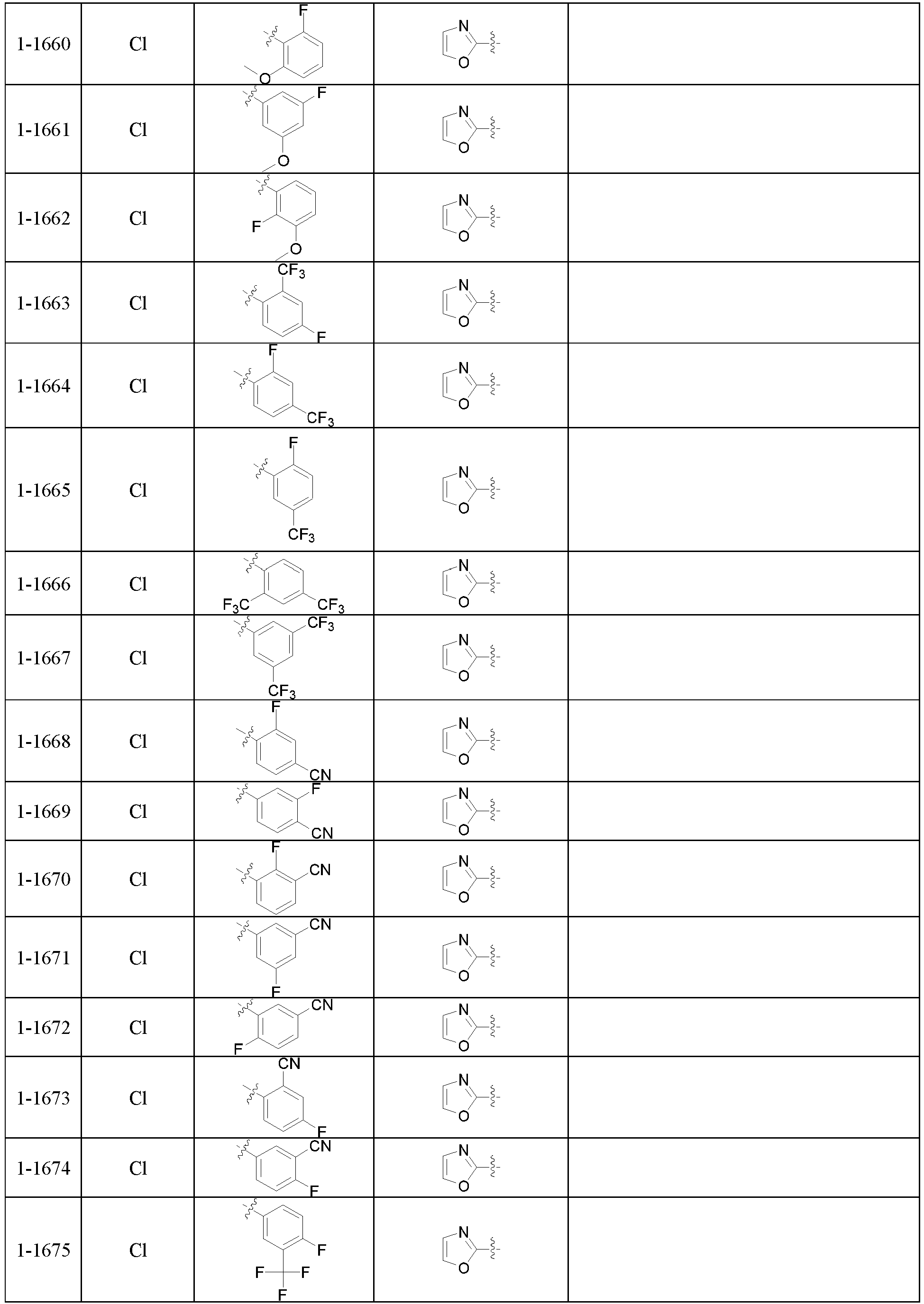

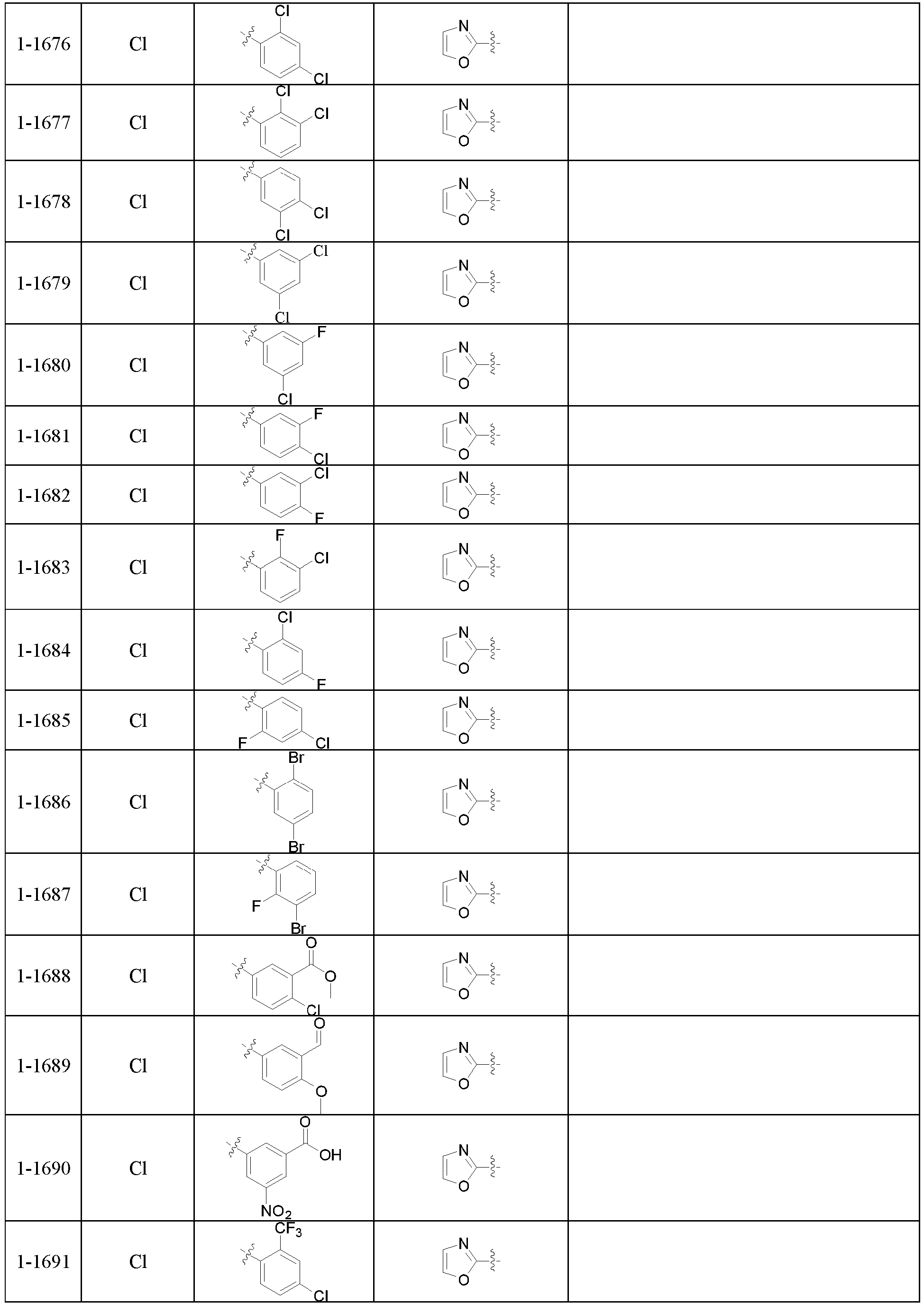

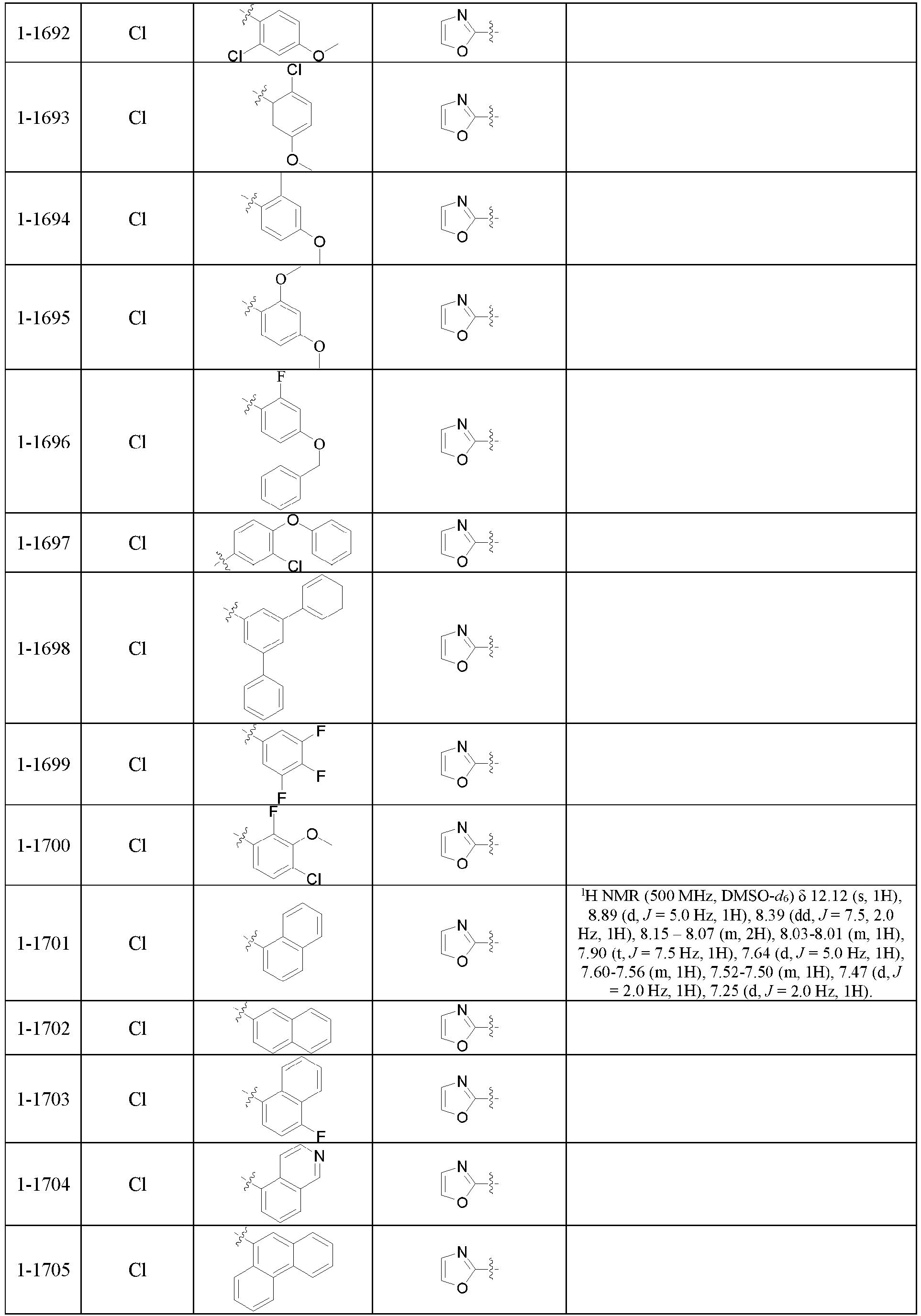

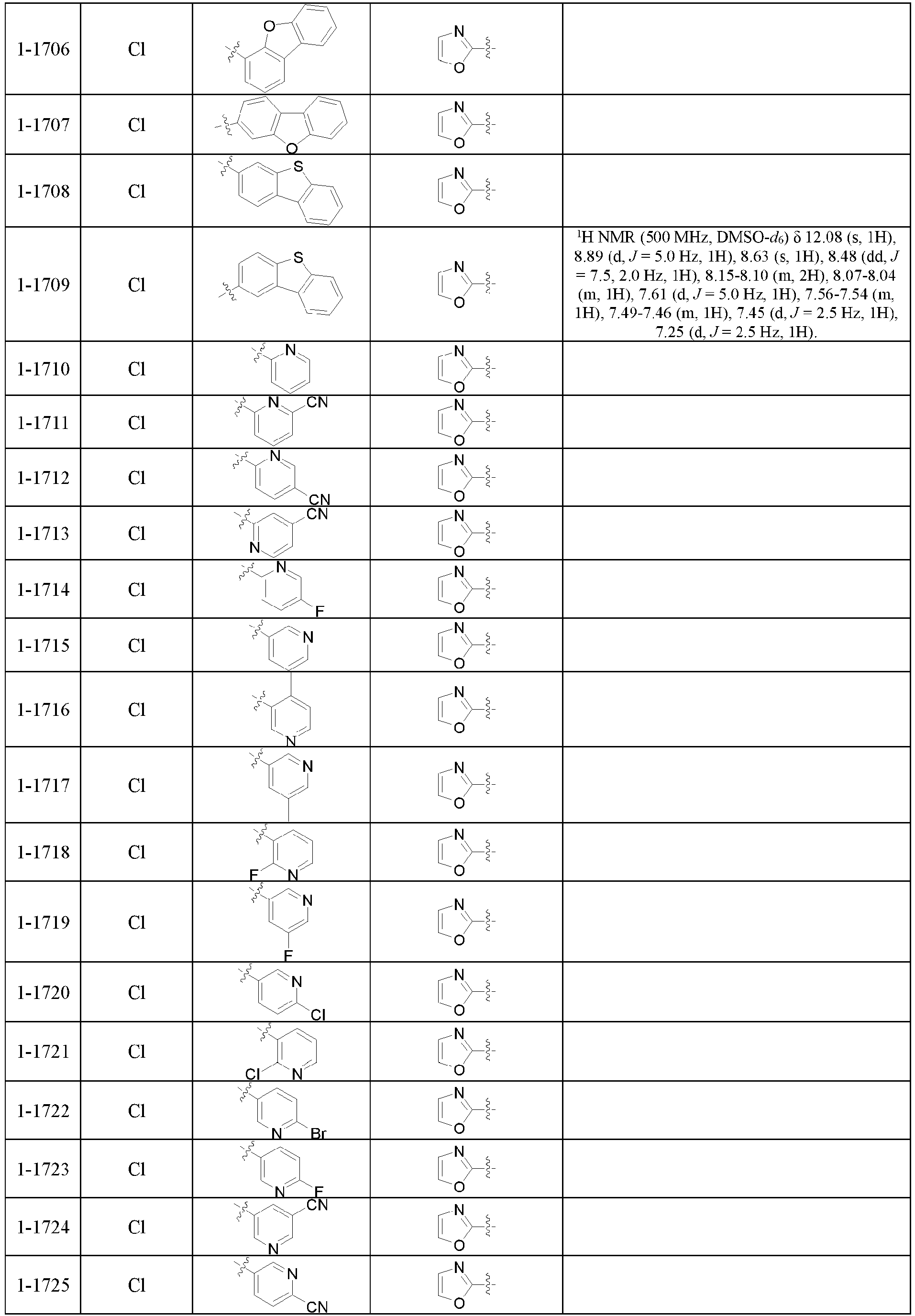

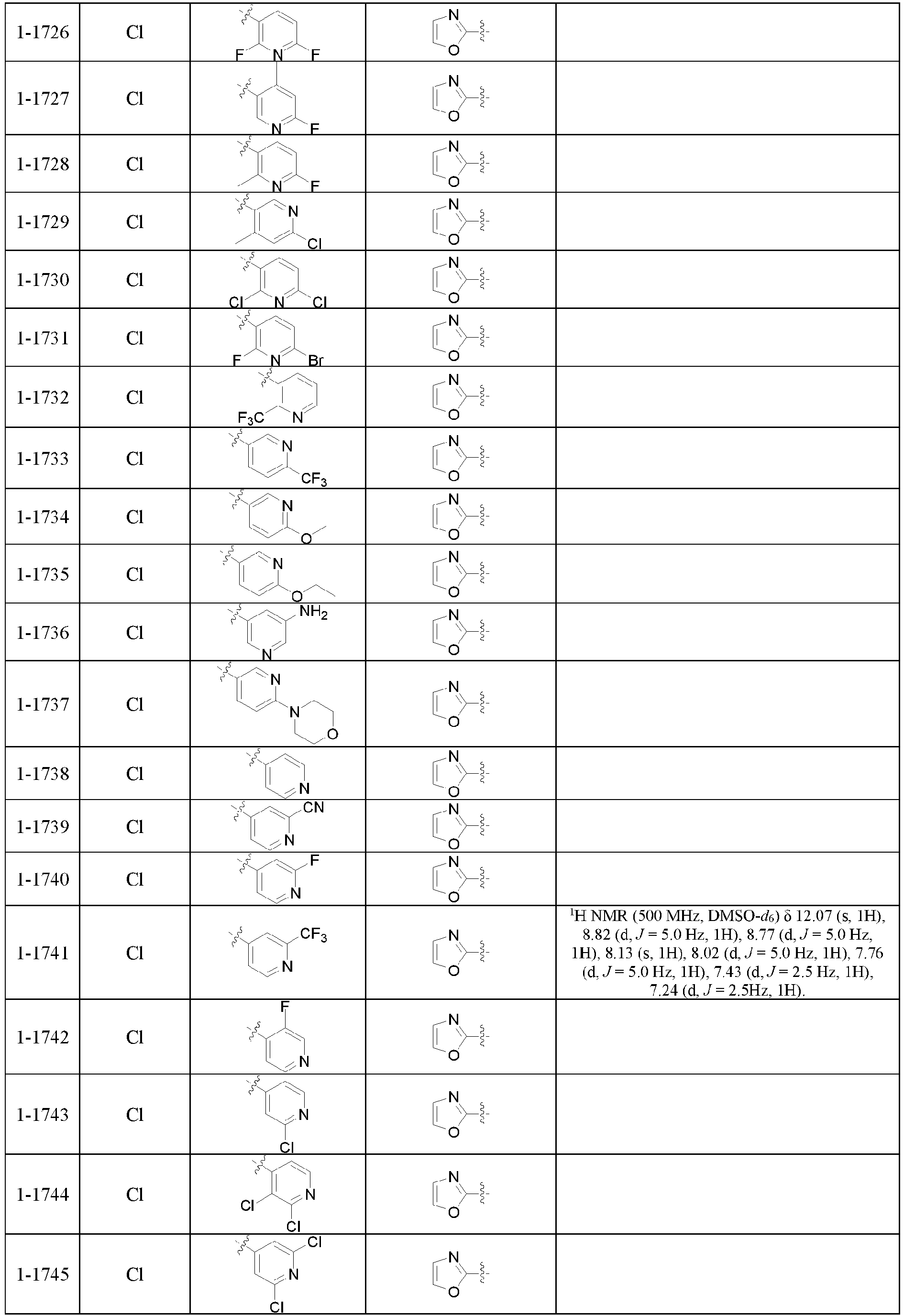

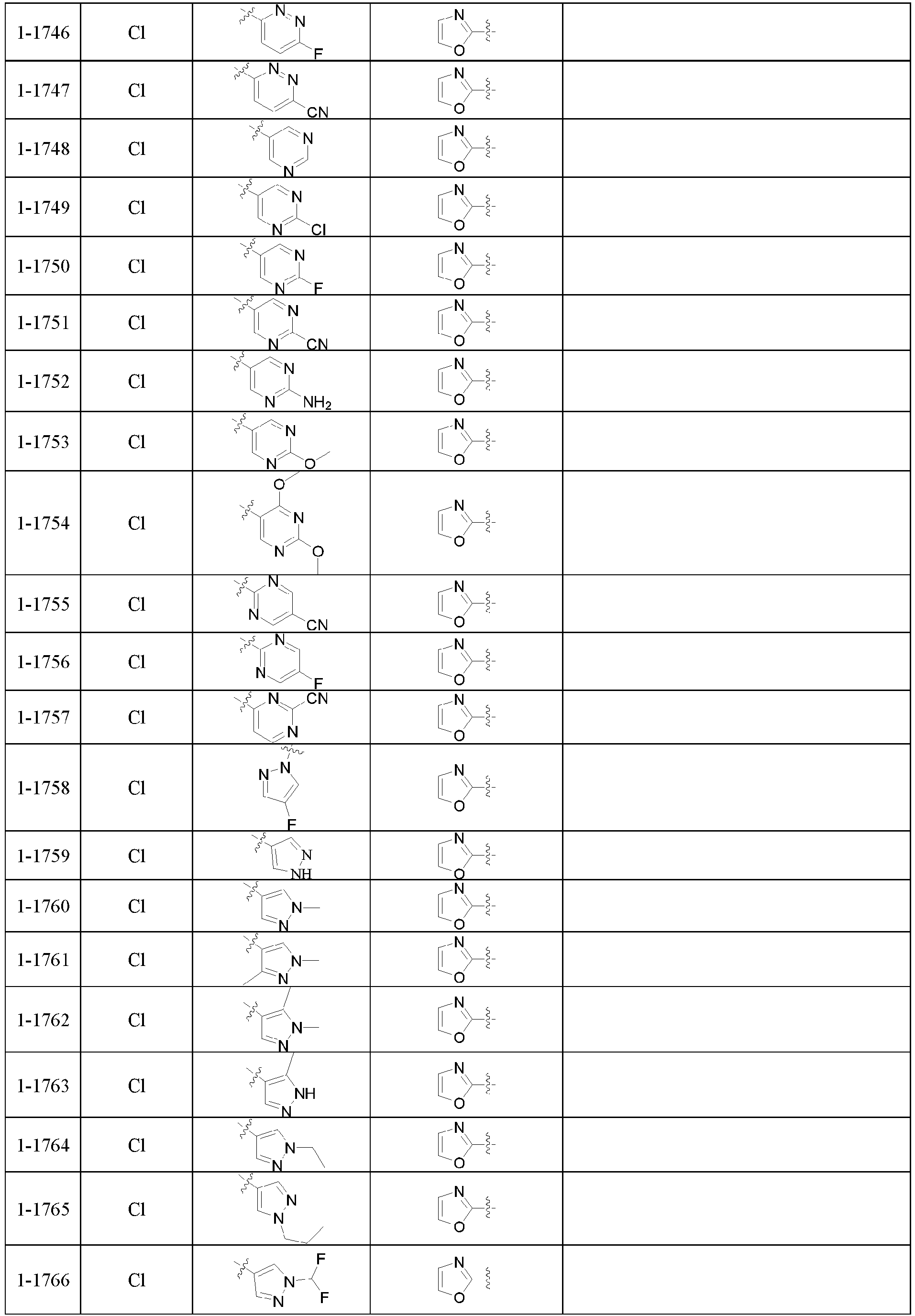

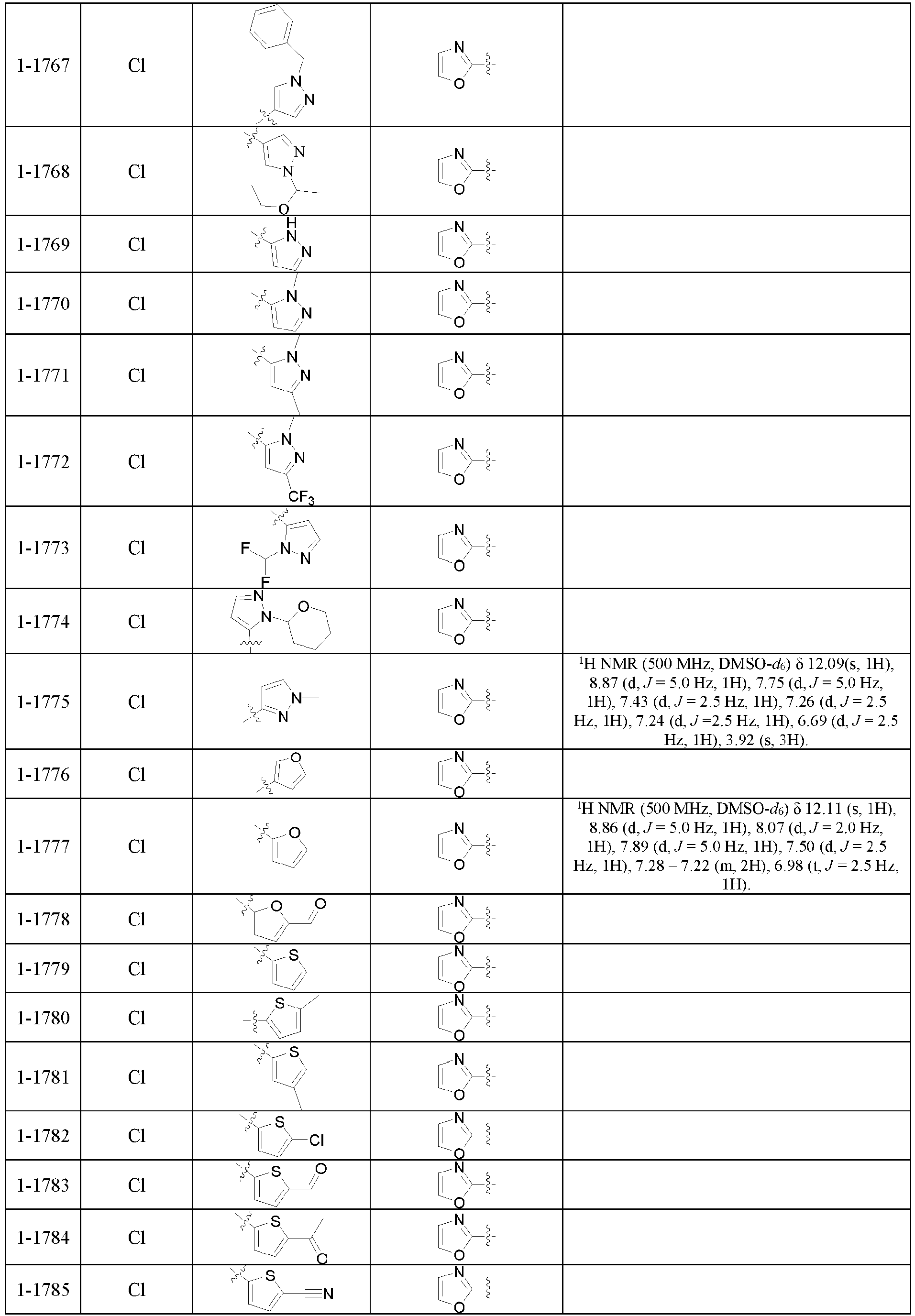

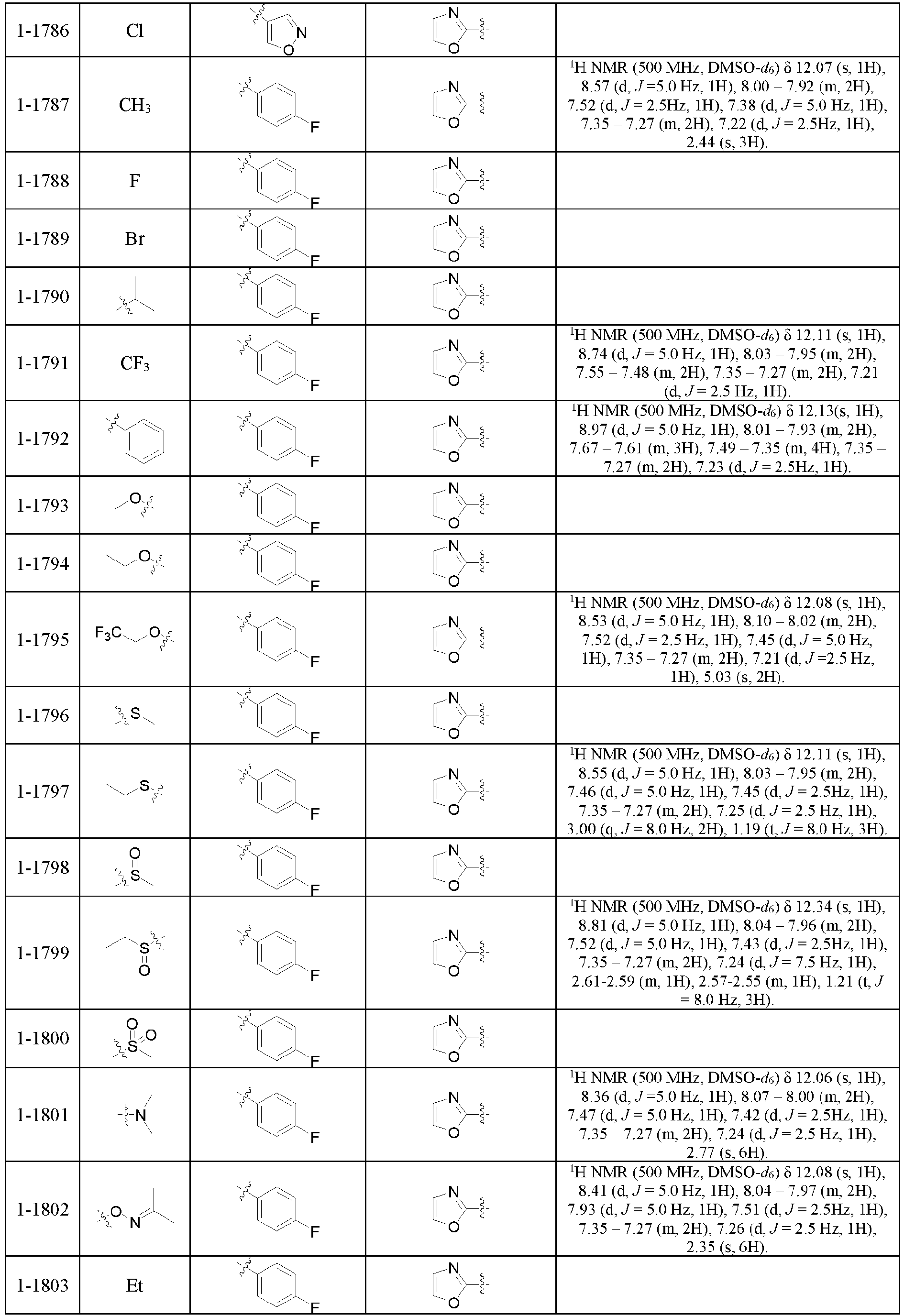

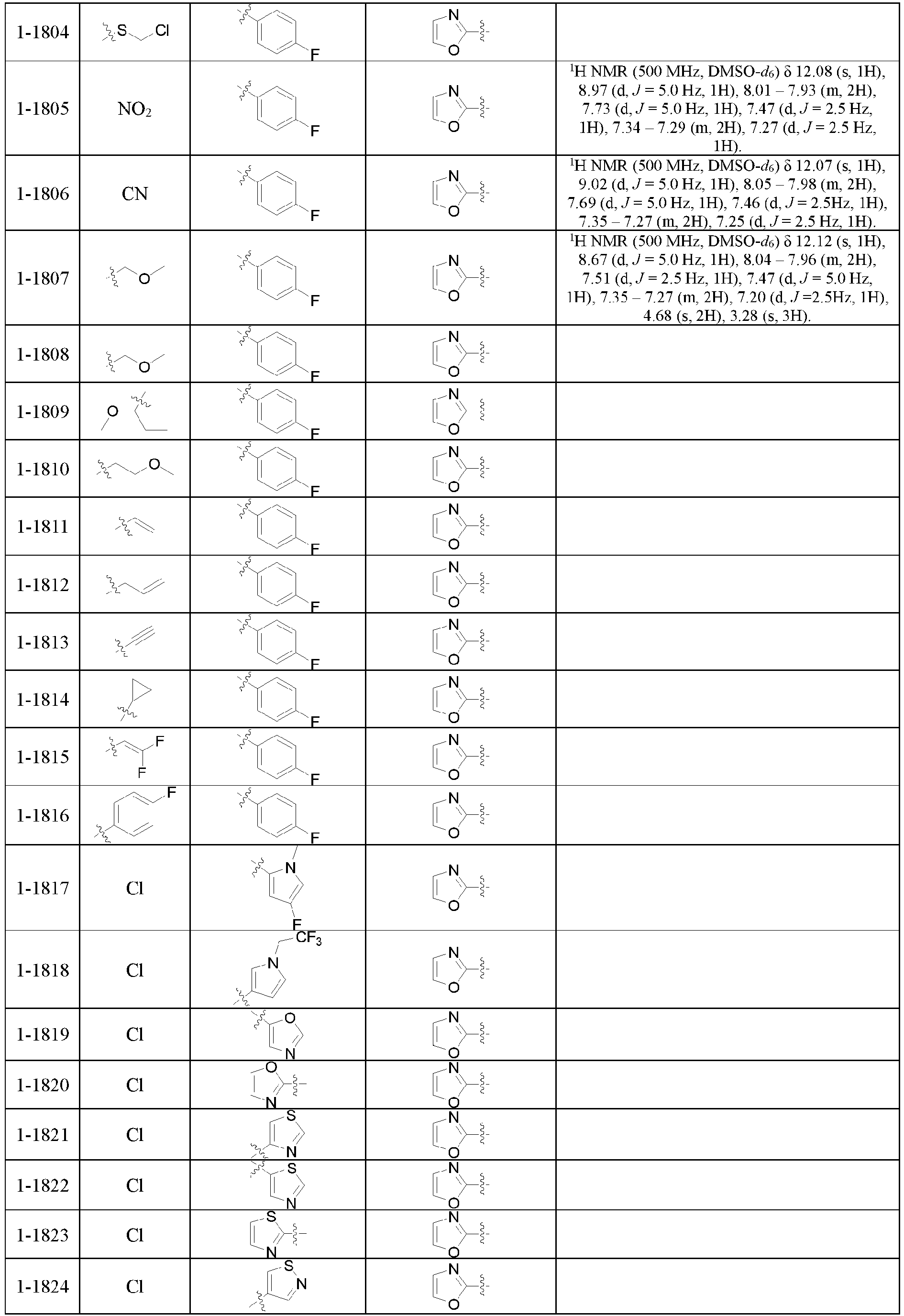

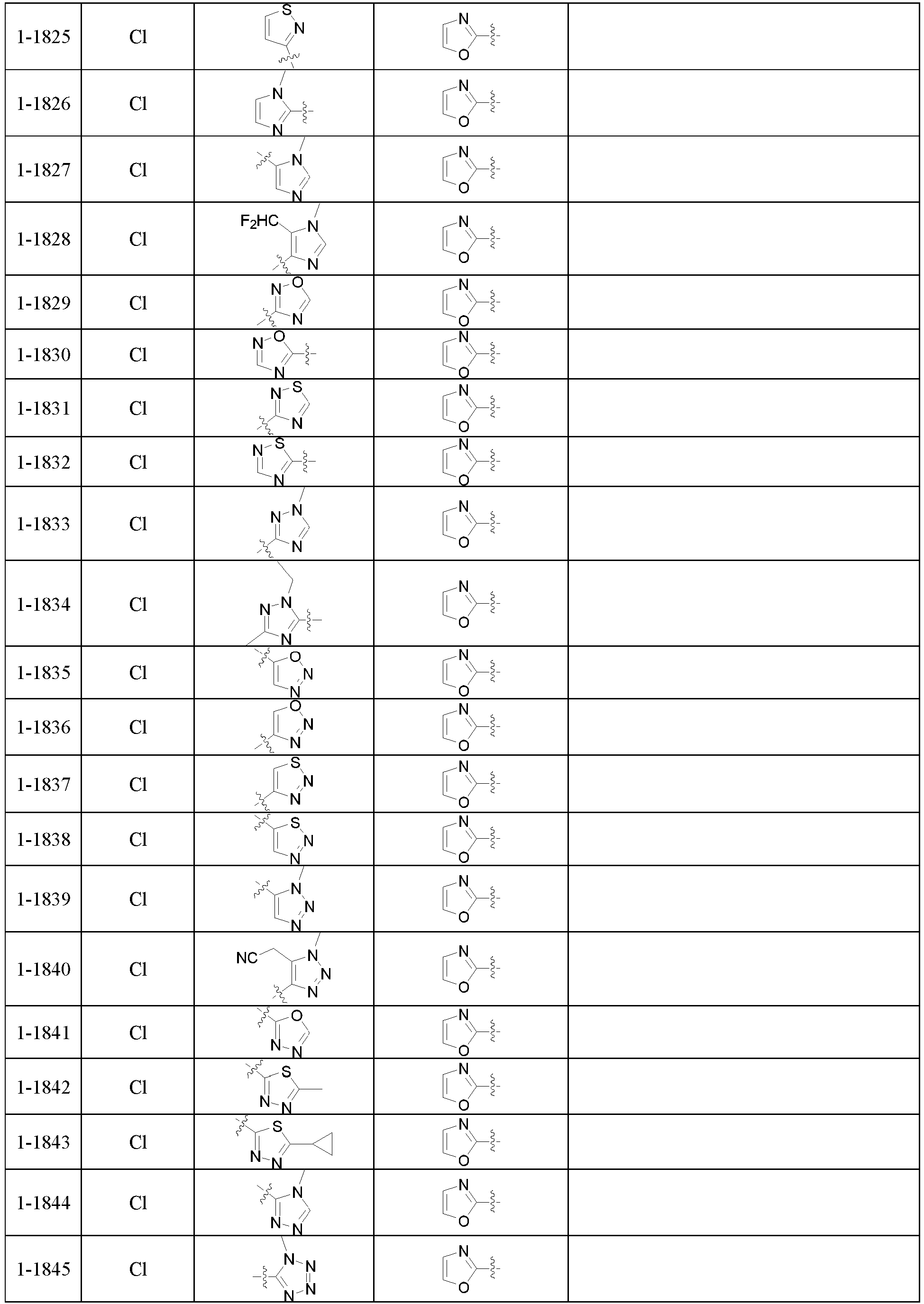

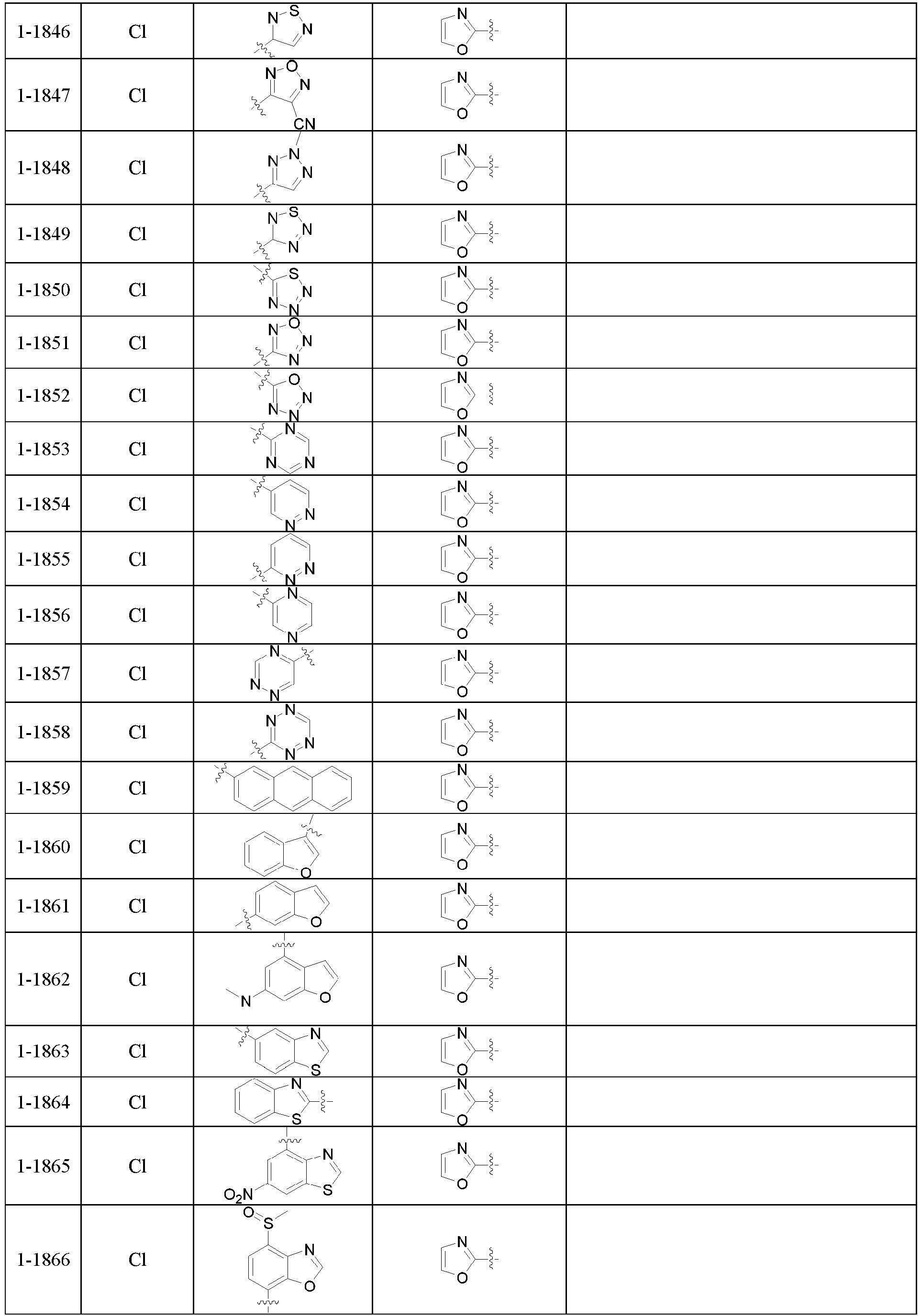

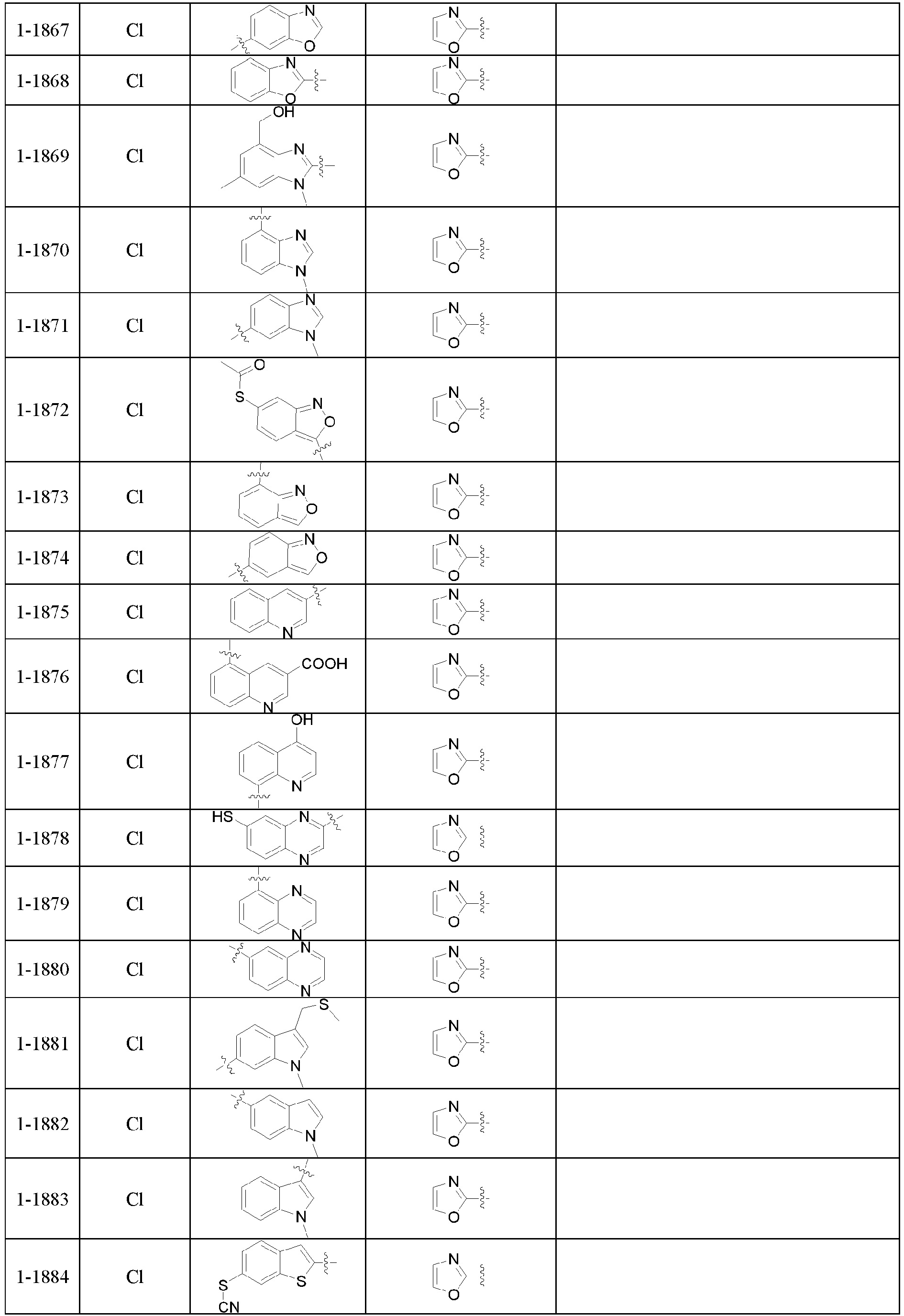

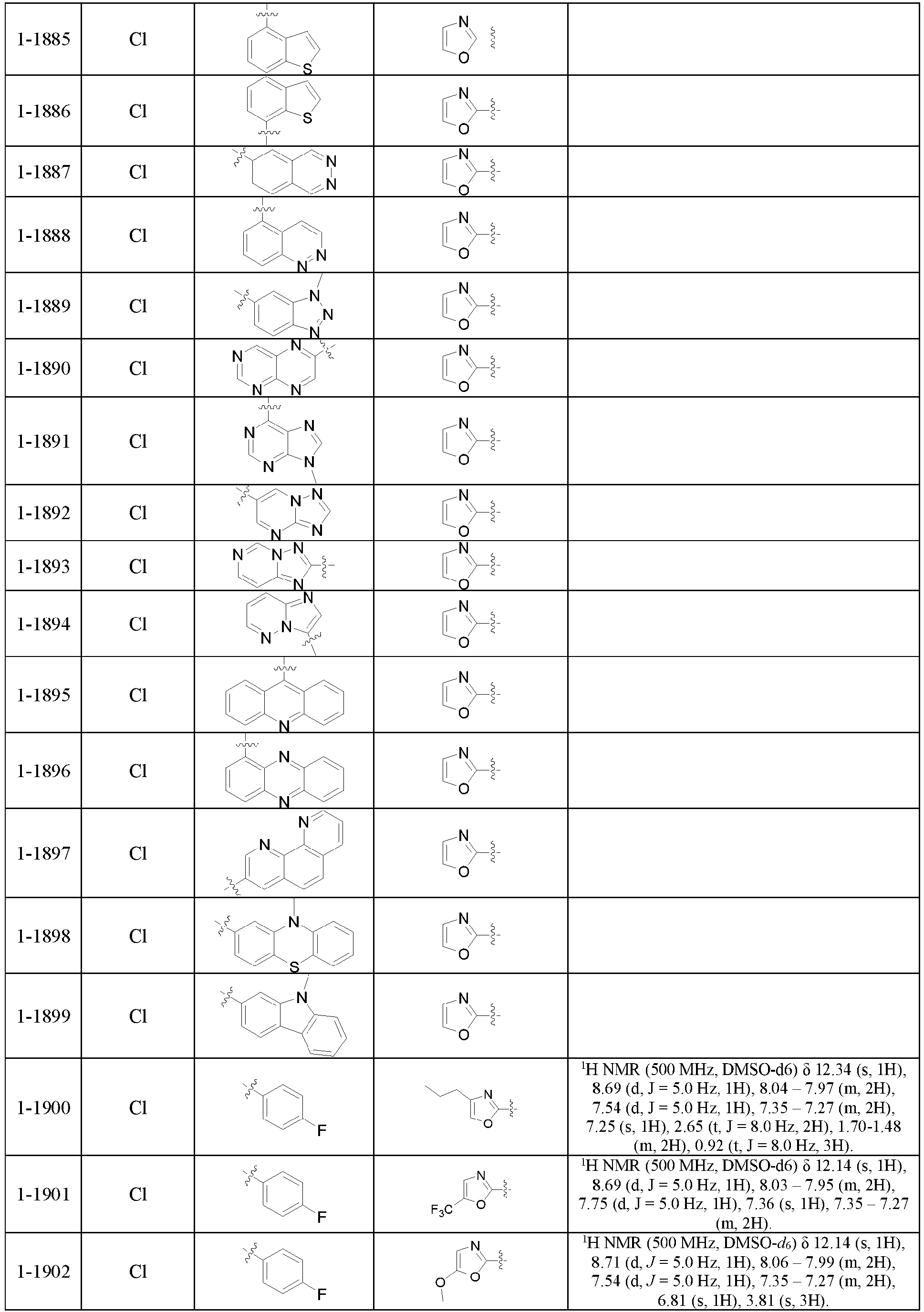

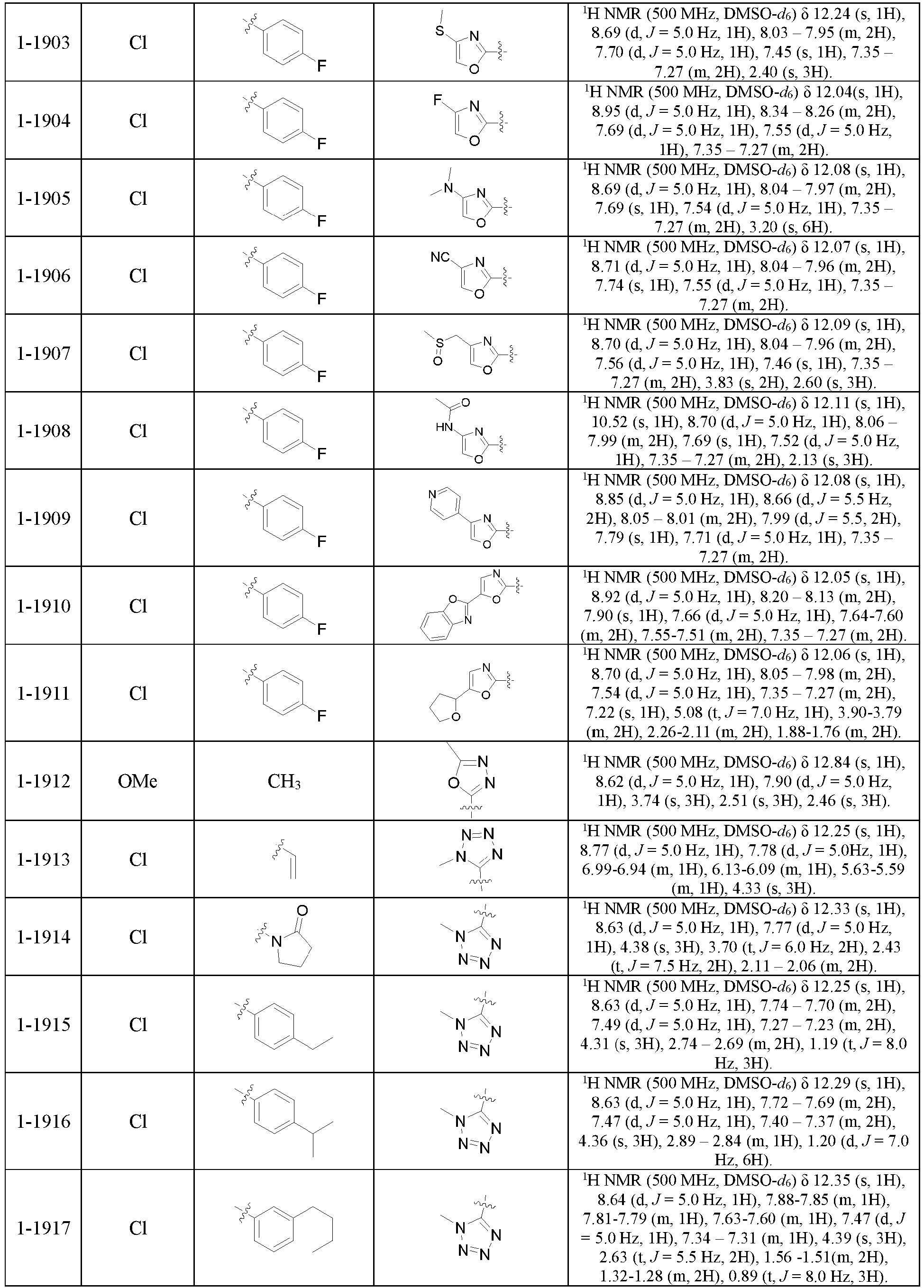

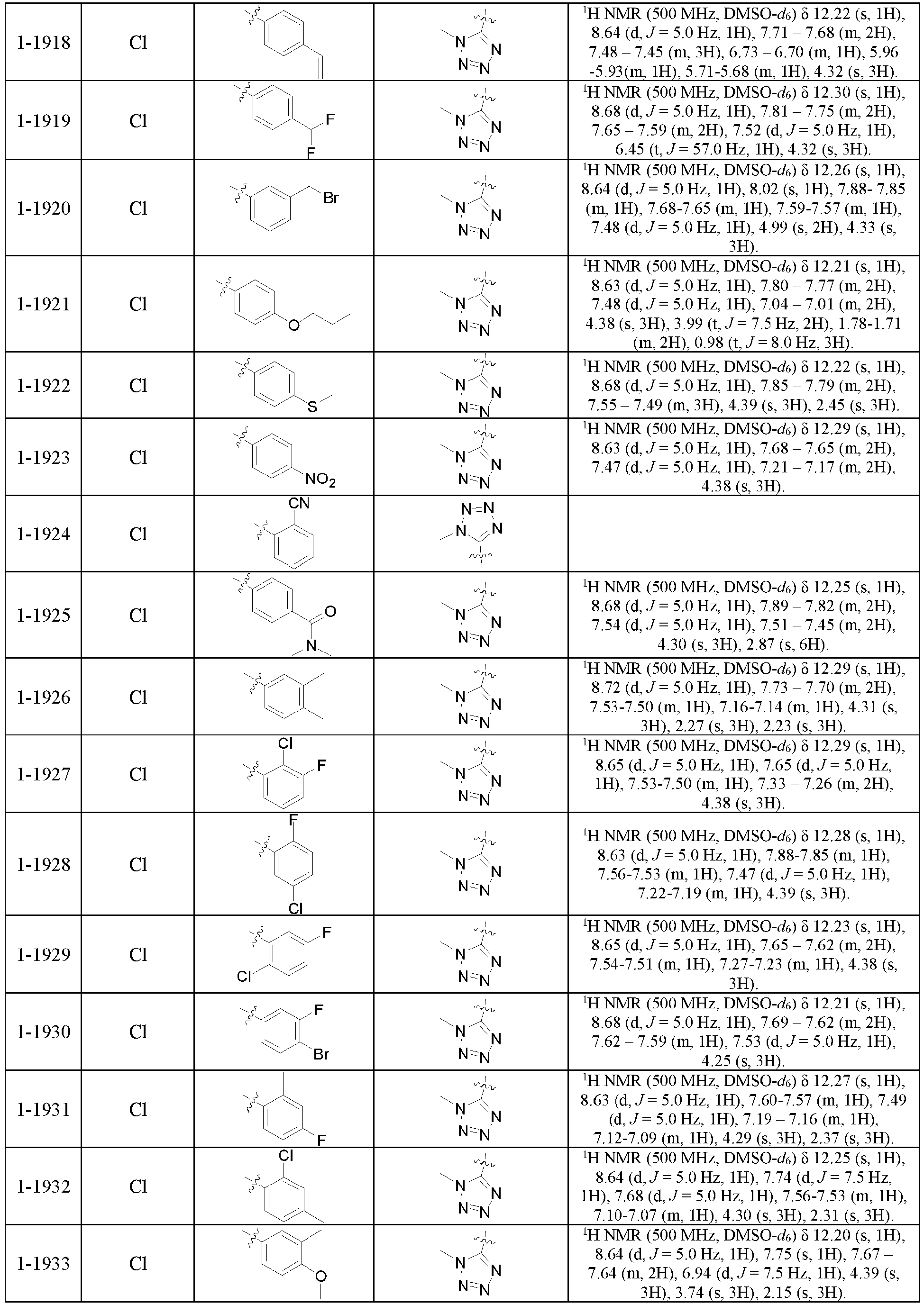

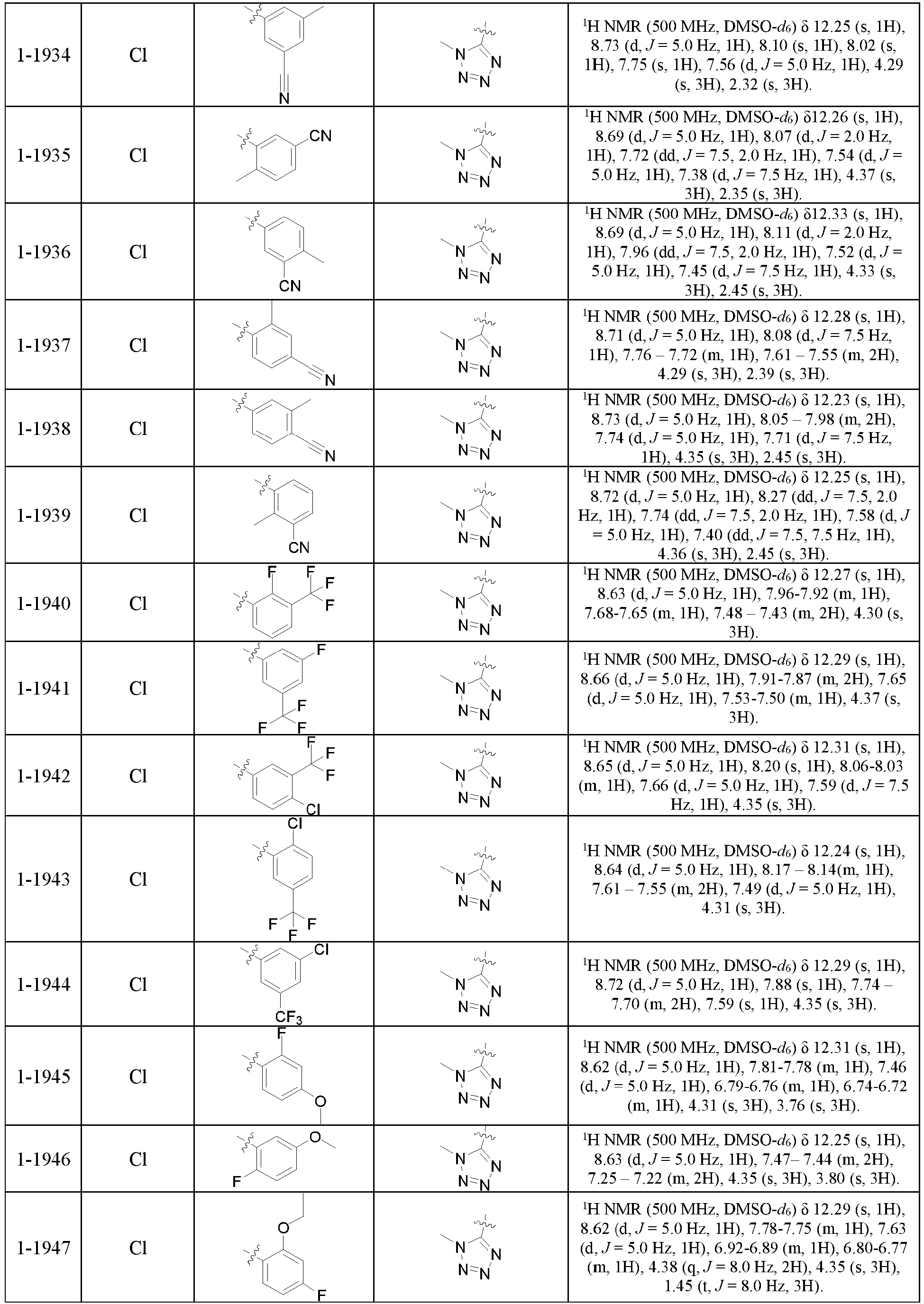

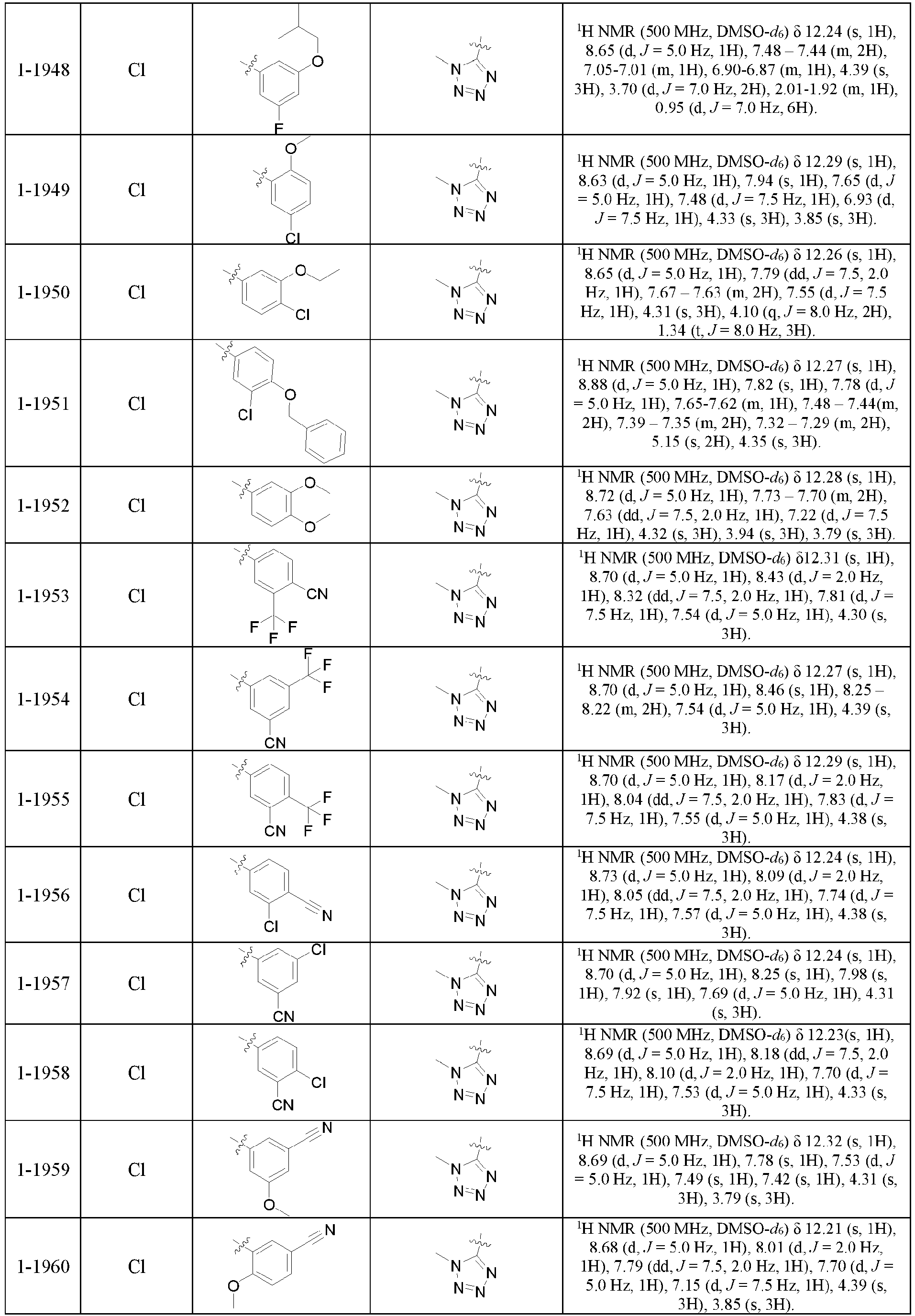

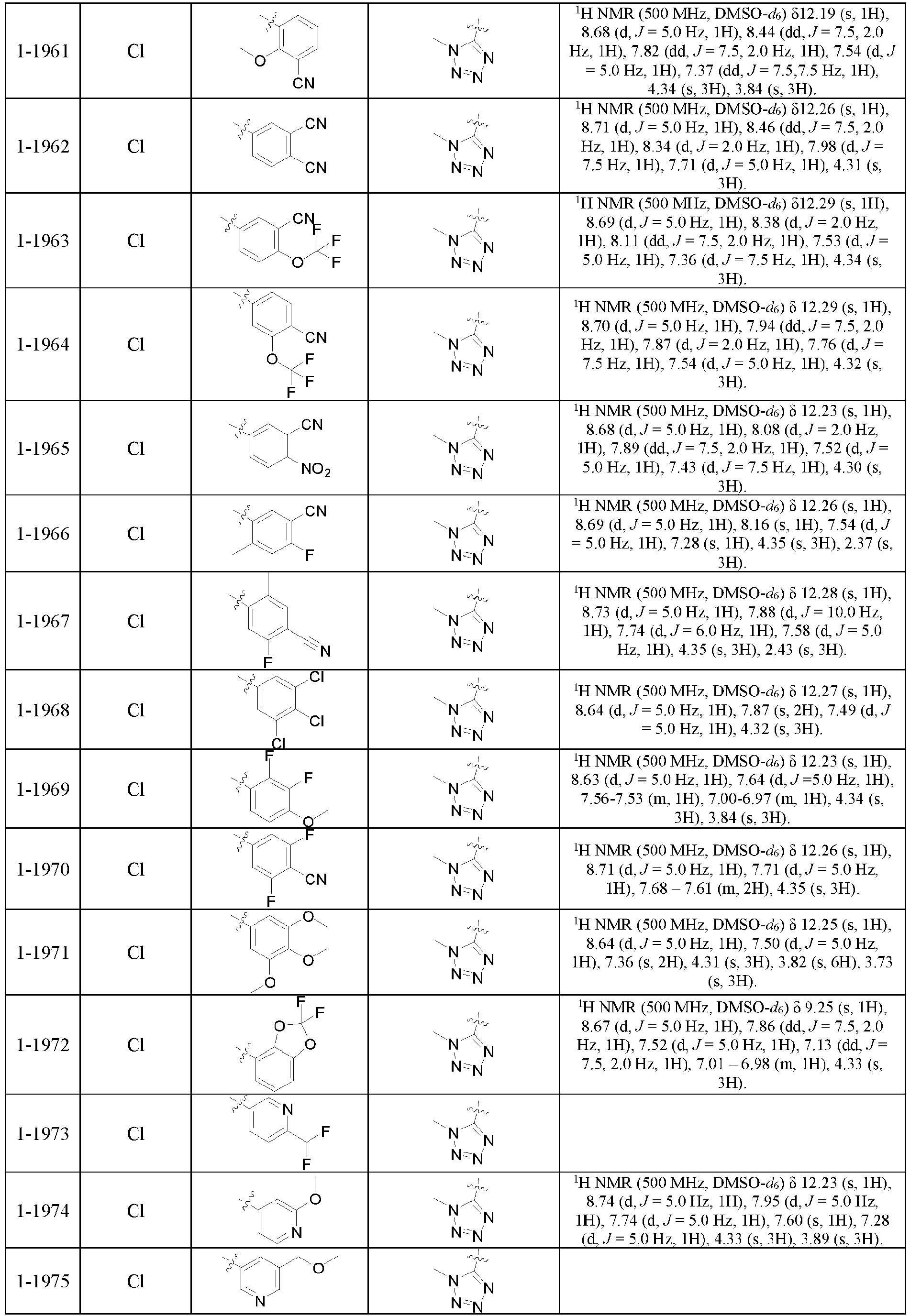

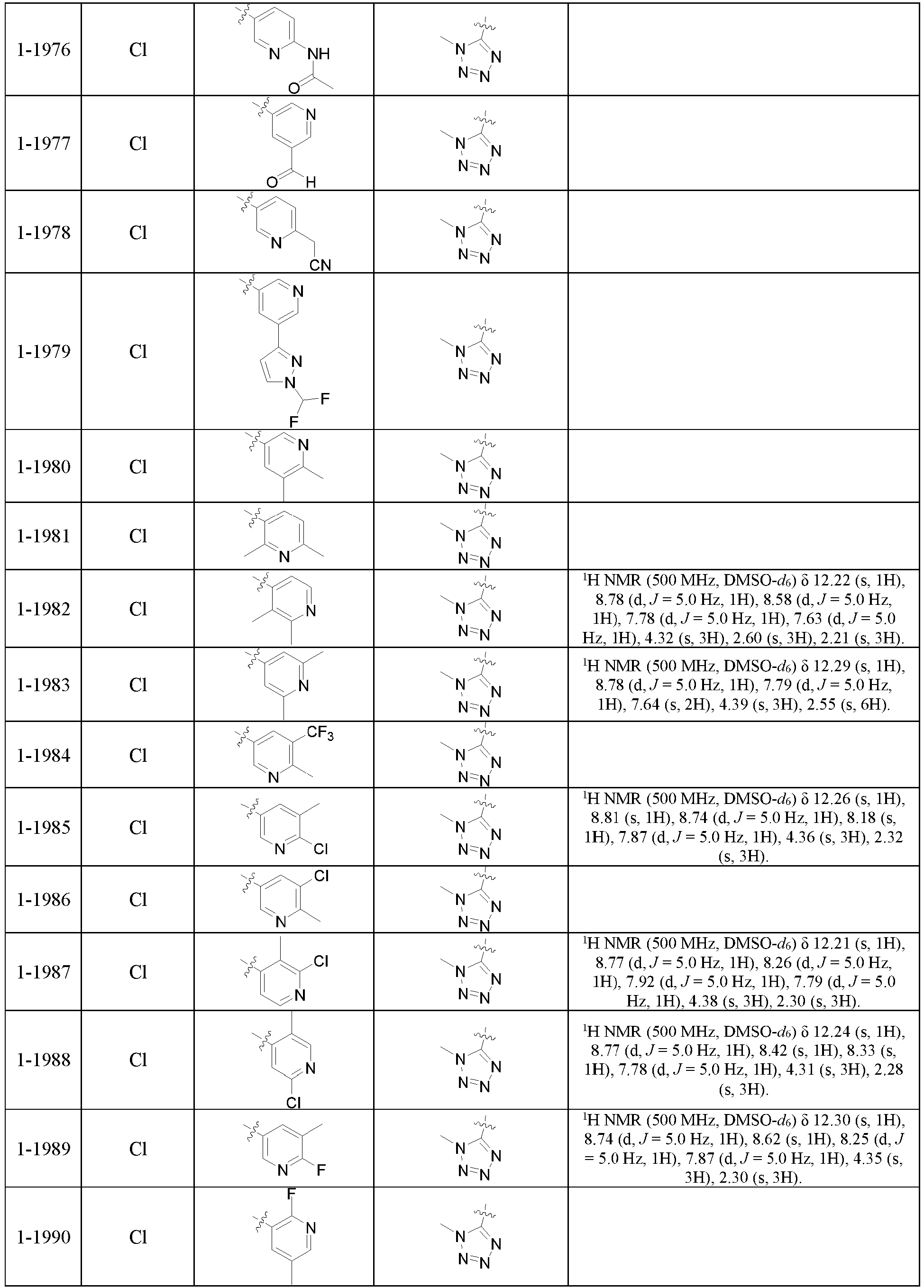

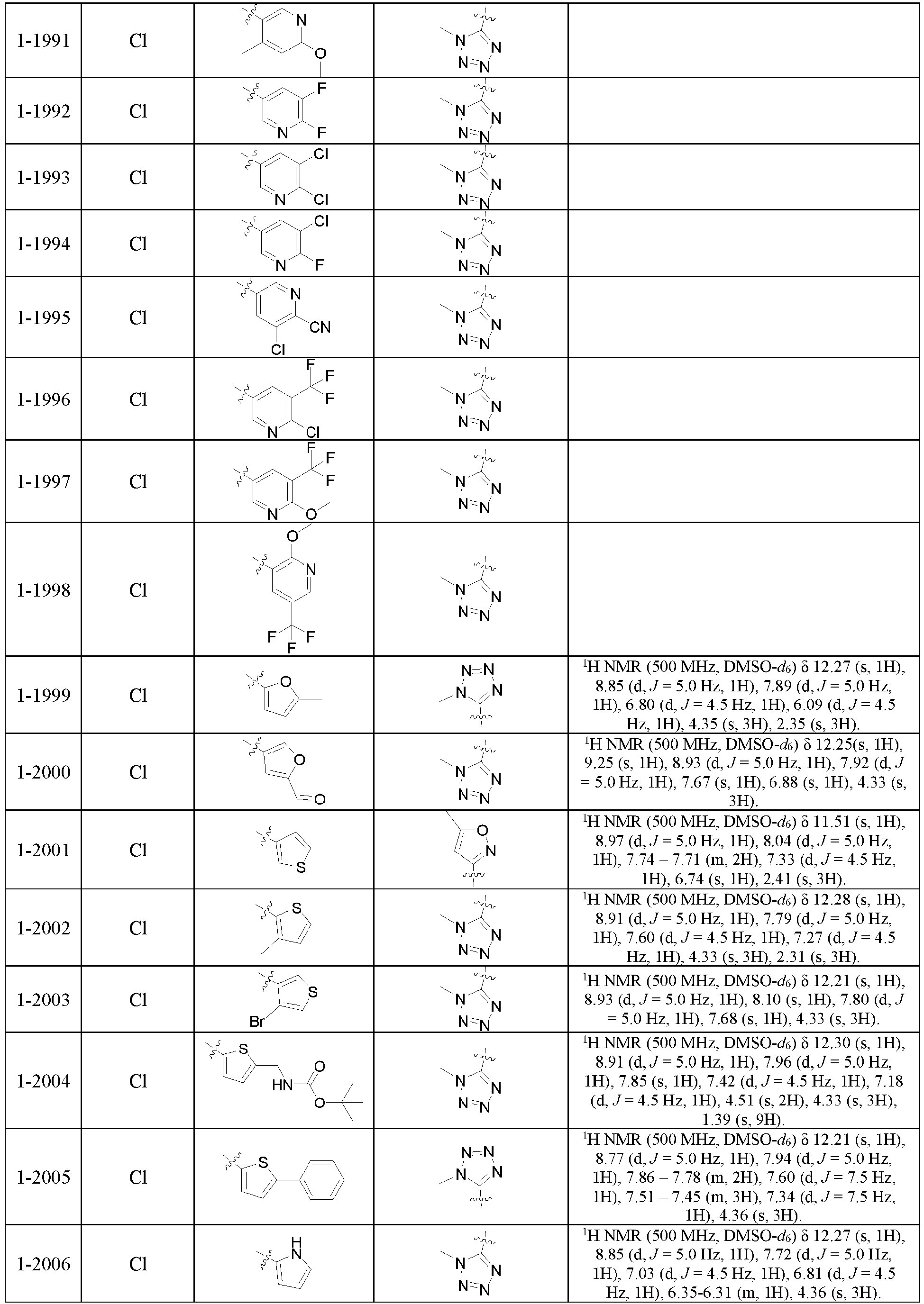

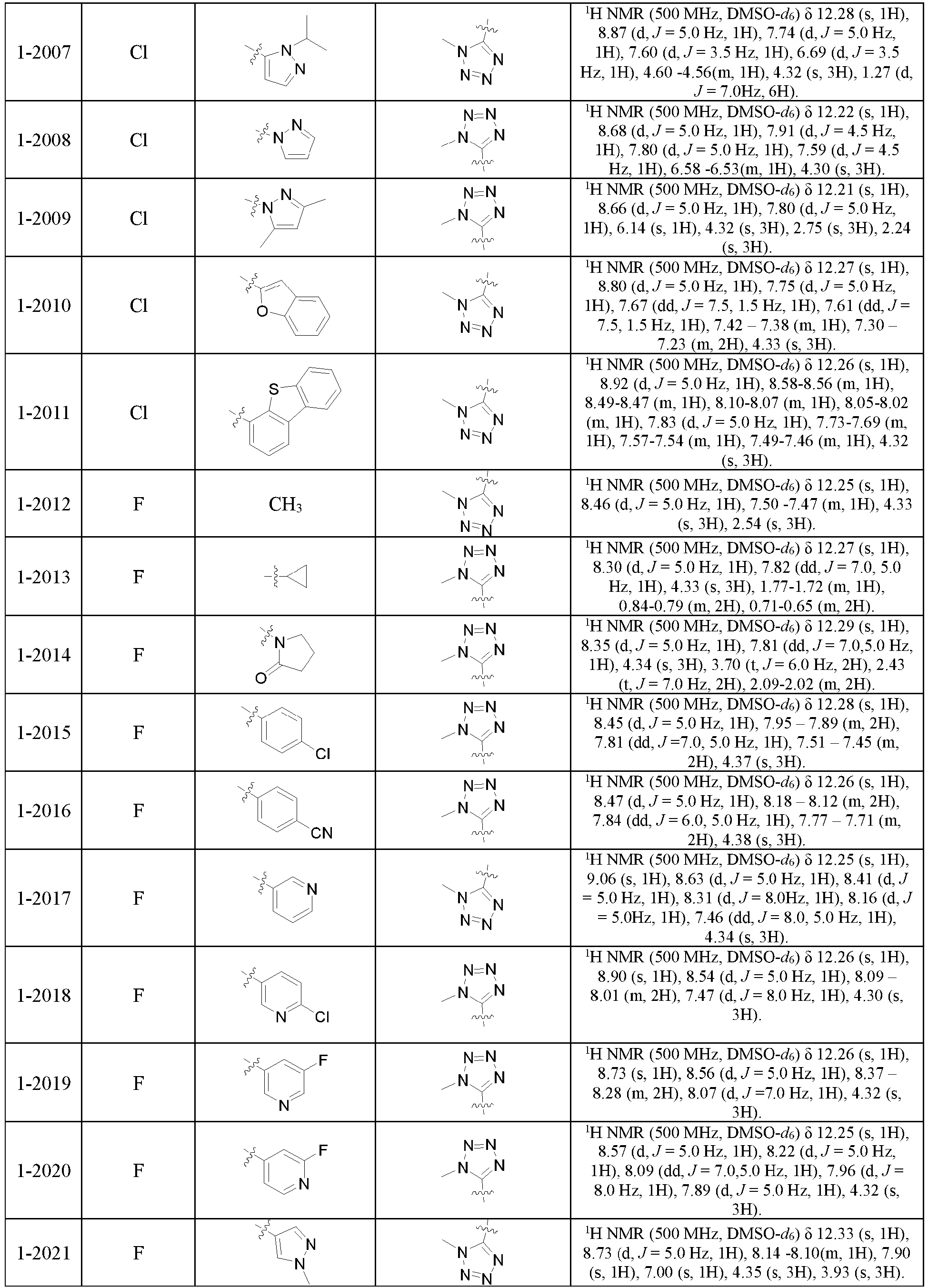

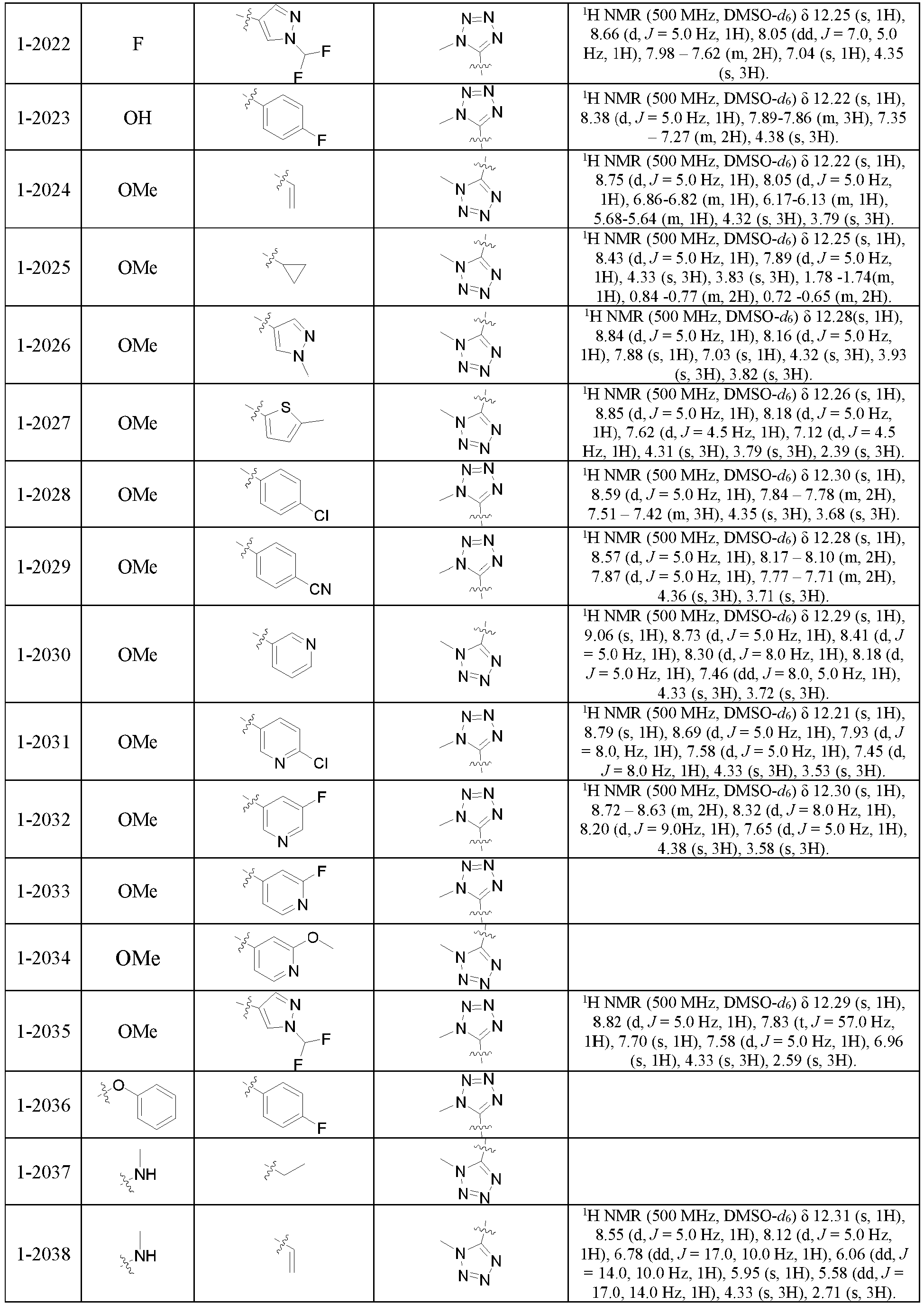

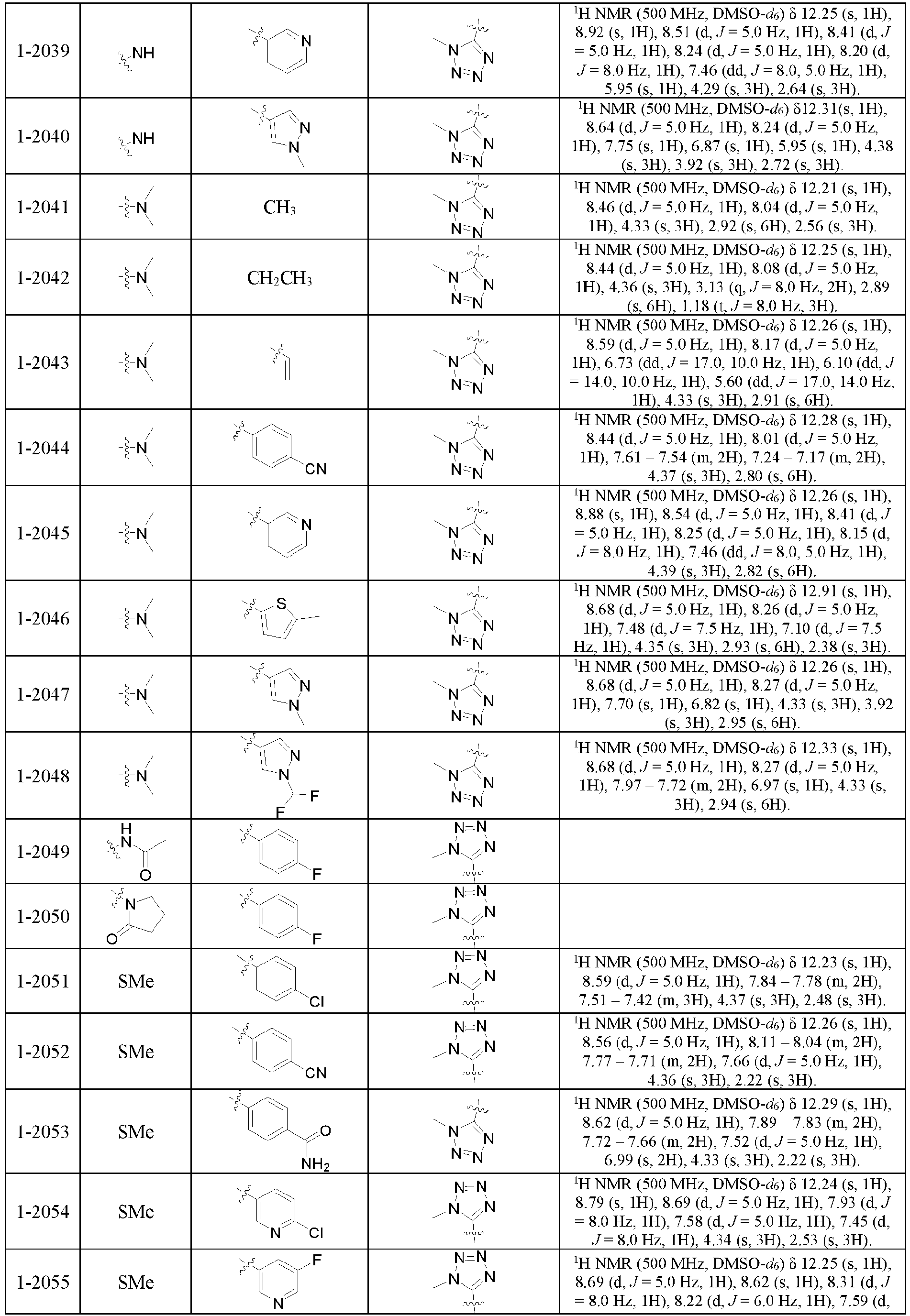

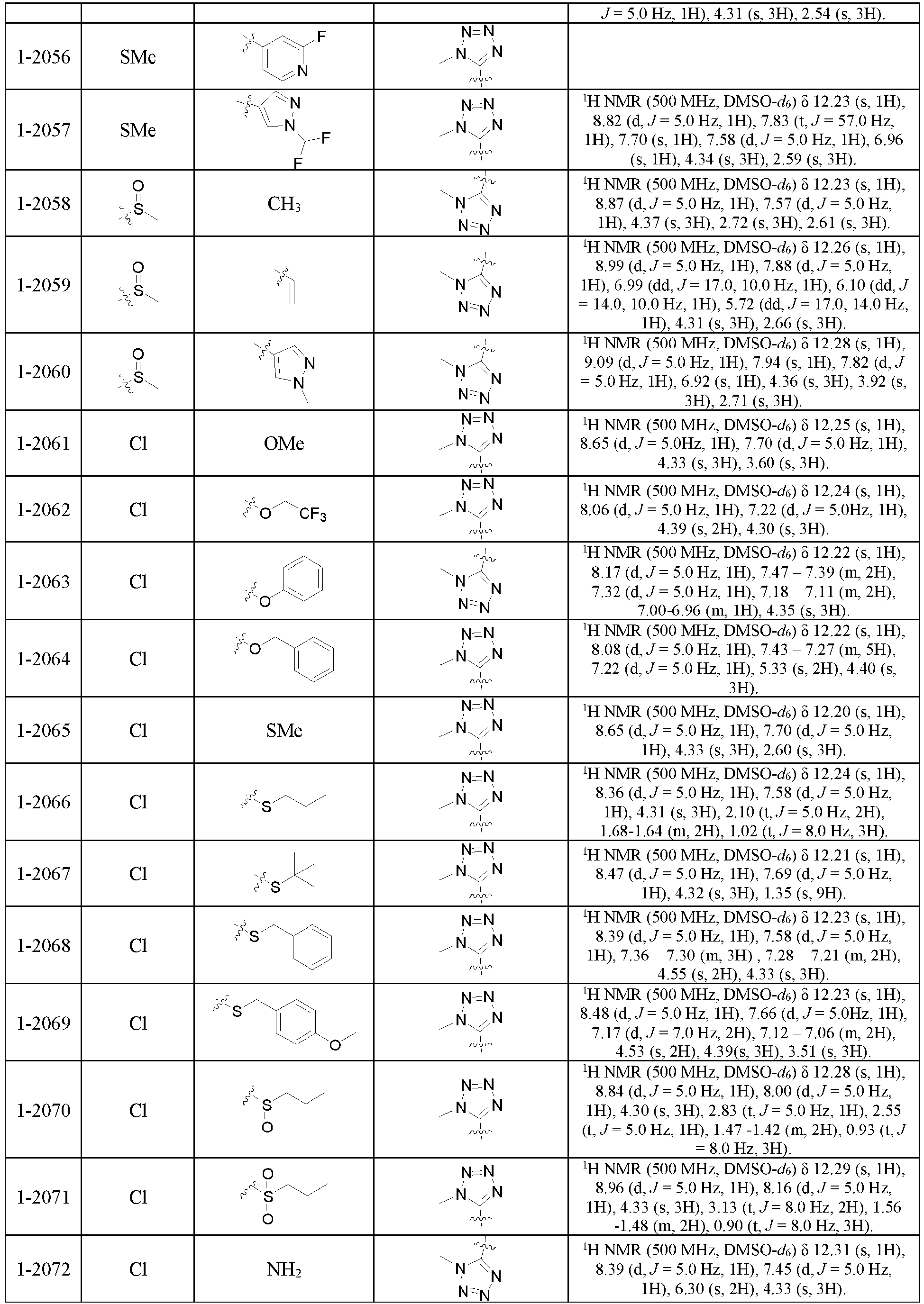

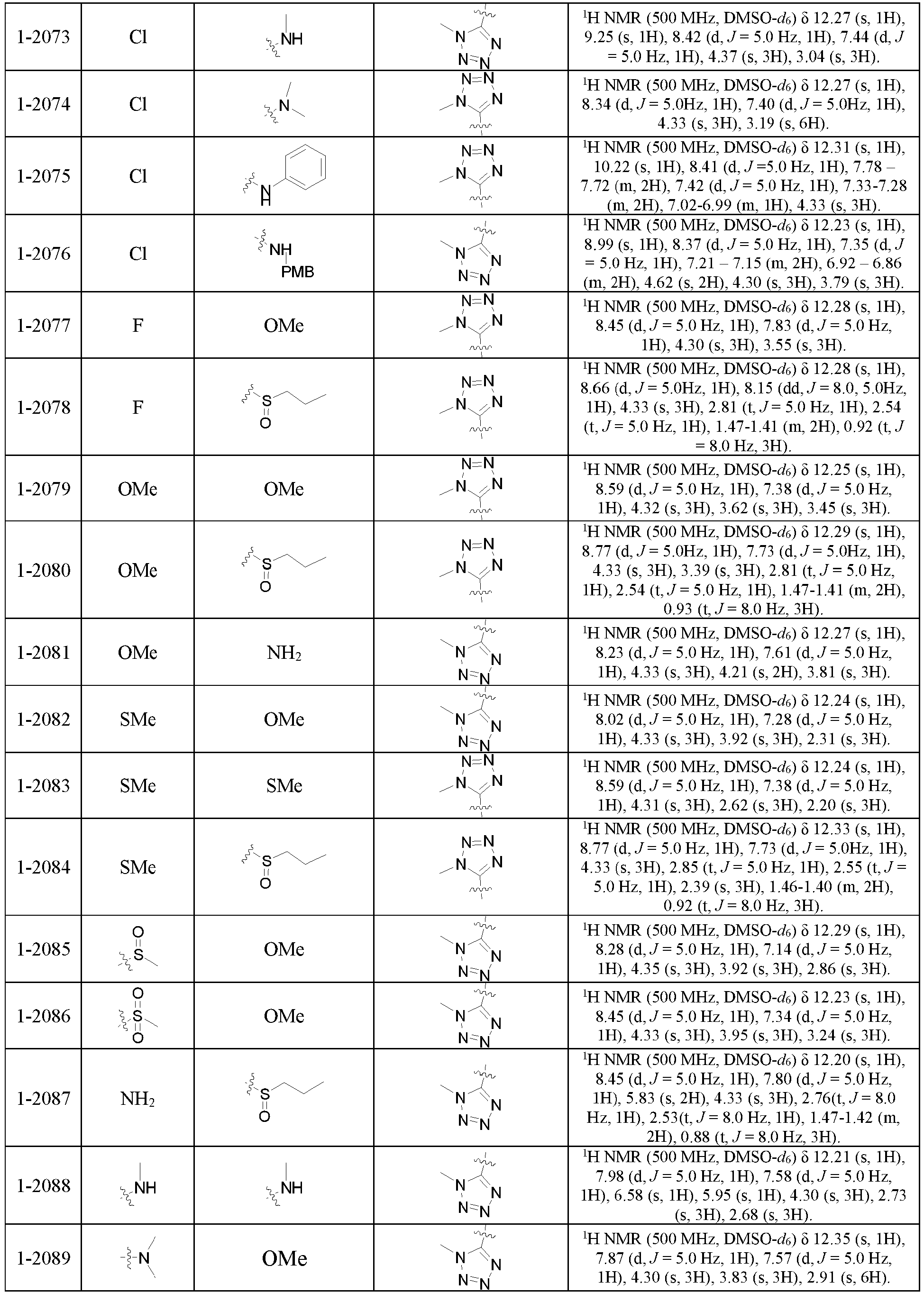

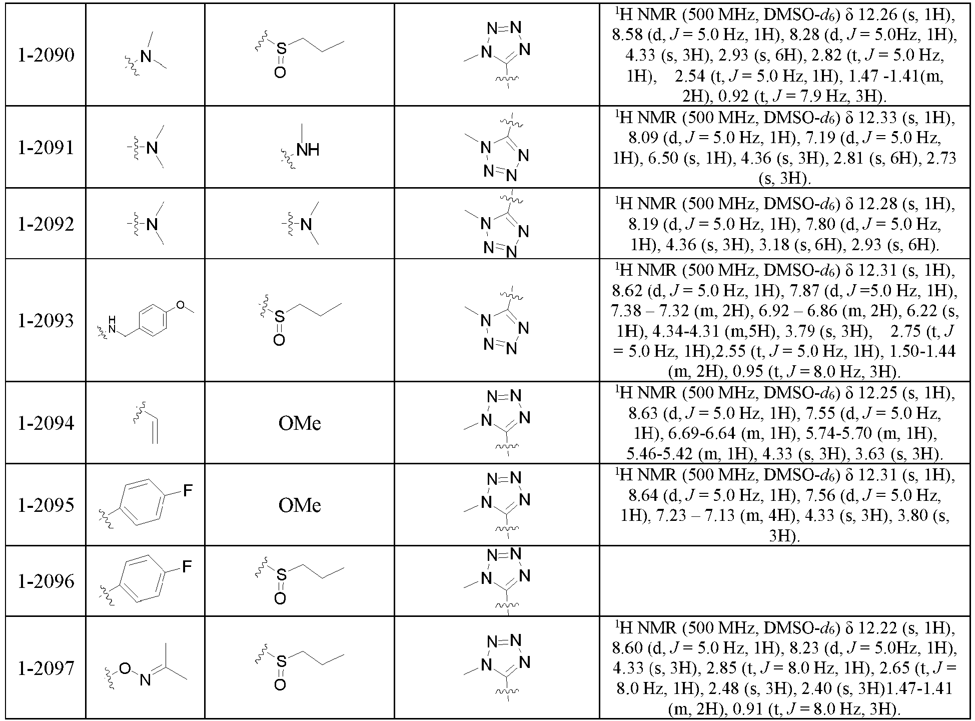

The method for preparing the compound of the invention will be explained in detail in the following program and embodiment. The material is commercial available or prepared through known method reported in the literature or shown in the route. Those skilled in the art should understand that the compound of the invention can also be synthesized by other synthetic route. Although the detailed material and reaction condition in the synthetic route have been explicated in the following text, it is still easy to be replaced by other similar material and condition. Isomer of the compound, for example, that produced with the variation of the preparation method of the present invention is included in the scope of the present invention. In addition, the following preparation method can be further modified according to the disclosures of the present invention by using common chemical method known to those skilled in the art, for example, protection of suitable group in the process of the reaction, etc.

The following method of application can be used to improve further understanding of the preparation method of the present invention. The specific material, class and condition have been determined to be further explication of the present invention, not to be any limit of the reasonable scope thereof. Reagents of the following synthetic compound showed in the table can either be purchased from the market or easily prepared by those skilled in the art.

Examples of representative compounds are as follows, the synthetic methods of other compounds are similar, and will not be described in detail here.

### 1. Synthesis of Compound1-1

(1) To a 50mL single-necked eggplant-shaped flask, 1.0 equivalent of compound a, 3.0 equivalents of sodium hydroxide, and 1.2 equivalents of 1-1-1 were charged sequentially at room temperature. After dissolved with 10:1 dioxane-water (10V), nitrogen replacement was performed three times under stirring, the catalyst 1,1'-bis(diphenylphosphino)ferrocene dichloride palladium(II) dichloromethane complex (1%) was added, nitrogen replacement was performed three times, then heated to 120 °C, and reacted at this temperature for 16-24 hours. The reaction solution was sampled and when the remaining raw materials were less than 5% according to LC-MS detection, the reaction was terminated. After the reaction was completed, 10V water was added, the insolubles were removed by filtration, and the mother liquor was adjusted to pH 1-2 with 2M hydrochloric acid solution, then extracted three times with ethyl acetate (5V). The organic phases were combined and distilled under reduced pressure to remove ethyl acetate. The product 1-1-2 was obtained by reverse-phase purification with yield of 78%. 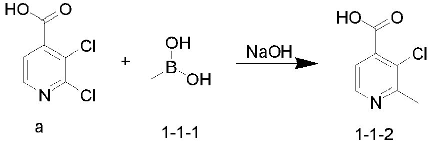
(2) To a 50mL single-necked eggplant-shaped flask, 1.0 equivalent of compound 1-1-2 was charged at room temperature. After dissolved with dichloromethane (10V), 1.1 equivalent of N,N'-carbonyldiimidazole (CDI) was added, and reacted at 30-40°C for 1 hour under stirring. After stirring, 1.2 equivalent of b and 1.0 equivalent of 1,8-diazabicycloundec-7-ene (DBU) were added, and continued to react at 30-40°C for 16-24 hours under stirring. The reaction solution was sampled and when the remaining raw materials were less than 5% according to LC-MS detection, the reaction was terminated. After the reaction was completed, 10V water was added to the reaction system, stirred, extracted, and the organic phase was poured into the waste liquid. The water phase was adjusted to pH 5-6 with 2M hydrochloric acid solution, and a large amount of solid was precipitated out at this time. After stirring for 15-30 minutes, the solid was filtered to obtain the product, and the product was dried to remove water with yield of about 72%.

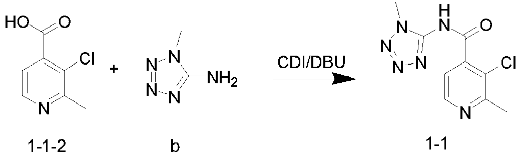

### 2. Synthesis of Compound1-17

It can be prepared by referring to the above method for preparing compound 1-1; or it can be prepared by the following method:
(1) To a 50mL single-necked eggplant-shaped flask, 1.0 equivalent of compound a was charged at room temperature. After dissolved with dichloromethane (10V), 1.1 equivalent of N,N'-carbonyldiimidazole (CDI) was added, and reacted at 30-40°C for 1 hour under stirring. After stirring, 1.2 equivalent of b and 1.0 equivalent of 1,8-diazabicycloundec-7-ene (DBU) were added, and continued to react at 30-40°C for 16-24 hours under stirring. The reaction solution was sampled and when the remaining raw materials were less than 5% according to LC-MS detection, the reaction was terminated. After the reaction was completed, 10V water was added to the reaction system, stirred, extracted, and the organic phase was poured into the waste liquid. The water phase was adjusted to pH 5-6 with 2M hydrochloric acid solution, and a large amount of solid was precipitated out at this time. After stirring for 15-30 minutes, the solid was filtered to obtain the product, and the product was dried to remove water with yield of about 75%. 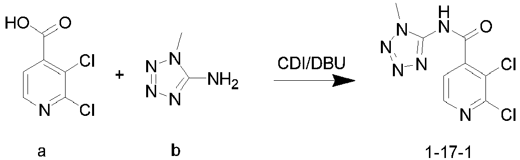
(2) To a 50mL single-necked eggplant-shaped flask, 1.0 equivalent of compound 1-17-1, 3.0 equivalents of potassium carbonate, and 1.2 equivalents of c were charged sequentially at room temperature. After dissolved with 10:1 dioxane-water (10V), nitrogen replacement was performed three times under stirring, the catalyst 1,1'-bis(diphenylphosphino)ferrocene dichloride palladium(II) dichloromethane complex (1%) was added, nitrogen replacement was performed three times, then heated to 110 °C, and reacted at this temperature for 16-24 hours. The reaction solution was sampled and when the remaining raw materials were less than 5% according to LC-MS detection, the reaction was terminated. After the reaction was completed, 10V water was added, the insolubles were removed by filtration, and the mother liquor was adjusted to pH 5-6 with 2M hydrochloric acid solution, then extracted three times with ethyl acetate (5V). The organic phases were combined and distilled under reduced pressure to remove ethyl acetate. The product was obtained by reverse-phase purification with yield of 50%.

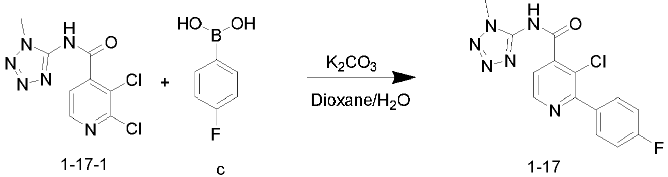

### 3. Synthesis of Compounds 1-203 and 1-205

(1) To a 50mL single-necked eggplant-shaped flask, 1.0 equivalent of compound 1-203-1 (It can be prepared by referring to the above method for preparing compound 1-17) was charged at room temperature. After dissolved with N,N-dimethylformamide (10V), 1.0 equivalent of sodium thiomethoxide aqueous solution (20% content) was added, and reacted at 50-60°C for 6-8 hours under stirring. The reaction solution was sampled and when the remaining raw materials were less than 2% according to LC-MS detection, the reaction was terminated. After the reaction was completed, 10V water was added, and adjusted to pH 5-6 with 2M hydrochloric acid solution, and the solid was precipitated out at this time, the insolubles were removed by filtration. The mother liquor was distilled under reduced pressure to remove the solvent, and the compound 1-203 was obtained by reverse-phase purification with yield of about 62%. 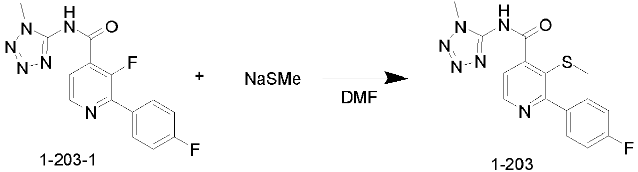
(2) To a 50mL single-necked eggplant-shaped flask, 1.0 equivalent of compound 1-203 was charged at room temperature. After dissolved with dichloromethane (10V), 1.0 equivalent of 1-205-1 (85% content) was added, and reacted at 10-20°C for 15-30 minutes under stirring. The reaction solution was sampled and when the remaining raw materials were less than 1% according to LC-MS detection, the reaction was terminated. After the reaction was completed, sodium thiosulfate aqueous solution was added to quench the reaction, the insolubles were removed by filtration, distilled under reduced pressure to remove the solvent, and the compound 1-205 was obtained by reverse-phase purification with yield of about 50%. 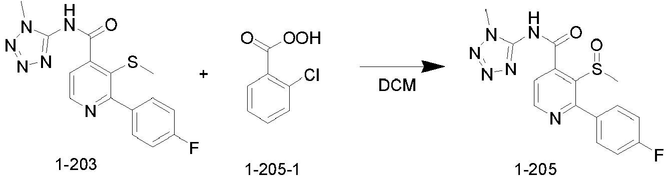 Or 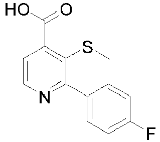 can be prepared from 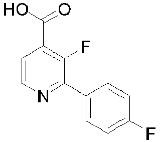 by referring to the above preparation method, then 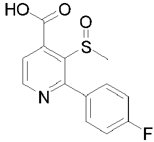 can be prepared, and reacts with 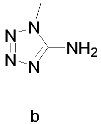 respectively to obtain compound 1-203 and compound 1-205 by referring to the synthesis step (2) of the above compound 1-1.

### 4. Synthesis of Compound 1-223

(1) To a 50mL single-necked eggplant-shaped flask, 1.0 equivalent of compound a, 3.0 equivalents of sodium hydroxide, and 2.5 equivalents of c were charged sequentially at room temperature. After dissolved with 10:1 dioxane-water (10V), nitrogen replacement was performed three times under stirring, the catalyst 1,1'-bis(diphenylphosphino)ferrocene dichloride palladium(II) dichloromethane complex (1%) was added, nitrogen replacement was performed three times, then heated to 120°C, and reacted at this temperature for 16-24 hours. The reaction solution was sampled and when the remaining raw materials were less than 5% according to LC-MS detection, the reaction was terminated. After the reaction was completed, 10V water was added, the insolubles were removed by filtration, and the mother liquor was adjusted to pH 1-2 with 2M hydrochloric acid solution, then extracted three times with ethyl acetate (5V). The organic phases were combined and distilled under reduced pressure to remove ethyl acetate. The product 1-223-1 was obtained by reverse-phase purification with yield of 45%. 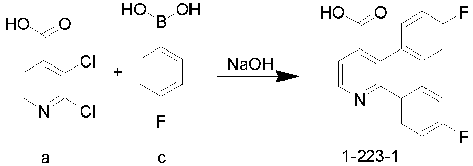
(2) To a 50mL single-necked eggplant-shaped flask, 1.0 equivalent of compound 1-223-1 was charged at 10-20°C. After dissolved with pyridine (10V), 10% 4-dimethylaminopyridine (DMAP) and 1.2 equivalents of compound b were charged sequentially, and 3.0 equivalents of thionyl chloride was added in a dropwise manner at 10-20°C under stirring, then heated to 30-40°C, and reacted for 2 hours under stirring. The reaction solution was sampled and when the remaining raw materials were less than 5% according to LC-MS detection, the reaction was terminated. After the reaction was completed, the pyridine was removed by distillation under reduced pressure, dissolved with 10V water, adjusted to pH 2-3 with 1M hydrochloric acid, then extracted three times with dichloromethane (5V). The organic phases were combined and distilled under reduced pressure to remove the solvent. The product 1-223 was obtained by reverse-phase purification with yield of about 52%.

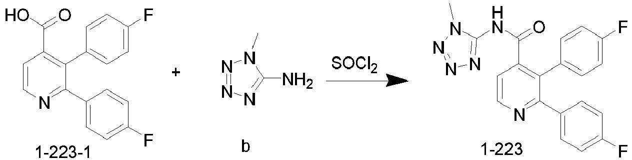

### 5. Synthesis of Compound 1-249

It can be prepared by referring to the above method for preparing compound 1-1; or it can be prepared by the following method:
To a 50mL single-necked eggplant-shaped flask, 1.0 equivalent of compound 1-17-1, 3.0 equivalents of potassium carbonate, and 1.2 equivalents of 1-249-1 were charged sequentially at room temperature. After dissolved with 10:1 dioxane-water (10V), nitrogen replacement was performed three times under stirring, the catalyst 1,1'-bis(diphenylphosphino)ferrocene dichloride palladium(II) dichloromethane complex (1%) was added, nitrogen replacement was performed three times, then heated to 110 °C, and reacted at this temperature for 16-24 hours. The reaction solution was sampled and when the remaining raw materials were less than 5% according to LC-MS detection, the reaction was terminated. After the reaction was completed, 10V water was added, the insolubles were removed by filtration, and the mother liquor was adjusted to pH 5-6 with 2M hydrochloric acid solution, then extracted three times with ethyl acetate (5V). The organic phases were combined and distilled under reduced pressure to remove ethyl acetate. The product was obtained by reverse-phase purification with yield of 50%.

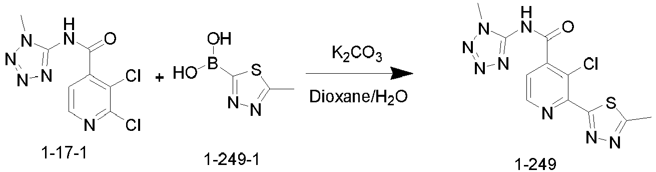

### 6. Synthesis of Compound 1-962

(1) To a 50mL single-necked eggplant-shaped flask, 1.0 equivalent of compound a, 3.0 equivalents of potassium carbonate, and 1.1 equivalents of c were charged sequentially at room temperature. After dissolved with 10:1 dioxane-water (10V), nitrogen replacement was performed three times under stirring, the catalyst 1,1'-bis(diphenylphosphino)ferrocene dichloride palladium(II) dichloromethane complex (1%) was added, nitrogen replacement was performed three times, then heated to 120 °C, and reacted at this temperature for 16-24 hours. The reaction solution was sampled and when the remaining raw materials were less than 1% according to LC-MS detection, the reaction was terminated. After the reaction was completed, 10V water was added, the insolubles were removed by filtration, extracted two times with ethyl acetate (5V), and the water phases were adjusted to pH 2-3 with 2M hydrochloric acid solution, then the solid was precipitated out, filtered and dried to obtain the product 1-962-1 with yield of 82%. 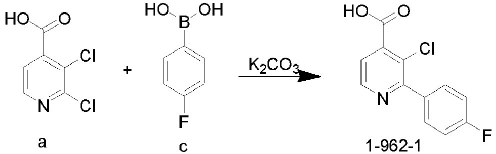
(2) To a 50mL single-necked eggplant-shaped flask, 1.0 equivalent of compound 1-962-1 was charged at room temperature. After dissolved with dichloromethane (10V), 1.1 equivalent of N,N'-carbonyldiimidazole (CDI) was added, and reacted at 30-40°C for 1 hour under stirring. After stirring, 1.2 equivalent of d and 1.0 equivalent of 1,8-diazabicycloundec-7-ene (DBU) were added, and continued to react at 30-40°C for 5-8 hours under stirring. The reaction solution was sampled and when the remaining raw materials were less than 5% according to LC-MS detection, the reaction was terminated. After the reaction was completed, 10V water was added to the reaction system, stirred, extracted, and the organic phase was poured into the waste liquid. The water phase was adjusted to pH 5-6 with 2M hydrochloric acid solution, and a large amount of solid was precipitated out at this time. After stirring for 15-30 minutes, the solid was filtered to obtain the product, and the product was dried to remove water with yield of about 69%.

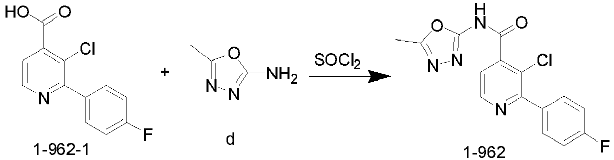

### Biological activity evaluation:

The activity level criteria for plant damage (i.e., growth control rate) are as follows:
Level 5: growth control rate is above 85%;
Level 4: growth control rate is greater than or equal to 60% and less than 85%;
Level 3: growth control rate is greater than or equal to 40% and less than 60%;
Level 2: growth control rate is greater than or equal to 20% and less than 40%;
Level 1: growth control rate is greater than or equal to 5% and less than 20%;
Level 0: growth control rate is less than 5%.

The above growth control rates are fresh weight control rates.

Experiment on weeding effect in post-emergence stage: monocotyledonous and dicotyledonous weed seeds and major crop seeds (wheat, corn, rice, soybean, cotton, oilseed rape, millet, sorghum) were placed in plastic pots filled with soil, then covered with 0.5-2 cm of soil, allowed to grow in a good greenhouse environment. After 2 weeks of sowing, the test plants were treated in the 2-3 leaf stage. The tested compounds of the present invention were respectively dissolved in acetone at the dosage of 2000, 1000, 500, 250, 125 g/ha, then added with Tween 80 and 1.5 liter/ha of emulsifiable concentrate of methyl oleate as synergist, diluted with a certain amount of water to obtain a solution with a certain concentration, and sprayed with a spray tower onto the plants. After the application, the plants were cultured for 3 weeks in the greenhouse, and then the experimental results were listed in Table 1.

**Table 1. Results on weeding effect in post-emergence stage**

| Compound No. | *Descurainia sophia* | *Abutilon theophrasti* | *Amaranthus retroflexus L.* | *Chenopodium album* |
|---|---|---|---|---|
| 1-1 | 5 | 5 | 5 | 5 |
| 1-2 | 5 | 5 | 5 | 5 |
| 1-3 | 5 | 5 | 5 | 5 |
| 1-4 | 5 | 5 | 5 | 5 |
| 1-5 | 5 | 5 | 5 | 5 |
| 1-6 | 5 | 5 | 5 | 5 |
| 1-7 | 5 | 5 | 5 | 5 |
| 1-8 | 5 | 5 | 5 | 5 |
| 1-9 | 5 | 5 | 5 | 5 |
| 1-10 | 5 | 5 | 5 | 5 |
| 1-11 | 5 | 5 | 5 | 5 |
| 1-12 | 5 | 5 | 5 | 5 |
| 1-13 | 5 | 5 | 5 | 5 |
| 1-14 | 5 | 5 | 5 | 5 |
| 1-15 | 5 | 5 | 5 | 5 |
| 1-16 | 5 | 5 | 5 | 5 |
| 1-17 | 5 | 5 | 5 | 5 |
| 1-18 | 5 | 5 | 5 | 5 |
| 1-19 | 5 | 5 | 5 | 5 |
| 1-20 | 5 | 5 | 5 | 5 |
| 1-21 | 5 | 5 | 5 | 5 |
| 1-22 | 5 | 5 | 5 | 5 |
| 1-23 | 5 | 5 | 5 | 5 |
| 1-24 | 5 | 5 | 5 | 5 |
| 1-25 | 5 | 5 | 5 | 5 |
| 1-26 | 5 | 5 | 5 | 5 |
| 1-27 | 5 | 5 | 5 | 5 |
| 1-28 | 5 | 5 | 5 | 5 |
| 1-29 | 5 | 5 | 5 | 5 |
| 1-30 | 5 | 5 | 5 | 5 |
| 1-31 | 5 | 5 | 5 | 5 |
| 1-32 | 5 | 5 | 5 | 5 |
| 1-33 | 5 | 5 | 5 | 5 |
| 1-34 | 5 | 5 | 5 | 5 |
| 1-35 | 5 | 5 | 5 | 5 |
| 1-36 | 5 | 5 | 5 | 5 |
| 1-37 | 5 | 5 | 5 | 5 |
| 1-38 | 5 | 5 | 5 | 5 |
| 1-39 | 5 | 5 | 5 | 5 |
| 1-40 | 5 | 5 | 5 | 5 |
| 1-41 | 5 | 5 | 5 | 5 |
| 1-42 | 5 | 5 | 5 | 5 |
| 1-43 | 5 | 5 | 5 | 5 |
| 1-44 | 5 | 5 | 5 | 5 |
| 1-45 | 5 | 5 | 5 | 5 |
| 1-46 | 5 | 5 | 5 | 5 |
| 1-47 | 5 | 5 | 5 | 5 |
| 1-48 | 5 | 5 | 5 | 5 |
| 1-49 | 5 | 5 | 5 | 5 |
| 1-50 | 5 | 5 | 5 | 5 |
| 1-51 | 5 | 5 | 5 | 5 |
| 1-52 | 5 | 5 | 5 | 5 |
| 1-53 | 5 | 5 | 5 | 5 |
| 1-54 | 5 | 5 | 5 | 5 |
| 1-55 | 5 | 5 | 5 | 5 |
| 1-56 | 5 | 5 | 5 | 5 |
| 1-57 | 5 | 5 | 5 | 5 |
| 1-58 | 5 | 5 | 5 | 5 |
| 1-59 | 5 | 5 | 5 | 5 |
| 1-60 | 5 | 5 | 5 | 5 |
| 1-61 | 5 | 5 | 5 | 5 |
| 1-62 | 5 | 5 | 5 | 5 |
| 1-63 | 5 | 5 | 5 | 5 |
| 1-64 | 5 | 5 | 5 | 5 |
| 1-65 | 5 | 5 | 5 | 5 |
| 1-66 | 5 | 5 | 5 | 5 |
| 1-67 | 5 | 5 | 5 | 5 |
| 1-68 | 5 | 5 | 5 | 5 |
| 1-69 | 5 | 5 | 5 | 5 |
| 1-70 | 5 | 5 | 5 | 5 |
| 1-71 | 5 | 5 | 5 | 5 |
| 1-72 | 5 | 5 | 5 | 5 |
| 1-73 | 5 | 5 | 5 | 5 |
| 1-74 | 5 | 5 | 5 | 5 |
| 1-75 | 5 | 5 | 5 | 5 |
| 1-76 | 5 | 5 | 5 | 5 |
| 1-77 | 5 | 5 | 5 | 5 |
| 1-78 | 5 | 5 | 5 | 5 |
| 1-79 | 5 | 5 | 5 | 5 |
| 1-80 | 5 | 5 | 5 | 5 |
| 1-81 | 5 | 5 | 5 | 5 |
| 1-82 | 5 | 5 | 5 | 5 |
| 1-83 | 5 | 5 | 5 | 5 |
| 1-84 | 5 | 5 | 5 | 5 |
| 1-85 | 5 | 5 | 5 | 5 |
| 1-86 | 5 | 5 | 5 | 5 |
| 1-87 | 5 | 5 | 5 | 5 |
| 1-88 | 5 | 5 | 5 | 5 |
| 1-89 | 5 | 5 | 5 | 5 |
| 1-90 | 5 | 5 | 5 | 5 |
| 1-91 | 5 | 5 | 5 | 5 |
| 1-92 | 5 | 5 | 5 | 5 |
| 1-93 | 5 | 5 | 5 | 5 |
| 1-94 | 5 | 5 | 5 | 5 |
| 1-95 | 5 | 5 | 5 | 5 |
| 1-96 | 5 | 5 | 5 | 5 |
| 1-97 | 5 | 5 | 5 | 5 |
| 1-98 | 5 | 5 | 5 | 5 |
| 1-99 | 5 | 5 | 5 | 5 |
| 1-100 | 5 | 5 | 5 | 5 |
| 1-101 | 5 | 5 | 5 | 5 |
| 1-102 | 5 | 5 | 5 | 5 |
| 1-103 | 5 | 5 | 5 | 5 |
| 1-104 | 5 | 5 | 5 | 5 |
| 1-105 | 5 | 5 | 5 | 5 |
| 1-106 | 5 | 5 | 5 | 5 |
| 1-107 | 5 | 5 | 5 | 5 |
| 1-108 | 5 | 5 | 5 | 5 |
| 1-109 | 5 | 5 | 5 | 5 |
| 1-110 | 5 | 5 | 5 | 5 |
| 1-111 | 5 | 5 | 5 | 5 |
| 1-112 | 5 | 5 | 5 | 5 |
| 1-113 | 5 | 5 | 5 | 5 |
| 1-114 | 5 | 5 | 5 | 5 |
| 1-115 | 5 | 5 | 5 | 5 |
| 1-116 | 5 | 5 | 5 | 5 |
| 1-117 | 5 | 5 | 5 | 5 |
| 1-118 | 5 | 5 | 5 | 5 |
| 1-119 | 5 | 5 | 5 | 5 |
| 1-120 | 5 | 5 | 5 | 5 |
| 1-121 | 5 | 5 | 5 | 5 |
| 1-122 | 5 | 5 | 5 | 5 |
| 1-123 | 5 | 5 | 5 | 5 |
| 1-124 | 5 | 5 | 5 | 5 |
| 1-125 | 5 | 5 | 5 | 5 |
| 1-126 | 5 | 5 | 5 | 5 |
| 1-127 | 5 | 5 | 5 | 5 |
| 1-128 | 5 | 5 | 5 | 5 |
| 1-129 | 5 | 5 | 5 | 5 |
| 1-130 | 5 | 5 | 5 | 5 |
| 1-131 | 5 | 5 | 5 | 5 |
| 1-132 | 5 | 5 | 5 | 5 |
| 1-133 | 5 | 5 | 5 | 5 |
| 1-134 | 5 | 5 | 5 | 5 |
| 1-135 | 5 | 5 | 5 | 5 |
| 1-136 | 5 | 5 | 5 | 5 |
| 1-137 | 5 | 5 | 5 | 5 |
| 1-138 | 5 | 5 | 5 | 5 |
| 1-139 | 5 | 5 | 5 | 5 |
| 1-140 | 5 | 5 | 5 | 5 |
| 1-141 | 5 | 5 | 5 | 5 |
| 1-142 | 5 | 5 | 5 | 5 |
| 1-143 | 5 | 5 | 5 | 5 |
| 1-144 | 5 | 5 | 5 | 5 |
| 1-145 | 5 | 5 | 5 | 5 |
| 1-146 | 5 | 5 | 5 | 5 |
| 1-147 | 5 | 5 | 5 | 5 |
| 1-148 | 5 | 5 | 5 | 5 |
| 1-149 | 5 | 5 | 5 | 5 |
| 1-150 | 5 | 5 | 5 | 5 |
| 1-151 | 5 | 5 | 5 | 5 |
| 1-152 | 5 | 5 | 5 | 5 |
| 1-153 | 5 | 5 | 5 | 5 |
| 1-154 | 5 | 5 | 5 | 5 |
| 1-155 | 5 | 5 | 5 | 5 |
| 1-156 | 5 | 5 | 5 | 5 |
| 1-157 | 5 | 5 | 5 | 5 |
| 1-158 | 5 | 5 | 5 | 5 |
| 1-159 | 5 | 5 | 5 | 5 |
| 1-160 | 5 | 5 | 5 | 5 |
| 1-161 | 5 | 5 | 5 | 5 |
| 1-162 | 5 | 5 | 5 | 5 |
| 1-163 | 5 | 5 | 5 | 5 |
| 1-164 | 5 | 5 | 5 | 5 |
| 1-165 | 5 | 5 | 5 | 5 |
| 1-166 | 5 | 5 | 5 | 5 |
| 1-167 | 5 | 5 | 5 | 5 |
| 1-168 | 5 | 5 | 5 | 5 |
| 1-169 | 5 | 5 | 5 | 5 |
| 1-170 | 5 | 5 | 5 | 5 |
| 1-171 | 5 | 5 | 5 | 5 |
| 1-172 | 5 | 5 | 5 | 5 |
| 1-173 | 5 | 5 | 5 | 5 |
| 1-174 | 5 | 5 | 5 | 5 |
| 1-175 | 5 | 5 | 5 | 5 |
| 1-176 | 5 | 5 | 5 | 5 |
| 1-177 | 5 | 5 | 5 | 5 |
| 1-178 | 5 | 5 | 5 | 5 |
| 1-179 | 5 | 5 | 5 | 5 |
| 1-180 | 5 | 5 | 5 | 5 |
| 1-181 | 5 | 5 | 5 | 5 |
| 1-182 | 5 | 5 | 5 | 5 |
| 1-183 | 5 | 5 | 5 | 5 |
| 1-184 | 5 | 5 | 5 | 5 |
| 1-185 | 5 | 5 | 5 | 5 |
| 1-186 | 5 | 5 | 5 | 5 |
| 1-187 | 5 | 5 | 5 | 5 |
| 1-188 | 5 | 5 | 5 | 5 |
| 1-189 | 5 | 5 | 5 | 5 |
| 1-190 | 5 | 5 | 5 | 5 |
| 1-191 | 5 | 5 | 5 | 5 |
| 1-192 | 5 | 5 | 5 | 5 |
| 1-193 | 5 | 5 | 5 | 5 |
| 1-194 | 5 | 5 | 5 | 5 |
| 1-195 | 5 | 5 | 5 | 5 |
| 1-196 | 5 | 5 | 5 | 5 |
| 1-197 | 5 | 5 | 5 | 5 |
| 1-198 | 5 | 5 | 5 | 5 |
| 1-199 | 5 | 5 | 5 | 5 |
| 1-200 | 5 | 5 | 5 | 5 |
| 1-201 | 5 | 5 | 5 | 5 |
| 1-202 | 5 | 5 | 5 | 5 |
| 1-203 | 5 | 5 | 5 | 5 |
| 1-204 | 5 | 5 | 5 | 5 |
| 1-205 | 5 | 5 | 5 | 5 |
| 1-206 | 5 | 5 | 5 | 5 |
| 1-207 | 5 | 5 | 5 | 5 |
| 1-208 | 5 | 5 | 5 | 5 |
| 1-209 | 5 | 5 | 5 | 5 |
| 1-210 | 5 | 5 | 5 | 5 |
| 1-211 | 5 | 5 | 5 | 5 |
| 1-212 | 5 | 5 | 5 | 5 |
| 1-213 | 5 | 5 | 5 | 5 |
| 1-214 | 5 | 5 | 5 | 5 |
| 1-215 | 5 | 5 | 5 | 5 |
| 1-216 | 5 | 5 | 5 | 5 |
| 1-217 | 5 | 5 | 5 | 5 |
| 1-218 | 5 | 5 | 5 | 5 |
| 1-219 | 5 | 5 | 5 | 5 |
| 1-220 | 5 | 5 | 5 | 5 |
| 1-221 | 5 | 5 | 5 | 5 |
| 1-222 | 5 | 5 | 5 | 5 |
| 1-223 | 5 | 5 | 5 | 5 |
| 1-228 | 5 | 5 | 5 | 5 |
| 1-229 | 5 | 5 | 5 | 5 |
| 1-230 | 5 | 5 | 5 | 5 |
| 1-233 | 5 | 5 | 5 | 5 |
| 1-234 | 5 | 5 | 5 | 5 |
| 1-249 | 5 | 5 | 5 | 5 |
| 1-307 | 5 | 5 | 5 | 5 |
| 1-334 | 5 | 5 | 5 | 5 |
| 1-650 | 5 | 5 | 5 | 5 |
| 1-962 | 5 | 5 | 5 | 5 |
| 1-1257 | 5 | 5 | 5 | 5 |
| 1-1286 | 5 | 5 | 5 | 5 |
| 1-1577 | 5 | 5 | 5 | 5 |
| 1-1607 | 5 | 5 | 5 | 5 |
| 1-1918 | 5 | 5 | 5 | 5 |
| 1-1925 | 5 | 5 | 5 | 5 |
| 1-1972 | 5 | 5 | 5 | 5 |
| 1-2004 | 5 | 5 | 5 | 5 |
| 1-2006 | 5 | 5 | 5 | 5 |
| 1-2010 | 5 | 5 | 5 | 5 |
| 1-2016 | 5 | 5 | 5 | 5 |
| 1-2029 | 5 | 5 | 5 | 5 |
| 1-2044 | 5 | 5 | 5 | 5 |
| 1-2052 | 5 | 5 | 5 | 5 |
| 1-2053 | 5 | 5 | 5 | 5 |
| 1-2061 | 5 | 5 | 5 | 5 |
| 1-2062 | 5 | 5 | 5 | 5 |
| 1-2063 | 5 | 5 | 5 | 5 |
| 1-2064 | 5 | 5 | 5 | 5 |
| 1-2065 | 5 | 5 | 5 | 5 |
| 1-2069 | 5 | 5 | 5 | 5 |
| 1-2070 | 5 | 5 | 5 | 5 |
| 1-2071 | 5 | 5 | 5 | 5 |
| 1-2075 | 5 | 5 | 5 | 5 |

| | | | | |
|---|---|---|---|---|
| Note: The application dose was active ingredient 2000 g/ha. | | | | |

In addition, under the active ingredient of 250 g/ha, many compounds also show good crop selectivity and excellent control effect on key weeds. For example, see Table 2:

**Table 2. Test results**

| No. | *Alopecurus aequalis* | *Beckmannia syzigachne* | *Alopecurus myosuroides* | *Sclerochloa dura* | *Descurainia sophia* | Wheat |
|---|---|---|---|---|---|---|
| 1-15 | 5 | 5 | 5 | 5 | 5 | 0 |
| 1-17 | 5 | 5 | 5 | 5 | 5 | 0 |
| Control compound A | 0 | 0 | 0 | 0 | 0 | 0 |

Control compound A:

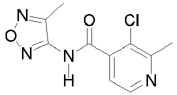

### Experiment on weed effect in pre-emergence stage

Seeds of monocotyledonous and dicotyledonous weeds and main crops (e.g. wheat, corn, rice, soybean, cotton, oilseed rape, millet and sorghum) were put into a plastic pot loaded with soil and covered with 0.5-2cm soil. The test compounds of the present invention was dissolved with acetone, then added with tween 80, diluted by a certain amount of water to reach a certain concentration, and sprayed immediately after sowing. The obtained seeds were incubated for 4 weeks in the greenhouse after spraying and the test results were observed. It was observed that the herbicide mostly had excellent effect at the application rate of 2000g a.i./ha, especially to weeds such as *Echinochloa crusgalli, Digitaria sanguinalis, Abutilon theophrasti,* etc. And many compounds had good selectivity for corn, wheat, rice, soybean, oilseed rape, etc.

It is indicated from the experiment that the compounds of the present invention generally have good weed control efficacy, especially for the major cyperaceae weeds like *Echinochloa crusgalli, Digitaria sanguinalis,* and *Setaria viridis,* etc., and the major broad-leaved weeds like *Abutilon theophrasti, Rorippa indica,* and *Bidens pilosa L.,* etc., which are widely occurring in corn, rice, and wheat fields, and have excellent commercial value. Above all, it is noted that they have extremely high activity to weeds, which are resistant to ALS inhibitor, like *Rorippa indica, Descurainia sophia, Capsella bursa-pastoris, Lithospermum arvense, Galium aparine L., Stellaria media, Setaria viridis, Echinochloa crusgalli, Digitaria sanguinalis, Alopecurus aequalis, Beckmannia syzigachne,* etc..

Transplanted rice safety evaluation and weed control effect evaluation in rice field:
Rice field soil was loaded into a 1/1,000,000 ha pot. The seeds of *Echinochloa crusgalli, Scirpus juncoides, Bidens tripartita L., Sagittaria trifolia,* and *Monochoria vaginalis* were sowed and gently covered with soil, then left to stand still in greenhouse in the state of 0.5-1cm of water storage. The tuber of *Sagittaria trifolia* was planted in the next day or 2 days later. It was kept at 3-4cm of water storage thereafter. The weeds were treated by dripping the WP or SC water diluents prepared according to the common preparation method of the compounds of the present invention with pipette homogeneously to achieve specified effective amount when *Echinochloa crusgalli, Scirpus juncoides, Bidens tripartita L.,* and *Monochoria vaginalis* reached 0.5 leaf stage and *Sagittaria trifolia* reached the time point of primary leaf stage.

In addition, the rice field soil that loaded into the 1/1,000,000 ha pot was leveled to keep water storage at 3-4cm depth. The 3 leaf stage rice (japonica rice) was transplanted at 3cm of transplanting depth the next day. The compound of the present invention was treated by the same way after 5 days of transplantation.

The fertility condition of *Echinochloa crusgalli, Scirpus juncoides, Bidens tripartita L., Sagittaria trifolia* and *Monochoria vaginalis* 14 days after the treatment of the compound of the invention and the fertility condition of rice 21 days after the treatment of the compound of the invention respectively with the naked eye. Evaluate the weed control effect with the above 0-5 activity standard level, many compounds show excellent activity and selectivity. Wherein, the seeds of *Echinochloa crusgali, Scirpus juncoides, Bidens tripartita, Sagittaria trifolia* and *Monochoria vaginalis* were collected from Heilongjiang Province of China. The tests indicated that the weeds were resistant to the common doses of Pyrazosulfuron-ethyl and Penoxsulam.

It could be seen from this experiment that the compound of the present invention has excellent activity against the anti-ALS weeds which is a serious challenge in the production, and could solve the increasingly serious problem of resistance.

At the same time, it is found after several tests that the compound of the present invention and its composition have good selectivity to many gramineae grass such as zoysia japonica, bermuda grass, tall fescue, bluegrass, ryegrass and seashore paspalum etc, and is able to control many important grass weeds and broadleaf weeds. The compound also shows excellent selectivity and commercial value in the tests on wheat, corn, rice, sugarcane, soybean, cotton, oil sunflower, potato, orchards and vegetables in different herbicide application methods.

## Claims

1. A 4-pyridinyl formamide compound or derivative thereof, as shown in Formula I: 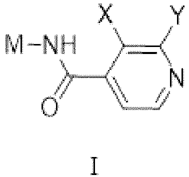
wherein, X represent nitro, halogen, cyano, formyl, thiocyano, mercapto; alkyl, alkenyl, alkynyl, cycloalkyl, cycloalkenyl, cycloalkylalkyl, or cycloalkenylalkyl, which is with or without halogen; OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -alkyl-C(S)R¹, -alkyl-C(S)OR¹, -alkyl-C(O)SR¹, -alkyl-C(S)SR¹, -alkyl-SC(O)R¹, -alkyl-OC(S)R¹, -alkyl-SC(S)R¹, -O-alkyl-NR⁴R⁵, -S-alkyl-NR⁴R⁵, -alkyl-O-alkyl-NR⁴R⁵, -alkyl-S-alkyl-NR⁴R⁵, -alkyl-(C=S)ₙ-NR⁴R⁵, -NH-alkyl-NR⁴R⁵, -alkyl-OR¹, -alkyl-COR¹, -alkyl-CO₂R¹, -alkyl-OCOR¹, -alkyl-NR³COR¹, -alkyl-SO₂OR¹, -alkyl-NR³SO₂R², -alkyl-OSO₂R², -alkyl-S(O)ₘR², -alkyl-CONR⁴R⁵, -alkyl-SO₂NR⁴R⁵, NR⁴R⁵, 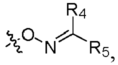 P(O)(OR⁶)₂, CH₂P(O)(OR⁶)₂, SO₂NR⁴R⁵-alkyl-S(O)ₘR², -alkyl-CN, -alkylaryl, -alkylheteroaryl, -alkylheterocyclyl, aryl, heteroaryl, or heterocyclyl;
Y represent nitro, cyano, formyl, thiocyano, mercapto; alkyl, alkenyl, alkynyl, cycloalkyl, cycloalkenyl, cycloalkylalkyl, or cycloalkenylalkyl, which is with or without halogen; OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -alkyl-C(S)R¹, -alkyl-C(S)OR¹, -alkyl-C(O)SR¹, -alkyl-C(S)SR¹, -alkyl-SC(O)R¹, -alkyl-OC(S)R¹, -alkyl-SC(S)R¹, -O-alkyl-NR⁴R⁵, -S-alkyl-NR⁴R⁵, -alkyl-O-alkyl-NR⁴R⁵, -alkyl-S-alkyl-NR⁴R⁵, -alkyl-(C=S)ₙ-NR⁴R⁵, -NH-alkyl-NR⁴R⁵, -alkyl-OR¹, -alkyl-COR¹, -alkyl-CO₂R¹, -alkyl-OCOR¹, -alkyl-NR³COR¹, -alkyl-SO₂OR¹, -alkyl-NR³SO₂R², -alkyl-OSO₂R², -alkyl-S(O)ₘR², -alkyl-CONR⁴R⁵, -alkyl-SO₂NR⁴R⁵, NR⁴R⁵, 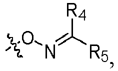 P(O)(OR⁶)₂, CH₂P(O)(OR⁶)₂, SO₂NR⁴R⁵-alkyl-S(O)ₘR², -alkyl-CN, -alkylaryl, -alkylheteroaryl, -alkylheterocyclyl, aryl, heteroaryl, or heterocyclyl;
R¹, R³, R⁴, and R⁵ each independently represent hydrogen, aryl, arylalkyl, heteroaryl, heteroarylalkyl, alkyl, halogenated alkyl, alkenyl, halogenated alkenyl, alkynyl, halogenated alkynyl, cycloalkyl, halogenated cycloalkyl, alkoxyalkyl, or cycloalkylalkyl, wherein the last 10 groups as mentioned are each substituted by s groups selected from the group consisting of cyano, halogen, nitro, thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and alkoxyalkoxycarbonyl;
R² represents aryl, arylalkyl, heteroaryl, heteroarylalkyl, alkyl, alkenyl, alkynyl, cycloalkyl, or cycloalkylalkyl, wherein the last 5 groups as mentioned are each substituted by s groups selected from the group consisting of cyano, halogen, nitro, thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and alkoxyalkoxycarbonyl;
R⁶ represents methyl, or ethyl;
R⁷, and R⁸ each independently represent hydrogen, alkyl, alkenyl, or alkynyl;
R⁹ represents alkyl, alkenyl, or alkynyl;
M represents 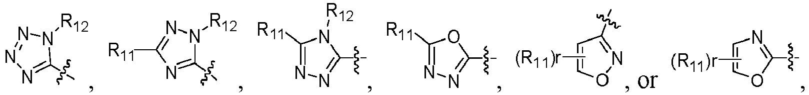 which is unsubstituted or substituted;
R₁₁ represents hydrogen, halogen, cyano, nitro, alkyl which is unsubstituted or substituted with a substituent selected from R₁₃, cycloalkyl which is unsubstituted or substituted with a substituent selected from R₁₄, alkenyl, halogenated alkenyl, alkynyl, halogenated alkynyl, cycloalkenyl, NH₂, aminoacyl, carboxyl, alkoxyalkoxycarbonyl, OR₁₅, -alkyl-OR₁₅, C(O)R₁₆, -alkyl-C(O)R₁₆, C(O)OR₁₆, -alkyl-C(O)OR₁₆, S(O)ₘR₁₆, -alkyl-S(O)ₘR₁₆, -N(R₁₆)₂, -NHR₁₆, -C(O)NHR₁₆, -C(O)N(R₁₆)₂, -NHC(O)R₁₇, heterocyclyl, heterocyclylalkyl, heterocyclyloxy, heterocyclylcarbonyl, aryl, arylalkyl, aryloxy, arylcarbonyl, heteroaryl, heteroarylalkyl, heteroaryloxy, or heteroarylcarbonyl;
R₁₂ represents hydrogen, alkyl which is unsubstituted or substituted with a substituent selected from R₁₈, cycloalkyl, halogenated cycloalkyl, alkenyl, halogenated alkenyl, alkynyl, halogenated alkynyl, cycloalkenyl, alkoxyalkyl, alkylthioalkyl, alkoxycarbonylalkyl, aryl, or arylalkyl;
when M is 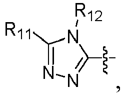 -(CH₂)₄- or -CH=CH-CH=CH- formed by R₁₁ and R₁₂, the nitrogen atom bound to R₁₂ and the carbon atom bound to R₁₁ together form a 6-membered ring;
R₁₅ represents alkyl which is unsubstituted or substituted with a substituent selected from R₂₁, cycloalkyl, halogenated cycloalkyl, alkenyl, halogenated alkenyl, alkynyl, halogenated alkynyl, cycloalkenyl, or phenyl;
R₁₆ represents alkyl, halogenated alkyl, cycloalkyl, alkenyl, halogenated alkenyl, alkynyl, halogenated alkynyl, or cycloalkenyl;
R₂₁ represents halogen, cyano, cycloalkyl, hydroxy, mercapto, alkoxy, C(O)R₂₂, carboxyl, alkoxycarbonyl, alkoxyalkoxycarbonyl, -S(O)ₘ-alkyl, heteroaryl, heterocyclyl, or phenyl which is unsubstituted or substituted with one or more groups selected from R₂₃;
R₁₇, and R₂₂ each independently represent hydrogen, alkyl, or N(R₂₄)R₂₅;
R₂₃ represents halogen, cyano, nitro, alkyl, alkyl which is unsubstituted or substituted with a substituent selected from R₃₁, cycloalkyl, halogenated cycloalkyl, alkenyl, halogenated alkenyl, alkynyl, halogenated alkynyl, cycloalkenyl, alkylcarbonyl, cycloalkylcarbonyl, halogenated alkylcarbonyl, halogenated cycloalkylcarbonyl, alkoxycarbonyl, halogenated alkoxycarbonyl, alkylaminocarbonyl, halogenated alkylaminocarbonyl, bis(alkyl)aminocarbonyl, OR₃₂, S(O)ₘR₃₃, alkylaminosulfonyl, bis(alkyl)aminosulfonyl, NH₂, alkylamino, bis(alkyl)amino, aryl, heteroaryl, or heterocyclyl;
R₂₄, and R₂₅ each independently represent hydrogen, alkyl, or phenyl; or,
alkylidene chain formed by R₂₄ and R₂₅, and the nitrogen atom(s) bound to R₂₄ and R₂₅ together form a 3-7-membered ring, said alkylidene chain optionally contains one O, S, S(O), S(O)₂, NH, orN-alkyl and optionally substituted by oxo or thio group;
R₁₃, R₁₄, R₁₈, and R₃₁ each independently represent halogen, cyano, nitro, carboxyl, alkoxycarbonyl, alkoxyalkoxycarbonyl, S(O)ₘR₄₁, OR₄₂, aryl, heteroaryl, or heterocyclyl;
R₃₂ represents hydrogen, alkyl, halogenated alkyl, cycloalkyl, halogenated cycloalkyl, alkenyl, halogenated alkenyl, alkynyl, halogenated alkynyl, or cycloalkenyl;
R₃₃ represents alkyl, halogenated alkyl, cycloalkyl, alkenyl, halogenated alkenyl, alkynyl, halogenated alkynyl, or cycloalkenyl;
R₄₁, and R₄₂ each independently represent hydrogen, alkyl, halogenated alkyl, cycloalkyl, halogenated cycloalkyl, alkenyl, halogenated alkenyl, alkynyl, halogenated alkynyl, cycloalkenyl, or phenyl;
r represents 1 or 2;
m represents 0, 1 or 2;
n represents 0, or 1;
s represents 0, 1, 2 or 3;
wherein, when X is fluorine, Y is not amino, monomethylamino, monoethylamino and monopropylamino; when M is 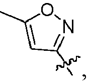 X and Y are not methyl at the same time.

2. The 4-pyridinyl formamide compound or derivative thereof according to claim 1, which is **characterized in that**,
X represent nitro, halogen, cyano, formyl, thiocyano, mercapto; C1-C8 alkyl, C2-C8 alkenyl, C2-C8 alkynyl, C3-C8 cycloalkyl, C3-C8 cycloalkenyl, C3-C8 cycloalkyl-C1-C6 alkyl, or C3-C8 cycloalkenyl-C1-C6 alkyl, which is with or without halogen; OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -(C1-C6)alkyl-C(S)R¹, -(C1-C6)alkyl-C(S)OR¹, -(C1-C6)alkyl-C(O)SR¹, -(C1-C6)alkyl-C(S)SR¹, -(C1-C6)alkyl-SC(O)R¹, -(C1-C6)alkyl-OC(S)R¹, -(C1-C6)alkyl-SC(S)R¹, -O-(C1-C6)alkyl-NR⁴R⁵, -S-(C1-C6)alkyl-NR⁴R⁵, -(C1-C6)alkyl-O-(C1-C6)alkyl-NR⁴R⁵, -(C1-C6)alkyl-S-(C1-C6)alkyl-NR⁴R⁵, -(C1-C6)alkyl-(C=S)ₙ-NR⁴R⁵, -NH-(C1-C6)alkyl-NR⁴R⁵, -(C1-C6)alkyl-OR¹, -(C1-C6)alkyl-COR¹, -(C1-C6)alkyl-CO₂R¹, -(C1-C6)alkyl-OCOR¹, -(C1-C6)alkyl-NR³COR¹, -(C1-C6)alkyl-SO₂OR¹, -(C1-C6)alkyl-NR³SO₂R², -(C1-C6)alkyl-OSO₂R², -alkyl-S(O)ₘR², -(C1-C6)alkyl-CONR⁴R⁵, -(C1-C6)alkyl-SO₂NR⁴R⁵, NR⁴R⁵, 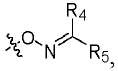 P(O)(OR⁶)₂, CH₂P(O)(OR⁶)₂, SO₂NR⁴R⁵-(C1-C6)alkyl-S(O)ₘR², -(C1-C6)alkyl-CN, -(C1-C6)alkylaryl, -(C1-C6)alkylheteroaryl, -(C1-C6)alkylheterocyclyl, aryl, heteroaryl, or heterocyclyl;
Y represent nitro, cyano, formyl, thiocyano, mercapto; C1-C8 alkyl, C2-C8 alkenyl, C2-C8 alkynyl, C3-C8 cycloalkyl, C3-C8 cycloalkenyl, C3-C8 cycloalkyl-C1-C6 alkyl, or C3-C8 cycloalkenyl-C1-C6 alkyl, which is with or without halogen; OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -(C1-C6)alkyl-C(S)R¹, -(C1-C6)alkyl-C(S)OR¹, -(C1-C6)alkyl-C(O)SR¹, -(C1-C6)alkyl-C(S)SR¹, -(C1-C6)alkyl-SC(O)R¹, -(C1-C6)alkyl-OC(S)R¹, -(C1-C6)alkyl-SC(S)R¹, -O-(C1-C6)alkyl-NR⁴R⁵, -S-(C1-C6)alkyl-NR⁴R⁵, -(C1-C6)alkyl-O-(C1-C6)alkyl-NR⁴R⁵, -(C1-C6)alkyl-S-(C1-C6)alkyl-NR⁴R⁵, -(C1-C6)alkyl-(C=S)ₙ-NR⁴R⁵, -NH-(C1-C6)alkyl-NR⁴R⁵, -(C1-C6)alkyl-OR¹, -(C1-C6)alkyl-COR¹, -(C1-C6)alkyl-CO₂R¹, -(C1-C6)alkyl-OCOR¹, -(C1-C6)alkyl-NR³COR¹, -(C1-C6)alkyl-SO₂OR¹, -(C1-C6)alkyl-NR³SO₂R², -(C1-C6)alkyl-OSO₂R², -alkyl-S(O)ₘR², -(C1-C6)alkyl-CONR⁴R⁵, -(C1-C6)alkyl-SO₂NR⁴R⁵, NR⁴R⁵, 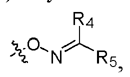 P(O)(OR⁶)₂, CH₂P(O)(OR⁶)₂, SO₂NR⁴R⁵-(C1-C6)alkyl-S(O)ₘR², -(C1-C6)alkyl-CN, -(C1-C6)alkylaryl, -(C1-C6)alkylheteroaryl, -(C1-C6)alkylheterocyclyl, aryl, heteroaryl, or heterocyclyl;
R¹, R³, R⁴, and R⁵ each independently represent hydrogen, aryl, aryl-C1-C6 alkyl, heteroaryl, heteroaryl-C1-C6 alkyl, C1-C8 alkyl, halogenated C1-C8 alkyl, C2-C8 alkenyl, halogenated C2-C8 alkenyl, C2-C8 alkynyl, halogenated C2-C8 alkynyl, C3-C8 cycloalkyl, halogenated C3-C8 cycloalkyl, C1-C8 alkoxy-C1-C6 alkyl, or C3-C8 cycloalkyl-C1-C6 alkyl, wherein the last 10 groups as mentioned are each substituted by s groups selected from the group consisting of cyano, halogen, nitro, thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and C1-C8 alkoxy-C1-C6 alkoxycarbonyl;
R² represents aryl, aryl-C1-C6 alkyl, heteroaryl, heteroaryl-C1-C6 alkyl, C1-C8 alkyl, C2-C8 alkenyl, C2-C8 alkynyl, C3-C8 cycloalkyl, or C3-C8 cycloalkyl-C1-C6 alkyl, wherein the last 5 groups as mentioned are each substituted by s groups selected from the group consisting of cyano, halogen, nitro, thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and C1-C8 alkoxy-C1-C6 alkoxycarbonyl;
R⁶ represents methyl, or ethyl;
R⁷, and R⁸ each independently represent hydrogen, C1-C8 alkyl, C2-C8 alkenyl, or C2-C8 alkynyl;
R⁹ represents C1-C8 alkyl, C2-C8 alkenyl, or C2-C8 alkynyl;
M represents 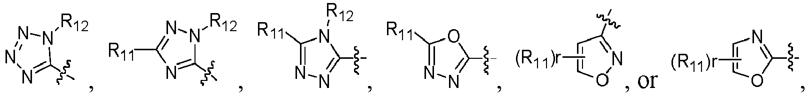 which is unsubstituted or substituted;
R₁₁ represents hydrogen, halogen, cyano, nitro, C1-C8 alkyl which is unsubstituted or substituted with a substituent selected from R₁₃, C3-C8 cycloalkyl which is unsubstituted or substituted with a substituent selected from R₁₄, C2-C8 alkenyl, halogenated C2-C8 alkenyl, C2-C8 alkynyl, halogenated C2-C8 alkynyl, C3-C8 cycloalkenyl, NH₂, aminoacyl, carboxyl, C1-C8 alkoxy-C1-C6 alkoxycarbonyl, OR₁₅, -(C1-C6)alkyl-OR₁₅, C(O)R₁₆, -(C1-C6)alkyl-C(O)R₁₆, C(O)OR₁₆, -(C1-C6)alkyl-C(O)OR₁₆, S(O)ₘR₁₆, -(C1-C6)alkyl-S(O)ₘR₁₆, -N(R₁₆)₂, -NHR₁₆, -C(O)NHR₁₆, -C(O)N(R₁₆)₂, -NHC(O)R₁₇, heterocyclyl, heterocyclyl-C1-C6 alkyl, heterocyclyloxy, heterocyclylcarbonyl, aryl, aryl-C1-C6 alkyl, aryloxy, arylcarbonyl, heteroaryl, heteroaryl-C1-C6 alkyl, heteroaryloxy, or heteroarylcarbonyl;
R₁₂ represents hydrogen, C1-C8 alkyl which is unsubstituted or substituted with a substituent selected from R₁₈, C3-C8 cycloalkyl, halogenated C3-C8 cycloalkyl, C2-C8 alkenyl, halogenated C2-C8 alkenyl, C2-C8 alkynyl, halogenated C2-C8 alkynyl, C3-C8 cycloalkenyl, C1-C8 alkoxy-C1-C6 alkyl, C1-C8 alkylthio-C1-C6 alkyl, C1-C8 alkoxycarbonyl-C1-C6 alkyl, aryl, or aryl-C1-C6 alkyl;
when M is 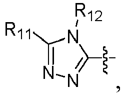 -(CH₂)₄- or -CH=CH-CH=CH- formed by R₁₁ and R₁₂, the nitrogen atom bound to R₁₂ and the carbon atom bound to R₁₁ together form a 6-membered ring;
R₁₅ represents C1-C8 alkyl which is unsubstituted or substituted with a substituent selected from R₂₁, C3-C8 cycloalkyl, halogenated C3-C8 cycloalkyl, C2-C8 alkenyl, halogenated C2-C8 alkenyl, C2-C8 alkynyl, halogenated C2-C8 alkynyl, C3-C8 cycloalkenyl, or phenyl;
R₁₆ represents C1-C8 alkyl, halogenated C1-C8 alkyl, C3-C8 cycloalkyl, C2-C8 alkenyl, halogenated C2-C8 alkenyl, C2-C8 alkynyl, halogenated C2-C8 alkynyl, or C3-C8 cycloalkenyl;
R₂₁ represents halogen, cyano, C3-C8 cycloalkyl, hydroxy, mercapto, C1-C8 alkoxy, C(O)R₂₂, carboxyl, C1-C8 alkoxycarbonyl, C1-C8 alkoxy-C1-C6 alkoxycarbonyl, -S(O)ₘ-(C1-C8)alkyl, heteroaryl, heterocyclyl, or phenyl which is unsubstituted or substituted with one or more groups selected from R₂₃;
R₁₇, and R₂₂ each independently represent hydrogen, C1-C8 alkyl, or N(R₂₄)R₂₅;
R₂₃ represents halogen, cyano, nitro, C1-C8 alkyl, C1-C8 alkyl which is unsubstituted or substituted with a substituent selected from R₃₁, C3-C8 cycloalkyl, halogenated C3-C8 cycloalkyl, C2-C8 alkenyl, halogenated C2-C8 alkenyl, C2-C8 alkynyl, halogenated C2-C8 alkynyl, C3-C8 cycloalkenyl, C1-C8 alkylcarbonyl, C3-C8 cycloalkylcarbonyl, halogenated C1-C8 alkylcarbonyl, halogenated C3-C8 cycloalkylcarbonyl, C1-C8 alkoxycarbonyl, halogenated C1-C8 alkoxycarbonyl, C1-C8 alkylaminocarbonyl, halogenated C1-C8 alkylaminocarbonyl, bis(C1-C8 alkyl)aminocarbonyl, OR₃₂, S(O)ₘR₃₃, C1-C8 alkylaminosulfonyl, bis(C1-C8 alkyl)aminosulfonyl, NH₂, C1-C8 alkylamino, bis(C1-C8 alkyl)amino, aryl, heteroaryl, or heterocyclyl;
R₂₄, and R₂₅ each independently represent hydrogen, C1-C8 alkyl, or phenyl; or,
C2-C8 alkylidene chain formed by R₂₄ and R₂₅, and the nitrogen atom(s) bound to R₂₄ and R₂₅ together form a 3-7-membered ring, said C2-C8 alkylidene chain optionally contains one O, S, S(O), S(O)₂, NH, orN-alkyl and optionally substituted by oxo or thio group;
R₁₃, R₁₄, R₁₈, and R₃₁ each independently represent halogen, cyano, nitro, carboxyl, C1-C8 alkoxycarbonyl, C1-C8 alkoxy-C1-C8 alkoxycarbonyl, S(O)ₘR₄₁, OR₄₂, aryl, heteroaryl, or heterocyclyl;
R₃₂ represents hydrogen, C1-C8 alkyl, halogenated C1-C8 alkyl, C3-C8 cycloalkyl, halogenated C3-C8 cycloalkyl, C2-C8 alkenyl, halogenated C2-C8 alkenyl, C2-C8 alkynyl, halogenated C2-C8 alkynyl, or C3-C8 cycloalkenyl;
R₃₃ represents C1-C8 alkyl, halogenated C1-C8 alkyl, C3-C8 cycloalkyl, C2-C8 alkenyl, halogenated C2-C8 alkenyl, C2-C8 alkynyl, halogenated C2-C8 alkynyl, or C3-C8 cycloalkenyl;
R₄₁, and R₄₂ each independently represent hydrogen, C1-C8 alkyl, halogenated C1-C8 alkyl, C3-C8 cycloalkyl, halogenated C3-C8 cycloalkyl, C2-C8 alkenyl, halogenated C2-C8 alkenyl, C2-C8 alkynyl, halogenated C2-C8 alkynyl, C3-C8 cycloalkenyl, or phenyl;
r represents 1, or 2;
m represents 0, 1, or 2;
n represents 0, or 1;
s represents 0, 1, 2, or 3;
wherein, the "heterocyclyl" is 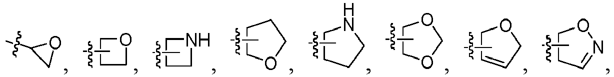 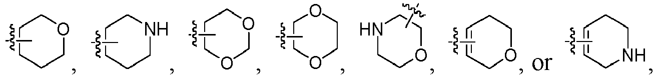 which has 0, 1, or 2 oxo groups; the "aryl" is phenyl, naphthyl, 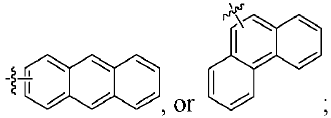 the "heteroaryl" is 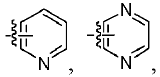 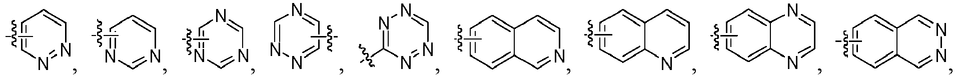 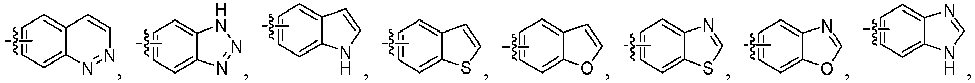 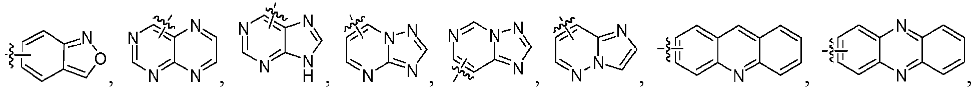 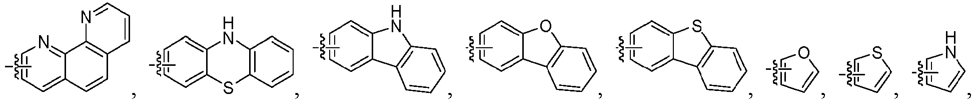 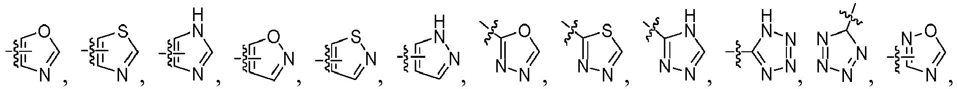 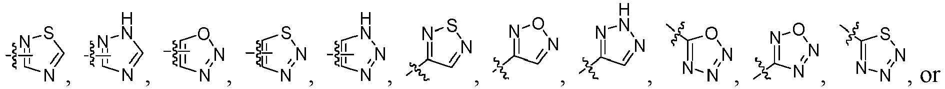 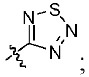 the above-mentioned groups are respectively unsubstituted or substituted by at least one group selected from: halogen, nitro, cyano, thiocyano, cyanoalkyl, mercapto, hydroxy, hydroxyalkyl, carboxyl, formyl, trialkylsilyl, dialkylphosphonyl; heterocyclyl, heterocyclylalkyl, aryl, arylalkyl, heteroaryl, or heteroarylalkyl, which is unsubstituted or substituted; alkyl, alkenyl, alkynyl, cycloalkyl, cycloalkenyl, cycloalkylalkyl, cycloalkyl substituted by alkyl, OR", SR", - alkyl-OR", -O-alkyl-OR", -alkyl-SR", COR", -alkyl-COR", -O-alkyl-COR", COOR", -alkyl-COOR", -O-alkyl-COOR", COSR", SOR", SO₂R", -O-SO₂R", -alkyl-SO₂R", OCOR", -alkyl-OCOR", or SCOR" group, which is with or without halogen; and amino, aminoalkyl, aminocarbonyl, aminocarbonylalkyl, or aminosulfonyl group which is unsubstituted or substituted by one or two groups selected from R", COR", COOR", SO₂R", and OR", wherein the R", COR", COOR", SO₂R", or OR" is with or without halogen; or, two adjacent substitutable positions of the above-mentioned "heterocyclyl", "aryl", "heteroaryl" groups are linked with -OCH₂CH₂-, -OCH₂O-, -OCH₂CH₂O- or -CH=CH-CH=CH- group to form a ring, wherein the -OCH₂CH₂-, -OCH₂O-, -OCH₂CH₂O- or -CH=CH-CH=CH- group is with or without halogen;
R" each independently represents alkyl, alkenyl, alkynyl, cycloalkyl, cycloalkylalkyl, cycloalkenyl; or heterocyclyl, heterocyclylalkyl, aryl, arylalkyl, heteroaryl, or heteroarylalkyl, which is unsubstituted or substituted.

3. The 4-pyridinyl formamide compound or derivative thereof according to claim 1 or 2, which is **characterized in that**,
X represents nitro, halogen, cyano, formyl, thiocyano, mercapto; C1-C6 alkyl, C2-C6 alkenyl, C2-C6 alkynyl, C3-C6 cycloalkyl, C3-C6 cycloalkenyl, C3-C6 cycloalkyl-C1-C3 alkyl, or C3-C6 cycloalkenyl-C1-C3 alkyl, which is with or without halogen; OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -(C1-C3)alkyl-C(S)R¹, -(C1-C3)alkyl-C(S)OR¹, -(C1-C3)alkyl-C(O)SR¹, -(C1-C3)alkyl-C(S)SR¹, -(C1-C3)alkyl-SC(O)R¹, -(C1-C3)alkyl-OC(S)R¹, -(C1-C3)alkyl-SC(S)R¹, -O-(C1-C3)alkyl-NR⁴R⁵, -S-(C1-C3)alkyl-NR⁴R⁵, -(C1-C3)alkyl-O-(C1-C3)alkyl-NR⁴R⁵, -(C1-C3)alkyl-S-(C1-C3)alkyl-NR⁴R⁵, -(C1-C3)alkyl-(C=S)ₙ-NR⁴R⁵, -NH-(C1-C3)alkyl-NR⁴R⁵, -(C1-C3)alkyl-OR¹, -(C1-C3)alkyl-COR¹, -(C1-C3)alkyl-CO₂R¹, -(C1-C3)alkyl-OCOR¹, -(C1-C3)alkyl-NR³COR¹, -(C1-C3)alkyl-SO₂OR¹, -(C1-C3)alkyl-NR³SO₂R², -(C1-C3)alkyl-OSO₂R², -alkyl-S(O)ₘR², -(C1-C3)alkyl-CONR⁴R⁵, -(C1-C3)alkyl-SO₂NR⁴R⁵, NR⁴R⁵, 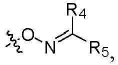 P(O)(OR⁶)₂, CH₂P(O)(OR⁶)₂, SO₂NR⁴R⁵-(C1-C3)alkyl-S(O)ₘR², -(C1-C3)alkyl-CN, -(C1-C3)alkylaryl, -(C1-C3)alkylheteroaryl, -(C1-C3)alkylheterocyclyl, aryl, heteroaryl, or heterocyclyl;
Y represents C1-C6 alkyl, C2-C6 alkenyl, C2-C6 alkynyl, C3-C6 cycloalkyl, or C3-C6 cycloalkenyl, which is with or without halogen; OR¹, S(O)ₘR², NR⁴R⁵, heterocyclyl, aryl, or heteroaryl;
R¹, R³, R⁴, and R⁵ each independently represent hydrogen, aryl, aryl-C1-C3 alkyl, heteroaryl, heteroaryl-C1-C3 alkyl, C1-C6 alkyl, halogenated C1-C6 alkyl, C2-C6 alkenyl, halogenated C2-C6 alkenyl, C2-C6 alkynyl, halogenated C2-C6 alkynyl, C3-C6 cycloalkyl, halogenated C3-C6 cycloalkyl, C1-C6 alkoxy-C1-C3 alkyl, or C3-C6 cycloalkyl-C1-C3 alkyl, wherein the last 10 groups as mentioned are each substituted by s groups selected from the group consisting of cyano, halogen, nitro, thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and C1-C6 alkoxy-C1-C3 alkoxycarbonyl;
R² represents aryl, aryl-C1-C3 alkyl, heteroaryl, heteroaryl-C1-C3 alkyl, C1-C6 alkyl, C2-C6 alkenyl, C2-C6 alkynyl, C3-C6 cycloalkyl, or C3-C6 cycloalkyl-C1-C3 alkyl, wherein the last 5 groups as mentioned are each substituted by s groups selected from the group consisting of cyano, halogen, nitro, thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸, and C1-C6 alkoxy-C1-C3 alkoxycarbonyl;
R⁶ represents methyl, or ethyl;
R⁷, and R⁸ each independently represent hydrogen, C1-C6 alkyl, C2-C6 alkenyl, or C2-C6 alkynyl;
R⁹ represents C1-C6 alkyl, C2-C6 alkenyl, or C2-C6 alkynyl;
M represents 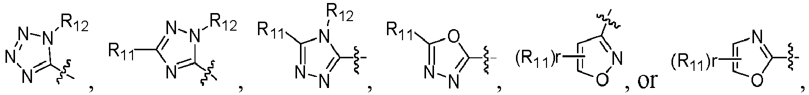 which is unsubstituted or substituted;
R₁₁ represents hydrogen, halogen, cyano, nitro, C1-C6 alkyl which is unsubstituted or substituted with a substituent selected from R₁₃, C3-C6 cycloalkyl which is unsubstituted or substituted with a substituent selected from R₁₄, C2-C6 alkenyl, halogenated C2-C6 alkenyl, C2-C6 alkynyl, halogenated C2-C6 alkynyl, C3-C6 cycloalkenyl, NH₂, aminoacyl, carboxyl, C1-C6 alkoxy-C1-C3 alkoxycarbonyl, OR₁₅, -(C1-C3)alkyl-OR₁₅, C(O)R₁₆, -(C1-C3)alkyl-C(O)R₁₆, C(O)OR₁₆, -(C1-C3)alkyl-C(O)OR₁₆, S(O)ₘR₁₆, -(C1-C3)alkyl-S(O)ₘR₁₆, -N(R₁₆)₂, -NHR₁₆, -C(O)NHR₁₆, -C(O)N(R₁₆)₂, -NHC(O)R₁₇, heterocyclyl, heterocyclyl-C1-C3 alkyl, heterocyclyloxy, heterocyclylcarbonyl, aryl, aryl-C1-C3 alkyl, aryloxy, arylcarbonyl, heteroaryl, heteroaryl-C1-C3 alkyl, heteroaryloxy, or heteroarylcarbonyl;
R₁₂ represents hydrogen, C1-C6 alkyl which is unsubstituted or substituted with a substituent selected from R₁₈, C3-C6 cycloalkyl, halogenated C3-C6 cycloalkyl, C2-C6 alkenyl, halogenated C2-C6 alkenyl, C2-C6 alkynyl, halogenated C2-C6 alkynyl, C3-C6 cycloalkenyl, C1-C6 alkoxy-C1-C3 alkyl, C1-C6 alkylthio-C1-C3 alkyl, C1-C6 alkoxycarbonyl-C1-C3 alkyl, aryl, or aryl-C1-C3 alkyl;
when M is 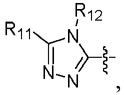 -(CH₂)₄- or -CH=CH-CH=CH- formed by R₁₁ and R₁₂, the nitrogen atom bound to R₁₂ and the carbon atom bound to R₁₁ together form a 6-membered ring;
R₁₅ represents C1-C6 alkyl which is unsubstituted or substituted with a substituent selected from R₂₁, C3-C6 cycloalkyl, halogenated C3-C6 cycloalkyl, C2-C6 alkenyl, halogenated C2-C6 alkenyl, C2-C6 alkynyl, halogenated C2-C6 alkynyl, C3-C6 cycloalkenyl, or phenyl;
R₁₆ represents C1-C6 alkyl, halogenated C1-C6 alkyl, C3-C6 cycloalkyl, C2-C6 alkenyl, halogenated C2-C6 alkenyl, C2-C6 alkynyl, halogenated C2-C6 alkynyl, or C3-C6 cycloalkenyl;
R₂₁ represents halogen, cyano, C3-C6 cycloalkyl, hydroxy, mercapto, C1-C6 alkoxy, C(O)R₂₂, carboxyl, C1-C6 alkoxycarbonyl, C1-C6 alkoxy-C1-C3 alkoxycarbonyl, -S(O)ₘ-(C1-C6)alkyl, heteroaryl, heterocyclyl, or phenyl which is unsubstituted or substituted with 1~3 groups selected from R₂₃;
R₁₇, and R₂₂ each independently represent hydrogen, C1-C6 alkyl, or N(R₂₄)R₂₅;
R₂₃ represents halogen, cyano, nitro, C1-C6 alkyl, C1-C6 alkyl which is unsubstituted or substituted with a substituent selected from R₃₁, C3-C6 cycloalkyl, halogenated C3-C6 cycloalkyl, C2-C6 alkenyl, halogenated C2-C6 alkenyl, C2-C6 alkynyl, halogenated C2-C6 alkynyl, C3-C6 cycloalkenyl, C1-C6 alkylcarbonyl, C3-C6 cycloalkylcarbonyl, halogenated C1-C6 alkylcarbonyl, halogenated C3-C6 cycloalkylcarbonyl, C1-C6 alkoxycarbonyl, halogenated C1-C6 alkoxycarbonyl, C1-C6 alkylaminocarbonyl, halogenated C1-C6 alkylaminocarbonyl, bis(C1-C6 alkyl)aminocarbonyl, OR₃₂, S(O)ₘR₃₃, C1-C6 alkylaminosulfonyl, bis(C1-C6 alkyl)aminosulfonyl, NH₂, C1-C6 alkylamino, bis(C1-C6 alkyl)amino, aryl, heteroaryl, or heterocyclyl;
R₂₄, and R₂₅ each independently represent hydrogen, C1-C6 alkyl, or phenyl; or,
C2-C6 alkylidene chain formed by R₂₄ and R₂₅, and the nitrogen atom(s) bound to R₂₄ and R₂₅ together form a 3-7-membered ring, said C2-C6 alkylidene chain optionally contains one O, S, S(O), S(O)₂, NH, orN-alkyl and optionally substituted by oxo or thio group;
R₁₃, R₁₄, R₁₈, and R₃₁ each independently represent halogen, cyano, nitro, carboxyl, C1-C6 alkoxycarbonyl, C1-C6 alkoxy-C1-C6 alkoxycarbonyl, S(O)ₘR₄₁, OR₄₂, aryl, heteroaryl, or heterocyclyl;
R₃₂ represents hydrogen, C1-C6 alkyl, halogenated C1-C6 alkyl, C3-C6 cycloalkyl, halogenated C3-C6 cycloalkyl, C2-C6 alkenyl, halogenated C2-C6 alkenyl, C2-C6 alkynyl, halogenated C2-C6 alkynyl, or C3-C6 cycloalkenyl;
R₃₃ represents C1-C6 alkyl, halogenated C1-C6 alkyl, C3-C6 cycloalkyl, C2-C6 alkenyl, halogenated C2-C6 alkenyl, C2-C6 alkynyl, halogenated C2-C6 alkynyl, or C3-C6 cycloalkenyl;
R₄₁, and R₄₂ each independently represent hydrogen, C1-C6 alkyl, halogenated C1-C6 alkyl, C3-C6 cycloalkyl, halogenated C3-C6 cycloalkyl, C2-C6 alkenyl, halogenated C2-C6 alkenyl, C2-C6 alkynyl, halogenated C2-C6 alkynyl, C3-C6 cycloalkenyl, or phenyl;
r represents 1, or 2;
m represents 0, 1, or 2;
n represents 0, or 1;
s represents 0, 1, 2, or 3;
wherein, the "heterocyclyl" is 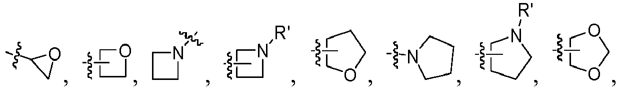 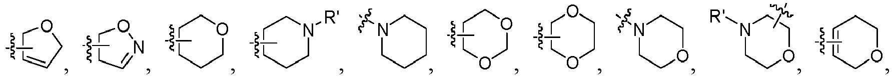 or 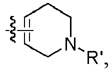 which has 0, 1, or 2 oxo groups; the "aryl" is phenyl, naphthyl, 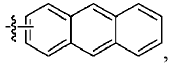 or 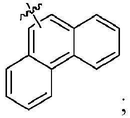 the "heteroaryl" is 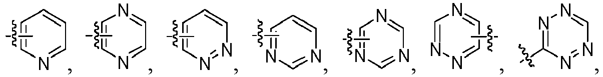 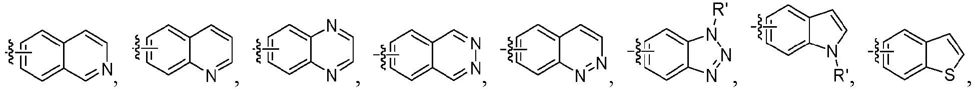 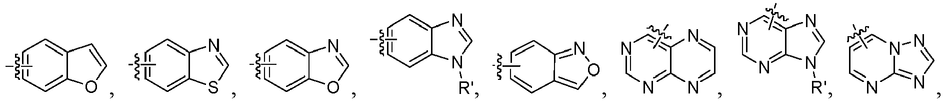 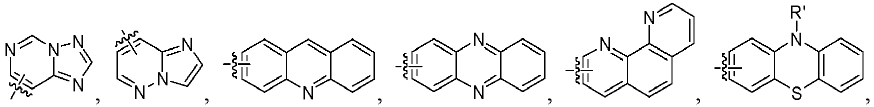 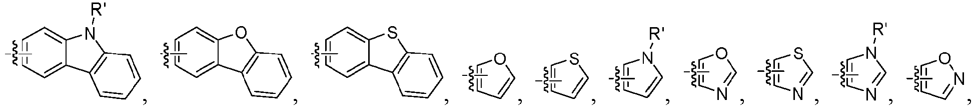 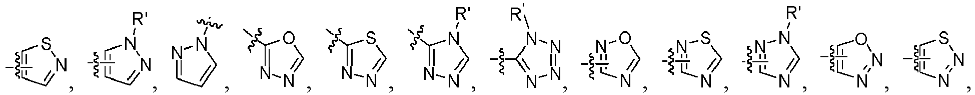 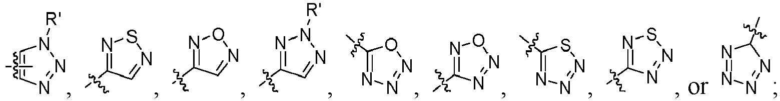 the above-mentioned groups are respectively unsubstituted or substituted by 1~3 groups selected from: halogen, nitro, cyano, thiocyano, cyano C1-C6 alkyl, mercapto, hydroxy, hydroxy C1-C6 alkyl, carboxyl, formyl; 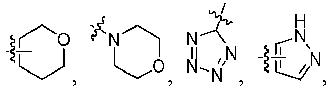 phenyl, or benzyl group, which is unsubstituted or substituted by 1~3 groups selected from halogen, hydroxy, nitro, cyano, amino, carboxyl, C1-C6 alkyl with or without halogen, C2-C6 alkenyl with or without halogen, C2-C6 alkynyl with or without halogen, C3-C6 cycloalkyl with or without halogen, C1-C6 alkoxy with or without halogen, C1-C6 alkoxycarbonyl with or without halogen, C1-C6 alkylacyl with or without halogen, C1-C6 alkylacyloxy with or without halogen, C1-C6 alkylamino with or without halogen, and C1-C6 alkylsulfonyl with or without halogen; C1-C6 alkyl, C2-C6 alkenyl, C2-C6 alkynyl, C3-C6 cycloalkyl, C3-C6 cycloalkyl-C1-C6 alkyl, C3-C6 cycloalkyl substituted by C1-C6 alkyl, OR", SR", -(C1-C6) alkyl -OR", -O-(C1-C6) alkyl -OR", -(C1-C6) alkyl -SR", COR", -(C1-C6) alkyl -COR", -O-(C1-C6) alkyl -COR", COOR", -(C1-C6) alkyl -COOR", -O-(C1-C6) alkyl -COOR", COSR", SOR", SO₂R", -O-SO₂R", -(C1-C6) alkyl -SO₂R", OCOR", -(C1-C6) alkyl -OCOR", or SCOR" group, which is with or without halogen; and amino, aminoalkyl, aminocarbonyl, or aminosulfonyl group, which is unsubstituted or substituted by one or two groups selected from R", COR", COOR", SO₂R", and OR", wherein the R", COR", COOR", SO₂R", or OR" is with or without halogen; or, two adjacent substitutable positions of the above-mentioned "heterocyclyl", "aryl", "heteroaryl" groups are linked with -OCH₂CH₂-, -OCH₂O-, -OCH₂CH₂O-, or -CH=CH-CH=CH- group to form a ring, wherein the -OCH₂CH₂-, -OCH₂O-, -OCH₂CH₂O-, or -CH=CH-CH=CH- group is with or without halogen;
R' each independently represents hydrogen, nitro, hydroxy, amino; C1-C6 alkyl, C2-C6 alkenyl, C2-C6 alkynyl, C3-C6 cycloalkyl, C3-C6 cycloalkenyl, C3-C6 cycloalkyl-C1-C6 alkyl, 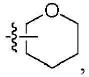 C1-C6 alkoxy, C2-C6 alkenyloxy, C2-C6 alkynyloxy, C3-C6 cycloalkyloxy, C1-C6 alkoxy-C1-C6 alkyl, C1-C6 alkoxycarbonyl, C1-C6 alkylthiocarbonyl, C1-C6 alkylsulfonyl, C1-C6 alkylsulfonyl-C1-C6 alkyl, C1-C6 alkylcarbonyl, C1-C6 alkylcarbonyl-C1-C6 alkyl, C1-C6 alkylacyloxy, C1-C6 alkylamino, C1-C6 alkylaminocarbonyl, C1-C6 alkoxyaminocarbonyl, C1-C6 alkoxycarbonyl-C1-C6 alkyl, C1-C6 alkylaminocarbonyl-C1-C6 alkyl, tri(C1-C6 alkyl)silyl, or di(C1-C6 alkyl)phosphonyl, which is with or without halogen; or phenyl, or benzyl which is unsubstituted or substituted by 1~3 groups selected from halogen, hydroxy, nitro, cyano, amino, carboxyl, C1-C6 alkyl with or without halogen, C2-C6 alkenyl with or without halogen, C2-C6 alkynyl with or without halogen, C3-C6 cycloalkyl with or without halogen, C1-C6 alkoxy with or without halogen, C1-C6 alkoxy carbonyl with or without halogen, C1-C6 alkylacyl with or without halogen, C1-C6 alkylacyloxy with or without halogen, C1-C6 alkylamino with or without halogen, and C1-C6 alkylsulfonyl with or without halogen;
R" each independently represents C1-C6 alkyl, C2-C6 alkenyl, C2-C6 alkynyl, C3-C6 cycloalkyl, C3-C6 cycloalkyl-C1-C6 alkyl, C3-C6 cycloalkenyl; or 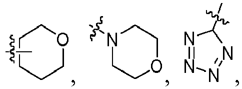 phenyl, or benzyl which is unsubstituted or substituted by 1~3 groups selected from halogen, hydroxy, nitro, cyano, amino, carboxyl, C1-C6 alkyl with or without halogen, C2-C6 alkenyl with or without halogen, C2-C6 alkynyl with or without halogen, C3-C6 cycloalkyl with or without halogen, C1-C6 alkoxy with or without halogen, C1-C6 alkoxycarbonyl with or without halogen, C1-C6 alkylacyl with or without halogen, C1-C6 alkylacyloxy with or without halogen, C1-C6 alkylamino with or without halogen, and C1-C6 alkylsulfonyl with or without halogen.

4. The 4-pyridinyl formamide compound or derivative thereof according to any one of claims 1 to 3, which is **characterized in that**,
X represents halogen, nitro, cyano, OR¹, S(O)ₘR², NR³COR¹, NR⁴R⁵, 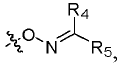 C1-C6 alkoxy-C1-C3 alkyl; C1-C6 alkyl, C2-C6 alkenyl, C2-C6 alkynyl, or C3-C6 cycloalkyl, which is with or without halogen; 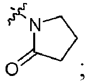 or phenyl which is unsubstituted or substituted with 1, 2, or 3 groups selected from halogen, and C1-C6 alkoxy;
Y represents C1-C6 alkyl, C2-C6 alkenyl, C2-C6 alkynyl, C3-C6 cycloalkyl, or C3-C6 cycloalkenyl, which is with or without halogen; OR¹, S(O)ₘR², NR⁴R⁵, heterocyclyl, aryl, or heteroaryl;
R¹, R³, R⁴, and R⁵ each independently represent hydrogen, C1-C6 alkyl, halogenated C1-C6 alkyl, phenyl; or benzyl which is unsubstituted or substituted with 1, 2, or 3 groups selected from halogen, and C1-C6 alkoxy;
R² represents C1-C6 alkyl, or halogenated C1-C6 alkyl;
M represents 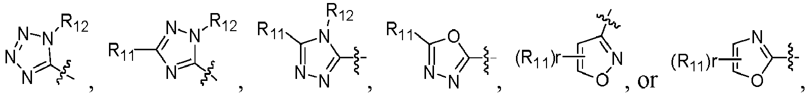 which is unsubstituted or substituted;
R₁₁ represents hydrogen, C1-C6 alkyl, C3-C6 cycloalkyl, halogenated C1-C6 alkyl, C1-C6 alkoxy, C1-C6 alkylthio, halogen, C1-C6 alkylamino, bis(C1-C6 alkyl)amino, cyano, nitro, C1-C6 alkylcarbonyl, C1-C6 alkoxycarbonyl, C1-C6 alkoxycarbonyl-C1-C3 alkyl, aminocarbonyl, C1-C6 alkylcarbonylamino, C1-C6 alkoxy-C1-C3 alkyl, C1-C6 alkylthio-C1-C3 alkyl, C1-C6 alkylsulfinyl-C1-C3 alkyl, C1-C6 alkylsulfonyl-C1-C3 alkyl; or benzyl, phenoxy, benzoyl, pyridyl, 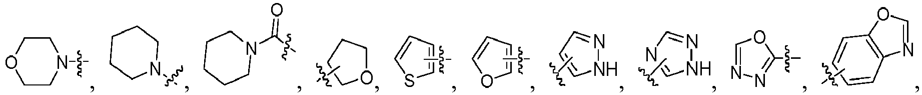 or 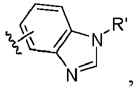 which is unsubstituted or substituted with 1, 2, or 3 groups selected from halogen, and C1-C6 alkoxy;
R₁₂ represents hydrogen, C1-C6 alkyl, C2-C6 alkenyl, C1-C6 alkoxy-C1-C3 alkyl, C1-C6 alkylthio-C1-C3 alkyl, C1-C6 alkoxycarbonyl-C1-C3 alkyl; or phenyl, or benzyl which is unsubstituted or substituted with at least one group selected from halogen, and halogenated C1-C6 alkyl;
r represents 1, or 2;
m represents 0, 1, or 2;
wherein, the "heterocyclyl" is 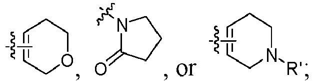 the "aryl" is phenyl, naphthyl, or 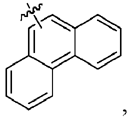 which is unsubstituted or substituted; the "heteroaryl" is 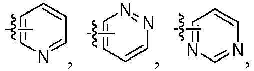 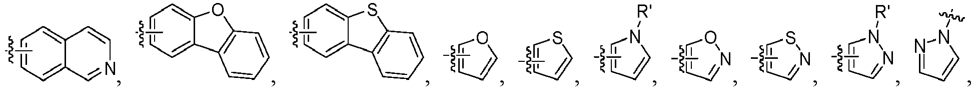 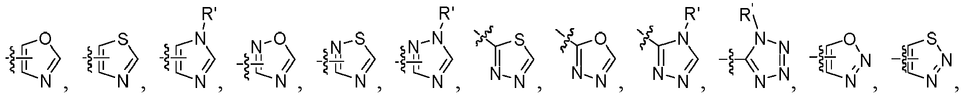 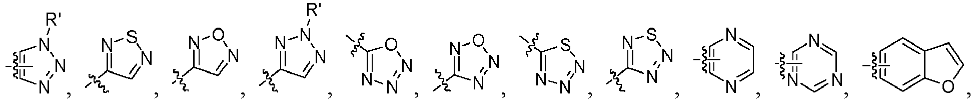 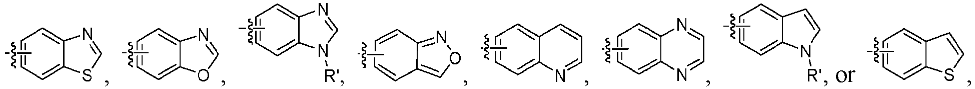 which is unsubstituted or substituted; the aforementioned "substituted" respectively refers to being substituted by 1~3 groups selected from: halogen, nitro, cyano, thiocyano, cyano C1-C3 alkyl, hydroxy, hydroxy C1-C3 alkyl, mercapto, carboxyl, formyl, 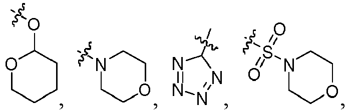 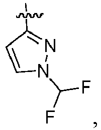 phenyl, phenyl substituted by C1-C6 alkyl, phenoxy, benzyloxy; amino, aminoalkyl, or aminocarbonyl group which is unsubstituted or substituted by one or two groups selected from C1-C6 alkyl, COR", and COOR"; and C1-C6 alkyl, C2-C6 alkenyl, C3-C6 cycloalkyl, C1-C6 alkoxy-C1-C3 alkyl, C1-C6 alkylthio-C1-C3 alkyl, C1 -C6 alkylcarbonylthio, C3-C6 cycloalkyl substituted with C1-C6 alkyl, OR", SR", SOR", COR", COOR", or SO₂R", which is with or without halogen; or, two adjacent substitutable positions of the above-mentioned "heterocyclyl", "aryl", "heteroaryl" groups are linked with -OCH₂CH₂-, -OCH₂O-, -OCH₂CH₂O-, or -CH=CH-CH=CH-group to form a ring, wherein the -OCH₂CH₂-, -OCH₂O-, -OCH₂CH₂O-, or -CH=CH-CH=CH-group, which is with or without halogen;
R' each independently represents hydrogen, C1-C6 alkyl, C1-C6 alkylcarbonyl, halogenated C1-C6 alkyl, 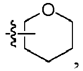 C1-C6 alkoxycarbonyl, C1-C6 alkoxy-C1-C3 alkyl, or benzyl;
R" each independently represents C1-C6 alkyl, or C2-C6 alkenyl.

5. The 4-pyridinyl formamide compound or derivative thereof according to any one of claims 1 to 4, which is **characterized in that**,
X represents chlorine, fluorine, bromine, methyl, ethyl, isopropyl, vinyl, allyl, ethynyl, cyclopropyl, trifluoromethyl, 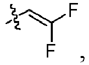 hydroxy, methoxy, ethyloxy, methylthio, ethylthio, 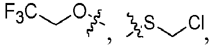 nitro, cyano, methylsulfinyl, ethylsulfinyl, methylsulfonyl, amino, monomethylamino, dimethylamino, acetamido, 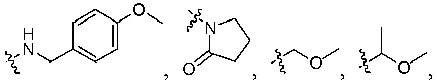 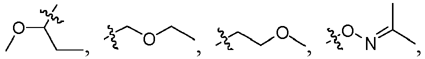 phenyl, phenoxy, or 4-fluorophenyl;
Y represents methyl, ethyl, vinyl, methoxy, 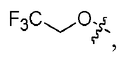 phenoxy, benzyloxy, methylthio, propylthio, tert-butylthio, benzylthio, 4-methoxybenzylthiol, propylsulfinyl, amino, monomethylamino, dimethylamino, anilino, p-methoxybenzylamino, tert-butyl, cyclopropyl, cyclohexyl, 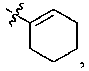 heterocyclyl, aryl, or heteroaryl;
M represents 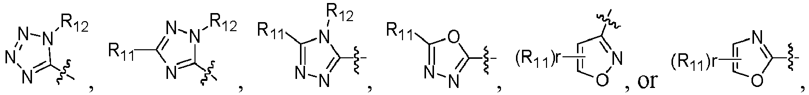 which is unsubstituted or substituted;
R₁₁ each independently represents hydrogen, methyl, ethyl, n-propyl, isopropyl, cyclopropyl, monochloromethyl, trifluoromethyl, methoxy, isopropyloxy, methylthio, fluorine, chlorine, bromine, iodine, dimethylamino, ethylamino, cyano, nitro, acetyl, methoxycarbonyl; phenyl which is unsubstituted or substituted by 1, 2, or 3 groups selected from chlorine, and methoxy; pyridyl, 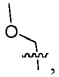 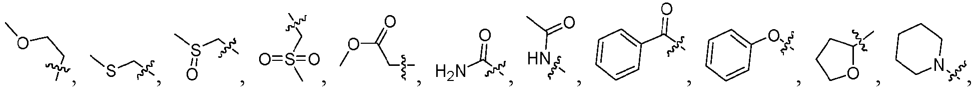 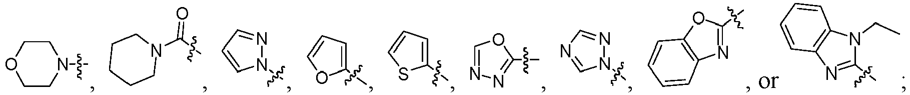
R₁₂ each independently represents hydrogen, methyl, ethyl, n-propyl, isopropyl, allyl, 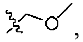 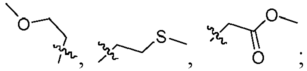 or phenyl, or benzyl which is unsubstituted or substituted by 1, 2, or 3 groups selected from chlorine, and trifluoromethyl;
r represents 1, or 2;
wherein, the "heterocyclyl" is 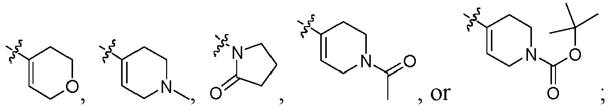 the "aryl" is phenyl, naphthyl, or 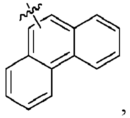 which is unsubstituted or substituted; the "heteroaryl" is 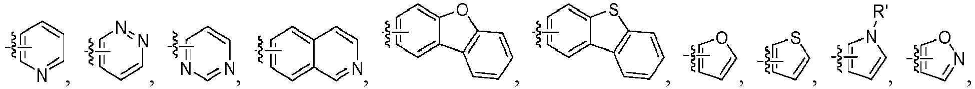 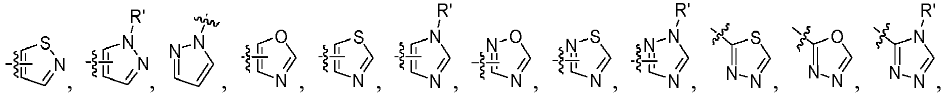 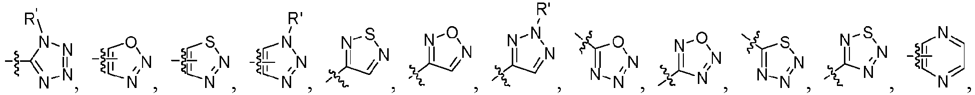 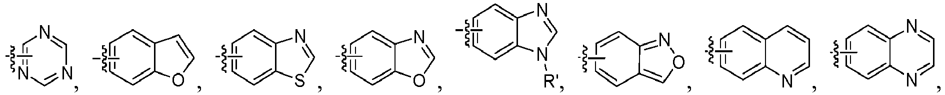 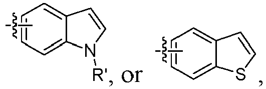 which is unsubstituted or substituted; the aforementioned "substituted" refers to being substituted by 1, 2, or 3 groups selected from: methyl, ethyl, isopropyl, n-butyl, tert-butyl, n-pentyl, vinyl, cyclopropyl, 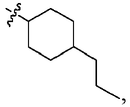 fluorine, chlorine, bromine, iodine, monobromomethyl, difluoromethyl, trifluoromethyl, methoxy, ethoxy, propoxy, butoxy, trifluoromethoxy, hydroxyl, hydroxymethyl, amino, cyano, cyanomethyl, thiocyano, mercapto, nitro, carboxy, formyl, acetyl, methoxycarbonyl, tert-butyloxycarbonyl, methylthio, isopropylthio, methylsulfinyl, methylsulfonyl, 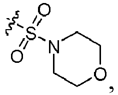 dimethylamino, aminocarbonyl, dimethylaminocarbonyl, acetylamino, 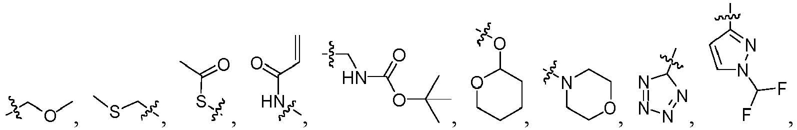 phenyl, 4-ethylphenyl, phenoxy, and benzyloxy; or, two adjacent substitutable positions of the above-mentioned "aryl", "heteroaryl" groups are linked with -OCH₂CH₂-, -OCH₂O-, -OCH₂CH₂O-, or -CH=CH-CH=CH- group to form a ring, wherein the -OCH₂CH₂-, -OCH₂O-, -OCH₂CH₂O-, or -CH=CH-CH=CH- group is with or without halogen;
R' each independently represents hydrogen, methyl, ethyl, n-propyl, isopropyl, difluoromethyl, 3,3,3-trifluoroethyl, benzyl, 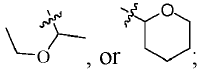
wherein, when X is fluorine, Y is not amino and monomethylamino; when M is 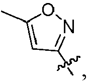 X and Y are not methyl at the same time.

6. The 4-pyridinyl formamide compound or derivative thereof according to any one of claims 1 to 5, which is **characterized in that**, the 4-pyridinyl formamide compound is represented by Formula I: 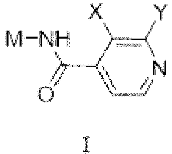 wherein, X, Y and M are listed as follows:

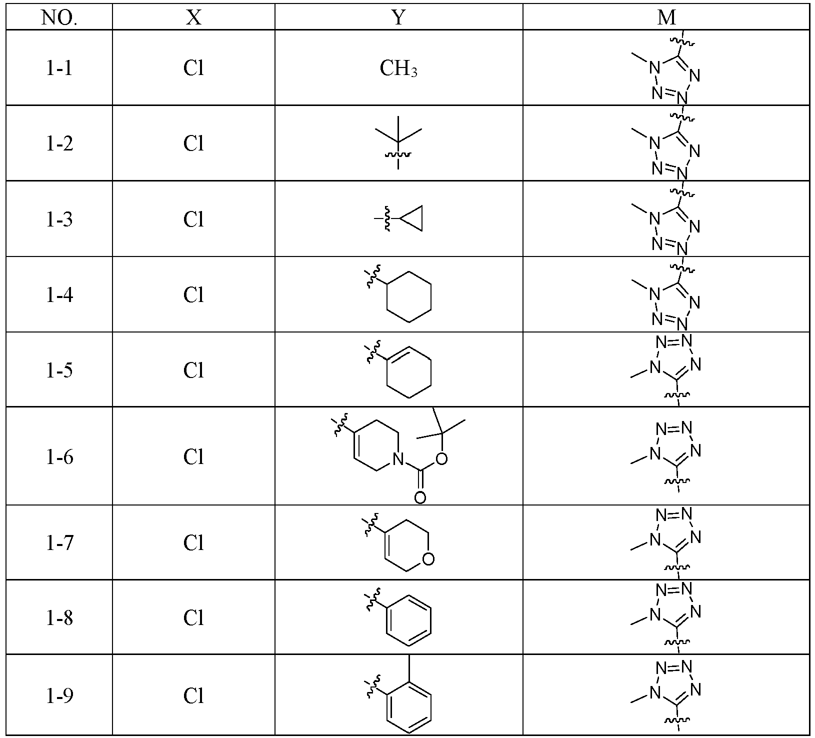

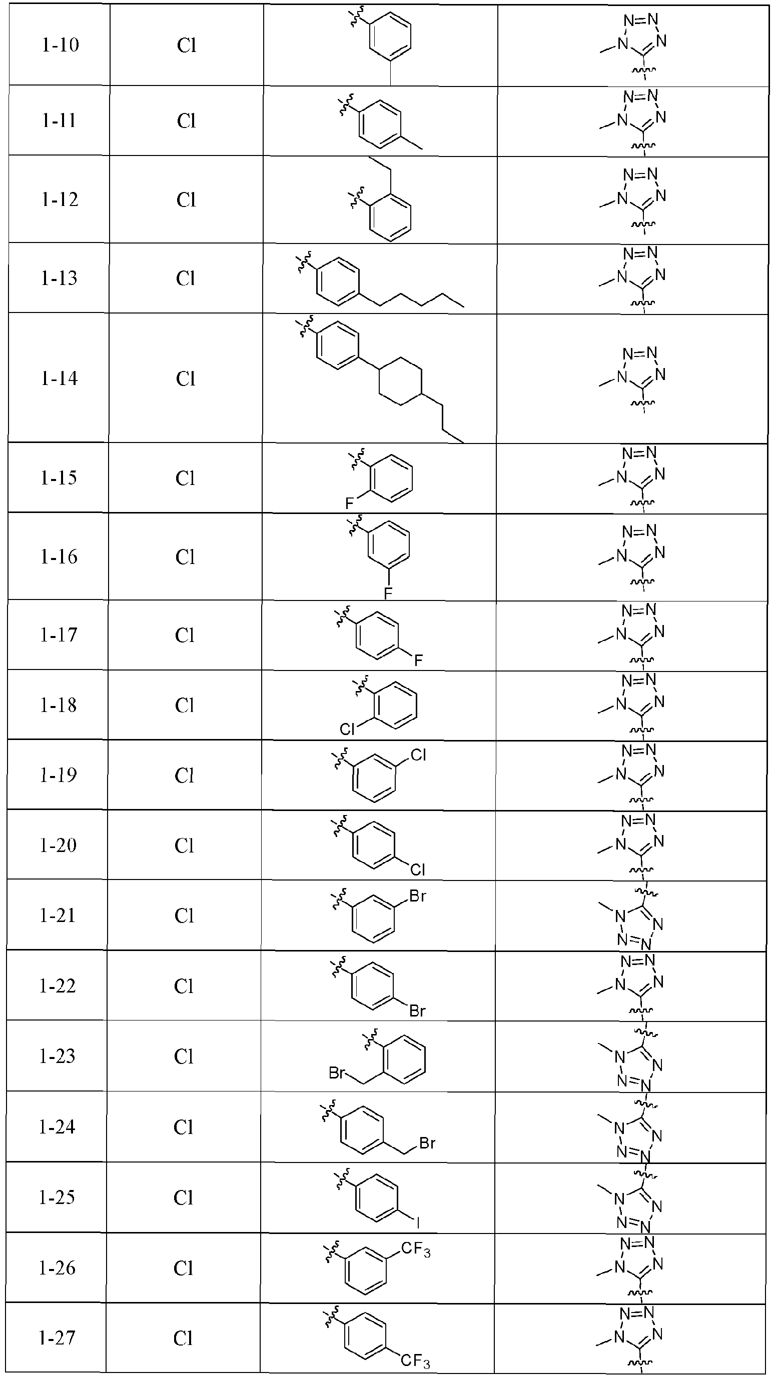

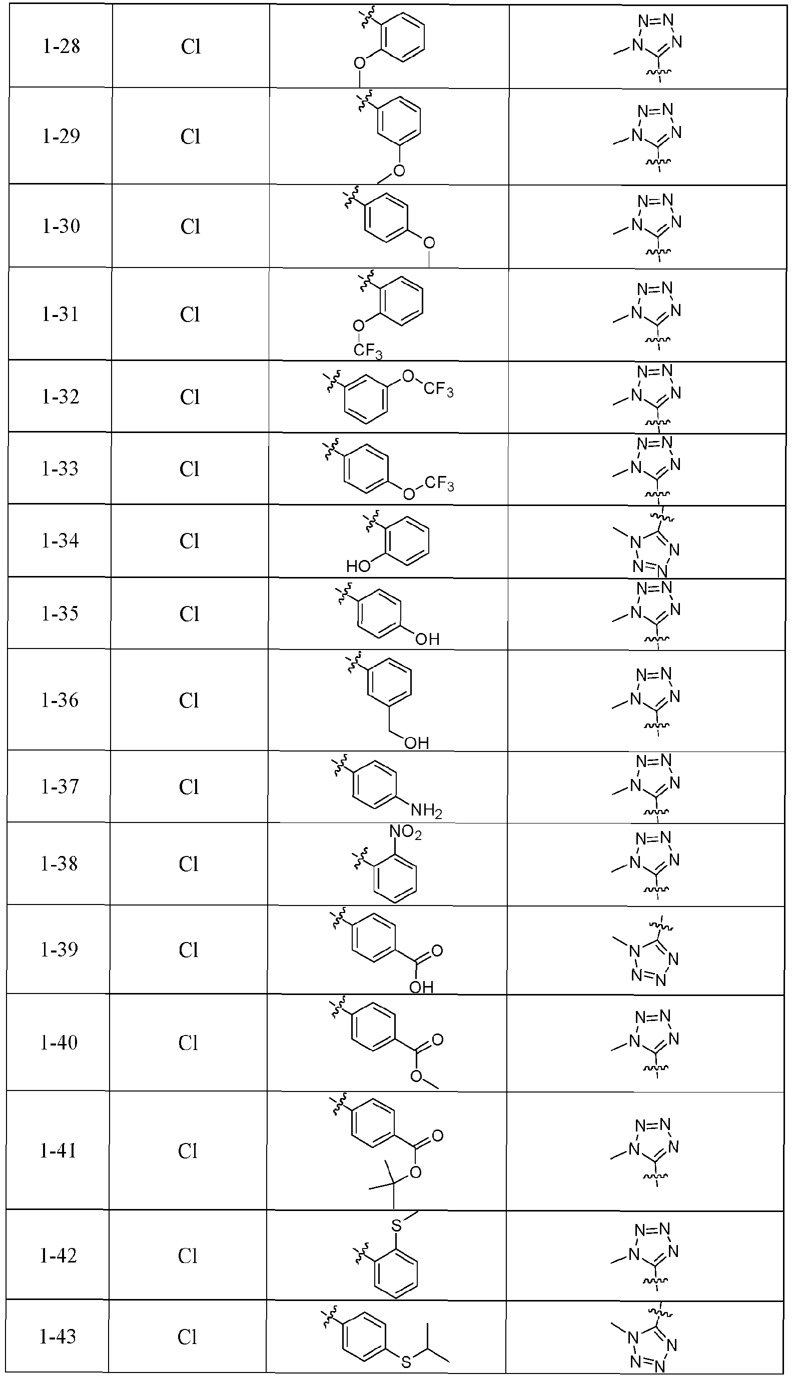

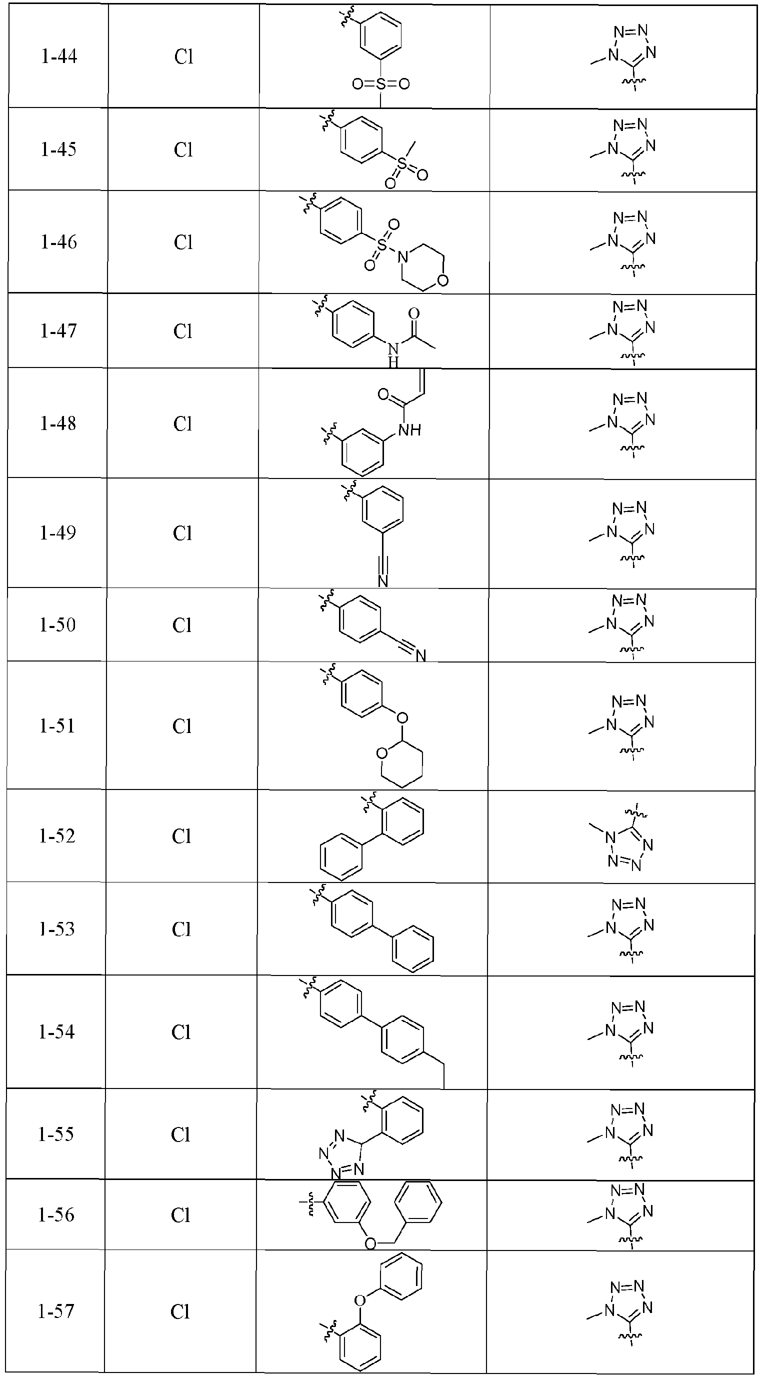

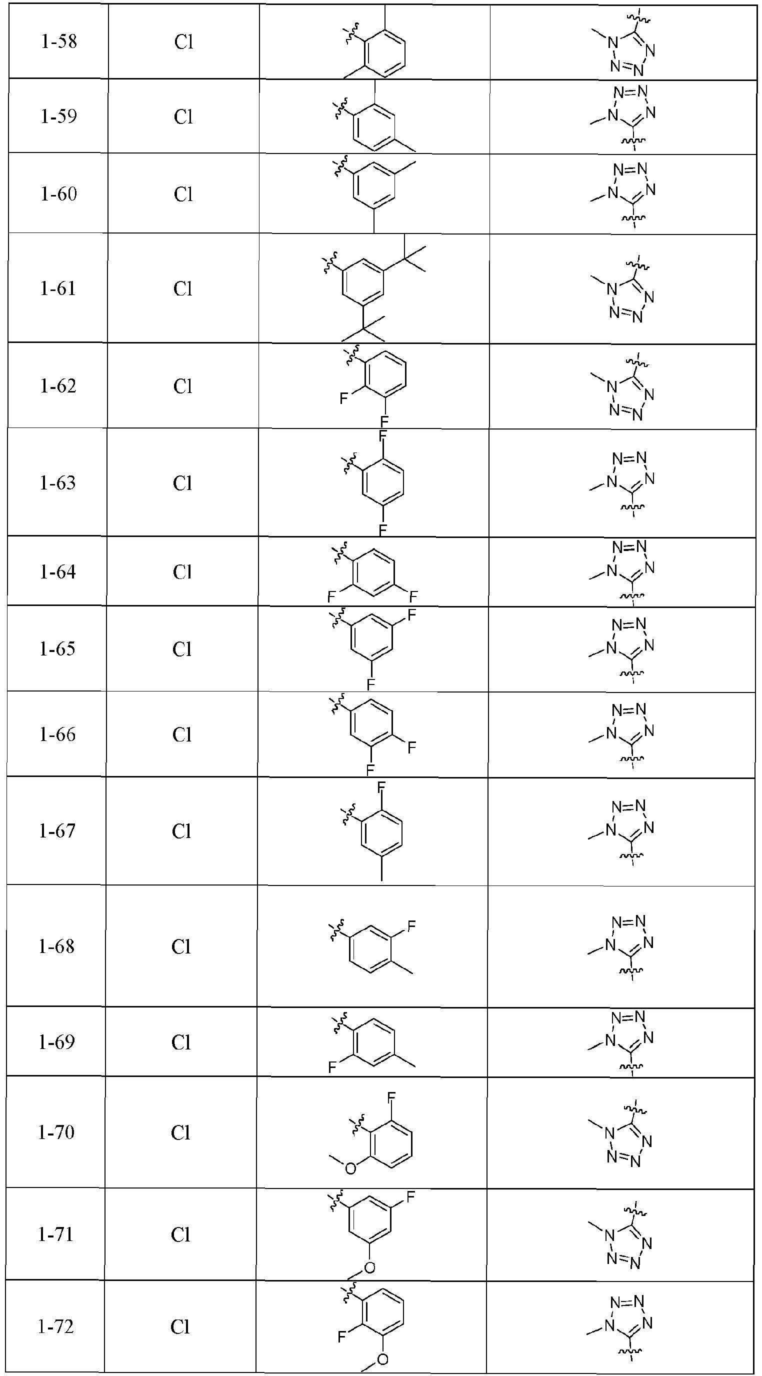

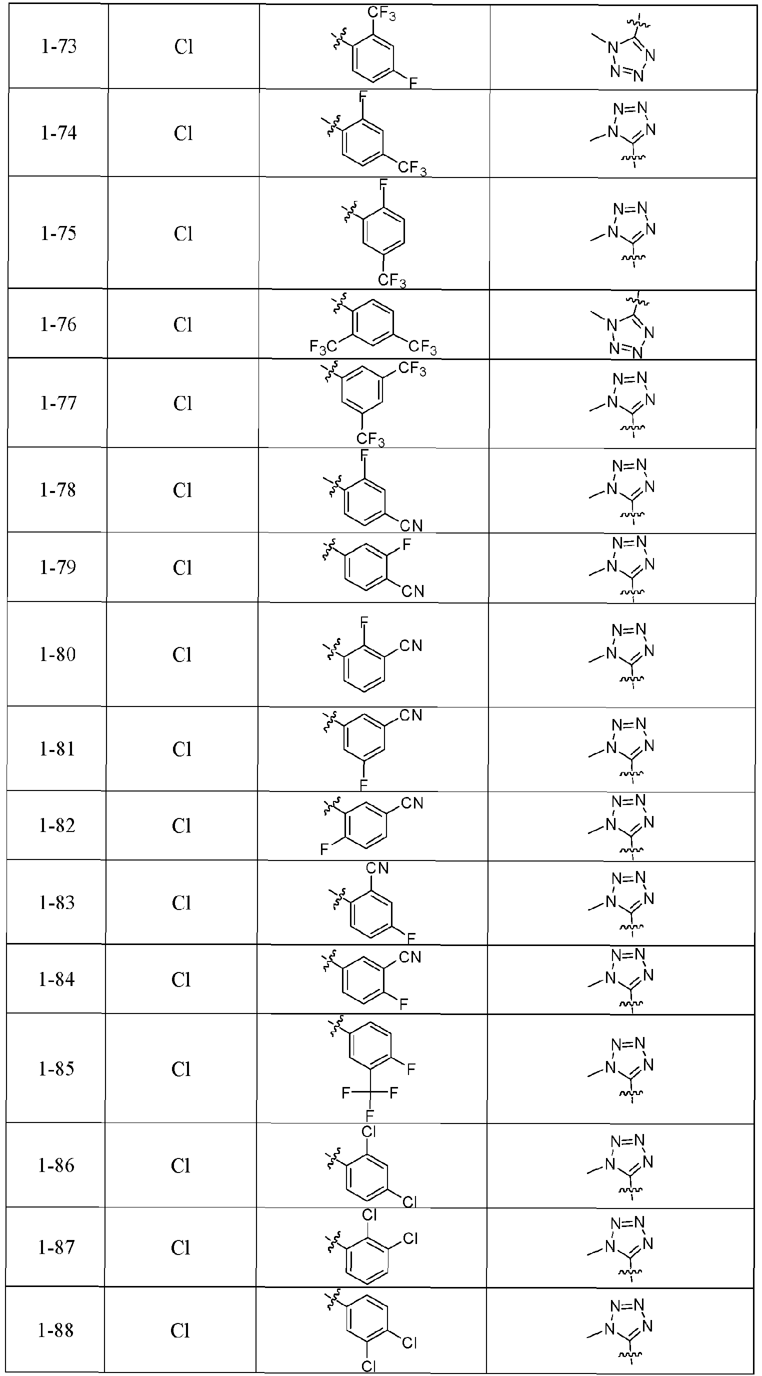

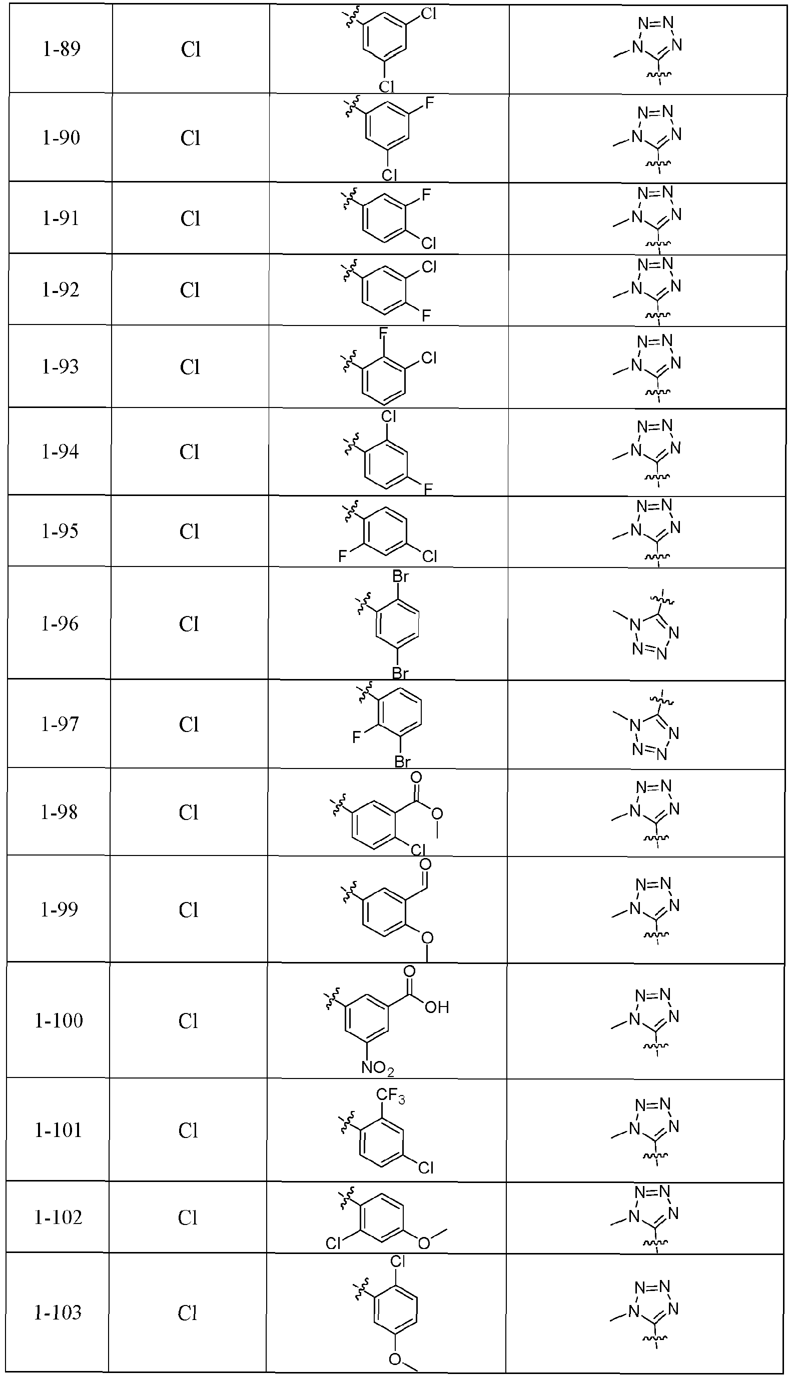

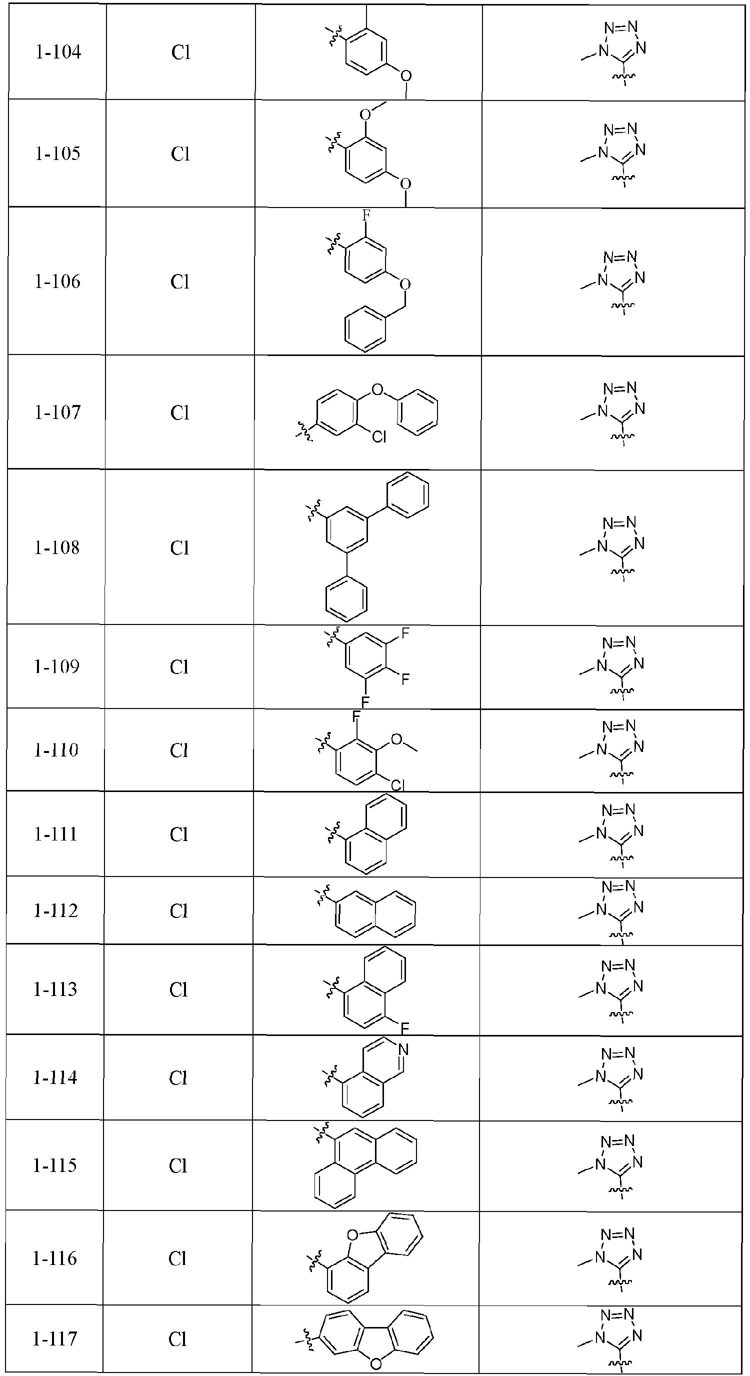

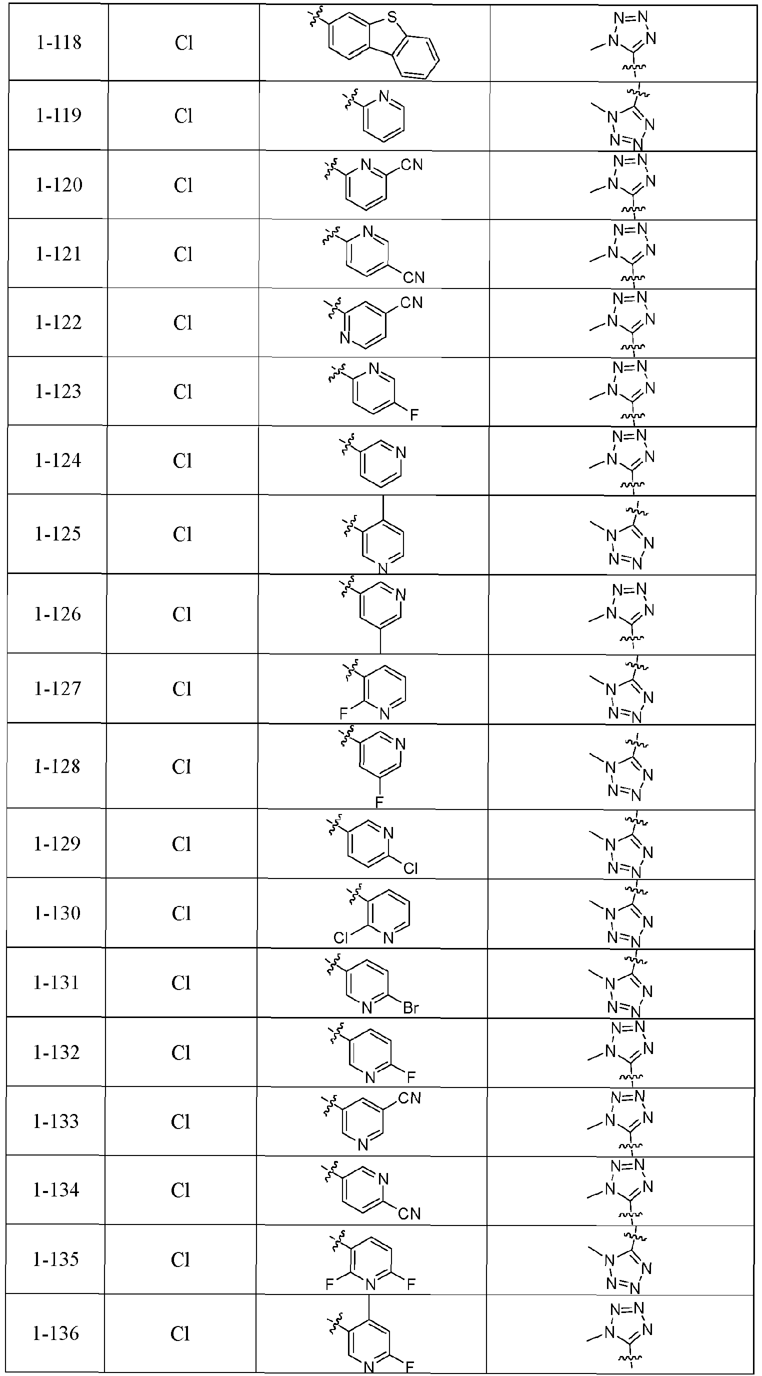

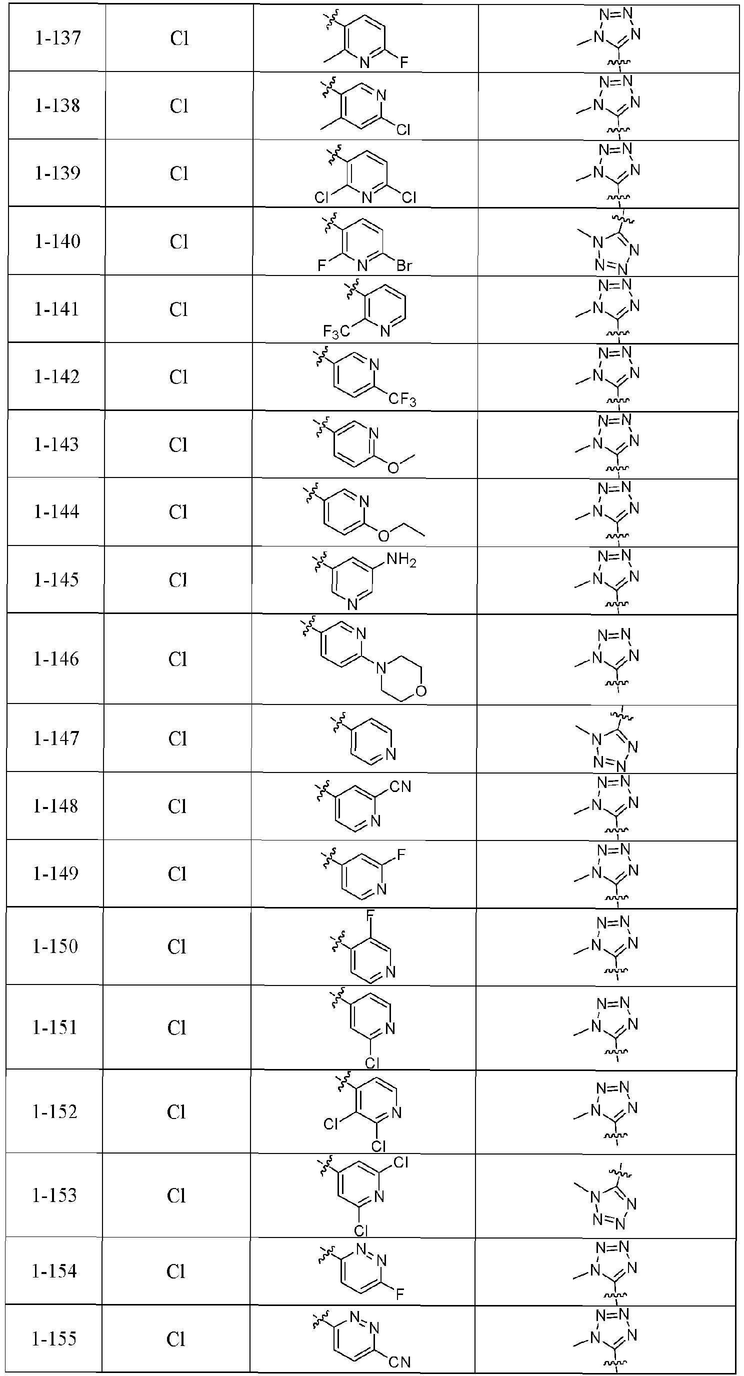

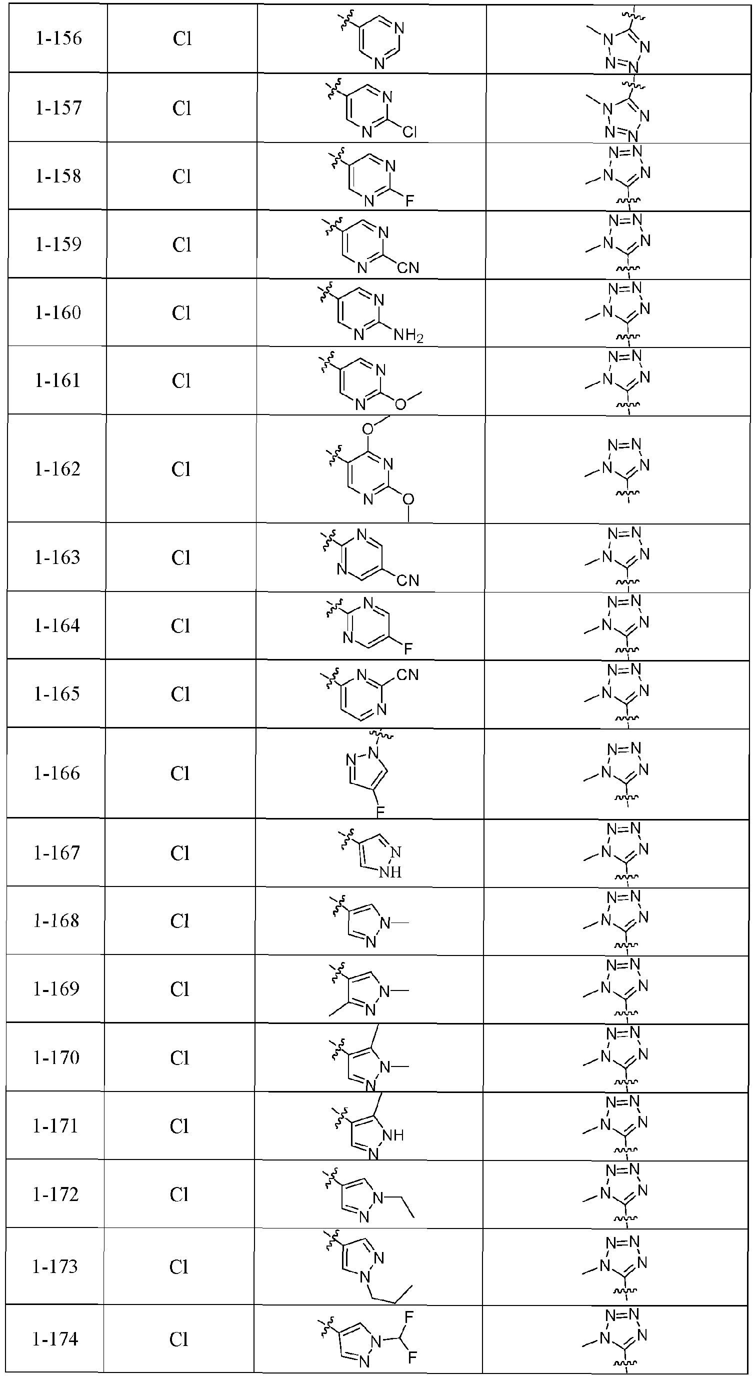

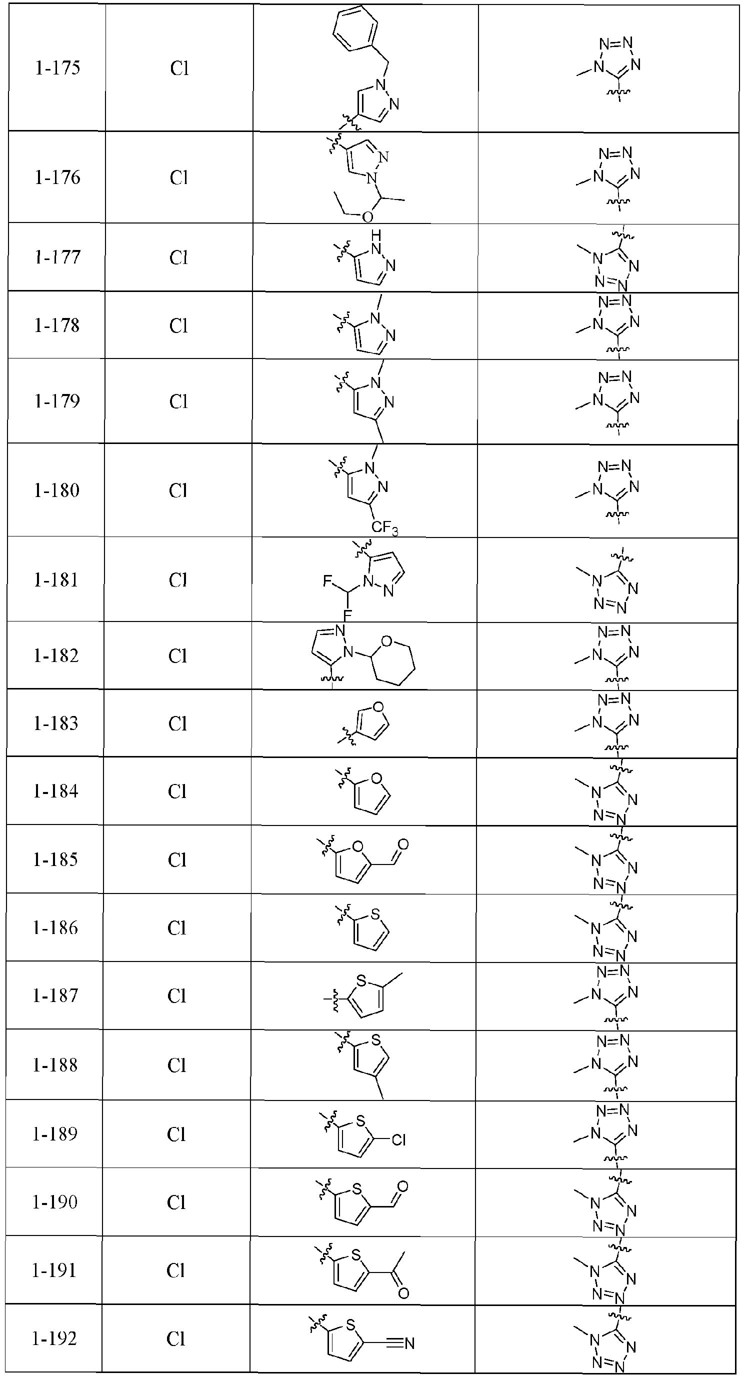

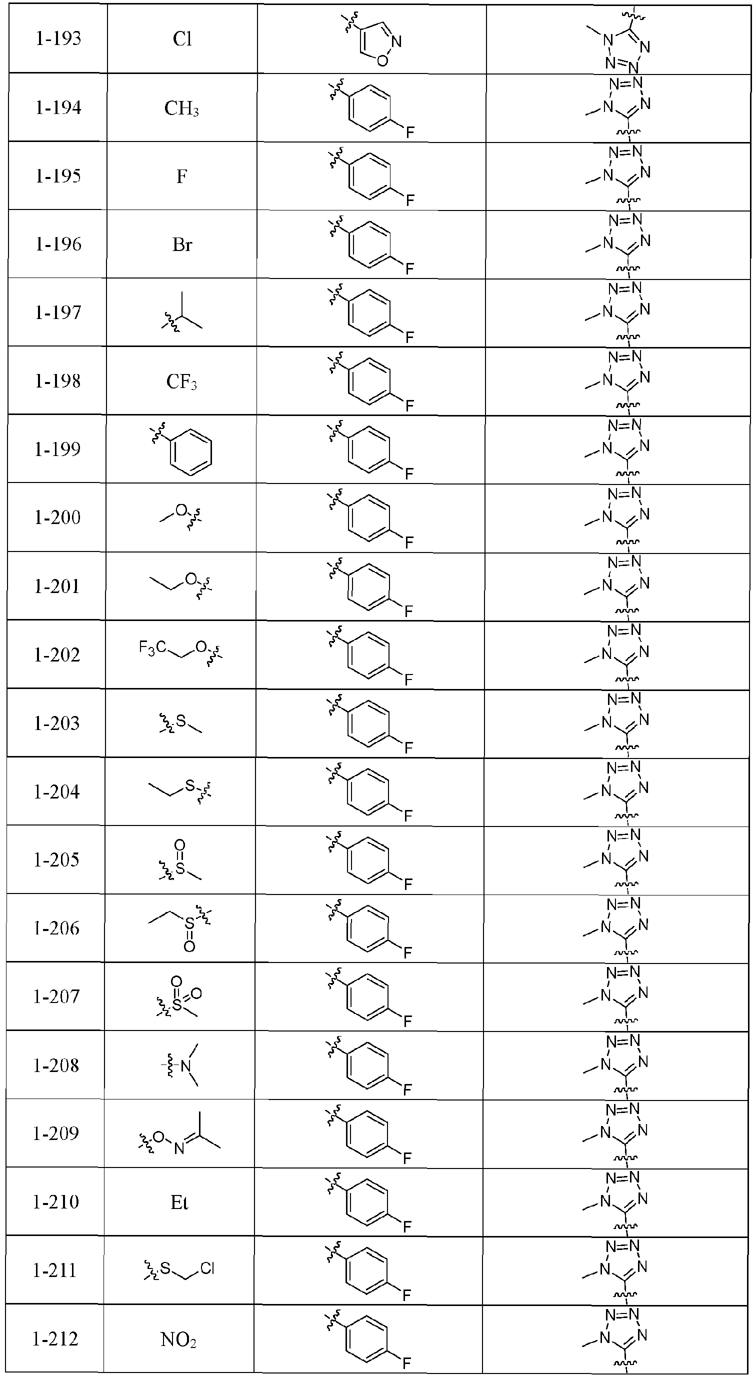

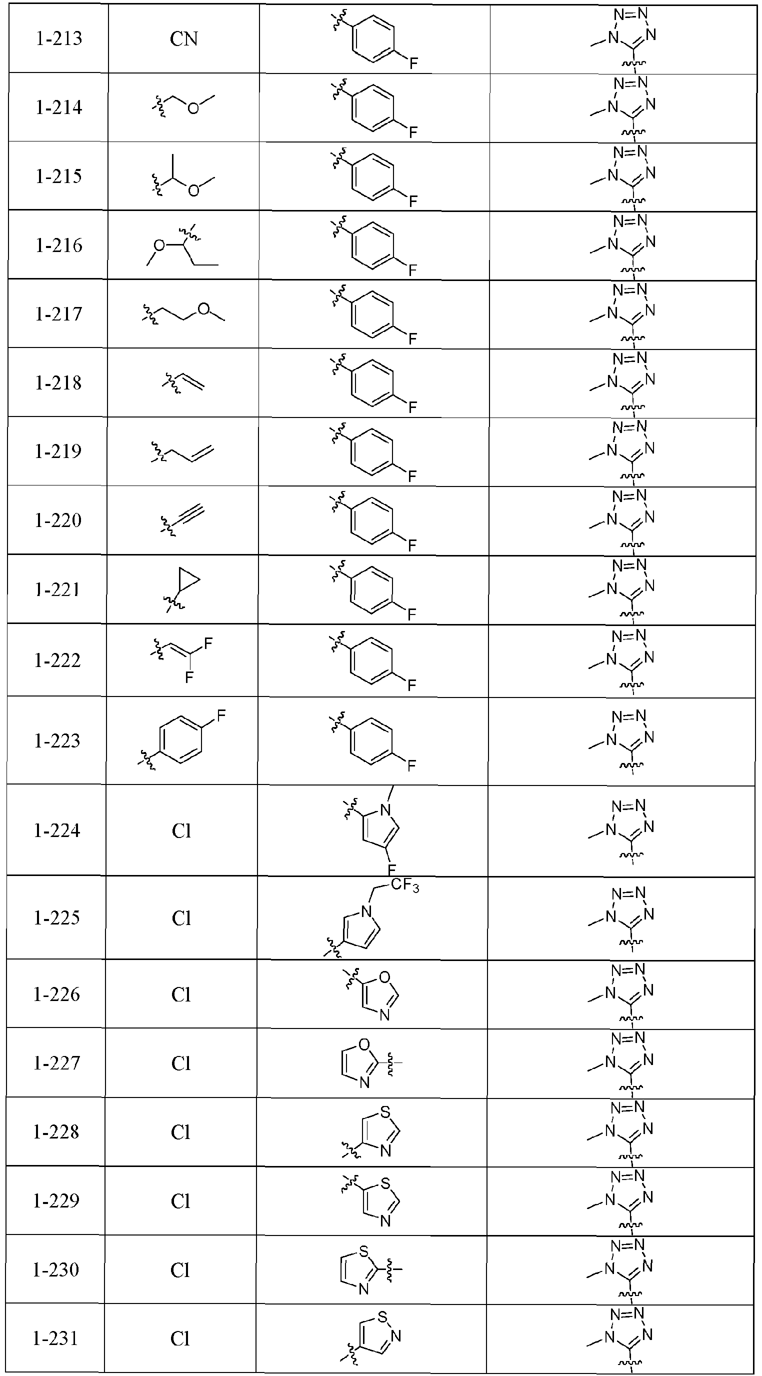

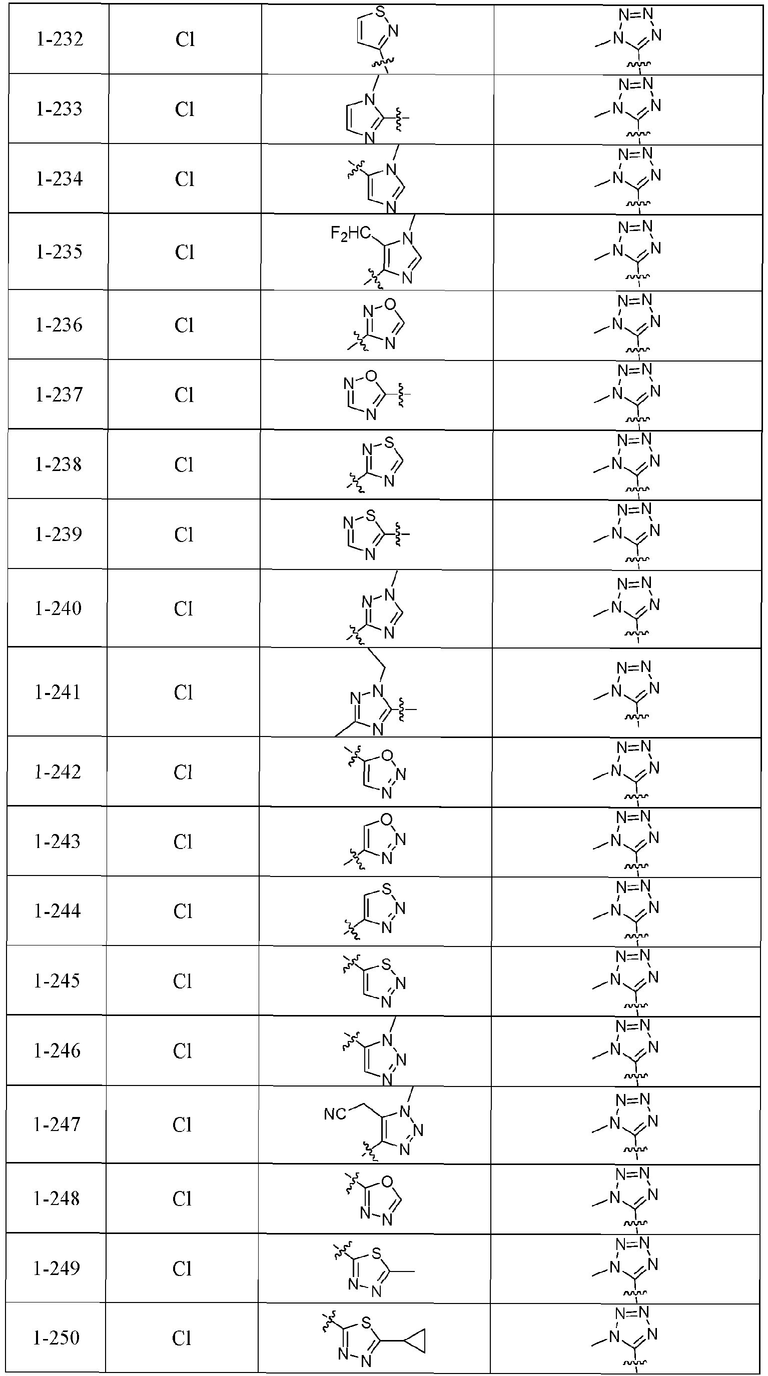

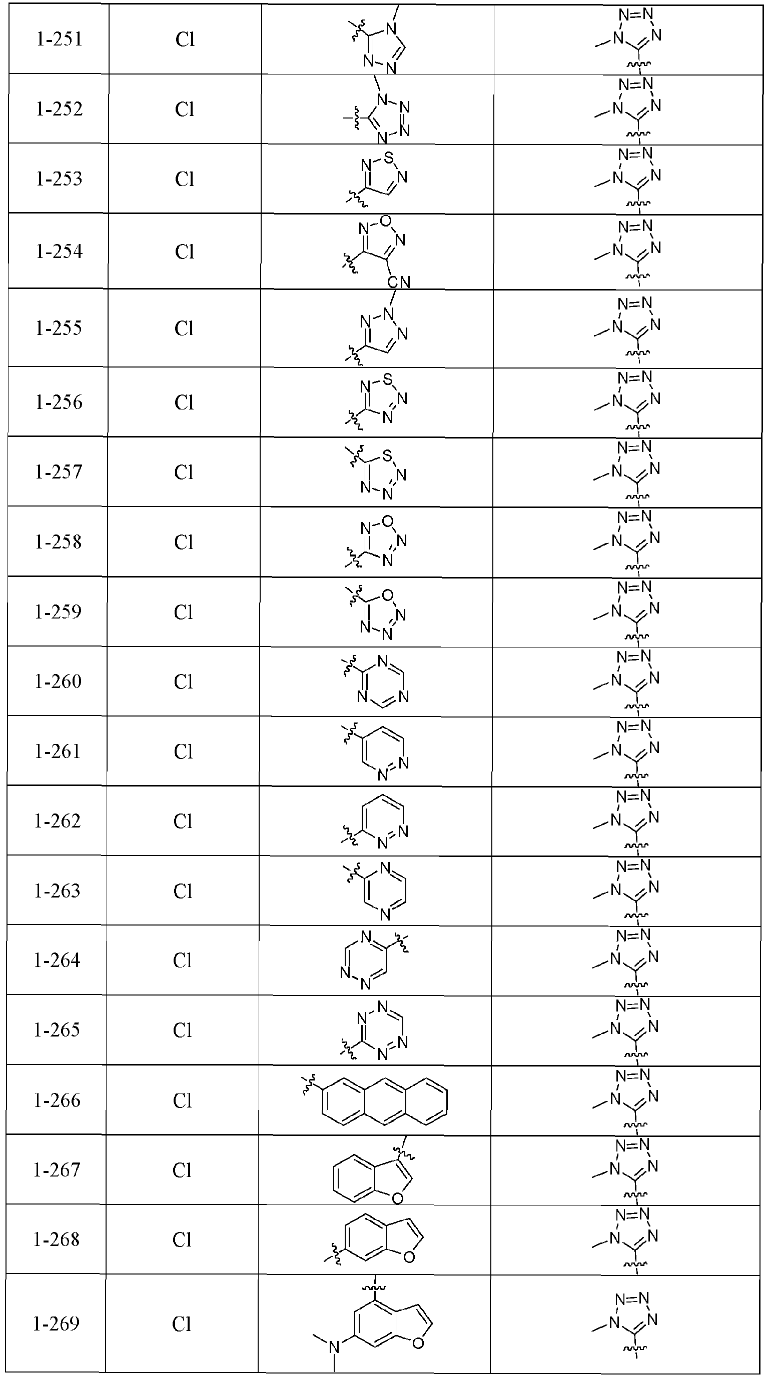

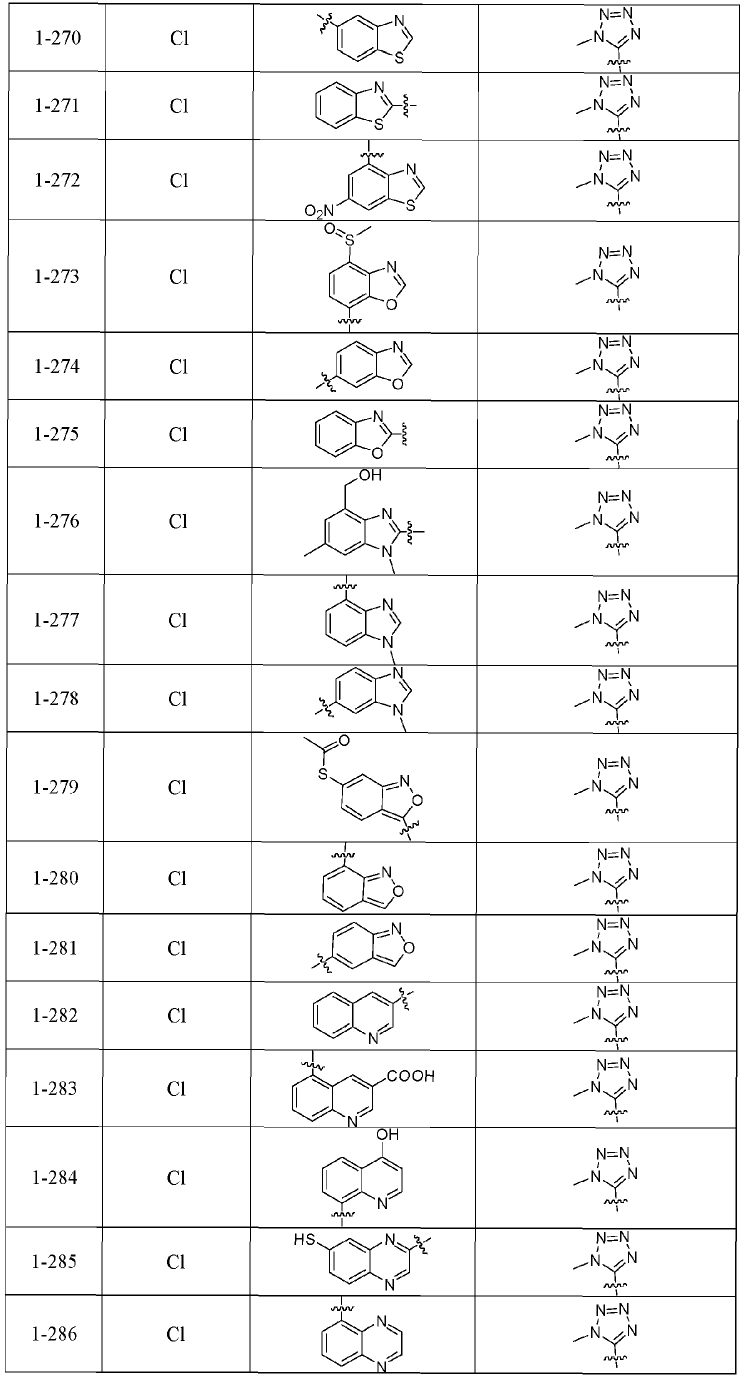

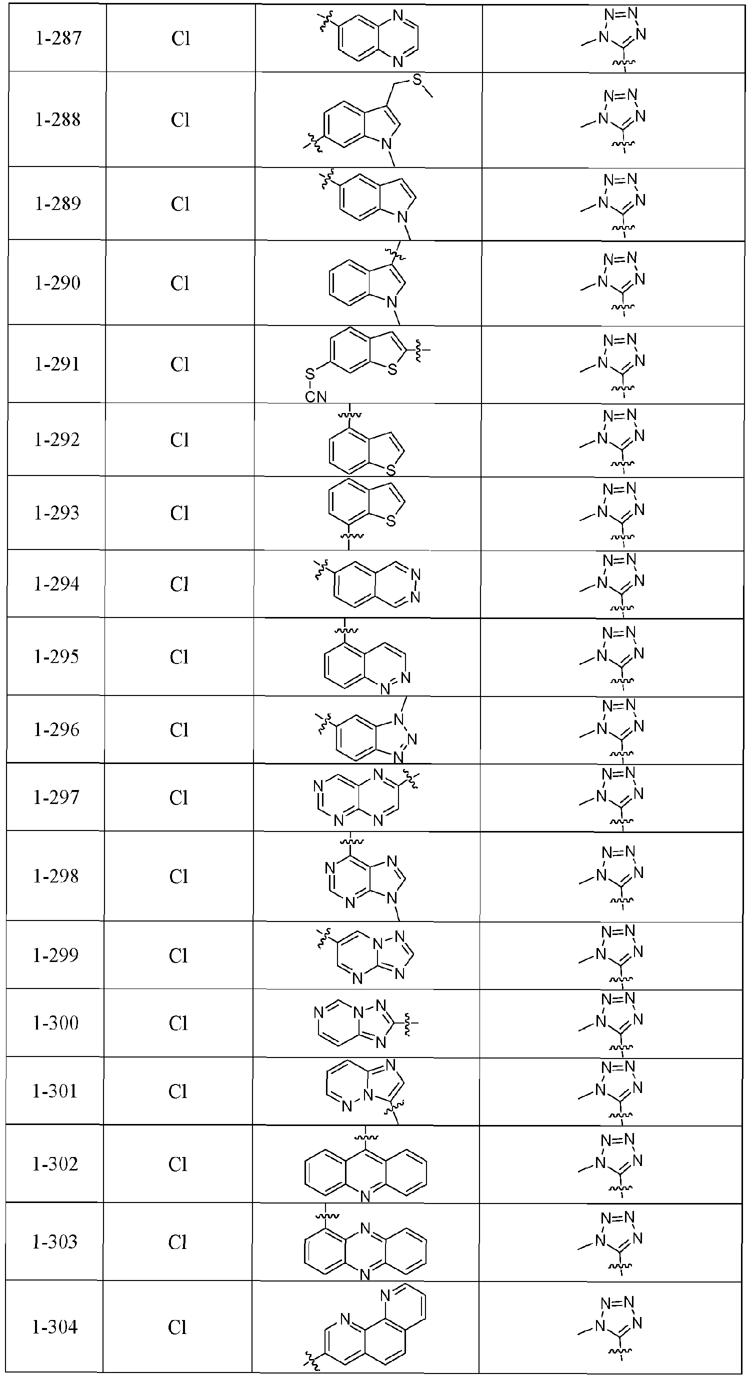

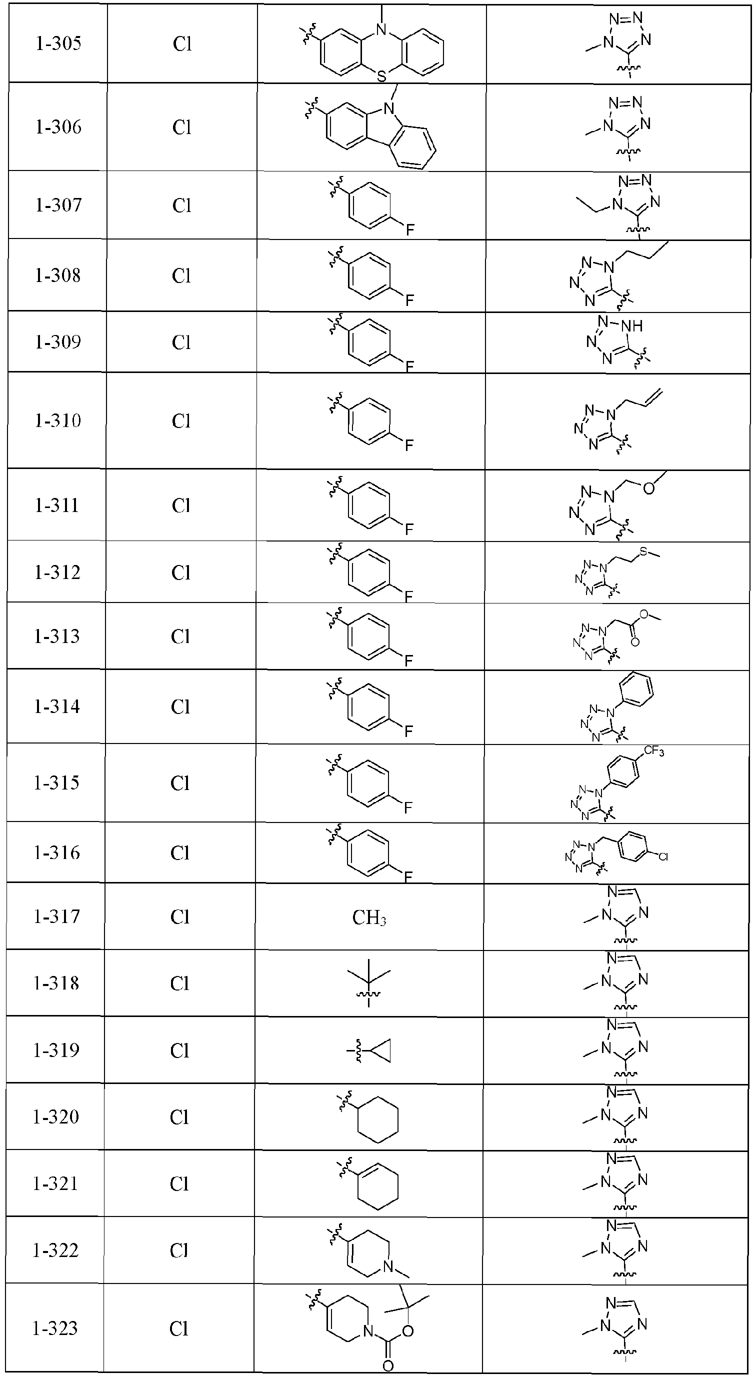

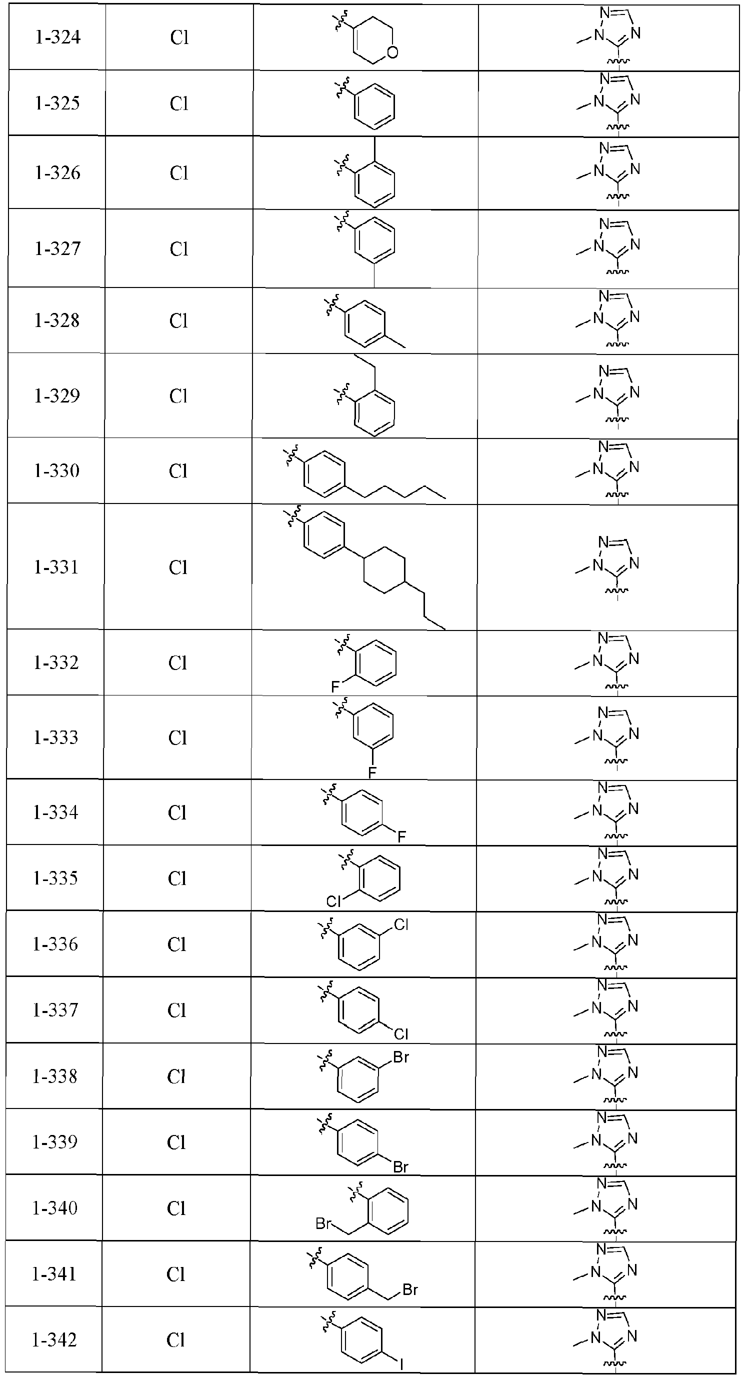

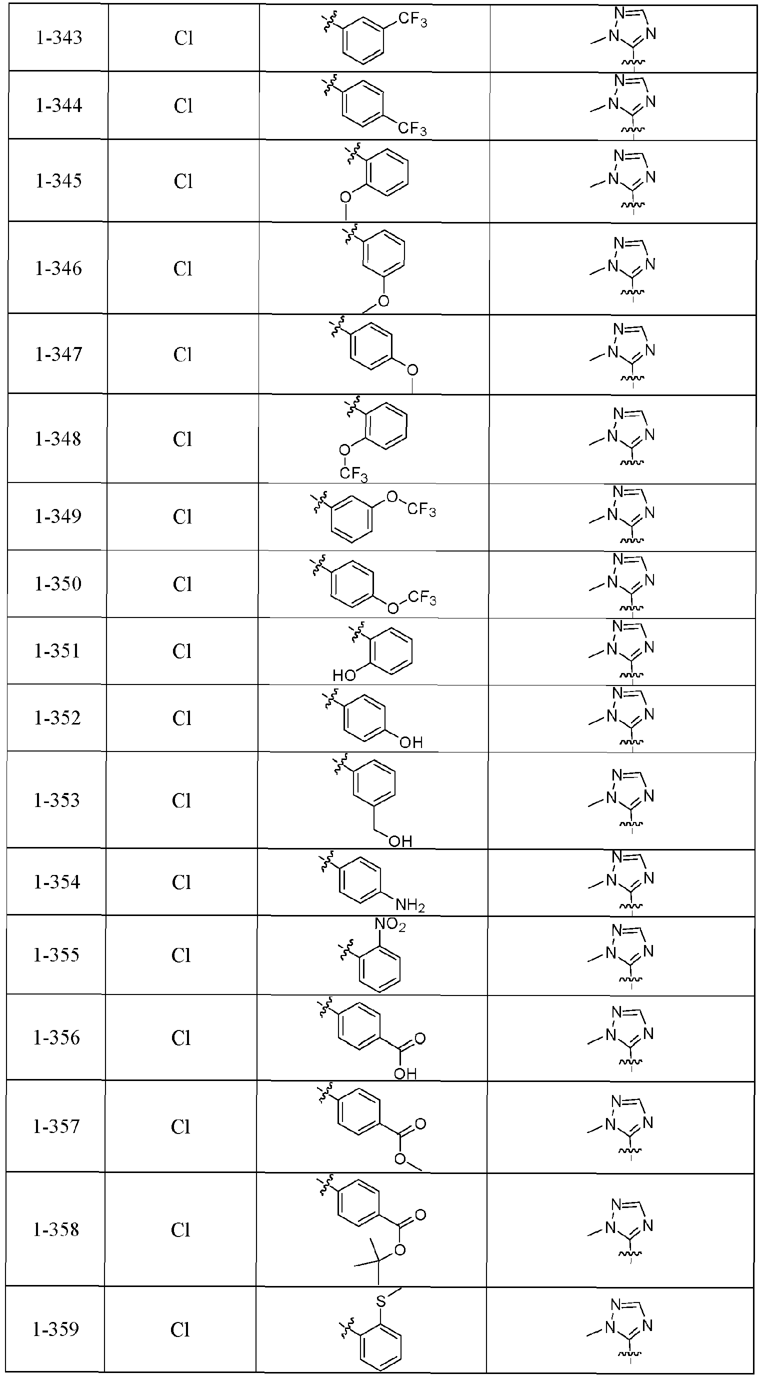

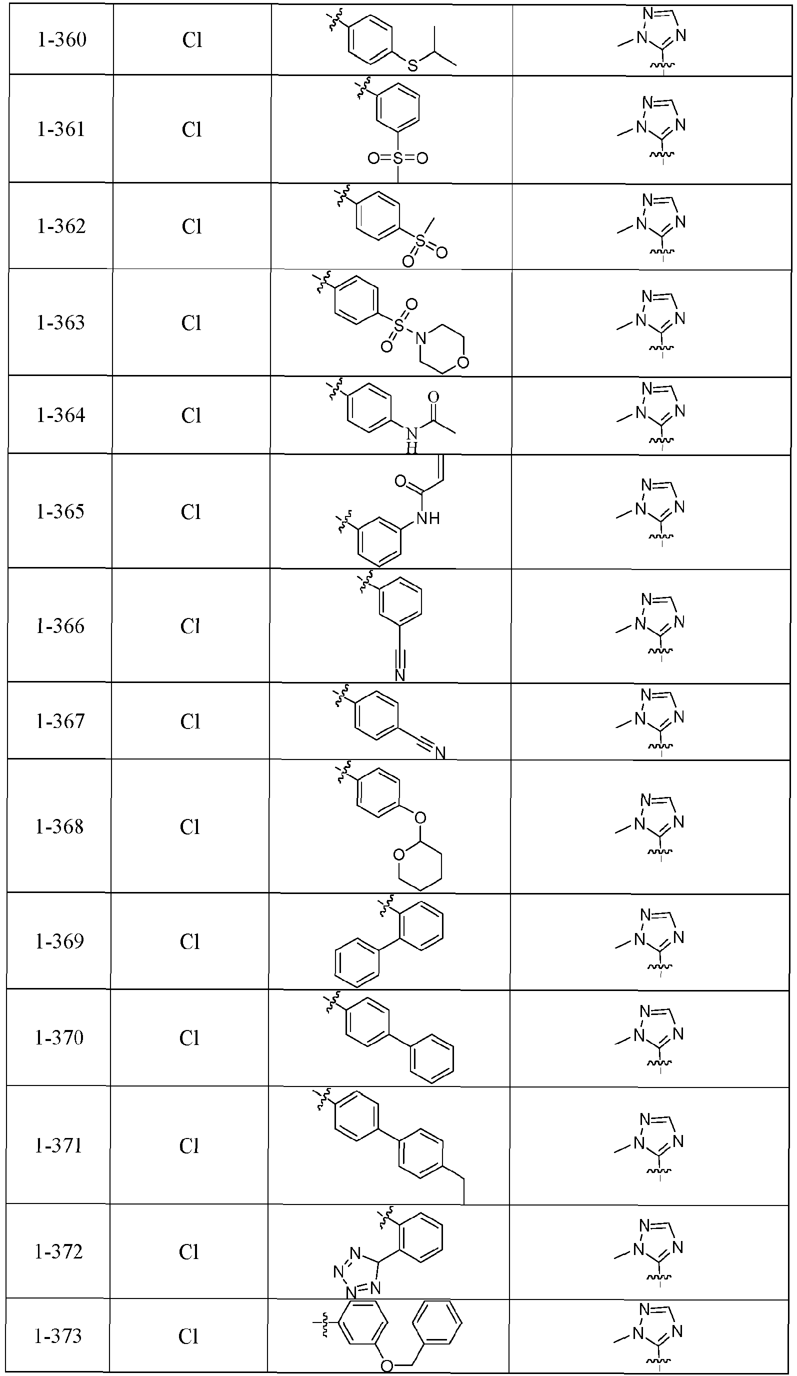

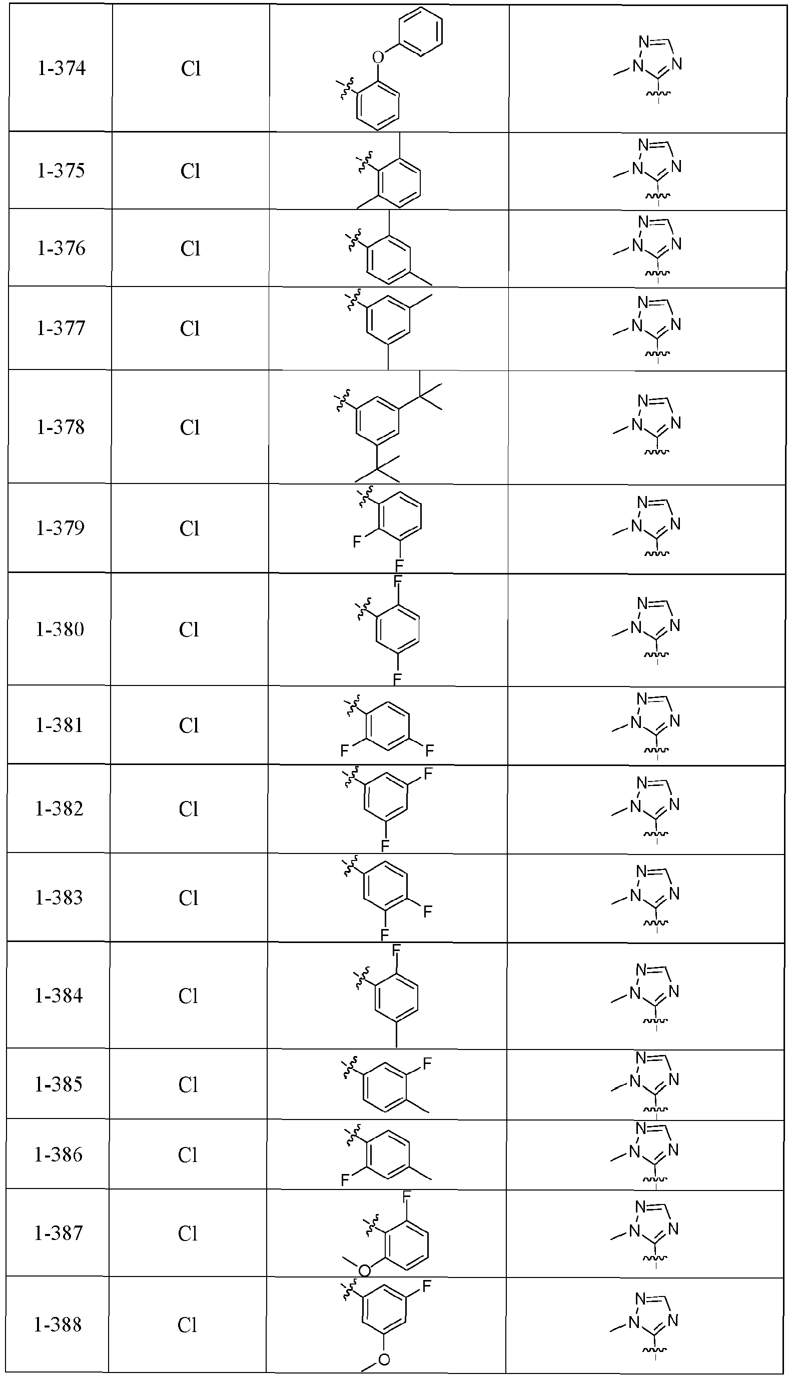

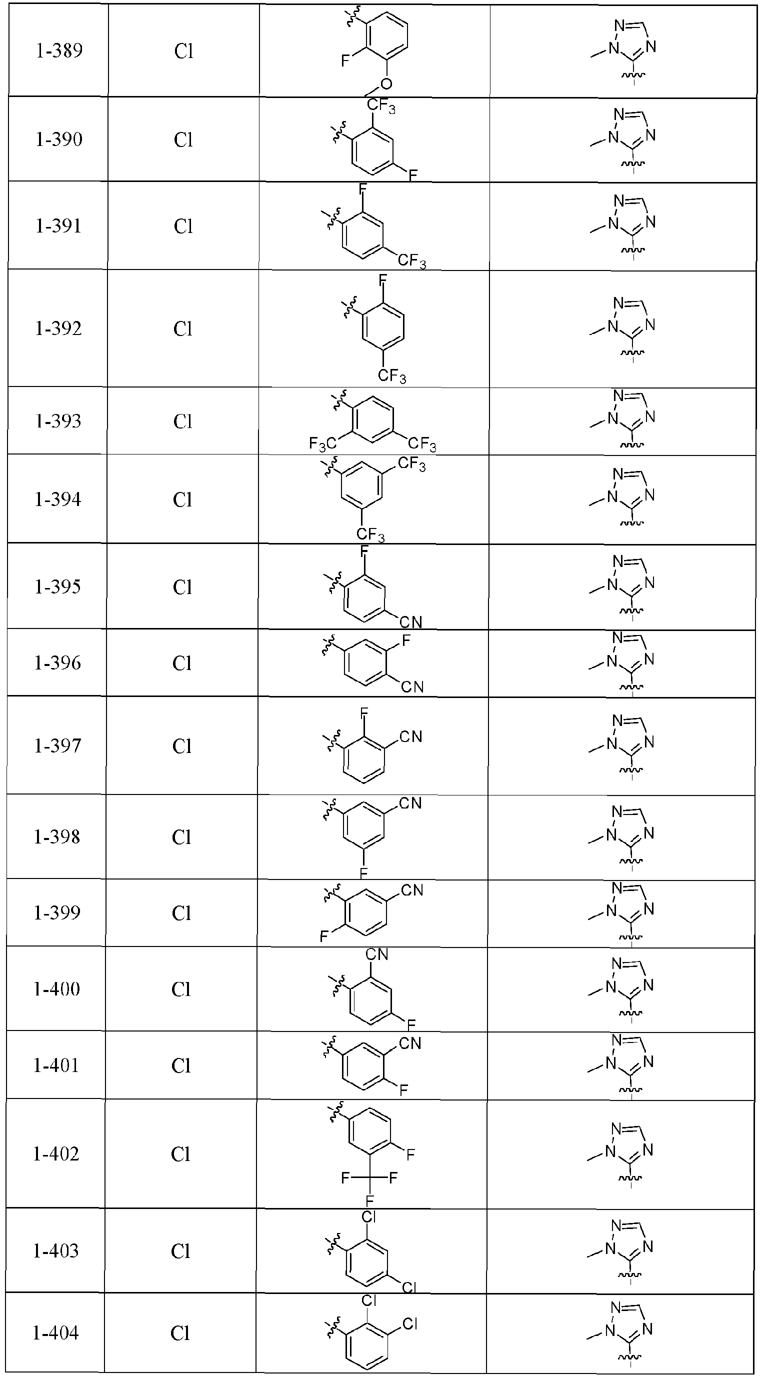

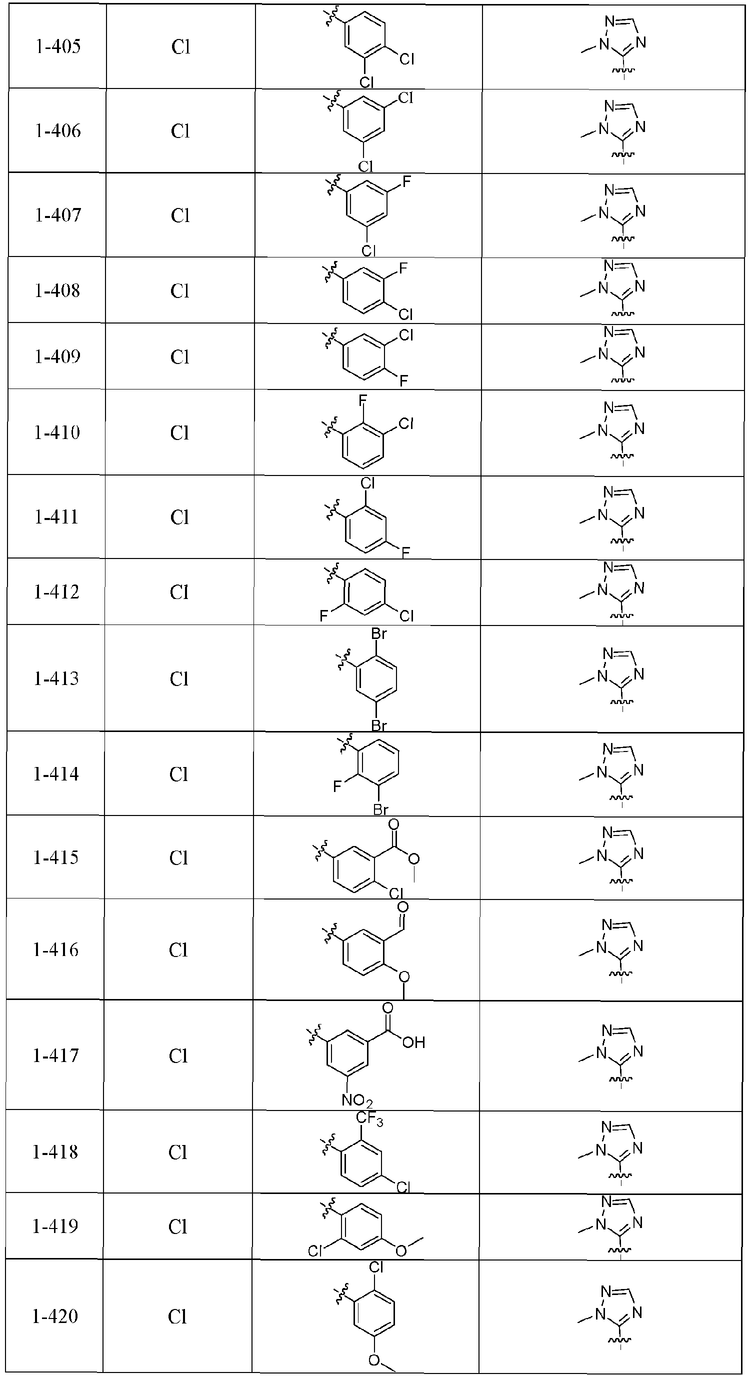

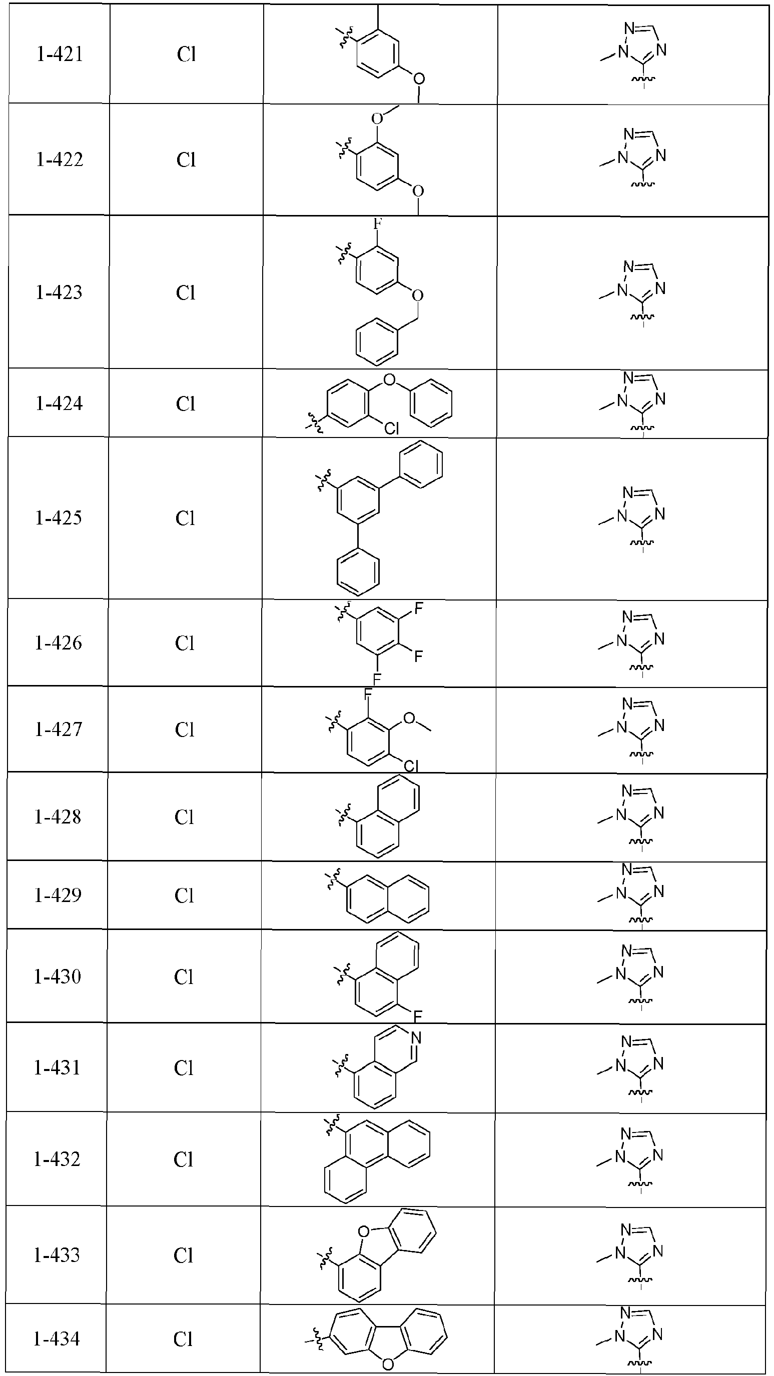

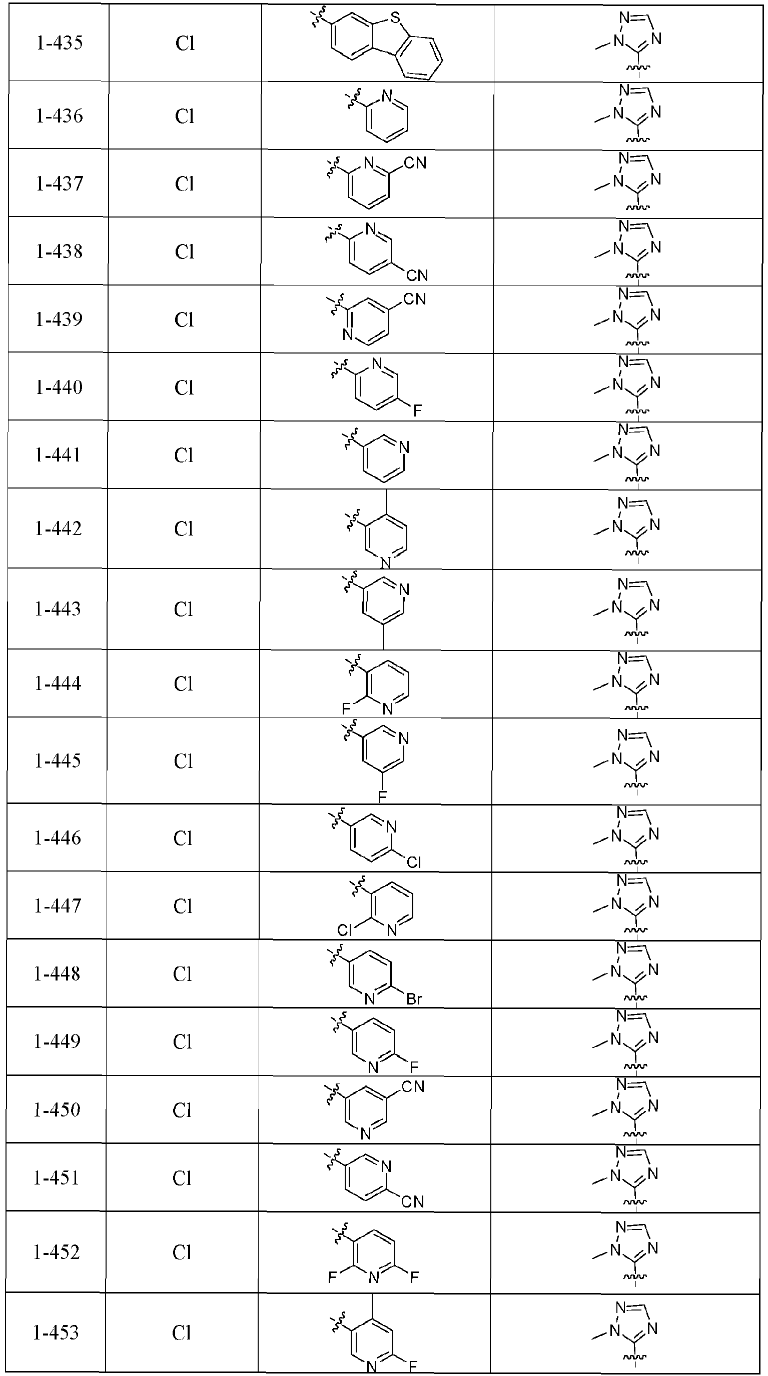

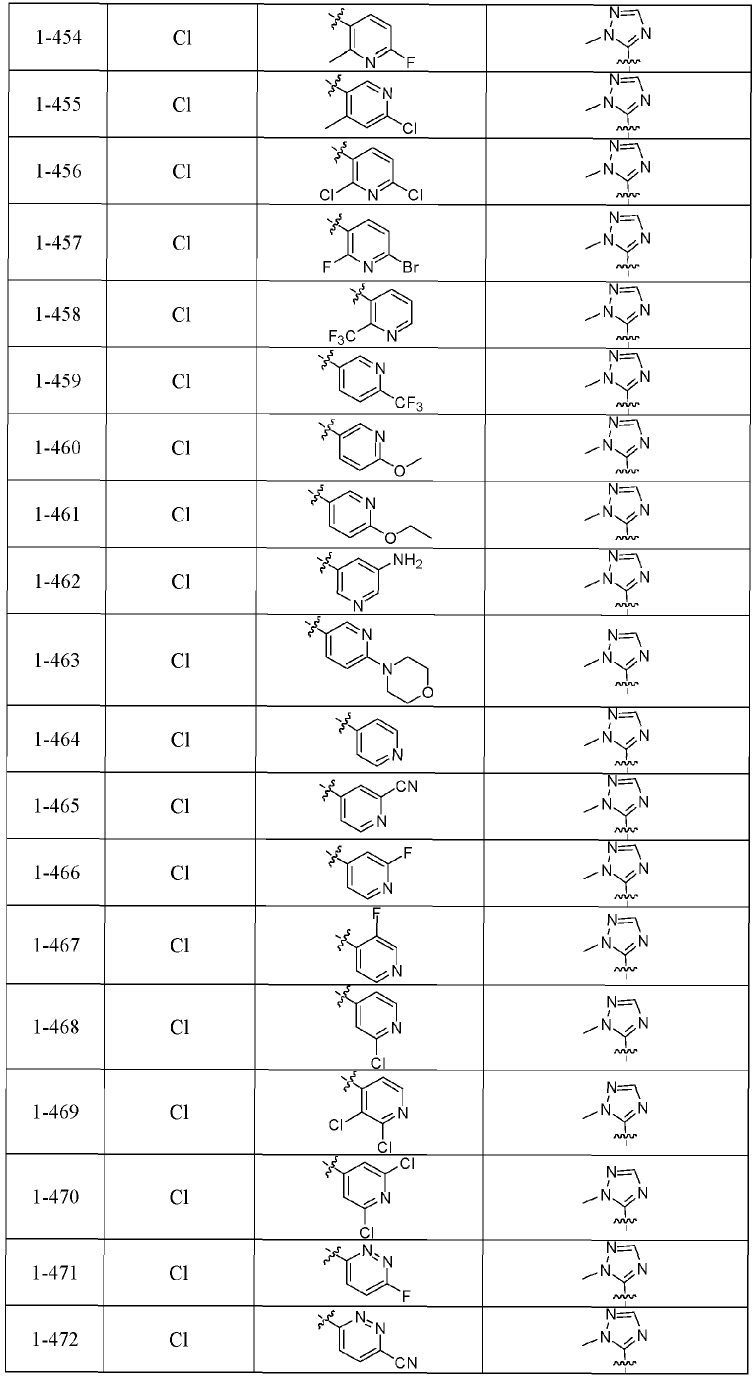

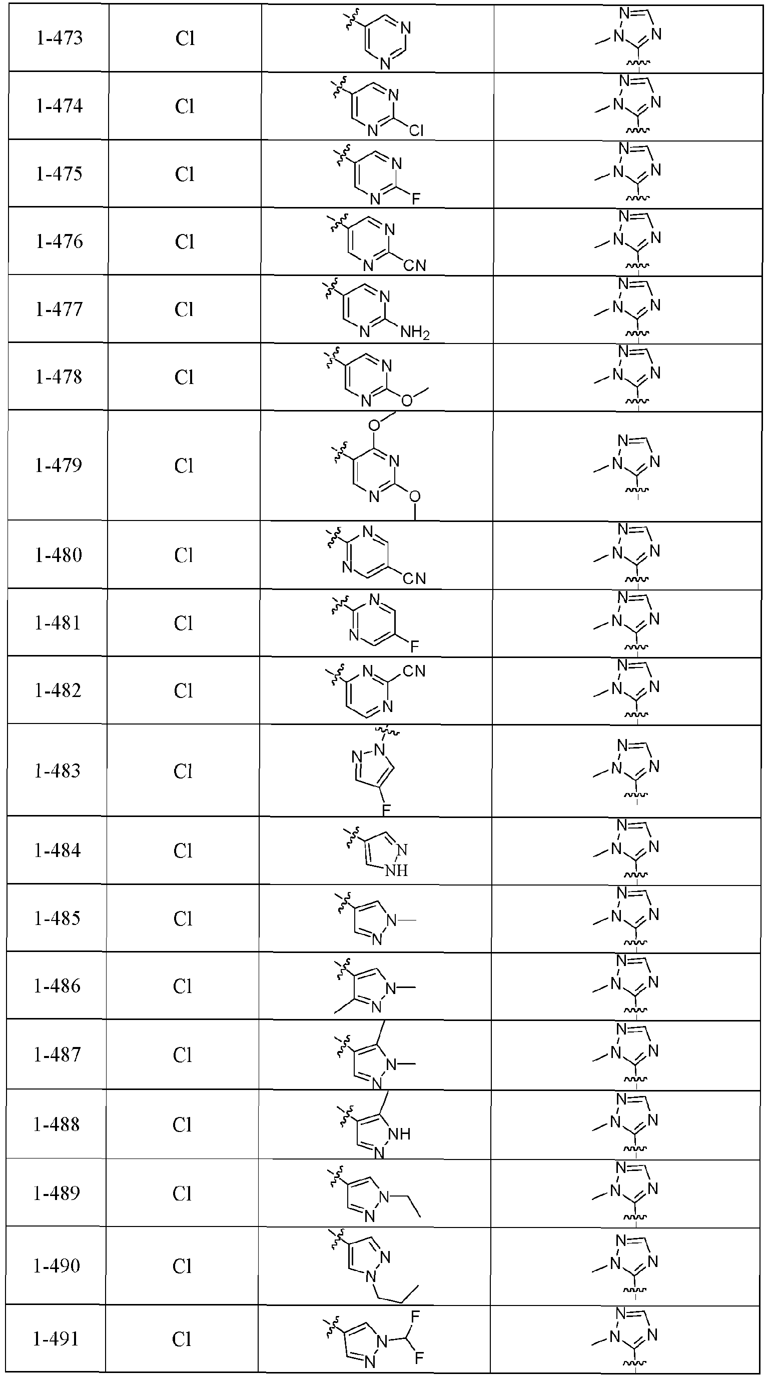

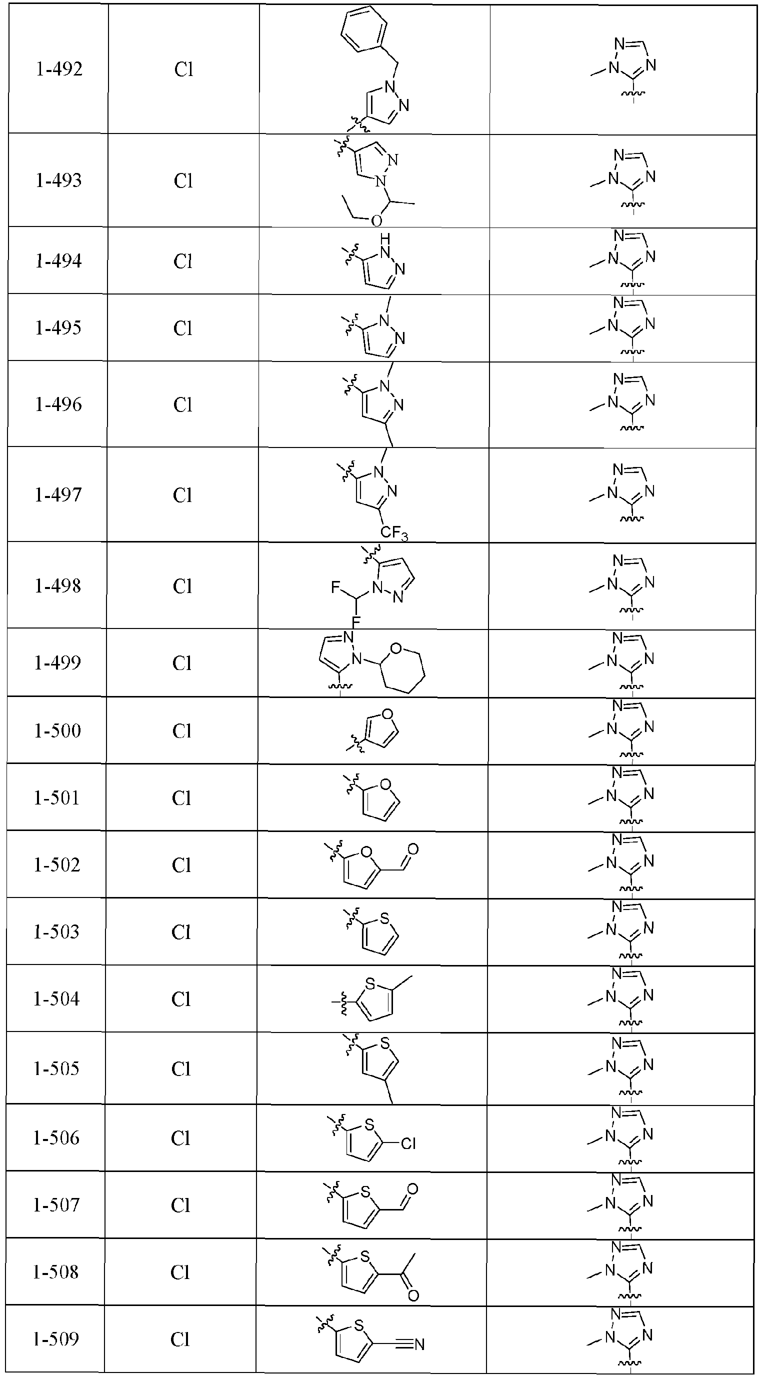

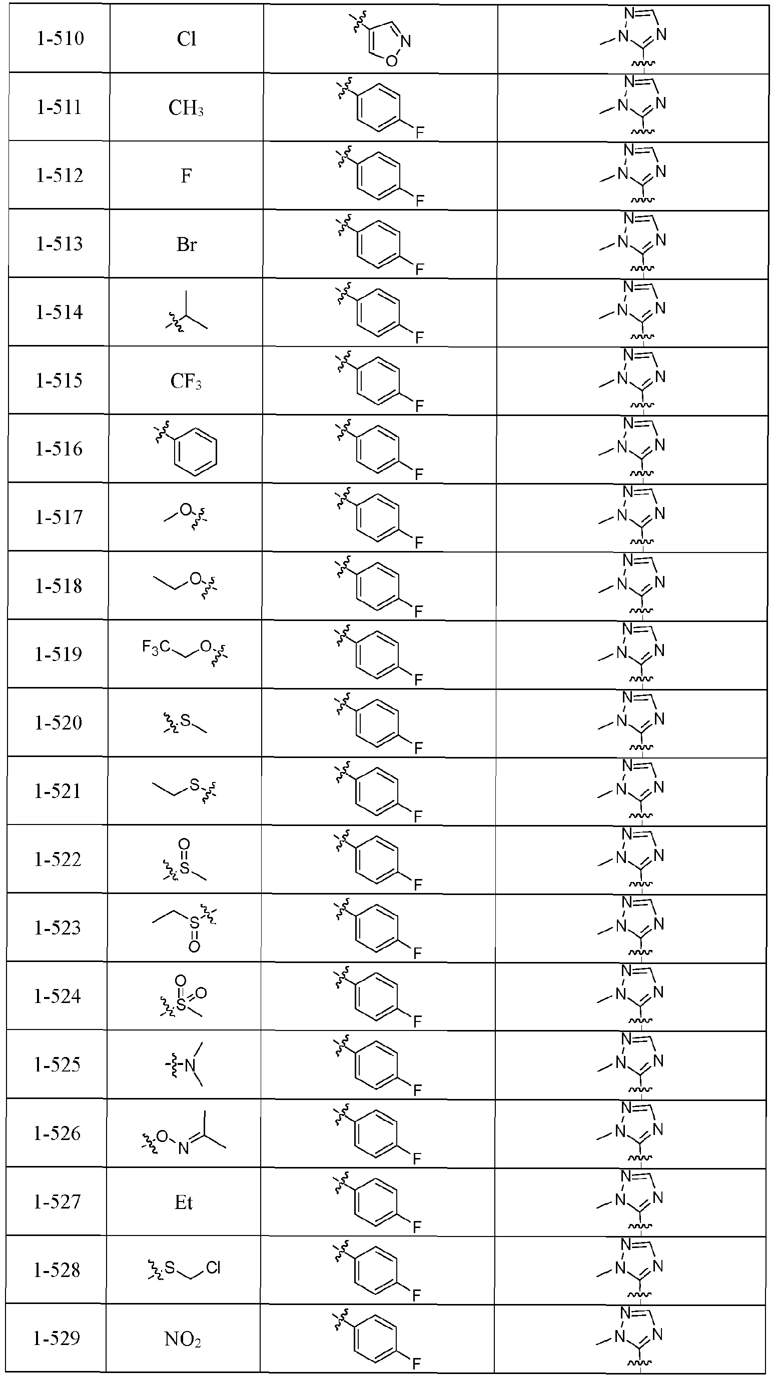

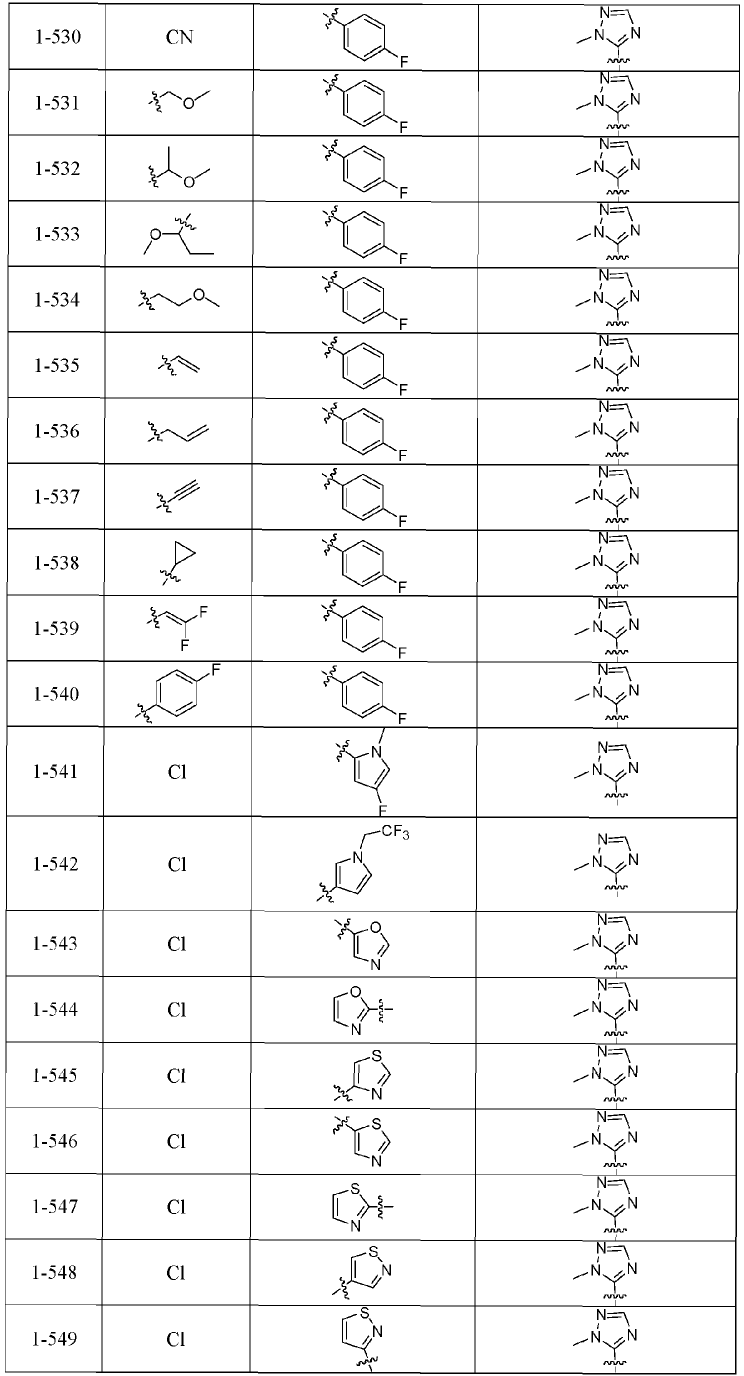

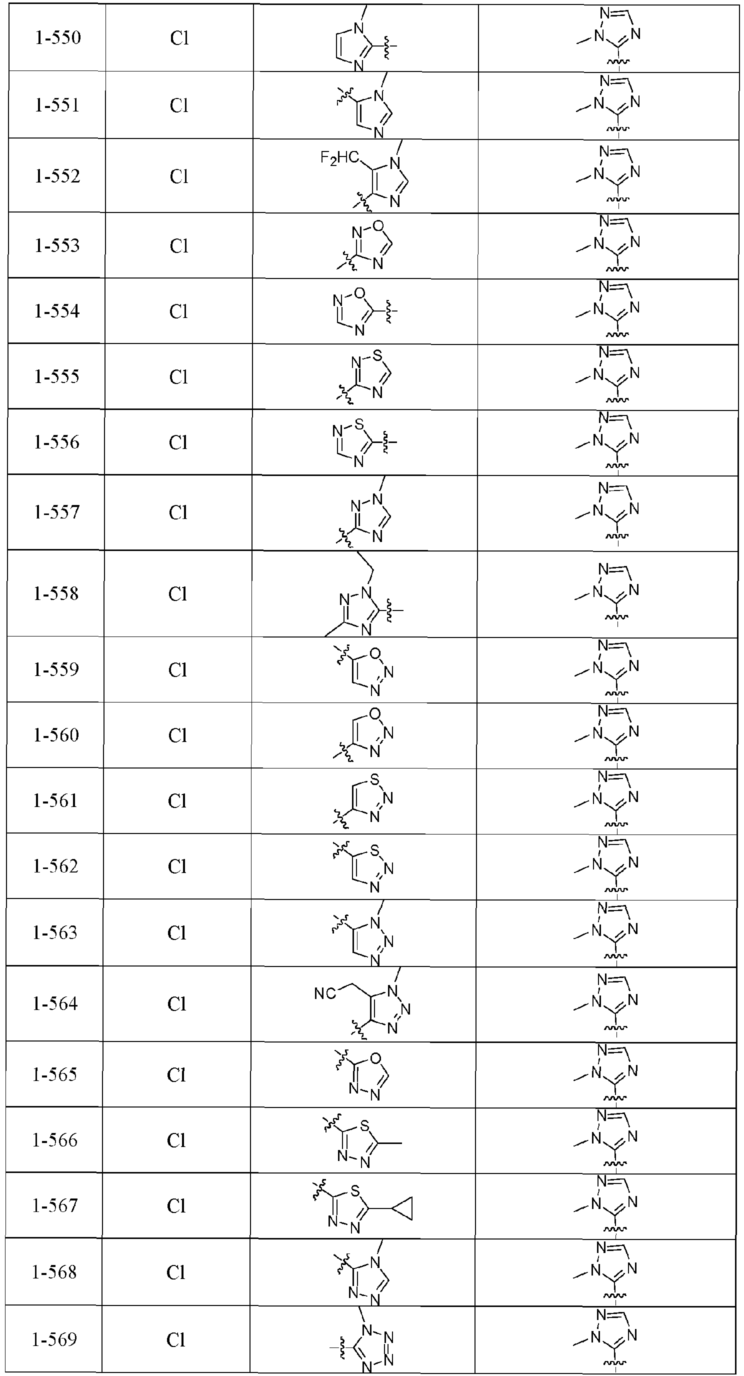

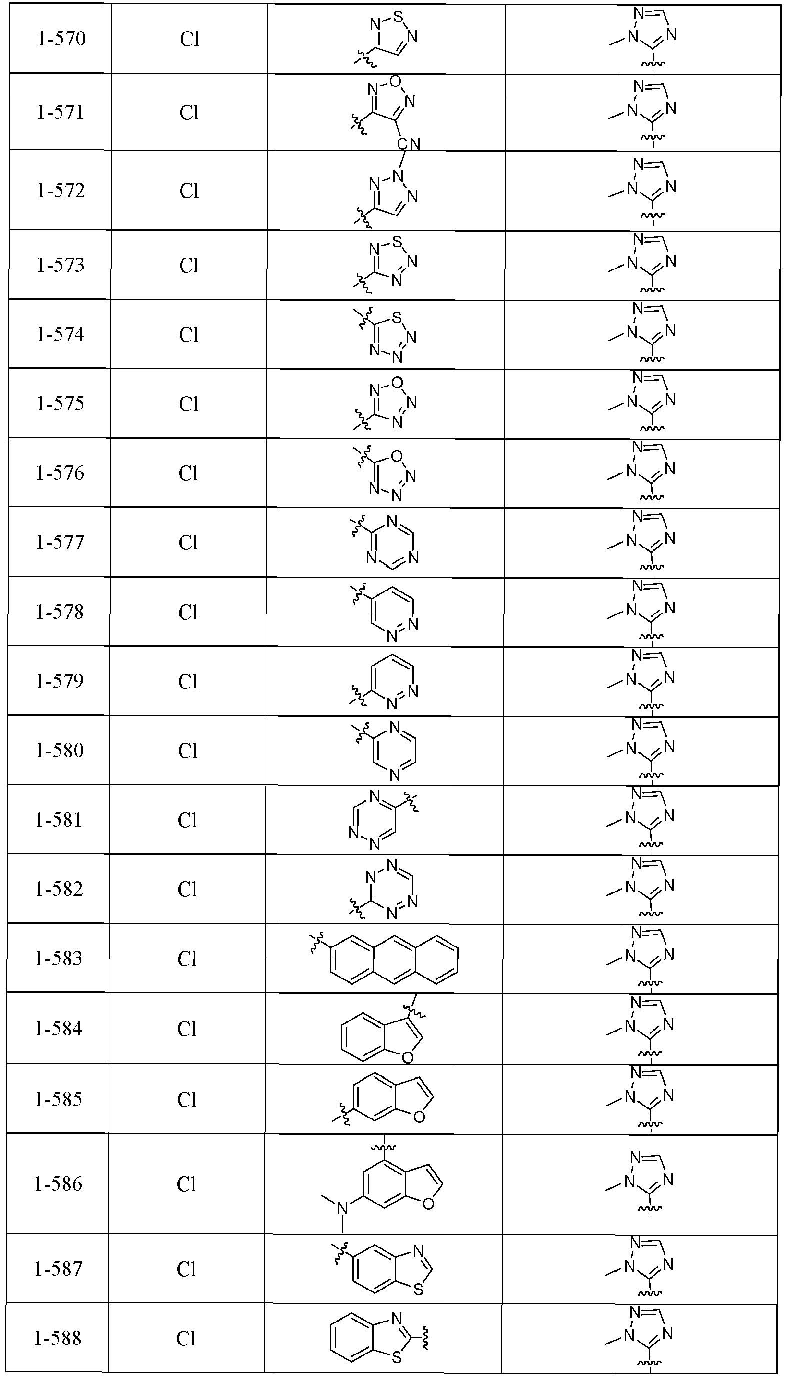

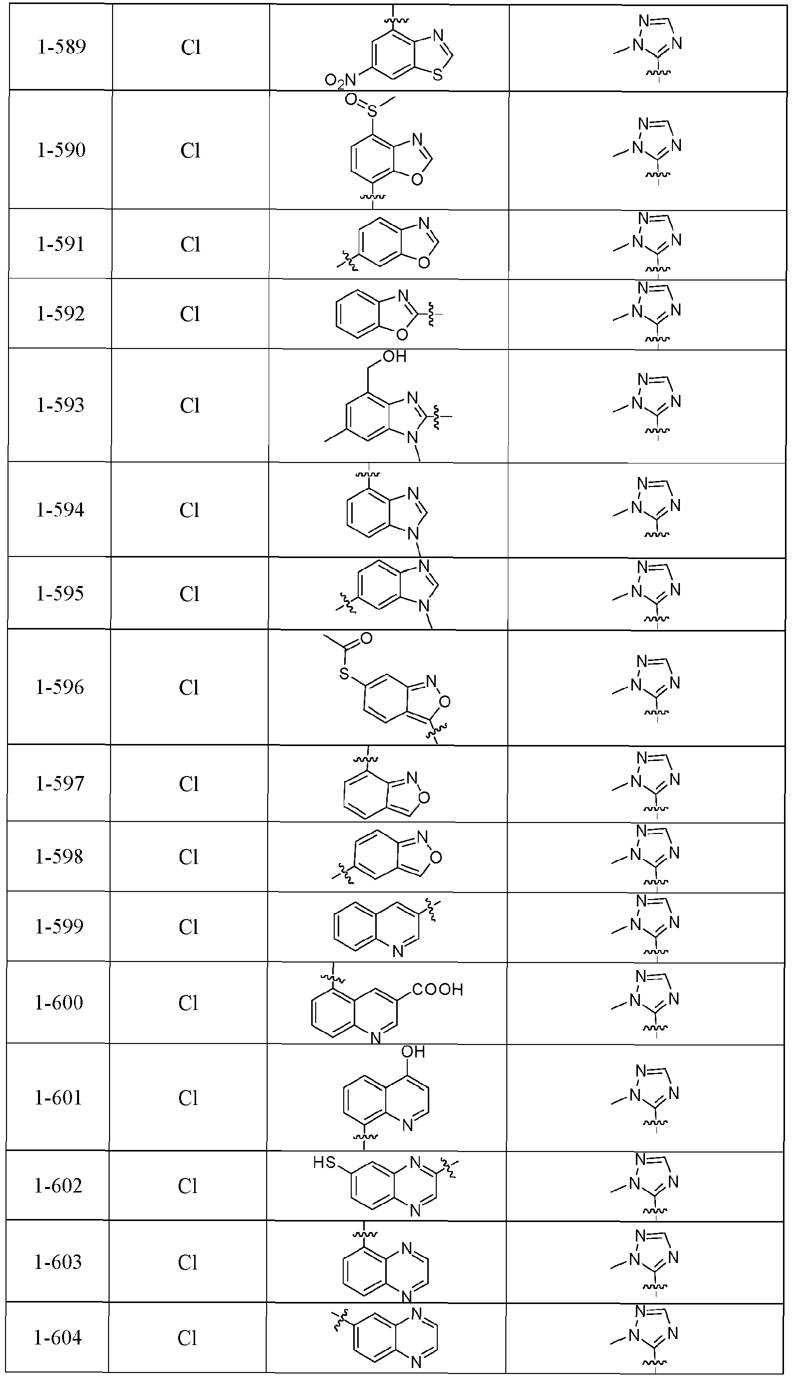

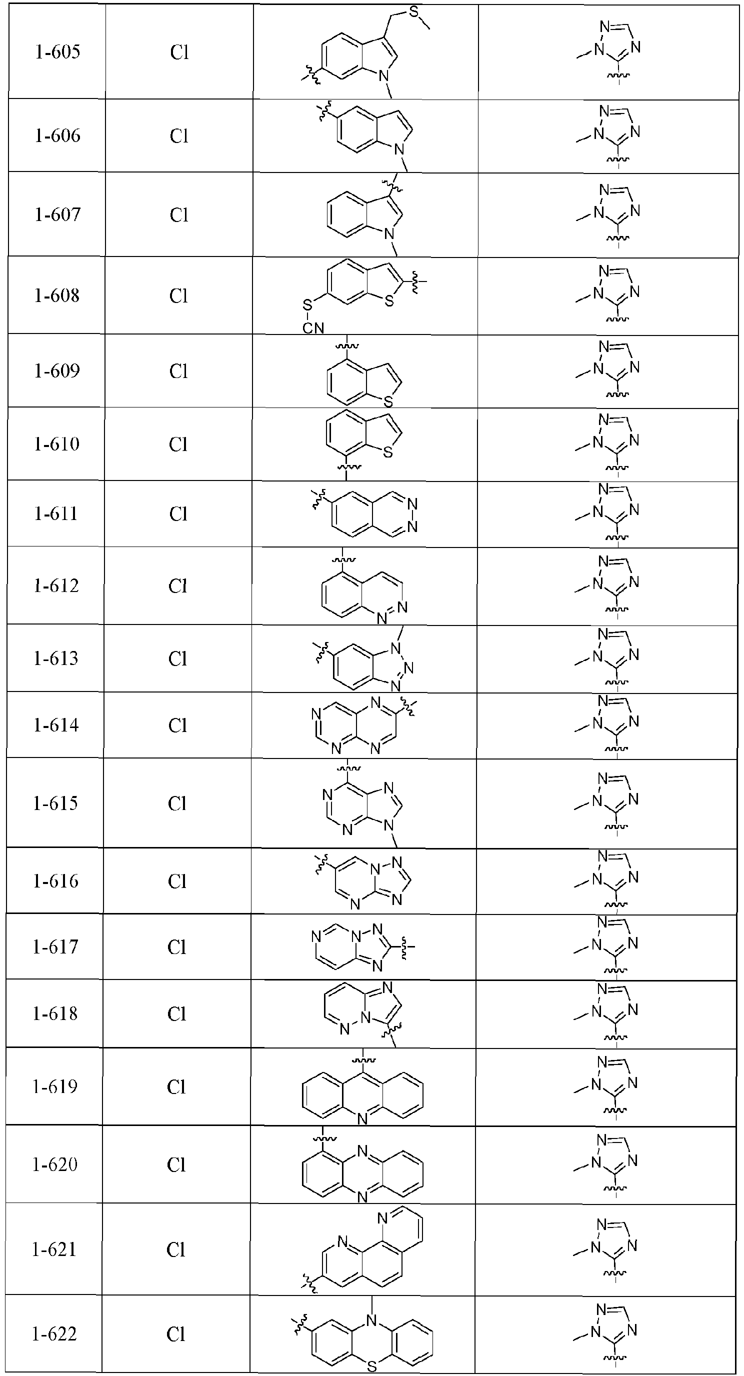

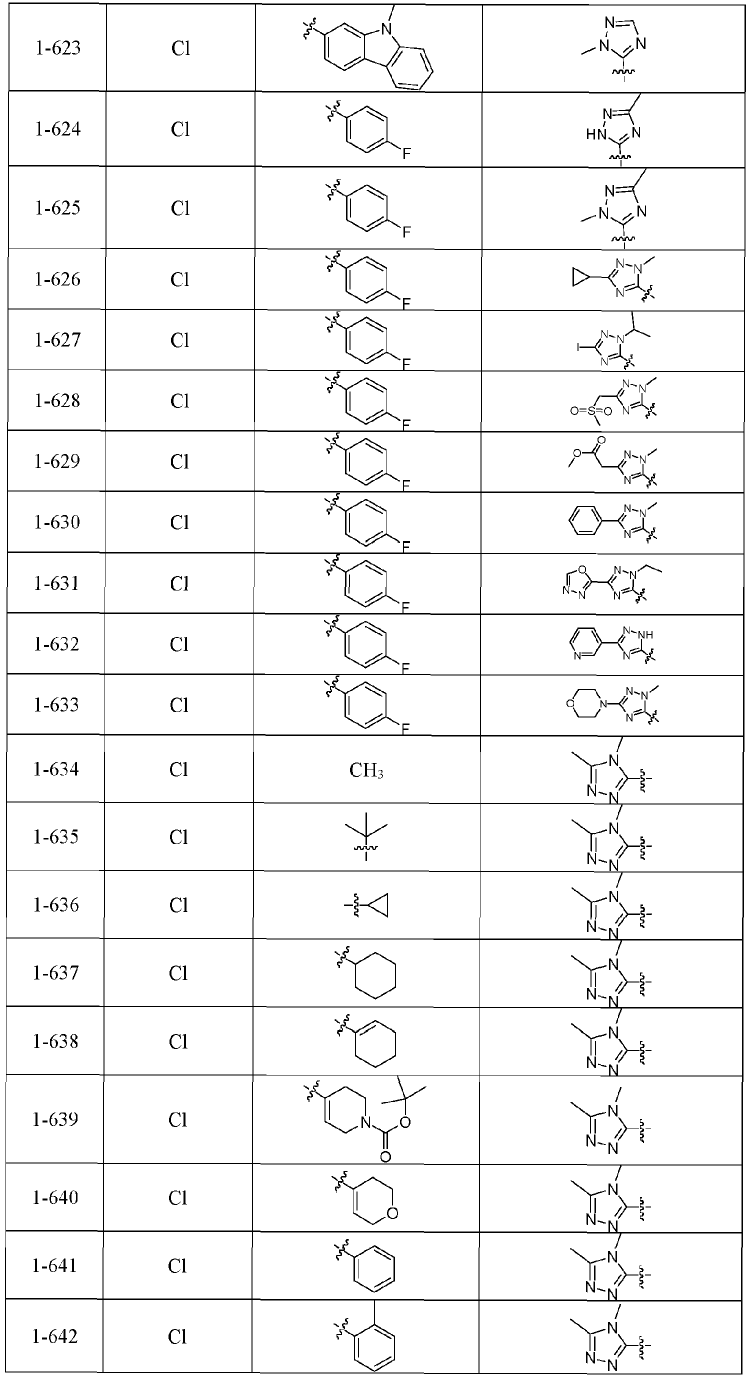

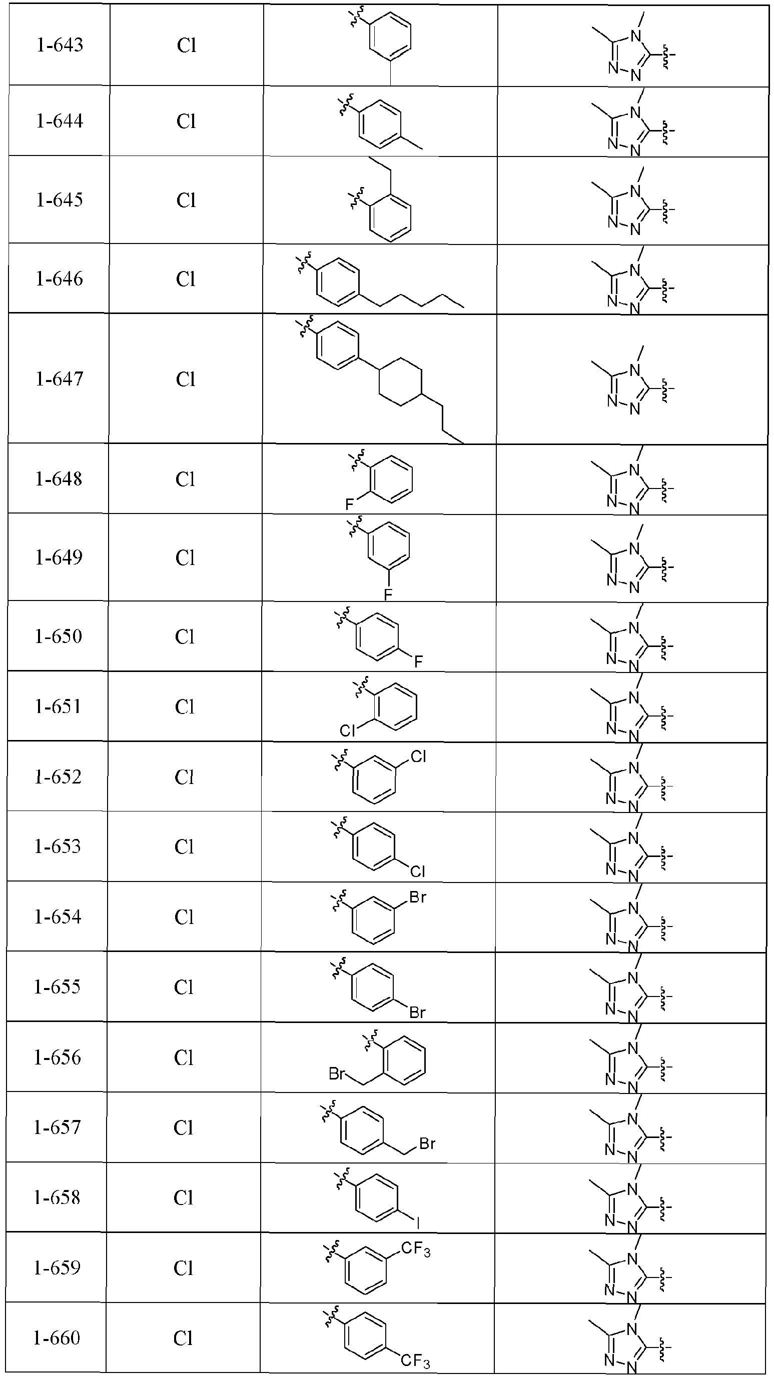

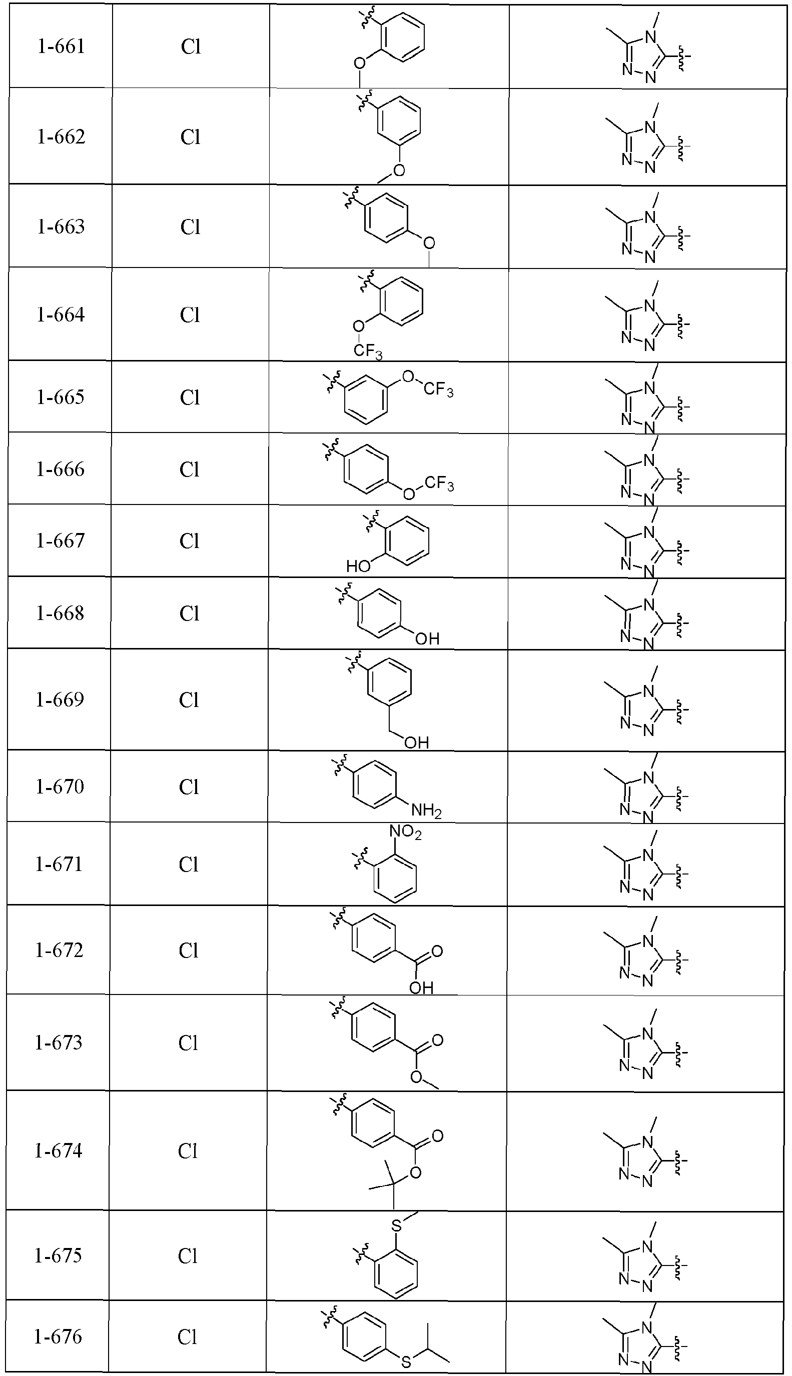

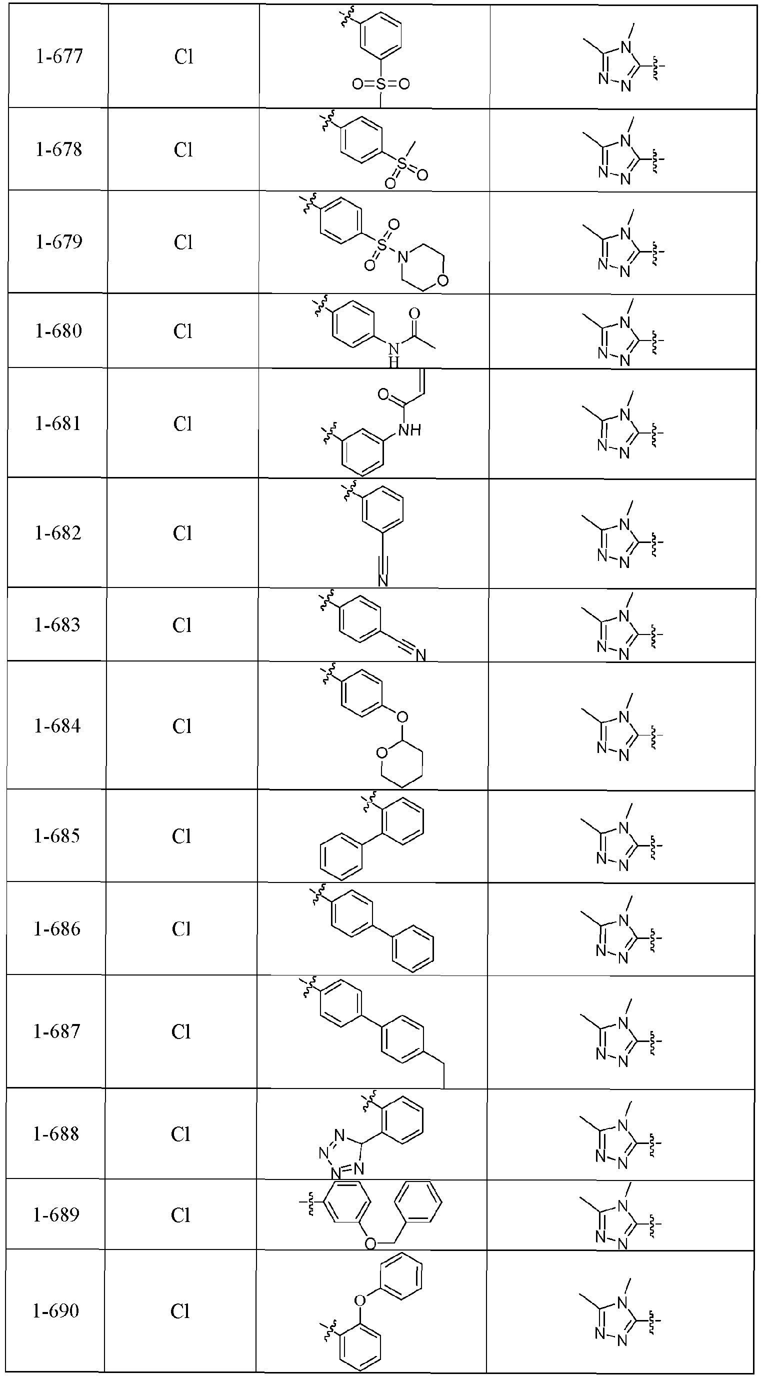

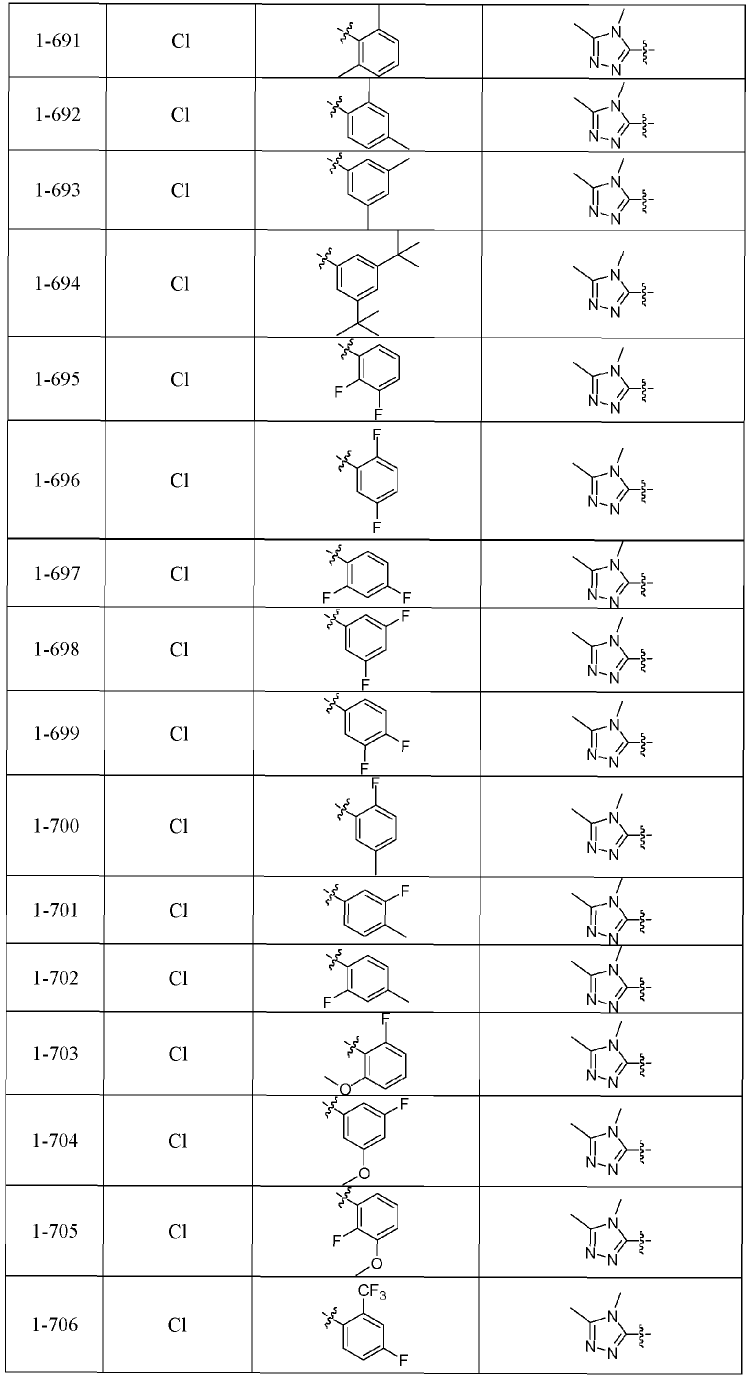

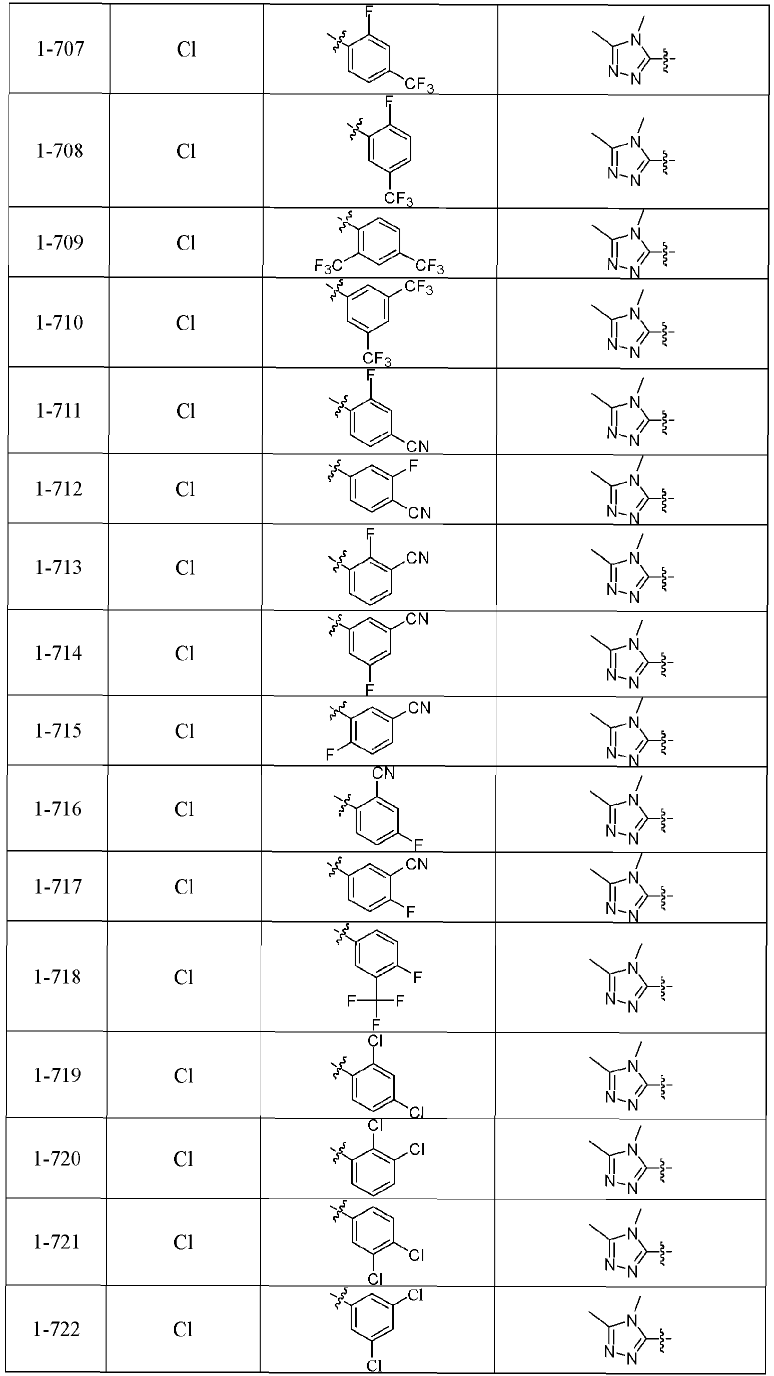

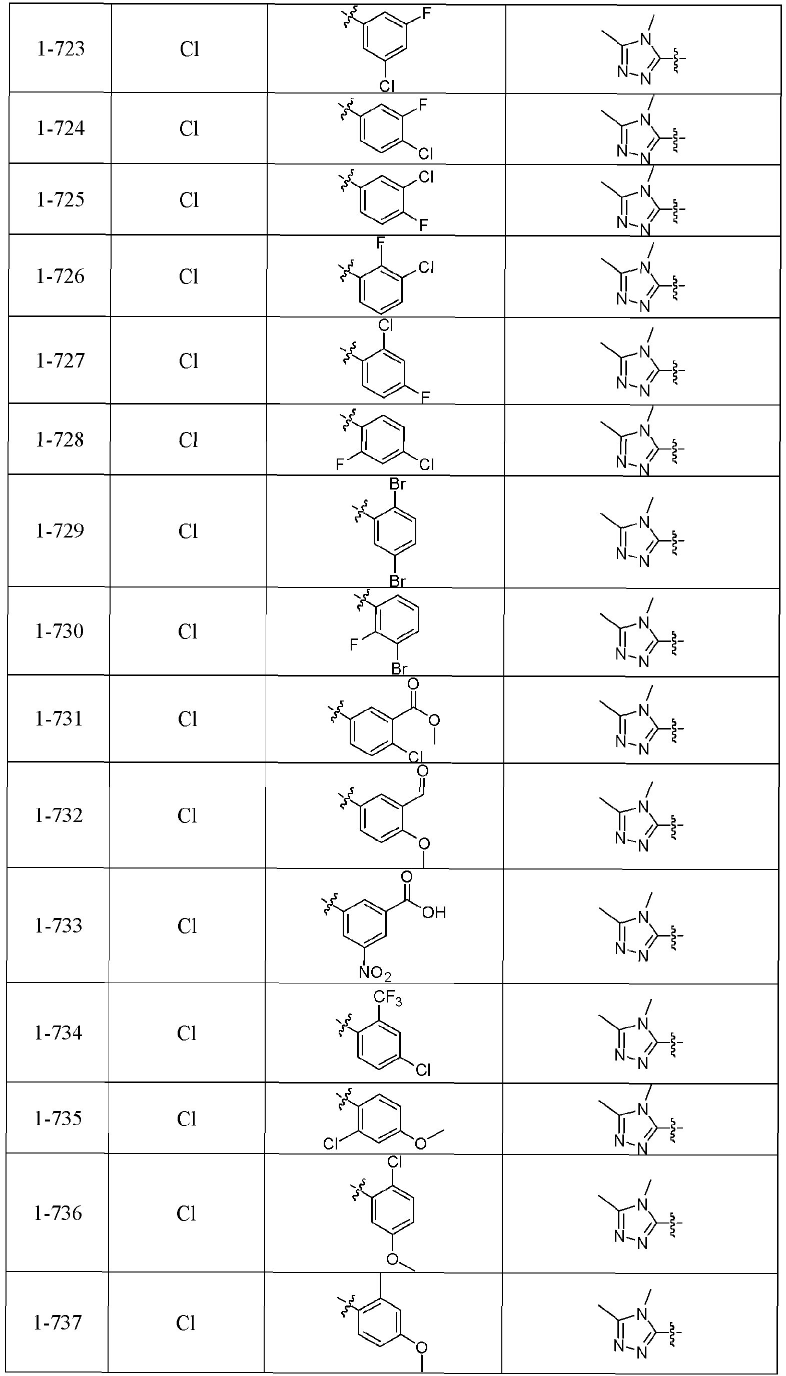

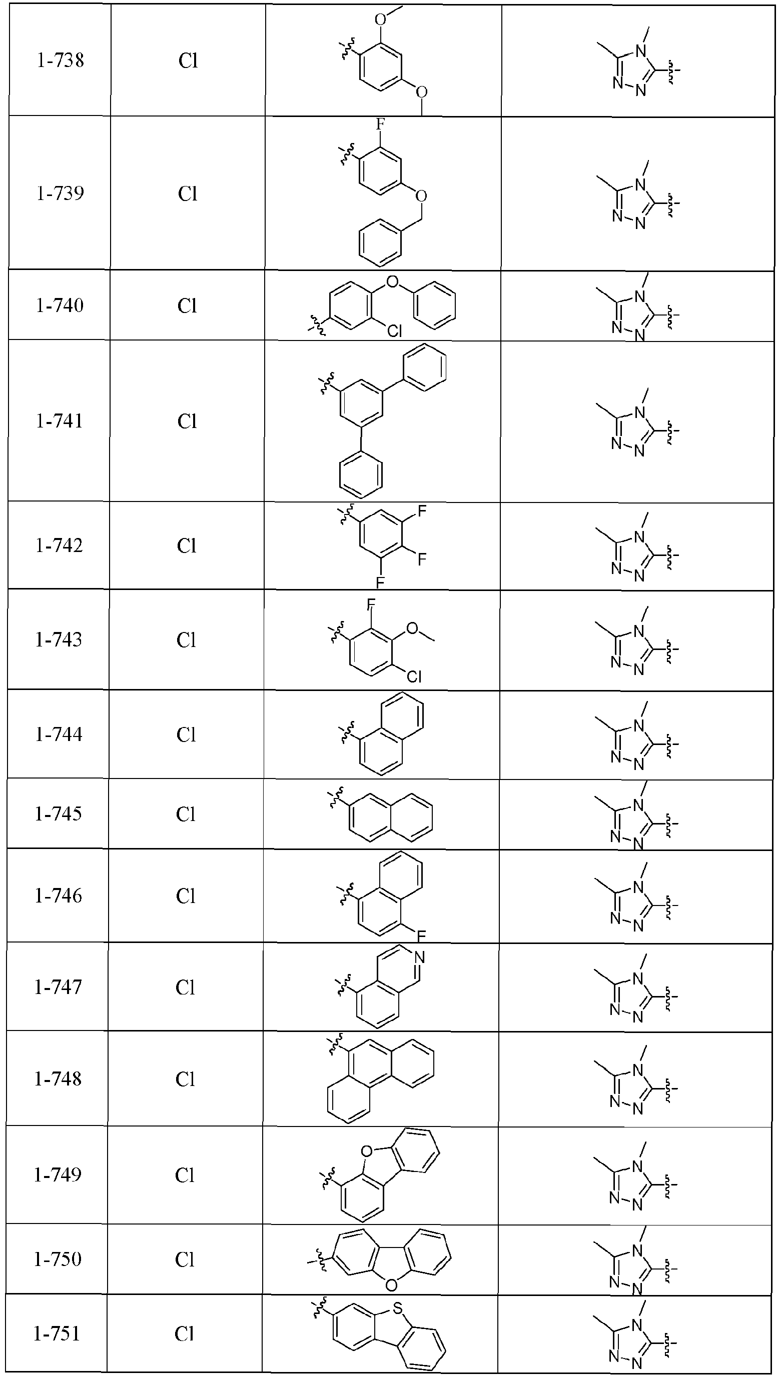

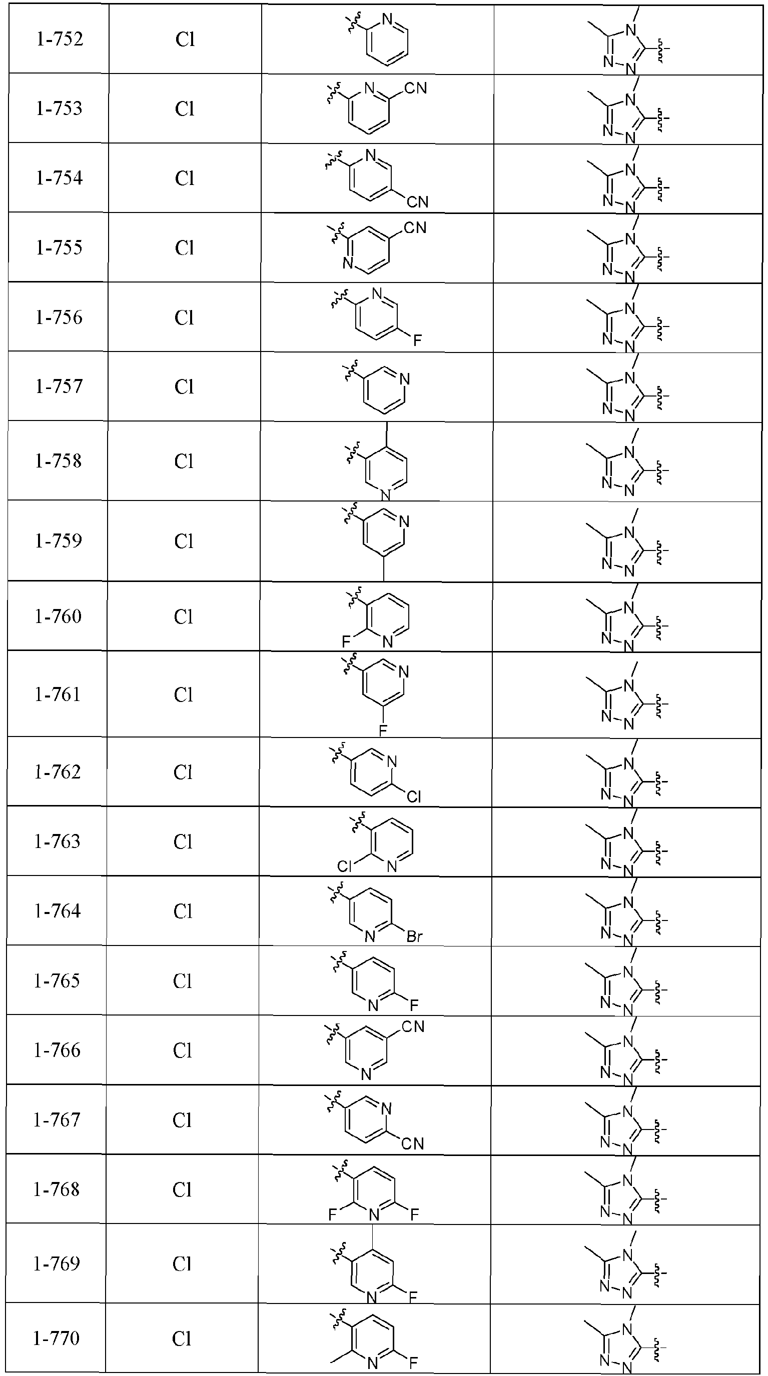

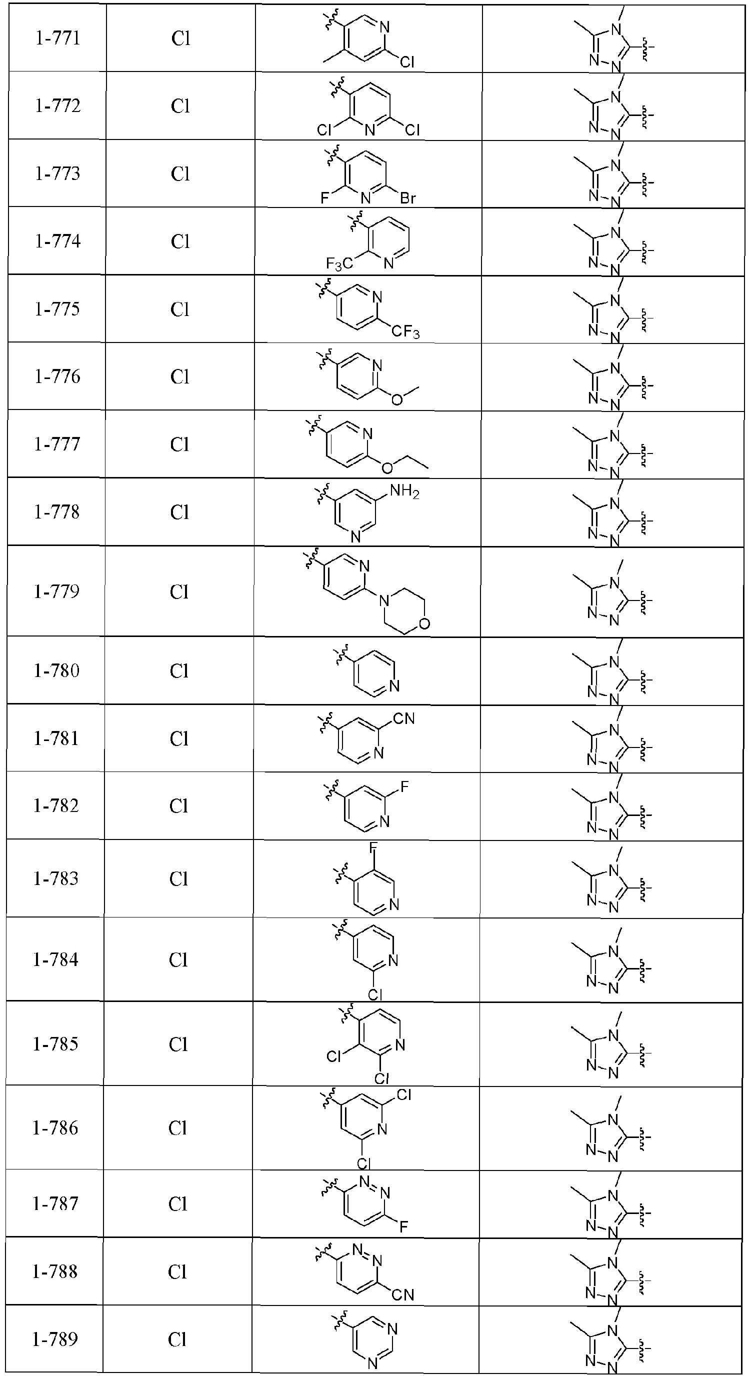

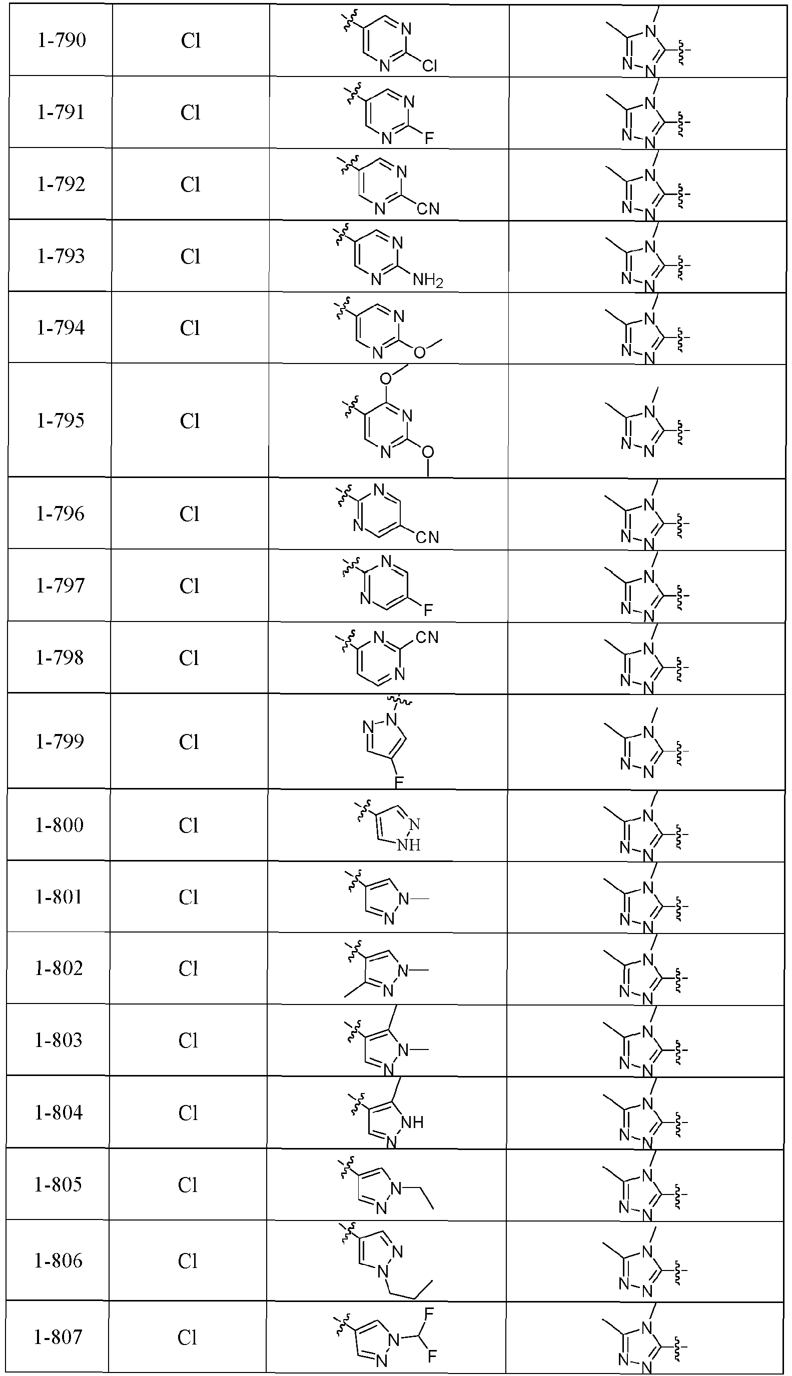

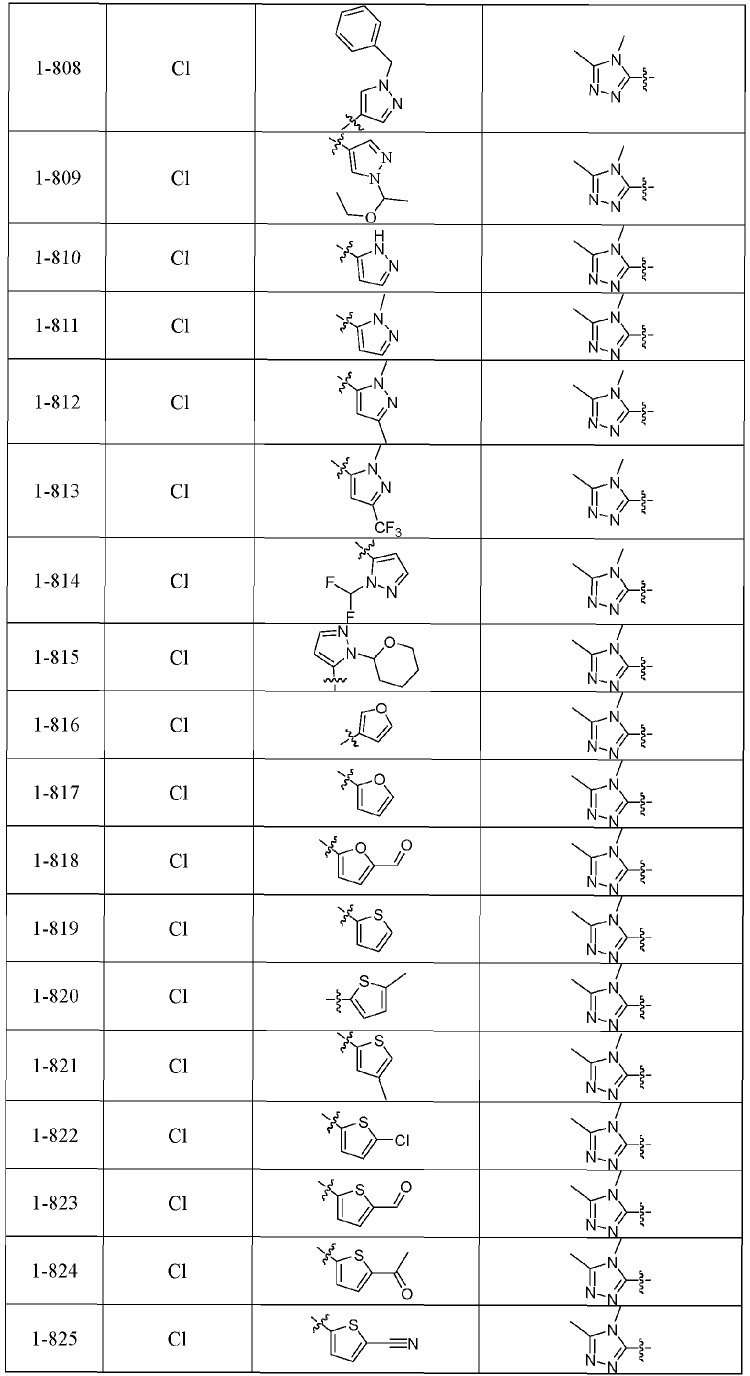

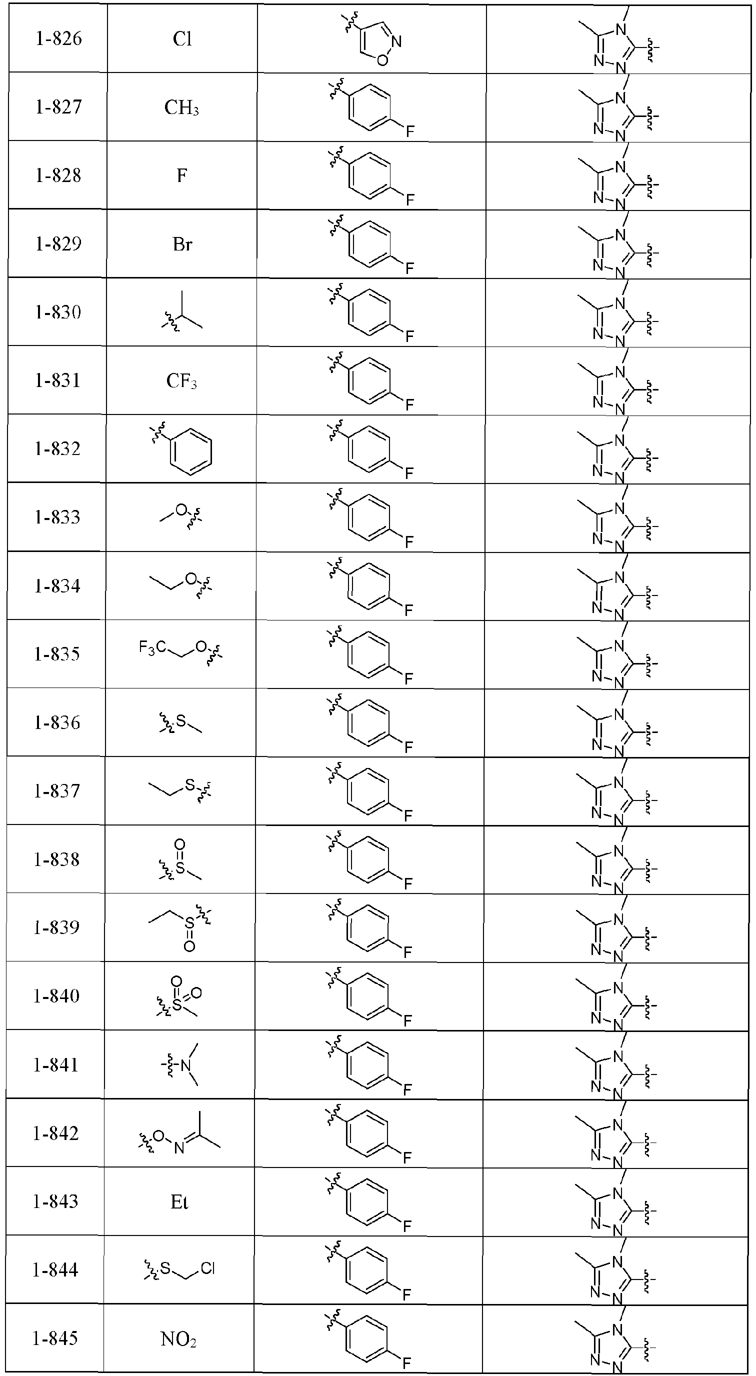

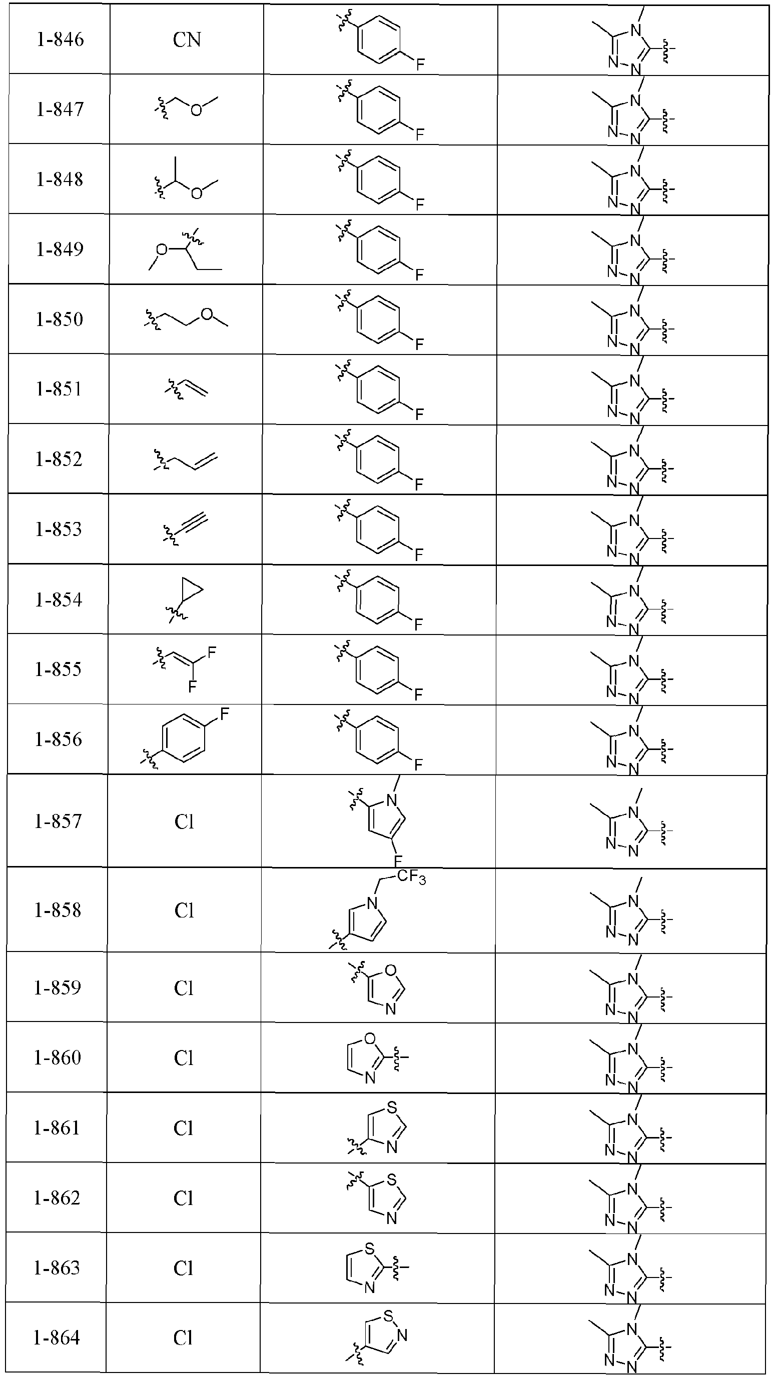

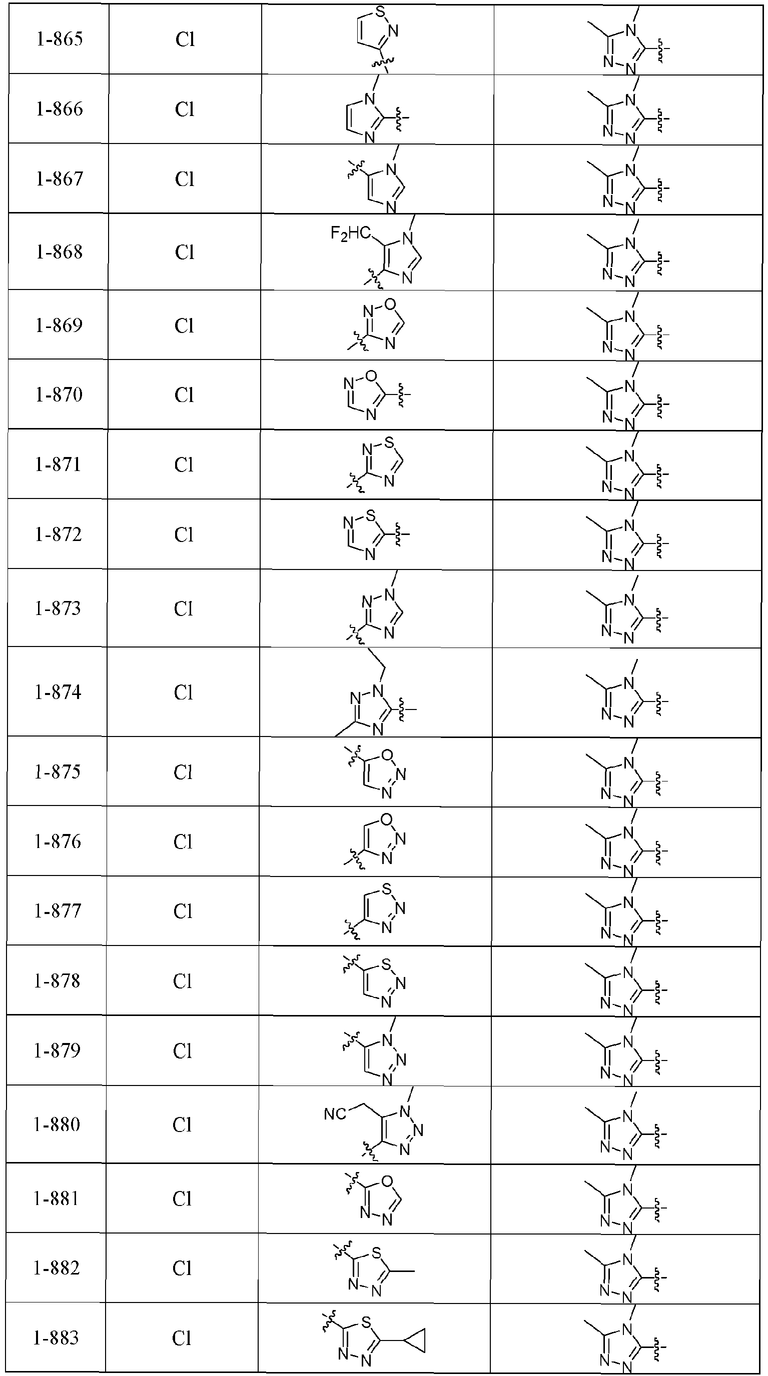

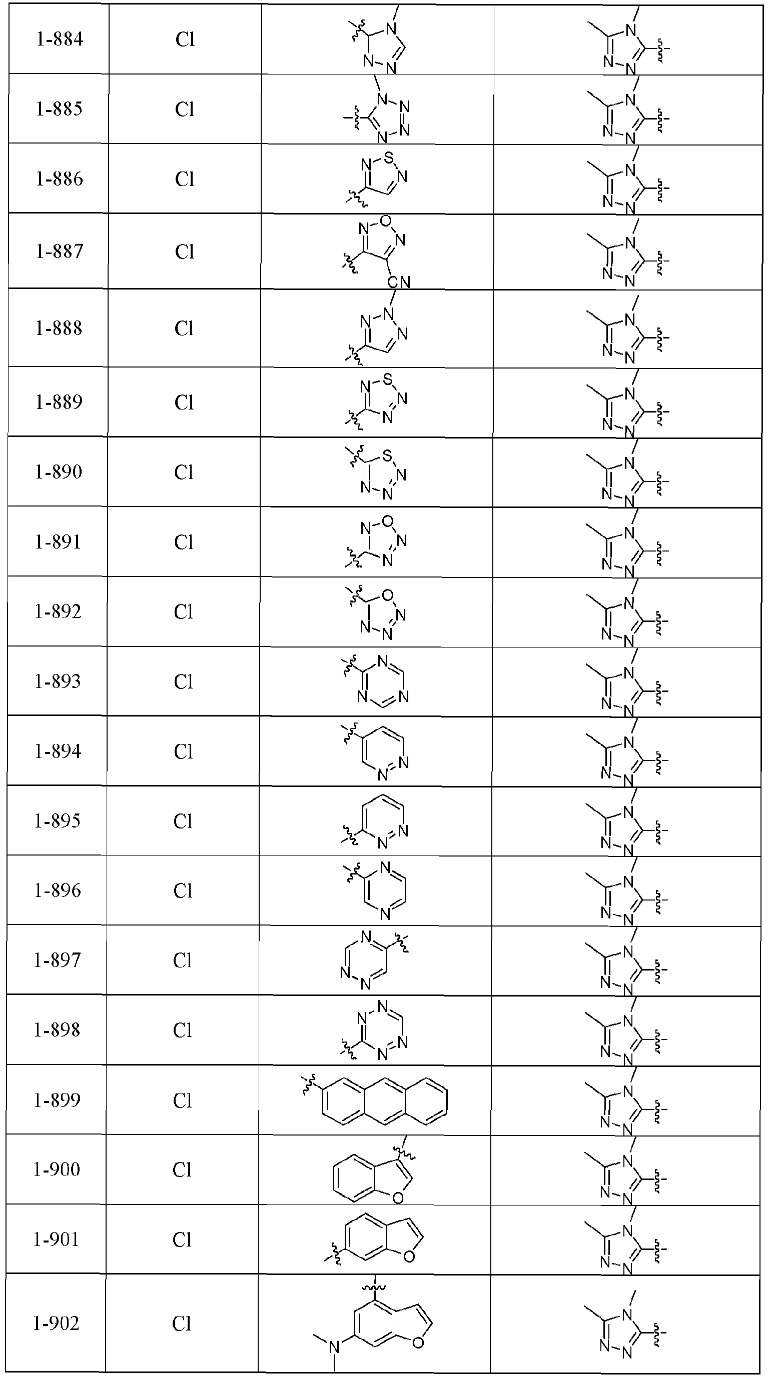

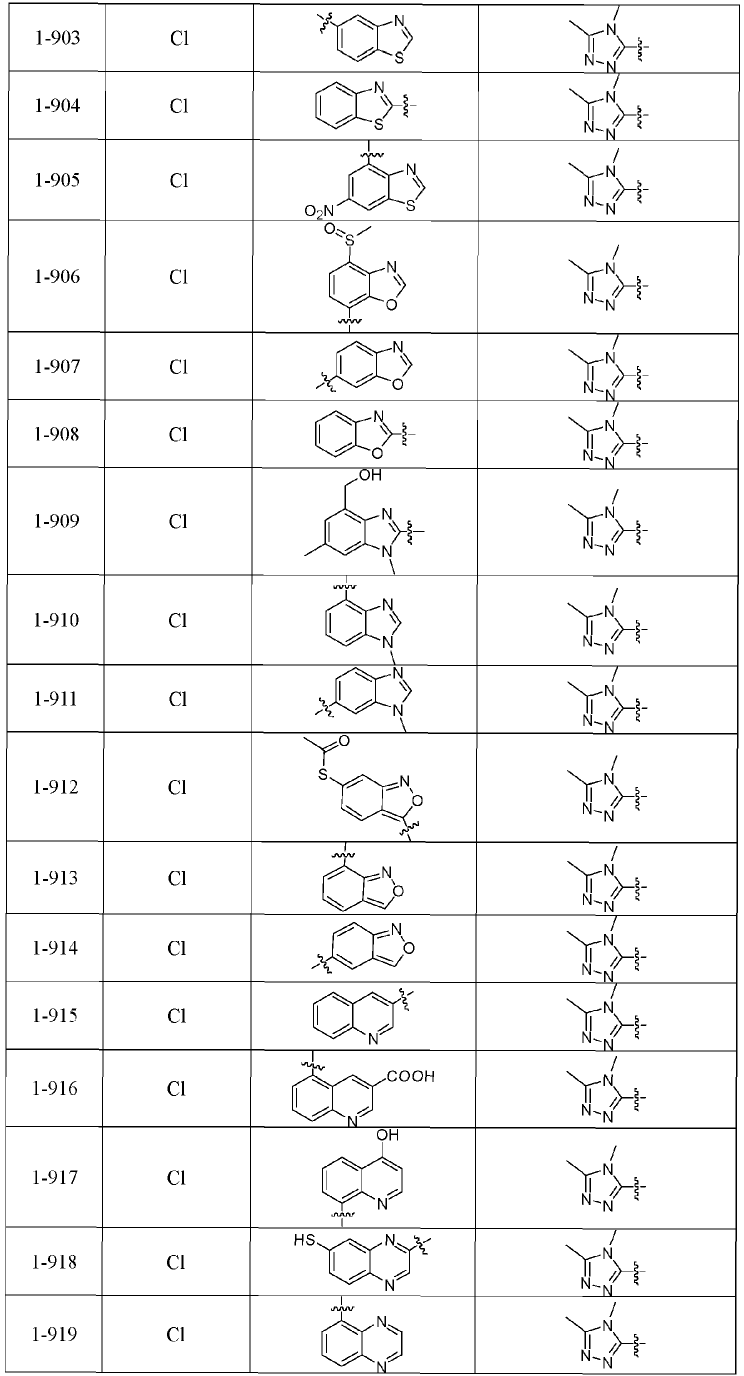

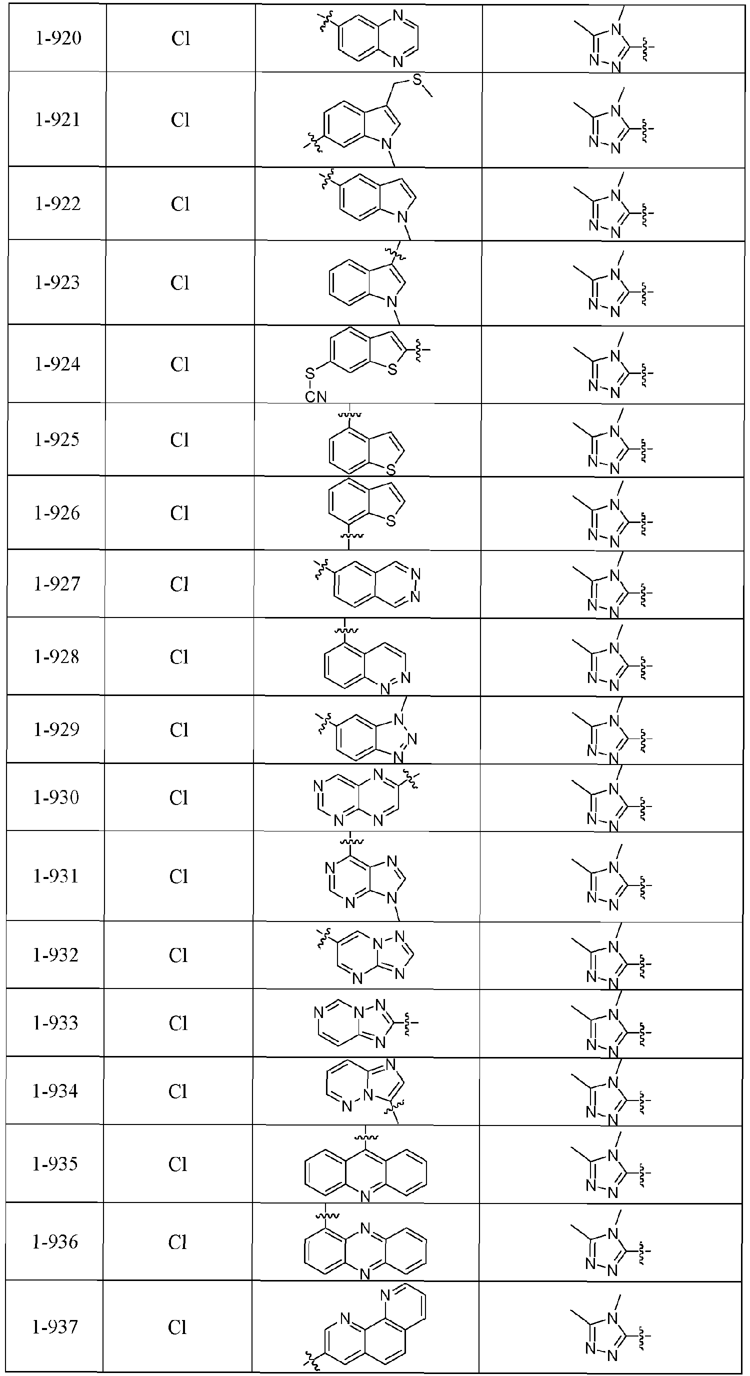

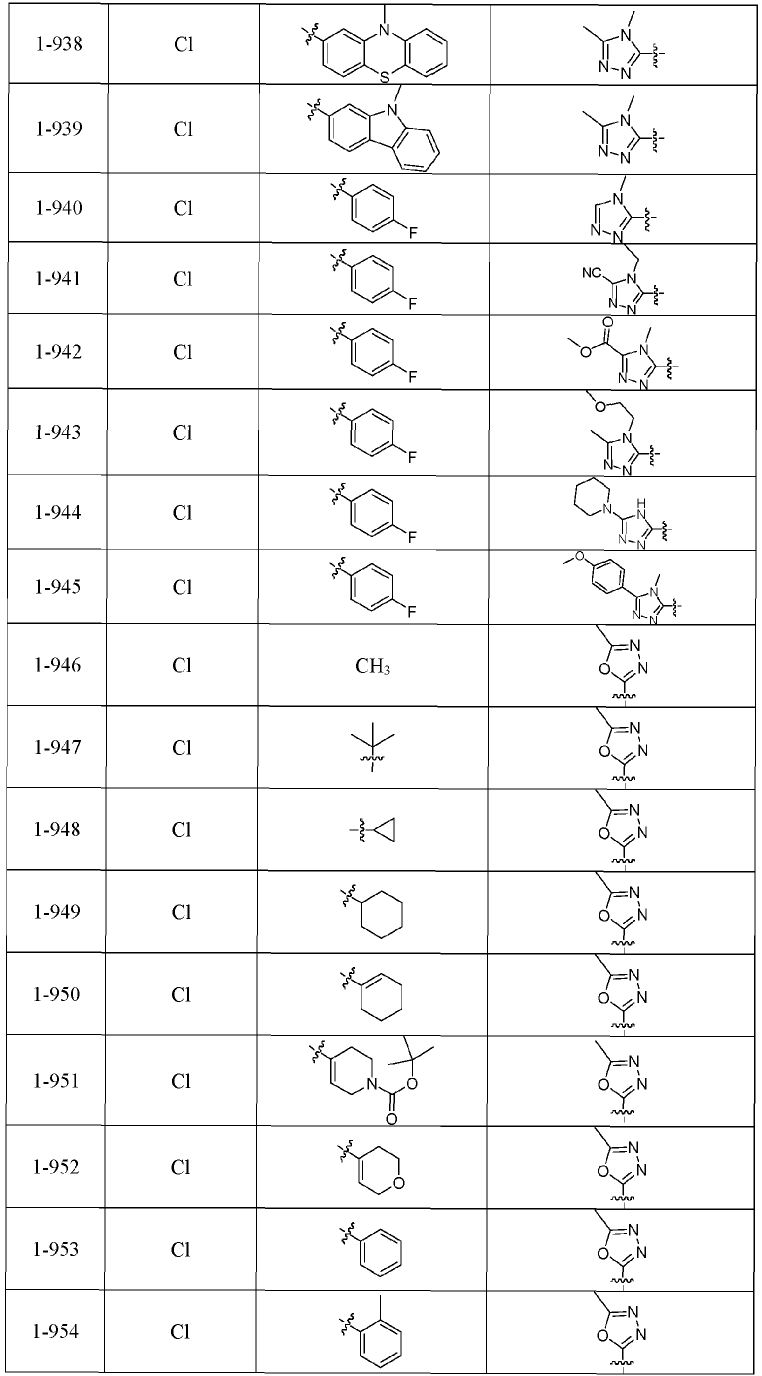

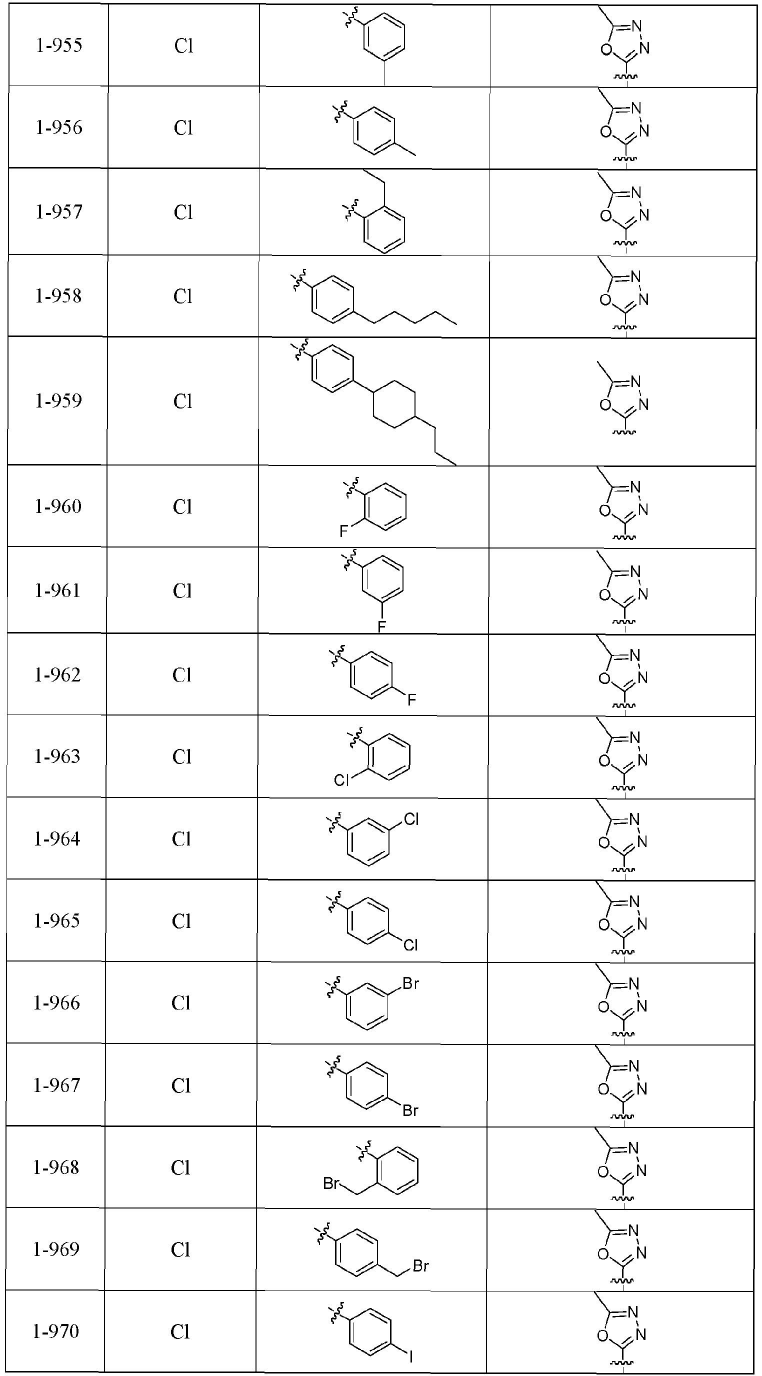

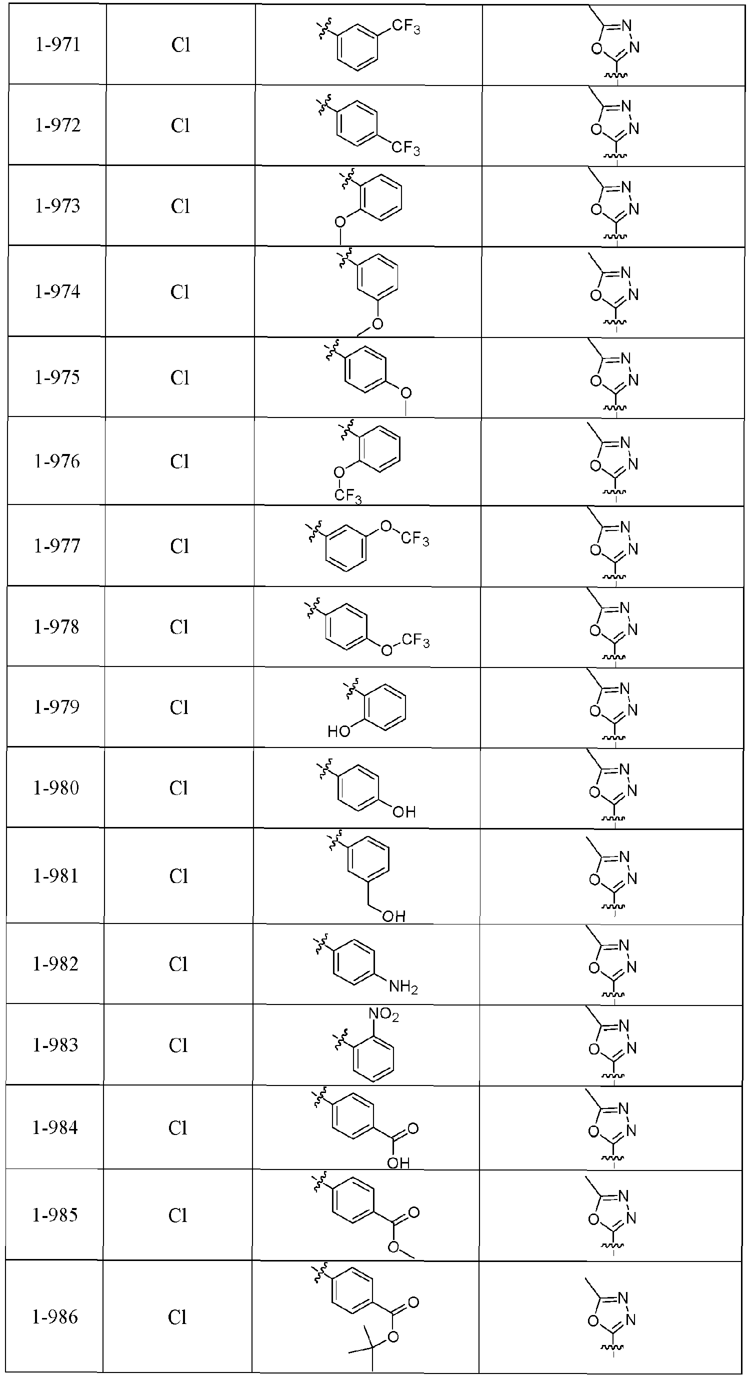

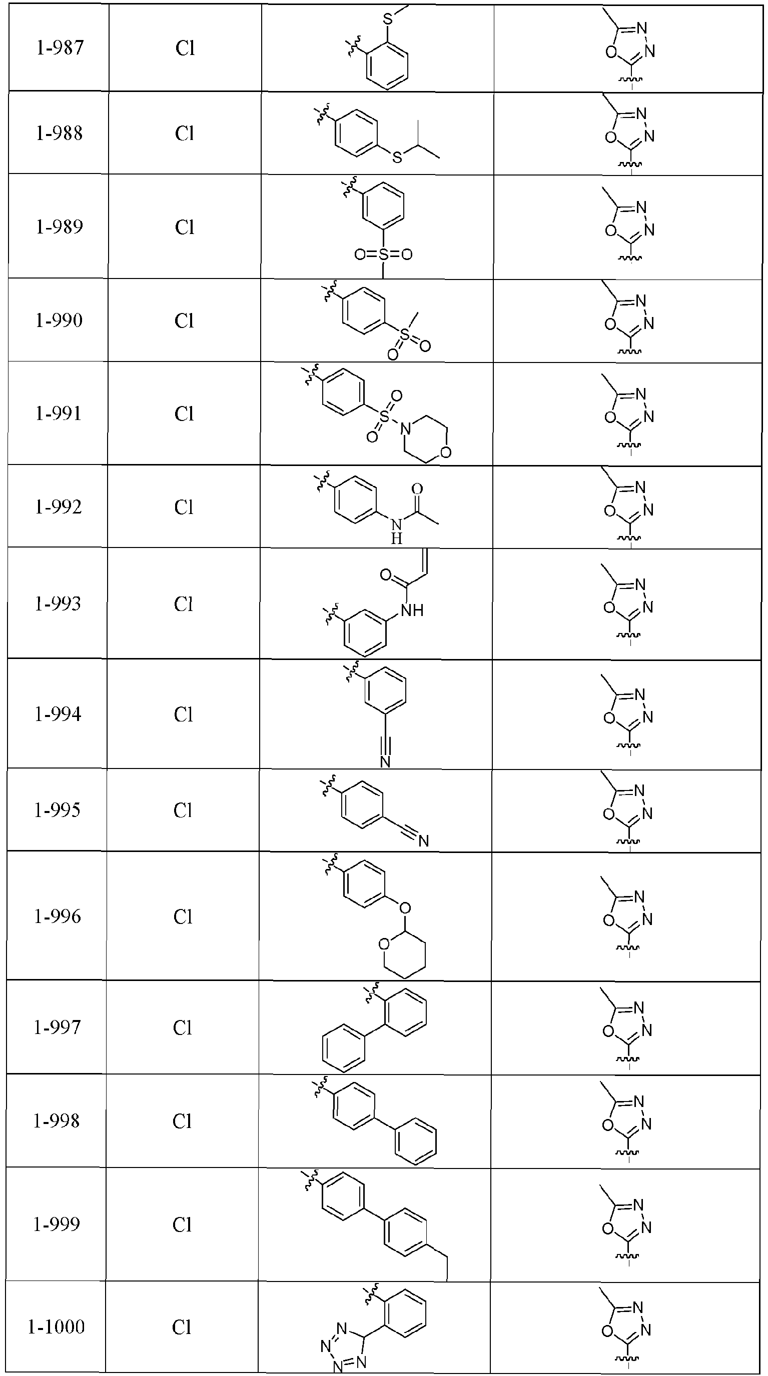

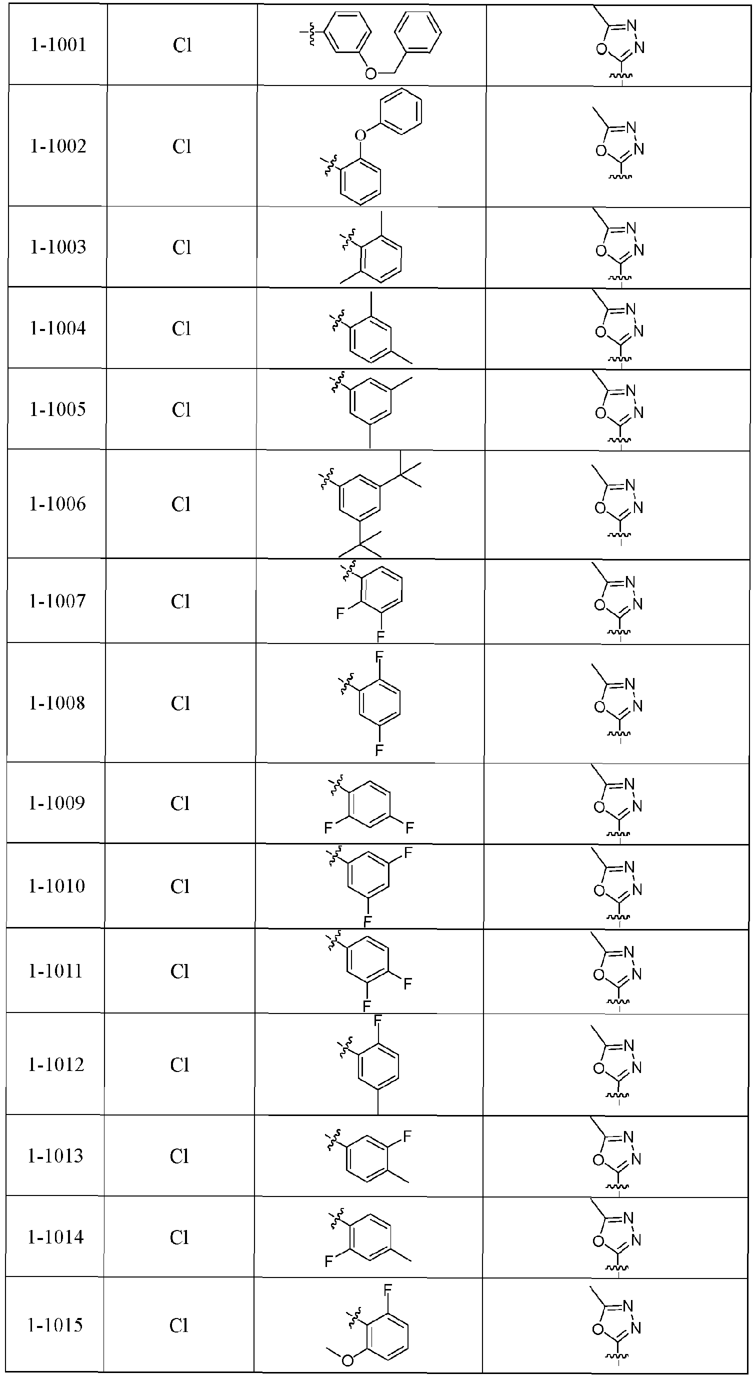

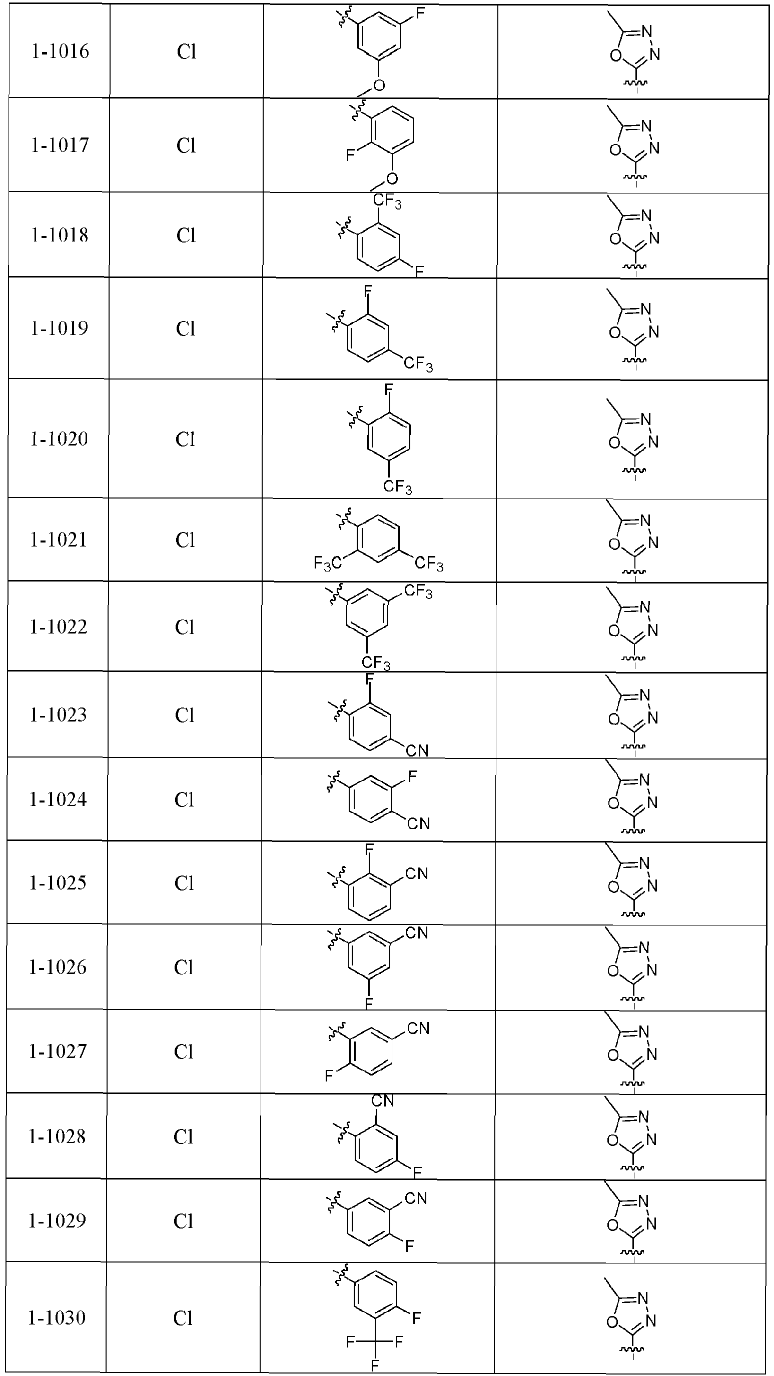

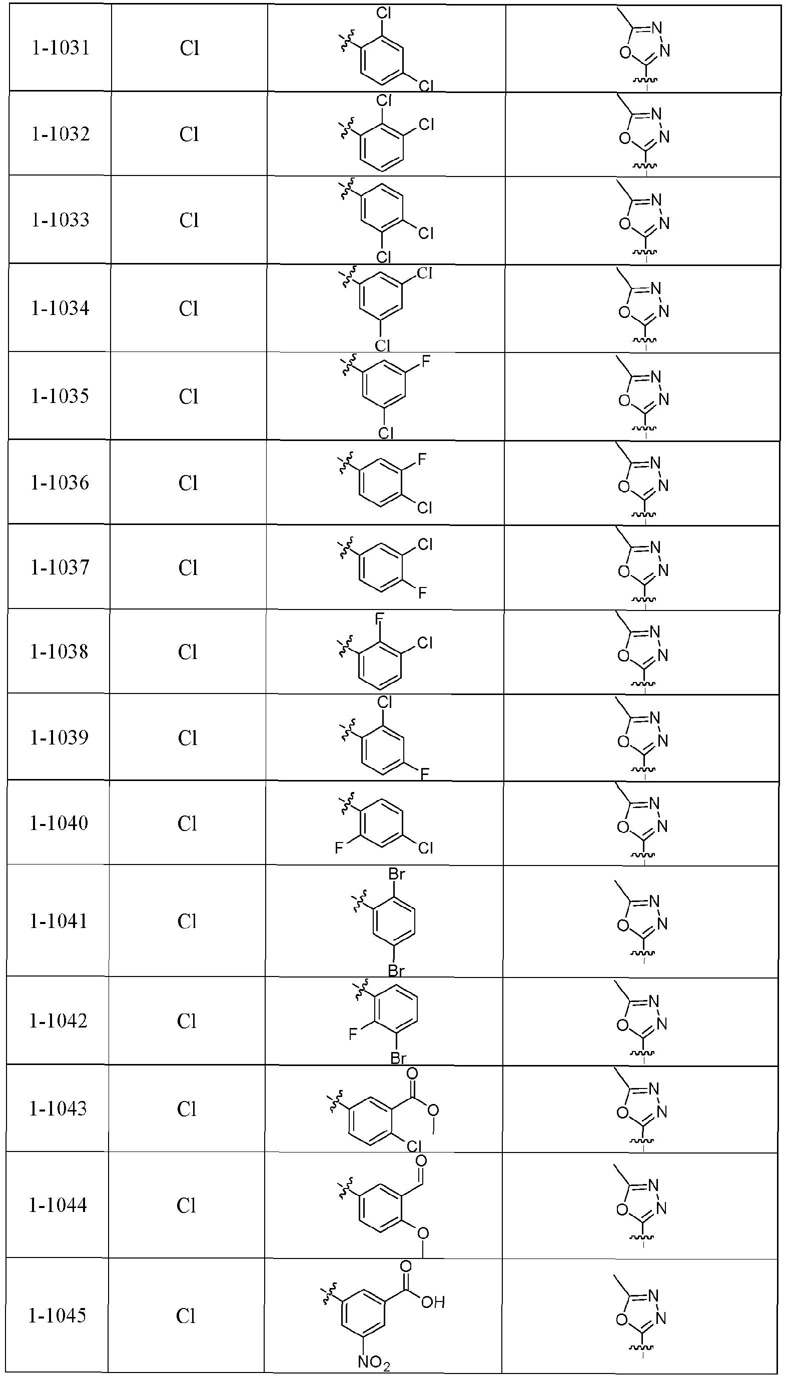

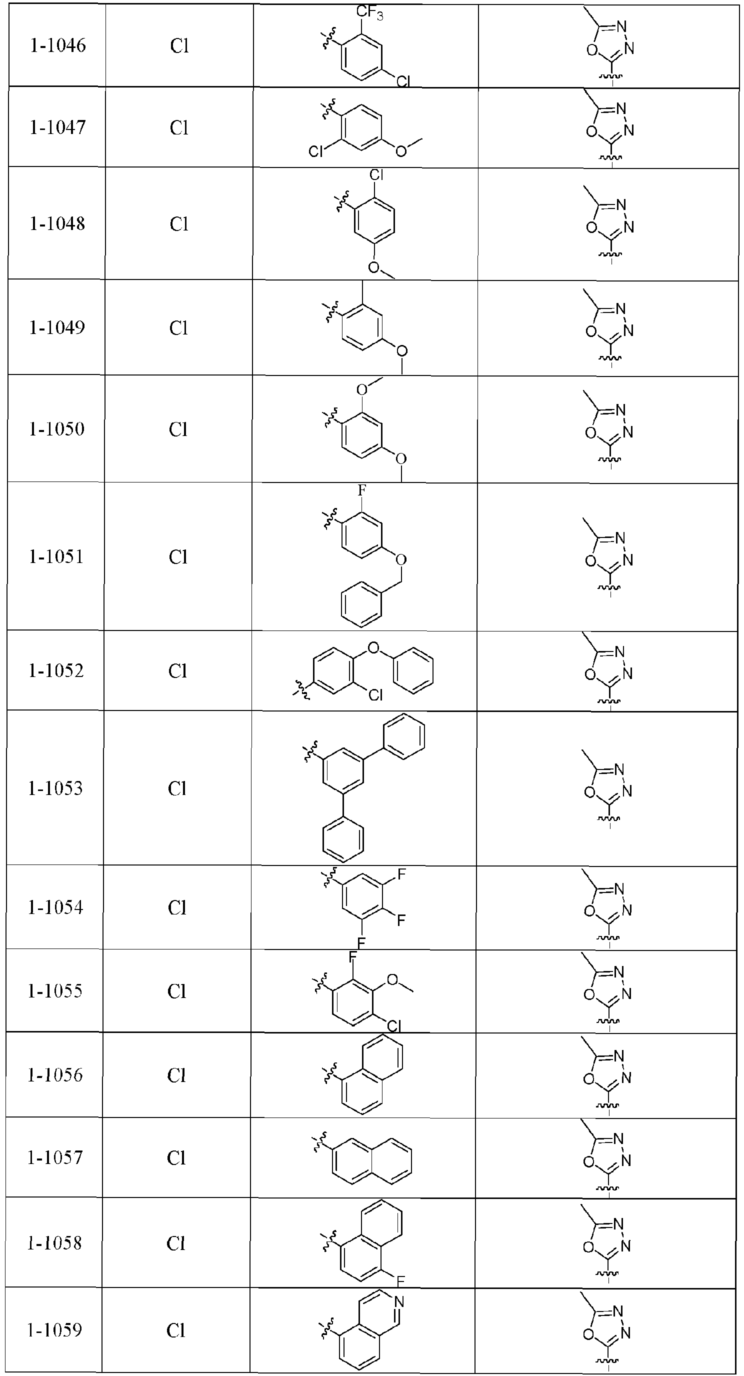

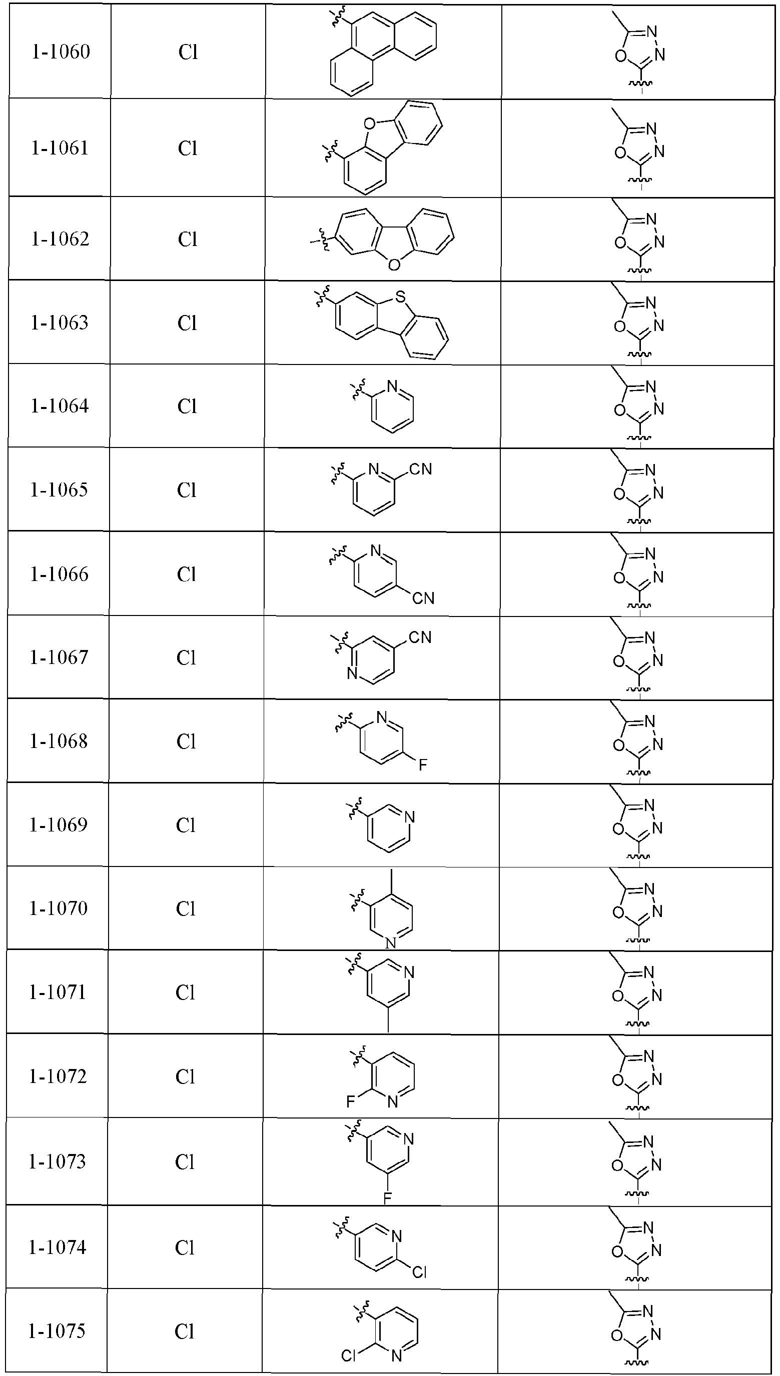

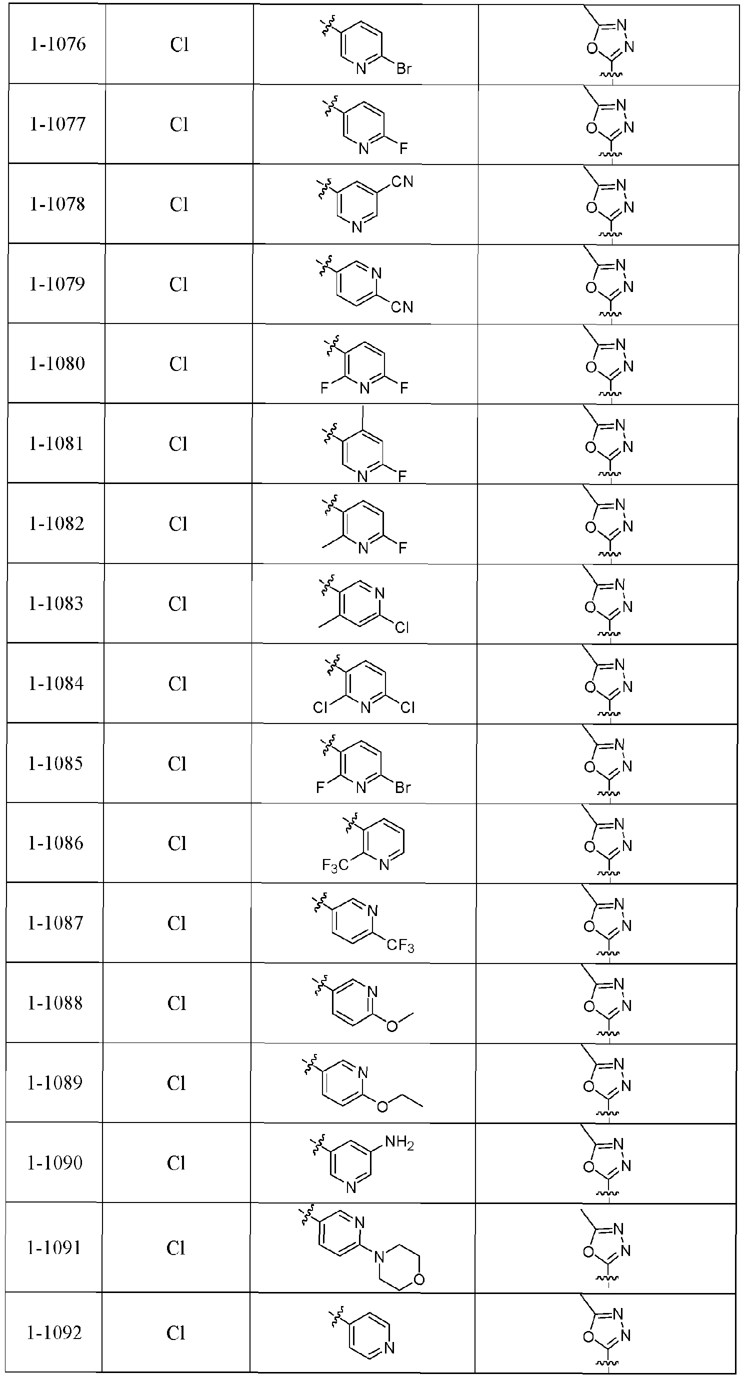

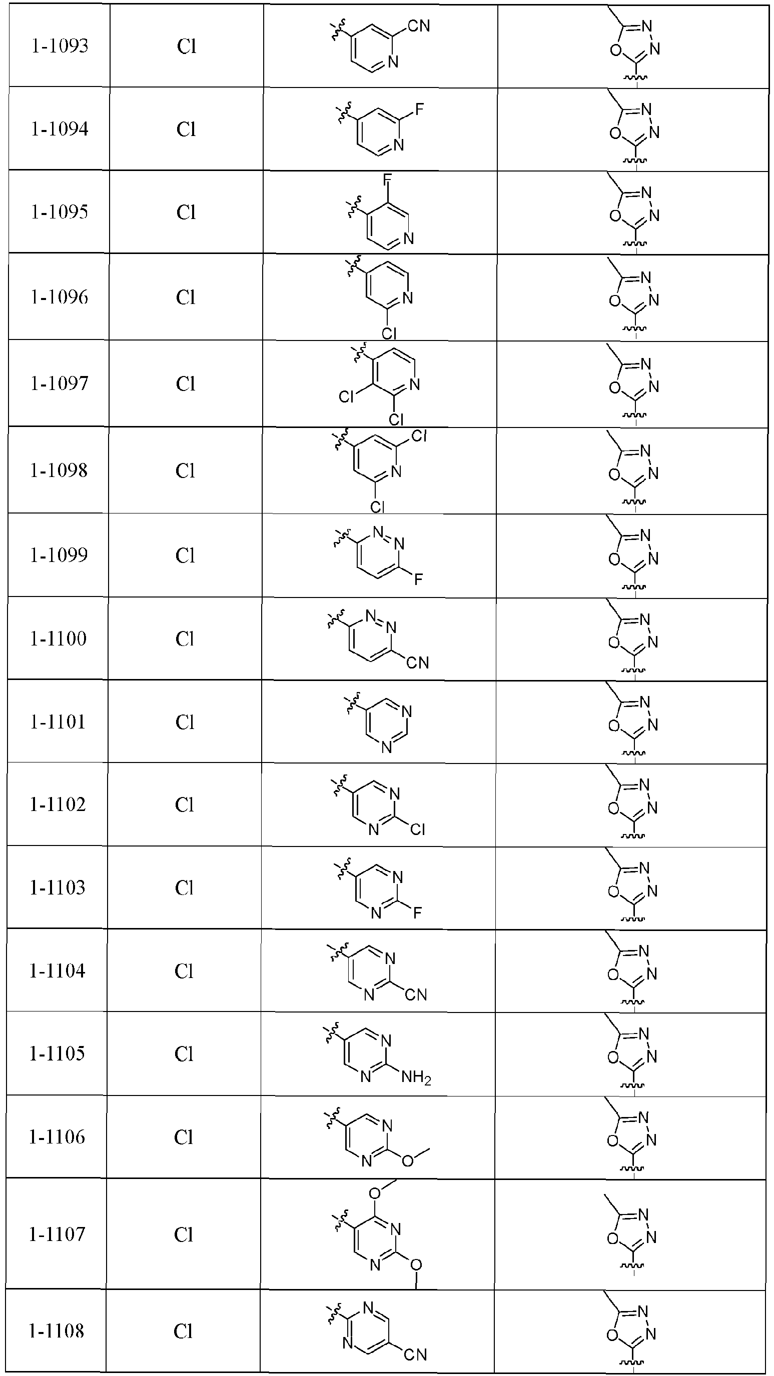

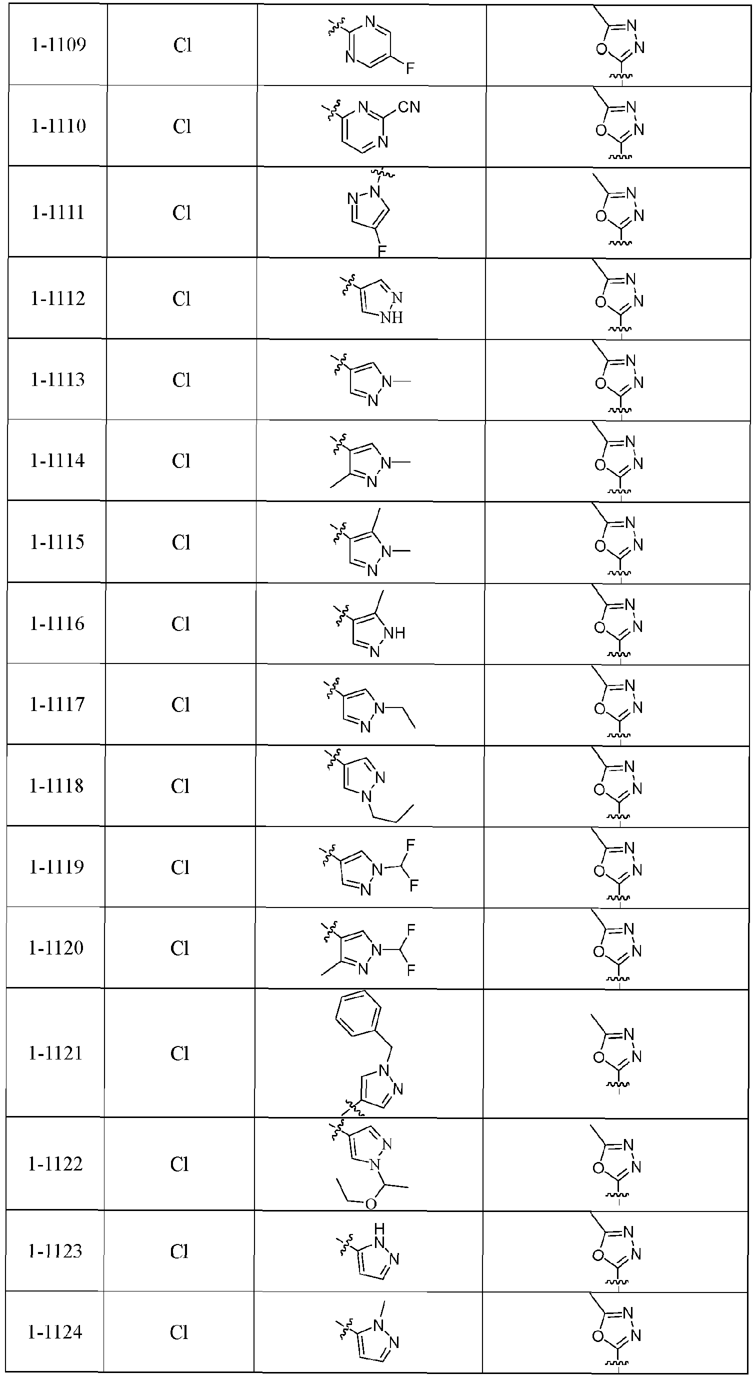

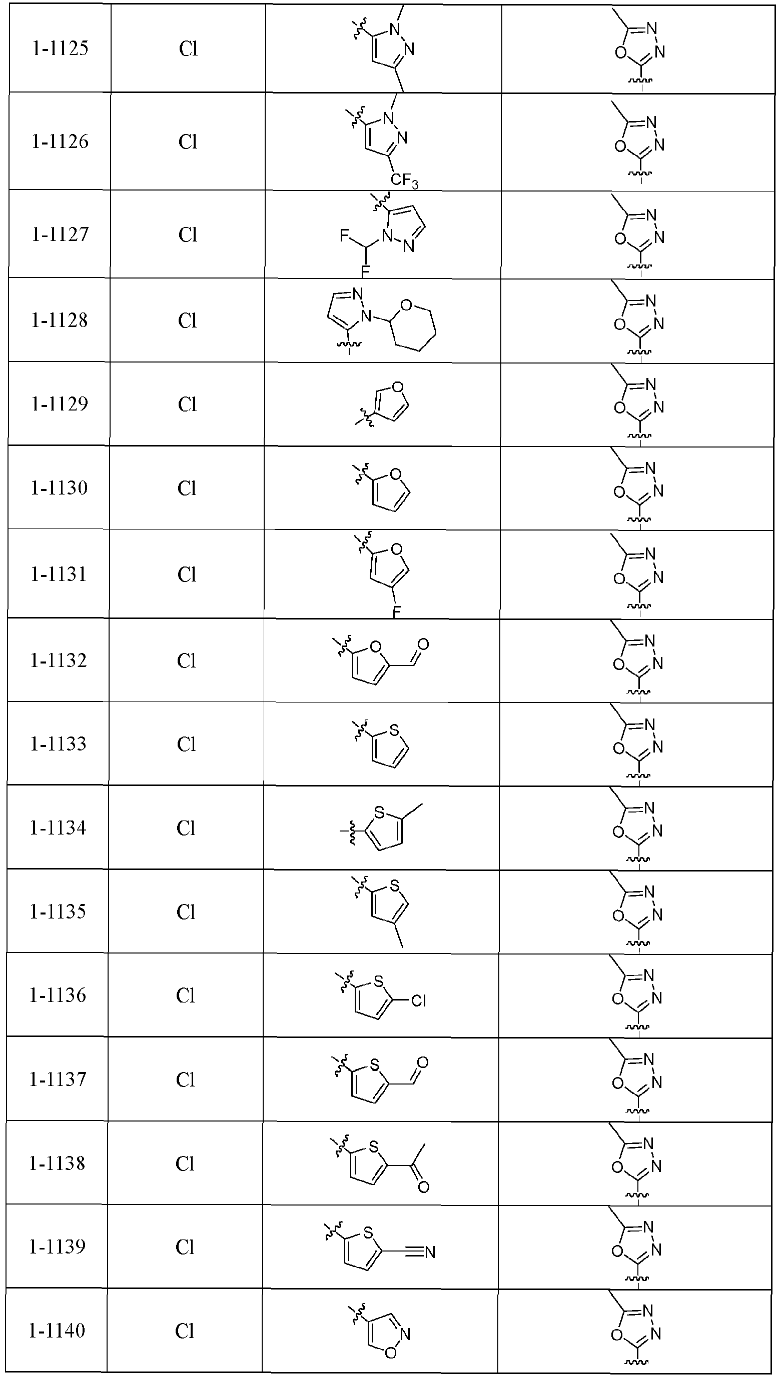

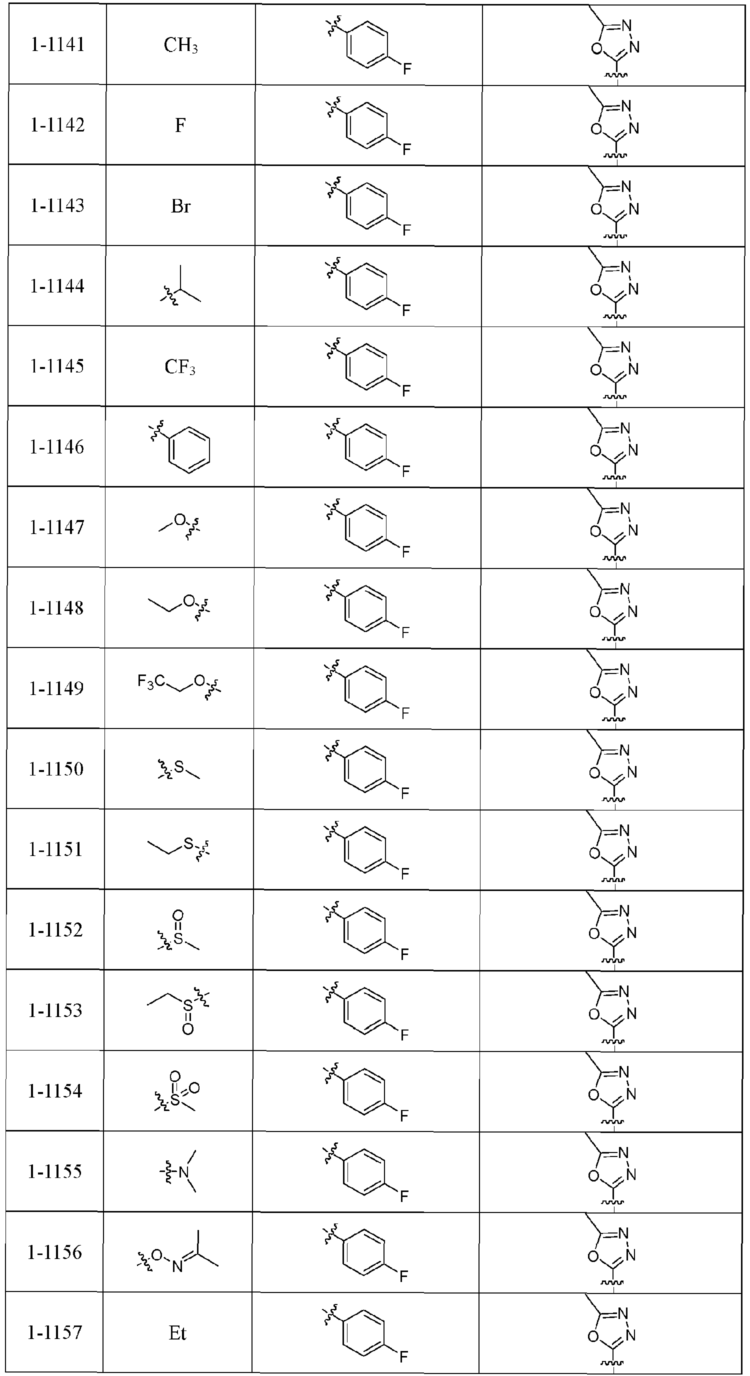

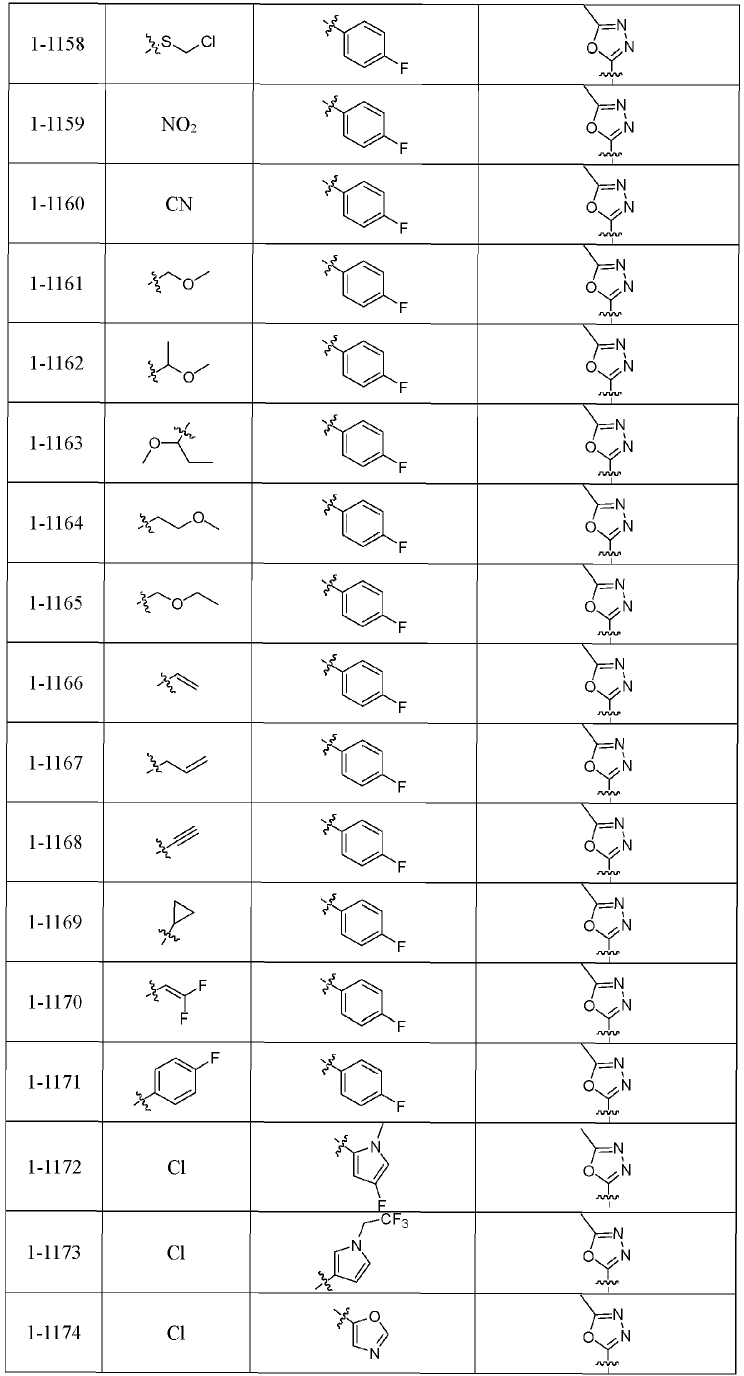

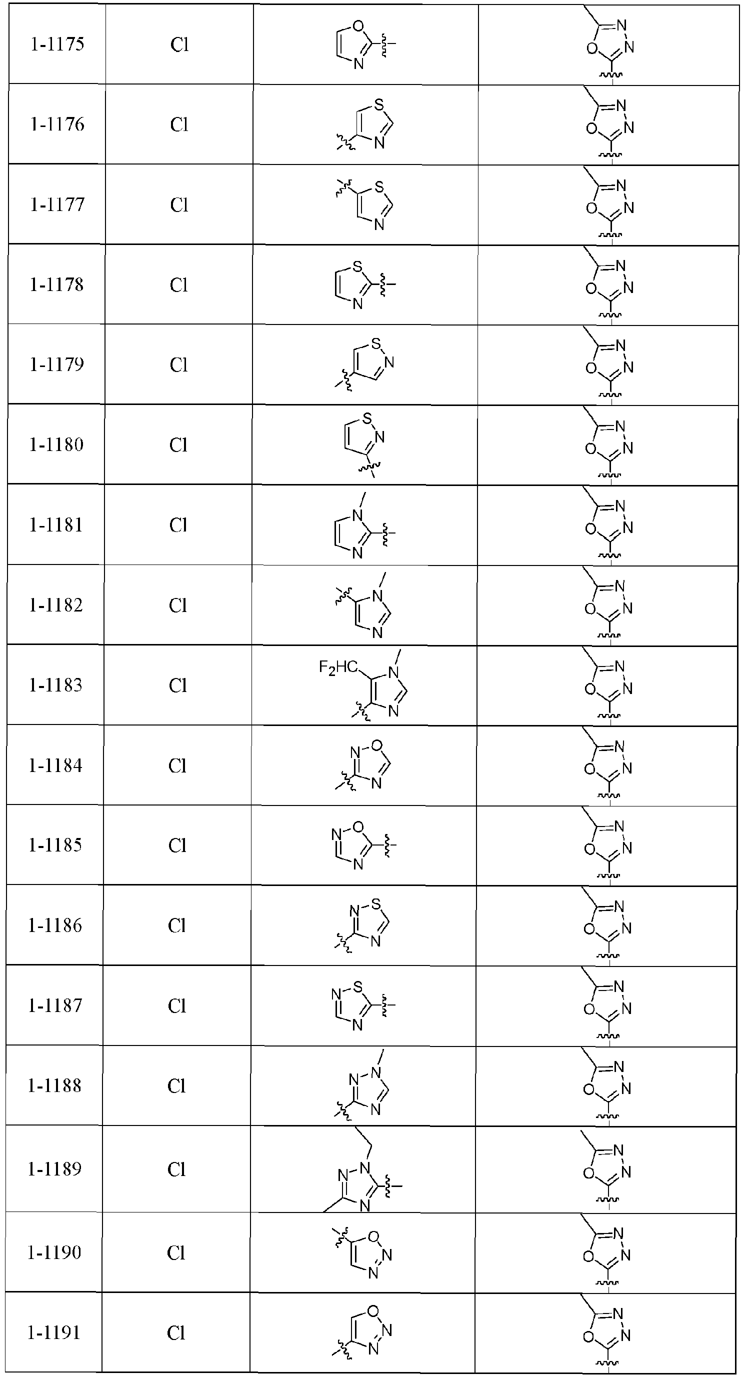

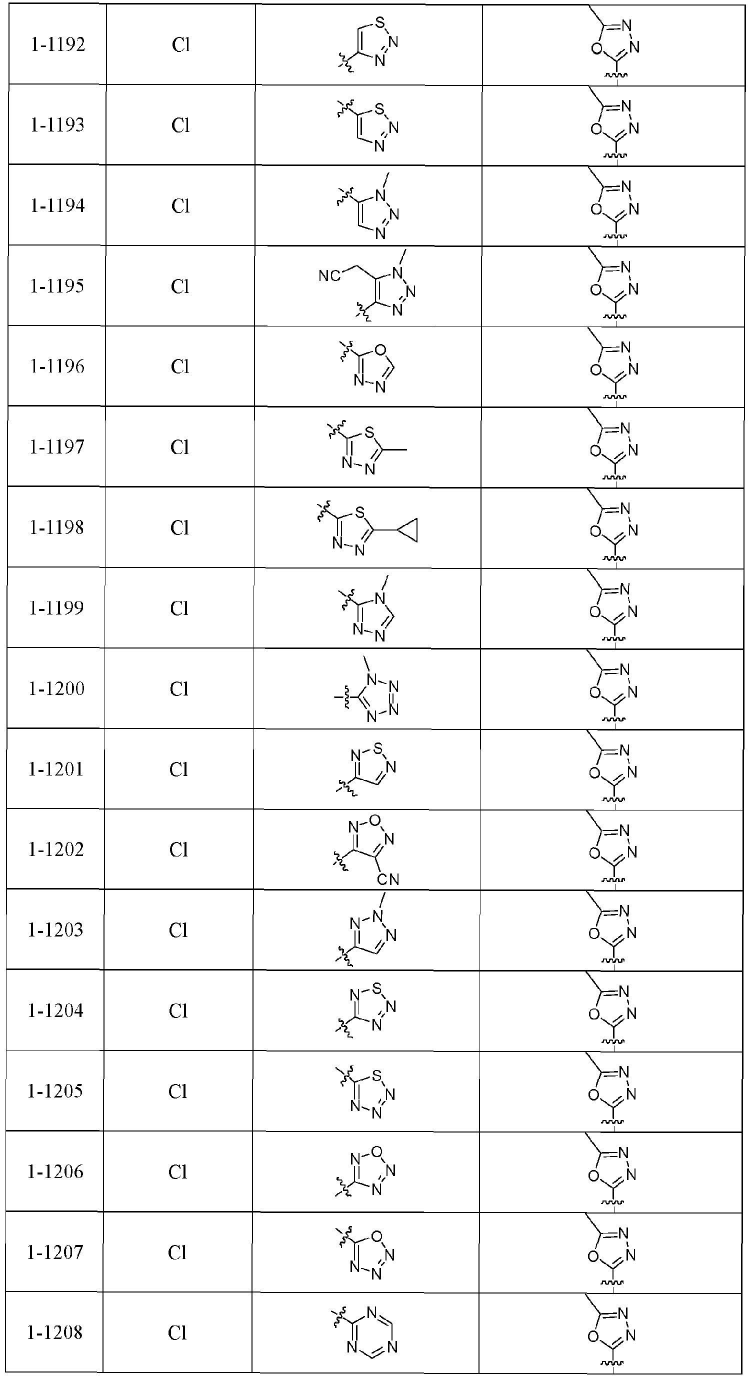

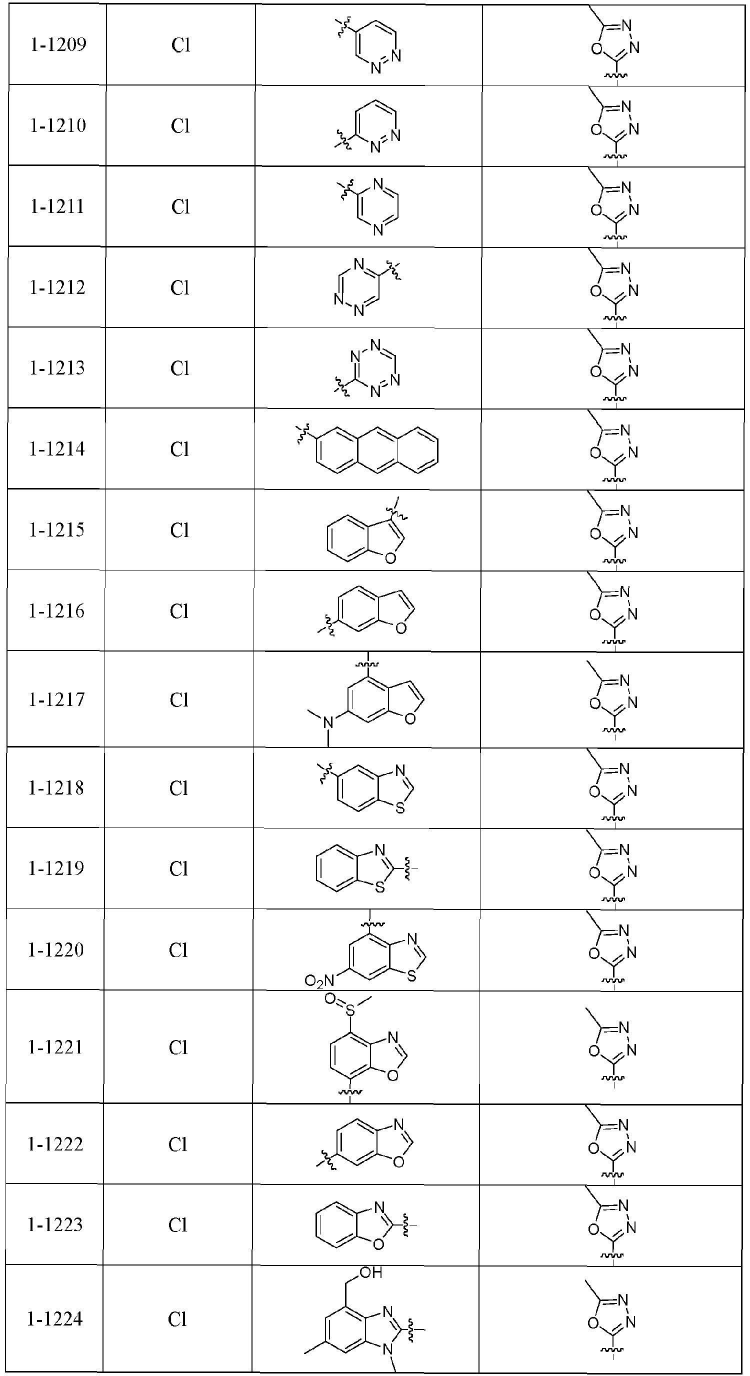

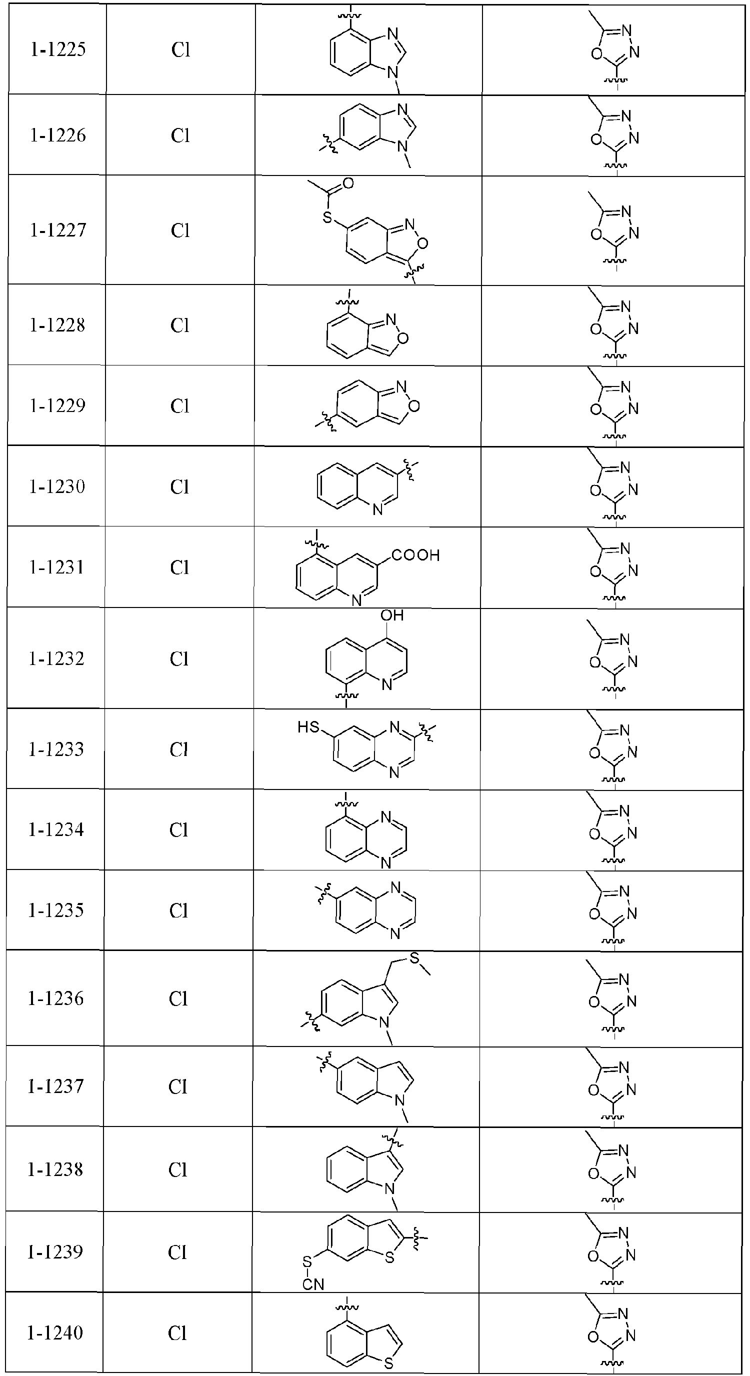

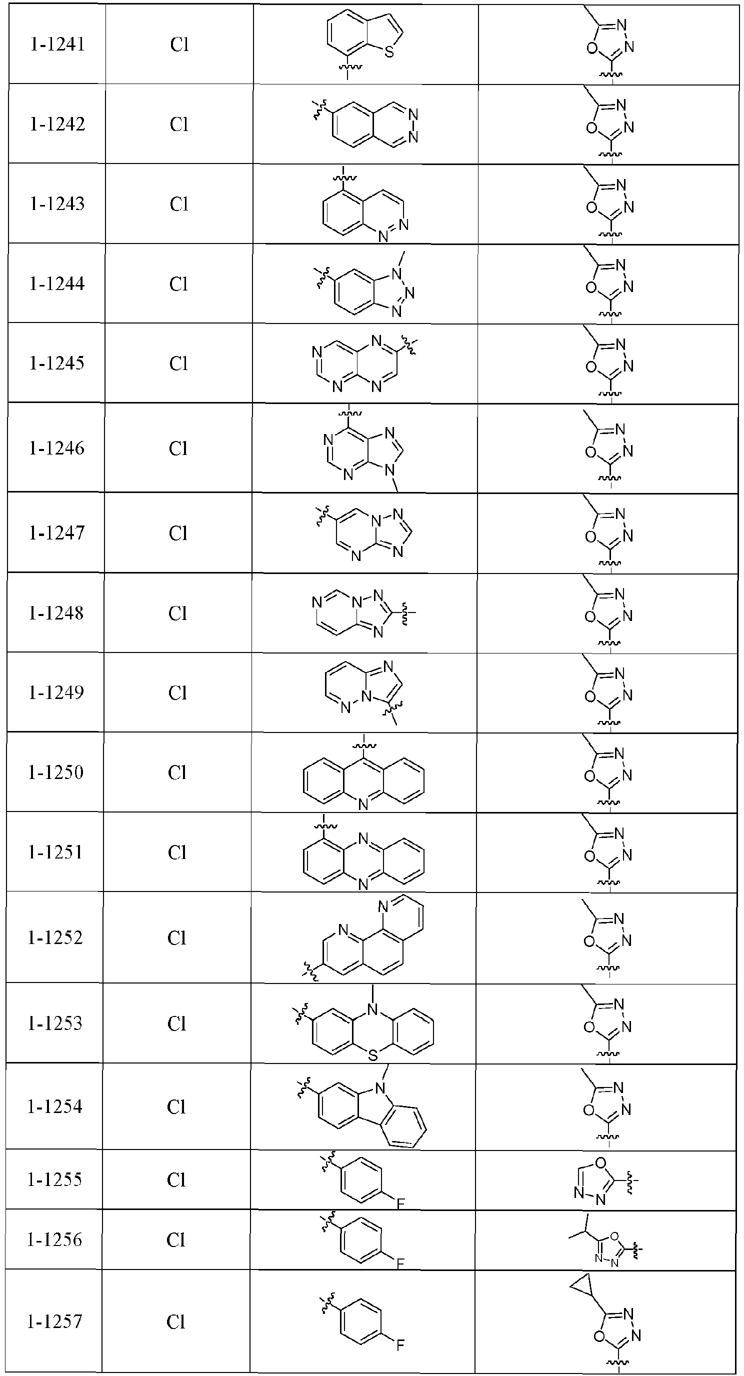

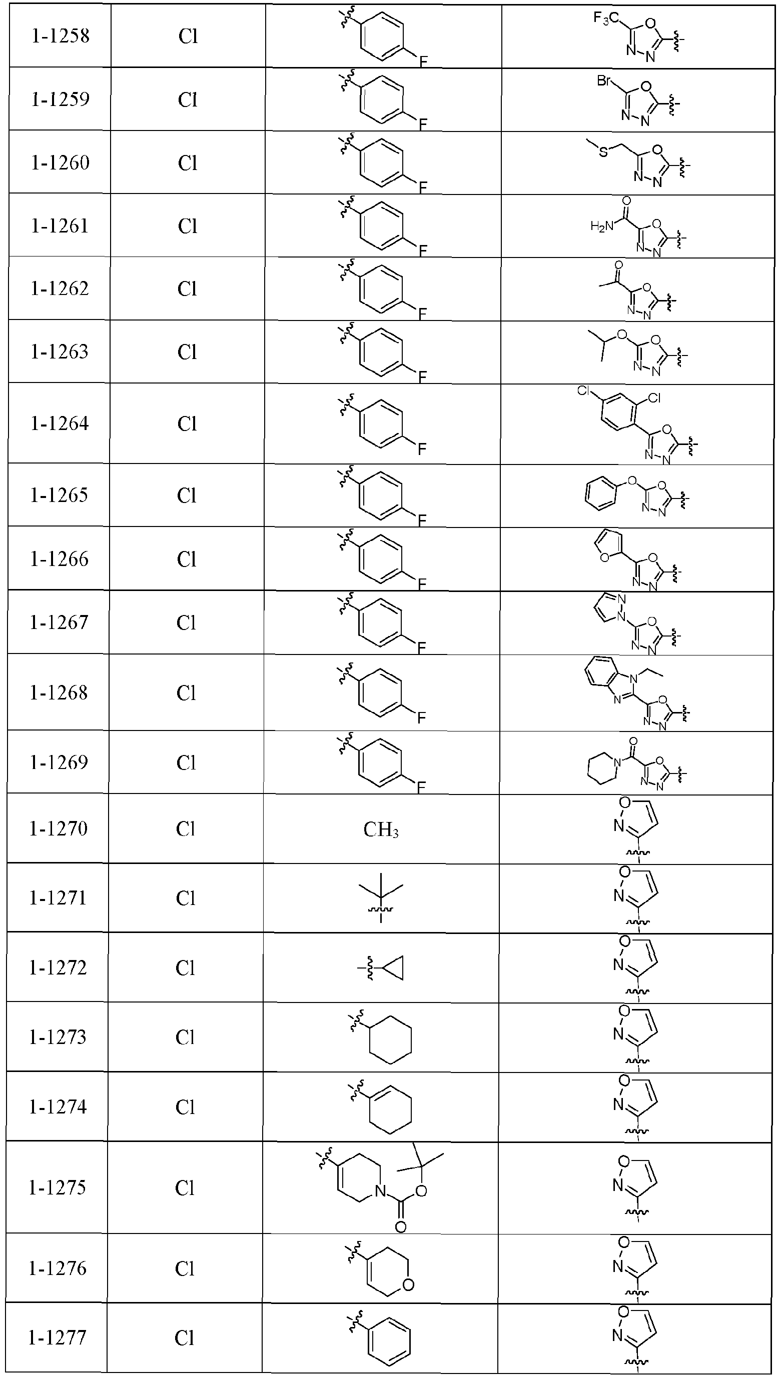

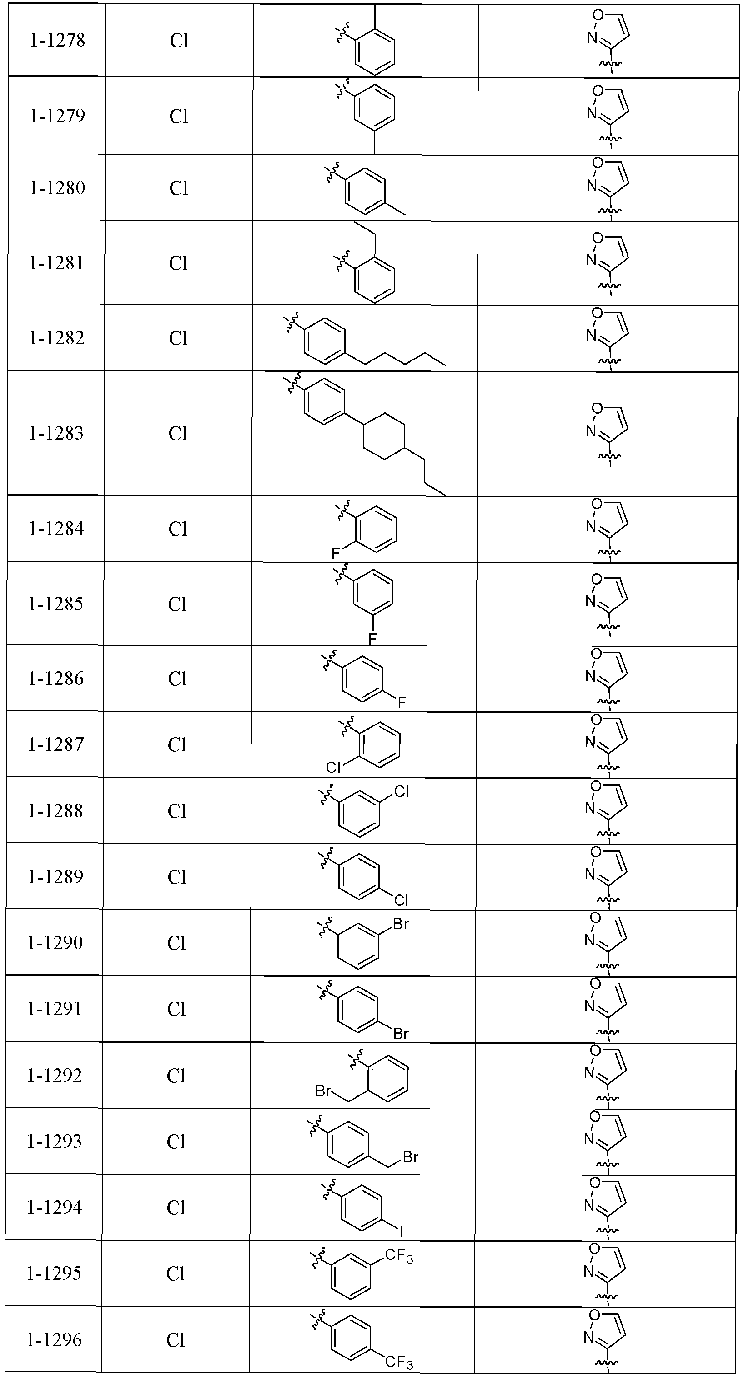

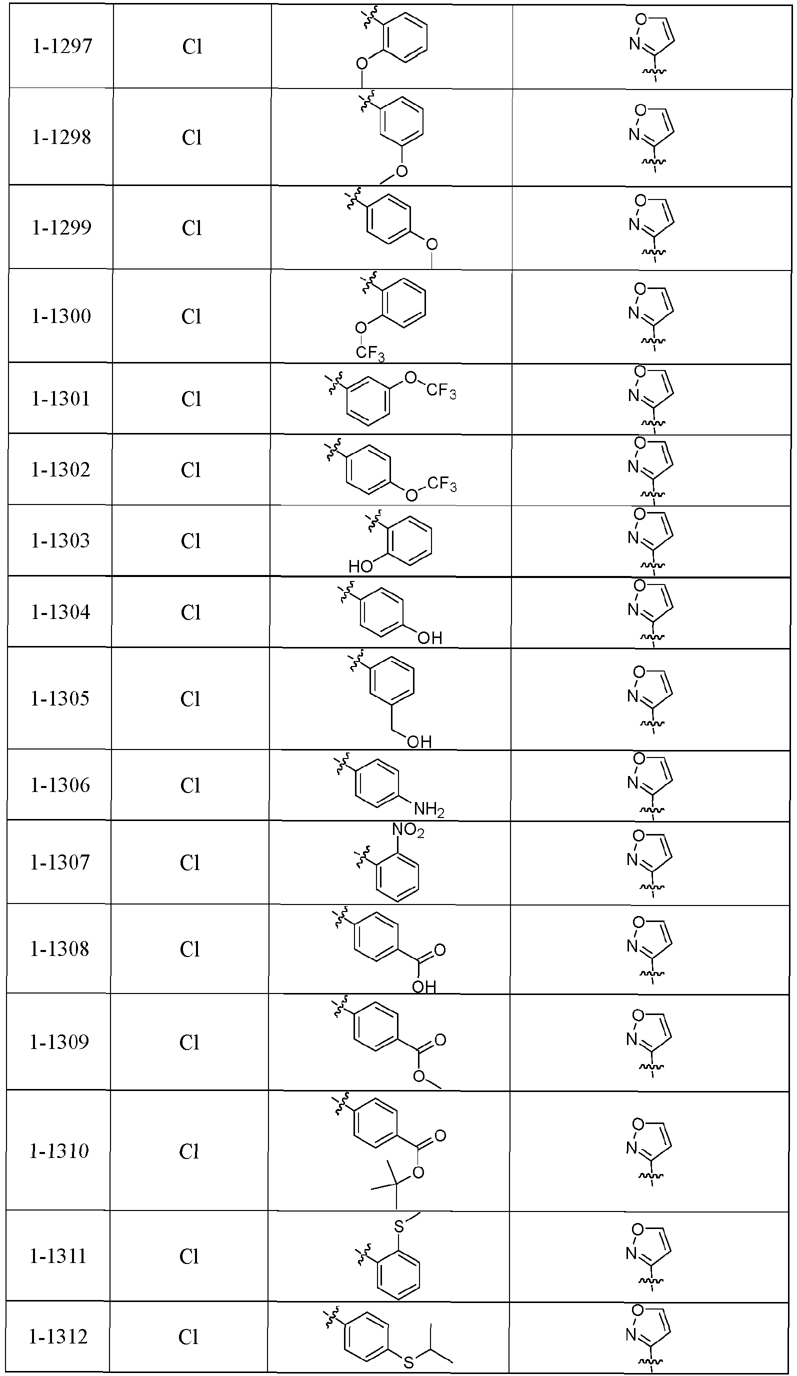

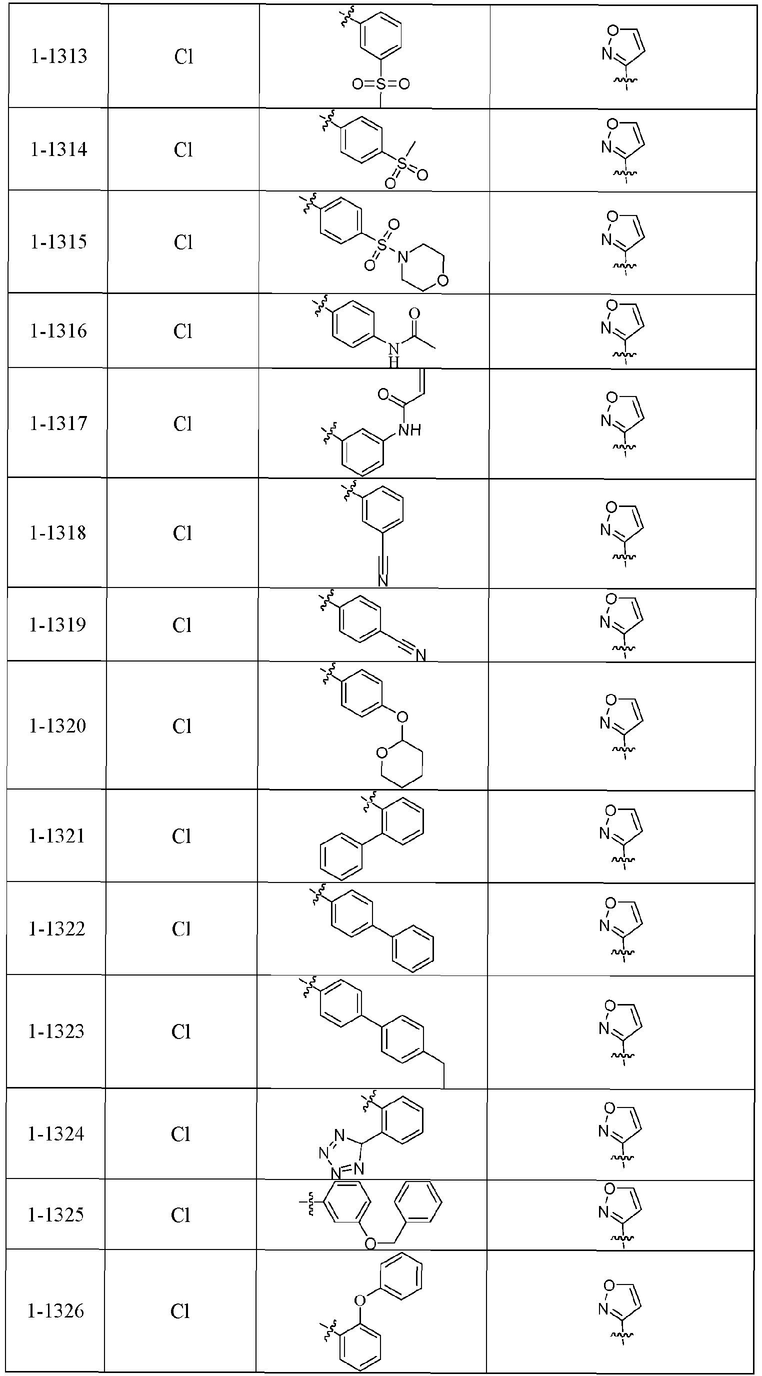

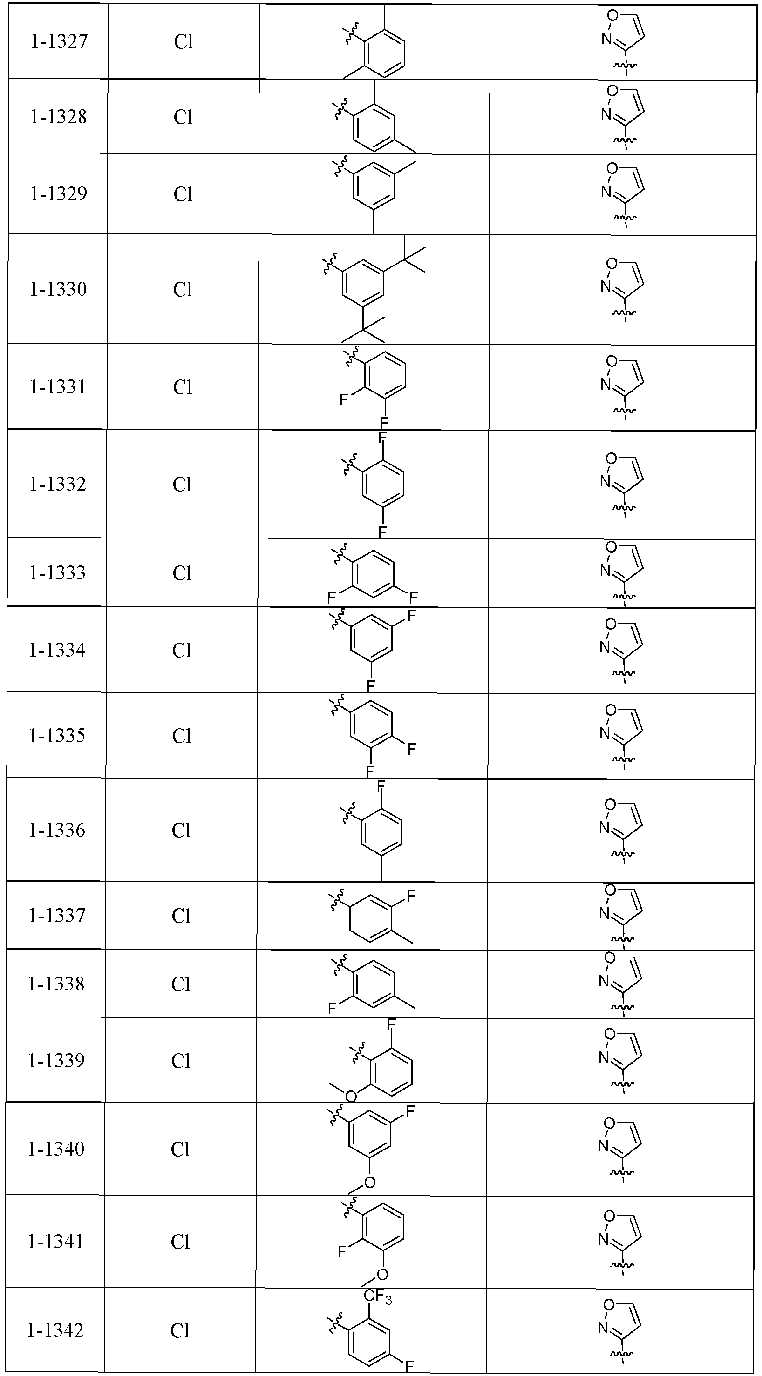

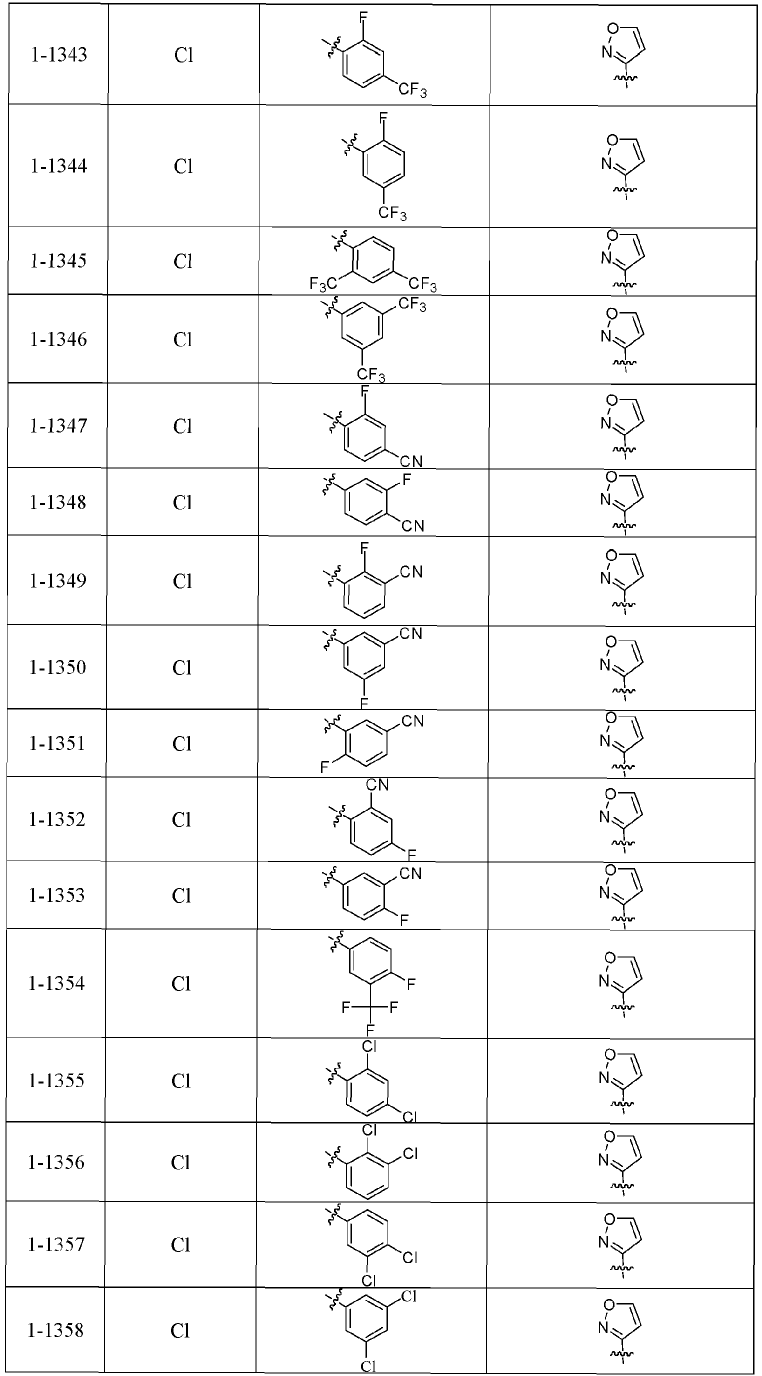

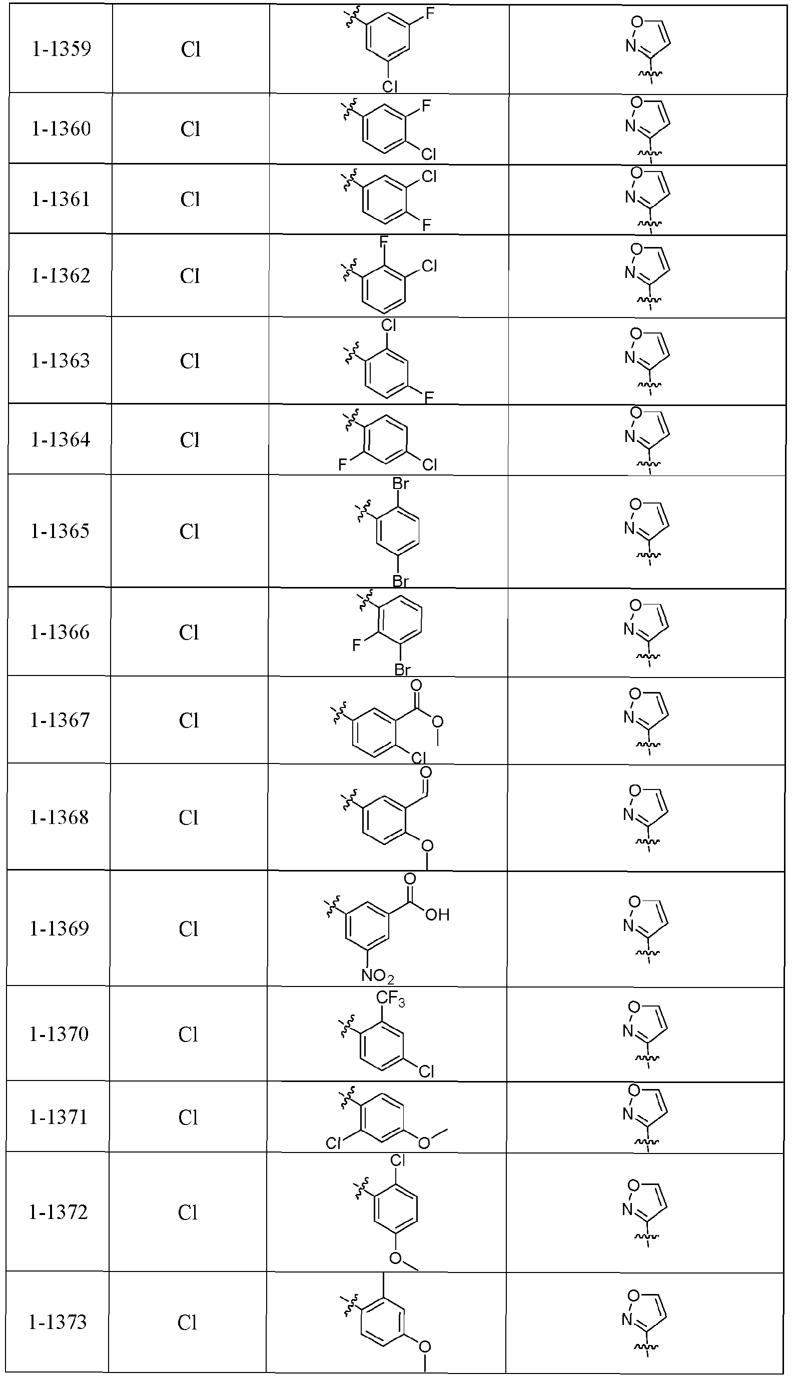

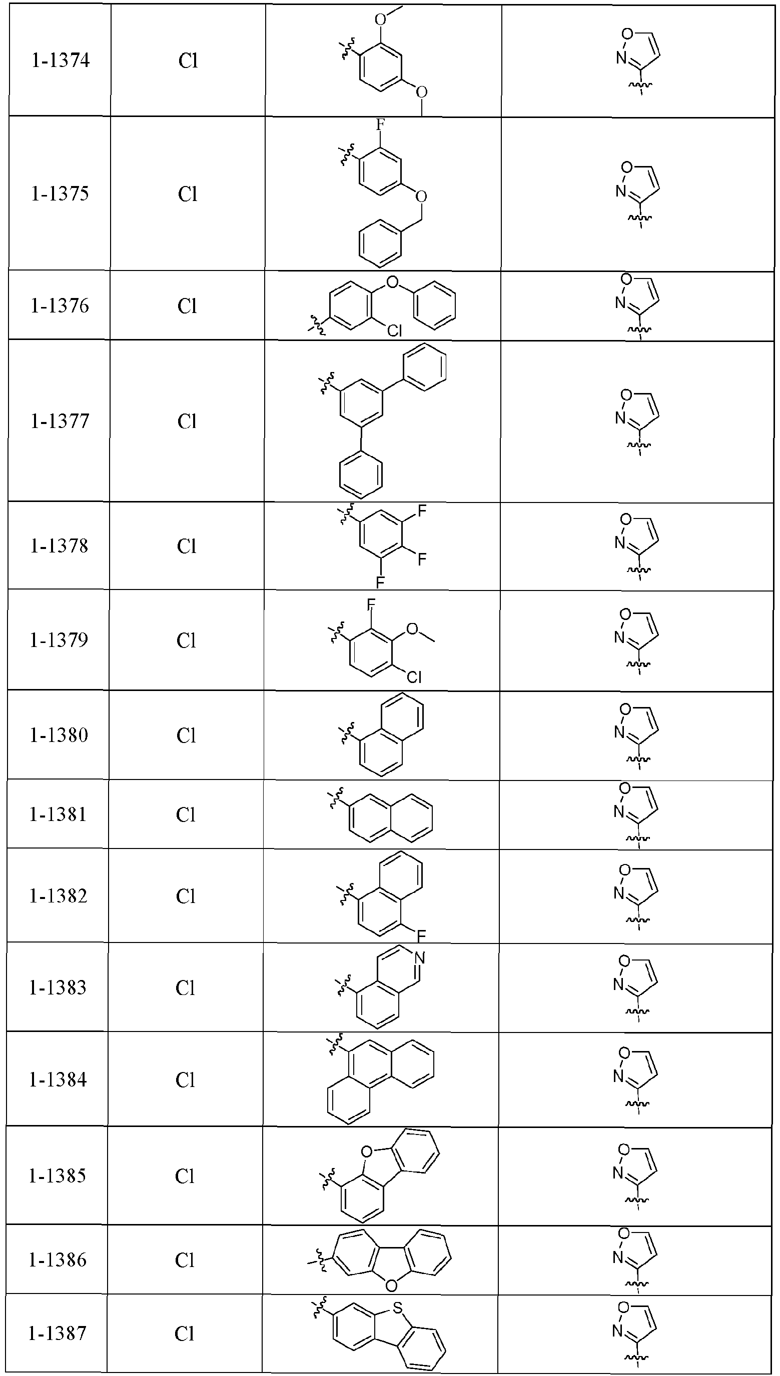

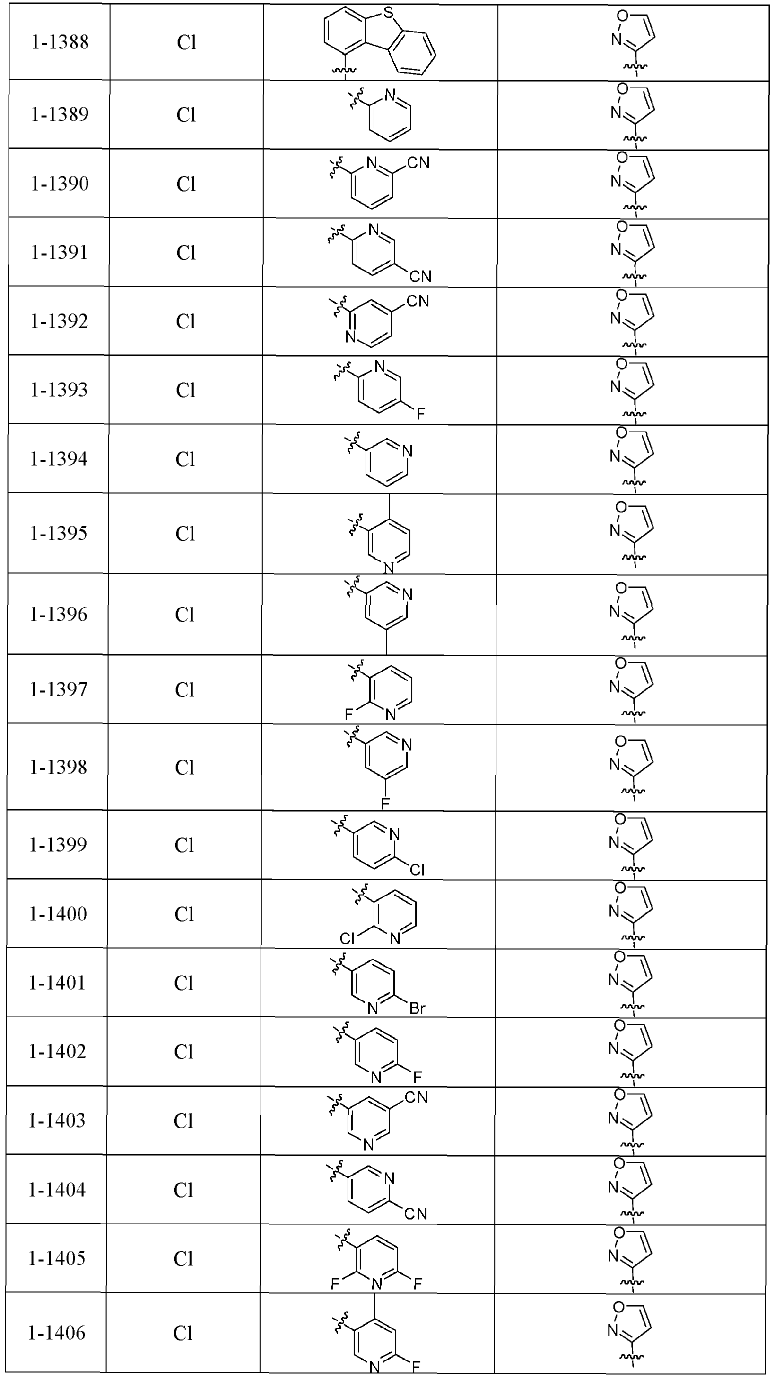

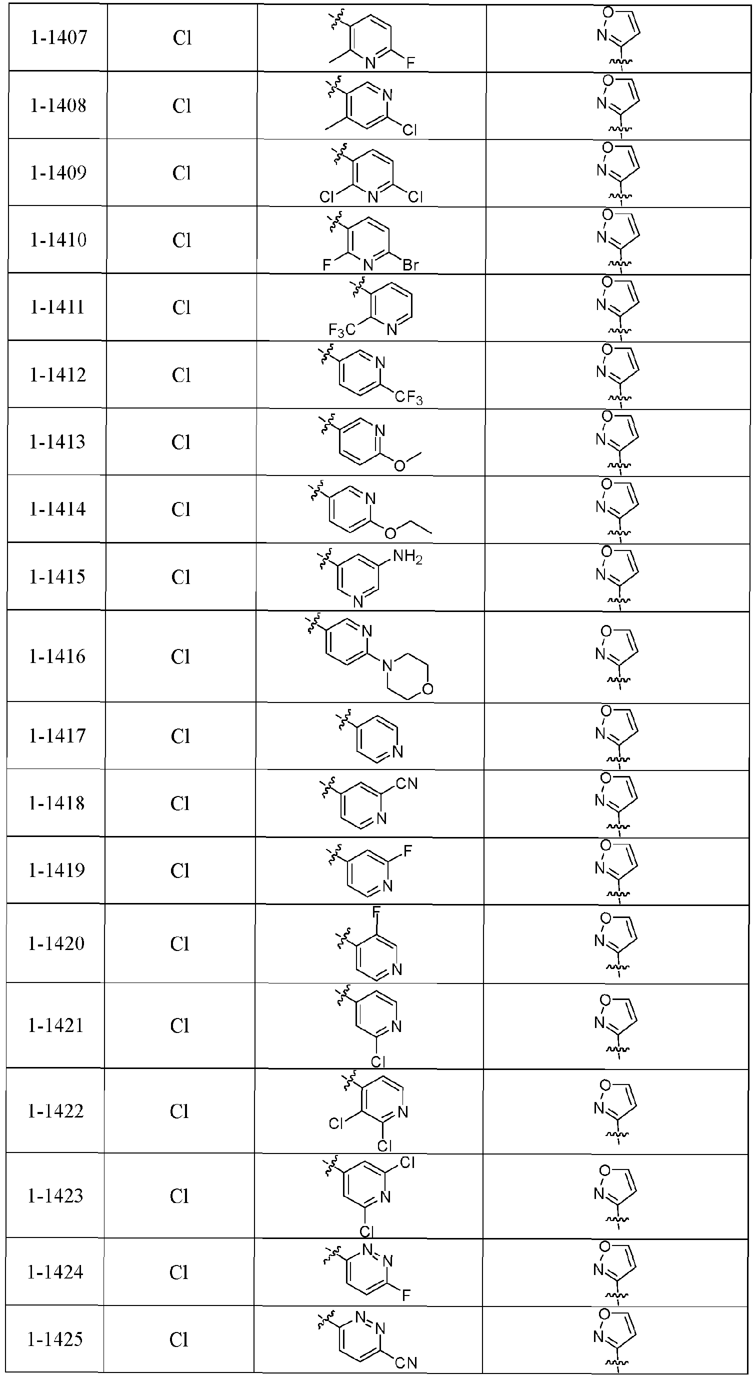

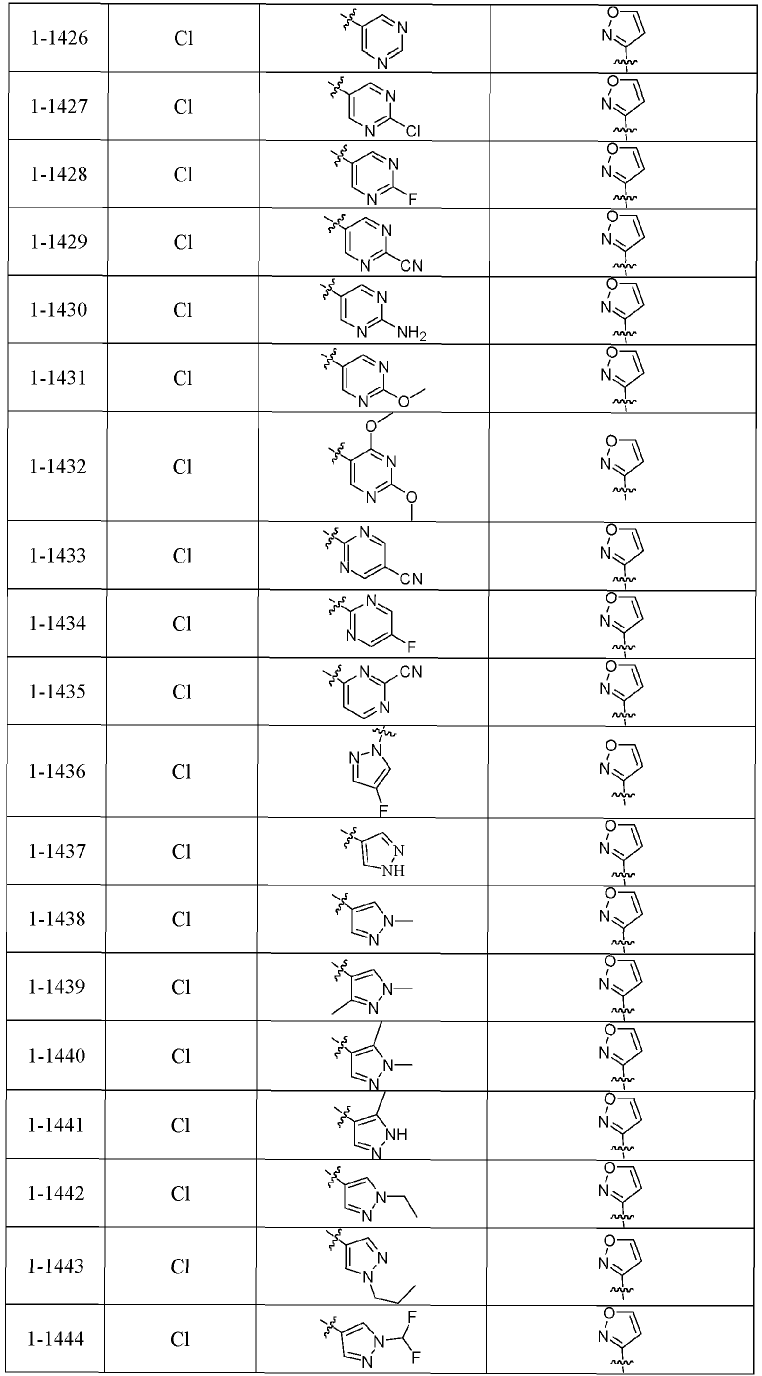

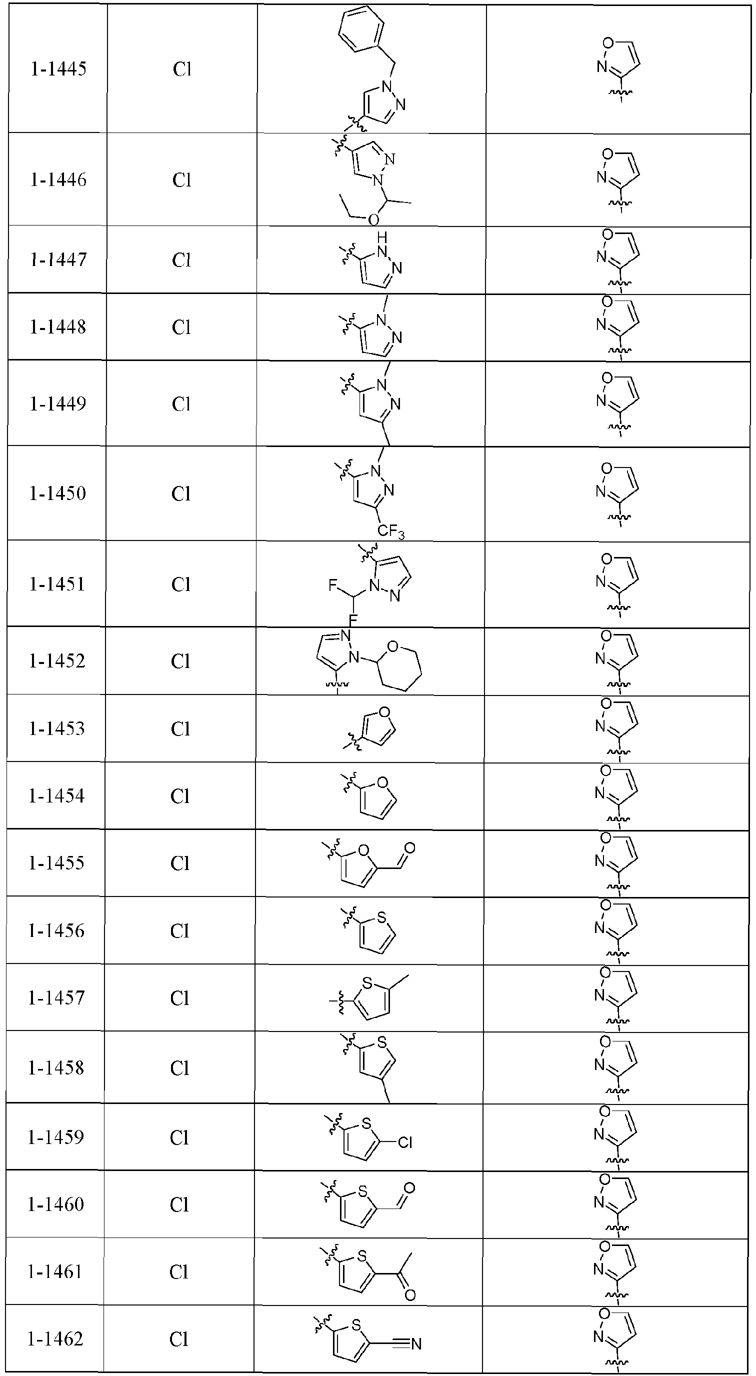

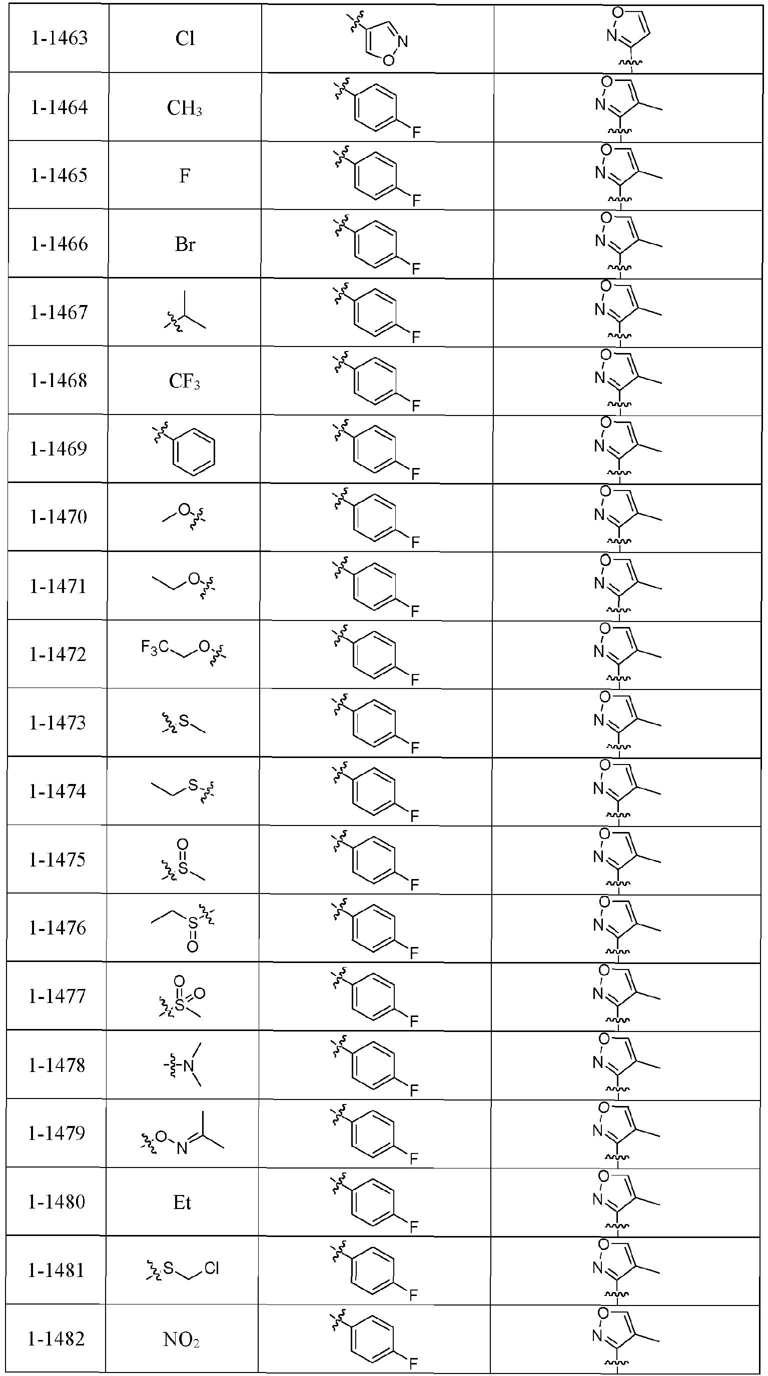

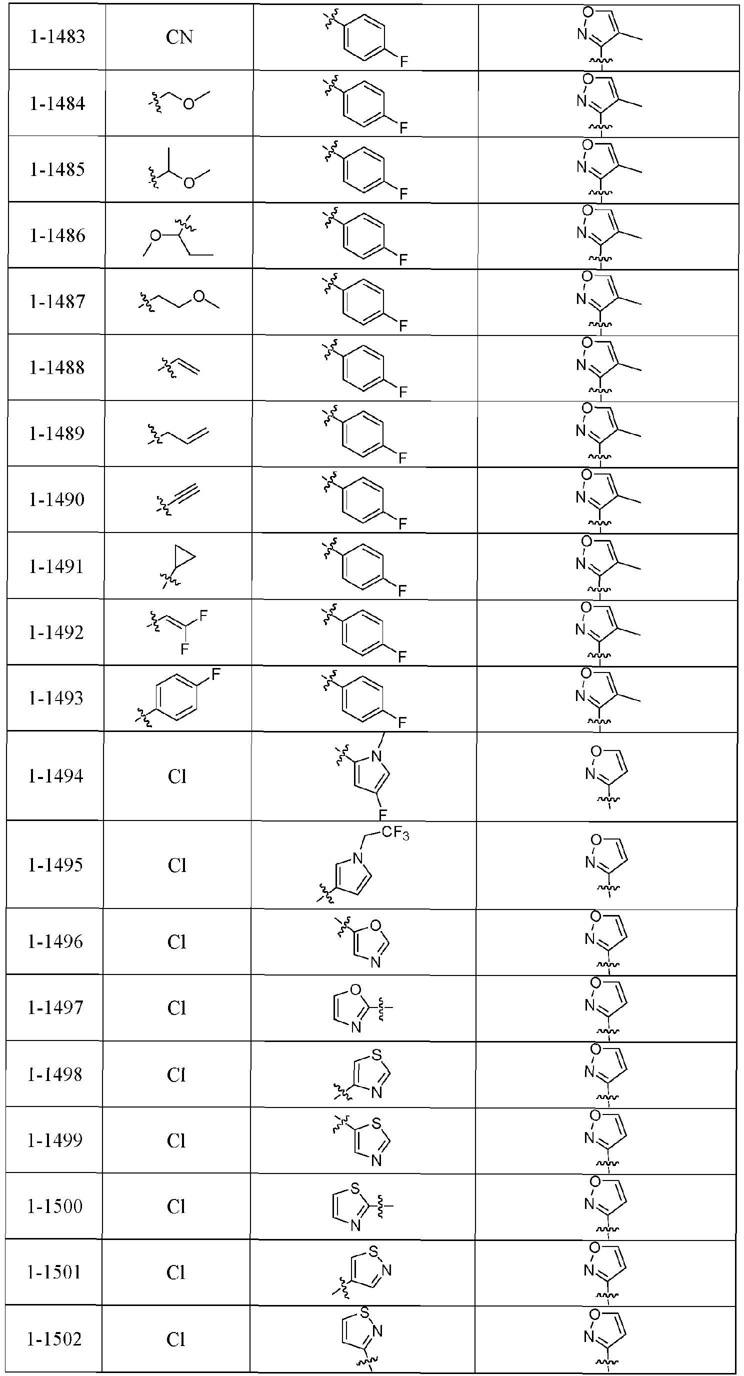

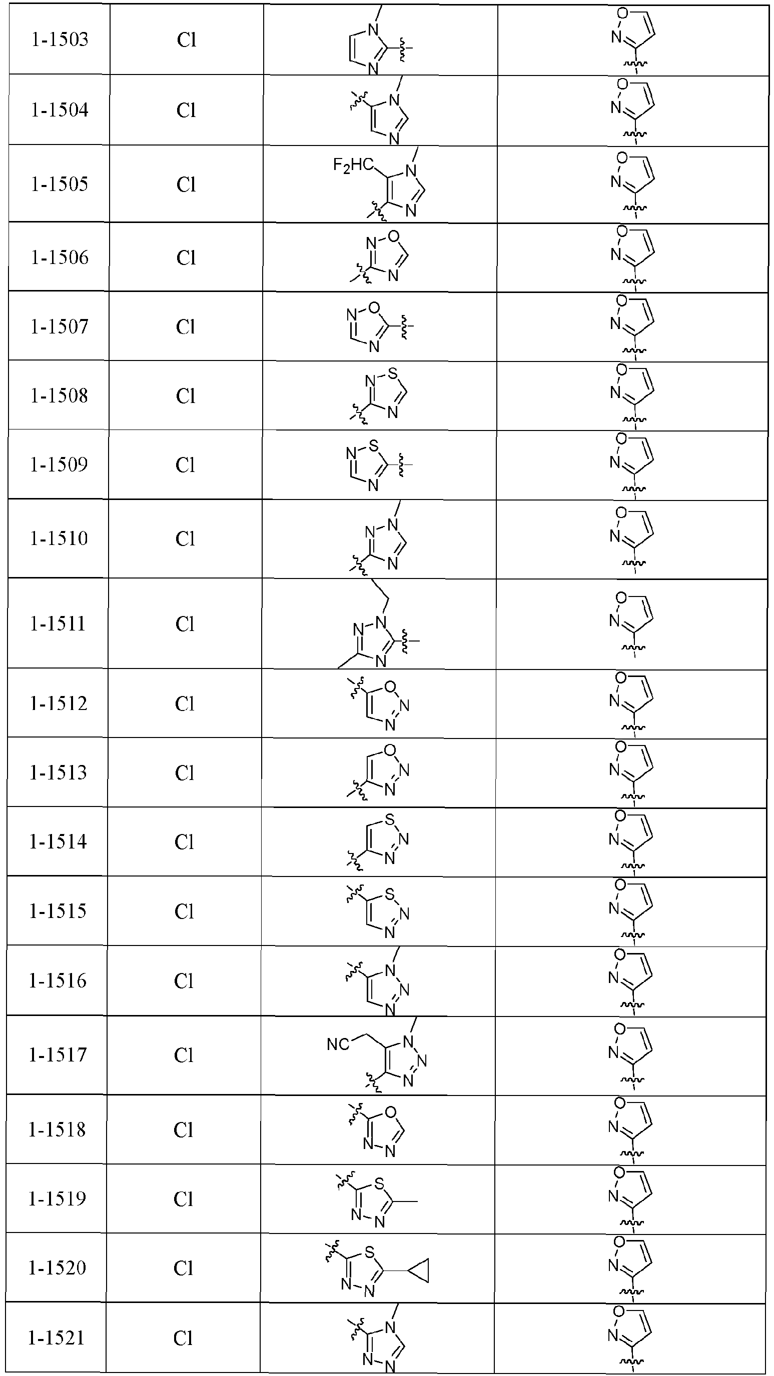

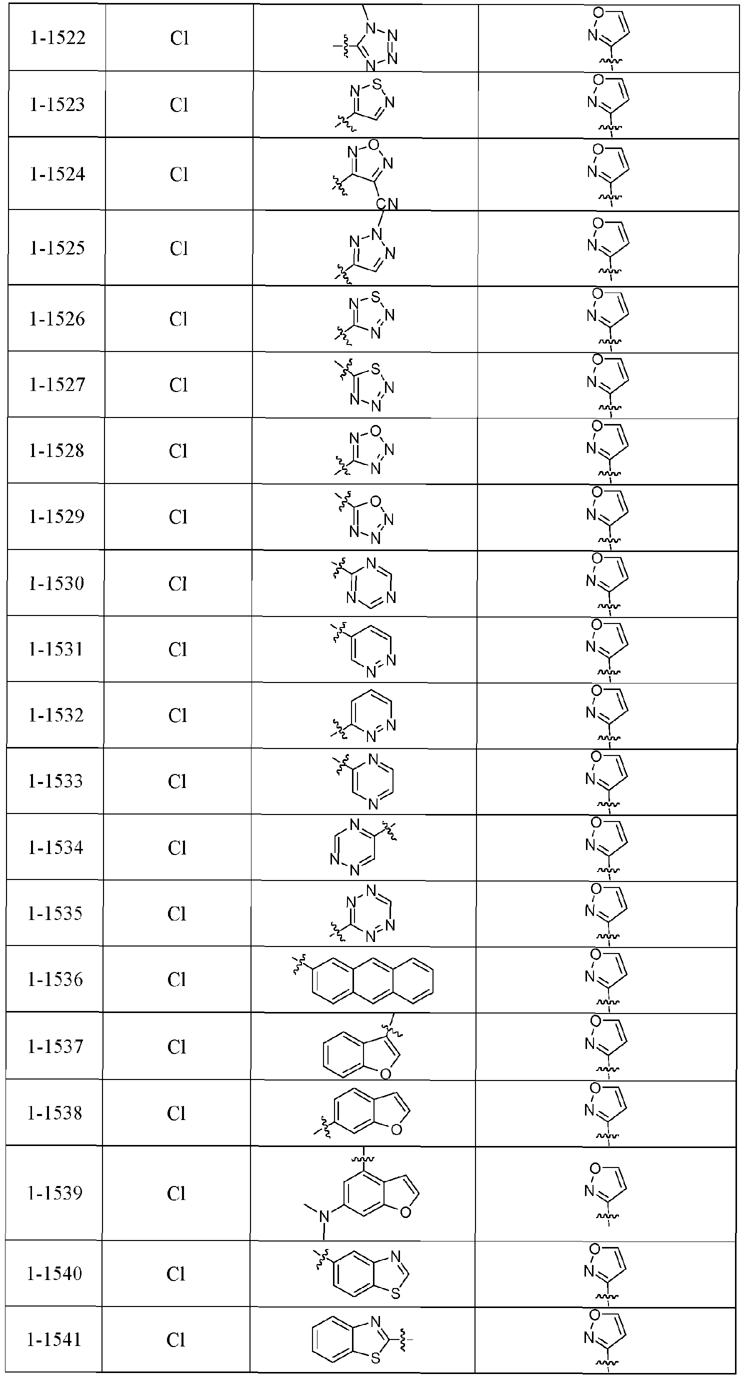

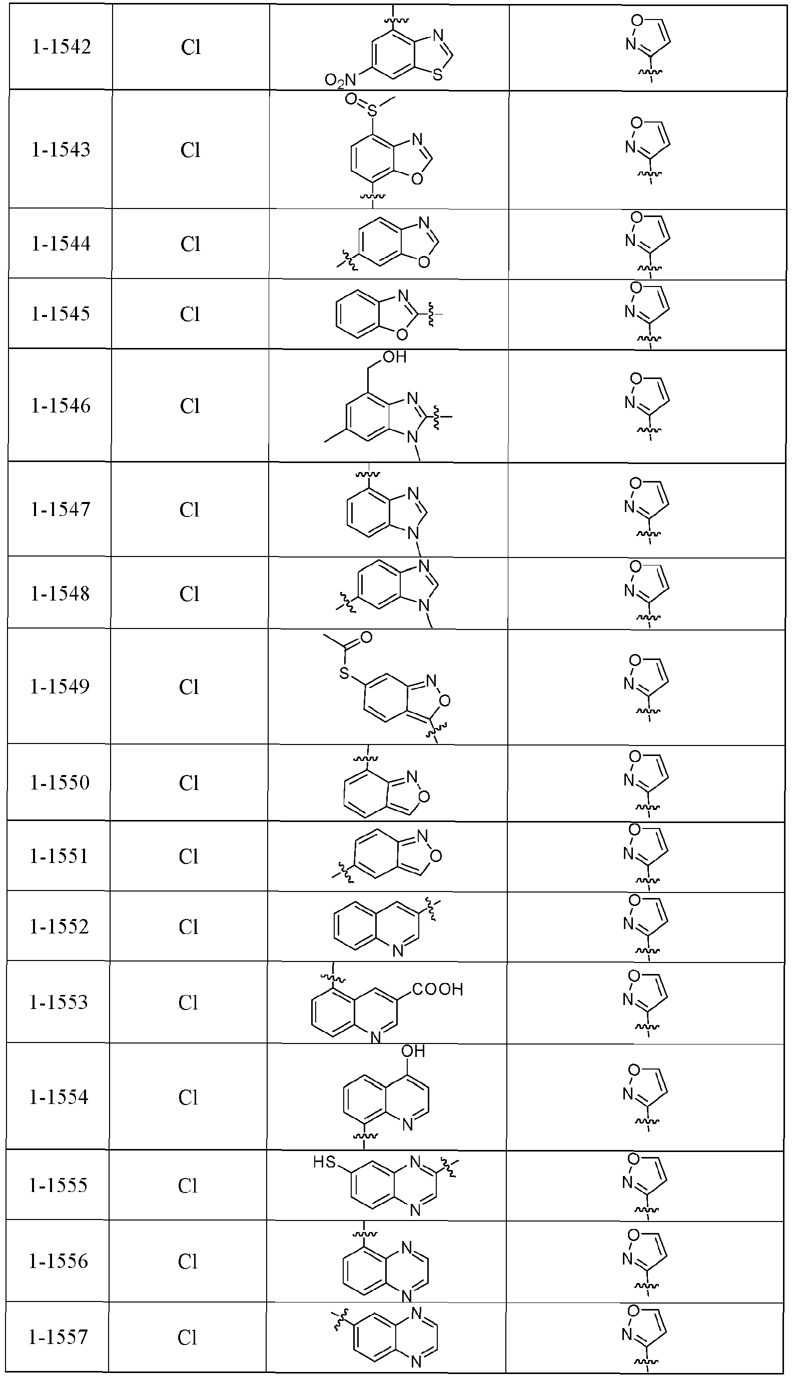

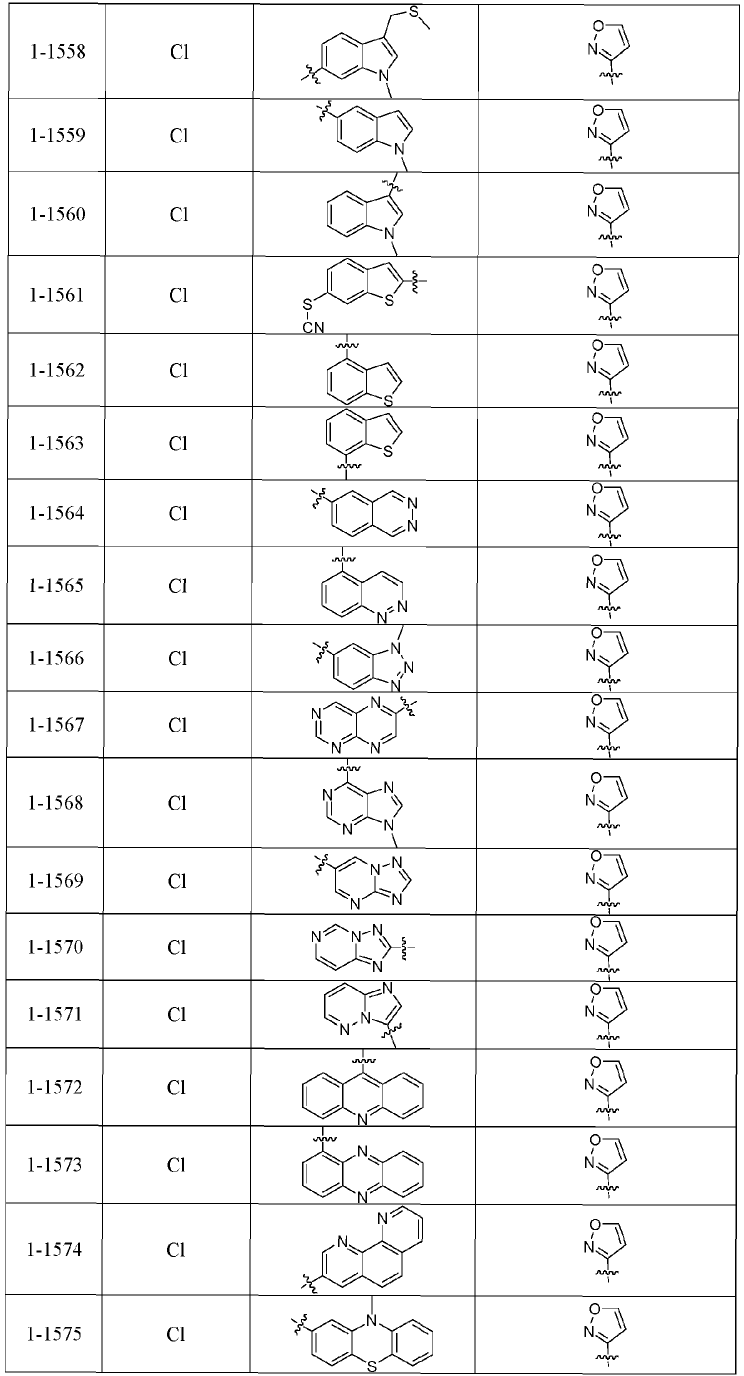

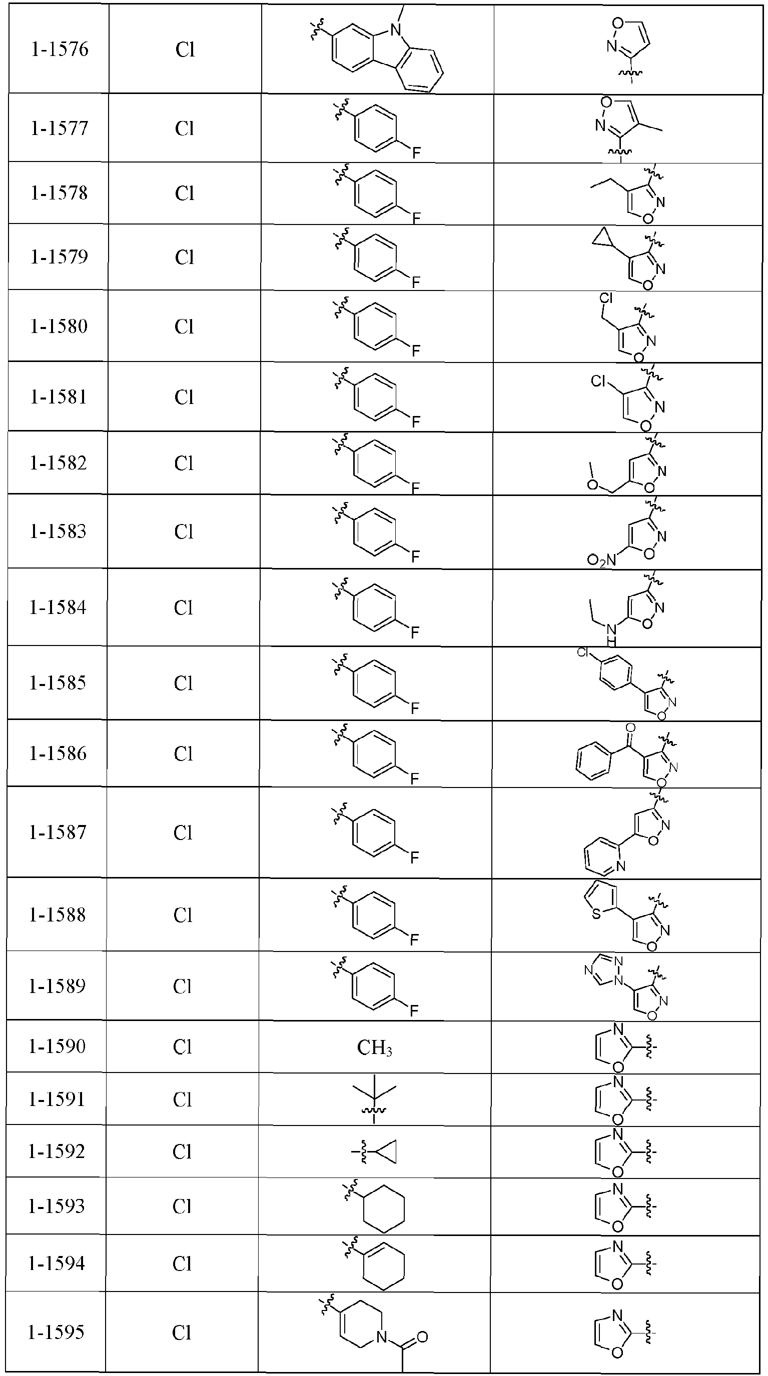

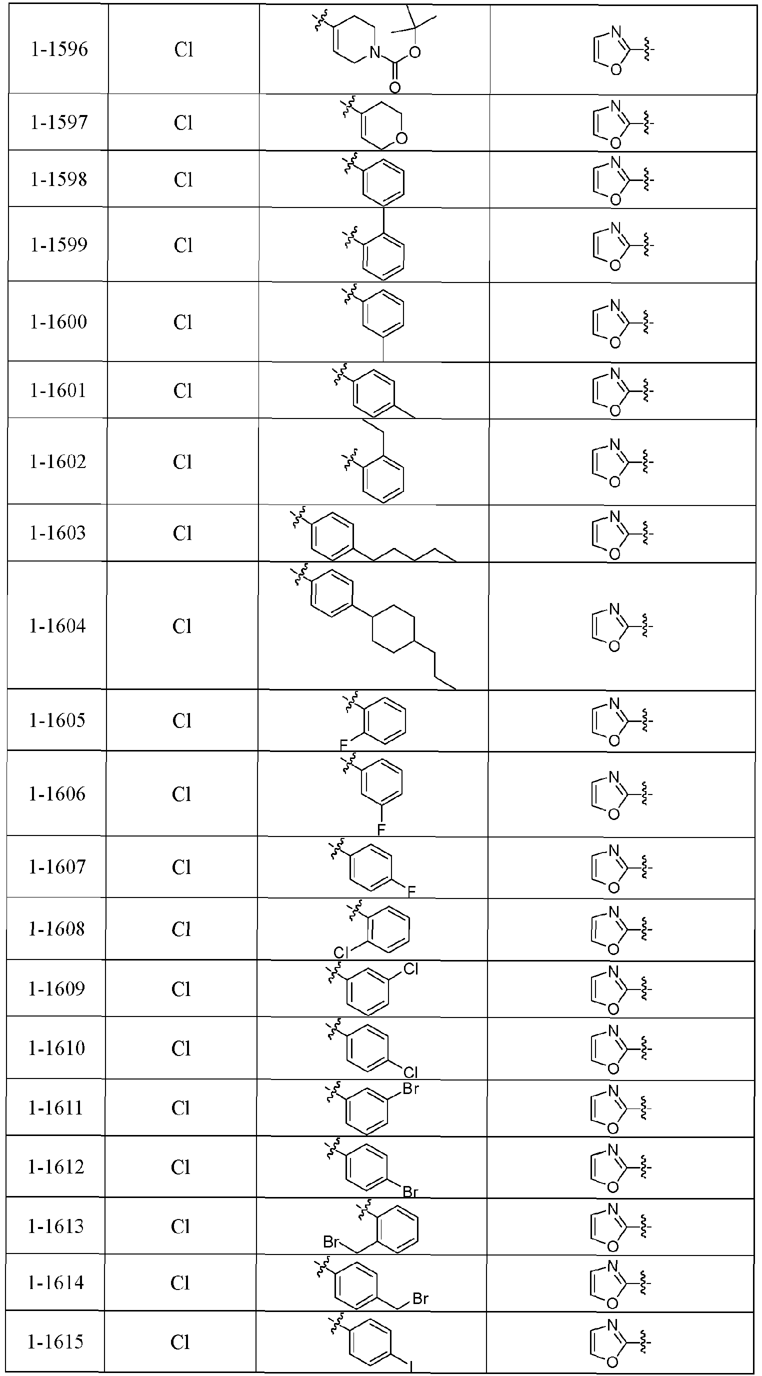

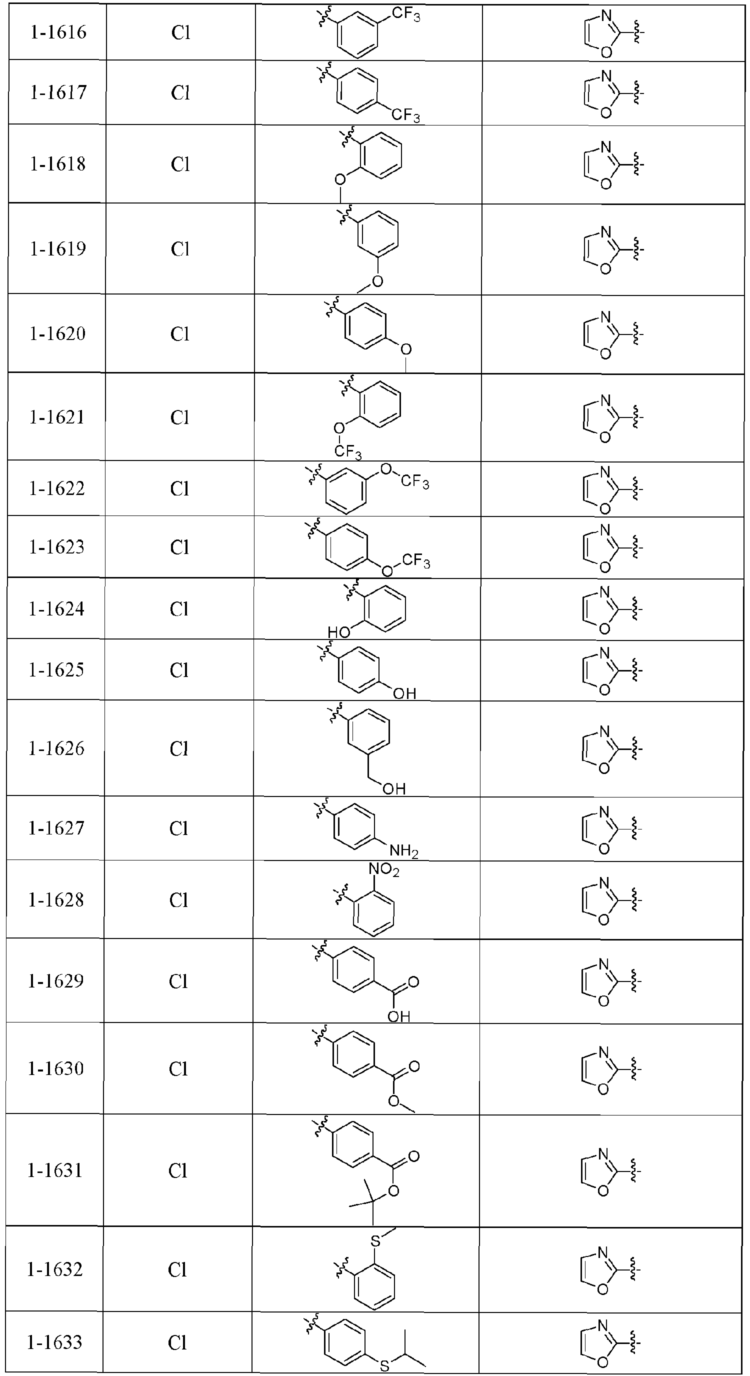

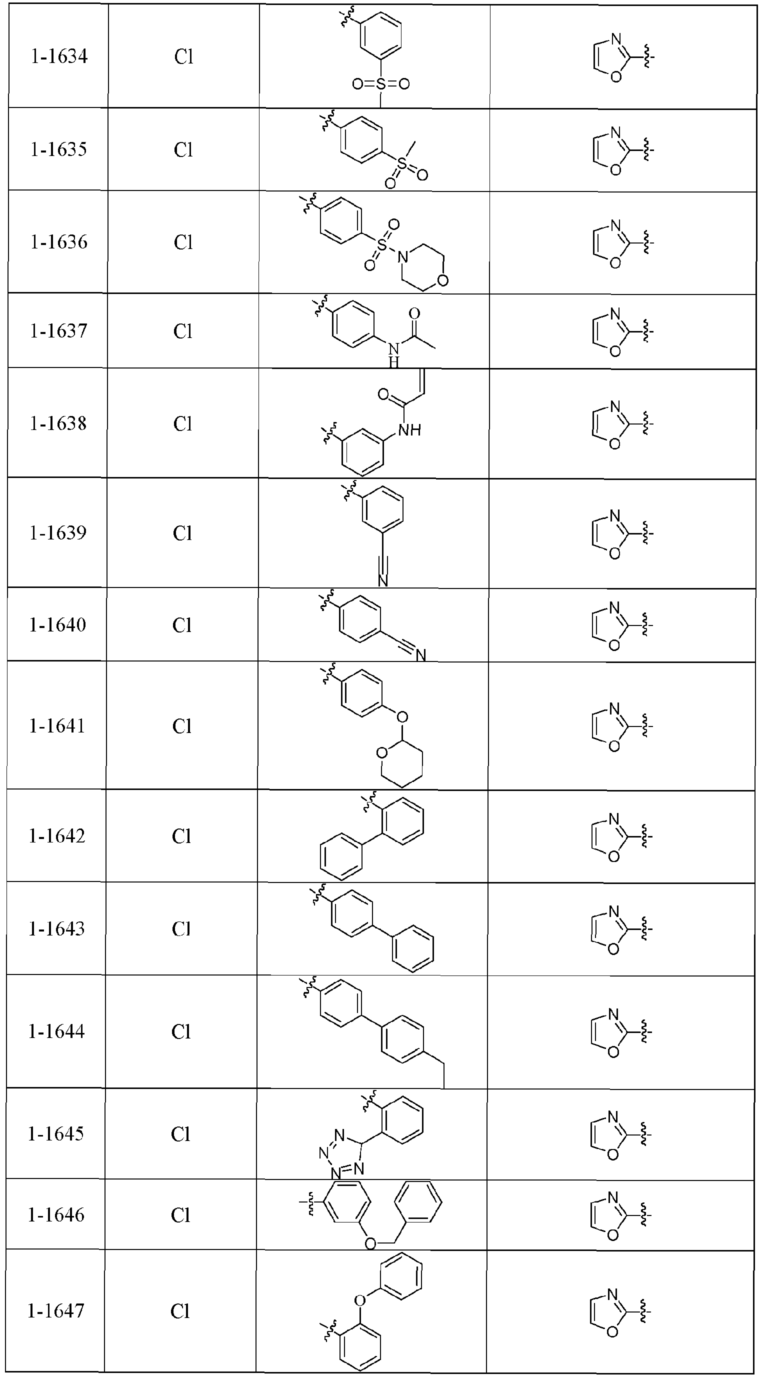

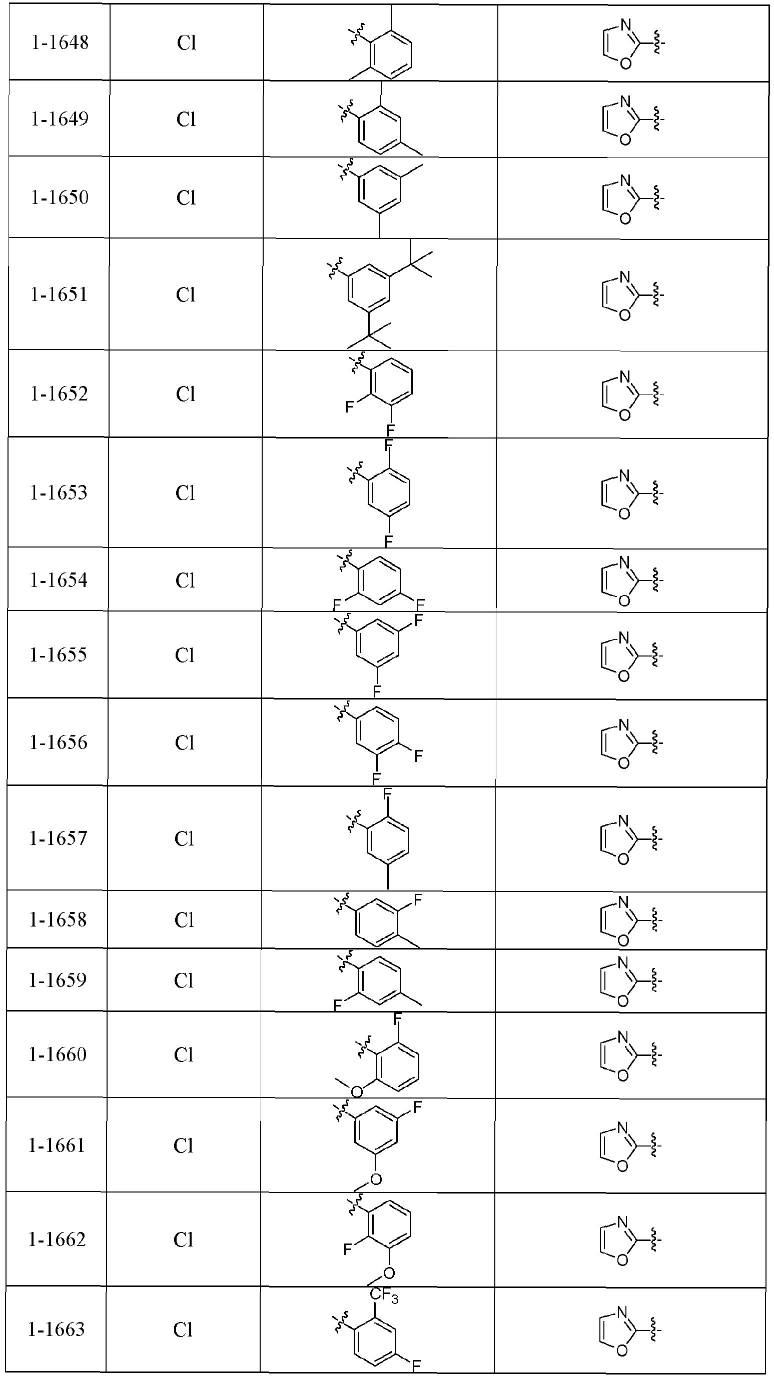

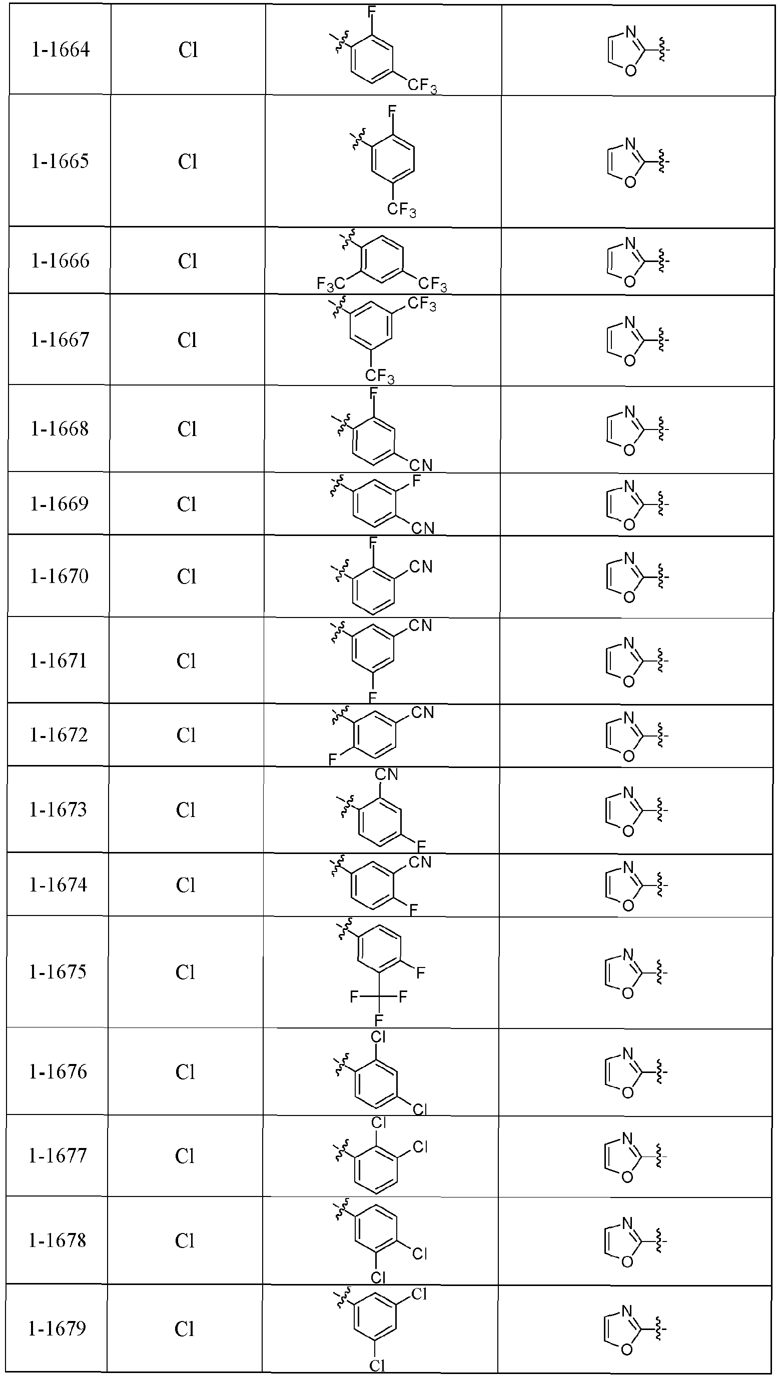

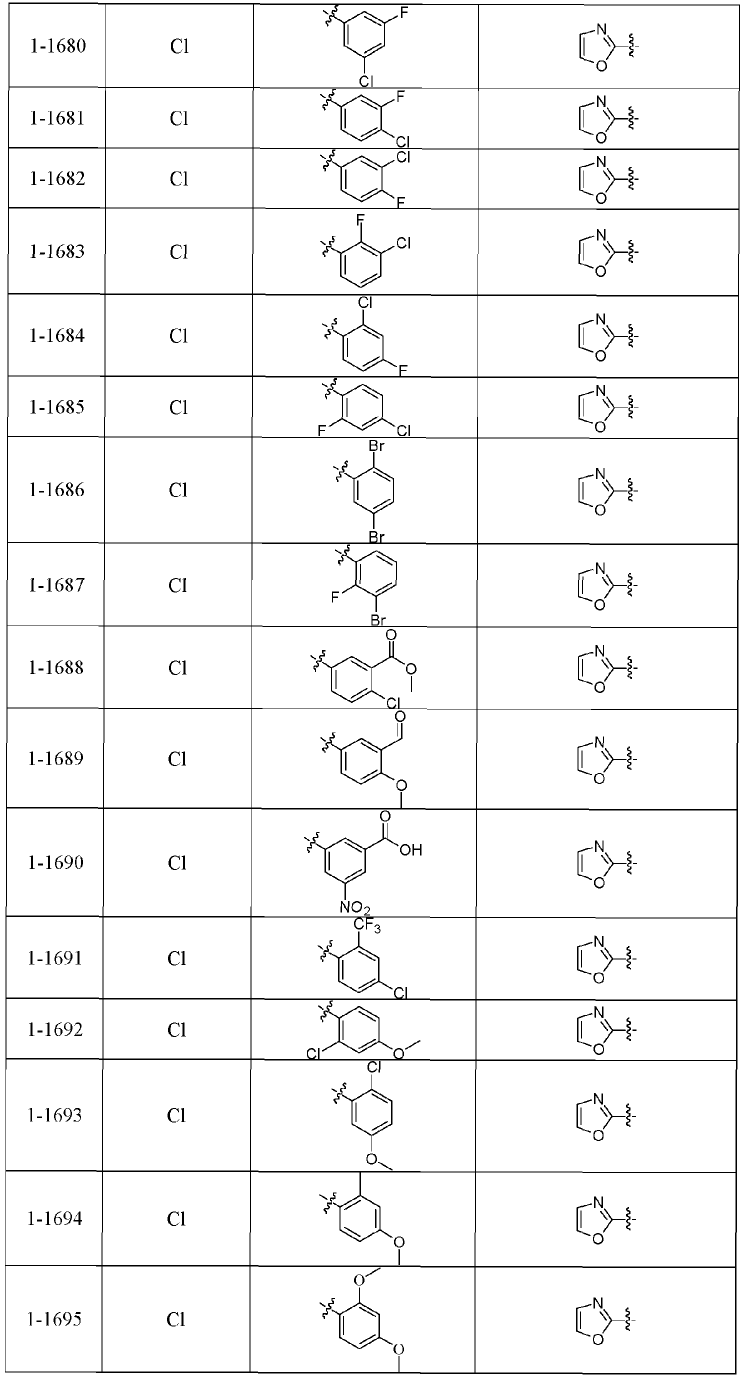

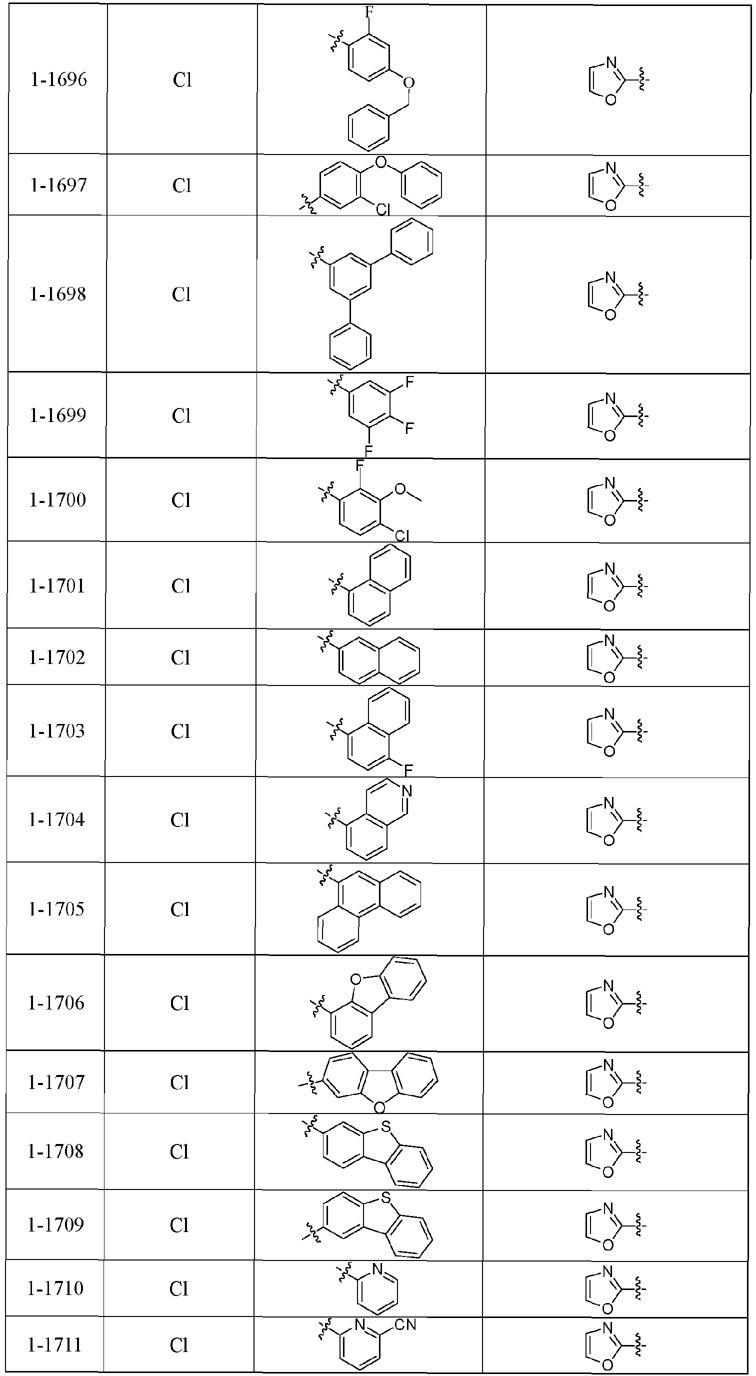

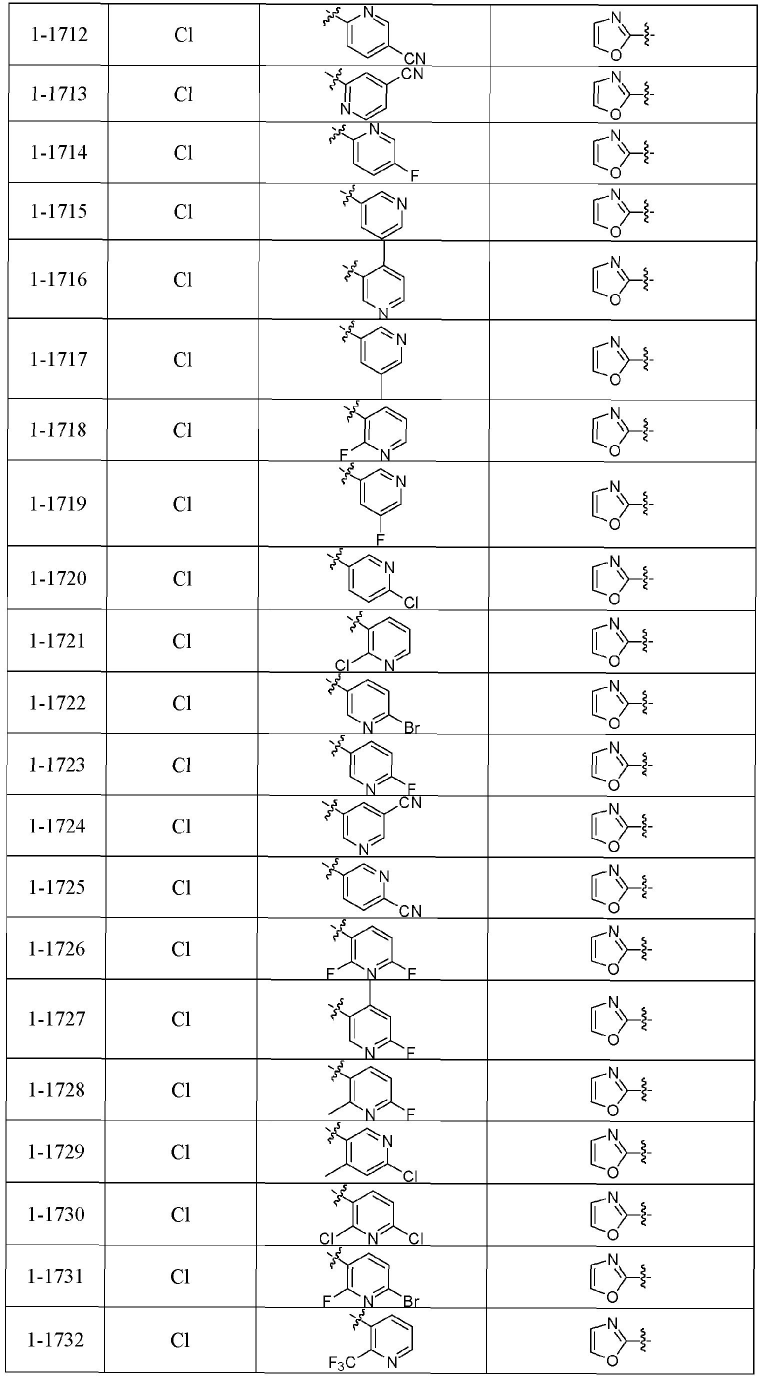

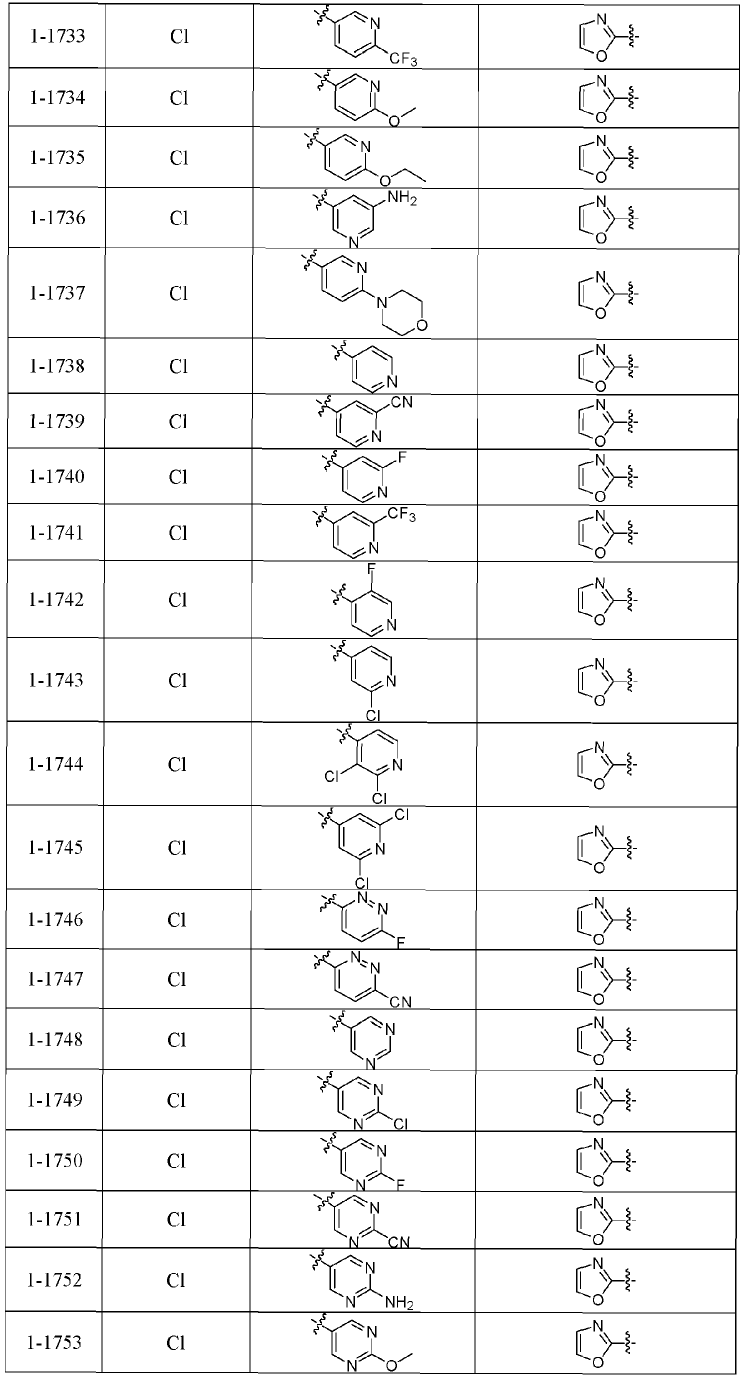

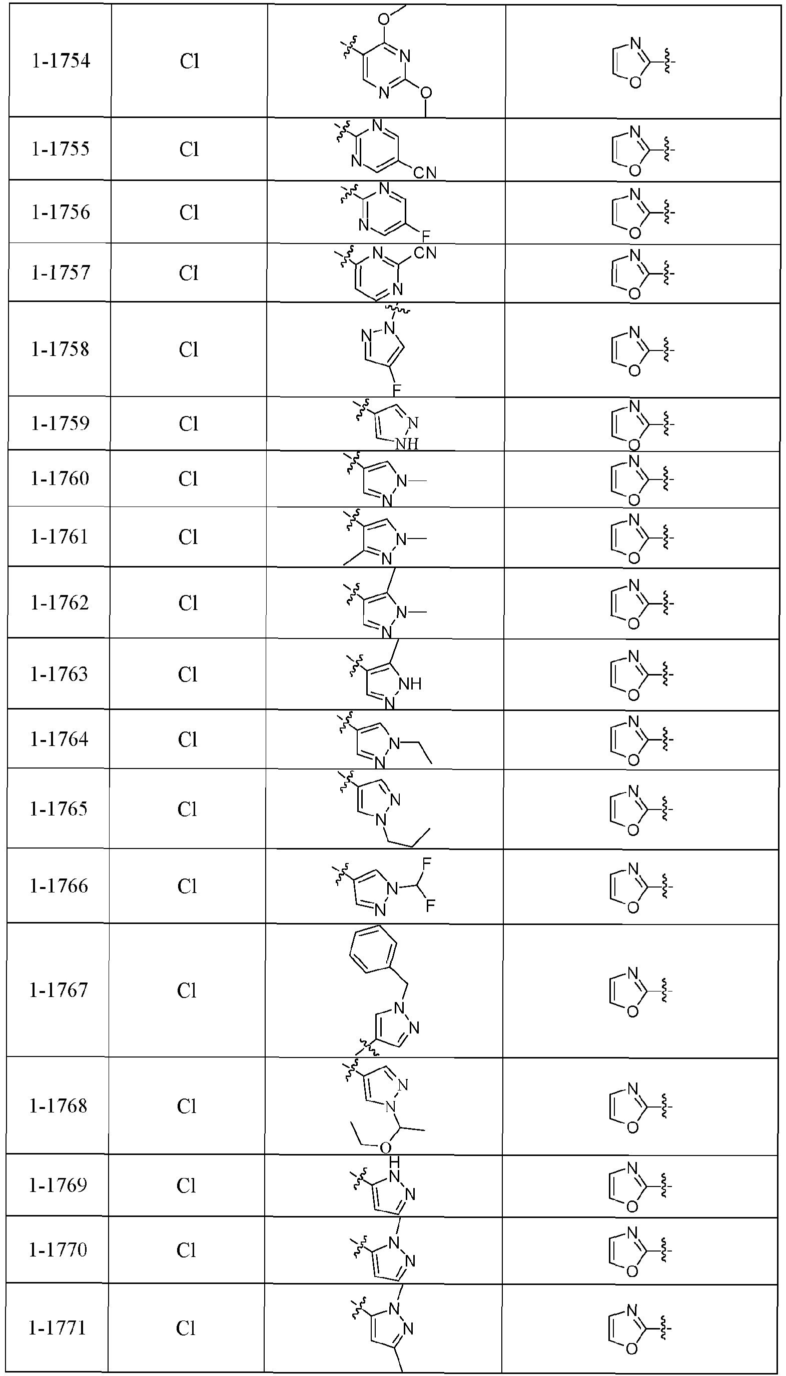

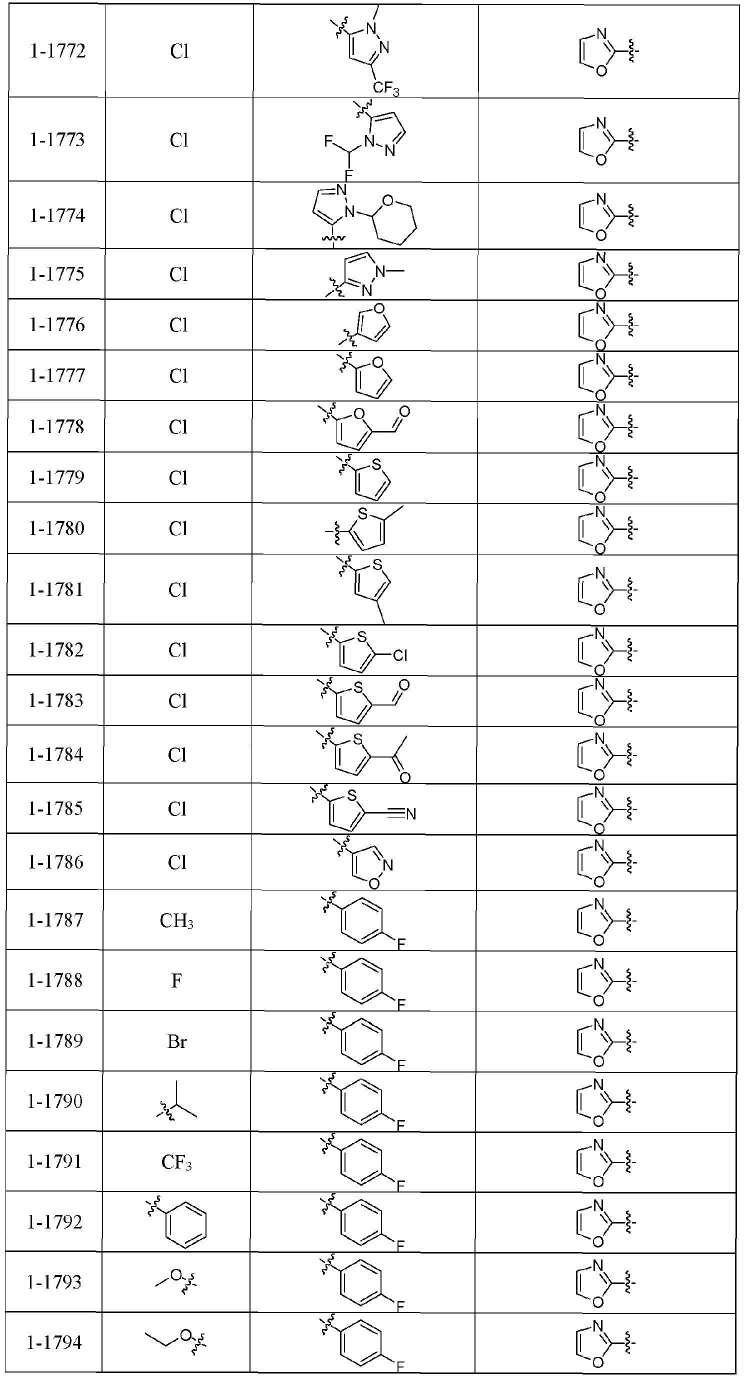

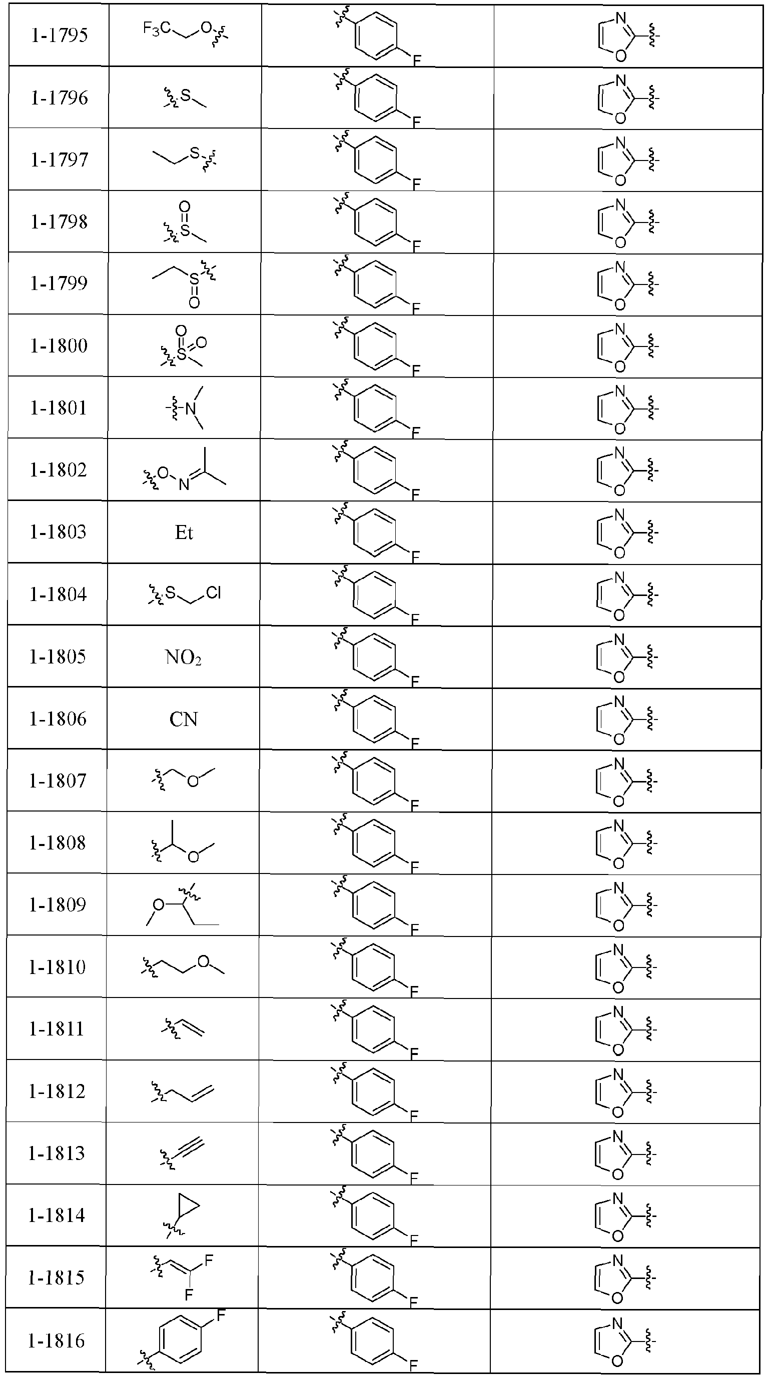

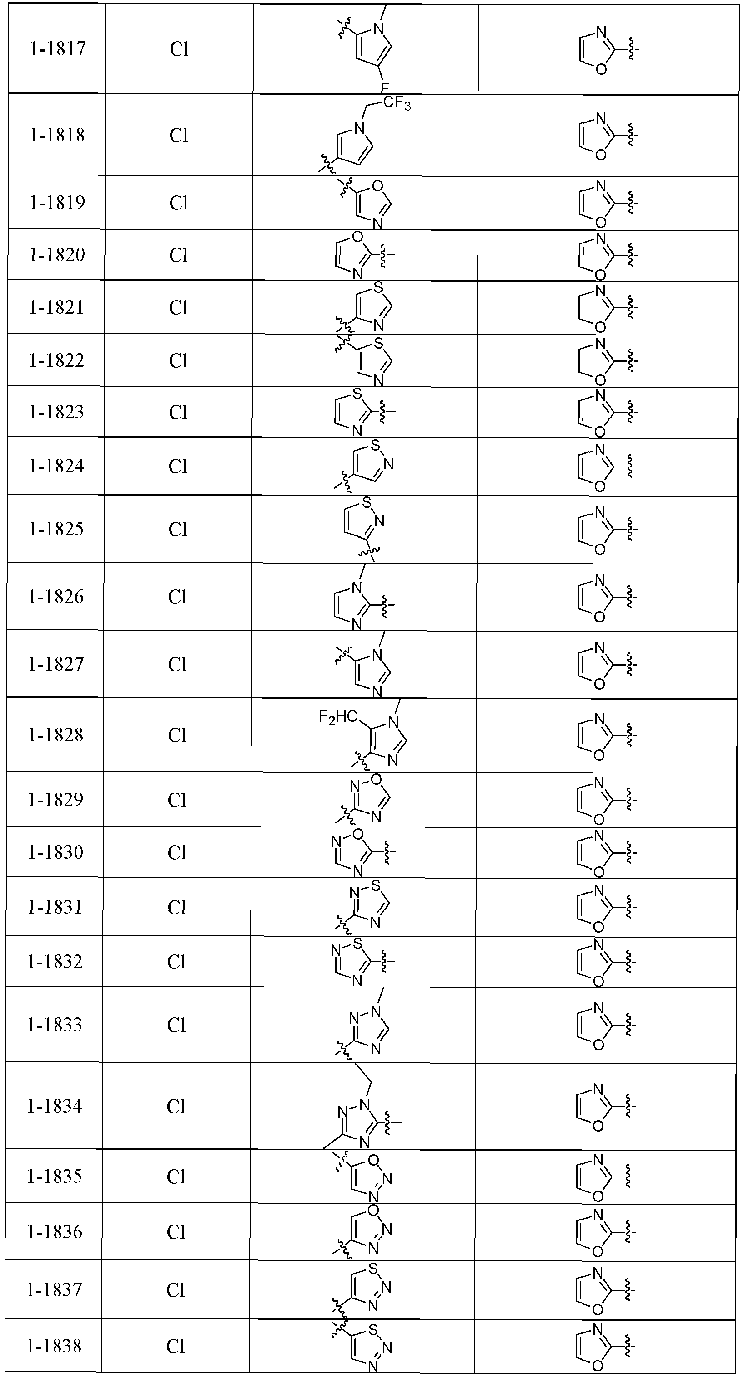

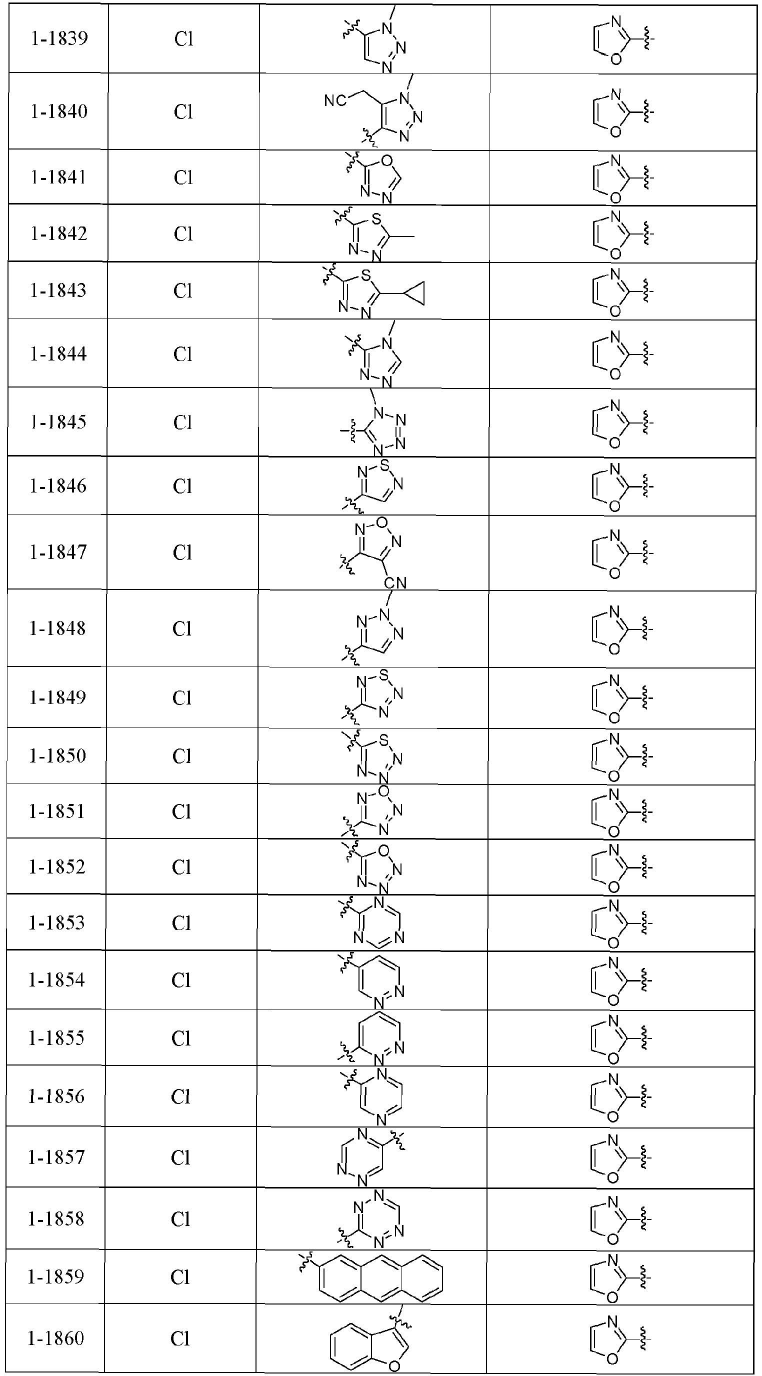

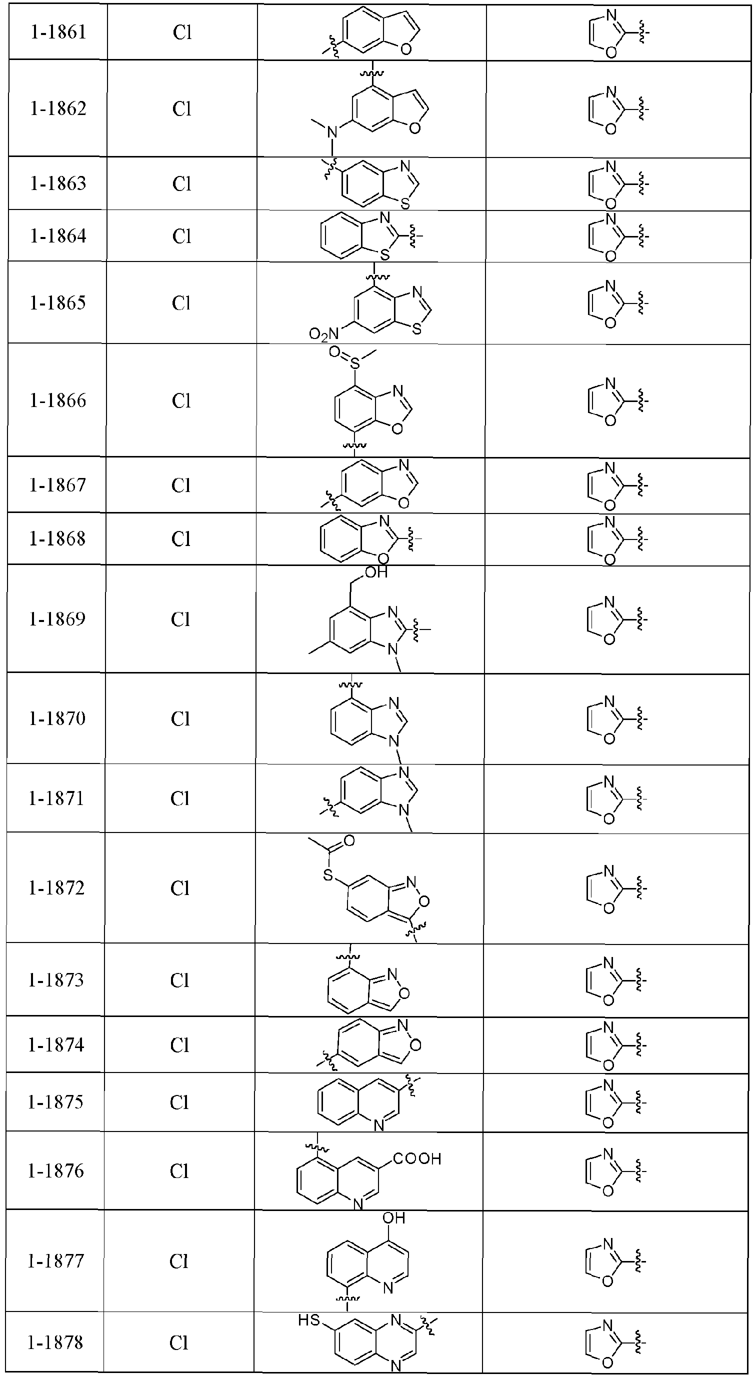

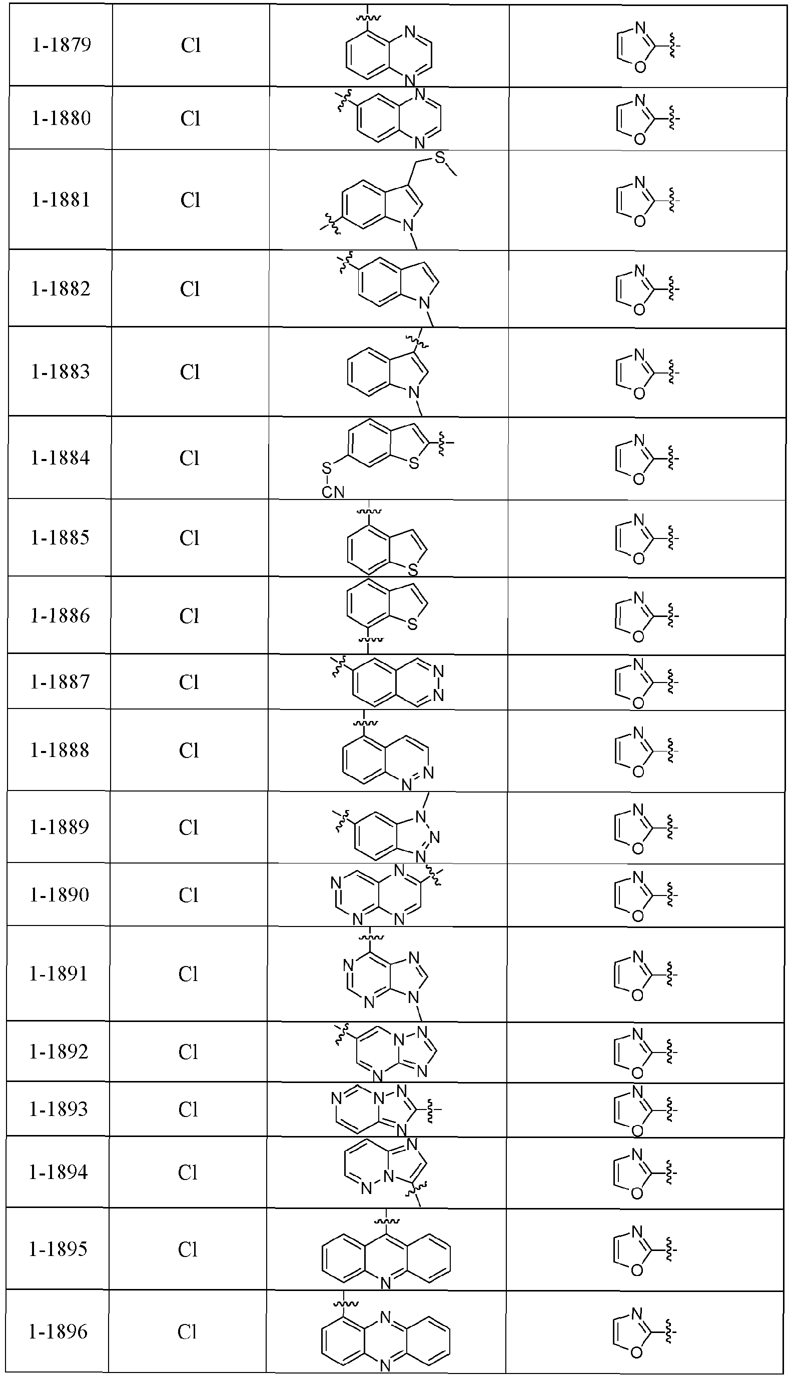

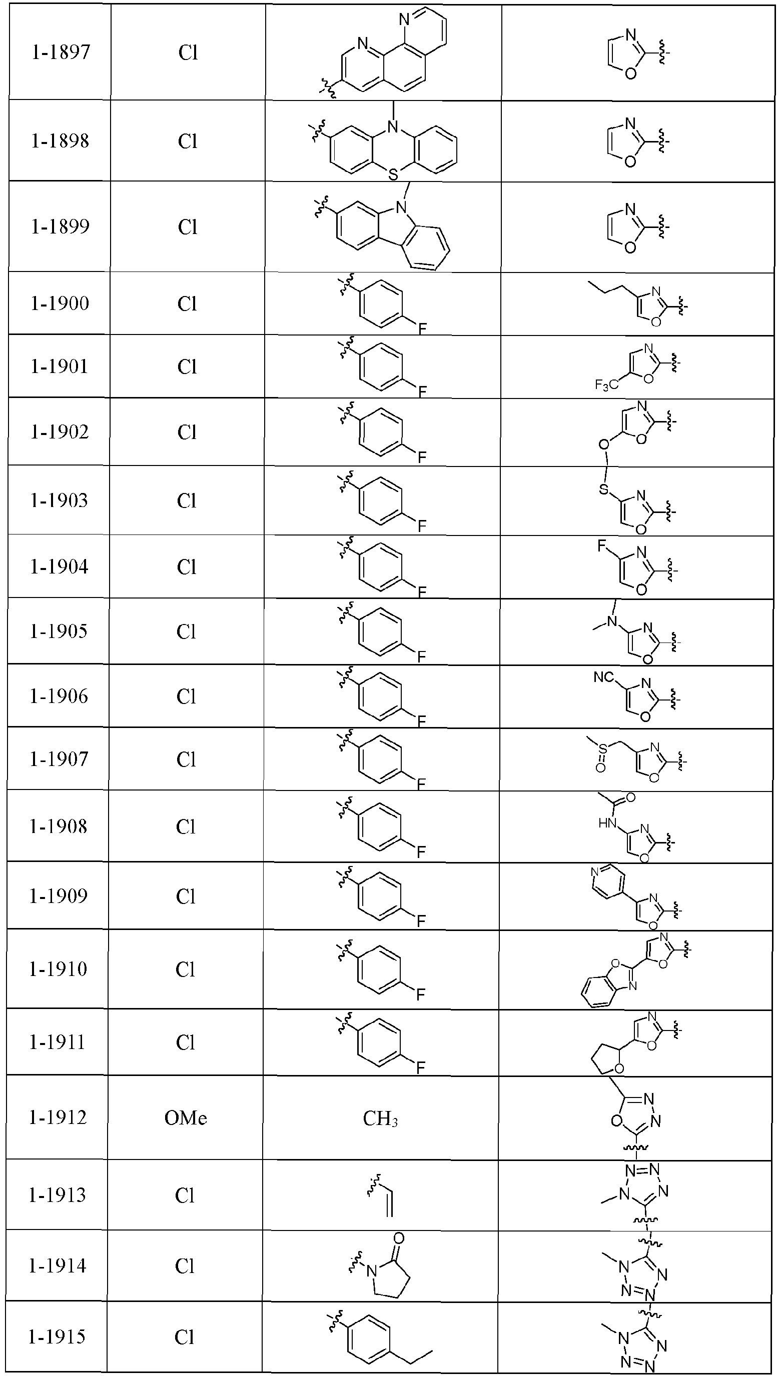

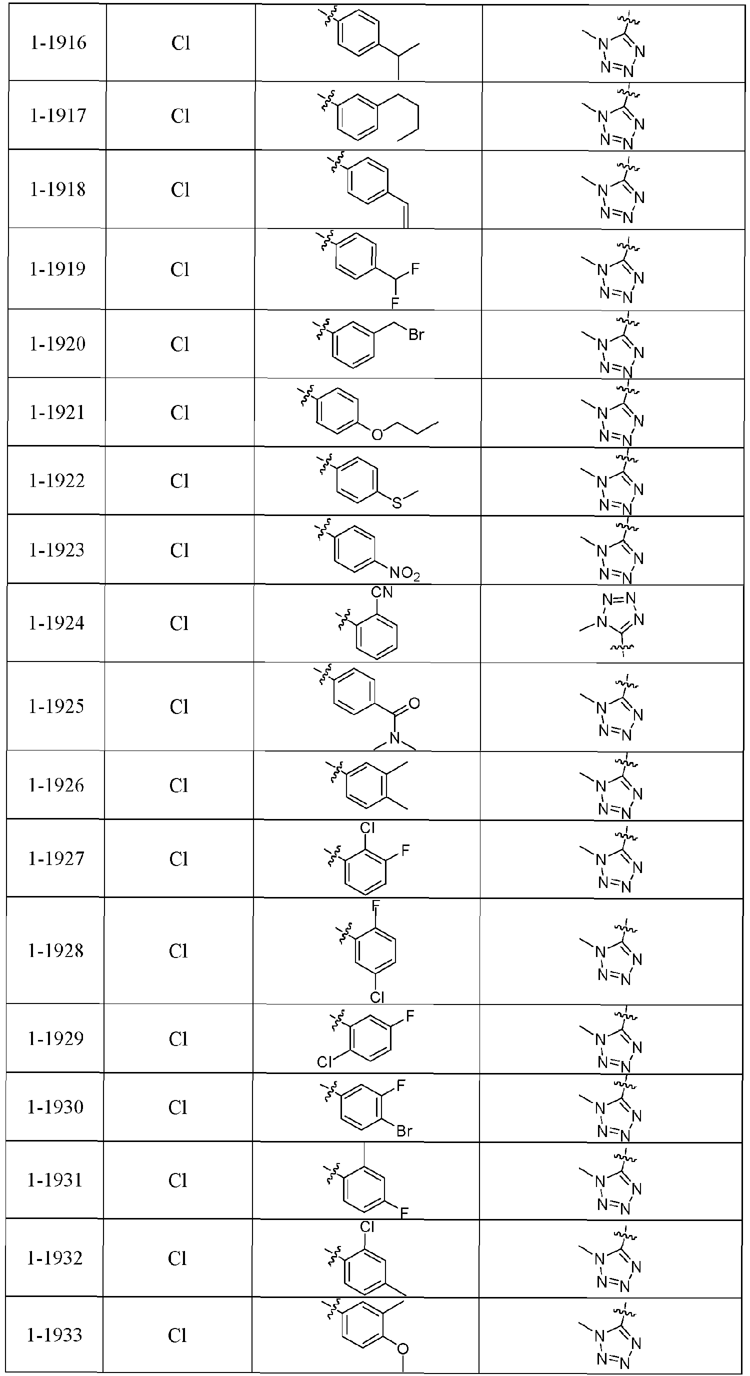

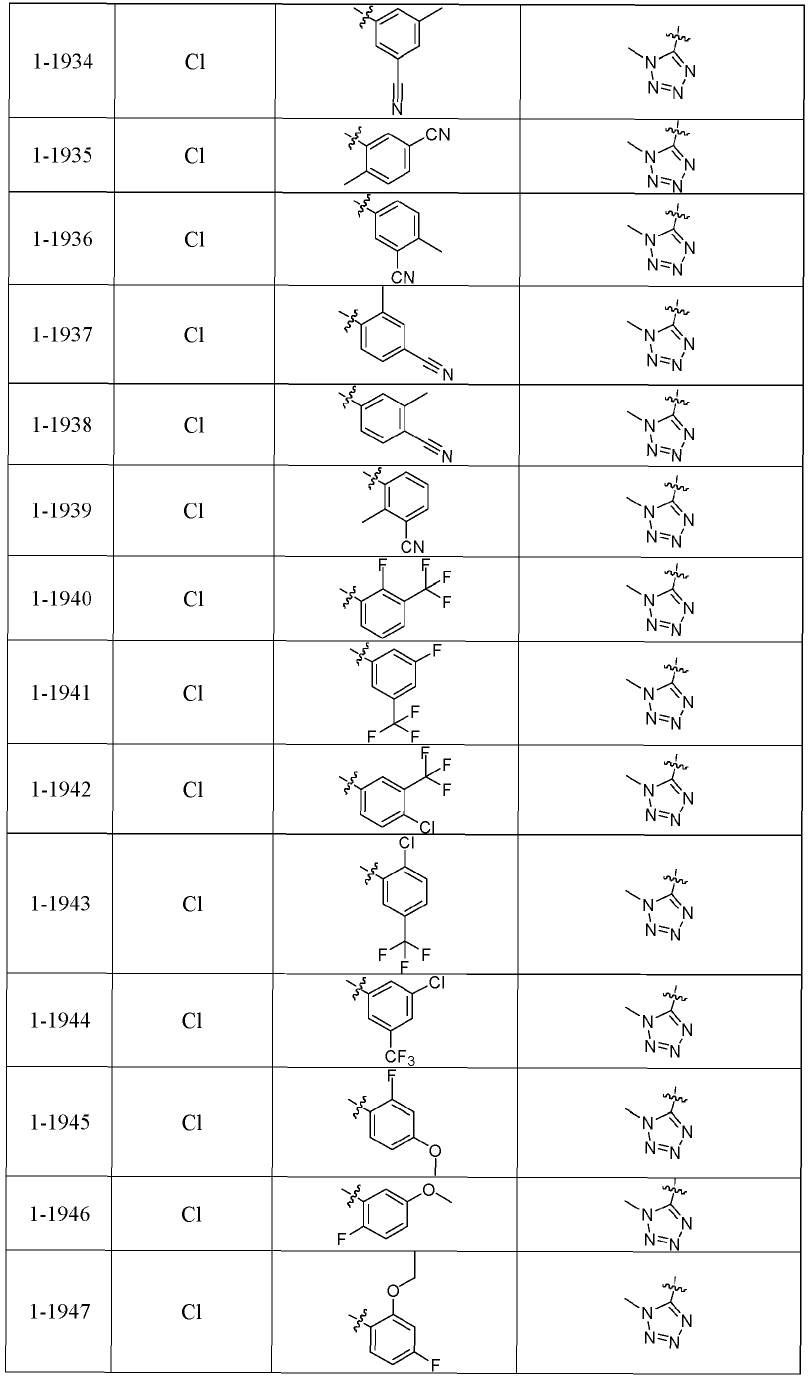

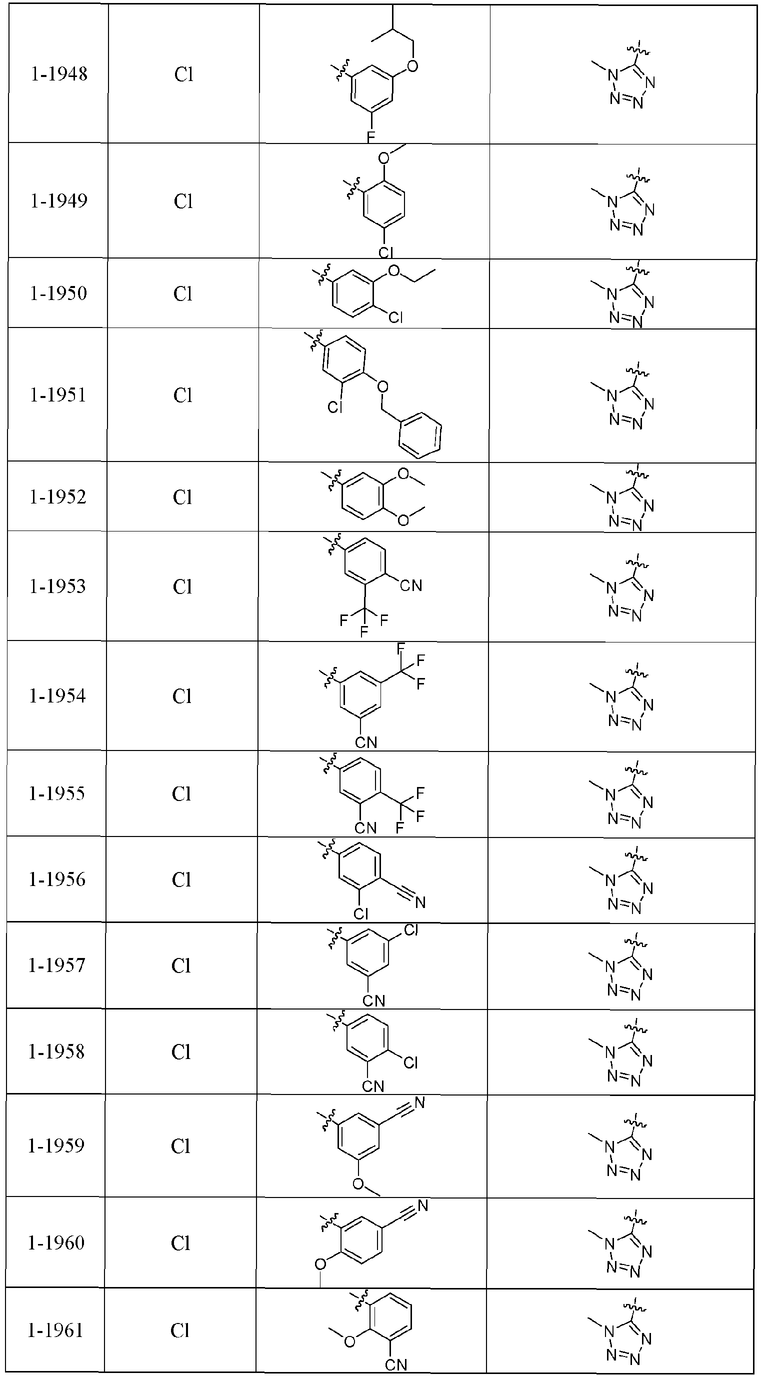

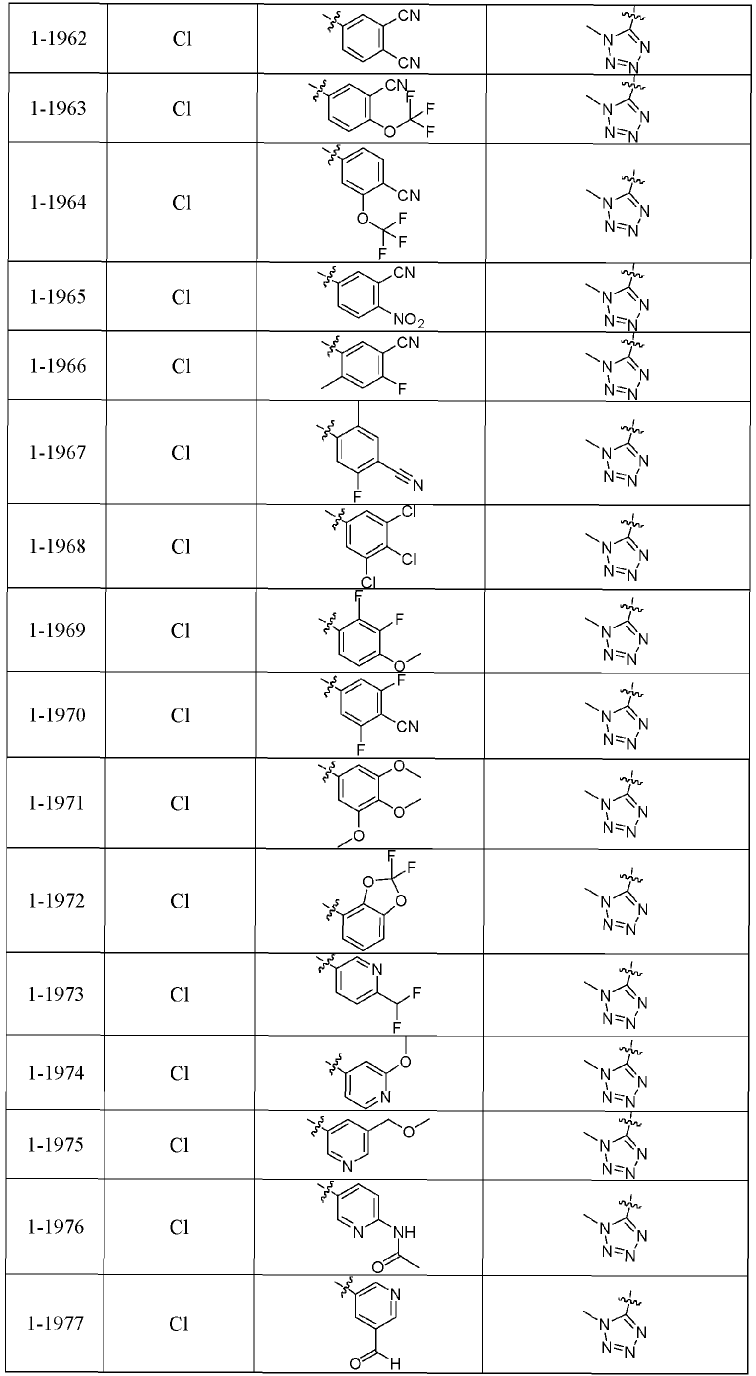

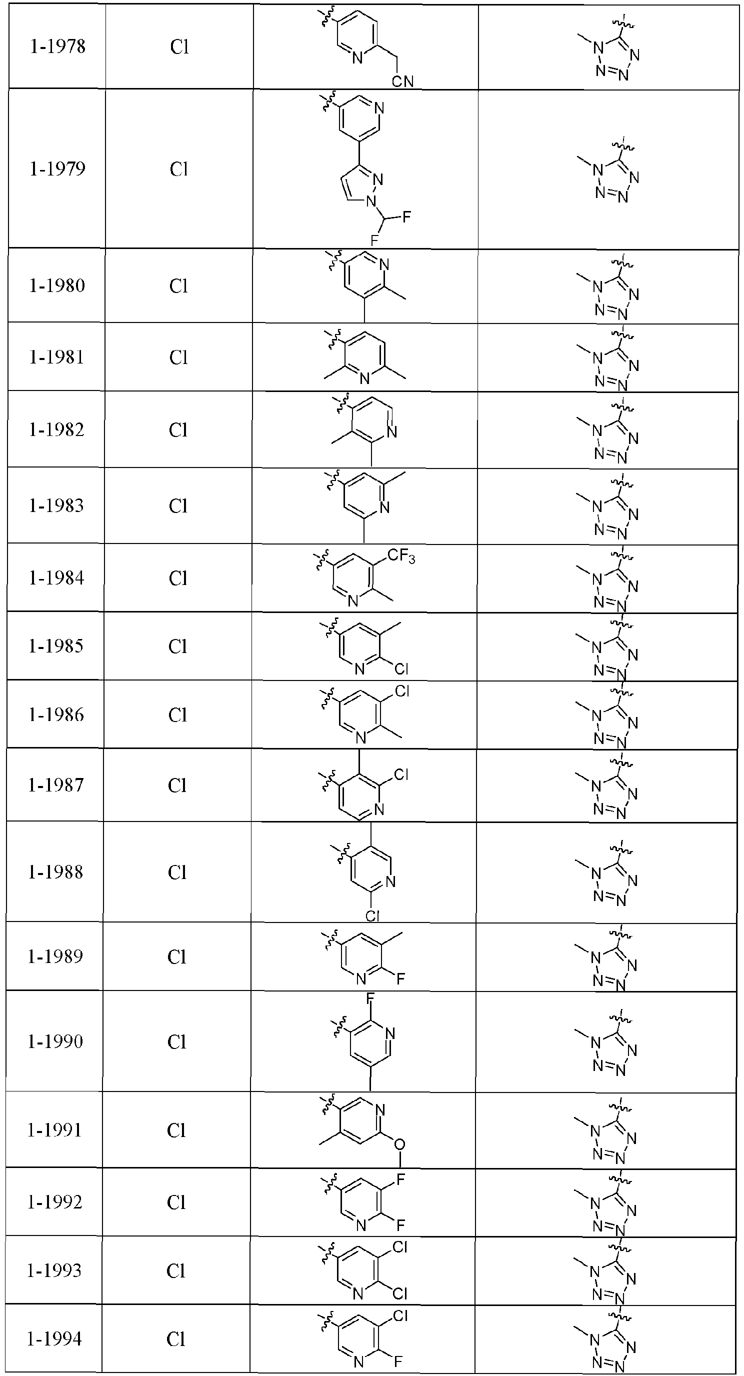

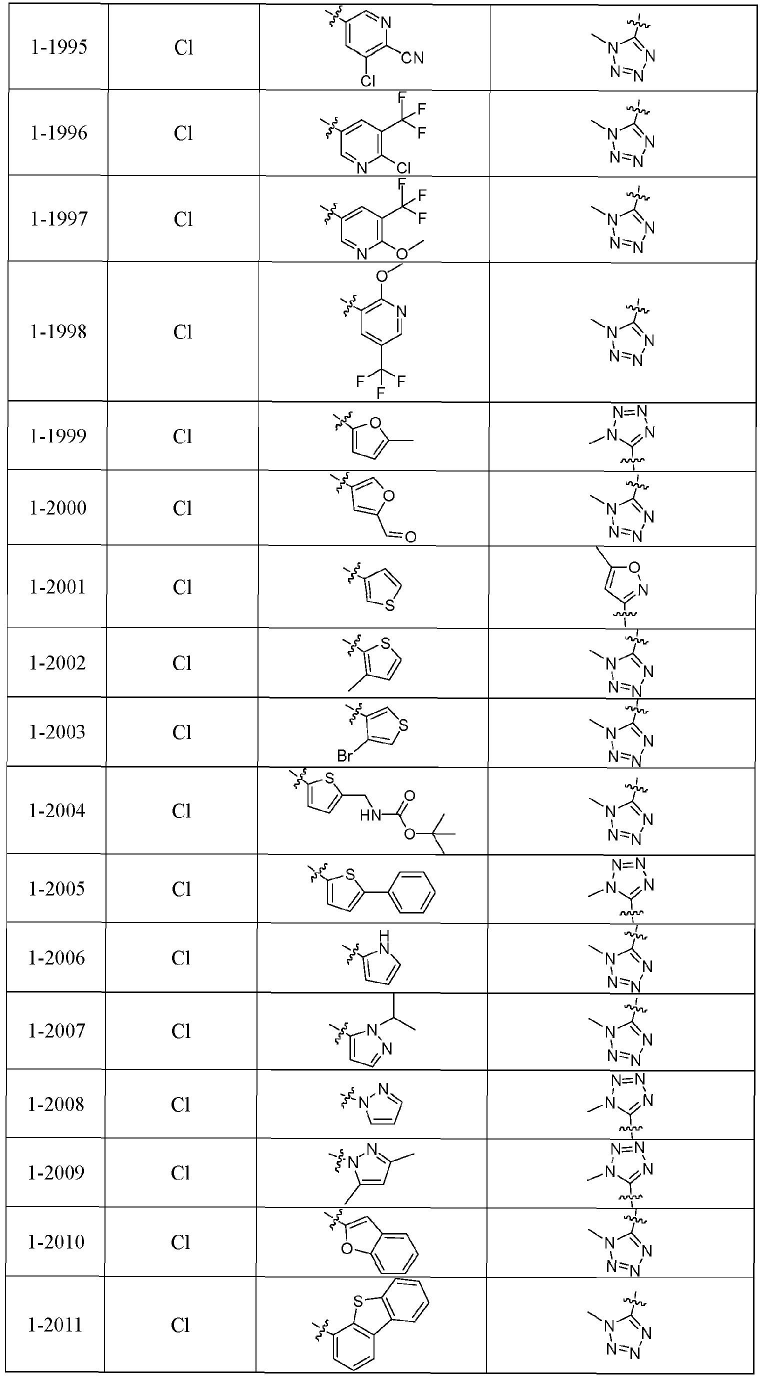

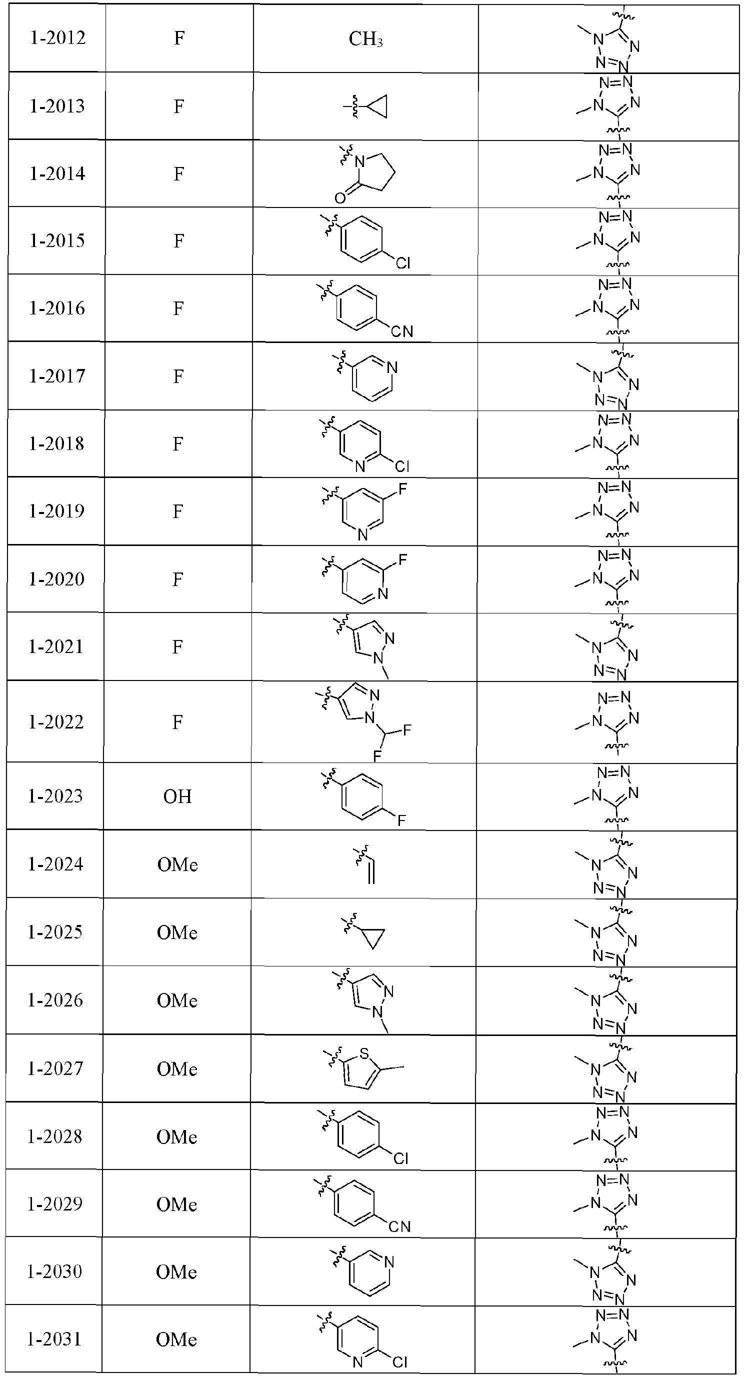

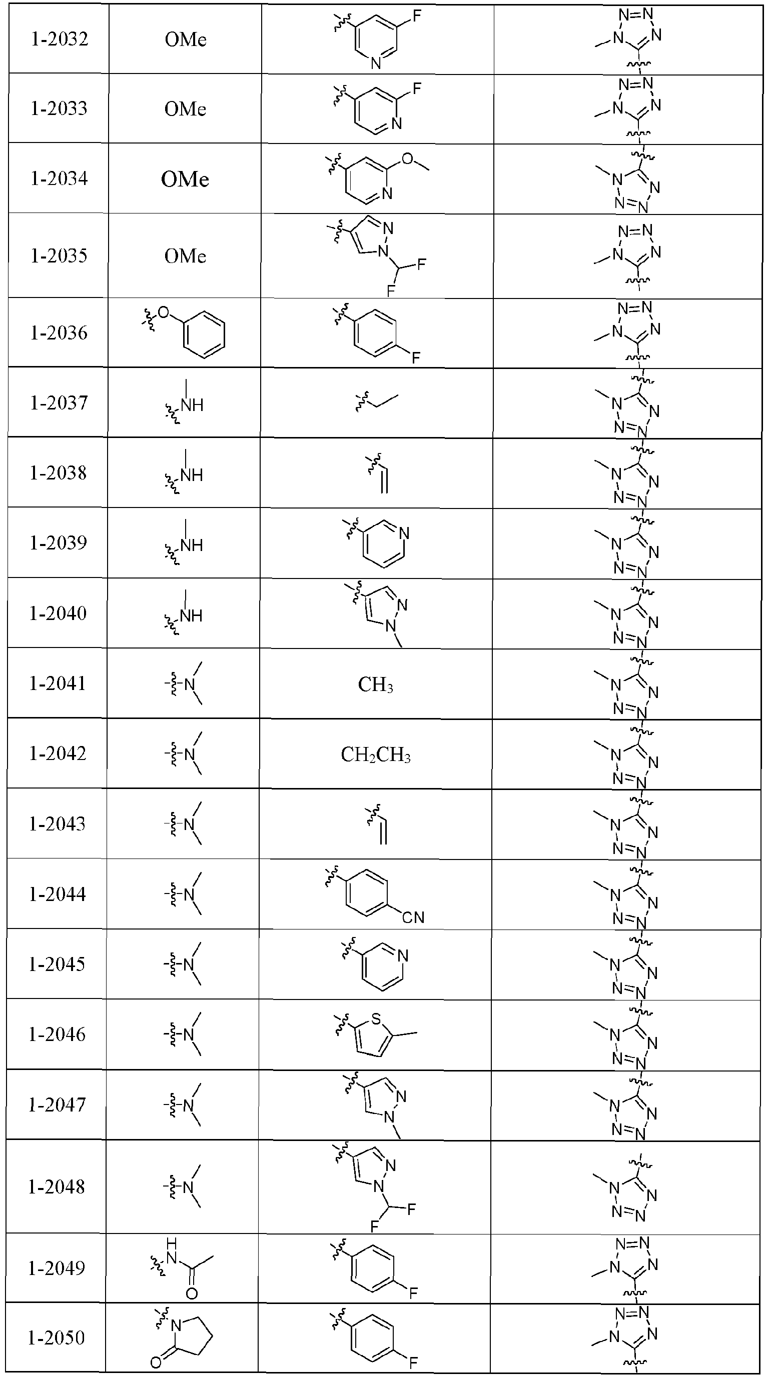

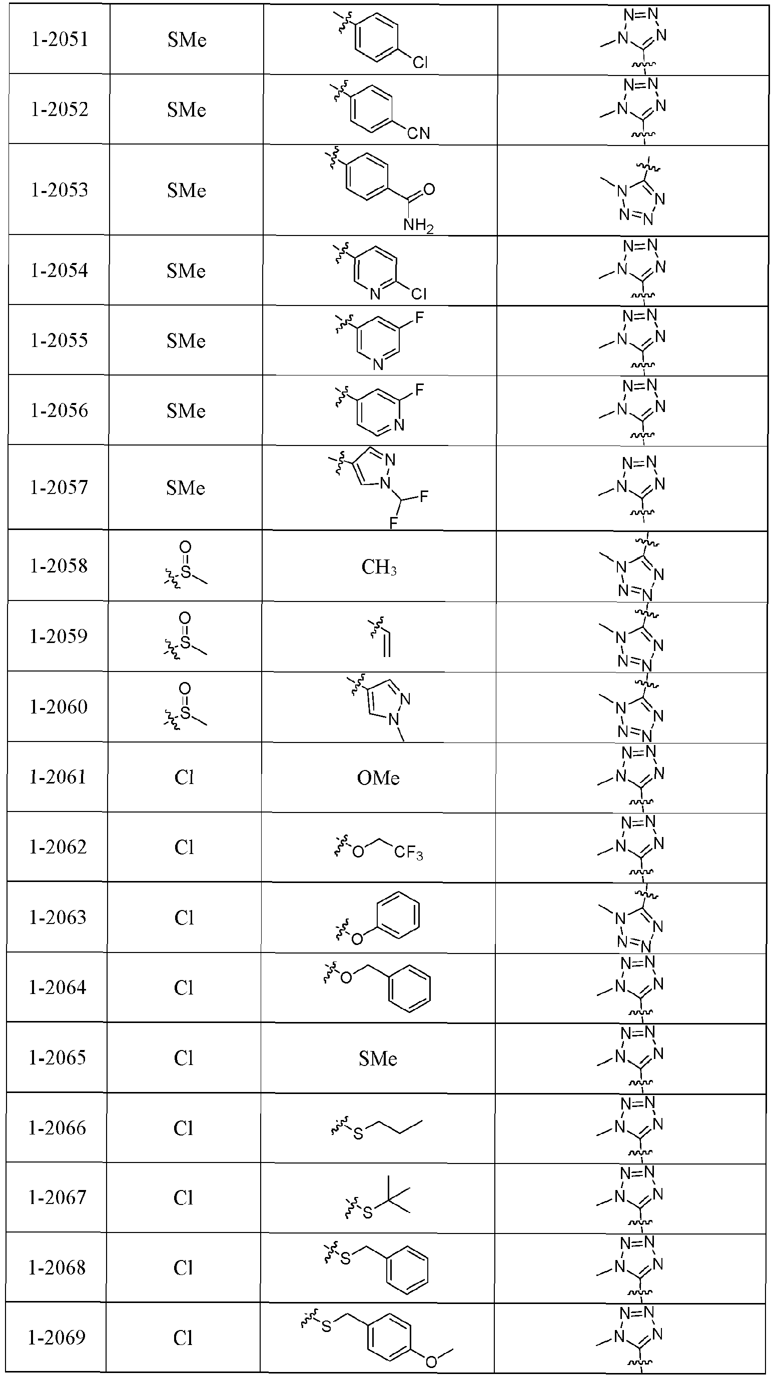

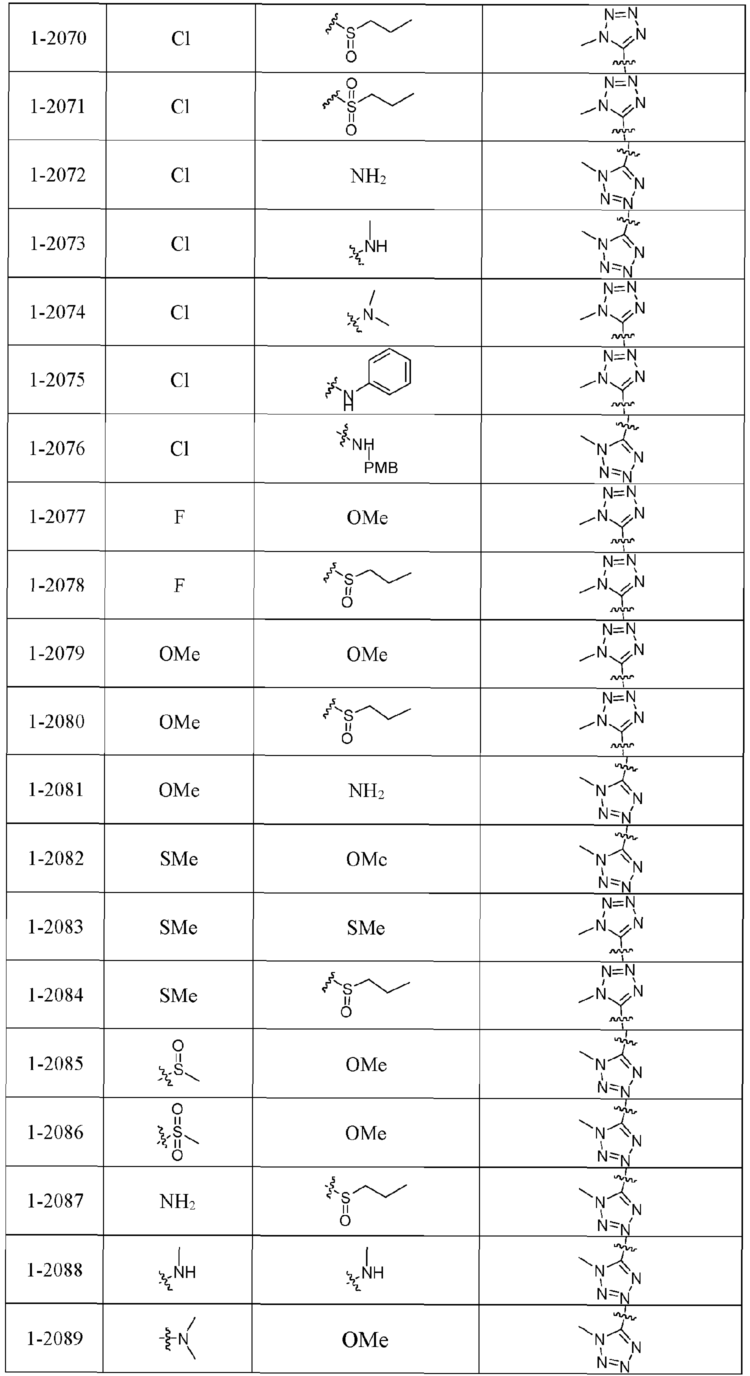

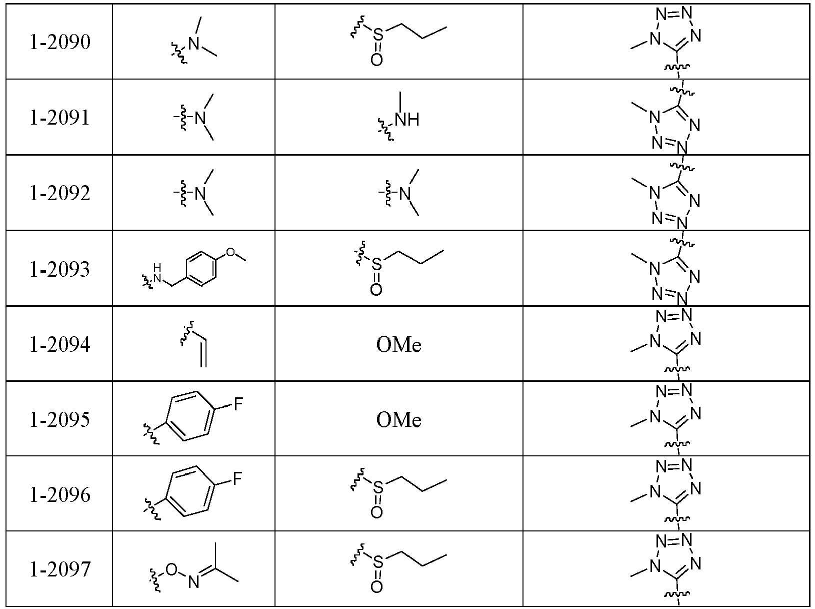

7. A method for preparing the 4-pyridinyl formamide compound according to any one of claims 1 to 6, which is **characterized in that**,
subjecting the compound of the general formula II and the compound of the general formula III to amidation reaction to obtain the compound of the general formula I, the chemical reaction scheme is as follows: 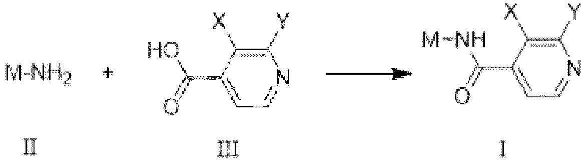
the reaction is carried out in the presence of a halogenating agent, a catalyst and a solvent; preferably, the halogenating agent is SOCl₂, the catalyst is 4-dimethylaminopyridine, and the solvent is pyridine;
or the reaction is carried out in the presence of a condensing agent and a solvent; preferably, the condensing agent is CDI, DCC, DBU or a combination thereof, and the solvent is methylene chloride.

8. A herbicidal composition, which is **characterized in that**, comprising (i) the 4-pyridinyl formamide compound or derivative thereof according to any one of claims 1 to 6; preferably, further comprising (ii) one or more further herbicides and/or safeners; more preferably, further comprising (iii) agrochemically acceptable formulation auxiliaries.

9. A method for controlling a weed, which is **characterized in that**, comprising applying a herbicidally effective amount of at least one of the 4-pyridinyl formamide compound or derivative thereof according to any one of claims 1 to 6 or the herbicidal composition according to claim 8 to a plant or a weed area.

10. Use of at least one of the 4-pyridinyl formamide compound or derivative thereof according to any one of claims 1 to 6; or the herbicidal composition according to claim 8 for controlling a weed, preferably, wherein the 4-pyridinyl formamide compound or derivative thereof is used for preventing and/or controlling a weed in a useful crop, wherein the useful crop is a transgenic crop or a crop treated by gene editing technique.

## Patentansprüche

1. 4-Pyridinylformamidverbindung oder Derivat davon, wie in Formel I gezeigt: 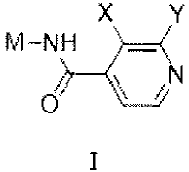
wobei X Nitro, Halogen, Cyano, Formyl, Thiocyano, Mercapto; Alkyl, Alkenyl, Alkinyl, Cycloalkyl, Cycloalkenyl, Cycloalkylalkyl oder Cycloalkenylalkyl, das mit oder ohne Halogen ist; OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -Alkyl-C(S)R¹, -Alkyl-C(S)OR¹, - Alkyl-C(O)SR¹, -Alkyl-C(S)SR¹, -Alkyl-SC(O)R¹, -Alkyl-OC(S)R¹, -Alkyl-SC(S)R¹, -O-Alkyl-NR⁴R⁵, -S-Alkyl-NR⁴R⁵, -Alkyl-O-alkyl-NR⁴R⁵, -Alkyl-S-alkyl-NR⁴R⁵, -Alkyl-(C=S)ₙ-NR⁴R⁵, - NH-Alkyl-NR⁴R⁵, -Alkyl-OR¹, -Alkyl-COR¹, -Alkyl-CO₂R¹, -Alkyl-OCOR¹, -Alkyl-NR³COR¹, -Alkyl-SO₂OR¹, -Alkyl-NR³SO₂R², -Alkyl-OSO₂R², -Alkyl-S(O)ₘR², -Alkyl-CONR⁴R⁵, -Alkyl-SO₂NR⁴R⁵, NR⁴R⁵, 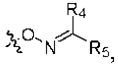 P(O)(OR⁶)₂, CH₂P(O)(OR⁶)₂, SO₂NR⁴R⁵-Alkyl-S(O)ₘR², -Alkyl-CN, -Alkylaryl, -Alkylheteroaryl, -Alkylheterocyclyl, Aryl, Heteroaryl oder Heterocyclyl darstellt;
Y Nitro, Cyano, Formyl, Thiocyano, Mercapto; Alkyl, Alkenyl, Alkinyl, Cycloalkyl, Cycloalkenyl, Cycloalkylalkyl oder Cycloalkenylalkyl, das mit oder ohne Halogen ist; OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -Alkyl-C(S)R¹, -Alkyl-C(S)OR¹, - Alkyl-C(O)SR¹, -Alkyl-C(S)SR¹, -Alkyl-SC(O)R¹, -Alkyl-OC(S)R¹, -Alkyl-SC(S)R¹, -O-Alkyl-NR⁴R⁵, -S-Alkyl-NR⁴R⁵, -Alkyl-O-alkyl-NR⁴R⁵, -Alkyl-S-alkyl-NR⁴R⁵, -Alkyl-(C=S)ₙ-NR⁴R⁵, - NH-Alkyl-NR⁴R⁵, -Alkyl-OR¹, -Alkyl-COR¹, -Alkyl-CO₂R¹, -Alkyl-OCOR¹, -Alkyl-NR³COR¹, -Alkyl-SO₂OR¹, -Alkyl-NR³SO₂R², -Alkyl-OSO₂R², -Alkyl-S(O)ₘR², -Alkyl-CONR⁴R⁵, -Alkyl-SO₂NR⁴R⁵, NR⁴R⁵, 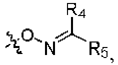 P(O)(OR⁶)₂, CH₂P(O)(OR⁶)₂, SO₂NR⁴R⁵-Alkyl-S(O)ₘR², -Alkyl-CN, -Alkylaryl, -Alkylheteroaryl, -Alkylheterocyclyl, Aryl, Heteroaryl oder Heterocyclyl darstellt;
R¹, R³, R⁴ und R⁵ jeweils unabhängig Wasserstoff, Aryl, Arylalkyl, Heteroaryl, Heteroarylalkyl, Alkyl, halogeniertes Alkyl, Alkenyl, halogeniertes Alkenyl, Alkinyl, halogeniertes Alkinyl, Cycloalkyl, halogeniertes Cycloalkyl, Alkoxyalkyl oder Cycloalkylalkyl darstellen, wobei die letzten 10 Gruppen wie genannt jeweils substituiert sind durch s Gruppen ausgewählt aus der Gruppe bestehend aus Cyano, Halogen, Nitro, Thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸ und Alkoxyalkoxycarbonyl;
R² Aryl, Arylalkyl, Heteroaryl, Heteroarylalkyl, Alkyl, Alkenyl, Alkinyl, Cycloalkyl oder Cycloalkylalkyl darstellt, wobei die letzten 5 Gruppen wie genannt jeweils substituiert sind durch s Gruppen ausgewählt aus der Gruppe bestehend aus Cyano, Halogen, Nitro, Thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸ und Alkoxyalkoxycarbonyl;
R⁶ Methyl oder Ethyl darstellt;
R⁷ und R⁸ jeweils unabhängig Wasserstoff, Alkyl, Alkenyl oder Alkinyl darstellen;
R⁹ Alkyl, Alkenyl oder Alkinyl darstellt;
M 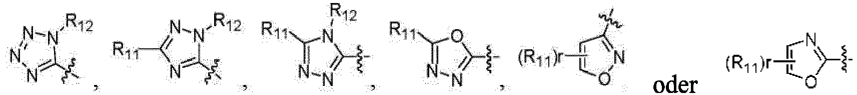 darstellt, das nicht substituiert oder substituiert ist;
R₁₁ Wasserstoff, Halogen, Cyano, Nitro, Alkyl, das nicht substituiert oder substituiert ist mit einem Substituenten ausgewählt aus R₁₃, Cycloalkyl, das nicht substituiert oder substituiert ist mit einem Substituenten ausgewählt aus R₁₄, Alkenyl, halogeniertes Alkenyl, Alkinyl, halogeniertes Alkinyl, Cycloalkenyl, NH₂, Aminoacyl, Carboxyl, Alkoxyalkoxycarbonyl, OR₁₅, - Alkyl-OR₁₅, C(O)R₁₆, -Alkyl-C(O)R₁₆, C(O)OR₁₆, -Alkyl-C(O)OR₁₆, S(O)ₘR₁₆, -Alkyl-S(O)ₘR₁₆, -N(R₁₆)₂, -NHR₁₆, -C(O)NHR₁₆, -C(O)N(R₁₆)₂, -NHC(O)R₁₇, Heterocyclyl, Heterocyclylalkyl, Heterocyclyloxy, Heterocyclylcarbonyl, Aryl, Arylalkyl, Aryloxy, Arylcarbonyl, Heteroaryl, Heteroarylalkyl, Heteroaryloxy oder Heteroarylcarbonyl darstellt;
R₁₂ Wasserstoff, Alkyl, das nicht substituiert oder substituiert ist mit einem Substituenten ausgewählt aus R₁₈, Cycloalkyl, halogeniertes Cycloalkyl, Alkenyl, halogeniertes Alkenyl, Alkinyl, halogeniertes Alkinyl, Cycloalkenyl, Alkoxyalkyl, Alkylthioalkyl, Alkoxycarbonylalkyl, Aryl oder Arylalkyl darstellt;
wenn M 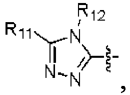 -(CH₂)₄- oder -CH=CH-CH=CH- gebildet durch R₁₁ und R₁₂ ist, das Stickstoffatom gebunden an R₁₂ und das Kohlenstoffatom gebunden an R₁₁ zusammen einen 6-gliedrigen Ring bilden;
R₁₅ Alkyl, das nicht substituiert oder substituiert ist mit einem Substituenten ausgewählt aus R₂₁, Cycloalkyl, halogeniertes Cycloalkyl, Alkenyl, halogeniertes Alkenyl, Alkinyl, halogeniertes Alkinyl, Cycloalkenyl oder Phenyl darstellt;
R₁₆ Alkyl, halogeniertes Alkyl, Cycloalkyl, Alkenyl, halogeniertes Alkenyl, Alkinyl, halogeniertes Alkinyl oder Cycloalkenyl darstellt;
R₂₁ Halogen, Cyano, Cycloalkyl, Hydroxy, Mercapto, Alkoxy, C(O)R₂₂, Carboxyl, Alkoxycarbonyl, Alkoxyalkoxycarbonyl, -S(O)ₘ-Alkyl, Heteroaryl, Heterocyclyl oder Phenyl, das nicht substituiert oder substituiert ist mit einer oder mehreren Gruppen ausgewählt aus R₂₃, darstellt;
R₁₇ und R₂₂ jeweils unabhängig Wasserstoff, Alkyl oder N(R₂₄)R₂₅ darstellen;
R₂₃ Halogen, Cyano, Nitro, Alkyl, Alkyl, das nicht substituiert oder substituiert ist mit einem Substituenten ausgewählt aus R₃₁, Cycloalkyl, halogeniertes Cycloalkyl, Alkenyl, halogeniertes Alkenyl, Alkinyl, halogeniertes Alkinyl, Cycloalkenyl, Alkylcarbonyl, Cycloalkylcarbonyl, halogeniertes Alkylcarbonyl, halogeniertes Cycloalkylcarbonyl, Alkoxycarbonyl, halogeniertes Alkoxycarbonyl, Alkylaminocarbonyl, halogeniertes Alkylaminocarbonyl, bis(Alkyl)aminocarbonyl, OR₃₂, S(O)ₘR₃₃, Alkylaminosulfonyl, bis(Alkyl)aminosulfonyl, NH₂, Alkylamino, bis(Alkyl)amino, Aryl, Heteroaryl oder Heterocyclyl darstellt;
R₂₄ und R₂₅ jeweils unabhängig Wasserstoff, Alkyl oder Phenyl darstellen; oder
Alkylidenkette gebildet durch R₂₄ und R₂₅ und das/die Stickstoffatom(e) gebunden an R₂₄ und R₂₅ zusammen einen 3-7-gliedrigen Ring bilden, wobei die Alkylidenkette optional ein O, S, S(O), S(O)₂, NH oder N-Alkyl und optional substituiert durch Oxo- oder Thiogruppe enthält;
R₁₃, R₁₄, R₁₈ und R₃₁ jeweils unabhängig Halogen, Cyano, Nitro, Carboxyl, Alkoxycarbonyl, Alkoxyalkoxycarbonyl, S(O)ₘR₄₁, OR₄₂, Aryl, Heteroaryl oder Heterocyclyl darstellen;
R₃₂ Wasserstoff, Alkyl, halogeniertes Alkyl, Cycloalkyl, halogeniertes Cycloalkyl, Alkenyl, halogeniertes Alkenyl, Alkinyl, halogeniertes Alkinyl oder Cycloalkenyl darstellt;
R₃₃ Alkyl, halogeniertes Alkyl, Cycloalkyl, Alkenyl, halogeniertes Alkenyl, Alkinyl, halogeniertes Alkinyl oder Cycloalkenyl darstellt;
R₄₁ und R₄₂ jeweils unabhängig Wasserstoff, Alkyl, halogeniertes Alkyl, Cycloalkyl, halogeniertes Cycloalkyl, Alkenyl, halogeniertes Alkenyl, Alkinyl, halogeniertes Alkinyl, Cycloalkenyl oder Phenyl darstellen;
r 1 oder 2 darstellt;
m 0, 1 oder 2 darstellt;
n 0 oder 1 darstellt;
s 0, 1, 2 oder 3 darstellt;
wobei, wenn X Fluor ist, Y nicht Amino, Monomethylamino, Monoethylamino und Monopropylamino ist; wenn M 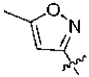 ist, X und Y nicht gleichzeitig Methyl sind.

2. 4-Pyridinylformamidverbindung oder Derivat davon nach Anspruch 1, **dadurch gekennzeichnet, dass**
X Nitro, Halogen, Cyano, Formyl, Thiocyano, Mercapto; C1-C8-Alkyl, C2-C8-Alkenyl, C2-C8-Alkinyl, C3-C8-Cycloalkyl, C3-C8-Cycloalkenyl, C3-C8-Cycloalkyl-C1-C6-alkyl oder C3-C8-Cycloalkenyl-Cl-C6-alkyl, das mit oder ohne Halogen ist; OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -(C1-C6)Alkyl-C(S)R¹, -(C1-C6)Alkyl-C(S)OR¹, -(C1-C6)Alkyl-C(O)SR¹, -(C1-C6)Alkyl-C(S)SR¹, -(C1-C6)Alkyl-SC(O)R¹, -(C1-C6)Alkyl-OC(S)R¹, -(C1-C6)Alkyl-SC(S)R¹, -O-(C1-C6)Alkyl-NR⁴R⁵, -S-(C1-C6)Alkyl-NR⁴R⁵, -(C1-C6)Alkyl-O-(C1-C6)alkyl-NR⁴R⁵, -(C1-C6)Alkyl-S-(C1-C6)alkyl-NR⁴R⁵, -(C1-C6)Alkyl-(C=S)ₙ-NR⁴R⁵, -NH-(C1-C6)Alkyl-NR⁴R⁵, -(C1-C6)Alkyl-OR¹, -(C1-C6)Alkyl-COR¹, -(C1-C6)Alkyl-CO₂R¹, -(C1-C6)Alkyl-OCOR¹, -(C1-C6)Alkyl-NR³COR¹, -(C1-C6)Alkyl-SO₂OR¹, -(C1-C6)Alkyl-NR³SO₂R², -(C1-C6)Alkyl-OSO₂R², -Alkyl-S(O)ₘR², -(C1-C6)Alkyl-CONR⁴R⁵, -(C1-C6)Alkyl-SO₂NR⁴R⁵, NR⁴R⁵, 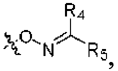 P(O)(OR⁶)₂, CH₂P(O)(OR⁶)₂, SO₂NR⁴R⁵-(C1-C6)Alkyl-S(O)ₘR², -(C1-C6)Alkyl-CN, -(C1-C6)Alkylaryl, -(C1-C6)Alkylheteroaryl, -(C1-C6)Alkylheterocyclyl, Aryl, Heteroaryl oder Heterocyclyl darstellt;
Y Nitro, Cyano, Formyl, Thiocyano, Mercapto; C1-C8-Alkyl, C2-C8-Alkenyl, C2-C8-Alkinyl, C3-C8-Cycloalkyl, C3-C8-Cycloalkenyl, C3-C8-Cycloalkyl-C1-C6-Alkyl oder C3-C8-Cycloalkenyl-C1-C6-alkyl, das mit oder ohne Halogen ist; OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -(C1-C6)Alkyl-C(S)R¹, -(C1-C6)Alkyl-C(S)OR¹, -(C1-C6)Alkyl-C(O)SR¹, -(C1-C6)Alkyl-C(S)SR¹, -(C1-C6)Alkyl-SC(O)R¹, -(C1-C6)Alkyl-OC(S)R¹, -(C1-C6)Alkyl-SC(S)R¹, -O-(C1-C6)Alkyl-NR⁴R⁵, -S-(C1-C6)Alkyl-NR⁴R⁵, -(C1-C6)Alkyl-O-(C1-C6)alkyl-NR⁴R⁵, -(C1-C6)Alkyl-S-(C1-C6)alkyl-NR⁴R⁵, -(C1-C6)Alkyl-(C=S)ₙ-NR⁴R⁵, -NH-(C1-C6)Alkyl-NR⁴R⁵, -(C1-C6)Alkyl-OR¹, -(C1-C6)Alkyl-COR¹, -(C1-C6)Alkyl-CO₂R¹, -(C1-C6)Alkyl-OCOR¹, -(C1-C6)Alkyl-NR³COR¹, -(C1-C6)Alkyl-SO₂OR¹, -(C1-C6)Alkyl-NR³SO₂R², -(C1-C6)Alkyl-OSO₂R², -Alkyl-S(O)ₘR², -(C1-C6)Alkyl-CONR⁴R⁵, -(C1-C6)Alkyl-SO₂NR⁴R⁵, NR⁴R⁵, 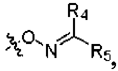 P(O)(OR⁶)₂, CH₂P(O)(OR⁶)₂, SO₂NR⁴R⁵-(C1-C6)Alkyl-S(O)ₘR², -(C1-C6)Alkyl-CN, -(C1-C6)Alkylaryl, -(C1-C6)Alkylheteroaryl, -(C1-C6)Alkylheterocyclyl, Aryl, Heteroaryl oder Heterocyclyl darstellt;
R¹, R³, R⁴ und R⁵ jeweils unabhängig Wasserstoff, Aryl, Aryl-C1-C6-alkyl, Heteroaryl, Heteroaryl-C1-C6-alkyl, C1-C8-Alkyl, halogeniertes C1-C8-Alkyl, C2-C8-Alkenyl, halogeniertes C2-C8-Alkenyl, C2-C8-Alkinyl, halogeniertes C2-C8-Alkinyl, C3-C8-Cycloalkyl, halogeniertes C3-C8-Cycloalkyl, C1-C8-Alkoxy-C1-C6-alkyl oder C3-C8-Cycloalkyl-C1-C6-alkyl darstellen, wobei die letzten 10 Gruppen wie genannt jeweils substituiert sind durch s Gruppen ausgewählt aus der Gruppe bestehend aus Cyano, Halogen, Nitro, Thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸ und C1-C8-Alkoxy-C1-C6-alkoxycarbonyl;
R² Aryl, Aryl-C1-C6-alkyl, Heteroaryl, Heteroaryl-C1-C6-alkyl, C1-C8-Alkyl, C2-C8-Alkenyl, C2-C8-Alkinyl, C3-C8-Cycloalkyl oder C3-C8-Cycloalkyl-C1-C6-alkyl darstellt, wobei die letzten 5 Gruppen wie genannt jeweils substituiert sind durch s Gruppen ausgewählt aus der Gruppe bestehend aus Cyano, Halogen, Nitro, Thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸ und C1-C8-Alkoxy-C1-C6-alkoxycarbonyl;
R⁶ Methyl oder Ethyl darstellt;
R⁷ und R⁸ jeweils unabhängig Wasserstoff, C1-C8-Alkyl, C2-C8-Alkenyl oder C2-C8-Alkinyl darstellen;
R⁹ C1-C8-Alkyl, C2-C8-Alkenyl oder C2-C8-Alkinyl darstellt;
M 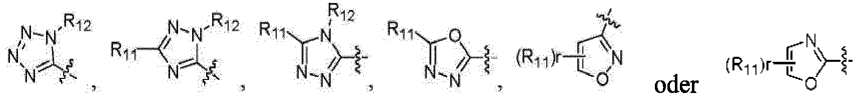 darstellt, das nicht substituiert oder substituiert ist;
R₁₁ Wasserstoff, Halogen, Cyano, Nitro, C1-C8-Alkyl, das nicht substituiert oder substituiert ist mit einem Substituenten ausgewählt aus R₁₃, C3-C8-Cycloalkyl, das nicht substituiert oder substituiert ist mit einem Substituenten ausgewählt aus R₁₄, C2-C8-Alkenyl, halogeniertes C2-C8-Alkenyl, C2-C8-Alkinyl, halogeniertes C2-C8-Alkinyl, C3-C8-Cycloalkenyl, NH₂, Aminoacyl, Carboxyl, C1-C8-Alkoxy-C1-C6-alkoxycarbonyl, OR₁₅, -(C1-C6)Alkyl-OR₁₅, C(O)R₁₆, -(C1-C6)Alkyl-C(O)R₁₆, C(O)OR₁₆, -(C1-C6)Alkyl-C(O)OR₁₆, S(O)ₘR₁₆, -(C1-C6)Alkyl-S(O)ₘR₁₆, -N(R₁₆)₂, -NHR₁₆, -C(O)NHR₁₆, -C(O)N(R₁₆)₂, -NHC(O)R₁₇, Heterocyclyl, Heterocyclyl-C1-C6-a.lkyl, Heterocyclyloxy, Heterocyclylcarbonyl, Aryl, Aryl-C1-C6-alkyl, Aryloxy, Arylcarbonyl, Heteroaryl, Heteroaryl-C1-C6-alkyl, Heteroaryloxy oder Heteroarylcarbonyl darstellt;
R₁₂ Wasserstoff, C1-C8-Alkyl, das nicht substituiert oder substituiert ist mit einem Substituenten ausgewählt aus R₁₈, C3-C8-Cycloalkyl, halogeniertes C3-C8-Cycloalkyl, C2-C8-Alkenyl, halogeniertes C2-C8-Alkenyl, C2-C8-Alkinyl, halogeniertes C2-C8-Alkinyl, C3-C8-Cycloalkenyl, C 1-C8-Alkoxy-C 1-C6-alkyl, C1-C8-Alkylthio-C1-C6-alkyl, C1-C8-Alkoxycarbonyl-C1-C6-a.lkyl, Aryl oder Aryl-C1-C6-alkyl darstellt;
wenn M 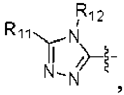 -(CH₂)₄- oder -CH=CH-CH=CH- gebildet durch R₁₁ und R₁₂ ist, das Stickstoffatom, das an R₁₂ gebunden ist und das Kohlenstoffatom, das an R₁₁ gebunden ist, zusammen einen 6-gliedrigen Ring bilden;
R₁₅ C1-C8-Alkyl, das nicht substituiert oder substituiert ist mit einem Substituenten ausgewählt aus R₂₁, C3-C8-Cycloalkyl, halogeniertes C3-C8-Cycloalkyl, C2-C8-Alkenyl, halogeniertes C2-C8-Alkenyl, C2-C8-Alkinyl, halogeniertes C2-C8-Alkinyl, C3-C8-Cycloalkenyl oder Phenyl darstellt;
R₁₆ C1-C8-Alkyl, halogeniertes C1-C8-Alkyl, C3-C8-Cycloalkyl, C2-C8-Alkenyl, halogeniertes C2-C8-Alkenyl, C2-C8-Alkinyl, halogeniertes C2-C8-Alkinyl oder C3-C8-Cycloalkenyl darstellt;
R₂₁ Halogen, Cyano, C3-C8-Cycloalkyl, Hydroxy, Mercapto, C1-C8-Alkoxy, C(O)R₂₂, Carboxyl, C1-C8-Alkoxycarbonyl, C1-C8-Alkoxy-C1-C6-alkoxycarbonyl, -S(O)ₘ-(C1-C8)Alkyl, Heteroaryl, Heterocyclyl oder Phenyl, das nicht substituiert oder substituiert ist mit einer oder mehreren Gruppen ausgewählt aus R₂₃, darstellt;
R₁₇ und R₂₂ jeweils unabhängig Wasserstoff, C1-C8-Alkyl oder N(R₂₄)R₂₅ darstellen;
R₂₃ Halogen, Cyano, Nitro, C1-C8-Alkyl, C1-C8-Alkyl, das nicht substituiert oder substituiert ist mit einem Substituenten ausgewählt aus R₃₁, C3-C8-Cycloalkyl, halogeniertes C3-C8-Cycloalkyl, C2-C8-Alkenyl, halogeniertes C2-C8-Alkenyl, C2-C8-Alkinyl, halogeniertes C2-C8-Alkinyl, C3-C8-Cycloalkenyl, C1-C8-Alkylcarbonyl, C3-C8-Cycloalkylcarbonyl, halogeniertes C1-C8-Alkylcarbonyl, halogeniertes C3-C8-Cycloalkylcarbonyl, C1-C8-Alkoxycarbonyl, halogeniertes C1-C8-Alkoxycarbonyl, C1-C8-Alkylaminocarbonyl, halogeniertes C1-C8-Alkylaminocarbonyl, bis(C1-C8-Alkyl)aminocarbonyl, OR₃₂, S(O)ₘR₃₃, C1-C8-Alkylaminosulfonyl, bis(C1-C8-Alkyl)aminosulfonyl, NH₂, C1-C8-Alkylamino, bis(C1-C8-Alkyl)amino, Aryl, Heteroaryl oder Heterocyclyl darstellt;
R₂₄ und R₂₅ jeweils unabhängig Wasserstoff, C1-C8-Alkyl oder Phenyl darstellen; oder
C2-C8-Alkylidenkette gebildet durch R₂₄ und R₂₅, und das/die Stickstoffatom(e) gebunden an R₂₄ und R₂₅ zusammen einen 3-7-gliedrigen Ring bilden, wobei die C2-C8-Alkylidenkette optional ein O, S, S(O), S(O)₂, NH oder N-Alkyl und optional substituiert durch Oxo- oder Thiogruppe enthält;
R₁₃, R₁₄, R₁₈ und R₃₁ jeweils unabhängig Halogen, Cyano, Nitro, Carboxyl, C1-C8-Alkoxycarbonyl, C1-C8-Alkoxy-C1-C8-alkoxycarbonyl, S(O)ₘR₄₁, OR₄₂, Aryl, Heteroaryl oder Heterocyclyl darstellen;
R₃₂ Wasserstoff, C1-C8-Alkyl, halogeniertes C1-C8-Alkyl, C3-C8-Cycloalkyl, halogeniertes C3-C8-Cycloalkyl, C2-C8-Alkenyl, halogeniertes C2-C8-Alkenyl, C2-C8-Alkinyl, halogeniertes C2-C8-Alkinyl oder C3 -C8-Cycloalkenyl darstellt;
R₃₃ C1-C8-Alkyl, halogeniertes C1-C8-Alkyl, C3-C8-Cycloalkyl, C2-C8-Alkenyl, halogeniertes C2-C8-Alkenyl, C2-C8-Alkinyl, halogeniertes C2-C8-Alkinyl oder C3-C8-Cycloalkenyl darstellt;
R₄₁ und R₄₂ jeweils unabhängig Wasserstoff, C1-C8-Alkyl, halogeniertes C1-C8-Alkyl, C3-C8-Cycloalkyl, halogeniertes C3-C8-Cycloalkyl, C2-C8-Alkenyl, halogeniertes C2-C8-Alkenyl, C2-C8-Alkinyl, halogeniertes C2-C8-Alkinyl, C3-C8-Cycloalkenyl oder Phenyl darstellen;
r 1 oder 2 darstellt;
m 0, 1 oder 2 darstellt;
n 0 oder 1 darstellt;
s 0, 1, 2 oder 3 darstellt;
wobei das "Heterocyclyl" 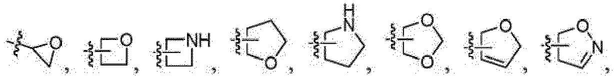 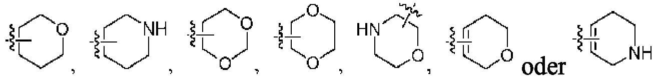 ist, das 0, 1 oder 2 Oxogruppen aufweist; das "Aryl" Phenyl, Naphthyl, 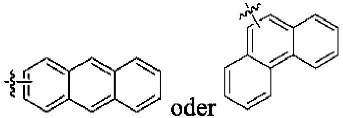 ist; das "Heteroaryl" 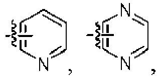 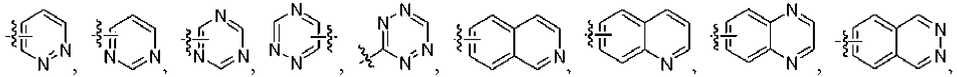 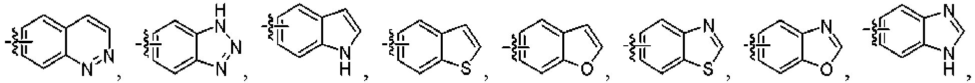 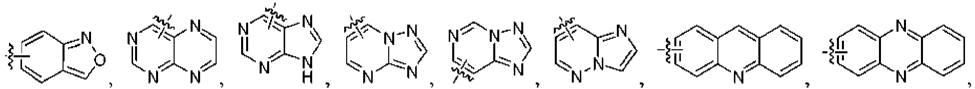 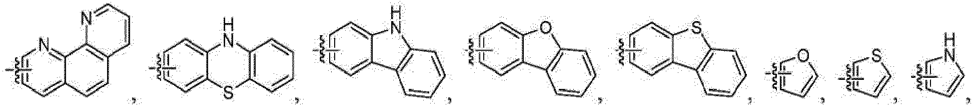 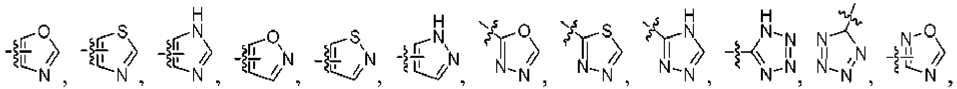 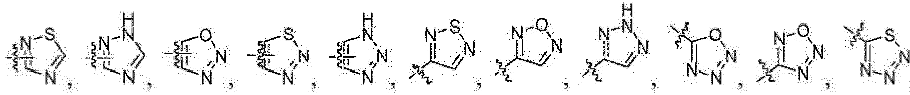 oder 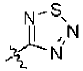 ist; die oben genannten Gruppen jeweils nicht substituiert oder substituiert sind durch zumindest eine Gruppe ausgewählt aus: Halogen, Nitro, Cyano, Thiocyano, Cyanoalkyl, Mercapto, Hydroxy, Hydroxyalkyl, Carboxyl, Formyl, Trialkylsilyl, Dialkylphosphonyl; Heterocyclyl, Heterocyclylalkyl, Aryl, Arylalkyl, Heteroaryl oder Heteroarylalkyl, das nicht substituiert oder substituiert ist; Alkyl, Alkenyl, Alkinyl, Cycloalkyl, Cycloalkenyl, Cycloalkylalkyl, Cycloalkyl substituiert durch Alkyl, OR", SR", -Alkyl-OR", -O-Alkyl-OR", - Alkyl-SR", COR", -Alkyl-COR", -O-Alkyl-COR", COOR", -Alkyl-COOR", -O-Alkyl-COOR", COSR", SOR", SO₂R", -O-SO₂R", -Alkyl-SO₂R", OCOR", -Alkyl-OCOR" oder SCOR"-Gruppe, die mit oder ohne Halogen ist; und Amino-, Aminoalkyl-, Aminocarbonyl-, Aminocarbonylalkyl- oder Aminosulfonylgruppe, die nicht substituiert oder substituiert ist durch eine oder zwei Gruppen ausgewählt aus R", COR", COOR", SO₂R" und OR", wobei das R", COR", COOR", SO₂R" oder OR" mit oder ohne Halogen ist; oder zwei benachbarte substituierbare Positionen der oben genannten "Heterocyclyl-", "Aryl-", "Heteroaryl-"Gruppen verknüpft sind mit Gruppe - OCH₂CH₂-, -OCH₂O-, -OCH₂CH₂O- oder -CH=CH-CH=CH-, um einen Ring zu bilden, wobei die Gruppe -OCH₂CH₂-, -OCH₂O-, -OCH₂CH₂O- oder -CH=CH-CH=CH- mit oder ohne Halogen ist;
R" jeweils unabhängig Alkyl, Alkenyl, Alkinyl, Cycloalkyl, Cycloalkylalkyl, Cycloalkenyl; oder Heterocyclyl, Heterocyclylalkyl, Aryl, Arylalkyl, Heteroaryl oder Heteroarylalkyl, das nicht substituiert oder substituiert ist, darstellt.

3. 4-Pyridinylformamidverbindung oder Derivat davon nach Anspruch 1 oder 2, **dadurch gekennzeichnet, dass**
X Nitro, Halogen, Cyano, Formyl, Thiocyano, Mercapto; C1-C6-Alkyl, C2-C6-Alkenyl, C2-C6-Alkinyl, C3-C6-Cycloalkyl, C3-C6-Cycloalkenyl, C3-C6-Cycloalkyl-C1-C3-alkyl oder C3-C6-Cycloalkenyl-Cl-C3-alkyl, das mit oder ohne Halogen ist; OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -(C1-C3)Alkyl-C(S)R¹, -(C1-C3)Alkyl-C(S)OR¹, -(C1-C3)Alkyl-C(O)SR¹, -(C1-C3)Alkyl-C(S)SR¹, -(C1-C3)Alkyl-SC(O)R¹, -(C1-C3)Alkyl-OC(S)R¹, -(C1-C3)Alkyl-SC(S)R¹, -O-(C1-C3)Alkyl-NR⁴R⁵, -S-(C1-C3)Alkyl-NR⁴R⁵, -(C1-C3)Alkyl-O-(C1-C3)alkyl-NR⁴R⁵, -(C1-C3)Alkyl-S-(C1-C3)alkyl-NR⁴R⁵, -(C1-C3)Alkyl-(C=S)ₙ-NR⁴R⁵, -NH-(C1-C3)Alkyl-NR⁴R⁵, -(C1-C3)Alkyl-OR¹, -(C1-C3)Alkyl-COR¹, -(C1-C3)Alkyl-CO₂R¹, -(C1-C3)Alkyl-OCOR¹, -(C1-C3)Alkyl-NR³COR¹, -(C1-C3)Alkyl-SO₂OR¹, -(C1-C3)Alkyl-NR³SO₂R², -(C1-C3)Alkyl-OSO₂R², -Alkyl-S(O)ₘR², -(C1-C3)Alkyl-CONR⁴R⁵, -(C1-C3)Alkyl-SO₂NR⁴R⁵, NR⁴R⁵, 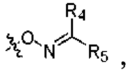 P(O)(OR⁶)₂, CH₂P(O)(OR⁶)₂, SO₂NR⁴R⁵-(C1-C3)AlkylS(O)ₘR², -(C1-C3)Alkyl-CN, -(C1-C3)Alkylaryl, -(C1-C3)Alkylheteroaryl, -(C1-C3)Alkylheterocyclyl, Aryl, Heteroaryl oder Heterocyclyl darstellt;
Y C1-C6-Alkyl, C2-C6-Alkenyl, C2-C6-Alkinyl, C3-C6-Cycloalkyl oder C3-C6-Cycloalkenyl, das mit oder ohne Halogen ist; OR¹, S(O)ₘR², NR⁴R⁵, Heterocyclyl, Aryl oder Heteroaryl darstellt;
R¹, R³, R⁴ und R⁵ jeweils unabhängig Wasserstoff, Aryl, Aryl-C1-C3-alkyl, Heteroaryl, Heteroaryl-C1-C3-alkyl, C1-C6-Alkyl, halogeniertes C1-C6-Alkyl, C2-C6-Alkenyl, halogeniertes C2-C6-Alkenyl, C2-C6-Alkinyl, halogeniertes C2-C6-Alkinyl, C3-C6-Cycloalkyl, halogeniertes C3-C6-Cycloalkyl, C1-C6-Alkoxy-C1-C3-alkyl oder C3-C6-Cycloalkyl-C1-C3-alkyl darstellen, wobei die letzten 10 Gruppen wie genannt jeweils substituiert sind durch s Gruppen ausgewählt aus der Gruppe bestehend aus Cyano, Halogen, Nitro, Thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸ und C1-C6-Alkoxy-C1-C3-alkoxycarbonyl;
R² Aryl, Aryl-C1-C3-alkyl, Heteroaryl, Heteroaryl-C1-C3-alkyl, C1-C6-Alkyl, C2-C6-Alkenyl, C2-C6-Alkinyl, C3-C6-Cycloalkyl oder C3-C6-Cycloalkyl-C1-C3-alkyl darstellt, wobei die letzten 5 Gruppen wie genannt jeweils substituiert sind durch s Gruppen ausgewählt aus der Gruppe bestehend aus Cyano, Halogen, Nitro, Thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸ und C1-C6-Alkoxy-C1-C3-alkoxycarbonyl;
R⁶ Methyl oder Ethyl darstellt;
R⁷ und R⁸ jeweils unabhängig Wasserstoff, C1-C6-Alkyl, C2-C6-Alkenyl oder C2-C6-Alkinyl darstellen;
R⁹ C1-C6-Alkyl, C2-C6-Alkenyl oder C2-C6-Alkinyl darstellt;
M 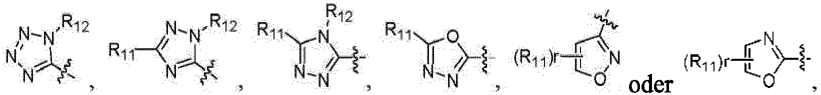 das nicht substituiert oder substituiert ist, darstellt;
R₁₁ Wasserstoff, Halogen, Cyano, Nitro, C1-C6-Alkyl, das nicht substituiert oder substituiert ist mit einem Substituenten ausgewählt aus R₁₃, C3-C6-Cycloalkyl, das nicht substituiert oder substituiert ist mit einem Substituenten ausgewählt aus R₁₄, C2-C6-Alkenyl, halogeniertes C2-C6-Alkenyl, C2-C6-Alkinyl, halogeniertes C2-C6-Alkinyl, C3-C6-Cycloalkenyl, NH₂, Aminoacyl, Carboxyl, C1-C6-Alkoxy-C1-C3-alkoxycarbonyl, OR₁₅, -(C1-C3)Alkyl-OR₁₅, C(O)R₁₆, -(C1-C3)Alkyl-C(O)R₁₆, C(O)OR₁₆, -(C1-C3)Alkyl-C(O)OR₁₆, S(O)ₘR₁₆, -(C1-C3)Alkyl-S(O)ₘR₁₆, -N(R₁₆)₂, -NHR₁₆, -C(O)NHR₁₆, -C(O)N(R₁₆)₂, -NHC(O)R₁₇, Heterocyclyl, Heterocyclyl-C1-C3-alkyl, Heterocyclyloxy, Heterocyclylcarbonyl, Aryl, Aryl-C1-C3-alkyl, Aryloxy, Arylcarbonyl, Heteroaryl, Heteroaryl-C1-C3-alkyl, Heteroaryloxy oder Heteroarylcarbonyl darstellt;
R₁₂ Wasserstoff, C1-C6-Alkyl, das nicht substituiert oder substituiert ist mit einem Substituenten ausgewählt aus R₁₈, C3-C6-Cycloalkyl, halogeniertes C3-C6-Cycloalkyl, C2-C6-Alkenyl, halogeniertes C2-C6-Alkenyl, C2-C6-Alkinyl, halogeniertes C2-C6-Alkinyl, C3-C6-Cycloalkenyl, C1-C6-Alkoxy-C1-C3-alkyl, C1-C6-Alkylthio-C1-C3-alkyl, C1-C6-Alkoxycarbonyl-C1-C3-alkyl, Aryl oder Aryl-C1-C3-alkyl darstellt;
wenn M 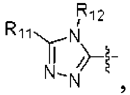 -(CH₂)₄- oder -CH=CH-CH=CH- gebildet durch R₁₁ und R₁₂ ist, das Stickstoffatom, das an R₁₂ gebunden ist, und das Kohlenstoffatom, das an R₁₁ gebunden ist, zusammen einen 6-gliedrigen Ring bilden;
R₁₅ C1-C6-Alkyl, das nicht substituiert oder substituiert ist mit einem Substituenten ausgewählt aus R₂₁, C3-C6-Cycloalkyl, halogeniertes C3-C6-Cycloalkyl, C2-C6-Alkenyl, halogeniertes C2-C6-Alkenyl, C2-C6-Alkinyl, halogeniertes C2-C6-Alkinyl, C3-C6-Cycloalkenyl oder Phenyl darstellt;
R₁₆ C1-C6-Alkyl, halogeniertes C1-C6-Alkyl, C3-C6-Cycloalkyl, C2-C6-Alkenyl, halogeniertes C2-C6-Alkenyl, C2-C6-Alkinyl, halogeniertes C2-C6-Alkinyl oder C3-C6-Cycloalkenyl darstellt;
R₂₁ Halogen, Cyano, C3-C6-Cycloalkyl, Hydroxy, Mercapto, C1-C6-Alkoxy, C(O)R₂₂, Carboxyl, C1-C6-Alkoxycarbonyl, C1-C6-Alkoxy-C1-C3-alkoxycarbonyl, -S(O)ₘ-(C1-C6)Alkyl, Heteroaryl, Heterocyclyl oder Phenyl, das nicht substituiert oder substituiert ist mit 1~3 Gruppen ausgewählt aus R₂₃, darstellt;
R₁₇ und R₂₂ jeweils unabhängig Wasserstoff, C1-C6-Alkyl oder N(R₂₄)R₂₅ darstellen;
R₂₃ Halogen, Cyano, Nitro, C1-C6-Alkyl, C1-C6-Alkyl, das nicht substituiert oder substituiert ist mit einem Substituenten ausgewählt aus R₃₁, C3-C6-Cycloalkyl, halogeniertes C3-C6-Cycloalkyl, C2-C6-Alkenyl, halogeniertes C2-C6-Alkenyl, C2-C6-Alkinyl, halogeniertes C2-C6-Alkinyl, C3-C6-Cycloalkenyl, C1-C6-Alkylcarbonyl, C3-C6-Cycloalkylcarbonyl, halogeniertes C1-C6-Alkylcarbonyl, halogeniertes C3-C6-Cycloalkylcarbonyl, C1-C6-Alkoxycarbonyl, halogeniertes C1-C6-Alkoxycarbonyl, C1-C6-Alkylaminocarbonyl, halogeniertes C1-C6-Alkylaminocarbonyl, bis(C1-C6-Alkyl)aminocarbonyl, OR₃₂, S(O)ₘR₃₃, C1-C6-Alkylaminosulfonyl, bis(C1-C6-Alkyl)aminosulfonyl, NH₂, C1-C6-Alkylamino, bis(C1-C6-Alkyl)amino, Aryl, Heteroaryl oder Heterocyclyl darstellt;
R₂₄ und R₂₅ jeweils unabhängig Wasserstoff, C1-C6-Alkyl oder Phenyl darstellen; oder
C2-C6-Alkylidenkette gebildet durch R₂₄ und R₂₅, und das/die Stickstoffatom(e) gebunden an R₂₄ und R₂₅ zusammen einen 3-7-gliedrigen Ring bilden, wobei die C2-C6-Alkylidenkette optional ein O, S, S(O), S(O)₂, NH oder N-Alkyl und optional substituiert durch Oxo- oder Thiogruppe enthält;
R₁₃, R₁₄, R₁₈ und R₃₁ jeweils unabhängig Halogen, Cyano, Nitro, Carboxyl, C1-C6-Alkoxycarbonyl, C1-C6-Alkoxy-C1-C6-alkoxycarbonyl, S(O)ₘR₄₁, OR₄₂, Aryl, Heteroaryl oder Heterocyclyl darstellen;
R₃₂ Wasserstoff, C1-C6-Alkyl, halogeniertes C1-C6-Alkyl, C3-C6-Cycloalkyl, halogeniertes C3-C6-Cycloalkyl, C2-C6-Alkenyl, halogeniertes C2-C6-Alkenyl, C2-C6-Alkinyl, halogeniertes C2-C6-Alkinyl oder C3-C6-Cycloalkenyl darstellt;
R₃₃ C1-C6-Alkyl, halogeniertes C1-C6-Alkyl, C3-C6-Cycloalkyl, C2-C6-Alkenyl, halogeniertes C2-C6-Alkenyl, C2-C6-Alkinyl, halogeniertes C2-C6-Alkinyl oder C3-C6-Cycloalkenyl darstellt;
R₄₁ und R₄₂ jeweils unabhängig Wasserstoff, C1-C6-Alkyl, halogeniertes C1-C6-Alkyl, C3-C6-Cycloalkyl, halogeniertes C3-C6-Cycloalkyl, C2-C6-Alkenyl, halogeniertes C2-C6-Alkenyl, C2-C6-Alkinyl, halogeniertes C2-C6-Alkinyl, C3-C6-Cycloalkenyl oder Phenyl darstellen;
r 1 oder 2 darstellt;
m 0, 1 oder 2 darstellt;
n 0 oder 1 darstellt;
s 0, 1, 2 oder 3 darstellt;
wobei das "Heterocyclyl" 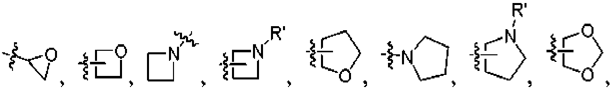 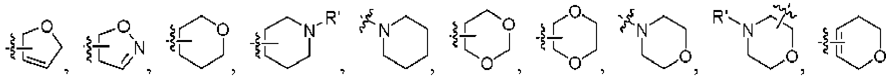 oder 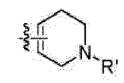 ist, das 0, 1 oder 2 Oxogruppen aufweist; das "Aryl" Phenyl, Naphthyl, 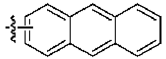 oder 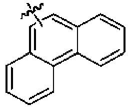 ist; das "Heteroaryl" 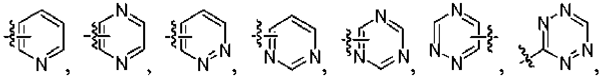 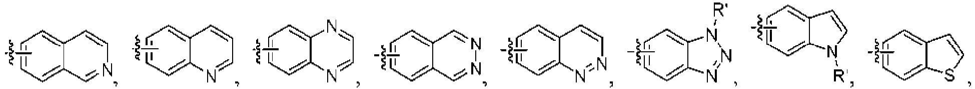 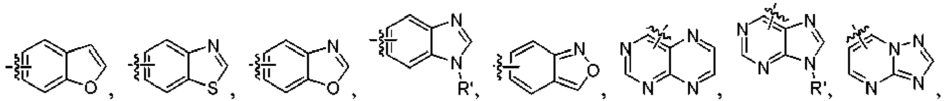 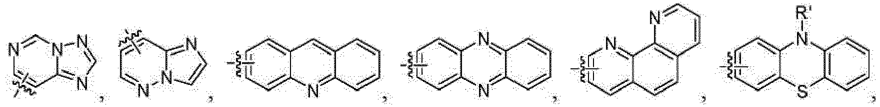 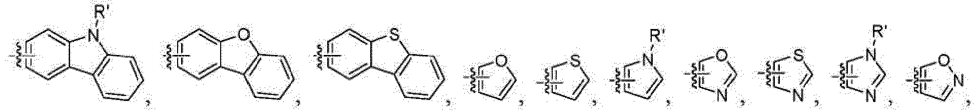 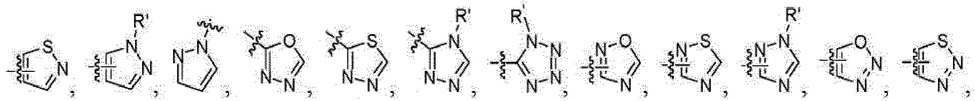 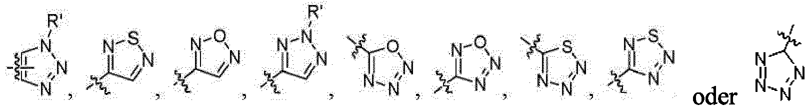 ist; die oben genannten Gruppen jeweils nicht substituiert oder substituiert sind durch 1~3 Gruppen ausgewählt aus: Halogen, Nitro, Cyano, Thiocyano, Cyano-Cl-C6-alkyl, Mercapto, Hydroxy, Hydroxy-C1-C6-alkyl, Carboxyl, Formyl; 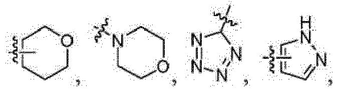 Phenyl oder Benzylgruppe, die nicht substituiert oder substituiert ist durch 1~3 Gruppen ausgewählt aus Halogen, Hydroxy, Nitro, Cyano, Amino, Carboxyl, C1-C6-Alkyl mit oder ohne Halogen, C2-C6-Alkenyl mit oder ohne Halogen, C2-C6-Alkinyl mit oder ohne Halogen, C3-C6-Cycloalkyl mit oder ohne Halogen, C1-C6-Alkoxy mit oder ohne Halogen, C1-C6-Alkoxycarbonyl mit oder ohne Halogen, C1-C6-Alkylacyl mit oder ohne Halogen, C1-C6-Alkylacyloxy mit oder ohne Halogen, C1-C6-Alkylamino mit oder ohne Halogen und C1-C6-Alkylsulfonyl mit oder ohne Halogen; C1-C6-Alkyl, C2-C6-Alkenyl, C2-C6-Alkinyl, C3-C6-Cycloalkyl, C3-C6-Cycloalkyl-C1-C6-alkyl, C3-C6-Cycloalkyl substituiert durch C1-C6-Alkyl, OR", SR", -(C1-C6)Alkyl -OR", -O-(C1-C6)Alkyl -OR", -(C1-C6)Alkyl -SR", COR", -(C1-C6)Alkyl -COR", -O-(C1-C6)Alkyl -COR", COOR", -(C1-C6)Alkyl -COOR", -O-(C1-C6)Alkyl -COOR", COSR", SOR", SO₂R", -O-SO₂R", -(C1-C6)Alkyl -SO₂R", OCOR", -(C1-C6)Alkyl -OCOR" oder SCOR"-Gruppe, die mit oder ohne Halogen ist; und Amino-, Aminoalkyl-, Aminocarbonyl- oder Aminosulfonylgruppe, die nicht substituiert oder substituiert ist durch eine oder zwei Gruppen ausgewählt aus R", COR", COOR", SO₂R" und OR", wobei das R", COR", COOR", SO₂R" oder OR" mit oder ohne Halogen ist; oder zwei benachbarte substituierbare Positionen der oben genannten "Heterocyclyl-", "Aryl-", "Heteroaryl-"Gruppen verknüpft sind mit Gruppe -OCH₂CH₂-, -OCH₂O-, -OCH₂CH₂O- oder - CH=CH-CH=CH-, um einen Ring zu bilden, wobei die Gruppe -OCH₂CH₂-, -OCH₂O-, - OCH₂CH₂O- oder -CH=CH-CH=CH- mit oder ohne Halogen ist;
R' jeweils unabhängig Wasserstoff, Nitro, Hydroxy, Amino; C1-C6-Alkyl, C2-C6-Alkenyl, C2-C6-Alkinyl, C3-C6-Cycloalkyl, C3-C6-Cycloalkenyl, C3-C6-Cycloalkyl-C1-C6-alkyl, 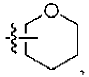 C1-C6-Alkoxy, C2-C6-Alkenyloxy, C2-C6-Alkinyloxy, C3-C6-Cycloalkyloxy, C 1-C6-Alkoxy-C 1-C6-alkyl, C1-C6-Alkoxycarbonyl, C1-C6-Alkylthiocarbonyl, C1-C6-Alkylsulfonyl, C1-C6-Alkylsulfonyl-C1-C6-alkyl, C1-C6-Alkylcarbonyl, C1-C6-Alkylcarbonyl-C1-C6-alkyl, C1-C6-Alkylacyloxy, C1-C6-Alkylamino, C1-C6-Alkylaminocarbonyl, C1-C6-Alkoxyaminocarbonyl, C1-C6-Alkoxycarbonyl-C1-C6-alkyl, C1-C6-Alkylaminocarbonyl-C1-C6-alkyl, tri(C1-C6-Alkyl)silyl oder di(C1-C6-Alkyl)phosphonyl, das mit oder ohne Halogen ist; oder Phenyl oder Benzyl, das nicht substituiert oder substituiert ist durch 1~3 Gruppen ausgewählt aus Halogen, Hydroxy, Nitro, Cyano, Amino, Carboxyl, C1-C6-Alkyl mit oder ohne Halogen, C2-C6-Alkenyl mit oder ohne Halogen, C2-C6-Alkinyl mit oder ohne Halogen, C3-C6-Cycloalkyl mit oder ohne Halogen, C1-C6-Alkoxy mit oder ohne Halogen, C1-C6-Alkoxycarbonyl mit oder ohne Halogen, C1-C6-Alkylacyl mit oder ohne Halogen, C1-C6-Alkylacyloxy mit oder ohne Halogen, C1-C6-Alkylamino mit oder ohne Halogen und C1-C6-Alkylsulfonyl mit oder ohne Halogen darstellt;
R" jeweils unabhängig C1-C6-Alkyl, C2-C6-Alkenyl, C2-C6-Alkinyl, C3-C6-Cycloalkyl, C3-C6-Cycloalkyl-C1-C6-alkyl, C3-C6-Cycloalkenyl; oder 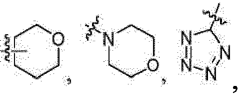 Phenyl oder Benzyl, das nicht substituiert oder substituiert ist durch 1~3 Gruppen ausgewählt aus Halogen, Hydroxy, Nitro, Cyano, Amino, Carboxyl, C1-C6-Alkyl mit oder ohne Halogen, C2-C6-Alkenyl mit oder ohne Halogen, C2-C6-Alkinyl mit oder ohne Halogen, C3-C6-Cycloalkyl mit oder ohne Halogen, C1-C6-Alkoxy mit oder ohne Halogen, C1-C6-Alkoxycarbonyl mit oder ohne Halogen, C1-C6-Alkylacyl mit oder ohne Halogen, C1-C6-Alkylacyloxy mit oder ohne Halogen, C1-C6-Alkylamino mit oder ohne Halogen und C1-C6-Alkylsulfonyl mit oder ohne Halogen, darstellt.

4. 4-Pyridinylformamidverbindung oder Derivat davon nach einem der Ansprüche 1 bis 3, **dadurch gekennzeichnet, dass**
X Halogen, Nitro, Cyano, OR¹, S(O)ₘR², NR³COR¹, NR⁴R⁵, 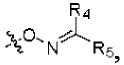 C 1-C6-Alkoxy-C1-C3-alkyl; C1-C6-Alkyl, C2-C6-Alkenyl, C2-C6-Alkinyl oder C3-C6-Cycloalkyl, das mit oder ohne Halogen ist; 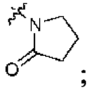 oder Phenyl, das nicht substituiert oder substituiert ist mit 1, 2 oder 3 Gruppen ausgewählt aus Halogen und C1-C6-Alkoxy, darstellt;
Y C1-C6-Alkyl, C2-C6-Alkenyl, C2-C6-Alkinyl, C3-C6-Cycloalkyl oder C3-C6-Cycloalkenyl, das mit oder ohne Halogen ist; OR¹, S(O)ₘR², NR⁴R⁵, Heterocyclyl, Aryl oder Heteroaryl darstellt;
R¹, R³, R⁴ und R⁵ jeweils unabhängig Wasserstoff, C1-C6-Alkyl, halogeniertes C1-C6-Alkyl, Phenyl; oder Benzyl, das nicht substituiert oder substituiert ist mit 1, 2 oder 3 Gruppen ausgewählt aus Halogen und C1-C6-Alkoxy, darstellen;
R² C1-C6-Alkyl oder halogeniertes C1-C6-Alkyl darstellt;
M 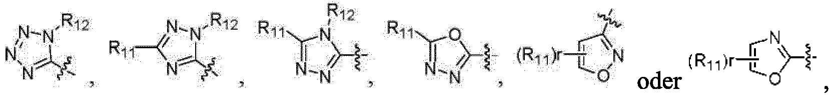 das nicht substituiert oder substituiert ist, darstellt;
R₁₁ Wasserstoff, C1-C6-Alkyl, C3-C6-Cycloalkyl, halogeniertes C1-C6-Alkyl, C1-C6-Alkoxy, C1-C6-Alkylthio, Halogen, C1-C6-Alkylamino, bis(C1-C6-Alkyl)amino, Cyano, Nitro, C1-C6-Alkylcarbonyl, C1-C6-Alkoxycarbonyl, C1-C6-Alkoxycarbonyl-C1-C3 -alkyl, Aminocarbonyl, C1-C6-Alkylcarbonylamino, C1-C6-Alkoxy-C1-C3-alkyl, C1-C6-Alkylthio-C1-C3-alkyl, C1-C6-Alkylsulfinyl-C1-C3-alkyl, C1-C6-Alkylsulfonyl-C1-C3-alkyl; oder Benzyl, Phenoxy, Benzoyl, Pyridyl, 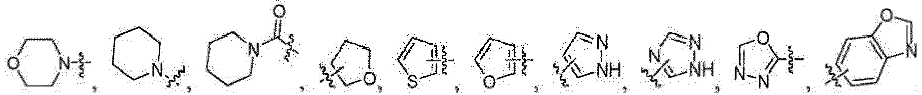 oder 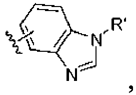 das nicht substituiert oder substituiert ist mit 1, 2 oder 3 Gruppen ausgewählt aus Halogen und C1-C6-Alkoxy, darstellt;
R₁₂ Wasserstoff, C1-C6-Alkyl, C2-C6-Alkenyl, C1-C6-Alkoxy-C1-C3-alkyl, C1-C6-Alkylthio-C1-C3-alkyl, C1-C6-Alkoxycarbonyl-C1-C3-alkyl; oder Phenyl oder Benzyl, das nicht substituiert oder substituiert ist mit zumindest einer Gruppe ausgewählt aus Halogen, und halogeniertes C1-C6-Alkyl darstellt;
r 1 oder 2 darstellt;
m 0, 1 oder 2 darstellt;
wobei das "Heterocyclyl" 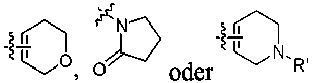 ist; das "Aryl" Phenyl, Naphthyl oder 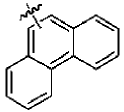 ist, das nicht substituiert oder substituiert ist; das "Heteroaryl" 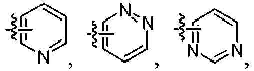 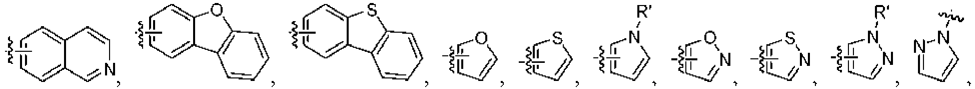 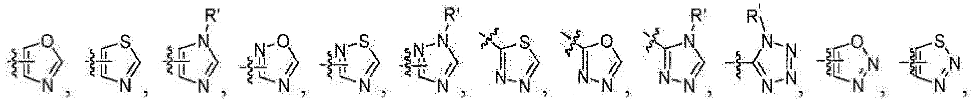 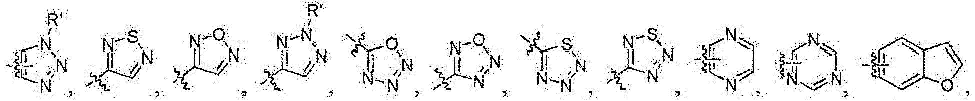 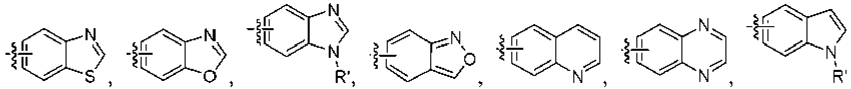 oder 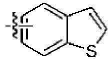 ist, das nicht substituiert oder substituiert ist; sich das zuvor genannte "substituiert" jeweils darauf bezieht, durch 1~3 Gruppen substituiert zu sein, ausgewählt aus: Halogen, Nitro, Cyano, Thiocyano, Cyano-C1-C3-alkyl, Hydroxy, Hydroxy-C1-C3-alkyl, Mercapto, Carboxyl, Formyl, 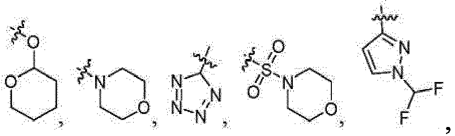 Phenyl, Phenyl substituiert durch C1-C6-Alkyl, Phenoxy, Benzyloxy; Amino, Aminoalkyl oder Aminocarbonylgruppe, die nicht substituiert oder substituiert ist durch eine oder zwei Gruppen ausgewählt aus C1-C6-Alkyl, COR" und COOR"; und C1-C6-Alkyl, C2-C6-Alkenyl, C3-C6-Cycloalkyl, C1-C6-Alkoxy-C1-C3-alkyl, C1-C6-Alkylthio-C1-C3-alkyl, Cl -C6-Alkylcarbonylthio, C3-C6-Cycloalkyl substituiert mit C1-C6-Alkyl, OR", SR", SOR", COR", COOR" oder SO₂R", das mit oder ohne Halogen ist; oder zwei benachbarte substituierbare Positionen der oben genannten "Heterocyclyl-", "Aryl-", "Heteroaryl-"Gruppen verknüpft sind mit Gruppe -OCH₂CH₂-, -OCH₂O-, -OCH₂CH₂O- oder -CH=CH-CH=CH-, um einen Ring zu bilden, wobei die Gruppe -OCH₂CH₂-, -OCH₂O-, -OCH₂CH₂O- oder -CH=CH-CH=CH-, die mit oder ohne Halogen ist;
R' jeweils unabhängig Wasserstoff, C1-C6-Alkyl, C1-C6-Alkylcarbonyl, halogeniertes C1-C6-Alkyl, 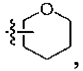 C1-C6-Alkoxycarbonyl, C1-C6-Alkoxy-C1-C3-alkyl oder Benzyl darstellt;
R" jeweils unabhängig C1-C6-Alkyl oder C2-C6-Alkenyl darstellt.

5. 4-Pyridinylformamidverbindung oder Derivat davon nach einem der Ansprüche 1 bis 4, **dadurch gekennzeichnet, dass**
X Chlor, Fluor, Brom, Methyl, Ethyl, Isopropyl, Vinyl, Allyl, Ethinyl, Cyclopropyl, Trifluormethyl, 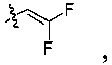 Hydroxy, Methoxy, Ethyloxy, Methylthio, Ethylthio, 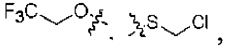 Nitro, Cyano, Methylsulfinyl, Ethylsulfinyl, Methylsulfonyl, Amino, Monomethylamino, Dimethylamino, Acetamido, 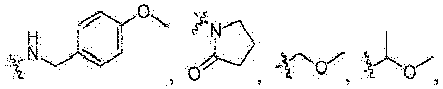 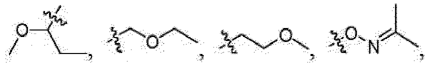 Phenyl, Phenoxy oder 4-Fluorphenyl darstellt;
Y Methyl, Ethyl, Vinyl, Methoxy, 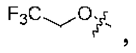 Phenoxy, Benzyloxy, Methylthio, Propylthio, tert-Butylthio, Benzylthio, 4-Methoxybenzylthiol, Propylsulfinyl, Amino, Monomethylamino, Dimethylamino, Anilino, p-Methoxybenzylamino, tert-Butyl, Cyclopropyl, Cyclohexyl, 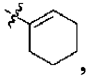 Heterocyclyl, Aryl oder Heteroaryl darstellt;
M 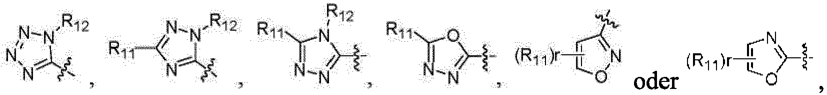 das nicht substituiert oder substituiert ist, darstellt;
R₁₁ jeweils unabhängig Wasserstoff, Methyl, Ethyl, n-Propyl, Isopropyl, Cyclopropyl, Monochlormethyl, Trifluormethyl, Methoxy, Isopropyloxy, Methylthio, Fluor, Chlor, Brom, Iod, Dimethylamino, Ethylamino, Cyano, Nitro, Acetyl, Methoxycarbonyl; Phenyl, das
nicht substituiert oder substituiert ist durch 1, 2 oder 3 Gruppen ausgewählt aus Chlor und Methoxy; Pyridyl, 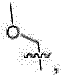 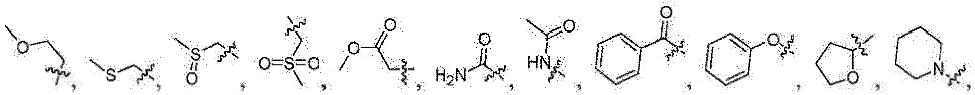 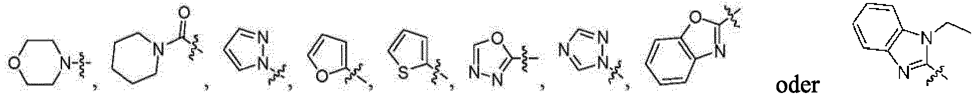 darstellt;
R₁₂ jeweils unabhängig Wasserstoff, Methyl, Ethyl, n-Propyl, Isopropyl, Allyl, 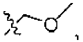 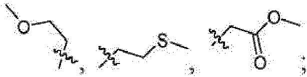 oder Phenyl oder Benzyl, das nicht substituiert oder substituiert ist durch 1, 2 oder 3 Gruppen ausgewählt aus Chlor und Trifluormethyl darstellt;
r 1 oder 2 darstellt; wobei das "Heterocyclyl" 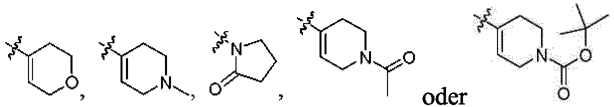 ist; das "Aryl" Phenyl, Naphthyl oder 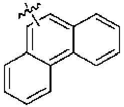 ist, das nicht substituiert oder substituiert ist; das "Heteroaryl" 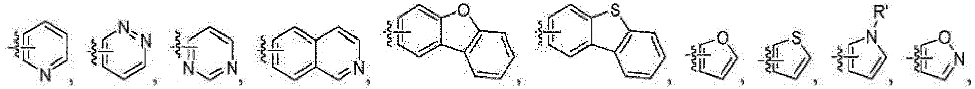 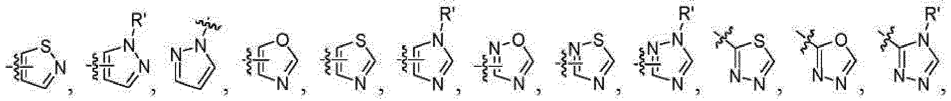 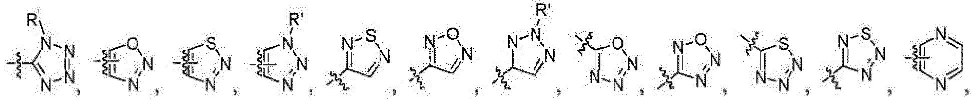 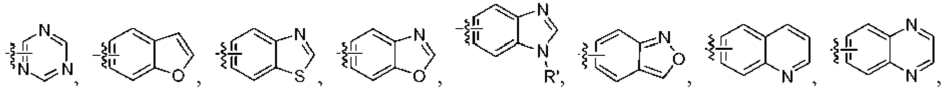 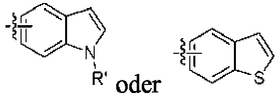 ist, das nicht substituiert oder substituiert ist; sich das zuvor genannte "substituiert" darauf bezieht, durch 1, 2 oder 3 Gruppen substituiert zu sein, ausgewählt aus: Methyl, Ethyl, Isopropyl, n-Butyl, tert-Butyl, n-Pentyl, Vinyl, Cyclopropyl, 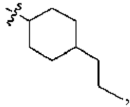 Fluor, Chlor, Brom, Iod, Monobrommethyl, Difluormethyl, Trifluormethyl, Methoxy, Ethoxy, Propoxy, Butoxy, Trifluormethoxy, Hydroxyl, Hydroxymethyl, Amino, Cyano, Cyanomethyl, Thiocyano, Mercapto, Nitro, Carboxy, Formyl, Acetyl, Methoxycarbonyl, tert-Butyloxycarbonyl, Methylthio, Isopropylthio, Methylsulfmyl, Methylsulfonyl, 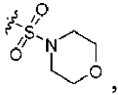 Dimethylamino, Aminocarbonyl, Dimethylaminocarbonyl, Acetylamino, 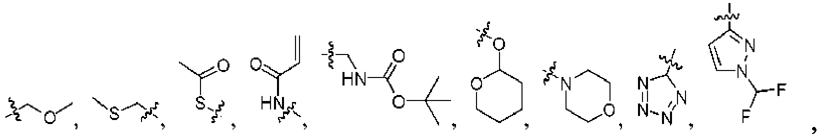 Phenyl, 4-Ethylphenyl, Phenoxy und Benzyloxy; oder zwei benachbarte substituierbare Positionen der oben genannten "Aryl-", "Heteroaryl-"Gruppen verknüpft sind mit Gruppe -OCH₂CH₂-, -OCH₂O-, - OCH₂CH₂O- oder -CH=CH-CH=CH-, um einen Ring zu bilden, wobei die Gruppe -OCH₂CH₂-, - OCH₂O-, -OCH₂CH₂O- oder -CH=CH-CH=CH- mit oder ohne Halogen ist;
R' jeweils unabhängig Wasserstoff, Methyl, Ethyl, n-Propyl, Isopropyl, Difluormethyl, 3,3,3-Trifluorethyl, Benzyl, 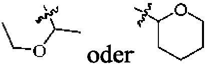 darstellt;
wobei, wenn X Fluor ist, Y nicht Amino und Monomethylamino ist; wenn M 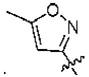 ist, X und Y nicht gleichzeitig Methyl sind.

6. 4-Pyridinylformamidverbindung oder Derivat davon nach einem der Ansprüche 1 bis 5, **dadurch gekennzeichnet, dass** die 4-Pyridinylformamidverbindung dargestellt ist durch Formel I: 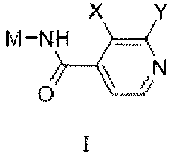 wobei X, Y und M wie folgt aufgelistet sind:

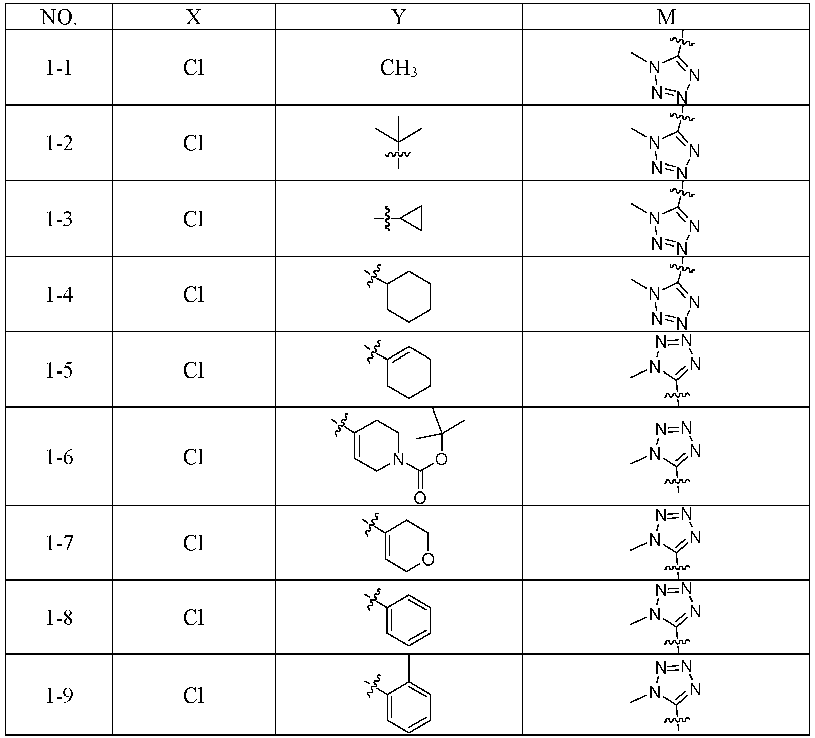

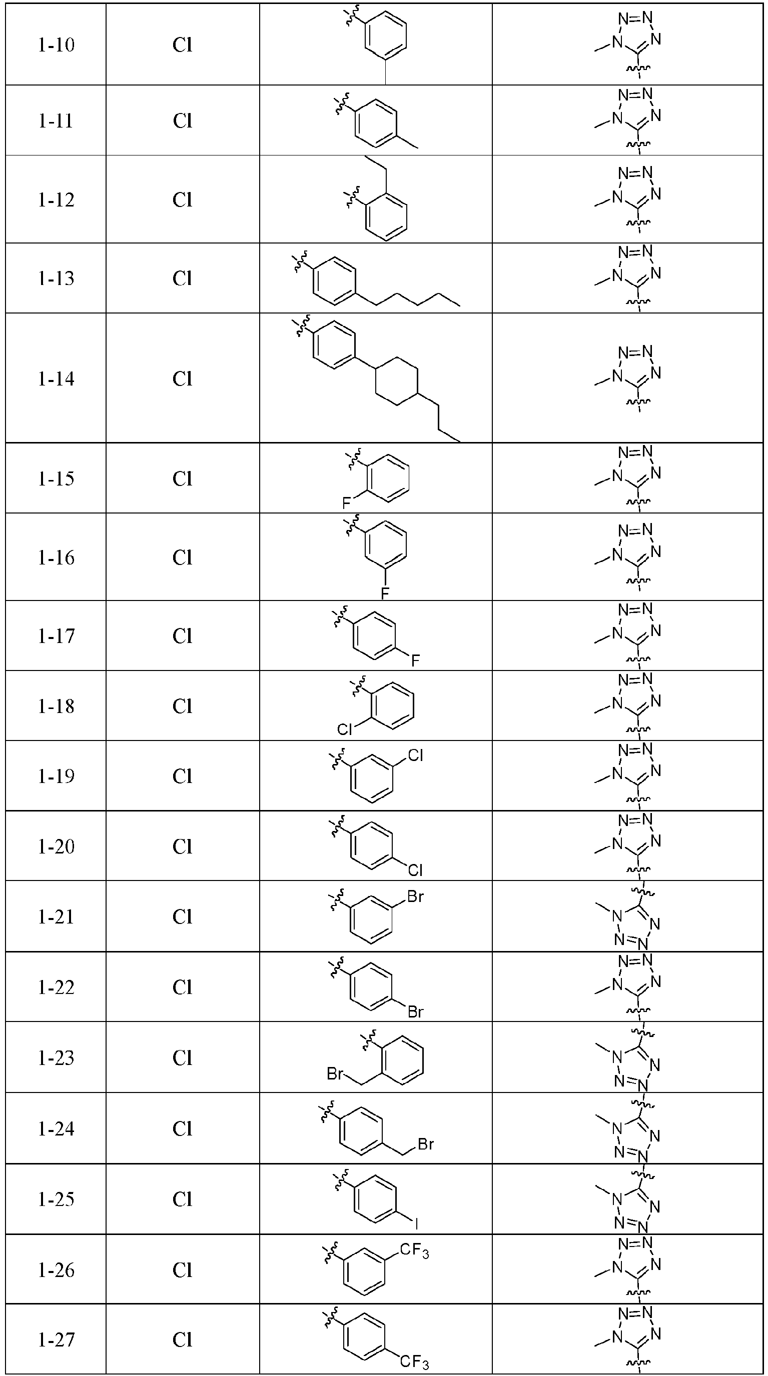

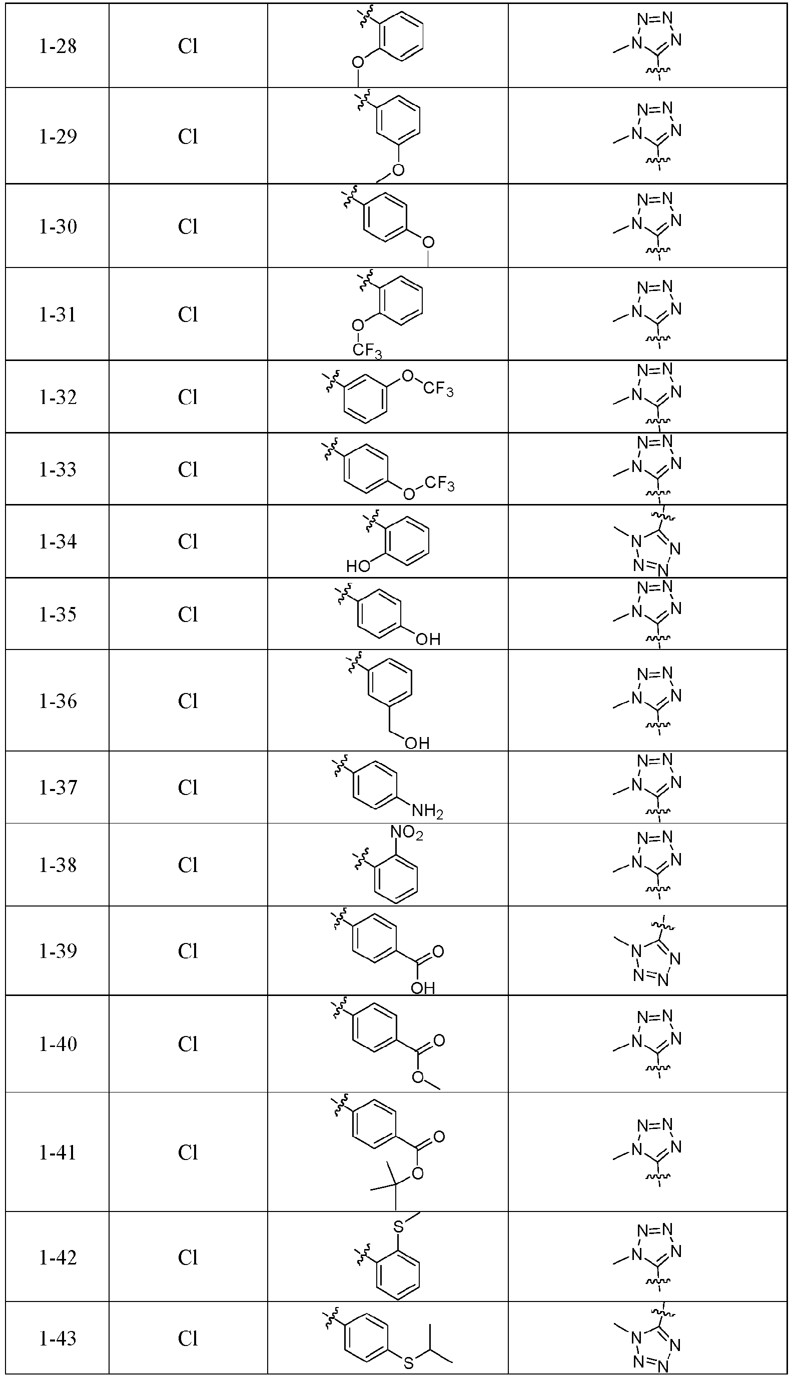

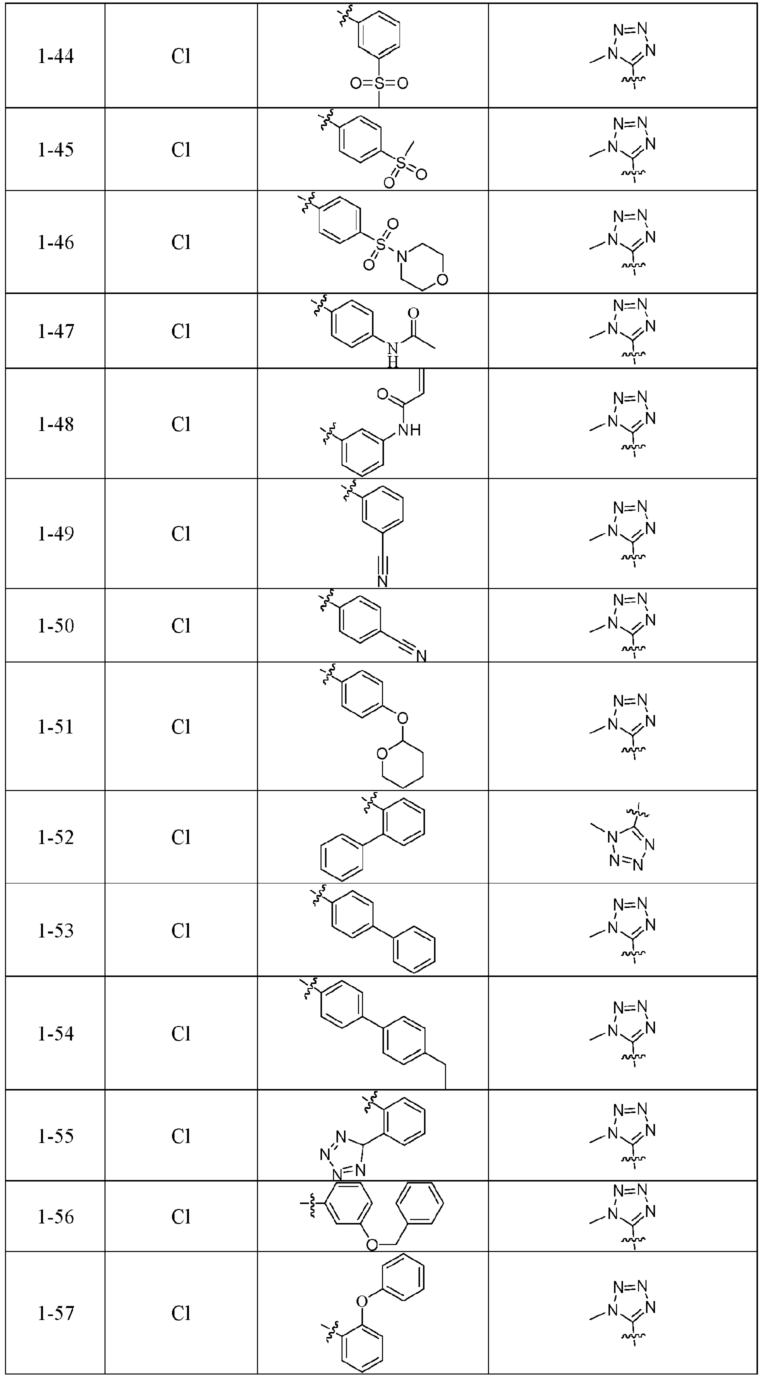

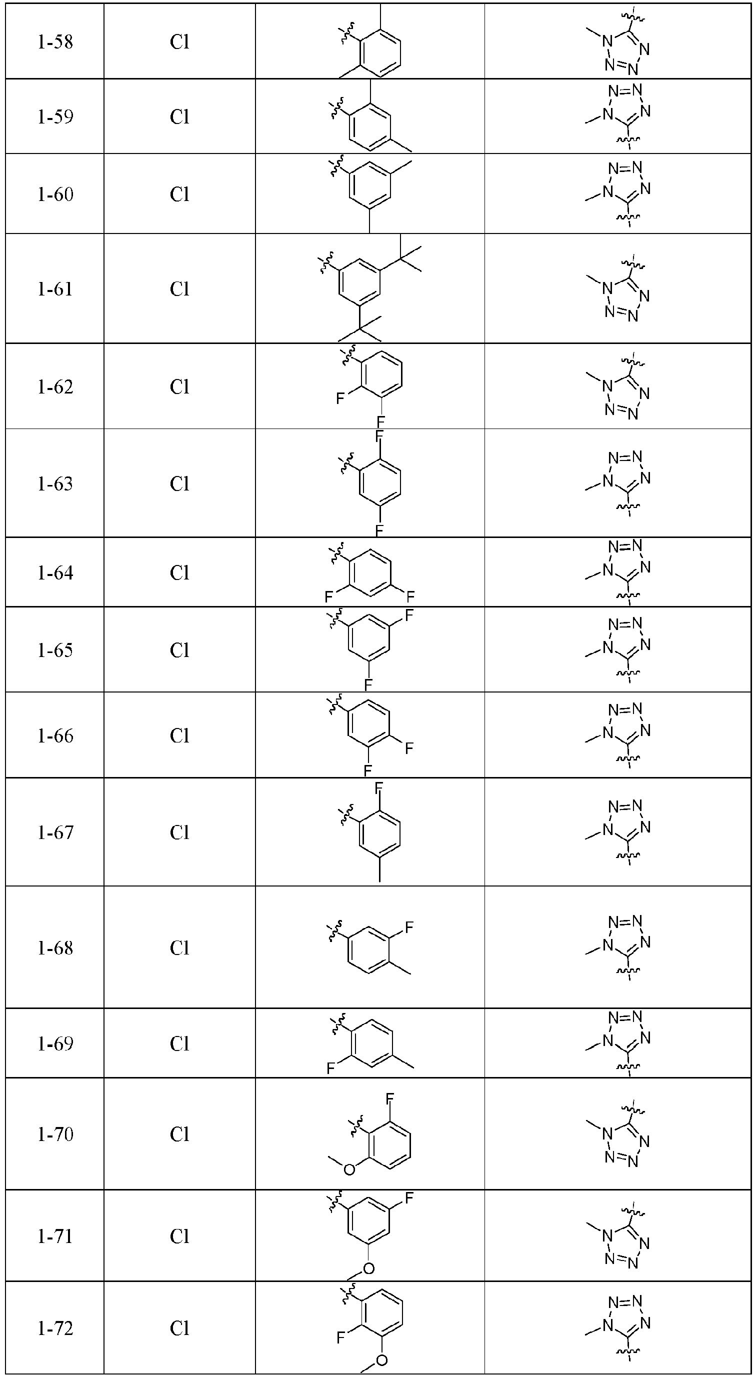

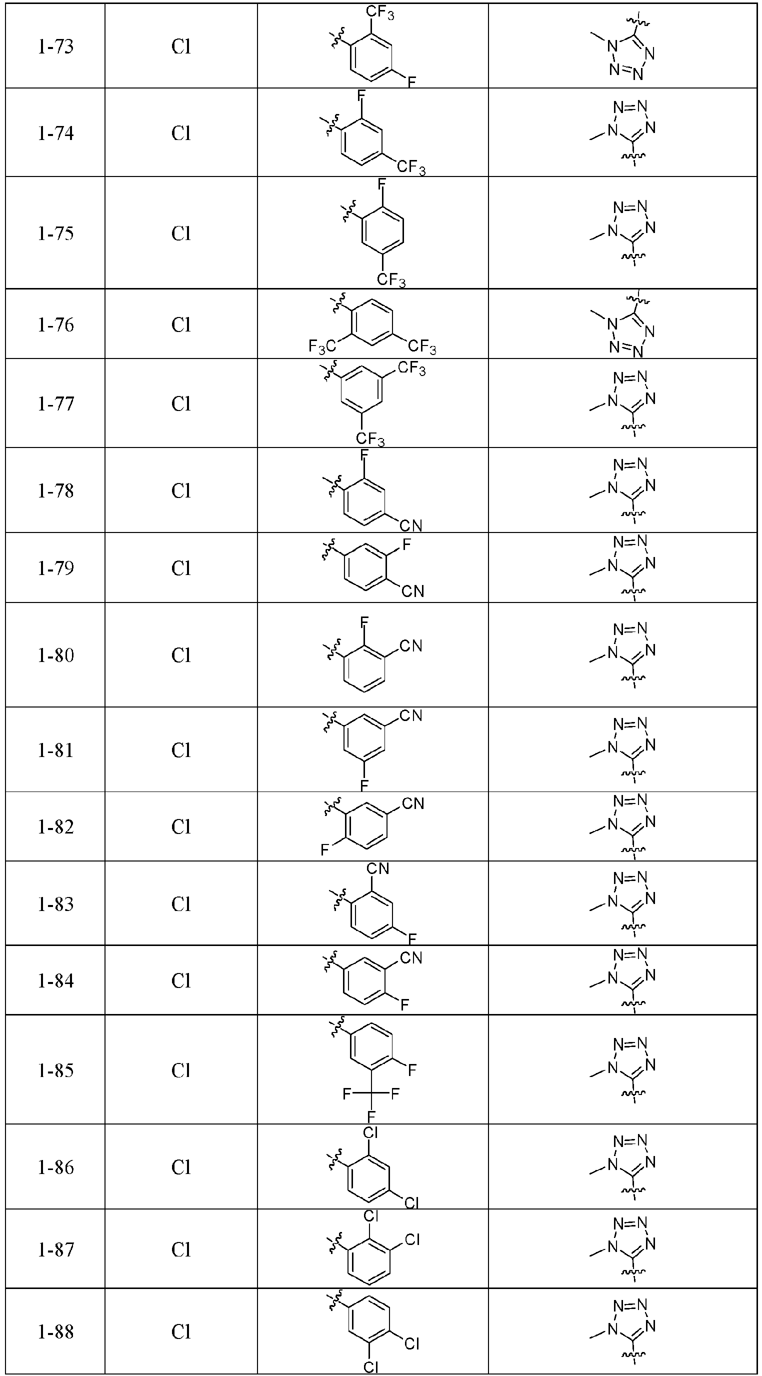

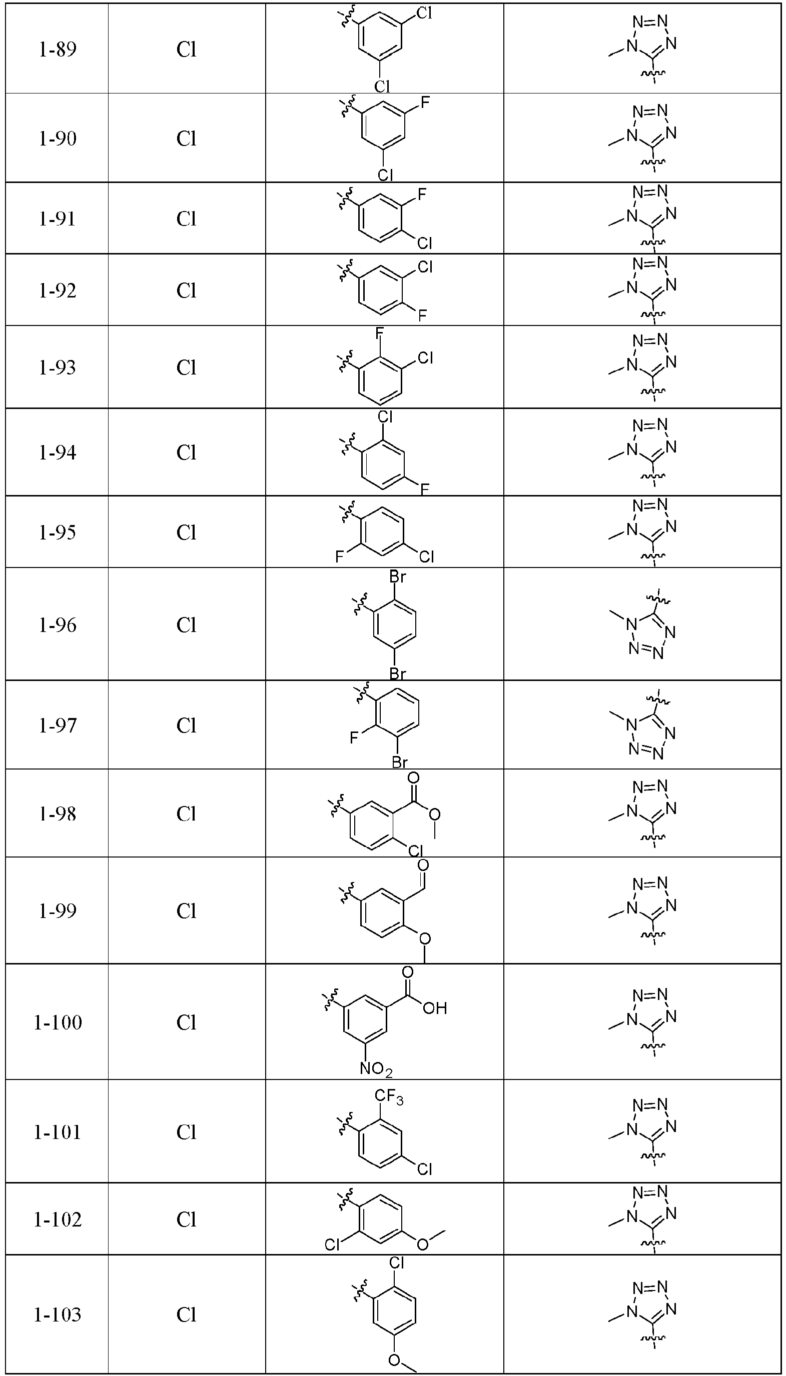

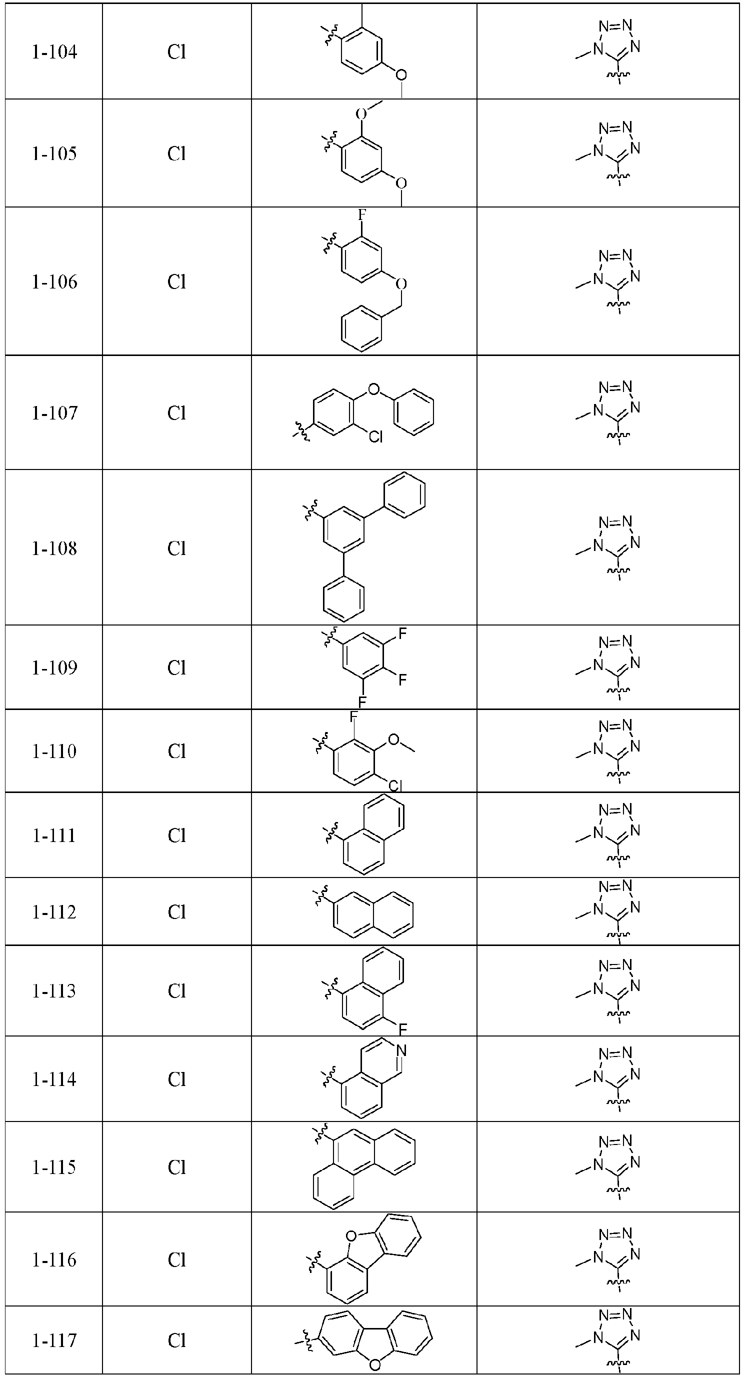

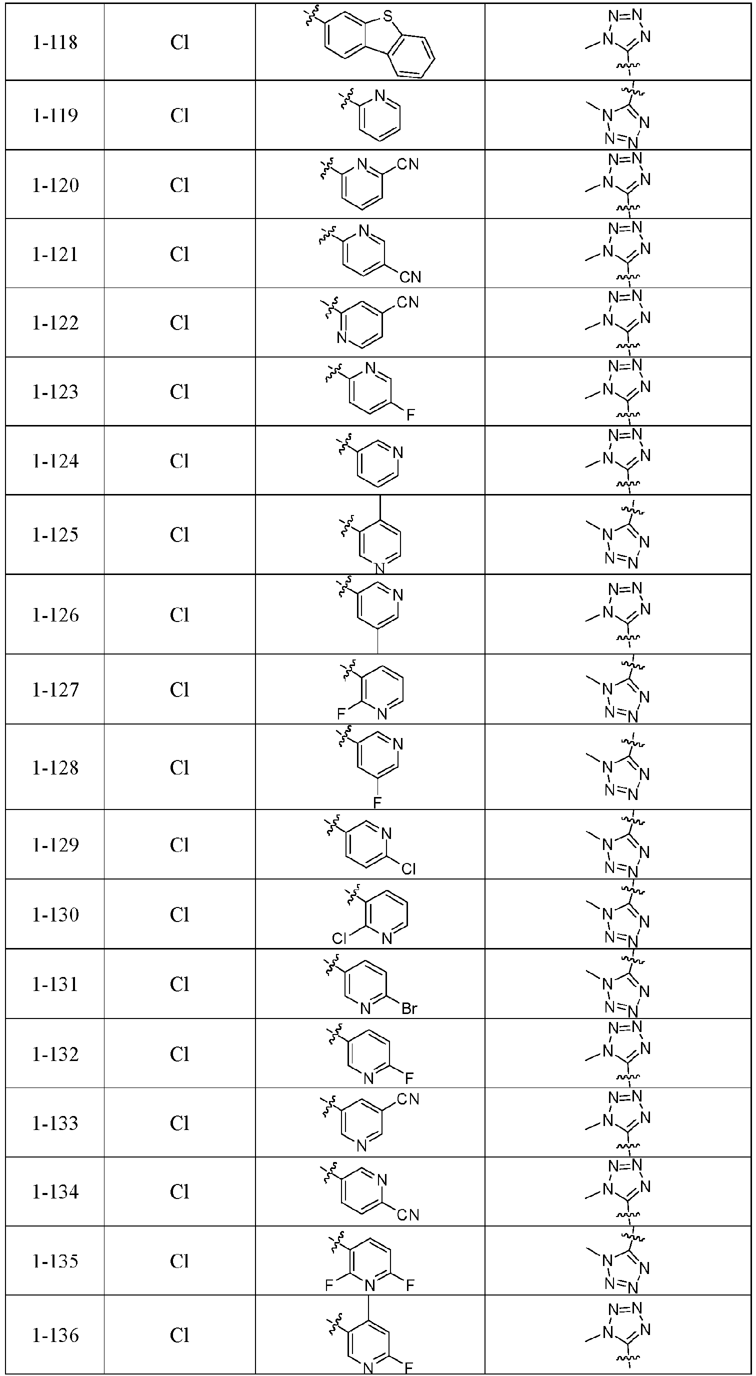

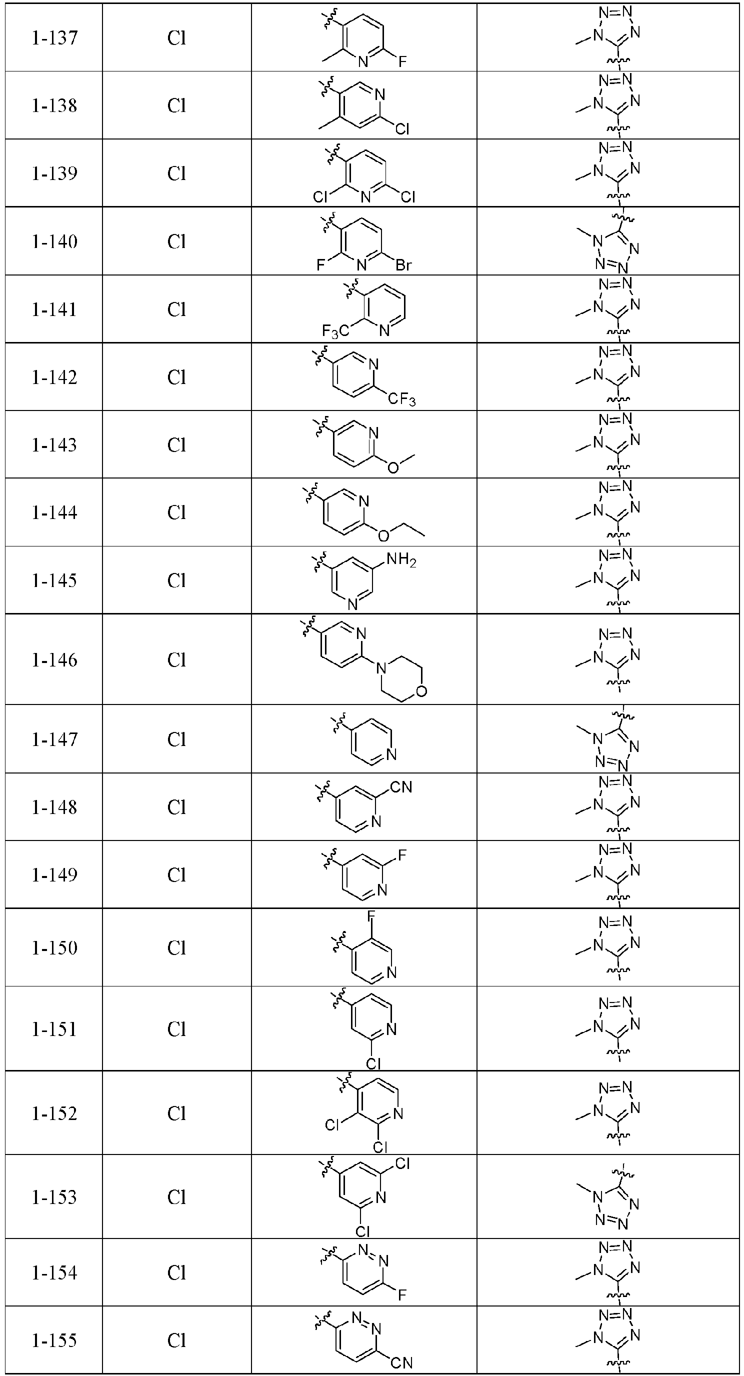

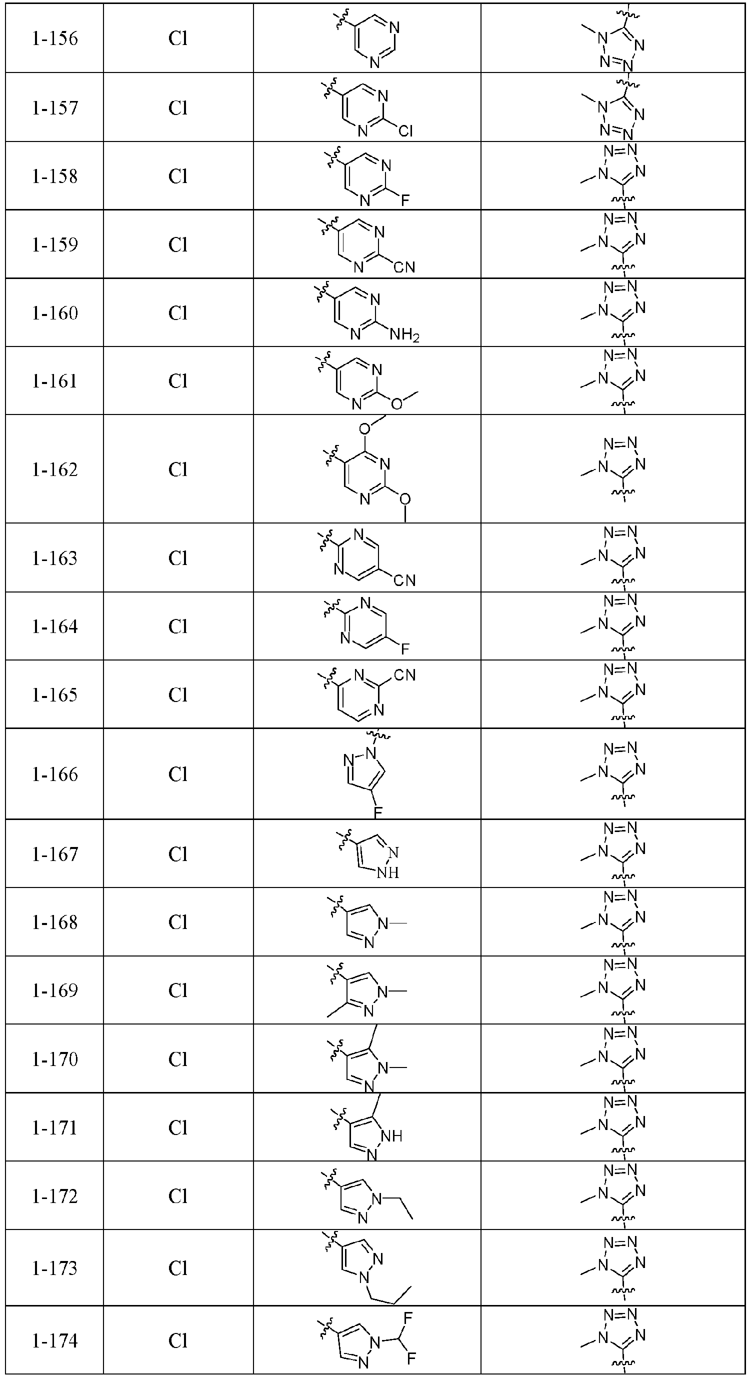

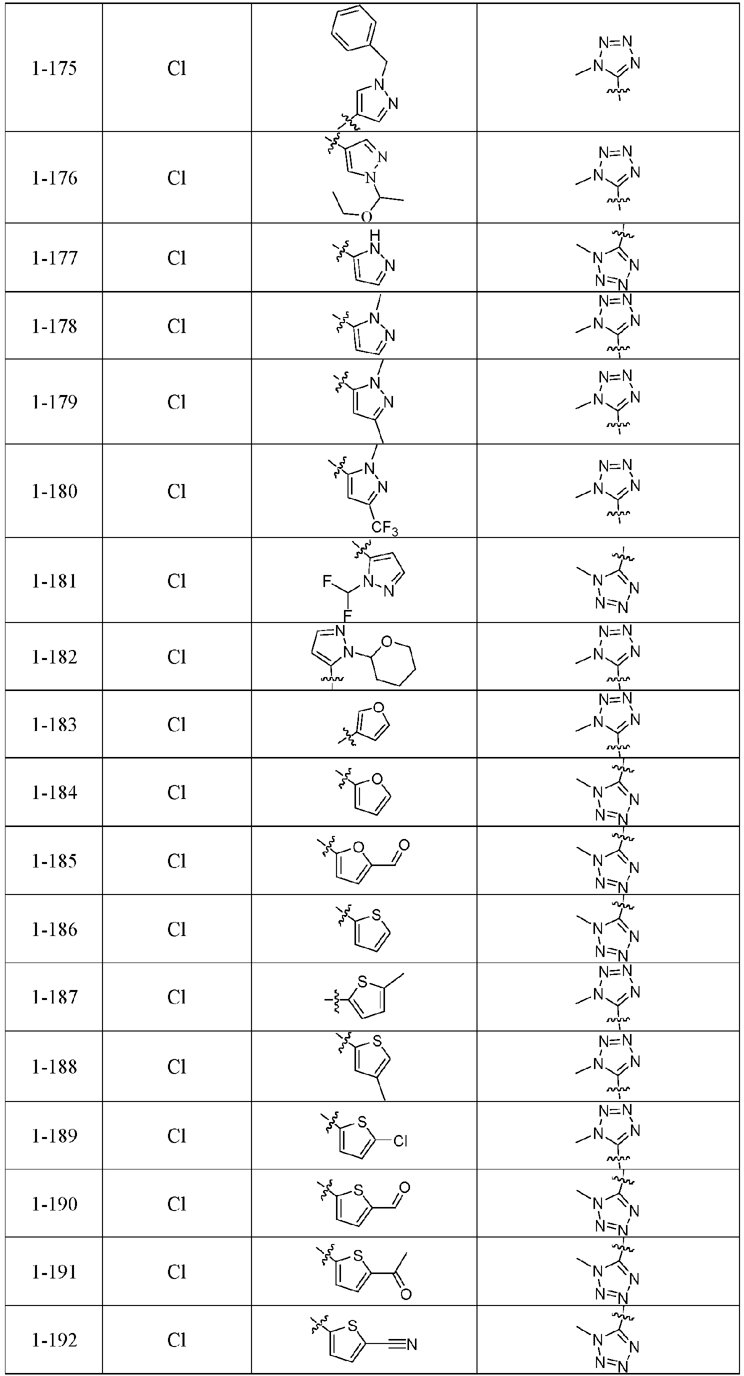

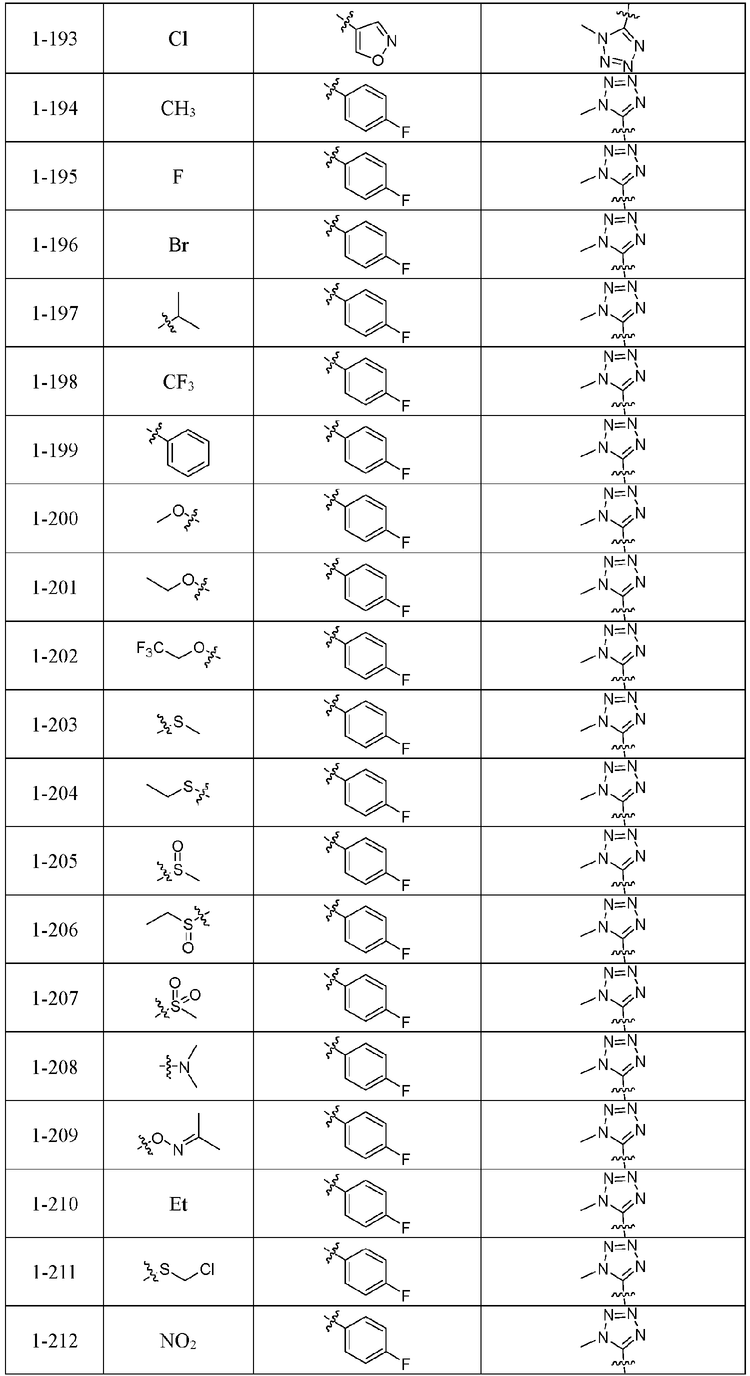

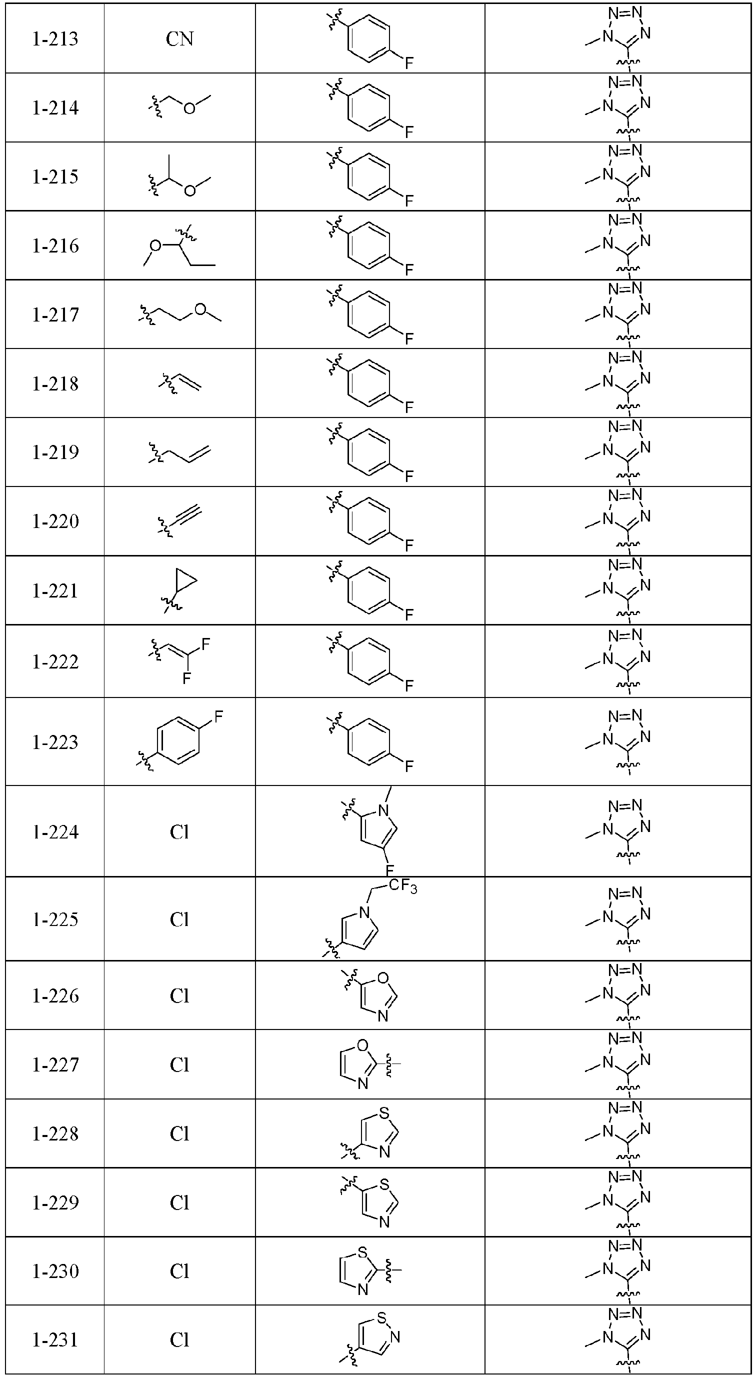

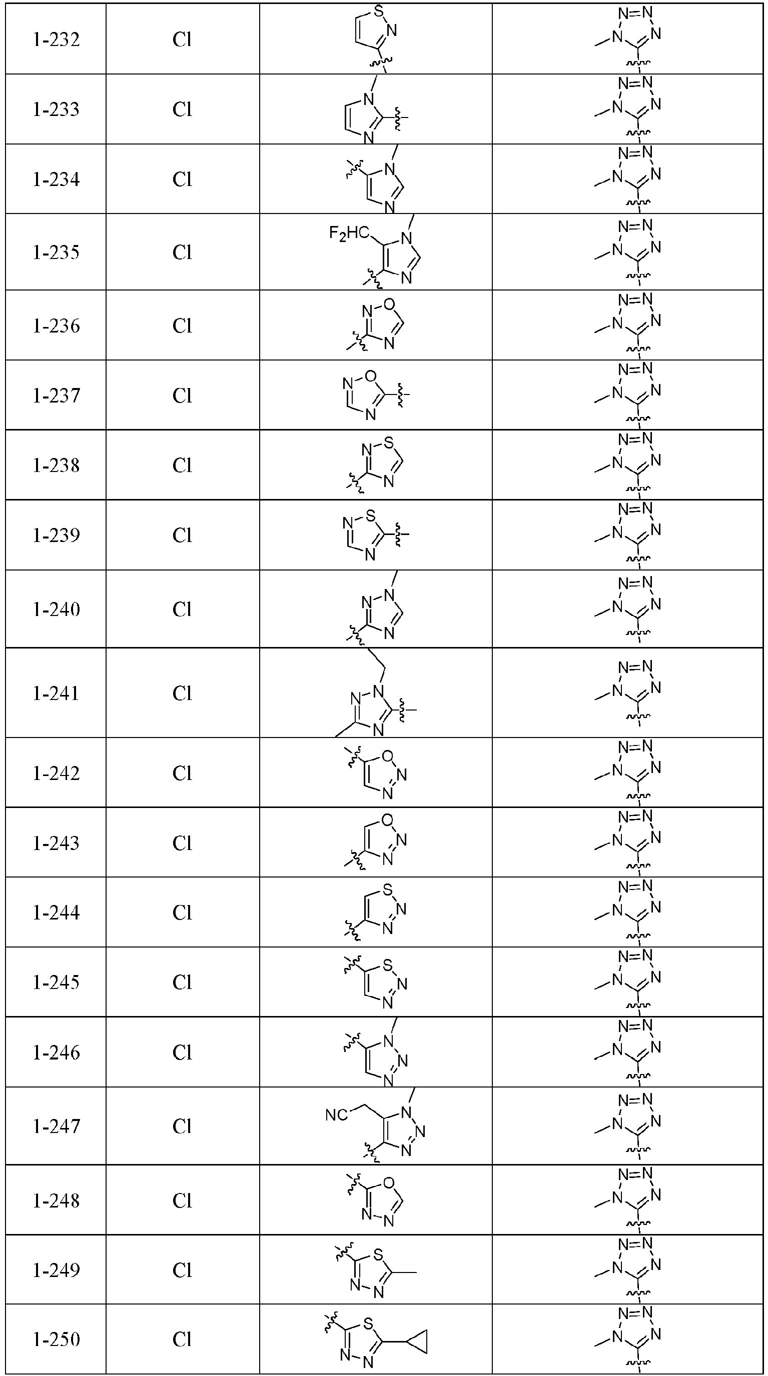

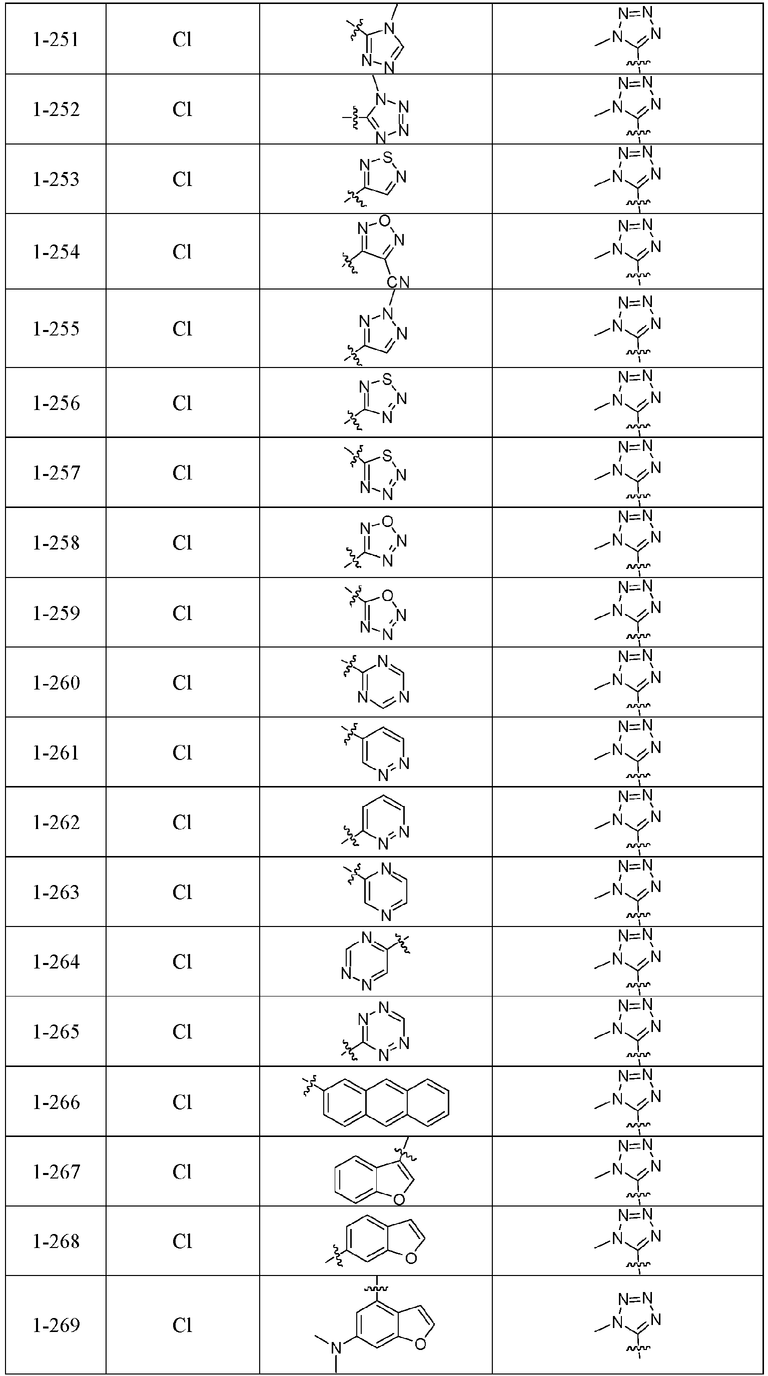

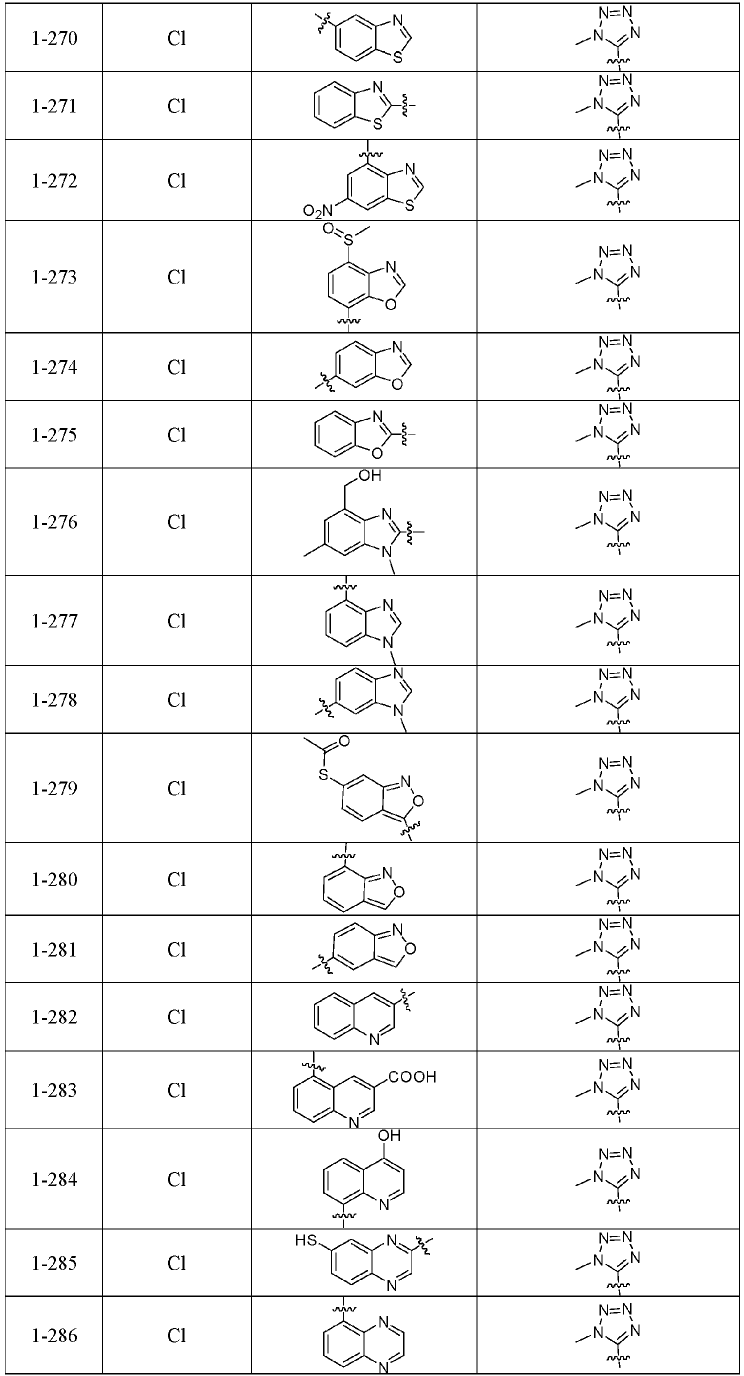

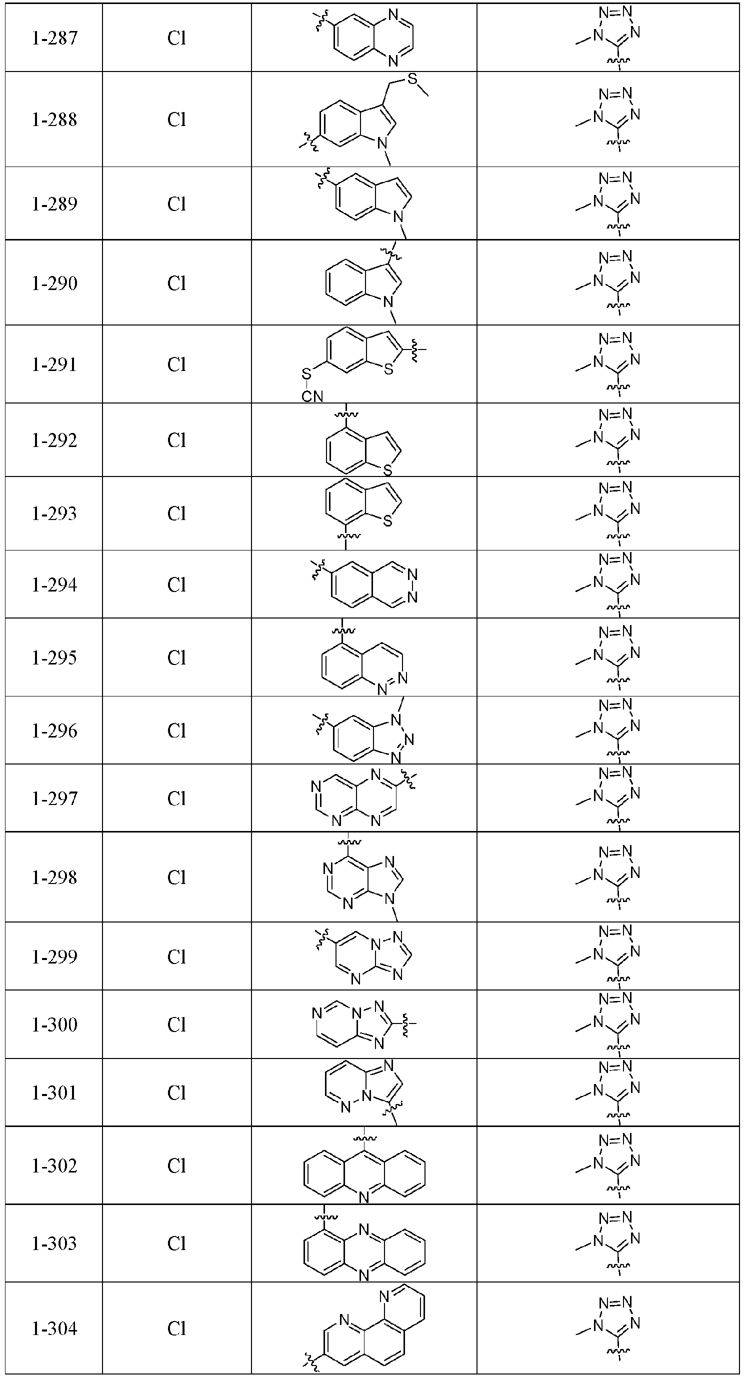

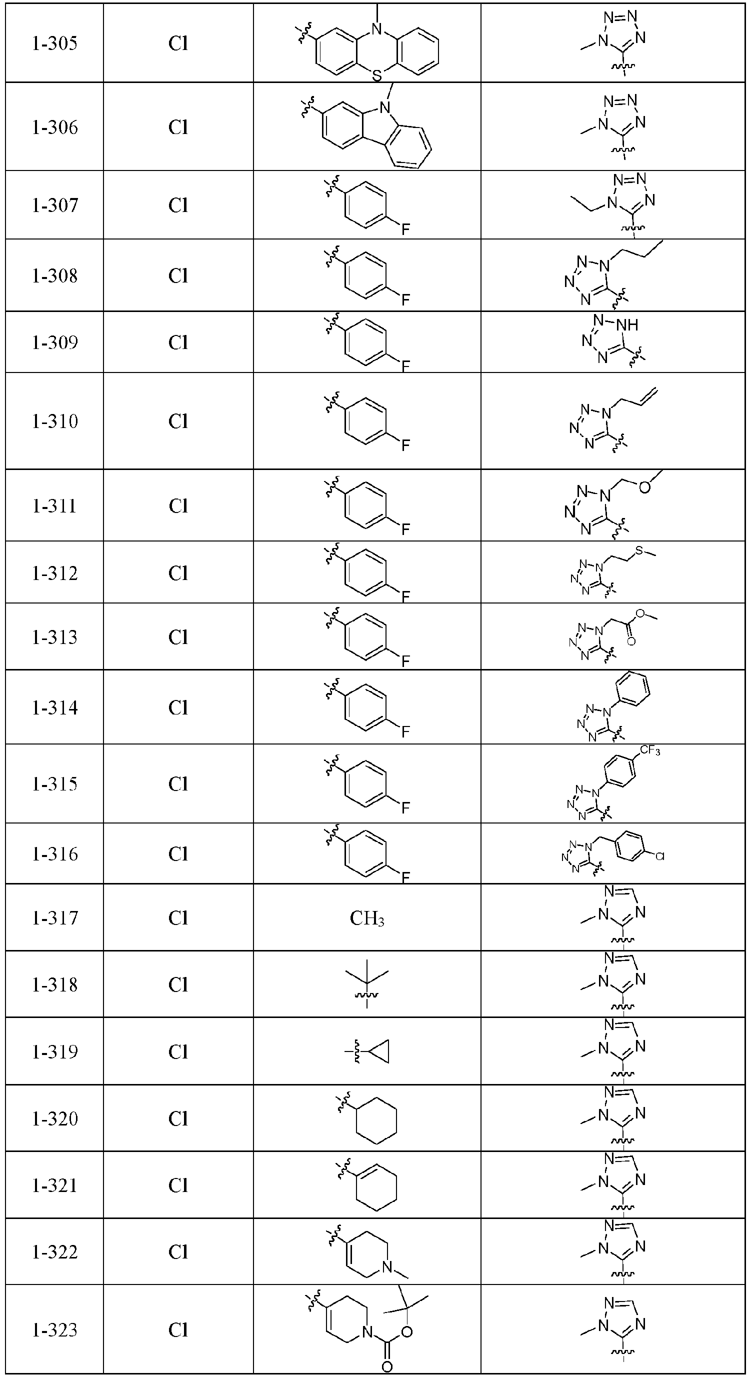

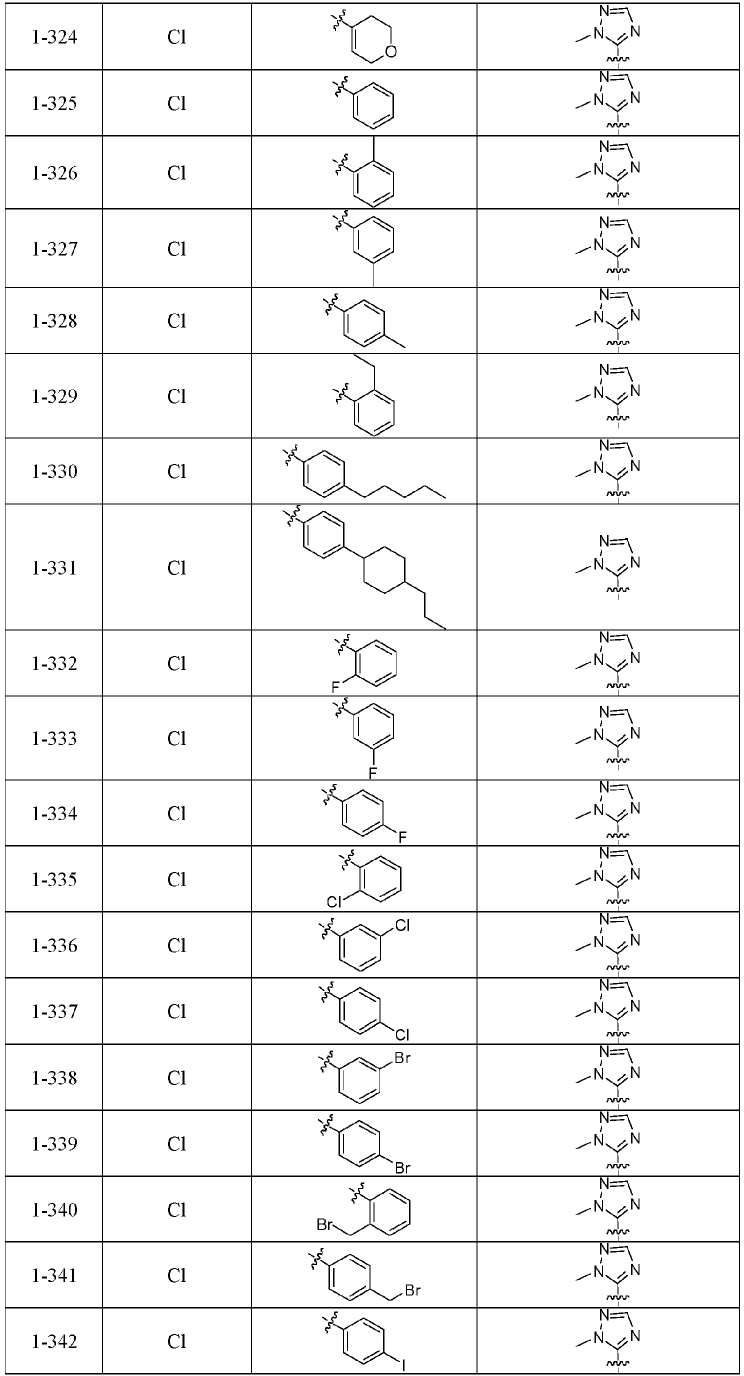

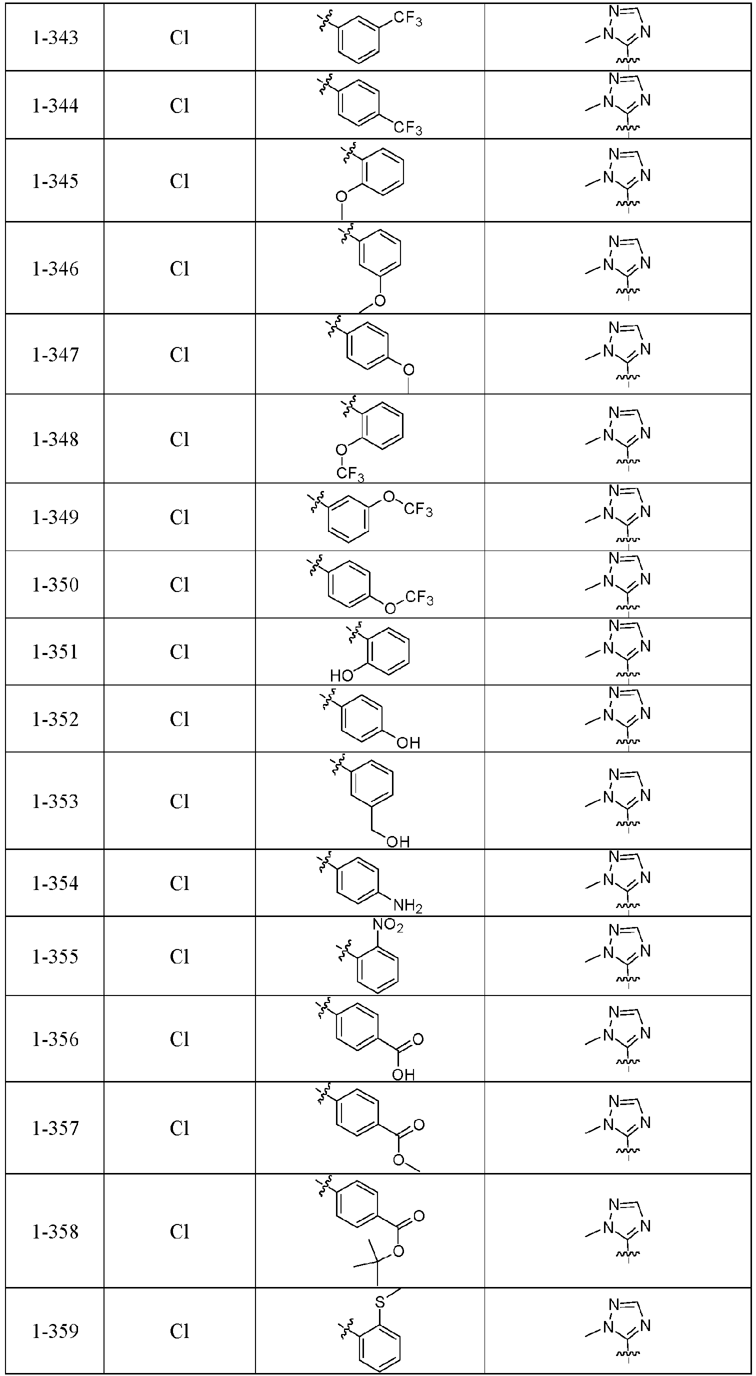

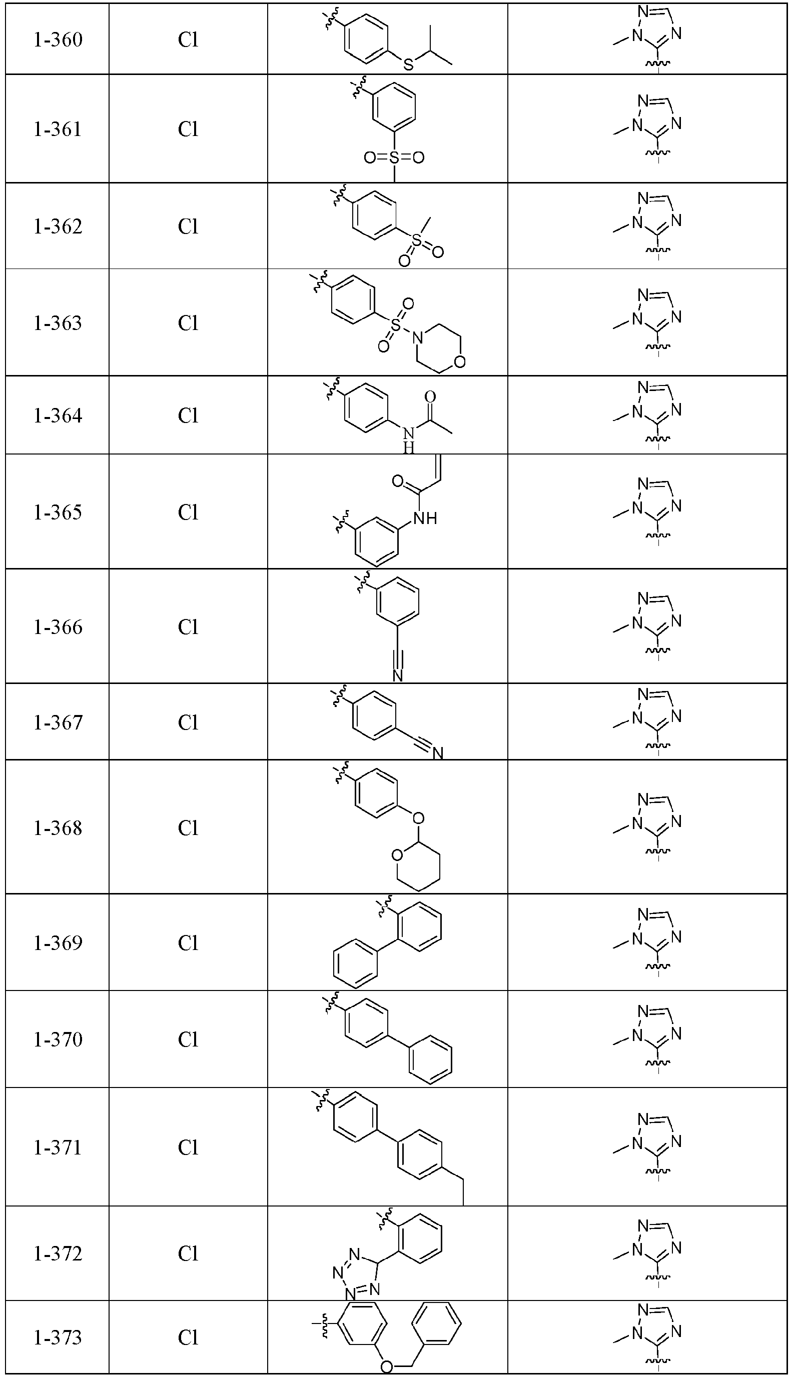

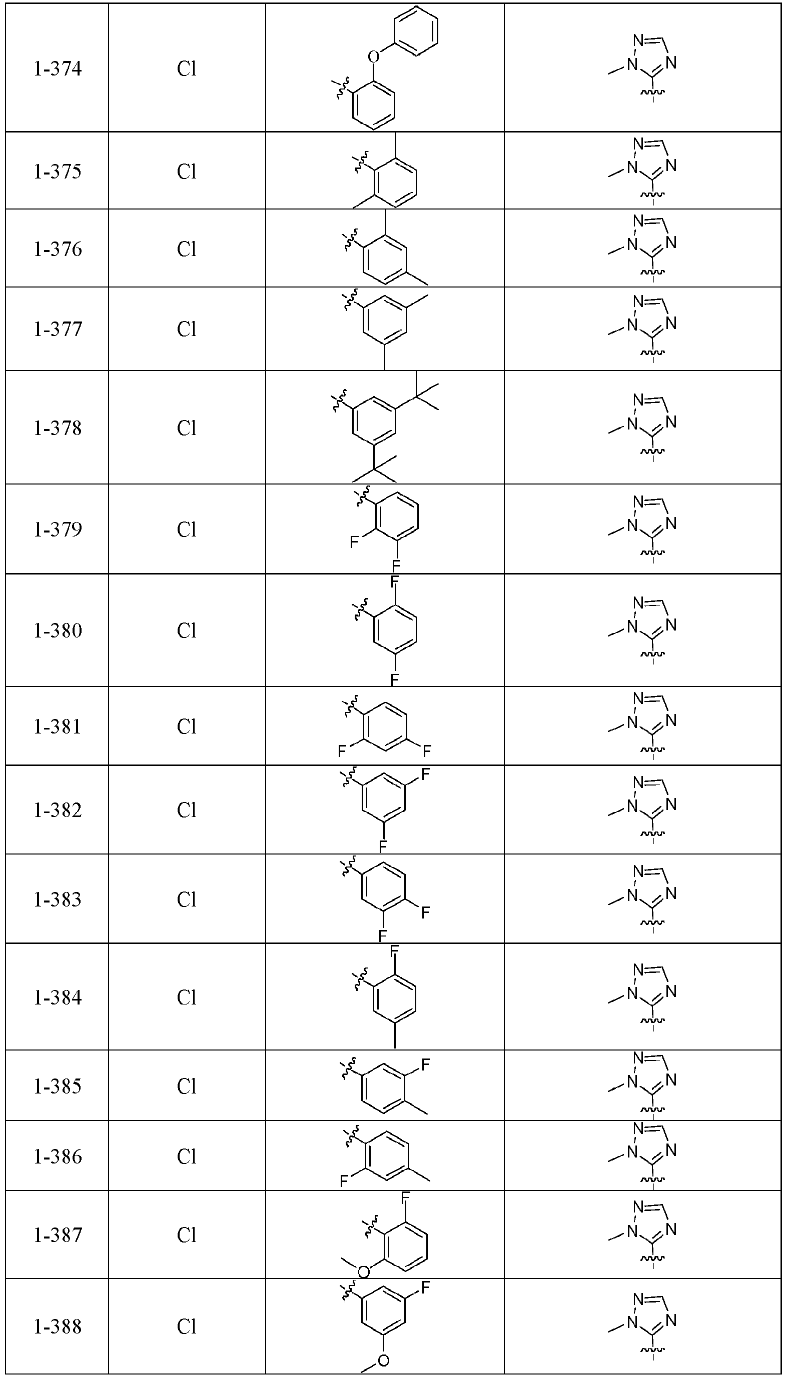

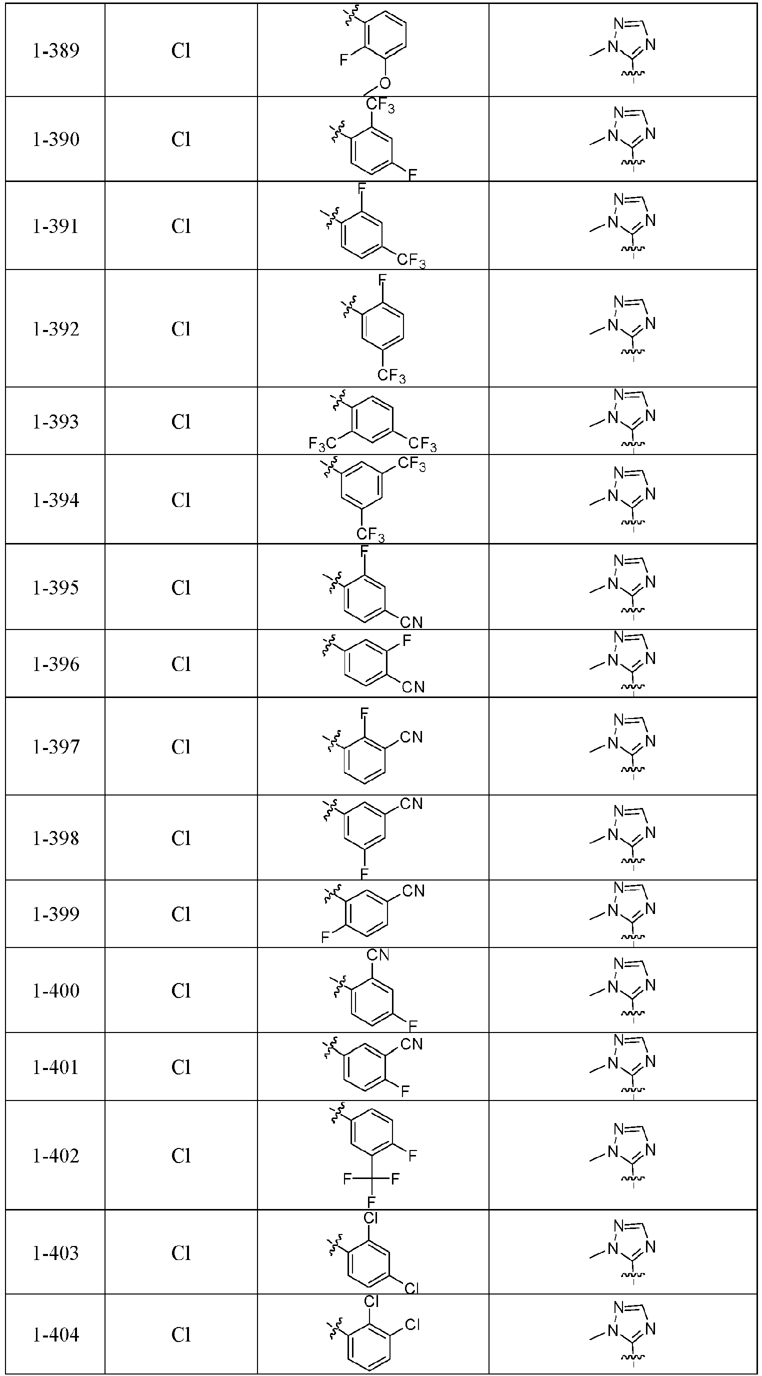

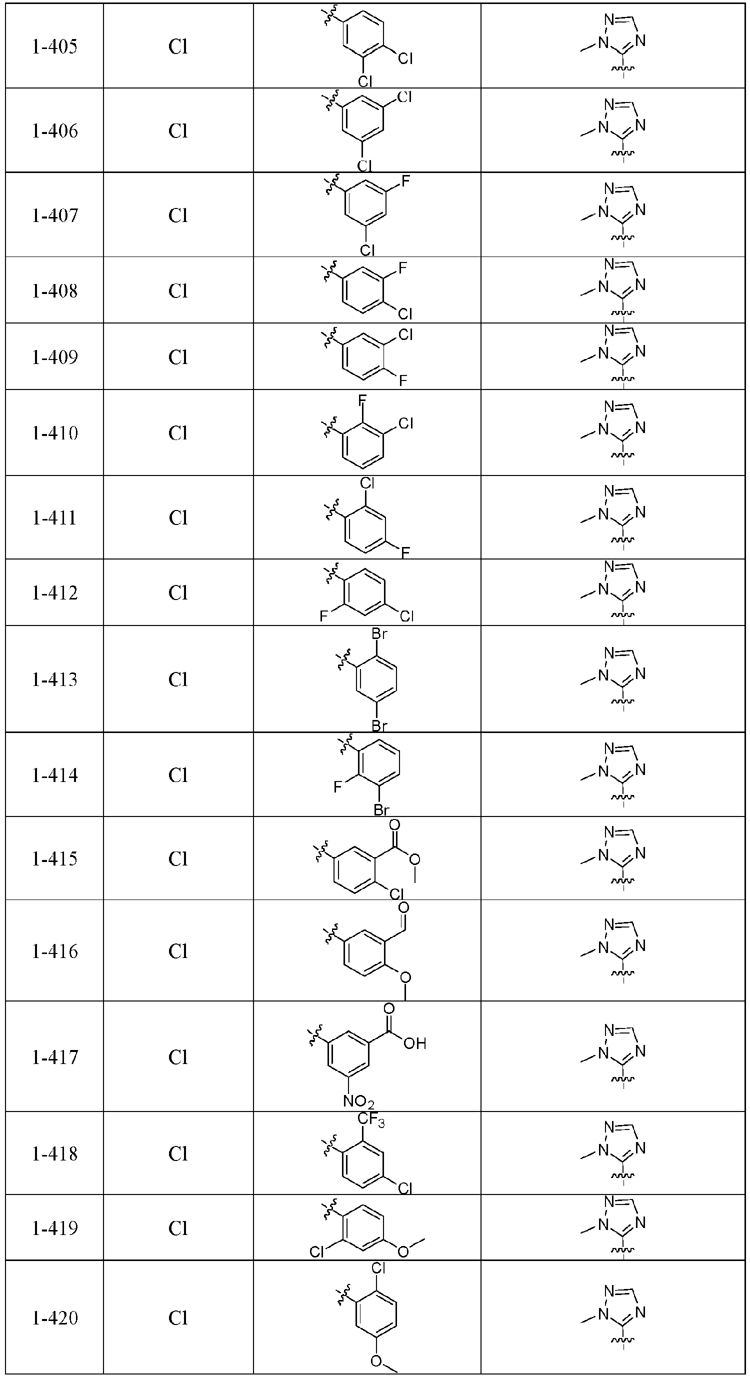

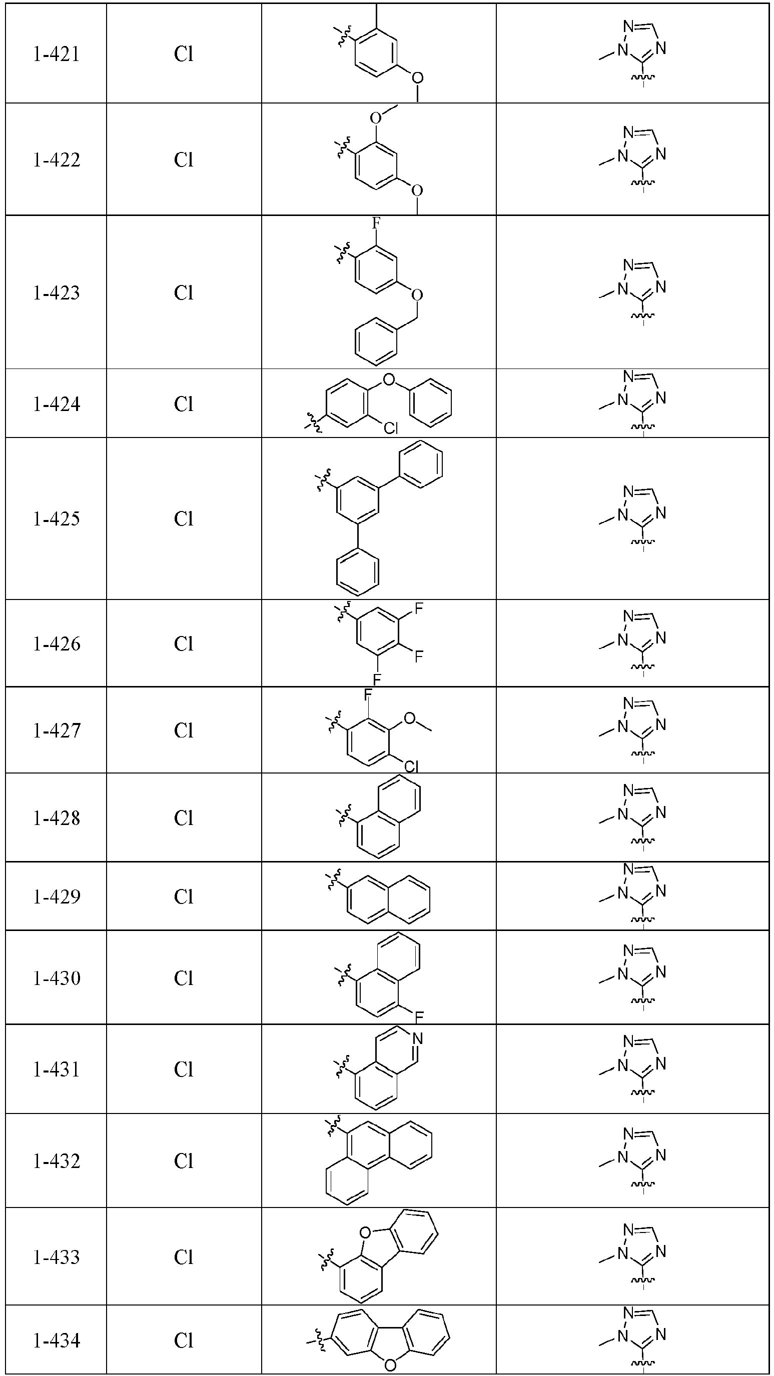

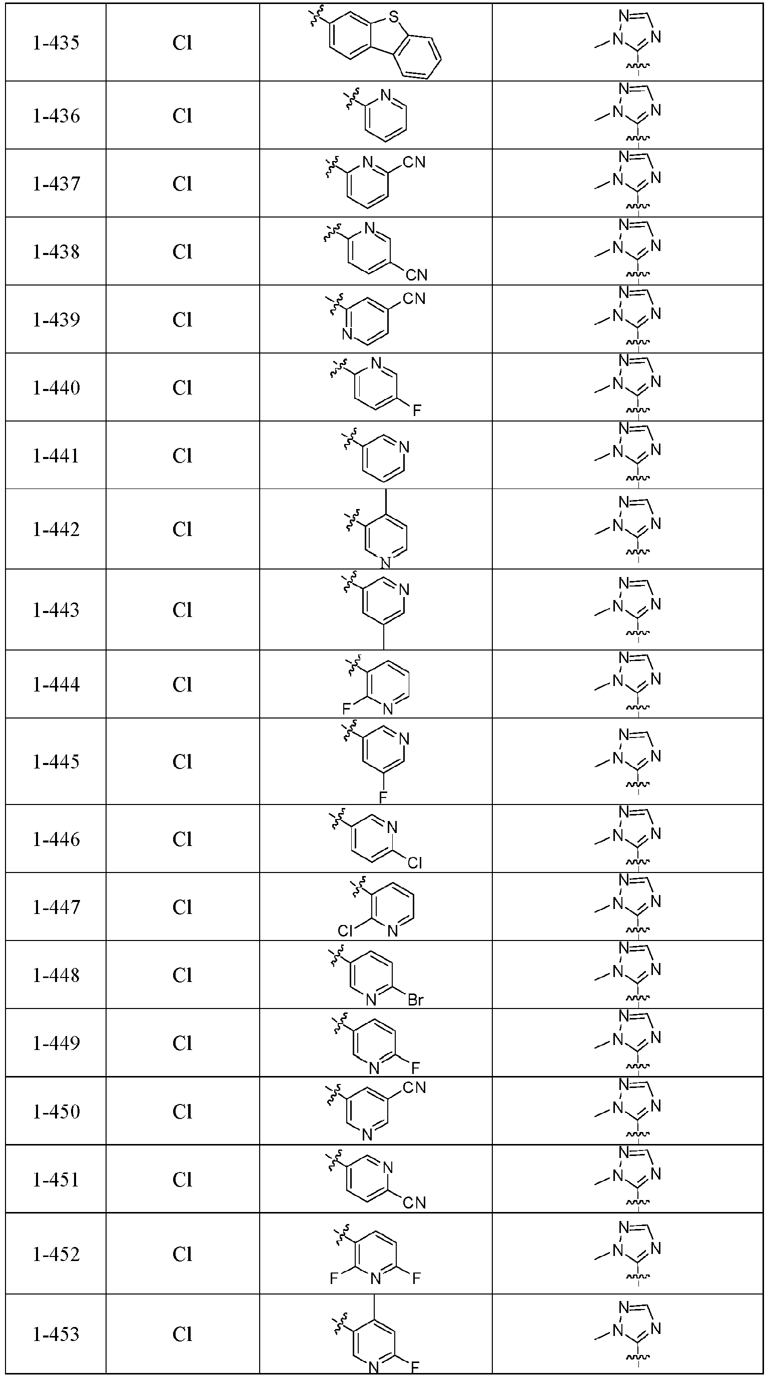

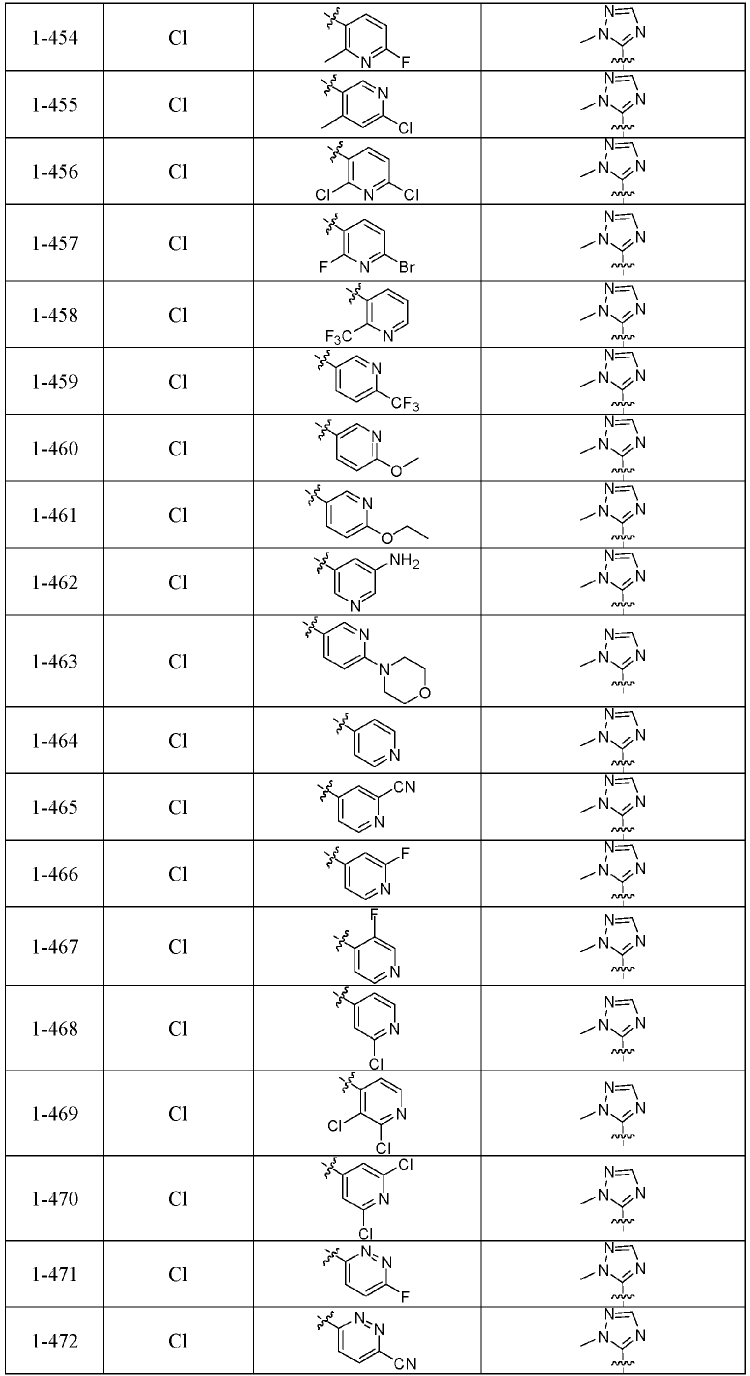

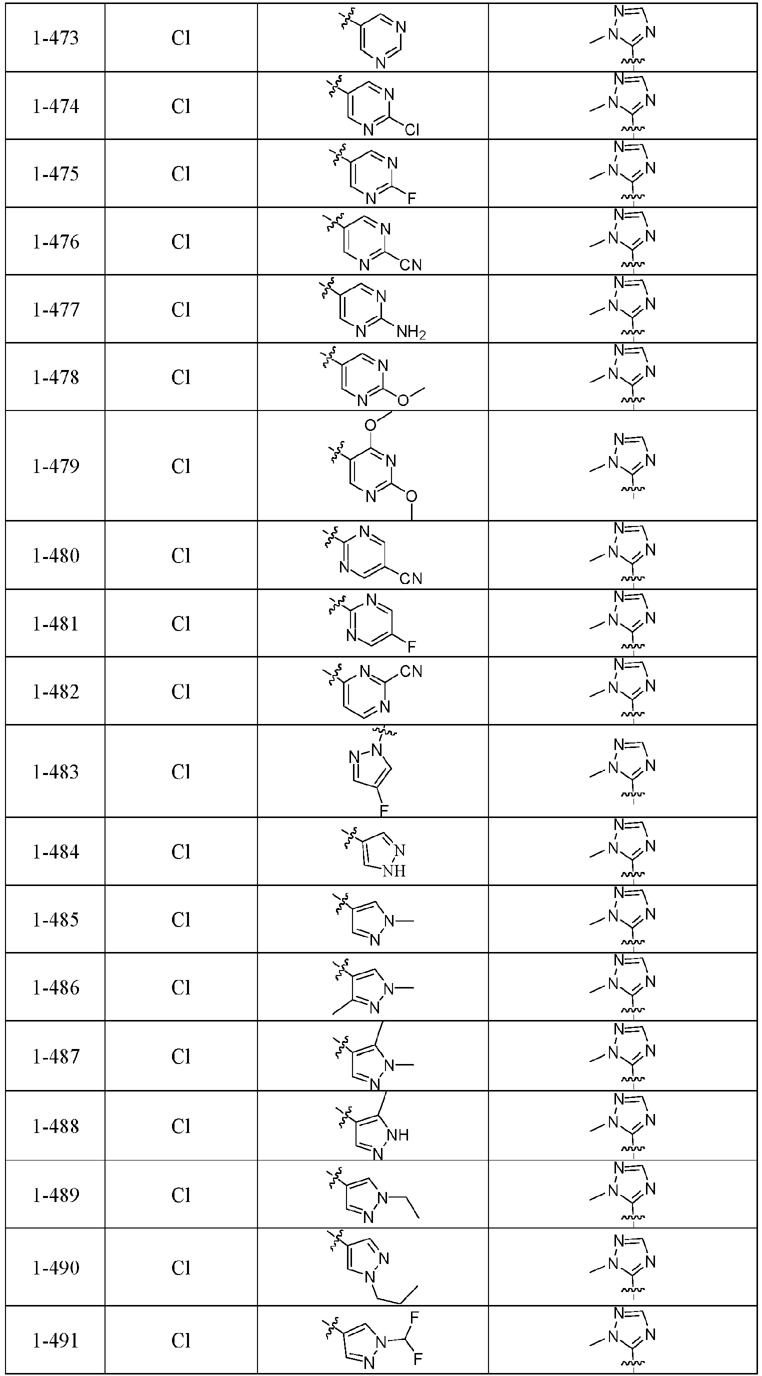

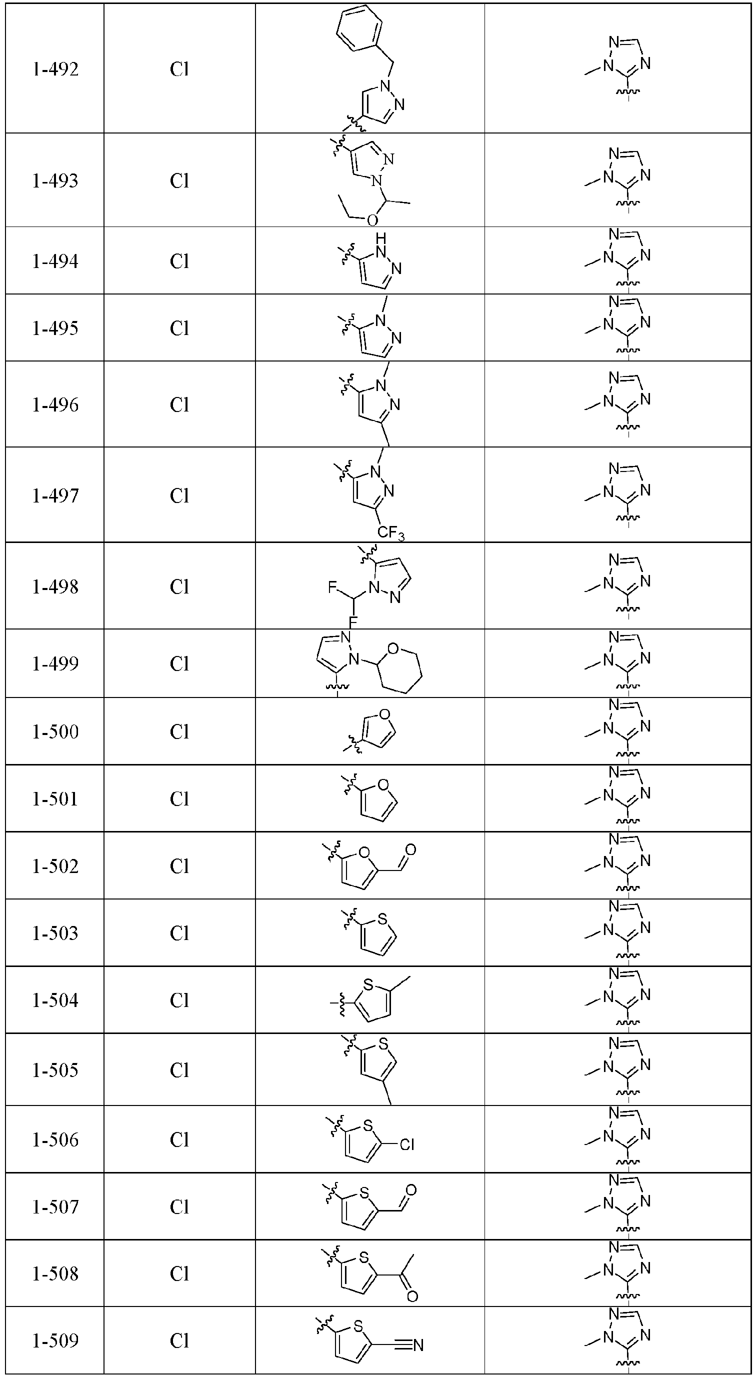

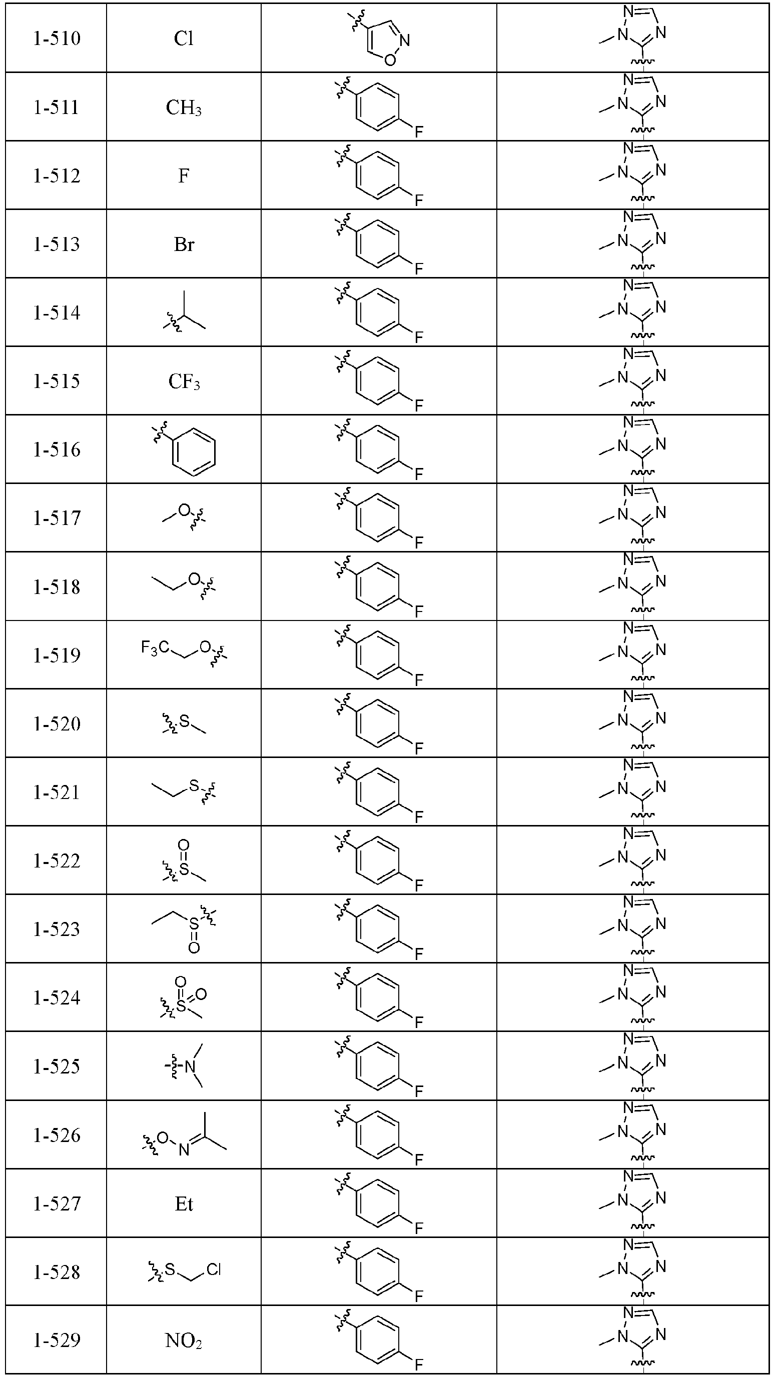

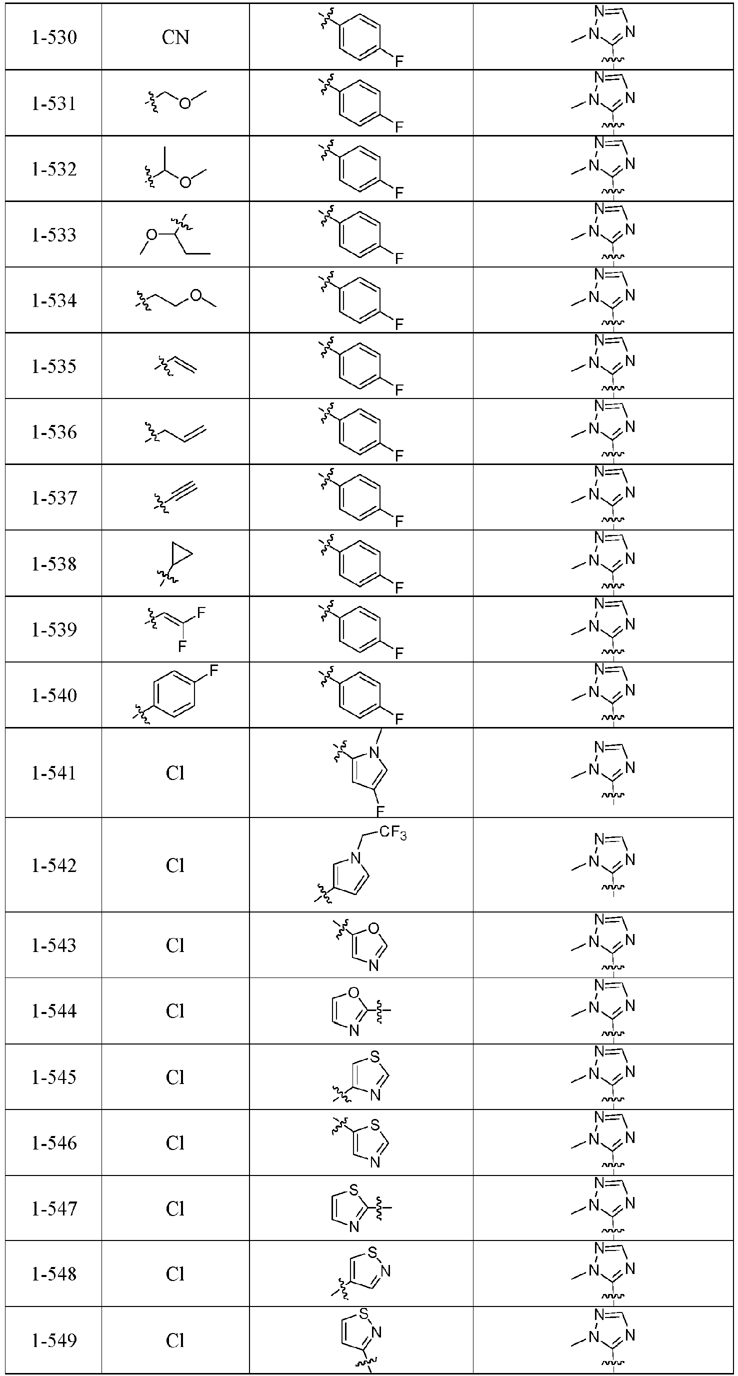

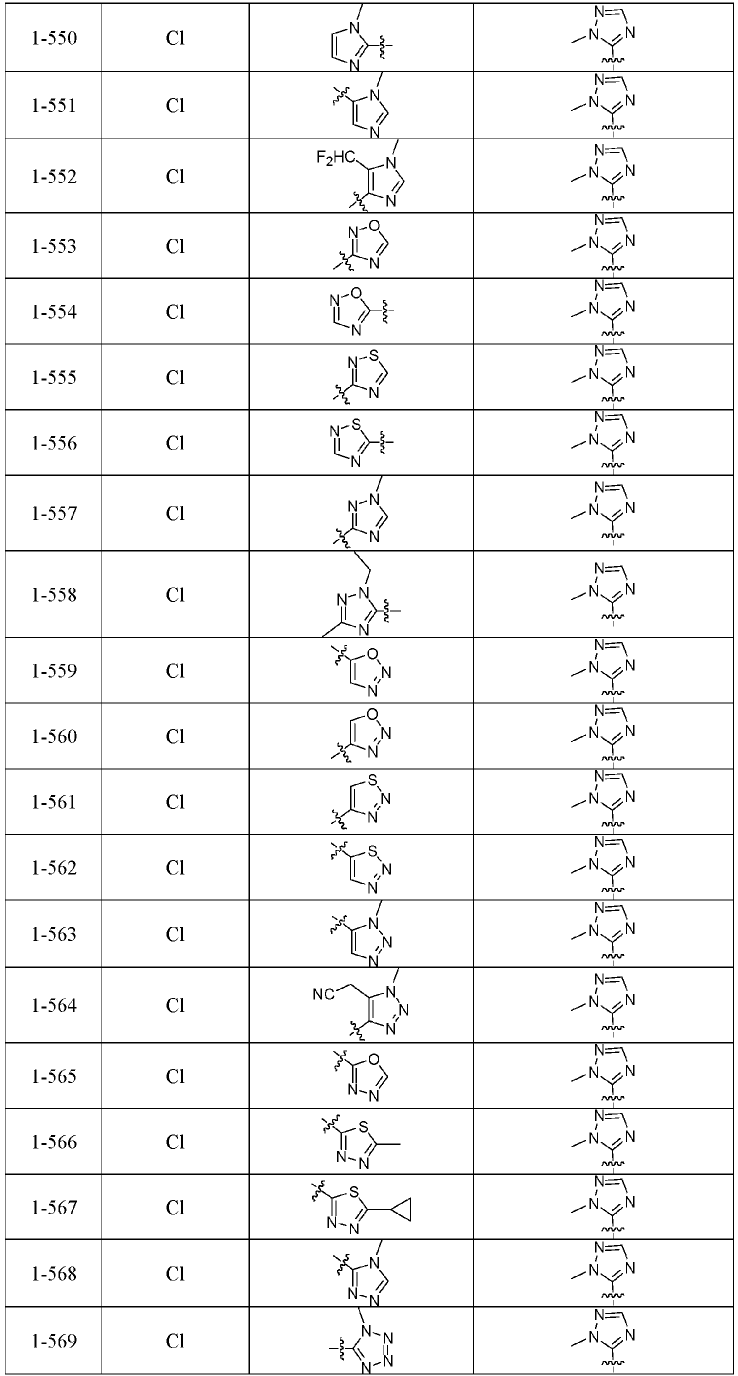

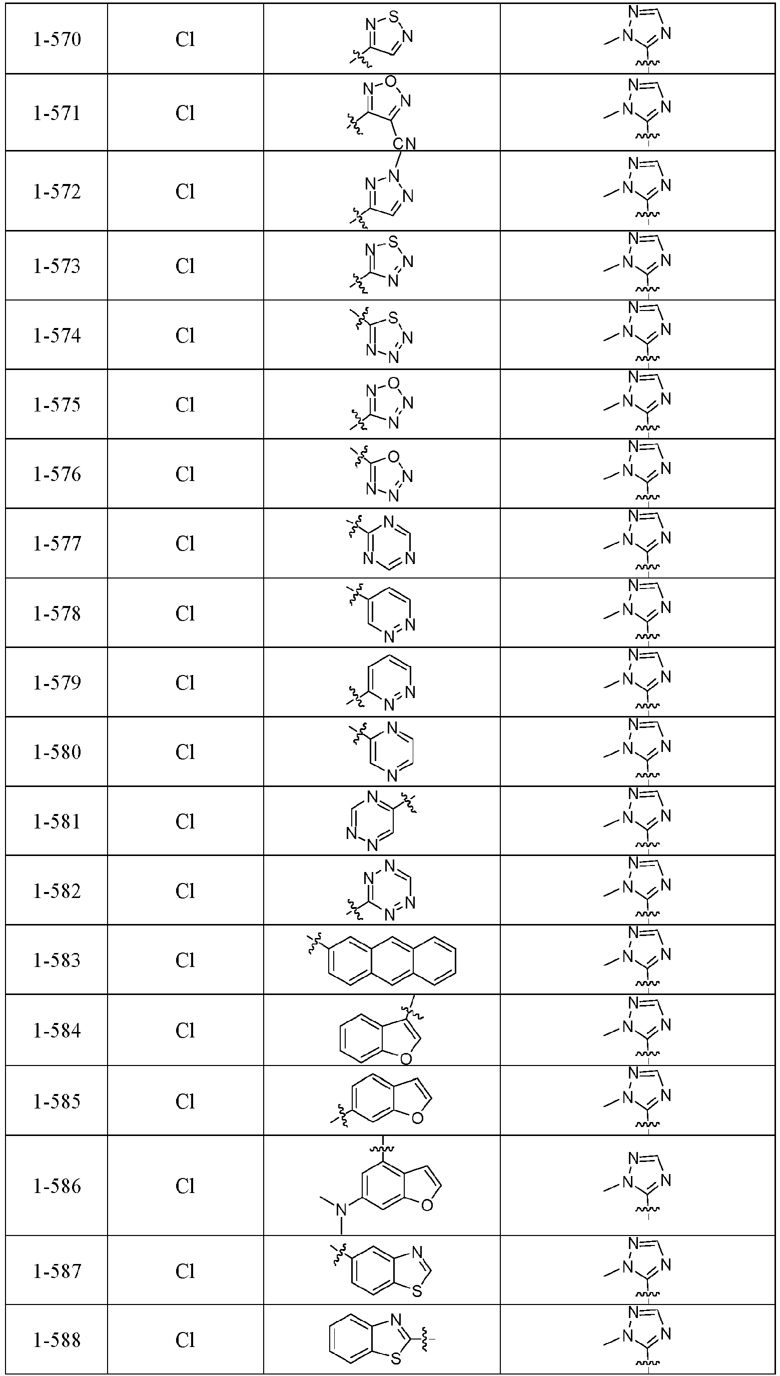

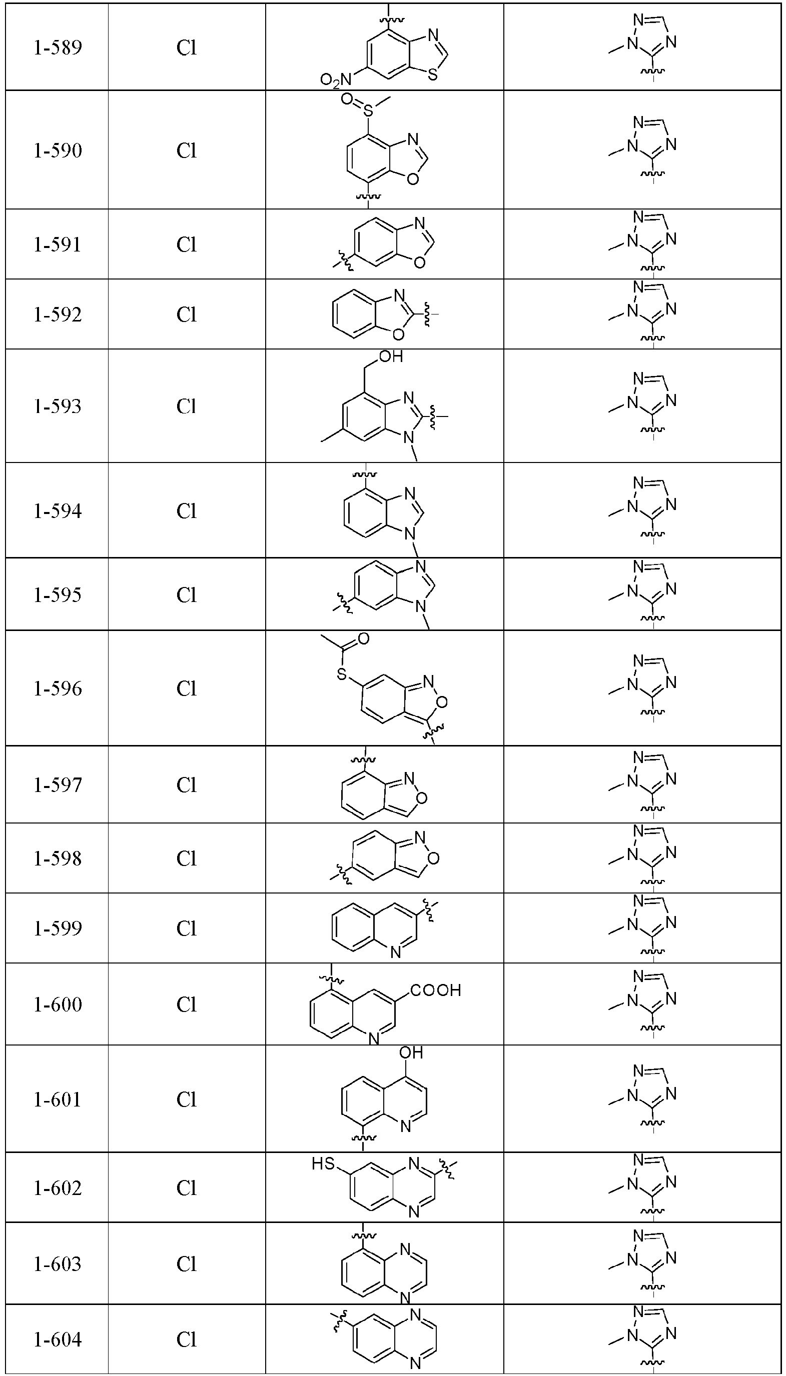

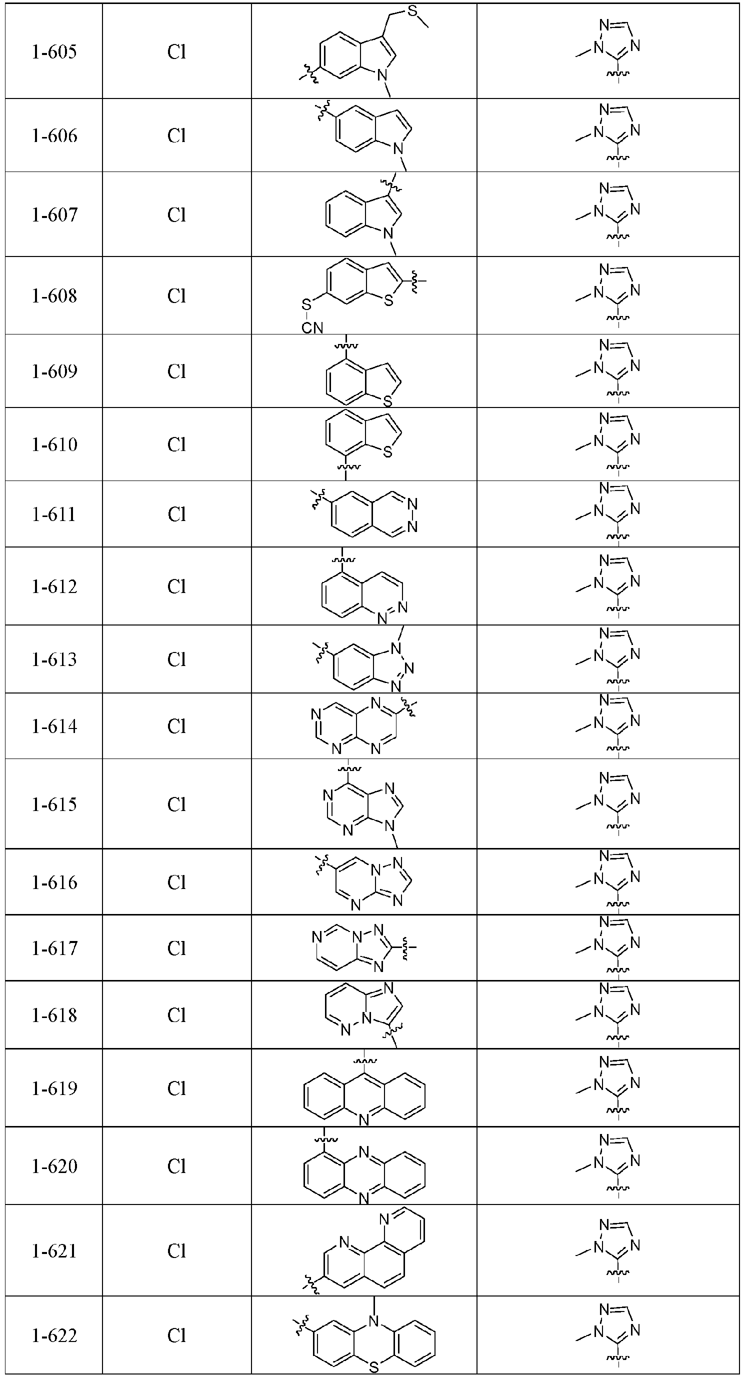

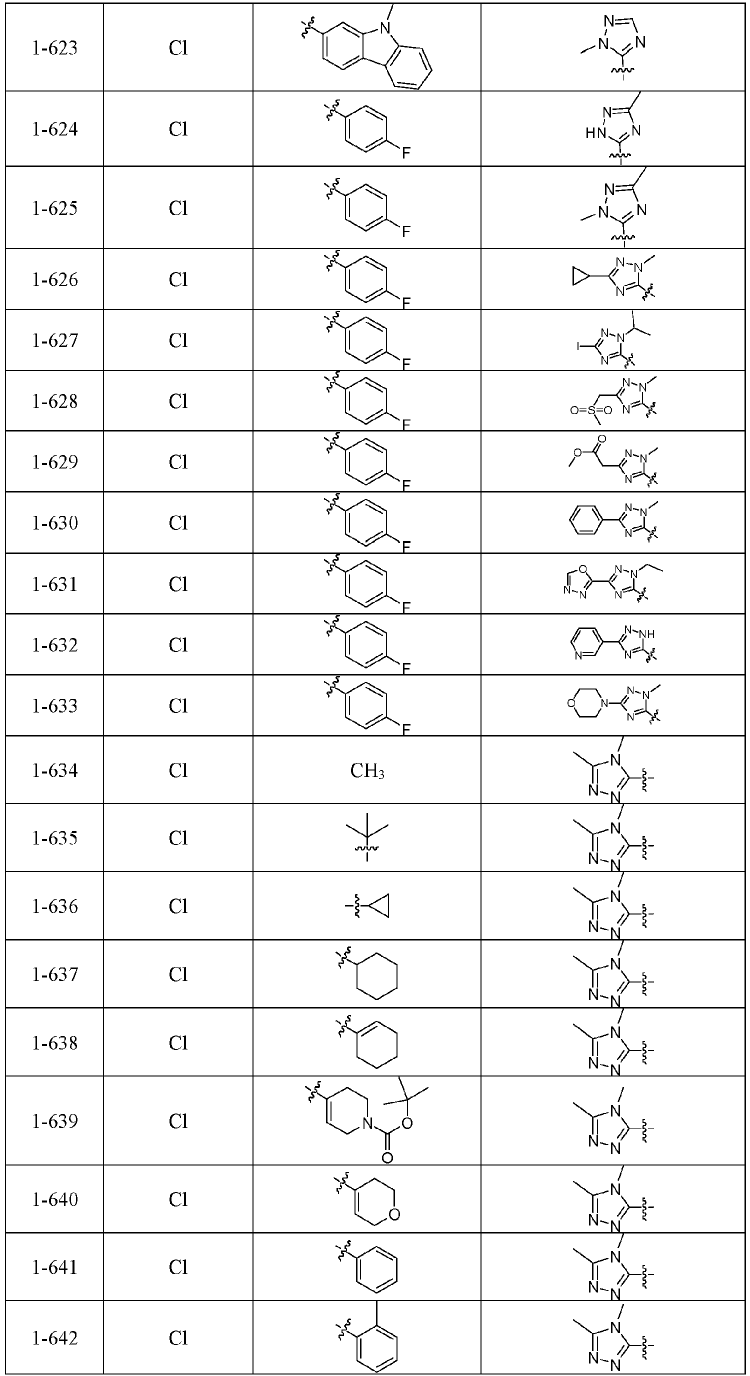

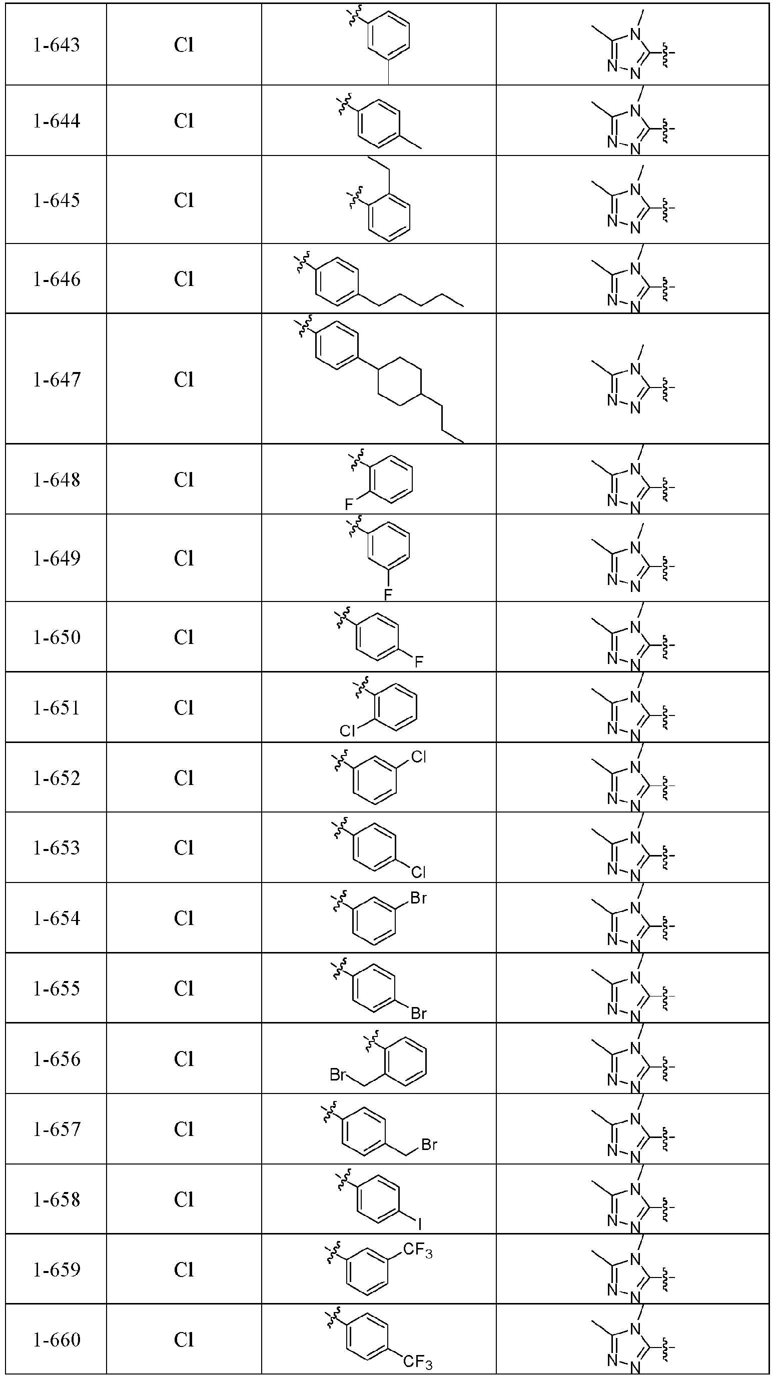

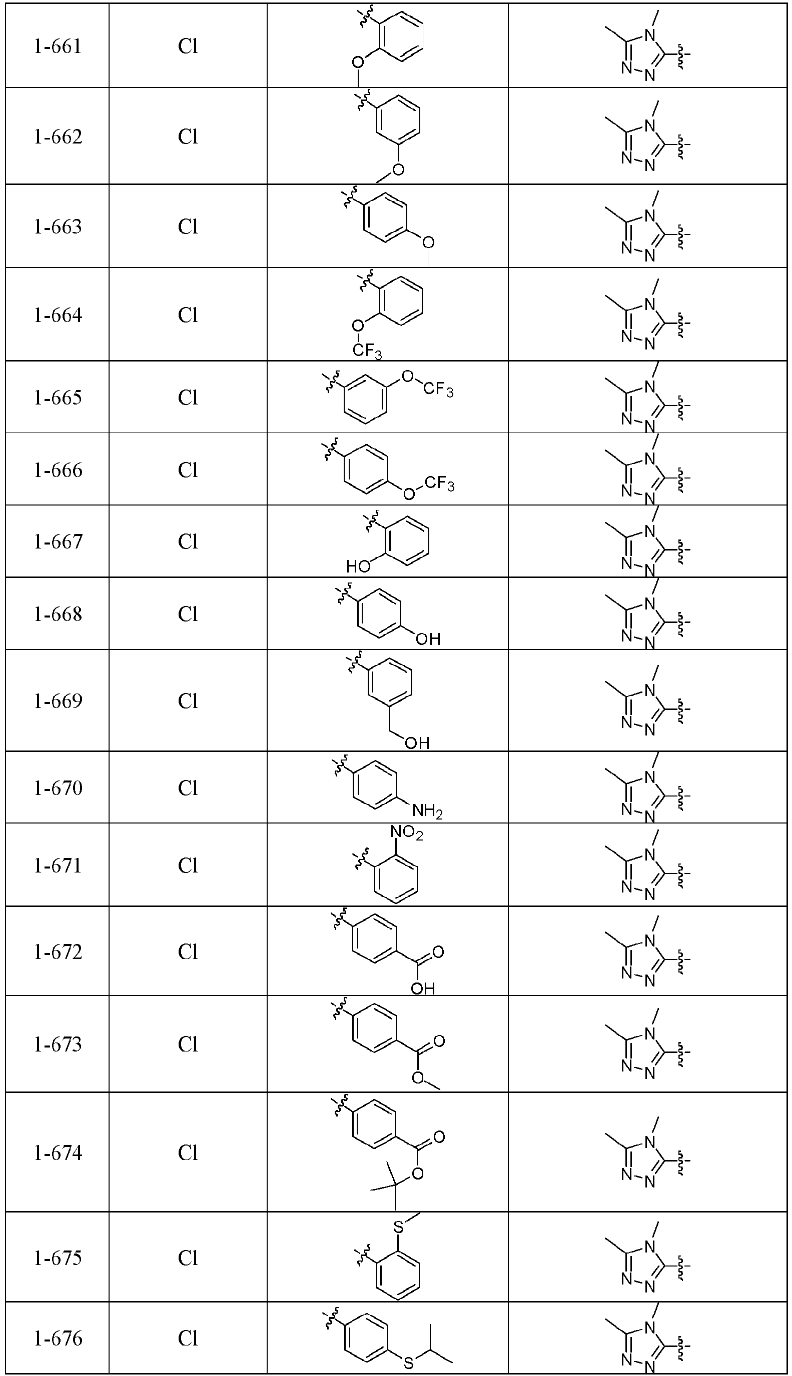

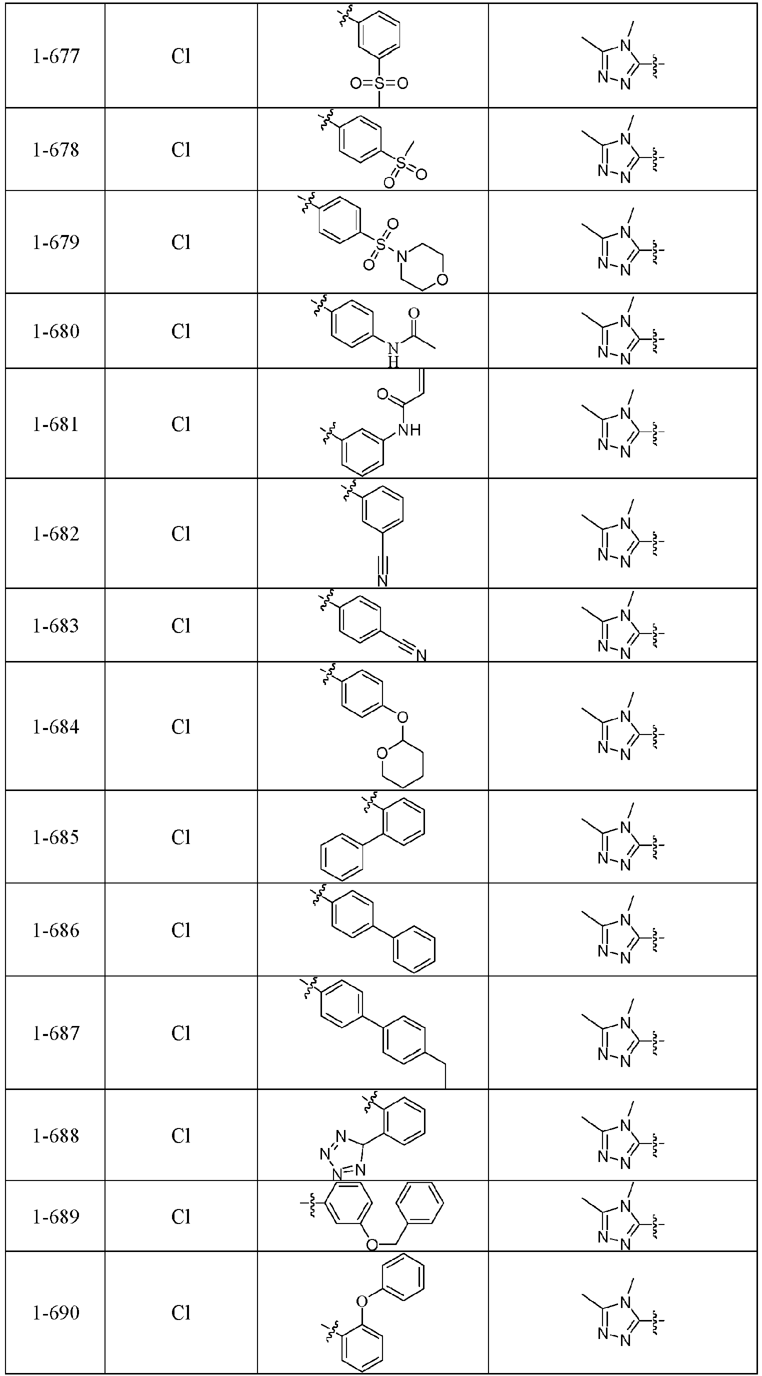

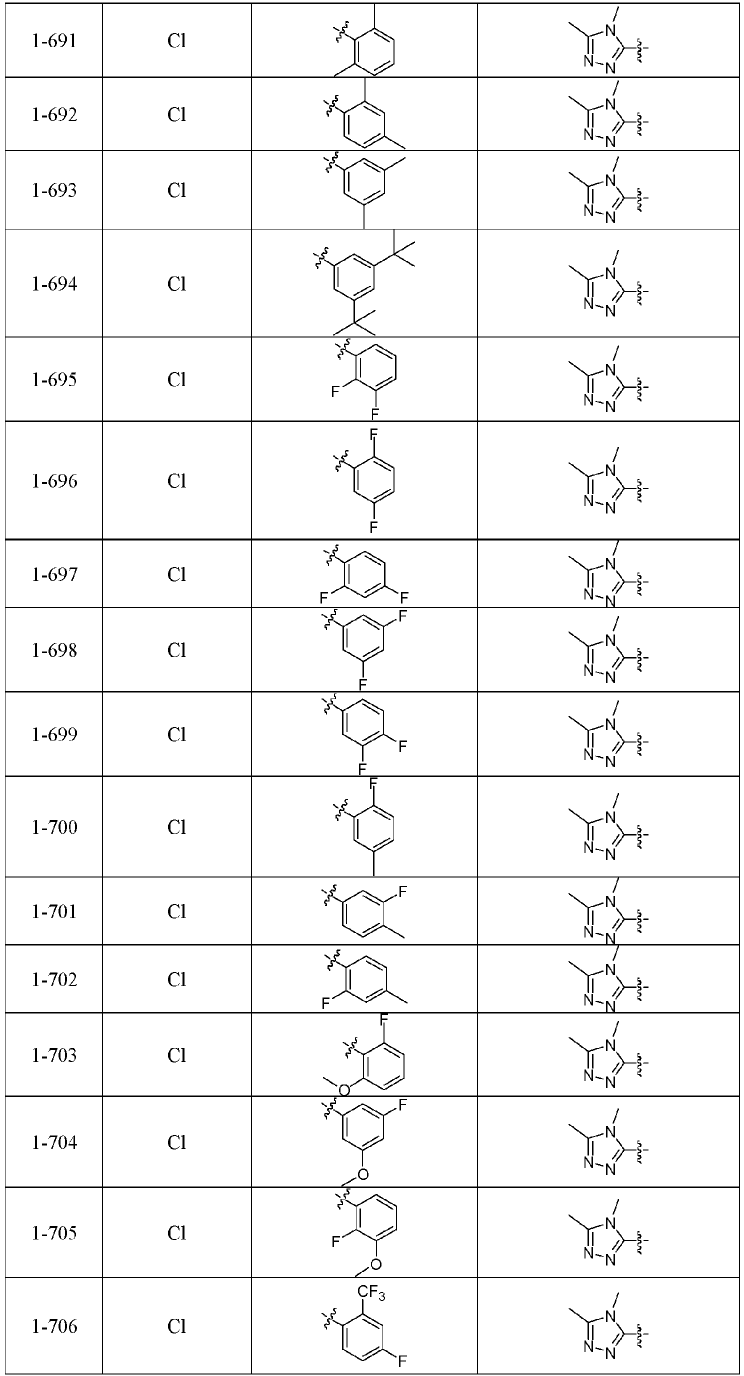

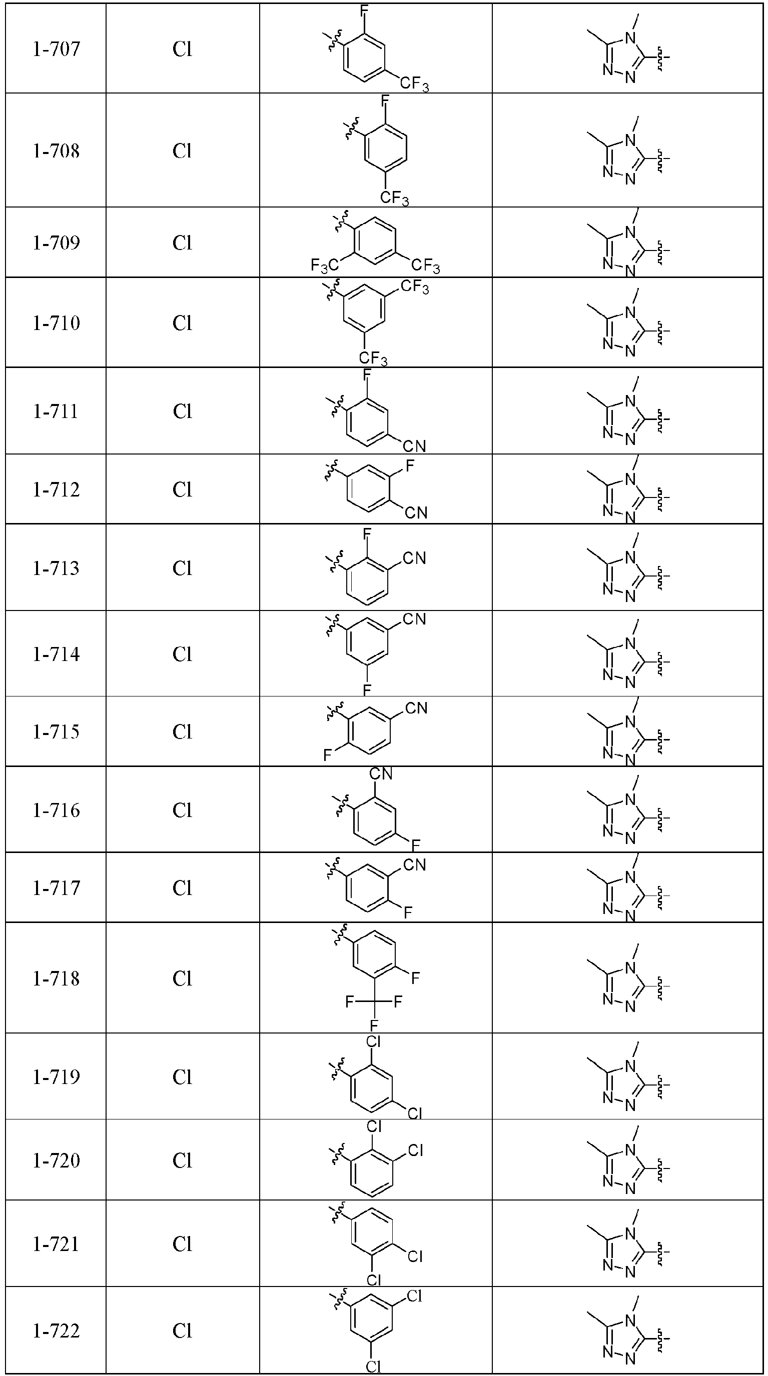

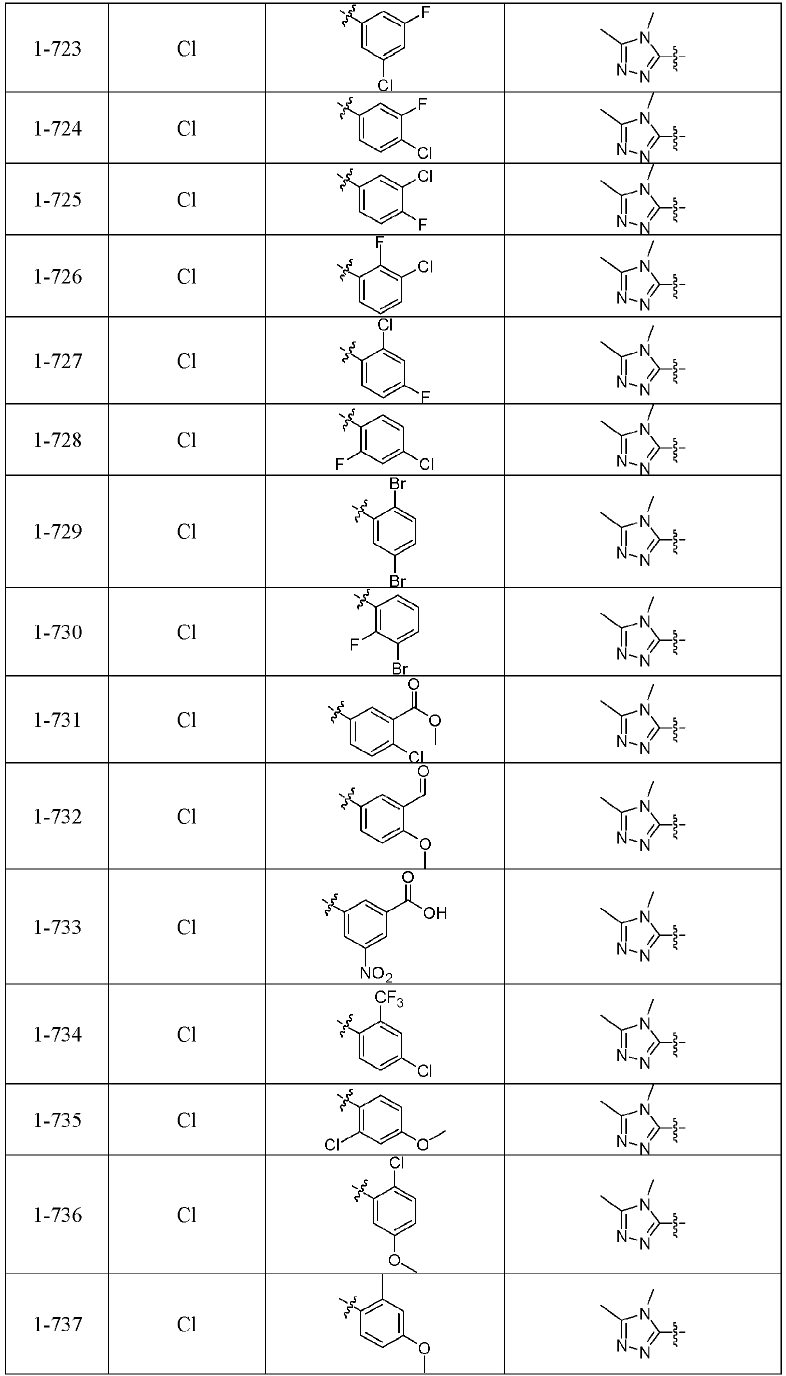

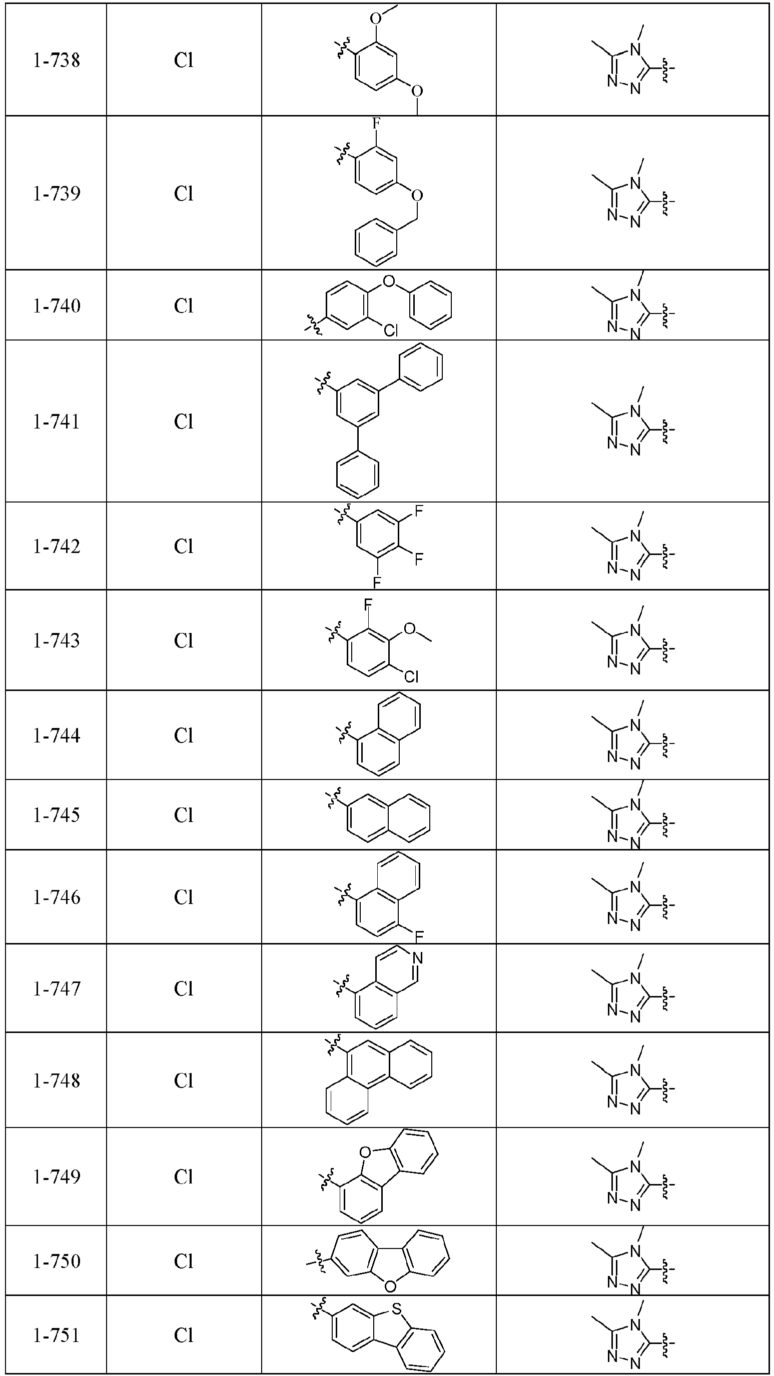

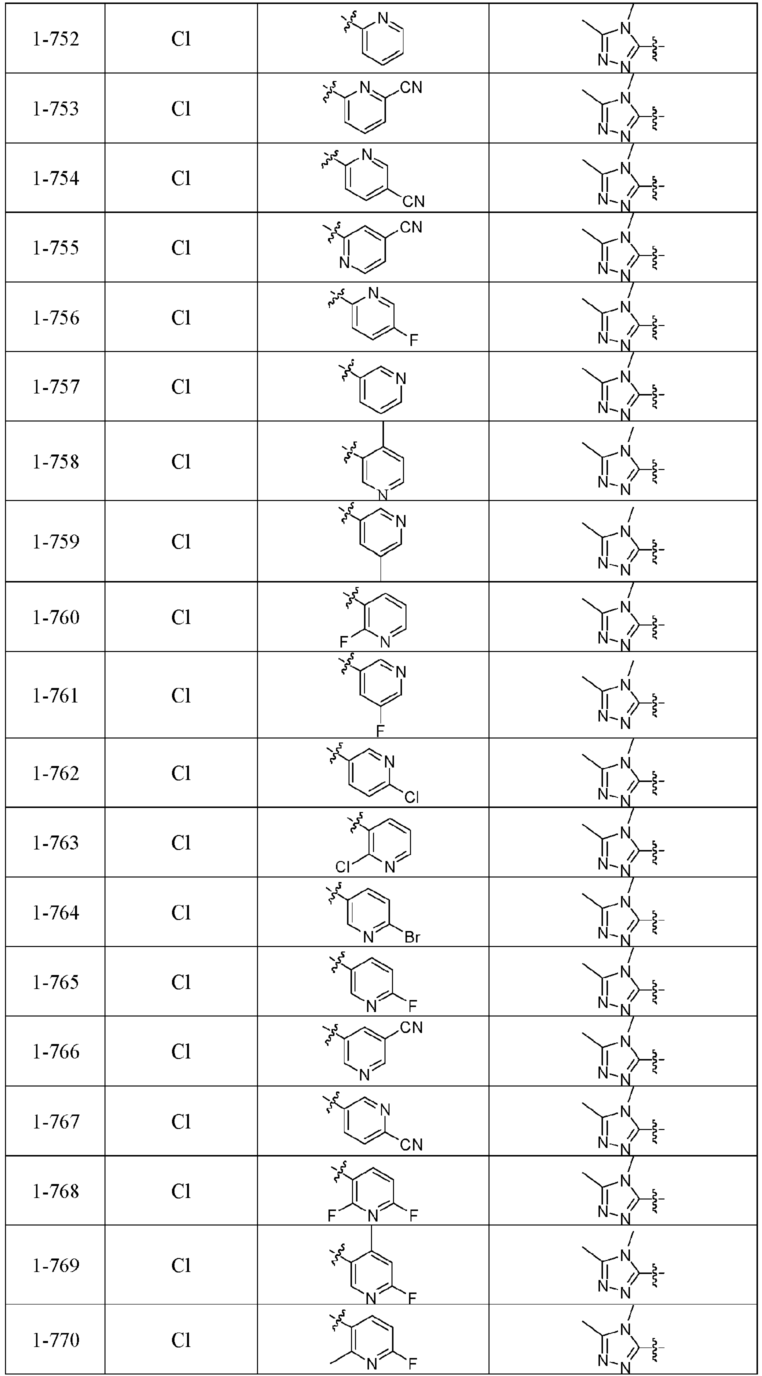

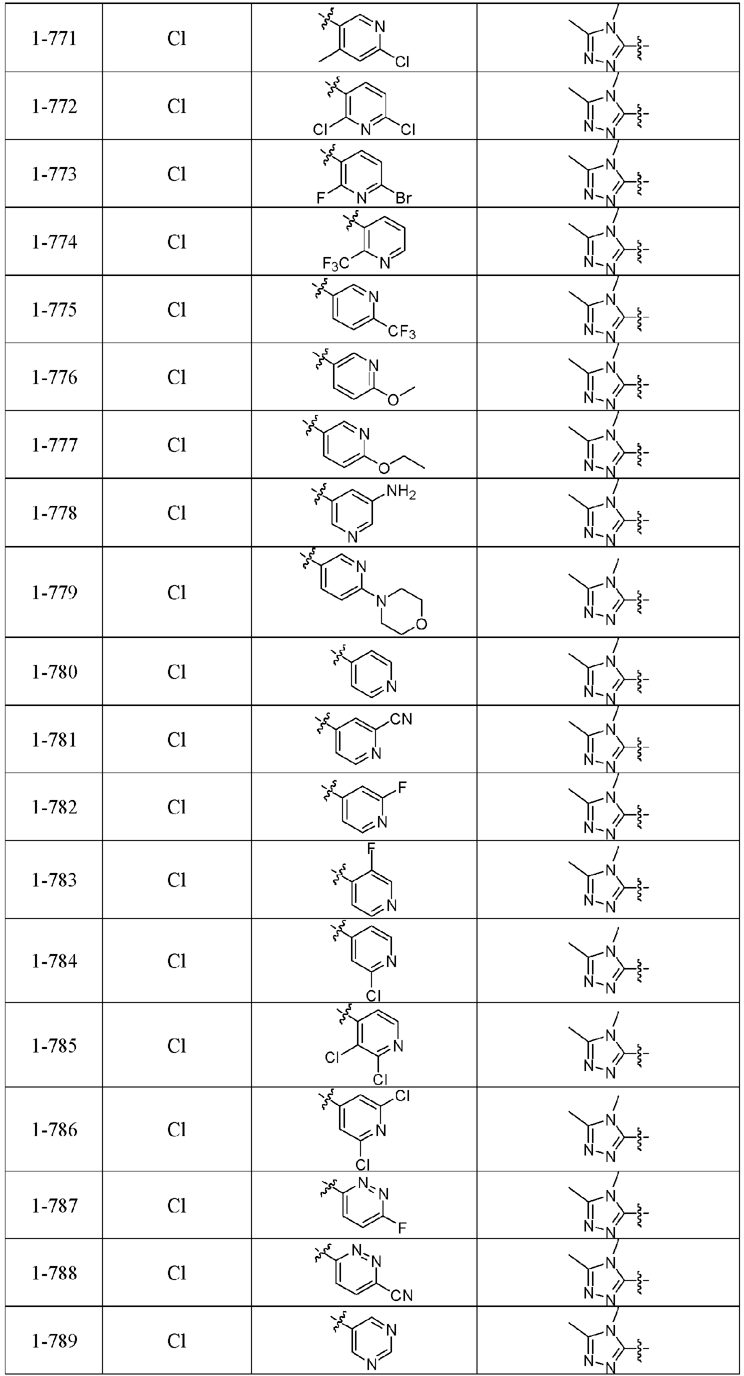

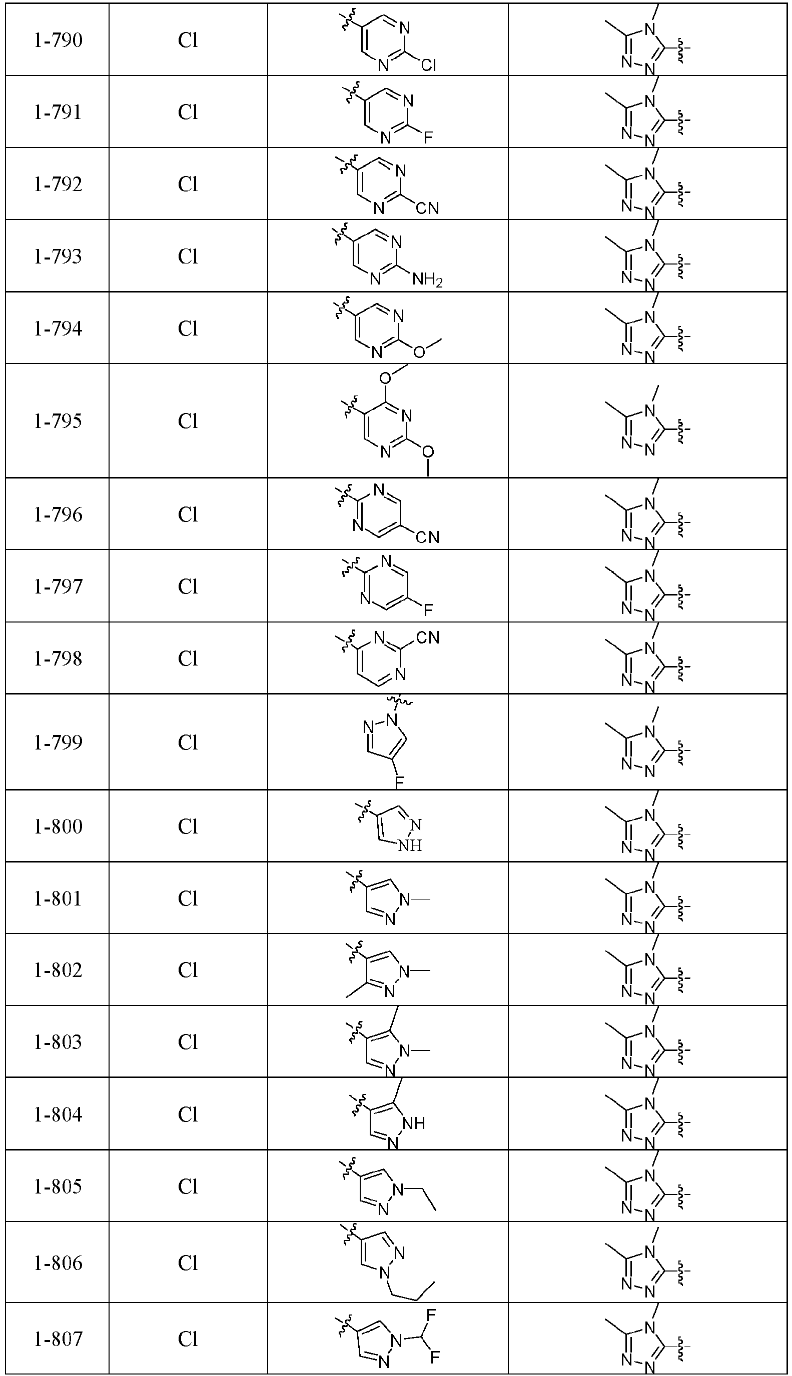

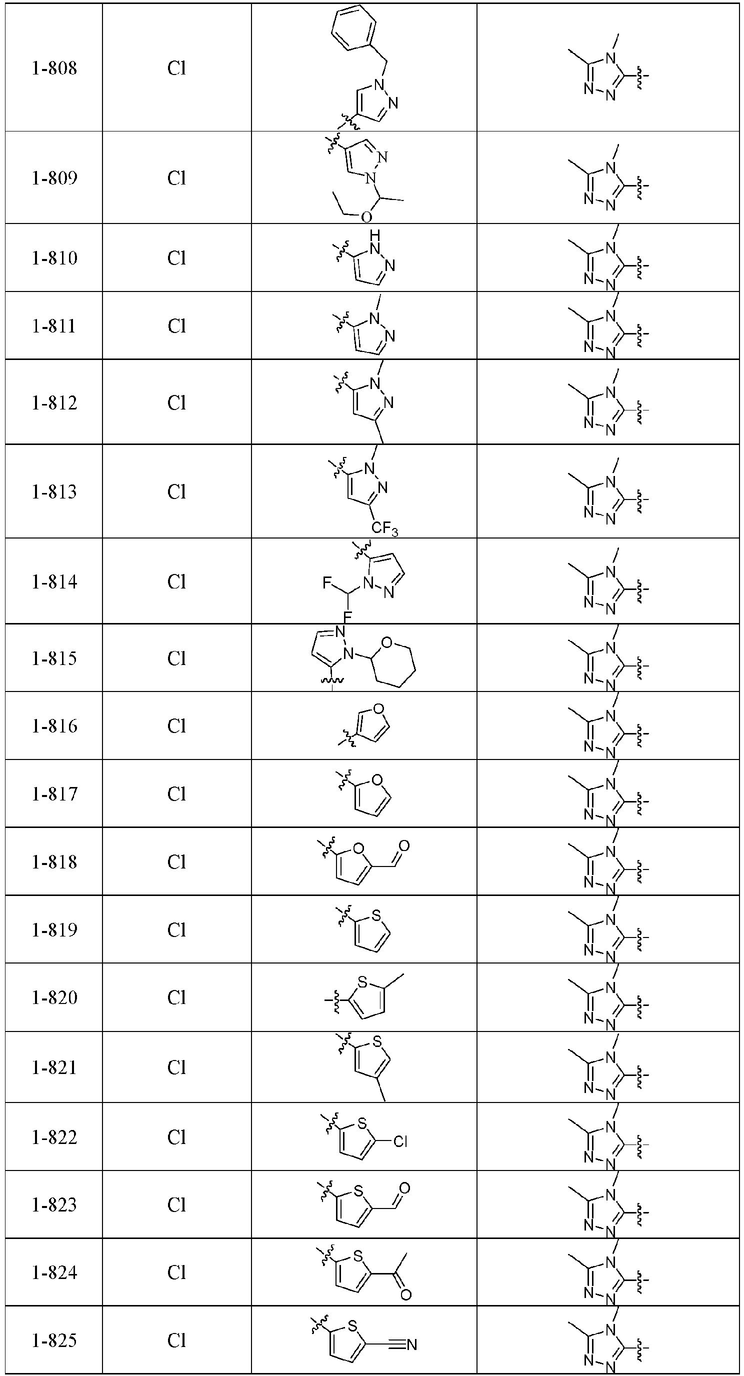

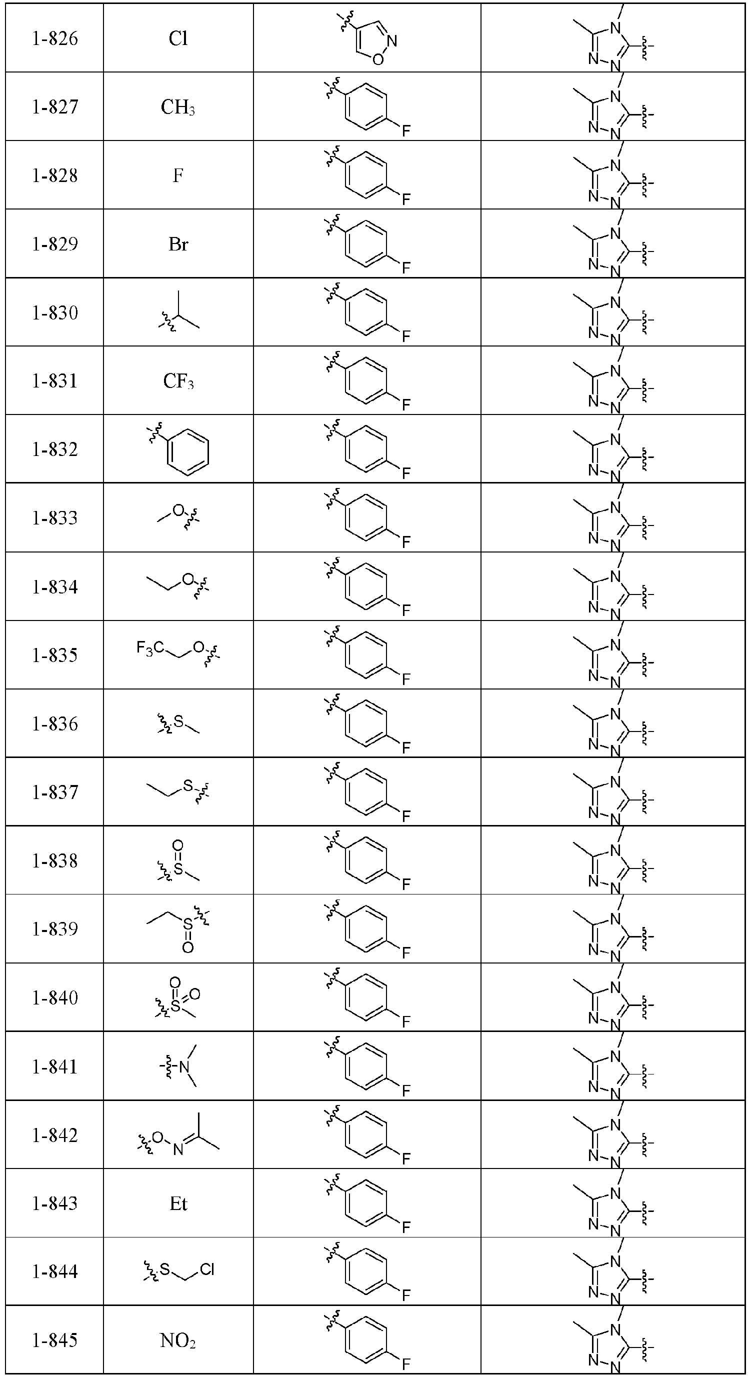

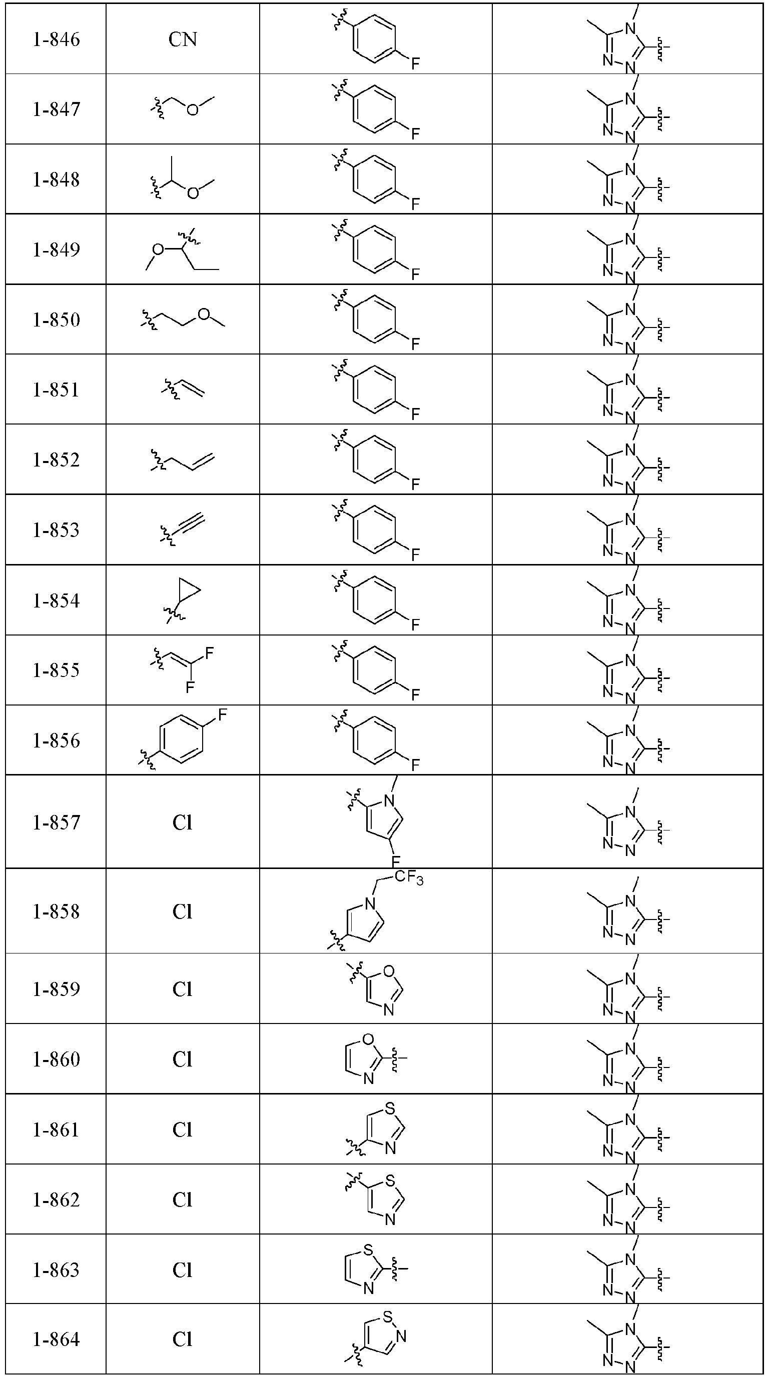

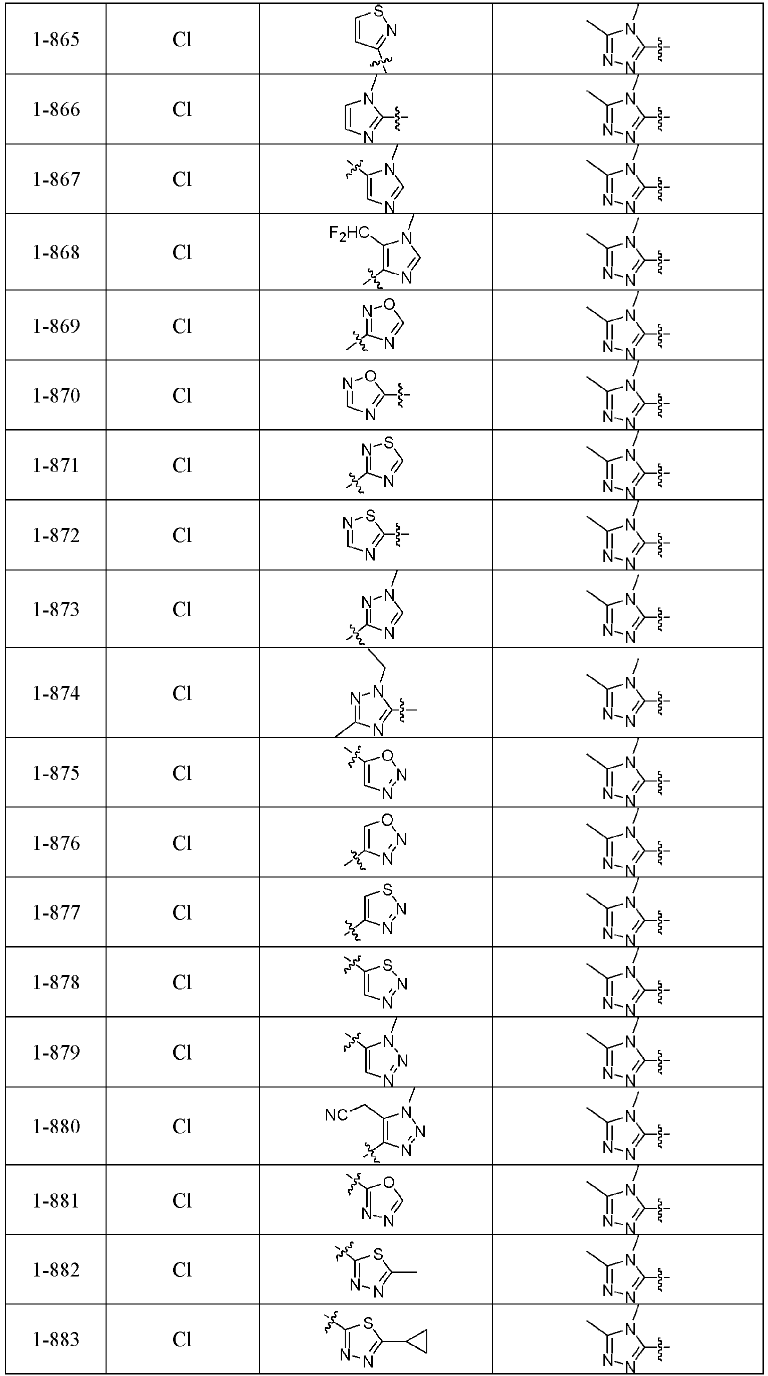

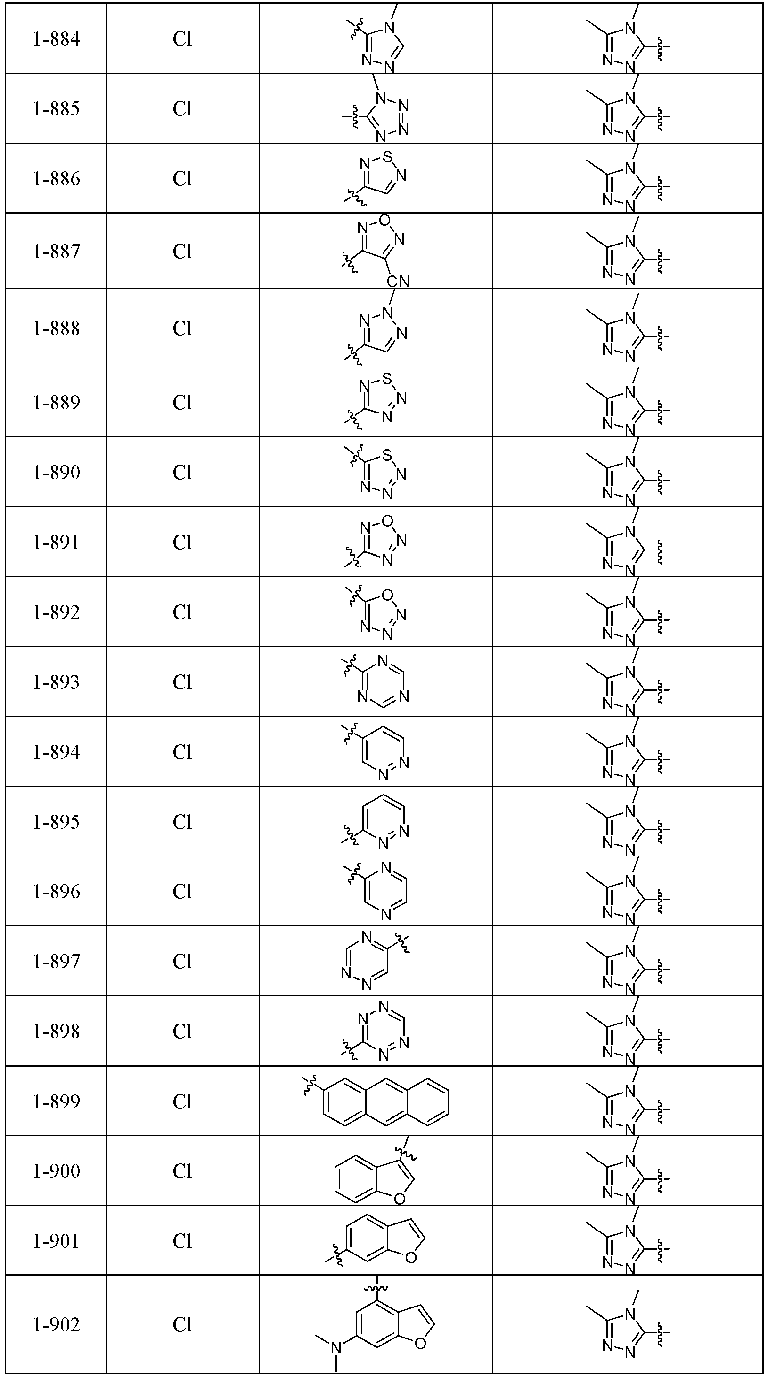

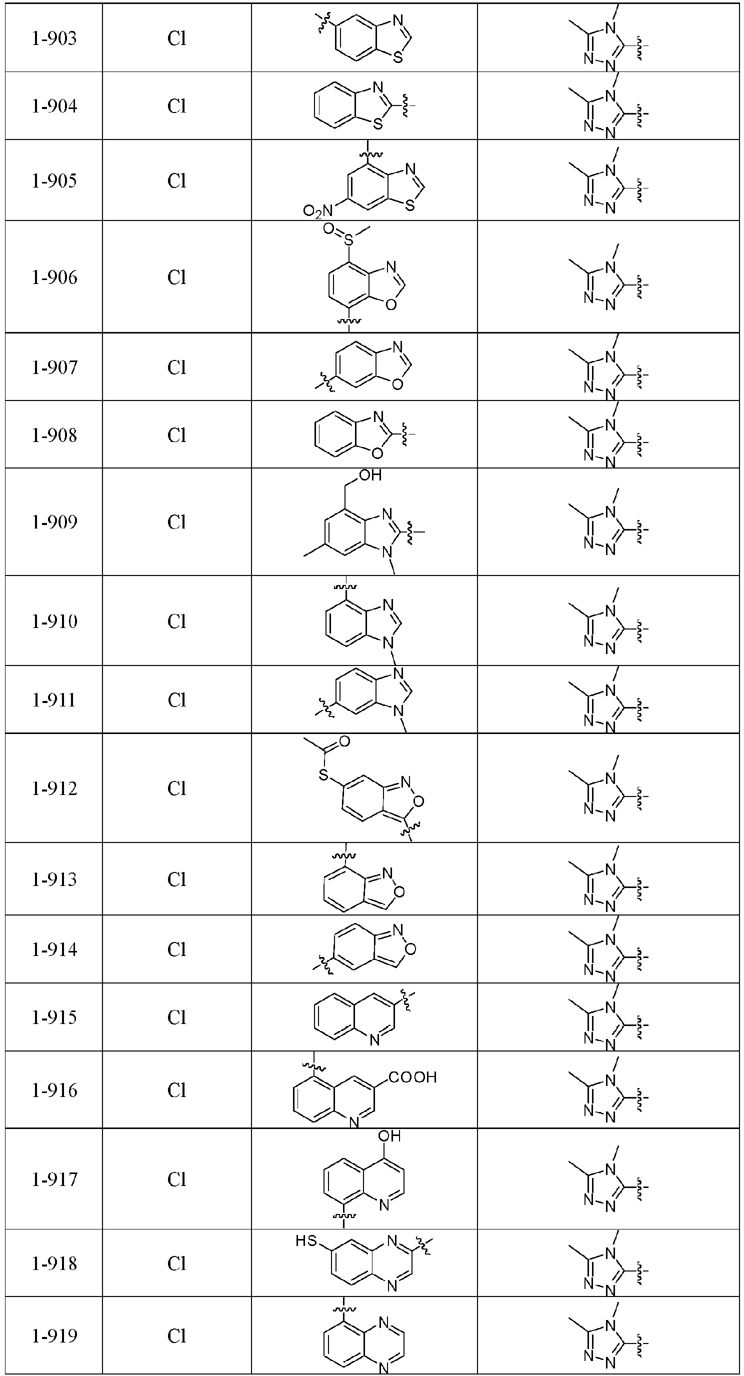

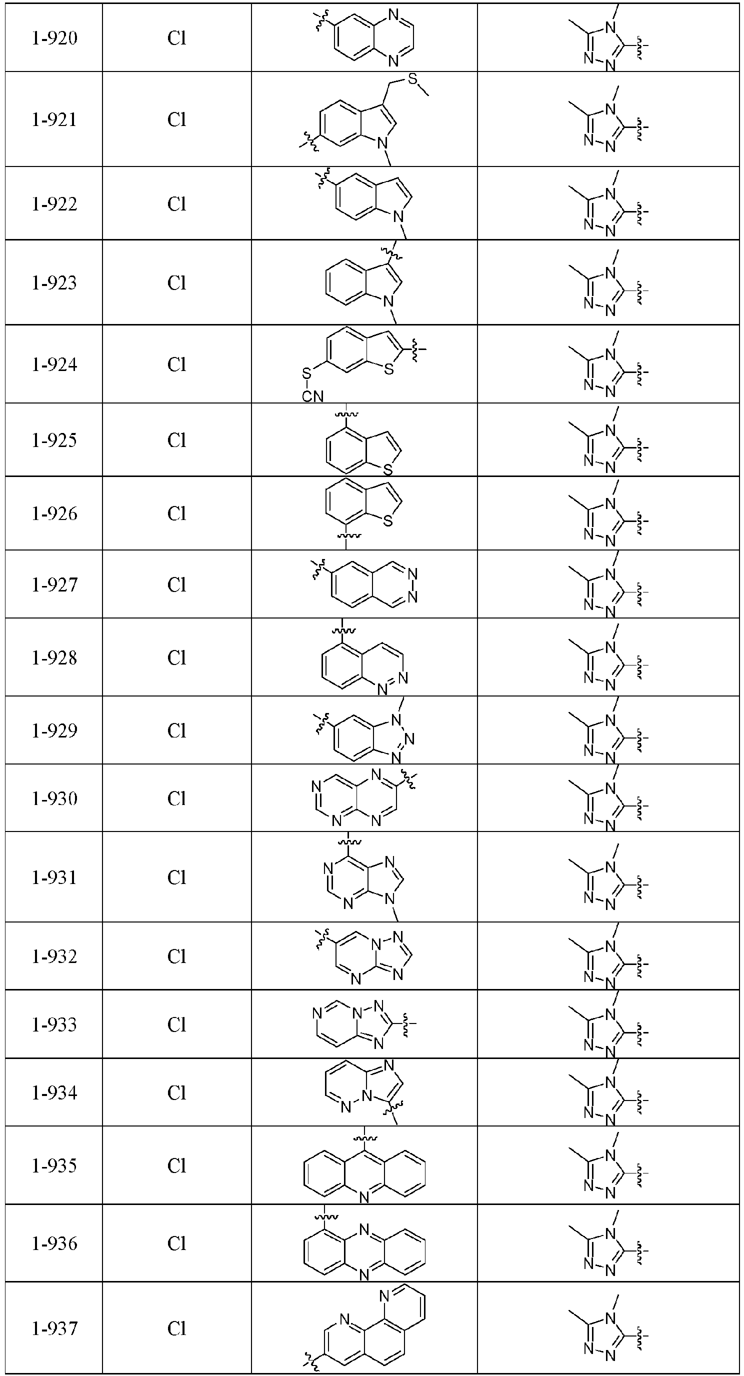

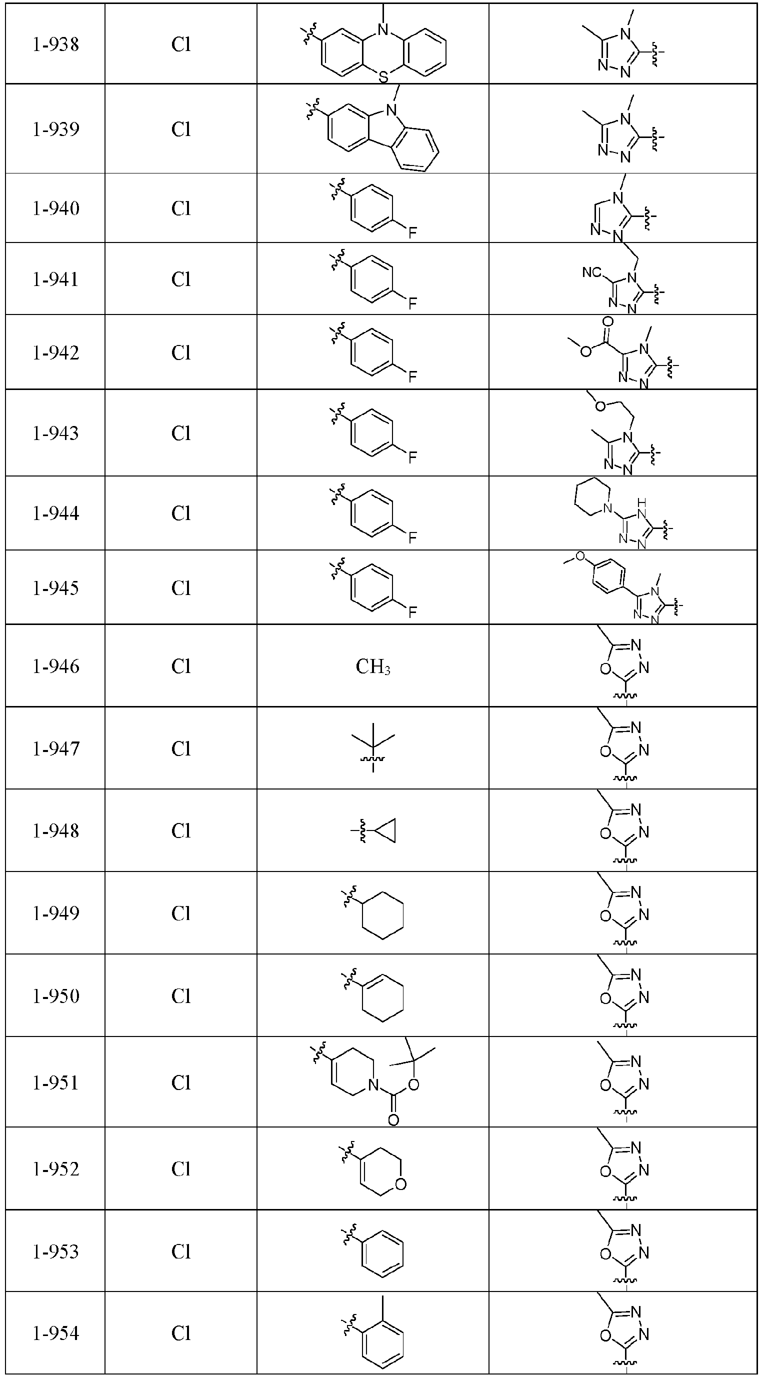

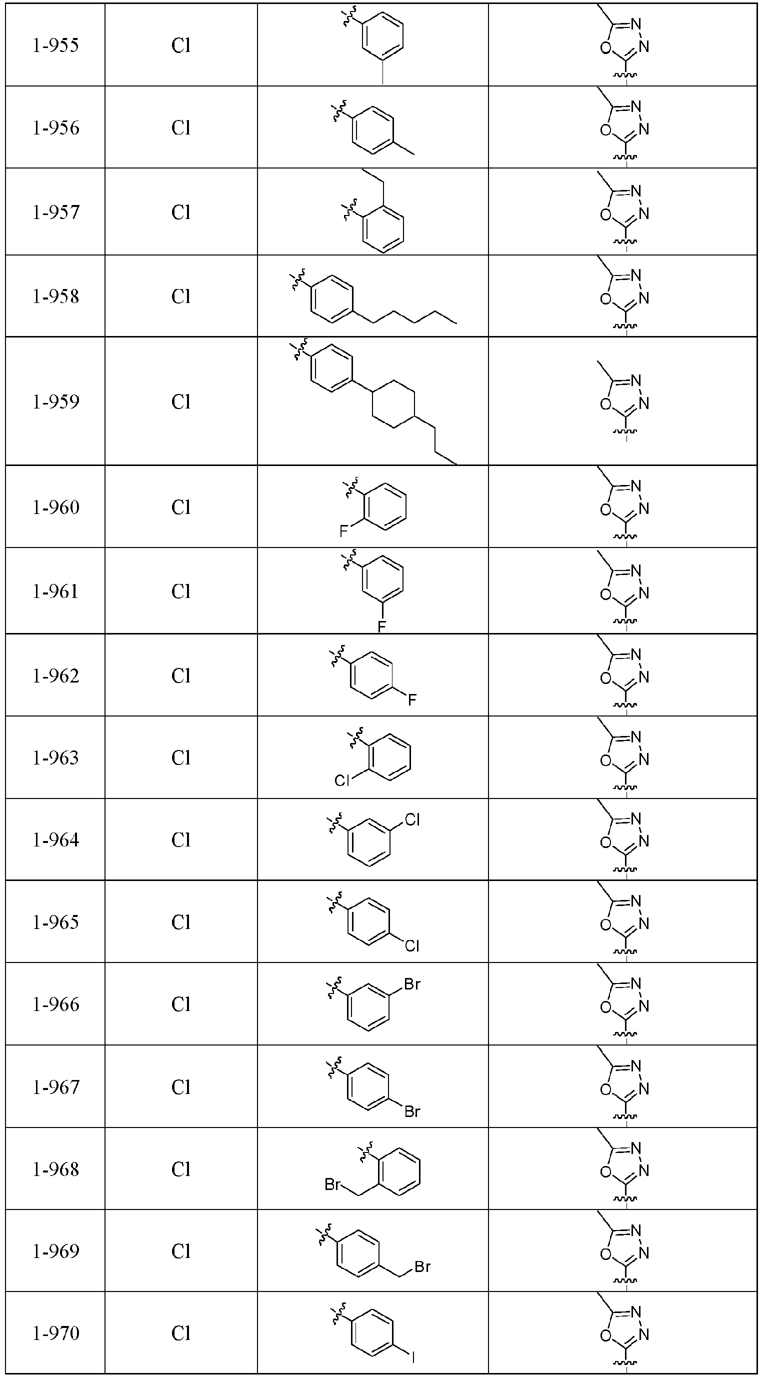

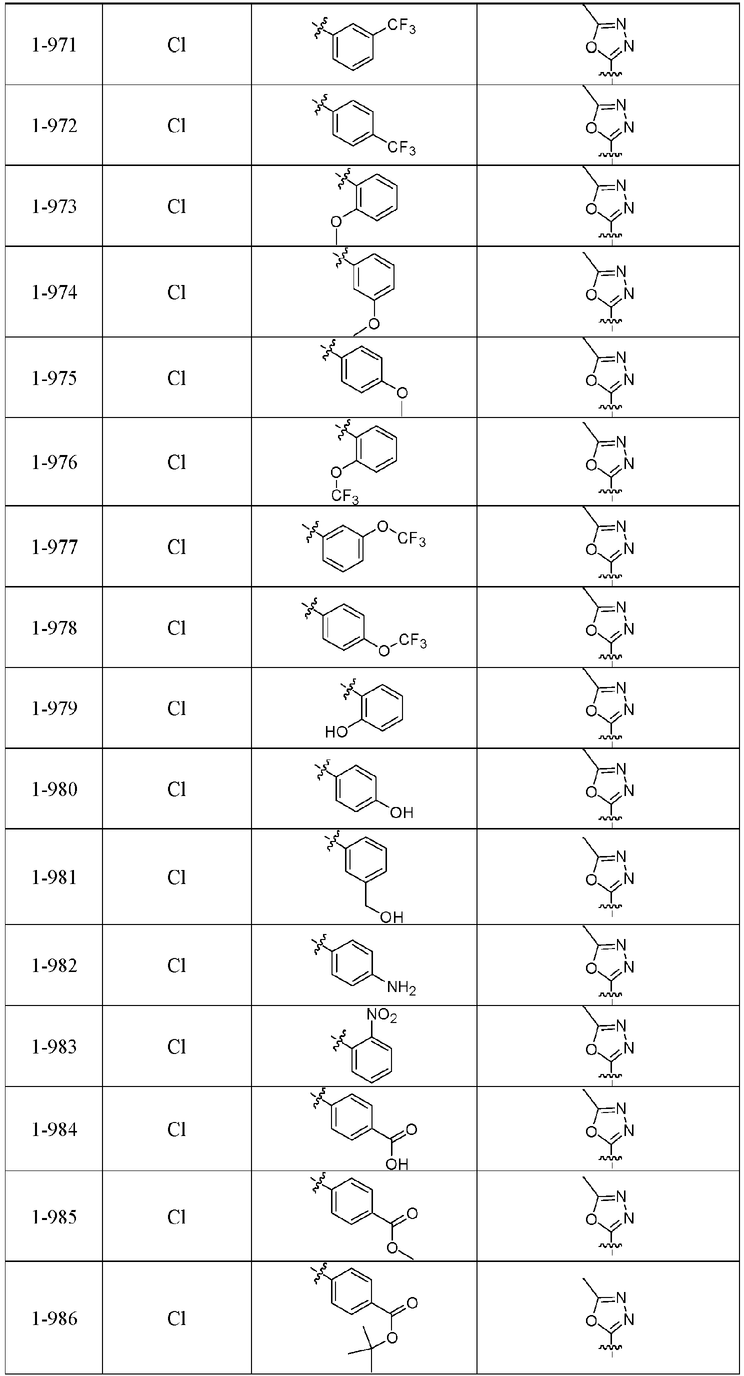

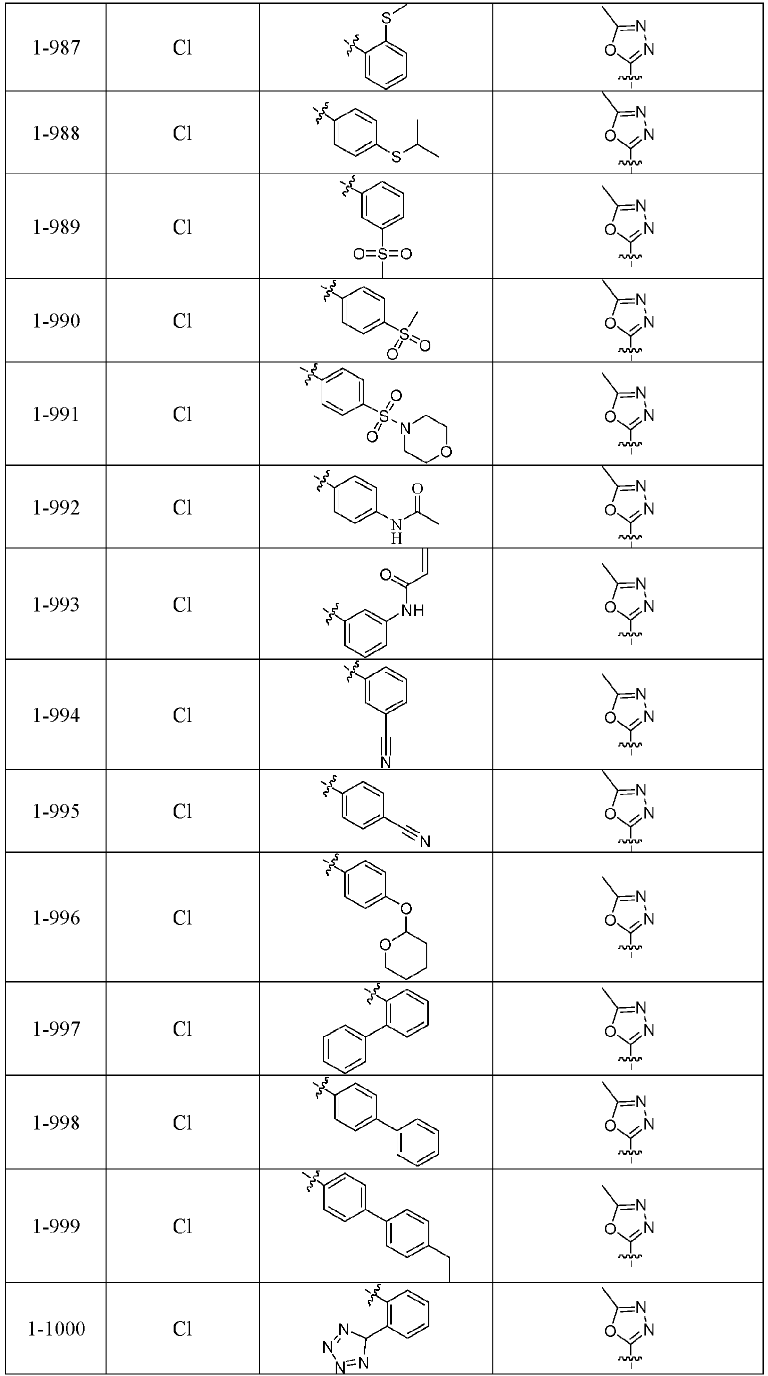

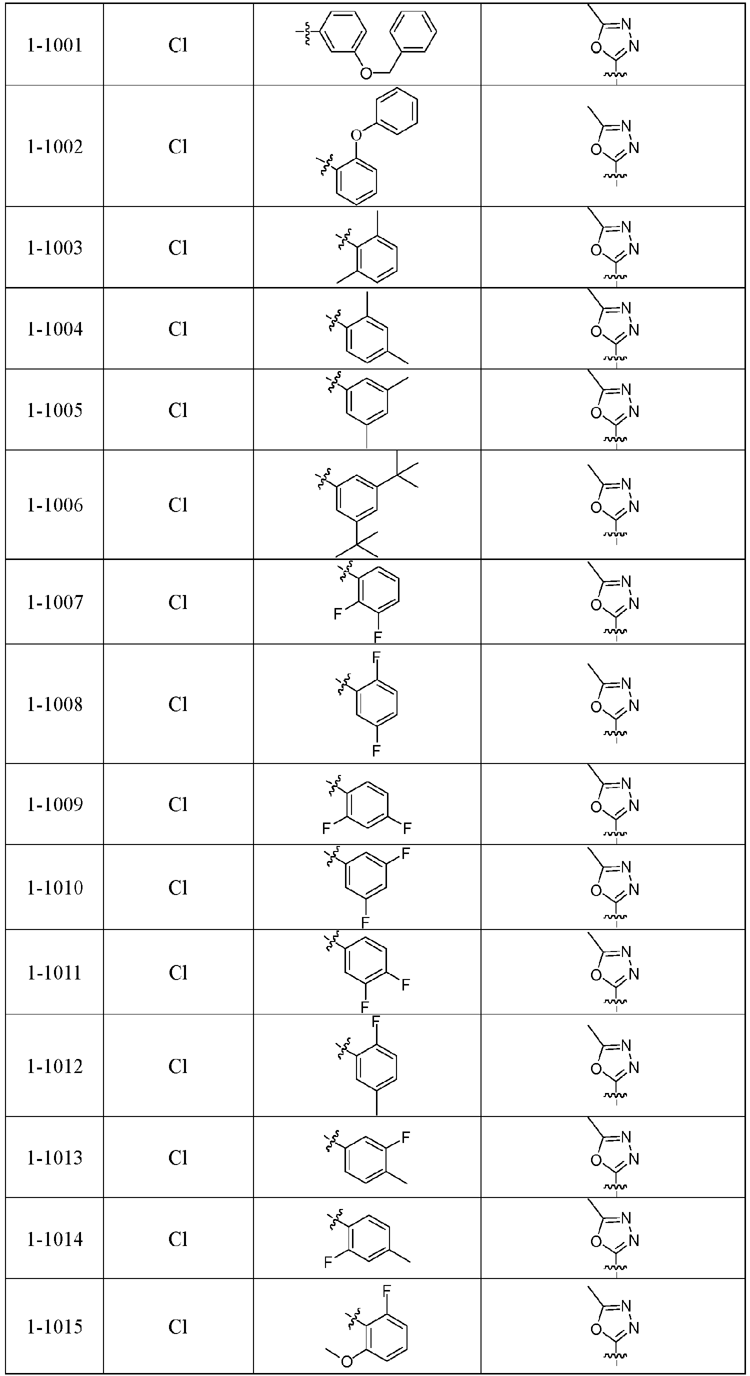

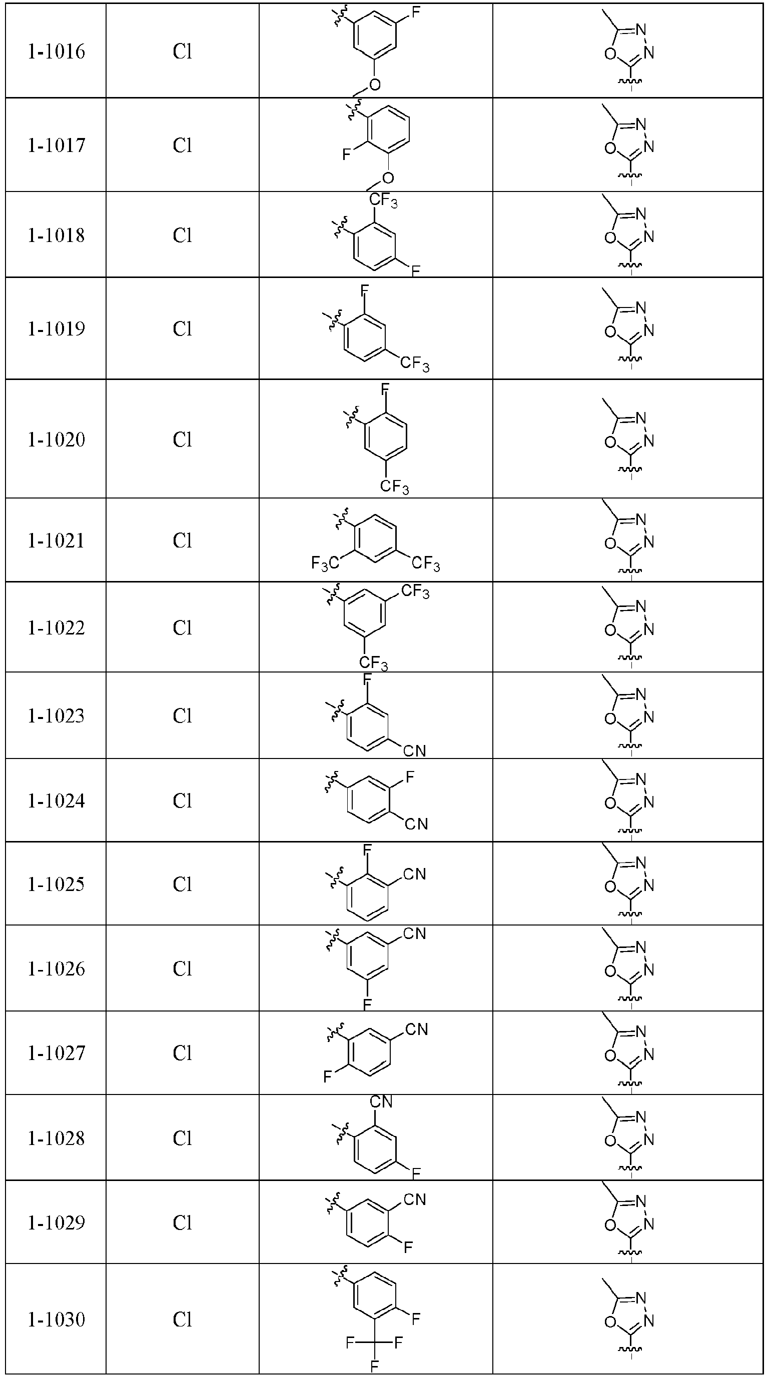

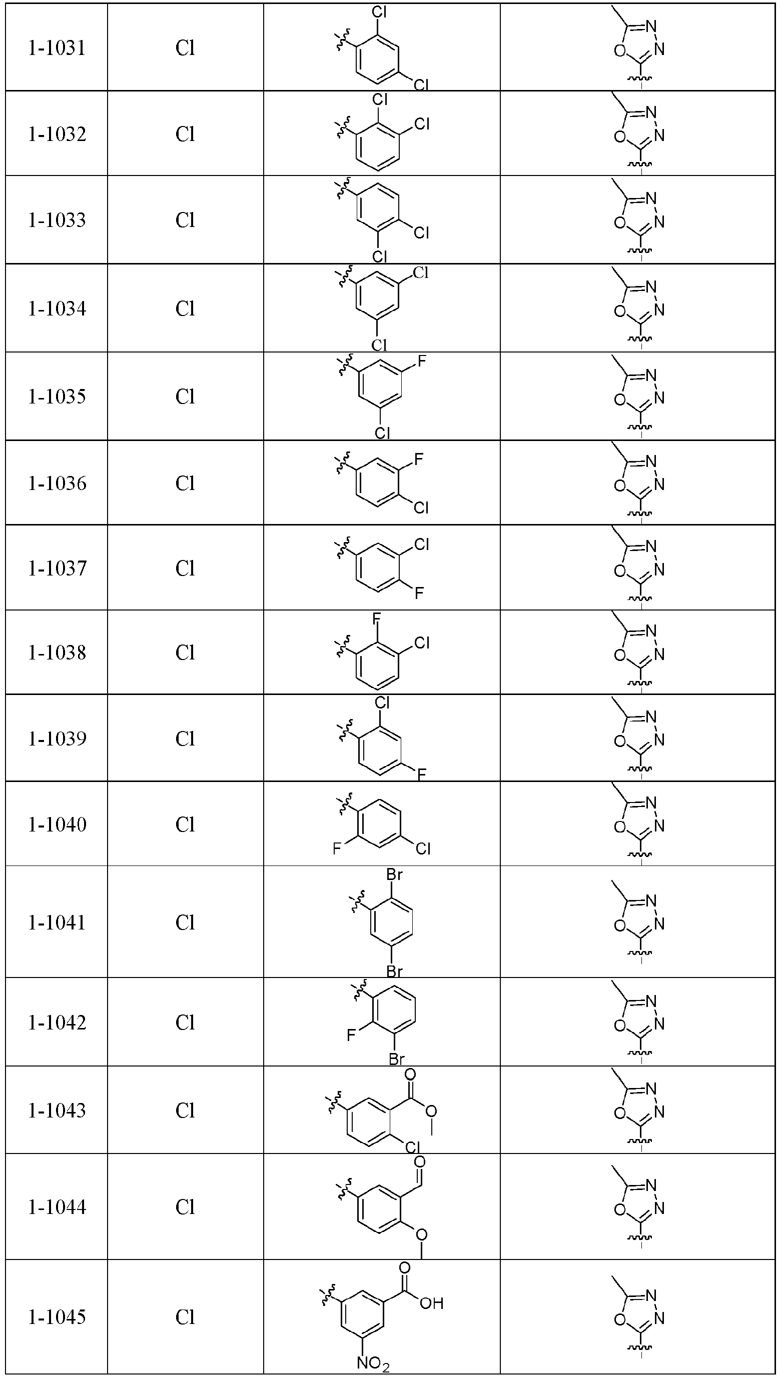

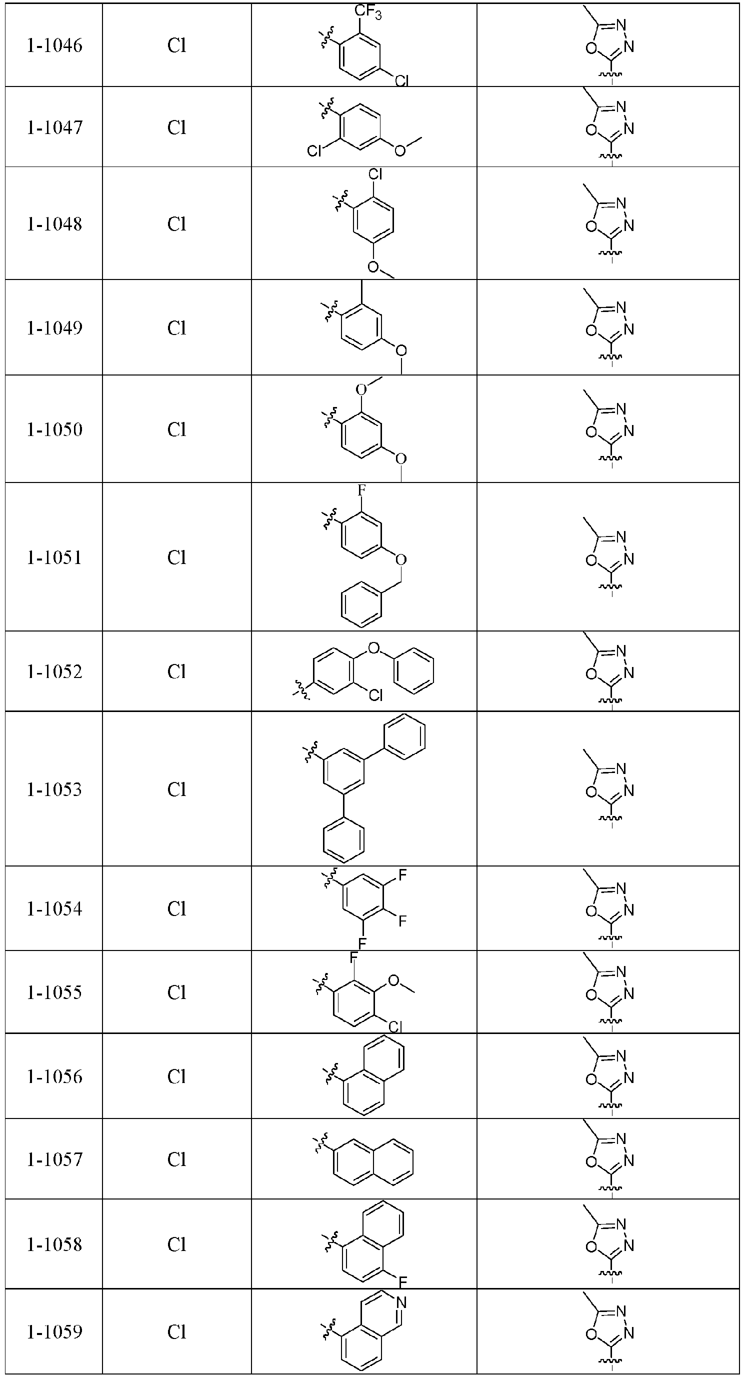

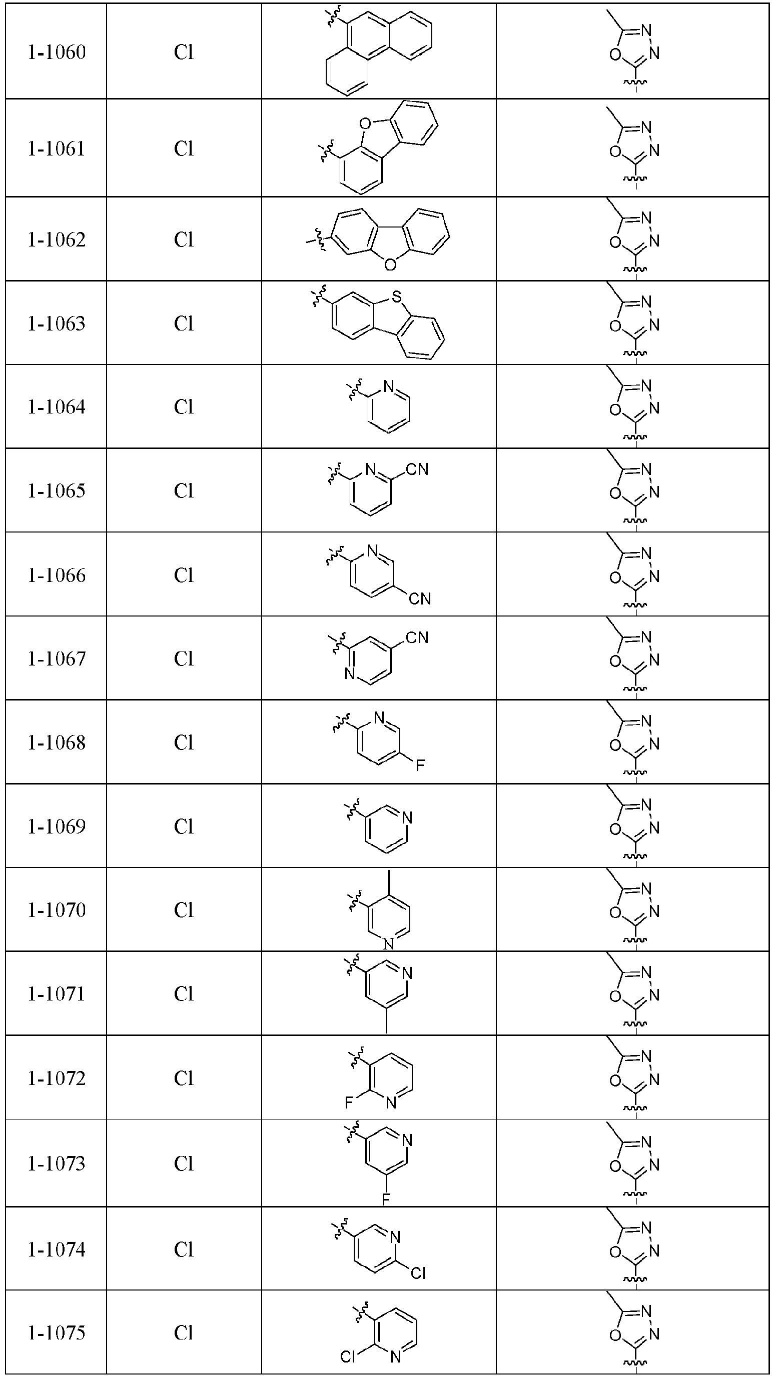

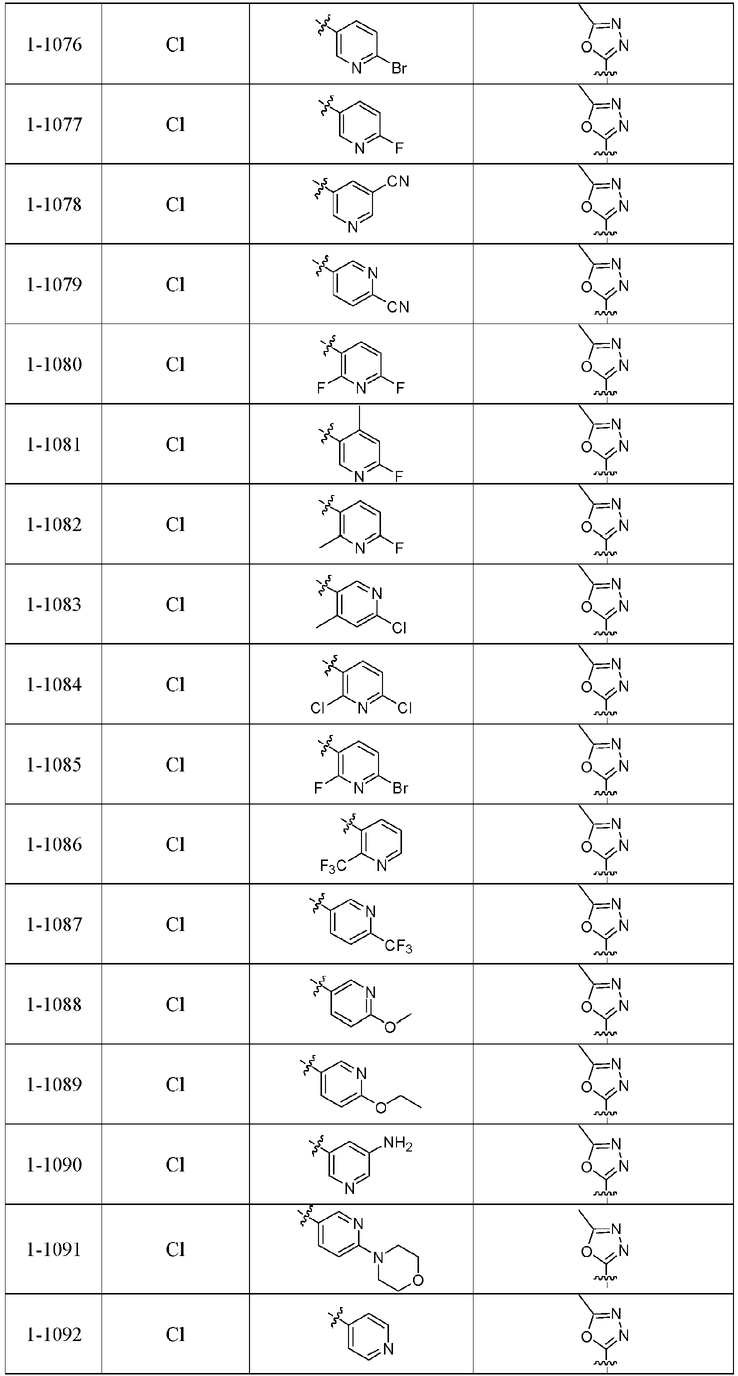

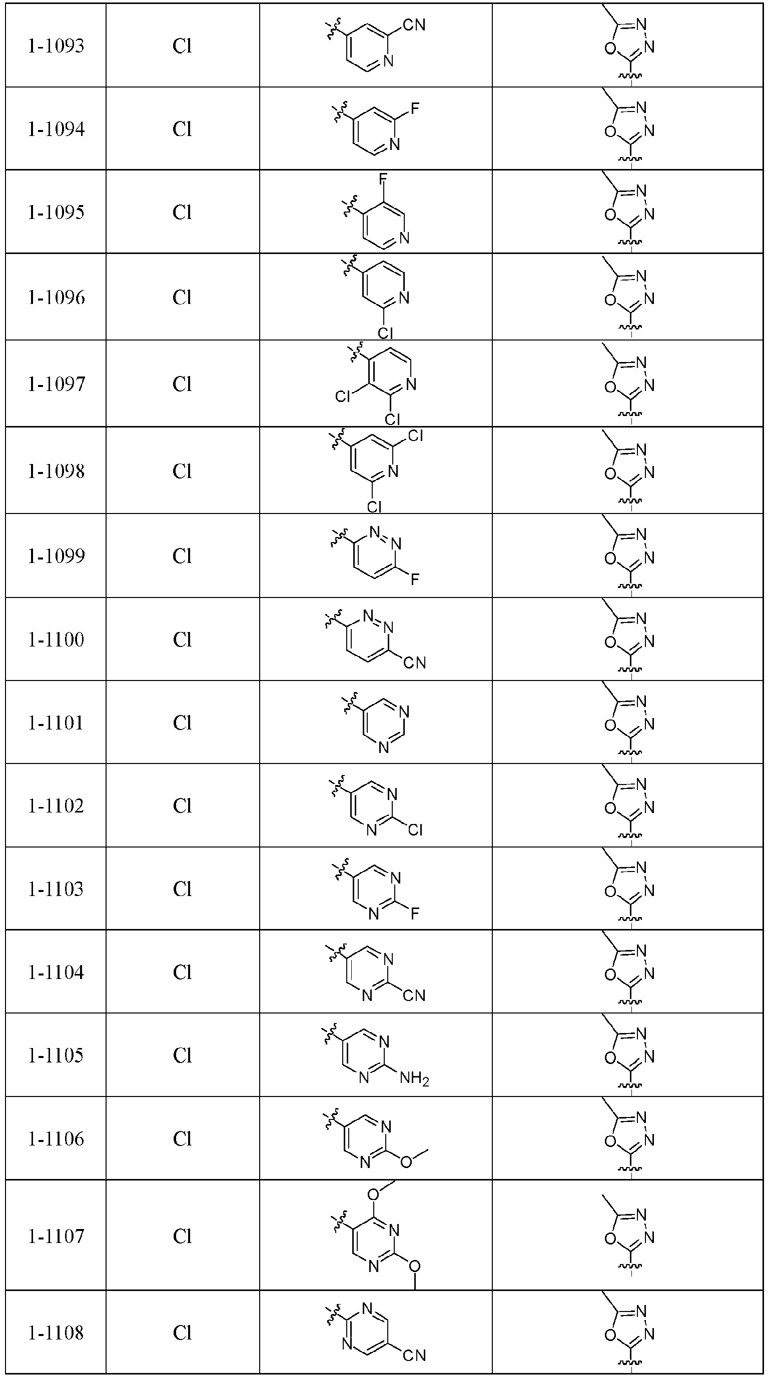

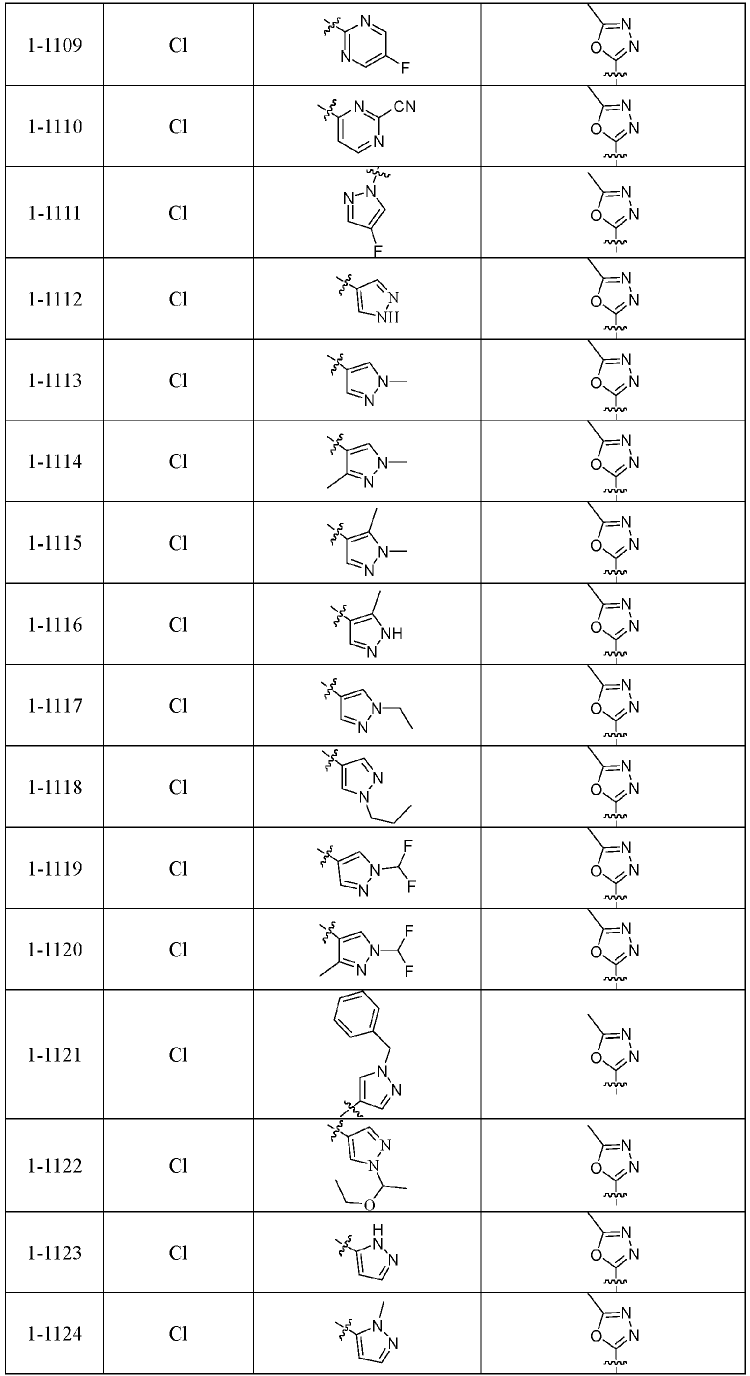

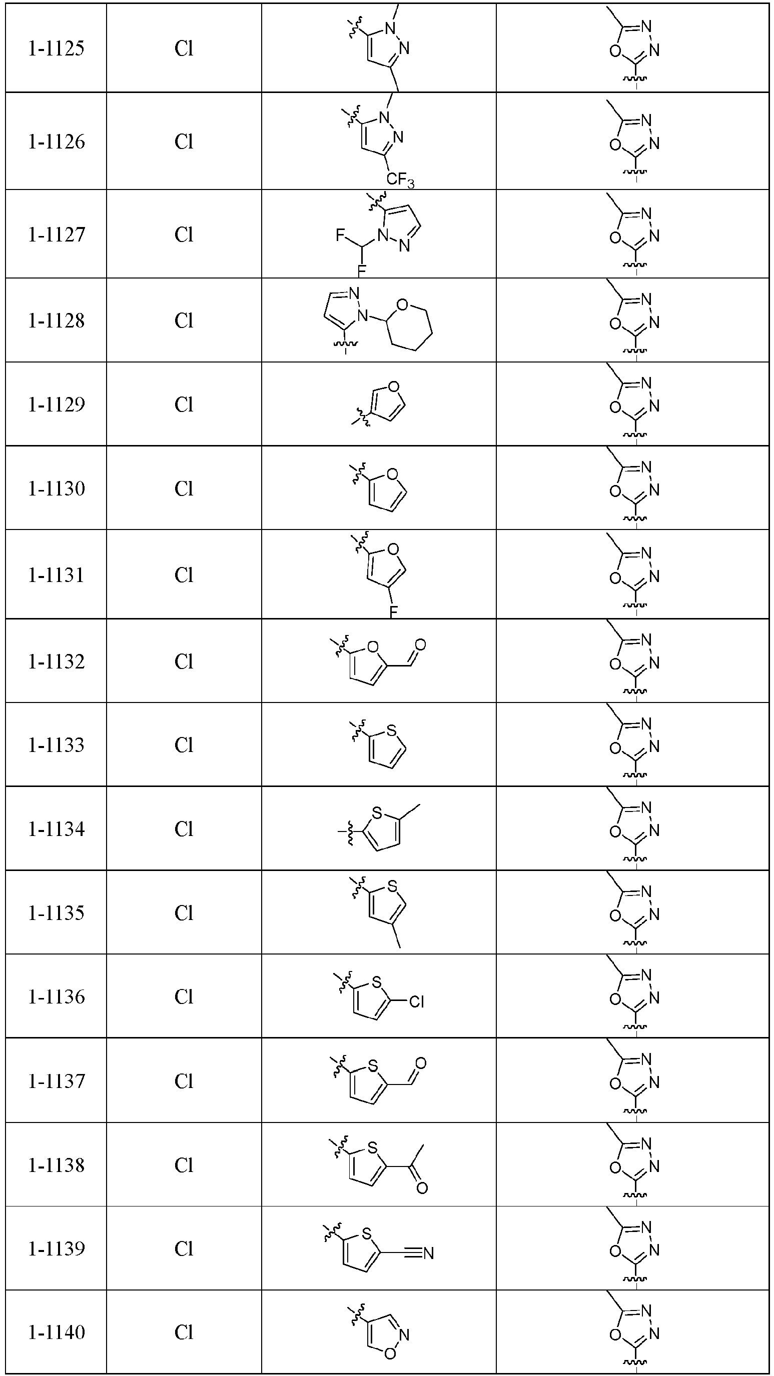

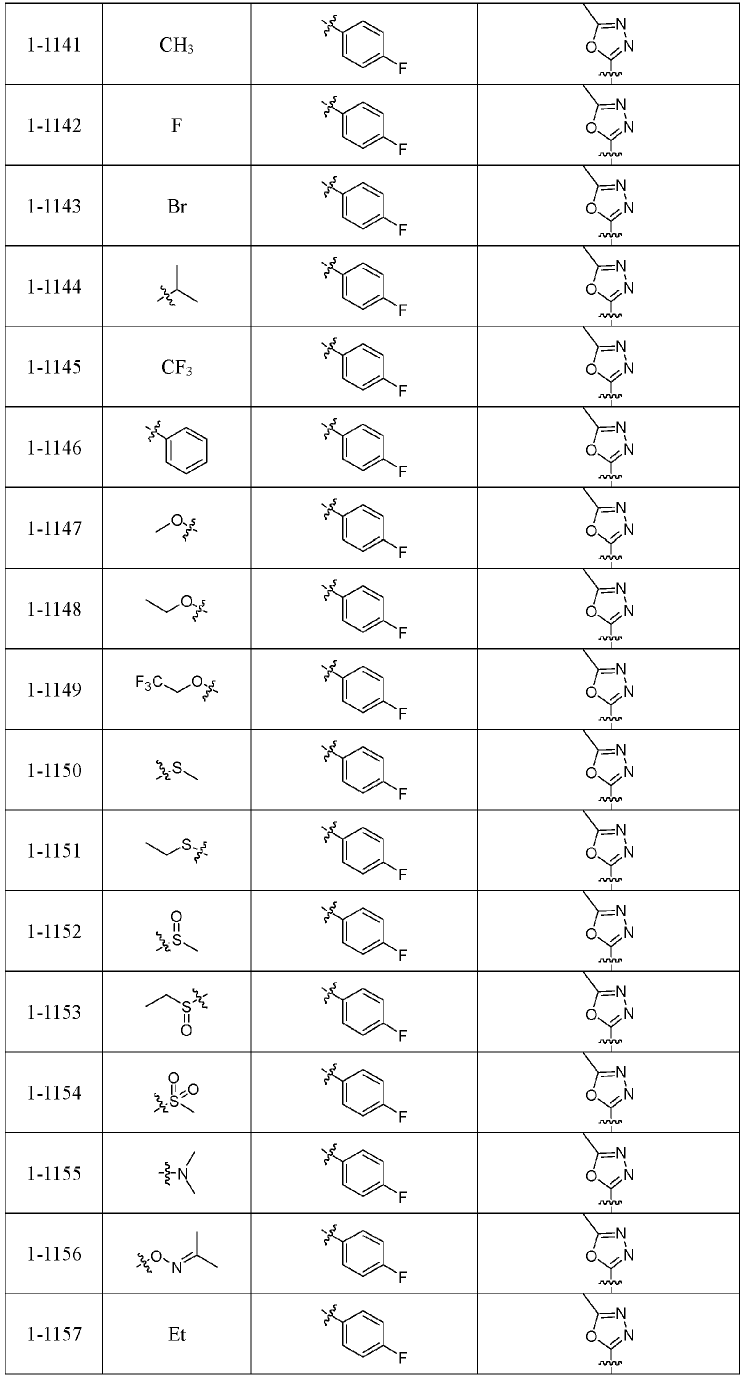

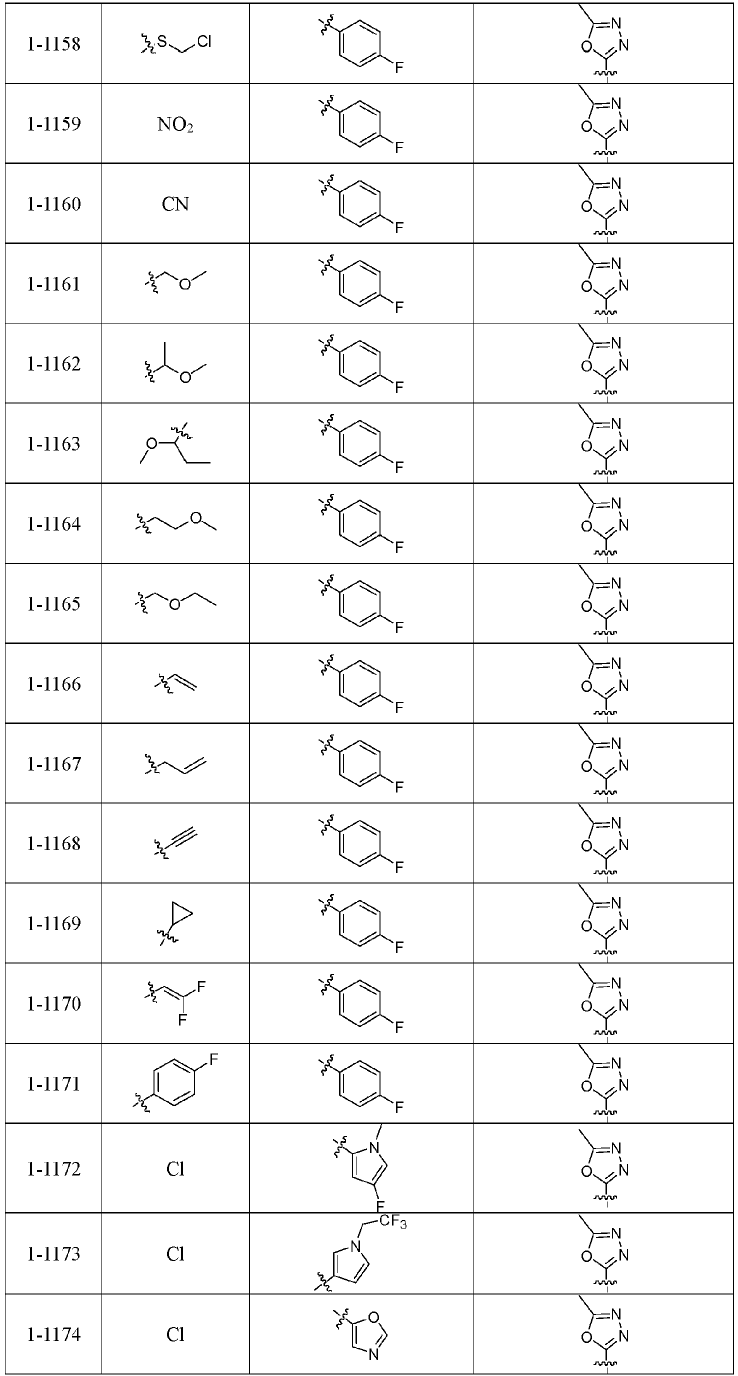

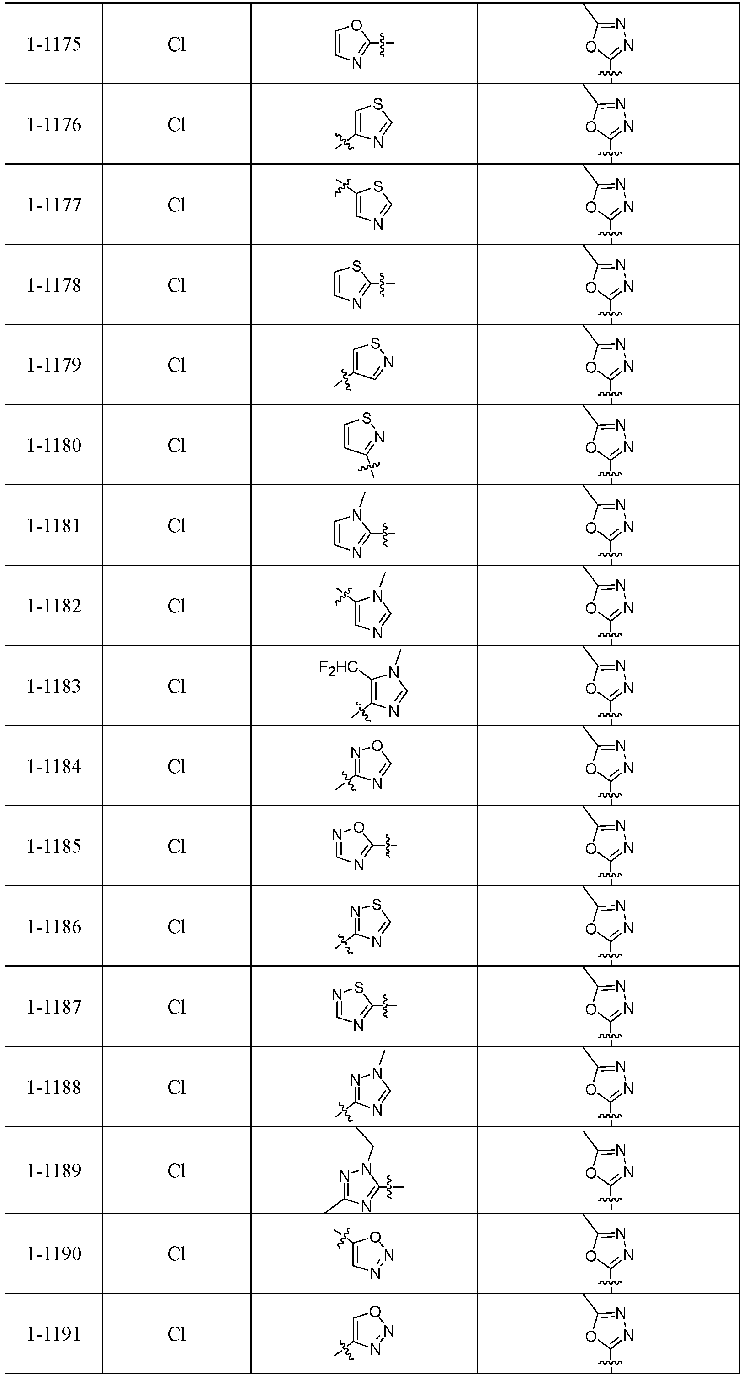

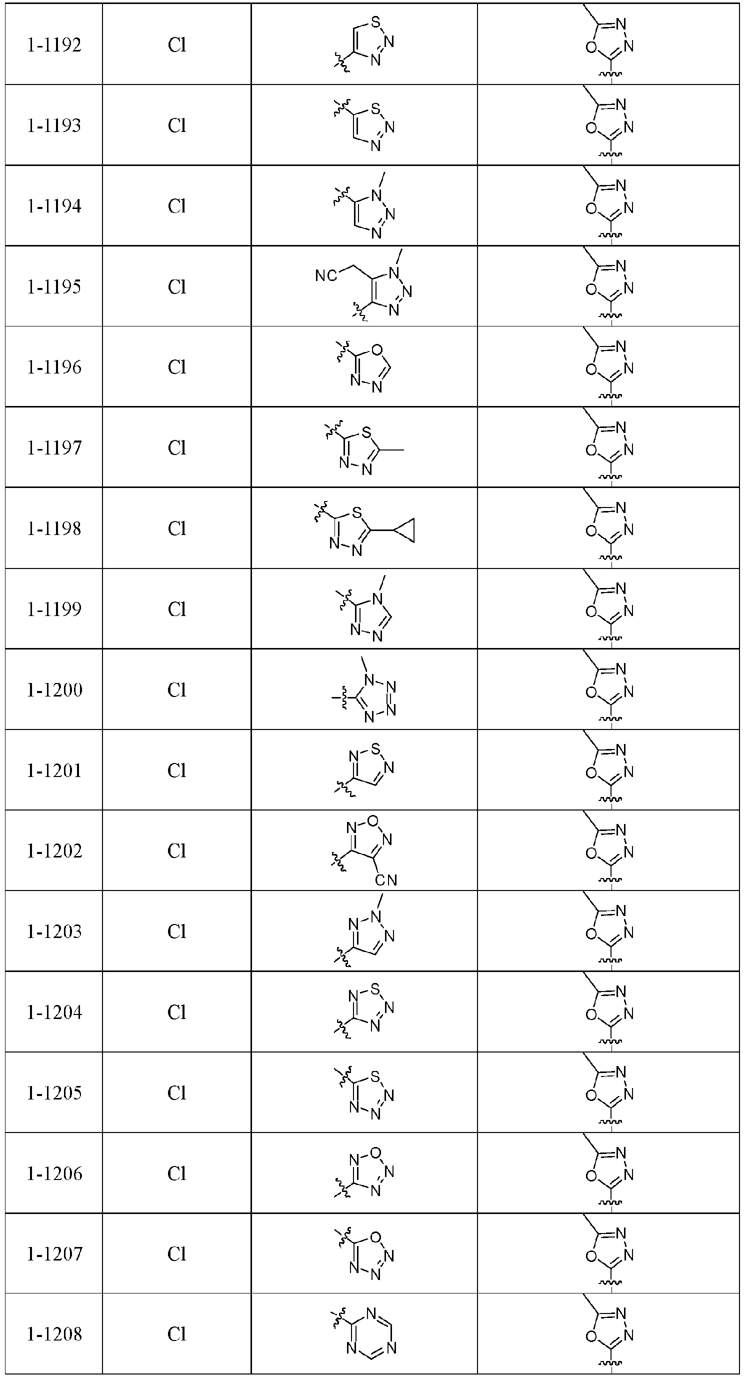

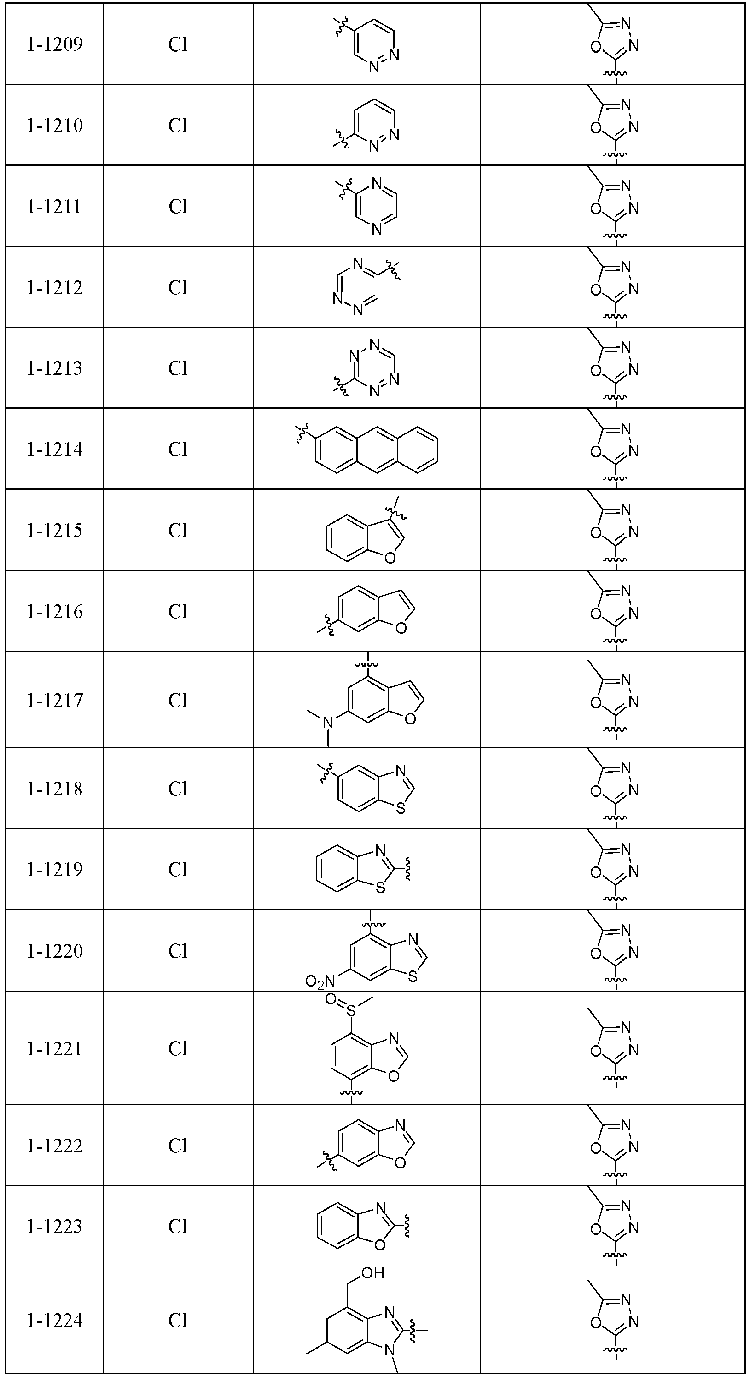

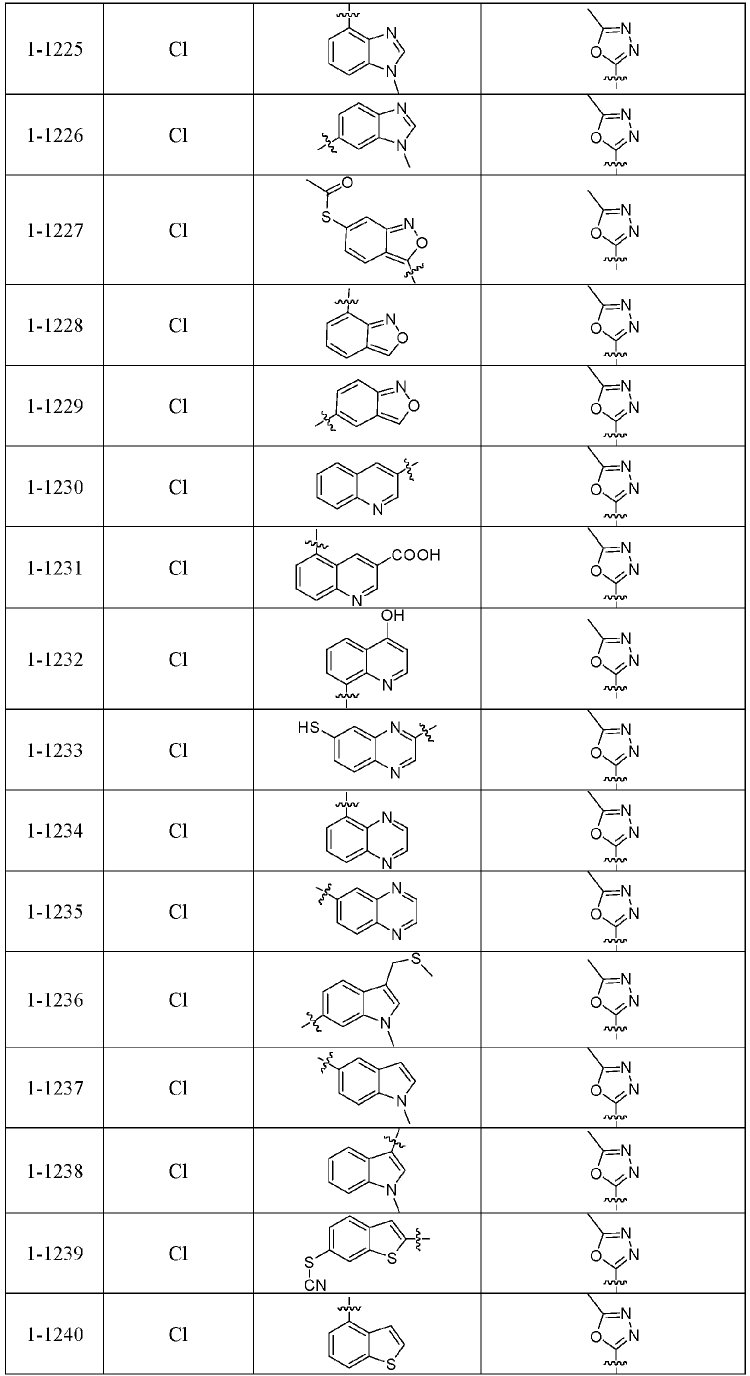

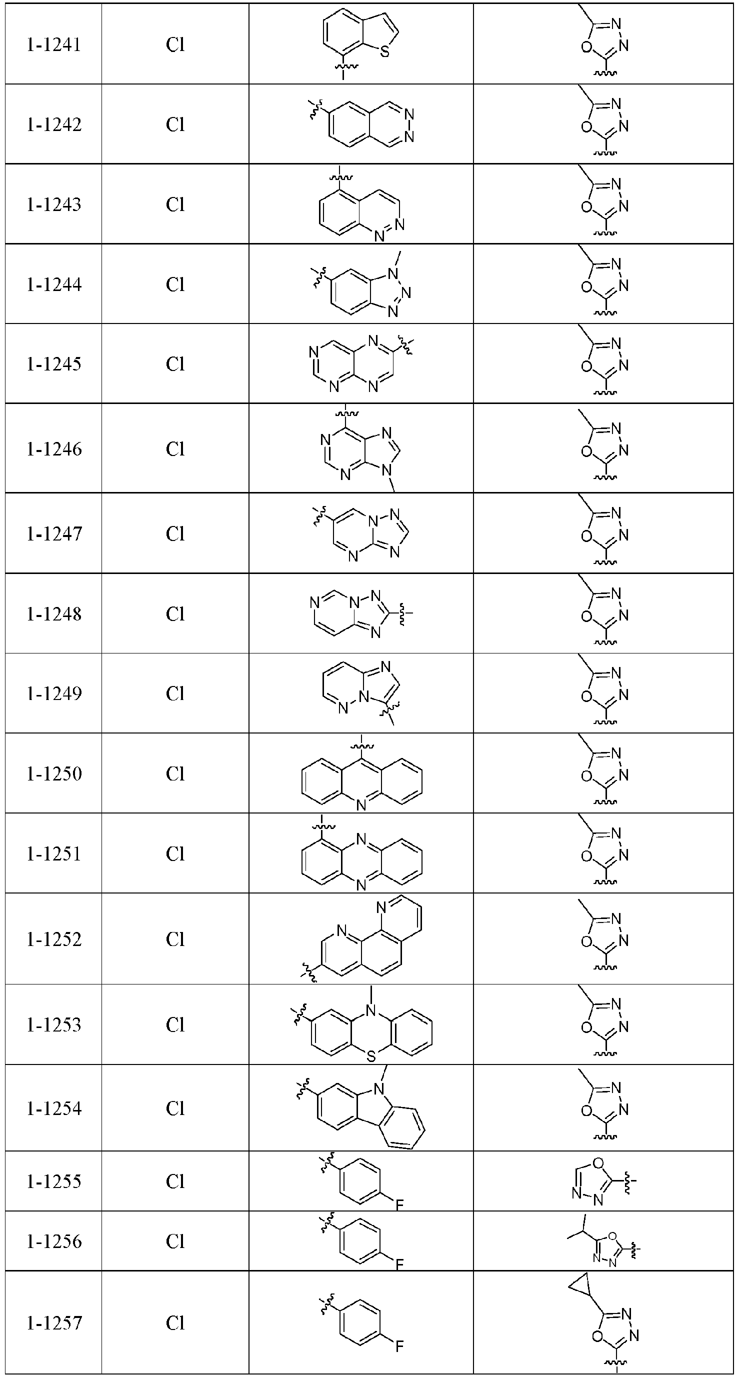

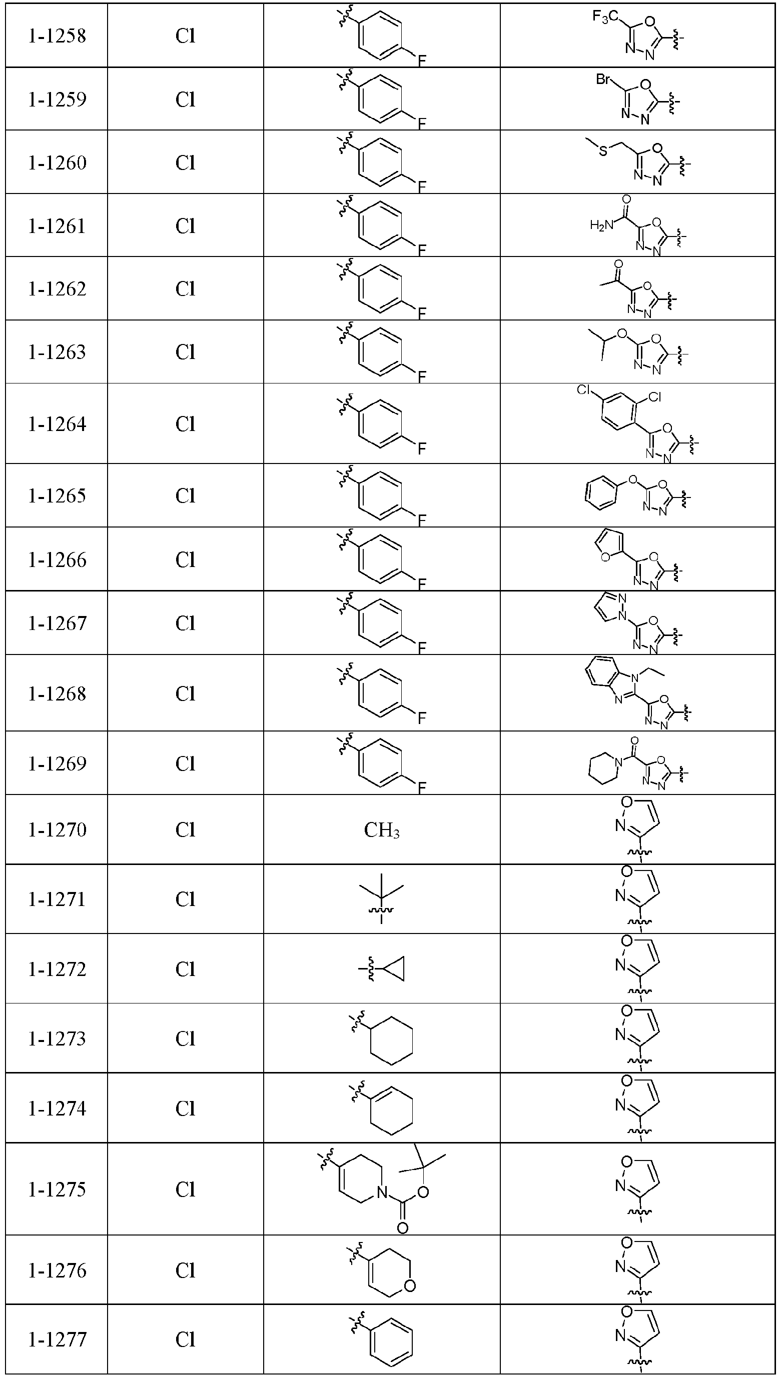

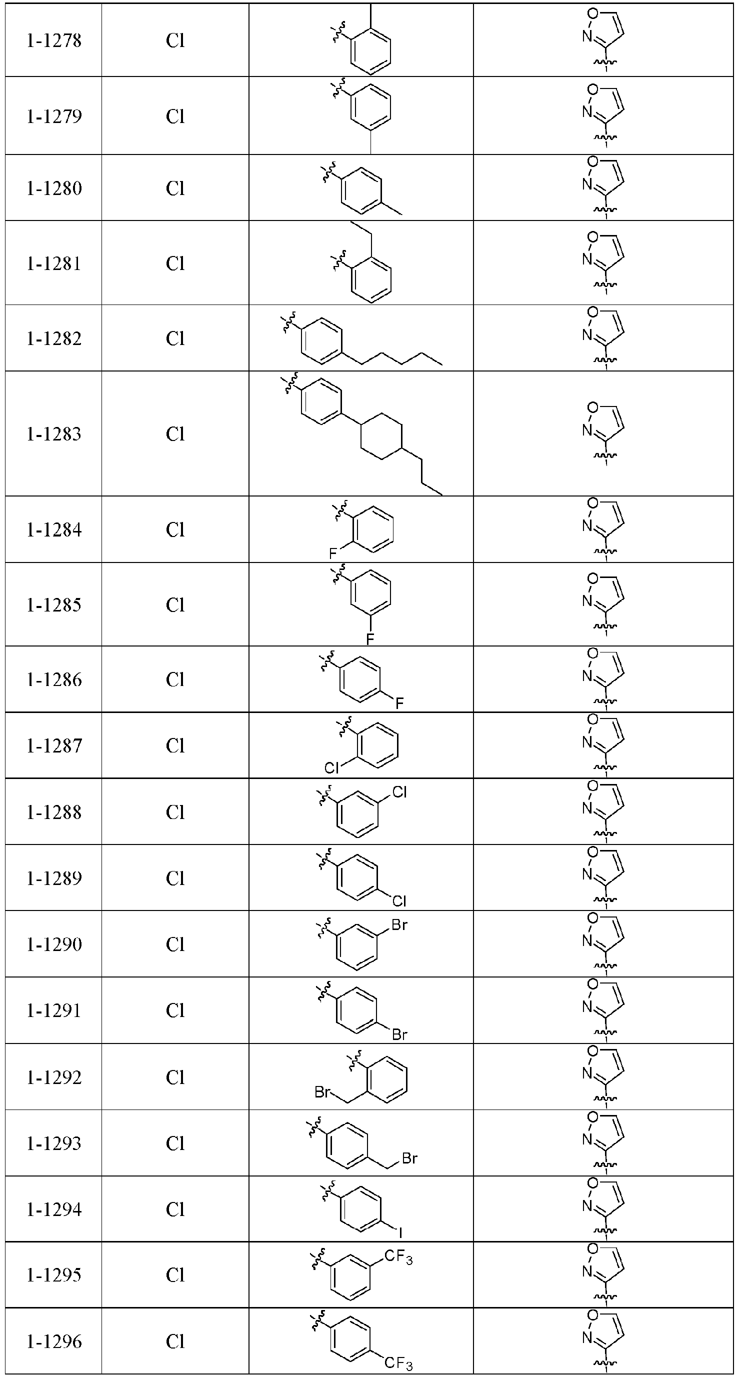

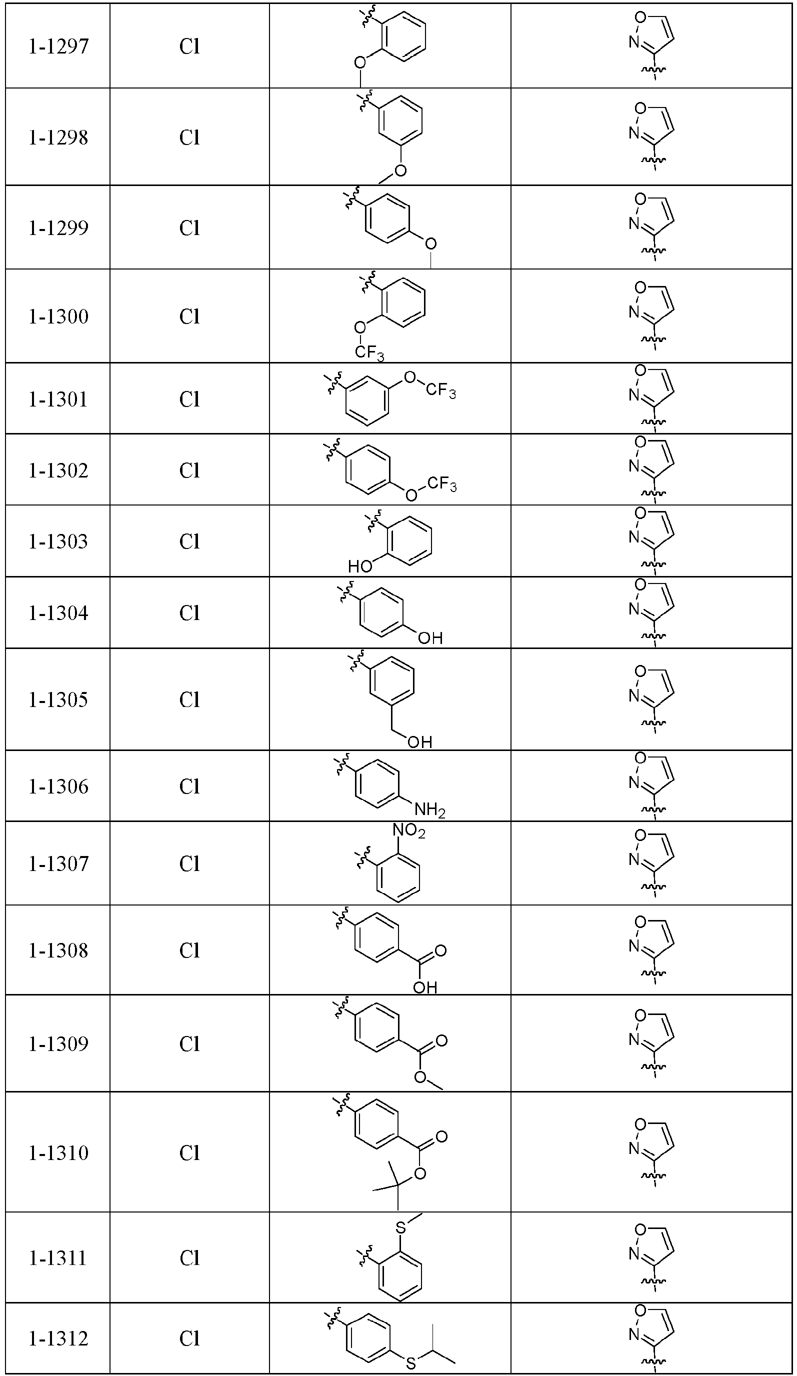

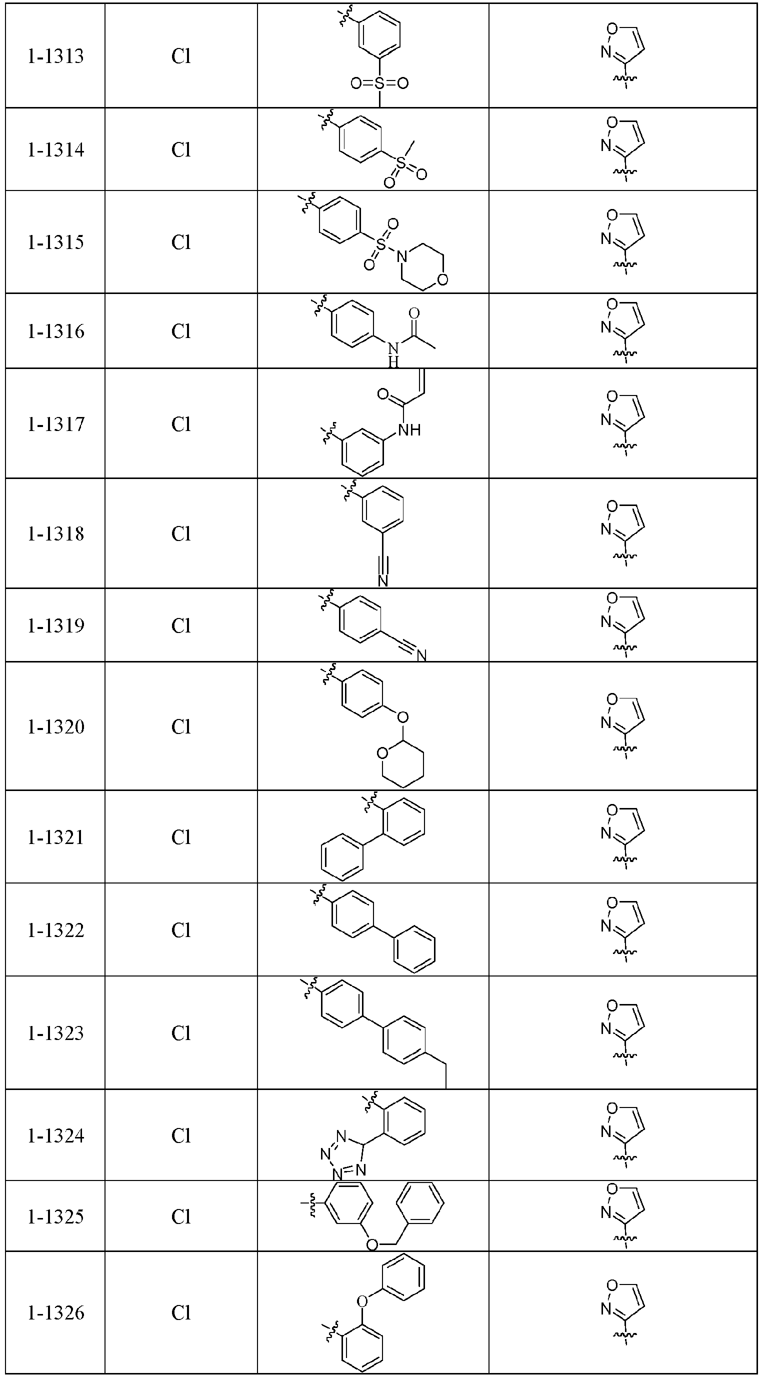

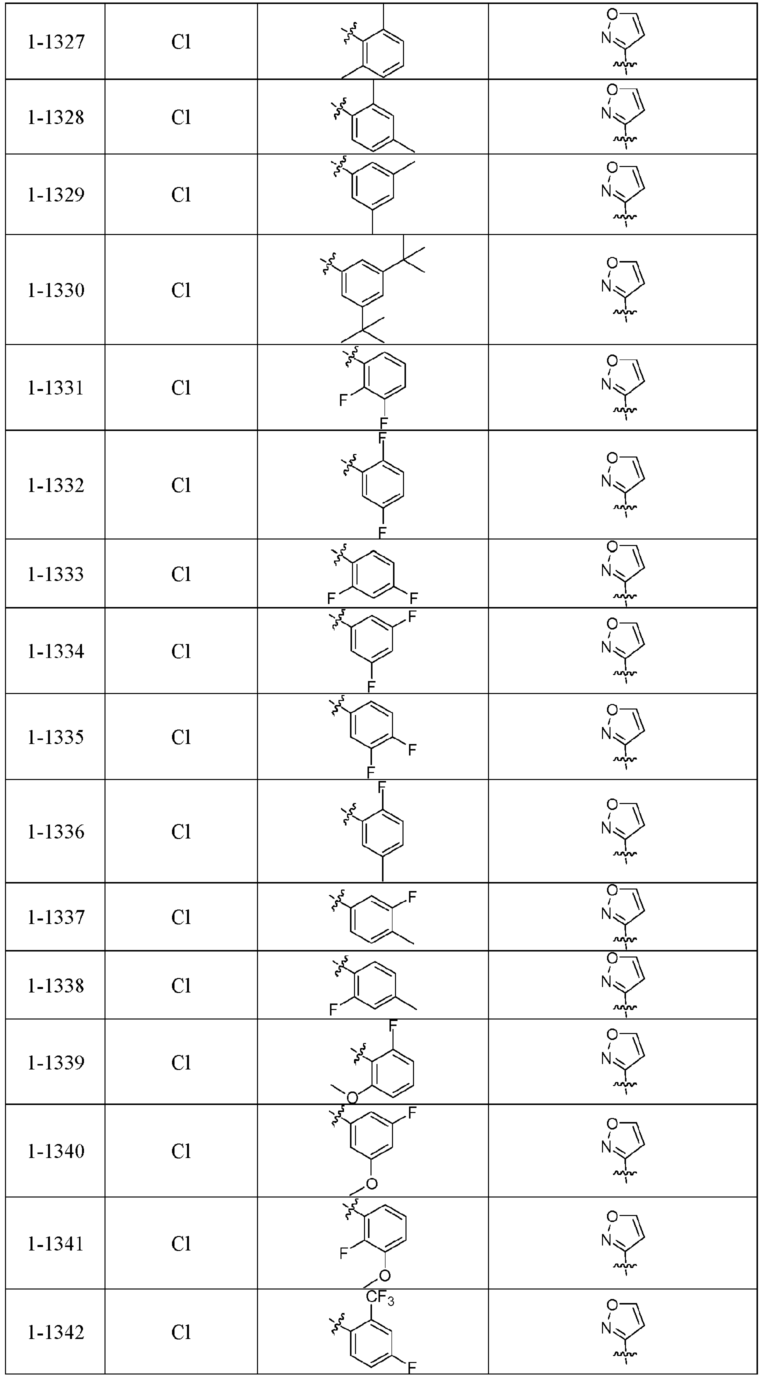

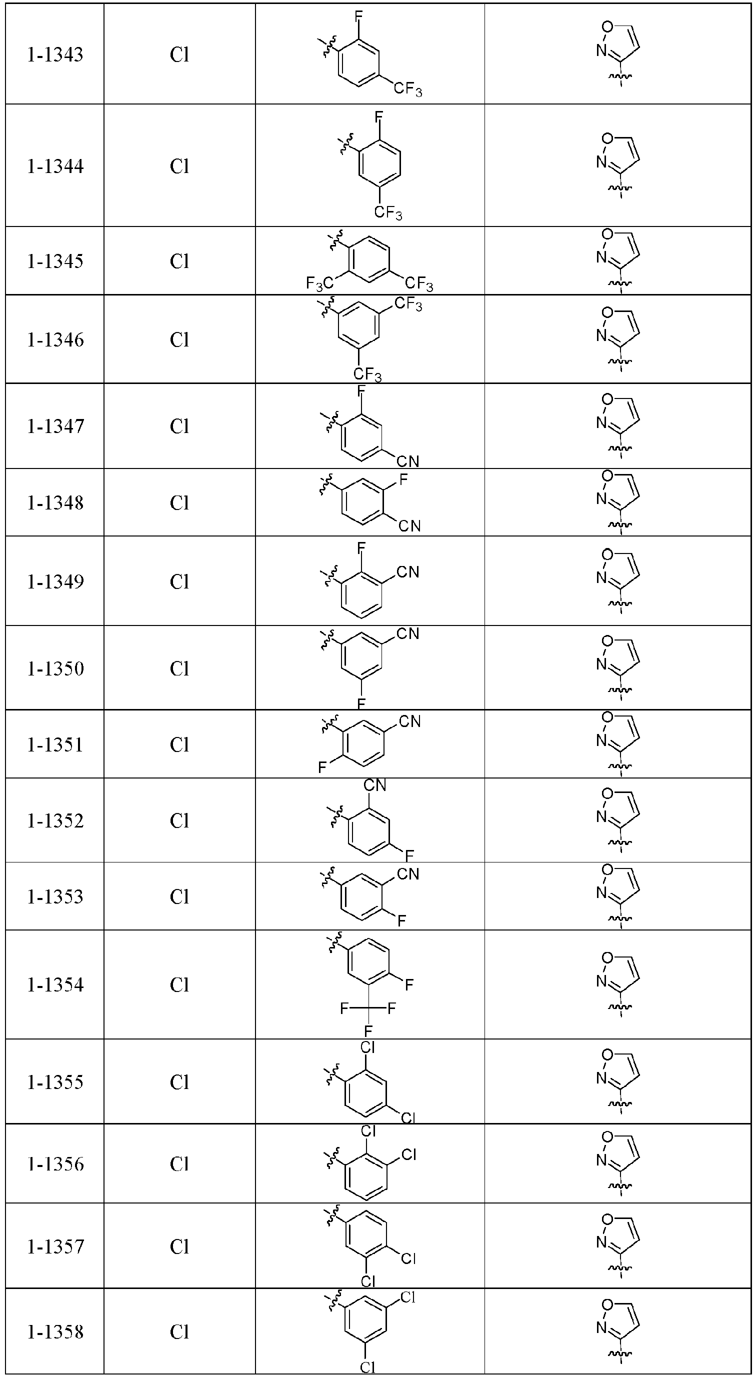

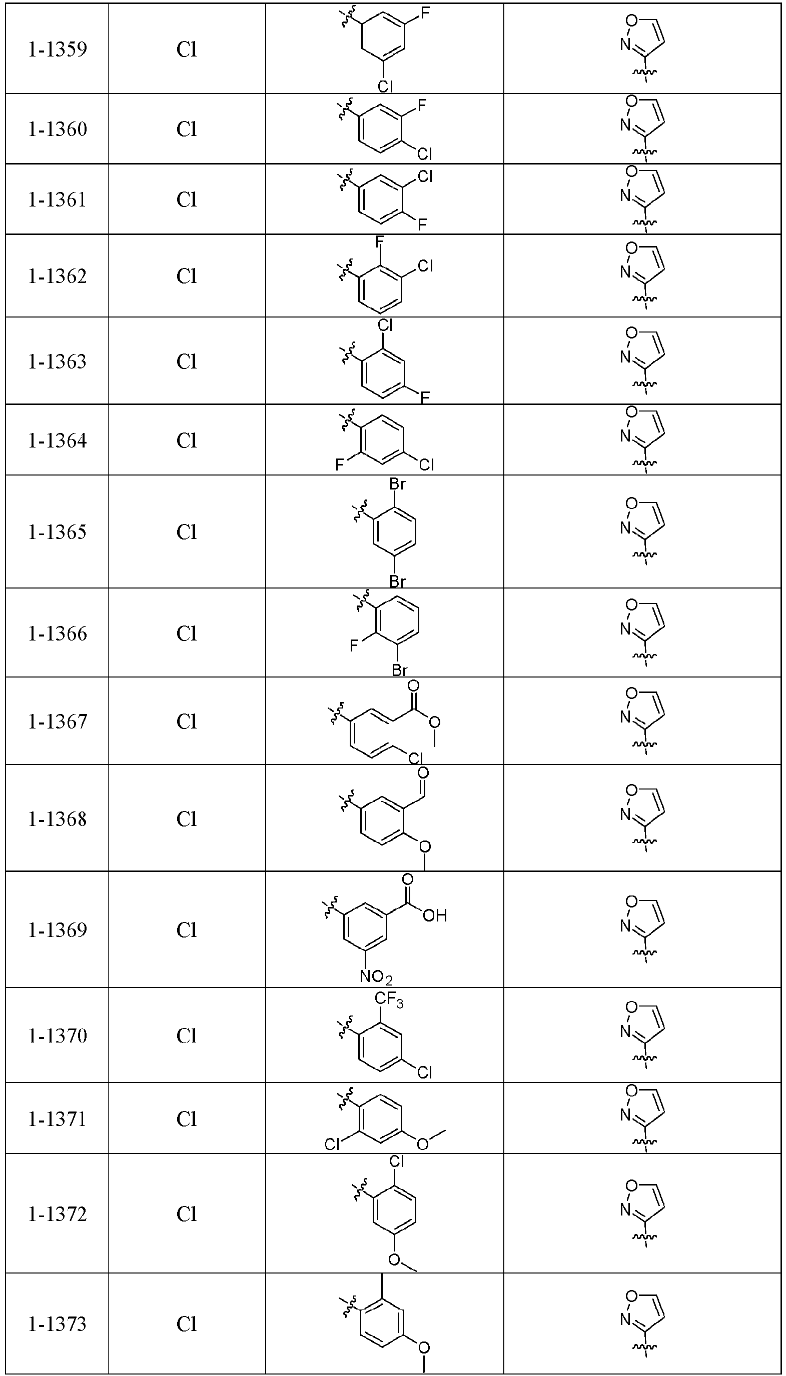

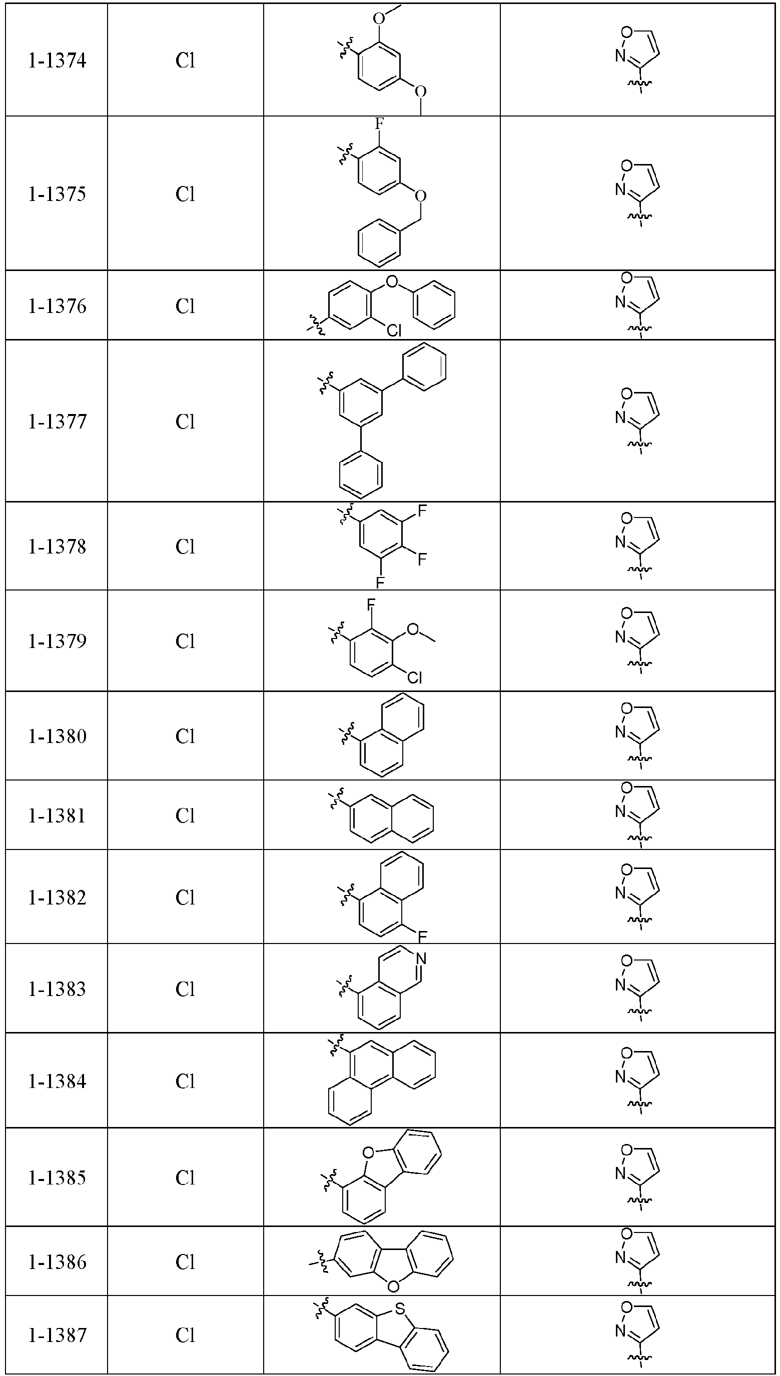

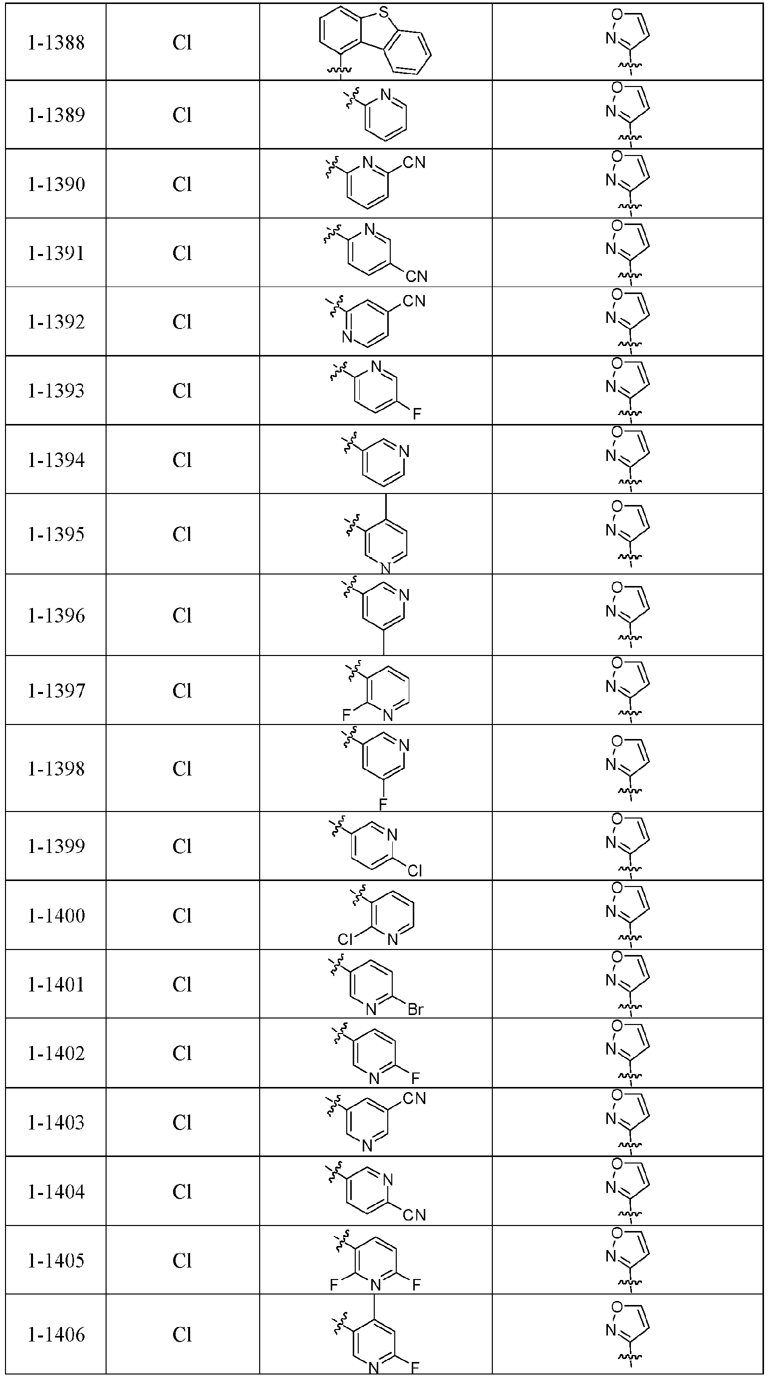

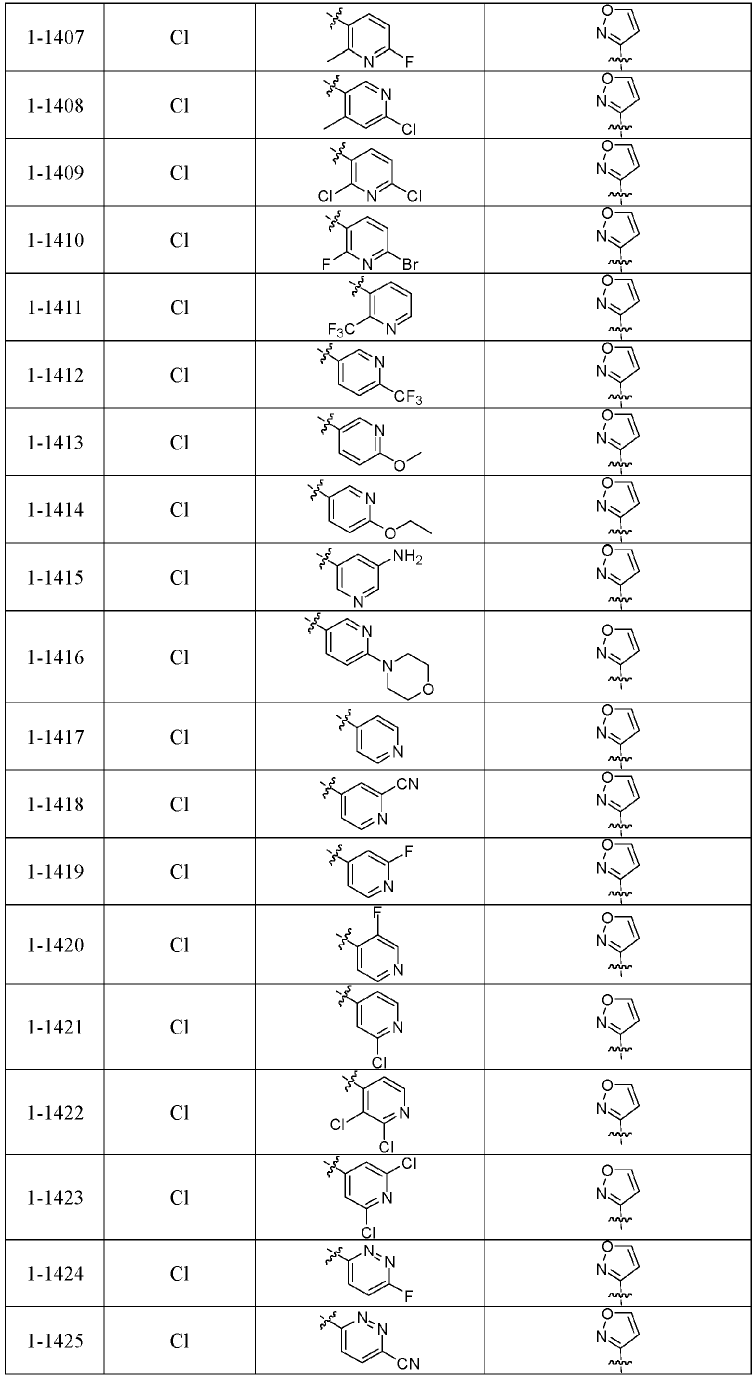

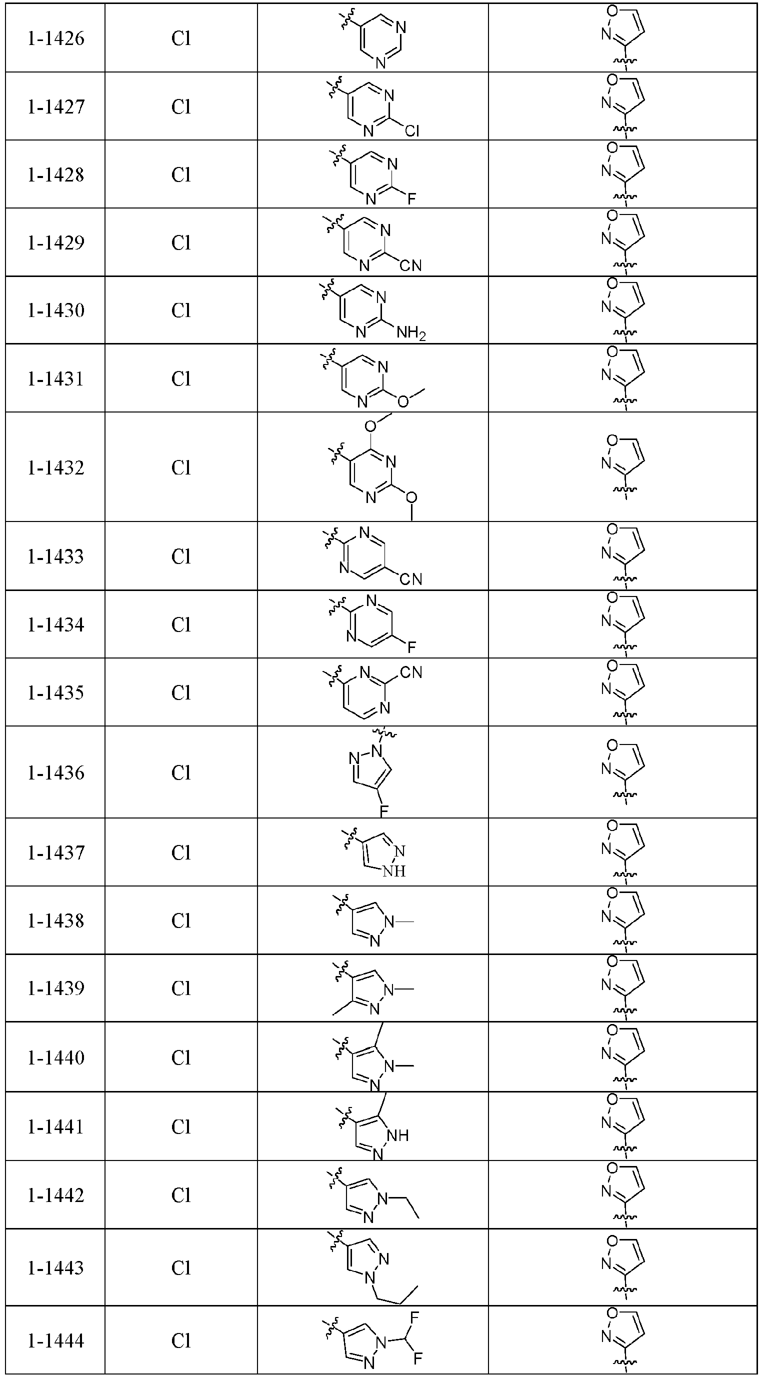

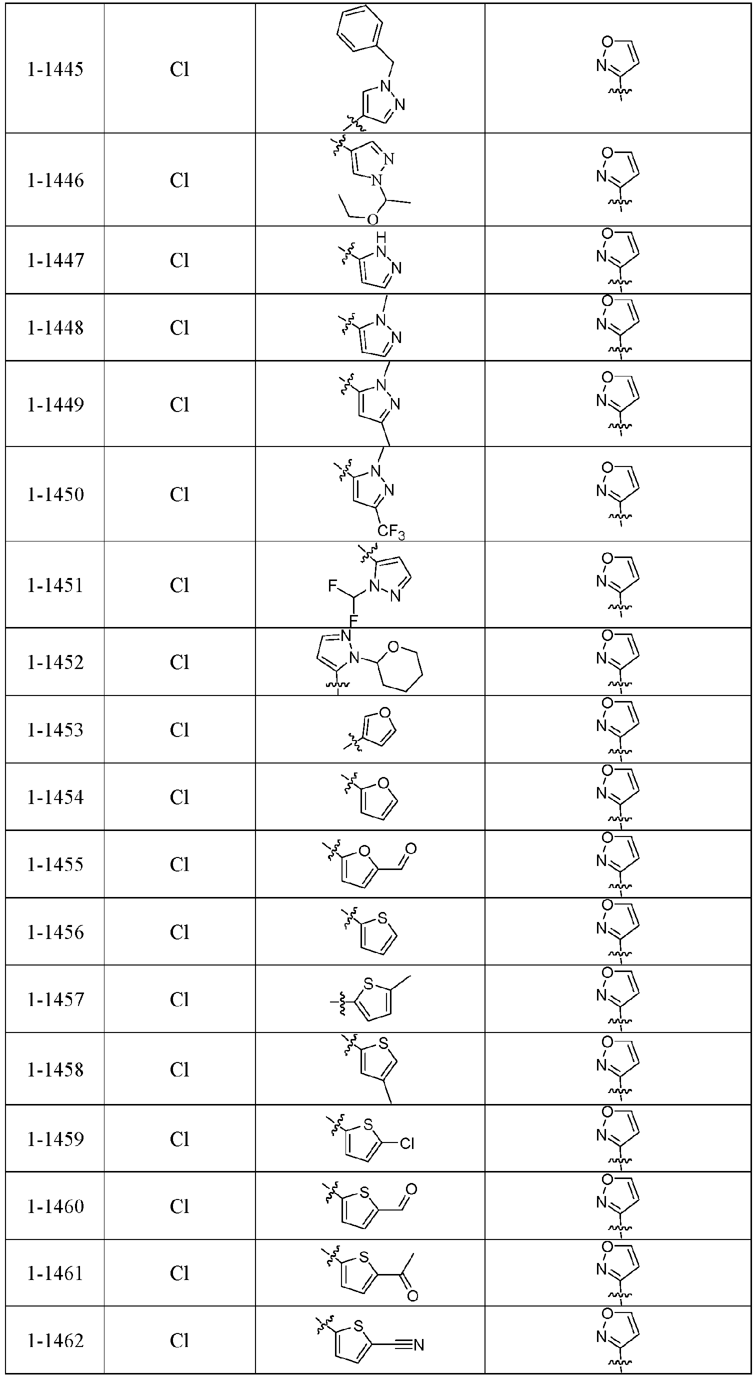

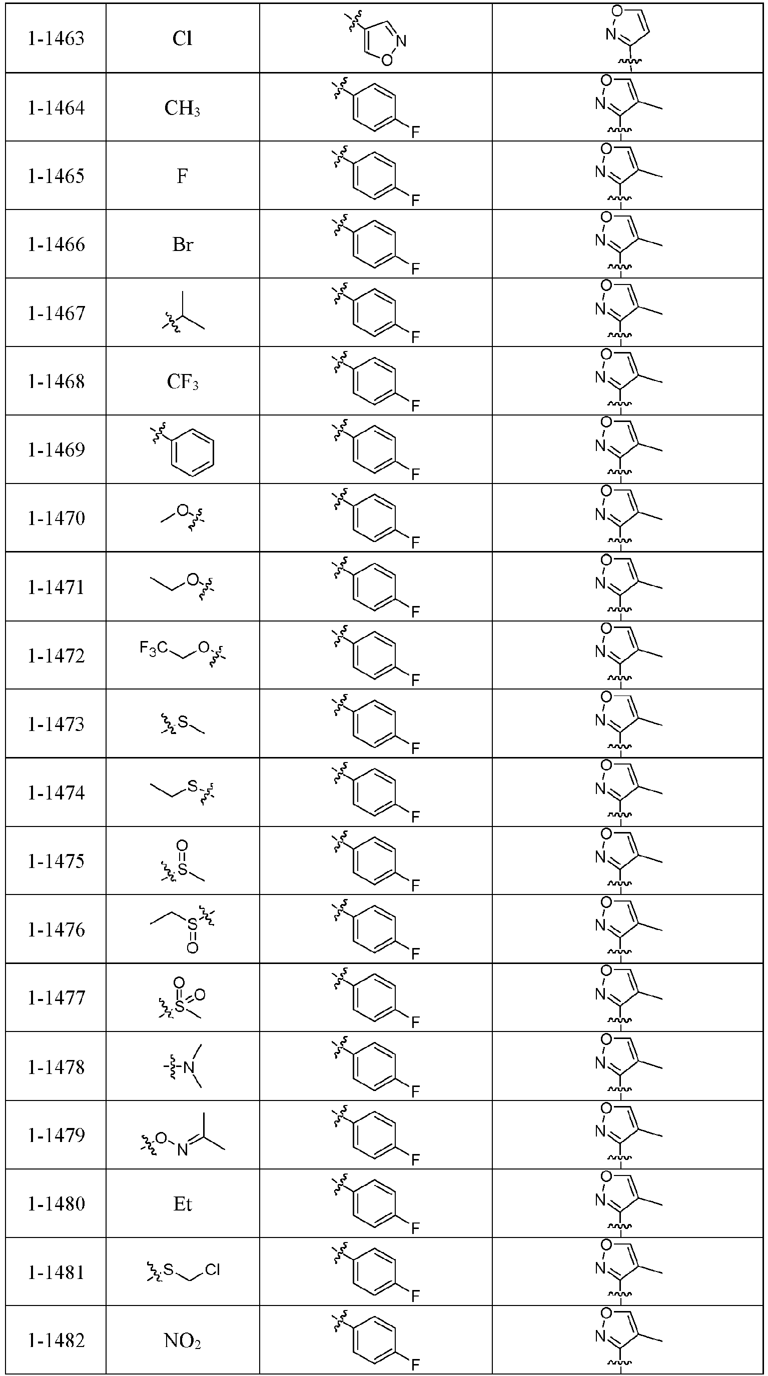

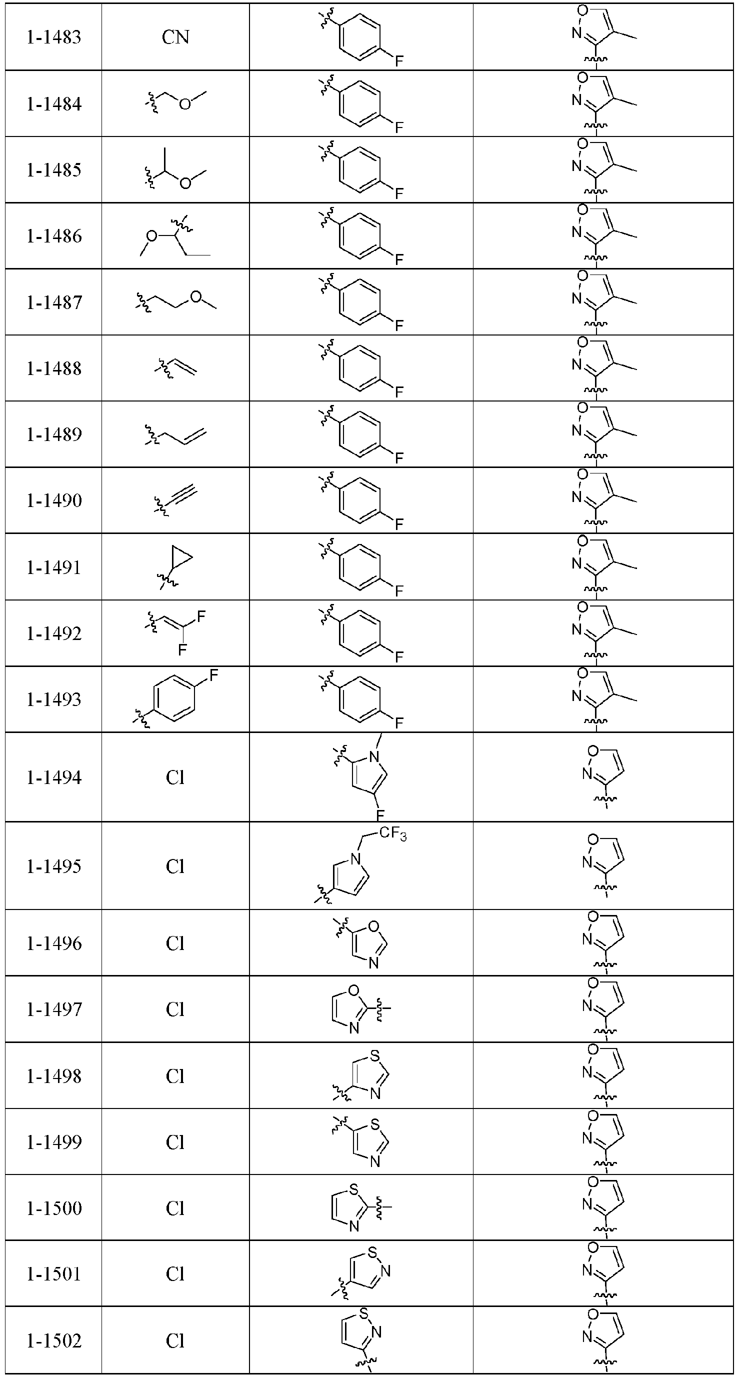

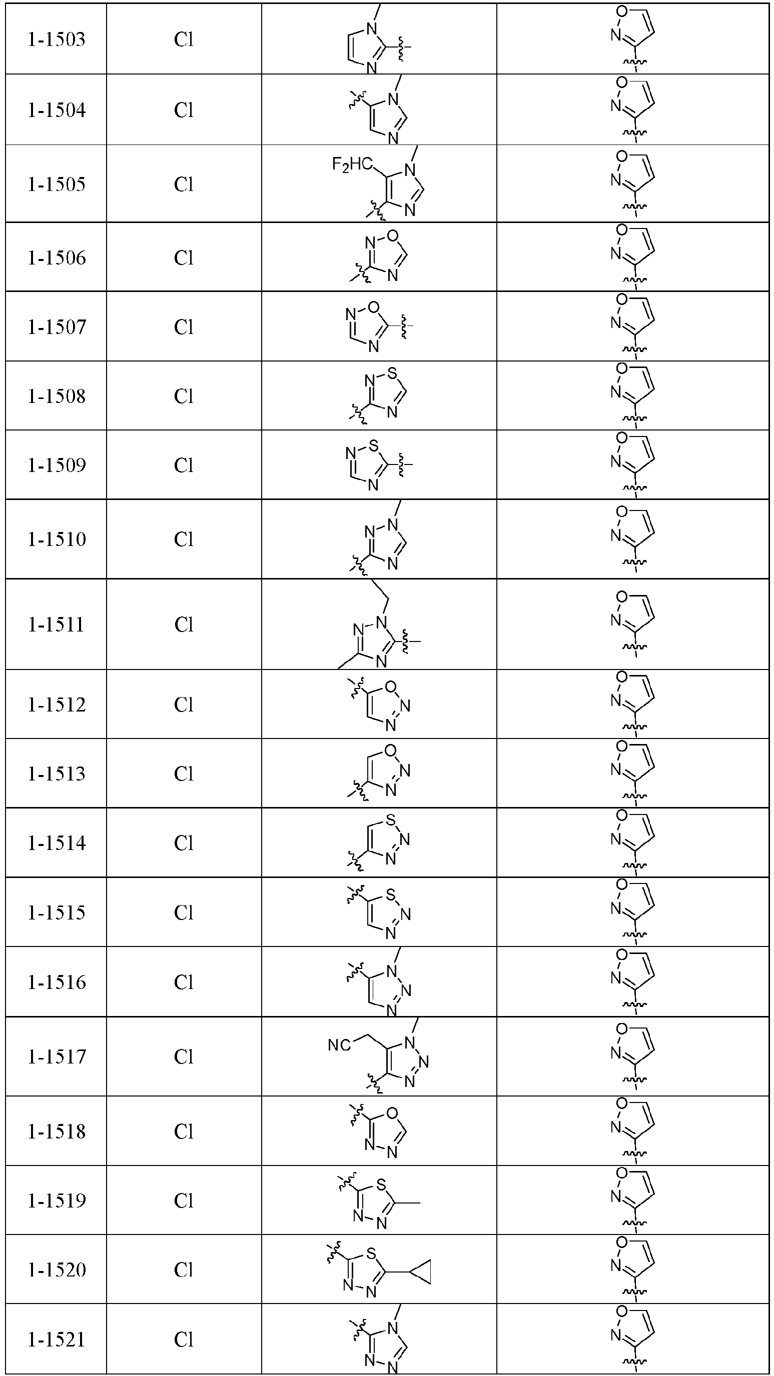

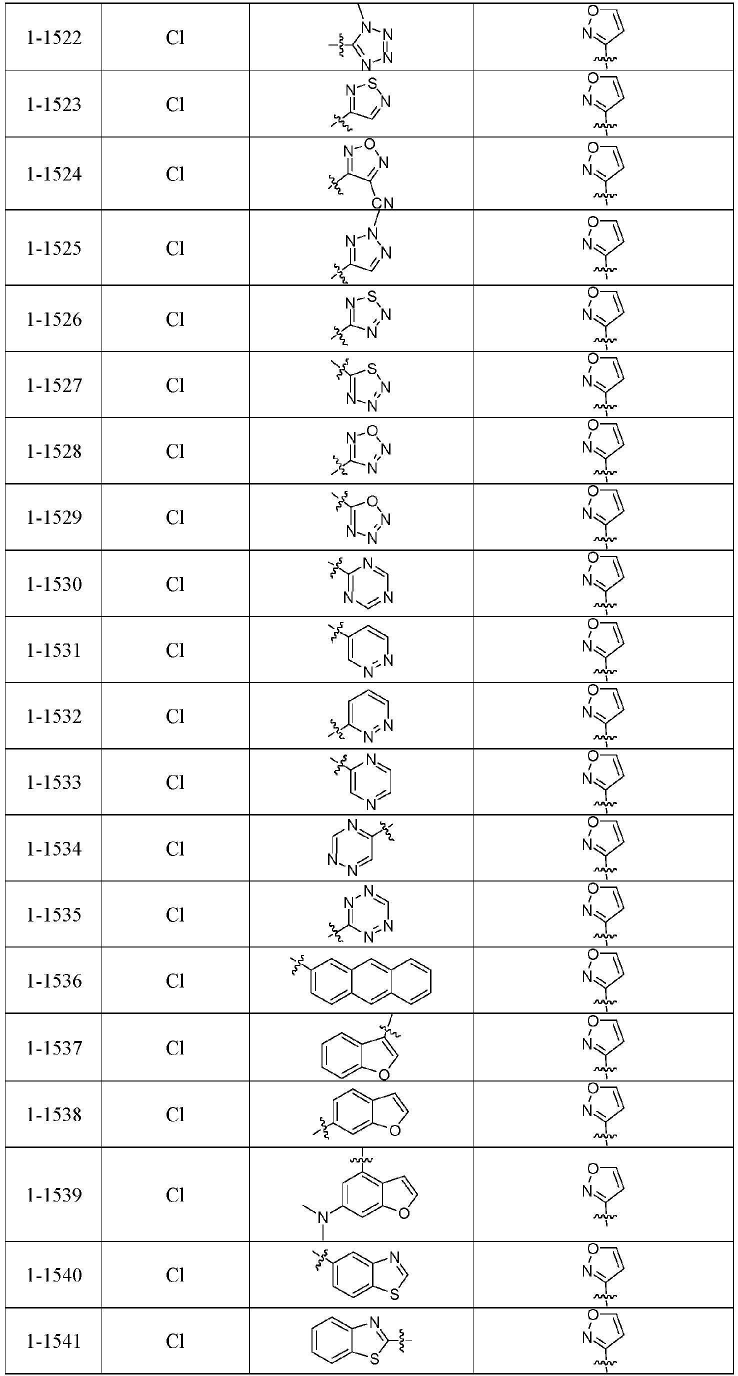

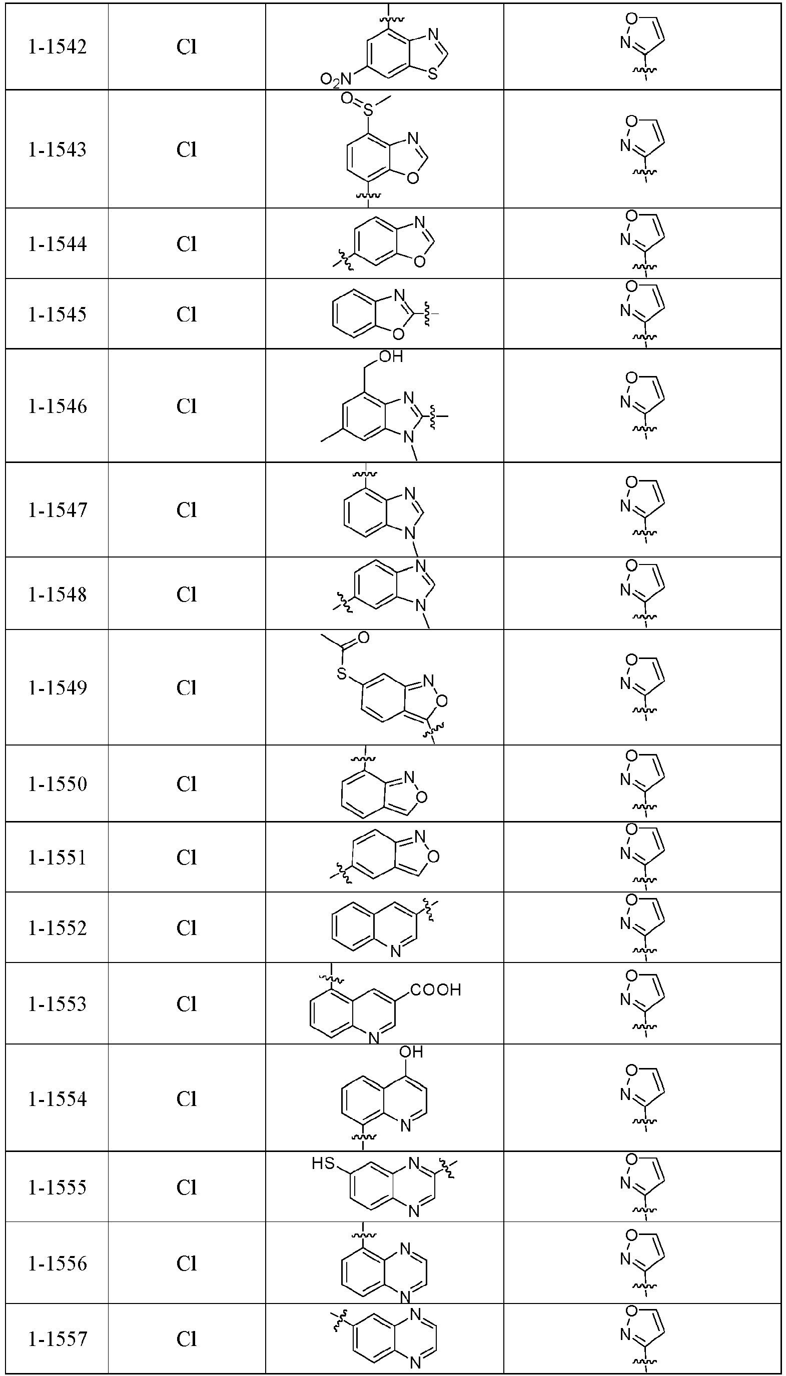

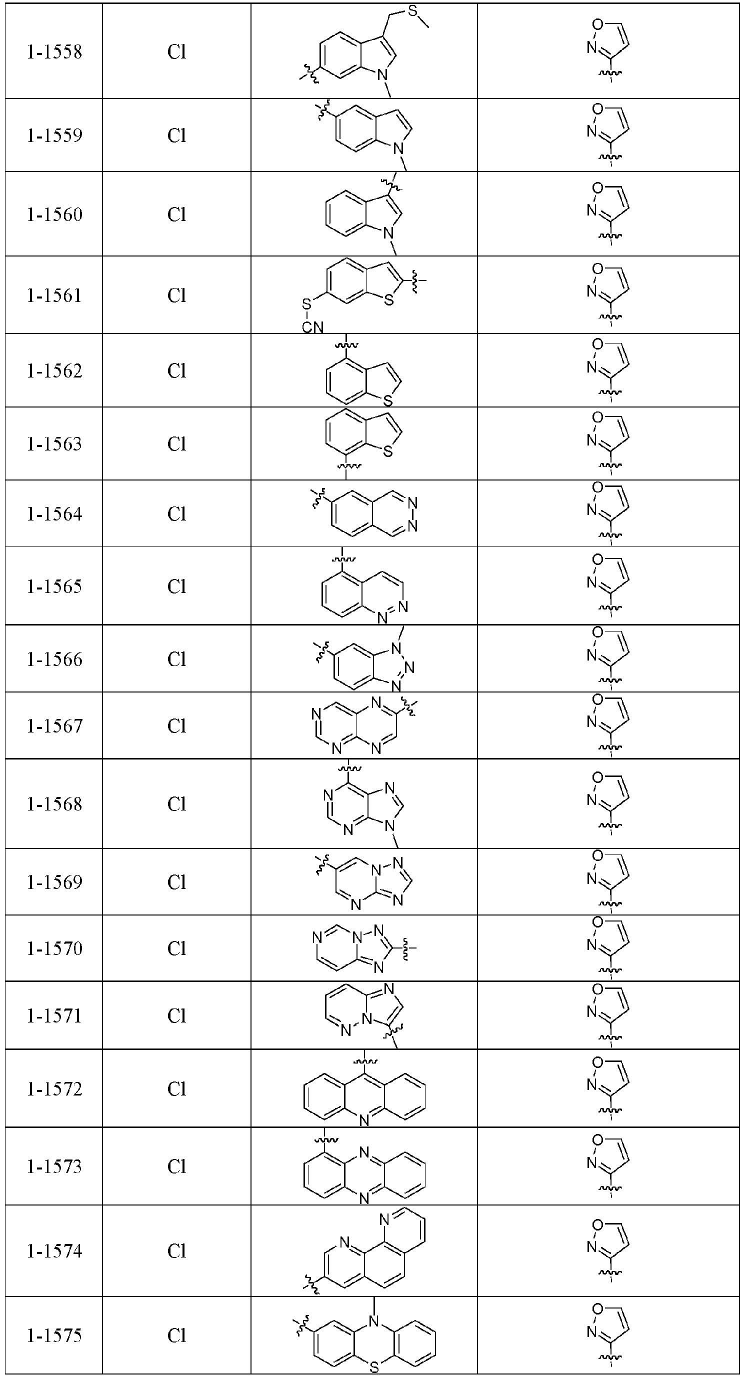

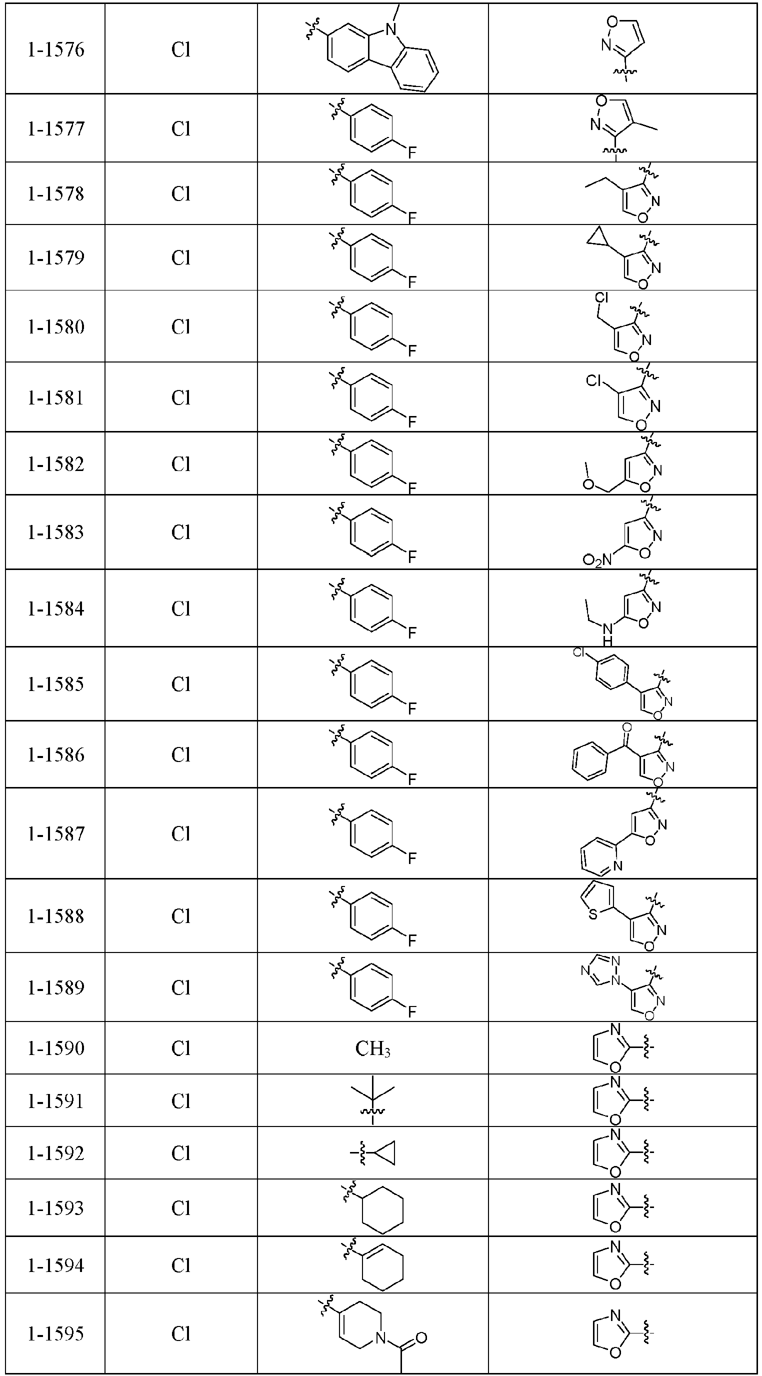

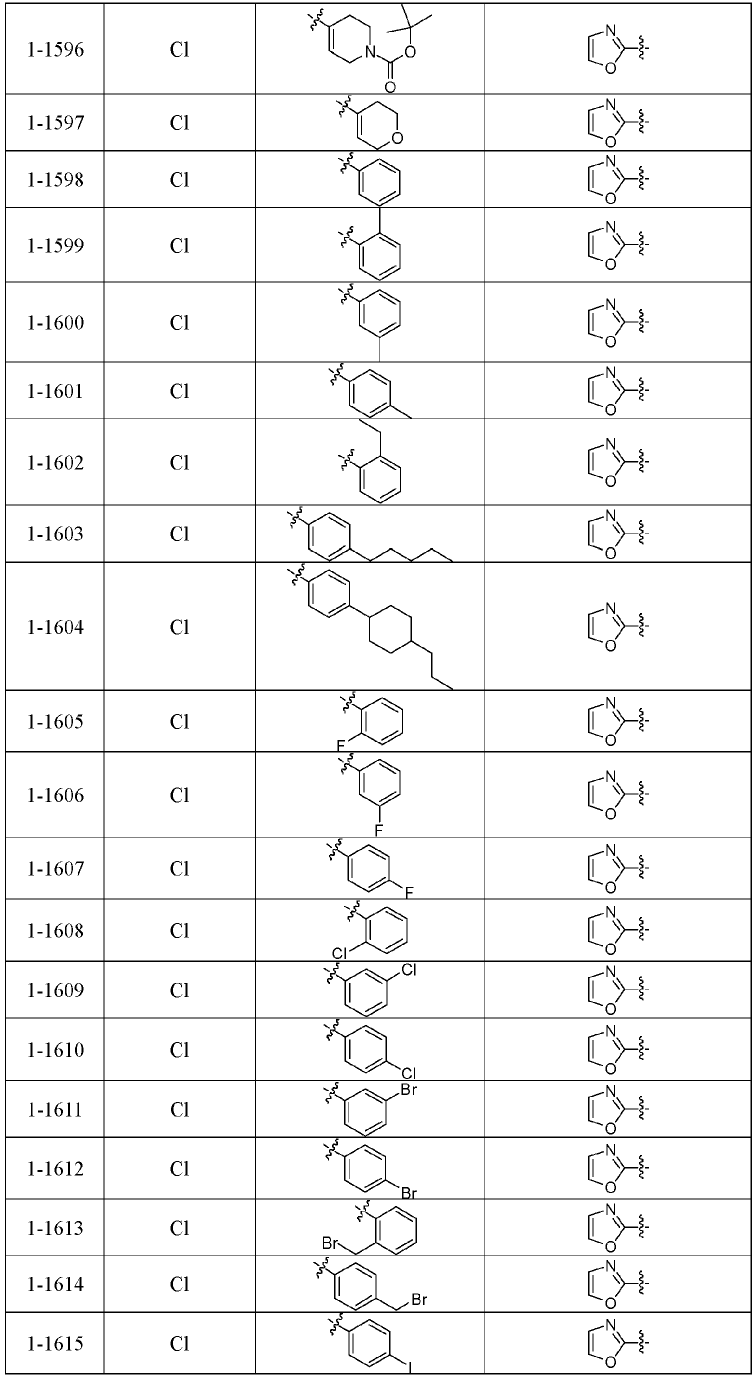

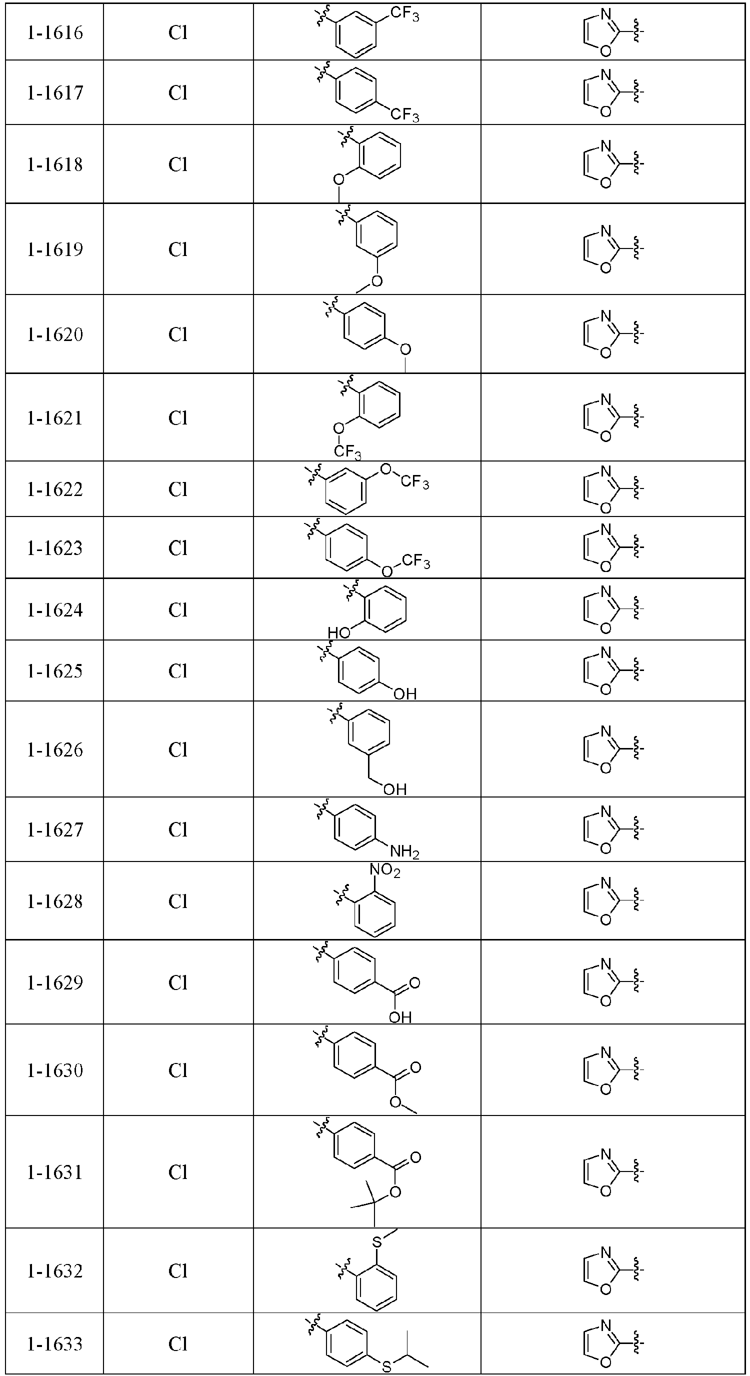

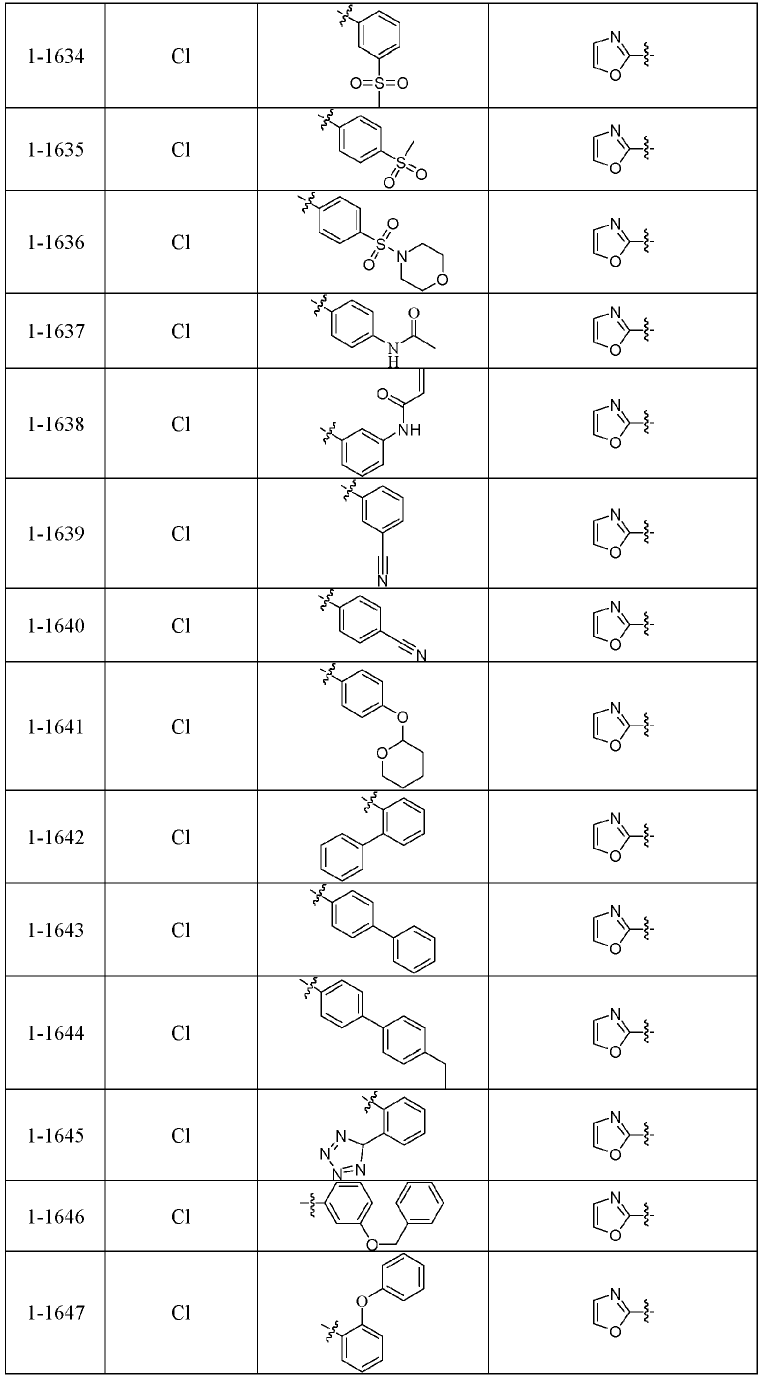

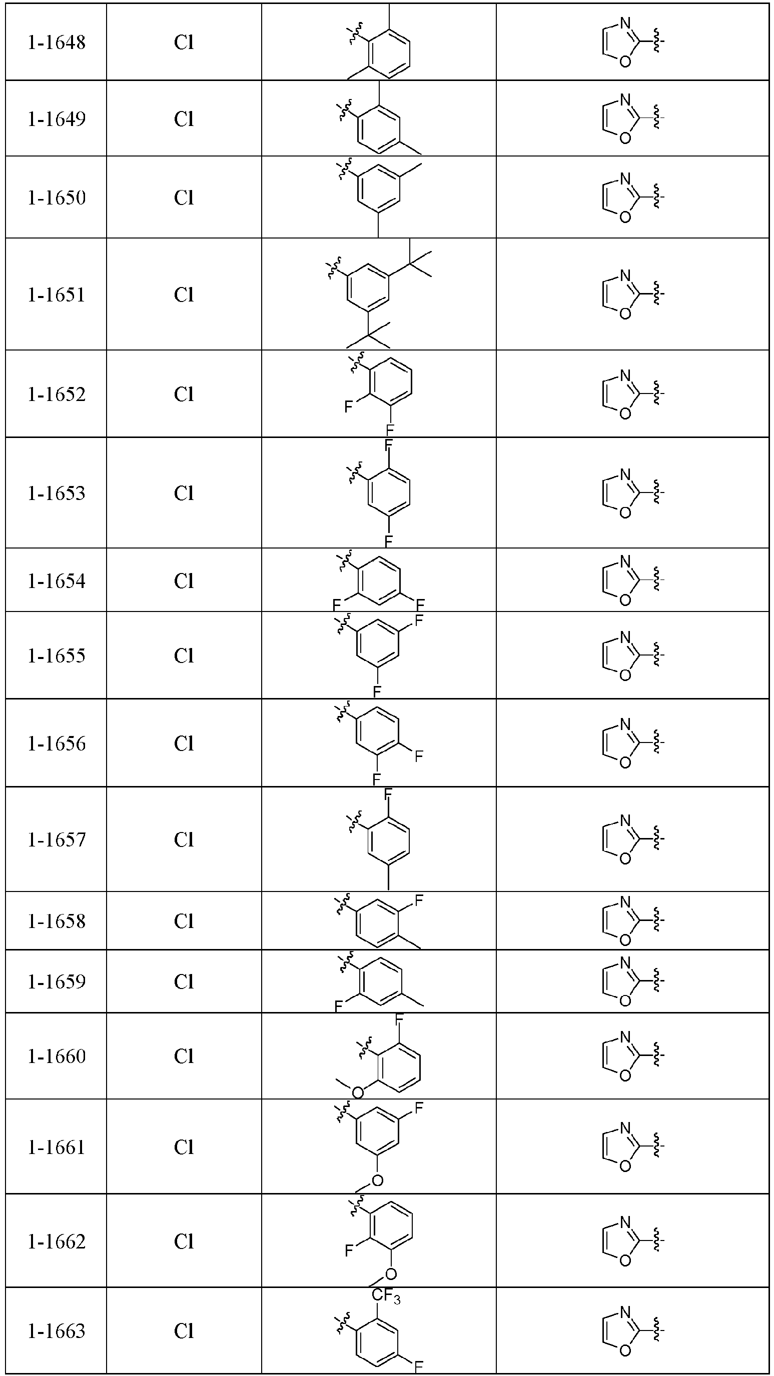

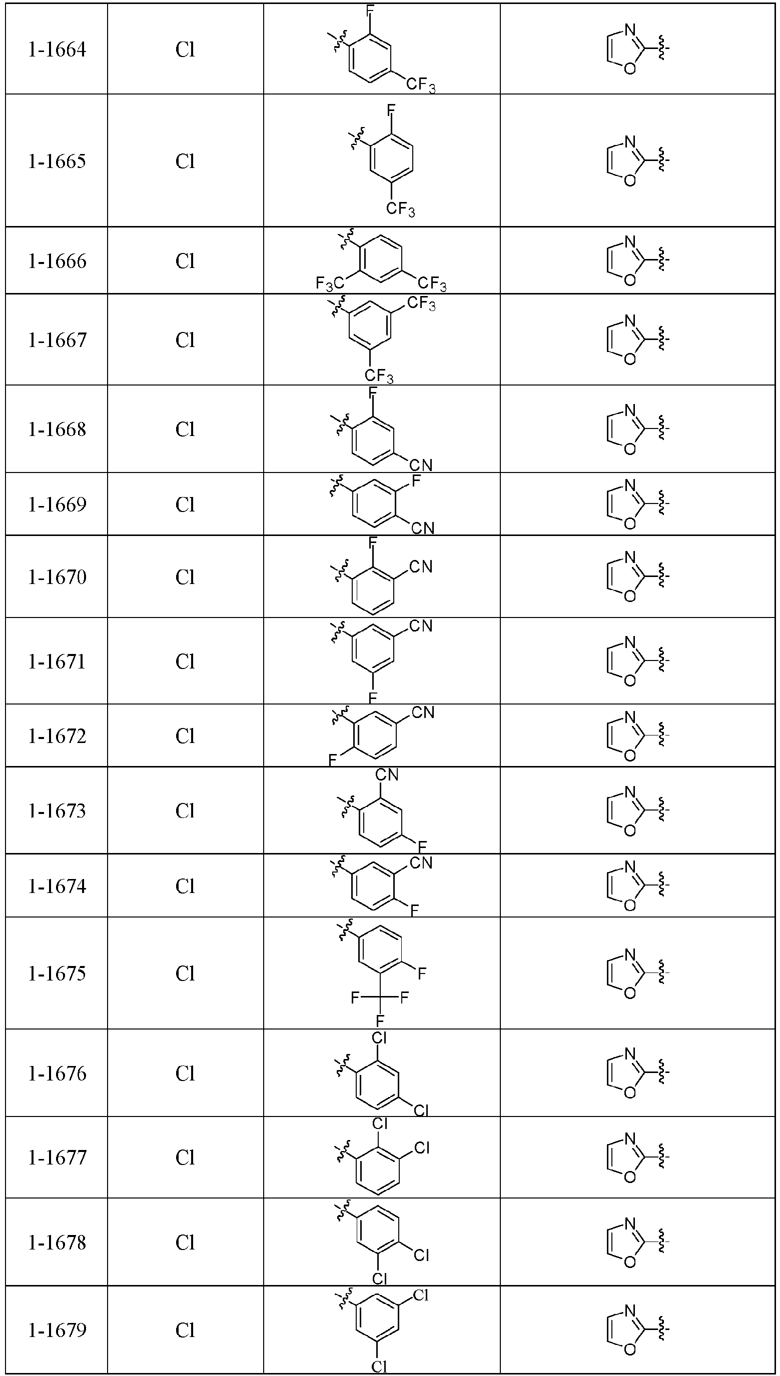

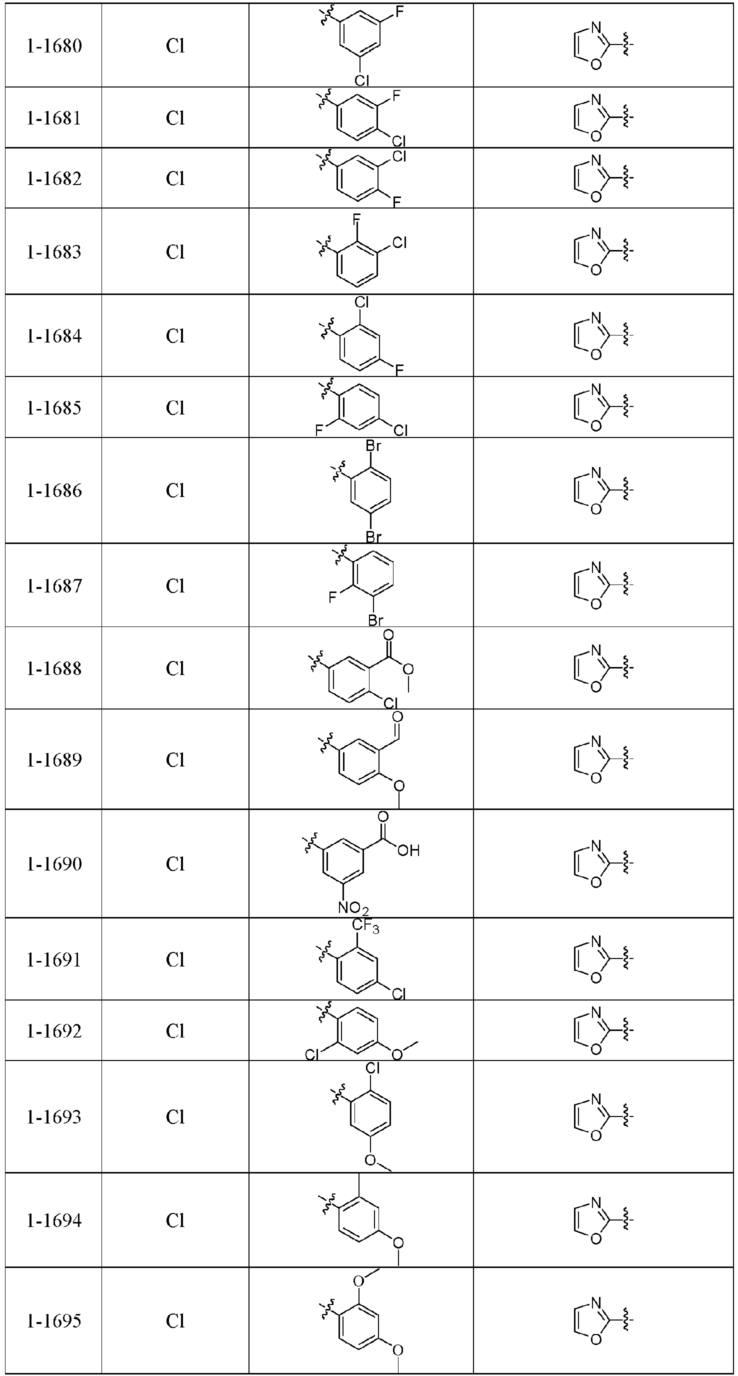

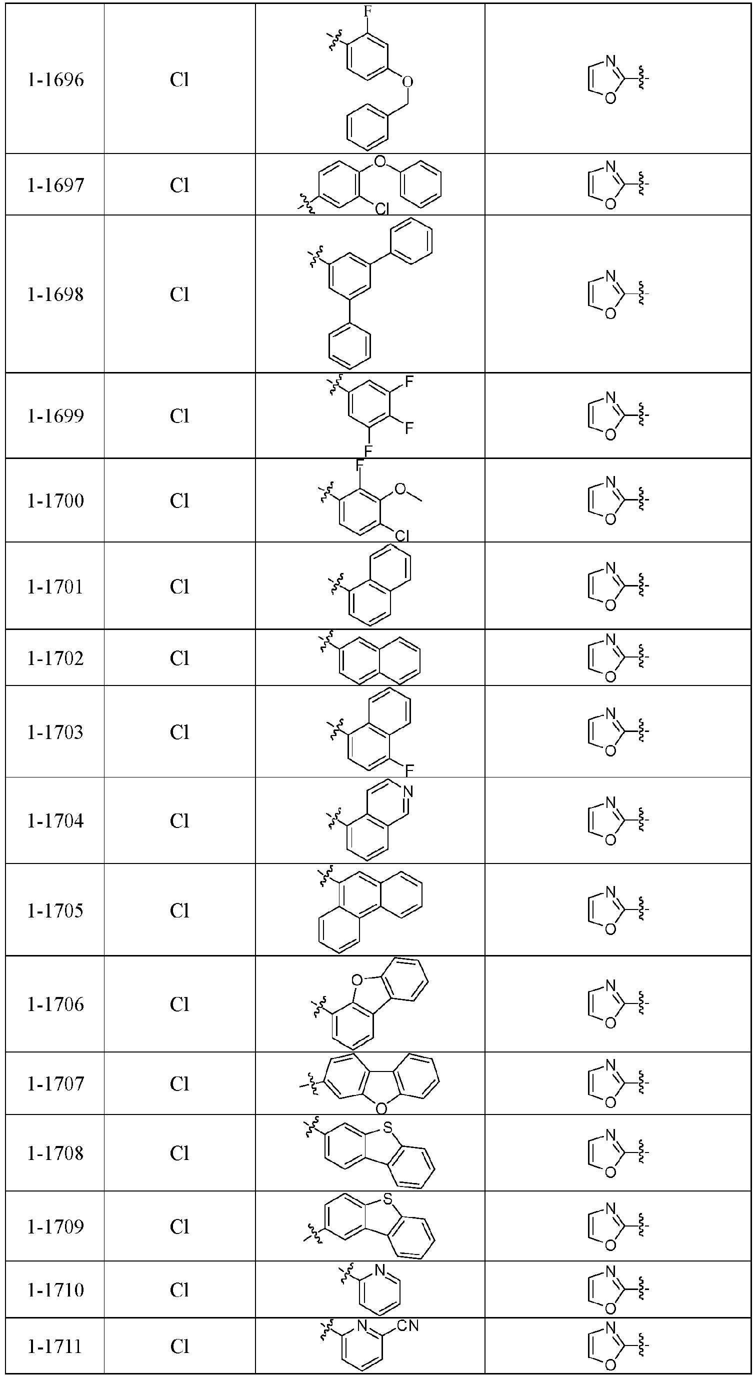

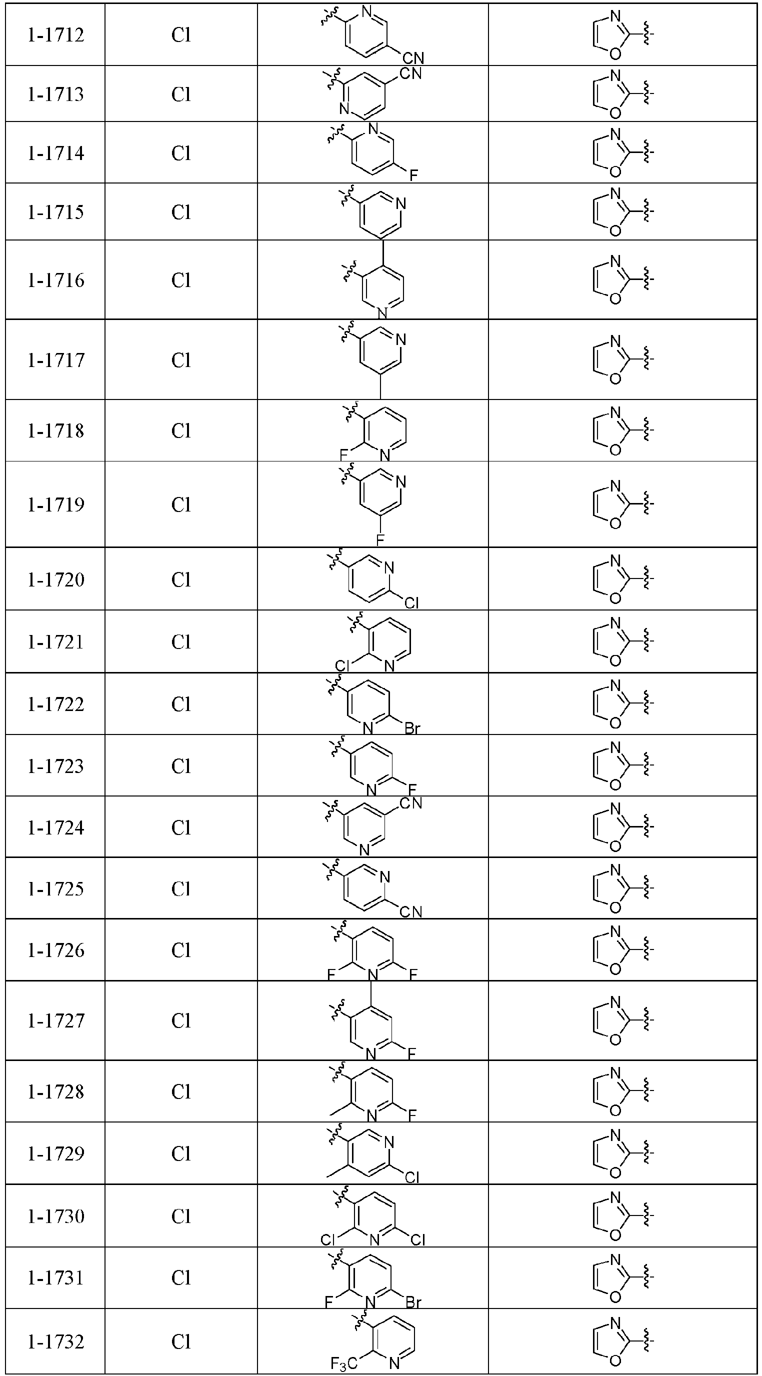

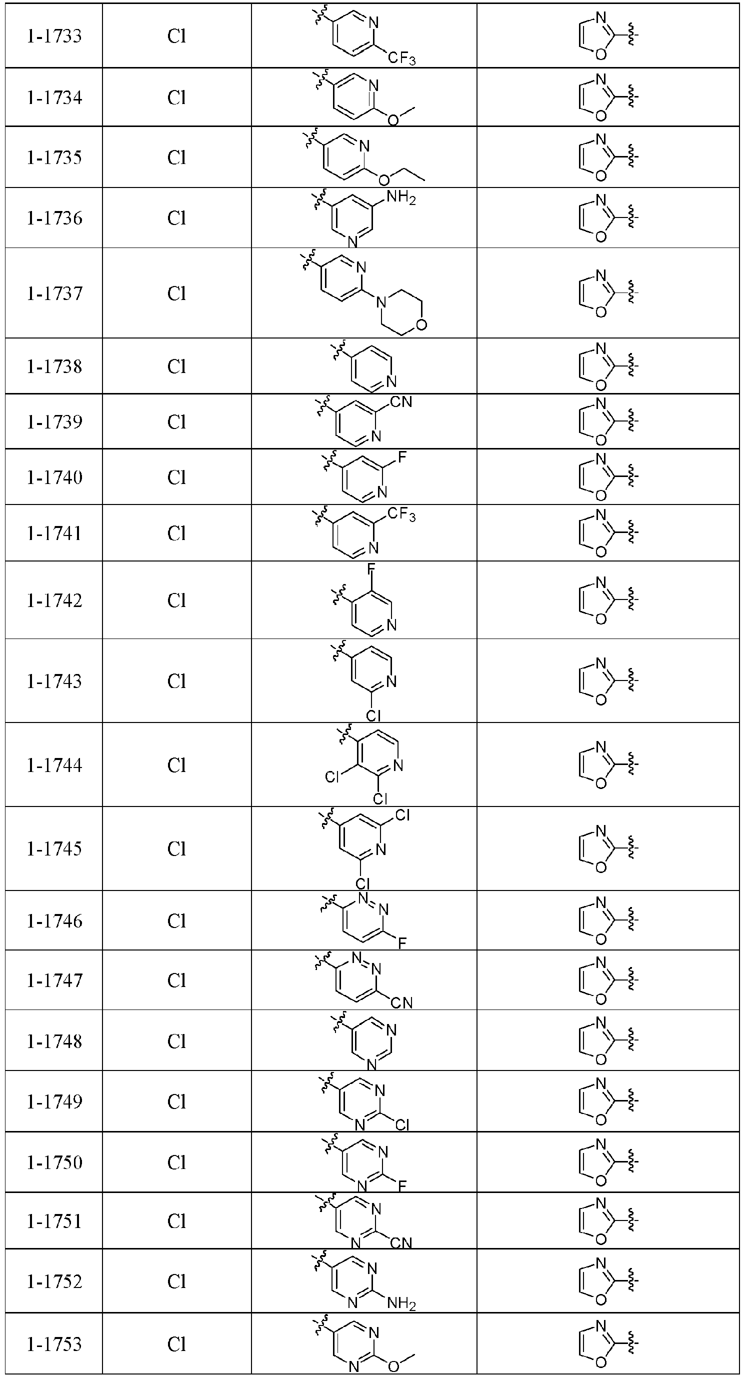

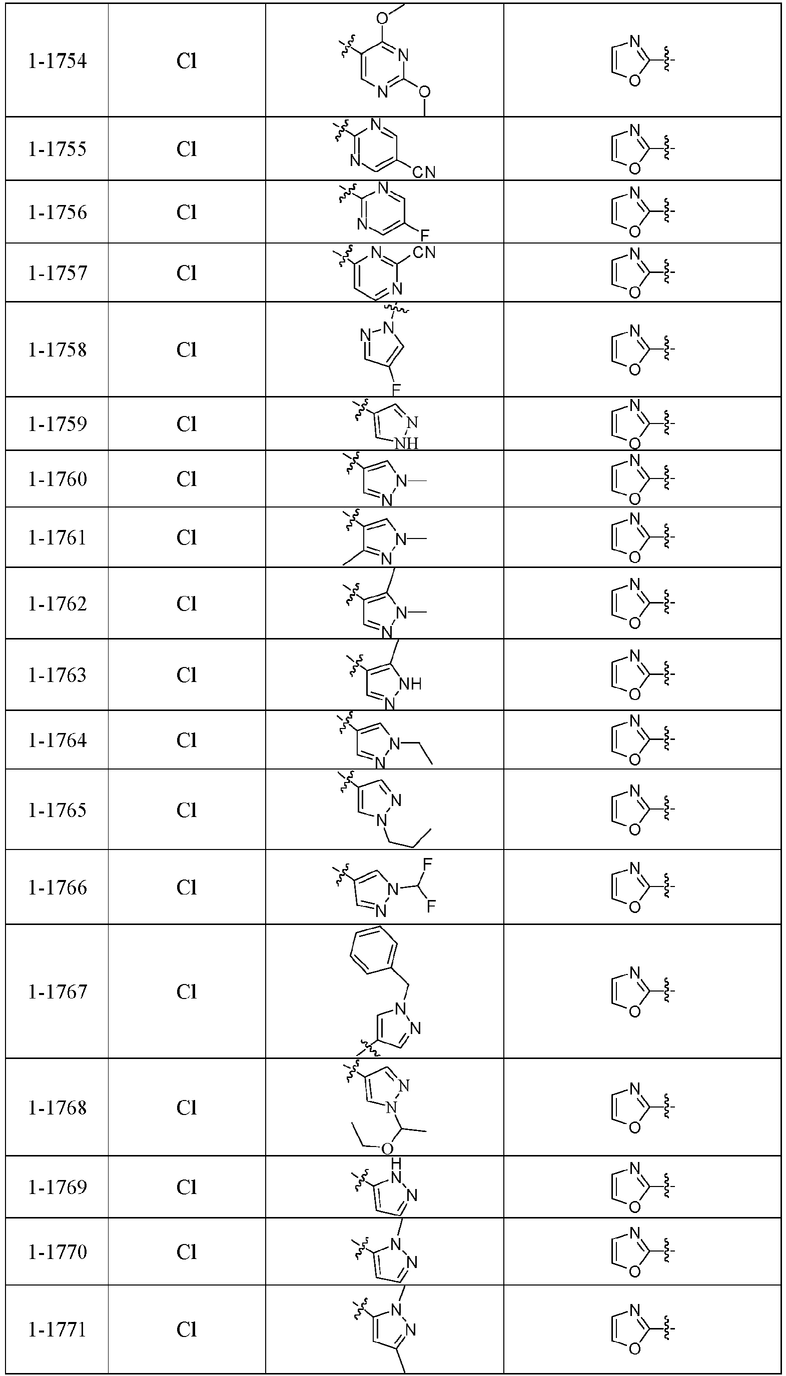

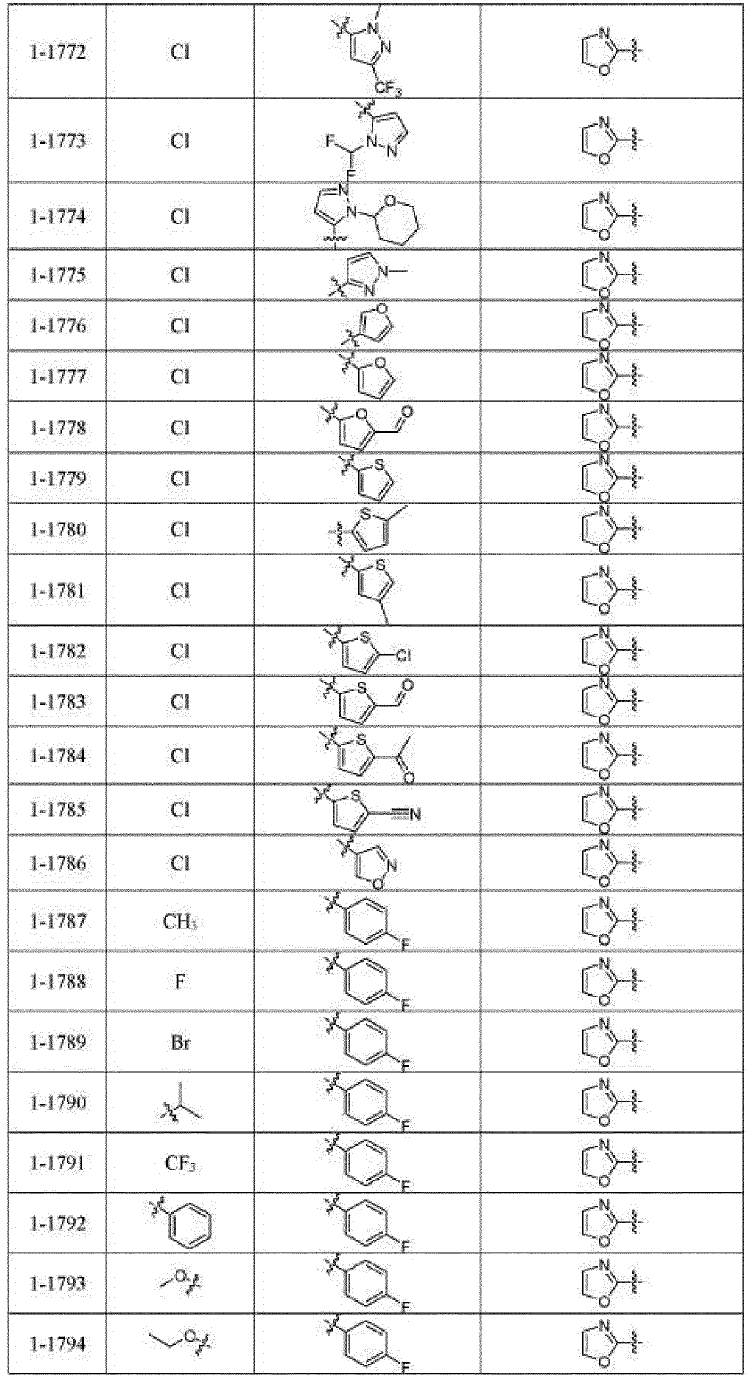

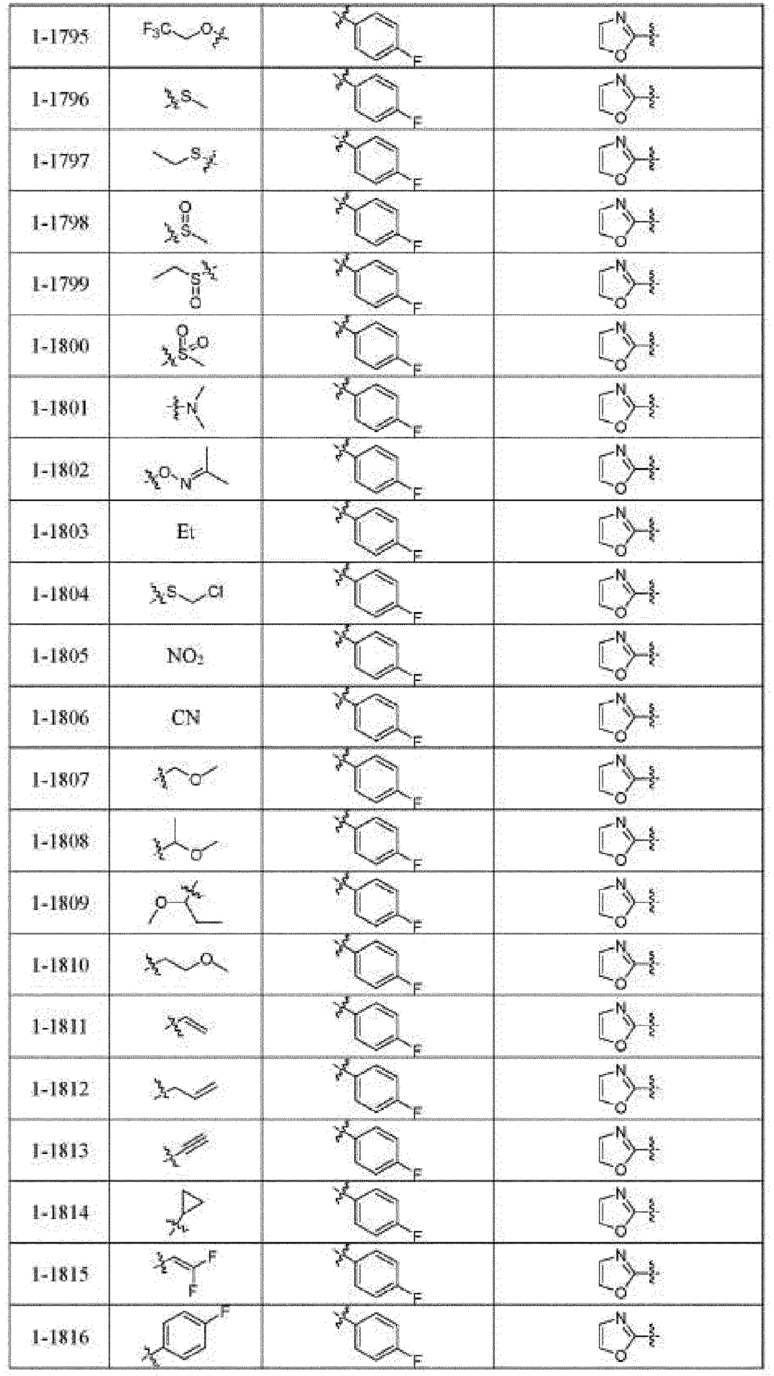

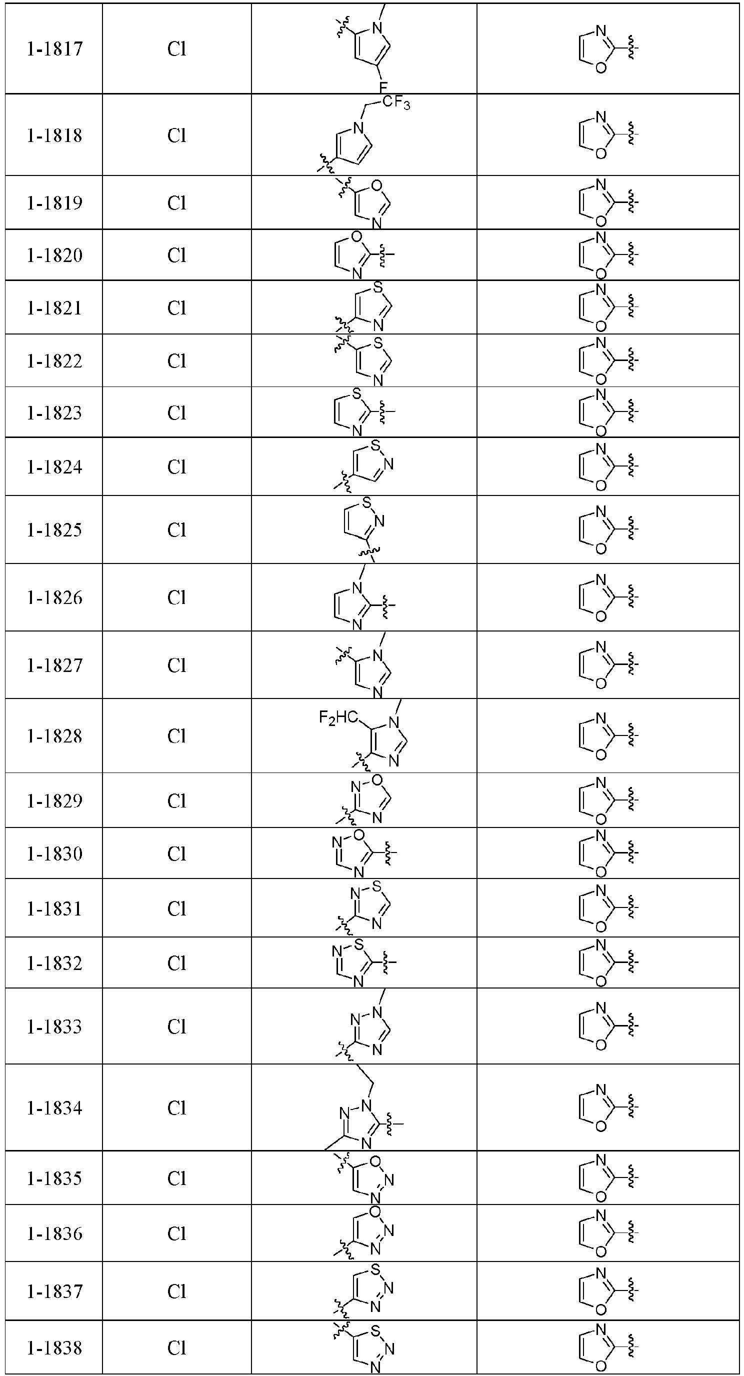

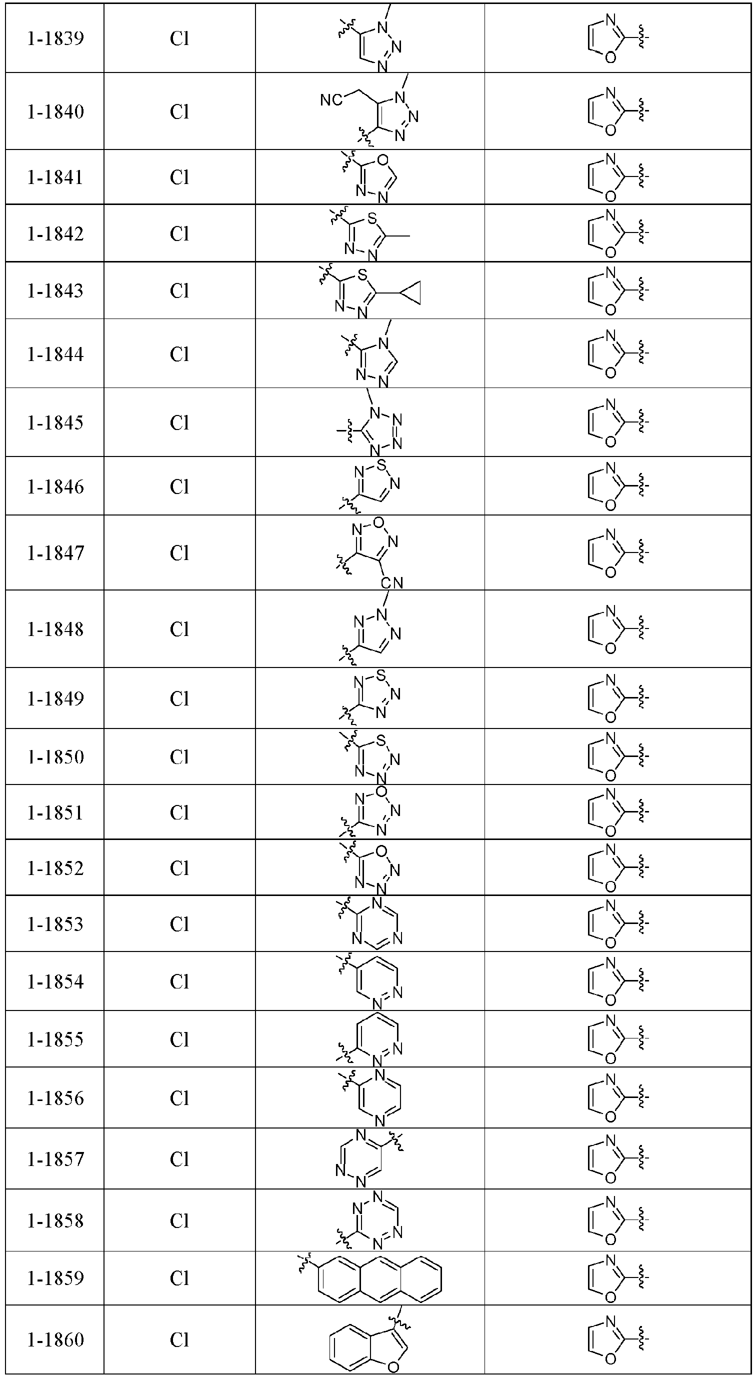

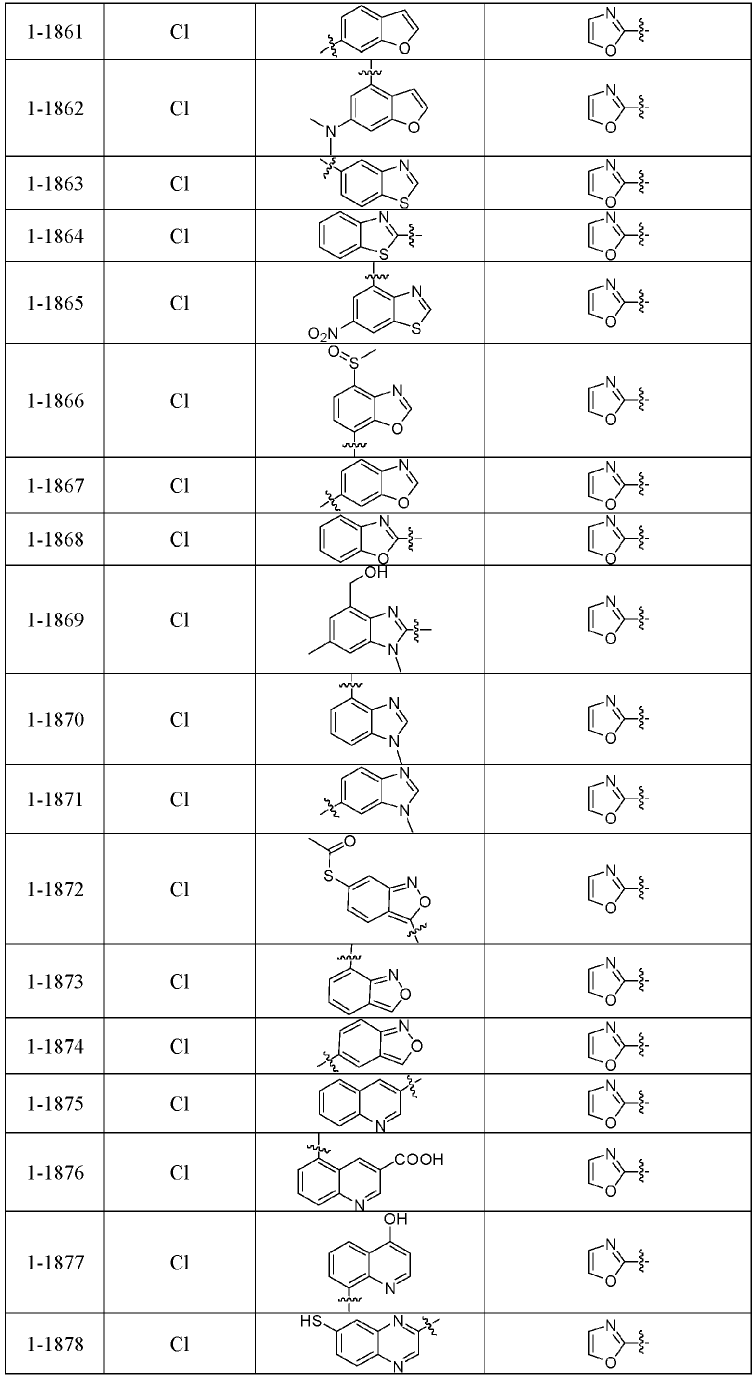

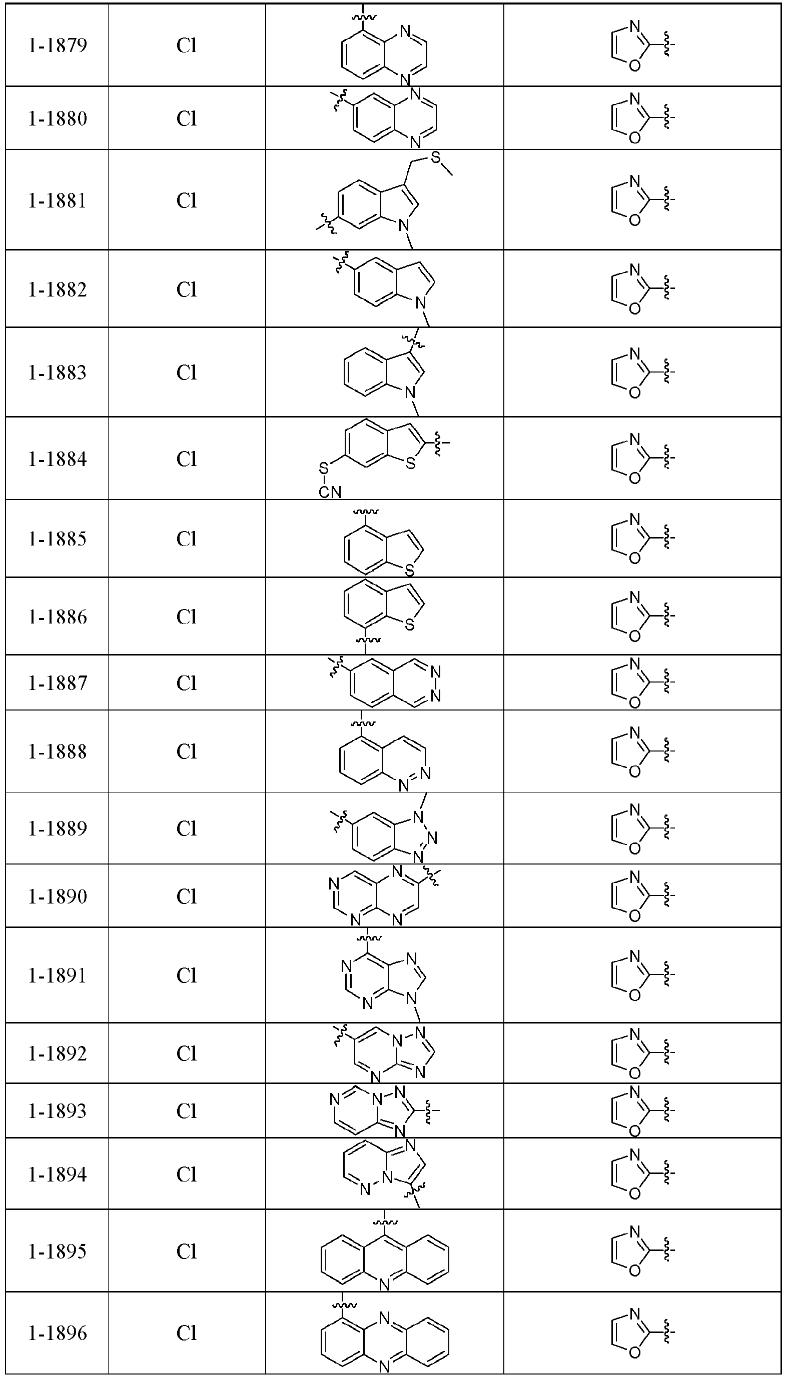

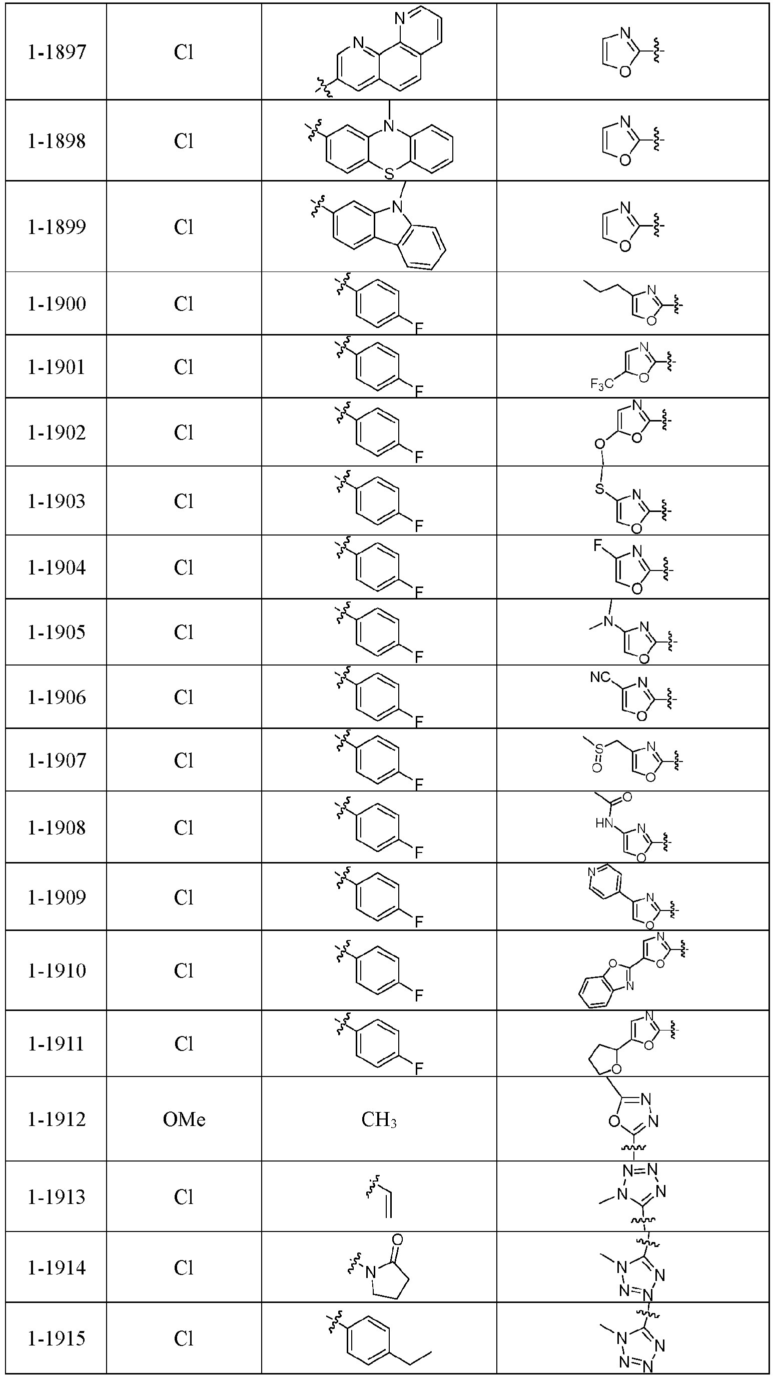

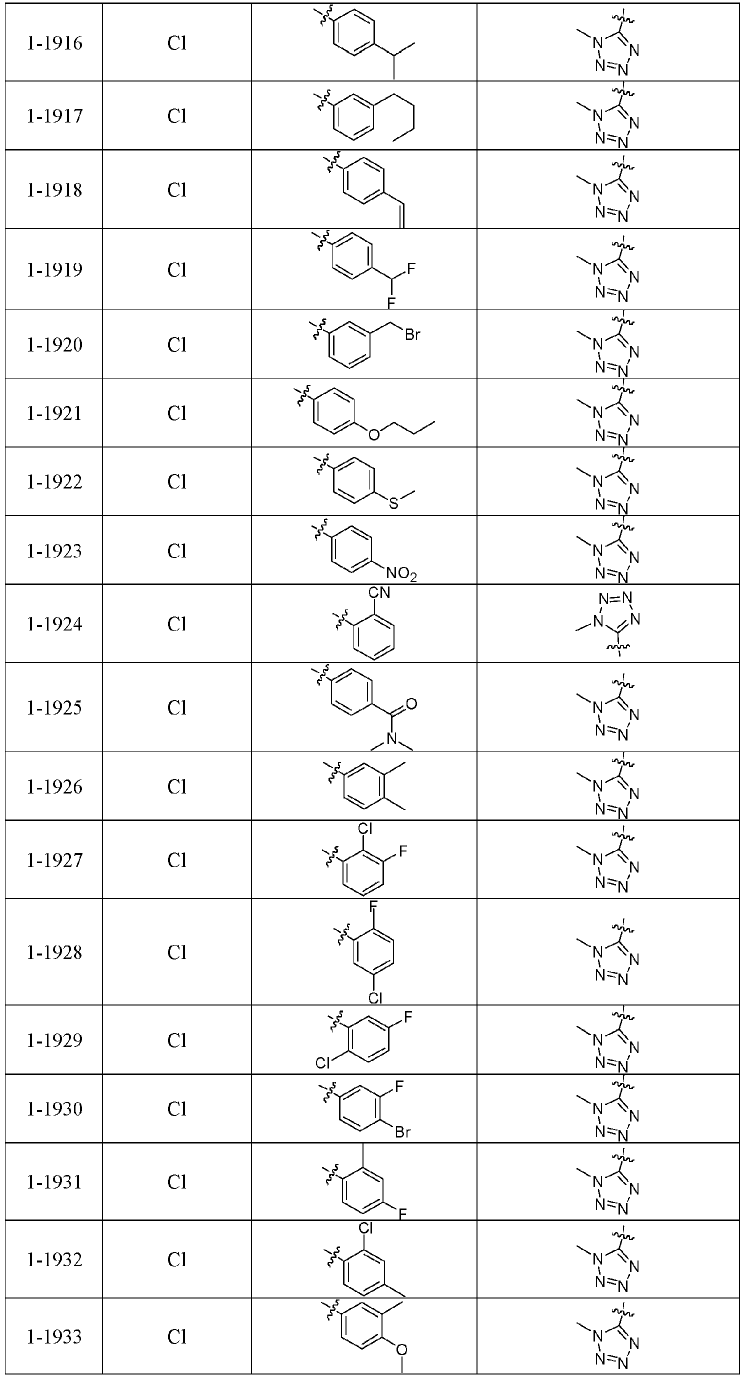

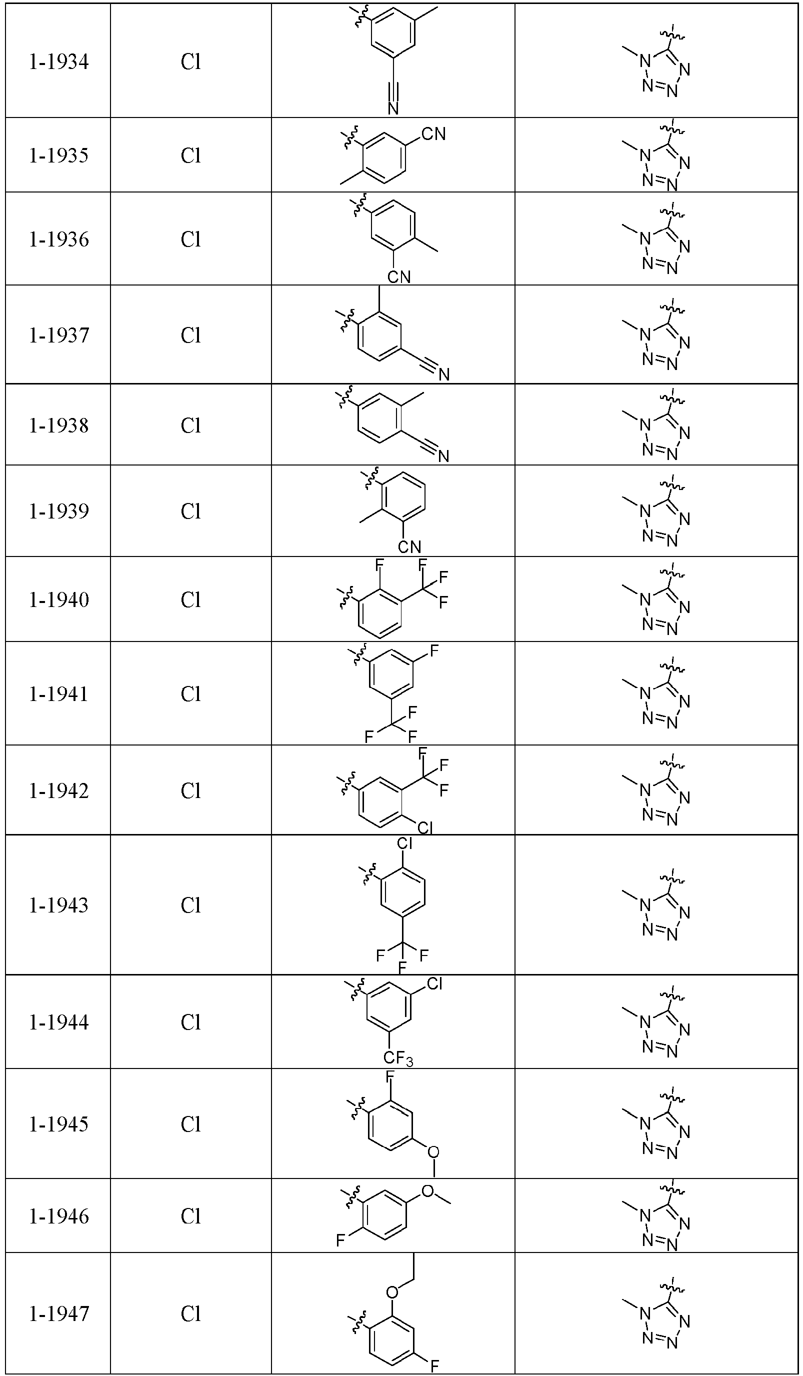

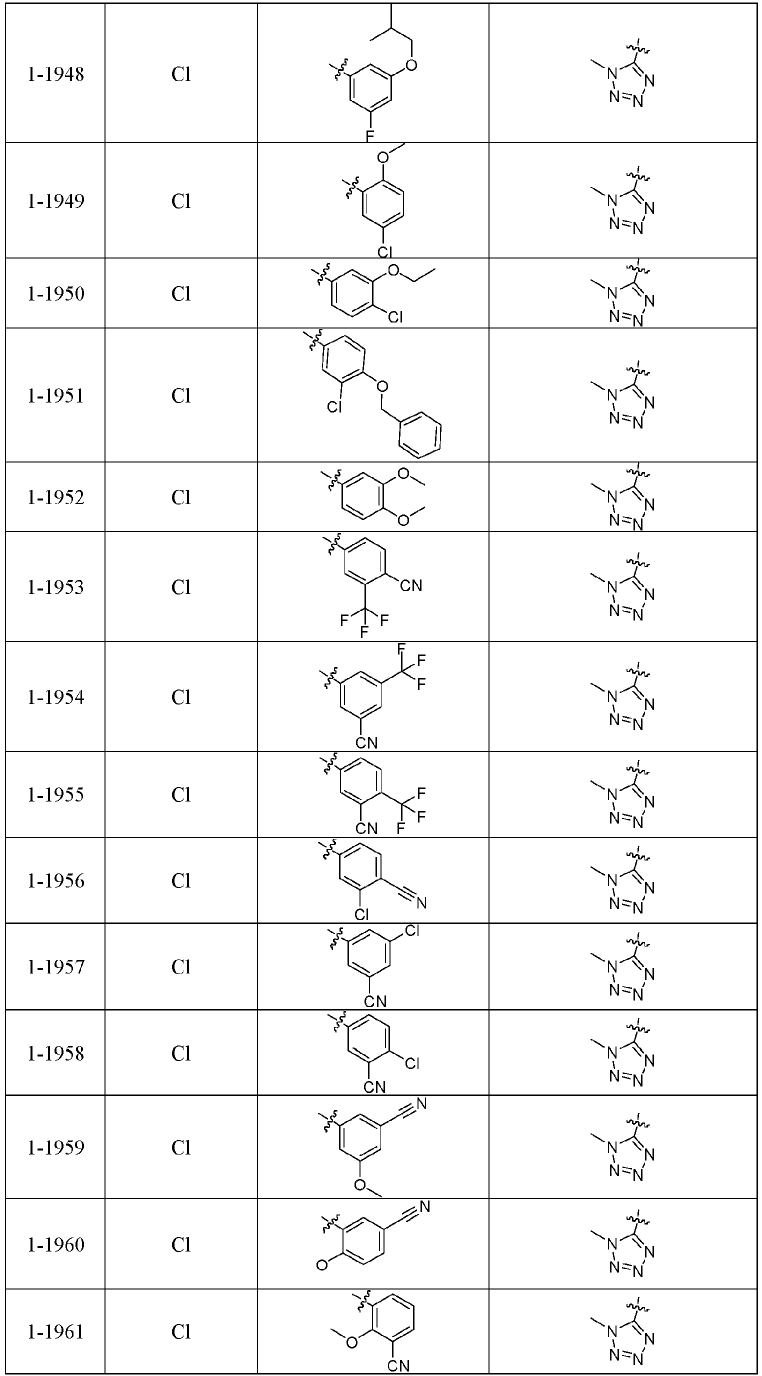

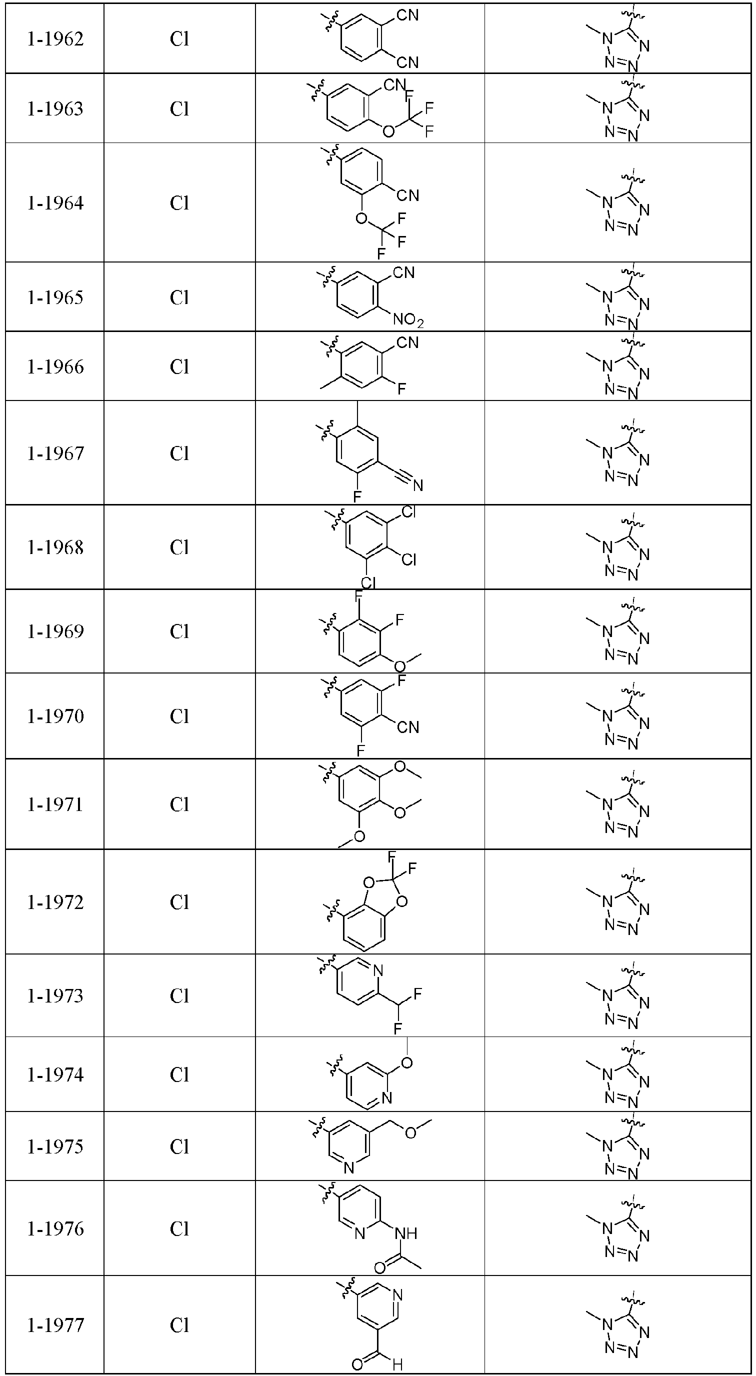

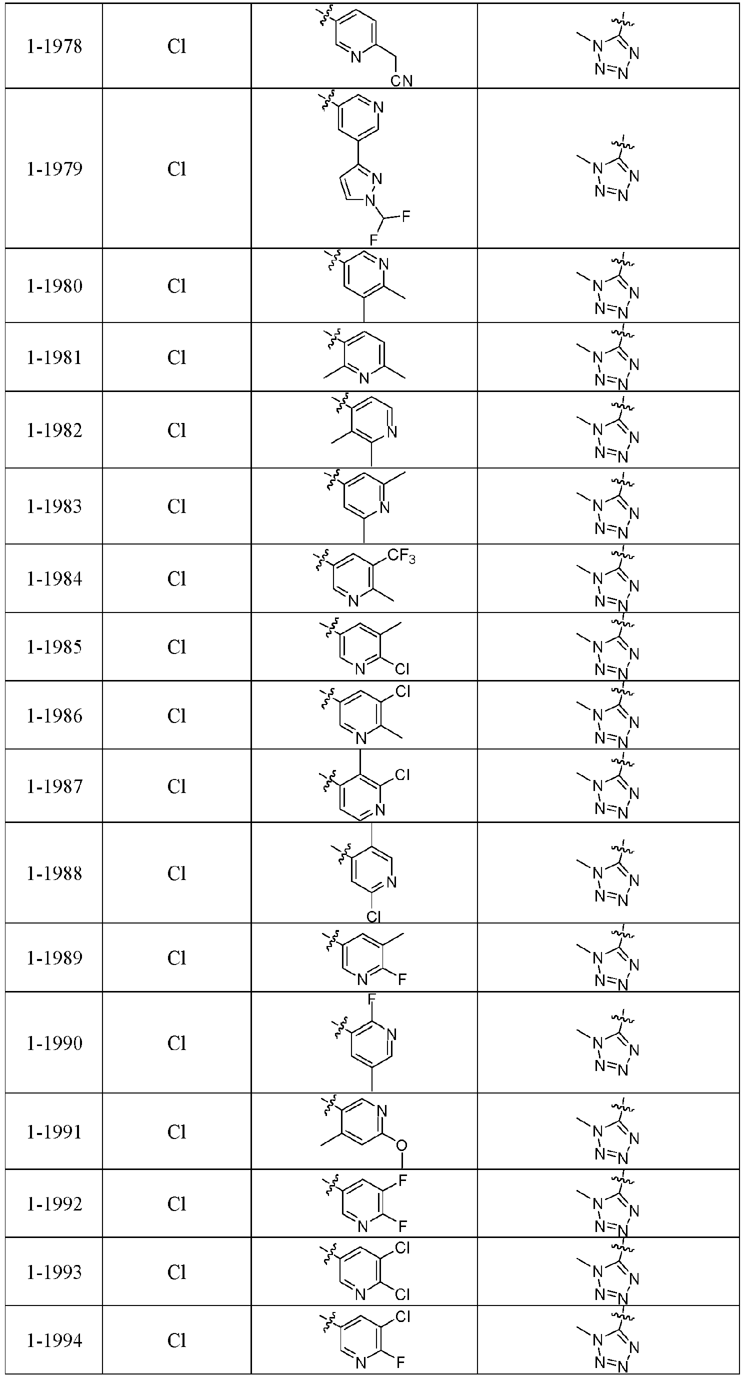

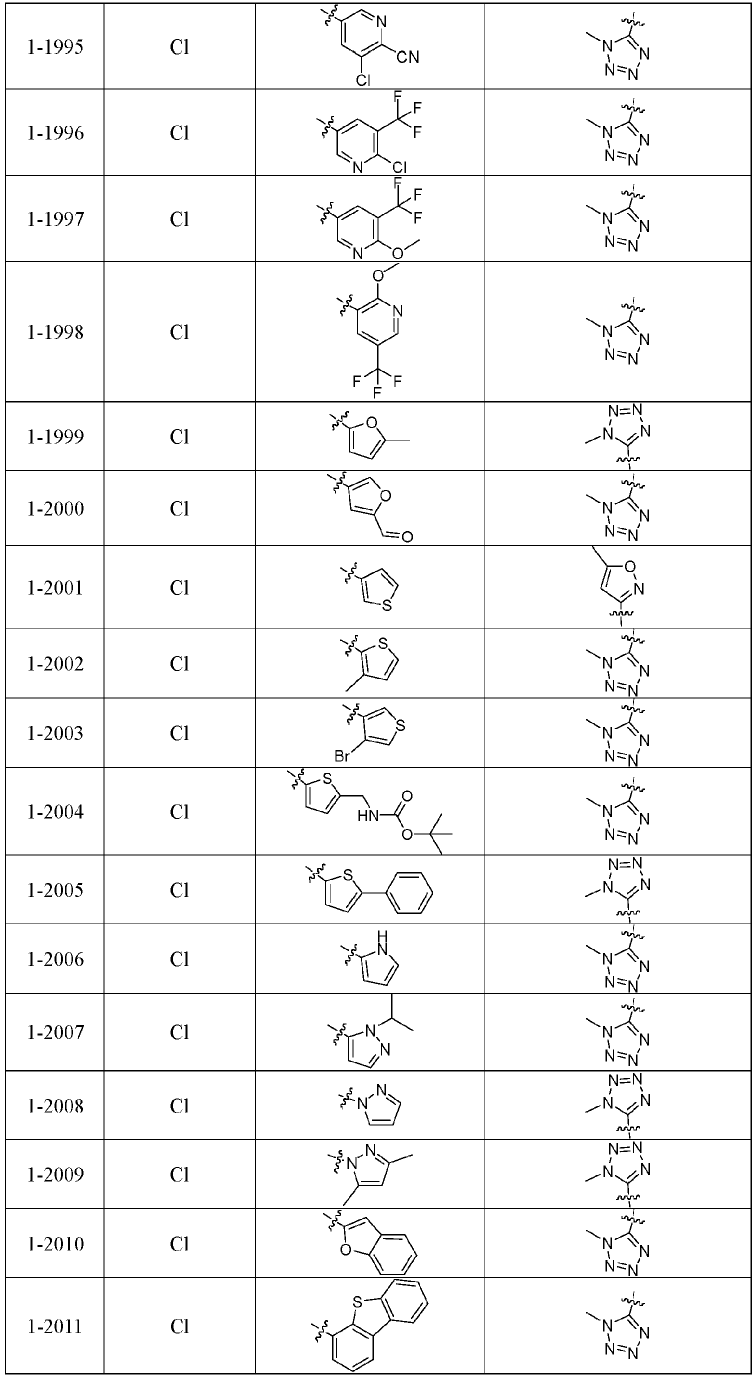

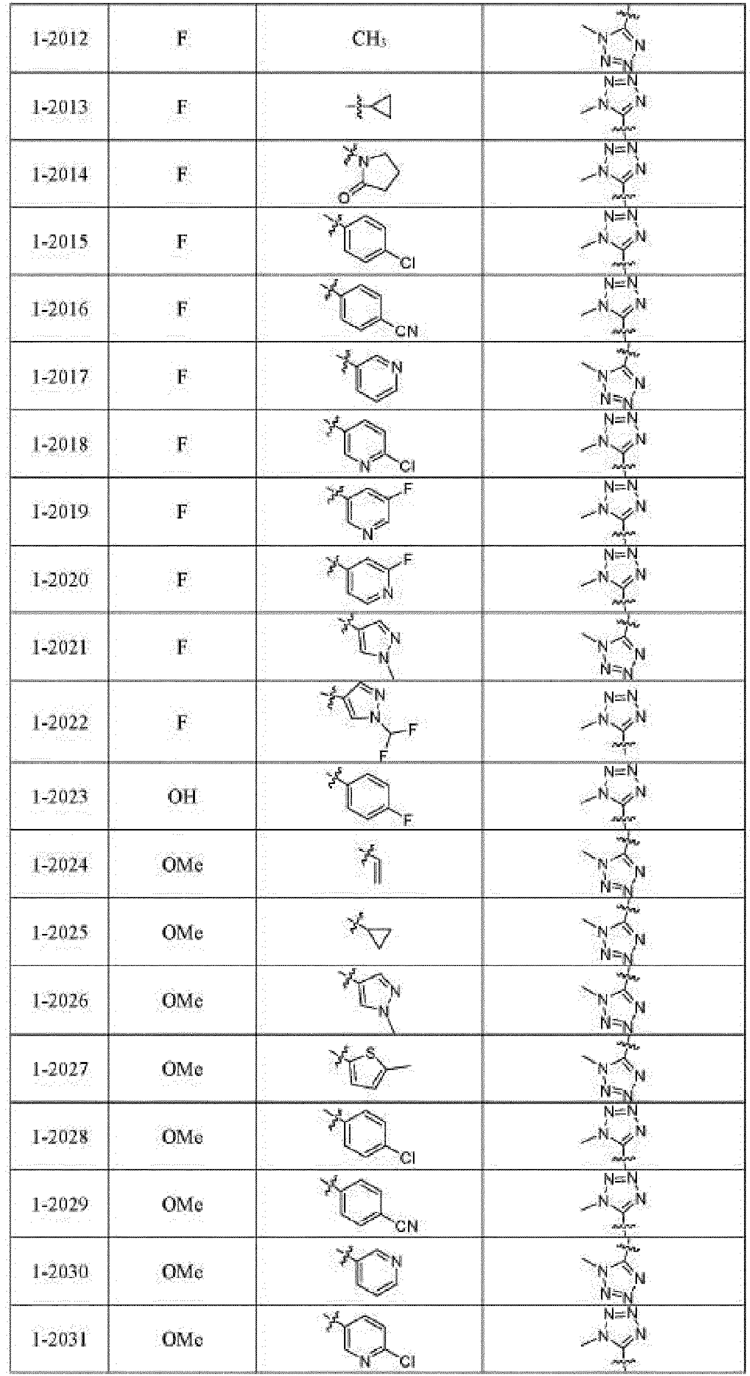

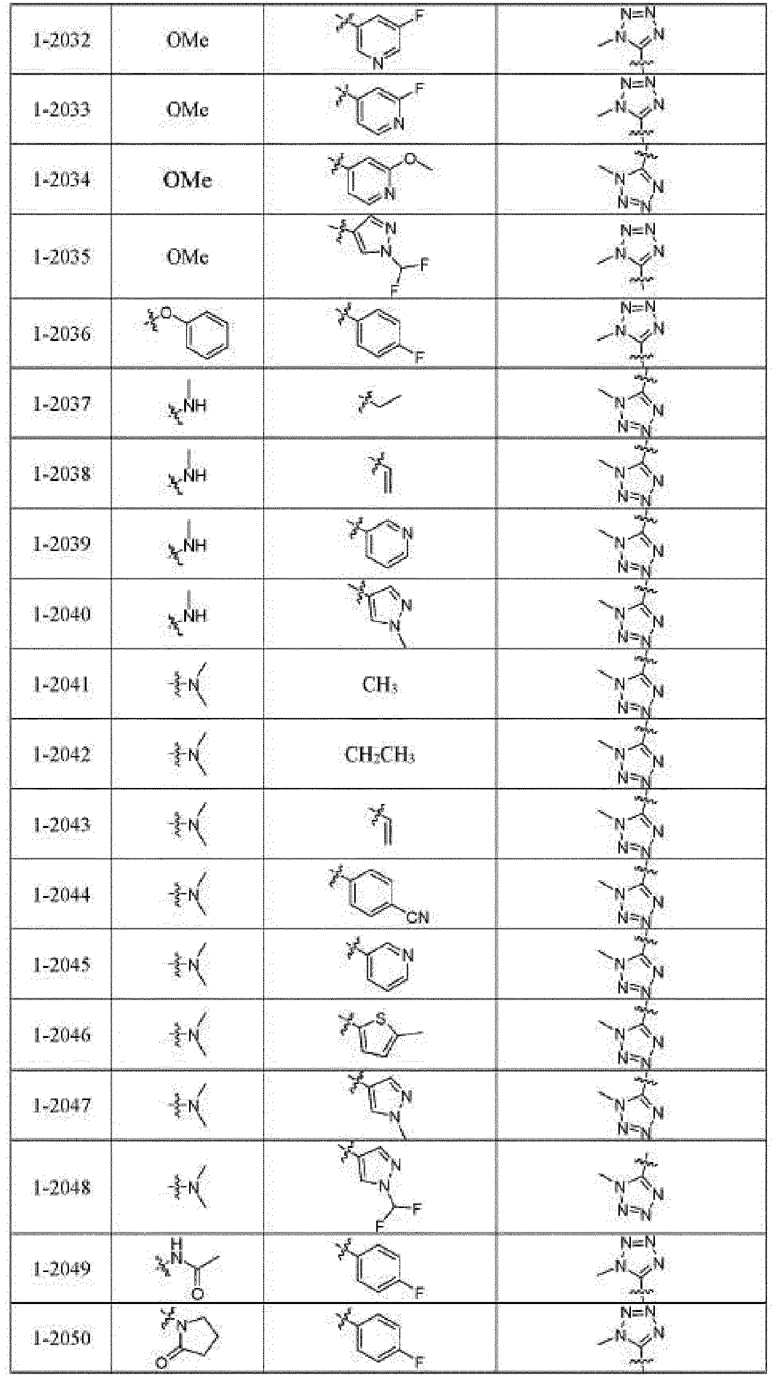

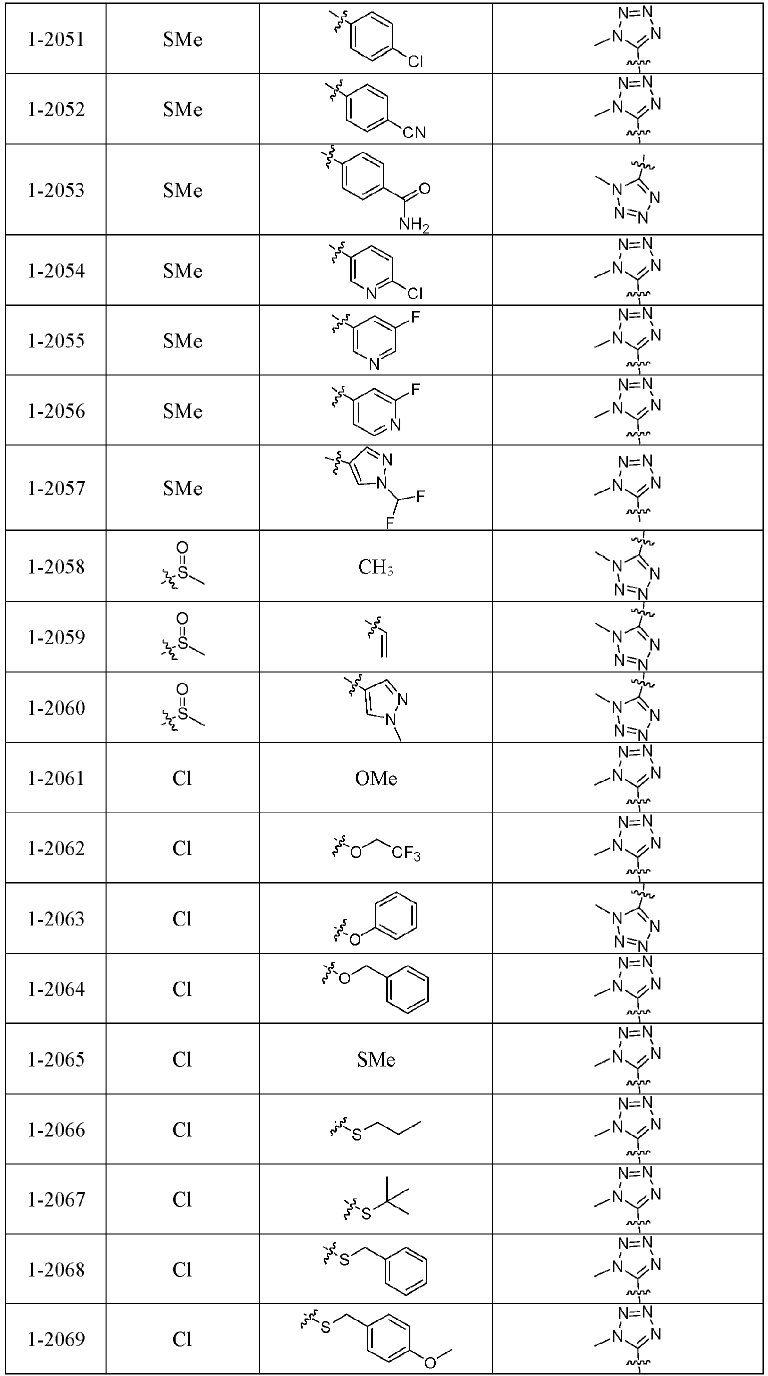

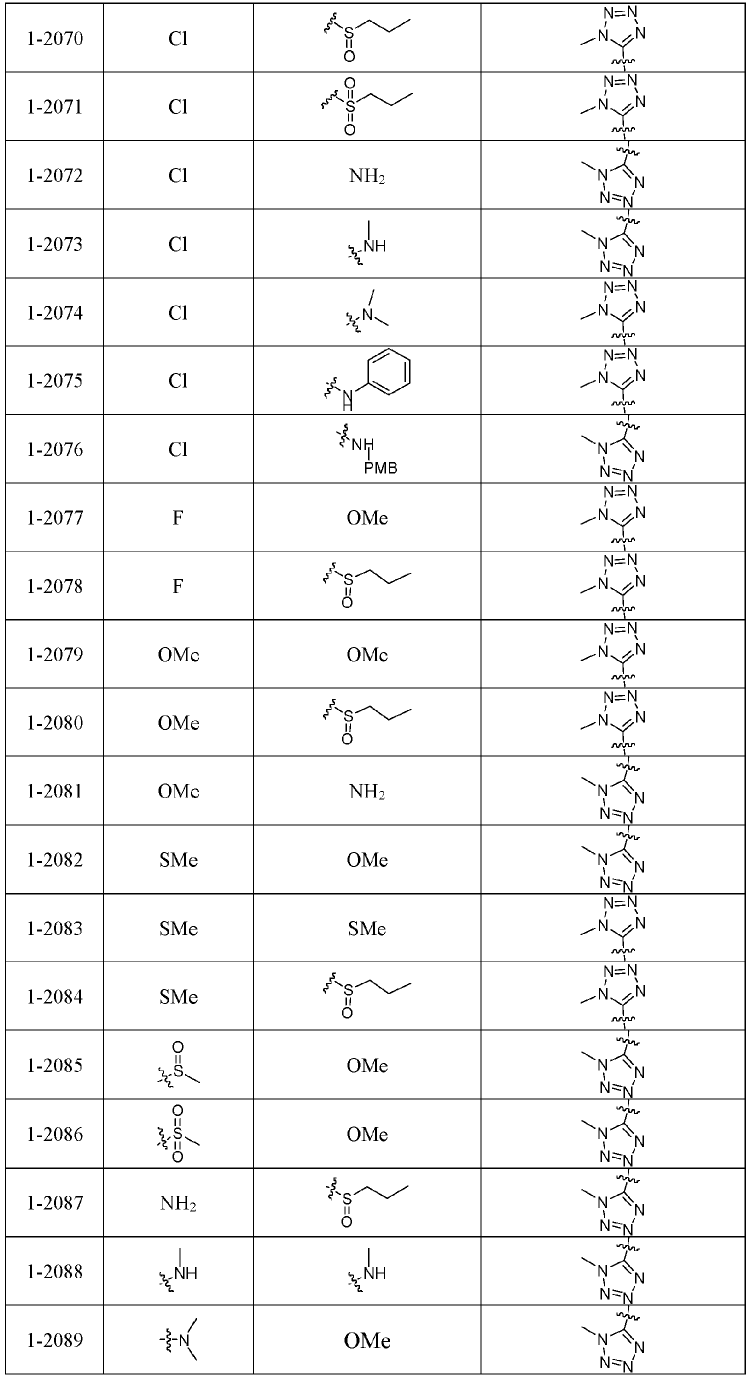

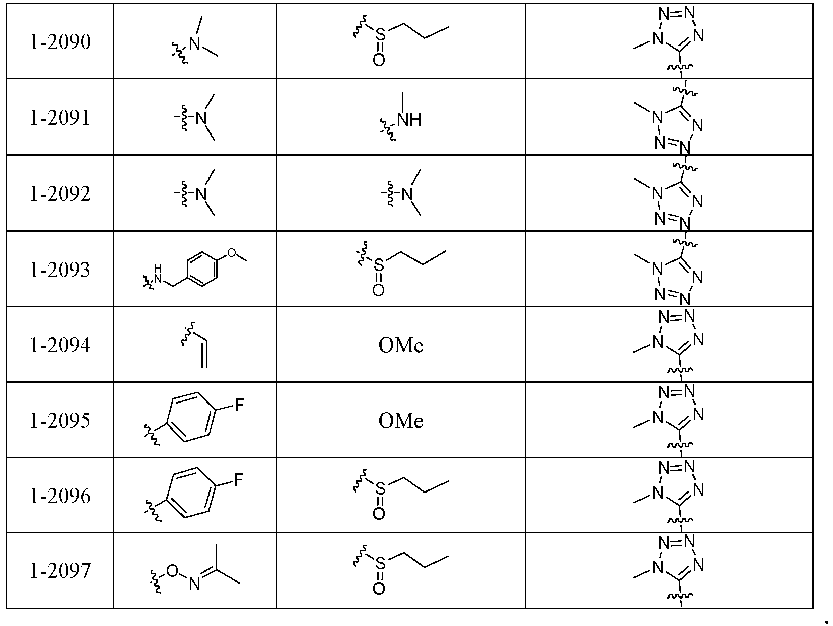

7. Verfahren zur Herstellung der 4-Pyridinylformamidverbindung nach einem der Ansprüche 1 bis 6, **gekennzeichnet durch**
Aussetzen der Verbindung der allgemeinen Formel II und der Verbindung der allgemeinen Formel III Amidierungsreaktion, um die Verbindung der allgemeinen Formel I zu erhalten, wobei das chemische Reaktionsschema wie folgt ist: 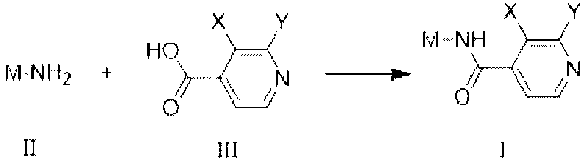
wobei die Reaktion in der Gegenwart eines Halogenierungsmittels, eines Katalysators und eines Lösungsmittels durchgeführt wird; bevorzugt das Halogenierungsmittel SOCl₂ ist, der Katalysator 4-Dimethylaminopyridin ist und das Lösungsmittel Pyridin ist;
oder die Reaktion in der Gegenwart eines Kondensationsmittels und eines Lösungsmittels durchgeführt wird; bevorzugt das Kondensationsmittel CDI, DCC, DBU oder eine Kombination davon ist und das Lösungsmittel Methylenchlorid ist.

8. Herbizidzusammensetzung, die **dadurch gekennzeichnet ist, dass** sie (i) die 4-Pyridinylformamidverbindung oder Derivat davon nach einem der Ansprüche 1 bis 6 umfasst; bevorzugt ferner umfassend (ii) ein oder mehrere weitere Herbizide und/oder Safener; bevorzugter ferner umfassend (iii) agrochemisch verträgliche Formulierungshilfsmittel.

9. Verfahren zur Unkrautbekämpfung, das **dadurch gekennzeichnet ist, dass** es Anwenden einer herbizid wirksamen Menge von zumindest einem von der 4-Pyridinylformamidverbindung oder Derivat davon nach einem der Ansprüche 1 bis 6 oder der Herbizidzusammensetzung nach Anspruch 8 an einer Pflanze oder einem Unkrautbereich umfasst.

10. Verwendung von zumindest einem von der 4-Pyridinylformamidverbindung oder Derivat davon nach einem der Ansprüche 1 bis 6; oder der Herbizidzusammensetzung nach Anspruch 8 zur Bekämpfung eines Unkrauts, wobei bevorzugt die 4-Pyridinylformamidverbindung oder Derivat davon zur Vorbeugung und/oder Bekämpfung eines Unkrauts in einer Nutzpflanze verwendet wird, wobei die Nutzpflanze eine transgene Pflanze oder eine Pflanze ist, die durch Gen-Editing-Technik behandelt wird.

## Revendications

1. Composé de 4-pyridinyle formamide ou dérivé de celui-ci, tel que représenté dans la Formule I : 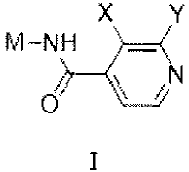
dans lequel X représente nitro, halogène, cyano, formyle, thiocyano, mercapto; alkyle, alcényle, alcynyle, cycloalkyle, cycloalcényle, cycloalkylalkyle ou cycloalcénylalkyle, qui est avec ou sans halogène ; OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)Rl, OC(S)R¹, -alkyl-C(S)R¹, - alkyl-C(S)OR¹, -alkyl-C(O)SR¹, -alkyl-C(S)SR¹, -alkyl-SC(O)R¹, -alkyl-OC(S)R¹, -alkyl-SC(S)R¹, -O-alkyl-NR⁴R⁵, -S-alkyl-NR⁴R⁵, -alkyl-O-alkyl-NR⁴R⁵, -alkyl-S-alkyl-NR⁴R⁵, -alkyl-(C=S)ₙ-NR⁴R⁵, -NH-alkyl-NR⁴R⁵, -alkyl-OR¹, -alkyl-COR¹, -alkyl-CO₂R¹, -alkyl-OCOR¹, -alkyl-NR³COR¹, -alkyl-SO₂OR¹, -alkyl-NR³SO₂R², -alkyl-OSO₂R², -alkyl-S(O)ₘR², -alkyl-CONR⁴R⁵, -alkyl-SO₂NR⁴R⁵, NR⁴R⁵, 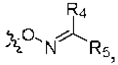 P(O)(OR⁶)₂, CH₂P(O)(OR⁶)₂, SO₂NR⁴R⁵-alkyl-S(O)ₘR², - alkyl-CN, -alkylaryle, -alkylhétéroaryle, -alkylhétérocyclyle, aryle, hétéroaryle ou hétérocyclyle ;
Y représente nitro, cyano, formyle, thiocyano, mercapto; alkyle, alcényle, alcynyle, cycloalkyle, cycloalcényle, cycloalkylalkyle ou cycloalcénylalkyle, qui est avec ou sans halogène ; OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -alkyl-C(S)R¹, -alkyl-C(S)OR¹, - alkyl-C(O)SR¹, -alkyl-C(S)SR¹, -alkyl-SC(O)R¹, -alkyl-OC(S)R¹, -alkyl-SC(S)R¹, -O-alkyl-NR⁴R⁵, -S-alkyl-NR⁴R⁵, -alkyl-O-alkyl-NR⁴R⁵, -alkyl-S-alkyl-NR⁴R⁵, -alkyl-(C=S)ₙ-NR⁴R⁵, - NH-alkyl-NR⁴R⁵, -alkyl-OR¹, -alkyl-COR¹, -alkyl-CO₂R¹, -alkyl-OCOR¹, -alkyl-NR³COR¹, - alkyl-SO₂OR¹, -alkyl-NR³SO₂R², -alkyl-OSO₂R², -alkyl-S(O)ₘR², -alkyl-CONR⁴R⁵, -alkyl-SO₂NR⁴R⁵, NR⁴R⁵, 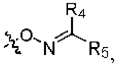 P(O)(OR⁶)₂, CH₂P(O)(OR⁶)₂, SO₂NR⁴R⁵-alkyl-S(O)ₘR², -alkyl-CN, -alkylaryle, -alkylhétéroaryle, -alkylhétérocyclyle, aryle, hétéroaryle ou hétérocyclyle ;
R¹, R³, R⁴ et R⁵ représentent chacun indépendamment hydrogène, aryle, arylalkyle, hétéroaryle, hétéroarylalkyle, alkyle, alkyle halogéné, alcényle, alcényle halogéné, alcynyle, alcynyle halogéné, cycloalkyle, cycloalkyle halogéné, alkoxyalkyle ou cycloalkylalkyle, les 10 derniers groupes tels que mentionnés étant chacun substitués par des groupes s choisis parmi le groupe constitué de cyano, halogène, nitro, thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸ et alkoxyalkoxycarbonyle ;
R² représente aryle, arylalkyle, hétéroaryle, hétéroarylalkyle, alkyle, alcényle, alcynyle, cycloalkyle ou cycloalkylalkyle, les 5 derniers groupes tels que mentionnés étant chacun substitués par des groupes s choisis parmi le groupe constitué de cyano, halogène, nitro, thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸ et alkoxyalkoxycarbonyle ;
R⁶ représente méthyle ou éthyle ;
R⁷ et R⁸ représentent chacun indépendamment hydrogène, alkyle, alcényle ou alcynyle ;
R⁹ représente alkyle, alcényle ou alcynyle ;
M représente 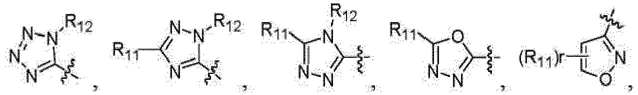 ou 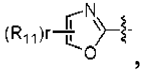 qui est non substitué ou substitué ;
R₁₁ représente hydrogène, halogène, cyano, nitro, alkyle qui est non substitué ou substitué par un substituant choisi parmi R₁₃, cycloalkyle qui est non substitué ou substitué par un substituant choisi parmi R₁₄, alcényle, alcényle halogéné, alcynyle, alcynyle halogéné, cycloalcényle, NH₂, aminoacyle, carboxyle, alkoxyalkoxycarbonyle, OR₁₅, -alkyl-OR₁₅, C(O)R₁₆, -alkyl-C(O)R₁₆, C(O)OR₁₆, -alkyl-C(O)OR₁₆, S(O)ₘR₁₆, -alkyl-S(O)ₘR₁₆, -N(R₁₆)₂, -NHR₁₆, -C(O)NHR₁₆, - C(O)N(R₁₆)₂, -NHC(O)R₁₇, hétérocyclyle, hétérocyclylalkyle, hétérocyclyloxy, hétérocyclylcarbonyle, aryle, arylalkyle, aryloxy, arylcarbonyle, hétéroaryle, hétéroarylalkyle, hétéroaryloxy ou hétéroarylcarbonyle ;
R₁₂ représente hydrogène, alkyle qui est non substitué ou substitué par un substituant choisi parmi R₁₈, cycloalkyle, cycloalkyle halogéné, alcényle, alcényle halogéné, alcynyle, alcynyle halogéné, cycloalcényle, alkoxyalkyle, alkylthioalkyle, alkoxycarbonylalkyle, aryle ou arylalkyle ;
lorsque M est 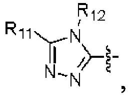 -(CH₂)₄- ou -CH=CH-CH=CH- formé par Ru et R₁₂, l'atome d'azote lié à R₁₂ et l'atome de carbone lié à R₁₁ forment conjointement un cycle à 6 chaînons ;
R₁₅ représente alkyle qui est non substitué ou substitué par un substituant choisi parmi R₂₁, cycloalkyle, cycloalkyle halogéné, alcényle, alcényle halogéné, alcynyle, alcynyle halogéné, cycloalcényle ou phényle ;
R₁₆ représente alkyle, alkyle halogéné, cycloalkyle, alcényle, alcényle halogéné, alcynyle, alcynyle halogène ou cycloalcényle ;
R₂₁ représente halogène, cyano, cycloalkyle, hydroxy, mercapto, alkoxy, C(O)R₂₂, carboxyle, alkoxycarbonyle, alkoxyalkoxycarbonyle, -S(O)ₘ-alkyle, heteroaryle, hétérocyclyle ou phényle qui est non substitué ou substitué par un ou plusieurs groupes choisis parmi R₂₃ ;
R₁₇ et R₂₂ représentent chacun indépendamment hydrogène, alkyle ou N(R₂₄)R₂₅ ;
R₂₃ représente halogène, cyano, nitro ; alkyle, alkyle qui est non substitué ou substitué par un substituant choisi parmi R₃₁, cycloalkyle, cycloalkyle halogéné, alcényle, alcényle halogéné, alcynyle, alcynyle halogéné, cycloalcényle, alkylcarbonyle, cycloalkylcarbonyle, alkylcarbonyle halogéné, cycloalkylcarbonyle halogéné, alkoxycarbonyle, alkoxycarbonyle halogéné, alkylaminocarbonyle, alkylaminocarbonyle halogéné, bis(alkyl)aminocarbonyle, OR₃₂, S(O)ₘR₃₃, alkylaminosulfonyle, bis(alkyl)aminosulfonyle, NH₂, alkylamino, bis(alkyl)amino, aryle, hétéroaryle ou hétérocyclyle ;
R₂₄ et R₂₅ représentent chacun indépendamment hydrogène, alkyle ou phényle ; ou,
chaîne alkylidène formée par R₂₄ et R₂₅, et le(s) atome(s) d'azote lié(s) à R₂₄ et R₂₅ forment conjointement un cycle à 3-7 chaînons, ladite chaîne alkylidène contenant éventuellement un O, S, S(O), S(O)₂, NH ou N-alkyle et étant éventuellement substituée par un groupe oxo ou thio ;
R₁₃, R₁₄, R₁₈ et R₃₁ représentent chacun indépendamment halogène, cyano, nitro, carboxyle, alkoxycarbonyle, alkoxyalkoxycarbonyle, S(O)ₘR₄₁, OR₄₂, aryle, hétéroaryle ou hétérocyclyle ;
R₃₂ représente hydrogène, alkyle, alkyle halogéné, cycloalkyle, cycloalkyle halogéné, alcényle, alcényle halogéné, alcynyle, alcynyle halogéné ou cycloalcényle ;
R₃₃ représente alkyle, alkyle halogéné, cycloalkyle, alcényle, alcényle halogéné, alcynyle, alcynyle halogéné ou cycloalcényle ;
R₄₁ et R₄₂ représentent chacun indépendamment hydrogène, alkyle, alkyle halogéné, cycloalkyle, cycloalkyle halogéné, alcényle, alcényle halogéné, alcynyle, alcynyle halogéné, cycloalcényle ou phényle ;
r représente 1 ou 2 ;
m représente 0, 1 ou 2 ;
n représente 0 ou 1 ;
s représente 0, 1, 2 ou 3 ;
dans lequel, lorsque X est fluorine, Y n'est pas amino, monométhylamino, monoéthylamino et monopropylamino ; lorsque M est 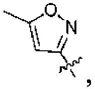 X et Y ne sont pas méthyle en même temps.

2. Le composé de 4-pyridinyle formamide ou dérivé de celui-ci selon la revendication 1, qui est **caractérisé en ce que**,
X représente nitro, halogène, cyano, formyle, thiocyano, mercapto ; C1-C8 alkyle, C2-C8 alcényle, C2-C8 alcynyle, C3-C8 cycloalkyle, C3-C8 cycloalcényle, C3-C8 cycloalkyl-C1-C6 alkyle ou C3-C8 cycloalcényl-C1-C6 alkyle, qui est avec ou sans halogène ; OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -(C1-C6)alkyl-C(S)R¹, -(C1-C6)alkyl-C(S)OR¹, -(C1-C6)alkyl-C(O)SR¹, -(C1-C6)alkyl-C(S)SR¹, -(C1-C6)alkyl-SC(O)R¹, -(C1-C6)alkyl-OC(S)R¹, - (C1-C6)alkyl-SC(S)R¹, -O-(C1-C6)alkyl-NR⁴R⁵, -S-(C1-C6)alkyl-NR⁴R⁵, -(C1-C6)alkyl-O-(C1-C6)alkyl-NR⁴R⁵, -(C1-C6)alkyl-S-(C1-C6)alkyl-NR⁴R⁵, -(C1-C6)alkyl-(C=S)ₙ-NR⁴R⁵, -NH-(C1-C6)alkyl-NR⁴R⁵, -(C1-C6)alkyl-OR¹, -(C1-C6)alkyl-COR¹, -(C1-C6)alkyl-CO₂R¹, -(C1-C6)alkyl-OCOR¹, -(C1-C6)alkyl-NR³COR¹, -(C1-C6)alkyl-SO₂OR¹, -(C1-C6)alkyl-NR³SO₂R², - (C1-C6)alkyl-OSO₂R², -alkyl-S(O)ₘR², -(C1-C6)alkyl-CONR⁴R⁵, -(C1-C6)alkyl-SO₂NR⁴R⁵, NR⁴R⁵, 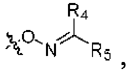 P(O)(OR⁶)₂, CH₂P(O)(OR⁶)₂, SO₂NR⁴R⁵-(C1-C6)alkyl-S(O)ₘR², -(C1-C6)alkyl-CN, -(C1-C6)alkylaryle, -(C1-C6)alkylhétéroaryle, -(C1-C6)alkylhétérocyclyle, aryle, hétéroaryle ou hétérocyclyle ;
Y représente nitro, cyano, formyle, thiocyano, mercapto ; C1-C8 alkyle, C2-C8 alcényle, C2-C8 alcynyle, C3-C8 cycloalkyle, C3-C8 cycloalcényle, C3-C8 cycloalkyl-C1-C6 alkyle ou C3-C8 cycloalcényl-C1-C6 alkyle, qui est avec ou sans halogène ; OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -(C1-C6)alkyl-C(S)R¹, -(C1-C6)alkyl-C(S)OR¹, -(C1-C6)alkyl-C(O)SR¹, -(C1-C6)alkyl-C(S)SR¹, -(C1-C6)alkyl-SC(O)R¹, -(C1-C6)alkyl-OC(S)R¹, -(C1-C6)alkyl-SC(S)R¹, -O-(C1-C6)alkyl-NR⁴R⁵, -S-(C1-C6)alkyl-NR⁴R⁵, -(C1 -C6)alkyl-O-(C1-C6)alkyl-NR⁴R⁵, -(C1-C6)alkyl-S-(C1-C6)alkyl-NR⁴R⁵, -(C1-C6)alkyl-(C=S)ₙ-NR⁴R⁵, -NH-(C1-C6)alkyl-NR⁴R⁵, -(C1-C6)alkyl-OR¹, -(C1-C6)alkyl-COR¹, -(C1-C6)alkyl-CO₂R¹, -(C1-C6)alkyl-OCOR¹, -(C1-C6)alkyl-NR³COR¹, -(C1-C6)alkyl-SO₂OR¹, -(C1-C6)alkyl-NR³SO₂R², - (C1-C6)alkyl-OSO₂R², -alkyl-S(O)ₘR², -(C1-C6)alkyl-CONR⁴R⁵, -(C1-C6)alkyl-SO₂NR⁴R⁵, NR⁴R⁵, 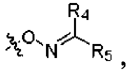 P(O)(OR⁶)₂, CH₂P(O)(OR⁶)₂, SO₂NR⁴R⁵-(C1-C6)alkyl-S(O)ₘR², -(C1-C6)alkyl-CN, -(C1-C6)alkylaryle, -(C1-C6)alkylhétéroaryle, -(C1-C6)alkylhétérocyclyle, aryle, hétéroaryle ou hétérocyclyle ;
R¹, R³, R⁴ et R⁵ représentent chacun indépendamment hydrogène, aryle, aryl-C1-C6 alkyle, hétéroaryle, hétéroaryl-C1-C6 alkyle, C1-C8 alkyle, C1-C8 alkyle halogéné, C2-C8 alcényle, C2-C8 alcényle halogéné, C2-C8 alcynyle, C2-C8 alcynyle halogéné, C3-C8 cycloalkyle, C3-C8 cycloalkyle halogéné, C1-C8 alkoxy-C1-C6 alkyle ou C3-C8 cycloalkyl-C1-C6 alkyle, les 10 derniers groupes tels que mentionnés étant chacun substitués par des groupes s choisis parmi le groupe constitué de cyano, halogène, nitro, thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸ et C1-C8 alkoxy-C1-C6 alkoxycarbonyle ;
R² représente aryle, aryl-C1-C6 alkyle, hétéroaryle, hétéroaryl-C1-C6 alkyle, C1-C8 alkyle, C2-C8 alcényle, C2-C8 alcynyle, C3-C8 cycloalkyle ou C3-C8 cycloalkyl-C1-C6 alkyle, les 5 derniers groupes tels que mentionnés étant chacun substitués par des groupes s choisis parmi le groupe constitué de cyano, halogène, nitro, thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸ et C1-C8 alkoxy-C1-C6 alkoxycarbonyle ;
R⁶ représente méthyle ou éthyle ;
R⁷ et R⁸ représentent chacun indépendamment hydrogène, C1-C8 alkyle, C2-C8 alcényle ou C2-C8 alcynyle ;
R⁹ représente C1-C8 alkyle, C2-C8 alcényle ou C2-C8 alcynyle ;
M représente 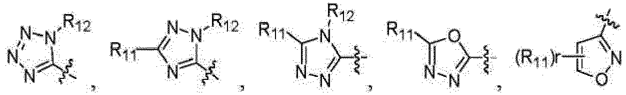 ou 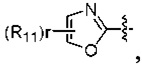 qui est non substitué ou substitué ;
R₁₁ représente hydrogène, halogène, cyano, nitro, C1-C8 alkyle qui est non substitué ou substitué par un substituant choisi parmi R₁₃, C3-C8 cycloalkyle qui est non substitué ou substitué par un substituant choisi parmi R₁₄, C2-C8 alcényle, C2-C8 alcényle halogéné, C2-C8 alcynyle, C2-C8 alcynyle halogéné, C3-C8 cycloalcényle, NH₂, aminoacyle, carboxyle, C1-C8 alkoxy-C1-C6 alkoxycarbonyle, OR₁₅, -(C1-C6)alkyl-OR₁₅, C(O)R₁₆, -(C1-C6)alkyl-C(O)R₁₆, C(O)OR₁₆, - (C1-C6)alkyl-C(O)OR₁₆, S(O)ₘR₁₆, -(C1-C6)alkyl-S(O)ₘR₁₆, -N(R₁₆)₂, -NHR₁₆, -C(O)NHR₁₆, - C(O)N(R₁₆)₂, -NHC(O)R₁₇, hétérocyclyle, hétérocyclyl-C1-C6 alkyle, hétérocyclyloxy, hétérocyclylcarbonyle, aryle, aryl-C1-C6 alkyle, aryloxy, arylcarbonyle, hétéroaryle, hétéroaryl-C1-C6 alkyle, hétéroaryloxy ou hétéroarylcarbonyle ;
R₁₂ représente hydrogène, C1-C8 alkyle qui est non substitué ou substitué par un substituant choisi parmi R₁₈, C3-C8 cycloalkyle, C3-C8 cycloalkyle halogéné, C2-C8 alcényle, C2-C8 alcényle halogéné, C2-C8 alcynyle, C2-C8 alcynyle halogéné, C3-C8 cycloalcényle, C1-C8 alkoxy-C1-C6 alkyle, C1-C8 alkylthio-C1-C6 alkyle, C1-C8 alkoxycarbonyl-C1-C6 alkyle, aryle ou aryl-C1-C6 alkyle ;
lorsque M est 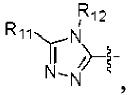 -(CH₂)₄- ou -CH=CH-CH=CH- formé par R₁₁ et R₁₂, l'atome d'azote lié à R₁₂ et l'atome de carbone lié à R₁₁ forment conjointement un cycle à 6 chaînons ;
R₁₅ représente C1-C8 alkyle qui est non substitué ou substitué par un substituant choisi parmi R₂₁, C3-C8 cycloalkyle, C3-C8 cycloalkyle halogéné, C2-C8 alcényle, C2-C8 alcényle halogéné, C2-C8 alcynyle, C2-C8 alcynyle halogéné, C3-C8 cycloalcényle ou phényle ;
R₁₆ représente C1-C8 alkyle, C1-C8 alkyle halogéné, C3-C8 cycloalkyle, C2-C8 alcényle, C2-C8 alcényle halogéné, C2-C8 alcynyle, C2-C8 alcynyle halogéné ou C3-C8 cycloalcényle ;
R₂₁ représente halogène, cyano, C3-C8 cycloalkyle, hydroxy, mercapto, C1-C8 alkoxy, C(O)R₂₂, carboxyle, C1-C8 alkoxycarbonyle, C1-C8 alkoxy-C1-C6 alkoxycarbonyle, -S(O)ₘ-(C1-C8)alkyle, hétéroaryle, hétérocyclyle ou phényle qui est non substitué ou substitué par un ou plusieurs groupes choisis parmi R₂₃ ;
R₁₇ et R₂₂ représentent chacun indépendamment hydrogène, C1-C8 alkyle ou N(R₂₄)R₂₅ ;
R₂₃ représente halogène, cyano, nitro, C1-C8 alkyle, C1-C8 alkyle qui est non substitué ou substitué par un substituant choisi parmi R₃₁, C3-C8 cycloalkyle, C3-C8 cycloalkyle halogéné, C2-C8 alcényle, C2-C8 alcényle halogéné, C2-C8 alcynyle, C2-C8 alcynyle halogéné, C3-C8 cycloalcényle, C1-C8 alkylcarbonyle, C3-C8 cycloalkylcarbonyle, C1-C8 alkylcarbonyle halogéné, C3-C8 cycloalkylcarbonyle halogéné, C1-C8 alkoxycarbonyle, C1-C8 alkoxycarbonyle halogéné, C1-C8 alkylaminocarbonyle, C1-C8 alkylaminocarbonyle halogéné, bis(C1-C8 alkyl)aminocarbonyle, OR₃₂, S(O)ₘR₃₃, C1-C8 alkylaminosulfonyle, bis(C1-C8 alkyl)aminosulfonyle, NH₂, C1-C8 alkylamino, bis(C1-C8 alkyl)amino, aryle, hétéroaryle ou hétérocyclyle ;
R₂₄ et R₂₅ représentent chacun indépendamment hydrogène, C1(C8 alkyle ou phényle ; ou,
chaîne C2-C8 alkylidène formée par R₂₄ et R₂₅, et le(s) atome(s) d'azote lié(s) à R₂₄ et R₂₅ forment conjointement un cycle à 3-7 chaînons, ladite chaîne C2-C8 alkylidène contenant éventuellement un O, S, S(O), S(O)₂, NH ou N-alkyle et étant éventuellement substituée par un groupe oxo ou thio ;
R₁₃, R₁₄, R₁₈ et R₃₁ représentent chacun indépendamment halogène, cyano, nitro, carboxyle, C1-C8 alkoxycarbonyle, C1-C8 alkoxy-C1-C8 alkoxycarbonyle, S(O)ₘR₄₁, OR₄₂, aryle, hétéroaryle ou hétérocyclyle ;
R₃₂ représente hydrogène, C1-C8 alkyle, C1-C8 alkyle halogéné, C3-C8 cycloalkyle, C3-C8 cycloalkyle halogéné, C2-C8 alcényle, C2-C8 alcényle halogéné, C2-C8 alcynyle, C2-C8 alcynyle halogéné ou C3-C8 cycloalcényle ;
R₃₃ représente C1-C8 alkyle, C1-C8 alkyle halogéné, C3-C8 cycloalkyle, C2-C8 alcényle, C2-C8 alcényle halogéné, C2-C8 alcynyle, C2-C8 alcynyle halogéné ou C3-C8 cycloalcényle ;
R₄₁ et R₄₂ représentent chacun indépendamment hydrogène, C1-C8 alkyle, C1-C8 alkyle halogéné, C3-C8 cycloalkyle, C3-C8 cycloalkyle halogéné, C2-C8 alcényle, C2-C8 alcényle halogéné, C2-C8 alcynyle, C2-C8 alcynyle halogéné, C3-C8 cycloalcényle ou phényle ;
r représente 1 ou 2 ;
m représente 0, 1 ou 2 ;
n représente 0 ou 1 ;
s représente 0, 1, 2 ou 3 ;
dans lequel, l'« hétérocyclyle » est 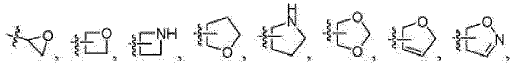 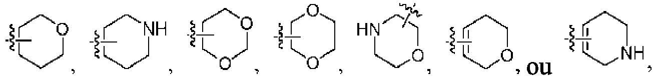 qui comporte 0, 1 ou 2 groupes oxo ; l'« aryle » est phényle, naphthyle, 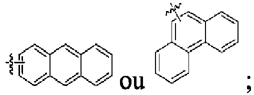 l'« hétéroaryle » est 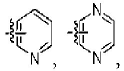 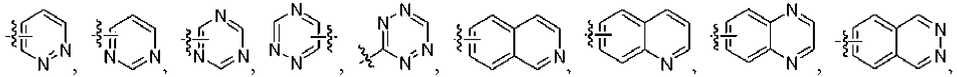 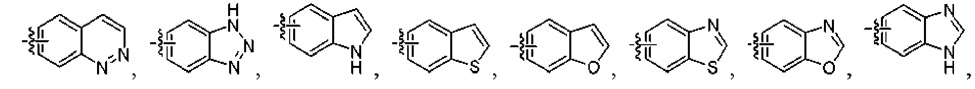 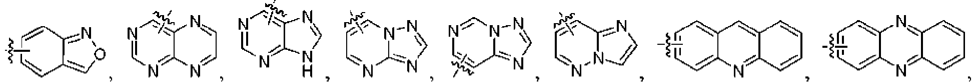 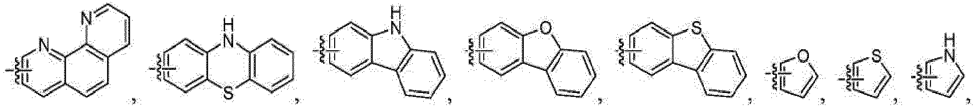 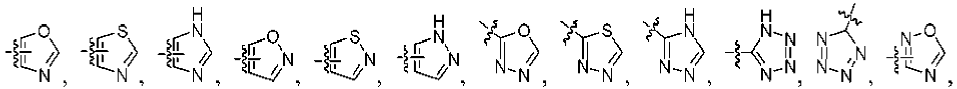 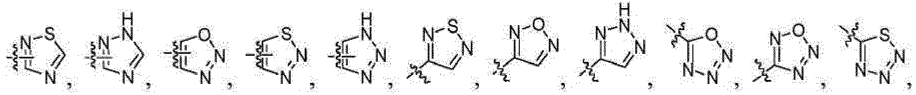 ou 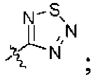 les groupes susmentionnés sont respectivement non substitués ou substitués par au moins un groupe choisi parmi : halogène, nitro, cyano, thiocyano, cyanoalkyle, mercapto, hydroxy, hydroxyalkyle, carboxyle, formyle, trialkylsilyle, dialkylphosphonyle ; hétérocyclyle, hétérocyclylalkyle, aryle, arylalkyle, hétéroaryle ou hétéroarylalkyle, qui est non substitué ou substitué ; groupe alkyle, alcényle, alcynyle, cycloalkyle, cycloalcényle, cycloalkylalkyle, cycloalkyle substitué par alkyle, OR", SR", - alkyl-OR", -O-alkyl-OR", -alkyl-SR", COR", -alkyl-COR", -O-alkyl-COR", COOR", -alkyl-COOR", -O-alkyl-COOR", COSR", SOR", SO₂R", -O-SO₂R", -alkyl-SO₂R", OCOR", -alkyl-OCOR" ou SCOR", qui est avec ou sans halogène ; et groupe amino, aminoalkyle, aminocarbonyle, aminocarbonylalkyle ou aminosulfonyle qui est non substitué ou substitué par un ou deux groupes choisi parmi R", COR", COOR", SO₂R" et OR", R", COR", COOR", SO₂R" ou OR" étant avec ou sans halogène ; ou, deux positions substituables adjacentes des groupes « hétérocyclyle », « aryle », « hétéroaryle » susmentionnés sont liées au groupe -OCH₂CH₂-, -OCH₂O-, -OCH₂CH₂O- ou -CH=CH-CH=CH- pour former un cycle, le groupe -OCH₂CH₂-, -OCH₂O-, -OCH₂CH₂O- ou -CH=CH-CH=CH- étant avec ou sans halogène ;
R" représente chacun indépendamment alkyle, alcényle, alcynyle, cycloalkyle, cycloalkylalkyle, cycloalcényle ; ou hétérocyclyle, hétérocyclylalkyle, aryle, arylalkyle, hétéroaryle ou hétéroarylalkyle, qui est non substitué ou substitué.

3. Le composé de 4-pyridinyle formamide ou dérivé de celui-ci selon la revendication 1 ou 2, qui est **caractérisé en ce que**,
X représente nitro, halogène, cyano, formyle, thiocyano, mercapto; C1-C6 alkyle, C2-C6 alcényle, C2-C6 alcynyle, C3-C6 cycloalkyle, C3-C6 cycloalcényle, C3-C6 cycloalkyl-C1-C3 alkyle ou C3-C6 cycloalcényl-C1-C3 alkyle, qui est avec ou sans halogène ; OR¹, COR¹, COOR¹, OCOR¹, OCOOR¹, NR³SO₂R², OSO₂R², S(O)ₘR², NR³COR¹, NR³COOR¹, C(O)NR³OR¹, SO₂OR¹, C(O)NR⁴R⁵, NR³C(O)NR⁴R⁵, OC(O)NR⁴R⁵, SO₂NR⁴R⁵, C(S)R¹, C(S)OR¹, C(S)SR², C(O)SR², SC(O)R¹, SC(S)R¹, OC(S)R¹, -(C1-C3)alkyl-C(S)R¹, -(C1-C3)alkyl-C(S)OR¹, -(C1-C3)alkyl-C(O)SR¹, -(C1-C3)alkyl-C(S)SR¹, -(C1-C3)alkyl-SC(O)R¹, -(C1-C3)alkyl-OC(S)R¹, - (C1-C3)alkyl-SC(S)R¹, -O-(C1-C3)alkyl-NR⁴R⁵, -S-(C1-C3)alkyl-NR⁴R⁵, -(C1-C3)alkyl-O-(C1-C3)alkyl-NR⁴R⁵, -(C1-C3)alkyl-S-(C1-C3)alkyl-NR⁴R⁵, -(C1-C3)alkyl-(C=S)ₙ-NR⁴R⁵, -NH-(C1-C3)alkyl-NR⁴R⁵, -(C1-C3)alkyl-OR¹, -(C1-C3)alkyl-COR¹, -(C1-C3)alkyl-CO₂R¹, -(C1-C3)alkyl-OCOR¹, -(C1-C3)alkyl-NR³COR¹, -(C1-C3)alkyl-SO₂OR¹, -(C1-C3)alkyl-NR³SO₂R², - (C1-C3)alkyl-OSO₂R², -alkyl-S(O)ₘR², -(C1-C3)alkyl-CONR⁴R⁵, -(C1-C3)alkyl-SO₂NR⁴R⁵, NR⁴R⁵, 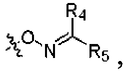 P(O)(OR⁶)₂, CH₂P(O)(OR⁶)₂, SO2NR⁴R⁵-(C1-C3)alkyl-S(O)ₘR², -(C1-C3)alkyl-CN, -(C1-C3)alkylaryle, -(C1-C3)alkylhétéroaryle, -(C1-C3)alkylhétérocyclyle, aryle, hétéroaryle ou hétérocyclyle ;
Y représente C1-C6 alkyle, C2-C6 alcényle, C2-C6 alcynyle, C3-C6 cycloalkyle ou C3-C6 cycloalcényle, qui est avec ou sans halogène ; OR¹, S(O)ₘR², NR⁴R⁵, hétérocyclyle, aryle ou hétéroaryle ;
R¹, R³, R⁴ et R⁵ représentent chacun indépendamment hydrogène, aryle, aryl-C1-C3 alkyle, hétéroaryle, hétéroaryl-C1-C3 alkyle, C1-C6 alkyle, C1-C6 alkyle halogéné, C2-C6 alcényle, C2-C6 alcényle halogéné, C2-C6 alcynyle, C2-C6 alcynyle halogéné, C3-C6 cycloalkyle, C3-C6 cycloalkyle halogéné, C1-C6 alkoxy-C1-C3 alkyle ou C3-C6 cycloalkyl-C1-C3 alkyle, les 10 derniers groupes tels que mentionnés étant chacun substitués par des groupes s choisis parmi le groupe constitué de cyano, halogène, nitro, thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸ et C1-C6 alkoxy-C1-C3 alkoxycarbonyle ;
R² représente aryle, aryl-C1-C3 alkyle, hétéroaryle, hétéroaryl-C1-C3 alkyle, C1-C6 alkyle, C2-C6 alcényle, C2-C6 alcynyle, C3-C6 cycloalkyle ou C3-C6 cycloalkyl-C1-C3 alkyle, les 5 derniers groupes tels que mentionnés étant chacun substitués par des groupes s choisis parmi le groupe constitué de cyano, halogène, nitro, thiocyano, OR⁷, S(O)ₘR⁹, NR⁷R⁸, NR⁸OR⁷, COR⁷, OCOR⁷, SCOR⁷, NR⁸COR⁷, CO₂R⁷, COSR⁷, CONR⁷R⁸ et C1-C6 alkoxy-C1-C3 alkoxycarbonyle ;
R⁶ représente méthyle ou éthyle ;
R⁷ et R⁸ représentent chacun indépendamment hydrogène, C1-C6 alkyle, C2-C6 alcényle ou C2-C6 alcynyle ;
R⁹ représente C1-C6 alkyle, C2-C6 alcényle ou C2-C6 alcynyle ;
M représente 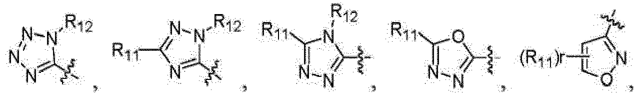 ou 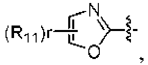 qui est non substitué ou substitué ;
R₁₁ représente hydrogène, halogène, cyano, nitro, C1-C6 alkyle qui est non substitué ou substitué par un substituant choisi parmi R₁₃, C3-C6 cycloalkyle qui est non substitué ou substitué par un substituant choisi parmi R₁₄, C2-C6 alcényle, C2-C6 alcényle halogéné, C2-C6 alcynyle, C2-C6 alcynyle halogéné, C3-C6 cycloalcényle, NH₂, aminoacyle, carboxyle, C1-C6 alkoxy-C1-C3 alkoxycarbonyle, OR₁₅, -(C1-C3)alkyl-OR₁₅, C(O)R₁₆, -(C1-C3)alkyl-C(O)R₁₆, C(O)OR₁₆, - (C1-C3)alkyl-C(O)OR₁₆, S(O)ₘR₁₆, -(C1-C3)alkyl-S(O)ₘR₁₆, -N(R₁₆)₂, -NHR₁₆, -C(O)NHR₁₆, - C(O)N(R₁₆)₂, -NHC(O)R₁₇, hétérocyclyle, hétérocyclyl-C1-C3 alkyle, hétérocyclyloxy, hétérocyclylcarbonyle, aryle, aryl-C1-C3 alkyle, aryloxy, arylcarbonyle, hétéroaryle, hétéroaryl-C1-C3 alkyle, hétéroaryloxy ou hétéroarylcarbonyle ;
R₁₂ représente hydrogène, C1-C6 alkyle qui est non substitué ou substitué par un substituant choisi parmi R₁₈, C3-C6 cycloalkyle, C3-C6 cycloalkyle halogéné, C2-C6 alcényle, C2-C6 alcényle halogéné, C2-C6 alcynyle, C2-C6 alcynyle halogéné, C3-C6 cycloalcényle, C1-C6 alkoxy-C1-C3 alkyle, C1-C6 alkylthio-C1-C3 alkyle, C1-C6 alkoxycarbonyl-C1-C3 alkyle, aryle ou aryl-C1-C3 alkyle ;
lorsque M est 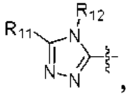 -(CH₂)₄- ou -CH=CH-CH=CH- formé par R₁₁ et R₁₂, l'atome d'azote lié à R₁₂ et l'atome de carbone lié à R₁₁ forment conjointement un cycle à 6 chaînons ;
R₁₅ représente C1-C6 alkyle qui est non substitué ou substitué par un substituant choisi parmi R₂₁, C3-C6 cycloalkyle, C3-C6 cycloalkyle halogéné, C2-C6 alcényle, C2-C6 alcényle halogéné, C2-C6 alcynyle, C2-C6 alcynyle halogéné, C3-C6 cycloalcényle ou phényle ;
R₁₆ représente C1-C6 alkyle, C1-C6 alkyle halogéné, C3-C6 cycloalkyle, C2-C6 alcényle, C2-C6 alcényle halogéné, C2-C6 alcynyle, C2-C6 alcynyle halogéné ou C3-C6 cycloalcényle ;
R₂₁ représente halogène, cyano, C3-C6 cycloalkyle, hydroxy, mercapto, C1-C6 alkoxy, C(O)R₂₂, carboxyle, C1-C6 alkoxycarbonyle, C1-C6 alkoxy-C1-C3 alkoxycarbonyle, -S(O)ₘ-(C1-C6)alkyle, hétéroaryle, hétérocyclyle ou phényle qui est non substitué ou substitué par 1~3 groupes choisis parmi R₂₃ ;
R₁₇ et R₂₂ représentent chacun indépendamment hydrogène, C1-C6 alkyle ou N(R₂₄)R₂₅ ;
R₂₃ représente halogène, cyano, nitro, C1-C6 alkyle, C1-C6 alkyle qui est non substitué ou substitué par un substituant choisi parmi R₃₁, C3-C6 cycloalkyle, C3-C6 cycloalkyle halogéné, C2-C6 alcényle, C2-C6 alcényle halogéné, C2-C6 alcynyle, C2-C6 alcynyle halogéné, C3-C6 cycloalcényle, C1-C6 alkylcarbonyle, C3-C6 cycloalkylcarbonyle, C1-C6 alkylcarbonyle halogéné, C3-C6 cycloalkylcarbonyle halogéné, C1-C6 alkoxycarbonyle, C1-C6 alkoxycarbonyle halogéné, C1-C6 alkylaminocarbonyle, C1-C6 alkylaminocarbonyle halogéné, bis(C1-C6 alkyl)aminocarbonyle, OR₃₂, S(O)ₘR₃₃, C1-C6 alkylaminosulfonyle, bis(C1-C6 alkyl)aminosulfonyle, NH₂, C1-C6 alkylamino, bis(C1-C6 alkyl)amino, aryle, hétéroaryle ou hétérocyclyle ;
R₂₄ et R₂₅ représentent chacun indépendamment hydrogène, C1-C6 alkyle ou phényle ; ou,
chaîne C2-C6 alkylidène formée par R₂₄ et R₂₅, et le(s) atome(s) d'azote lié(s) à R₂₄ et R₂₅ forment conjointement un cycle à 3-7 chaînons, ladite chaîne C2-C6 alkylidène contenant éventuellement un O, S, S(O), S(O)₂, NH ou N-alkyle et étant éventuellement substituée par un groupe oxo ou thio ;
R₁₃, R₁₄, R₁₈ et R₃₁ représentent chacun indépendamment halogène, cyano, nitro, carboxyle, C1-C6 alkoxycarbonyle, C1-C6 alkoxy-C1-C6 alkoxycarbonyle, S(O)ₘR₄₁, OR₄₂, aryle, hétéroaryle ou hétérocyclyle ;
R₃₂ représente hydrogène, C1-C6 alkyle, C1-C6 alkyle halogéné, C3-C6 cycloalkyle, C3-C6 cycloalkyle halogéné, C2-C6 alcényle, C2-C6 alcényle halogéné, C2-C6 alcynyle, C2-C6 alcynyle halogéné ou C3-C6 cycloalcényle ;
R₃₃ représente C1-C6 alkyle, C1-C6 alkyle halogéné, C3-C6 cycloalkyle, C2-C6 alcényle, C2-C6 alcényle halogéné, C2-C6 alcynyle, C2-C6 alcynyle halogéné ou C3-C6 cycloalcényle ;
R₄₁ et R₄₂ représentent chacun indépendamment hydrogène, C1-C6 alkyle, C1-C6 alkyle halogéné, C3-C6 cycloalkyle, C3-C6 cycloalkyle halogéné, C2-C6 alcényle, C2-C6 alcényle halogéné, C2-C6 alcynyle, C2-C6 alcynyle halogéné, C3-C6 cycloalcényle ou phényle ;
r représente 1 ou 2 ;
m représente 0, 1 ou 2 ;
n représente 0 ou 1 ;
s représente 0, 1, 2 ou 3 ;
dans lequel l'« hétérocyclyle » est 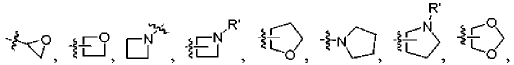 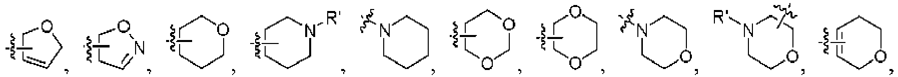 ou 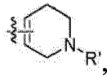 qui comporte 0, 1 ou 2 groupes oxo ; l'« aryle » est phényle, naphthyle, 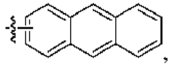 ou 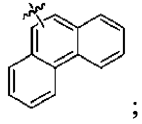 l'« hétéroaryle » est 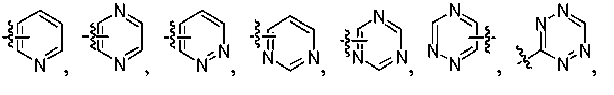 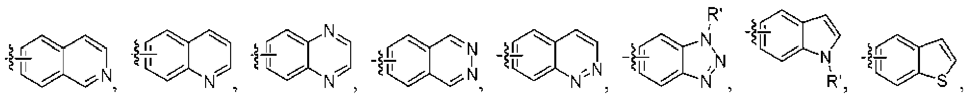 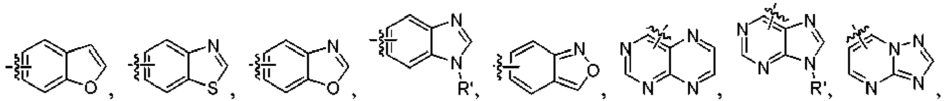 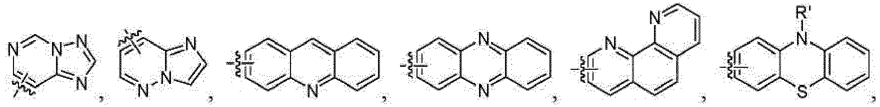 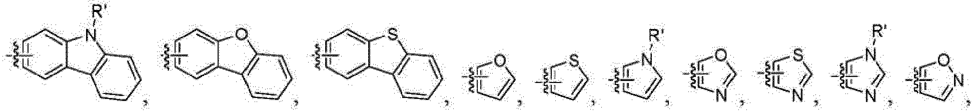 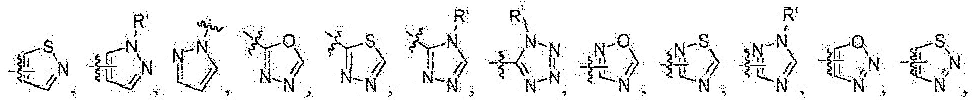 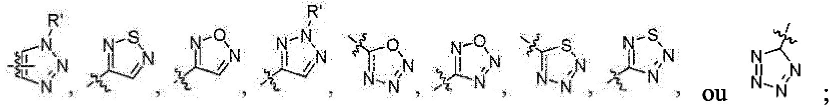 les groupes susmentionnés sont respectivement non substitués ou substitués par 1~3 groupes choisis parmi : halogène, nitro, cyano, thiocyano, cyano C1-C6 alkyle, mercapto, hydroxy, hydroxy C1-C6 alkyle, carboxyle, formyle ; 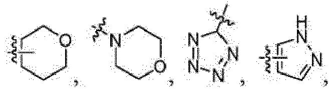 groupe phényle ou benzyle, qui est non substitué ou substitué par 1~3 groupes choisis parmi halogène, hydroxy, nitro, cyano, amino, carboxyle, C1-C6 alkyle avec ou sans halogène, C2-C6 alcényle avec ou sans halogène, C2-C6 alcynyle avec ou sans halogène, C3-C6 cycloalkyle avec ou sans halogène, C1-C6 alkoxy avec ou sans halogène, C1-C6 alkoxycarbonyle avec ou sans halogène, C1-C6 alkylacyle avec ou sans halogène, C1-C6 alkylacyloxy avec ou sans halogène, C1-C6 alkylamino avec ou sans halogène et C1-C6 alkylsulfonyle avec ou sans halogène ; groupe C1-C6 alkyle, C2-C6 alcényle, C2-C6 alcynyle, C3-C6 cycloalkyle, C3-C6 cycloalkyl-C1-C6 alkyle, C3-C6 cycloalkyle substitué par C1-C6 alkyle, OR", SR", -(C1-C6) alkyl -OR", -O-(C1-C6) alkyl -OR", -(C1-C6) alkyl -SR", COR", -(C1-C6) alkyl -COR", -O-(C1-C6) alkyl -COR", COOR", -(C1-C6) alkyl -COOR", -O-(C1-C6) alkyl -COOR", COSR", SOR", SO₂R", -O-SO₂R", -(C1-C6) alkyl -SO₂R", OCOR", - (C1-C6) alkyl -OCOR" ou SCOR", qui est avec ou sans halogène ; et groupe amino, aminoalkyle, aminocarbonyle ou aminosulfonyle, qui est non substitué ou substitué par un ou deux groupes choisis parmi R", COR", COOR", SO₂R" et OR", R", COR", COOR", SO₂R" ou OR" étant avec ou sans halogène ; ou, deux positions substituables adjacentes des groupes « hétérocyclyle », « aryle », « hétéroaryle » susmentionnés sont liées au groupe -OCH₂CH₂-, -OCH₂O-, - OCH₂CH₂O- ou -CH=CH-CH=CH- pour former un cycle, -le groupe OCH₂CH₂-, -OCH₂O-, - OCH₂CH₂O- ou -CH=CH-CH=CH- étant avec ou sans halogène ;
R' représente chacun indépendamment hydrogène, nitro, hydroxy, amino; C1-C6 alkyle, C2-C6 alcényle, C2-C6 alcynyle, C3-C6 cycloalkyle, C3-C6 cycloalcényle, C3-C6 cycloalkyl-C1-C6 alkyle, 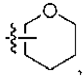 C1-C6 alkoxy, C2-C6 alcényloxy, C2-C6 alcynyloxy, C3-C6 cycloalkyloxy, C1-C6 alkoxy-C1-C6 alkyle, C1-C6 alkoxycarbonyle, C1-C6 alkylthiocarbonyle, C1-C6 alkylsulfonyle, C1-C6 alkylsulfonyl-C1-C6 alkyle, C1-C6 alkylcarbonyle, C1-C6 alkylcarbonyl-C1-C6 alkyle, C1-C6 alkylacyloxy, C1-C6 alkylamino, C1-C6 alkylaminocarbonyle, C1-C6 alkoxyaminocarbonyle, C1-C6 alkoxycarbonyl-C1-C6 alkyle, C1-C6 alkylaminocarbonyl-C1-C6 alkyle, tri(C1-C6 alkyl)silyle ou di(C1-C6 alkyl)phosphonyle, qui est avec ou sans halogène ; ou phényle, ou benzyle qui est non substitué ou substitué par 1~3 groupes choisis parmi halogène, hydroxy, nitro, cyano, amino, carboxyle, C1-C6 alkyle avec ou sans halogène, C2-C6 alcényle avec ou sans halogène, C2-C6 alcynyle avec ou sans halogène, C3-C6 cycloalkyle avec ou sans halogène, C1-C6 alkoxy avec ou sans halogène, C1-C6 alkoxycarbonyle avec ou sans halogène, C1-C6 alkylacyle avec ou sans halogène, C1-C6 alkylacyloxy avec ou sans halogène, C1-C6 alkylamino avec ou sans halogène et C1-C6 alkylsulfonyle avec ou sans halogène ;
R" représente chacun indépendamment C1-C6 alkyle, C2-C6 alcényle, C2-C6 alcynyle, C3-C6 cycloalkyle, C3-C6 cycloalkyl-C1-C6 alkyle, C3-C6 cycloalcényle ; ou 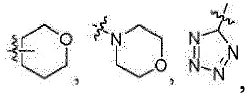 phényle ou benzyle qui est non substitué ou substitué par 1~3 groupes choisis parmi halogène, hydroxy, nitro, cyano, amino, carboxyle, C1-C6 alkyle avec ou sans halogène, C2-C6 alcényle avec ou sans halogène, C2-C6 alcynyle avec ou sans halogène, C3-C6 cycloalkyle avec ou sans halogène, C1-C6 alkoxy avec ou sans halogène, C1-C6 alkoxycarbonyle avec ou sans halogène, C1-C6 alkylacyle avec ou sans halogène, C1-C6 alkylacyloxy avec ou sans halogène, C1-C6 alkylamino avec ou sans halogène et C1-C6 alkylsulfonyle avec ou sans halogène.

4. Le composé de 4-pyridinyle formamide ou dérivé de celui-ci selon l'une quelconque des revendications 1 à 3, qui est **caractérisé en ce que**,
X représente halogène, nitro, cyano, OR¹, S(O)ₘR², NR³COR¹, NR⁴R⁵, 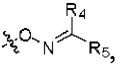 C1-C6 alkoxy-C1-C3 alkyle ; C1-C6 alkyle, C2-C6 alcényle, C2-C6 alcynyle ou C3-C6 cycloalkyle, qui est avec ou sans halogène ; 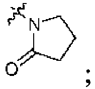 ou phényle qui est non substitué ou substitué par 1, 2 ou 3 groupes choisis parmi halogène et C1-C6 alkoxy ;
Y représente C1-C6 alkyle, C2-C6 alcényle, C2-C6 alcynyle, C3-C6 cycloalkyle ou C3-C6 cycloalcényle, qui est avec ou sans halogène ; OR¹, S(O)ₘR², NR⁴R⁵, hétérocyclyle, aryle ou hétéroaryle ;
R¹, R³, R⁴ et R⁵ représentent chacun indépendamment hydrogène, C1-C6 alkyle, C1-C6 alkyle halogéné, phényle ; ou benzyle qui est non substitué ou substitué par 1, 2 ou 3 groupes choisis parmi halogène et C1-C6 alkoxy ;
R² représente C1-C6 alkyle ou C1-C6 alkyle halogéné ;
M représente 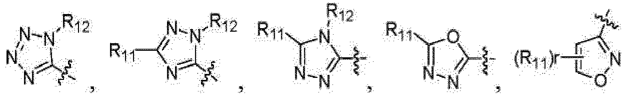 ou 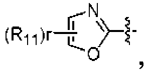 qui est non substitué ou substitué ;
R₁₁ représente hydrogène, C1-C6 alkyle, C3-C6 cycloalkyle, C1-C6 alkyle halogéné, C1-C6 alkoxy, C1-C6 alkylthio, halogène, C1-C6 alkylamino, bis(C1-C6 alkyl)amino, cyano, nitro, C1-C6 alkylcarbonyle, C1-C6 alkoxycarbonyle, C1-C6 alkoxycarbonyl-C1-C3 alkyle, aminocarbonyle, C1-C6 alkylcarbonylamino, C1-C6 alkoxy-C1-C3 alkyle, C1-C6 alkylthio-C1-C3 alkyle, C1-C6 alkylsulfinyl-C1-C3 alkyle, C1-C6 alkylsulfonyl-C1-C3 alkyle ; ou benzyle, phénoxy, benzoyle, pyridyle, 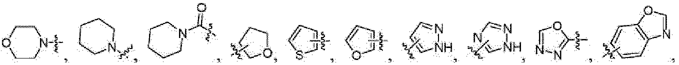 ou 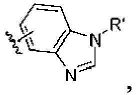 qui est non substitué ou substitué par 1, 2 ou 3 groupes choisis parmi halogène et C1-C6 alkoxy ;
R₁₂ représente hydrogène, C1-C6 alkyle, C2-C6 alcényle, C1-C6 alkoxy-C1-C3 alkyle, C1-C6 alkylthio-C1-C3 alkyle, C1-C6 alkoxycarbonyl-C1-C3 alkyle ; ou phényle ou benzyle qui est non substitué ou substitué par au moins un groupe choisi parmi halogène et C1-C6 alkyle halogéné ;
r représente 1 ou 2 ;
m représente 0, 1 ou 2 ;
dans lequel, l'« hétérocyclyle » est 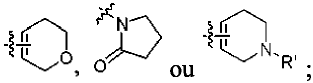 l'« aryle » est phényle,
naphthyle ou 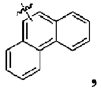 qui est non substitué ou substitué ; l'« hétéroaryle » est 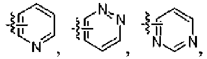 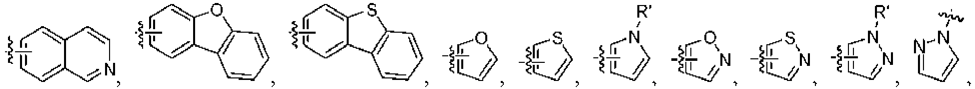 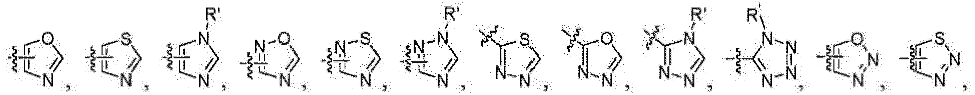 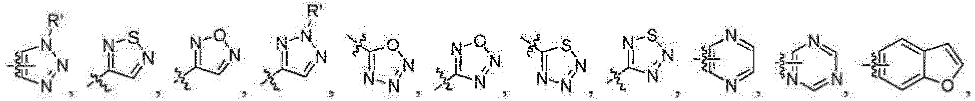 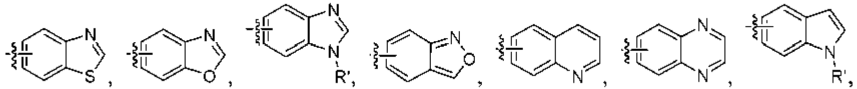 ou 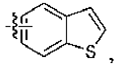 qui est non substitué ou substitué ; le terme « substitué » susmentionné fait respectivement référence à une substitution par 1~3 groupes choisis parmi : groupe halogène, nitro, cyano, thiocyano, cyano C1-C3 alkyle, hydroxy, hydroxy C1-C3 alkyle, mercapto, carboxyle, formyle, 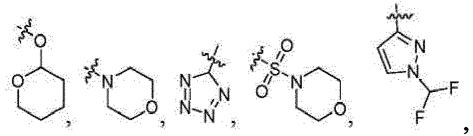 phényle, phényle substitué par C1-C6 alkyle, phénoxy, benzyloxy; amino, aminoalkyle ou aminocarbonyle qui est non substitué ou substitué par un ou deux groupes choisis parmi C1-C6 alkyle, COR" et COOR" ; et C1-C6 alkyle, C2-C6 alcényle, C3-C6 cycloalkyle, C1-C6 alkoxy-C1-C3 alkyle, C1-C6 alkylthio-C1-C3 alkyle, C1-C6 alkylcarbonylthio, C3-C6 cycloalkyle substitué par C1-C6 alkyle, OR", SR", SOR", COR", COOR" ou SO₂R", qui est avec ou sans halogène ; ou, deux positions substituables adjacentes des groupes « hétérocyclyle », « aryle », « hétéroaryle » susmentionnés sont liées au groupe - OCH₂CH₂-, -OCH₂O-, -OCH₂CH₂O- ou -CH=CH-CH=CH- pour former un cycle, le groupe - OCH₂CH₂-, -OCH₂O-, -OCH₂CH₂O- ou -CH=CH-CH=CH- étant avec ou sans halogène ;
R' représente chacun indépendamment hydrogène, C1-C6 alkyle, C1-C6 alkylcarbonyle, C1-C6 alkyle halogéné, 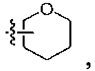 C1-C6 alkoxycarbonyle, C1-C6 alkoxy-C1-C3 alkyle ou benzyle ;
R" représente chacun indépendamment C1-C6 alkyle ou C2-C6 alcényle.

5. Le composé de 4-pyridinyle formamide ou dérivé de celui-ci selon l'une quelconque des revendications 1 à 4, qui est **caractérisé en ce que**,
X représente chlore, fluorine, bromine, méthyle, éthyle, isopropyle, vinyle, allyle, éthynyle, cyclopropyle, trifluorométhyle, 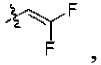 hydroxy, méthoxy, éthyloxy, méthylthio, éthylthio, 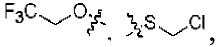 nitro, cyano, méthylsulfinyle, éthylsulfinyle, méthylsulfonyle, amino, monométhylamino, diméthylamino, acétamido, 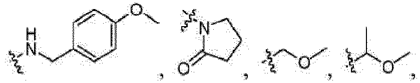 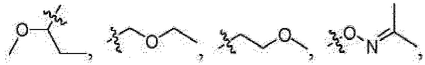 phényle, phénoxy ou 4-fluorophényle ;
Y représente méthyle, éthyle, vinyle, méthoxy, 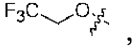 phénoxy, benzyloxy, méthylthio, propylthio, tert-butylthio, benzylthio, 4-méthoxybenzylthiol, propylsulfmyle, amino, monométhylamino, diméthylamino, anilino, p-méthoxybenzylamino, tert-butyle, cyclopropyle, cyclohexyle, 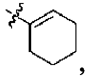 hétérocyclyle, aryle ou hétéroaryle ;
M représente 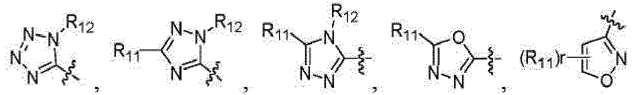 ou 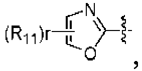 qui est non substitué ou substitué ;
R₁₁ représente chacun indépendamment hydrogène, méthyle, éthyle, n-propyle, isopropyle, cyclopropyle, monochlorométhyle, trifluorométhyle, méthoxy, isopropyloxy, méthylthio, fluorine, chlore, bromine, iodine, diméthylamino, éthylamino, cyano, nitro, acétyle, méthoxycarbonyle ; phényle qui est non substitué ou substitué par 1, 2 ou 3 groupes choisis parmi chlore et méthoxy ; pyridyle, 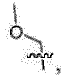 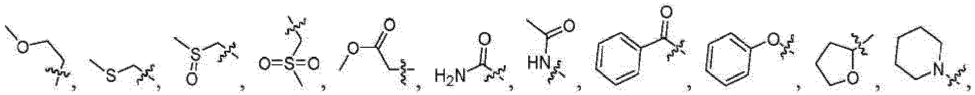 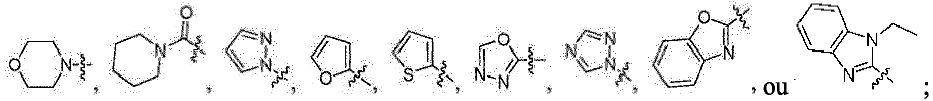
R₁₂ représente chacun indépendamment hydrogène, méthyle, éthyle, n-propyle, isopropyle, allyle, 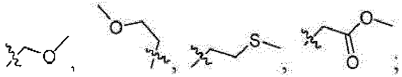 ou phényle, ou benzyle qui est non substitué ou substitué par 1, 2 ou 3 groupes choisis parmi chlore et trifluorométhyle ;
r représente 1 ou 2 ; dans lequel l'« hétérocyclyle » est 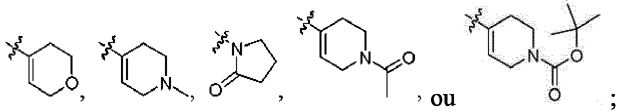 l'« aryle » est phényle, naphthyle ou 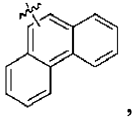 qui est non substitué ou substitué ; l'« hétéroaryle » est 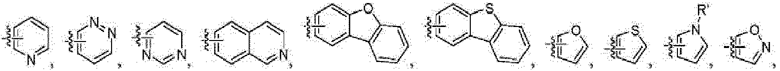 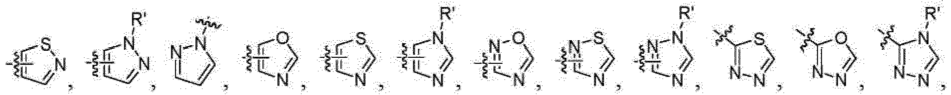 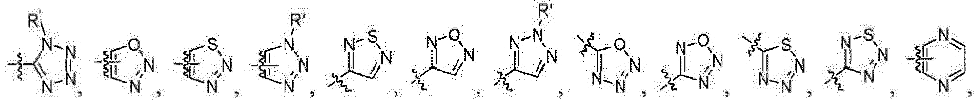 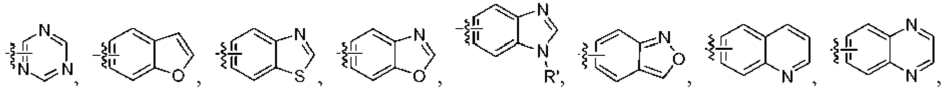 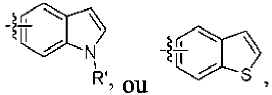 qui est non substitué ou substitué ; le terme « substitué » susmentionné fait référence à une substitution par 1, 2 ou 3 groupes choisis parmi : méthyle, éthyle, isopropyle, n-butyle, tert-butyle, n-pentyle, vinyle, cyclopropyle, 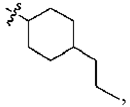 fluorine, chlore, bromine, iodine, monobromométhyle, difluorométhyle, trifluorométhyle, méthoxy, éthoxy, propoxy, butoxy, trifluorométhoxy, hydroxyle, hydroxyméthyle, amino, cyano, cyanométhyle, thiocyano, mercapto, nitro, carboxy, formyle, acétyle, méthoxycarbonyle, tert-butyloxycarbonyle, méthylthio, isopropylthio, méthyle sulfinyle, méthyle sulfonyle, 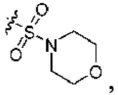 diméthylamino, aminocarbonyle, diméthylaminocarbonyle, acétylamino, 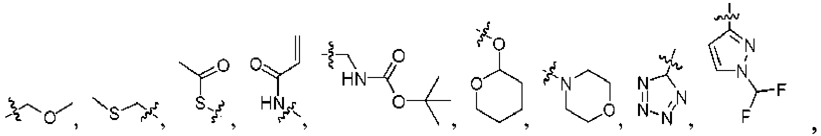 phényl, 4-éthylphényle, phénoxy et benzyloxy ; ou, deux positions substituables adjacentes des groupes « aryle », « hétéroaryle » susmentionnés sont liées au groupe -OCH₂CH₂-, -OCH₂O-, - OCH₂CH₂O- ou -CH=CH-CH=CH- pour former un cycle, le groupe -OCH₂CH₂-, -OCH₂O-, - OCH₂CH₂O- ou -CH=CH-CH=CH- étant avec ou sans halogène ;
R' représente chacun indépendamment hydrogène, méthyle, éthyle, n-propyle, isopropyle, difluorométhyle, 3,3,3-trifluoroéthyle, benzyle, 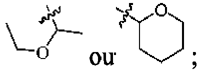
dans lequel, lorsque X est fluorine, Y n'est pas amino et monométhylamino ; lorsque M est 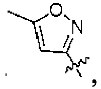 X et Y ne sont pas méthyle en même temps.

6. Le composé de 4-pyridinyle formamide ou dérivé de celui-ci selon l'une quelconque des revendications 1 à 5, qui est **caractérisé en ce que**, le composé de 4-pyridinyle formamide est représenté par la Formule I : 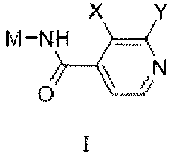 dans lequel, X, Y et M sont listés comme suit :

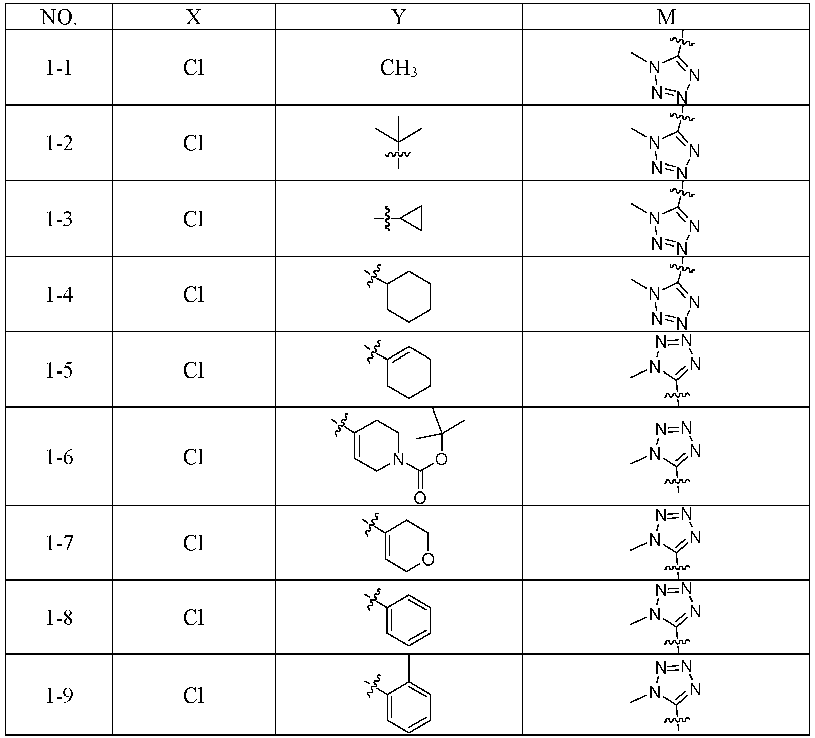

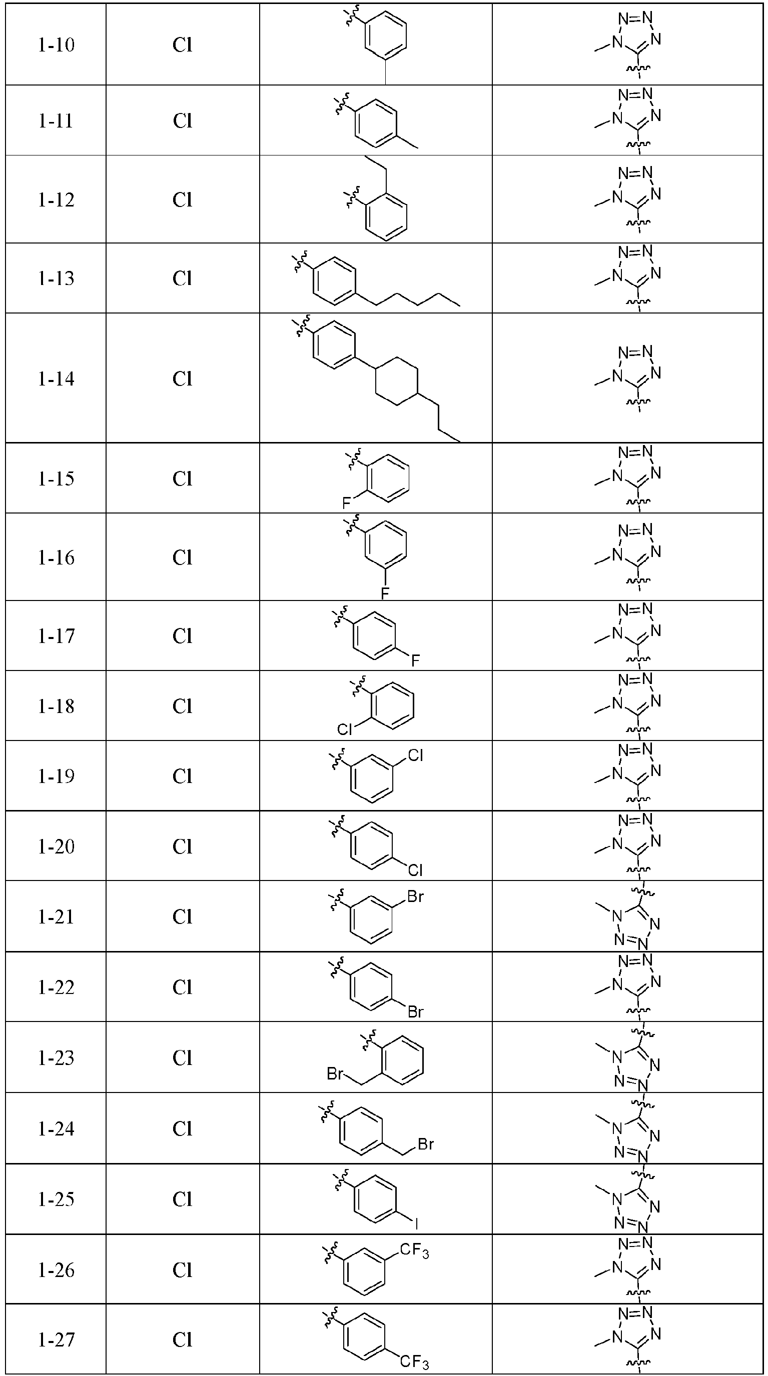

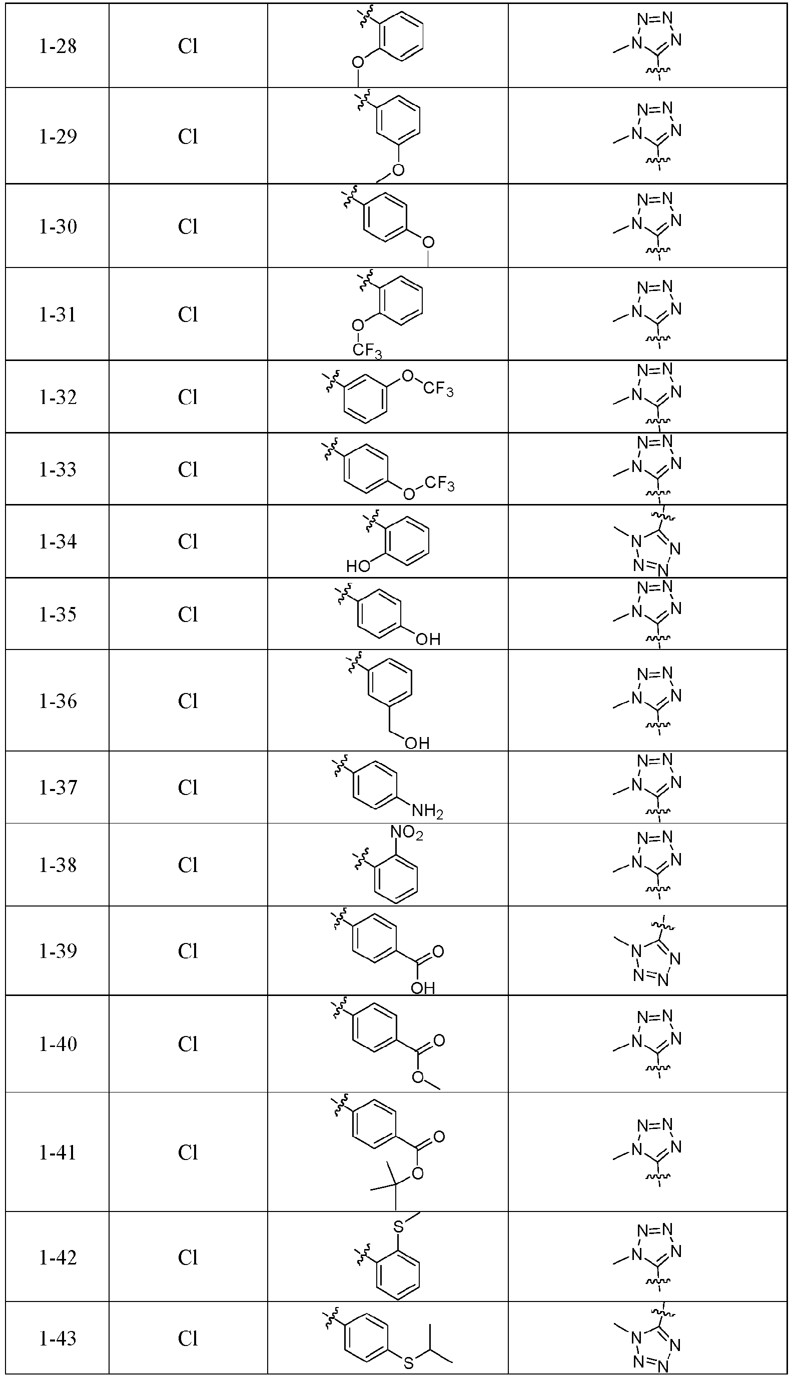

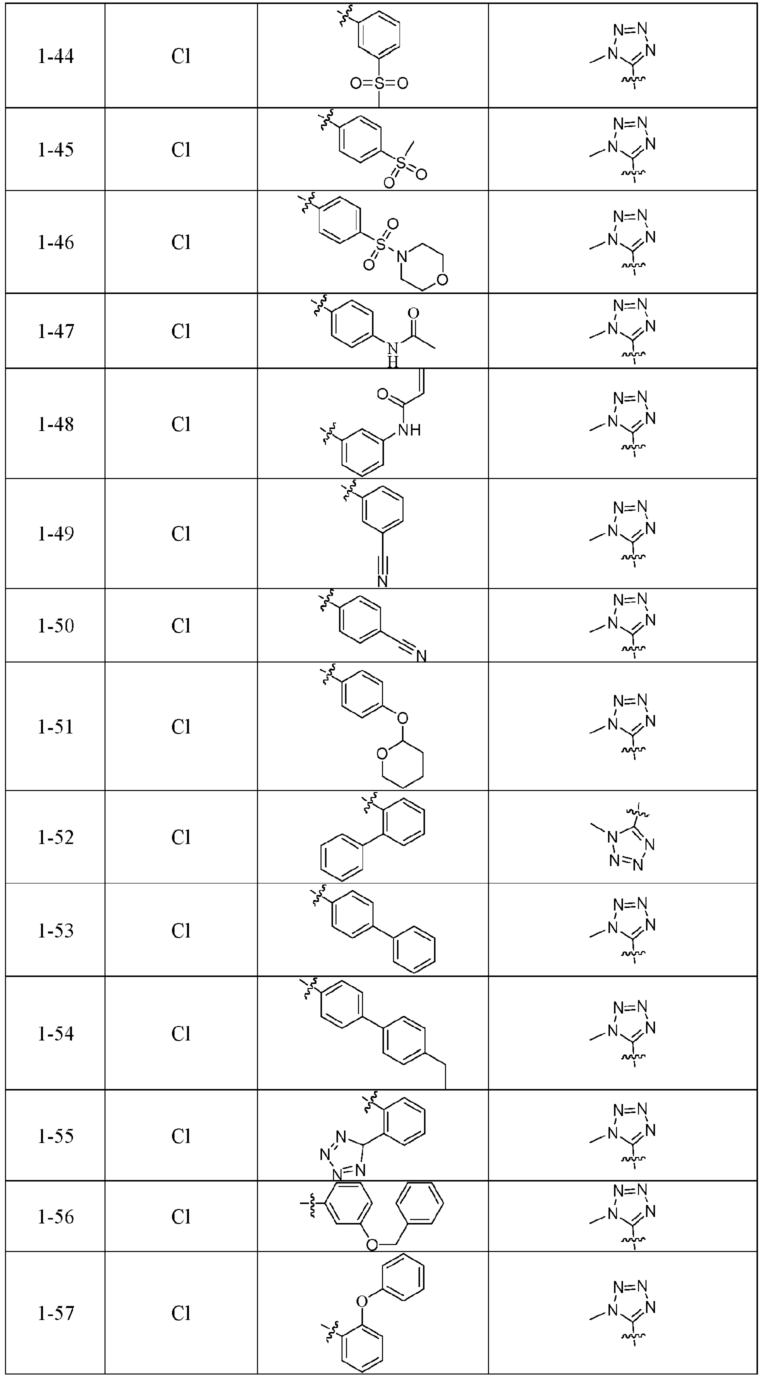

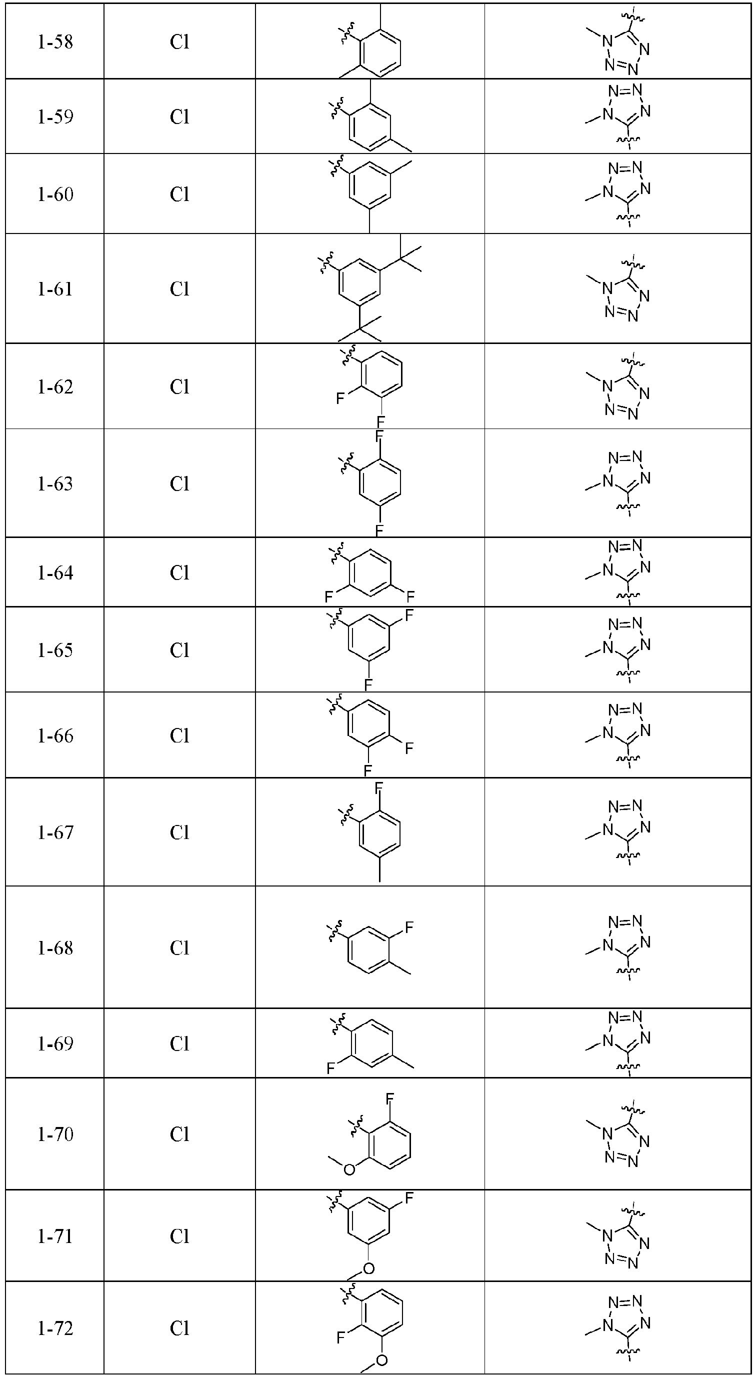

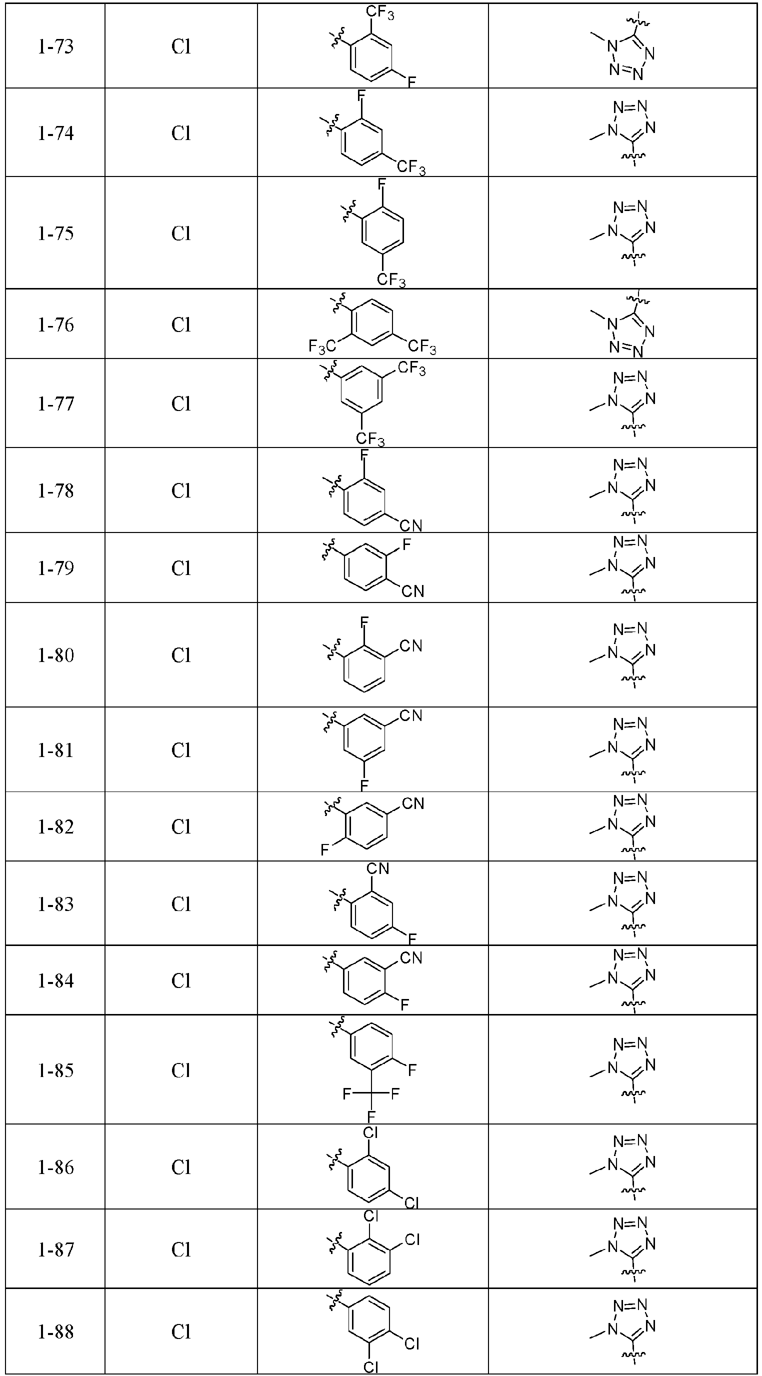

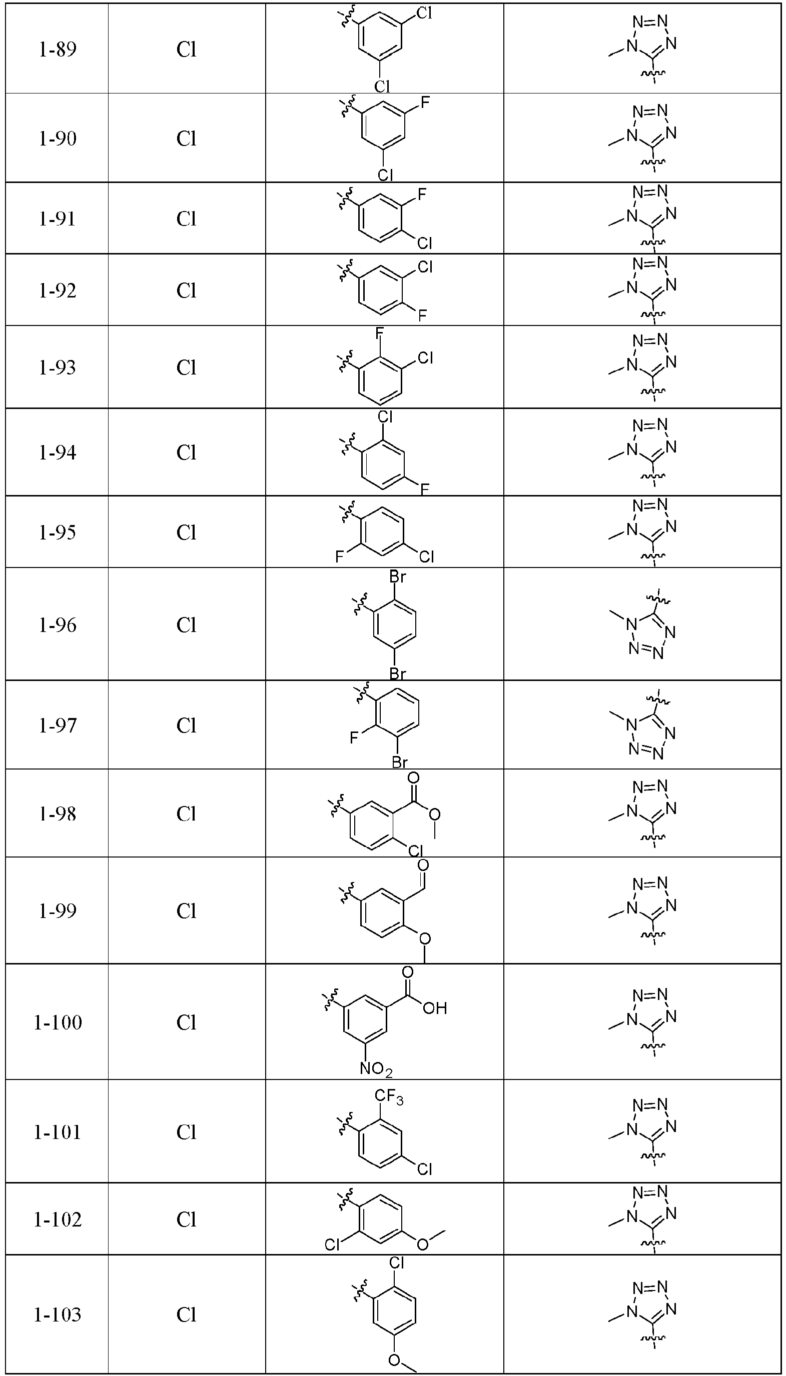

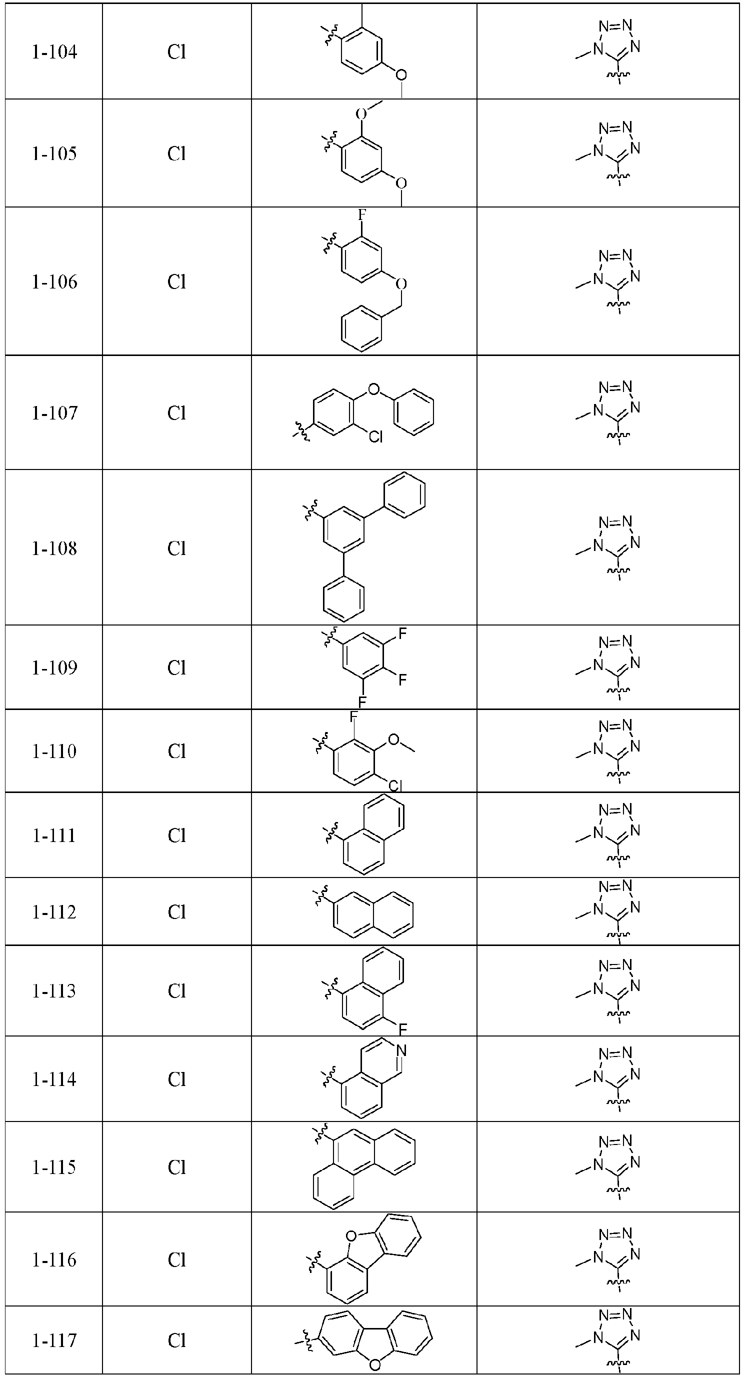

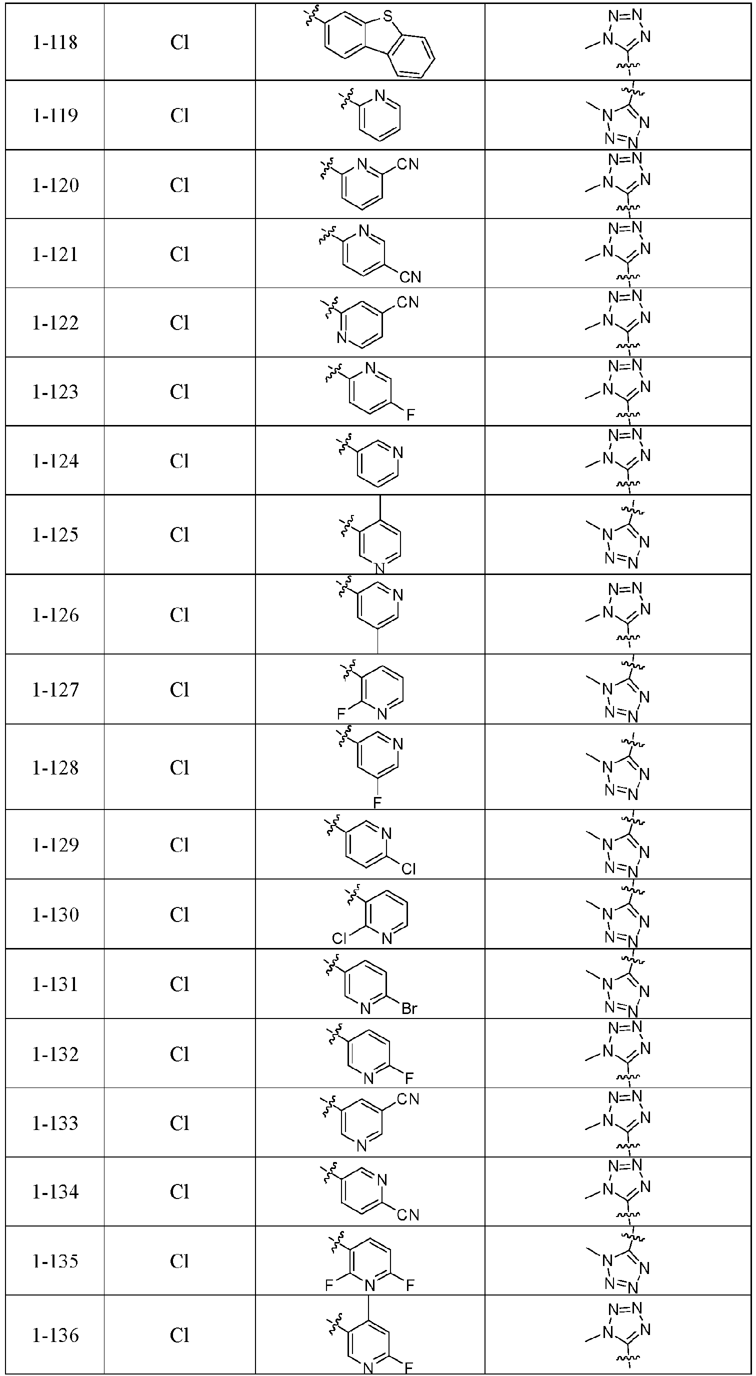

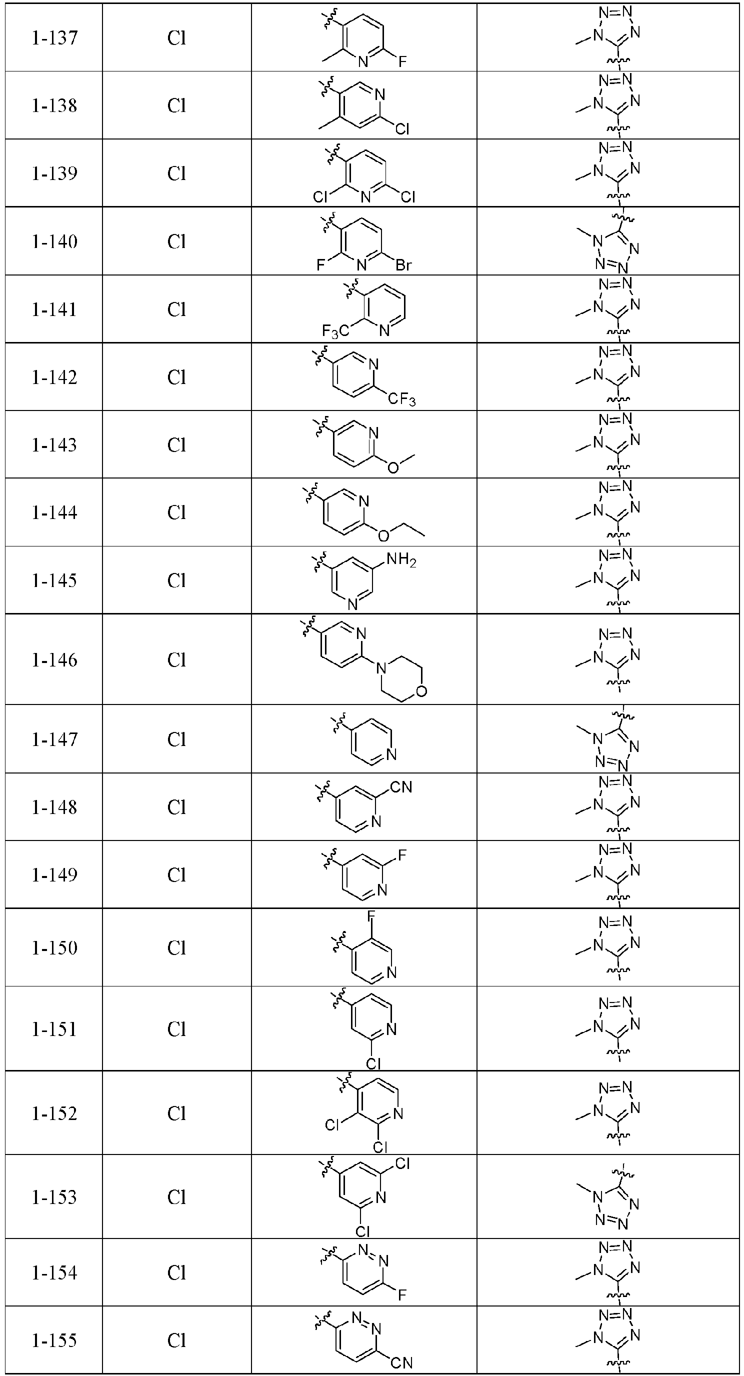

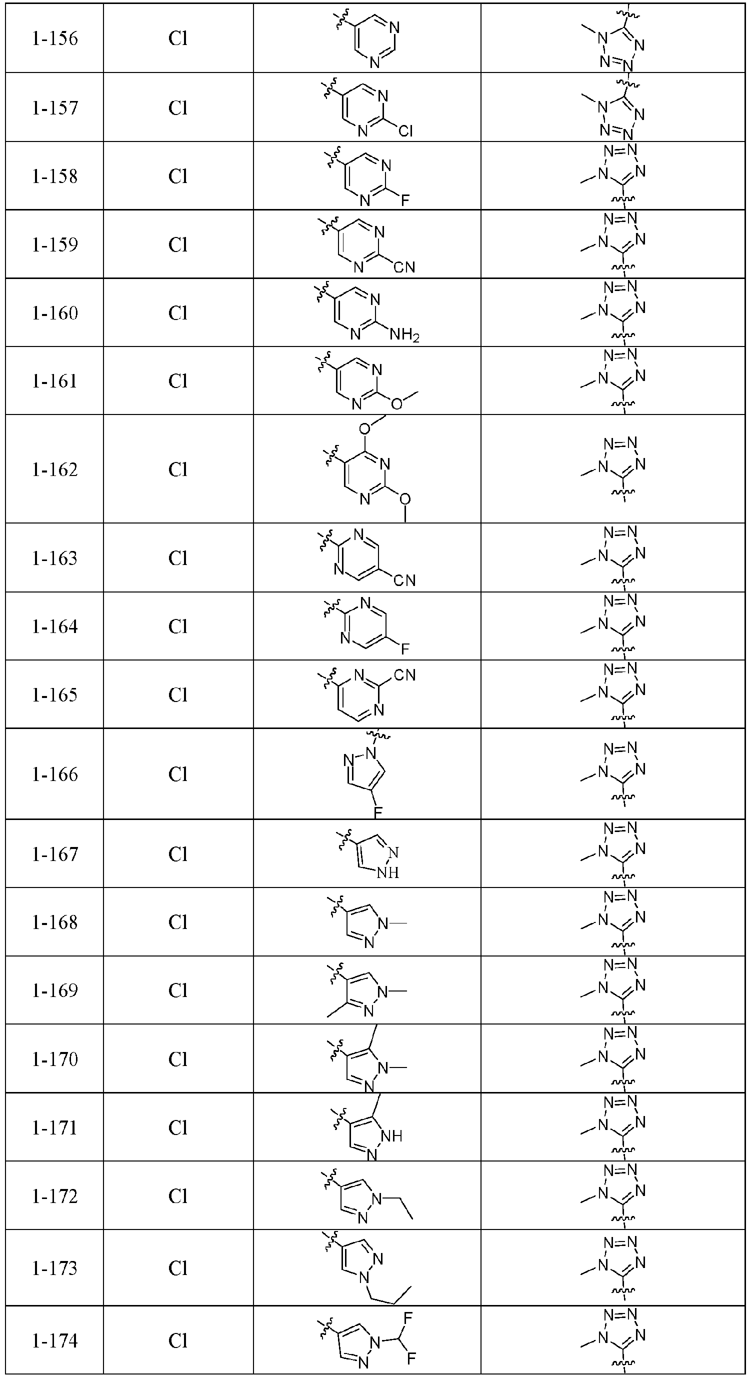

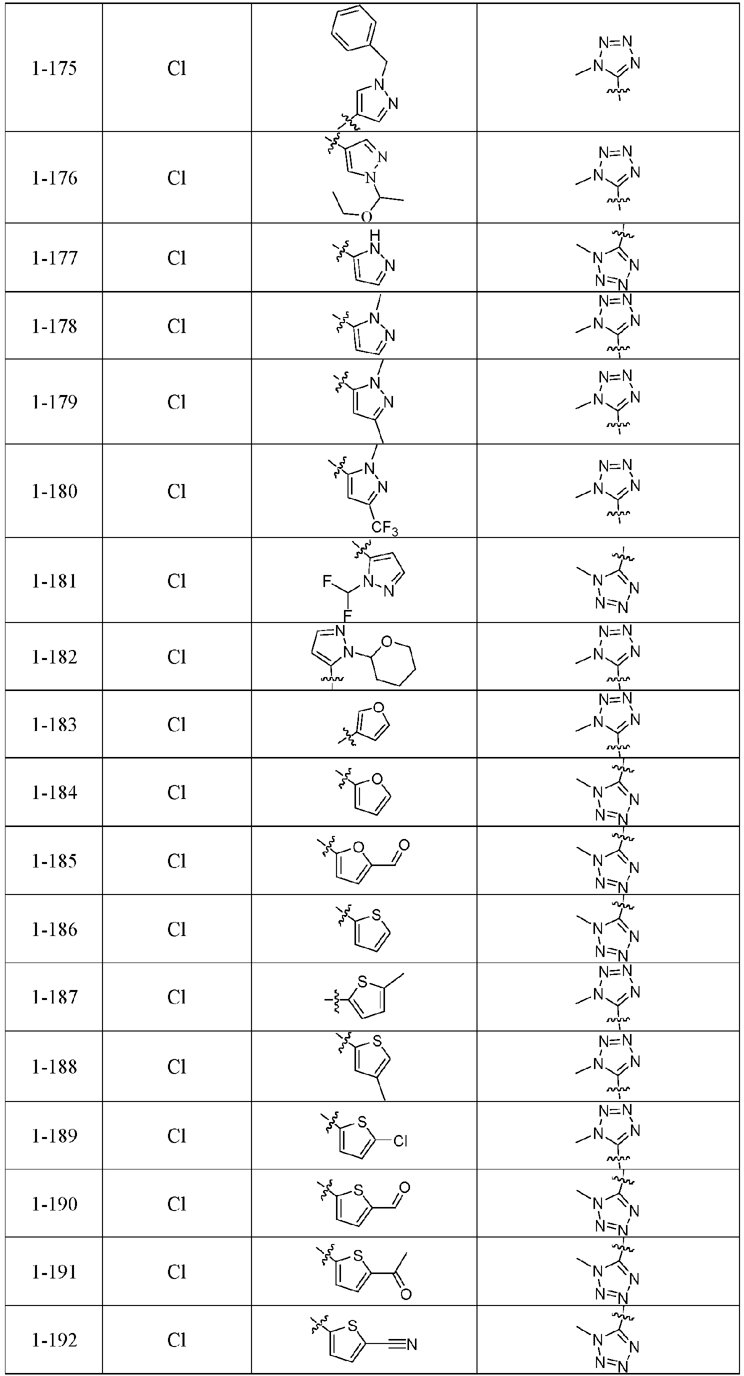

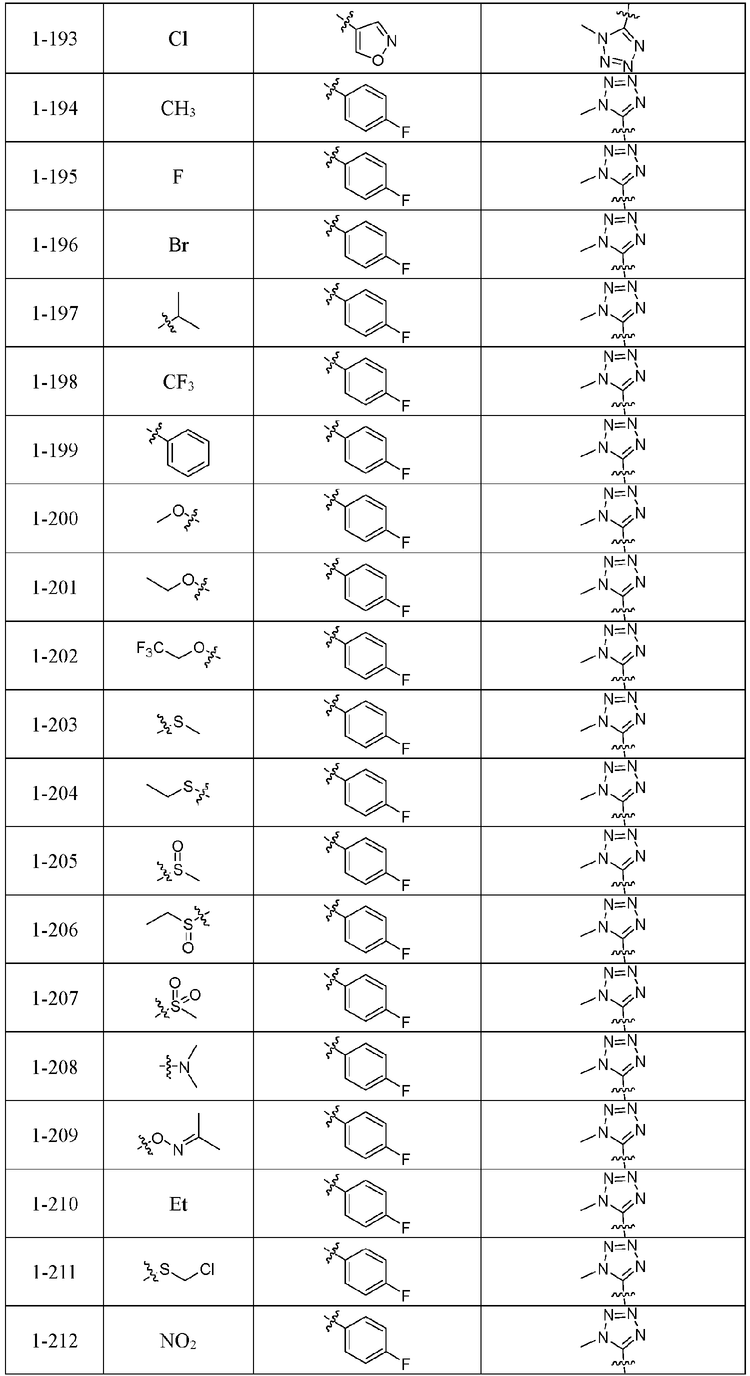

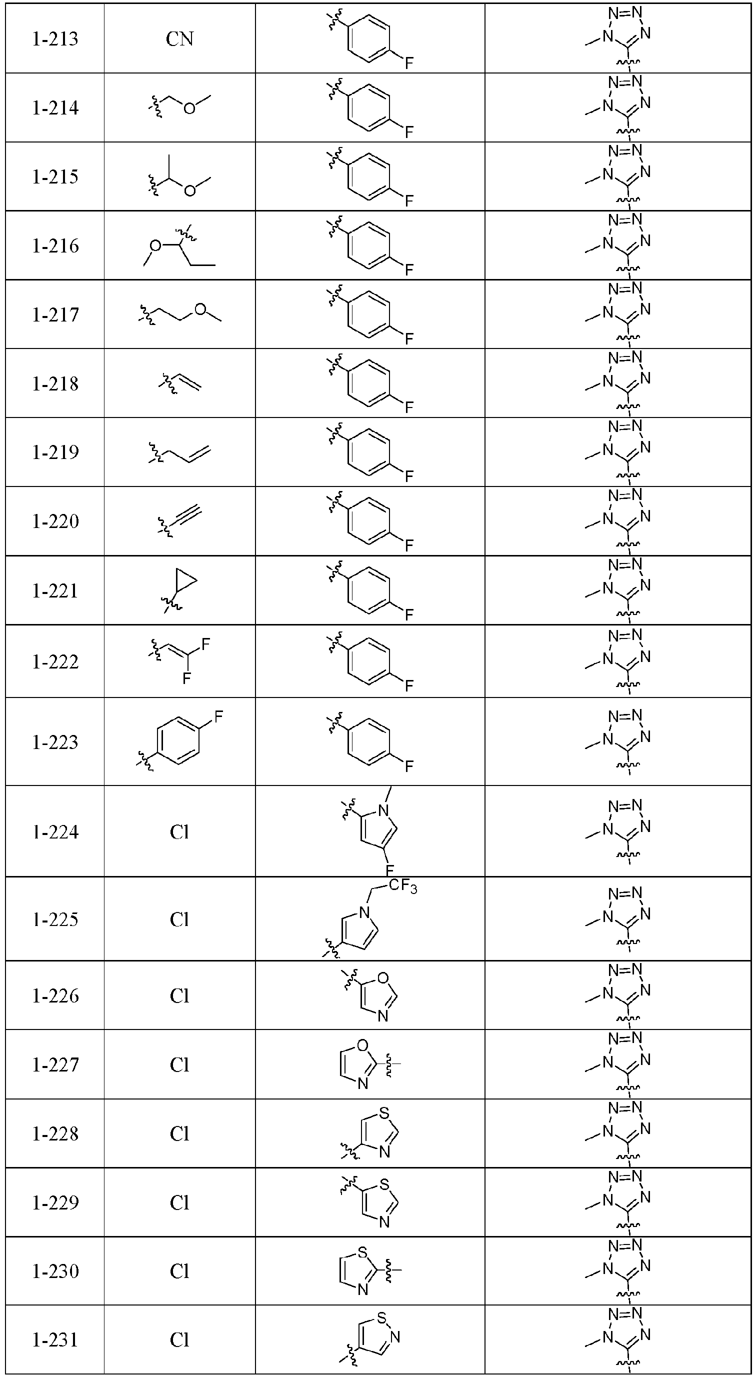

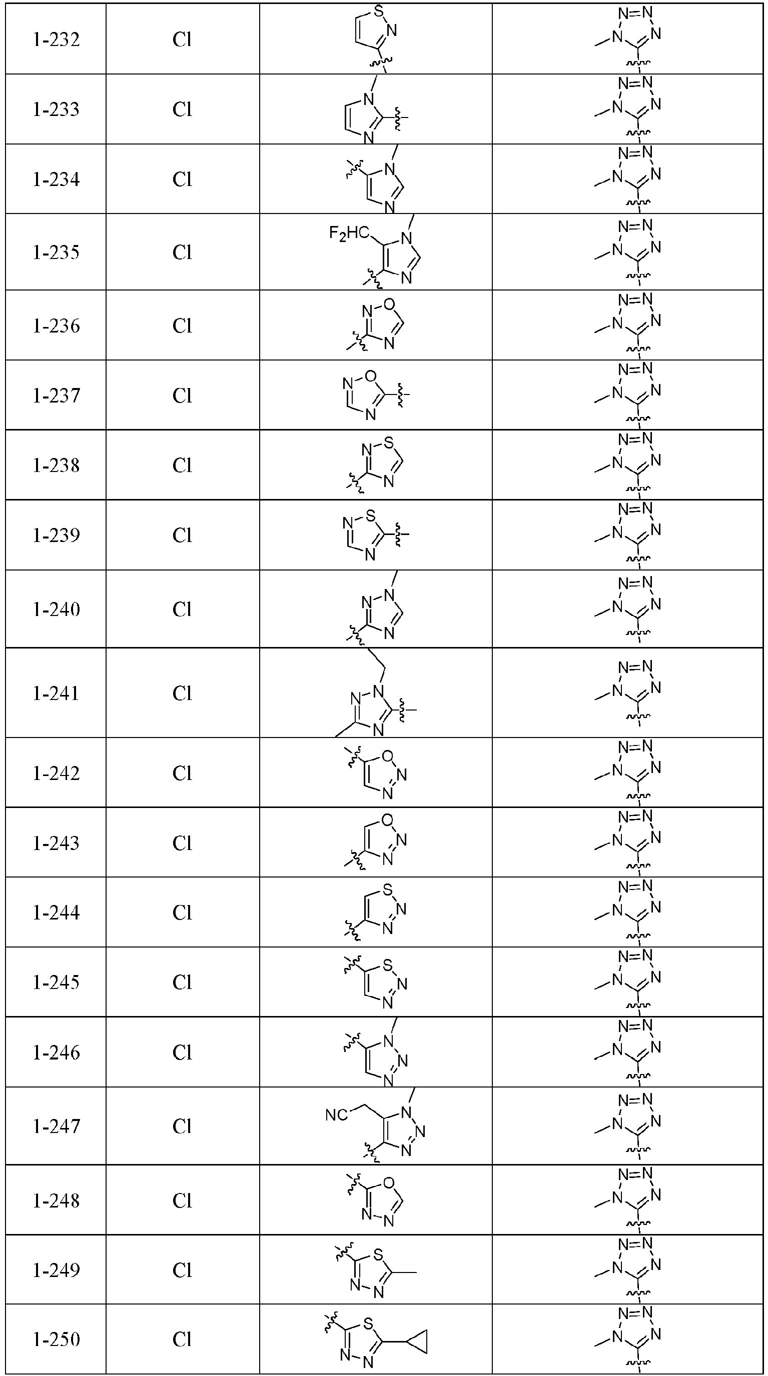

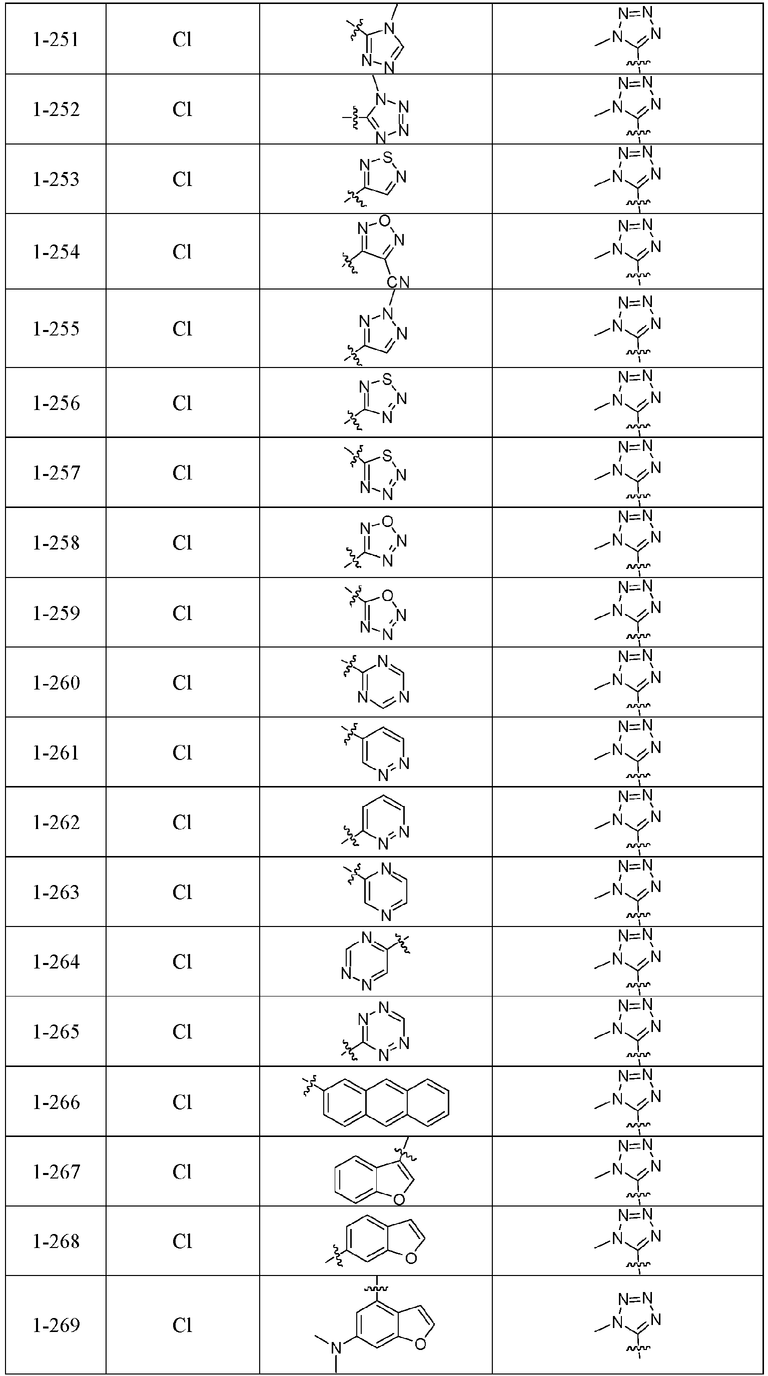

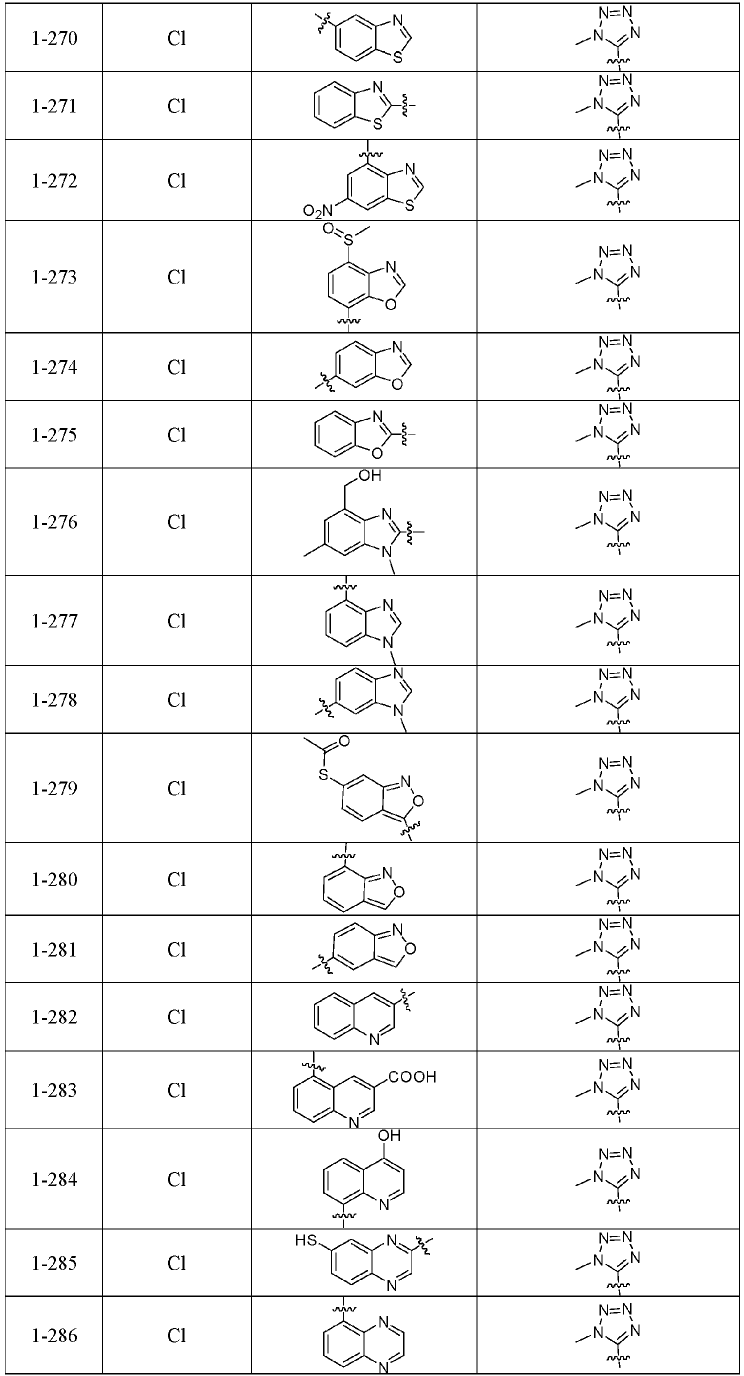

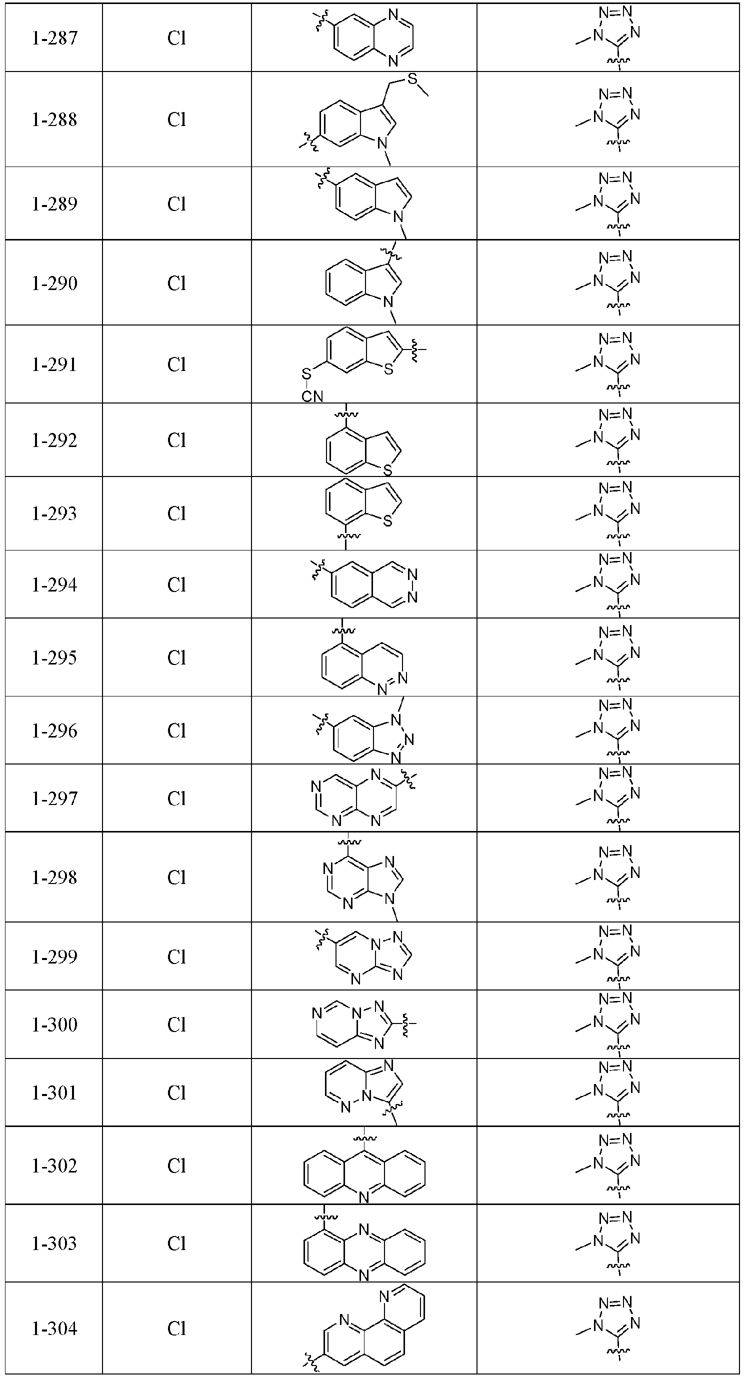

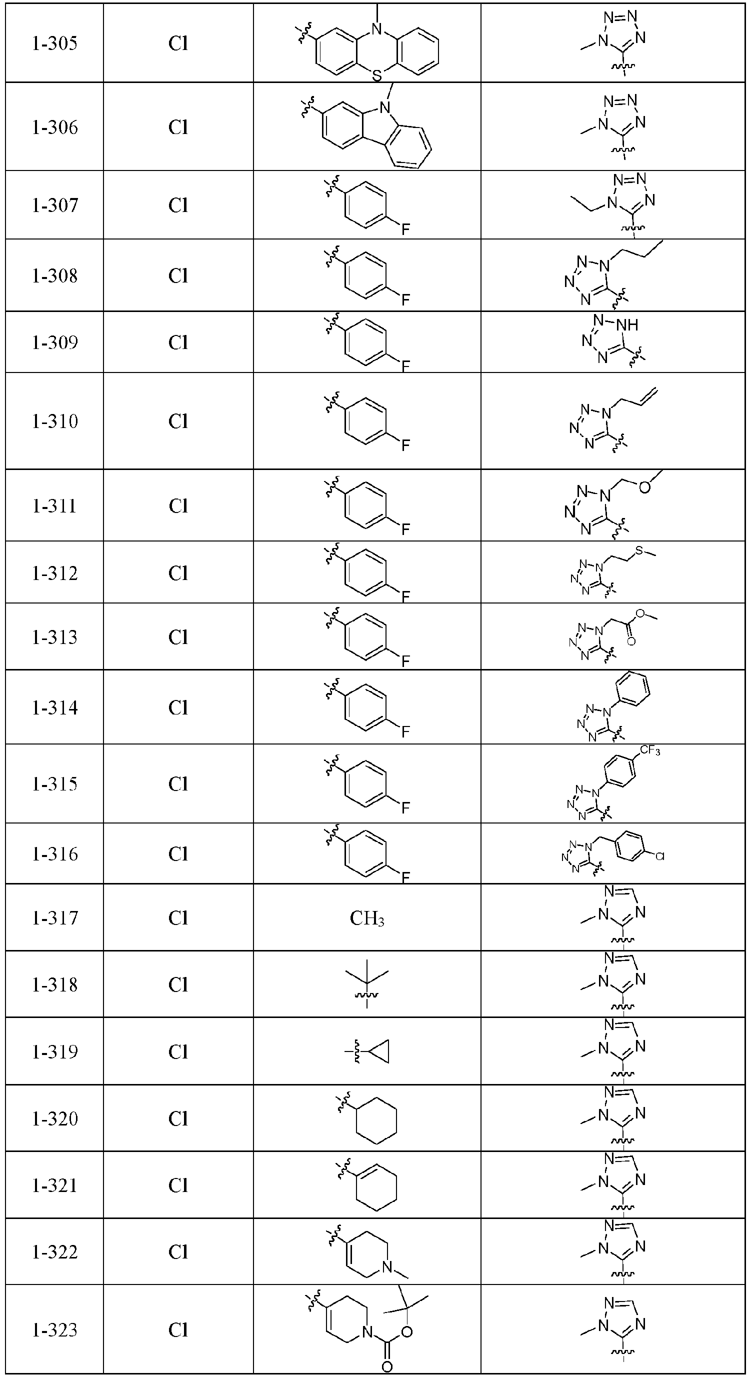

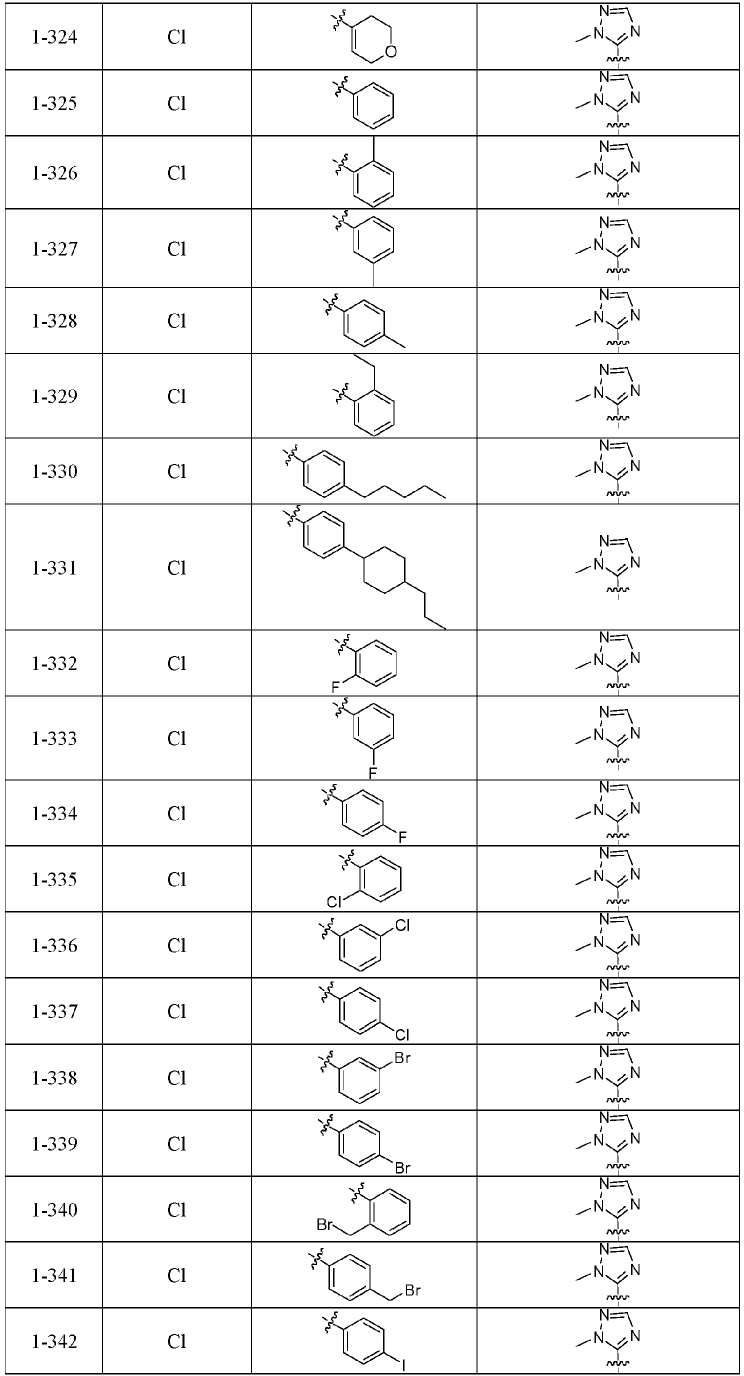

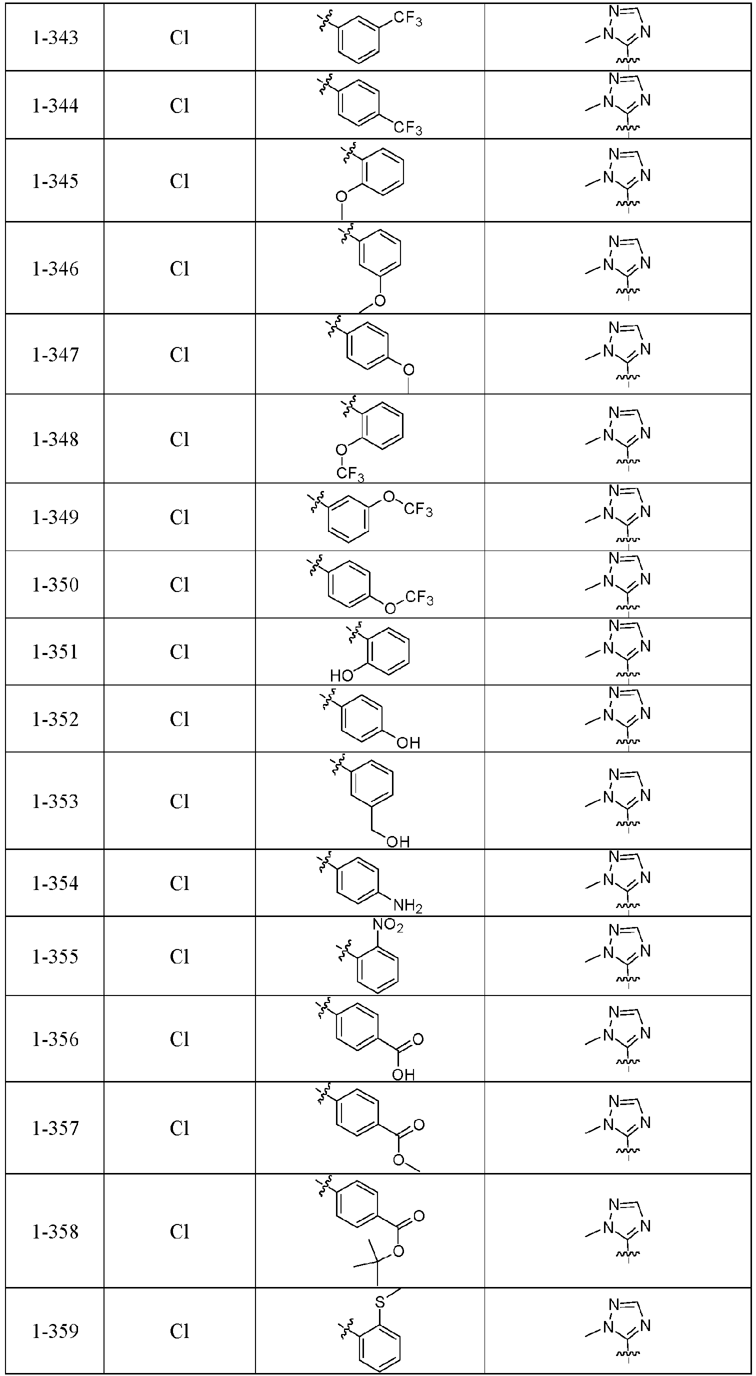

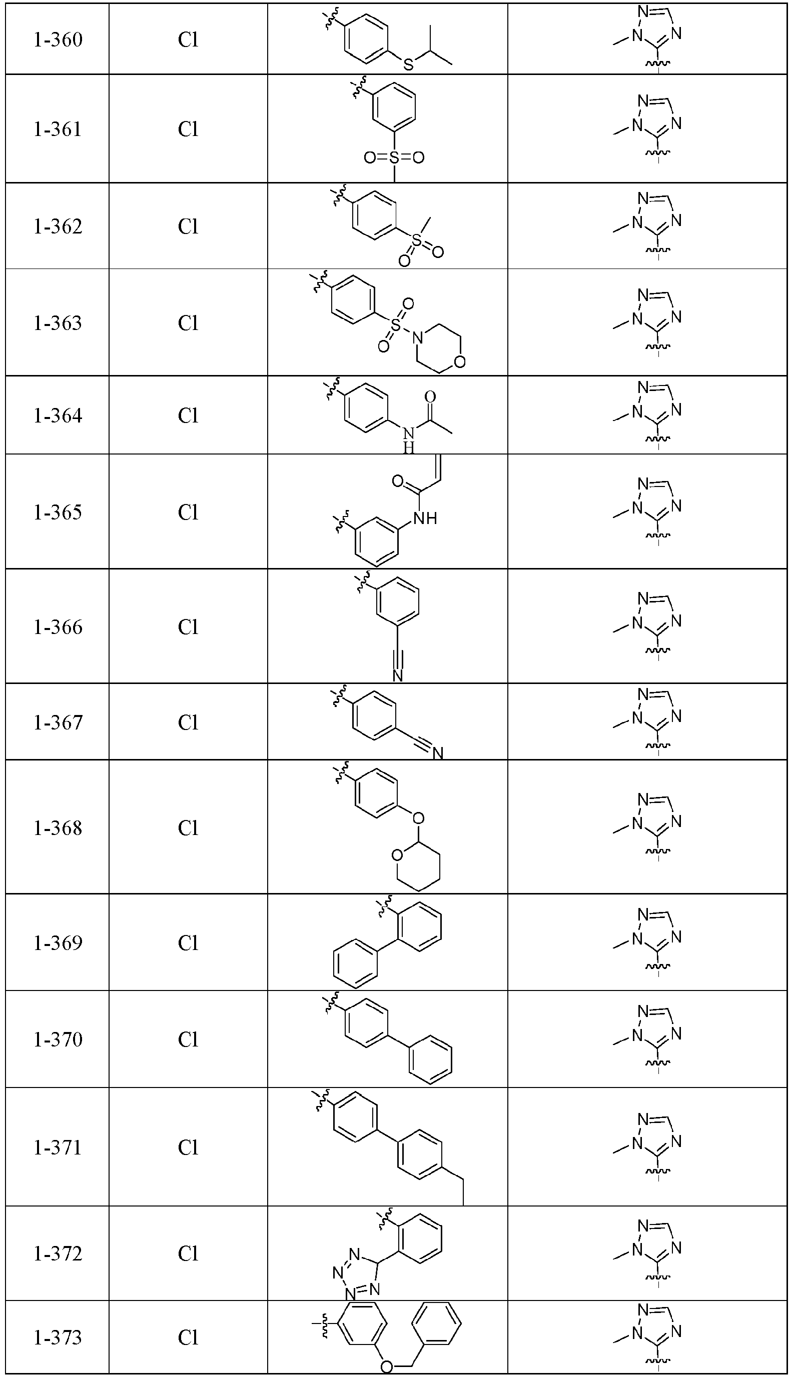

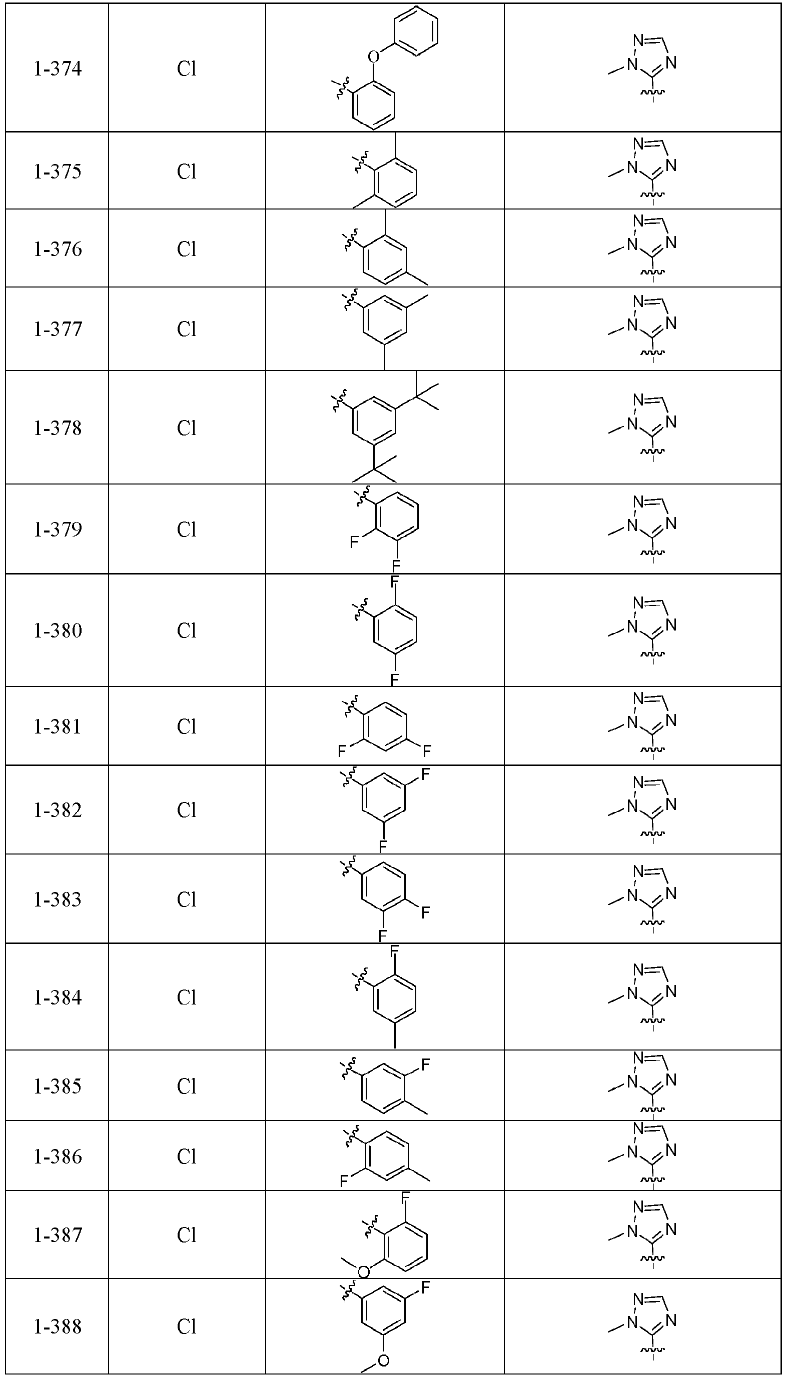

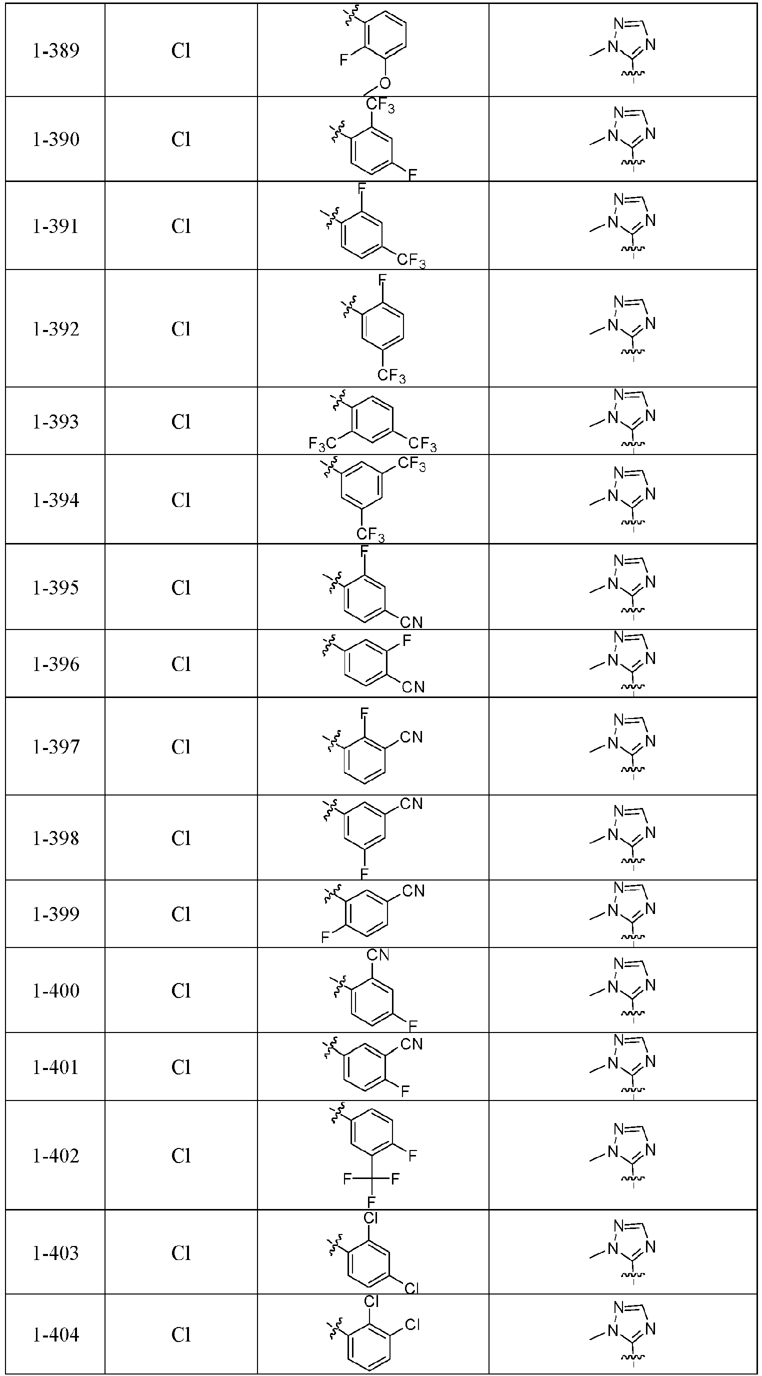

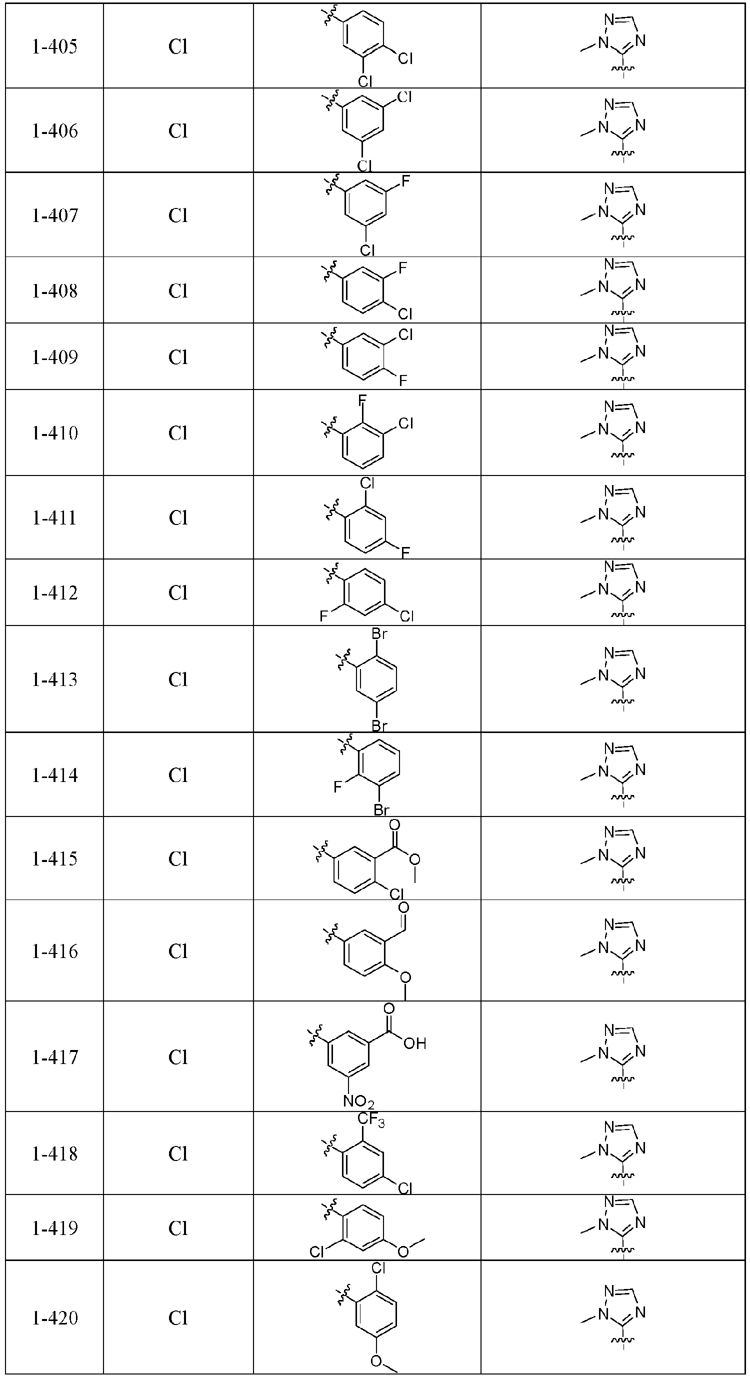

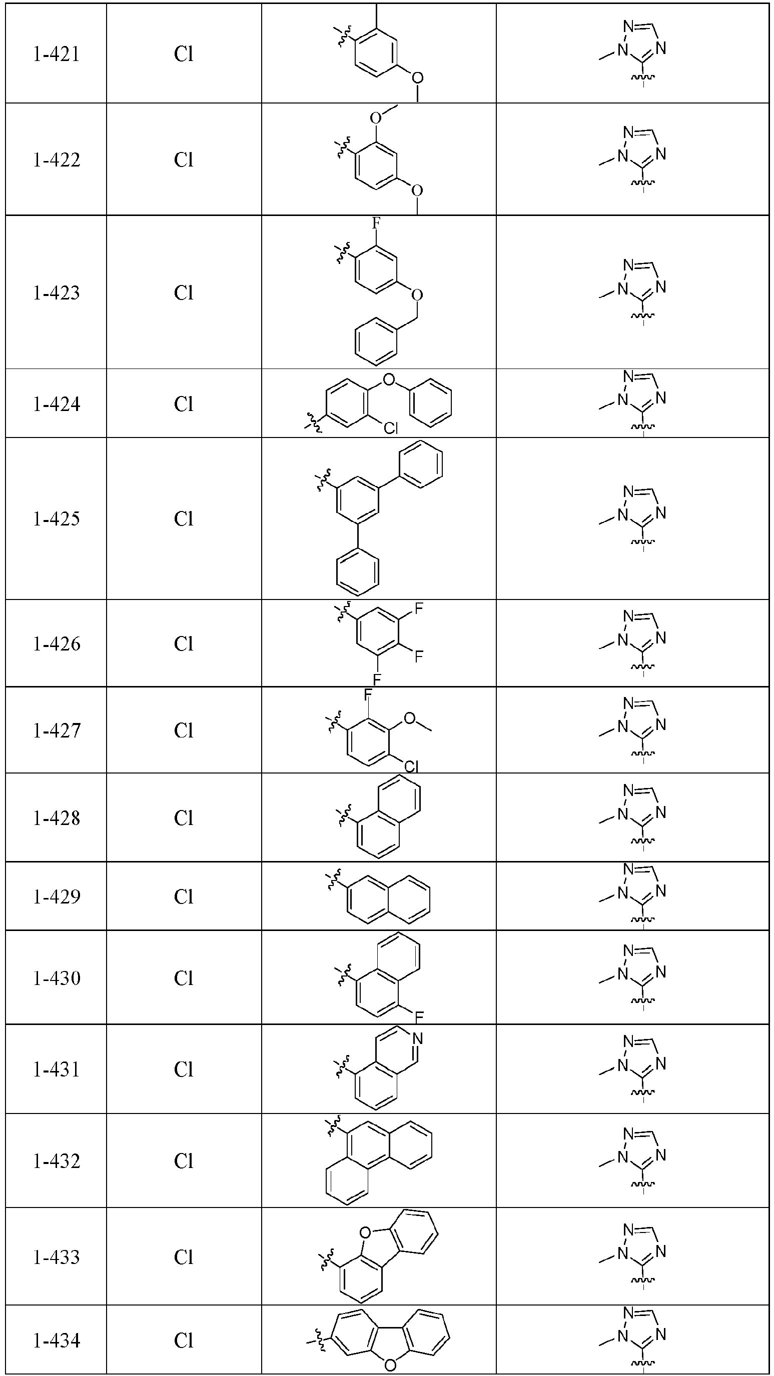

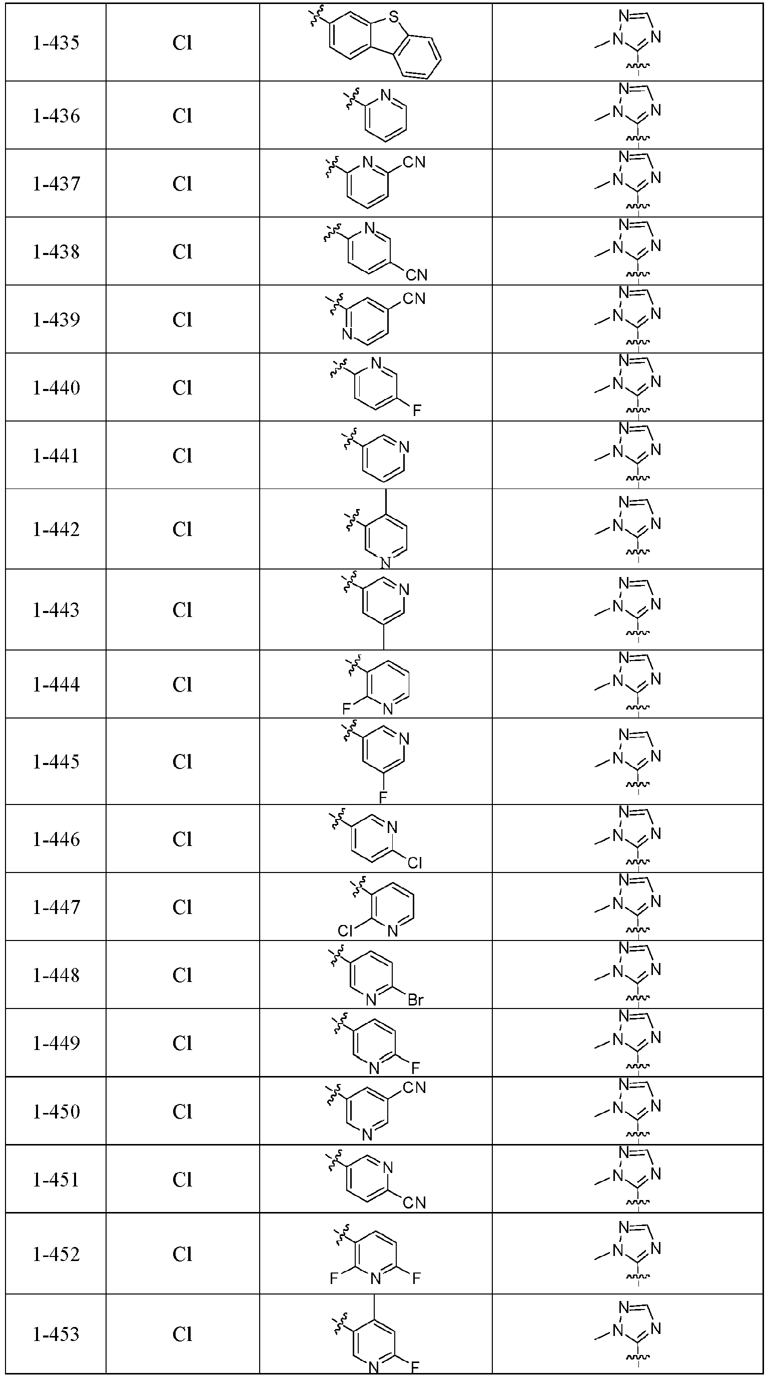

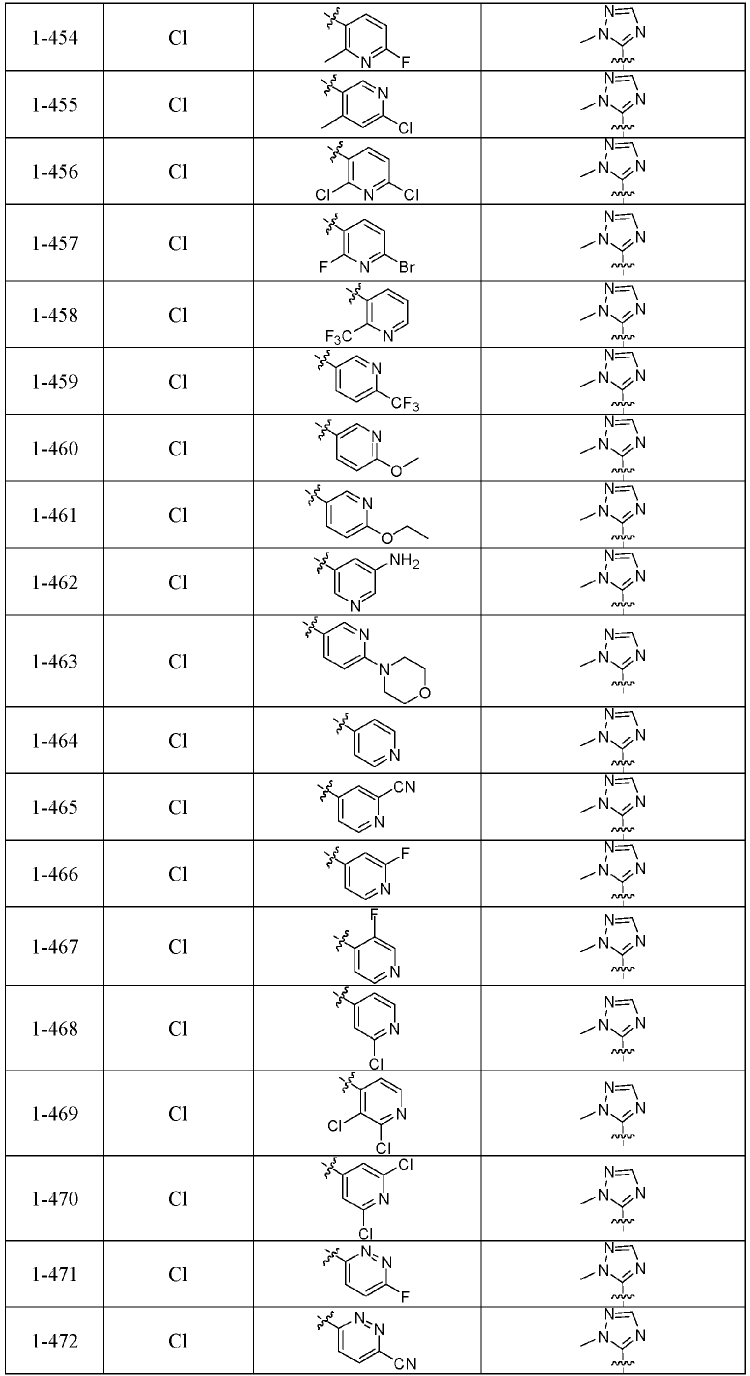

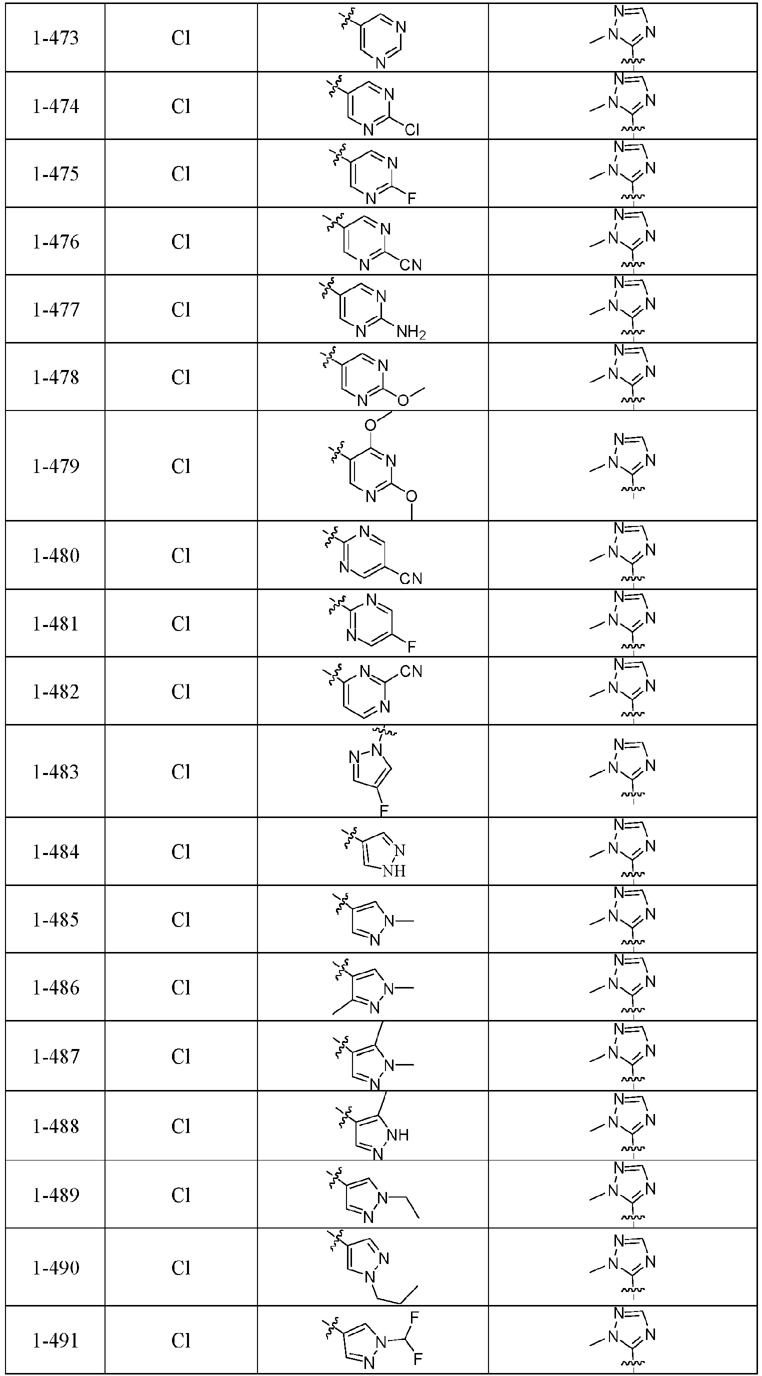

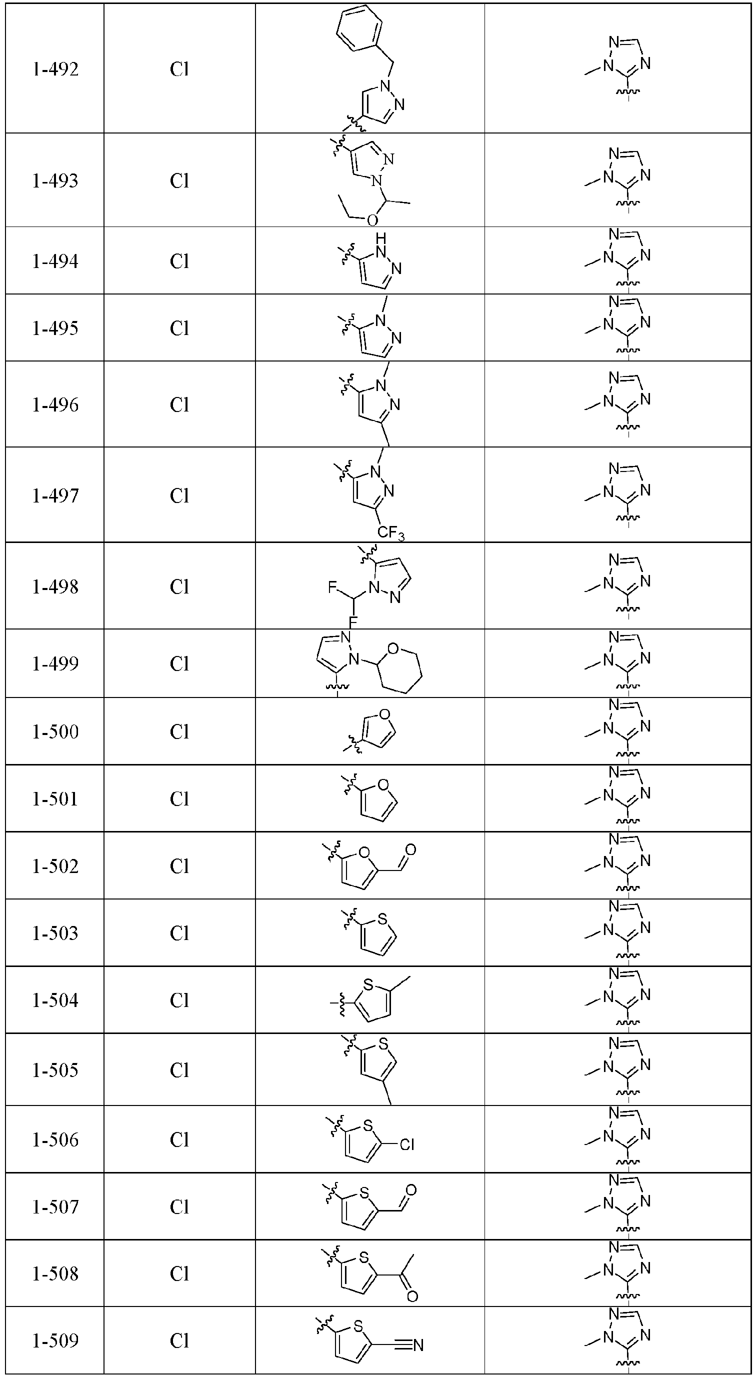

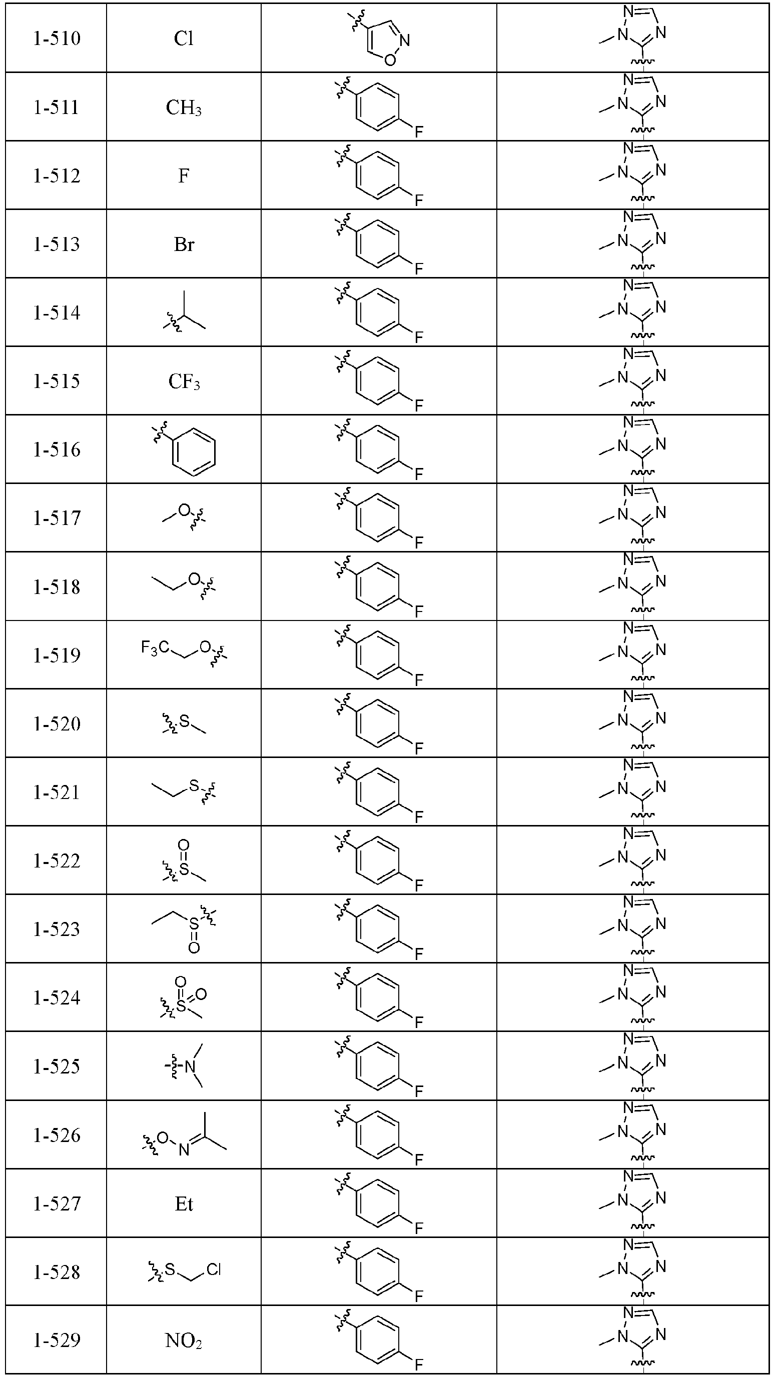

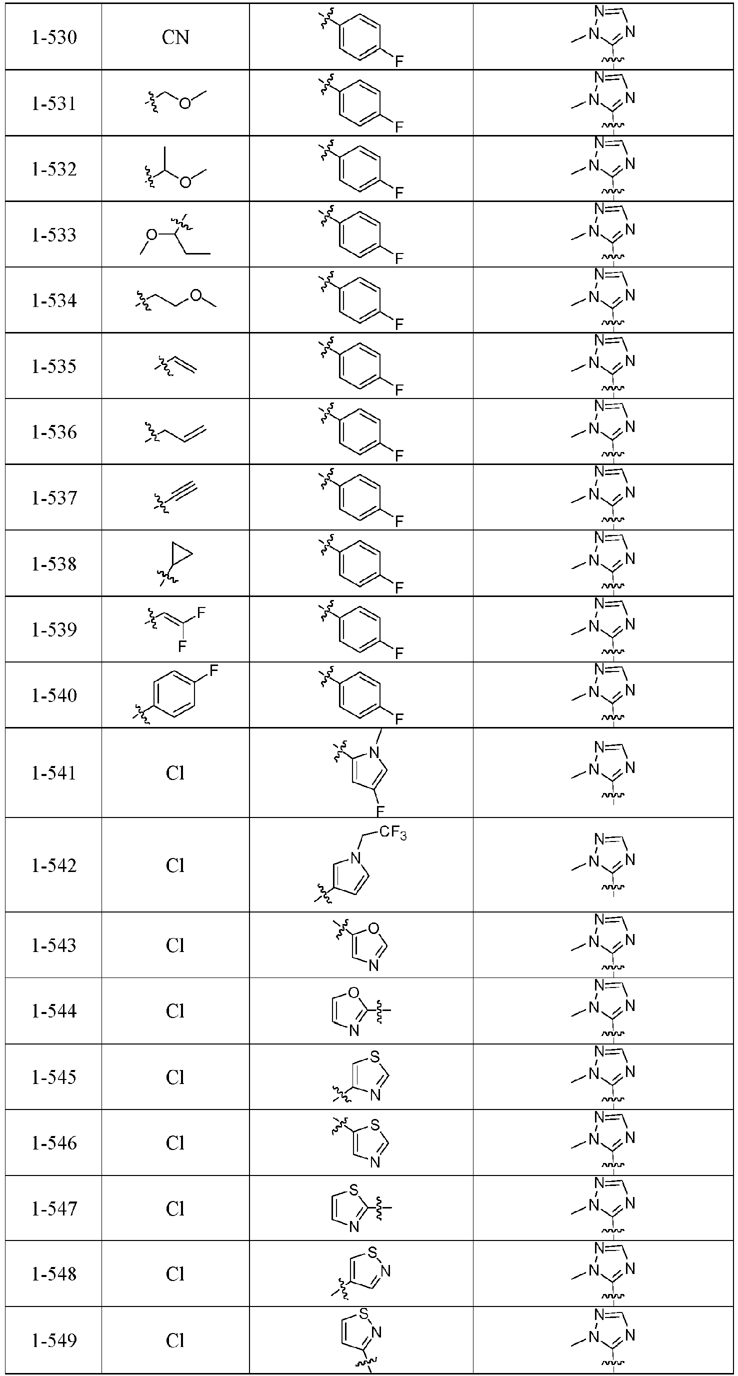

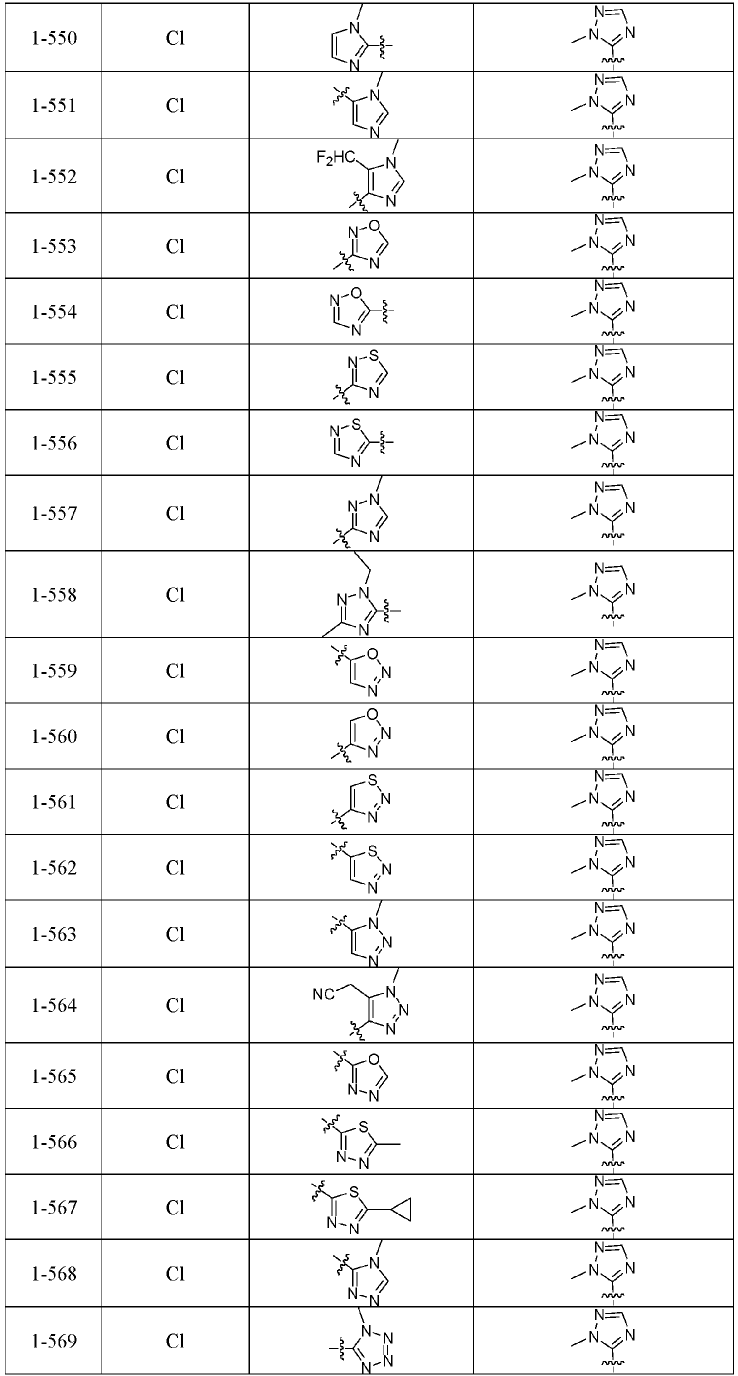

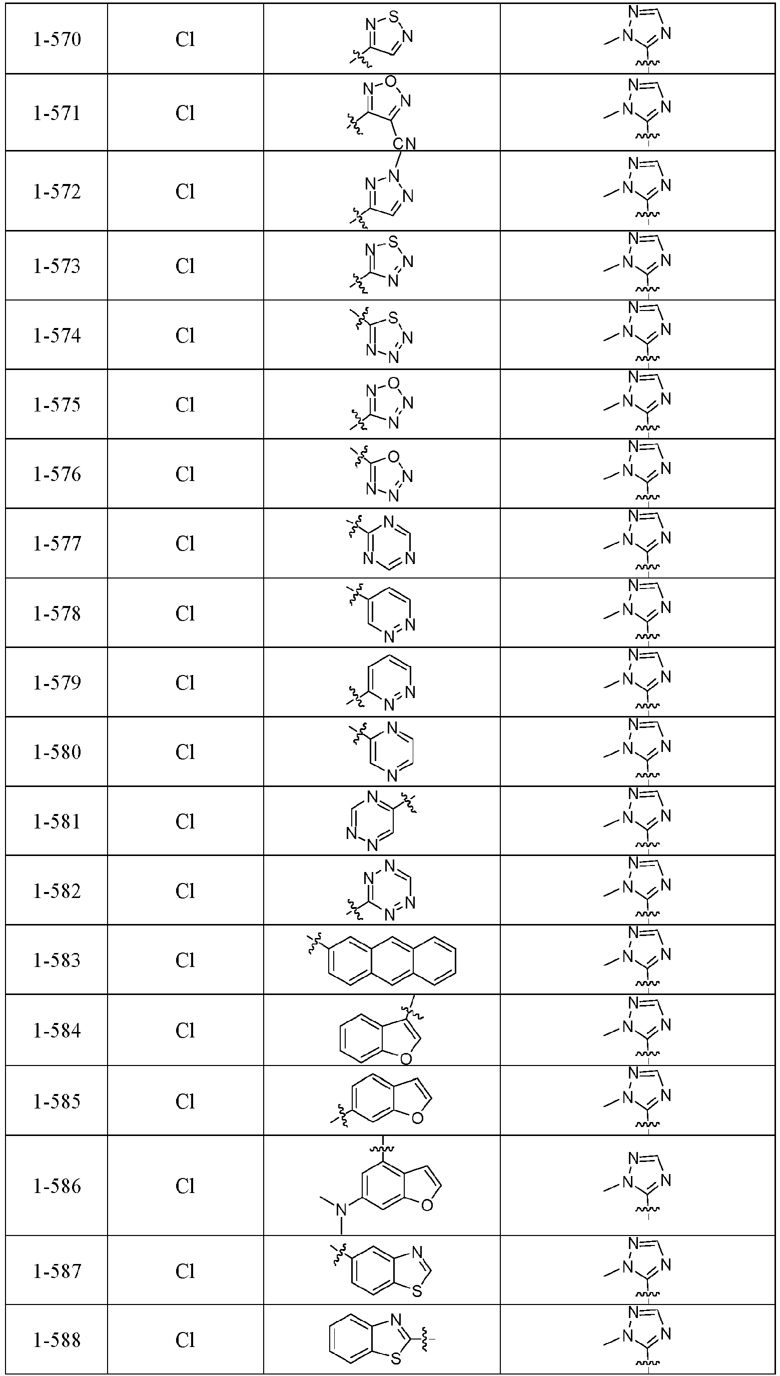

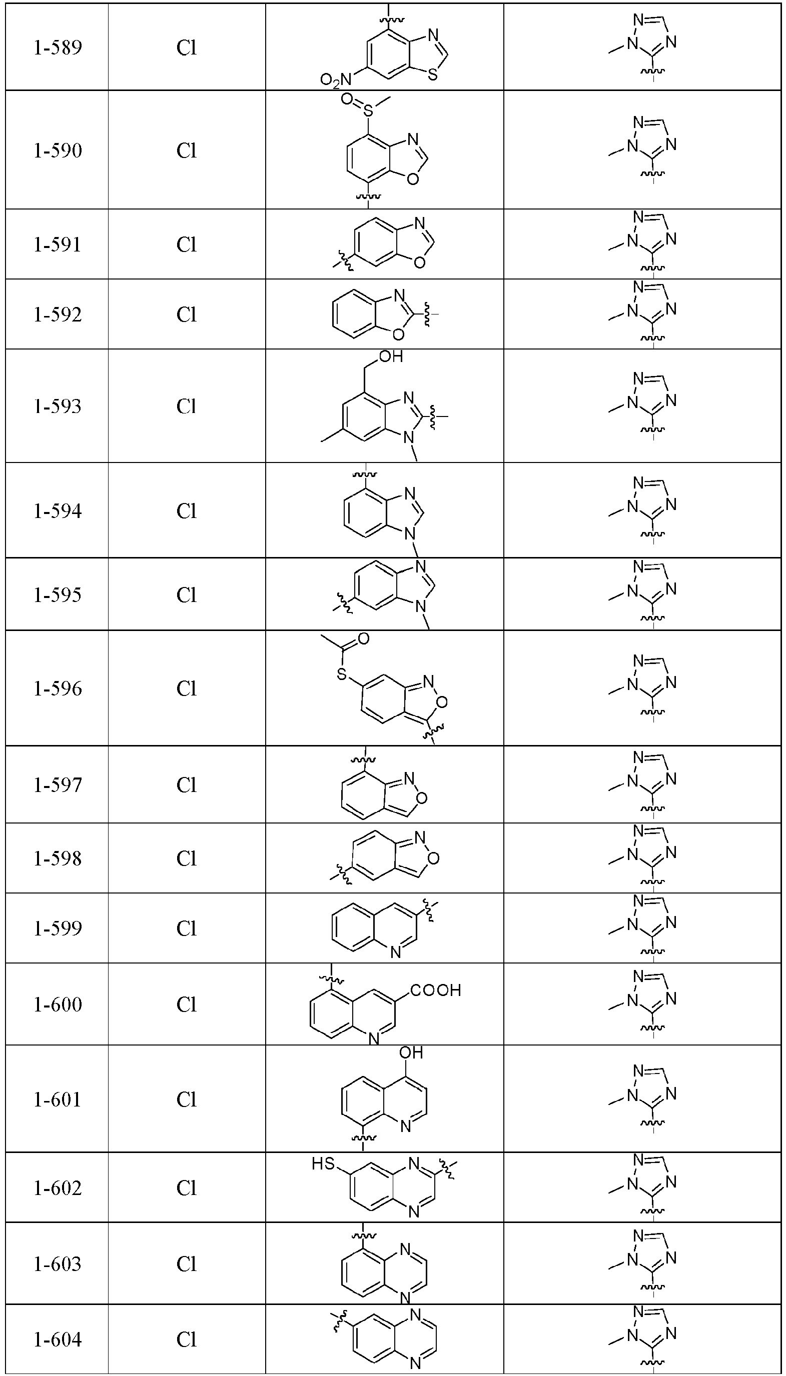

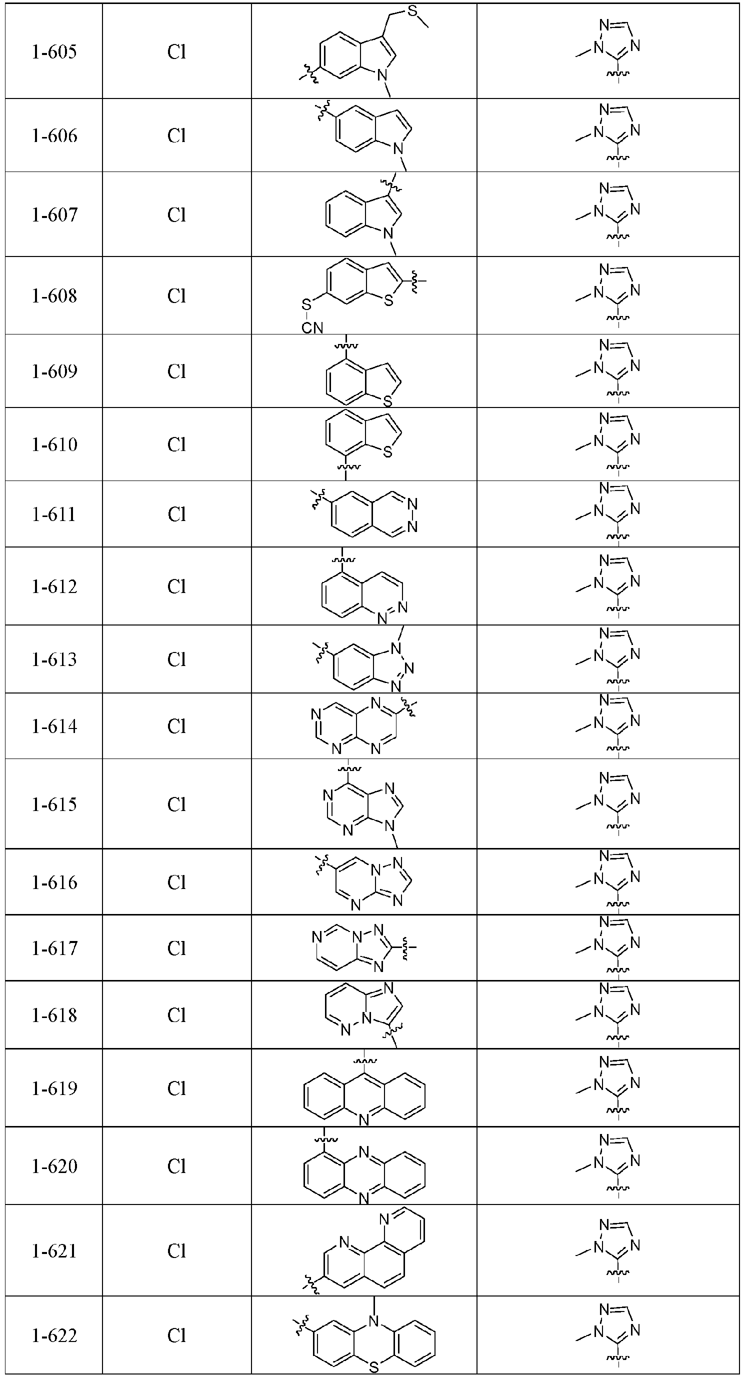

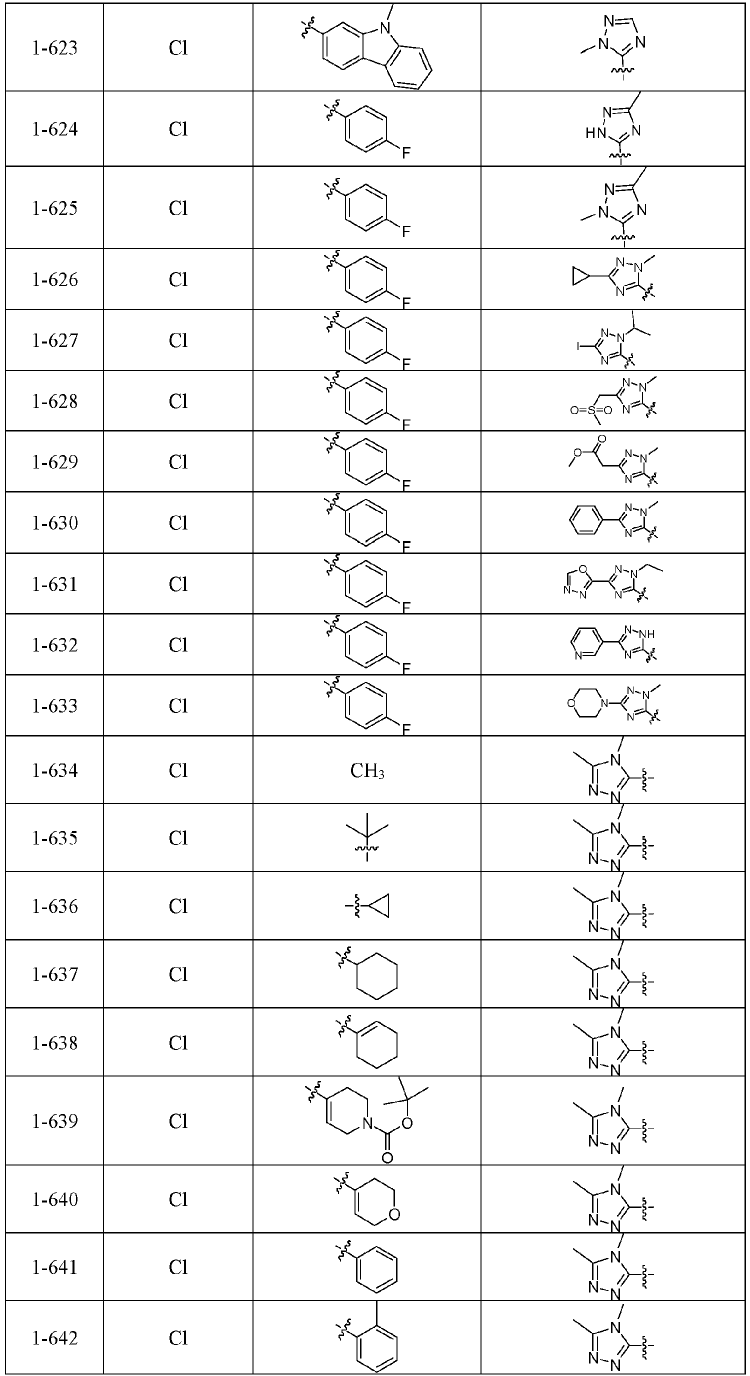

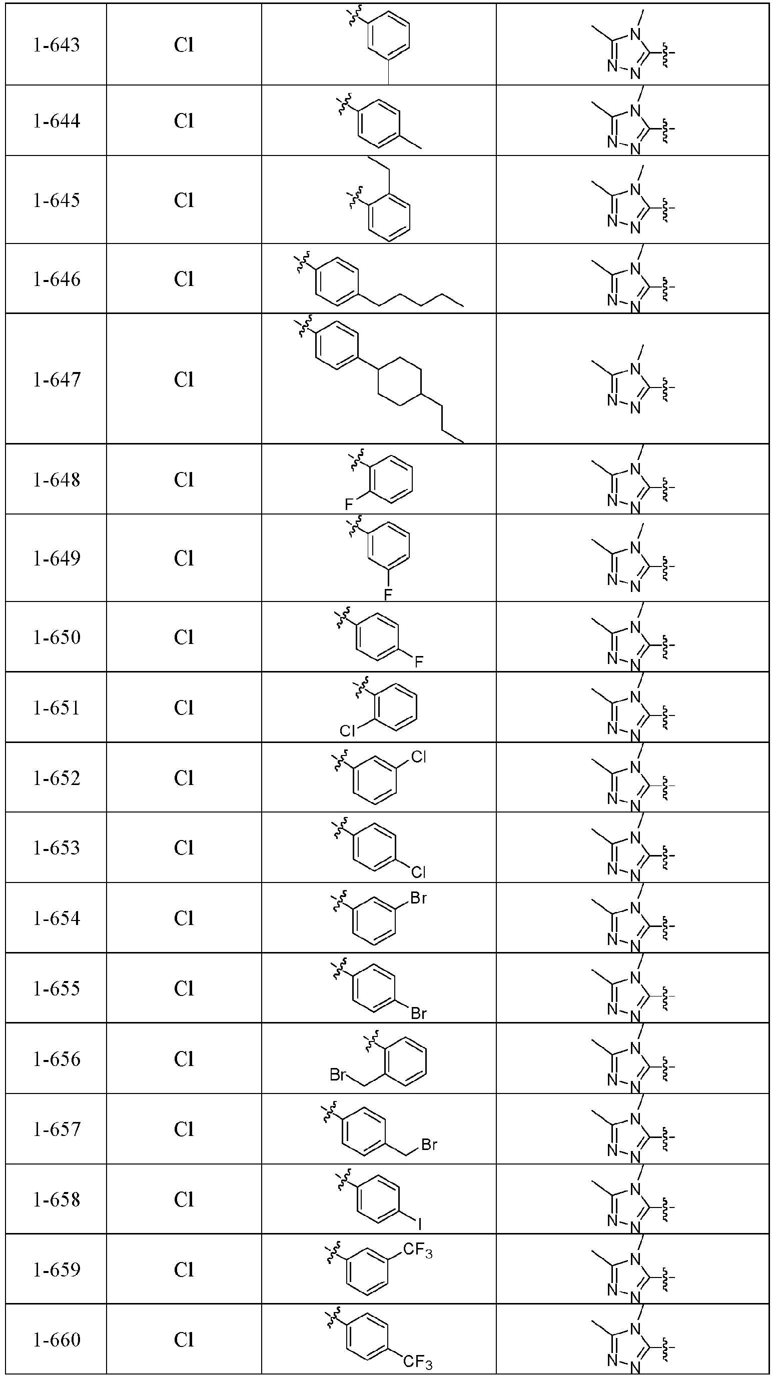

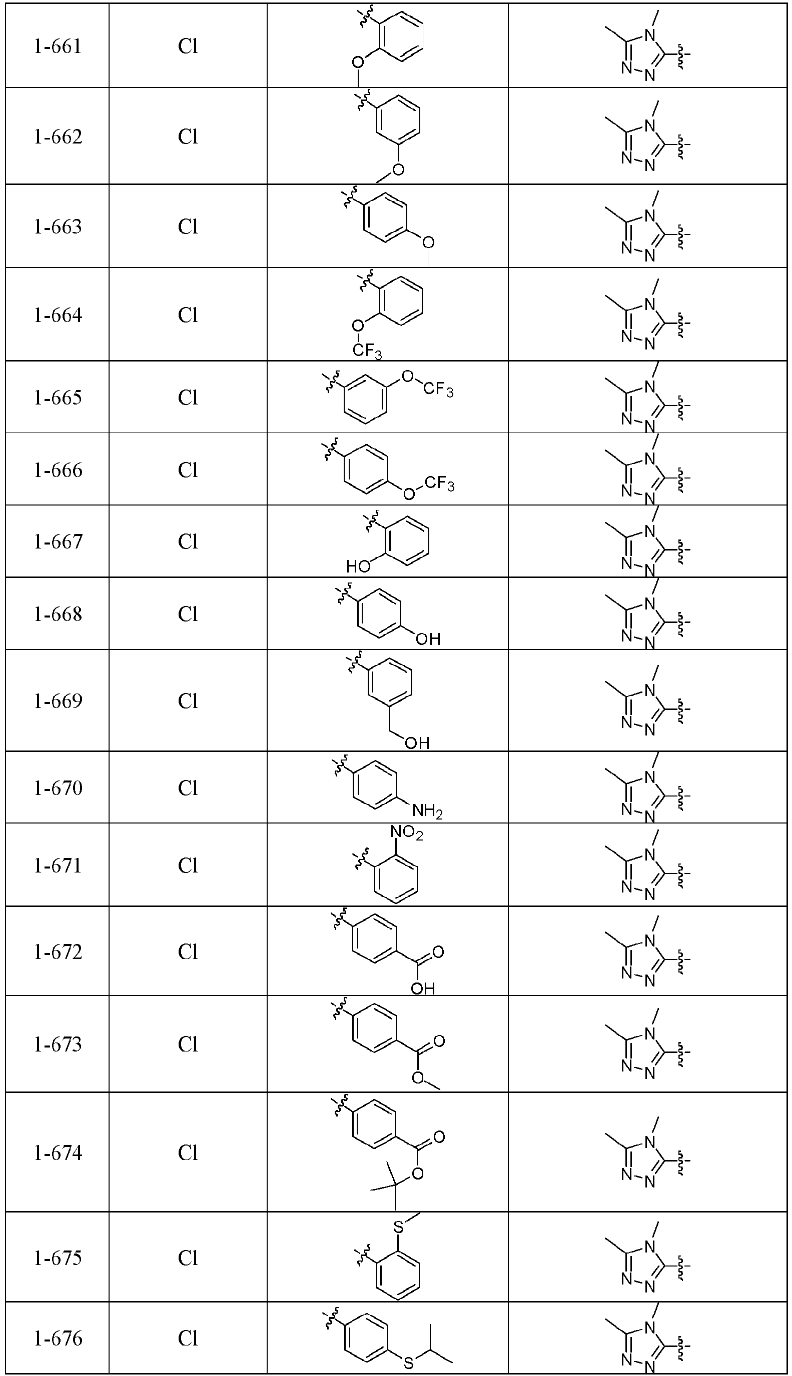

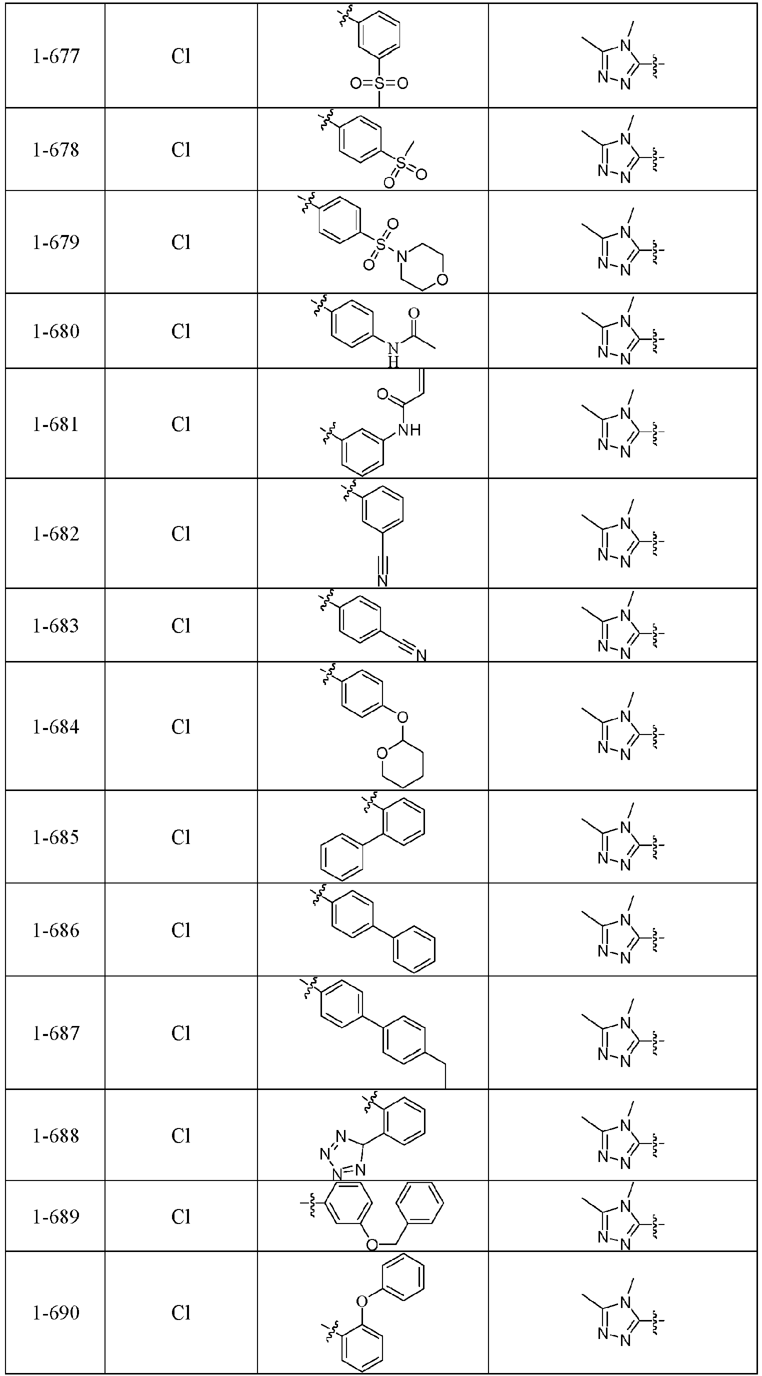

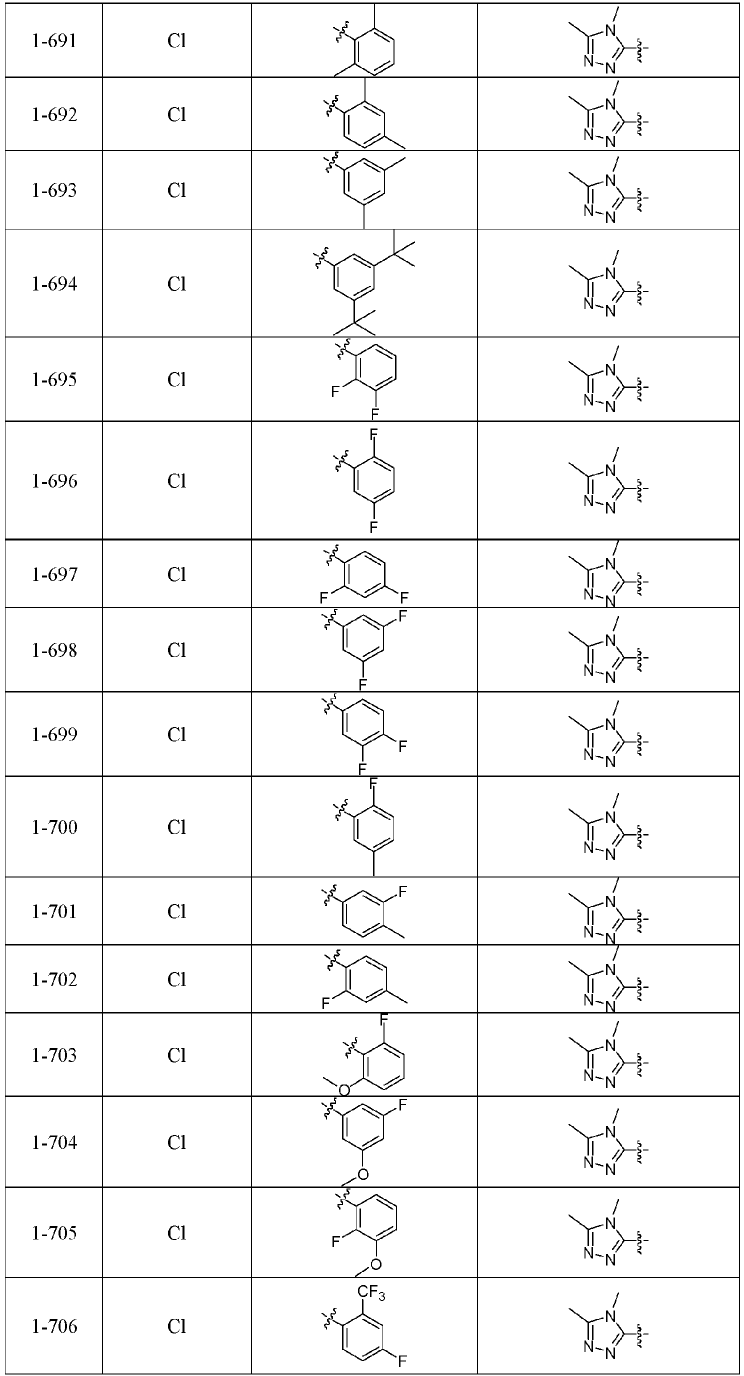

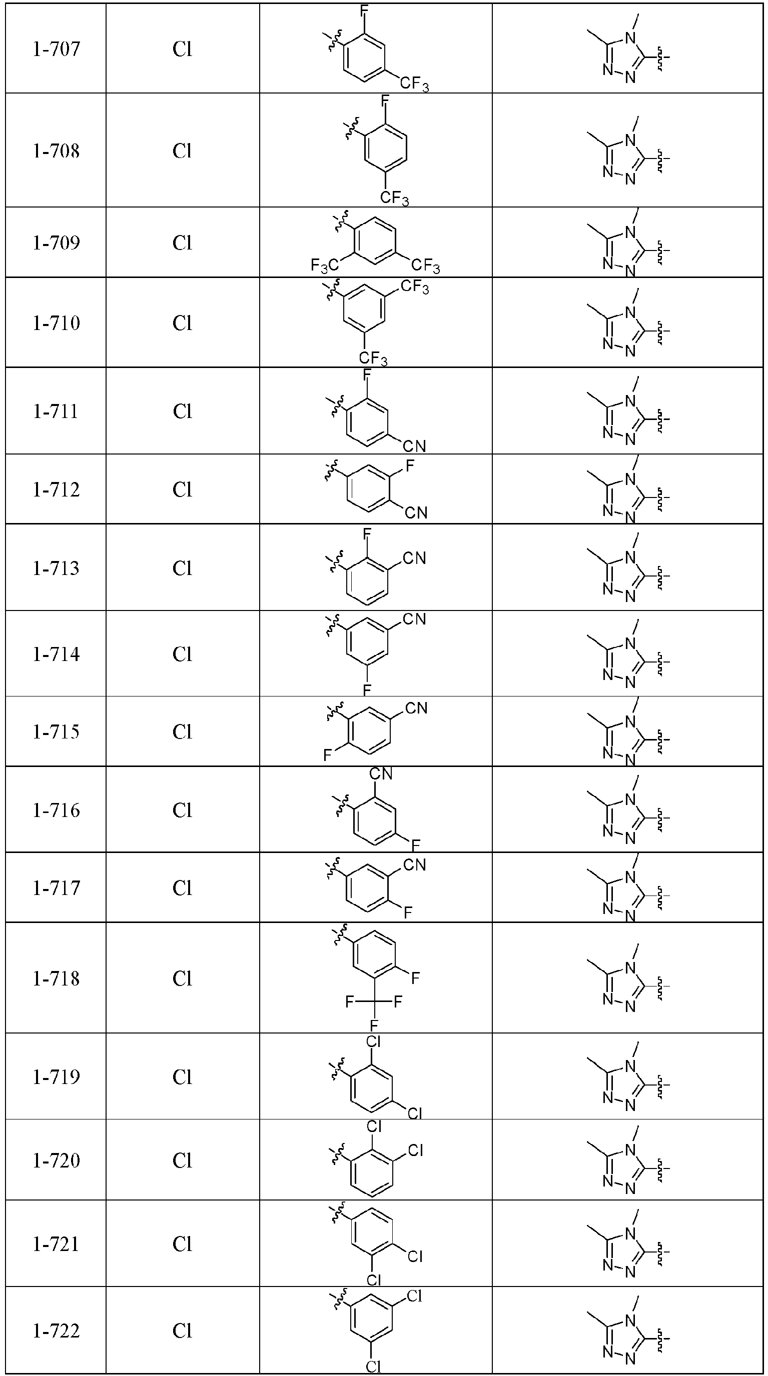

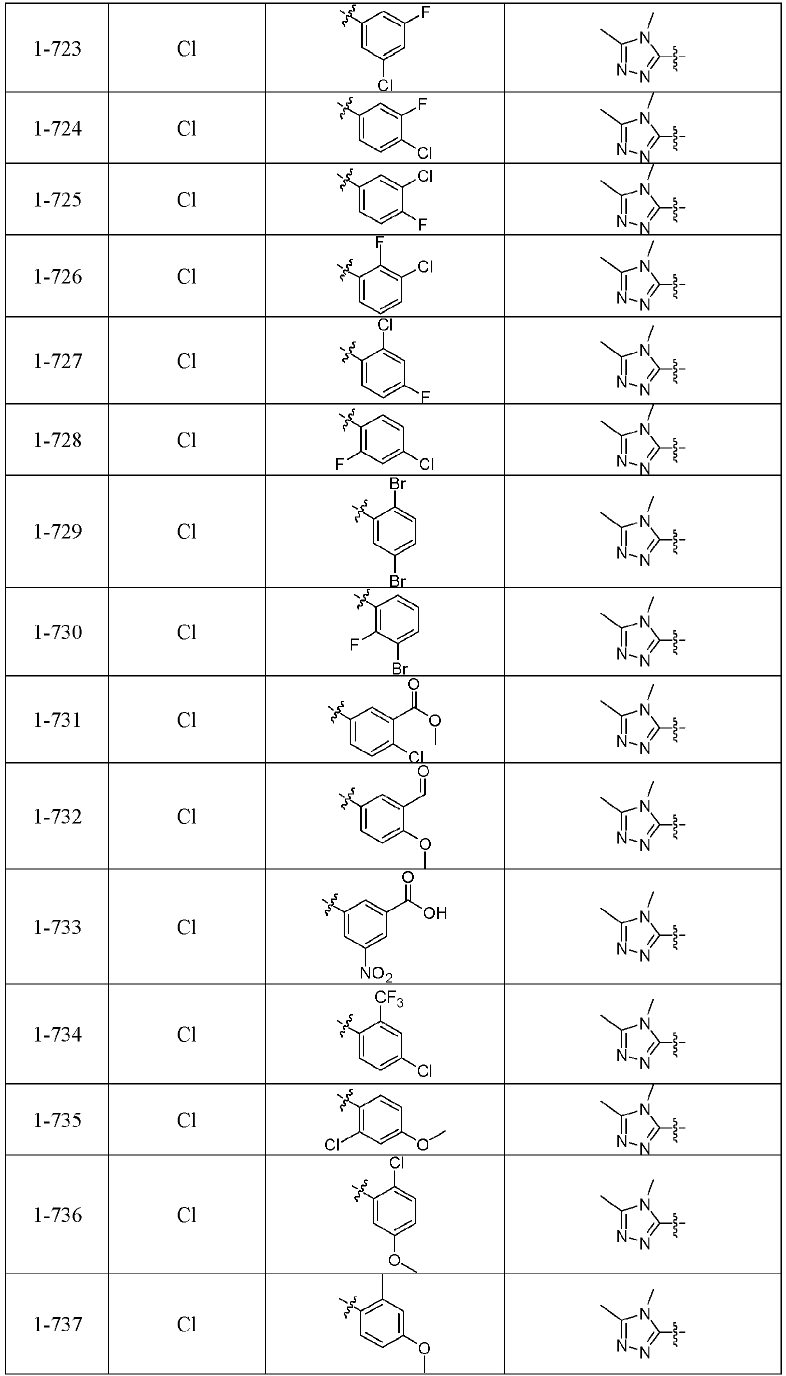

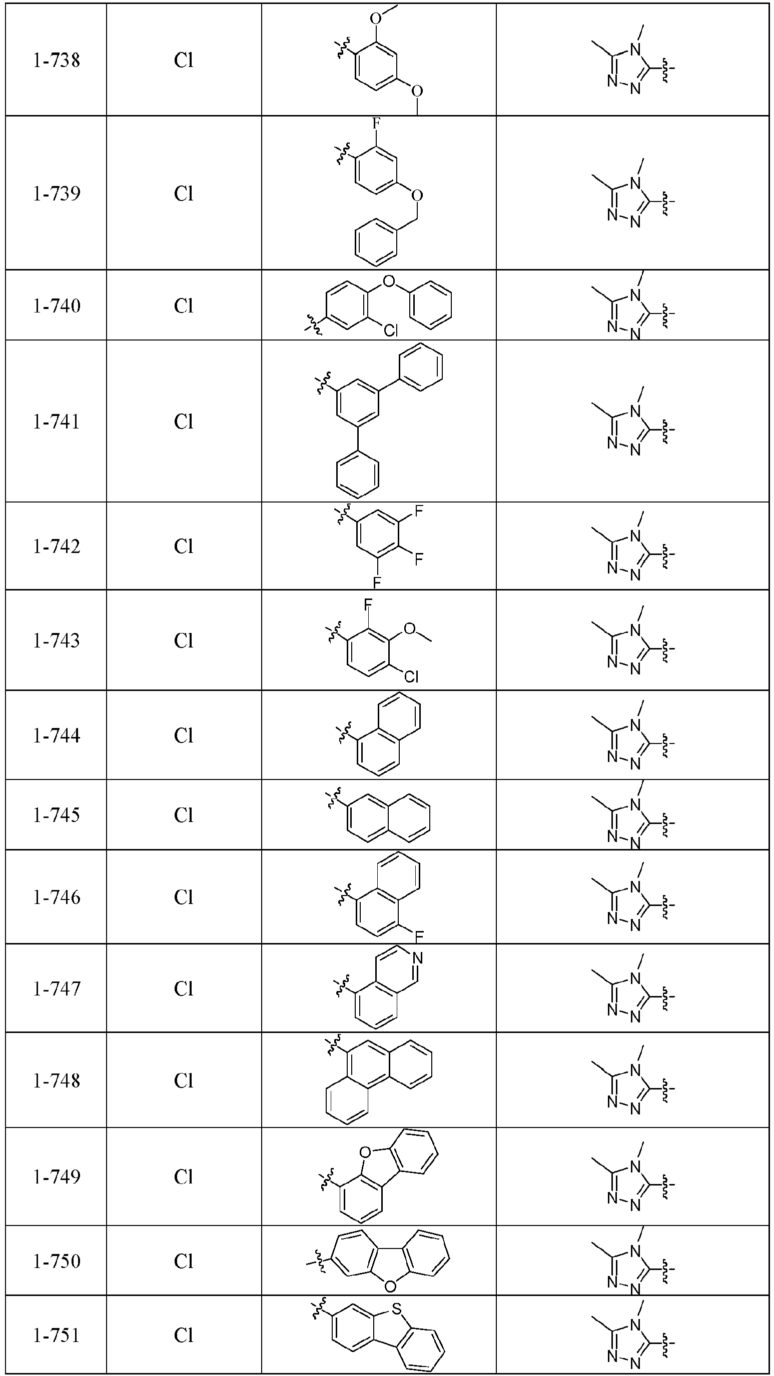

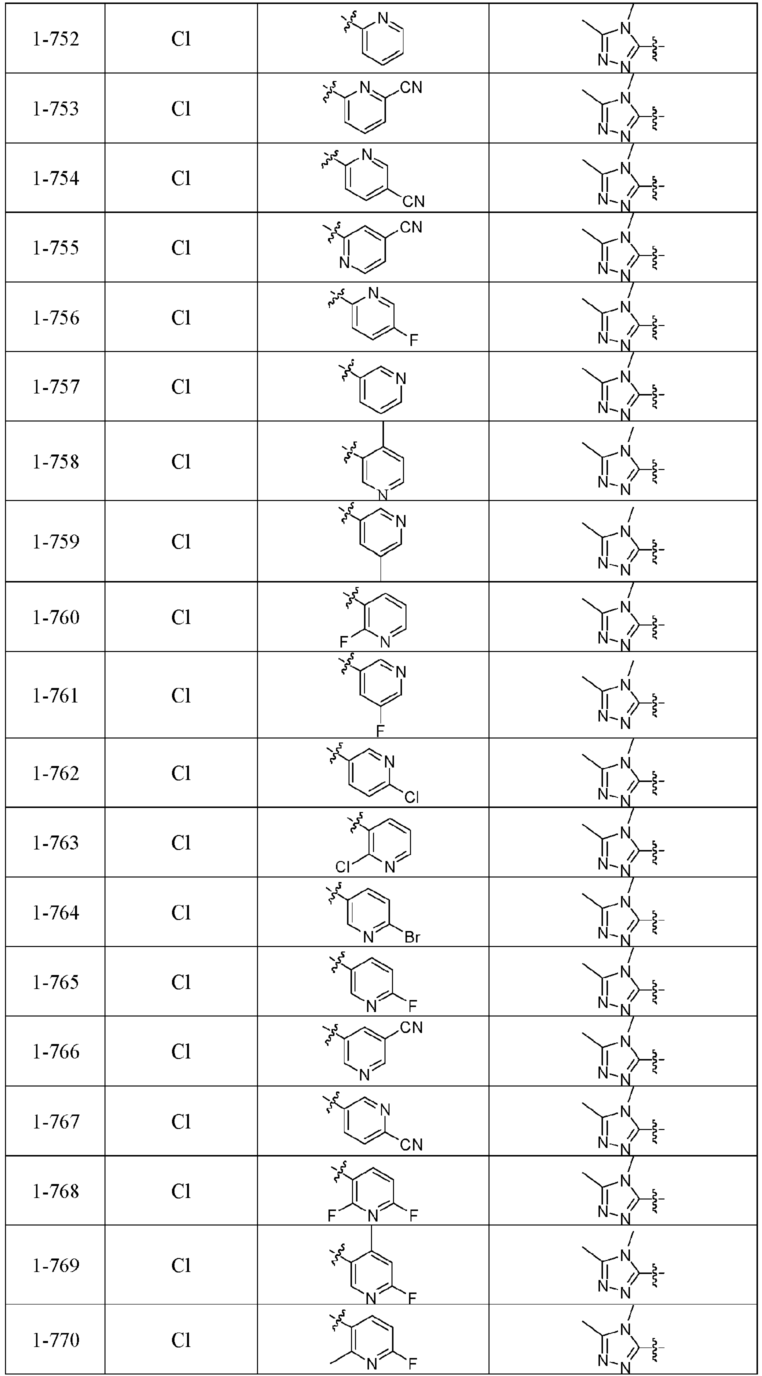

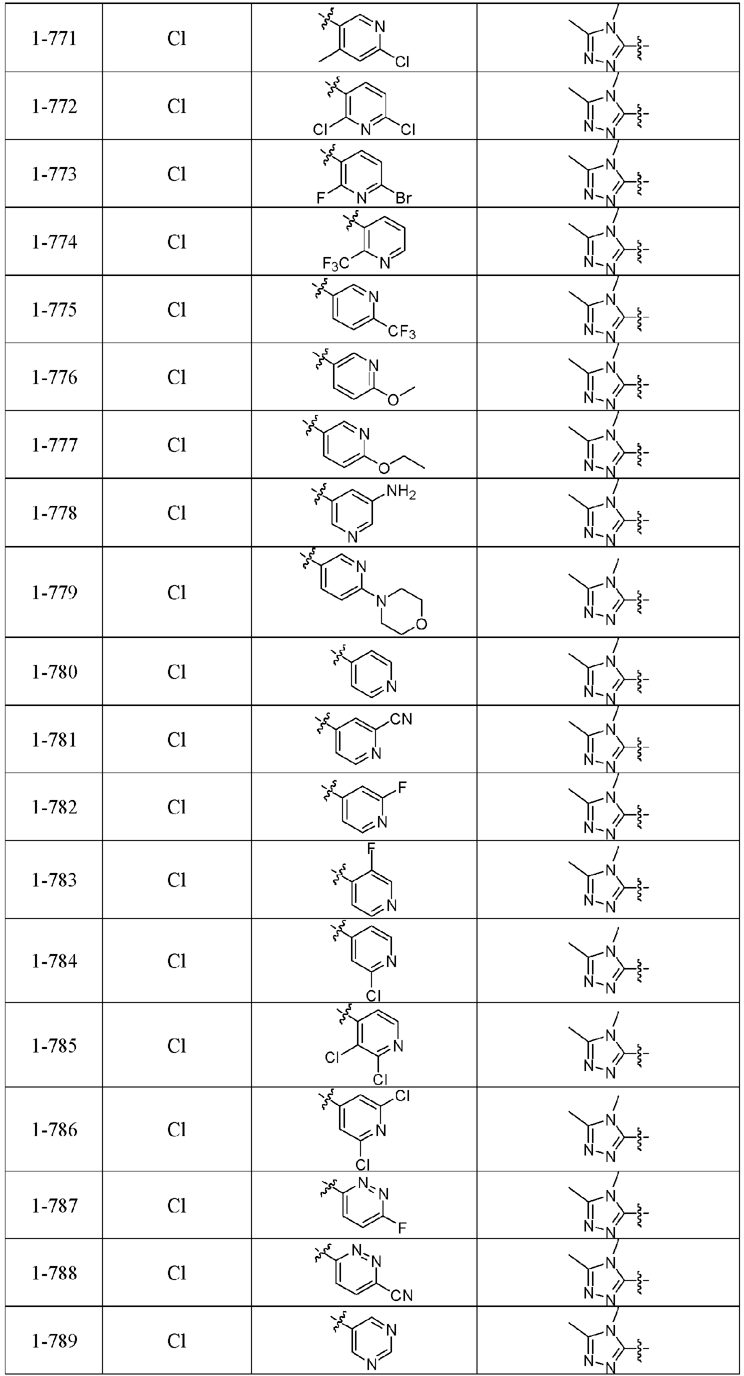

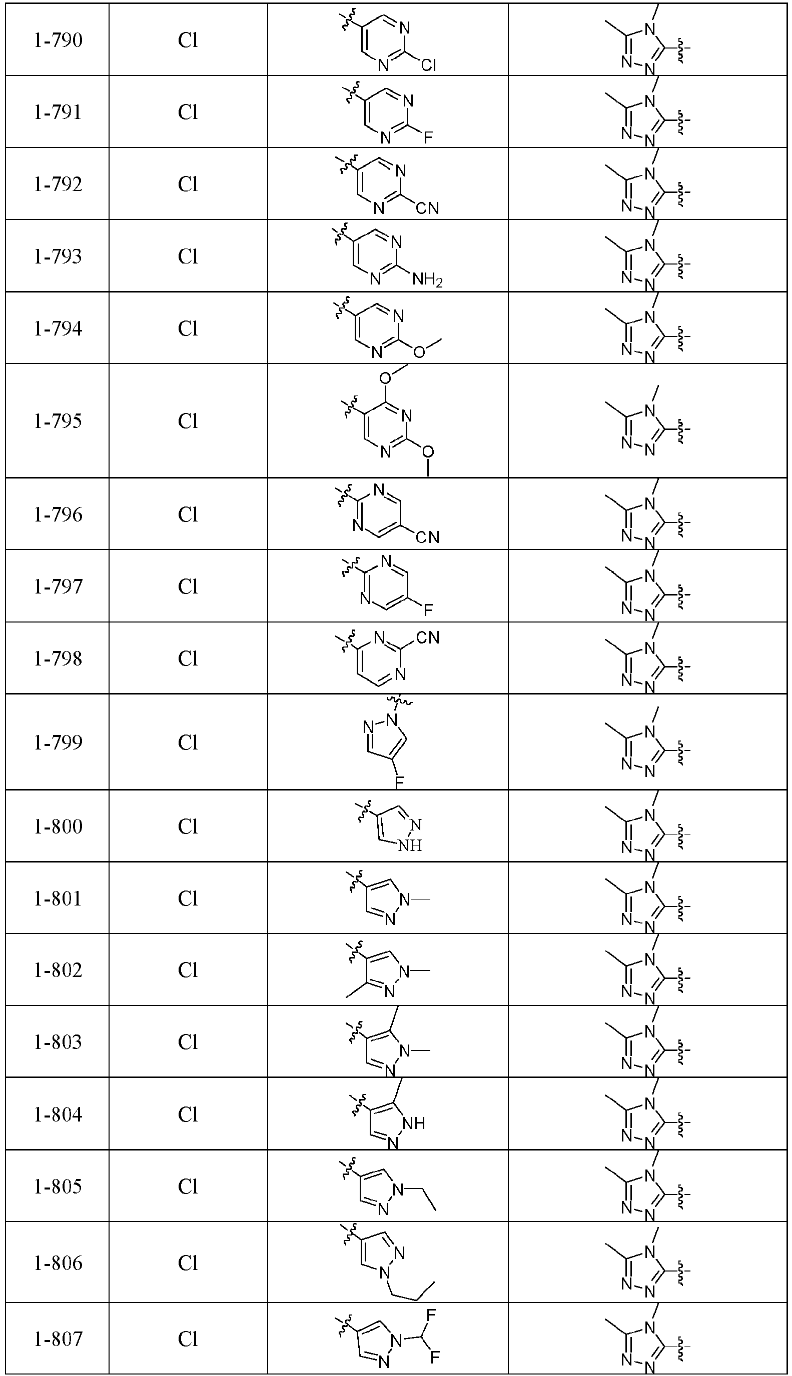

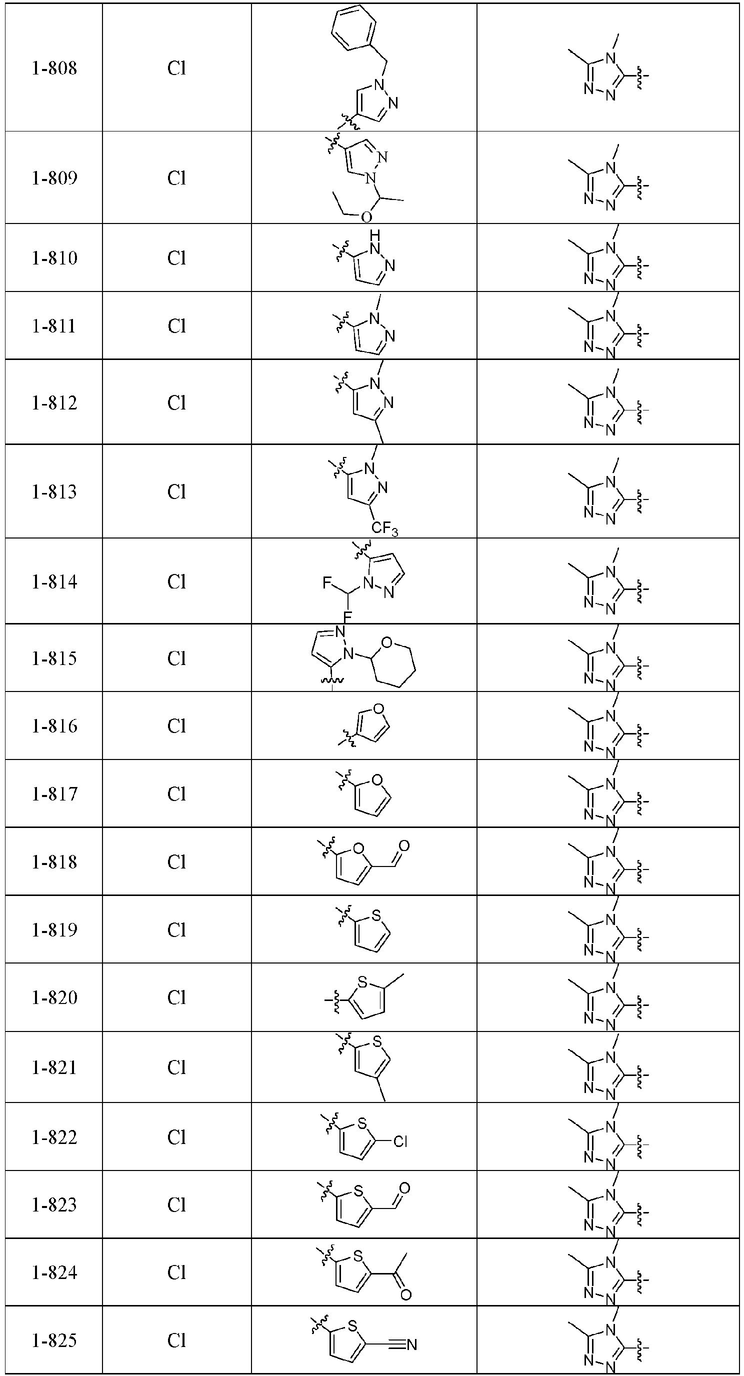

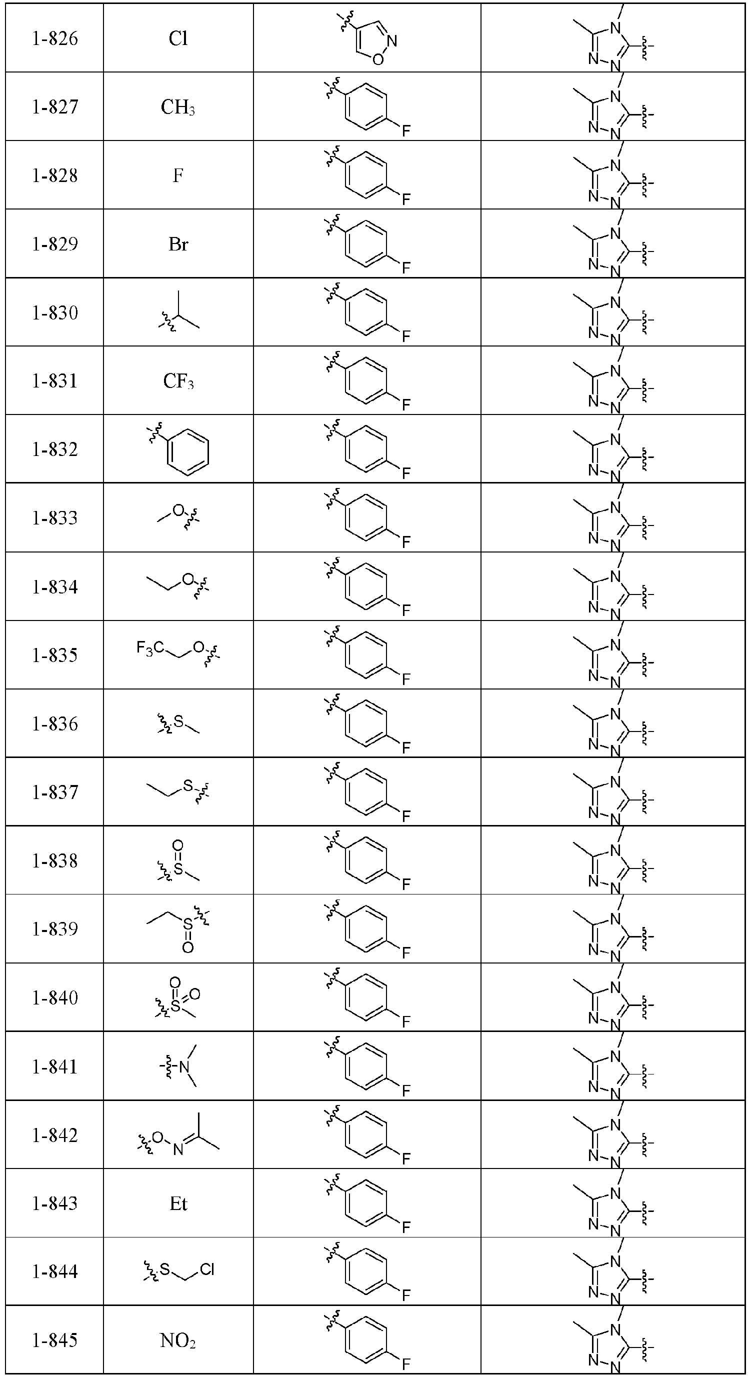

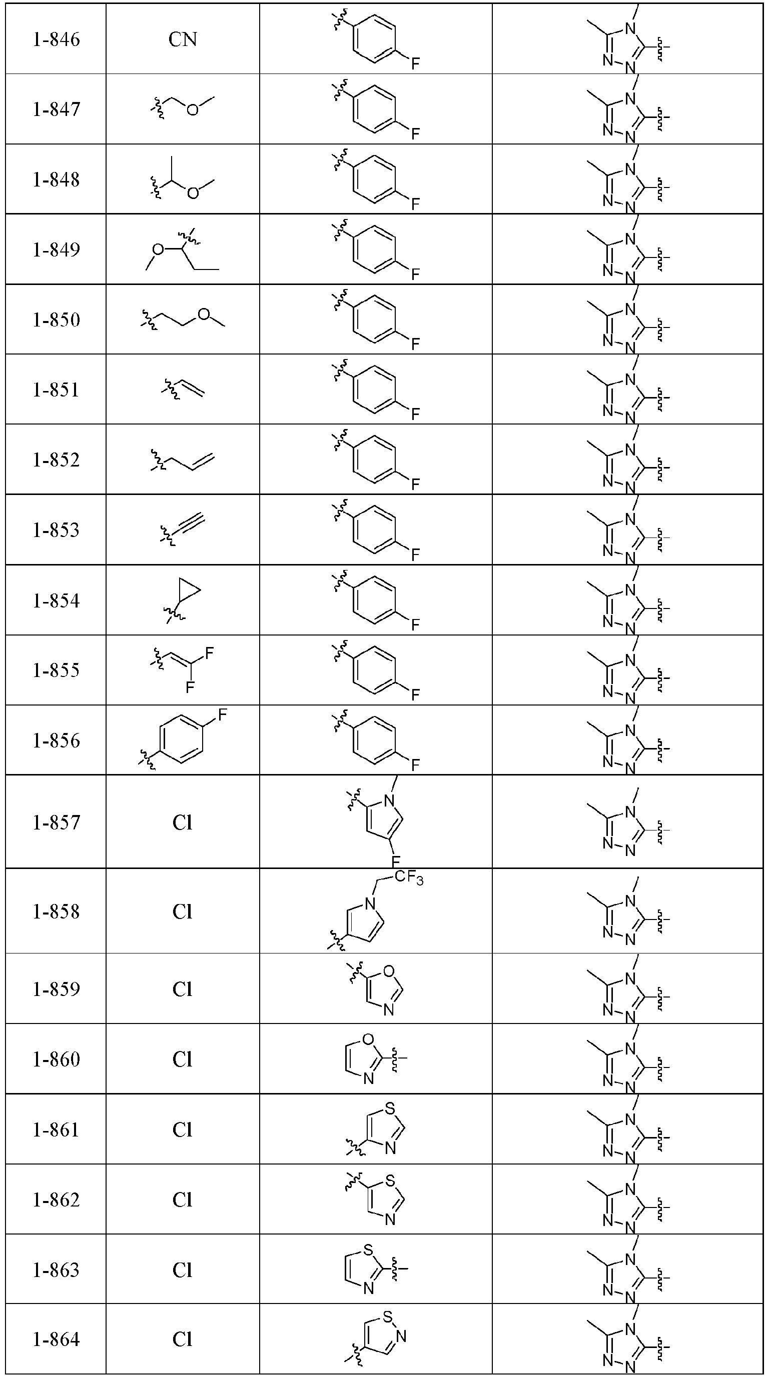

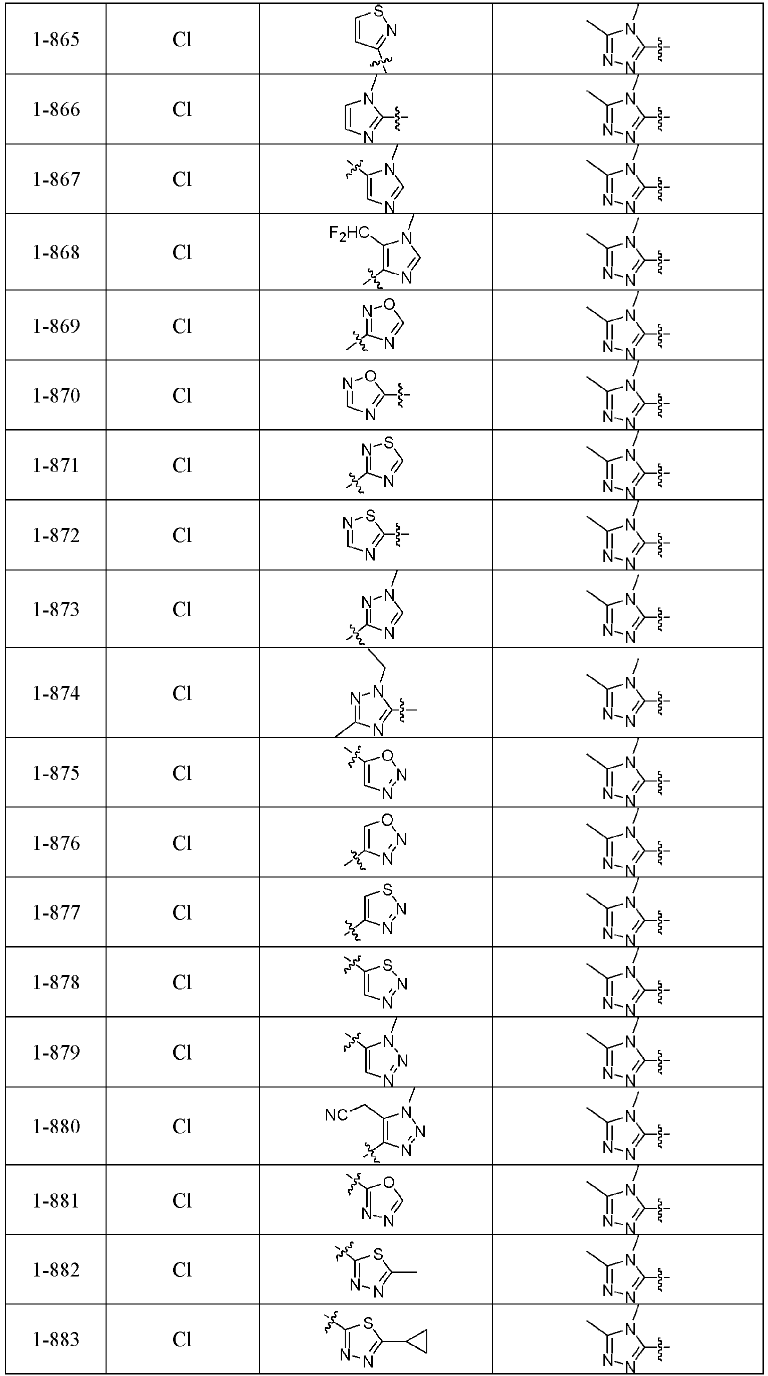

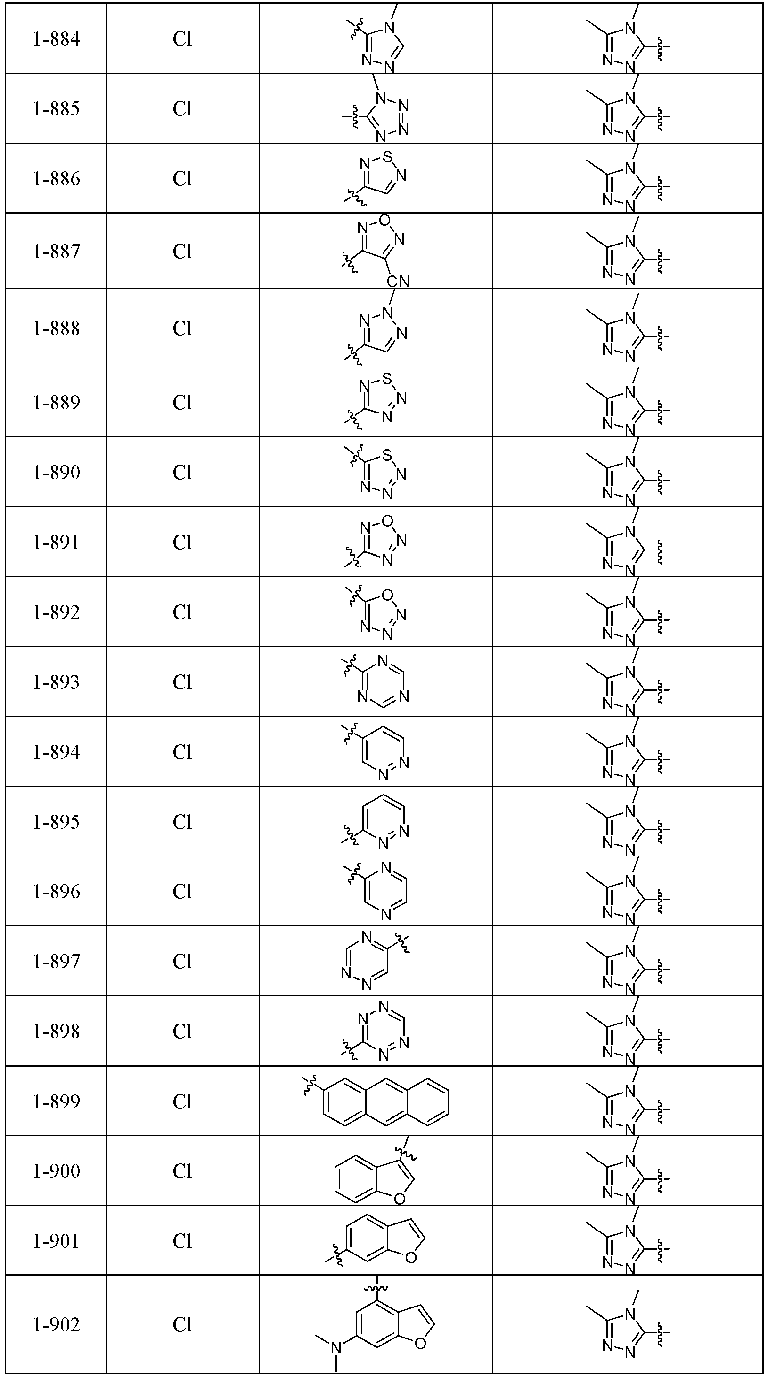

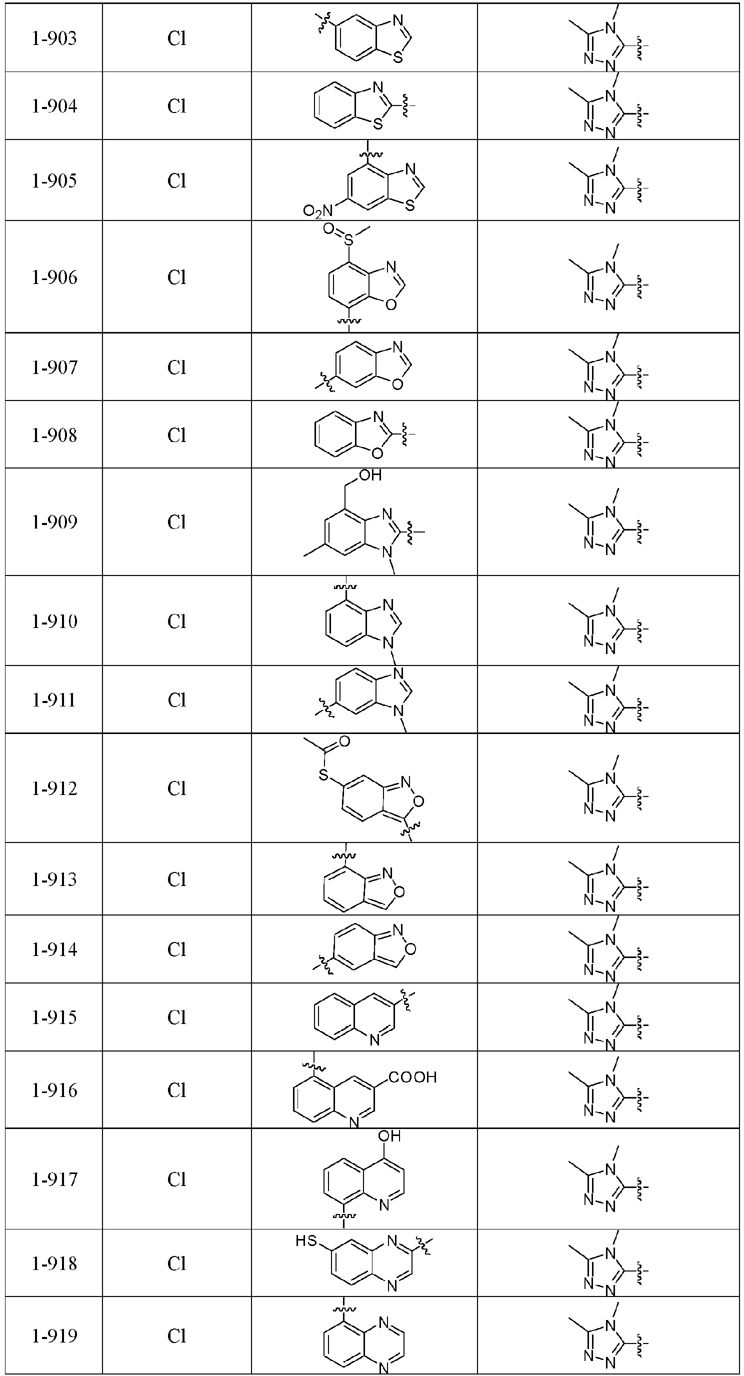

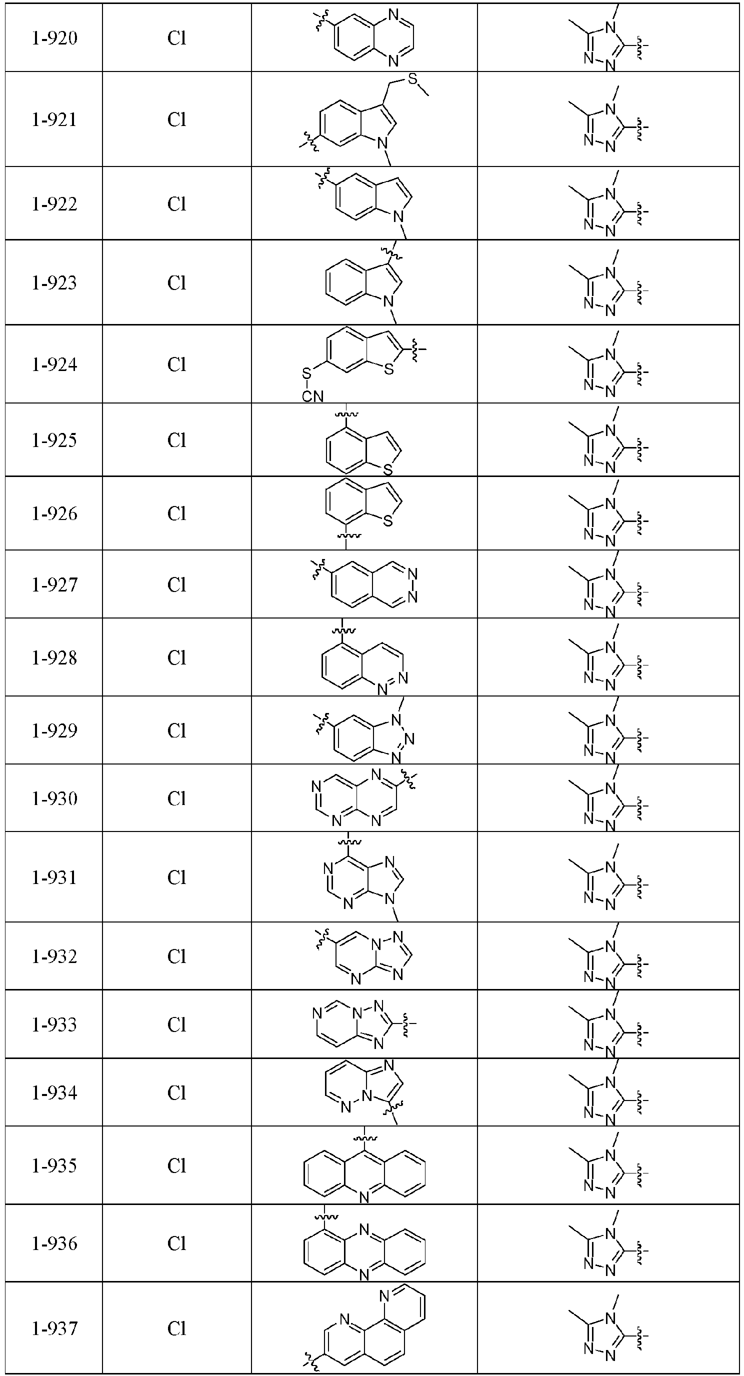

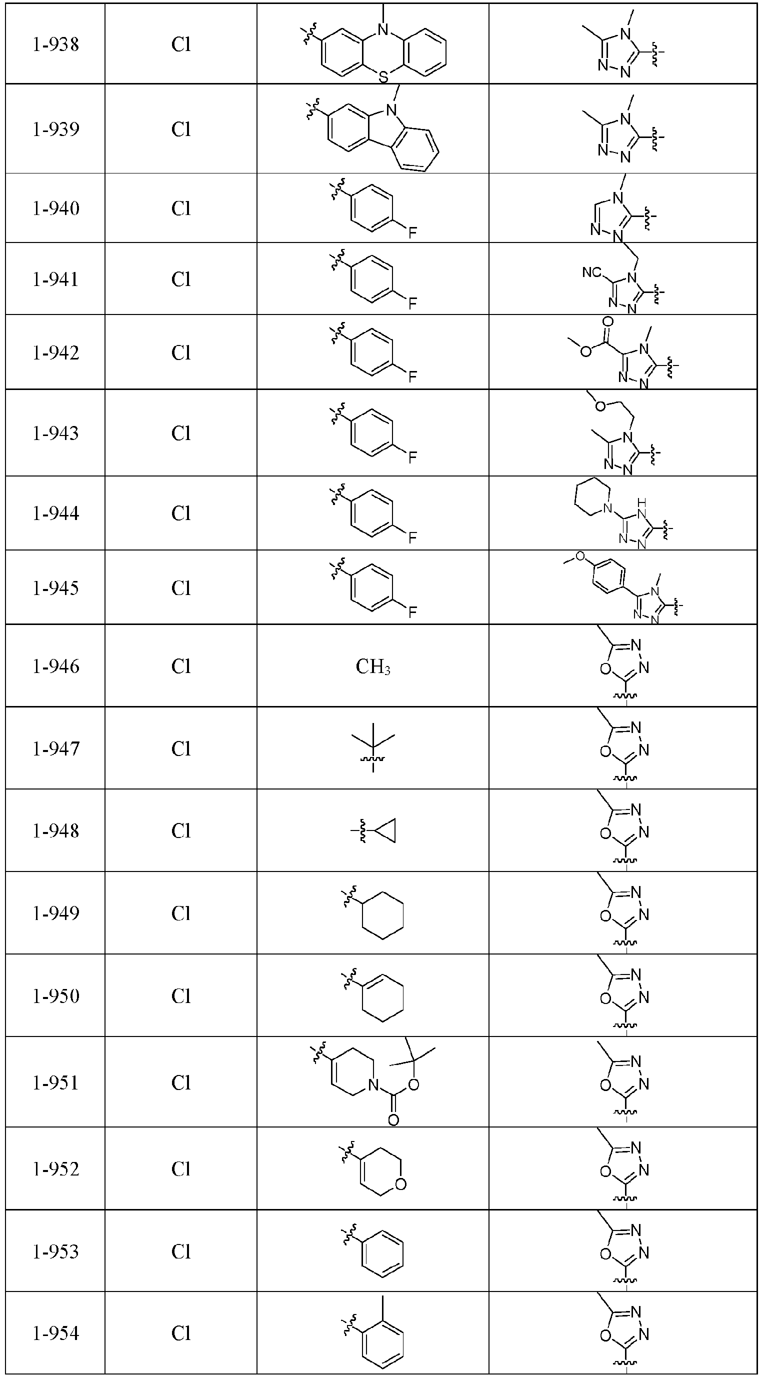

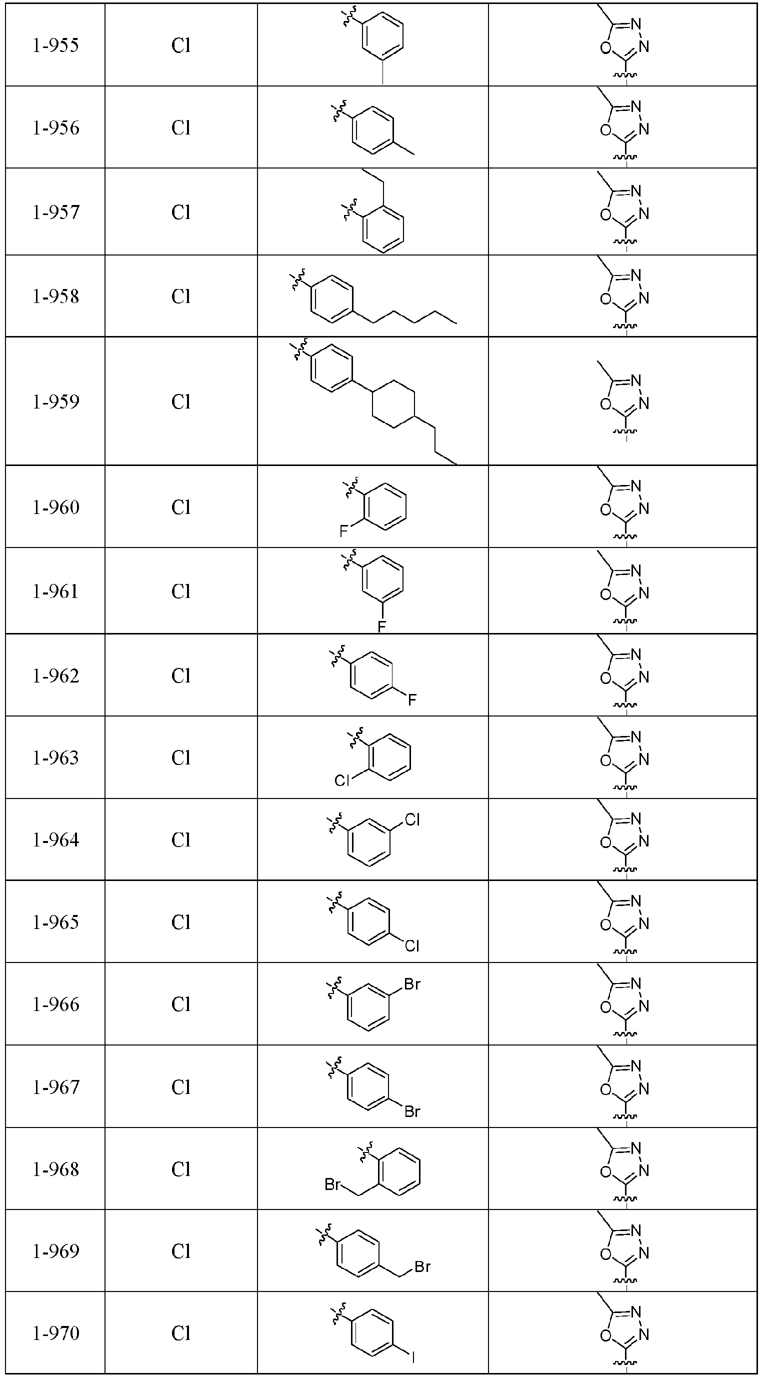

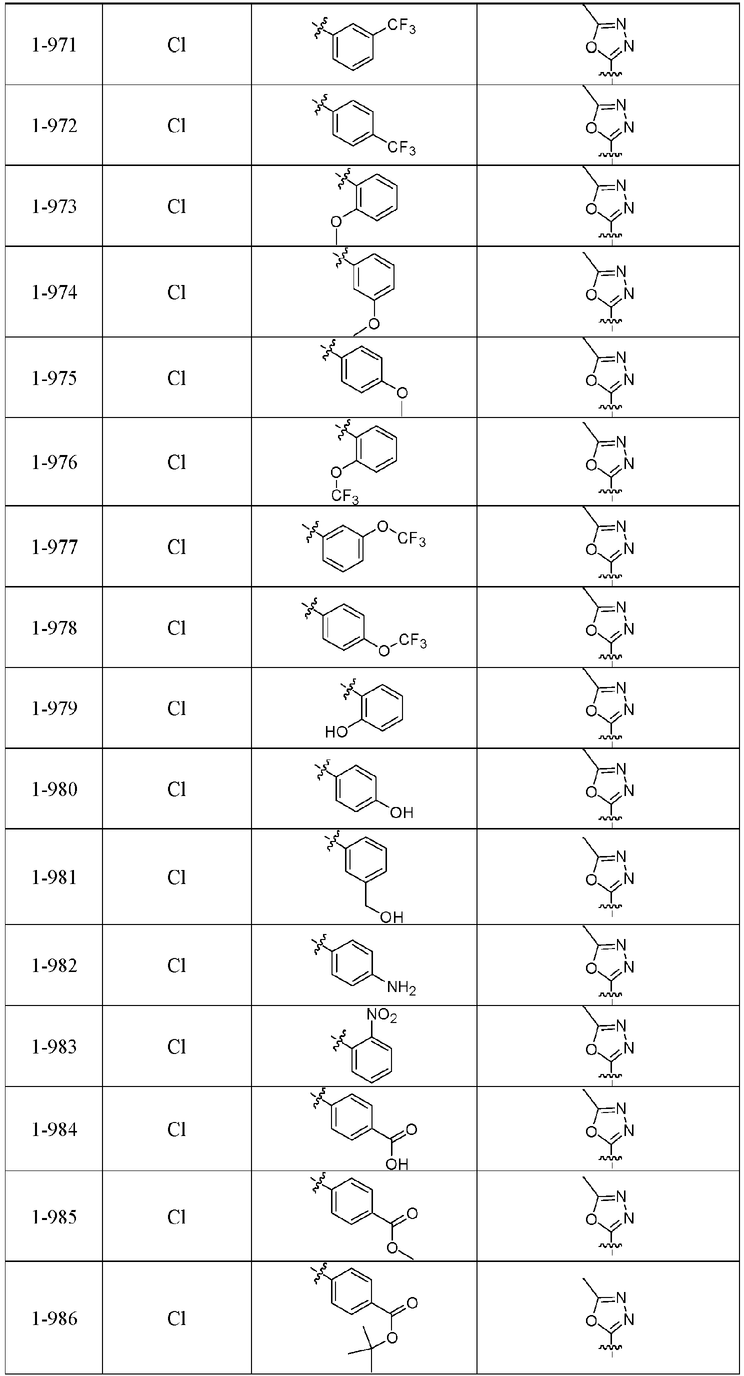

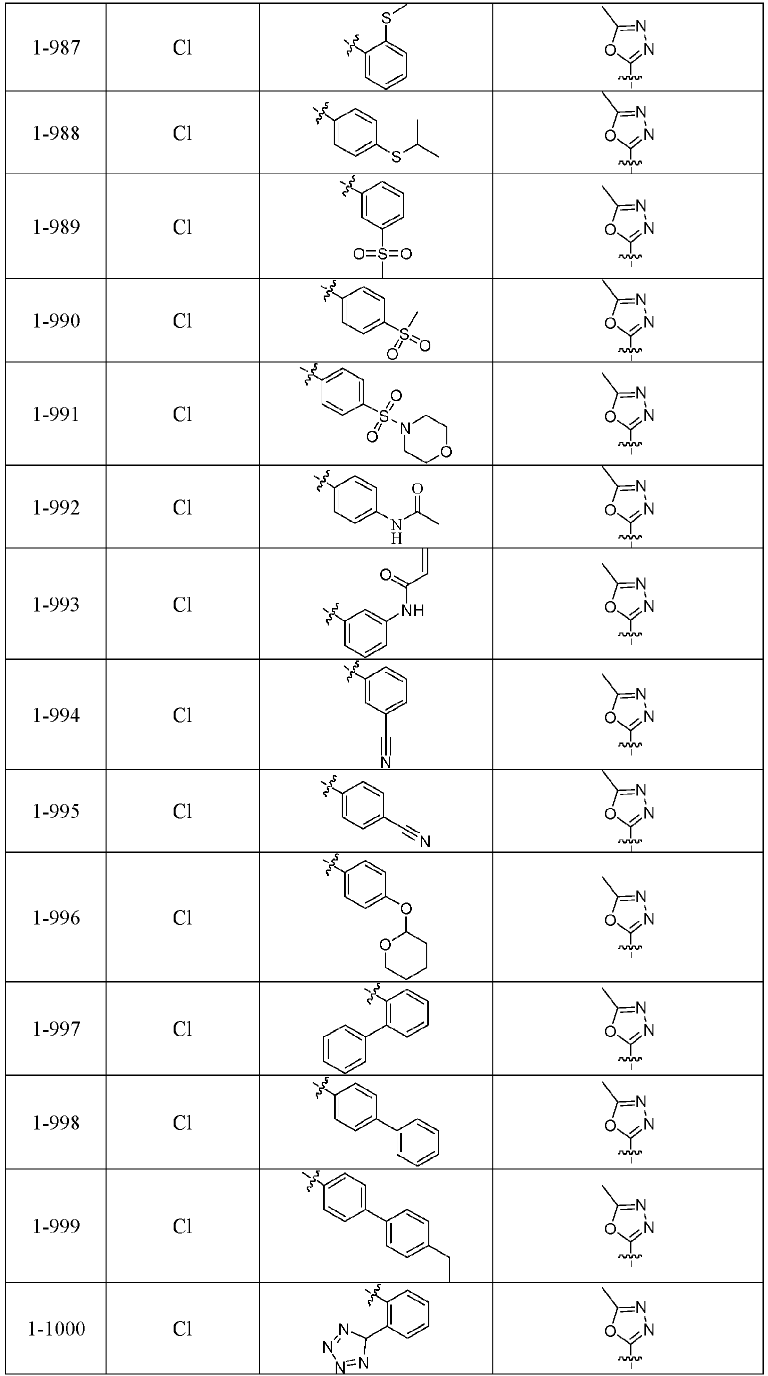

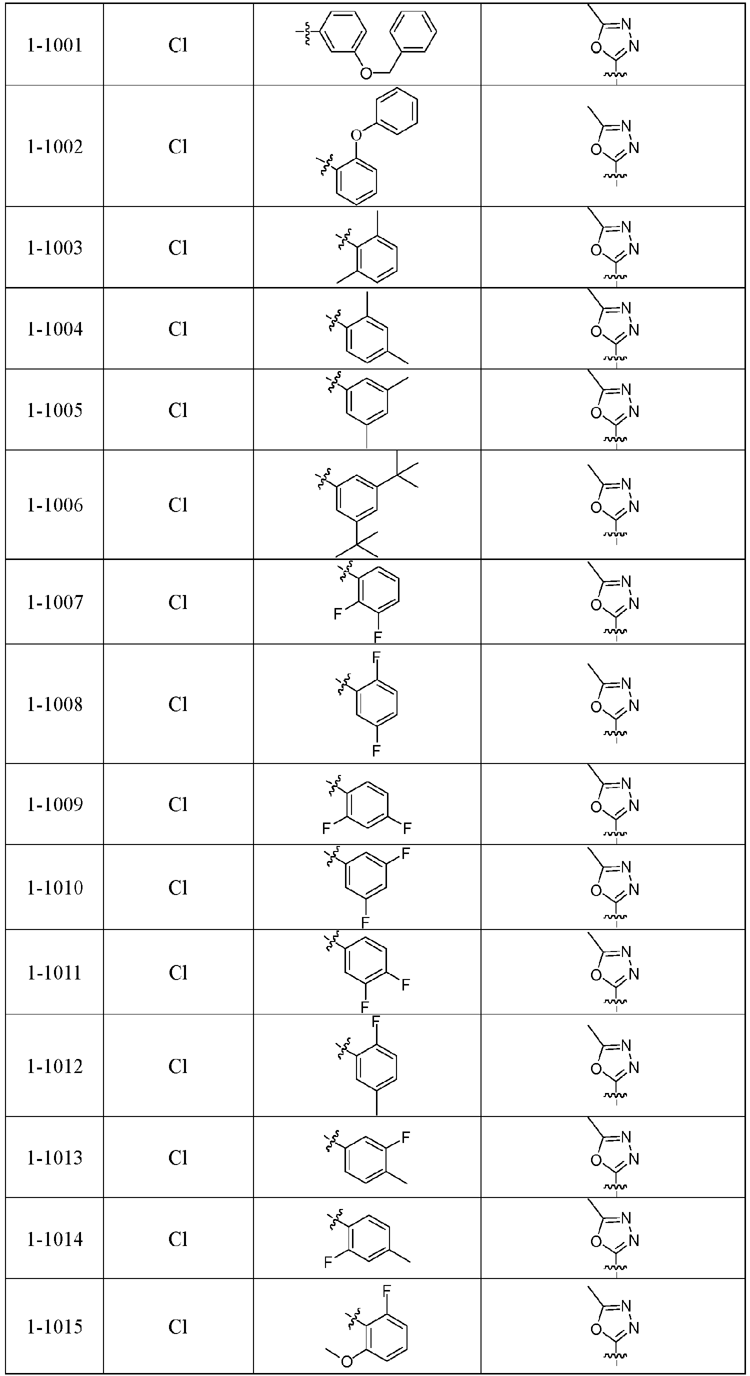

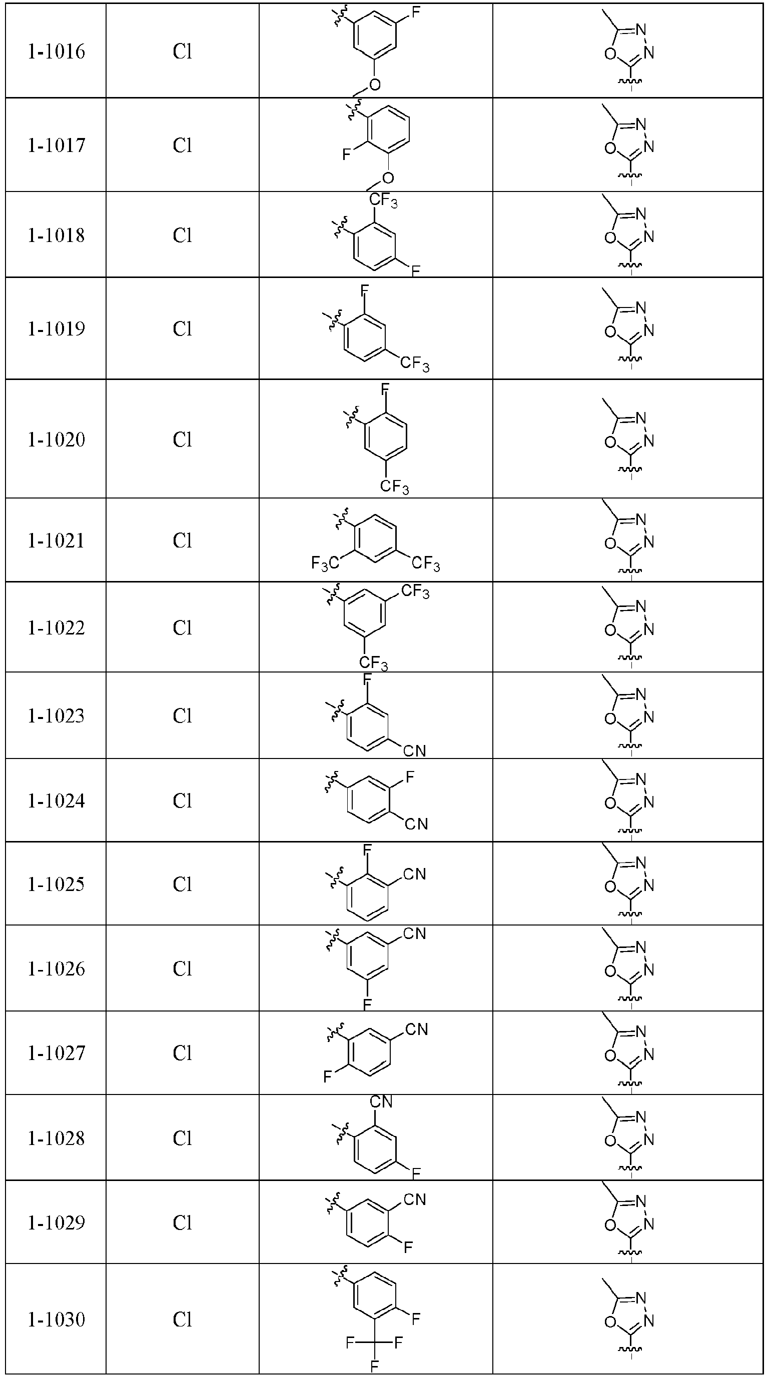

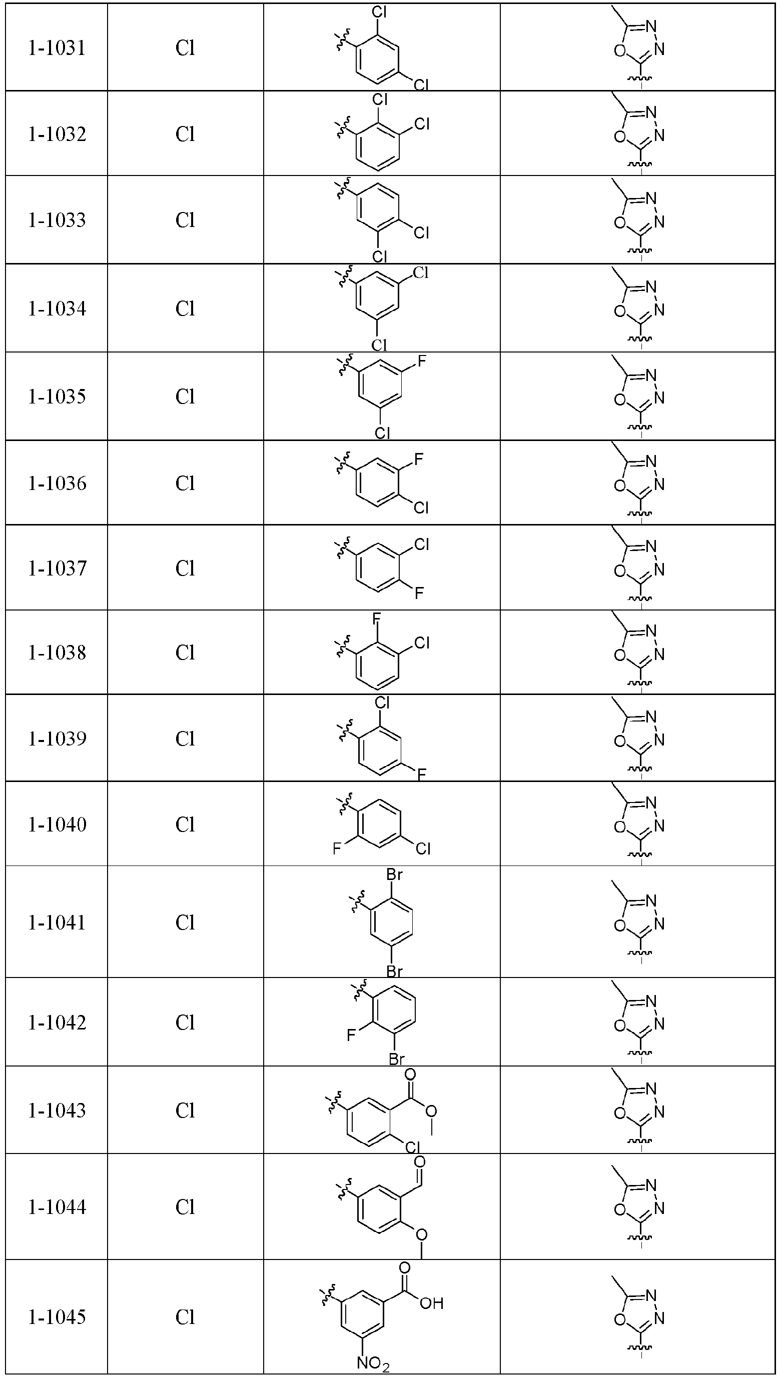

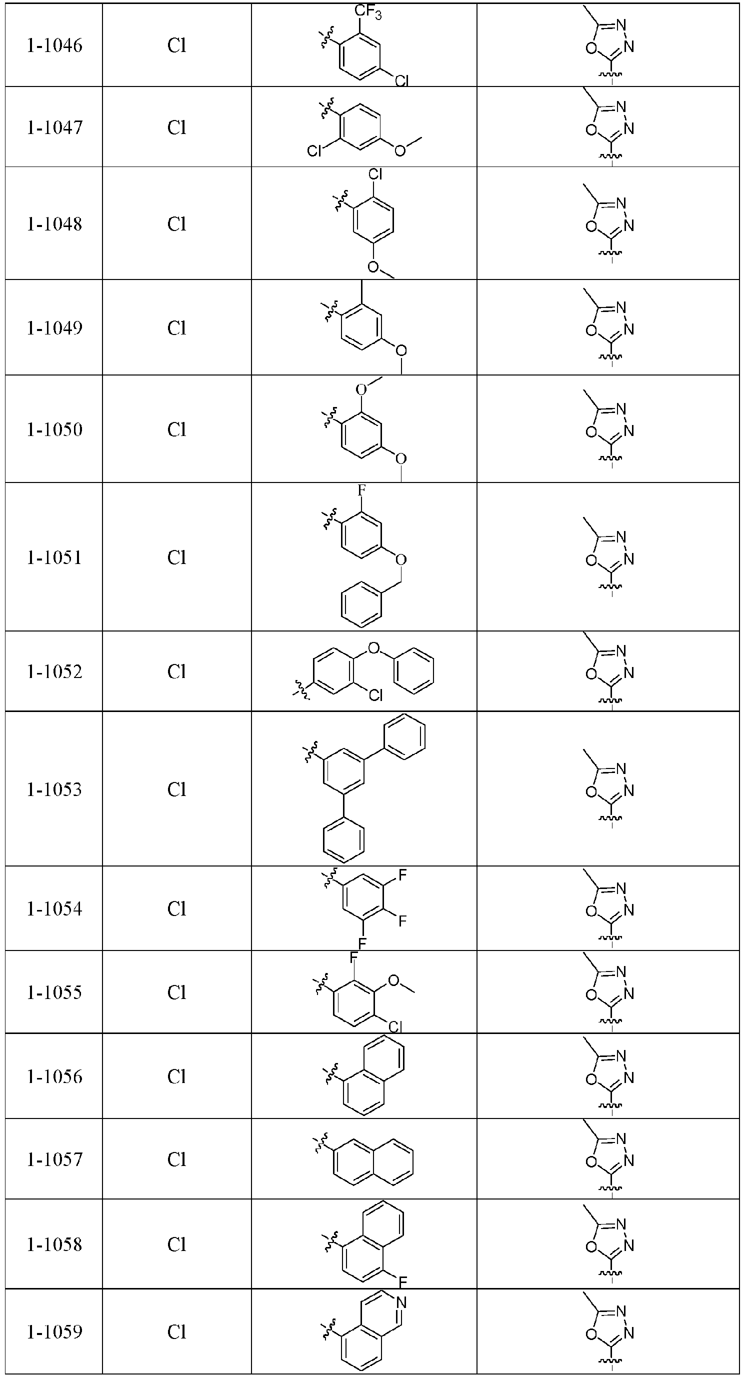

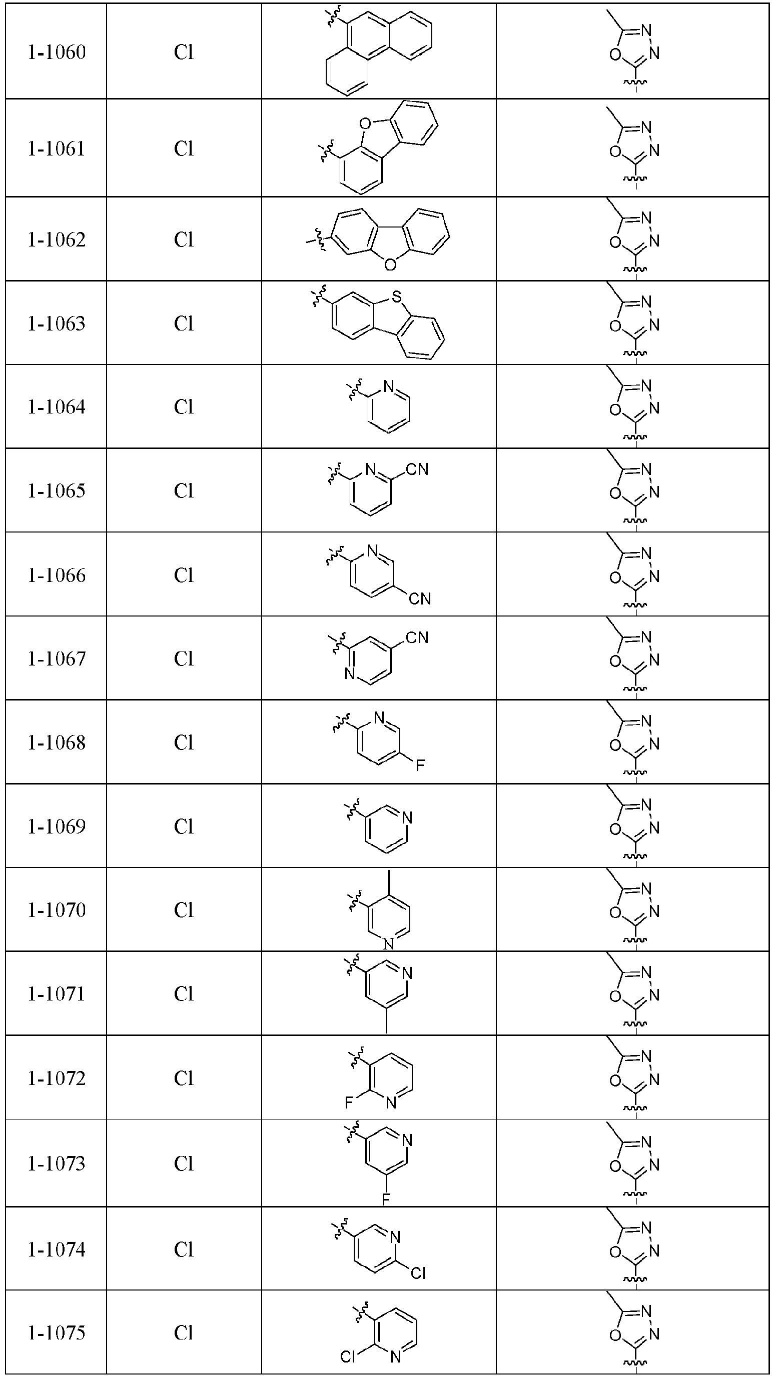

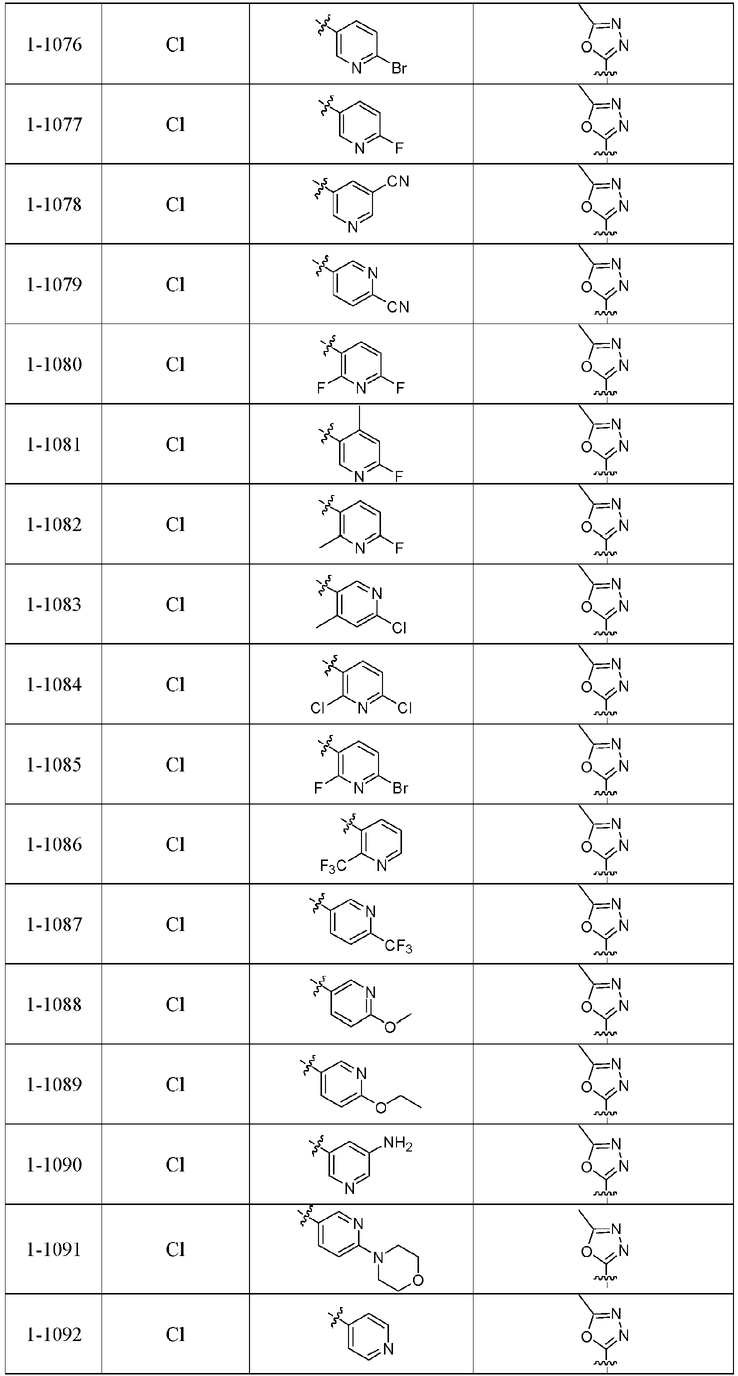

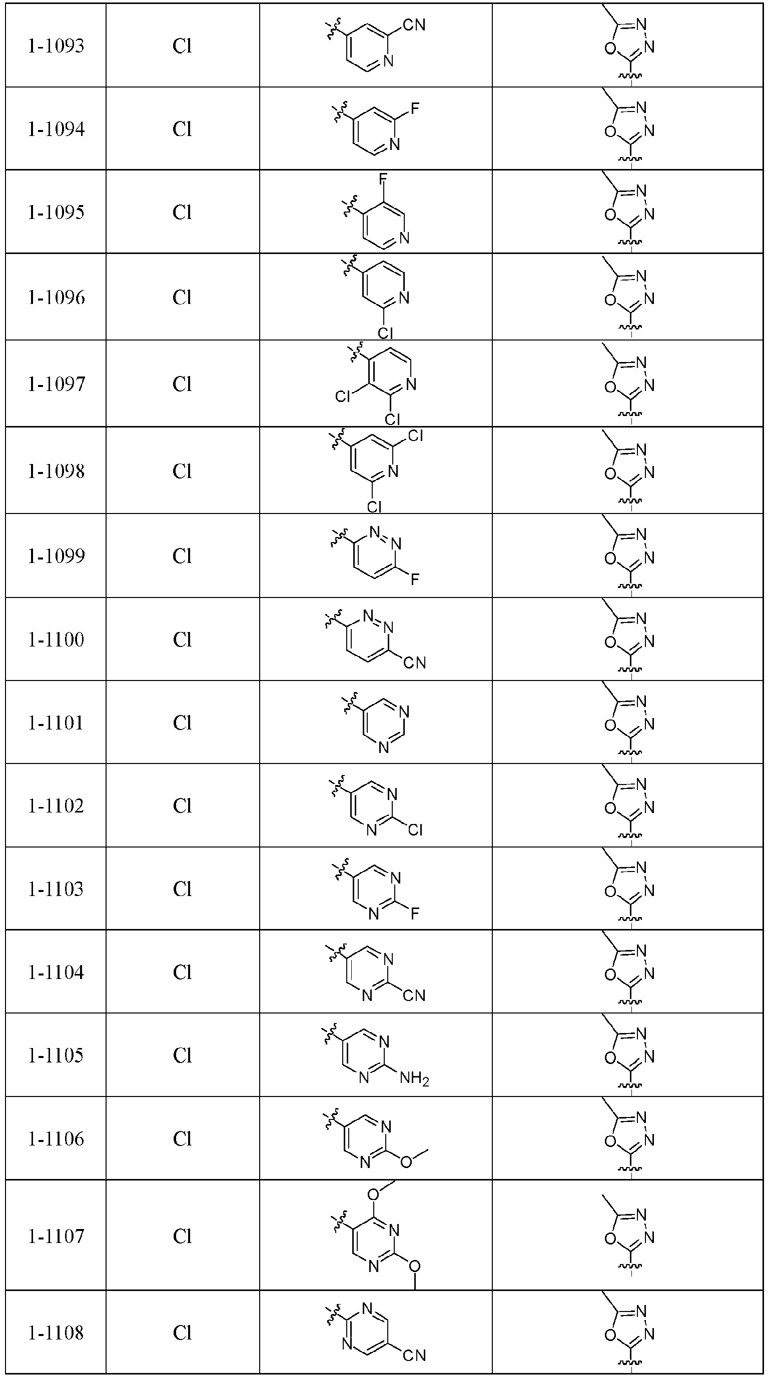

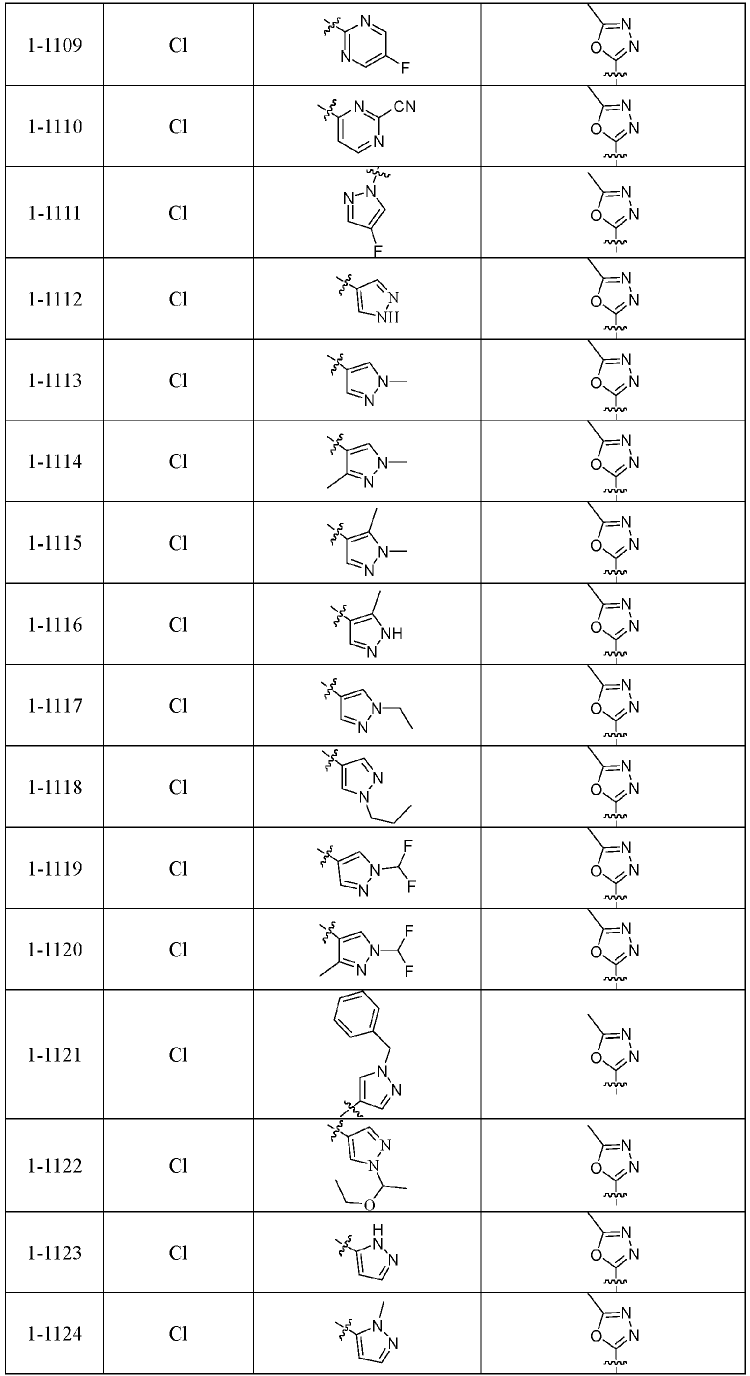

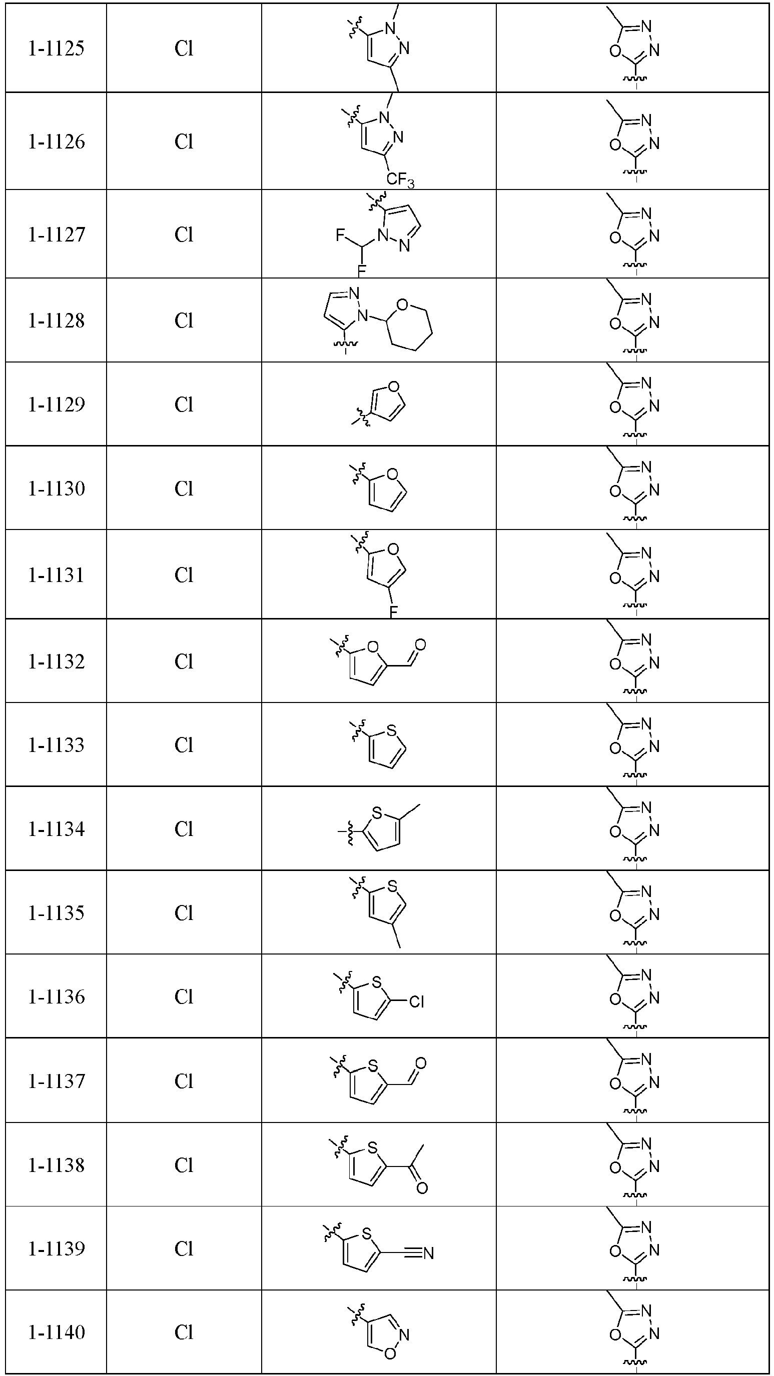

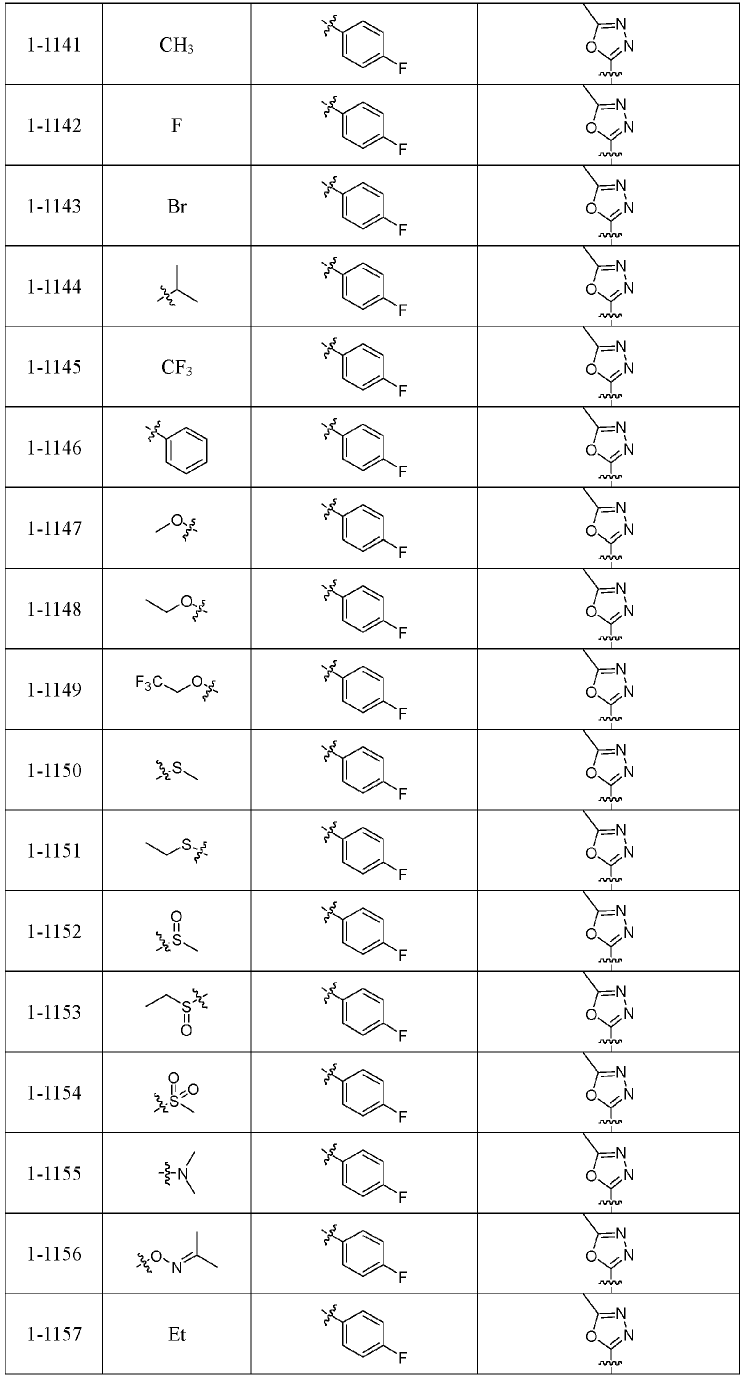

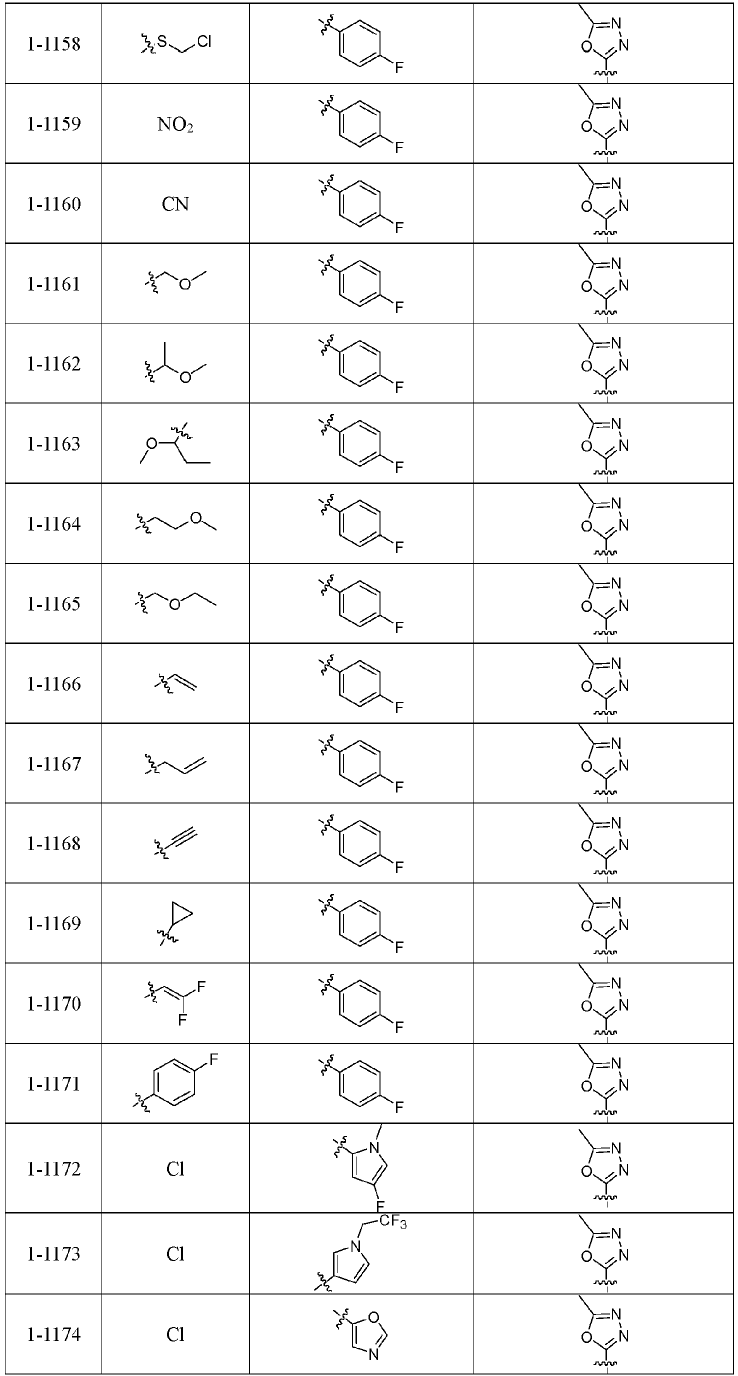

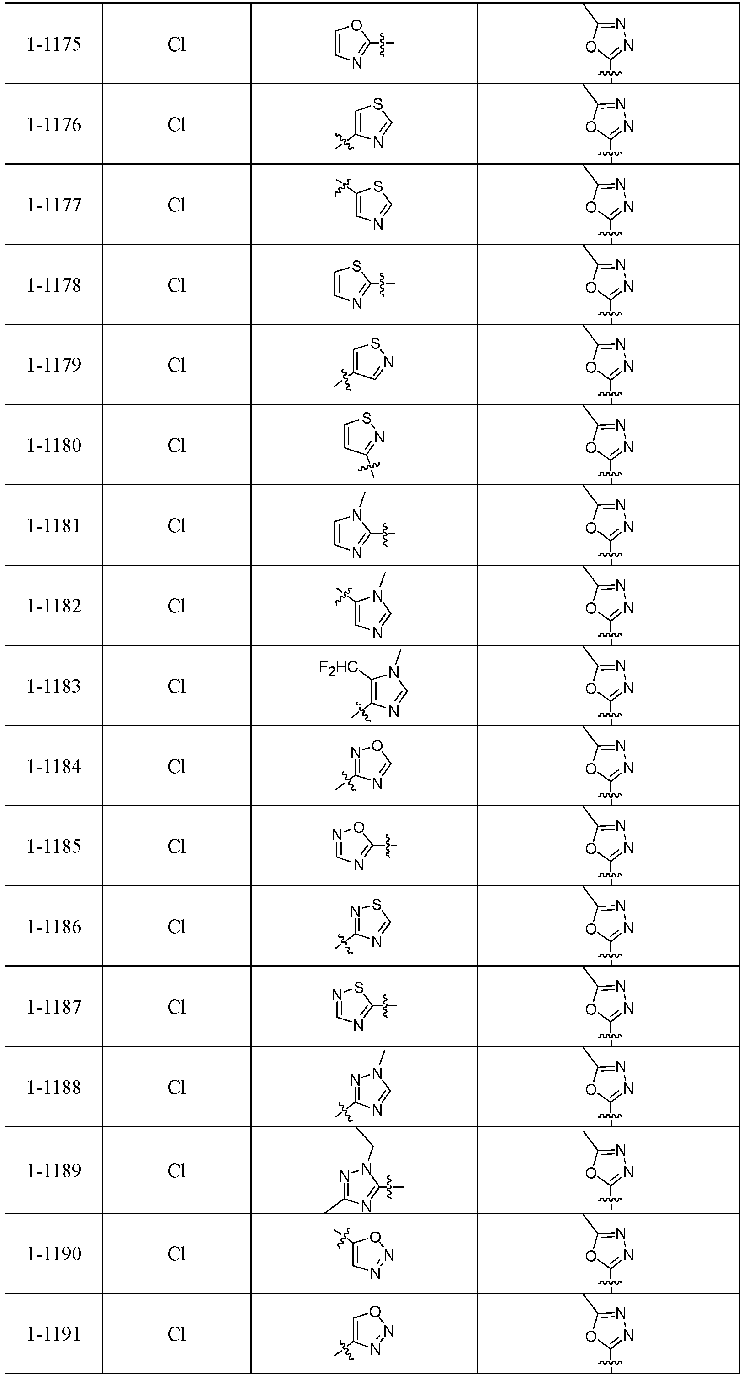

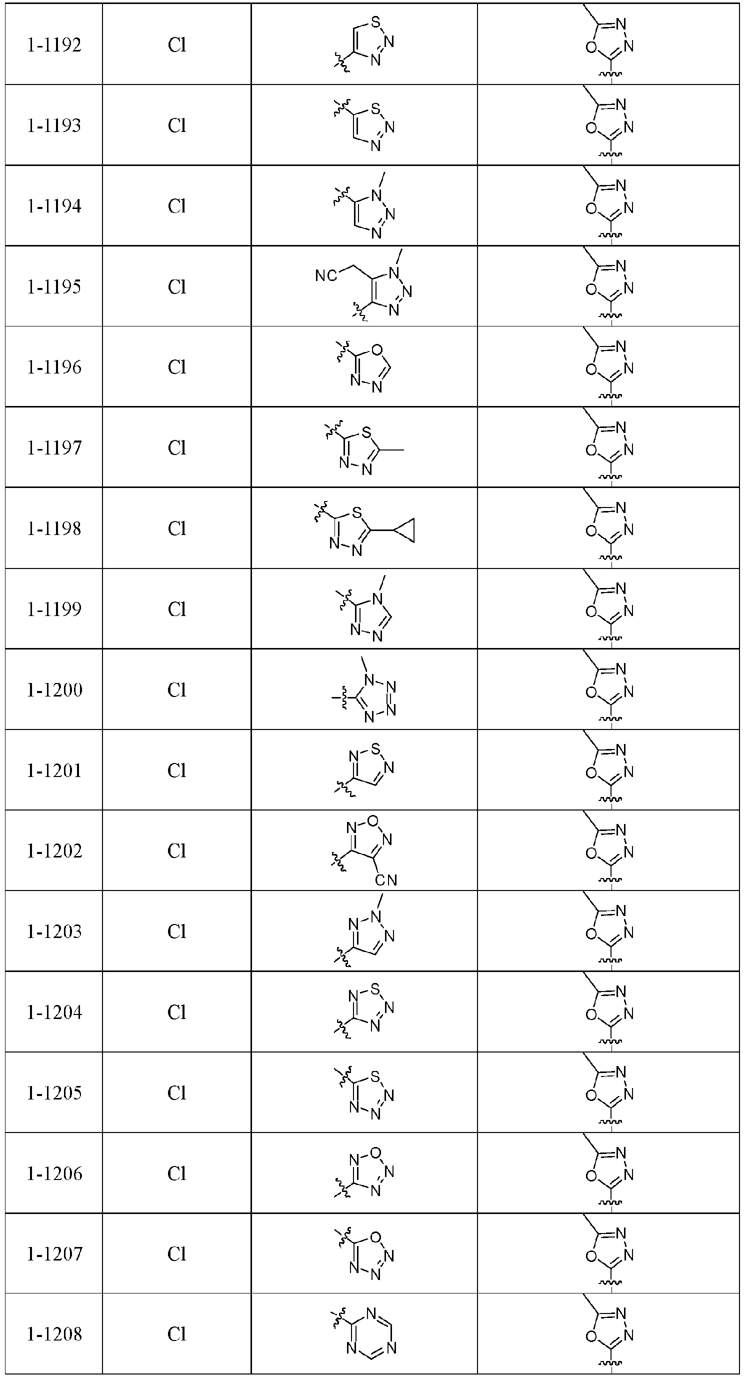

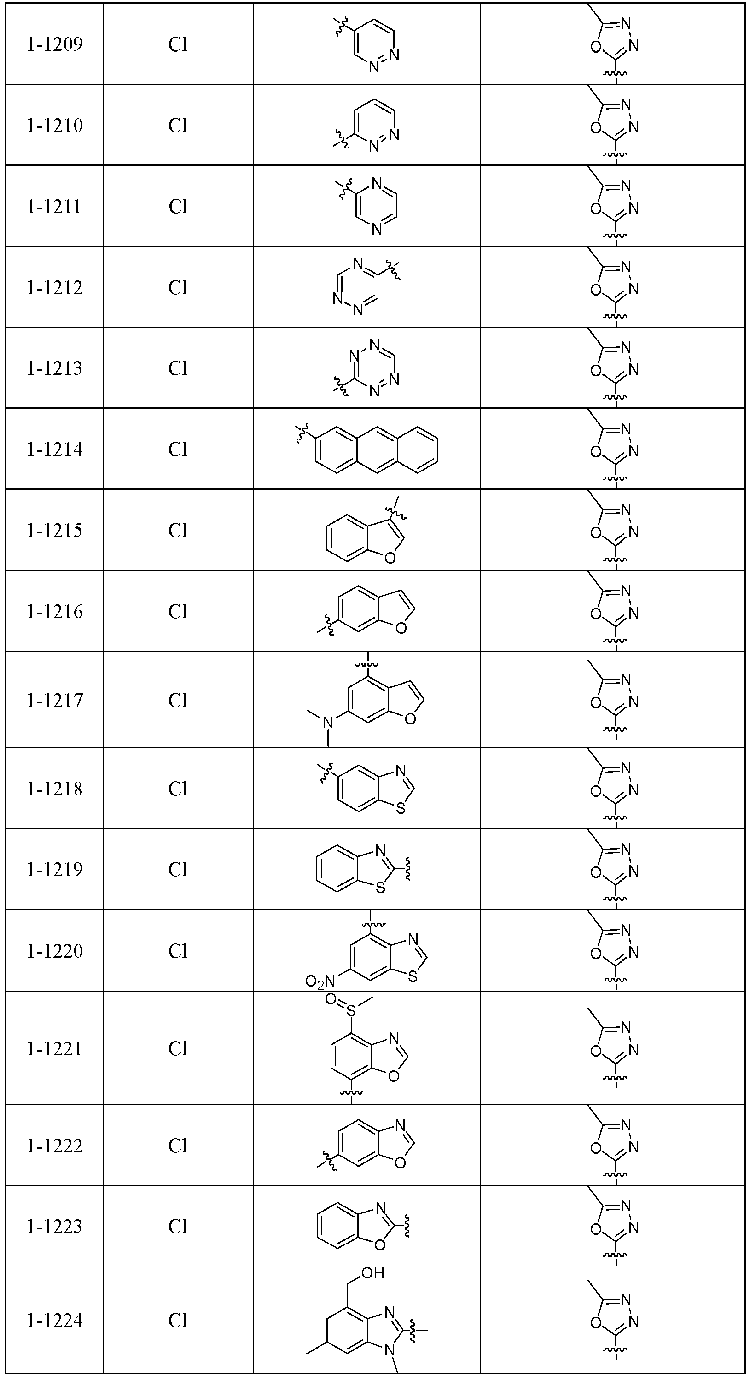

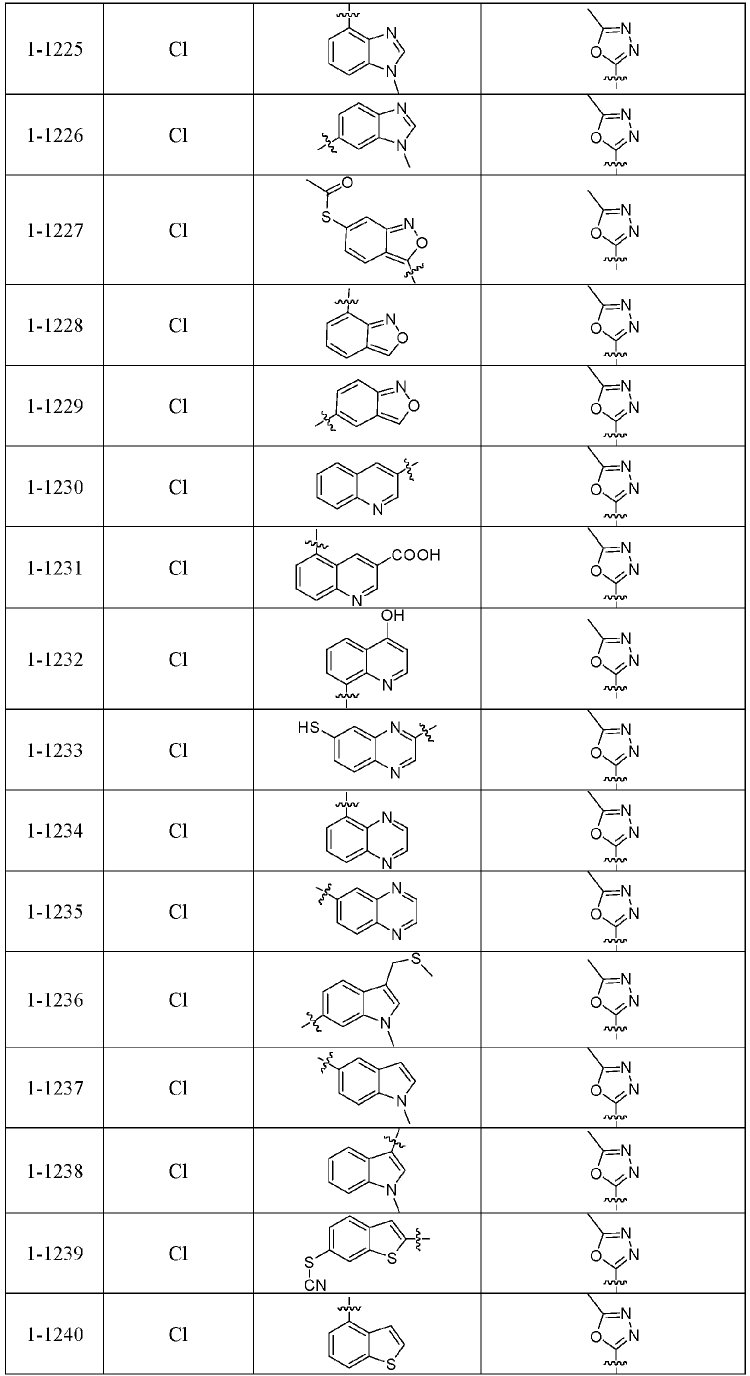

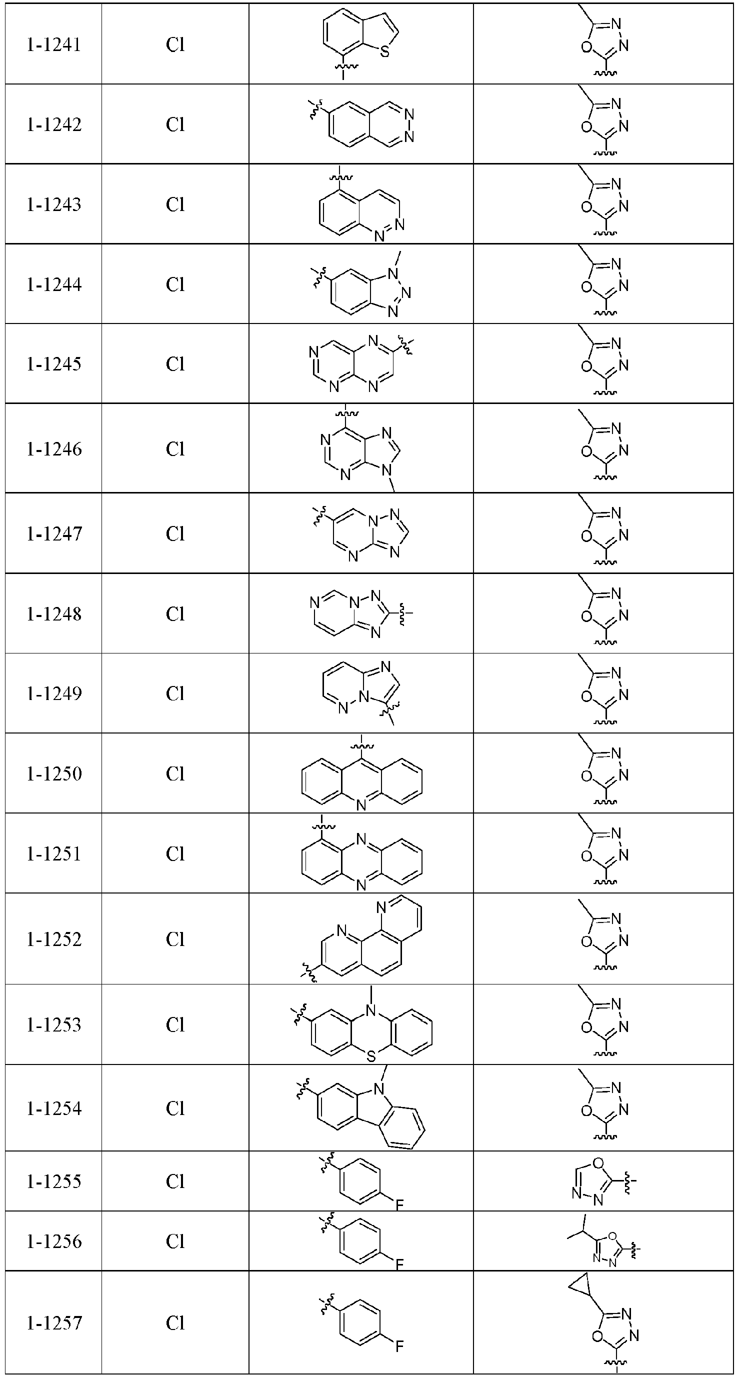

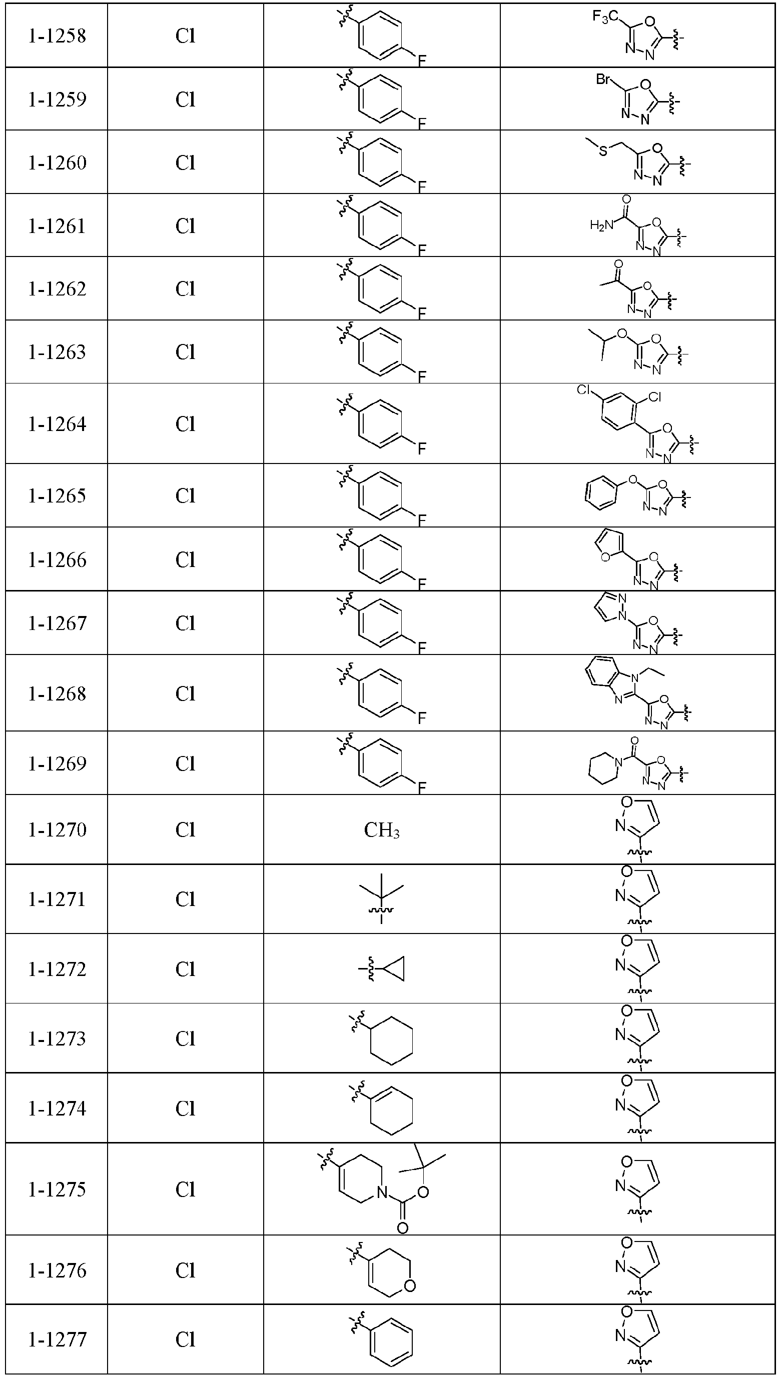

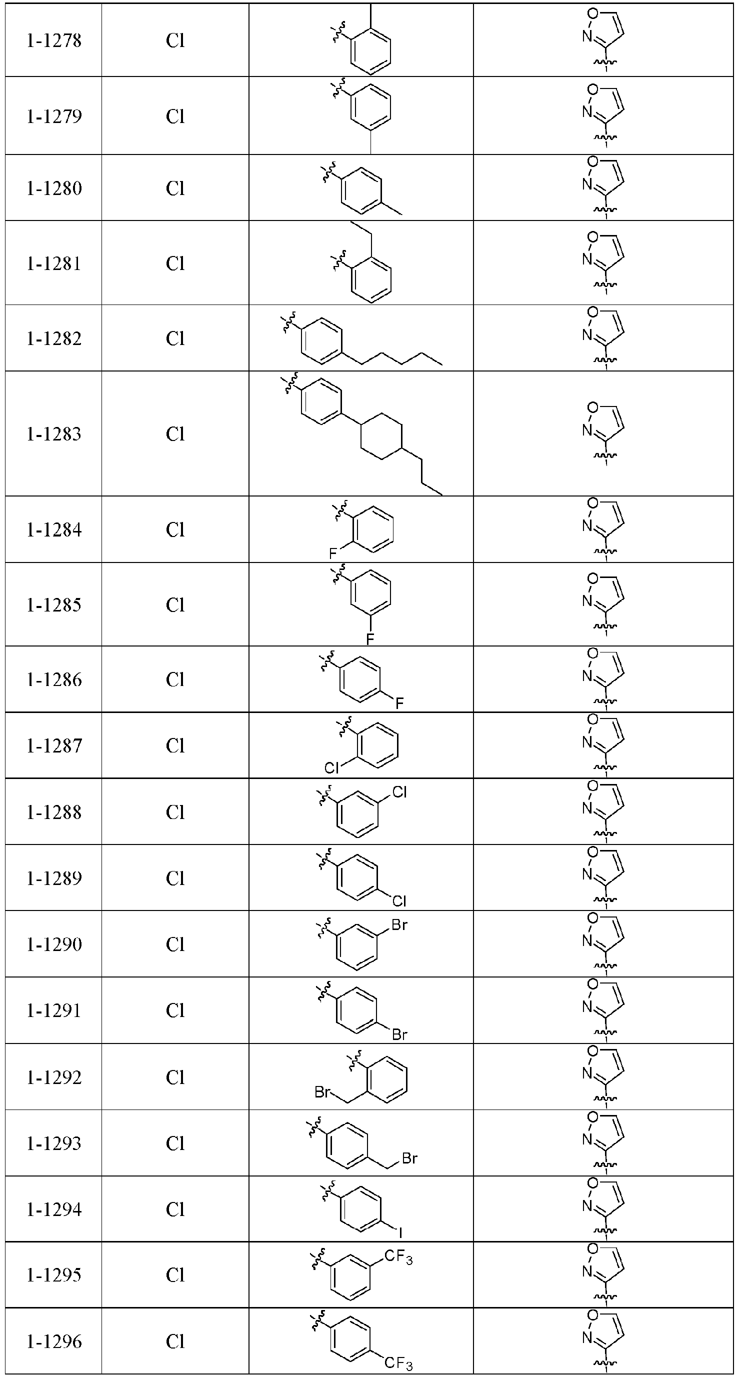

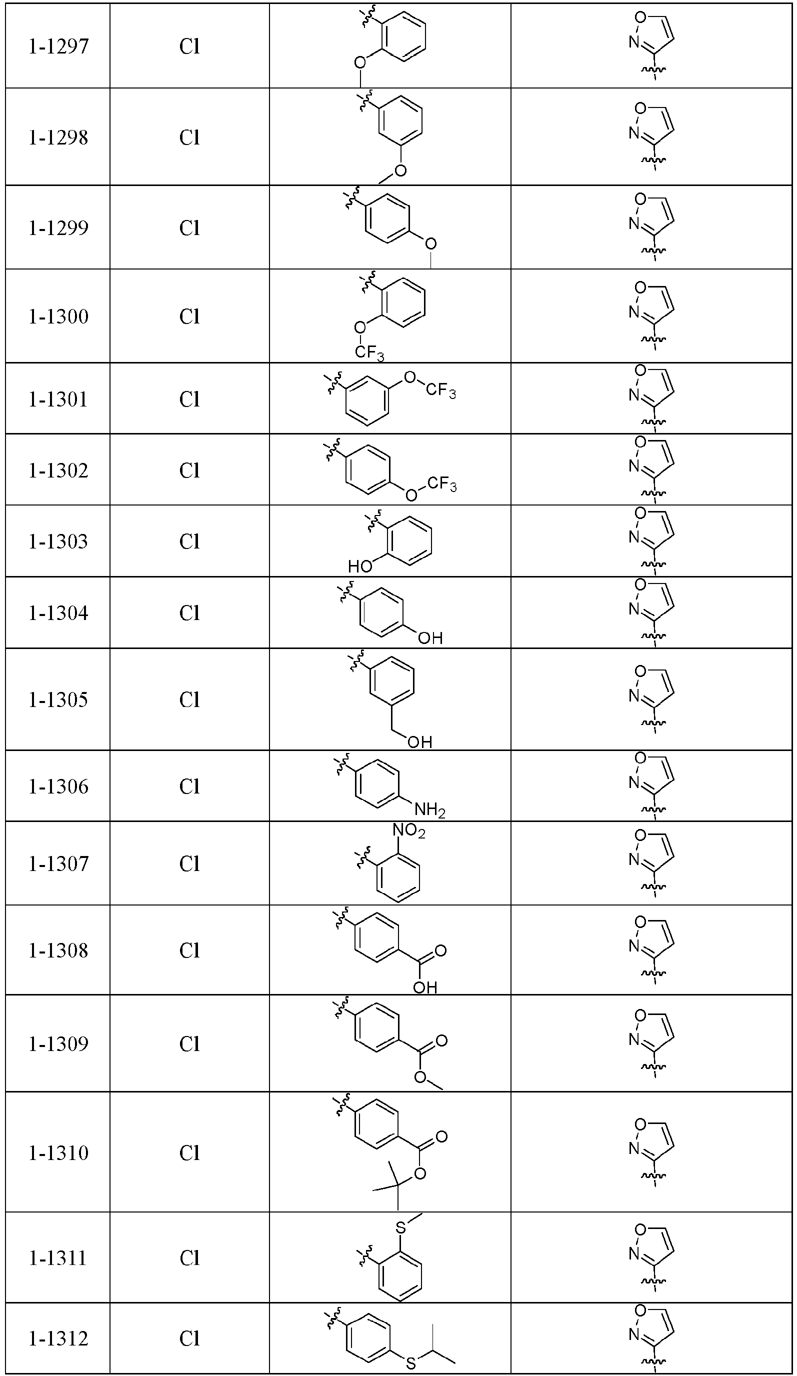

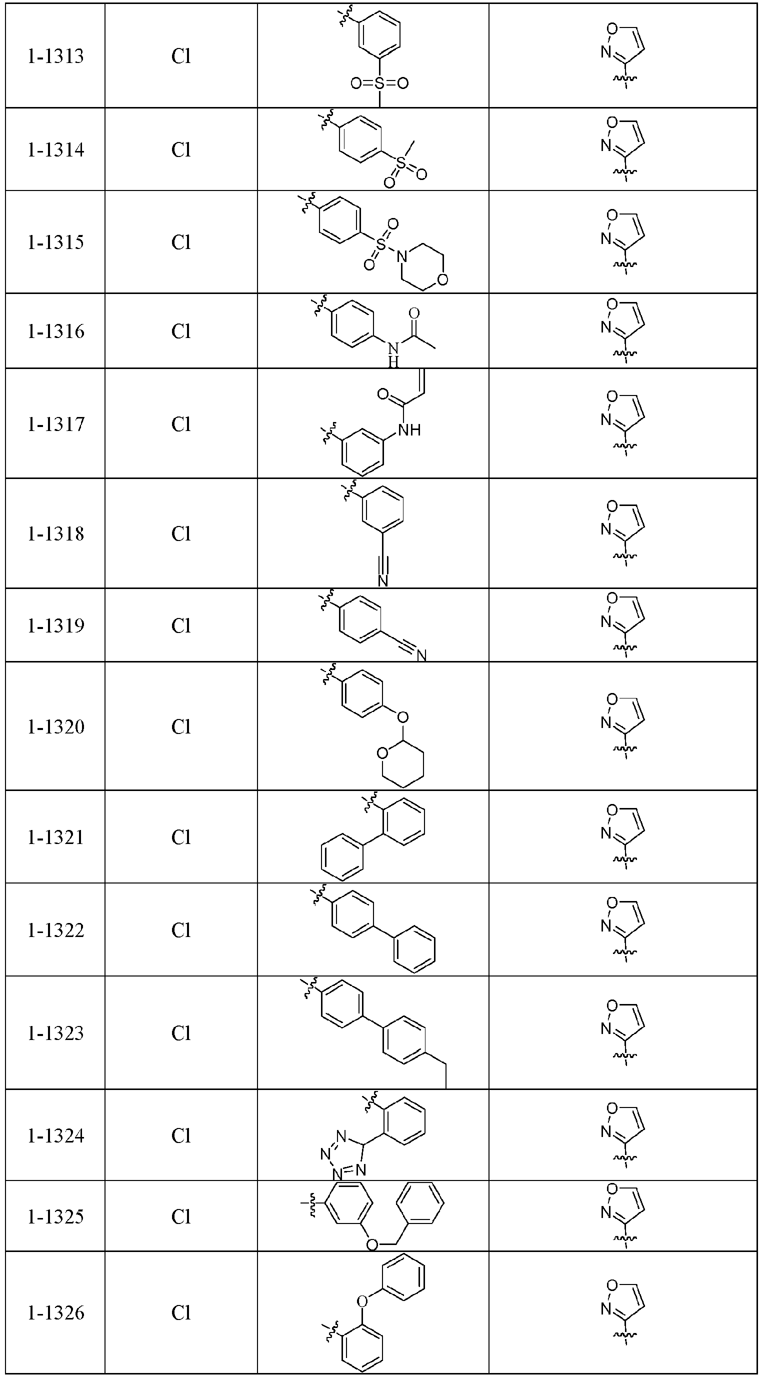

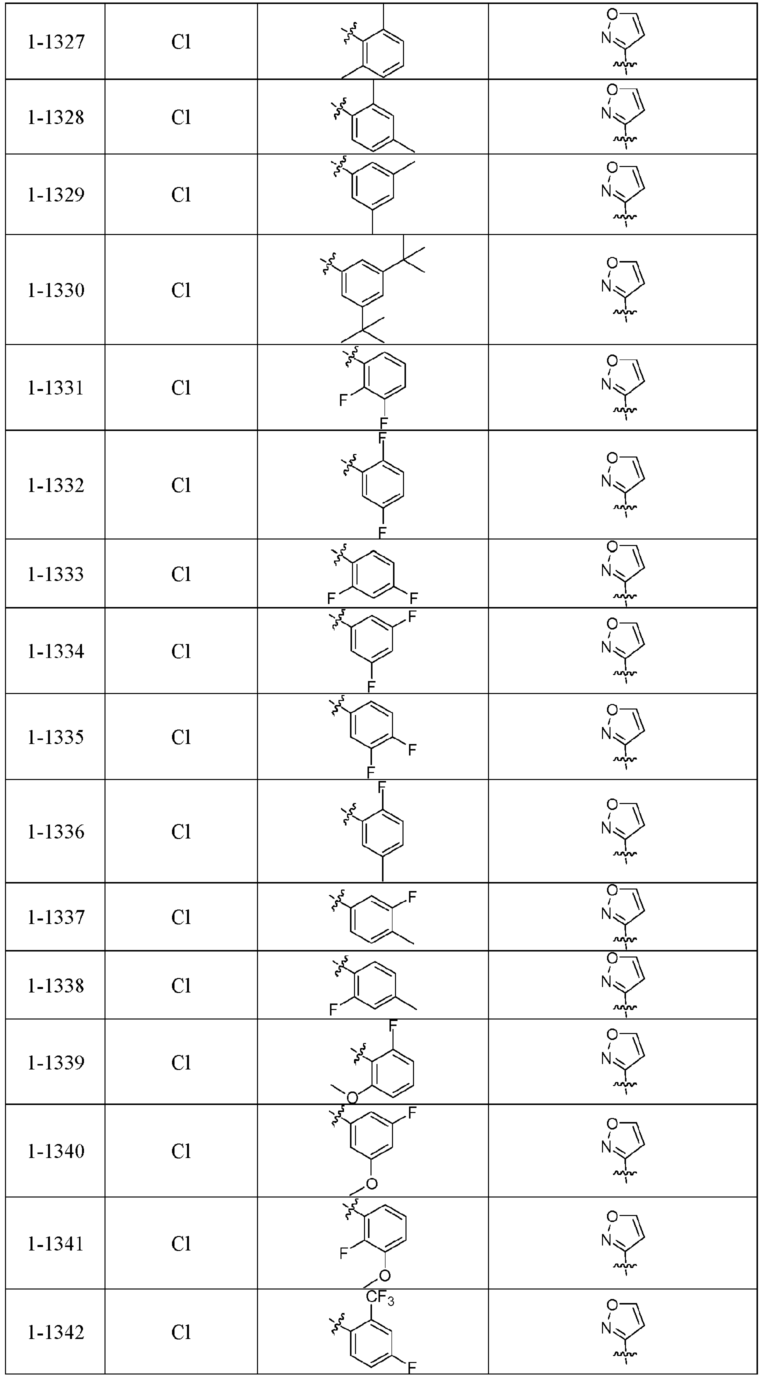

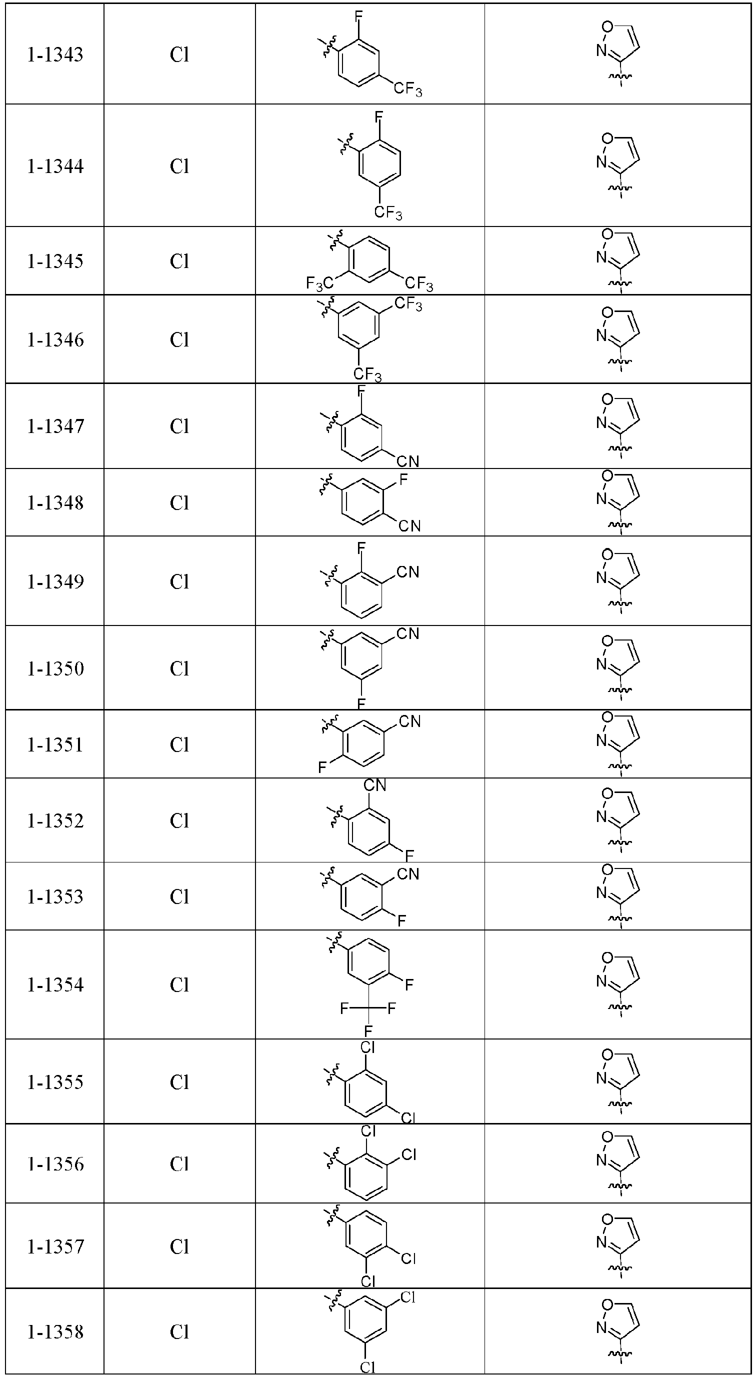

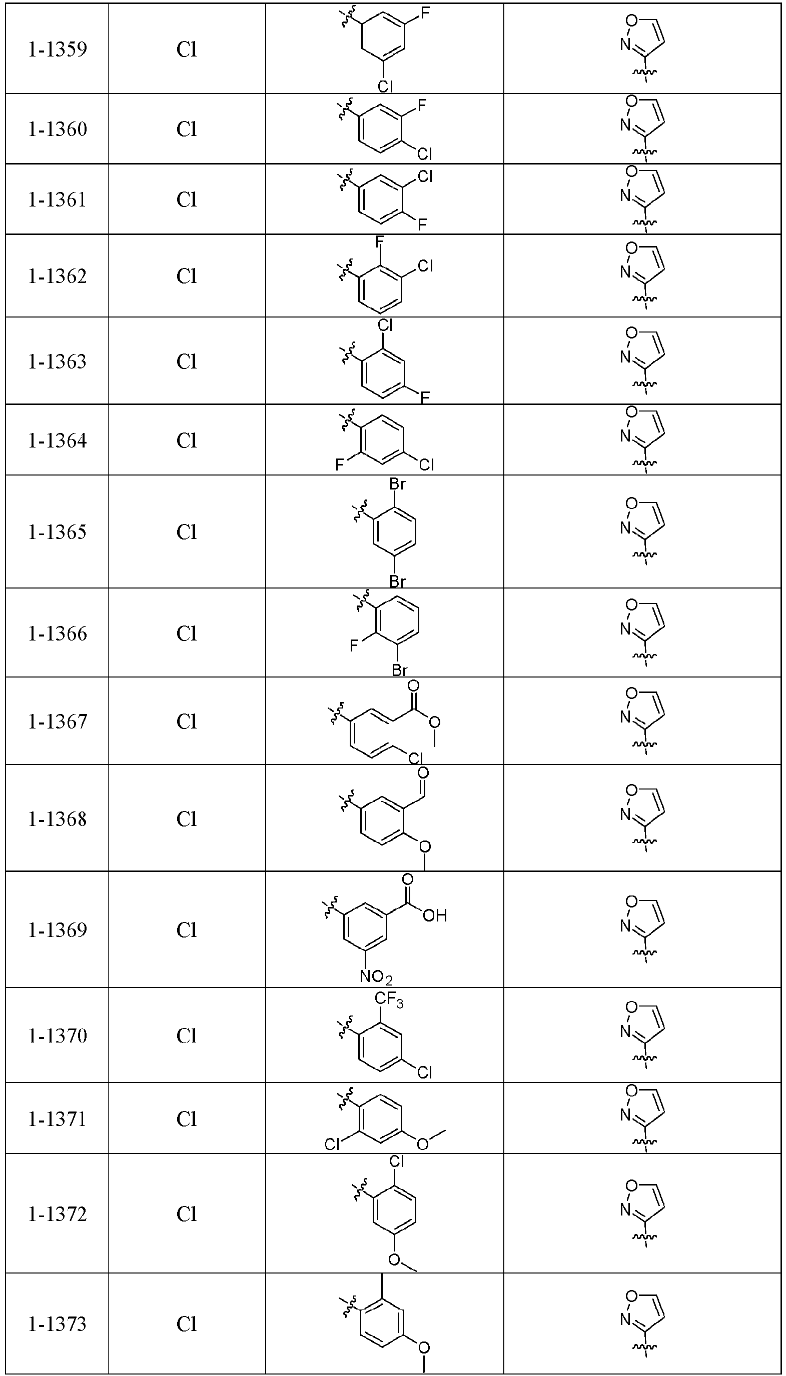

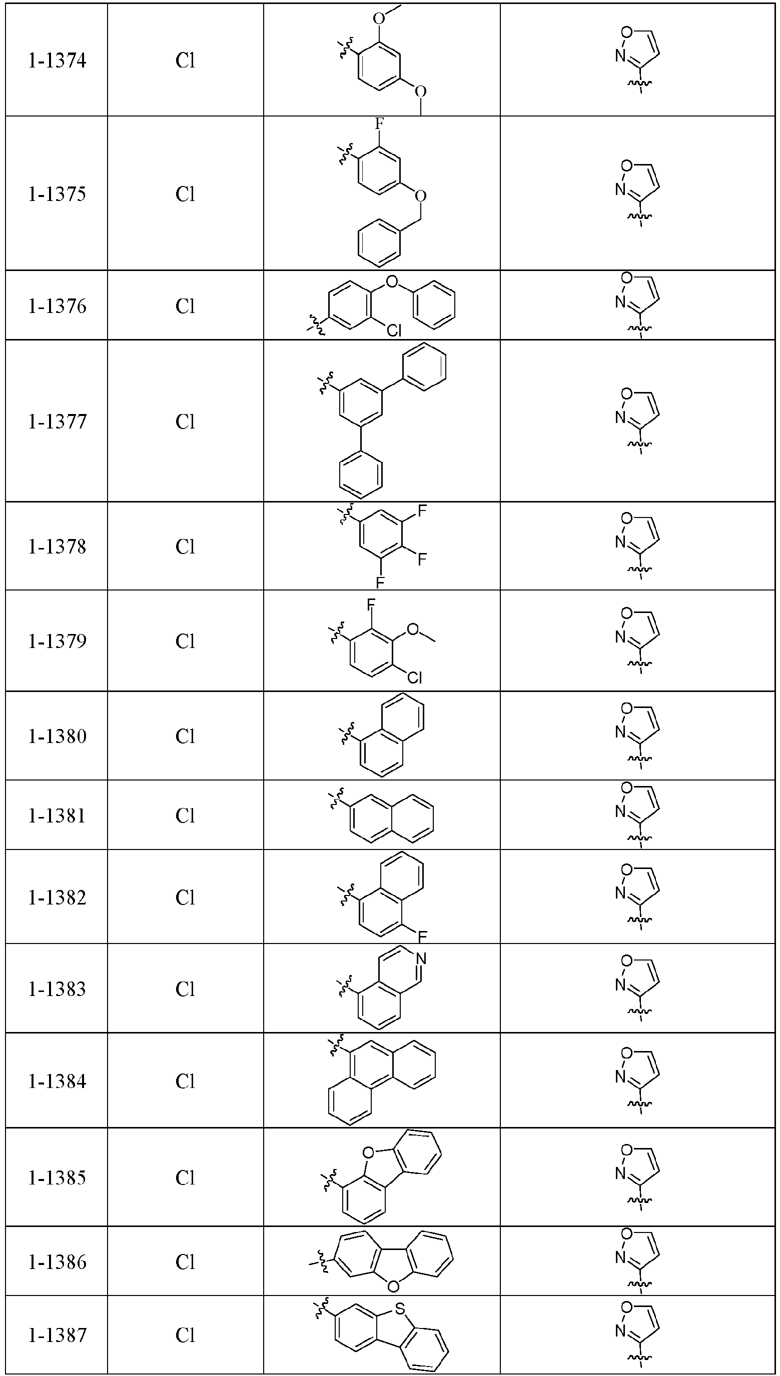

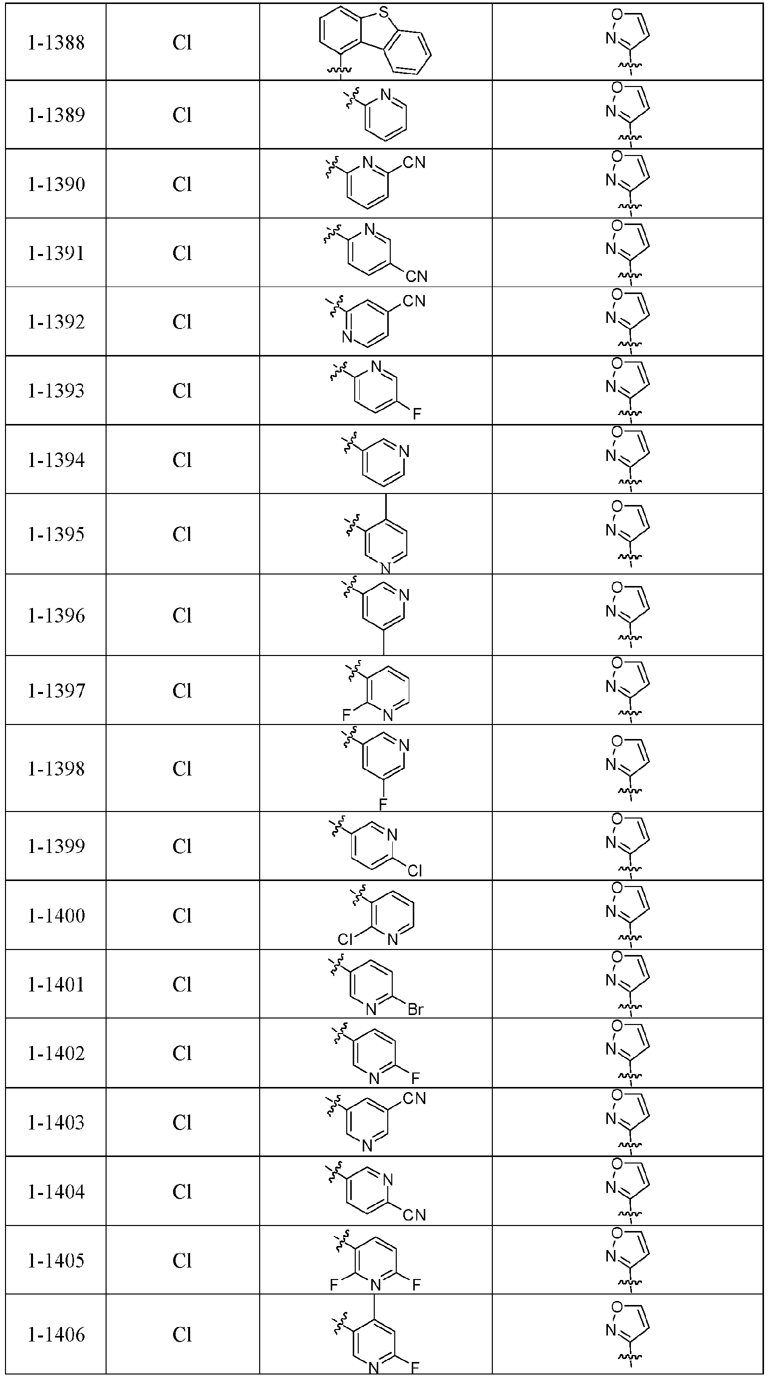

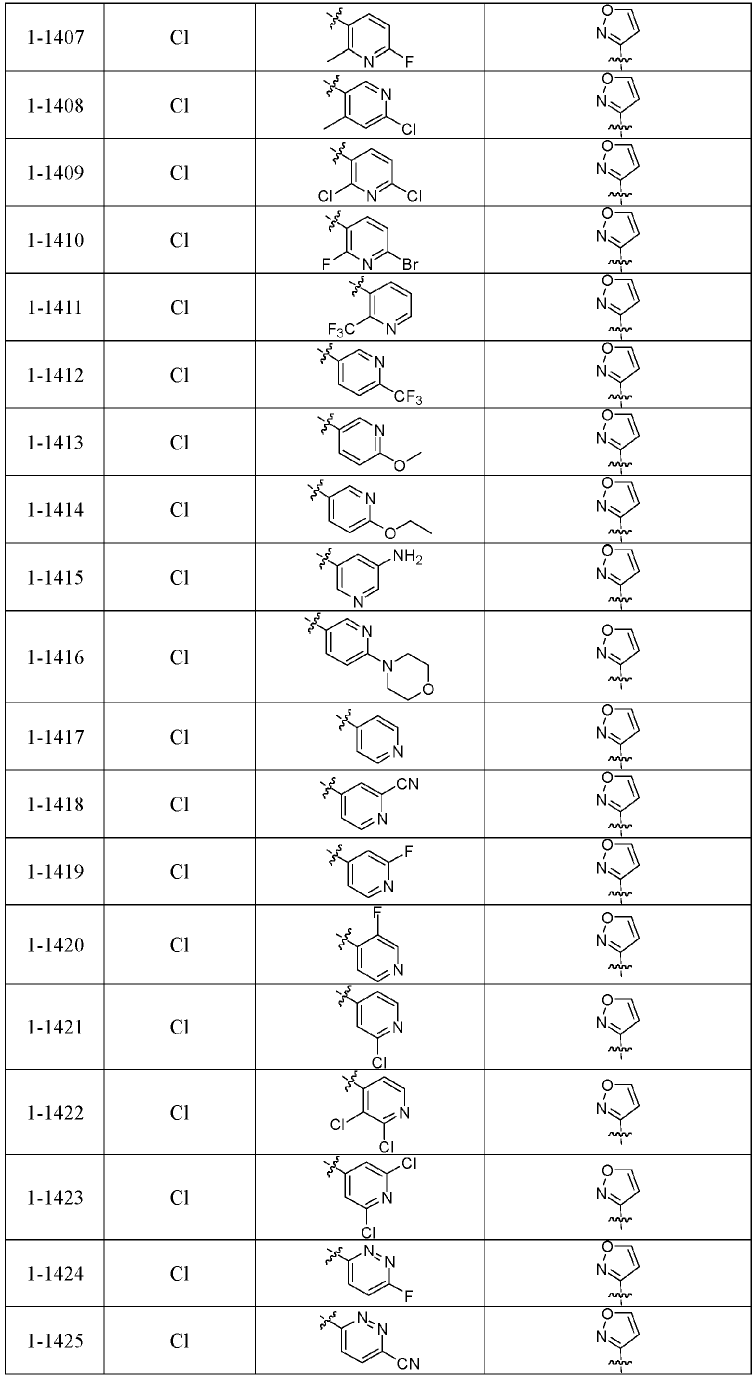

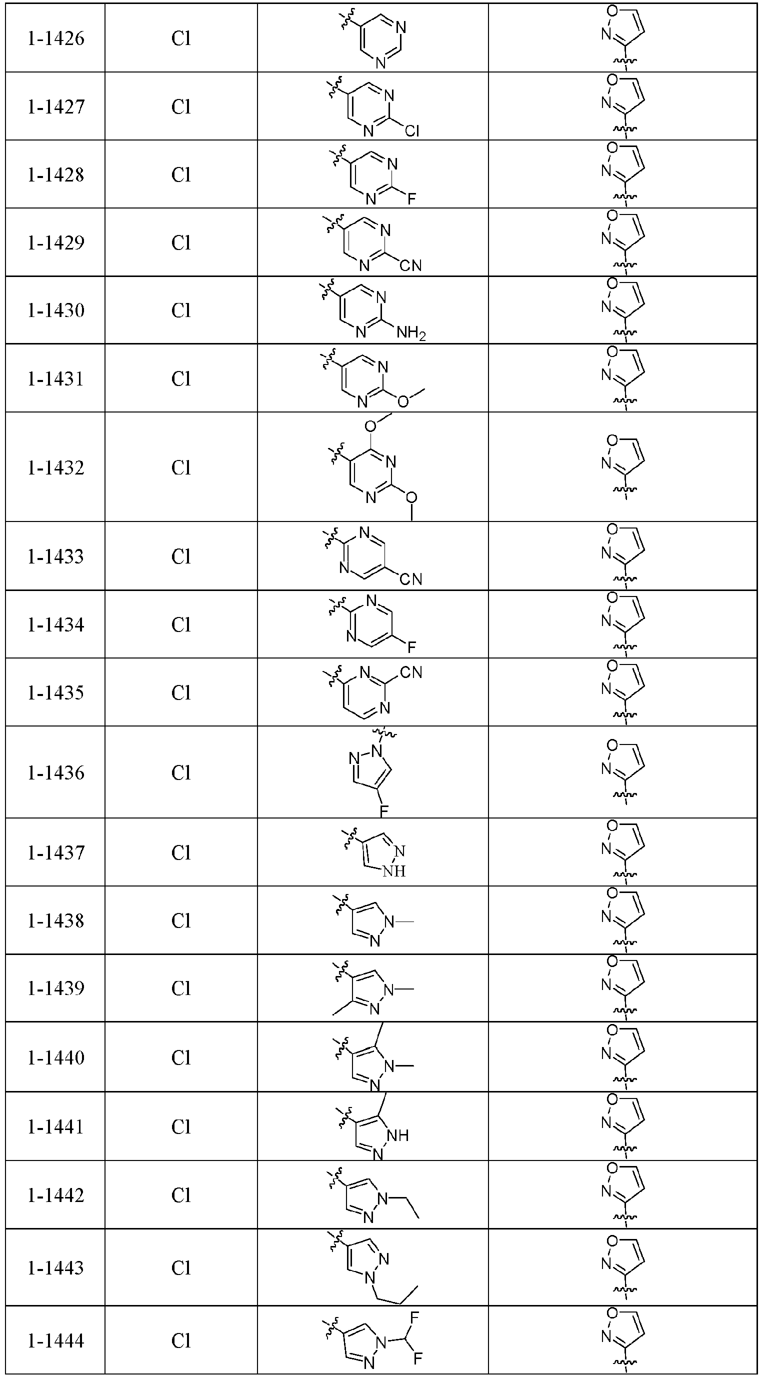

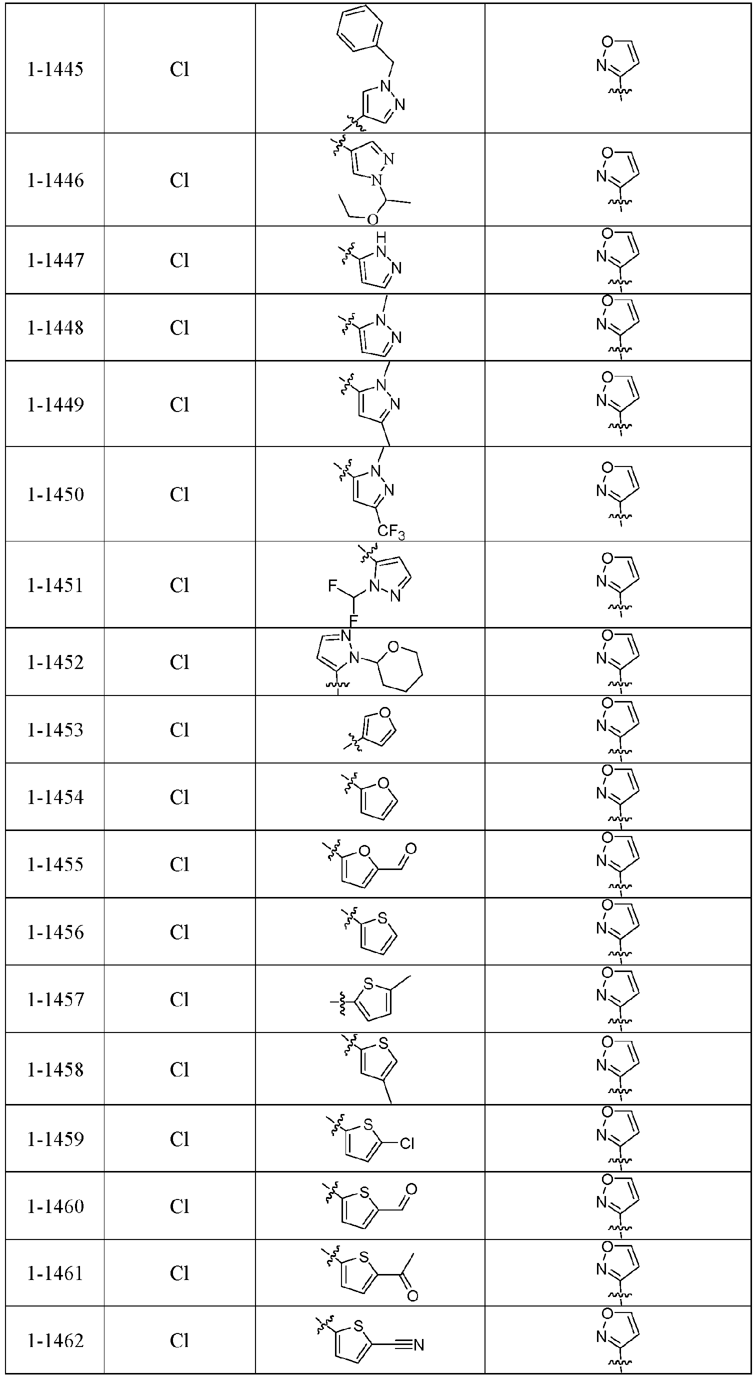

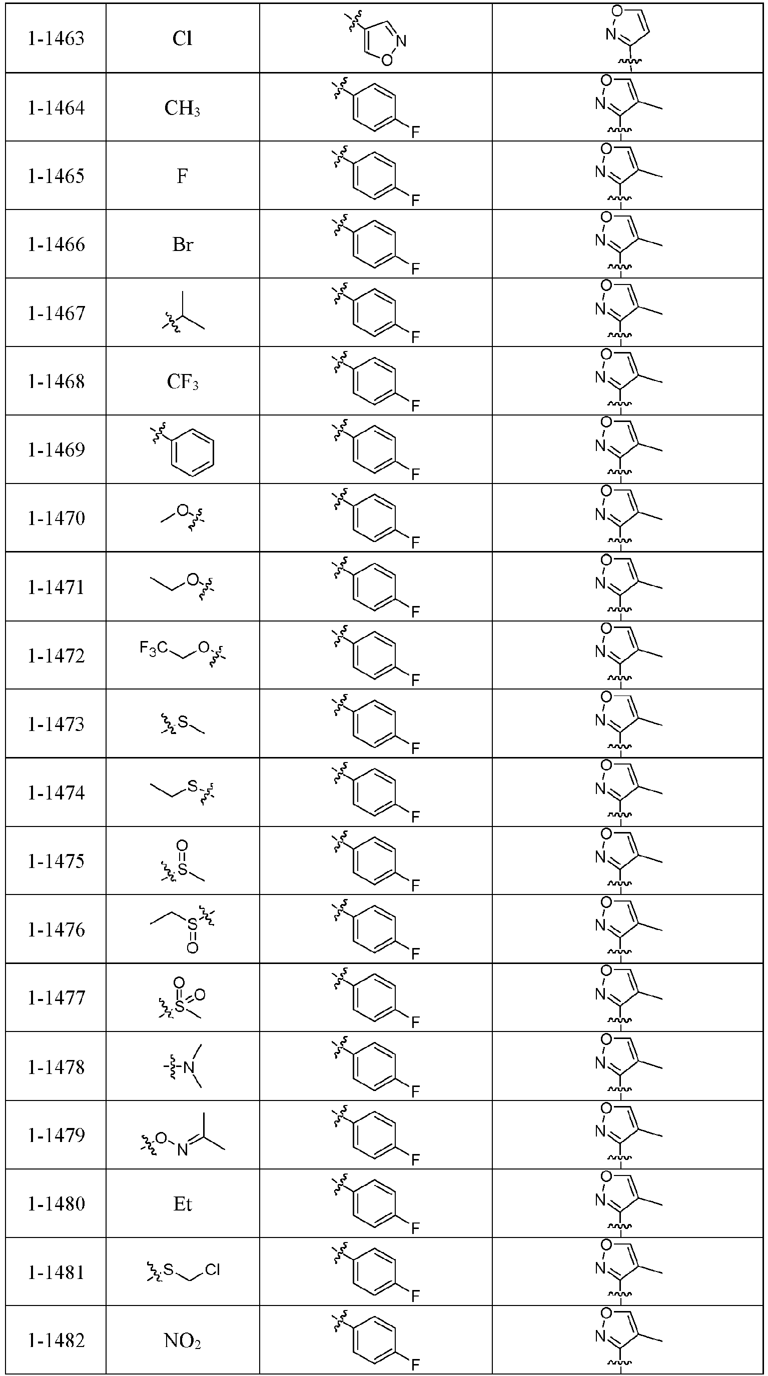

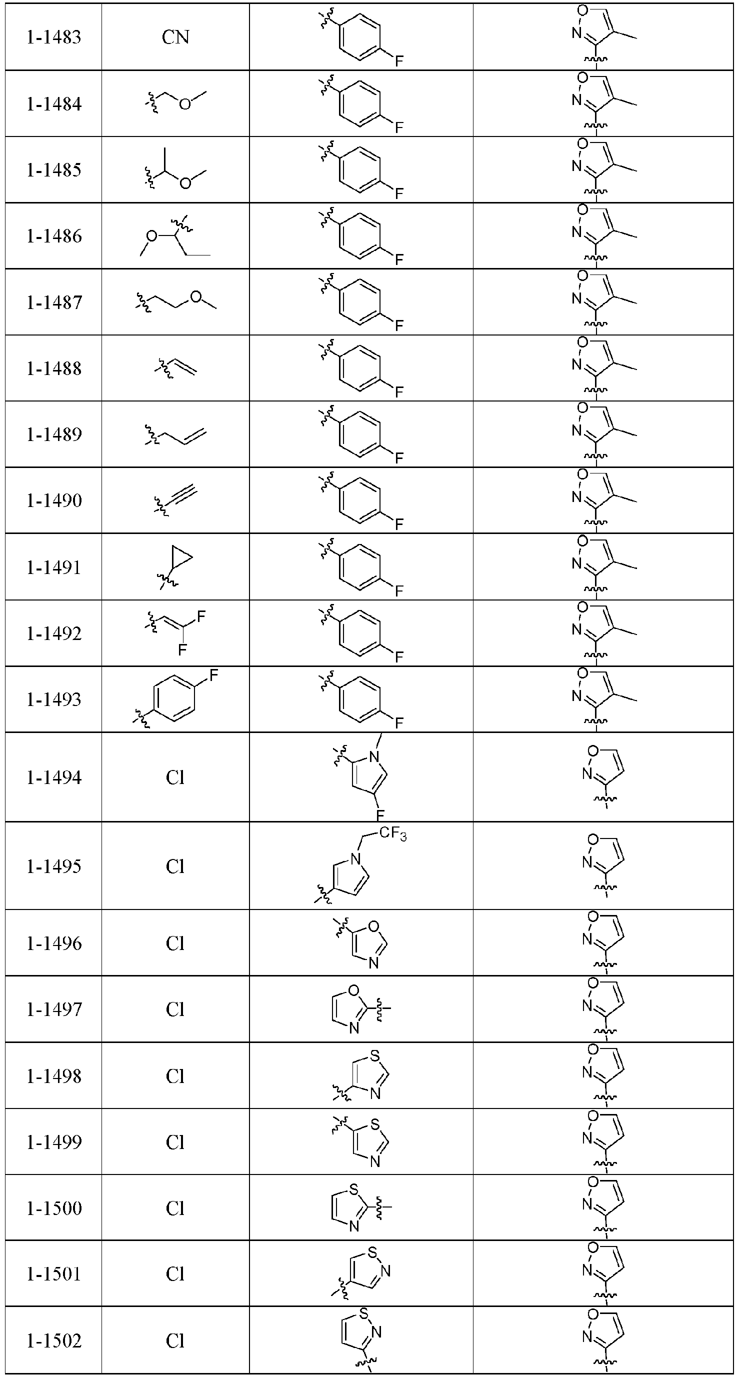

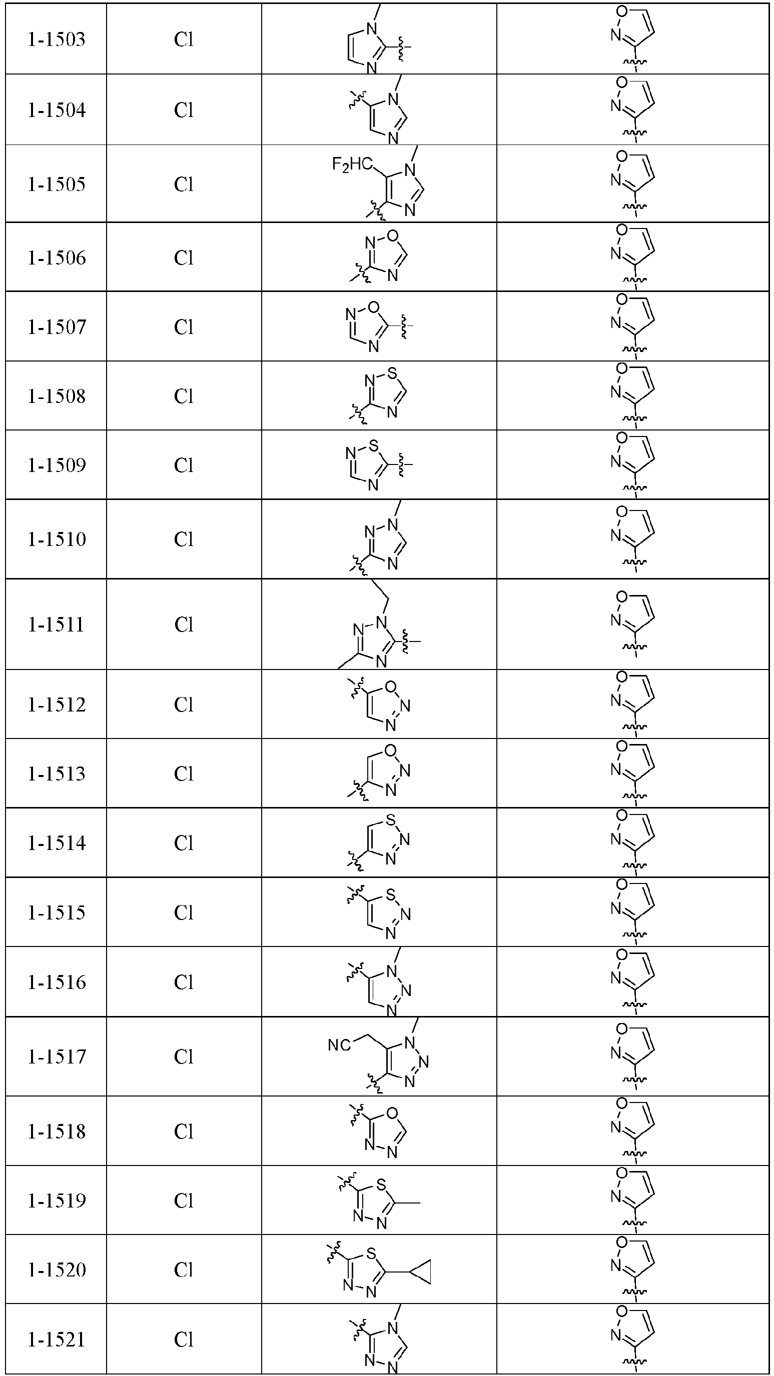

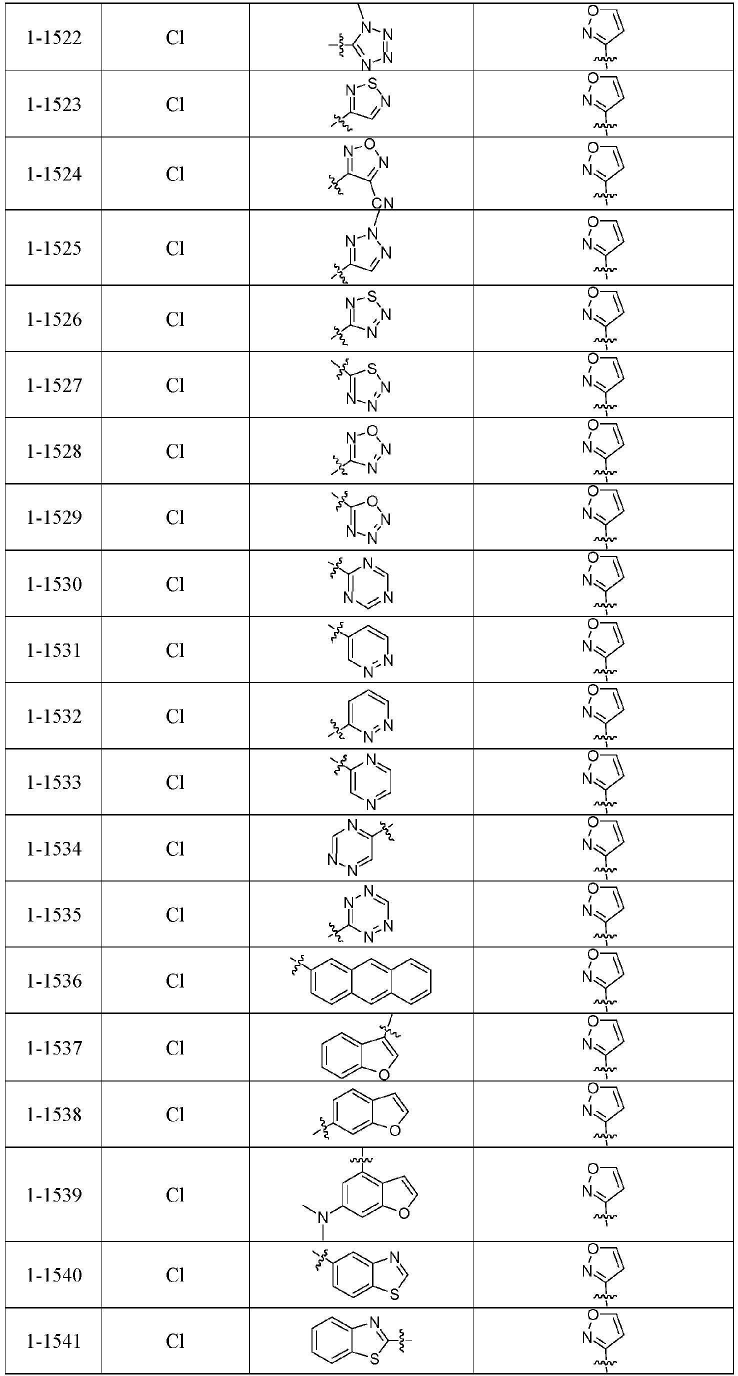

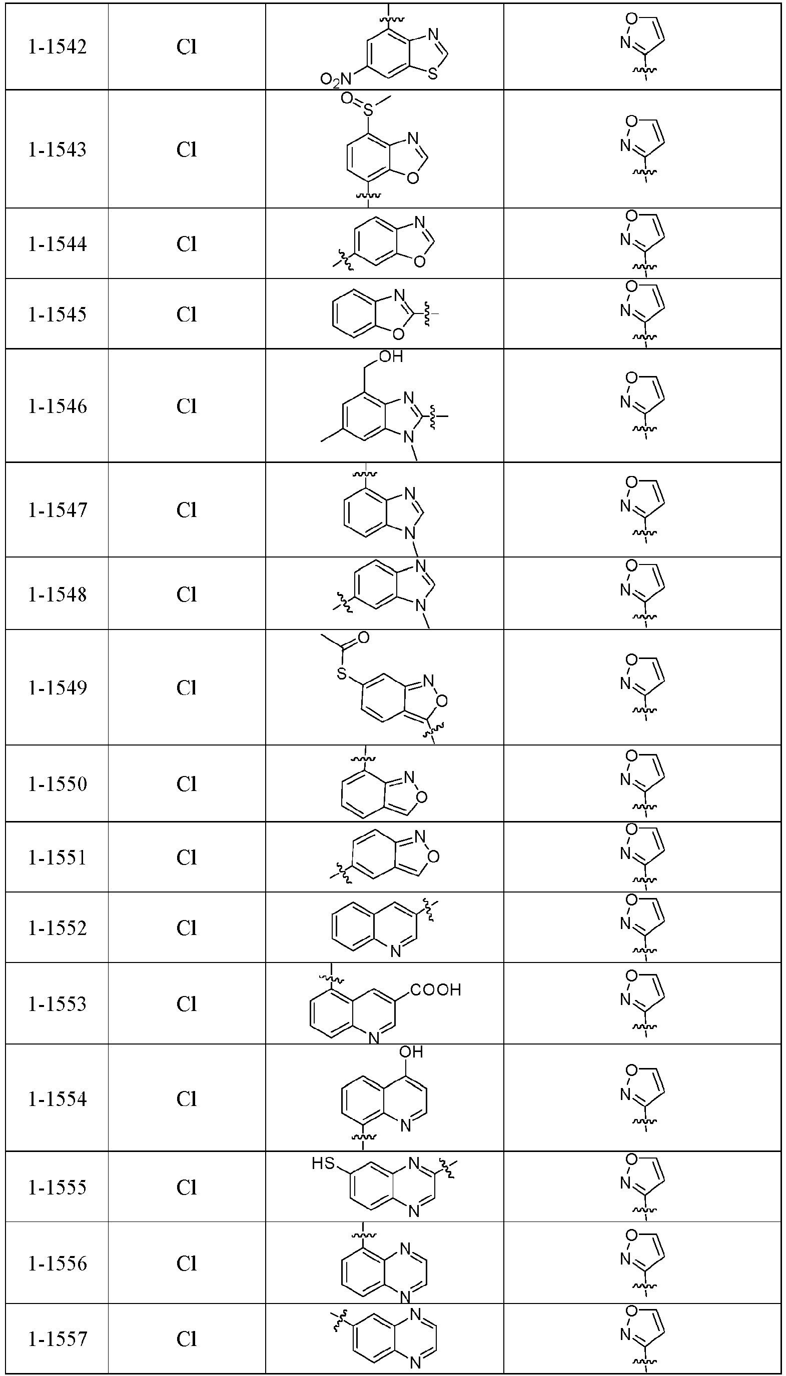

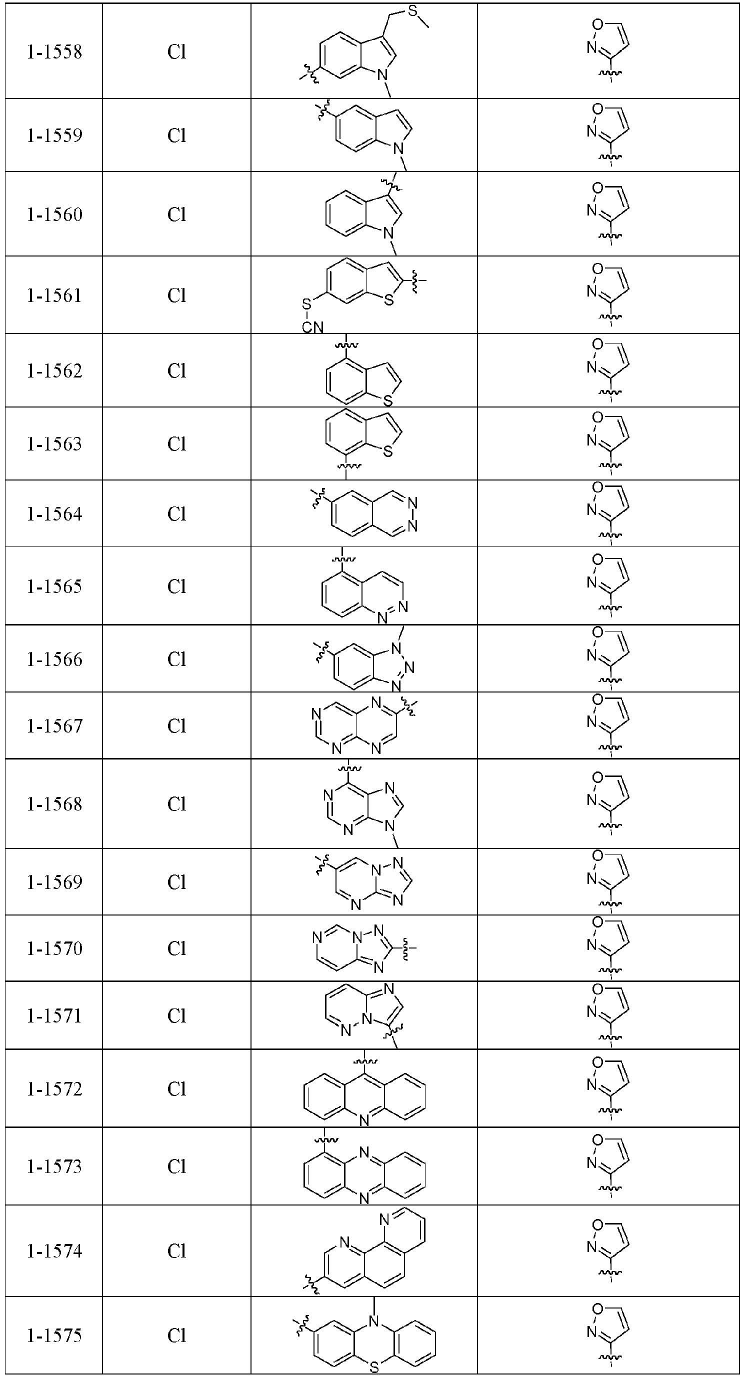

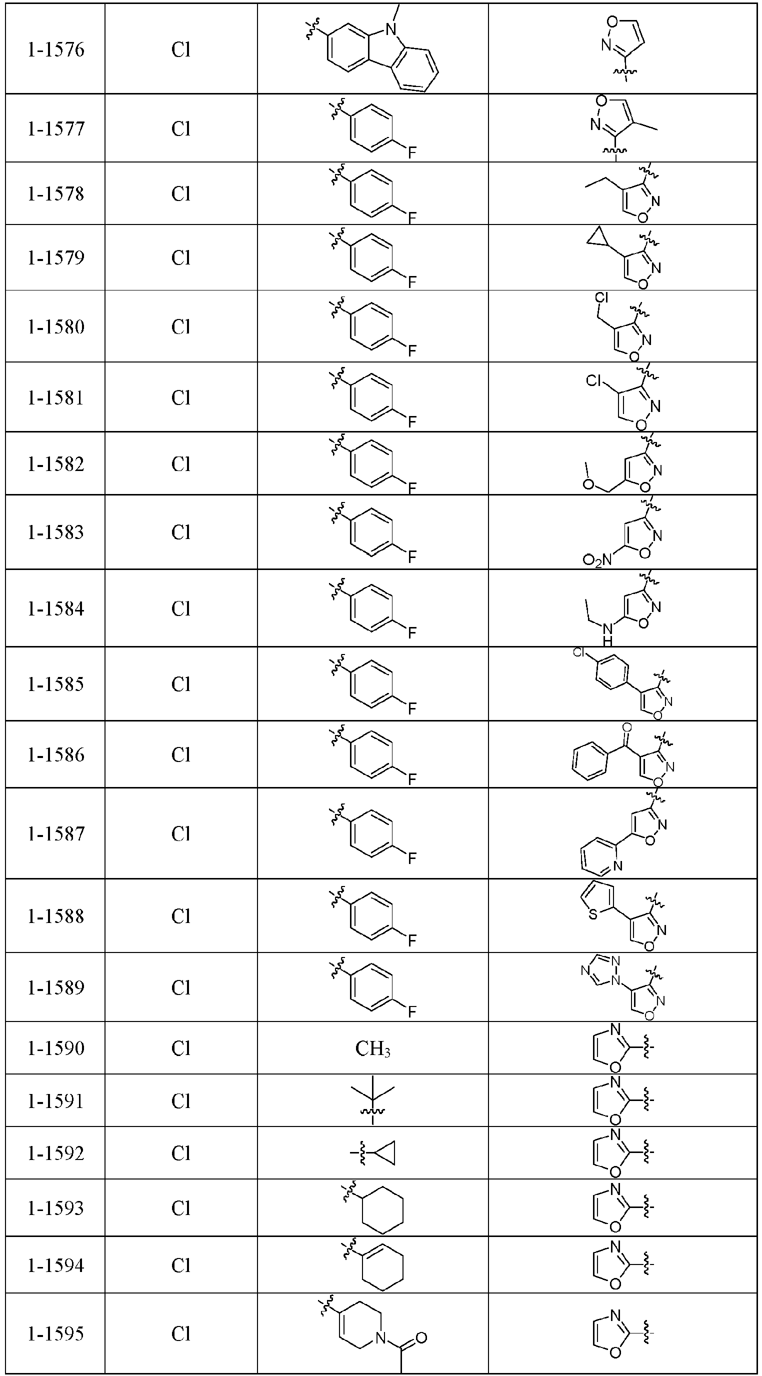

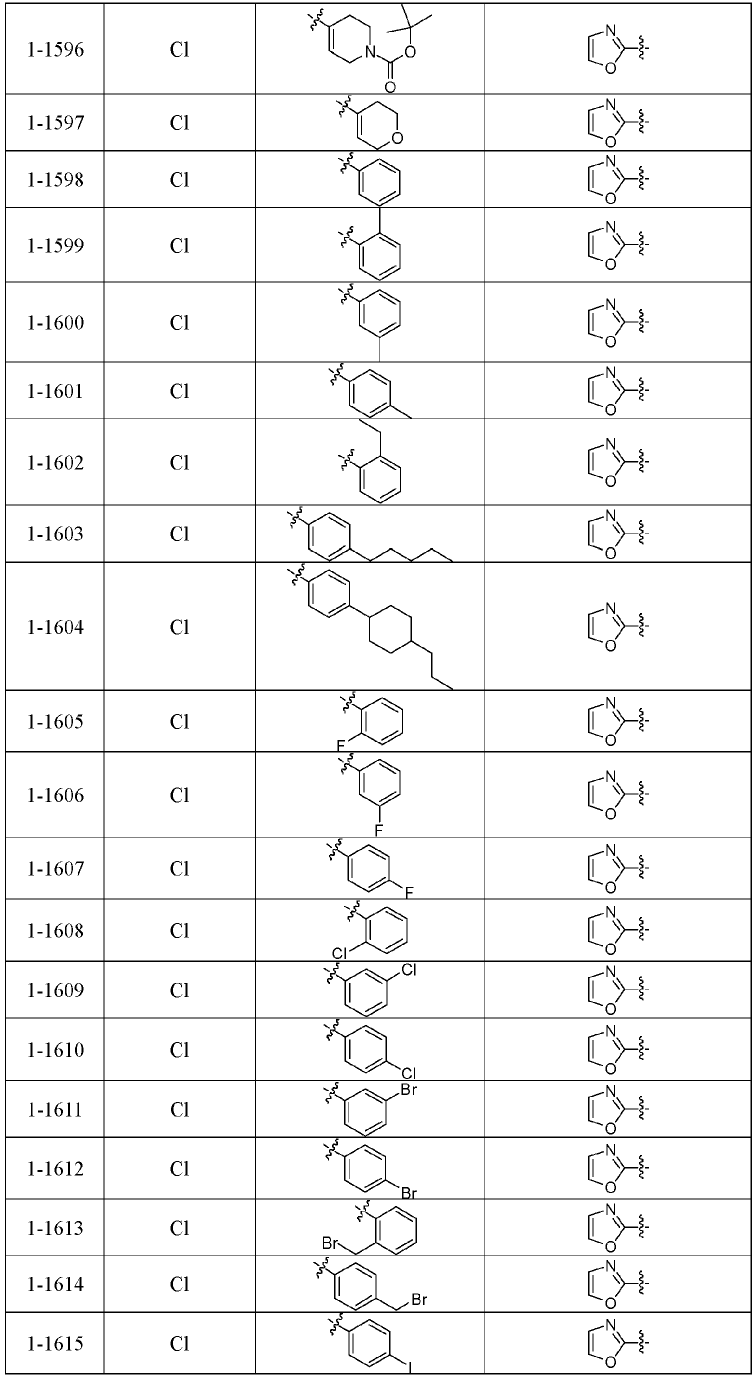

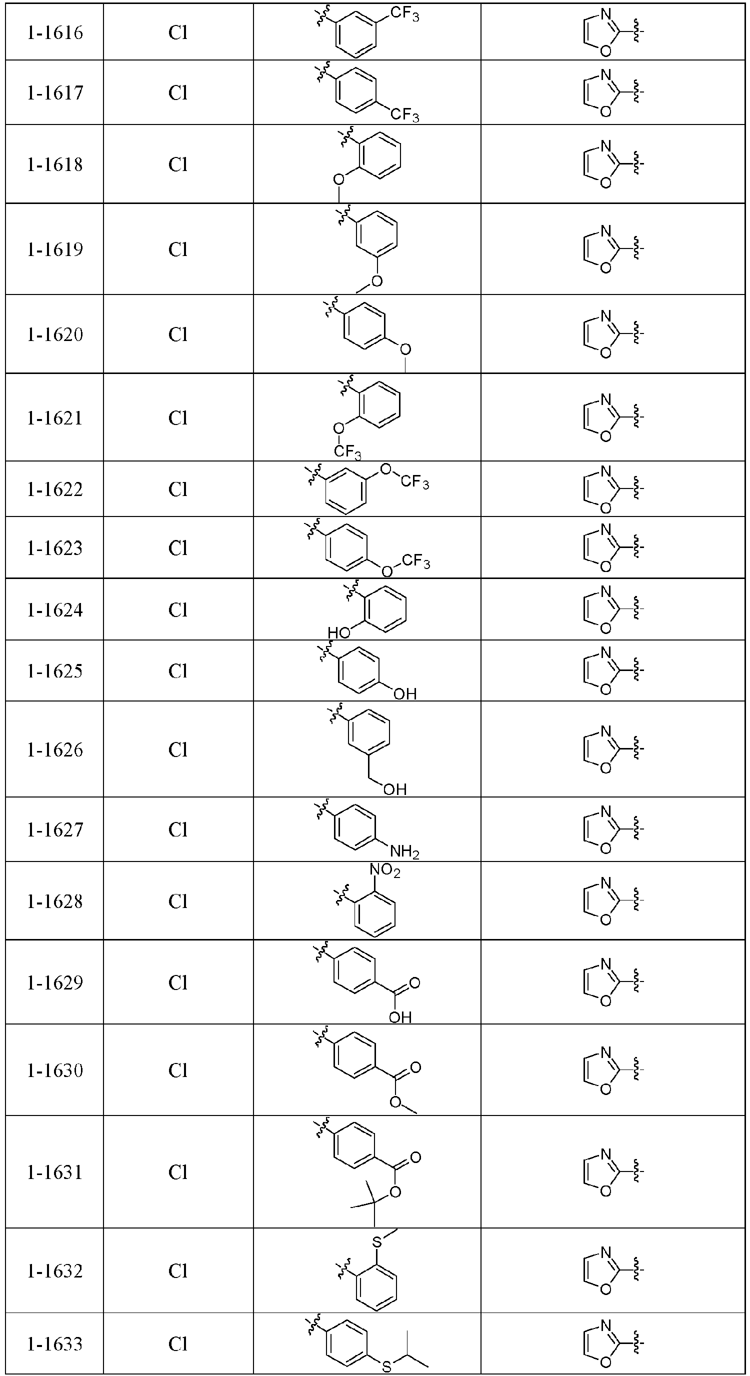

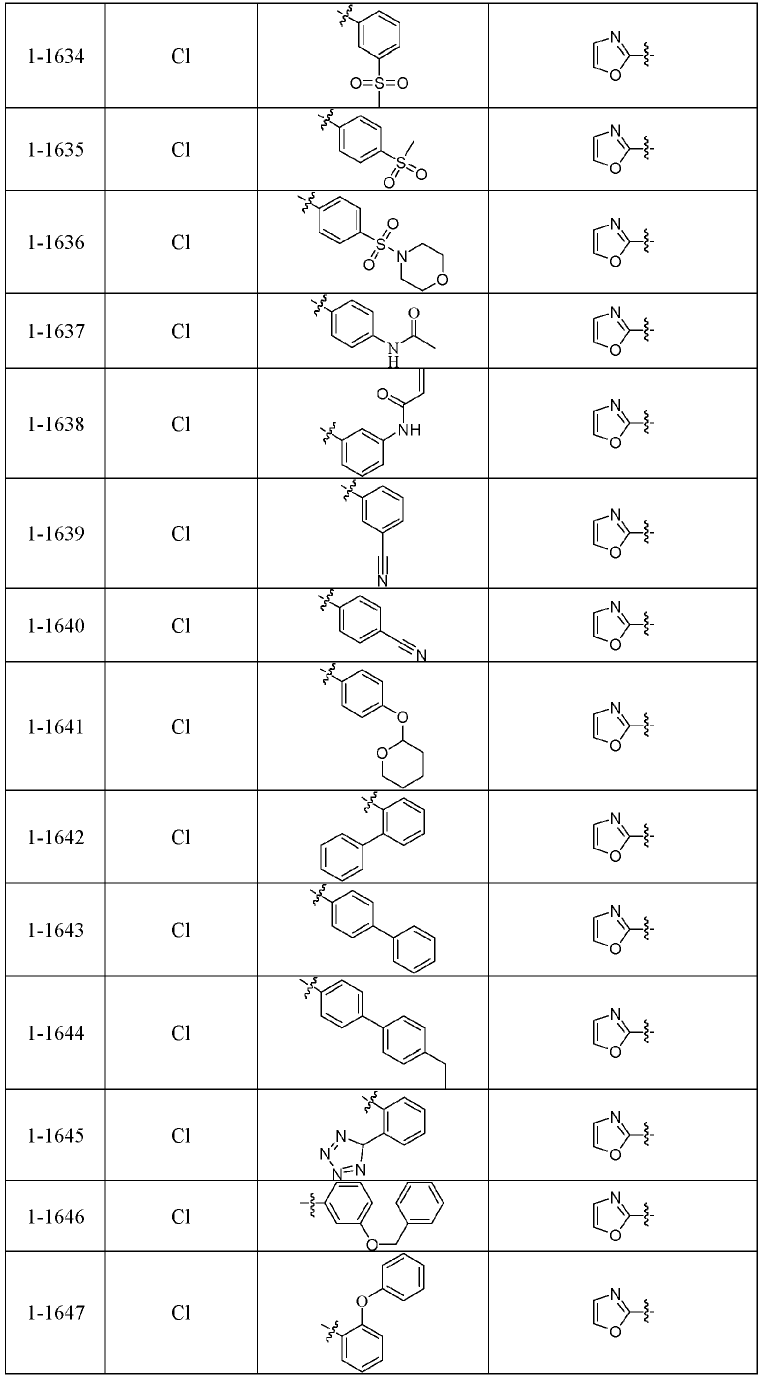

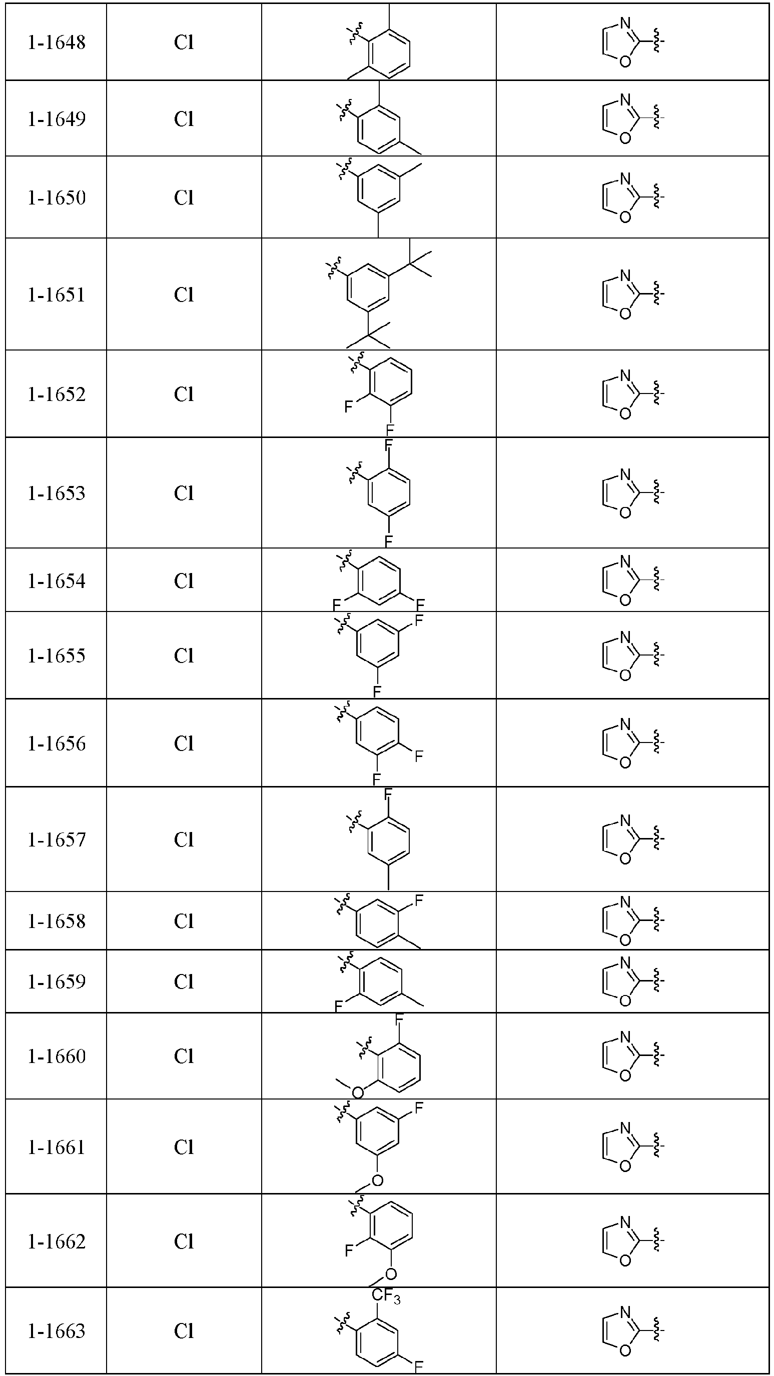

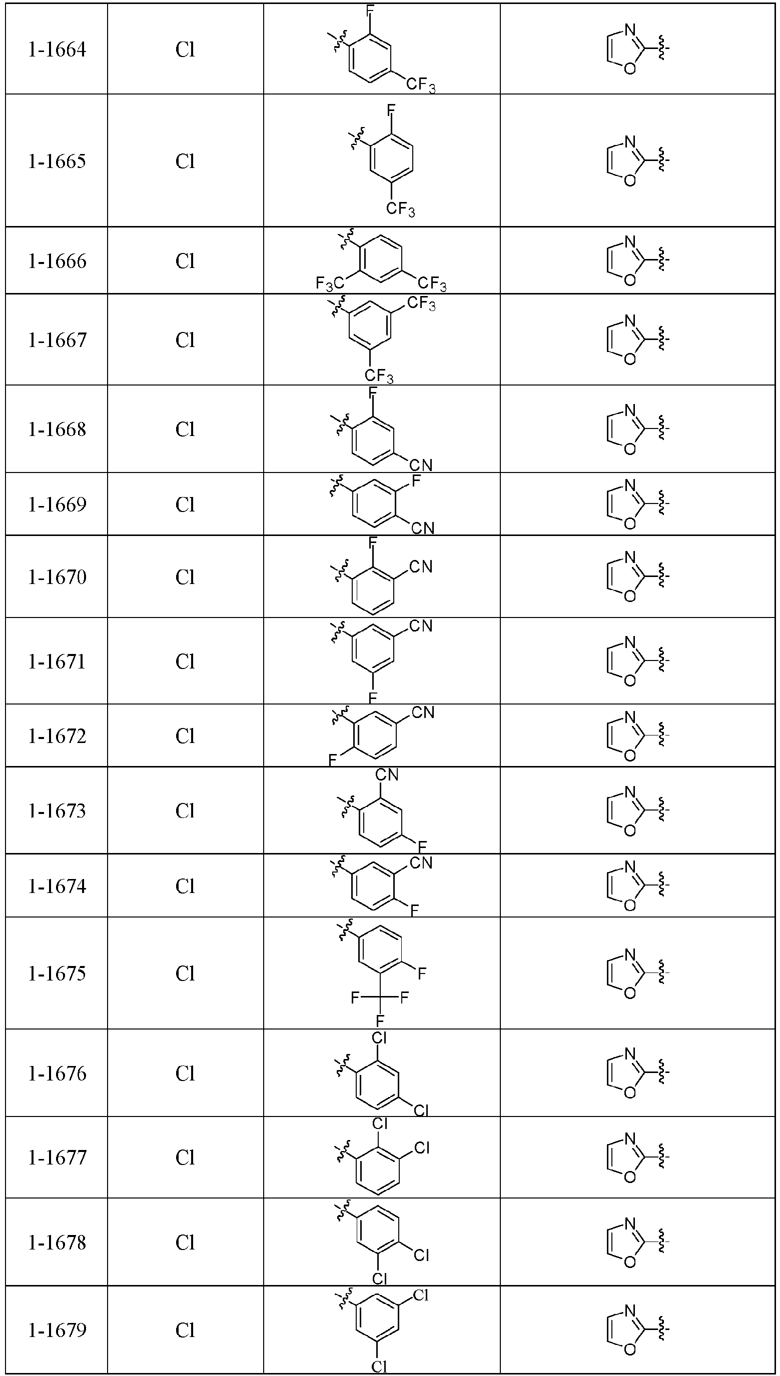

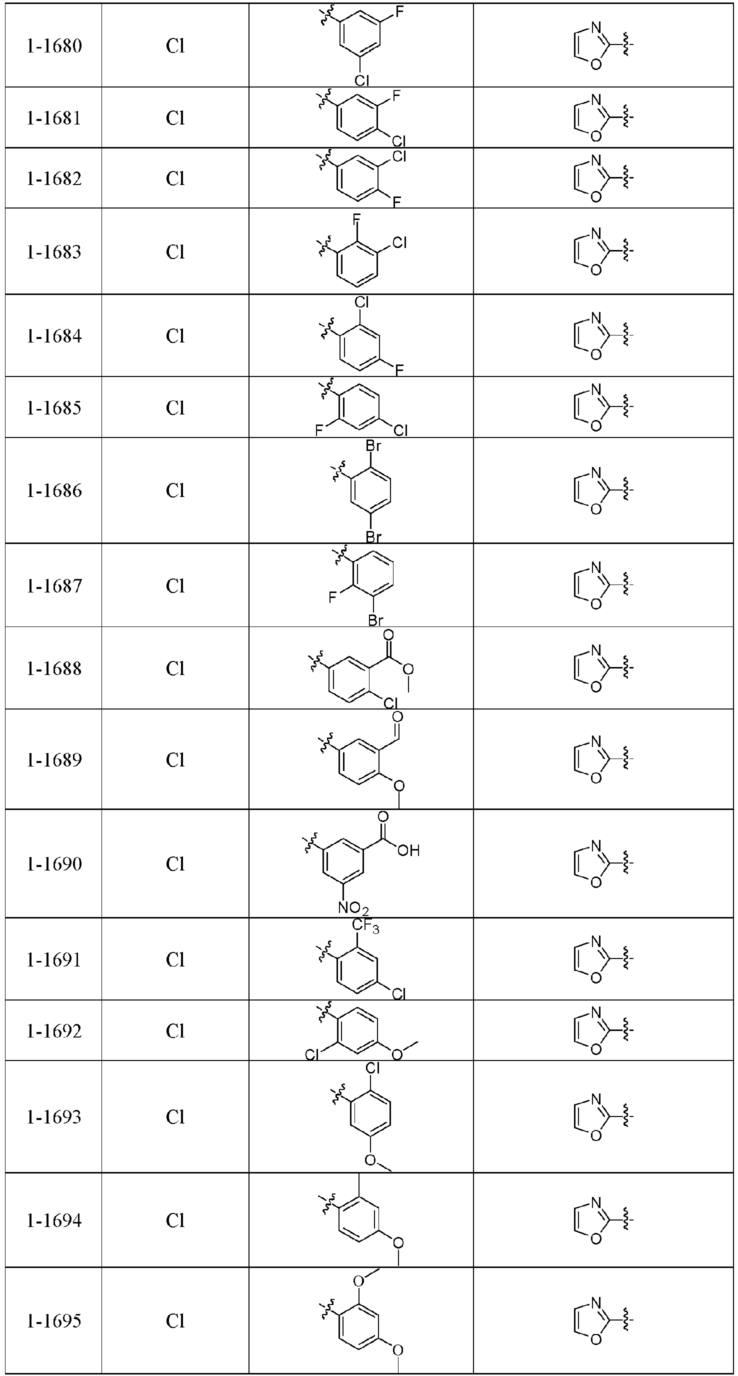

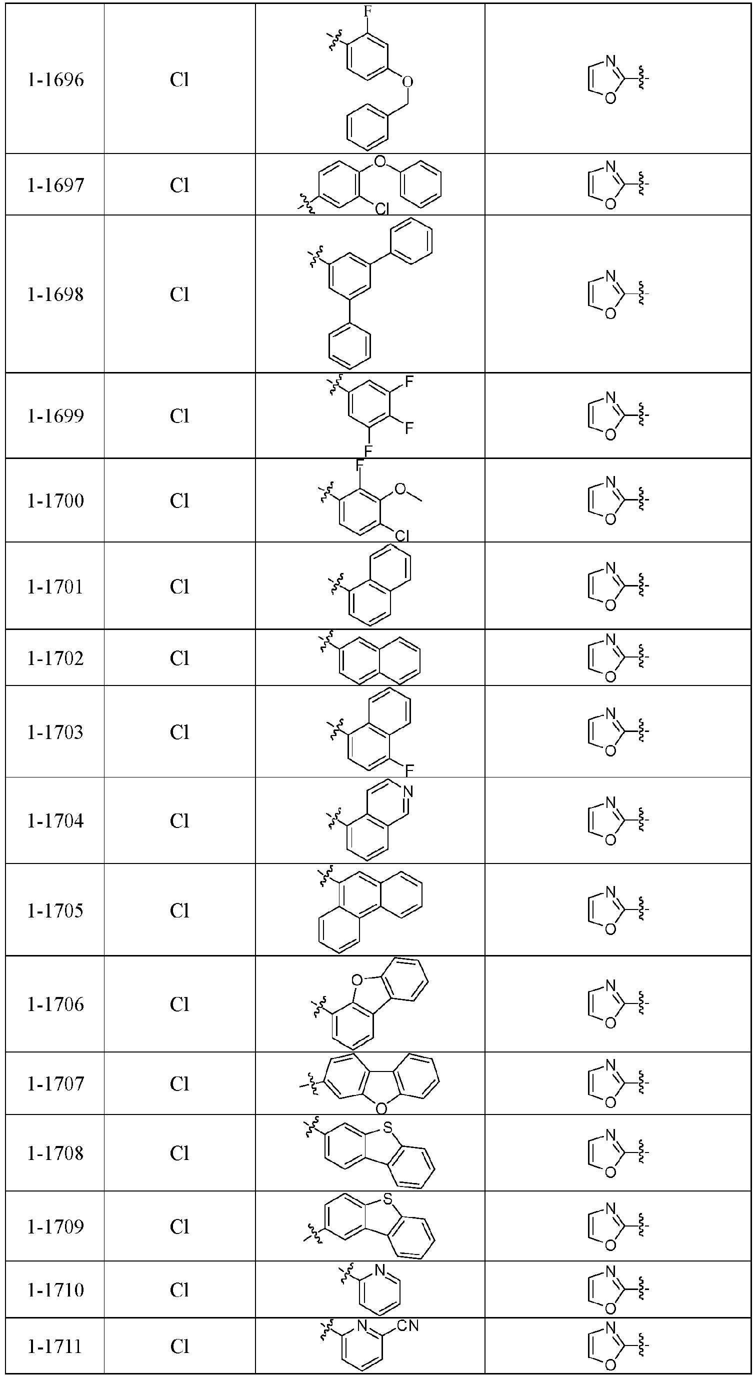

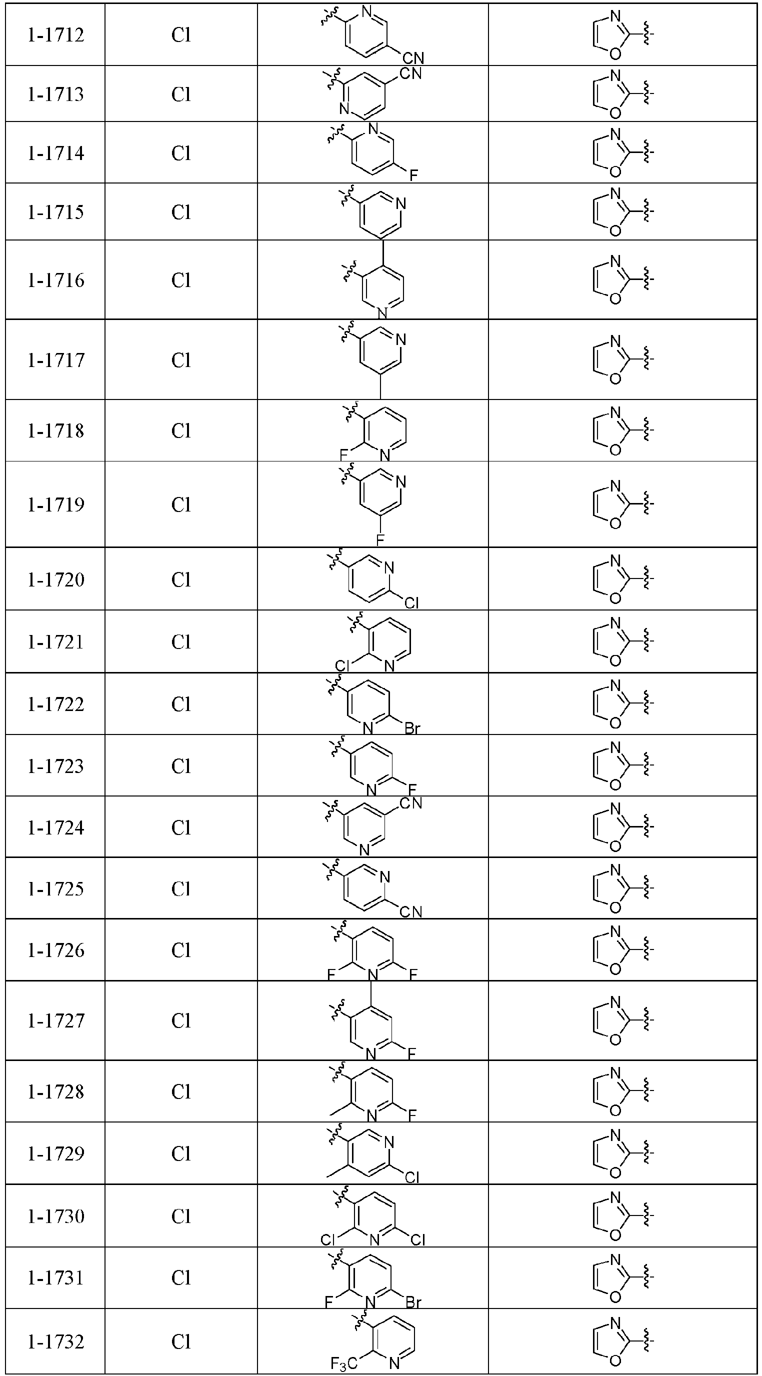

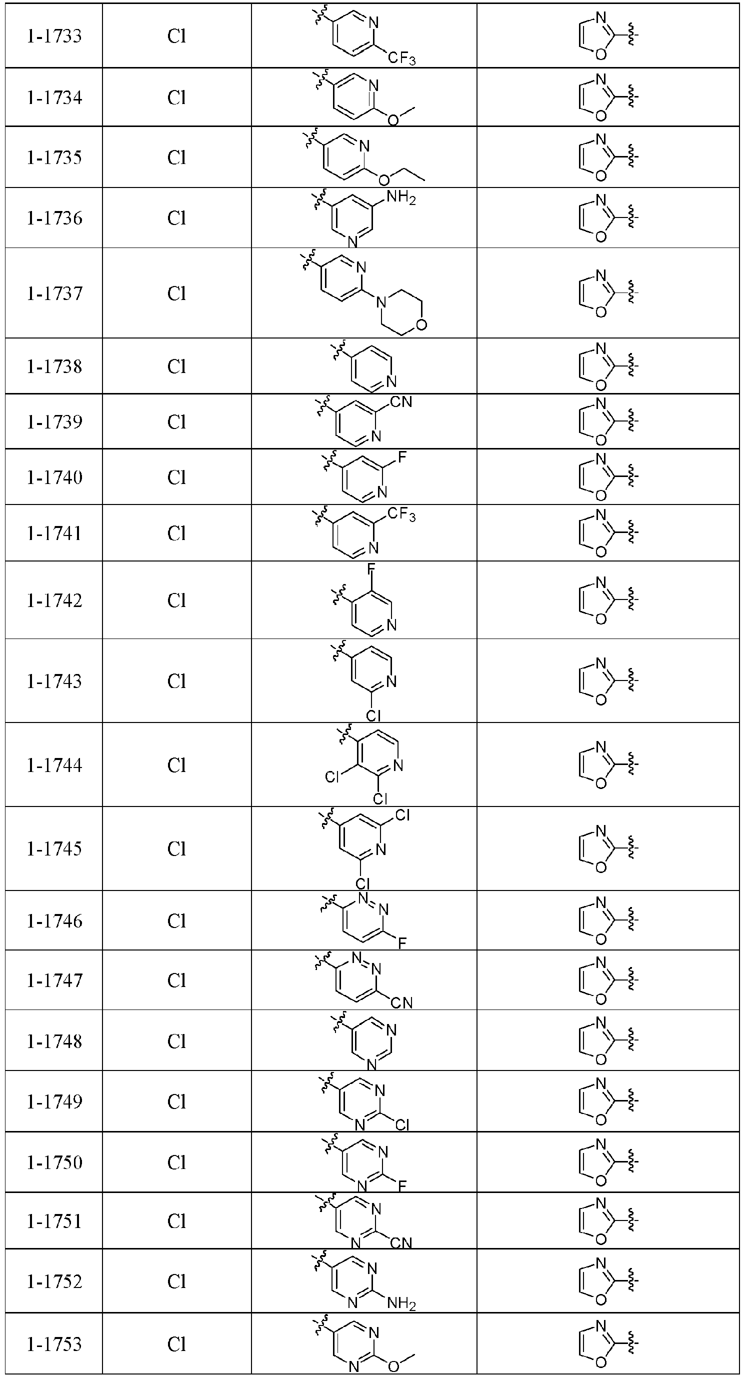

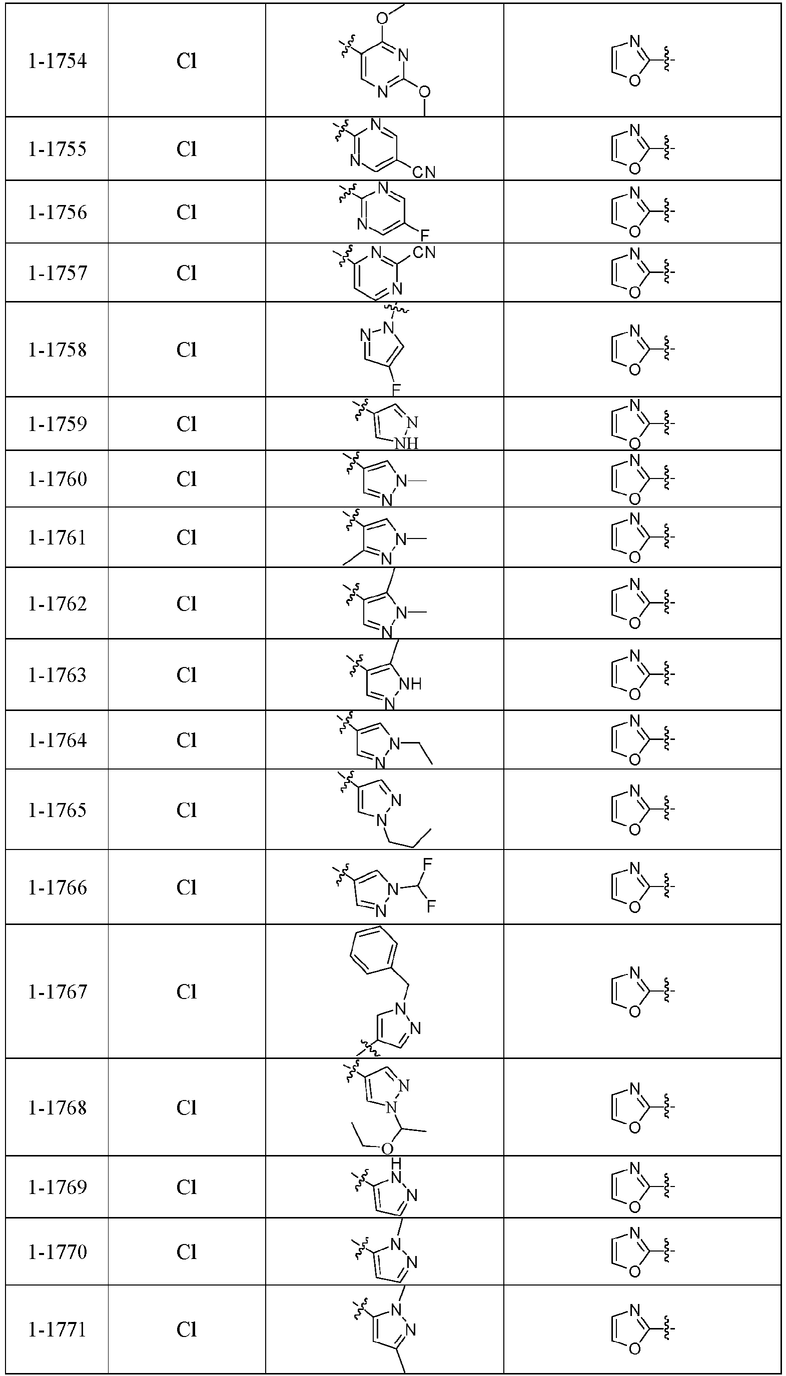

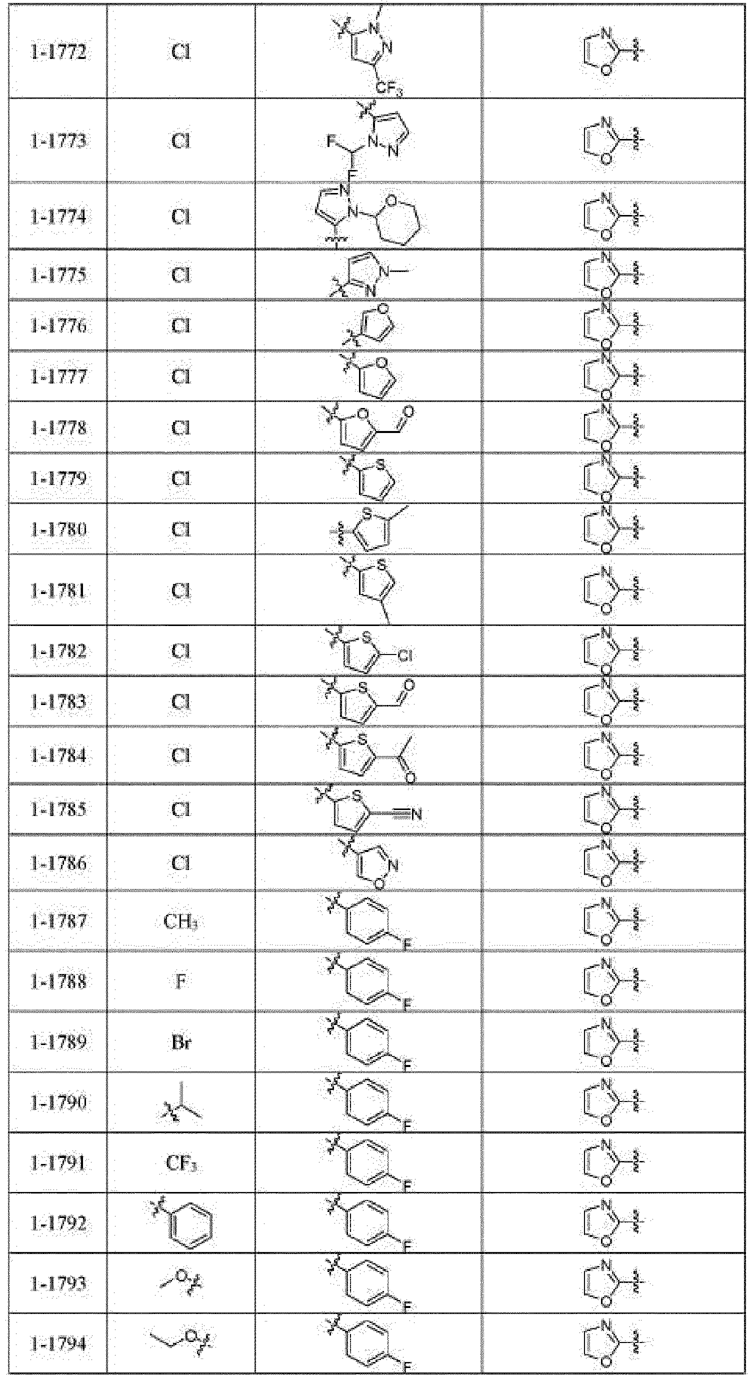

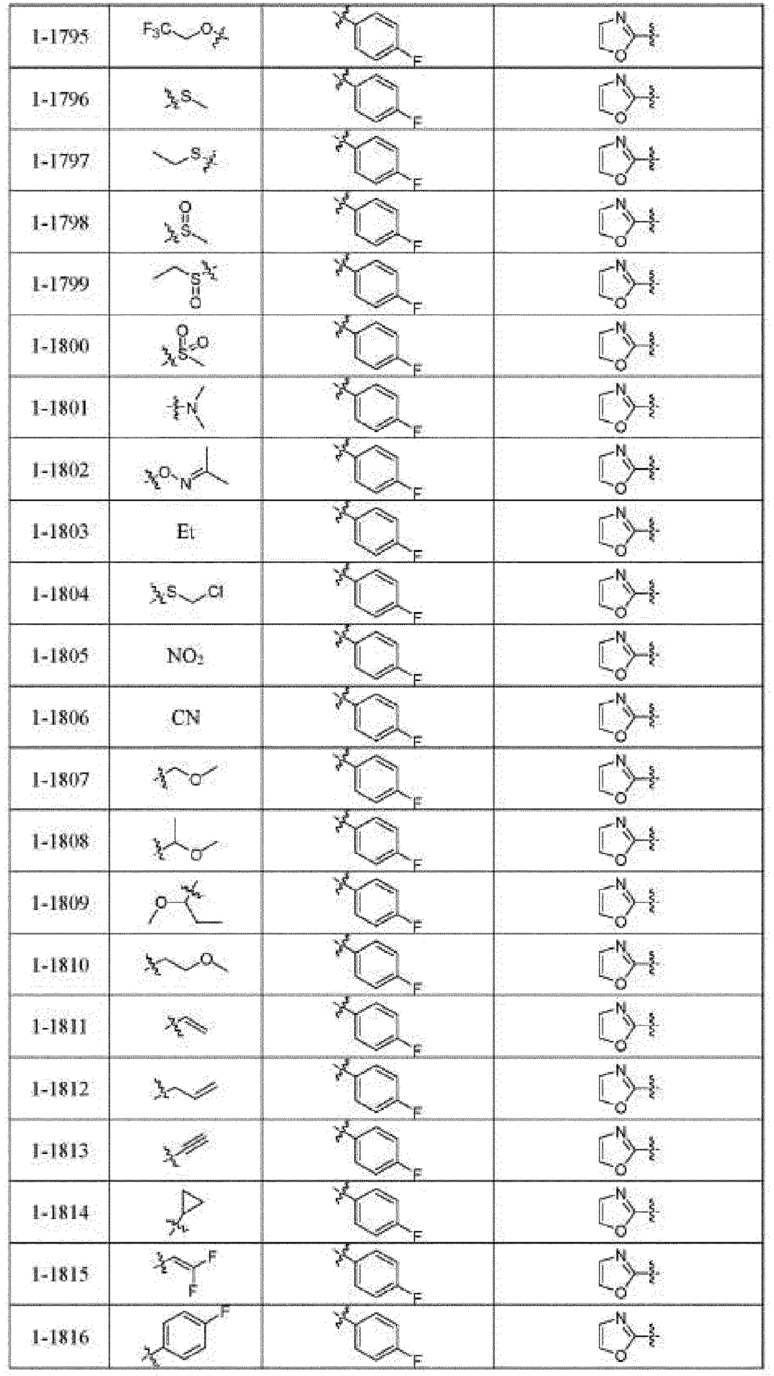

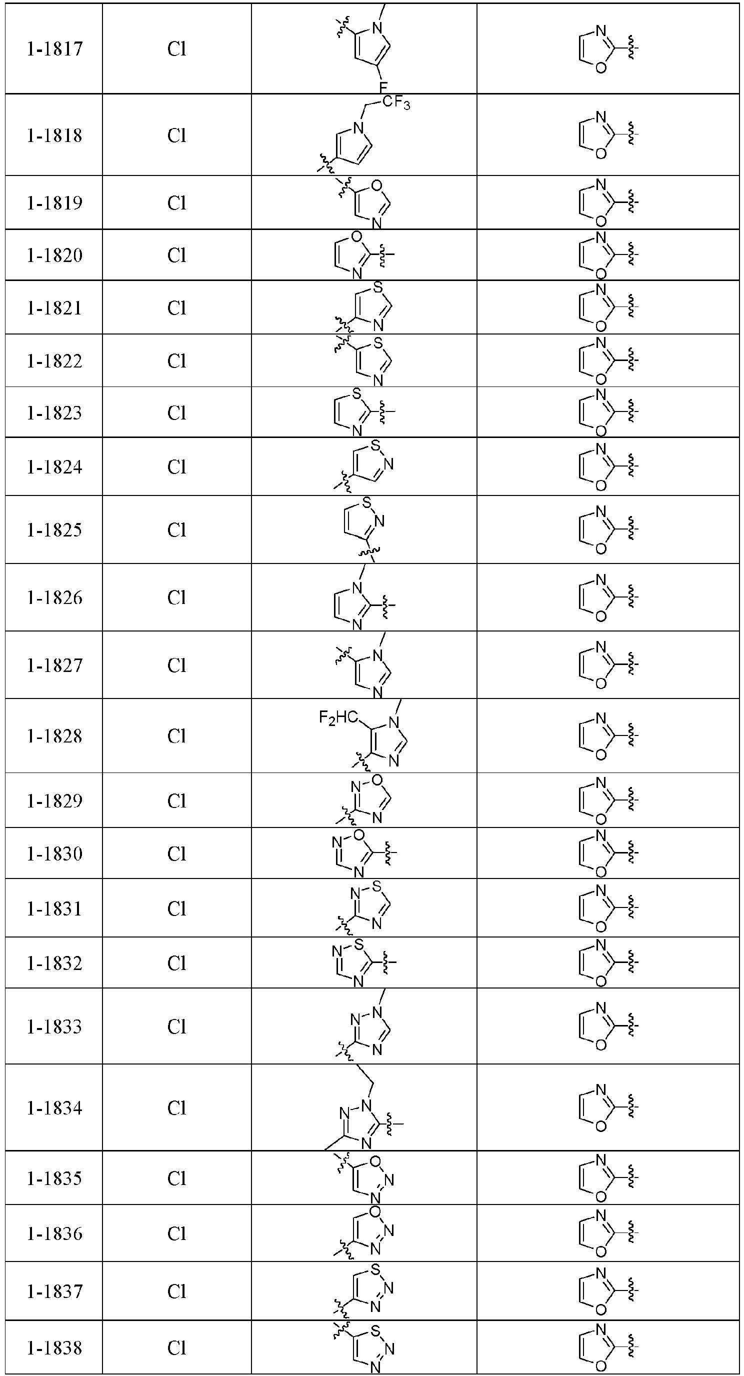

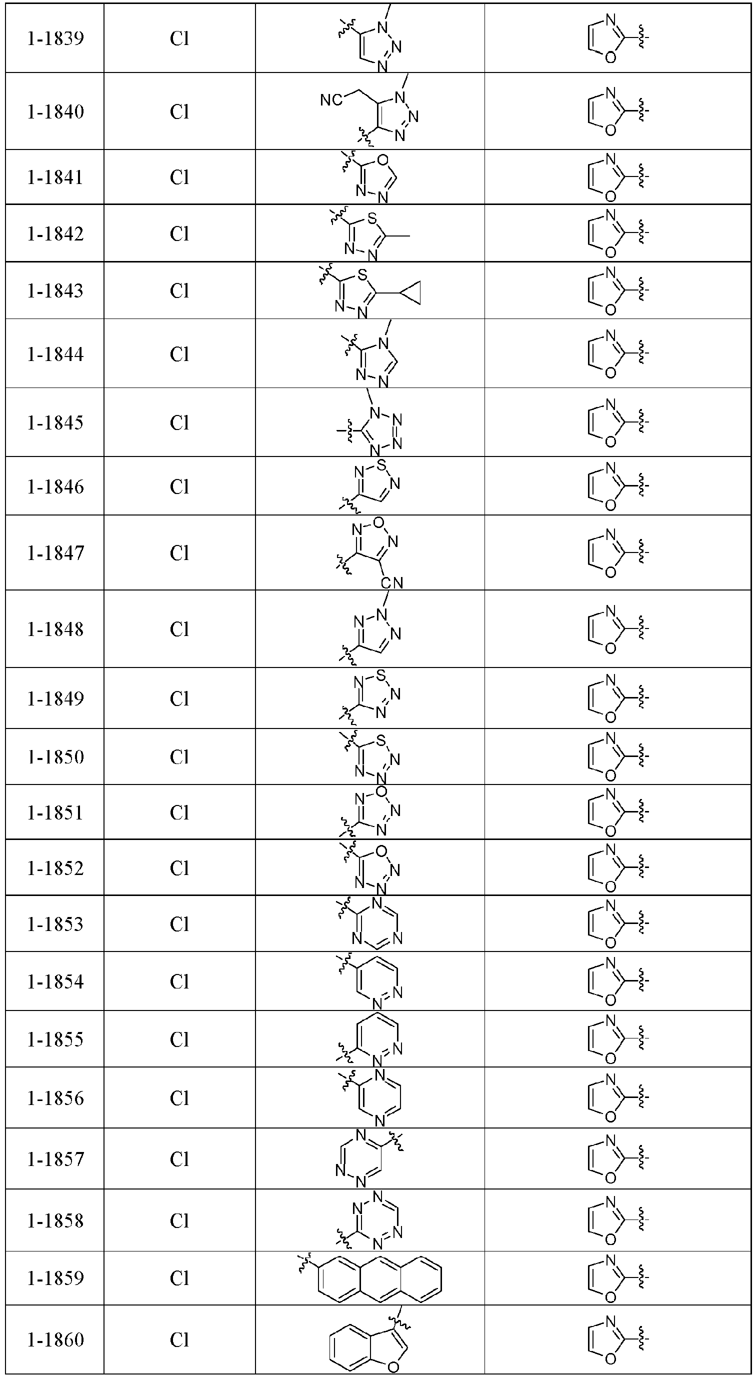

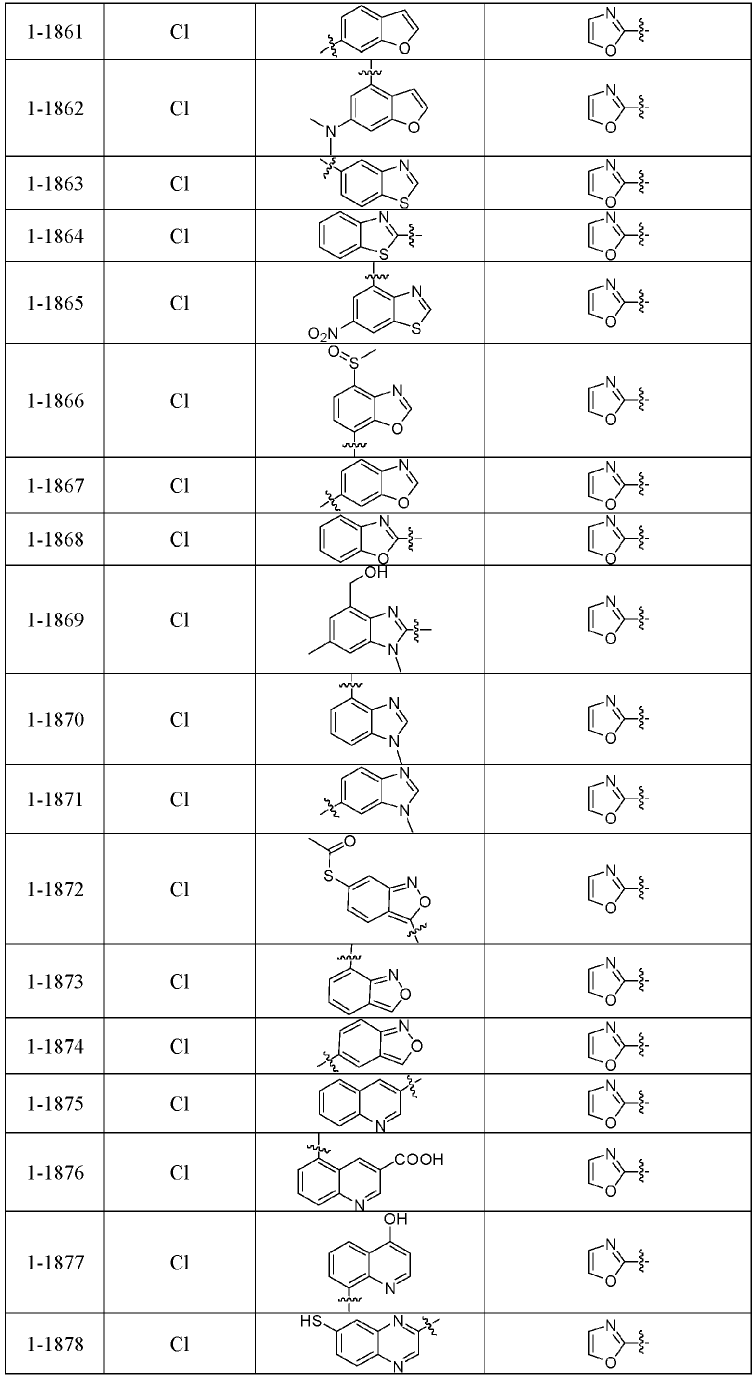

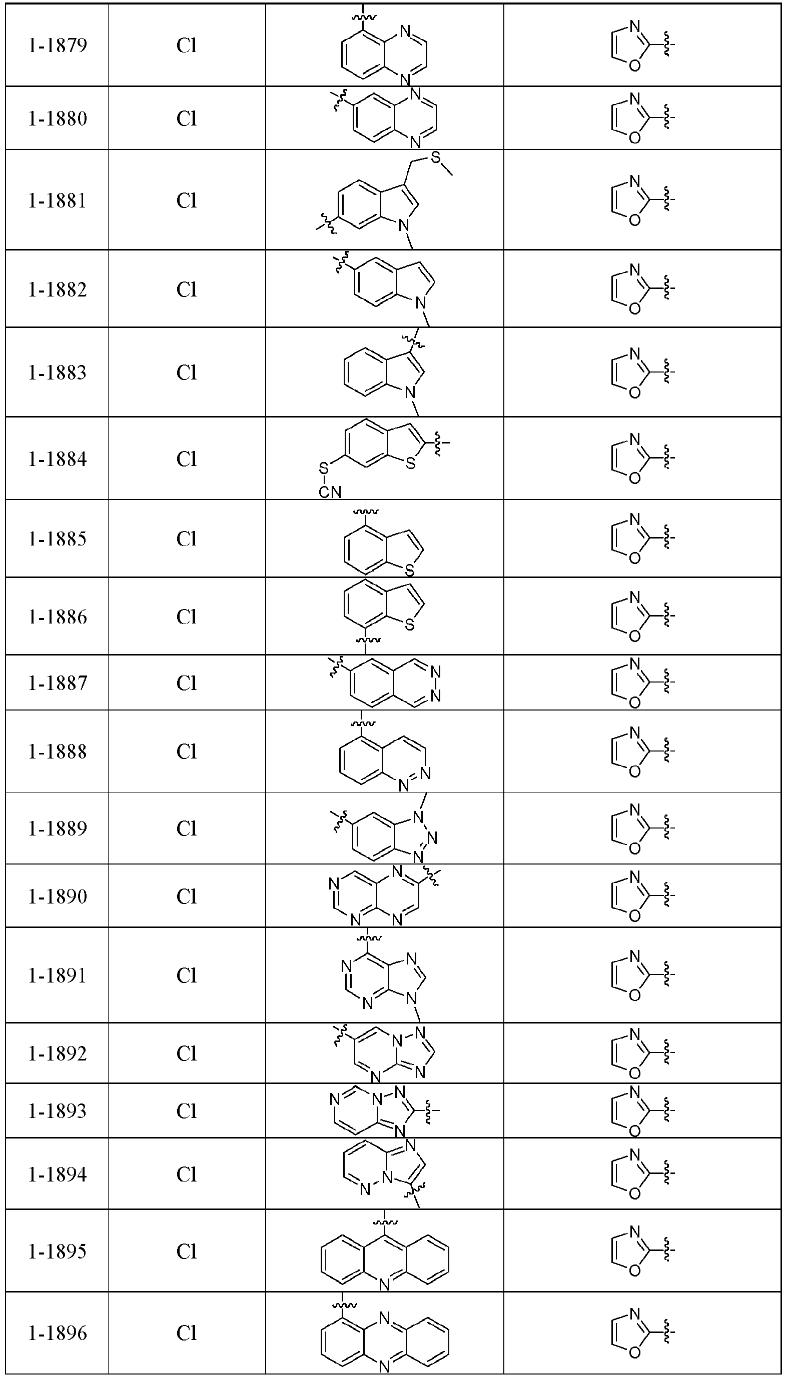

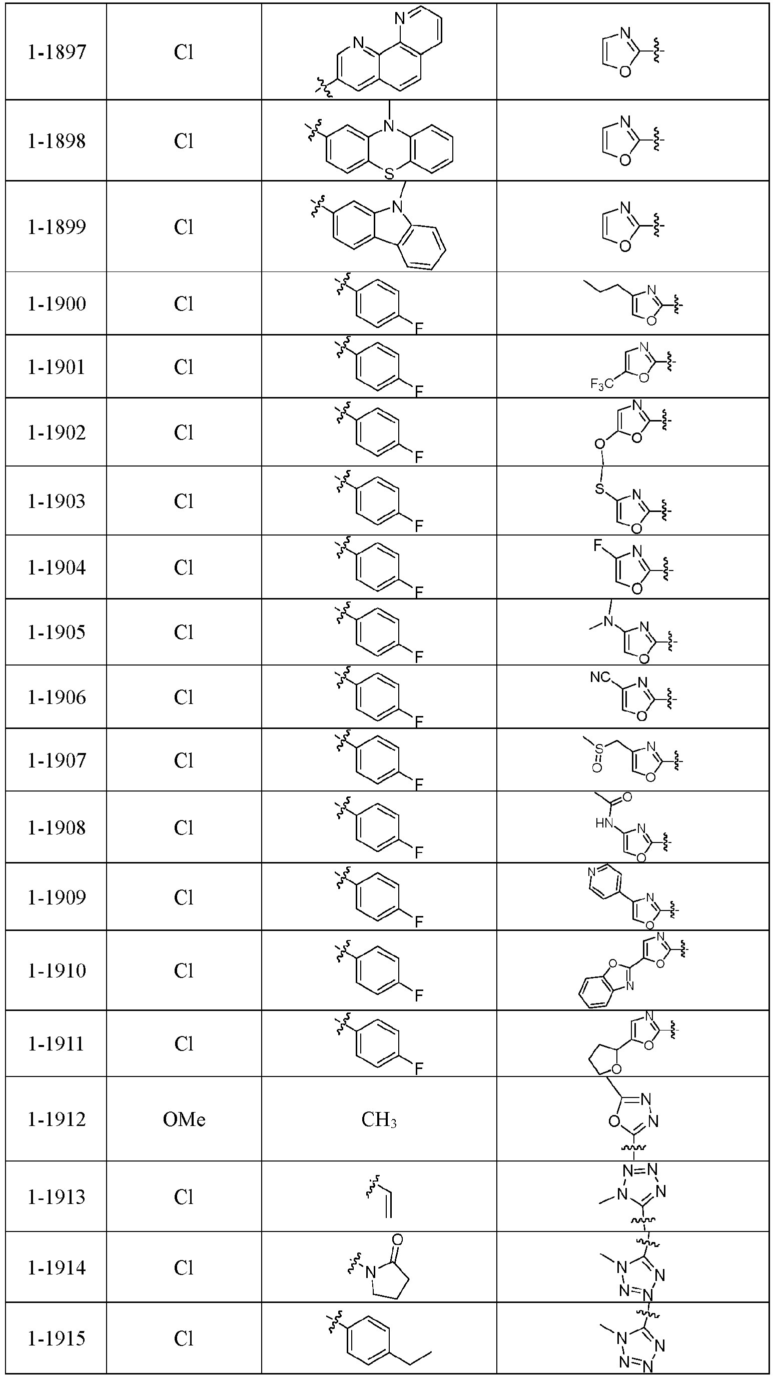

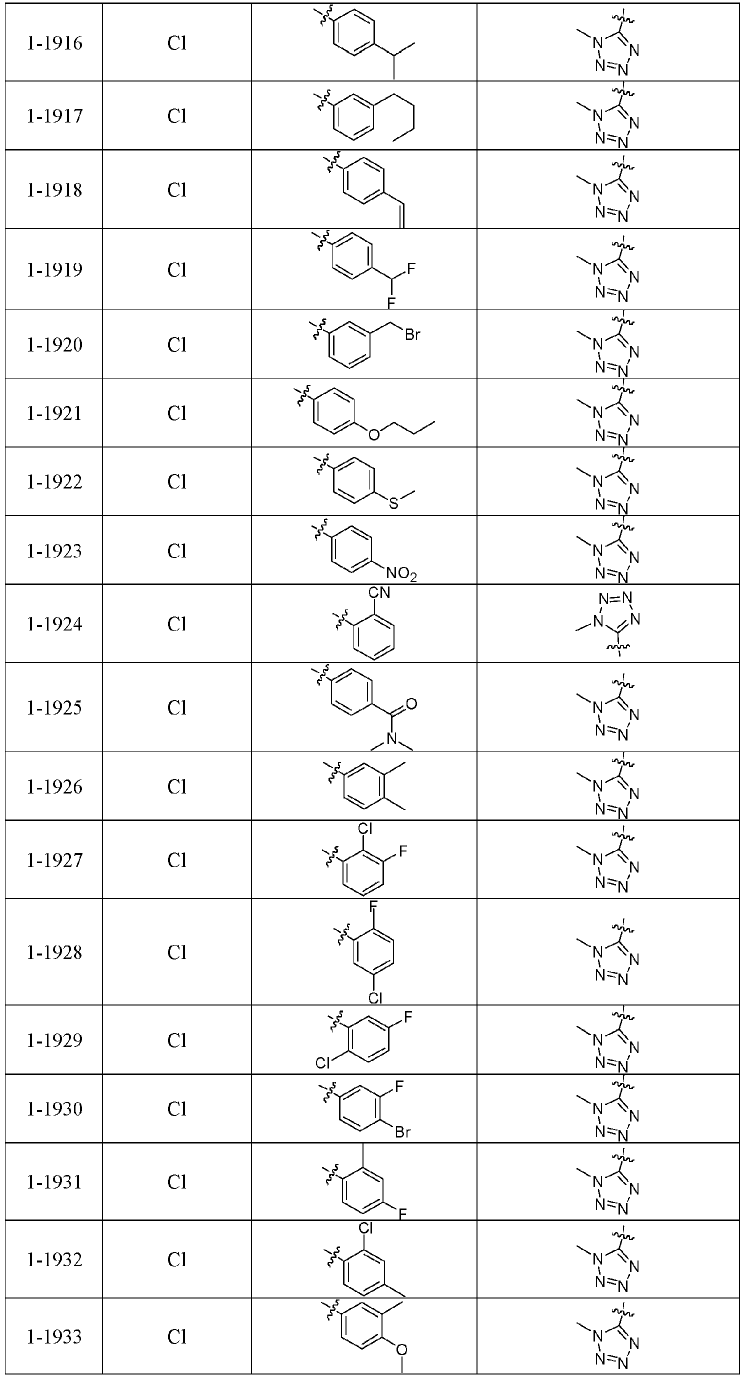

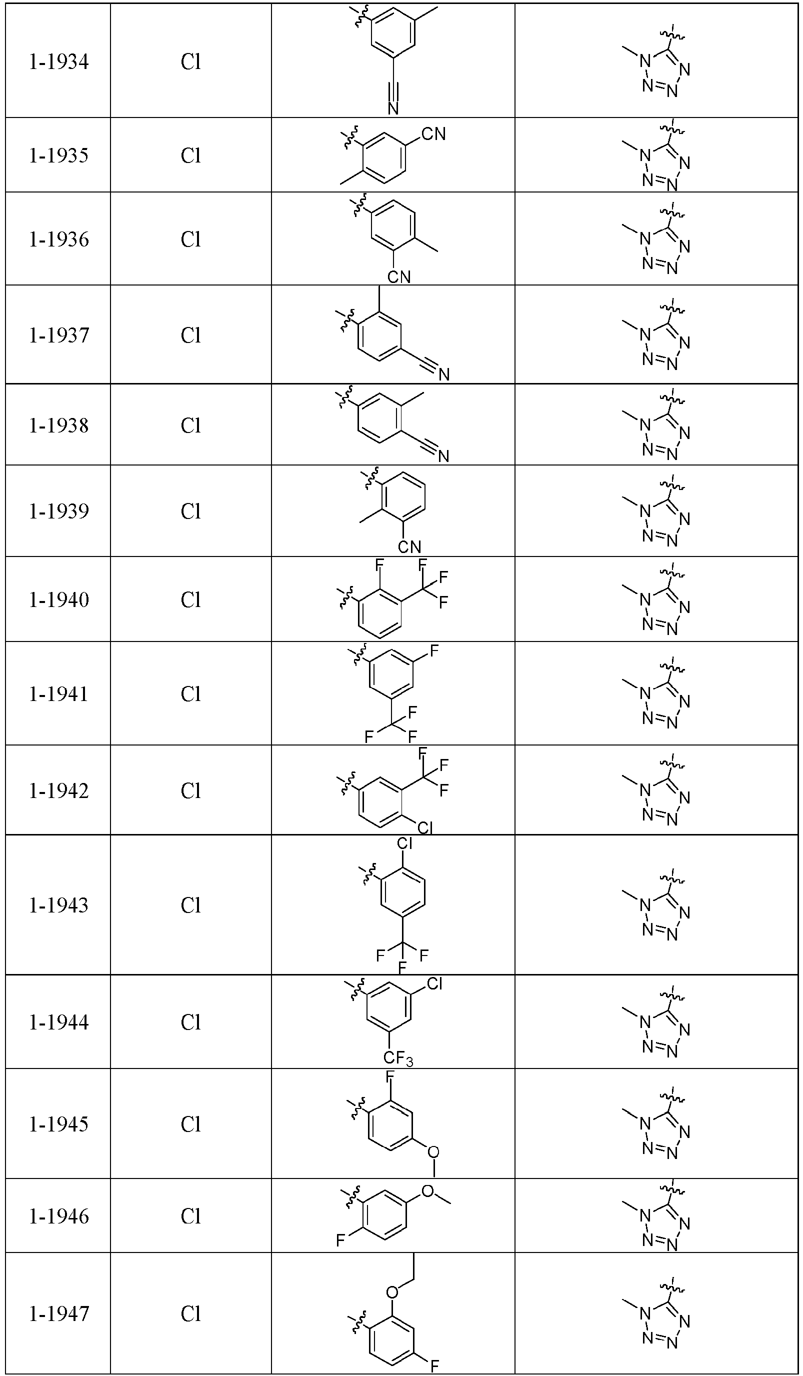

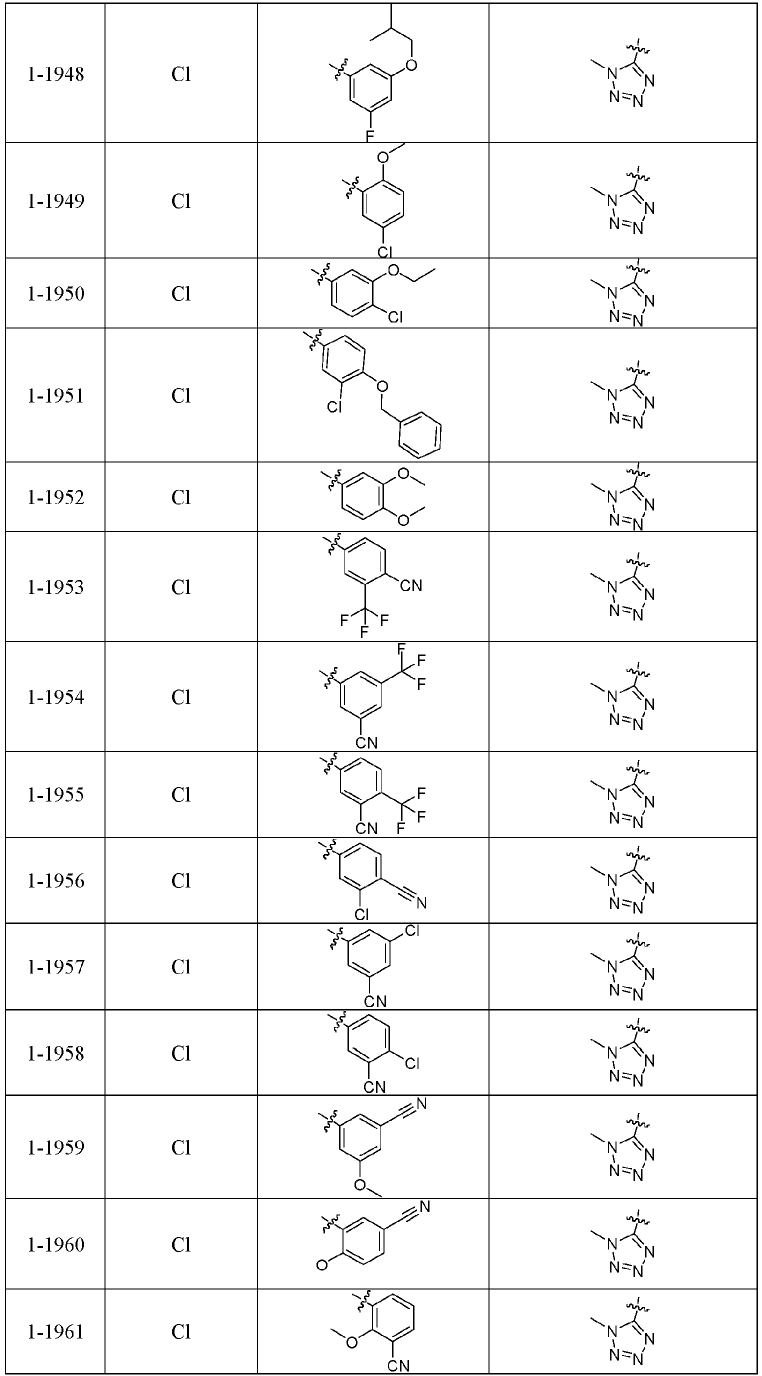

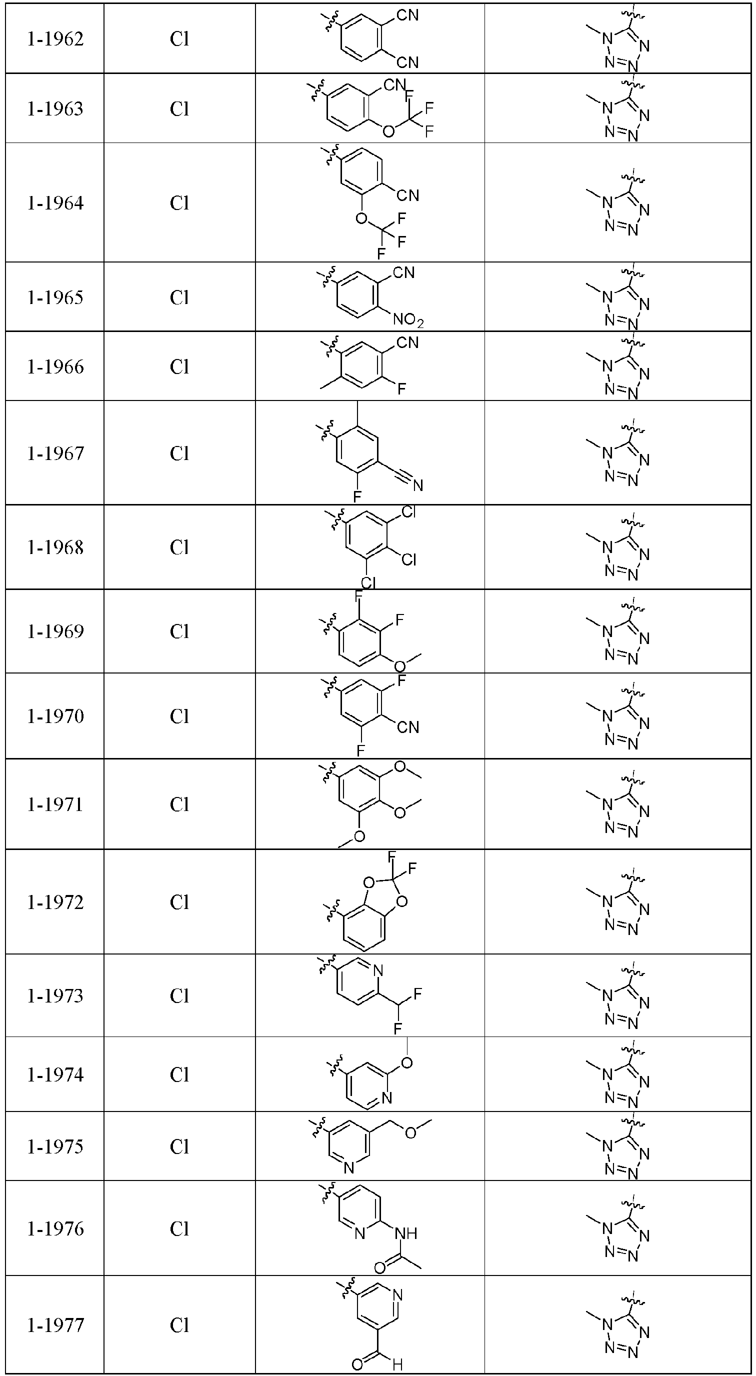

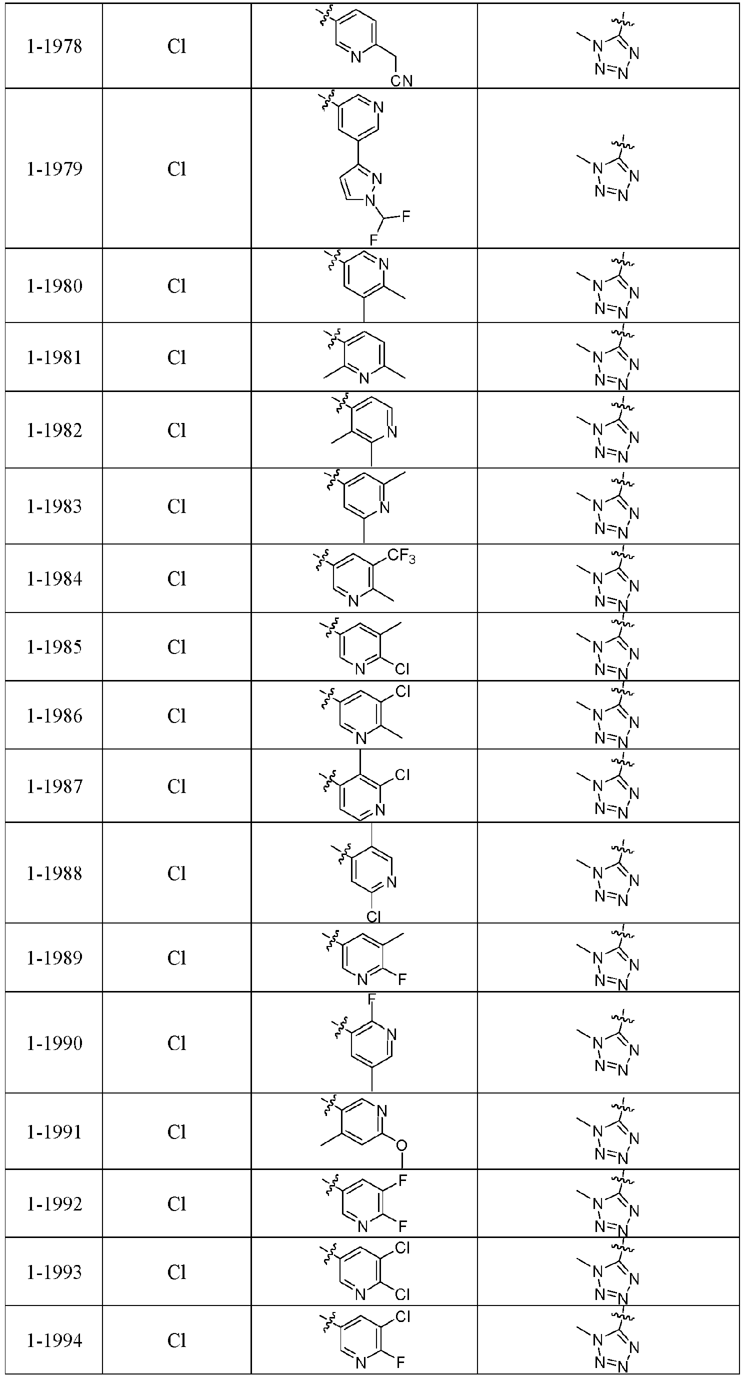

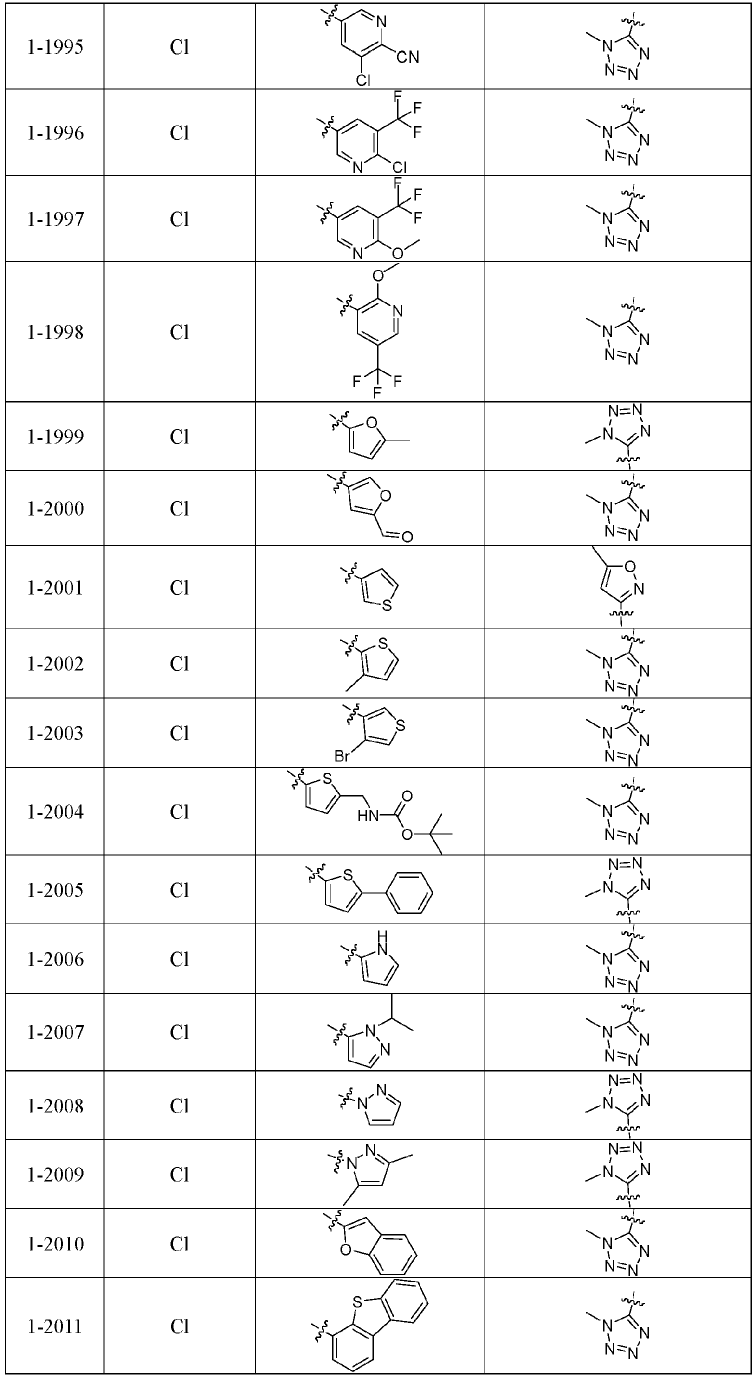

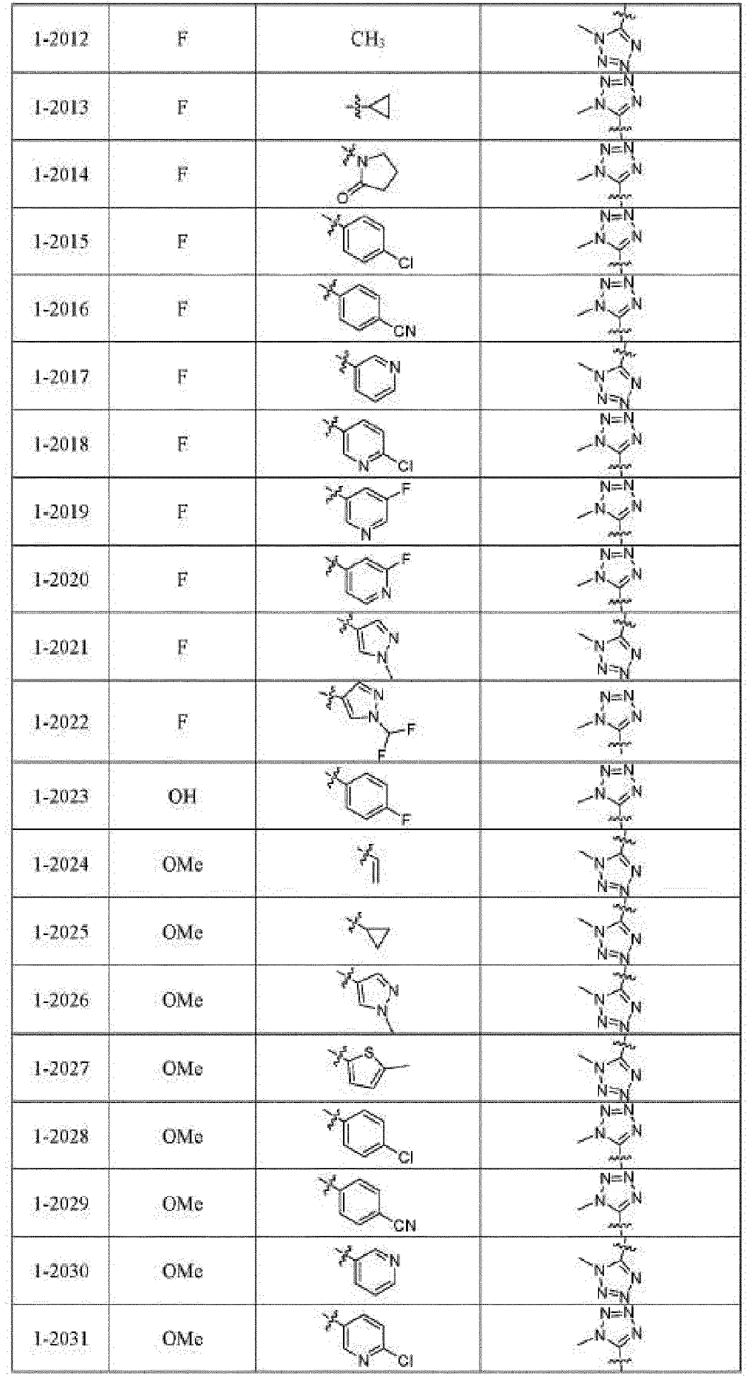

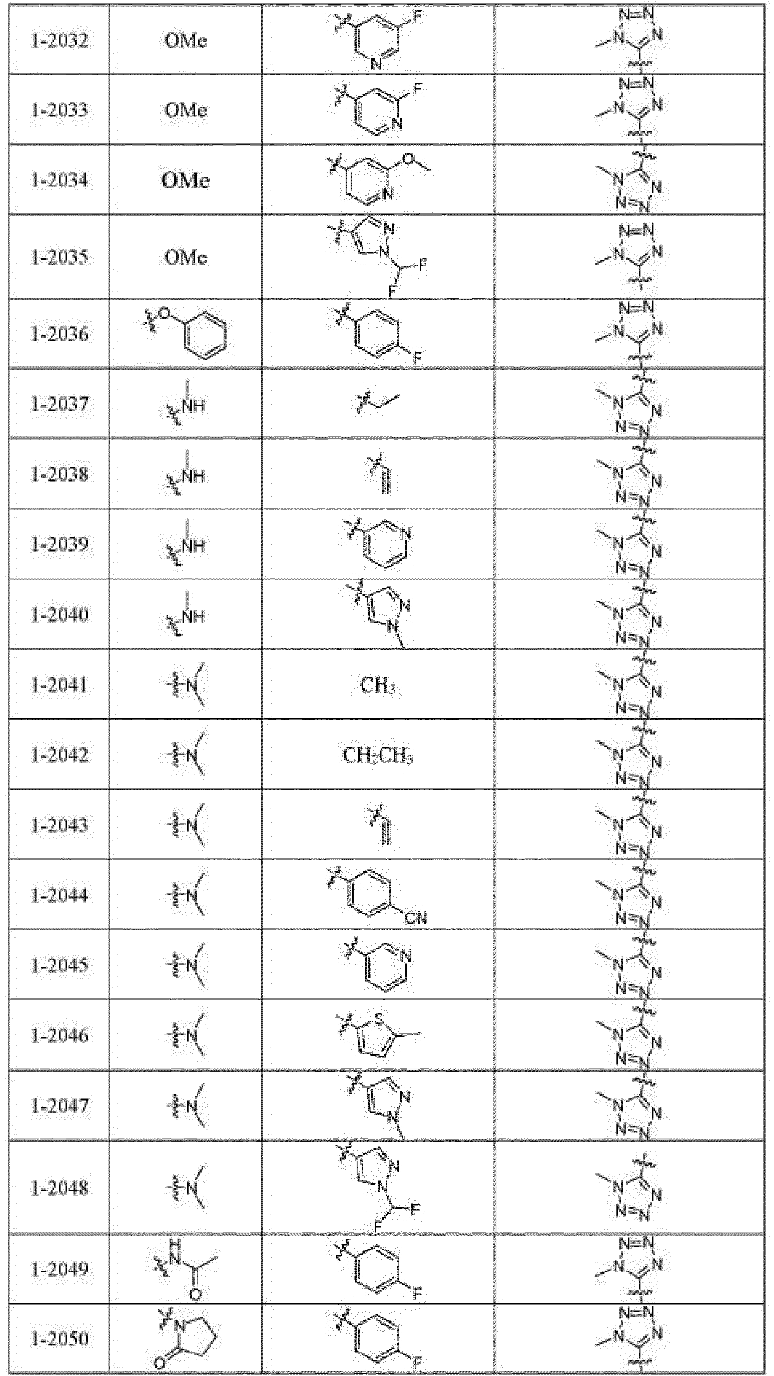

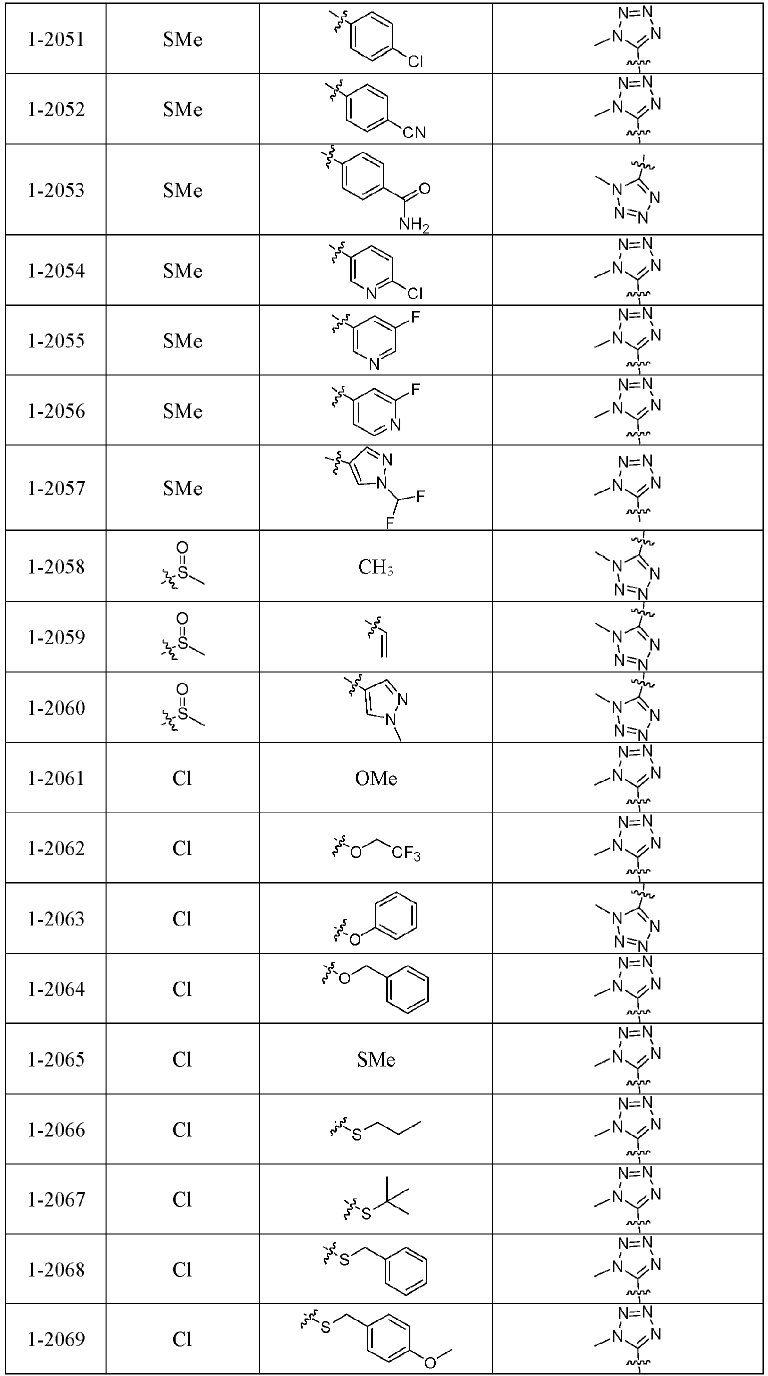

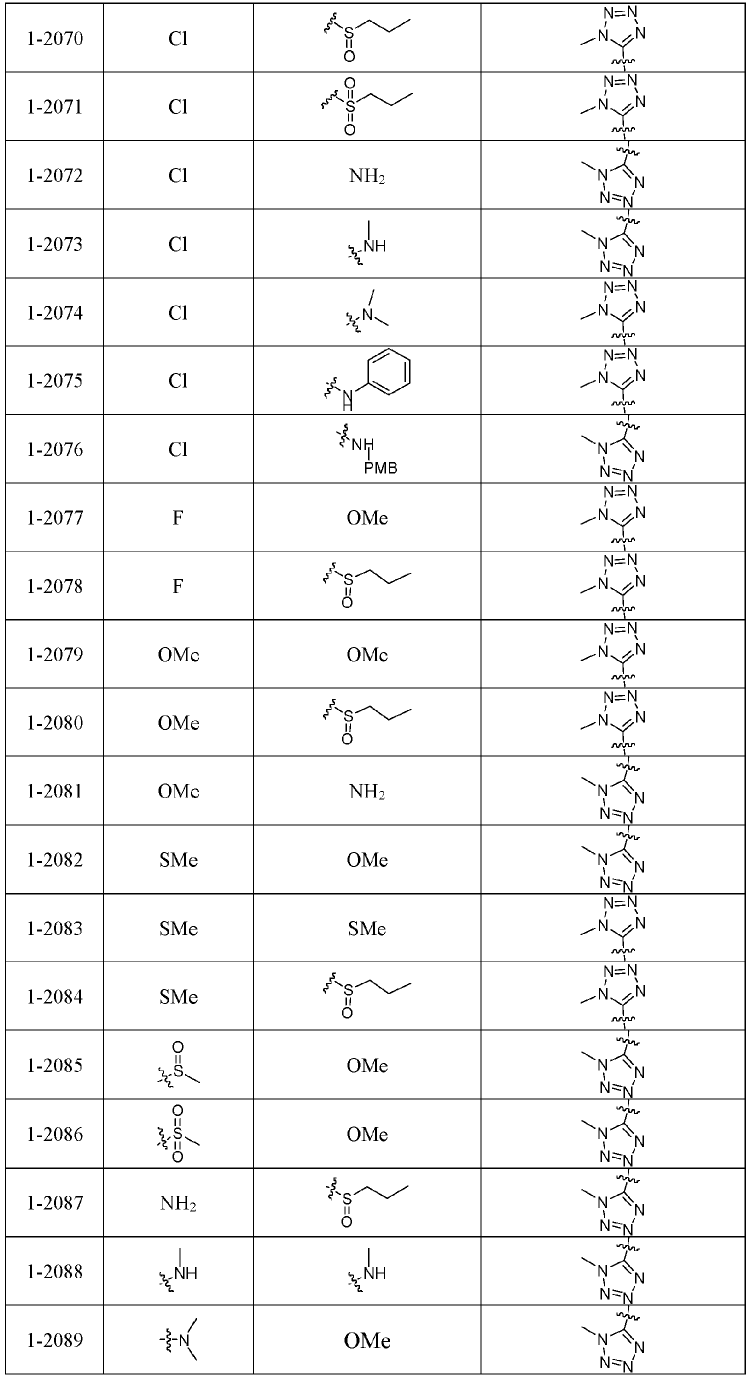

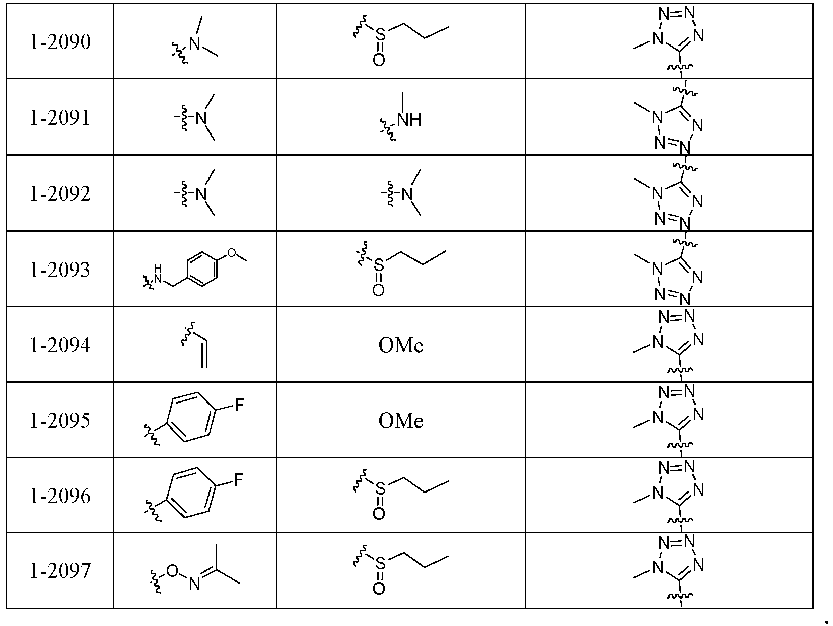

7. Procédé de préparation du composé de 4-pyridinyle formamide selon l'une quelconque des revendications 1 à 6, qui est **caractérisé en ce que**,
lorsque l'on soumet le composé de formule générale II et le composé de formule générale III à la réaction d'amidation pour obtenir le composé de formule générale I, le schéma de réaction chimique est le suivant : 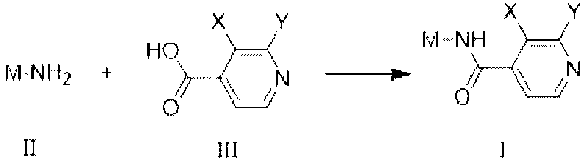
la réaction est effectuée en présence d'un agent d'halogénation, d'un catalyseur et d'un solvant ; de préférence, l'agent d'halogénation est SOCl₂, le catalyseur est 4-diméthylaminopyridine et le solvant est pyridine ;
ou la réaction est effectuée en présence d'un agent de condensation et d'un solvant ; de préférence, l'agent de condensation est CDI, DCC, DBU ou une combinaison de ceux-ci, et le solvant est le chlorure de méthylène.

8. Composition herbicide, qui est **caractérisée en ce que**, comprenant (i) le composé de 4-pyridinyle formamide ou dérivé de celui-ci selon l'une quelconque des revendications 1 à 6 ; de préférence, comprenant en outre (ii) un ou plusieurs autres herbicides et/ou phytoprotecteurs ; idéalement, comprenant en outre (iii) des auxiliaires de formulation agrochimiquement acceptable.

9. Procédé de lutte contre les mauvaises herbes, qui est **caractérisé en ce que**, comprenant l'application d'une quantité herbicide efficace d'au moins l'un du composé de 4-pyridinyle formamide ou dérivé de celui-ci selon l'une quelconque des revendications 1 à 6 ou de la composition herbicide selon la revendication 8 à une plante ou une zone de mauvaises herbes.

10. Utilisation d'au moins l'un du composé de 4-pyridinyle formamide ou dérivé de celui-ci selon l'une quelconque des revendications 1 à 6 ; ou de la composition herbicide selon la revendication 8 pour lutter contre une mauvaise herbe, de préférence, le composé de 4-pyridinyle formamide ou dérivé de celui-ci étant utilisé pour prévenir et/ou lutter contre une mauvaise herbe dans une culture utile, la culture utile étant une culture transgénique ou une culture traitée par une technique d'édition génomique.
